(19) Europäisches Patentamt
European Patent Office
Office européen des brevets

(11) EP 3 307 741 B1

(12) # EUROPÄISCHE PATENTSCHRIFT

(45) Veröffentlichungstag und Bekanntmachung des Hinweises auf die Patenterteilung:
**28.10.2020 Patentblatt 2020/44**

(21) Anmeldenummer: **16727193.1**

(22) Anmeldetag: **06.06.2016**

(51) Int Cl.:
***C07D 471/04*** *(2006.01)* ***C07D 519/00*** *(2006.01)*
***A61P 9/04*** *(2006.01)* ***A61P 13/12*** *(2006.01)*
***A61K 31/4375*** *(2006.01)* ***A61K 31/4545*** *(2006.01)*
***A61K 31/496*** *(2006.01)* ***A61K 31/513*** *(2006.01)*
***A61K 31/5377*** *(2006.01)*

(86) Internationale Anmeldenummer:
**PCT/EP2016/062737**

(87) Internationale Veröffentlichungsnummer:
**WO 2016/198342 (15.12.2016 Gazette 2016/50)**

(54) **POSITIV ALLOSTERISCHE MODULATOREN DES MUSKARINERGEN M2 REZEPTORS**
POSITIVE ALLOSTERIC MODULATORS OF THE MUSCARIC M2 RECEPTOR
MODULATEURS ALLOSTERIQUES POSITIFS DU RÉCEPTEUR MUSCARINIQUE M2

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA**

(30) Priorität: **09.06.2015 EP 15171127**
**22.02.2016 EP 16156676**

(43) Veröffentlichungstag der Anmeldung:
**18.04.2018 Patentblatt 2018/16**

(60) Teilanmeldung:
**19216135.4 / 3 692 991**

(73) Patentinhaber: **Bayer Pharma Aktiengesellschaft**
**13353 Berlin (DE)**

(72) Erfinder:
- **TELLER, Henrik**
  **18258 Schwaan (DE)**
- **STRAUB, Alexander**
  **42113 Wuppertal (DE)**
- **BRECHMANN, Markus**
  **San Francisco, CA 94114 (DE)**
- **MÜLLER, Thomas**
  **51377 Leverkusen (DE)**
- **MEININGHAUS, Mark**
  **42105 Wuppertal (DE)**
- **NOWAK-REPPEL, Katrin**
  **13187 Berlin (DE)**
- **TINEL, Hanna**
  **42113 Wuppertal (DE)**
- **MÜNTER, Klaus**
  **42489 Wülfrath (DE)**
- **FLIEGNER, Daniela**
  **13507 Berlin (DE)**
- **MONDRITZKI, Thomas**
  **42549 Velbert (DE)**
- **BOULTADAKIS ARAPINIS, Melissa**
  **40215 Düsseldorf (DE)**
- **MARQUARDT, Tobias**
  **42115 Wuppertal (DE)**
- **VAKALOPOULOS, Alexandros**
  **40721 Hilden (DE)**
- **REBSTOCK, Anne-Sophie**
  **69410 Champagne au Mont d'Or (FR)**
- **WITTWER, Matthias, Beat**
  **4125 Riehen (CH)**

(74) Vertreter: **BIP Patents**
**c/o Bayer Intellectual Property GmbH**
**Alfred-Nobel-Straße 10**
**40789 Monheim am Rhein (DE)**

(56) Entgegenhaltungen:
**EP-A1- 1 650 192 WO-A1-2005/028451**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 307 741 B1

## Beschreibung

Die vorliegende Anmeldung betrifft positiv allosterische Modulatoren des muskarinergen M2 Rezeptors, insbesondere neue 7-substituierte 1-Aryl-naphthyridin-3-carbonsäureamide, Verfahren zu ihrer Herstellung, ihre Verwendung allein oder in Kombinationen zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention von kardiovaskulären Erkrankungen und/oder Nierenerkrankungen.

Muskarinerge Rezeptoren, sind membranständige Rezeptoren, die als endogenen Liganden den Neurotransmitter Acetylcholin (ACh) binden (Acetylcholinrezeptoren), aber auch von Muskarin aktiviert werden können. Diese G-Protein-gekoppelte Rezeptoren gibt es als fünf Subtypen (M1-M5), die in fast allen Geweben im menschlichen Organismus exprimiert werden. Man findet sie sowohl im zentralen als auch im peripheren Nervensystem sowie in vielen Organen des vegetativen Nervensystems.

Der M2-Typ (M2R) wird überwiegend im Herzen exprimiert. Auf der zellulären Ebene bewirkt eine M2R Stimulation durch den Agonisten Acetylcholin eine Hemmung der Adenylcyclase und eine Aktivierung des einwärtsgleichrichtenden Kaliumkanals (IKACh-Kanal, GIRK engl.: G protein activated inwardly rectifying K+ channel; auch Kir3.x). Hierdurch vergrößert sich die Kaliumleitfähigkeit, was zu einer Hyperpolarisation der Muskelzellen führt. Dementsprechend werden die Zellen schwerer depolarisierbar, was zu einer negativ chronotropen und dromotropen Wirkung führt, so dass die Herzfrequenz sinkt. Der M2R ist der Hauptmediator der parasympathischen Kontrolle der Herzfunktion, die durch den Nervus vagus gesteuert wird. Dabei vermindert der rechte Nervus vagus über den Sinusknoten die Herzfrequenz, der linke erhöht über den Atrioventrikularknoten (AV-Knoten) überwiegend die atrioventrikuläre Überleitungszeit. Insgesamt überwiegt im Vergleich zum Sympathikus der Einfluss des Vagus auf die Ruhefrequenz des Herzens. Die Auswirkungen einer Stimulation von M2R sind somit entgegengesetzt zu denen der beta-adrenergen Stimulation..

Die Aktivierung des M2-Rezeptors durch den endogenen Agonisten Acetylcholin, aber auch durch synthetische Analoga wie Carbachol, Oxotremorin-M oder Iperoxo (Schrage et al., Biochem. Pharmacol. 2014, 90(3), 307-319) erfolgt durch die Bindung des Agonisten an die sog. orthosterische Bindestelle des Rezeptors und einer hierdurch ausgelösten Konformationsänderung des Rezeptors bzw. Stabilisierung der aktiven Rezeptorkonformation. Zu den klassischen natürlich vorkommenden Muskarin-Rezeptor-Agonisten gehören neben dem endogenen Agonisten Acetylcholin (ACh) verschiedene pflanzliche Alkaloide, wie Arecolin, Muscarin, sowie auch Pilocarpin (Neubig et al., Pharmacol Rev., 2003, 55, 597-606). Die orthosterische Bindungstelle aller muskarinergen Acetylcholin-Rezeptoren ist evolutionär stark konserviert und weist eine hohe Sequenz- und Strukturhomologie zwischen den verschiedenen Subtypen auf. Daher sind viele der bekannten Agonisten unselektiv gegenüber den verschiedenen Subtypen der muskarinergen Acetylcholin-Rezeptoren (Kruse et al., Mol Pharmacol., 2013, 84(4), 528-540). Der M2R weist neben einer orthosterischen auch eine allosterische Bindungstelle auf (Gregory et al., Current Neuropharmacol., 2007, 5(3), 157-167). Der älteste bekannte allosterische Modulator ist das Gallamin (Clark and Mitchelson, Er. J. Pharmac., 1976, 58, 323-331).

Allosterische Modulatoren weisen gegenüber klassischen, orthosterischen Liganden deutliche Unterschiede auf. Der allosterische Modulator selbst hat keinen direkten Einfluss auf die Rezeptoraktivierung. Durch die Bindung des Allosters kommt es vielmehr zu einer Modulation der Bindungsaffinität und/oder Effektivität des orthosterischen Agonisten. Die Wirkung eines allosterischen Modulators kann sich also nur in der Anwesenheit des endogenen Liganden entfalten. Daraus ergibt sich eine räumliche und zeitliche Spezifität der allosterischen Wirkung (Conn et al., Nat. Rev. Drug Disc., 2009, 8, 41-54; Conn et al, Nat. Rev. Drug. Disc., 2014, 13, 692-708). Darüber hinaus ist der Effekt eines allosterischen Modulators selbstlimitierend, wenn er in hohen Konzentrationen die Bindung des Agonisten stabilisiert. Hieraus wiederum resultiert grundsätzlich ein günstigeres sicherheitspharmakologisches Profil im Vergleich zu Agonisten, da toxische Effekte bedingt durch eine Rezeptorüberaktivierung begrenzt sind (Christopoulos, Mol. Pharmacol., 2014, 86, 463-478).

Die als Kooperativität bezeichnete gegenseitige Beeinflussung von allosterischen und orthosterischen Liganden hinsichtlich Affinität und intrinsischer Aktivität wird von beiden Liganden bestimmt. Im Falle eines positiven allosterischen Modulators des M2R werden die Effekte des ACh (orthosterischer Ligand) verstärkt (positive Kooperativität). Aufgrund ihrer Fähigkeit, Rezeptorkonformationen in Gegenwart eines orthosterischen Liganden zu modulieren, können allosterische Liganden eine Feinabstimmung pharmakologischer Effekte hervorrufen (Wang et al., J. Pharmacol. Exp. Therap., 2009, 331, 340-348). Daraus ist im Falle des positiven allosterischen Modulators des M2R ein vorteilhaftes Wirkungsprofil, reduziertes Nebenwirkungsrisiko und ein Ansatzpunkt für die Entwicklung subtypselektiver Liganden gegenüber einem vollen Agonisten zu erwarten.

Von dem positiv allosterischen M4R und M2R-Liganden LY2119620 (3-Amino-5-chlor-*N*-cyclopropyl-4-methyl-6- [2-(4-methylpiperazin-1-yl)-2-oxoethoxy]thieno [2,3 -b]pyridin-2-carboxamid) wurde die Kristallstruktur im Komplex mit dem M2R veröffentlicht. Die allosterische Bindungsstelle des M2R befindet sich räumlich benachbart, aber klar abgegrenzt zur orthosterischen Bindungsstelle und zeigt im Vergleich mit den anderen muskarinergen Rezeptor-Subtypen eine geringere Konservierung bzw. weist größere Sequenzunterschiede auf (Kruse et al., Nature, 2013, 504, 101-106). LY2119620 wurde als ein unselektiver M2R/M4R positiv allosterischer Modulator beschrieben (Croy et al.,

Molecular Pharmacology, July 2014 86, 1, 106-115; Schober et al., Molecular Pharmacology, July 2014 86, 1, 116-123).

Der M2R spielt als ein Bestandteil des autonomen Nervensystems eine wichtige Rolle in der Pathogenese und Progression von kardiovaskulären Erkrankungen. Autonomes Ungleichgewicht gekennzeichnet durch vagale (parasympathische) Abschwächung und eine Dominanz des sympathischen Nervensystems steht im engen Zusammenhang mit einer erhöhten Morbidität und Mortalität. Die klinische und prognostische Bedeutung des autonomen Ungleichgewichts ist gut dokumentiert in diversen Herz-Kreislauf-Erkrankungen, einschließlich Herzinsuffizienz (HF), Herzrhythmusstörungen, Ischämie /Reperfusion (I / R), Hypertension (He et al., Br. J. Pharmacol. 2014**,** Epub) und chronischer Nierenerkrankung (Ranpuria et al., Nephrol Dial Transplant. 2008, 23(2), 444-4499). Besonders bei Patienten mit Komorbiditäten wie Diabetes kann die autonome Imbalance zu gesteigerter Morbidität und Mortalität beitragen (Vinik et al., Diabet Med., 2011, 28(6), 643-651). Barorezeptor-Reflex-Funktionsstörungen, wie hypertensive Krisen oder Bluthochdruck-Variabilität, als Anzeichen einer Störung des autonomen Nervensystems, begleiten oft die akute Phase des ischämischen oder hämorrhagischen Schlaganfalls (Sykora et al., Stroke, 2009, 40(12), 678-682).

Die oft beobachtete Komorbidität zwischen kardiovaskulären und psychischen Erkrankungen, wie zwischen Herzinsuffizienz und Depression, basiert wahrscheinlich auf gemeinsamen Pathomechanismen, die mit der autonomen Imbalance einhergehen (Halaris et al., Mod Trends Pharmacopsychiatri., 2013, 28, 144-161). Chronischer Stress verschiebt das homöostatische Gleichgewicht des autonomen Nervensystems. Der verminderte Vagotonus trägt zu einem pro-inflammatorischen Status bei, wobei die Neurotransmitter-Regulierung, insbesondere die serotonerge Transmission, beeinträchtigt ist. Mit der autonomen Dysregulation wurden auch andere psychische Erkrankungen in Zusammenhang gebracht, wie z.B. die Aufmerksamkeitsdefizit-/Hyperaktivitätsstörung (ADHS), die sich durch Enthemmung, mangelhafte emotionale Selbstkontrolle, Unaufmerksamkeit und Hyperaktivität kennzeichnet (Rash and Aguirre-Camacho, Atten Defic Hyperact Disord., 2012, 4(4), 167-177).

Eine Stärkung der parasympathischen Aktivität durch einen positiven allosterischen Modulator einschließlich zu erwartender antiinflammatorischer Effekte, Erhöhung des Stickstoffmonoxids (NO), Regulierung des Redox-Zustands, Verbesserung der mitochondrialen Funktion und der Calcium-Regulation könnte daher ein neues Therapieprinizip insbesondere bei Herz-Kreislauferkrankungen darstellen. Es gibt zahlreiche Hinweise, dass die Modulation der parasympathischen Aktivität als potenzielles Therapieziel bei chronischer Herzinsuffizienz in Betracht gezogen werden kann. Vagusnervstimulation bei Hunden mit abgeheiltem Myokardinfarkt konnte die Inzidenz des plötzlichen Herztodes sowie bei chronisch herzinsuffizienten Ratten die Mortalität signifikant senken (De Ferrari, J. Cardiovasc. Transl. Res., 2014, 7(3), 310-320). In einem Hundemodell mit Herzinsuffizienz (LVEF 35%) und implantiertem Vagusstimulator konnte nachgewiesen werden, dass bei der Therapiegruppe im Vergleich zur Sham-Gruppe eine signifikante Verbesserung der linksventrikulären Ejektionsfraktion (LVEF) und Reduktion der endsystolischen und -diastolischen Volumina (LVESV; LVEDV) auftrat ebenso wie eine signifikante Reduktion der Herzfrequenz innerhalb von 3 Monaten. Der beschriebene Effekt der VNS war additiv zur Betablockergabe (De Ferrari, J. Cardiovasc. Transl. Res., 2014, 7(3), 310-320). Die Plasmalevel für TNF-$\alpha$ und IL-6 als auch deren myokardiale Proteinexpression konnte durch eine Vagusstimulation in diesem Tiermodel gesenkt werden, was dafür spricht, dass eine Stärkung des parasympatischen Nervensystems neben den Effekten auf LV-Remodeling auch positive Effekte auf proinflammatorische Zytokine hat.

Basierend auf den experimentellen präklinischen Daten erfolgten mittlerweile die ersten klinischen Studien zur vagalen Stimulation bei Patienten mit chronischer Herzinsuffizienz, wie bereits in der Behandlung der Epilepsie und Depression etabliert. Der Effekt der Stärkung des Parasympatikus über direkte Vagusnervstimulation (VNS) wurde in einer nicht randomisierten Beobachtungsstudie mit 32 Patienten mit einer linksventrikulären (LV) systolischen Dysfunktion bewertet, wobei die Ergebnisse darauf hindeuteten, daß eine Vagusreizung die Lebensqualität, die körperliche Belastbarkeit und das LV-Remodeling günstig beeinflusst (De Ferrari GM et al., Eur. Heart J., 2011, 32, 847-855) In der multizentrischen Open-Label-Machbarkeitsstudie ANTHEM-HF wurden die Sicherheit, Verträglichkeit und Wirksamkeit der Vagus-Stimulation bei Patienten mit chronischer stabiler, symptomatischer Herzinsuffizienz mit reduzierter Auswurffraktion (HFrEF) zusätzlich zur Standardtherapie geprüft (Premchand RK et al., J. Card. Fail., 2014, 20(11), 808-816). Die in dieser Studie angewandte kontinuierliche Vagusnervstimulation führte zu einer Verbesserung der Auswurffraktion, Herzfrequenzvariabilität, NYHA-Klasse und Lebensqualität. Die erste Placebo-kontrollierte klinische Studie NECTAR-HF konnte hingegen keine signifikante Wirkung der Vagusnervstimulation auf die Herzfunktion von HF Patienten nach 6 Monaten zeigen (Zannad et al., Eur. Heart J., 2015, 36(7), 425-433). Lediglich die Lebensqualität konnte verbessert werden. Die INOVATE-HF Studie mit 650 HF Patienten konnte keine Effekte dieser Therapie in Bezug auf die Mortalität und Hospitalisierung zeigen. (Gold et al., J Am Coll Cardiol., 2016, Mar 29. pii: S0735-1097(16)32404-4. doi: 10.1016/j.jacc.2016.03.525). Lebensqualität und Gehstrecke konnten signifikant verbessert werden.

Neben dem Infektionsrisiko und den potentiellen Risiken eines chirurgischen Eingriffs ist eine Therapie mittels elektrischer Stimulierung des Vagusnervs limitiert durch Nebenwirkungen wie: Dysphonie, Husten und Mund-/Rachenschmerzen (Premchand RK et al., J. Card. Fail., 2014, 20(11), 808-816). Eine medikamentöse Stärkung des parasympatischen Nervensystems durch eine direkte Wirkung auf den M2R könnte eine neue Therapieoption darstellen.

Vorhofflimmern ist die häufigste anhaltende Herzrhythmusstörung und ihre Prävalenz nimmt mit dem Lebensalter zu (Chen et al., Circ. Res., 2014, 114(9), 1500-1515). Oft sind Vorhofflimmern und Herzinsuffizienz miteinander

vergesellschaftet und verstärken sich gegenseitig. So nimmt die Prävalenz von Vorhofflimmern mit dem klinischen Schweregrad der Herzinsuffizienz zu (Maisel and Stevenson, Am. J. Cardiol., 2003, 91, (suppl) 2D-8D). Klinische Daten legen nahe, dass Patienten, bei denen eine Herzinsuffizienz von Vorhofflimmern begleitet wird, eine schlechte Prognose haben. Sowohl die Letalität (Gesamtletalität, plötzlicher Tod und Pumpversagen) als auch die Morbidität (Hospitalisierung) erwies sich bei dieser Patientengruppe als signifikant erhöht.

Bei der Therapie des Vorhofflimmerns werden zwei Behandlungsstrategien unterschieden: die sog. Frequenzkontrolle mit Einstellung und möglichst Normalisierung der Kammerfrequenz im Rahmen des Vorhofflimmerns und die sog. Rhythmuskontrolle, welche Maßnahmen umfasst, mit denen ein Sinusrhythmus hergestellt oder erhalten werden soll. Eine effektive Behandlung besteht aus einer Kombination von nichtmedikamentösen sowie medikamentösen oder interventionellen Maßnahmen (Levalter T, Fortbildungsprogramm Pharmazie, 2011, 5, 106-127).

Zur medikamentösen Rhythmuskontrolle nach Kardioversion werden Betablocker, Klasse-I- und Klasse-III-Antiarrhythmika nach Maßgabe der kardialen Grunderkrankung und dem Ausmaß der linksventrikulären Pumpfunktionseinschränkung eingesetzt. Bei Patienten mit permanentem Vorhofflimmern als auch bei oligosymptomatischen (oft älteren) Patienten mit persistierendem oder paroxysmalem Vorhofflimmern ist die reine Frequenzkontrolle unter Beibehaltung und Belassen des Vorhofflimmerns oft die Therapie der Wahl. Eingesetzt werden primär Medikamente, die einen Einfluss auf die Refraktärperiode bzw. die Leitungskapazität des AV-Knotens haben. Prinzipiell könnte diese Wirkung durch eine Stimulation des M2R, der physiologisch die Schlüsselrolle an dieser Stelle spielt, z.B. mit Hilfe eines positiven allosterischen Modulators erreicht werden. Bis jetzt stehen Betablocker, Digitalis, Calciumantagonisten sowie in Einzelfällen Amiodaron zur Verfügung, die unter Berücksichtigung des Lebensstils, der kardialen Grunderkrankung bzw. etwaiger Begleiterkrankungen individualisiert zum Einsatz kommen. Insbesondere bei Patienten mit reduzierter linksventrikulärer Pumpfunktion und schwerer Herzinsuffizienz sind allerdings die Möglichkeiten der medikamentösen Therapie unzureichend. Calciumantagonisten sind kontraindiziert in dieser Patientengruppe. Therapie mit Digoxin führt, wie die neuesten Studien gezeigt haben, zu einer erhöhten Mortalität von Patienten mit Vorhofflimmern (Leong-Sit and Tang, Curr. Opin. Cardiol., 2015, Epub). Für Betablocker wurde in einer Metaanalyse eine fehlende Wirksamkeit bei Patienten mit Vorhofflimmern und Herzinsuffizienz (Leong-Sit and Tang, Curr. Opin. Cardiol., 2015, Epub). Entsprechend hoch ist der medizinische Bedarf nach neuen effizienten und sicheren Therapien zur Frequenzkontrolle. Dies könnte durch eine medikamentöse Stimulation des M2R erreicht werden.

Die Aufgabe der vorliegenden Erfindung liegt in der Identifizierung und Bereitstellung neuer Substanzen, die potente, positiv allosterische Modulatoren des muskarinergen M2 Rezeptors darstellen und sich als solche zur Behandlung und/oder Prävention insbesondere von kardiovaskulären Erkrankungen und/oder Nierenerkrankungen eignen.

1-Benzyl-substituierte 4-Oxo-1,4-Dihydrochinoline-3-carbonsäuren sind als allosterische Modulatoren des M1-Muskarinrezeptors zur Behandlung von neurodegenerativen Erkrankungen wie Alzheimer und Schizophrenie beschrieben (Scammells et al., ACS Chem. Neurosci., 2013, 4 (7), 1026-1048; Mistry et al., J. Med. Chem. 2013, 56, 5151-5172). Unter anderem aus EP 0945435 B1 sind Pyridoncarbonsäurederivate bekannt, welche eine antibakterielle Antivität aufweisen. In WO 2002/085886-A2, WO 2003/050107-A1 und WO 2005/026145-A2 werden 7-Piperidino-substituierte Chinolon-Carbonsäurederivate, sowie in WO 2005/026165-A1 und WO 2005/049602-A1 verschiedene 7-Pyrrolidino-substituierte Chinolon-Carbonsäurederivate sowie in EP 1650192-A1 spezielle 7-Azetidinyl-Chinolon-Carbonsäurederivate mit einer antimikrobiellen / antibakteriellen Aktivität beansprucht. Aus WO 2005/009971-A1 und JP 2005012561 sind Chinolonderivate bekannt, welche als Thrombozytenaggregationshemmer eingesetzt werden können.

Positiv allosterische Modulatoren des muskarinergen M2 Rezeptors können bei der Behandlung und/oder Prävention von Krankheiten, insbesondere von kardiovaskulären Erkrankungen und/oder Nierenerkrankungen verwendet werden.

Die Erfinder haben überraschend gefunden, dass sich die positiv allosterische Modulation des muskarinergen M2 Rezeptors besonders für die Behandlung kardiovaskulärer Erkrankungen, bevorzugt gemäß der zuvorgenannten Liste von Indikationen, eignet.

Der positiv allosterische M4R und M2R-Ligand LY2119620 wird vorwiegend mit neuronalen und psychischen Erkrankungen in Verbindung gebracht (Croy et al., Molecular Pharmacology, July 2014, 86, 1, 106-115). Moleküle mit einem Profil, das dem von LY2119620 enspricht oder ähnelt, sind also für eine selektiv allosterische Modulation des muskarinergen M2 Rezeptors, und damit eine nebenwirkungsarme Behandlung kardiovaskulärer Erkrankungen gemäß der zuvorgenannten Liste von Indikationen, nicht geeignet.

Die positiv allosterischen Modulatoren des muskarinergen M2 Rezeptors weisen eine Subtyp-Selektivität für den M2-Rezeptor hinsichtlich der positiv-allosterischen Wirkung auf.

Diese weisen in einem Konzentrationsbereich von 1 $\mu$M - 10 $\mu$M eine identische oder höhere Selektivität gegenüber dem muskarinergen M2-Rezeptor als gegenüber dem muskarinergen M4-Rezeptor auf. Bevorzugt ist ferner vorgesehen, dass die Selektivität des allosterischen Modulators gegenüber dem muskarinergen M2-Rezeptor mindestens 1,1-fach, 1,2-fach, 1,3-fach, oder besonders bevorzugt, 1,4-fach höher ist als gegenüber dem muskarinergen M4-Rezeptor.

Diese weisen in einem Konzentrationsbereich von 5 $\mu$M - 20 $\mu$M eine mindestens 4-fach höhere Selektivität

gegenüber dem muskarinergen M2-Rezeptor als gegenüber dem muskarinergen M1-Rezeptor auf. Bevorzugt ist vorgesehen, dass die Selektivität des allosterischen Modulators gegenüber dem muskarinergen M2-Rezeptor mindestens 4,2-fach, 4,3-fach, 4,4-fach, 4,5-fach, 4,6-fach, 4,7-fach, 4,8-fach, 4,9-fach, 5-fach, 5,1-fach, 5,2-fach, 5,3-fach, 5,4-fach, 5,5-fach, 5,6-fach, 5,7-fach, oder besonders bevorzugt 5,8-fach höher ist als gegenüber dem muskarinergen M1-Rezeptor.

Die Selektivität wird hierbei determiniert als Quotient der jeweiligen modulatorbedingten allosterischen Verschiebung des $EC_{50}$-Wertes der ACh-Dosis-Wirkungskurve für den M2-Rezeptor relativ zu dem jeweiligen anderen Mx-Rezeptortyp. Zur Determinierung des besagten Quotienten wird zunächst der $EC_{50}$ -Wert der ACh-Dosis-Wirkungskurve für die jeweiligen Rezeptoren bestimmt ("$EC_{50}$ ACh"). Anschließend wird die allosterische Verschiebung des $EC_{50}$-Wertes von ACh ("Shift $EC_{50}$") nach Gabe von 1 μM oder 10 μM des zu testenden allosterischen Modulators bestimmt. Hierzu eignet sich insbesondere das Protokoll des auf Seite 610-612, Abschnitt B-3. beschriebenen funktionellen $Ca^{2+}$ Freisetzungstests der Firma Eurofins (GPCRProfiler® "Services in agonistic and allosteric mode for Mx Receptors"). Abschließend

werden Quotienten der allosterischen Verschiebung für den M2-Rezeptor relativ zu dem jeweiligen Mx-Rezeptor (z.B. M1R, M4R) gebildet, die ihrerseits als Maß für die jeweilige Selektivität fungieren.

Die Erfindung betrifft insbesondere Verbindungen der allgemeinen Formel (I)

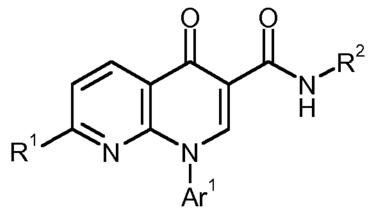

(I),

in welcher

$R^1$ für $NR^4R^5$ steht,

worin

$R^4$ Wasserstoff, Methyl, $(C_2-C_4)$-Alkyl oder $(C_3-C_6)$-Cycloalkyl bedeutet, wobei $(C_2-C_4)$-Alkyl mit Hydroxy oder bis zu dreifach mit Fluor substituiert sein kann

und

$R^5$ $(C_1-C_6)$-Alkyl, $(C_3-C_6)$-Cycloalkyl, 3- bis 6-gliedriges gesättigtes Heterocyclyl oder $(C_1-C_4)$-Alkylsulfonyl bedeutet,

wobei $(C_1-C_6)$-Alkyl, $(C_3-C_6)$-Cycloalkyl und 3- bis 6-gliedriges gesättigtes Heterocyclyl, bis zu dreifach, gleich oder verschieden, mit Methyl, Difluormethyl, Trifluormethyl, Hydroxy, Hydroxycarbonyl, Oxo, Methoxy, Difluormethoxy, Trifluormethoxy, Cyano und weiterhin bis zu vierfach mit Fluor, substituiert sein können,

oder

$R^4$ und $R^5$ bilden zusammen mit dem Stickstoffatom, an das sie gebunden sind, einen gesättigten oder partiell ungesättigten, 3- bis 6-gliedrigen monocyclischen oder 6- bis 10-gliedrigen bicyclischen Heterocyclus, der ein oder zwei weitere, gleiche oder verschiedene Heteroatome aus der Reihe N, O, S, SO und/oder $SO_2$ als Ringglieder enthalten kann,

wobei der 3- bis 6-gliedrige monocyclische und der 6- bis 10-gliedrige bicyclische Heterocyclus jeweils mit 1 bis 5 Substituenten unabhängig voneinander ausgewählt aus der Gruppe $(C_1-C_4)$-Alkyl, Difluormethyl, Trifluormethyl, Hydroxy, Hydroxycarbonyl, Oxo, $(C_1-C_3)$-Alkoxy, Difluormethoxy, Trifluormethoxy, Cyano, $(C_1-C_3)$-Alkoxycarbonyl, Aminocarbonyl, Mono-$(C_1-C_3)$-alkylaminocarbonyloxy, -NHC(=O)$R^{22A}$, -CHzNHC(=O)$R^{22B}$, und weiterhin bis zu vierfach mit Fluor, substituiert sein können, worin

$R^{22A}$ und $R^{22B}$ unabhängig voneinander $(C_1-C_3)$-Alkyl oder Cyclopropyl darstellen,

und

worin $(C_1-C_4)$-Alkyl ein- oder zweifach, gleich oder verschieden, mit Hydroxy, $(C_1-C_3)$-Alkoxy, und bis zu vierfach mit Fluor, substituiert sein kann,

$R^2$ für eine Gruppe der Formel

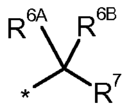
,
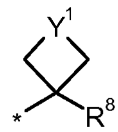

oder *-$L^1$-$Ar^2$
steht, worin
* die Verknüpfungsstelle mit dem N-Atom der Amid-Gruppierung markiert,
$R^{6A}$ Wasserstoff oder ($C_1$-$C_4$)-Alkyl bedeutet,
$R^{6B}$ Wasserstoff, ($C_1$-$C_4$)-Alkyl, Cyclopropyl, Trifluormethyl, Methoxymethyl oder Trifluormethoxymethyl bedeutet,
$R^7$ ($C_1$-$C_4$)-Alkyl, Cyclopropyl oder Cyclobutyl bedeutet,
wobei ($C_1$-$C_4$)-Alkyl bis zu fünffach und Cyclopropyl und Cyclobutyl bis zu vierfach mit Fluor substituiert sein können,
$Y^1$ -$(CH_2)_k$-, -$CF_2$-, -O-$CH_2$-, -$CH_2$-O- oder -$CH_2$-O-$CH_2$- bedeutet,
worin
k für 0, 1, 2 oder 3 steht,
$R^8$ bis zu fünffach mit Fluor substituiertes ($C_1$-$C_2$)-Alkyl oder Trifluormethoxymethyl bedeutet,
$L^1$ eine Bindung oder eine Gruppe der Formel -$C(R^{9A}R^{9B})$-$(C(R^{10A}R^{10B}))_m$ bedeutet,
worin
m 0 oder 1 darstellt,
$R^{9A}$ Wasserstoff oder Methyl darstellt,
$R^{9B}$ Wasserstoff, Methyl, Trifluormethyl, Pentafluorethyl oder Trifluormethoxymethyl darstellt,
$R^{10A}$ und $R^{10b}$ unabhängig voneinander Wasserstoff oder Methyl darstellen,
$Ar^2$ Phenyl bedeutet, wobei Phenyl ein- bis dreifach, gleich oder verschieden, mit Fluor, Chlor, ($C_1$-$C_3$)-Alkyl, Difluormethoxymethyl, Trifluormethoxymethyl und/oder Trifluormethyl substituiert sein kann,oder
für einen 5- bis 10-gliedrigen bicyclischen oder tricyclischen Carbocyclus steht,
wobei der 5- bis 10-gliedrige bicyclische oder tricyclische Carbocyclus bis zu dreifach, gleich oder verschieden, mit ($C_1$-$C_3$)-Alkyl, Trifluormethyl, und weiterhin bis zu vierfach mit Fluor substituiert sein kann,

$Ar^1$ für eine Gruppe der Formel

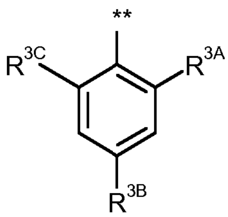

steht, worin
** die Verknüpfungsstelle mit dem N-Atom markiert,
$R^{3A}$ für Fluor, Chlor oder Trifluormethyl steht,
$R^{3B}$ für Wasserstoff oder Fluor steht
und
$R^{3C}$ für Wasserstoff, Fluor oder Chlor steht

sowie ihre *N*-Oxide, Salze, Solvate, Salze der *N*-Oxide und Solvate der *N*-Oxide und Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Erfindungsgemäße Verbindungen sind ebenso *N*-Oxide der Verbindungen der Formel (I) sowie deren Salze, Solvate und Solvate der Salze.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung, Reinigung oder Lagerung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze), Zinksalze sowie Ammoniumsalze abgeleitet von Ammoniak oder organischen Aminen mit 1 bis 16 C-Atomen, wie beispielhaft und vorzugsweise Ethylamin, Diethylamin, Triethylamin, DIPEA, Monoethanolamin, Diethanolamin, Triethanolamin, Dimethylaminoethanol, Diethylaminoethanol, Tris(hydroxymethyl)aminomethan, Cholin, Prokain, Dicyclohexylamin, Dibenzylamin, *N*-Methylmorpholin, *N*-Methylpiperidin, Arginin, Lysin und 1,2-Ethylendiamin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere sowie ihre jeweiligen Mischungen. Aus solchen Mischungen von Enantiomeren und/ oder Diastereomeren lassen sich die stereoisomer einheitlichen Bestandteile in bekannter Weise isolieren. Vorzugsweise werden hierfür chromatographische Verfahren angewandt, insbesondere die HPLC-Chromatographie an achiralen bzw. chiralen Trennphasen. Im Falle von Carbonsäuren als Zwischen- oder Endprodukten kann alternativ auch eine Trennung über diastereomere Salze mit Hilfe chiraler Amin-Basen erfolgen.

Der Begriff "enantiomerenrein" wird im Rahmen der vorliegenden Erfindung dahingehend verstanden, dass die betreffende Verbindung hinsichtlich der Absolutkonfiguration der chiralen Zentren in einem Enantiomerenüberschuss von mehr als 95%, bevorzugt von mehr als 98% vorliegt. Der Enantiomerenüberschuss (engl. *enantiomeric excess,* ee-Wert) wird hierbei durch Auswertung des Chromatogramms einer HPLC-Analyse an chiraler Phase nach der folgenden Formel berechnet:

$$ee = \left| \frac{\text{Enantiomer 1 (Flächenprozent)} - \text{Enantiomer 2 (Flächenprozent)}}{\text{Enantiomer 1 (Flächenprozent)} + \text{Enantiomer 2 (Flächenprozent)}} \right| \text{ x } 100\%.$$

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ("unnatürlicher Anteil") ausgetauscht ist. Unter dem Ausdruck "unnatürlicher Anteil" ist ein Anteil eines derartigen Isotops zu verstehen, der höher als dessen natürliche Häufigkeit ist. Die in diesem Zusammenhang anzuwendenden natürlichen Häufigkeiten von Isotopen finden sich in "Isotopic Compositions of the Elements 1997", Pure Appl. Chem., 70(1), 217-235, 1998. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie $^{2}H$ (Deuterium), $^{3}H$ (Tritium), $^{13}C$, $^{14}C$, $^{15}N$, $^{17}O$, $^{18}O$, $^{32}P$, $^{33}P$, $^{33}S$, $^{34}S$, $^{35}S$, $^{36}S$, $^{18}F$ , $^{36}Cl$, $^{82}Br$, $^{123}I$, $^{124}I$, $^{129}I$ und $^{131}I$. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit $^{3}H$- oder $^{14}C$-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise zu einer Verlängerung der Halbwertszeit im Körper oder zu einer Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Im Hinblick auf die Behandlung und/oder Prophylaxe der hier angegebenen Störungen enthalten die isotopischen Variante(n) der Verbindungen der allgemeinen Formel (I) vorzugsweise Deuterium ("deuteriumhaltige Verbindungen der allgemeinen Formel (I)"). Isotopische Varianten der Verbindungen der allgemeinen Formel (I), in die ein oder mehrere radioaktive Isotope, wie $^{3}H$ oder $^{14}C$, eingebaut sind, sind beispielsweise bei Arzneistoff- und/oder

Substratsgewebeverteilungsstudien von Nutzen. Diese Isotope sind wegen ihrer leichten Einbaubarkeit und Nachweisbarkeit besonders bevorzugt. In eine Verbindung der allgemeinen Formel (I) können Positronen emittierende Isotope wie $^{18}F$ oder $^{11}C$ eingebaut werden. Diese isotopischen Varianten der Verbindungen der allgemeinen Formel (I) eignen sich zur Verwendung bei in-vivo-Bildgebungsanwendungen. Deuteriumhaltige und $^{13}C$-haltige Verbindungen der allgemeinen Formel (I) können im Rahmen präklinischer oder klinischer Studien bei Massenspektrometrie-Analysen verwendet werden (H. J. Leis et al., Curr. Org. Chem., 1998, 2, 131). Isotopische Varianten der erfindungsgemäßen Verbindungen können nach allgemein gebräuchlichen, dem Fachmann bekannten Verfahren hergestellt werden, so beispielsweise nach den weiter unten beschriebenen Methoden und den bei den Ausführungsbeispielen wiedergegebenen Vorschriften, indem hierbei entsprechende isotopische Modifikationen der jeweiligen Reagentien und/oder Ausgangsverbindungen eingesetzt werden.

Isotopische Varianten der Verbindungen der allgemeinen Formel (I) können im Allgemeinen nach dem Fachmann bekannten Verfahren wie in den hier beschriebenen Schemata und/oder Beispielen beschrieben hergestellt werden, indem man ein Reagenz durch eine isotopische Variante des Reagenzes, vorzugsweise ein deuteriumhaltiges Reagenz, ersetzt. Je nach den gewünschten Deuterierungsstellen kann in einigen Fällen Deuterium aus $D_2O$ entweder direkt in die Verbindungen oder in Reagenzien, die für die Synthese derartiger Verbindungen verwendet werden können, eingebaut werden (Esaki et al., Tetrahedron, 2006, 62, 10954; Esaki et al., Chem. Eur. J., 2007, 13, 4052). Ein nützliches Reagenz zum Einbau von Deuterium in Moleküle ist auch Deuteriumgas. Eine schnelle Route zum Einbau von Deuterium ist die katalytische Deuterierung von olefinischen Bindungen (H. J. Leis et al., Curr. Org. Chem., 1998, 2, 131; J. R. Morandi et al., J. Org. Chem., 1969, 34 (6), 1889) und acetylenischen Bindungen (N. H. Khan, J. Am. Chem. Soc., 1952, 74 (12), 3018; S. Chandrasekhar et al., Tetrahedron, 2011, 52, 3865). Zum direkten Austausch von Wasserstoff gegen Deuterium in funktionelle Gruppen enthaltenden Kohlenwasserstoffen können auch Metallkatalysatoren (d.h. Pd, Pt und Rh) in Gegenwart von Deuteriumgas verwendet werden (J. G. Atkinson et al., US-Patent 3966781). Verschiedene deuterierte Reagenzien und Synthesebausteine sind im Handel von Firmen wie beispielsweise C/D/N Isotopes, Quebec, Kanada; Cambridge Isotope Laboratories Inc., Andover, MA, USA; und CombiPhos Catalysts, Inc., Princeton, NJ, USA, erhältlich. Weitere Informationen bezüglich des Standes der Technik im Hinblick auf Deuterium-Wasserstoff-Austausch finden sich beispielsweise in Hanzlik et al., J. Org. Chem., 1990, 55, 3992-3997; R. P. Hanzlik et al., Biochem. Biophys. Res. Commun., 1989, 160, 844; P. J. Reider et al., J. Org. Chem., 1987, 52, 3326-3334; M. Jarman et al., Carcinogenesis, 1993, 16(4), 683-688; J. Atzrodt et al., Angew. Chem., Int. Ed. 2007, 46, 7744; K. Matoishi et al., 2000, J. Chem. Soc, Chem. Commun., 1519-1520; K. Kassahun et al., WO 2012/112363.

Der Begriff "deuteriumhaltige Verbindung der allgemeinen Formel (I)" ist definiert als eine Verbindung der allgemeinen Formel (I), in der ein oder mehrere Wasserstoffatome durch ein oder mehrere Deuteriumatome ersetzt sind und in der die Häufigkeit von Deuterium in jeder deuterierten Position der Verbindung der allgemeinen Formel (I) höher ist als die natürliche Häufigkeit von Deuterium, die etwa 0,015% beträgt. Insbesondere ist in einer deuteriumhaltigen Verbindung der allgemeinen Formel (I) die Häufigkeit von Deuterium in jeder deuterierten Position der Verbindung der allgemeinen Formel (I) höher als 10%, 20%, 30%, 40%, 50%, 60%, 70% oder 80%, vorzugsweise höher als 90%, 95%, 96% oder 97%, noch weiter bevorzugt höher als 98% oder 99% in dieser Position bzw. diesen Positionen. Es versteht sich, dass die Häufigkeit von Deuterium in jeder deuterierten Position von der Häufigkeit von Deuterium in anderen deuterierten Positionen unabhängig ist.

Durch den selektiven Einbau eines oder mehrerer Deuteriumatome in eine Verbindung der allgemeinen Formel (I) können die physikalisch-chemischen Eigenschaften (wie beispielsweise Acidität [A. Streitwieser et al., J. Am. Chem. Soc., 1963, 85, 2759; C. L. Perrin et al., J. Am. Chem. Soc., 2007, 129, 4490], Basizität [C. L. Perrin, et al., J. Am. Chem. Soc., 2003, 125, 15008; C. L. Perrin in Advances in Physical Organic Chemistry, 44, 144; C. L. Perrin et al., J. Am. Chem. Soc., 2005, 127, 9641], Lipophilie [B. Testa et al., Int. J, Pharm., 1984, 19(3), 271]) und/oder das Stoffwechselprofil des Moleküls verändert und Veränderungen des Verhältnisses von Stammverbindung zu Metaboliten oder der gebildeten Mengen von Metaboliten verursacht werden. Derartige Veränderungen können zu bestimmten therapeutischen Vorteilen führen und daher unter bestimmten Umständen bevorzugt sein. Es ist über verringerte Stoffwechselraten und Stoffwechselumschaltung, bei der sich das Verhältnis von Metaboliten ändert, berichtet worden (D. J. Kushner et al., Can. J. Physiol. Pharmacol., 1999, 77, 79; A. E. Mutlib et al., Toxicol. Appl. Pharmacol., 2000, 169, 102). Diese Veränderungen der Exposition gegenüber Stammverbindung und Metaboliten können wichtige Konsequenzen hinsichtlich Pharmakodynamik, Verträglichkeit und Wirksamkeit einer deuteriumhaltigen Verbindung der allgemeinen Formel (I) haben. In einigen Fällen wird durch den Deuteriumersatz die Bildung eines unerwünschten oder toxischen Metaboliten verringert oder eliminiert und die Bildung eines gewünschten Metaboliten verstärkt (z.B. Nevirapin: A. M. Sharma et al., Chem. Res.Toxicol., 2013, 26, 410; Uetrecht et al., Chemical Research in Toxicology, 2008, 21, 9, 1862; Efavirenz: A. E. Mutlib et al., Toxicol. Appl. Pharmacol., 2000, 169, 102). In anderen Fällen besteht der Haupteffekt der Deuterierung darin, die Geschwindigkeit der systemischen Clearance zu verringern. Dadurch wird die biologische Halbwertszeit der Verbindung erhöht. Zu den potentiellen klinischen Vorteilen gehören die Fähigkeit zur Aufrechterhaltung einer ähnlichen systemischen Exposition mit verringerten Spitzenspiegeln und erhöhten Talspiegeln. Dies könnte je nach der Pharmakokinetik/Pharmakodynamik-Beziehung der jeweiligen Verbindung zu geringeren Nebenwirkungen und erhöhter Wirksamkeit führen. Beispiele für diesen Deuterium-Effekt sind Indiplon (A. J. Morales et al., Abstract 285, The 15th North American Meeting of the International Society of Xenobiotics, San Diego, CA, 12.-16. Oktober, 2008), ML-337 (C. J. Wenthur et al., J. Med. Chem., 2013, 56, 5208) und Odanacatib (K. Kassahun et al., WO2012/112363). Es ist auch noch über weitere Fälle berichtet worden, bei denen verringerte Verstoffwechslungsraten zu einer Erhöhung der Arzneistoffexposition ohne Änderung der Rate der systemischen Clearance führen (z.B. Rofecoxib: F. Schneider et al., Arzneim. Forsch. Drug. Res., 2006, 56, 295; Telaprevir: F. Maltais et al., J. Med. Chem., 2009, 52, 7993). Deuterierte Arzneistoffe, die diesen Effekt zeigen, können verringerte Dosierungsanforderungen haben (z.B. geringere Zahl von Dosen oder niedrigere Dosierung zur Erzielung der gewünschten Wirkung) und/oder zu geringeren Metabolitenbelastungen führen.

Eine Verbindung der allgemeinen Formel (I) kann mehrere potenzielle Angriffsstellen für die Verstoffwechslung aufweisen. Zur Optimierung der oben beschriebenen Effekte auf die physikalisch-chemischen Eigenschaften und das Stoffwechselprofil können deuteriumhaltige Verbindungen der allgemeinen Formel (I) mit einem bestimmten Muster eines oder mehrerer Deuterium-Wasserstoff-Austausche gewählt werden. Insbesondere ist/sind das Deuteriumatom/die Deuteriumatome deuteriumhaltiger Verbindung(en) der allgemeinen Formel (I) an ein Kohlenstoffatom gebunden und/oder steht/stehen in den Positionen der Verbindung der allgemeinen Formel (I), bei denen es sich um Angriffsstellen für Verstoffwechslungsenzyme wie z.B. Cytochrom $P_{450}$ handelt.

Im Rahmen der vorliegenden Erfindung haben die Substituenten, soweit nicht anders spezifiziert, die folgende Bedeutung:

Alkyl per se und "Alk" und "Alkyl" in Alkoxy, Alkylsulfonyl, Alkylaminocarbonyloxy und Alkoxycarbonyl stehen für einen linearen oder verzweigten Alkylrest mit in der Regel 1 bis 6, vorzugsweise 1 bis 4 Kohlenstoffatomen, beispielhaft und vorzugsweise für Methyl, Ethyl, n-Propyl, Isopropyl, tert.-Butyl, isoButyl (2-Methyl-prop-1-yl), n-Pentyl und n-Hexyl.

Alkoxy steht beispielhaft und vorzugsweise für Methoxy, Ethoxy, n-Propoxy, Isopropoxy, tert.-Butoxy, n-Pentoxy und n-Hexoxy.

Alkylaminocarbonyloxy steht für einen Alkylaminocarbonyloxyrest mit einem oder zwei (unabhängig voneinander gewählten) Alkylsubstituenten. ($C_1$-$C_3$)-Alkylaminocarbonyloxy steht beispielsweise für einen Monoalkylaminocarbonyloxyrest mit 1 bis 3 Kohlenstoffatomen oder für einen Dialkylaminocarbonyloxyrest mit jeweils 1 bis 3 Kohlenstoffatomen pro Alkylsubstituent. Beispielhaft und vorzugsweise seien genannt: Methylaminocarbonyloxy, Ethylaminocarbonyloxy, n-Propylaminocarbonyloxy, Isopropylaminocarbonyloxy, tert.-Butylaminocarbonyloxy, n-Pentylaminocarbonyloxy, n-Hexylaminocarbonyloxy, *N,N*-Dimethylaminocarbonyloxy, *N,N*-Diethylaminocarbonyloxy, *N*-Ethyl-*N*-methylaminocarbonyloxy, *N*-Methyl-*N*-n-propylaminocarbonyloxy, *N*-Isopropyl-*N*-n-propylaminocarbonyloxy, *N*-tert.-Butyl-*N*-methylaminocarbonyl, *N*-Ethyl-*N*-n-pentylamino-carbonyl und *N*-n-Hexyl-*N*-methylaminocarbonyloxy.

Alkylsulfonyl steht in Rahmen der Erfindung für einen linearen oder verzweigten Alkylrest mit 1 bis 4 Kohlenstoffatomen, der über eine Sulfonylgruppe gebunden ist. Beispielhaft und vorzugsweise seinen genannt: Methylsulfonyl, Ethylsulfonyl, n-Propylsulfonyl, iso-Propylsulfonyl, n-Butylsulfonyl und tert.-Butylsulfonyl.

Alkoxycarbonyl steht beispielhaft und vorzugsweise für Methoxycarbonyl, Ethoxycarbonyl, n-Propoxycarbonyl, Isopropoxycarbonyl, tert.-Butoxycarbonyl, n-Pentoxycarbonyl und n-Hexoxycarbonyl.

Carbocyclus steht im Rahmen der Erfindung für einen mono-, bi-, tri- oder spirocyclischen, gesättigten oder partiell ungesättigten Carbocyclus mit insgesamt 3 bis 10 Ringatomen und bis zu 2 Doppelbindungen. Ein monocyclischer, gesättigter Carbocyclus wird synonym mit Cycloalkyl bezeichnet. Beispielhaft seien genannt: Cyclopropyl, Cyclobutyl, Cyclopentyl, Cyclohexyl, Cycloheptyl, Cyclopentenyl, Cyclohexenyl, Cyclohexadienyl, Cycloheptenyl, Cycloheptadienyl, Spiro[2.3]hexyl, Spiro[2.4]heptyl, Spiro[2.5]oktyl, Bicyclo[1.1.1]pentyl, Bicyclo[2.2.1]heptyl, Bicyclo[2.2.2]oktyl, Tricyclo[3.3.1.1$^{3,7}$]-decyl. Bevorzugt ist monocyclisches Cycloalkyl mit 3 bis 6 Kohlenstoffatomen sowie bicyclisches oder tricyclisches, gesättigtes Carbocyclyl mit 7 bis 10 Kohlenstoffatomen. Beispielhaft und bevorzugt seien genannt: Cyclopropyl, Cyclobutyl, Cyclopentyl, Cyclohexyl, Bicyclo[1.1.1]pentyl, Spiro[2.5]oktyl, Bicyclo[2.2.1]heptyl, Bicyclo[2.2.2]oktyl, Tricyclo[3.3.1.1$^{3,7}$]decyl.

Cycloalkyl steht im Rahmen der Erfindung für eine monocyclische, gesättigte Cycloalkylgruppe mit in der Regel 3 bis 8, bevorzugt 3 bis 6 Kohlenstoffatomen, beispielhaft und vorzugsweise für Cyclopropyl, Cyclobutyl, Cyclopentyl und Cyclohexyl.

Heterocyclyl steht für einen mono-, poly- oder spirocyclischen, vorzugsweise mono- bi- oder spirocyclischen, nicht-aromatischen heterocyclischen Rest mit in der Regel 3 bis 10 Ringatomen und bis zu 3, vorzugsweise bis zu 2 Heteroatomen und/oder Heterogruppen aus der Reihe N, O, S, SO, $SO_2$. Die Heterocyclyl-Reste können gesättigt oder partiell ungesättigt sein. Bevorzugt sind 4- bis 6-gliedrige monocyclische gesättigte Heterocyclylreste mit einem Stickstoffatom sowie solche mit einem weiteren Heteroatom aus der Reihe N, O, S, SO und $SO_2$, sowie 6- bis 10-gliedrige bicyclische gesättigte Heterocyclylreste mit einem Stickstoffatom sowie solche mit einem weiteren Heteroatom aus der Reihe N, O, S, SO und $SO_2$. Beispielhaft und vorzugsweise seien genannt: Aziridinyl, Azetidinyl, Pyrrolidinyl, Piperidinyl, Piperazinyl, Oxazolidinyl, Thiazolidinyl, Thiadiazolidinyl, Imidazolidinyl, Imidazolidin-2-yliden, Morpholinyl, Azaspiro[2.4]heptyl, Azaspiro[3.3]heptyl, Azabicyclo[3.1.0]hexyl, Azabicyclo[3.2.1]oktyl, Perhydropyrrolo[3,4-c]pyrrolyl.

Halogen steht für Fluor, Chlor, Brom und Iod.

In der Gruppe der Formel, für die $R^2$, $Ar^1$ bzw. Q stehen kann, steht der Endpunkt der Linie, an dem das Zeichen

#[1], #[2], #[2];*, ** und *** steht, nicht für ein Kohlenstoffatom beziehungsweise eine $CH_2$-Gruppe, sondern ist Bestandteil der Bindung zu dem jeweils bezeichneten Atom, an das $R^2$, $Ar^1$; $Ar^2$ bzw. Q gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Eine Substitution mit einem oder mit zwei gleichen oder verschiedenen Substituenten ist bevorzugt.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Bevorzugt im Rahmen der vorliegenden Erfindung sind Verbindungen der Formel (I), in welcher

$R^1$ für $NR^4R^5$ steht, worin

$R^4$ Wasserstoff, Methyl, bis zu dreifach mit Fluor substituiertes $(C_2\text{-}C_4)$-Alkyl oder $(C_3\text{-}C_6)$-Cycloalkyl bedeutet, und

$R^5$ $(C_1\text{-}C_6)$-Alkyl, $(C_3\text{-}C_6)$-Cycloalkyl, 3- bis 6-gliedriges gesättigtes Heterocyclyl oder $(C_1\text{-}C_4)$-Alkylsulfonyl bedeutet, wobei $(C_1\text{-}C_6)$-Alkyl, $(C_3\text{-}C_6)$-Cycloalkyl und 3- bis 6-gliedriges gesättigtes Heterocyclyl, bis zu dreifach, gleich oder verschieden, mit Methyl, Difluormethyl, Trifluormethyl, Hydroxy, Oxo, Methoxy, Difluormethoxy, Trifluormethoxy, Cyano und weiterhin bis zu vierfach mit Fluor, substituiert sein können,

oder

$R^4$ und $R^5$ bilden zusammen mit dem Stickstoffatom, an das sie gebunden sind, einen gesättigten oder partiell ungesättigten, 3- bis 6-gliedrigen monocyclischen oder 6- bis 10-gliedrigen bicyclischen Heterocyclus, der ein oder zwei weitere, gleiche oder verschiedene Heteroatome aus der Reihe N, O, S, SO und/oder $SO_2$ als Ringglieder enthalten kann,

wobei der 3- bis 6-gliedrige monocyclische und der 6- bis 10-gliedrige bicyclische Heterocyclus jeweils mit 1 bis 5 Substituenten unabhängig voneinander ausgewählt aus der Gruppe $(C_1\text{-}C_4)$-Alkyl, Difluormethyl, Trifluormethyl, Hydroxy, Oxo, $(C_1\text{-}C_3)$-Alkoxy, Difluormethoxy, Trifluormethoxy, Cyano, $(C_1\text{-}C_3)$-Alkoxycarbonyl, Aminocarbonyl, Mono-$(C_1\text{-}C_3)$-alkylaminocarbonyloxy, und weiterhin bis zu vierfach mit Fluor, substituiert sein können, worin $(C_1\text{-}C_4)$-Alkyl ein- oder zweifach, gleich oder verschieden, mit Hydroxy, $(C_1\text{-}C_3)$-Alkoxy, und bis zu vierfach mit Fluor, substituiert sein kann,

$R^2$ für tert.-Butyl, 2-Methylbutyl

oder

für eine Gruppe der Formel

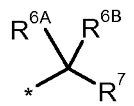 , 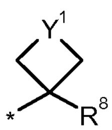

oder *-$L^1$-$Ar^2$ steht, worin

* die Verknüpfungsstelle mit dem N-Atom der Amid-Gruppierung markiert,

$R^{6A}$ Wasserstoff oder $(C_1\text{-}C_4)$-Alkyl bedeutet,

$R^{6B}$ Wasserstoff, Trifluormethyl oder Trifluormethoxymethyl bedeutet,

$R^7$ $(C_1\text{-}C_4)$-Alkyl oder Cyclopropyl bedeutet,

wobei $(C_1\text{-}C_4)$-Alkyl bis zu fünffach und Cyclopropyl bis zu vierfach mit Fluor substituiert sein kann,

$Y^1$ $-(CH_2)_k-$, $-O\text{-}CH_2-$, $-CH_2\text{-}O-$ oder $-CH_2\text{-}O\text{-}CH_2-$ bedeutet,

worin

k für 1, 2 oder 3 steht,

$R^8$ bis zu fünffach mit Fluor substituiertes $(C_1\text{-}C_2)$-Alkyl bedeutet,

$L^1$ eine Bindung oder eine Gruppe der Formel $-CR^{9A}R^{9B}-(CR^{10A}R^{10B})_m$ bedeutet,
worin
m 0 oder 1 darstellt,
$R^{9A}$ Wasserstoff oder Methyl darstellt,
$R^{9B}$ Wasserstoff, Methyl, Trifluormethyl, Pentafluorethyl oder Trifluormethoxymethyl darstellt,
$R^{10A}$ und $R^{10b}$ unabhängig voneinander Wasserstoff oder Methyl darstellen,

$Ar^2$ Phenyl bedeutet,
wobei Phenyl ein- bis dreifach, gleich oder verschieden, mit Fluor, Chlor, $(C_1-C_3)$-Alkyl, Difluormethoxymethyl, Trifluormethoxymethyl und/oder Trifluormethyl substituiert sein kann,
oder
für einen 7- bis 10-gliedrigen bicyclischen oder tricyclischen Carbocyclus steht,
wobei der 7- bis 10-gliedrige bicyclische oder tricyclische Carbocyclus bis zu dreifach, gleich oder verschieden, mit $(C_1-C_3)$-Alkyl, Trifluormethyl, und weiterhin bis zu vierfach mit Fluor substituiert sein kann,

$Ar^1$ für eine Gruppe der Formel

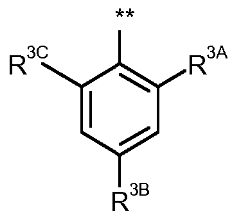

steht, worin
** die Verknüpfungsstelle mit dem N-Atom markiert,
$R^{3A}$ für Fluor, Chlor oder Trifluormethyl steht,
$R^{3B}$ für Wasserstoff oder Fluor steht
und
$R^{3C}$ für Wasserstoff, Fluor oder Chlor stehtsowie ihre N-Oxide, Salze, Solvate, Salze der N-Oxide und Solvate der N-Oxide und Salze.

Bevorzugt im Rahmen der vorliegenden Erfindung sind Verbindungen der Formel (I), in welcher in welcher

$R^1$ für $NR^4R^5$ steht,
worin
$R^4$ Wasserstoff oder Methyl bedeutet,
und
$R^5$ $(C_1-C_4)$-Alkyl oder Methylsulfonyl bedeutet,
wobei $(C_1-C_4)$-Alkyl bis zu zweifach mit Hydroxy und weiterhin bis zu dreifach mit Fluor, substituiert sein kann,
oder
$R^4$ und $R^5$ bilden zusammen mit dem Stickstoffatom, an das sie gebunden sind, einen gesättigten oder partiell ungesättigten 4- bis 6-gliedrigen monocyclischen oder 6- bis 10-gliedrigen bicyclischen Heterocyclus, der ein oder zwei weitere Heteroatome aus der Reihe N, O, S, SO oder $SO_2$ als Ringglied enthalten kann,
wobei der 4- bis 6-gliedrige monocyclische und der 6- bis 10-gliedrige bicyclische Heterocyclus jeweils mit 1 bis 5 Substituenten unabhängig voneinander ausgewählt aus der Gruppe $(C_1-C_3)$-Alkyl, Difluormethyl, Trifluormethyl, Hydroxymethyl, Hydroxyethyl, Hydroxy, Oxo, Methoxy, Difluormethoxy, Trifluormethoxy, Methoxymethyl, Cyano, Methoxycarbonyl, Aminocarbonyl, Monomethylaminocarbonyloxy, und weiterhin bis zu vierfach mit Fluor, substituiert sein können,

$R^2$ für eine Gruppe der Formel

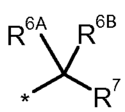 , 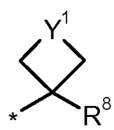 , 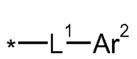 , 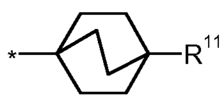 ,

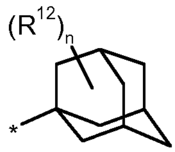

oder

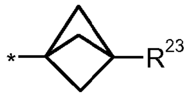

steht, worin
* die Verknüpfungsstelle mit dem N-Atom der Amid-Gruppierung markiert,
$R^{6A}$ Wasserstoff oder Methyl bedeutet,
$R^{6B}$ Wasserstoff, ($C_1$-$C_4$)-Alkyl, Cyclopropyl, Trifluormethyl oder Trifluormethoxymethyl bedeu tet,
$R^7$ ($C_1$-$C_4$)-Alkyl, Cyclopropyl oder Cyclobutyl bedeutet,
wobei ($C_1$-$C_4$)-Alkyl bis zu fünffach mit Fluor substituiert sein kann,
$Y^1$ $-(CH_2)_k-$, $-CF_2-$, $-O-CH_2-$, $-CH_2-O-$ oder $-CH_2-O-CH_2-$ bedeutet,
worin
k für 0, 1, 2 oder 3 steht,
$R^8$ Methyl, Trifluormethyl oder 2,2,2-Trifluorethyl bedeutet,
$L^1$ eine Bindung oder eine Gruppe der Formel $-CR^{9A}R^{9B}-$ bedeutet,
worin
$R^{9A}$ Wasserstoff oder Methyl darstellt,
$R^{9B}$ Wasserstoff, Methyl, Trifluormethyl oder Trifluormethoxymethyl darstellt,
$Ar^2$ Phenyl bedeutet,
welches ein- oder zweifach, gleich oder verschieden, mit Fluor, Chlor, Methyl und/oder Trifluormethyl substituiert sein kann,
$R^{11}$, $R^{12}$ und $R^{23}$ unabhängig voneinander Wasserstoff, Fluor, Methyl, Ethyl oder Trifluormethyl bedeuten,
n für die Zahl 1 oder 2 steht,
wobei im Fall, dass einer der Substituenten $R^{11}$, $R^{12}$ oder $R^{23}$ jeweils zweifach auftritt, seine Bedeutungen unabhängig voneinander gleich oder verschieden sein können,

$Ar^1$ für eine Gruppe der Formel

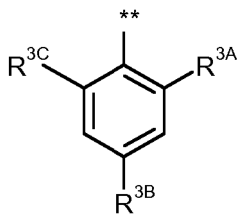

steht, worin
** die Verknüpfungsstelle mit dem N-Atom markiert,
$R^{3A}$ für Fluor, Chlor oder Trifluormethyl steht,
$R^{3B}$ für Wasserstoff oder Fluor steht
und
$R^{3C}$ für Wasserstoff, Fluor oder Chlor steht,

sowie ihre Salze, Solvate und Solvate der Salze.

Bevorzugt im Rahmen der vorliegenden Erfindung sind Verbindungen der Formel (I), in welcher

$R^1$ für $NR^4R^5$ steht,
worin
$R^4$ Wasserstoff oder Methyl bedeutet,
und
$R^5$ ($C_1$-$C_4$)-Alkyl oder Methylsulfonyl bedeutet,
wobei ($C_1$-$C_4$)-Alkyl mit Hydroxy und weiterhin bis zu dreifach mit Fluor substituiert sein kann,
oder
$R^4$ und $R^5$ bilden zusammen mit dem Stickstoffatom, an das sie gebunden sind, einen gesättigten 4- bis 6-gliedrigen monocyclischen oder 6- bis 10-gliedrigen bicyclischen Heterocyclus, der ein weiteres Heteroatom aus der Reihe N, O, S, SO oder $SO_2$ als Ringglied enthalten kann, wobei der 4- bis 6-gliedrige monocyclische und der 6- bis 10-gliedrige bicyclische Heterocyclus jeweils mit 1 bis 4 Substituenten unabhängig voneinander ausgewählt aus der Gruppe ($C_1$-$C_3$)-Alkyl, Difluormethyl, Trifluormethyl, Hydroxymethyl, Hydroxyethyl, Hydroxy, Oxo,

Methoxy, Difluormethoxy, Trifluormethoxy, Methoxymethyl, Cyano, Methoxycarbonyl, Aminocarbonyl, Monomethylaminocarbonyloxy, und weiterhin bis zu vierfach mit Fluor, substituiert sein können,

$R^2$ für tert.-Butyl

oder

für eine Gruppe der Formel

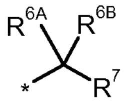 , 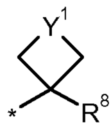 , $*-L^1-Ar^2$ , 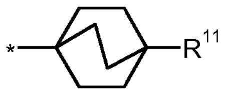 oder 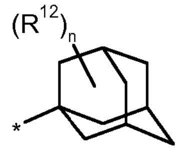

steht, worin

* die Verknüpfungsstelle mit dem N-Atom der Amid-Gruppierung markiert,

$R^{6A}$ Wasserstoff oder Methyl bedeutet,

$R^{6B}$ Wasserstoff, Trifluormethyl oder Trifluormethoxymethyl bedeutet,

$R^7$ $(C_1-C_4)$-Alkyl oder Cyclopropyl bedeutet,

wobei $(C_1-C_4)$-Alkyl bis zu fünffach mit Fluor substituiert sein kann,

$Y^1$ $-(CH_2)_k-$, $-O-CH_2-$, $-CH_2-O-$ oder $-CH_2-O-CH_2-$ bedeutet,

worin

k für 1, 2 oder 3 steht,

$R^8$ Methyl oder Trifluormethyl bedeutet,

$L^1$ eine Bindung oder eine Gruppe der Formel $-CR^{9A}R^{9B}-$ bedeutet,

worin

$R^{9A}$ Wasserstoff oder Methyl darstellt,

$R^{9B}$ Wasserstoff, Methyl, Trifluormethyl oder Trifluormethoxymethyl darstellt,

$Ar^2$ Phenyl bedeutet,

welches ein- oder zweifach, gleich oder verschieden, mit Fluor, Chlor, Methyl und/oder Trifluormethyl substituiert sein kann,

$R^{11}$ und $R^{12}$ unabhängig voneinander Wasserstoff, Fluor, Methyl, Ethyl oder Trifluormethyl bedeuten,

n für die Zahl 1 oder 2 steht,

wobei im Fall, dass der Substituent $R^{12}$ zweifach auftritt, seine Bedeutungen gleich oder verschieden sein können,

$Ar^1$ für eine Gruppe der Formel

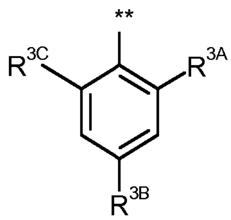

steht, worin

** die Verknüpfungsstelle mit dem N-Atom markiert,

$R^{3A}$ für Fluor, Chlor oder Trifluormethyl steht,

$R^{3B}$ für Wasserstoff oder Fluor steht

und

$R^{3C}$ für Wasserstoff, Fluor oder Chlor steht

sowie ihre Salze, Solvate und Solvate der Salze.

Eine besondere Ausführungsform der vorliegenden Erfindung umfasst Verbindungen der Formel (I), in welcher $R^1$ für eine Gruppe der Formel

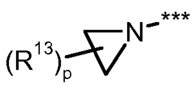 , 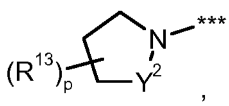 , 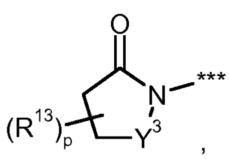 , 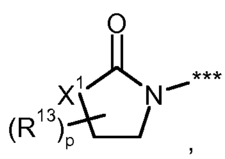 ,

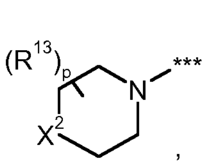, 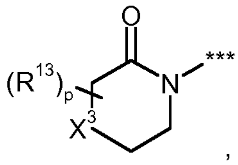, 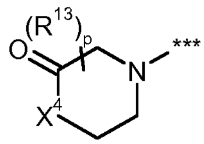, 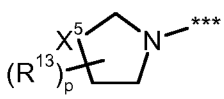,

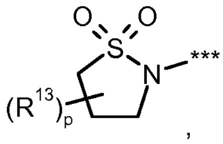, 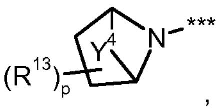, 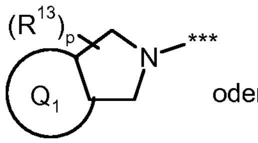 oder 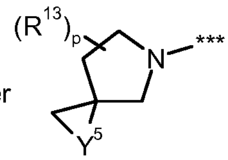

steht, worin

| | |
|---|---|
| *** | die Verknüpfungsstelle mit dem C-Atom des Pyridin-Rings markiert, |
| $Y^2$ und $Y^3$ | unabhängig voneinander eine Bindung, $-CH_2-$ oder $-(CH_2)_2-$ bedeuten, |
| $Y^4$ | $-(CH_2)_2-$, $-(CH_2)_3-$ oder $-CH_2-O-CH_2-$ bedeutet, |
| $Y^5$ | $-CF_2-$ bedeutet |
| $X^1$, $X^3$ und $X^4$ | unabhängig voneinander -O- oder -NH- bedeuten, |
| $X^2$ | -O- oder $-NR^{14}-$ bedeutet, worin $R^{14}$ für Wasserstoff, $(C_1-C_3)$-Alkoxycarbonyl oder Aminocarbonyl steht, |
| $X^5$ | $S(O)_t$ bedeutet, worin |
| t | für 0, 1 oder 2 steht, |
| der Ring $Q_1$ | zusammen mit den Atomen, an die er gebunden ist, einen dreigliedrigen gesättigten Carbocyclus bildet, wobei der dreigliedrige gesättigte Carbocyclus einfach mit Hydroxy, Hydroxymethyl oder bis zu zweifach mit Fluor substituiert sein kann, oder für eine Gruppe der Formel |

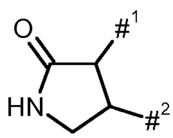

steht, worin

$\#^1$ und $\#^2$ die Verknüpfungsstelle mit dem C-Atom des Pyrrolidinrings markieren,

$R^{13}$ Fluor, $(C_1-C_3)$-Alkyl, Difluormethyl, Trifluormethyl, Hydroxy, Hydroxymethyl, Hydroxyethyl, Methoxy, Difluormethoxy, Trifluormethoxy, Methoxymethyl, Cyano, Methoxycarbonyl oder Monomethylaminocarbonyloxy bedeutet,

p die Zahl 0, 1, 2, 3 oder 4 bedeutet,

wobei im Fall, dass die Substituenten $R^{13D}$, $R^{13E}$ sowie $R^{13F}$ mehrfach auftreten, deren Bedeutungen jeweils gleich oder verschieden sein können.

Bevorzugt im Rahmen der vorliegenden Erfindung sind Verbindungen der Formel (I), in welcher

$R^1$ für $NR^4R^5$ steht,

worin

$R^4$ Wasserstoff oder Methyl bedeutet,

und

$R^5$ Methyl, Isopropyl, 2,2-Difluorethyl, 2,2,2-Trifluorethyl, 2-Hydroxyethyl oder 2-Hydroxypropyl bedeutet,

oder

für einen über ein Stickstoff-Atom gebundenen 4- bis 6-gliedrigen monocyclischen oder 6- bis 8-gliedrigen bicyclischen Heterocyclus der Formel

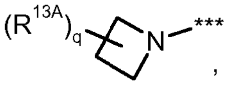, 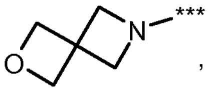, 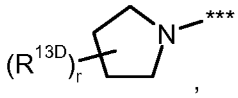, 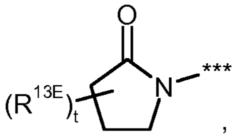,

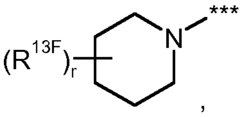, 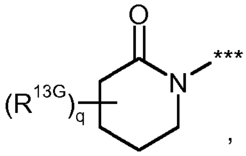, 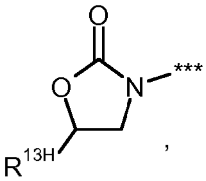, 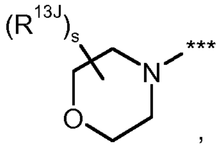,

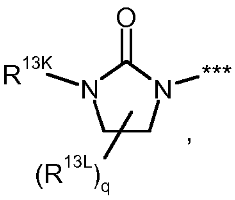, 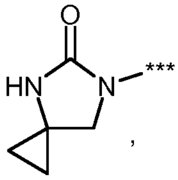, 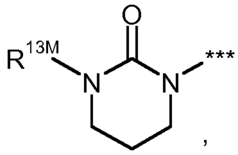, 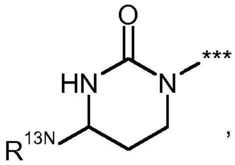,

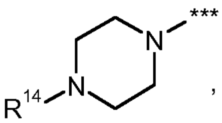, 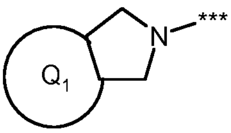 oder 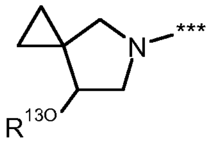

steht, worin

*** die Verknüpfungsstelle mit dem C-Atom des Pyridin-Rings markiert, der Ring $Q_1$ für eine Gruppe der Formel

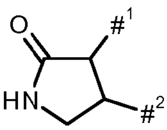, 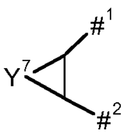 oder 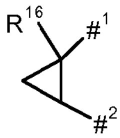

steht, worin

$\#^1$ und $\#^2$ die Verknüpfungsstelle mit dem C-Atom des Pyrrolidinrings markieren,

und

$Y^7$ für $-CF_2-$ oder $-CHR^{15}-$ steht,

worin

$R^{15}$ Methoxymethyl darstellt,

und

$R^{16}$ für Hydroxy steht,

$R^{13A}$ Fluor, Hydroxy, Hydroxymethyl, Methyl, Trifluormethyl oder Methoxy bedeutet,

$R^{13D}$ Wasserstoff, Fluor, Methyl, Hydroxy, Hydroxymethyl, Methoxy oder Difluormethoxy bedeutet,

$R^{13E}$ Wasserstoff, Fluor, Methyl, Hydroxy, Hydroxymethyl oder Methoxy bedeutet,

$R^{13F}$ Fluor, Methyl, Hydroxy, Hydroxymethyl oder Cyano bedeutet,

$R^{13G}$ Fluor oder Hydroxy bedeutet,

$R^{13H}$ Wasserstoff, Methyl, Hydroxymethyl, Aminocarbonyl oder Methoxycarbonyl bedeutet,

$R^{13J}$ Oxo, Hydroxymethyl oder Difluormethyl bedeutet,

$R^{13K}$ Wasserstoff, Methyl oder 2-Hydroxyethyl bedeutet,

$R^{13L}$ Wasserstoff oder Methyl bedeutet,

$R^{13M}$ Ethyl, 2-Hydroxyethyl oder Cyano bedeutet,

$R^{13N}$ Wasserstoff oder Ethyl bedeutet,

$R^{13O}$ Wasserstoff oder Hydroxy bedeutet,

$R^{14}$ Methyl, Methoxycarbonyl oder Aminocarbonyl bedeutet,

q die Zahl 0, 1 oder 2 bedeutet,
r die Zahl 0, 1, 2 oder 3 bedeutet,
s die Zahl 0 oder 1 bedeutet,
t die Zahl 0, 1, 2, 3 oder 4 bedeutet,
wobei im Fall, dass die Substituenten $R^{13A}$, $R^{13D}$, $R^{13E}$, $R^{13F}$, $R^{13G}$, $R^{13J}$, sowie $R^{13L}$ mehrfach auftreten, deren Bedeutungen jeweils gleich oder verschieden sein können,

$R^2$ für eine Gruppe der Formel

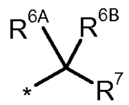 , 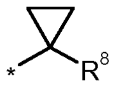 , 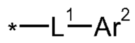 , 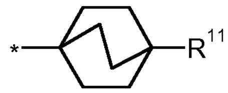 ,

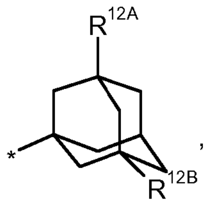 , 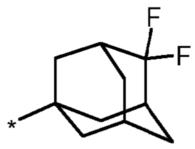 oder 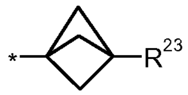

steht, worin
* die Verknüpfungsstelle mit dem N-Atom der Amid-Gruppierung markiert,
$R^{6A}$ Wasserstoff oder Methyl bedeutet,
$R^{6B}$ Methyl, Ethyl, Cyclopropyl, Trifluormethyl oder Trifluormethoxymethyl bedeutet,
$R^7$ Methyl, Ethyl, n-Propyl, Isopropyl, tert.-Butyl, 2-Methyl-prop-1-yl, Trifluormethyl, Difluormethyl, Pentafluorethyl, 2,2,2-Trifluorethyl oder Cyclopropyl bedeutet,
$R^8$ 2,2,2-Trifluorethyl bedeutet,
$L^1$ eine Bindung oder eine Gruppe der Formel $-CR^{9A}R^{9B}-$ bedeutet,
worin
$R^{9A}$ Wasserstoff oder Methyl darstellt,
$R^{9B}$ Wasserstoff, Methyl, Trifluormethyl oder Trifluormethoxymethyl darstellt,
$Ar^2$ Phenyl bedeutet,
welches ein- oder zweifach, gleich oder verschieden, mit Fluor, Chlor, Methyl und/oder Trifluormethyl substituiert sein kann,
$R^{11}$ Wasserstoff, Fluor oder Methyl bedeutet,
$R^{12A}$ Wasserstoff, Fluor, Methyl, Ethyl oder Trifluormethyl bedeutet,
$R^{12B}$ Wasserstoff oder Fluor bedeutet,
$R^{23}$ Wasserstoff, Fluor oder Trifluormethyl bedeutet,
und

$Ar^1$ für eine Gruppe der Formel

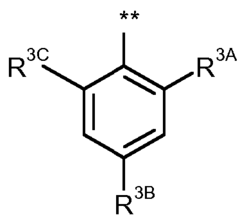

steht, worin
** die Verknüpfungsstelle mit dem N-Atom markiert,
$R^{3A}$ für Fluor oder Chlor steht,
$R^{3B}$ für Wasserstoff oder Fluor steht,
und
$R^{3C}$ für Wasserstoff, Fluor oder Chlor steht

sowie ihre Salze, Solvate und Solvate der Salze.

Besonders bevorzugt im Rahmen der vorliegenden Erfindung sind Verbindungen der Formel (I), in welcher

$R^1$ für eine Gruppe der Formel

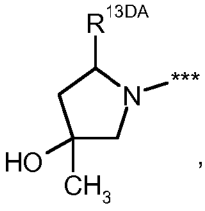 , 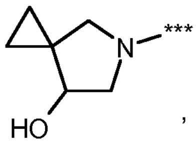 , 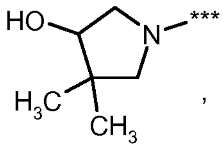 , 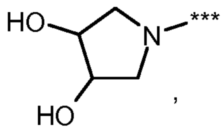 ,

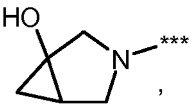 , 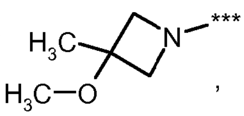 , 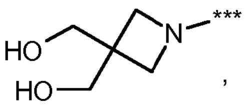 , 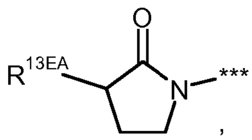 ,

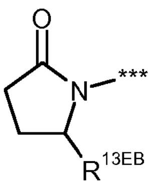 , 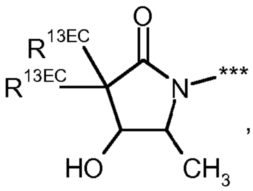 , 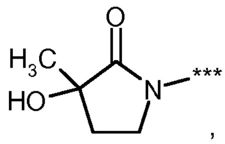 , 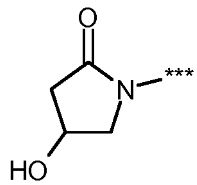

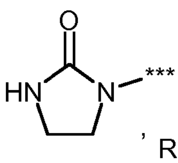 , 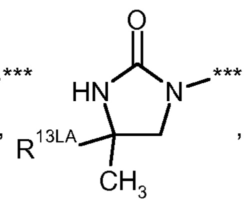 , 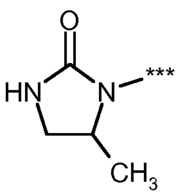 oder 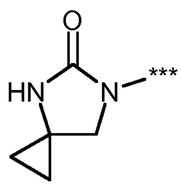

steht, worin

*** die Verknüpfungsstelle mit dem C-Atom des Pyridin-Rings markiert,

$R^{13DA}$ Wasserstoff oder Methyl bedeutet,

$R^{13EA}$ Hydroxy oder Hydroxymethyl bedeutet,

$R^{13EB}$ Methyl oder Hydroxymethyl bedeutet,

$R^{13EC}$ Wasserstoff oder Methyl bedeutet,

$R^{13LA}$ Wasserstoff oder Methyl bedeutet,

$R^2$ für eine Gruppe der Formel

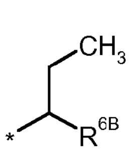 , 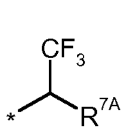 , 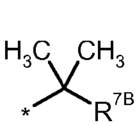 , 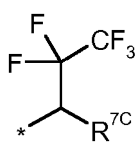 oder 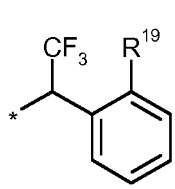

steht, worin

* die Verknüpfungsstelle mit dem N-Atom der Amid-Gruppierung markiert,

$R^{6B}$ Trifluormethoxymethyl bedeutet,

$R^{7A}$ Methyl, Ethyl, Trifluormethyl oder Cyclopropyl bedeutet,

$R^{7B}$ Trifluormethyl, Difluormethyl oder 2,2,2-Trifluorethyl bedeutet,

$R^{7C}$ Methyl oder Ethyl bedeutet,

$R^{19}$ Chlor bedeutet,

und

Ar1 für eine Gruppe der Formel

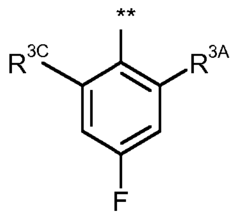 oder 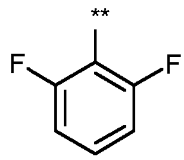

steht, worin
** die Verknüpfungsstelle mit dem N-Atom markiert,
$R^{3A}$ für Fluor oder Chlor steht, und
$R^{3C}$ für Wasserstoff oder Fluor steht,

sowie ihre Salze, Solvate und Solvate der Salze.

Ganz besonders bevorzugt im Rahmen der vorliegenden Erfindung sind Verbindungen der Formel (I), in welcher

$R^1$ für eine Gruppe der Formel

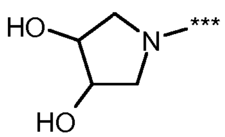 oder 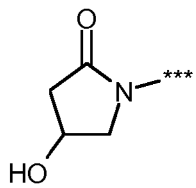

steht, worin
*** die Verknüpfungsstelle mit dem C-Atom des Pyridin-Rings markiert,

$R^2$ für eine Gruppe der Formel

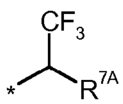, 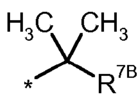 oder 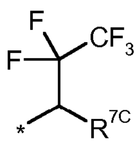

steht, worin
* die Verknüpfungsstelle mit dem N-Atom der Amid-Gruppierung markiert,
$R^{7A}$ Ethyl, Trifluormethyl oder Cyclopropyl bedeutet,
$R^{7B}$ Trifluormethyl bedeutet,
$R^{7C}$ Methyl oder Ethyl bedeutet,
und

Ar1 für eine Gruppe der Formel

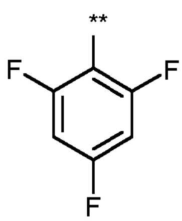 oder 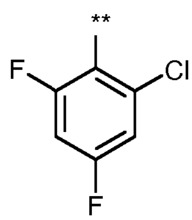

steht, worin
** die Verknüpfungsstelle mit dem N-Atom markiert,

sowie ihre Salze, Solvate und Solvate der Salze.

Bevorzugt im Rahmen der vorliegenden Erfindung sind Verbindungen der Formel (I), in welcher

$R^1$ für $NR^4R^5$ steht,
worin
$R^4$ Wasserstoff oder Methyl bedeutet,
und
$R^5$ Methyl, Isopropyl, 2,2-Difluorethyl, oder 2,2,2-Trifluorethyl bedeutet,
oder
für einen über ein Stickstoff-Atom gebundenen 4- bis 6-gliedrigen monocyclischen oder 6- bis 8-gliedrigen bicyclischen Heterocyclus der Formel

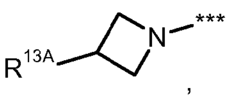 , 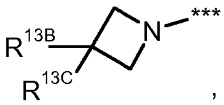 , 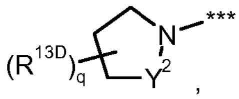 , 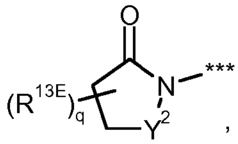 ,

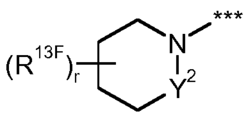 , 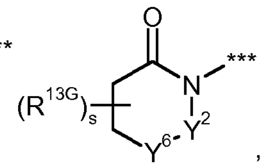 , 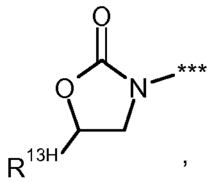 , 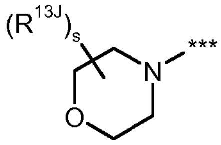 ,

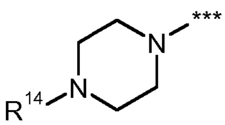 oder 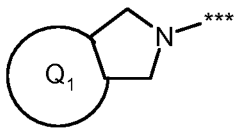

steht, worin
*** die Verknüpfungsstelle mit dem C-Atom des Pyridin-Rings markiert,
$Y^2$ $-CH_2-$ bedeutet,
$Y^6$ $-CH_2-$ oder $-CF_2-$ bedeutet,
der Ring $Q_1$ für eine Gruppe der Formel

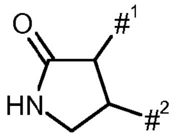 , 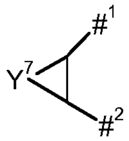 oder 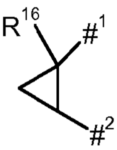

steht, worin
$\#^1$ und $\#^2$ die Verknüpfungsstelle mit dem C-Atom des Pyrrolidinrings markieren,
$Y^7$ für $-CH_2-$ oder $-CHR^{15}-$ steht,
worin
$R^{15}$ Methoxymethyl darstellt,
und
$R^{16}$ für Hydroxy steht,
$R^{13A}$ Fluor, Hydroxy oder Hydroxymethyl bedeutet,
$R^{13B}$ Hydroxy bedeutet,
$R^{13C}$ Trifluormethyl bedeutet,
$R^{13D}$ Fluor, Methyl, Hydroxy, Hydroxymethyl, Methoxy oder Difluormethoxy bedeutet,
$R^{13E}$ Fluor, Methyl, Hydroxy oder Methoxy bedeutet,
$R^{13F}$ Fluor, Methyl, Hydroxy, Hydroxymethyl oder Cyano bedeutet,
$R^{13G}$ Hydroxy bedeutet,
$R^{13H}$ Wasserstoff, Methyl, Hydroxymethyl oder Methoxycarbonyl bedeutet,
$R^{13J}$ Hydroxymethyl oder Difluormethyl bedeutet,

$R^{14}$ Methoxycarbonyl oder Aminocarbonyl bedeutet,
q die Zahl 0, 1 oder 2 bedeutet,
r die Zahl 0, 1, 2 oder 3 bedeutet,
s die Zahl 0 oder 1 bedeutet,
wobei im Fall, dass die Substituenten $R^{13D}$, $R^{13E}$ sowie $R^{13F}$ mehrfach auftreten, deren Bedeutungen jeweils gleich oder verschieden sein können,

$R^2$ für tert.-Butyl oder für eine Gruppe der Formel

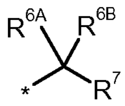 , 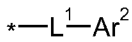 , 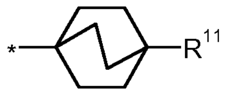 , 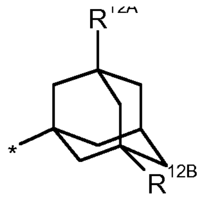 oder 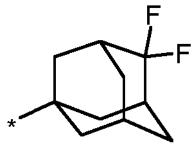

steht, worin
* die Verknüpfungsstelle mit dem N-Atom der Amid-Gruppierung markiert,
$R^{6A}$ Wasserstoff oder Methyl bedeutet,
$R^{6B}$ Trifluormethyl oder Trifluormethoxymethyl bedeutet,
$R^7$ Methyl, Ethyl, n-Propyl, Isopropyl, tert.-Butyl, 2-Methyl-prop-1-yl, Trifluormethyl, oder Cyclopropyl bedeutet,
$L^1$ eine Bindung oder eine Gruppe der Formel $-CR^{9A}R^{9B}-$ bedeutet,
worin
$R^{9A}$ Wasserstoff oder Methyl darstellt,
$R^{9B}$ Wasserstoff, Methyl, Trifluormethyl oder Trifluormethoxymethyl darstellt,
$Ar^2$ Phenyl bedeutet,
welches ein- oder zweifach, gleich oder verschieden, mit Fluor, Chlor, Methyl und/oder Trifluormethyl substituiert sein kann,
$R^{11}$ Wasserstoff, Fluor oder Methyl bedeutet,
$R^{12A}$ Wasserstoff, Fluor, Methyl, Ethyl oder Trifluormethyl bedeutet,
$R^{12b}$ Wasserstoff oder Fluor bedeutet,
und

$Ar^1$ für eine Gruppe der Formel

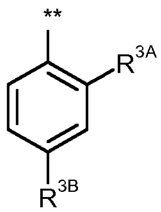 oder 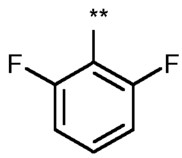

steht, worin
** die Verknüpfungsstelle mit dem N-Atom markiert,
$R^{3A}$ für Fluor oder Chlor steht,
und
$R^{3B}$ für Wasserstoff oder Fluor steht,

sowie ihre Salze, Solvate und Solvate der Salze.

Bevorzugt im Rahmen der vorliegenden Erfindung sind Verbindungen der Formel (I), in welcher

$R^1$ für eine Gruppe der Formel

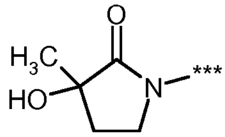 oder 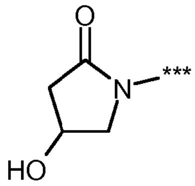

steht, worin
*** die Verknüpfungsstelle mit dem C-Atom des Pyridin-Rings markiert,

$R^2$ für eine Gruppe der Formel

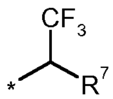 , 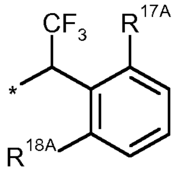 , 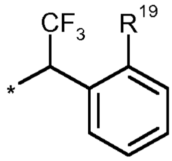 , 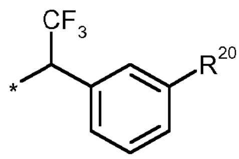 oder 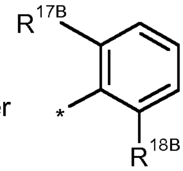

steht, worin
* die Verknüpfungsstelle mit dem N-Atom der Amid-Gruppierung markiert,
$R^7$ Ethyl oder Cyclopropyl bedeutet,
$R^{17A}$ Fluor oder Chlor bedeutet,
$R^{18A}$ Fluor bedeutet,
$R^{17B}$ und $R^{18b}$ jeweils Chlor bedeuten,
$R^{19}$ Fluor oder Chlor bedeutet,
$R^{20}$ Fluor bedeutet,
und

$Ar^1$ für eine Gruppe der Formel

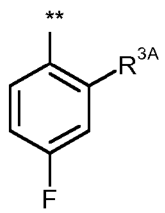 oder 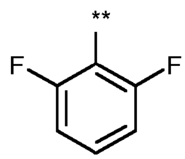

steht, worin
** die Verknüpfungsstelle mit dem N-Atom markiert,
$R^{3A}$ für Fluor oder Chlor steht

sowie ihre Salze, Solvate und Solvate der Salze.

Eine weitere besondere Ausführungsform der vorliegenden Erfindung umfasst Verbindungen der Formel (I), in welcher

$R^2$ für tert.-Butyl
oder
für eine Gruppe der Formel

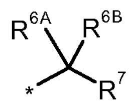

oder *-$L^1$-$Ar^2$ steht, worin
* die Verknüpfungsstelle mit dem N-Atom der Amid-Gruppierung markiert,
$R^{6A}$ Wasserstoff oder Methyl bedeutet,
$R^{6B}$ Trifluormethyl oder Trifluormethoxymethyl bedeutet,

$R^7$ Methyl, Ethyl, n-Propyl, Isopropyl, tert.-Butyl, 2-Methyl-prop-1-yl, Trifluormethyl, oder Cyclopropyl bedeutet,
$L^1$ eine Bindung oder eine Gruppe der Formel $-CR^{9A}R^{9B}-$ bedeutet,
worin
$R^{9A}$ Wasserstoff oder Methyl darstellt,
$R^{9B}$ Wasserstoff, Methyl, Trifluormethyl oder Trifluormethoxymethyl darstellt,
$Ar^2$ Phenyl bedeutet,
welches ein- oder zweifach, gleich oder verschieden, mit Fluor, Chlor, Methyl und/oder Trifluormethyl substituiert sein kann,

sowie ihre Salze, Solvate und Solvate der Salze.

Eine weitere besondere Ausführungsform der vorliegenden Erfindung umfasst Verbindungen der Formel (I), in welcher

$R^2$ für eine Gruppe der Formel

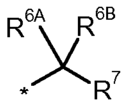 , 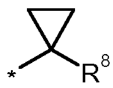 oder 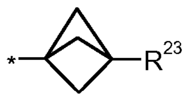

steht, worin
* die Verknüpfungsstelle mit dem N-Atom der Amid-Gruppierung markiert,
$R^{6A}$ Wasserstoff oder Methyl bedeutet,
$R^{6B}$ Methyl, Ethyl, Cyclopropyl, Trifluormethyl oder Trifluormethoxymethyl bedeutet,
$R^7$ Methyl, Ethyl, n-Propyl, Isopropyl, tert.-Butyl, 2-Methyl-prop-1-yl, Trifluormethyl, Difluormethyl, Pentafluorethyl, 2,2,2-Trifluorethyl oder Cyclopropyl bedeutet,
$R^8$ 2,2,2-Trifluorethyl bedeutet,
$R^{23}$ Wasserstoff, Fluor oder Trifluormethyl bedeutet,

sowie ihre Salze, Solvate und Solvate der Salze.

Eine weitere besondere Ausführungsform der vorliegenden Erfindung umfasst Verbindungen der Formel (I), in welcher

$R^2$ für eine Gruppe der Formel

$*L^1-Ar^2$

steht, worin
* die Verknüpfungsstelle mit dem N-Atom der Amid-Gruppierung markiert,
$L^1$ eine Bindung oder eine Gruppe der Formel $-CR^{9A}R^{9B}-$ bedeutet,
worin
$R^{9A}$ Wasserstoff darstellt,
$R^{9B}$ Wasserstoff, Methyl, Trifluormethyl oder Trifluormethoxymethyl darstellt,
$Ar^2$ Phenyl
oder
eine Gruppe der Formel

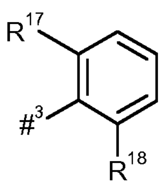 , 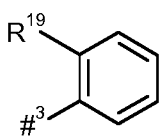 , 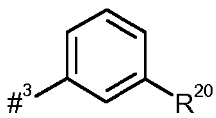 oder 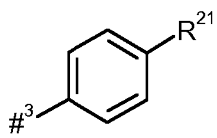

bedeutet,
worin
$\#^3$ die Verknüpfungsstelle markiert
$R^{17}$ und $R^{19}$ unabhängig voneinander Fluor, Chlor, Methyl oder Trifluormethyl darstellen,

$R^{18}$, $R^{20}$ und $R^{21}$ unabhängig voneinander Fluor, Chlor oder Methyl darstellen,

sowie ihre Salze, Solvate und Solvate der Salze.

Eine weitere besondere Ausführungsform der vorliegenden Erfindung umfasst Verbindungen der Formel (I), in welcher

$R^2$ für eine Gruppe der Formel

$$*L^1\text{-}Ar^2$$

steht, worin
* die Verknüpfungsstelle mit dem N-Atom der Amid-Gruppierung markiert,
$L^1$ eine Bindung oder eine Gruppe der Formel $-CR^{9A}R^{9B}-$ bedeutet,
worin
$R^{9A}$ Wasserstoff darstellt,
$R^{9B}$ Methyl, Trifluormethyl oder Trifluormethoxymethyl darstellt,

sowie ihre Salze, Solvate und Solvate der Salze.

Eine weitere besondere Ausführungsform der vorliegenden Erfindung umfasst Verbindungen der Formel (I), in welcher

$R^2$ für eine Gruppe der Formel

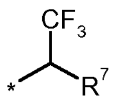 , 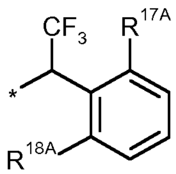 , 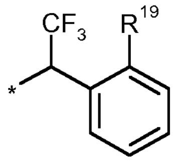 , 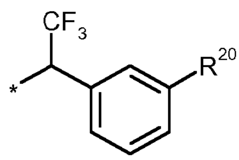 oder 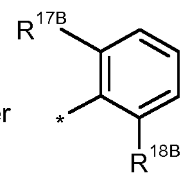

steht, worin
* die Verknüpfungsstelle mit dem N-Atom der Amid-Gruppierung markiert,
$R^7$ Ethyl oder Cyclopropyl bedeutet,
$R^{17A}$ Fluor oder Chlor bedeutet,
$R^{18A}$ Fluor bedeutet,
$R^{17B}$ und $R^{18B}$ jeweils Chlor bedeuten,
$R^{19}$ Fluor oder Chlor bedeutet,
und
$R^{20}$ Fluor bedeutet,

sowie ihre Salze, Solvate und Solvate der Salze.

Eine weitere besondere Ausführungsform der vorliegenden Erfindung umfasst Verbindungen der Formel (I), in welcher

$R^2$ für eine Gruppe der Formel

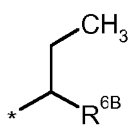 , 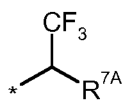 , 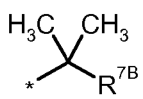 , 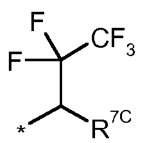 oder 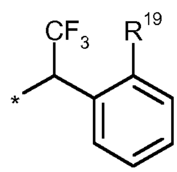

steht worin
* die Verknüpfungsstelle mit dem N-Atom der Amid-Gruppierung markiert,
$R^{6B}$ Trifluormethoxymethyl bedeutet,
$R^{7A}$ Methyl, Ethyl, Trifluormethyl oder Cyclopropyl bedeutet,
$R^{7B}$ Trifluormethyl, Difluormethyl oder 2,2,2-Trifluorethyl bedeutet,

$R^{7C}$ Methyl oder Ethyl bedeutet,
$R^{19}$ Chlor bedeutet,

sowie ihre Salze, Solvate und Solvate der Salze.

Eine weitere besondere Ausführungsform der vorliegenden Erfindung umfasst Verbindungen der Formel (I), in welcher

$R^2$ für eine Gruppe der Formel

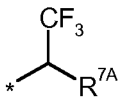 , 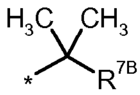 oder 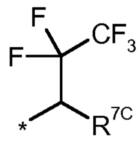

steht, worin
* die Verknüpfungsstelle mit dem N-Atom der Amid-Gruppierung markiert,
$R^{7A}$ Ethyl, Trifluormethyl oder Cyclopropyl bedeutet,
$R^{7B}$ Trifluormethyl bedeutet,
$R^{7C}$ Methyl oder Ethyl bedeutet,

sowie ihre Salze, Solvate und Solvate der Salze.

Eine weitere besondere Ausführungsform der vorliegenden Erfindung umfasst Verbindungen der Formel (I), in welcher

$R^2$ für (2*S*)-1,1,1-Trifluorbutan-2-yl der Formel

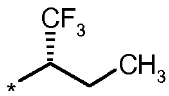

steht, worin
* die Verknüpfungsstelle mit dem N-Atom der Amid-Gruppierung markiert,

steht,
sowie ihre Salze, Solvate und Solvate der Salze.

Eine weitere besondere Ausführungsform der vorliegenden Erfindung umfasst Verbindungen der Formel (I), in welcher

$R^2$ für (1*S*)-1-Cyclopropyl-2,2,2-trifluorethyl

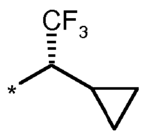

steht, worin
* die Verknüpfungsstelle mit dem N-Atom der Amid-Gruppierung markiert,

sowie ihre Salze, Solvate und Solvate der Salze.

Eine weitere besondere Ausführungsform der vorliegenden Erfindung umfasst Verbindungen der Formel (I), in welcher

$R^2$ für 1,1,1,3,3,3-Hexafluorpropan-2-yl steht,

sowie ihre Salze, Solvate und Solvate der Salze.

Eine weitere besondere Ausführungsform der vorliegenden Erfindung umfasst Verbindungen der Formel (I), in welcher

$R^2$ für 3,3,4,4,4-Pentafluorbutan-2-yl steht,

sowie ihre Salze, Solvate und Solvate der Salze.

Eine weitere besondere Ausführungsform der vorliegenden Erfindung umfasst Verbindungen der Formel (I), in welcher

$R^2$ für 1,1,1,2,2-Pentafluorpentan-3-yl steht,

sowie ihre Salze, Solvate und Solvate der Salze.

Eine weitere besondere Ausführungsform der vorliegenden Erfindung umfasst Verbindungen der Formel (I), in welcher

$R^2$ für 1,1,1-Trifluor-2-methylpropan-2-yl steht,

sowie ihre Salze, Solvate und Solvate der Salze.

Eine weitere besondere Ausführungsform der vorliegenden Erfindung umfasst Verbindungen der Formel (I), in welcher

$R^2$ für eine Gruppe der Formel

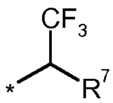

steht, worin
* die Verknüpfungsstelle mit dem N-Atom der Amid-Gruppierung markiert,
$R^7$ Ethyl oder Cyclopropyl bedeutet,

sowie ihre Salze, Solvate und Solvate der Salze.

Eine weitere besondere Ausführungsform der vorliegenden Erfindung umfasst Verbindungen der Formel (I), in welcher

$Ar^1$ für eine Gruppe der Formel

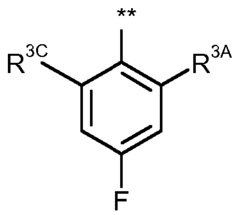

steht, worin
** die Verknüpfungsstelle mit dem N-Atom markiert,
$R^{3A}$ für Fluor oder Chlor steht, und
$R^{3C}$ für Wasserstoff oder Fluor steht

sowie ihre Salze, Solvate und Solvate der Salze.

Eine weitere besondere Ausführungsform der vorliegenden Erfindung umfasst Verbindungen der Formel (I), in welcher

$Ar^1$ für eine Gruppe der Formel

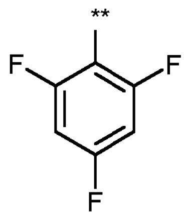

oder

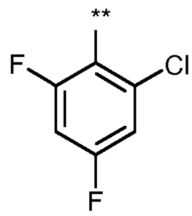

steht, worin
** die Verknüpfungsstelle mit dem N-Atom markiert,

sowie ihre Salze, Solvate und Solvate der Salze.

Eine weitere besondere Ausführungsform der vorliegenden Erfindung umfasst Verbindungen der Formel (I), in welcher

$Ar^1$ für eine Gruppe der Formel

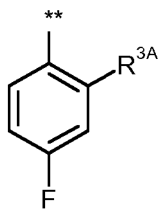

oder

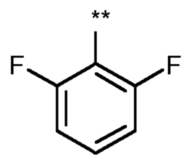

steht, worin
** die Verknüpfungsstelle mit dem N-Atom markiert,
$R^{3A}$ für Fluor oder Chlor steht,

sowie ihre Salze, Solvate und Solvate der Salze.

Eine weitere besondere Ausführungsform der vorliegenden Erfindung umfasst Verbindungen der Formel (I), in welcher

$Ar^1$ für eine Gruppe der Formel

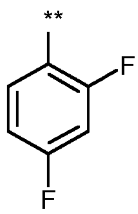

steht, worin
** die Verknüpfungsstelle mit dem N-Atom markiert,

sowie ihre Salze, Solvate und Solvate der Salze.

Eine weitere besondere Ausführungsform der vorliegenden Erfindung umfasst Verbindungen der Formel (I), in welcher

$Ar^1$ für eine Gruppe der Formel

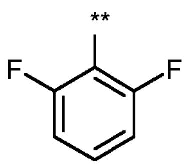

steht, worin
** die Verknüpfungsstelle mit dem N-Atom markiert,

sowie ihre Salze, Solvate und Solvate der Salze.

Eine weitere besondere Ausführungsform der vorliegenden Erfindung umfasst Verbindungen der Formel (I), in welcher

$R^1$ für $NR^4R^5$ steht,
worin
$R^4$ Wasserstoff oder Methyl bedeutet,
und
$R^5$ Methyl, Isopropyl, 2,2-Difluorethyl, oder 2,2,2-Trifluorethyl bedeutet,

sowie ihre Salze, Solvate und Solvate der Salze.

Eine weitere besondere Ausführungsform der vorliegenden Erfindung umfasst Verbindungen der Formel (I), in welcher

$R^1$ für eine Gruppe der Formel

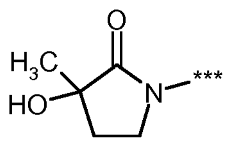 oder 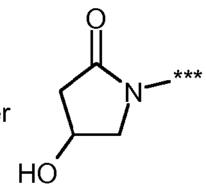

steht, worin
*** die Verknüpfungsstelle mit dem C-Atom des Pyridin-Rings markiert,

sowie ihre Salze, Solvate und Solvate der Salze.

Eine weitere besondere Ausführungsform der vorliegenden Erfindung umfasst Verbindungen der Formel (I), in welcher

$R^1$ für eine Gruppe der Formel

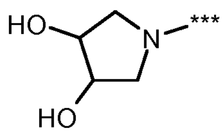

steht, worin
*** die Verknüpfungsstelle mit dem C-Atom des Pyridin-Rings markiert,

sowie ihre Salze, Solvate und Solvate der Salze.

Eine weitere besondere Ausführungsform der vorliegenden Erfindung umfasst Verbindungen der Formel (I), in welcher

$R^1$ für *trans*-(3*R*,4*R*)-3,4-Dihydroxypyrrolidin-1-yl der Formel

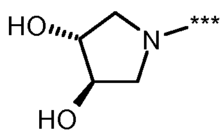

steht, worin
*** die Verknüpfungsstelle mit dem C-Atom des Pyridin-Rings markiert,

sowie ihre Salze, Solvate und Solvate der Salze.

Eine weitere besondere Ausführungsform der vorliegenden Erfindung umfasst Verbindungen der Formel (I), in welcher

$R^1$ für *cis*-(*R,S*)-3,4-Dihydroxypyrrolidin-1-yl der Formel

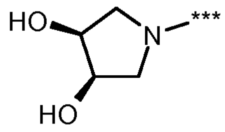

steht, worin

*** die Verknüpfungsstelle mit dem C-Atom des Pyridin-Rings markiert,

sowie ihre Salze, Solvate und Solvate der Salze.

Eine weitere besondere Ausführungsform der vorliegenden Erfindung umfasst Verbindungen der Formel (I), in welcher

$R^1$ für eine Gruppe der Formel

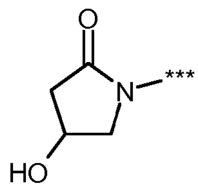

steht, worin

*** die Verknüpfungsstelle mit dem C-Atom des Pyridin-Rings markiert,

sowie ihre Salze, Solvate und Solvate der Salze.

Eine weitere besondere Ausführungsform der vorliegenden Erfindung umfasst Verbindungen der Formel (I), in welcher

$R^1$ für (4*S*)-4-Hydroxy-2-oxopyrrolidin-1-yl der Formel

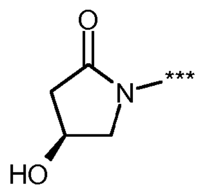

steht, worin

*** die Verknüpfungsstelle mit dem C-Atom des Pyridin-Rings markiert,

sowie ihre Salze, Solvate und Solvate der Salze.

Die in den jeweiligen Kombinationen bzw. bevorzugten Kombinationen von Resten im Einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche und Ausführungsformen.

Die als bevorzugt, besonders bevorzugt und ganz besonders bevorzugt genannten Restedefinitionen gelten sowohl für die Verbindungen der Formel (I) als auch in entsprechender Weise für alle Zwischenprodukte.

Weiterer Gegenstand der Erfindung ist ein Verfahren zur Herstellung von erfindungsgemäßen Verbindungen der Formel (I), dadurch gekennzeichnet, dass man

[A] eine Verbindung der Formel (II)

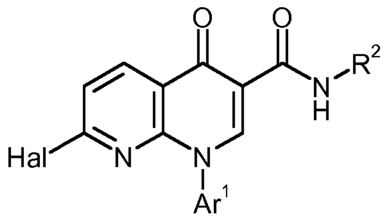

(II),

in welcher $R^2$ und $Ar^1$ die oben angegebenen Bedeutungen haben, und Hal für Fluor, Chlor, Brom oder Iod, bevorzugt für Chlor, steht,
mit einer Verbindung der Formel (III)

$R^1$-H (III),

in welcher $R^1$ die oben angegebene Bedeutung hat,
zum erfindungsgemäßen Carbonsäureamid der Formel (I)

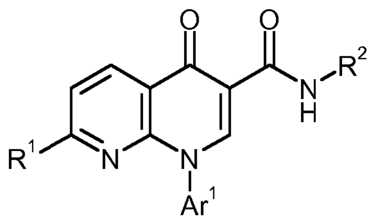

(I),

in welcher $R^1$, $R^2$ und $Ar^1$ die oben angegebenen Bedeutungen haben, umsetzt,
oder
[B] eine Verbindung der Formel (IV)

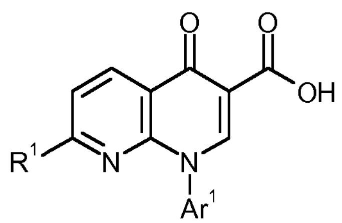

(IV),

in welcher $R^1$ und $Ar^1$ die oben angegebenen Bedeutungen haben,
mit einer Verbindung der Formel (V)

$R^2$-$NH_2$ (V),

in welcher $R^2$ die oben angegebene Bedeutung hat,
zum erfindungsgemäßen Carbonsäureamid der Formel (I)

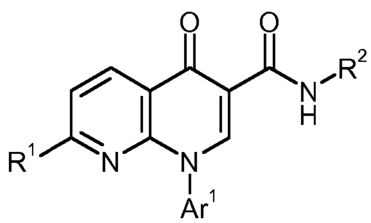

(I),

in welcher $R^1$, $R^2$ und $Ar^1$ die oben angegebenen Bedeutungen haben, umsetzt,
und gegebenenfalls die so erhaltenen Verbindungen der Formel (I) in ihre Enantiomere und/oder Diastereomere trennt und/oder mit den entsprechenden (*i*) Lösungsmitteln und/oder (*ii*) Basen oder Säuren in ihre Solvate, Salze und/oder Solvate der Salze überführt.

Die Umsetzung (II) + (III) → (I) kann über eine nukleophile Substitutionsreaktion oder eine Übergangsmetall-vermittelte Kupplungsreaktion erfolgen.

Die nukleophile Substitutionsreaktion wird vorzugsweise in Gegenwart einer Base durchgeführt. Als Basen für den Verfahrensschritt (II) + (III) → (I) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium- oder Cäsiumcarbonat, Alkali-Alkoholate wie Natrium- oder Kaliummethanolat, Natrium- oder Kaliumethanolat oder Lithium-, Natrium- oder Kalium-tert.-butylat, Alkalihydride wie Natrium- oder Kaliumhydrid, Amide wie Natriumamid, Lithium- oder Kalium-bis(trimethylsilyl)amid oder Lithiumdiisopropylamid, oder organische Amine wie *N,N*-Diisopropylethylamin (DIPEA), 1,5-Diazabicyclo[4.3.0]non-5-en (DBN) und 1,8-Diazabicyclo[5.4.0]undec-7-en (DBU). Bevorzugt wird *N,N*-Diisopropylethylamin (DIPEA) verwendet. Die Reaktion erfolgt im Allgemeinen in einem Temperaturbereich von 0°C bis +100°C, bevorzugt bei +23°C bis +80°C.

Inerte Lösungsmittel für den Verfahrensschritt (II) + (III) → (I) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Ethylacetat, Acetonitril, Pyridin, Dimethylsulfoxid, *N,N*-Dimethylformamid (DMF), *N,N'*-Dimethylpropylenharnstoff (DMPU) oder *N*-Methylpyrrolidon (NMP). Ebenso ist es möglich, Gemische der genannten Lösungsmittel zu verwenden. Bevorzugt wird Dimethylformamid (DMF) oder *N*-Methylpyrrolidon (NMP) verwendet.

Die Übergangsmetall-vermittelte Kupplungsreaktion für den Verfahrensschritt (II) + (III) → (I) wird in einer bevorzugten Ausführungsform in Gegenwart eines Palladiumkatalysators durchgeführt. Geeignete Palladium-Katalysatoren sind beispielsweise Palladium(II)acetat, Palladium(II)chlorid, Bis(triphenylphosphin)-palladium(II)chlorid, Bis(acetonitril)palladium(II)chlorid, Tetrakis(triphenylphosphin)palladium(0), Bis(di-benzylidenaceton)palladium(0), Tris(dibenzylidenaceton)dipalladium(0) oder [1,1'-Bis(diphenylphosphino)-ferrocen]palladium(II)chlorid, gegebenenfalls in Kombination mit einem geeigneten Phosphin-Liganden wie zum Beispiel Triphenylphosphin, Tri-*tert.*-butylphosphin, 2-Dicyclohexylphosphino-2',4',6'-triisopropylbiphenyl (X-Phos), 2-Dicyclohexylphosphino-2',6'-dimethoxybiphenyl (S-Phos), 1,2,3,4,5-Pentaphenyl-1'-(di-*tert*. -butylphosphino)ferrocen (Q-Phos), 4,5-Bis(diphenylphosphino)-9,9-dimethylxanthen (Xantphos), 2,2'-Bis(diphenylphosphino)-1,1'-binaphthyl (BINAP), 2-Dicyclohexylphosphino-2'-(*N,N*-dimethylamino)-biphenyl oder 2-Di-*tert.*-butylphosphino-2'-(*N,N*-dimethylamino)biphenyl.

Die Palladium-katalysierte Kupplungsreaktion (II) + (III) → (I) wird in der Regel in Gegenwart einer Base durchgeführt. Als solche eignen sich insbesondere Alkalicarbonate wie Natrium-, Kalium- oder Cäsiumcarbonat, Alkaliphosphate wie Natrium- oder Kaliumphosphat, Alkalifluoride wie Kalium- oder Cäsiumfluorid, oder Alkali-*tert.*-butylate wie Natrium- oder Kalium-*tert*.-butylat. Die Umsetzung erfolgt in einem inerten Lösungsmittel wie beispielsweise Toluol, 1,2-Dimethoxyethan, Tetrahydrofuran, 1,4-Dioxan, Dimethylsulfoxid (DMSO), *N,N*-Dimethylformamid (DMF), *N,N*-Dimethylacetamid (DMA) oder Mischungen hiervon in einem Temperaturbereich von +80°C bis +200°C, bevorzugt bei +80°C bis +150°C, wobei ein Erhitzen mittels Mikrowellenapparatur von Vorteil sein kann.

Bevorzugt wird für diese Kupplungsreaktion ein Katalysator/Ligand/Base-System bestehend aus Palladium(II)acetat, 4,5-Bis(diphenylphosphino)-9,9-dimethylxanthen (Xantphos) und Cäsium- oder Kaliumcarbonat eingesetzt und 1,4-Dioxan als Lösungsmittel verwendet.

Die Kupplungsreaktion (II) + (III) → (I) kann in einer weiteren bevorzugten Ausführungsform auch mit Hilfe eines Kupfer(I)-Katalysators, wie Kupfer(I)oxid, -bromid oder -iodid, in Gegenwart eines Kupfer-Liganden, wie trans-*N,N'*-Dimethyl-1,2-cyclohexandiamin, 8-Hydroxychinolin oder 1,10-Phenanthrolin, und einer anorganischen oder organischen Carbonat-Base, wie Kalium-, Cäsium- oder Bis(tetraethylammonium)carbonat, durchgeführt werden. Als inerte Lösungsmittel für diese Umsetzung eignen sich insbesondere Toluol, Xylol, 1,4-Dioxan, Acetonitril, Dimethylsulfoxid (DMSO), *N,N*-Dimethylformamid (DMF) oder Gemische hiervon, gegebenenfalls unter Zusatz von Wasser. Bevorzugt wird ein System bestehend aus Kupfer(I)iodid, trans-*N,N'*-Dimethyl-1,2-cyclohexandiamin und Kaliumcarbonat in Dimethylformamid eingesetzt. Die Reaktion erfolgt im Allgemeinen in einem Temperaturbereich von +50°C bis +200°C, bevorzugt bei +60°C bis +150°C.

Die Kupplungsreaktion (IV) + (V) → (I) [Amid-Bildung] kann entweder auf direktem Weg mit Hilfe eines Kondensations- oder Aktivierungsmittels oder über die Zwischenstufe eines aus (IV) erhältlichen Carbonsäurechlorids, Carbonsäureesters oder Carbonsäureimidazolids erfolgen.

Als solche Kondensations- oder Aktivierungsmittel eignen sich beispielsweise Carbodiimide wie *N,N'-Di*ethyl-, *N,N'*-Dipropyl-, *N,N'*-Diisopropyl-, *N,N'*-Dicyclohexylcarbodiimid (DCC) oder *N*-(3-Dimethylaminopropyl)-*N'*-ethylcarbodiimid-Hydrochlorid (EDC), Phosgen-Derivate wie *N,N'*-Carbonyldiimidazol (CDI), Chlorameisensäureisopropylester oder Chlorameisensäureisobutylester, 1,2-Oxazolium-Verbindungen wie 2-Ethyl-5-phenyl-1,2-oxazolium-3-sulfat oder

2-*tert*.-Butyl-5-methylisoxazolium-perchlorat, Acylamino-Verbindungen wie 2-Ethoxy-1-ethoxycarbonyl-1,2-dihydrochinolin, α-Chlorenamine wie 1-Chlor-*N,N*,2-tri-methylprop-1-en-1-amin, 1,3,5-Triazin-Derivate wie 4-(4,6-Dimethoxy-1,3,5-triazin-2-yl)-4-methylmorpholiniumchlorid, Phosphor-Verbindungen wie *n*-Propanphosphonsäureanhydrid (PPA), Cyanophosphonsäurediethylester, Diphenylphosphorylazid (DPPA), Bis-(2-oxo-3-oxazolidinyl)-phosphorylchlorid, Benzotriazol-1-yloxy-tris(dimethylamino)phosphonium-hexafluorophosphat oder Benzotriazol-1-yloxy-tris(pyrrolidino)-phosphonium-hexafluorophosphat (PyBOP), oder Uronium-Verbindungen wie *O*-(Benzotriazol-1-yl)-*N,N,N',N'*-tetramethyluronium-tetrafluoroborat (TBTU), *O*-(Benzotriazol-1-yl)-*N,N,N',N'*-tetramethyluronium-hexafluorophosphat (HBTU), *O*-(1*H*-6-Chlorbenzotriazol-1-yl)-1,1,3,3-tetramethyluronium-tetrafluoroborat (TCTU), *O*-(7-Azabenzotriazol-1-yl)-*N,N,N',N'*-tetramethyluronium-hexafluorophosphat (HATU) oder 2-(2-Oxo-1-(2*H*)-pyridyl)-1,1,3,3-tetramethyluronium-tetrafluoroborat (TPTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat, oder tertiäre Aminbasen wie Triethylamin, *N*-Methylmorpholin (NMM), *N*-Methylpiperidin (NMP), *N,N*-Diisopropylethylamin (DIPEA), Pyridin oder 4-*N,N*-Dimethylaminopyridin (DMAP). Als Kondensations- oder Aktivierungsmittel bevorzugt eingesetzt wird *O*-(7-Azabenzotriazol-1-yl)-*N,N,N',N'*-tetramethyluronium-hexafluorophosphat (HATU) in Kombination mit *N,N*-Diisopropylethylamin (DIPEA) sowie Chlorameisensäureisopropylester in Kombination mit *N*-Methylmorpholin (NMM) sowie Benzotriazol-l-yloxy-tris(pyrrolidino)phosphonium-hexafluorophosphat (PyBOP) in Kombination mit *N,N*-Diisopropylethylamin (DIPEA).

Bei zweistufiger Reaktionsführung über die aus (IV) erhältlichen Carbonsäurechloride oder Carbonsäureimidazolide wird die Kupplung mit der Amin-Komponente (V) in Gegenwart einer üblichen Base durchgeführt, wie beispielsweise Natrium- oder Kaliumcarbonat, Triethylamin, DIPEA, *N*-Methylmorpholin (NMM), *N*-Methylpiperidin (NMP), Pyridin, 2,6-Dimethylpyridin, 4-*N,N*-Dimethylaminopyridin (DMAP), 1,8-Diazabicyclo[5.4.0]undec-7-en (DBU), 1,5-Diazabicyclo[4.3.0]non-5-en (DBN), Natrium- oder Kaliummethanolat, Natrium- oder Kaliumethanolat, Natrium- oder Kalium-*tert*.-butylat oder Natrium- oder Kaliumhydrid.

Die Carbonsäureimidazolide selbst sind nach bekanntem Verfahren durch Umsetzung von (II) mit N,N'-Carbonyldiimidazol (CDI) bei erhöhter Temperatur (+60°C bis +150°C) in einem entsprechend höhersiedenden Lösungsmittel wie N,N-Dimethylformamid (DMF) erhältlich. Die Herstellung der Carbonsäurechloride geschieht auf übliche Weise durch Behandlung von (II) mit Thionylchlorid oder Oxalsäuredichlorid in einem inerten Lösungsmittel wie Dichlormethan oder THF.

Inerte Lösungsmittel für die genannten Kupplungsreaktionen sind - je nach eingesetztem Verfahren - beispielsweise Diethylether wie Diethylether, Diisopropylether, Methyl-*tert*.-butylether, Tetrahydrofuran, 1,4-Dioxan, 1,2-Dimethoxyethan oder Bis(2-methoxyethyl)ether, Kohlenwasserstoffe wie Benzol, Toluol, Xylol, Pentan, Hexan oder Cyclohexan, Halogenkohlenwasserstoffe wie Dichlormethan, Trichlormethan, Tetrachlormethan, 1,2-Dichlorethan, Trichlorethylen oder Chlorbenzol, oder polar-aprotische Lösungsmittel wie Aceton, Methylethylketon, Ethylacetat, Acetonitril, Butyronitril, Pyridin, Dimethylsulfoxid (DMSO), *N,N*-Dimethylformamid (DMF), *N,N'*-Dimethylpropylenharnstoff (DMPU) oder *N*-Methylpyrrolidinon (NMP). Auch können Gemische solcher Lösungsmittel eingesetzt werden. Bevorzugt wird *N,N*-Dimethylformamid (DMF) verwendet. Die Kupplungen werden im Allgemeinen in einem Temperaturbereich von 0°C bis +130°C, bevorzugt bei +20°C bis +30°C durchgeführt.

Bevorzugte Kupplungsmethode ist die direkte Umsetzung von (II) mit der Amin-Verbindung (III) mit Hilfe eines Kondensations- oder Aktivierungsmittels.

Bei zweistufiger Reaktionsführung über die aus (IV) erhältlichen Carbonsäureester kann die Kupplung mit einer aktivierten Amin-Komponente (V) durchgeführt werden. Die Amin-Komponente (V) wird vorzugsweise durch die Reaktion mit Trimethylaluminium aktiviert (vgl. Tetrahedron Lett. 1977, 18, 4171-4174). Bevorzugt wird Dichlormethan (DCM) als inertes Lösungmittel verwendet. Die Kupplungen werden im Allgemeinen in einem Temperaturbereich von 0°C bis +130°C, bevorzugt bei Raumtemperatur, durchgeführt.

Die Verbindungen der Formel (II) können dadurch hergestellt werden, dass eine Carbonsäure-Verbindung der Formel (VI)

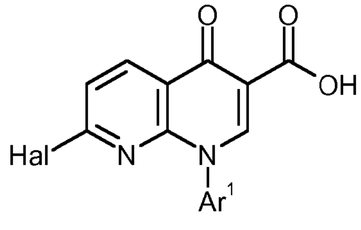

(VI),

in welcher Hal und $Ar^1$ die oben angegebenen Bedeutungen haben,
mit einer Verbindung der Formel (V)

$R^2$-$NH_2$ (V),

in welcher $R^2$ die oben angegebene Bedeutung hat, zum erfindungsgemäßen Carbonsäureamid der Formel (II)

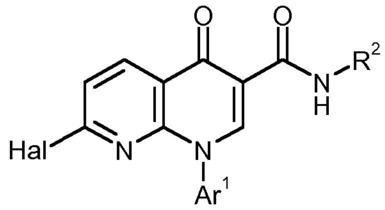

(II),

in welcher Hal, $R^1$, $R^2$ und $Ar^1$ die oben angegebenen Bedeutungen haben, umgesetzt wird.

Die Kupplungsreaktion (VI) + (V) → (II) [Amid-Bildung] kann entweder auf direktem Weg mit Hilfe eines Kondensations- oder Aktivierungsmittels oder über die Zwischenstufe eines aus (VI) erhältlichen Carbonsäurechlorids, Carbonsäureesters oder Carbonsäureimidazolids analog zu den bereits zur Umsetzung (IV) + (V) → (I) beschriebenen Bedingungen und Reagenzien erfolgen.

Wird bei der Kupplungsreaktion zu (II) HATU als Aktivierungmittel eingesetzt, ist es möglich, dass entweder ein einzelnes, definiertes Produkt der allgemeinen Formel (II) erhalten wird, oder aber ein Gemisch mit einem "HATU-Addukt". Unter einem "HATU-Addukt" wird vorliegend eine Pseudohalogenid-Verbindung bezeichnet, wobei der Substituent Hal in der allgemeinen Formel (II) mit der Gruppe 3*H*-[1,2,3]Triazolo[4,5-b]pyridin-3-ol, auch als 1-Hydroxy-7-azabenzotriazol bezeichnet, ersetzt ist. Ein derartiges Gemisch einer Halogenverbindung der allgemeinen Formel (II) und einem "HATU-Addukt" kann analog zur beschriebenen Umsetzung ebenfalls direkt als Edukt für die Folgereaktion (nach (I) oder (VIII)) eingesetzt werden.

Die Verbindungen der Formel (IV) können in Abhängigkeit vom jeweiligen Substitutionsmuster dadurch hergestellt werden, dass man entweder

[C] eine Verbindung der Formel (VII)

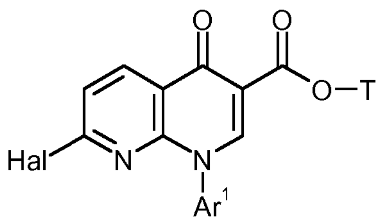

(VII),

in welcher Hal und $Ar^1$ die oben angegebenen Bedeutungen haben, und
T für ($C_1$-$C_4$)-Alkyl oder Benzyl steht,
in einem ersten Schritt mit einer Verbindung der Formel (III)

$R^1$-H (III),

in welcher $R^1$ die oben angegebene Bedeutung hat,
zu einer Verbindung der Formel (VIII)

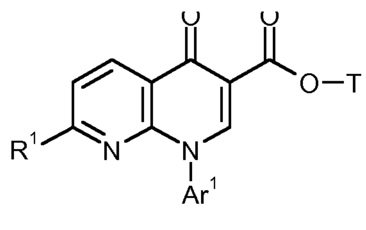

(VIII),

in welcher T, $R^1$ und $Ar^1$ die oben angegebenen Bedeutungen haben, umsetzt, und gegebenenfalls in einem zweiten Schritt den Ester-Rest T zur erfindungsgemäßen Carbonsäure der Formel (IV) abspaltet,

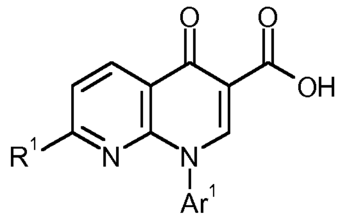

(IV),

in welcher $R^1$ und $Ar^1$ die oben angegebenen Bedeutungen haben, oder

[D] eine Verbindung der Formel (VI)

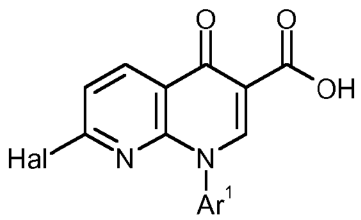

(VI),

in welcher Hal und Ar die oben angegebenen Bedeutungen haben, mit einer Verbindung der Formel (III)

$R^1$-H (III),

in welcher $R^1$ die oben angegebene Bedeutung hat, zur erfindungsgemäßen Carbonsäure der Formel (IV),

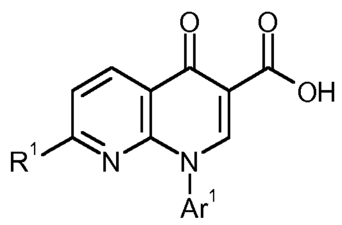

(IV),

in welcher $R^1$ und $Ar^1$ die oben angegebenen Bedeutungen haben, umsetzt.

Die Umsetzung (VII) + (III) → (VIII) [Weg C] oder die Umsetzung (VI) + (III) → (IV) [Weg D] kann jeweils über eine nukleophile Substitutionsreaktion oder eine Übergangsmetall-vermittelte Kupplungsreaktion analog zu den bereits zur Umsetzung (II) + (III) → (I) beschriebenen Bedingungen und Reagenzien erfolgen.

In einer bevorzugten Ausführungsform wird die Umsetzung nach Weg C als nukleophile Substitutionsreaktion in Gegenwart einer Base durchgeführt, bevorzugt wird *N,N*-Diisopropylethylamin (DIPEA) verwendet. Bevorzugt wird Dimethylformamid (DMF), *N*-Methylpyrrolidon (NMP) oder Acetonitril als Lösungsmittel verwendet.

In einer bevorzugten Ausfuhrungsform wird die Umsetzung nach Weg D als Übergangsmetall-vermittelte Kupplungsreaktion in Gegenwart eines geeigneten Palladiumkatalysators oder Kupfer(I)-Katalysators durchgeführt. Bevorzugt wird ein System bestehend aus Palladium(II)-acetat in Kombination mit 4,5-Bis-(diphenylphosphino)-9,9-dimethylxanthen (Xantphos), Cäsium- oder Kaliumcarbonat und 1,4-Dioxan als Lösungsmittel verwendet oder ebenfalls bevorzugt ein System bestehend aus Kupfer(I)iodid, trans-*N,N*'-Dimethyl-1,2-cyclohexandiamin und Kaliumcarbonat in Dimethylformamid als Lösungsmittel eingesetzt.

Die Abspaltung der Ester-Gruppe T im Verfahrensschritt (VIII) → (IV) wird nach üblichen Methoden durchgeführt, indem man den Ester in einem inerten Lösungsmittel mit einer Säure oder Base behandelt, wobei bei letzterer Variante das zunächst entstehende Salz der Carbonsäure durch nachfolgende Behandlung mit Säure in die freie Carbonsäure überführt wird. Im Falle der *tert.*-Butylester erfolgt die Esterspaltung vorzugsweise mit einer Säure. Benzylester können alternativ auch durch Hydrierung (Hydrogenolyse) in Gegenwart eines geeigneten Katalysators, wie beispielsweise Palladium auf Aktivkohle, abgespalten werden.

Als Lösungsmittel eignen sich für diese Reaktionen Wasser und die für eine Esterspaltung üblichen organischen Lösungsmittel. Hierzu zählen insbesondere Alkohole wie Methanol, Ethanol, *n*-Propanol, Isopropanol, *n*-Butanol oder *tert.*-Butanol, Ether wie Diethylether, Tetrahydrofuran, 1,4-Dioxan oder 1,2-Dimethoxyethan, oder andere Lösungsmittel wie Dichlormethan, Acetonitril, *N,N*-Dimethylformamid oder Dimethylsulfoxid. Ebenso ist es möglich, Gemische dieser Lösungsmittel einzusetzen. Im Falle einer basischen Ester-Hydrolyse werden bevorzugt Gemische von Wasser mit Tetrahydrofuran verwendet.

Als Basen sind die für eine Hydrolyse-Reaktion üblichen anorganischen Basen geeignet. Hierzu gehören insbesondere Alkali- oder Erdalkalihydroxide wie beispielsweise Lithium-, Natrium-, Kalium- oder Bariumhydroxid, oder Alkali- oder Erdalkalicarbonate wie Natrium-, Kalium- oder Calciumcarbonat. Bevorzugt werden wässrige Lithiumhydroxid-Lösung oder Natriumhydroxid-Lösung (Natronlauge) im Gemisch mit THF als Cosolvens eingesetzt.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt wird wässrige Salzsäure (18 proz.) in einem Wasser/Tetrahydrofuran-Gemisch eingesetzt.

Die Esterspaltung wird in der Regel in einem Temperaturbereich von -20°C bis +100°C, bevorzugt bei 23°C bis +120°C durchgeführt.

Die Verbindungen der Formel (VI) sowie der Formel (VIII) können in Abhängigkeit vom jeweiligen Substitutionsmuster dadurch hergestellt werden, dass man in Analogie zu bekannten Verfahren (s. z.B. EP 0607825 A1, S. 25-26) ein 2,6-Dichlornicotinoyl-Acrylsäureester-Derivat der Formel (IX)

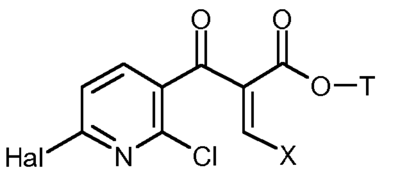

(IX),

in welcher Hal und T die oben angegebenen Bedeutungen haben, und
X für eine Abgangsgruppe wie Dimethylamino, Methoxy oder Ethoxy steht, sowie
in einer ersten Stufe, bevorzugt in Gegenwart einer geeigneten Base, mit einer Anilin-Verbindung der Formel (X)

$Ar^1$-$NH_2$ (X),

in welcher $Ar^1$ die oben angegebene Bedeutungen hat,
zu einem Intermediat der Formel (XI)

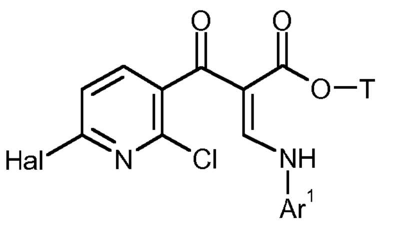

(XI),

in welcher Hal, $Ar^1$ und T die oben angegebenen Bedeutungen haben, umsetzt,
und dieses dann in Gegenwart einer geeigneten Base zur Ester-Verbindung der Formel (VII)

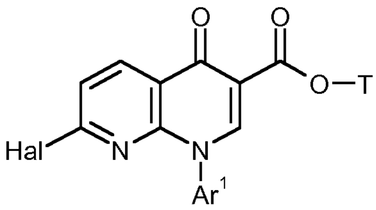

(VII),

in welcher Hal, $Ar^1$ und T die oben angegebene Bedeutung haben, umsetzt,
sowie dann gegebenenfalls unter Hydrolyse-Bedingungen in einem weiteren Schritt die Ester-Verbindung (VII) in die Carbonsäure-Verbindung (VI)

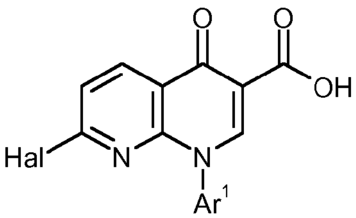

(VI),

in welcher Hal und $Ar^1$ die oben angegebenen Bedeutungen haben,
unter den in der Literatur bekannten Reaktionsbedingungen überfuhrt.

Die Verbindungen der Formel (IX) sind literaturbekannt (siehe z.B. EP 0607825 A1) oder können in Analogie zu literaturbekannten Verfahren hergestellt werden.

Die Verbindungen der Formel (III), (V) und (X) sind kommerziell erhältlich oder als solche in der Literatur beschrieben, oder sie können auf für den Fachmann offenkundigem Wege in Analogie zu in der Literatur publizierten Metho-den hergestellt werden. Zahlreiche ausführliche Vorschriften sowie Literaturangaben zur Herstellung der jeweiligen Ausgangsmaterialien befinden sich auch im Experimentellen Teil im Abschnitt zur Herstellung der Ausgangsverbindungen und Intermediate.

Die Auftrennung von Stereoisomeren (Enantio- und/oder Diastereomeren) der erfindungsgemäßen Verbindungen der Formel (I) läßt sich nach üblichen, dem Fachmann geläufigen Methoden erreichen. Vorzugsweise werden hierfür chromatographische Verfahren an achiralen bzw. chiralen Trennphasen angewandt.

Eine Trennung der erfindungsgemäßen Verbindungen in die entsprechenden Enantiomere und/ oder Diastereomere kann gegebenenfalls, je nach Zweckmäßigkeit, auch bereits auf der Stufe der Intermediate (II), (IV), oder (VIII) erfolgen, welche dann in separierter Form gemäß der zuvor beschriebenen Reaktionssequenz weiter umgesetzt werden. Für eine solche Auftrennung der Stereoisomere von Intermediaten werden gleichfalls bevorzugt chromatographische Verfahren an achiralen bzw. chiralen Trennphasen angewandt. Alternativ kann auch eine Trennung über diastereomere Salze der Carbonsäuren der Formel (IV) mit chiralen Amin-Basen erfolgen.

Die Herstellung der erfindungsgemäßen Verbindungen kann durch die folgenden Reaktionsschemata beispielhaft veranschaulicht werden:

**Schema 1**

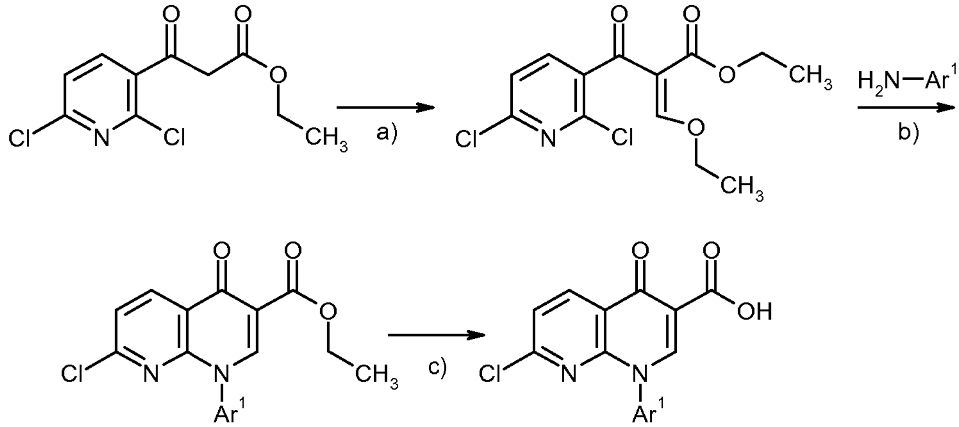


[a): Triethylorthoformiat, Acetanhydrid ; b): DIPEA, DCM, dann $K_2CO_3$; c): wässr. LiOH, THF oder 18-proz. Salzsäure, THF, Wasser].

**Schema 2**

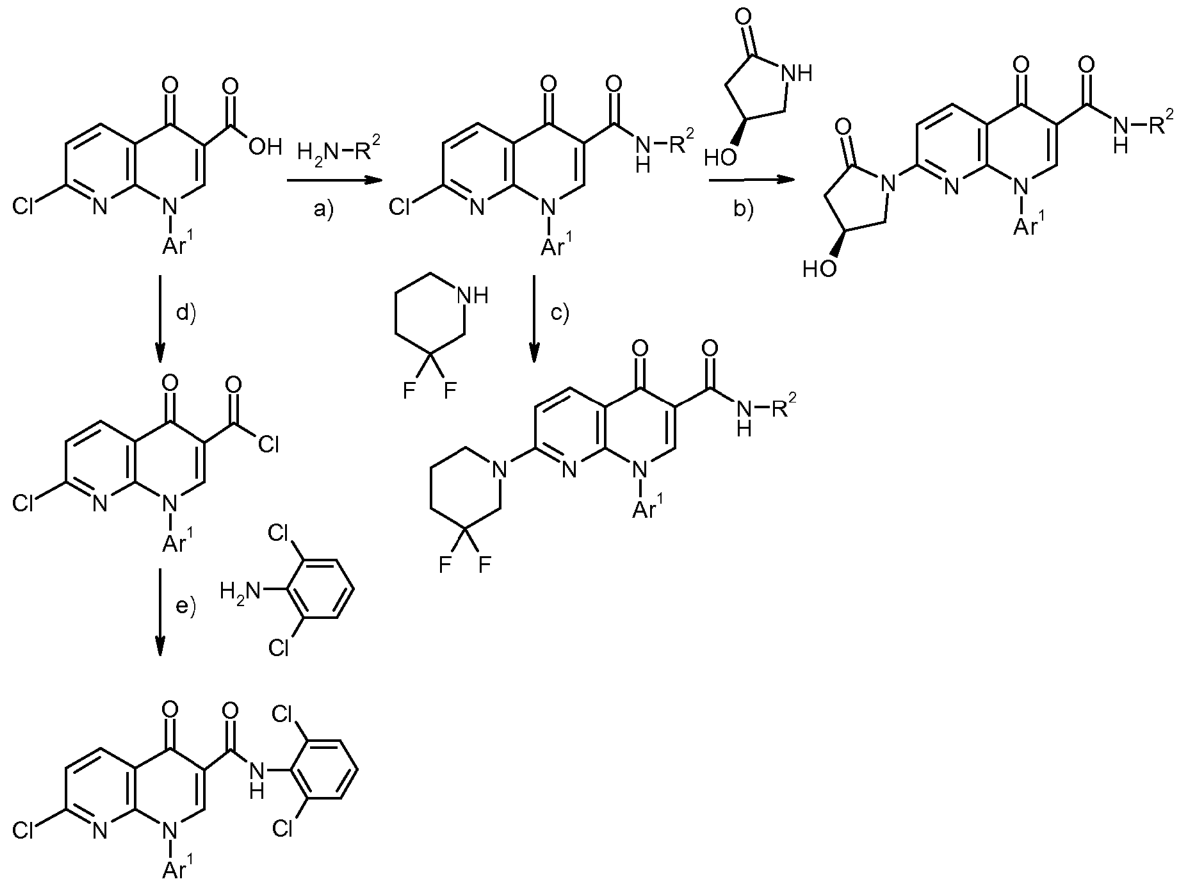


[a): $ClCO_2iPr$, NMM, NMP oder HATU, DIPEA, DMF oder PyBOP, DIPEA, DMF; b): $Pd(OAc)_2$, Xantphos, $Cs_2CO_3$, 1,4-Dioxan; c): DIPEA, DMF; d): $(COCl)_2$, kat. DMF, THF; e): NaH, DMF oder $NEt_3$, DCM].

**Schema 3**

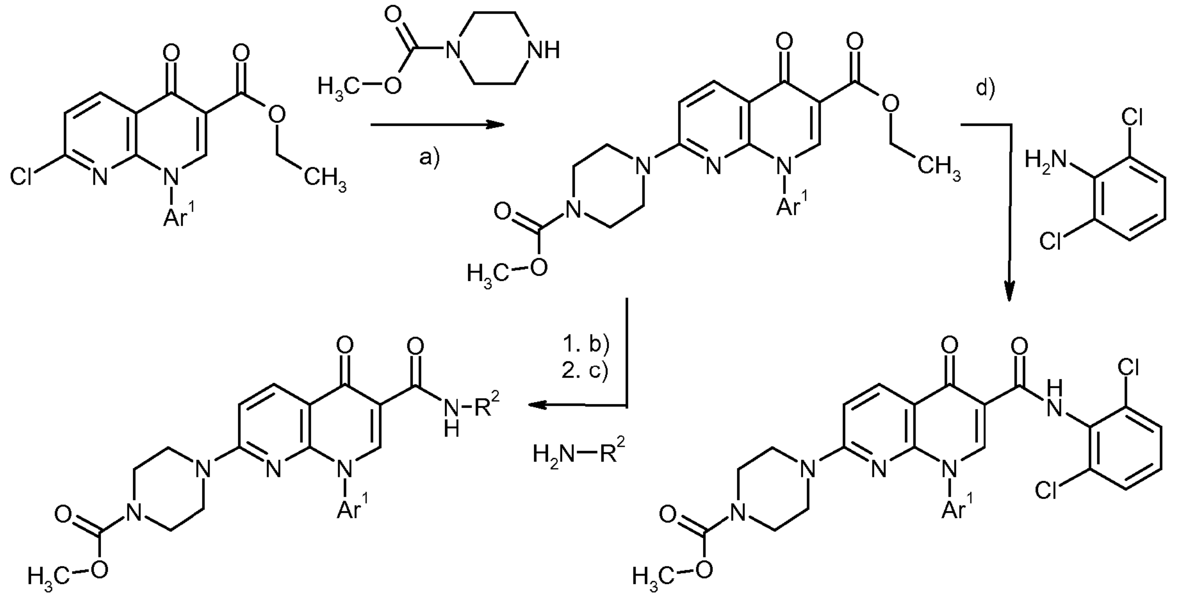


[a): DIPEA, DMF; b): wässr. LiOH, THF oder 18-proz. Salzsäure, THF, Wasser; c): $ClCO_2iPr$, NMM, NMP oder HATU, DIPEA, DMF oder PyBOP, DIPEA, DMF; d): $AlMe_3$, DCM].

**Schema 4**

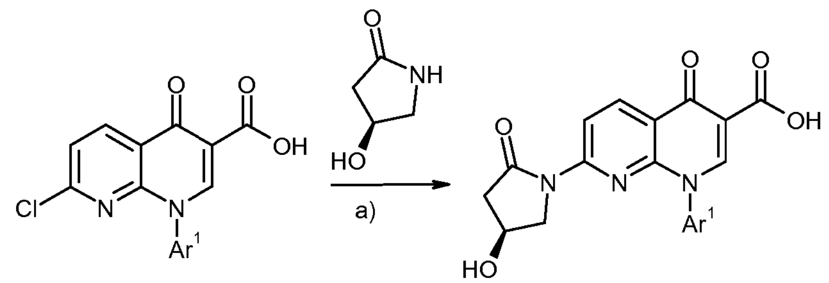


[a): $Pd(OAc)_2$, Xantphos, $CS_2CO_3$, 1,4-Dioxan].

**Schema 5**

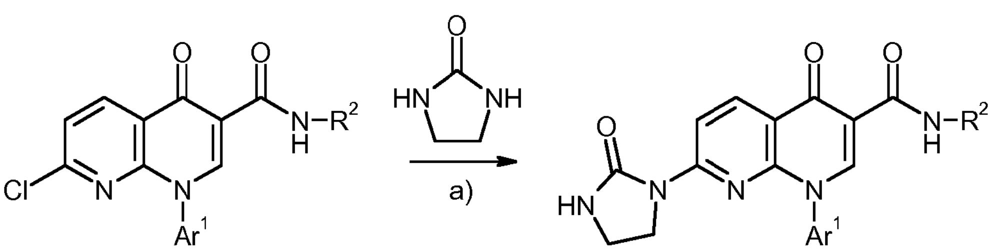


[a): CuI,, $K_2CO_3$, trans-*N,N'*-Dimethyl-1,2-cyclohexandiamin, DMF].

Weitere erfindungsgemäße Verbindungen der Formel (I) können, falls zweckmäßig, auch durch Umwandlungen von funktionellen Gruppen einzelner Reste und Substituenten, insbesondere den unter $R^1$ und $R^2$ aufgeführten, hergestellt werden, wobei von anderen, nach obigen Verfahren erhaltenen Verbindungen der Formel (I) oder deren Vorstufen ausgegangen wird. Diese Umwandlungen werden nach üblichen, dem Fachmann geläufigen Methoden durchgeführt und umfassen beispielsweise Reaktionen wie nukleophile oder elektrophile Substitutionsreaktionen, Übergangsmetall-vermittelte Kupplungsreaktionen, Herstellungs- und Additionsreaktionen von Metallorganylen (z.B. Grignard-Verbindungen oder Lithiumorganylen), Oxidations- und Reduktionsreaktionen, Hydrierung, Halogenierung (z.B. Fluorierung, Bromierung), Dehalogenierung, Aminierung, Alkylierung und Acylierung, die Bildung von Carbonsäureestern, Carbonsäureamiden und Sulfonamiden, die Esterspaltung und -hydrolyse sowie die Einführung und Entfernung temporärer Schutzgruppen.

Die Erfindung betrifft in einem weiteren Aspekt Intermediate der allgemeinen Formel (II)

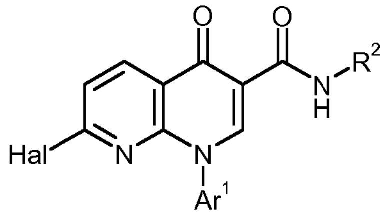

(II),

in welcher $R^2$ und $Ar^1$ die oben für Verbindungen der Formel (I) angegebenen Bedeutungen haben und
Hal für Fluor, Chlor, Brom oder Iod, bevorzugt für Chlor, steht.

Die Erfindung betrifft in einem weiteren Aspekt Intermediate der allgemeinen Formel (IV)

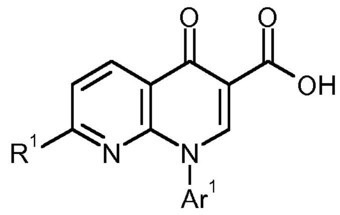

(IV),

in welcher $R^1$ und $Ar^1$ die oben für Verbindungen der Formel (I) angegebenen Bedeutungen haben. Die Erfindung betrifft in einem weiteren Aspekt die Verwendung einer Verbindung der allgemeinen Formel (II)

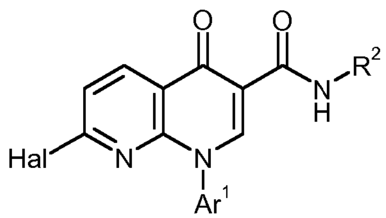

(II),

in welcher $R^2$ und $Ar^1$ die oben für Verbindungen der Formel (I) angegebenen Bedeutungen haben
und
Hal für Fluor, Chlor, Brom oder Iod, bevorzugt für Chlor, steht.
oder
einer Verbindung der allgemeinen Formel (IV)

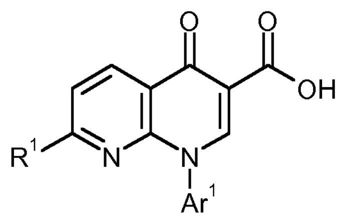

(IV),

in welcher $R^1$ und $Ar^1$ die oben für Verbindungen der Formel (I) angegebenen Bedeutungen haben,
zur Herstellung einer Verbindung der allgemeinen Formel (I) wie oben definiert.

Die erfindungsgemäßen Verbindungen zeigen ein nicht vorhersehbares, wertvolles pharmakologisches und pharmakokinetisches Wirkspektrum.

Sie eignen sich daher zur Verwendung als Arzneimittel zur Behandlung und/oder Prophylaxe von Krankheiten bei Menschen und Tieren. Die erfindungsgemäßen Verbindungen besitzen wertvolle pharmakologische Eigenschaften und können zur Behandlung und/ oder Prophylaxe von Erkrankungen bei Menschen und Tieren verwendet werden.

Die erfindungsgemäßen Verbindungen stellen positive allosterische Modulatoren des muskarinergen M2-Rezeptors dar und eignen sich daher zur Behandlung und/oder Prävention von Erkrankungen und pathologischen Prozessen, insbesondere kardiovaskulärer Erkrankungen und/oder Nierenerkrankungen, bei denen im Zuge einer Fehlregulation des autonomen Nervensystems bzw. eines Ungleichgewichtes zwischen der Aktivität des sympathischen und parasympathischen Teils des autonomen Nervensystems der M2-Rezeptor involviert ist.

Gegenstand der vorliegenden Erfindung sind positive allosterische Modulatoren des muskarinergen M2-Rezeptors der Formel (I). Allosterische Modulatoren weisen gegenüber klassischen, orthosterischen Liganden deutliche Unterschiede auf. Der Effekt eines allosterischen Modulators ist selbstlimitierend, wenn er in hohen Konzentrationen die Bindung des Agonisten stabilisiert. Darüber hinaus kann sich die Wirkung eines allosterischen Modulators nur in der Anwesenheit des endogenen Liganden entfalten. Der allosterische Modulator selbst hat keinen direkten Einfluss auf die Rezeptoraktivierung. Daraus ergibt sich eine räumliche und zeitliche Spezifität der allosterischen Wirkung. Die als Kooperativität bezeichnete gegenseitige Beeinflussung von allosterischen und orthosterischen Liganden hinsichtlich Affinität und intrinsischer Aktivität wird von den beiden Liganden bestimmt. Im Falle eines positiven allosterischen Modulators werden die Effekte des orthosterischen Liganden verstärkt (positive Kooperativität). Aufgrund ihrer Fähigkeit, Rezeptorkonformationen in Gegenwart eines orthosterischen Liganden zu modulieren, können allosterische Liganden eine Feinabstimmung pharmakologischer Effekte hervorrufen.

Im Sinne der vorliegenden Erfindung sind unter Erkrankungen des Herzkreislauf-Systems beziehungsweise kardiovaskulären Erkrankungen beispielsweise die folgenden Erkrankungen zu verstehen: akute und chronische Herzinsuffizienz, arterielle Hypertonie, koronare Herzerkrankung, stabile und instabile Angina pectoris, myokardiale Ischämie, Myokardinfarkt, Schock, Atherosklerose, Herzhypertrophie, Herzfibrose, atriale und ventrikuläre Arrhythmien, Tachykardie, transitorische und ischämische Attacken, Hirnschlag, Präeklampsie, entzündliche kardiovaskuläre Erkrankungen, periphere und kardiale Gefäßerkrankungen, periphere Durchblutungsstörungen, arterielle pulmonale Hypertonie, Spasmen der Koronararterien und peripherer Arterien, Thrombosen, thromboembolische Erkrankungen, Ödembildung wie zum Beispiel pulmonales Ödem, Hirnödem, renales Ödem oder Herzinsuffizienz-bedingtes Ödem, sowie Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädi-gungen (Vasculitis), Reperfusionsschäden, arterielle und venöse Thrombosen, Mikroalbuminurie, Herzmuskelschwäche, endotheliale Dysfunktion, periphere und kardiale Gefäßerkrankungen, periphere Durchblutungsstörungen, Herzinsuffizienz-bedingtes Ödem, erhöhte Spiegel von Fibrinogen und von LDL geringer Dichte sowie erhöhte Konzentrationen von Plasminogenaktivator-Inhibitor 1 (PAI 1).

Im Sinne der vorliegenden Erfindung umfasst der Begriff Herzinsuffizienz auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz.

Im Sinne der vorliegenden Erfindung umfasst der Begriff atriale und ventrikuläre Arrhythmien auch spezifischere oder verwandte Krankheitsformen wie: Vorhofflimmern, paroxysmales Vorhofflimmern intermittierendes Vorhofflimmern, permanentes Vorhofflimmern, Vorhofflattern, Sinusarrhythmie, Sinustachykardie, passive Heterotopie, aktive Heterotopie, Ersatzsystolen, Extrasystolen, Reizleitungsstörungen, Sick-sinus-Syndrom, hypersensitiver Karotissinus, Tachykardien, AV-Knoten Reentrytachykardie, atriventrikuläre Reentrytachykardie, WPW-Syndrom (Wolff-Parkinson-White), Mahaim-Tachykardie, verborgene akzessorische Leitungsbahn, permanente junktionale Re-entrytachykardie, fokale atriale Tachykardie, junktional ektope Tachykardie, atriale Reentrytachykardie, Kammertachykardie, Kammerflattern, Kammerflimmern, plötzlicher Herztod.

Im Sinne der vorliegenden Erfindung umfasst der Begriff koronare Herzerkrankung auch spezifischere oder verwandte Krankheitsformen wie: ischämische Herzkrankheit, stabile Angina pectoris, akutes Koronarsyndrom, instabile Angina pectoris, NSTEMI (nicht-ST-Streckenhebungsinfarkt), STEMI (ST-Streckenhebungsinfarkt), ischämische Herzmuskelschädigung, Herzrhythmusstörungen, und Myokardinfarkt.

Die erfindungsgemäßen Verbindungen sind weiter geeignet für die Prophylaxe und/oder Behandlung der polyzystischen Nierenkrankheit (PCKD) und des Syndroms der inadäquaten ADH-Sekretion (SIADH).

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen.

Im Sinne der vorliegenden Erfindung umfasst der Begriff akute Niereninsuffizienz akute Erscheinungsformen der Nierenerkrankung, des Nierenversagens und/ oder der Niereninsuffizienz mit und ohne Dialysepflicht, wie auch zugrundeliegende oder verwandte Nierenerkrankungen wie renale Hypoperfusion, intradialytische Hypotonie, Volumenmangel (z.B. Dehydratation, Blutverlust), Schock, akute Glomerulonephritis, hämolytisch-urämisches Syndrom (HUS), vaskuläre Kathastrophe (arterielle oder venöse Thrombose oder Embolie), Cholesterinembolie, akute Bence-Jones-Niere bei Plasmozytom, akute supravesikal oder subvesikale Abflussbehinderungen, immunlogische Nierenerkrankungen wie Nierentransplant-atabstoßung, Immunkomplex-induzierte Nierenerkrankungen, tubuläre Dilatation, Hyper-phosphatämie und/ oder akute Nierenerkrankungen, die durch die Notwendigkeit zur Dialyse charakterisiert werden können, sowie bei Teilresektionen der Niere, Dehydratation durch forcierte Diurese, unkontrolliertem Blutdruckanstieg mit maligner Hypertonie, Harnwegs-obstruktion und -infekt und Amyloidose sowie Systemerkrankungen mit glomerulärer Beteiligung, wie rheumatologisch-immunologische Systemerkrankungen, wie beispielsweise Lupus erythematodes, Nierenarterienthrombose, Nierenvenenthrombose, Analgetikanephropathie und renaltubuläre Azidose, sowie Röntgen-Kontrastmittel- sowie Medikamenten-induzierte akute interstitielle Nierenerkrankungen.

Im Sinne der vorliegenden Erfindung umfasst der Begriff chronische Niereninsuffizienz chronische Erscheinungsformen der Nierenerkrankung, des Nierenversagens und/ oder der Niereninsuffizienz mit und ohne Dialysepflicht, wie auch zugrundeliegende oder verwandte Nierenerkrankungen wie renale Hypoperfusion, intradialytische Hypotonie, obstruktive Uropathie, Glomerulopathien, glomeruläre und tubuläre Proteinurie, renale Ödeme, Hämaturie, primäre, sekundäre sowie chronische Glomerulonephritis, membranöse und membrano-proliferative Glomerulonephritis, Alport-Syndrom, Glomerulosklerose, tubulointerstitielle Erkrankungen, nephropathische Erkrankungen wie primäre und angeborene Nierenerkrankung, Nierenentzündung, immunlogische Nierenerkrankungen wie Nierentransplantatabstoßung, Immunkomplex-induzierte Nierenerkrankungen, diabetische und nicht-diabetische Nephro-pathie, Pyelonephritis, Nierenzysten, Nephrosklerose, hypertensive Nephrosklerose und nephrotisches Syndrom, welche diagnostisch beispielsweise durch abnorm verminderte Kreatinin- und/ oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/ oder die Notwendigkeit zur Dialyse charakterisiert werden können, sowie bei Nierenzellkarzinomen, nach Teilresektionen der Niere, Dehydratation durch forcierte Diurese, unkontrollierter Blutdruckanstieg mit maligner Hypertonie, Harnwegsobstruktion und -infekt und Amyloidose sowie Systemerkrankungen mit glomerulärer Beteiligung, wie rheumatologisch-immunologische Systemerkrankungen, wie beispielsweise Lupus erythematodes, sowie Nierenarterienstenose, Nierenarterienthrombose, Nierenvenenthrombose, Analgetikanephropathie und renaltubuläre Azidose zu verstehen. Weiterhin Röntgen-Kontrastmittel- sowie Medikamenten-induzierte chronische interstitielle Nierenerkrankungen, Metabolisches Syndrom und Dyslipidämie. Die vorliegende Erfindung umfasst auch die Verwendung der erfindungsgemäßen Verbindungen zur Behandlung und/ oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochenund Kohlenhydrat-Metabolismus.

Weiterhin eignen sich die erfindungsgemäßen Verbindungen auch zur Behandlung und/oder Prophylaxe von pulmonaler arterieller Hypertonie (PAH) und anderen Formen der pulmonalen Hypertonie (PH), der chronisch-obstruktiven Lungenerkrankung (COPD), des akuten Atemwegssyndroms (ARDS), der akuten Lungenschädigung (ALI), der alpha-1-Antitrypsin-Defizienz (AATD), der Lungenfibrose, des Lungenemphysems (z.B. durch Zigarettenrauch induziertes Lungenemphysem), der zystischen Fibrose (CF), von akutem Koronarsyndrom (ACS), Herzmuskelentzündungen (Myokarditis) und anderen autoimmunen Herzerkrankungen (Perikarditis, Endokarditis, Valvolitis, Aortitis, Kardiomyopathien), kardiogenem Schock, Aneurysmen, Sepsis (SIRS), multiplem Organversagen (MODS, MOF), entzündlichen Erkrankungen der Niere, chronischen Darmentzündungen (IBD, Crohn’s Disease, UC), Pankreatitis, Peritonitis, rheumatoiden Erkrankungen, entzündlichen Hauterkrankungen sowie entzündlichen Augenerkrankungen.

Die erfindungsgemäßen Verbindungen können weiterhin verwendet werden zur Behandlung und/ oder Prophylaxe von asthmatischen Erkrankungen unterschiedlicher Schweregrade mit intermittierendem oder persistierendem Verlauf (refraktives Asthma, bronchiales Asthma, allergisches Asthma, intrinsisches Asthma, extrinsisches Asthma, durch Medikamente oder durch Staub induziertes Asthma), von verschiedenen Formen der Bronchitis (chronische Bronchitis, infektiöse Bronchitis, eosinophile Bronchitis), von Bronchiolitis obliterans, Bronchiektasie, Pneumonie, idiopathischer interstitieller Pneumonie, Farmerlunge und verwandten Krankheiten, Husten- und Erkältungskrankheiten (chronischer entzündlicher Husten, iatrogener Husten), Nasenschleimhautentzündungen (einschließlich medikamentöse Rhinitis, vasomotorische Rhinitis und jahreszeitabhängige, allergische Rhinitis, z.B. Heuschnupfen) und von Polypen.

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierte Lern- und Gedächtnisstörungen, altersassoziierte Gedächtnisverluste, vaskuläre Demenz, Schädel-Hirn-Trauma, Schlaganfall, Demenz, die nach Schlaganfällen auftritt ("post stroke dementia"), post-traumatisches Schädel-Hirn-Trauma, allgemeine Konzentrationsstörungen, Konzentrationsstörungen bei Kindern mit Lern- und Gedächtnisproblemen, Alzheimer'sche Krankheit, Demenz mit Lewy-Körperchen, Demenz mit Degeneration der Frontallappen einschließlich des Pick's-Syndroms, Parkinson'sche Krankheit, progressiver nuclear palsy, Demenz mit corticobasaler Degeneration, Amyolateralsklerose (ALS), Huntington'sche Krankheit, Demyelinisation, Multiple Sklerose, thalamische Degeneration, Creutzfeld-Jacob-Demenz, HIV-Demenz, Schizophrenie mit Demenz oder Korsakoff-Psychose. Sie eignen sich auch zur Behandlung und/oder Prävention von Erkrankungen des Zentralnervensystems wie Angst-, Spannungs- und Depressionszuständen, zentralnervös bedingten Sexualdysfunktionen und Schlafstörungen sowie zur Regulierung krankhafter Störungen der Nahrungs-, Genuss- und Suchtmittelaufnahme.

Aufgrund ihres biochemischen und pharmakologischen Eigenschaftsprofils eignen sich die erfindungsgemäßen Verbindungen insbesondere zur Behandlung und/oder Prävention von Herzinsuffizienz, koronarer Herzerkrankung, atrialen und ventrikulären Arrhythmien, Niereninsuffizienz und Nephropathien.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Die erfindungsgemäßen Verbindungen eignen sich weiterhin zur Behandlung und/oder Prävention von ophthalmologischen Erkrankungen, wie beispielsweise von Glaukom, altersbedingter Makuladegeneration (AMD), trockener (nicht-exsudativer) AMD, feuchter (exsudativer, neovaskulärer) AMD, choroidaler Neovaskularisation (CNV), diabetiseher Retinopathie, atrophischen Veränderungen des retinalen Pigmentepithels (RPE), hypertrophischen Veränderungen des retinalen Pigmentepithels, Makulaödem, diabetischem Makulaödem, Netzhautvenenverschluss, choroidalem Netzhautvenenverschluss, Makulaödem auf grund von Netzhautvenenverschluss, Angiogenese an der Vorderseite des Auges wie kornealer Angiogenese beispielsweise nach Keratitis, Hornhauttransplantation oder Keratoplastik, kornealer Angiogenese auf grund von Hypoxie (durch extensives Tragen von Kontaktlinsen), Pterygium conjunctivae, subretinalem Ödem und intraretinalem Ödem. Des weiteren eignen sich die erfindungsgemäßen Verbindungen zur Behandlung und/oder Prävention von erhöhtem und hohem Augeninnendruck als Folge von traumatischem Hyphaema, periorbitalem Ödem, postoperativer viskoelastischer Retention oder intraokularer Entzündung.

Außerdem sind die erfindungsgemäßen Verbindungen zur Behandlung und/ oder Prophylaxe von Hepatitis, Neoplasma, Osteoporose, Glaukom und Gastroparese geeignet.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen und Tinnitus eingesetzt werden.

Die zuvor genannten, gut charakterisierten Krankheiten des Menschen können mit vergleichbarer Ätiologie auch in anderen Säugetieren vorkommen und dort ebenfalls mit den Verbindungen der vorliegenden Erfindung behandelt werden.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Weiterer Gegenstand der vorliegenden Erfindung ist somit die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Arzneimittel, enthaltend mindestens eine der erfindungsgemäßen Verbindungen, zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen in einem Verfahren zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Weiterer Gegenstand der vorliegenden Erfindung sind die erfindungsgemäßen Verbindungen zur Verwendung in einem Verfahren zur Behandlung und/oder Prävention von Erkrankungen, insbesondere der zuvor genannten Erkrankungen.

Die erfindungsgemäßen Verbindungen können allein oder bei Bedarf in Kombination mit einer oder mehreren anderen pharmakologisch wirksamen Substanzen eingesetzt werden, solange diese Kombination nicht zu unerwünschten und inakzeptablen Nebenwirkungen führt. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als hierfür geeignete Kombinationswirkstoffe seien beispielhaft und vorzugsweise genannt:

- den Blutdruck senkende Wirkstoffe, beispielhaft und vorzugsweise aus der Gruppe der Calcium-Antagonisten, Angiotensin AII-Antagonisten, ACE-Inhibitoren, NEP-Inhibitoren, Vasopeptidase-Inhibitoren, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika;
- antiarrhythmische Wirkstoffe, wie beispielsweise und vorzugsweise Natriumkanalblocker, Betarezeptorenblocker, Kaliumkanalblocker, Kalziumantagonisten, If-Kanalblocker, Digitalis, Parasympatholytika (Vagoliytika), Sympathomimetika und andere Antiarrhythmika wie Adenosin, Adenosinrezeptor Agonisten sowie Vernakalant.
- Vasopressin-Rezeptor-Antagonisten, wie beispielsweise und vorzugsweise Conivaptan, Tolvaptan, Lixivaptan, Mozavaptan, Satavaptan, SR-121463, RWJ 676070 oder BAY 86-8050;
- den Energiestoffwechsel des Herzens beeinflussende Verbindungen, wie beispielhaft und vorzugsweise Etomoxir, Dichloracetat, Ranolazine oder Trimetazidine;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- bronchodilatorisch wirkende Mittel, beispielhaft und vorzugsweise aus der Gruppe der betaadrenergen Rezeptor-Agonisten, wie insbesondere Albuterol, Isoproterenol, Metaproterenol, Terbutalin, Formoterol oder Salmeterol, oder aus der Gruppe der Anticholinergika, wie insbesondere Ipratropiumbromid;
- anti-inflammatorisch wirkende Mittel, beispielhaft und vorzugsweise aus der Gruppe der Glucocorticoide, wie insbesondere Prednison, Prednisolon, Methylprednisolon, Triamcinolon, Dexamethason, Beclomethason, Betamethason, Flunisolid, Budesonid oder Fluticason;
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-$\delta$-Agonisten, Cholesterin-Absorptionshemmer, Lipase-Inhibitoren, polymeren Gallensäureadsorber, Gallensäure-Reabsorptionshemmer und Lipoprotein(a)-Antagonisten.
- die Signaltransduktionskaskade inhibierende Verbindungen, beispielhaft und vorzugsweise aus der Gruppe der Kinase-Inhibitoren, insbesondere aus der Gruppe der Tyrosinkinase- und/oder Serin/Threoninkinase- Inhibitoren;
- Verbindungen, die den Ab- und Umbau der Extrazellulärmatrix inhibieren, beispielhaft und vorzugsweise Inhibitoren der Matrix-Metalloproteasen (MMPs), insbesondere Inhibitoren von Chymase, Stromelysin, Kollagenasen, Gelatinasen und Aggrecanasen (hierbei vor allem von MMP-1, MMP-3, MMP-8, MMP-9, MMP-10, MMP-11 und MMP-13) sowie der Metallo-Elastase (MMP-12);
- Verbindungen, die die Bindung von Serotonin an dessen Rezeptor blockieren, beispielhaft und vorzugsweise Antagonisten des 5-$HT_{2b}$-Rezeptors;
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- NO-unabhängige, jedoch Häm-abhängige Stimulatoren der löslichen Guanylatcyclase, wie insbesondere die in WO 00/06568, WO 00/06569, WO 02/42301 und WO 03/095451 beschriebenen Verbindungen;
- NO- und Häm-unabhängige Aktivatoren der löslichen Guanylatcyclase, wie insbesondere die in WO 01/19355, WO 01/19776, WO 01/19778, WO 01/19780, WO 02/070462 und WO 02/070510 beschriebenen Verbindungen;
- Verbindungen, die die Synthese von cGMP steigern, wie beispielsweise sGC Modulatoren, wie beispielhaft und vorzugsweise Riociguat, Cinaciguat, Vericiguat oder BAY 1101042
- Prostacyclin-Analoga, wie beispielhaft und vorzugsweise Iloprost, Beraprost, Treprostinil oder Epoprostenol;

- Verbindungen, die die lösliche Epoxidhydrolase (sEH) inhibieren, wie beispielsweise *N,N'*-Dicyclohexylharnstoff, 12-(3-Adamantan-1-yl-ureido)-dodecansäure oder 1-Adamantan-1-yl-3-{5-[2-(2-ethoxyethoxy)ethoxy]pentyl}-harnstoff.
- den Glucosestoffwechsel verändernde Wirkstoffe, wie beispielsweise Insuline, Sulfonylharnstoffe, Acarbose, DPP4 Inhibitoren, GLP-1 Analoga oder SGLT-1 Inhibitor.

Bei einer bevorzugten Ausführungsform der Erfindung werden die erfindungsgemäßen Verbindungen in Kombination mit einem Kinase-Inhibitor, wie beispielhaft und vorzugsweise Bortezomib, Canertinib, Erlotinib, Gefitinib, Imatinib, Lapatinib, Lestaurtinib, Lonafarnib, Pegaptinib, Pelitinib, Semaxanib, Sorafenib, Regorafenib, Sunitinib, Tandutinib, Tipifarnib, Vatalanib, Fasudil, Lonidamin, Leflunomid, BMS-3354825 oder Y-27632, eingesetzt.

Bei einer bevorzugten Ausführungsform der Erfindung werden die erfindungsgemäßen Verbindungen in Kombination mit einem Serotonin-Rezeptor-Antagonisten, wie beispielhaft und vorzugsweise PRX-08066, eingesetzt.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, DU-176b, Fidexaban, Razaxaban, Fondaparinux, Idraparinux, PMD-3112, Y*N*-150, KFA-1982, EMD-503982, MC*N*-17, mLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika ver-standen.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, Irbesartan, Olmesartan, Eprosartan oder Azilsartan oder einem dualen Angiotensin AII-Antagonisten/NEP-Inhibitor, wie beispielsweise und vorzugsweise Entresto (LCZ696, Valsartan/Sacubitril), verabreicht.

Bei einer bevorzugten Ausführungsform der Erfindung werden die erfindungsgemäßen Verbindungen in Kombination mit einem ACE-Inhibitor, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder

Eplerenon, Finerenon verabreicht.

Bei einer bevorzugten Ausfuhrungsform der Erfindung werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095, SB-772077, GSK-269962A oder BA-1049, verabreicht.

Bei einer bevorzugten Ausführungsform der Erfindung werden die erfindungsgemäßen Verbindungen in Kombination mit einem Diuretikum, wie beispielhaft und vorzugsweise Furosemid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase-oder Squalensynthese-Inhibitoren, der ACAT-Inhibitoren, MTP-Inhibitoren, PPAR-alpha-, PPAR-gamma-und/oder PPAR-δ-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-δ-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Bei einer bevorzugten Ausführungsform der Erfindung werden die erfindungsgemäßen Verbindungen in Kombination mit sGC Modulatoren, wie beispielhaft und vorzugsweise Riociguat, Cinaciguat, Vericiguat oder BAY 1101042 verabreicht.

Bei einer bevorzugten Ausführungsform der Erfindung werden die erfindungsgemäßen Verbindungen in Kombination mit einem den Glucosestoffwechsel verändernden Wirkstoff, wie beispielhaft und vorzugsweise Insulin, einem Sulfonylharnstoff, Acarbose, DPP4 Inhibitoren, GLP-1 Analoga oder SGLT-1 Inhibitor, verabreicht.

Besonders bevorzugt sind Kombinationen der erfindungsgemäßen Verbindungen mit einem oder mehreren weiteren Wirkstoffen ausgewählt aus der Gruppe bestehend aus den Blutdruck senkende Wirkstoffe, antiarrhythmische Wirkstoffe, Vasopressin-Rezeptor-Antagonisten, PDE 5-Inhibitoren, Thrombozytenaggregationshemmer, sGC-Aktivatoren und sGC-Stimulatoren.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat oder Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. inhalativ, intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer, Dosieraerosole), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale und die parenterale Applikation, insbesondere die orale, die intravenöse und die intrapulmonale (inhalative) Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier-oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

## A. Beispiele

### Abkürzungen und Akronyme:

| | |
|---|---|
| AAV | allgemeine Arbeitsvorschrift |
| abs. | absolut |
| aq. | wässrig, wässrige Lösung |
| br. | breit (bei NMR-Signal) |
| Bsp. | Beispiel |

| | |
|---|---|
| Bu | Butyl |
| c | Konzentration |
| ca. | circa, ungefähr |
| cat. | katalytisch |
| CI | chemische Ionisation (bei MS) |
| d | Dublett (bei NMR) |
| d | Tag(e) |
| DAST | *N,N*-Diethylaminoschwefeltrifluorid |
| DCI | direkte chemische Ionisation (bei MS) |
| DCM | Dichlormethan |
| dd | Dublett von Dublett (bei NMR) |
| de | Diastereomerenüberschuss |
| dest. | destilliert |
| DIPEA | *N*,*N*-Diisopropylethylamin |
| DMAP | 4-*N*,*N*-Dimethylaminopyridin |
| DMF | *N*,*N*-Dimethylformamid |
| DMSO | Dimethylsulfoxid |
| dt | Dublett von Triplett (bei NMR) |
| d. Th. | der Theorie (bei chemischer Ausbeute) |
| ee | Enantiomerenüberschuss |
| EI | Elektronenstoß-Ionisation (bei MS) |
| ent | enantiomerenrein, Enantiomer |
| Äq. | Äquivalent(e) |
| ESI | Elektrospray-Ionisation (bei MS) |
| Et | Ethyl |
| GC | Gaschromatographie |
| GC/MS | Gaschromatographie-gekoppelte Massenspektrometrie |
| h | Stunde(n) |
| HATU | O-(7-Azabenzotriazol-1-yl)-N,N,N',N'-tetramethyluroniumhexafluorophosphat; |
| HPLC | Hochdruck-, Hochleistungsflüssigchromatographie |
| konz. | konzentriert (bei Lösung) |
| LC | Flüssigchromatographie |
| LC/MS | Flüssigchromatographie-gekoppelte Massenspektrometrie |
| Lit. | Literatur(stelle) |
| m | Multiplett (bei NMR) |
| M | molar (bei Lösung) |
| Me | Methyl |
| min | Minute(n) |
| MS | Massenspektrometrie |
| NMP | *N*-Methyl-2-pyrrolidon |
| NMR | Kernresonanzspektrometrie |
| OXONE® | Kaliumperoxomonosulfat (2 $KHSO_5$*$KHSO_4$*$K_2SO_4$) |
| PyBOP | 1-*H* Benzotriazol-1-yloxy-tris(pyrrolidino)phosphonium-hexafluorophosphat |
| q (oder quart) | Quartett (bei NMR) |
| qd | Quartett von Dublett (bei NMR) |
| quant. | quantitativ (bei chemischer Ausbeute) |
| quint | Quintett (bei NMR) |
| rac | racemisch, Racemat |
| RP | reverse phase (Umkehrphase, bei HPLC) |
| RT | Raumtemperatur |
| $R_t$ | Retentionszeit (bei HPLC, LC/MS) |
| s | Singulett (bei NMR) |
| sept | Septett (bei NMR) |
| SFC | superkritische Flüssigchromatographie |
| t | Triplett (bei NMR) |
| tBu | tert.-Butyl |
| td | Triplett von Dublett (bei NMR) |
| THF | Tetrahydrofuran |

| | |
|---|---|
| UV | Ultraviolett-Spektrometrie |
| vgl. | vergleiche |
| v/v | Volumen zu Volumen-Verhältnis (einer Lösung) |
| Xantphos | 9,9-Dimethyl-4,5-bis-(diphenylphosphino)-xanthen |
| zus. | zusammen |

**HPLC- und LC/MS-Methoden:**

Methode 1 (LC/MS):

Instrument: Waters Acquity SQD UPLC System; Säule: Waters Acquity UPLC HSS T3, 1.8 µm, 50 x 1 mm; Eluent A: 1l Wasser + 0.25 ml 99%-ige Ameisensäure, Eluent B: 1l Acetonitril + 0.25 ml 99%-ige Ameisensäure; Gradient: 0.0 min 90% A → 1.2 min 5% A → 2.0 min 5% A; Ofen: 50°C; Fluss: 0.40 ml/min; UV-Detektion: 210-400 nm.

Methode 2 (LC/MS):

Instrument MS: Waters (Micromass) QM; Instrument HPLC: Agilent 1100 Serie; Säule : Agilent ZORBAX Extend-C18 3.0 x 50mm 3.5 µm; Eluent A: 1l Wasser + 0.01 mol Ammoniumcarbonat, Eluent B: 1 l Acetonitril; Gradient: 0.0 min 98% A → 0.2min 98% A → 3.0 min 5% A→ 4.5 min 5% A ; Ofen: 40°C; Fluss: 1.75 ml/min; UV-Detektion: 210 nm

Methode 3 (LC/MS):

Gerätetyp MS: Thermo Scientific FT-MS; Gerätetyp UHPLC+: Thermo Scientific UltiMate 3000; Säule: Waters, HSST3, 2.1 x 75 mm, C18 1.8 µm; Eluent A: 1 l Wasser + 0.01% Ameisensäure; Eluent B: 1 l Acetonitril + 0.01% Ameisensäure; Gradient: 0.0 min 10% B → 2.5 min 95% B → 3.5 min 95% B; Ofen: 50°C; Fluss: 0.90 ml/min; UV-Detektion: 210 nm/ Optimum Integration Path 210-300 nm.

Methode 4 (LC/MS):

Instrument: Waters ACQUITY SQD UPLC System; Säule: Waters Acquity UPLC HSS T3, 1.8 µm 50 x 1 mm; Eluent A: 1 l Wasser + 0.25 ml 99%ige Ameisensäure , Eluent B: 1 l Acetonitril + 0.25 ml 99%ige Ameisensäure; Gradient: 0.0 min 95% A → 6.0 min 5% A → 7.5 min 5% A Ofen: 50°C; Fluss: 0.35 ml/min; UV-Detektion: 210 - 400 nm.

Methode 5 (LC/MS):

Instrument: Agilent MS Quad 6150;HPLC: Agilent 1290; Säule: Waters Acquity UPLC HSS T3 1.8 µ 50 x 2.1 mm; Eluent A: 1 l Wasser + 0.25 ml 99%ige Ameisensäure , Eluent B: 1 l Acetonitril + 0.25 ml 99%ige Ameisensäure; Gradient: 0.0 min 90% A → 0.3 min 90% A → 1.7 min 5% A → 3.0 min 5% A Ofen: 50°C; Fluss: 1,20 ml/min; UV-Detektion: 205 - 305 nm.

Methode 6 (GC/MS):

Instrument: Thermo DFS, Trace GC Ultra; Säule: Restek RTX-35, 15 m x 200 µm x 0.33 µm; konstanter Fluss mit Helium: 1.20 ml/min; Ofen: 60°C; Inlet: 220°C; Gradient: 60°C, 30°C/min → 300°C (3.33 min halten).

Methode 7 (präparative HPLC):

Säule: Chromatorex C18, 250 x30 mm; Eluent A: Wasser + 0.1% Ameisensäure, Eluent B: Acetonitril; Probeninjektion bei 3.0 min, Gradient: 0.0 min 10% B → 5.0 min 10% B → 25 min 80% B → 30 min 95% B → 35 min 10% B; Fluss: 50 ml/min. UV-Detektion: 210 nm.

**Weitere Angaben:**

Die Prozentangaben in den folgenden Beispiel- und Testbeschreibungen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

Bei Aufreinigungen von erfindungsgemäßen Verbindungen per präparativer HPLC nach den oben beschriebenen Methoden, in denen die Elutionsmittel Zusatzstoffe wie beispielsweise Trifluoressigsäure, Ameisensäure oder

Ammoniak enthalten, können die erfindungsgemäßen Verbindungen in Salz-Form, beispielsweise als Trifluoracetat, Formiat oder Ammonium-Salz anfallen, sofern die erfindungsgemäßen Verbindungen ausreichend basische bzw. saure Funktionalitäten enthalten. Ein solches Salz kann durch verschiedene dem Fachmann bekannte Methoden in die entsprechende freie Base bzw. Säure überfuhrt werden.

Reinheitsangaben beziehen sich in der Regel auf entsprechende Peak-Integrationen im LC/MS-Chromatogramm, können aber zusätzlich auch unter Zuhilfenahme des H-NMR-Spcktrums ermittelt worden sein. Wenn keine Reinheit angegeben ist, handelt es sich in der Regel um eine 100%-Reinheit laut automatischer Peak-Integration im LC/MS-Chromatogramm oder die Reinheit wurde nicht explizit ermittelt.

Angaben zu Ausbeuten in % d. Th. sind in der Regel reinheitskorrigiert, sofern eine Reinheit <100% angegeben ist. Bei lösungsmittelhaltigen oder verunreinigten Chargen kann die Ausbeute formal ">100%" betragen; in diesen Fällen ist die Ausbeute nicht lösungsmittel- bzw. reinheitskorrigiert.

Die nachfolgenden Beschreibungen der Kopplungsmuster von H-NMR-Signalen wurden teilweise direkt den Vorschlägen des ACD SpecManagers (ACD/Labs Release 12.00, Product version 12.5) entnommen und nicht notwendigerweise streng hinterfragt. Teilweise wurden die Vorschläge des SpecManagers manuell angepasst. Manuell angepasste bzw. zugewiesene Beschreibungen orientieren sich in der Regel an dem optischen Erscheinungsbild der betreffenden Signale und entsprechen nicht notwendigerweise einer strengen, physikalisch korrekten Interpretation. In der Regel bezieht sich die Angabe zur chemischen Verschiebung auf das Zentrum des betreffenden Signals. Bei breiten Multipletts erfolgt die Angabe eines Intervalls. Durch Lösungsmittel oder Wasser verdeckte Signale wurden entweder tentativ zugeordnet oder sind nicht aufgeführt. Stark verbreiterte Signale - z.B. verursacht durch schnelle Rotation von Molekülteilen oder aufgrund von austauschenden Protonen - wurden ebenfalls tentativ zugeordnet (oft als breites Multiplett oder breites Singulett bezeichnet) oder sind nicht aufgeführt.

Die H-NMR-Daten ausgewählter Beispiele werden in Form von H-NMR-Peaklisten notiert. Zu jedem Signalpeak wird erst der $\delta$-Wert in ppm und dann die Signalintensität in runden Klammem aufgeführt. Die $\delta$-Wert-Signalintensitäts-Zahlenpaare von verschiedenen Signalpeaks werden durch Kommata voneinander getrennt aufgelistet. Die Peakliste eines Beispieles hat daher die Form: $\delta_1$ (Intensität$_1$), $\delta_2$ (Intensität$_2$), ... , $\delta_i$ (Intensität$_i$), ... , $\delta_n$ (Intensität$_n$).

Die Intensität scharfer Signale korreliert mit der Höhe der Signale in einem gedruckten Beispiel eines NMR-Spektrums in cm und zeigt im Vergleich mit anderen Signalen die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden. Die Listen der $^1$H-NMR-Peaks sind ähnlich den klassischen $^1$H-NMR-Ausdrucken und enthalten somit gewöhnlich alle Peaks, die bei einer klassischen NMR-Interpretation aufgeführt werden. Darüber hinaus können sie wie klassische H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen. Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%). Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen. Ein Experte, der die Peaks der Zielverbindungen mit bekannten Verfahren (MestreC, ACD-Simulation, oder unter Verwendung von empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen $^1$H-NMR-Interpretation. Eine detaillierte Beschreibung der Darstellung von NMR-Daten in Form von Peaklisten kann der Publikation "Citation of NMR Peaklist Data within Patent Applications" entnommen werden (vgl. Research Disclosure Database Number 605005, 2014, 1. August 2014 oder http://www.researchdisclosure.com/searching-disclosures). In der Peak Picking Routine, die in der Research Disclosure Database Number 605005 beschrieben ist, kann der Parameter "MinimumHeight" zwischen 1% und 4% eingestellt werden. Abhängig von der Art der chemischen Struktur und/oder abhängig von der Konzentration der zu vermessenden Verbindung kann es sinnvoll sein, den Parameter "MinimumHeight" auf Werte <1% einzustellen.

Schmelzpunkte und Schmelzbereiche, soweit angegeben, sind nicht korrigiert.

Für alle Reaktanden oder Reagenzien, deren Herstellung im Folgenden nicht explizit beschrieben ist, gilt, dass sie von allgemein zugänglichen Quellen kommerziell bezogen wurden. Für alle übrigen Reaktanden oder Reagenzien, deren Herstellung im Folgenden ebenfalls nicht beschrieben ist und die nicht kommerziell erhältlich waren oder von Quellen bezogen wurden, die nicht allgemein zugänglich sind, ist ein Verweis auf die veröffentlichte Literatur angegeben, in der ihre Herstellung beschrieben ist.

## Allgemeine Arbeitsvorschriften

### AAV1

Eine Lösung der entsprechenden Carbonsäure (1-2 Äq.) in DMF (0.08-0.12M) wurde mit *N,N*-Diisopropylethylamin (1.4-1.5 Äq. bzw. 2.4-3.0 Äq. wenn das Amin als Hydrochlorid eingesetzt wurde) und HATU (1.0-1.65 Äq.) versetzt und die Mischung für 30 min bei RT gerührt. Anschließend wurde das entsprechende Amin (1.04-1.5 Äq.) zugegeben und die Mischung für 0.25-2h bei Raumtemperatur nachgerührt. Die Reaktion wurde dann durch die Zugabe von Wasser und 1 M wässriger Salzsäure beendet. Der Niederschlag wurde ab filtriert, in DCM aufgenommen, über Magnesiumsulfat getrocknet, filtriert und das Lösungsmittel unter reduziertem Druck entfernt. Alternativ wurde nach dem Ansäuern mit Essigsäureethylester extrahiert, die vereinigten organischen Phasen über Magnesiumsulfat getrocknet, filtriert und das Lösungsmittel unter reduziertem Druck entfernt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient) gereinigt.

**AAV2**

Kalium- oder Cäsiumcarbonat (1.5-2.5 Äq.) wurden im Reaktionsgefäß im Vakuum ausgeheizt. Es wurde auf RT abgekühlt und mit Argon geflutet. Palladiumacetat (0.1-0.36 Äq.), 9,9-Dimethyl-4,5-bis-(diphenylphosphino)-xanthen (Xantphos, 0.18-0.36 Äq.) und Dioxan (0.04-0.12M) wurden zugegeben und die Suspension wurde für 10 min bei Raumtemperatur im Argonstrom entgast. Anschließend wurde das entsprechende Amid (1.0-1.2 Äq.) und das entsprechende 7-Chlor-4-oxo-1,4-dihydro-1,8-naphthyridin (1.0 Äq.) zugegeben. Es wurde für 1 h (oder bis zum vollständigen Umsatz nach analytischer HPLC oder Dünnschichtchromatographie mit entsprechenden Laufmittelgemischen) bei 80-110°C gerührt. Es wurde auf RT abgekühlt und alle flüchtigen Komponenten unter reduziertem Druck entfernt oder alternativ das Reaktionsgemisch auf Wasser gegossen, der pH-Wert mit 1M wässriger Salzsäure auf pH 1 eingestellt, mit Essigsäureethylester extrahiert, die vereinigten organischen Phasen mit gesättigter, wässriger Natriumchlorid-Lösung gewaschen, über Magnesiumsulfat getrocknet, filtriert und das Lösungsmittel unter reduziertem Druck entfernt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient) gereinigt.

**AAV3**

Eine Lösung des entsprechenden 7-Chlor-4-oxo-1,4-dihydro-1,8-naphthyridins in DMF (0.10-0.22M) wurde nacheinander mit dem entsprechenden Amin (1.2 Äq.) und DIPEA (1.5-3.5 eq) versetzt. Die Reaktionslösung wurde über Nacht bei RT gerührt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient) gereinigt.

**Ausgangsverbindungen und Intermediate:**

**Beispiel 1A**

*rac*-5-Methl-1,2-oxazolidin-5-carbonsäuremethlester

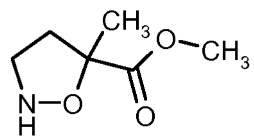

Zu einer Lösung von 20.0 g (288 mmol) Hydroxylamin Hydrochlorid und 11.5 g (288 mmol) Natriumhydroxid in 20 ml Methanol und 40 ml Wasser wurden 21.7 ml (290 mmol) Paraformaldehyd (37% in Wasser) so langsam zugetropft, dass die interne Temperatur 35°C nicht überstieg. Anschließend wurden 31.0 ml (288 mmol) Methylacrylsäuremethylester zugegeben und nach vollständiger Zugabe für 2h bei 70°C gerührt. Die Mischung wurde auf Raumtemperatur abgekühlt und mit DCM extrahiert. Die vereinigten organischen Phasen wurden über Natriumsulfat getrocknet, filtriert und das Lösungsmittel unter reduziertem Druck entfernt. Es wurden 3.70 g (8.5% d. Th., 96% Reinheit) der Titelverbindung nach Vakuumdestillation (0.7 mbar, 78-84°C) erhalten.
$^1$H-NMR (400 MHz, $CDCl_3$): δ [ppm] = 6.05 (br. s, 1H), 3.76 (s, 3H), 3.27-3.36 (m, 1H), 3.12-3.24 (m, 1H), 2.45-2.57 (m, 1H), 2.07-2.17 (m, 1H), 1.55 (s, 3H).
GC/MS [Methode 6]: $R_t$ = 3.51 min; MS: m/z = 115.

**Beispiel 2A**

*rac*-3-Hydroxy-3-methylpyrrolidin-2-on

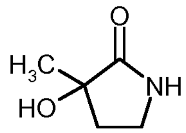

Eine Lösung von 3.70 g (25.5 mmol) der Verbindung aus Beispiel 1A in 300 ml Ethanol wurde mit 3.80 g (3.57 mmol) Palladium (10% auf Kohle) versetzt und über Nacht unter einer Wasserstoff-Atmosphäre (Normaldruck) gerührt. Die Mischung wurde dann über Celite filtriert und das Lösungsmittel unter reduziertem Druck entfernt. Der erhaltene Feststoff wurde anschließend mit Acetonitril verrührt, der Niederschlag abgesaugt, zweimal mit 1 ml Acetonitril gewaschen und im Hochvakuum getrocknet. Es wurden 1.97 g (55% d. Th.; 82% Reinheit) der Titelverbindung erhalten.
$^1$H-NMR (400 MHz, $CDCl_3$): $\delta$ [ppm] = 5.71 (br. s, 1H), 3.37-3.43 (m, 1H), 3.24-3.31 (m, 1H), 2.64 (s, 1H), 2.24-2.33 (m, 1H), 2.13-2.21 (m, 1H), 1.40 (s, 3H).
GC/MS [Methode 6]: $R_t$ = 3.14 min; MS: m/z = 145.

**Beispiel 3A**

7-Chlor-1-(2- fluorphenyl)-4-oxo-1,4-dihydro-1,8-naphthyridin-3-carbonsäureethylester

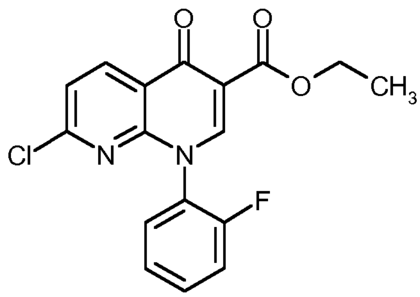

Zu einer Lösung von 11.1 g (35.0 mmol) 2-[(2,6-Dichlorpyridin-3-yl)carbonyl]-3-(dimethylamino)acryl-säureethylester (CAS 635309-52-3) in 80 ml Ethanol wurden 4.67 g (42.0 mmol) 2-Fluoranilin in 21 ml THF gegeben und die Reaktionsmischung über Nacht bei RT gerührt. Anschließend wurde das Lösungsmittel unter reduziertem Druck entfernt, der Rückstand in 110 ml DMF aufgenommen und mit 7.26 g (52.5 mmol) Kaliumcarbonat versetzt. Die Suspension wurde dann für 3 h bei 100 °C gerührt, anschließend auf RT abgekühlt und auf 200 ml Wasser gegeben. Der Niederschlag wurde abgesaugt, mit Wasser gewaschen, dann in 300 ml Essigsäureethylester gelöst, dreimal mit 50 ml Wasser gewaschen, über Natriumsulfat getrocknet, filtriert und das Lösungsmittel unter reduziertem Druck entfernt. Der Rückstand wurde in wenig DCM aufgenommen und mittels Flashchromatographie (Essigsäureethylester-Cyclohexan Gradient, dann Methanol-DCM, 5/95) gereinigt. Es wurden 1.53 g (12% d. Th., 99% Reinheit) der Titelverbindung erhalten. Weiterhin wurden 1.33 g (11% d. Th., 99% Reinheit) der Titelverbindung aus Beispiel 32A erhalten (Analytik s. Beispiel 32A).
$^1$H-NMR (400 MHz, DMSO-$d_6$): $\delta$ [ppm] = 8.71 (s, 1H), 8.62 (d, 1H), 7.73-7.78 (m, 1H), 7.65-7.69 (m, 1H), 7.65 (d, 1H), 7.50-7.56 (m, 1H), 7.43-7.48 (m, 1H), 4.24 (q, 2H), 1.27 (t, 3H).
LC-MS (Methode 1): $R_t$ = 0.94 min; 347 $[M+H]^+$.

**Beispiel 4A**

7-Chlor-1-(2-chlorphenyl)-4-oxo-1,4-dihydro-1,8-naphthyridin-3-carbonsäureethylester

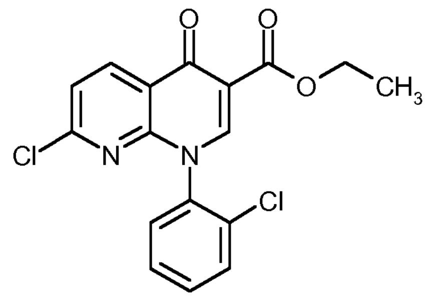

Eine Lösung aus 6.05 g (19.0 mmol) 2-[(2,6-Dichlorpyridin-3-yl)carbonyl]-3-ethoxyacrylsäureethylester (CAS 157373-27-8) und 3.39 g (26.6 mmol) 2-Chloranilin in 30.2 ml DCM wurde mit 23.2 ml (133 mmol) DIPEA versetzt und für 4h bei RT gerührt. Anschließend wurden 2.63 g (19.0 mmol) Kaliumcarbonat zugegeben und über Nacht unter Rückfluss erhitzt. Die Mischung wurde mit 200 ml DCM verdünnt und zweimal mit 75 ml 1M wässriger Salzsäure gewaschen. Die organische Phase wurde über Natriumsulfat getrocknet, filtriert und das Lösungsmittel unter reduziertem Druck entfernt. Die erhaltene Suspension wurde mit 40 ml *tert.*-Butylmethylether verrührt, der Niederschlag abgesaugt, mit 10 ml *tert.*-Butylmethylether gewaschen und im Hochvakuum getrocknet. Es wurden 3.71 g (53% d. Th., 99% Reinheit) der Titelverbindung erhalten.

$^1$H-NMR (400 MHz, DMSO-$d_6$): $\delta$ [ppm] = 8.65 (s, 1H), 8.63 (d, 1H), 7.82-7.75 (m, 2H), 7.59-7.68 (m, 3H), 4.24 (q, 2H), 1.27 (t, 3H).

LC-MS (Methode 3): $R_t$ = 1.81 min; 363 [M+H]$^+$.

**Beispiel 5A**

7-Chlor-1-(2-chlor-4-fluorphenyl)-4-oxo-1,4-dihydro-1,8-naphthyridin-3-carbonsäureethylester

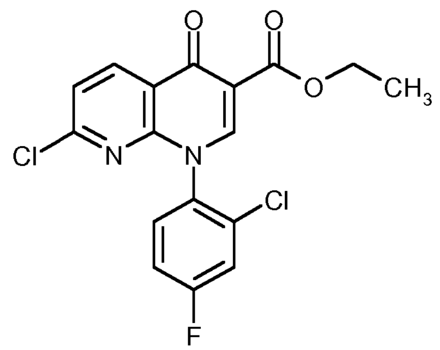

Eine Mischung aus 5 g (13.4 mmol) 2-[(2,6-Dichlorpyridin-3-yl)carbonyl]-3-ethoxyacrylsäureethylester (CAS 157373-27-8), 10.36 g (80.2 mmol) DIPEA und 2.92 g (20.1 mmol) 2-Chlor-4-fluoranilin in 50 ml Dichlormethan wurde 20 Stunden lang bei 23°C gerührt. Anschliessend wurde die Mischung im Vakuum eingeengt, dann in Ethylacetat aufgenommen und dreimal mit Wasser und einmal mit gesättigter Kochsalzlösung gewaschen. Die organische Phase wurde im Vakuum eingeengt und im Hochvakuum getrocknet. Der Rückstand wurde anschließend in 80 ml Dioxan gelöst, unter Eiskühlung mit einer Lösung von 1 g (9.3 mmol) Kalium-tert.-butoxid in 20 ml Dioxan versetzt und 15 h bei 23°C gerührt. Die Lösung wurde dann auf Eiswasser gegeben und der ausgefallene Feststoff abgesaugt, mit Wasser nachgewaschen und im Hochvakuum getrocknet. Es wurden 3.3 g (56 % d. Th., Reinheit 87%) der Titelverbindung erhalten.

LC-MS (Methode 1): $R_t$ = 1.04 min; m/z = 381.1 [M+H]$^+$.

**Beispiel 6A**

1-(2,4-Difluorphenyl)-7-(dimethylamino)-4-oxo-1,4-dihydro-1,8-naphthyridin-3-carbonsäureethylester

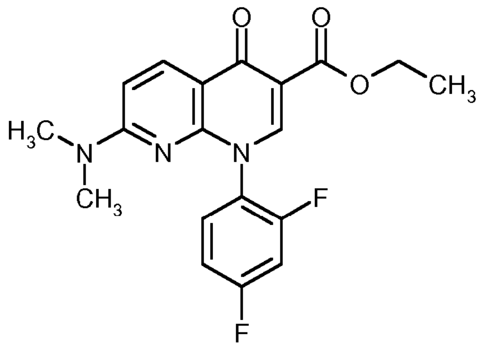

Eine Mischung aus 2 g (5.5 mmol) 7-Chlor-1-(2,4-difluorphenyl)-4-oxo-1,4-dihydro-1,8-naphthyridin-3-carbonsäureethylester (Darstellung beschrieben in DE 4301246, Beispiel U, S.26), 894 mg (11 mmol) Dimethylaminhydrochlorid und 2.48 g (19.2 mmol) DIPEA in 50 ml Acetonitril wurde 18 Stunden lang bei 23°C gerührt. Anschliessend wurde die Mischung im Vakuum eingeengt, mit Wasser versetzt und die wässrige Phase mit Ethylacetat extrahiert. Die vereinigten organischen Phasen wurde mit gesättigter Kochsalzlösung gewaschen, über Magnesiumsulfat getrocknet und im Vakuum eingeengt. Man erhielt 1.92 g (94 % d. Th.) der Titelverbindung.
LC-MS (Methode 1): $R_t$ = 0.95 min; m/z = 374.1 $[M+H]^+$.

In Analogie zu Beispiel 6A wurden die in Tabelle 1A gezeigten Beispielverbindungen hergestellt, indem 7-Chlor-1-(2,4-difluorphenyl)-4-oxo-1,4-dihydro-1,8-naphthyridin-3-carbonsäureethylester, bzw. die Verbindung aus Beispiel 5A, mit den entsprechenden Aminen (bzw. deren Salzen) und DIPEA unter den beschriebenen Reaktionsbedingungen umgesetzt wurden. Abweichungen sind bei den jeweiligen Beispielen angegeben.

Tabelle 1A:

| Bsp. | IUPAC-Name / Struktur / (Ausbeute) | Analytische Daten |
|---|---|---|
| **7A** | 1-(2,4-Difluorphenyl)-7-(methylamino)-4-oxo-1,4-dihydro-1, 8-naphthyridin-3 -carbonsäureethylester 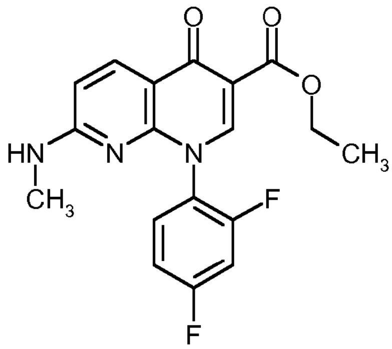 Lösungsmittel: THF / MeCN / NMP; 4 Äq. Methylamin (2 M in THF); 3.5 Äq. DIPEA; 23°C für 17 h, dann 40°C für 8h (61 % d. Th.) | LC-MS (Methode 1): $R_t$ = 0.89 min MS (ESpos): m/z = 360.2 $[M+H]^+$ |
| **8A** | 1-(2,4-Difluorphenyl)-4-oxo-7-(propan-2-yl-amino)-1,4-dihydro-1,8-naphthyridin-3-carbonsäureethylester 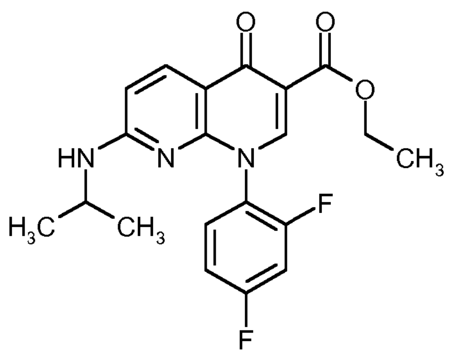 | LC-MS (Methode 1): $R_t$ = 0.99 min MS (ESpos): m/z = 388.3 $[M+H]^+$ |

(fortgesetzt)

| Bsp. | IUPAC-Name / Struktur / (Ausbeute) | Analytische Daten |
|---|---|---|
| | (68 % d. Th.) | |
| **9A** | 1 -(2,4-Difluorphenyl)-7- [(2-hydroxyethyl)-(methyl)amino]-4-oxo-1,4-dihydro-1,8-naphthyridin-3 -carbonsäureethylester 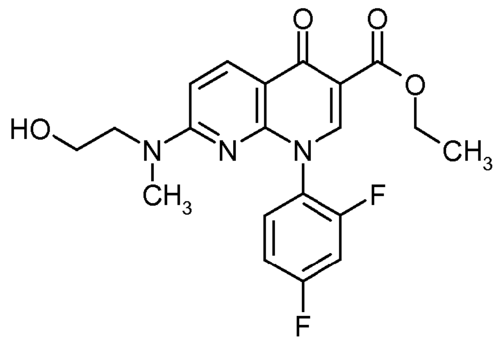 (93 % d. Th.) | LC-MS (Methode 1): $R_t$ = 0.85 min MS (ESpos): m/z = 404.2. $[M+H]^+$ |
| **10A** | 1-(2,4-Difluorphenyl)-7-[(2-hydroxyethyl)amino]-4-oxo-1,4-dihydro-1,8-naphthyridin-3-carbonsäureethylester 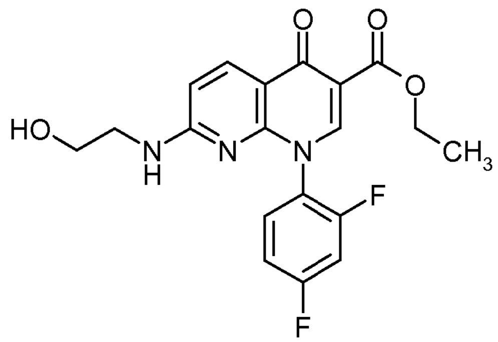 (63 % d. Th.) | LC-MS (Methode 1): $R_t$ = 0.80 min MS (ESpos): m/z = 390.2 $[M+H]^+$ |
| **11A** | 1-(2,4-Difluorphenyl)-7-[(2-fluoroethyl)amino]-4-oxo-1,4-dihydro-1,8-naphthyridin-3-carbonsäure-ethylester 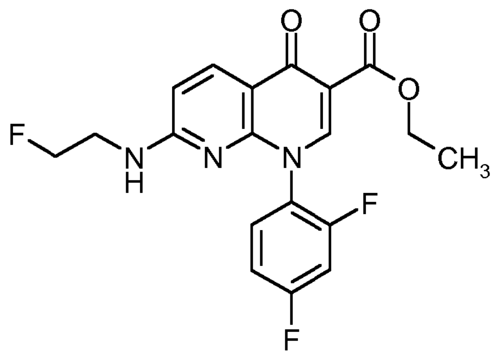 (38 % d. Th.) | LC-MS (Methode 1): $R_t$ = 0.90 min MS (ESpos): m/z = 392.1 $[M+H]^+$ |
| **12A** | 1 -(2,4-Difluorphenyl)-7- [(2-fluoroethyl)-(methyl)amino]-4-oxo-1,4-dihydro-1,8-naphthyridin-3 -carbonsäureethylester | LC-MS (Methode 1): $R_t$ = 0.96 min MS (ESpos): m/z = 406.1 $[M+H]^+$ |

(fortgesetzt)

| Bsp. | IUPAC-Name / Struktur / (Ausbeute) | Analytische Daten |
|---|---|---|
| | 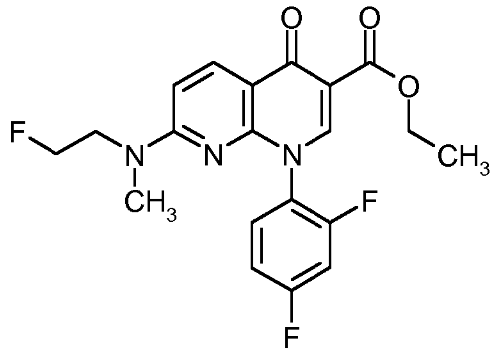 (64 % d. Th.) | |
| **13A** | 1-(2,4-Difluorphenyl)-7-(3,3-difluorpyrrolidin-1-yl)-4-oxo-1,4-dihydro-1,8-naphthyridin-3-carbonsäureethylester 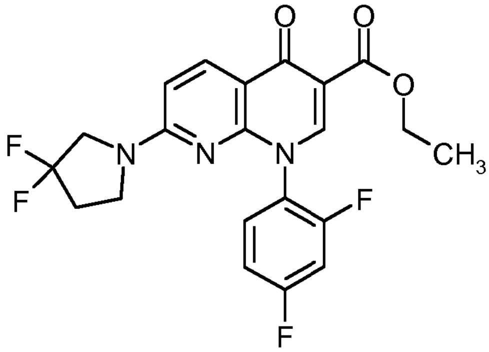 (83 % d. Th.) | LC-MS (Methode 1): $R_t$ = 1.02 min MS (ESpos): m/z = 436.1 [M+H]$^+$ |
| **14A** | 1-(2,4-Difluorphenyl)-7-[(3*R*)-3-fluorpyrrolidin-1-yl]-4-oxo-1,4-dihydro-1,8-naphthyridin-3-carbonsäureethylester 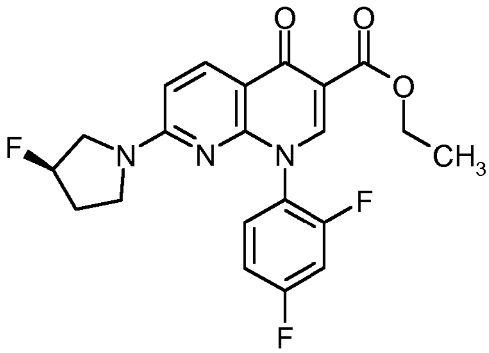 (72 % d. Th.) | LC-MS (Methode 1): $R_t$ = 0.97 min MS (ESpos): m/z = 417.9 [M+H]$^+$ |
| **15A** | 1-(2,4-Difluorphenyl)-7-[(3*S*)-3-fluorpyrrolidin-1-yl]-4-oxo-1,4-dihydro-1,8-naphthyridin-3-carbonsäureethylester | LC-MS (Methode 1): $R_t$ = 0.97 min MS (ESpos): m/z = 418.1 [M+H]$^+$ |

(fortgesetzt)

| Bsp. | IUPAC-Name / Struktur / (Ausbeute) | Analytische Daten |
|---|---|---|
| | 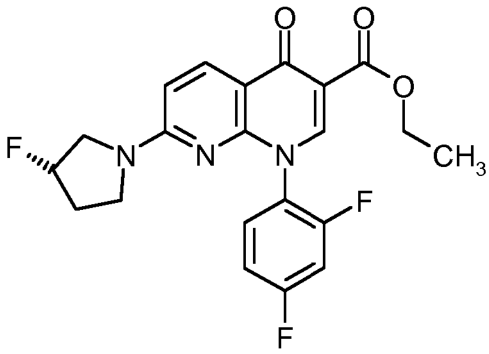 (90 % d. Th.) | |
| **16A** | 1-(2,4-Difluorphenyl)-4-oxo-7-(3,3,4,4-tetrafluorpyrrolidin-1-yl)-1,4-dihydro-1,8-naphthyridin-3 -carbonsäureethylester 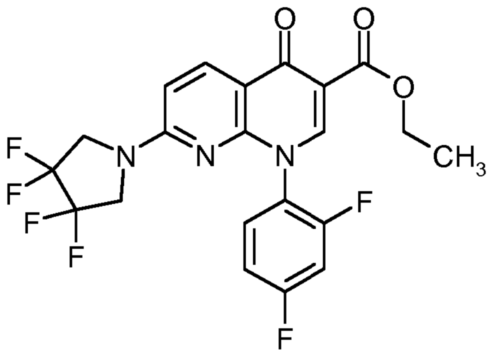 (50 % d. Th.) | LC-MS (Methode 1): $R_t$ = 1.05 min MS (ESpos): m/z = 472.2 $[M+H]^+$ |
| **17A** | 7-[(2,2-Difluorethyl)(methyl)amino] -1-(2,4-difluorphenyl)-4-oxo-1,4-dihydro-1,8-naphthyridin-3 -carbonsäureethylester 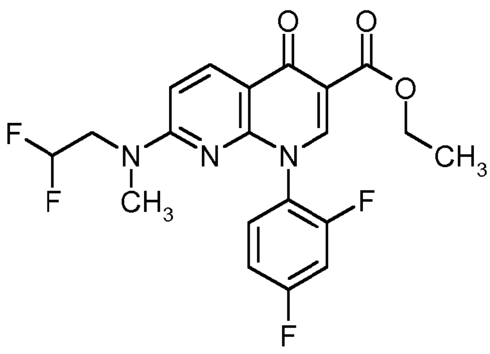 4 Tage bei 23°C (84 % d. Th.) | LC-MS (Methode 1): $R_t$ = 0.98 min MS (ESpos): m/z = 424.1 $[M+H]^+$ |
| **18A** | 7-[(2,2-Difluoroethyl)amino]-1-(2,4-difluorphenyl)-4-oxo-1 ,4-dihydro-1 ,8-naphthyridin-3-carbonsäureethylester | LC-MS (Methode 1): $R_t$ = 0.93 min MS (ESpos): m/z = 409.9 $[M+H]^+$ |

(fortgesetzt)

| Bsp. | IUPAC-Name / Struktur / (Ausbeute) | Analytische Daten |
|---|---|---|
| | 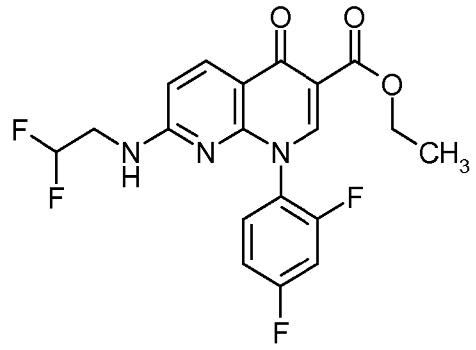 (47 % d. Th.) | |
| **19A** | 1-(2,4-Difluorphenyl)-4-oxo-7-(1,3-thiazolidin-3-yl)-1,4-dihydro-1,8-naphthyridin-3-carbonsäureethylester 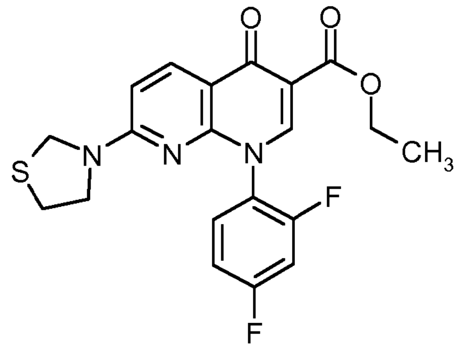 Lösungsmittel: DMF; 23°C für 2 Tage; dann 50°C für 18h; dann 70°C für 18h; (65 % d. Th.) | LC-MS (Methode 2): $R_t$ = 1.01 min MS (ESpos): m/z = 418.2 $[M+H]^+$ |
| **20A** | *rac*-1-(2,4-Difluorphenyl)-7-[2-(hydroxymethyl)-morpholin-4-yl]-4-oxo-1 ,4-dihydro-1 ,8-naphthyridin-3-carbonsäureethylester 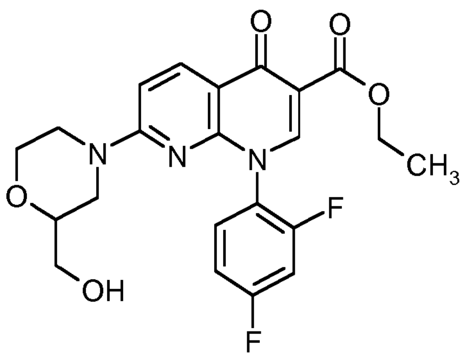 (20 % d. Th.) | LC-MS (Methode 1): $R_t$ = 0.79 min MS (ESpos): m/z = 446.3 $[M+H]^+$ |
| **21A** | *rac*-1-(2,4-Difluorphenyl)-7-[2-(hydroxymethyl)-pyrrolidin-1 -yl]-4-oxo-1,4-dihydro-1,8-naphthyridin-3-carbonsäureethylester | LC-MS (Methode 1): $R_t$ = 0.87 min MS (ESpos): m/z = 430.2 $[M+H]^+$ |

(fortgesetzt)

| Bsp. | IUPAC-Name / Struktur / (Ausbeute) | Analytische Daten |
|---|---|---|
| | 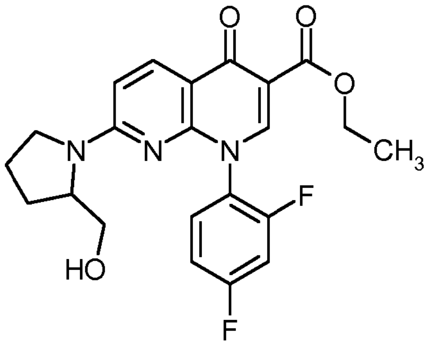 (76 % d. Th.) | |
| **22A** | *rac*-1-(2,4-Difluorphenyl)-7- [3 -(hydroxymethyl)-morpholin-4-yl]-4-oxo-1 ,4-dihydro-1 ,8-naphthyridin- 3 -carbonsäureethylester 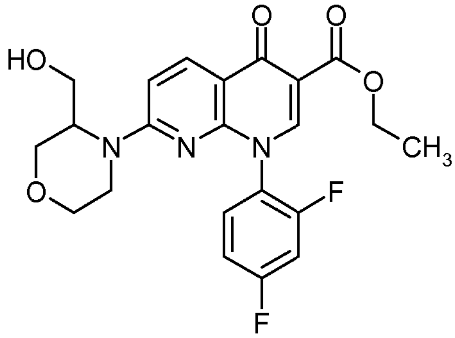 (30 % d. Th.) | LC-MS (Methode 1): $R_t$ = 0.81 min MS (ESpos): m/z = 446.2 $[M+H]^+$ |
| **23A** | *rac*-1-(2,4-Difluorphenyl)-7-(3 -hydroxy-3 -methylpiperidin-1-yl)-4-oxo-1,4-dihydro-1,8-naphthyridin-3 -carbonsäureethylester 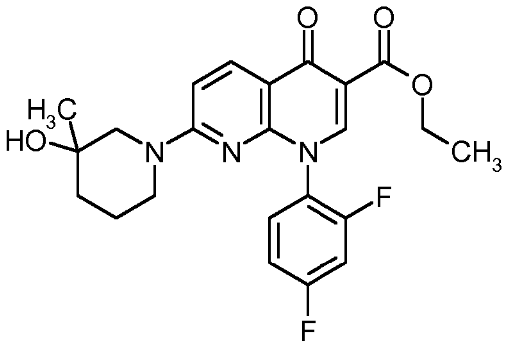 (37 % d. Th.) | LC-MS (Methode 1): $R_t$ = 0.91 min MS (ESpos): m/z = 444.3 $[M+H]^+$ |
| **24A** | 1-(2,4-Difluorphenyl)-7-[methyl(2,2,2-trifluorethyl)amino]-4-oxo-1,4-dihydro-1,8-naphthyridin-3 -carbonsäureethylester | LC-MS (Methode 1): $R_t$ = 1.02 min MS (ESpos): m/z = 442.2 $[M+H]^+$ |

(fortgesetzt)

| Bsp. | IUPAC-Name / Struktur / (Ausbeute) | Analytische Daten |
|---|---|---|
| | 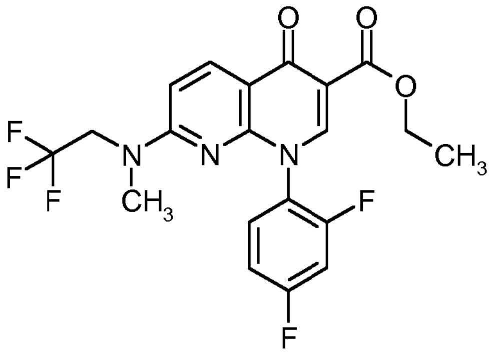<br>3.5 Äq. 2,2,2-Trifluor-*N*-methylethylamin Hydrochlorid und 5.5 Äq. DIPEA / 60-70°C/4 Tage (75 % d. Th.) | |
| **25A** | 1-(2,4-Difluorphenyl)-7-(morpholin-4-yl)-4-oxo-1,4-dihydro-1,8-naphthyridin-3-carbonsäureethylester<br>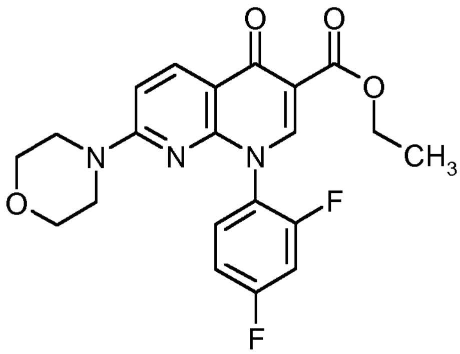<br>50°C / 4h in Acetonitril (82 % d. Th.) | LC-MS (Methode 1): $R_t$ = 0.93 min<br>MS (ESpos): m/z = 416.2 $[M+H]^+$ |
| **26A** | 1-(2,4-Difluorphenyl)-4-oxo-7-(pyrrolidin-1-yl)-1,4-dihydro-1,8-naphthyridin-3-carbonsäureethylester<br>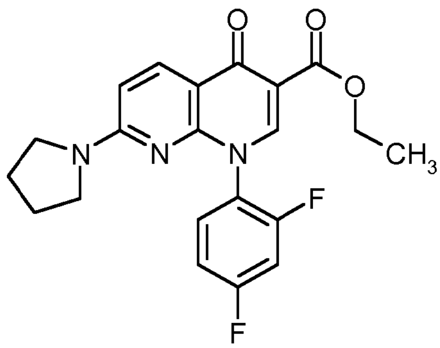<br>Lösungsmittel: DMF (96 % d. Th.) | LC-MS (Methode 1): $R_t$ = 1.06 min<br>MS (ESpos): m/z = 400.2 $[M+H]^+$ |
| **27A** | 1-(2,4-Difluorphenyl)-7-[4-hydroxy-4-(hydroxymethyl)piperidin-1-yl]-4-oxo-1,4-dihydro-1,8-naphthyridin-3-carbonsäureethylester | LC-MS (Methode 1): Rt = 0.76 min<br>MS (ESpos): m/z = 460.3 [M+H]+ |

(fortgesetzt)

| Bsp. | IUPAC-Name / Struktur / (Ausbeute) | Analytische Daten |
|---|---|---|
| | 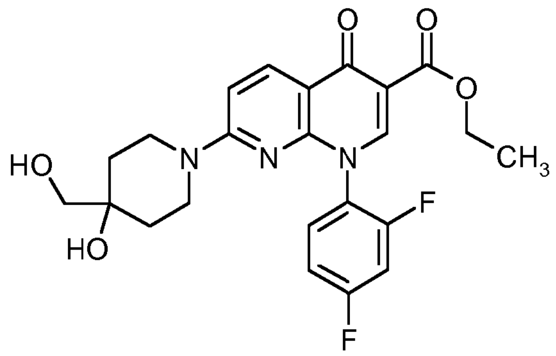<br>Lösungsmittel: NMP (17 % d. Th.) | |
| **28A** | 1-(2-Chlor-4-fluorphenyl)-7-(dimethylamino)-4-oxo-1,4-dihydro-1,8-naphthyridin-3-carbonsäureethylester<br>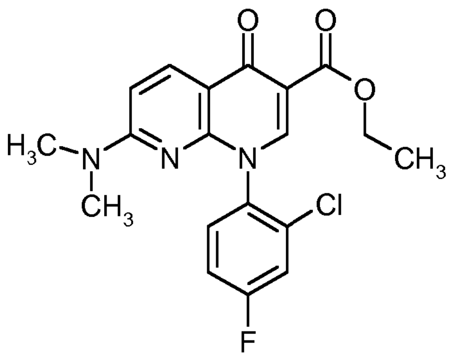<br>ausgehend von der Verbindung aus Beispiel 5A und Dimethylamin Hydrochlorid; Lösungsmittel: DMF; 17 h bei 23°C (82 % d. Th.) | LC-MS (Methode 1): $R_t$ = 1.01 min<br>MS (ESpos): m/z = 390.2 $[M+H]^+$ |
| **29A** | 1-(2,4-Difluorphenyl)-7-[4-(methoxycarbonyl)-piperazin-1-yl]-4-oxo-1,4-dihydro-1,8-naphthyridin-3-carbonsäureethylester<br>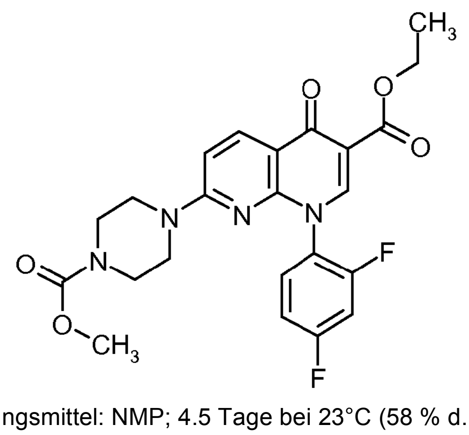<br>Lösungsmittel: NMP; 4.5 Tage bei 23°C (58 % d. Th.) | LC-MS (Methode 1): $R_t$ = 0.91 min<br>MS (ESpos): m/z = 473.3 $[M+H]^+$ |

**Beispiel 30A**

1-(2,4-Difluorphenyl)-4-oxo-7-[(2,2,2-trifluorethyl)amino]-1,4-dihydro-1,8-naphthyridin-3-carbonsäureethylester

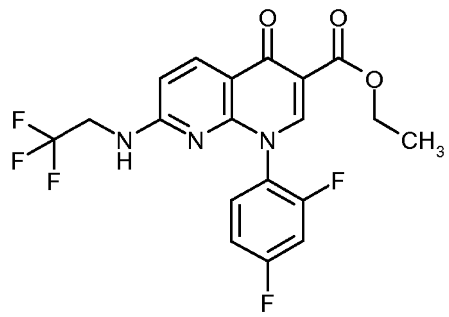

Eine Mischung aus 1 g (2.7 mmol) 7-Chlor-1-(2,4-difluorphenyl)-4-oxo-1,4-dihydro-1,8-naphthyridin-3-carbonsäureethylester (Darstellung beschrieben in DE 4301246, Beispiel U, S.26), und 1.9 g (19 mmol) 2,2,2-Trifluorethylamin in 3.5 ml NMP wurde eine Stunde lang bei 160°C in der Mikrowelle gerührt. Anschliessend wurde die Mischung mit 1 M wässr. Salzsäure auf pH 3 gebracht, mit Wasser versetzt, der ausgefallene Feststoff abgesaugt, mit Wasser und Petrolether gewaschen und im Hochvakuum getrocknet. Man erhielt 1.2 g (66 % d. Th.) der Titelverbindung.
$^1$H-NMR (400 MHz, DMSO-$d_6$): δ [ppm] = 2.40 (s, 3H), 7.49-7.56 (m, 3H), 7.61-7.63 (m, 2H), 7.90-7.94 (m, 2H), 8.01 (d, 1H), 14.39 (br. s, 1H).
LC-MS (Methode 1): $R_t$ = 0.91 min; MS (ESpos): m/z = 428.1 [M+H]$^+$

**Beispiel 31A**

1-(2,4-Difluorphenyl)-7-(1,1-dioxido-1,3-thiazolidin-3-yl)-4-oxo-1,4-dihydro-1,8-naphthyridin-3-carbonsäureethylester

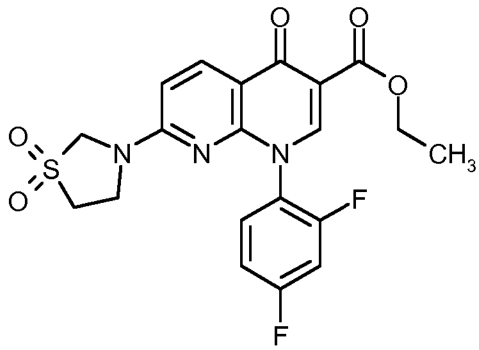

Eine Mischung aus 6.1 g (11.5 mmol; Reinheit 79%) der Verbindung aus Beispiel 19A, 28.3 g (46 mmol) OXONE® und 8 g (46 mmol) Dikaliumhydrogenphosphat in 88 ml Dioxan und 44 ml Wasser wurde 8 Stunden lang bei 23°C gerührt und dann 13 h stehengelassen. Anschliessend wurden 1 ml 1 M wässr. Salzsäure und 100 ml Wasser zugegeben, der ausgefallene Feststoff abgesaugt, mit Wasser und Petrolether gewaschen und im Hochvakuum getrocknet. Man erhielt 3.72 g (58 % d. Th.) der Titelverbindung .
LC-MS (Methode 1): Rt = 0.83 min; m/z = 450.2 [M+H]$^+$.

**Beispiel 32A**

7-(Dimethylamino)-1-(2-fluorphenyl)-4-oxo-1,4-dihydro-1,8-naphthyridin-3-carbonsäureethylester

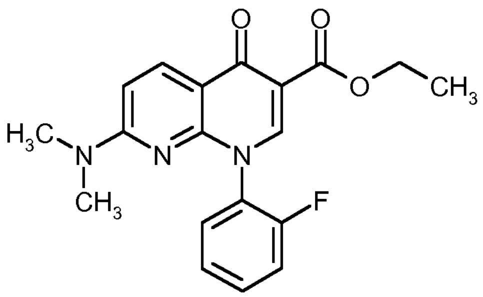

Wie bei der Darstellung der Verbindung aus Beispiel 3A beschrieben, wurden aus 11.1 g (35.0 mmol) 2-[(2,6-Dichlorpyridin-3-yl)carbonyl]-3-(dimethylamino)acrylsäureethylester, 1.33 g (11% d. Th., 99% Reinheit) der Titelverbindung erhalten.
$^1$H-NMR (400 MHz, DMSO-de): $\delta$ [ppm] = 8.43 (s, 1H), 8.21 (d, 1H), 7.70-7.65 (m, 1H), 7.64-7.57 (m, 1H), 7.50-7.44 (m, 1H), 7.43-7.38 (m, 1H), 6.82 (d, 1H), 4.20 (q, 2H), 2.90 (br. s, 6H), 1.25 (t, 3H).
LC-MS (Methode 3): $R_t$ = 1.64 min; 356 [M+H]$^+$.

**Beispiel 33A**

7-Chlor-1-(2-fluorphenyl)-4-oxo-1,4-dihydro-1,8-naphthyridin-3-carbonsäure

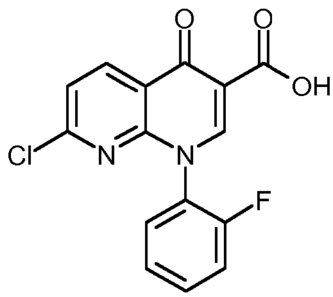

Zu einer Suspension von 1.52 g (4.38 mmol) der Verbindung aus Beispiel 3A in 21.7 ml THF wurden 8.8 ml wässr. Lithiumhydroxid-Lösung (1M, 8.8 mmol) gegeben und die Reaktionsmischung für 3h bei Raumtemperatur gerührt. Anschließend wurde mit 100 ml Wasser verdünnt und der pH-Wert mit 1M wässr. Salzsäure auf pH 1 eingestellt. Der Niederschlag wurde abgesaugt, mit Wasser gewaschen und über Nacht im Vakuumtrockenschrank bei 40°C getrocknet. Es wurden 1.22 g (86% d. Th., 99% Reinheit) der Titelverbindung erhalten.
$^1$H-NMR (400 MHz, DMSO-de): $\delta$ [ppm] = 14.1 (br. s, 1H), 9.02 (s, 1H), 8.80 (d, 1H), 7.81 (d, 1H), 7.79-7.74 (m, 1H), 7.72-7.66 (m, 1H), 7.58-7.52 (m, 1H), 7.50-7.44 (m, 1H).
LC-MS (Methode 3): $R_t$ = 1.66 min; 319 [M+H]$^+$.

**Beispiel 33B**

*rac*-1-(2-Fluorphenyl)-7-(3-hydroxy-2-oxopyrrolidin-1-yl)-4-oxo-1,4-dihydro-1,8-naphthyridin-3-carbonsäure

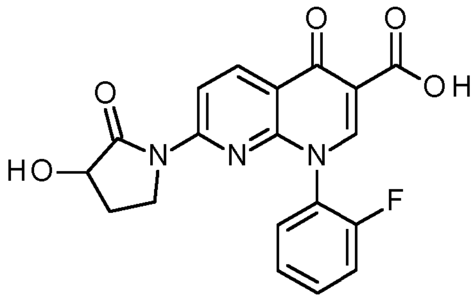

Gemäß AAV2 wurden 260 mg (816 μmol) der Verbindung aus Beispiel 33A mit 82.5 mg (816 μmol) 3-Hydroxypyrolidin-2-on (CAS: 15166-68-4) in Gegenwart von 282 mg (2.04 mmol) Kaliumcarbonat, 33.0 mg (147 μmol) Palladium(II)acetat und 170 mg (294 μmol) Xantphos in 8.24 ml 1,4-Dioxan bei 80°C umgesetzt. Das Reaktionsgemisch wurde auf 30 ml Wasser gegossen und mit 1N wässriger Salzsäure auf pH 1 eingestellt. Es wurde mit Dichlormethan extrahiert. Die vereinigten organischen Phasen wurden über Natriumsulfat getrocknet, filtriert und das Lösungsmittel unter reduziertem Druck entfernt. Das Rohprodukt wurde in zwei Läufen mittels präp. HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 161.5 mg (50% d. Th., 97.6% Reinheit) der Titelverbindung erhalten.
$^1$H-NMR (400 MHz, DMSO-de): δ [ppm] = 14.63 (s, 1H), 8.99 (s, 1H), 8.78 (d, 1H), 8.58 (dd, 1H), 7.81-7.73 (m, 1H), 7.72-7.65 (m, 1H), 7.58-7.50 (m, 1H), 7.49-7.42 (m, 1H), 5.92 (d, 1H), 4.46-4.32 (m, 1H), 3.60-3.45 (m, 1H), 3.34-3.20 (m, 1H, teilweise unter dem Wassersignal), 2.34-2.24 (m, 1H), 1.84-1.66 (m, 1H).
LC-MS (Methode 3): $R_t$ = 1.28 min; 384 $[M+H]^+$.

**Beispiel 34A**

7-Chlor-1-(2-chlorphenyl)-4-oxo-1,4-dihydro-1,8-naphthyridin-3-carbonsäure

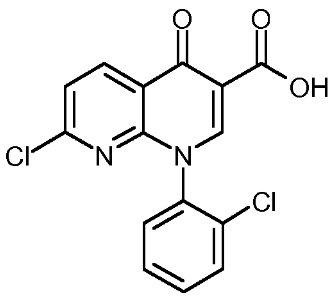

Zu einer Suspension von 3.70 g (10.2 mmol) der Verbindung aus Beispiel 4A in 50.5 ml THF wurden 20.4 ml wässrige Lithiumhydroxid-Lösung (1M, 20.4 mmol) gegeben und die Reaktionsmischung für 3h bei Raumtemperatur gerührt. Anschließend wurde mit 100 ml Wasser verdünnt und der pH-Wert mit 1N wässriger Salzsäure auf pH 1 eingestellt. Der Niederschlag wurde abgesaugt, mit Wasser gewaschen und über Nacht im Vakuumtrockenschrank bei 40°C getrocknet. Es wurden 3.18 g (92% d. Th., 99% Reinheit) der Titelverbindung erhalten.
$^1$H-NMR (400 MHz, DMSO-de): δ [ppm] = 14.1 (br. s, 1H), 8.92 (s, 1H), 8.79 (d, 1H), 7.83-7.74 (m, 3H), 7.70-7.59 (m, 2H).
LC-MS (Methode 3): $R_t$ = 1.79 min; 335 $[M+H]^+$.

**Beispiel 35A**

7-Chlor-1-(2,4-difluorphenyl)-4-oxo-1,4-dihydro-1,8-naphthyridin-3-carbonsäure

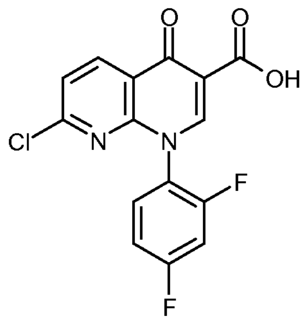

3 g (8.2 mmol) 7-Chlor-1-(2,4-difluorphenyl)-4-oxo-1,4-dihydro-1,8-naphthyridin-3-carbonsäureethylester (Darstellung beschrieben in DE 4301246, Beispiel U, S.26) in 60 ml THF wurden mit 16.5 ml (16.4 mmol) 1 M wässriger Lithiumhydroxid-Lösung versetzt und 2 h bei 23°C gerührt. Die Mischung wurde mit 120 ml Wasser verdünnt und anschließend mit konz. Salzsäure ein pH-Wert von 1 eingestellt. Der ausgefallene Feststoff wurde abgesaugt, mit Wasser gewaschen und im Hochvakuum getrocknet. Man erhielt 2.62 g (95 % d. Th.) der Titelverbindung.
LC-MS (Methode 1): $R_t$ = 0.93 min; m/z = 337.1 $[M+H]^+$.

## Beispiel 36A

1-(2,4-Difluorphenyl)-7-(dimethylamino)-4-oxo-1,4-dihydro-1,8-naphthyridin-3-carbonsäure

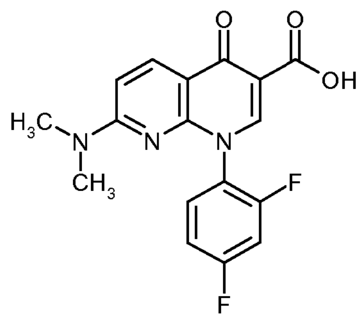

Methode A:

Eine Lösung von 1.9 g (5.1 mmol) der Verbindung aus Beispiel 6A in 24 ml 18 proz. wässriger Salzsäure wurde für 9 h bei 100°C gerührt. Anschliessend wurde der Ansatz abfiltriert, der Filterkuchen mit 0.5 M wässr. Salzsäure und Ethanol gewaschen und im Hochvakuum getrocknet. Man erhielt 1.58 g (89 % d. Th.) der Titelverbindung.

Methode B:

4.39 g (11.8 mmol) der Verbindung aus Beispiel 6A in 276 ml THF wurden mit 47 ml (47 mmol) 1 M wässriger Lithiumhydroxid-Lösung versetzt und 16 h bei 23°C gerührt. Nach 2.5 Tagen wurde durch Zugabe von 1 M wässriger Salzsäure ein pH-Wert 3 eingestellt. Nach Zugabe von dest. Wasser wurde der ausgefallene Feststoff abgesaugt, mit Wasser und Petrolether gewaschen und im Hochvakuum getrocknet. Man erhielt 4 g (99 % d. Th.) der Titelverbindung.
LC-MS (Methode 1): $R_t$ = 0.97 min; m/z = 346.2 $[M+H]^+$.

In Analogie zu Beispiel 36A wurden die in Tabelle 2A gezeigten Beispielverbindungen hergestellt, indem die entsprechenden Esterverbindungen aus den Beispielen 7A-31A mit 18 proz. wässriger Salzsäure oder wässriger 1 bis 2 M Lithiumhydroxid-Lösung unter den beschriebenen Reaktionsbedingungen umgesetzt wurden. Die Reaktionsdauer lag zwischen 2 h und 16 h. Abweichungen sind bei den jeweiligen Beispielen angegeben.

Tabelle 2A:

| Bsp. | IUPAC-Name / Struktur / (Ausbeute) | Analytische Daten |
|---|---|---|
| **37A** | 1-(2,4-Difluorphenyl)-7-(methylamino)-4-oxo-1,4-dihydro-1,8-naphthyridin-3-carbonsäure 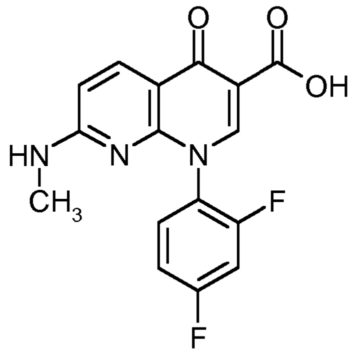 Methode A; (80 % d. Th.) | LC-MS (Methode 1): $R_t$ = 0.84 min MS (ESpos): m/z = 332.1 $[M+H]^+$ |
| **38A** | 1-(2,4-Difluorphenyl)-4-oxo-7-(propan-2-ylamino)-1,4-dihydro-1,8-naphthyridin-3-carbonsäure 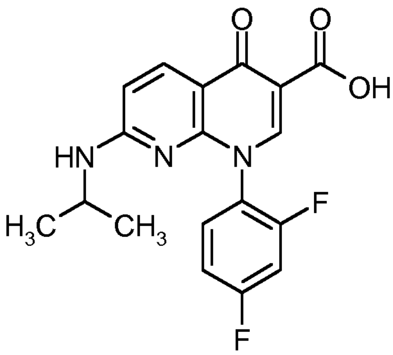 Methode B; (79 % d. Th.) | LC-MS (Methode 1): $R_t$ = 0.98 min MS (ESpos): m/z = 360.2 $[M+H]^+$ |
| **39A** | 1-(2,4-Difluorphenyl)-7-[(2-hydroxyethyl)-(methyl)amino]-4-oxo-1,4-dihydro-1,8-naphthyridin-3-carbonsäure 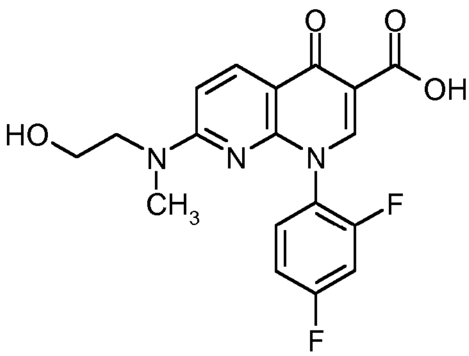 Methode B; (98 % d. Th.) | LC-MS (Methode 1): $R_t$ = 0.80 min MS (ESpos): m/z = 376.1 $[M+H]^+$ |
| **40A** | 1-(2,4-Difluorphenyl)-7-[(2-hydroxyethyl)-amino]-4-oxo-1,4-dihydro-1,8-naphthyridin-3-carbonsäure | LC-MS (Methode 1): $R_t$ = 1.34 min MS (ESpos): m/z = 362.0 $[M+H]^+$ |

(fortgesetzt)

| Bsp. | IUPAC-Name / Struktur / (Ausbeute) | Analytische Daten |
|---|---|---|
| | 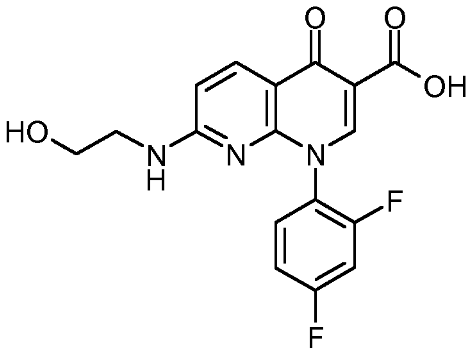 Methode B; (84 % d. Th.) | |
| **41A** | 1-(2,4-Difluorphenyl)-7-[(2-fluoroethyl)amino]-4-oxo-1,4-dihydro-1,8-naphthyridin-3-carbonsäure 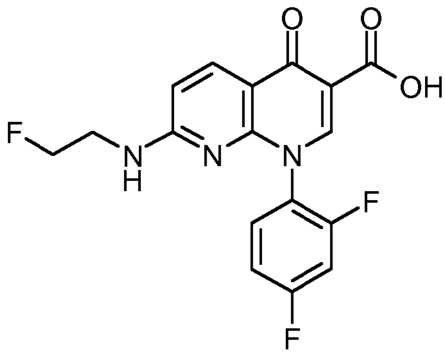 Methode B; (100 % d. Th.) | LC-MS (Methode 1): $R_t$ = 0.87 min MS (ESpos): m/z = 364.0 $[M+H]^+$ |
| **42A** | 1-(2,4-Difluorphenyl)-7-[(2-fluoroethyl)(methyl)-amino]-4-oxo-1,4-dihydro-1,8-naphthyridin-3-carbonsäure 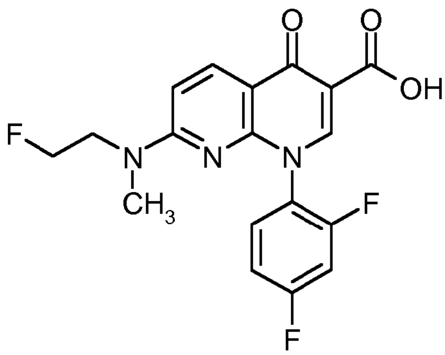 Methode B; (89 % d. Th.) | LC-MS (Methode 1): $R_t$ = 0.87 min MS (ESpos): m/z = 378.1 $[M+H]^+$ |
| **43A** | 1-(2,4-Difluorphenyl)-7-(3,3-difluorpyrrolidin-1-yl)-4-oxo-1,4-dihydro-1,8-naphthyridin-3-carbonsäure | LC-MS (Methode 1): $R_t$ = 1.01 min MS (ESpos): m/z = 408.1 $[M+H]^+$ |

(fortgesetzt)

| Bsp. | IUPAC-Name / Struktur / (Ausbeute) | Analytische Daten |
|---|---|---|
| | 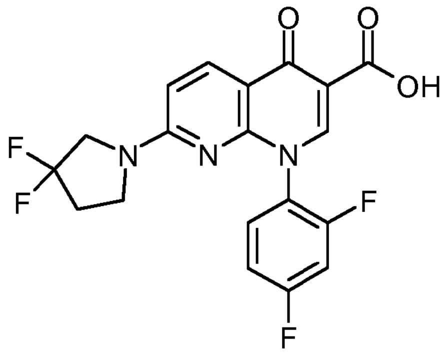 Methode B; (89 % d. Th.) | |
| 44A | 1-(2,4-Difluorphenyl)-7-[(3*R*)-3-fluorpyrrolidin-1-yl]-4-oxo-1,4-dihydro-1,8-naphthyridin-3-carbonsäure 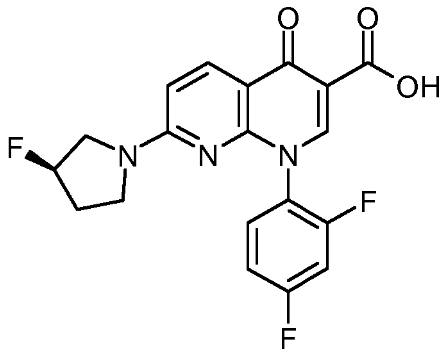 Methode B; (88 % d. Th.) | LC-MS (Methode 1): $R_t$ = 0.95 min MS (ESpos): m/z = 390.1 $[M+H]^+$ |
| **45A** | 1-(2,4-Difluorphenyl)-7-[(3*S*)-3-fluorpyrrolidin-1-yl]-4-oxo-1,4-dihydro-1,8-naphthyridin-3-carbonsäure 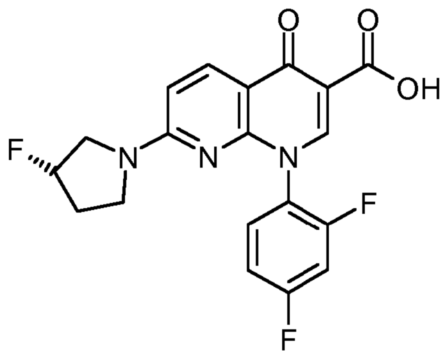 Methode B; (82 % d. Th.) | LC-MS (Methode 1): $R_t$ = 0.95 min MS (ESpos): m/z = 390.2 $[M+H]^+$ |
| **46A** | 1-(2,4-Difluorphenyl)-4-oxo-7-(3,3,4,4-tetrafluorpyrrolidin-1-yl)-1,4-dihydro-1,8-naphthyridin-3-carbonsäure | LC-MS (Methode 1): $R_t$ = 1.05 min MS (ESpos): m/z = 444.0 $[M+H]^+$ |

(fortgesetzt)

| Bsp. | IUPAC-Name / Struktur / (Ausbeute) | Analytische Daten |
|---|---|---|
| | 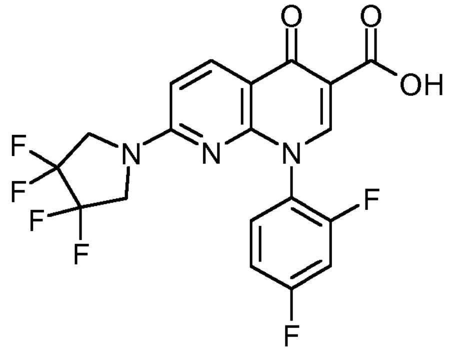 Methode B; (59 % d. Th.) | |
| **47A** | 1-(2,4-Difluorphenyl)-4-oxo-7-[(2,2,2-trifluorethyl)amino]-1,4-dihydro-1,8-naphthyridin-3-carbonsäure 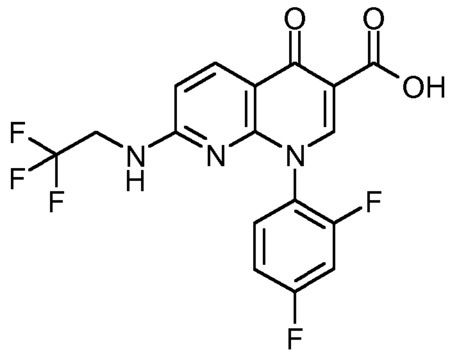 Methode B; (75 % d. Th.) | LC-MS (Methode 1): $R_t$ = 0.88 min MS (ESpos): m/z = 400.1 $[M+H]^+$ |
| **48A** | 7-[(2,2-Difluorethyl)(methyl)amino]-1-(2,4-Difluorphenyl)-4-oxo-1,4-dihydro-1,8-naphthyridin-3-carbonsäure 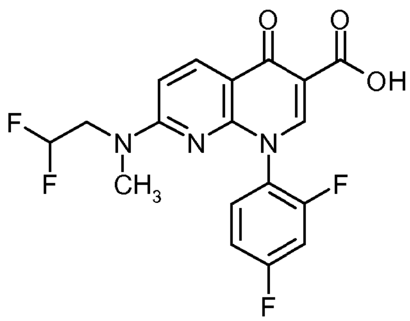 Methode B; (91 % d. Th.) | LC-MS (Methode 1): $R_t$ = 0.96 min MS (ESpos): m/z = 396.2 $[M+H]^+$ |
| **49A** | 7-[(2,2-Difluorethyl)amino]-1-(2,4-Difluorphenyl)-4-oxo-1,4-dihydro-1,8-naphthyridin-3-carbonsäure | LC-MS (Methode 1): $R_t$ = 0.88 min MS (ESpos): m/z = 382.2 $[M+H]^+$ |

(fortgesetzt)

| Bsp. | IUPAC-Name / Struktur / (Ausbeute) | Analytische Daten |
|---|---|---|
| | 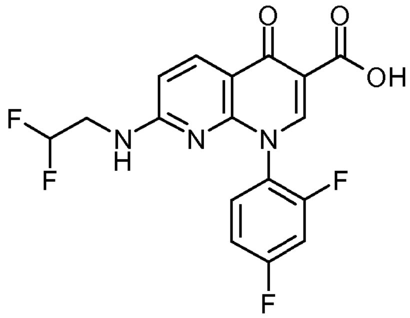<br>Methode B; (96 % d. Th.) | |
| **50A** | 1-(2,4-Difluorphenyl)-7-(1,1-dioxido-1,3-thiazolidin-3-yl)-4-oxo-1,4-dihydro-1,8-naphthyridin-3-carbonsäure<br>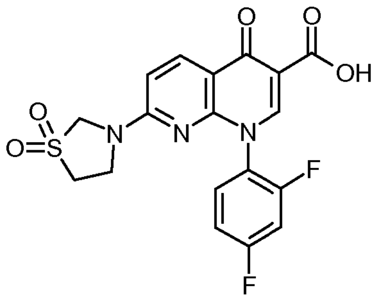<br>Methode B; (45 % d. Th.) | LC-MS (Methode 1): $R_t$ = 0.70 min MS (ESpos): m/z = 422.2 $[M+H]^+$ |
| **51A** | *rac*-1-(2,4-Difluorphenyl)-7-[2-(hydroxymethyl)-morpholin-4-yl]-4-oxo-1,4-dihydro-1,8-naphthyridin-3-carbonsäure<br>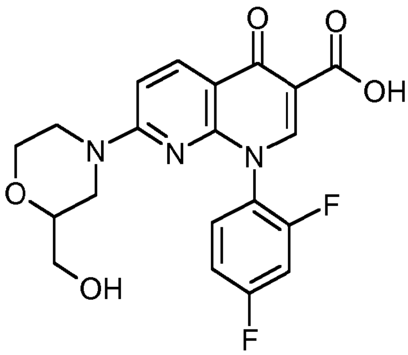<br>Methode B; (100 % d. Th.) | LC-MS (Methode 1): $R_t$ = 0.70 min MS (ESpos): m/z = 418.1 $[M+H]^+$ |
| **52A** | *rac*-1-(2,4-Difluorphenyl)-7-[2-(hydroxymethyl)-pyrrolidin-1--yl]-4-oxo-1,4-dihydro-1,8-naphthyridin-3-carbonsäure | LC-MS (Methode 1): $R_t$ = 0.85 min MS (ESpos): m/z = 402.2 $[M+H]^+$ |

(fortgesetzt)

| Bsp. | IUPAC-Name / Struktur / (Ausbeute) | Analytische Daten |
|---|---|---|
| | 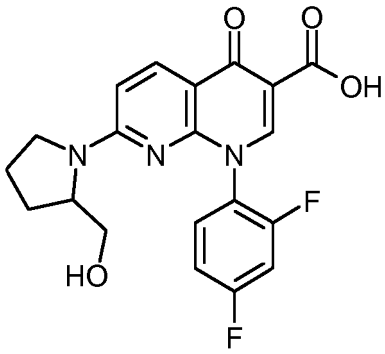 Methode B; (89 % d. Th.) | |
| **53A** | *rac*-1-(2,4-Difluorphenyl)-7-[3-(hydroxymethyl)-morpholin-4-yl]-4-oxo-1,4-dihydro-1,8-naphthyridin-3-carbonsäure 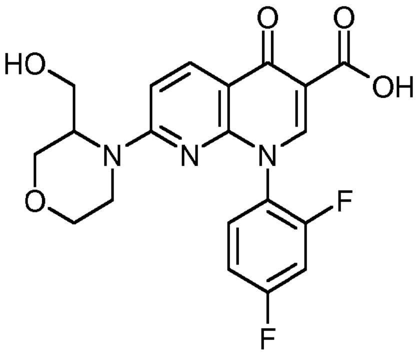 Methode B; (16 % d. Th.) | LC-MS (Methode 1): $R_t$ = 0.76 min MS (ESpos): m/z = 418. 2 $[M+H]^+$ |
| **54A** | *rac*-1-(2,4-Difluorphenyl)-7-(3-hydroxy-3- methylpiperidin-1-yl)-4-oxo-1,4-dihydro-1,8-naphthyridin-3-carbonsäure 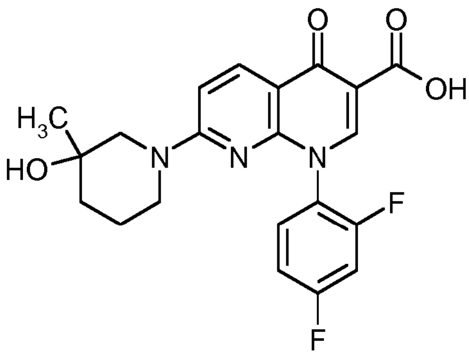 Methode B; (68 % d. Th.) | LC-MS (Methode 1): $R_t$ = 0.90 min MS (ESpos): m/z = 416.1 $[M+H]^+$ |
| **55A** | 1-(2,4-Difluorphenyl)-7-[methyl(2,2,2-trifluorethyl)amino]-4-oxo-1,4-dihydro-1,8-naphthyridin-3-carbonsäure | LC-MS (Methode 1): $R_t$ = 1.00 min MS (ESpos): m/z = 414.2 $[M+H]^+$ |

(fortgesetzt)

| Bsp. | IUPAC-Name / Struktur / (Ausbeute) | Analytische Daten |
|---|---|---|
| | 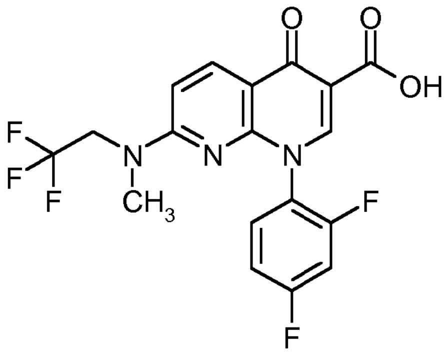 Methode B; (100 % d. Th.) | |
| **56A** | 1-(2,4-Difluorphenyl)-7-(morpholin-4-yl)-4-oxo-1,4-dihydro-1,8-naphthyridin-3-carbonsäure 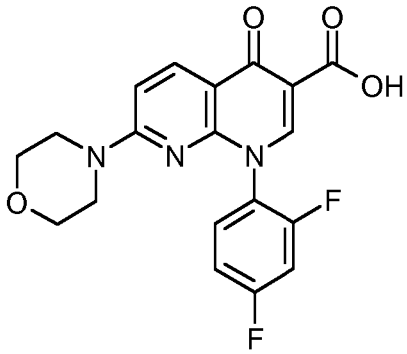 Methode A; (99 % d. Th.) | LC-MS (Methode 1): $R_t$ = 0.90 min MS (ESpos): m/z = 388.2 $[M+H]^+$ |
| **57A** | 1-(2,4-Difluorphenyl)-4-oxo-7-(pyrrolidin-1-yl)-1,4-dihydro-1,8-naphthyridin-3-carbonsäure 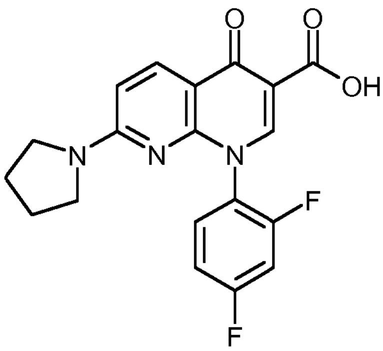 Methode A; (89 % d. Th.) | LC-MS (Methode 1): $R_t$ = 1.05 min MS (ESpos): m/z = 372.2 $[M+H]^+$ |
| **58A** | 1-(2,4-Difluorphenyl)-7-[4-hydroxy-4-(hydroxyl-methyl)piperidin-1-yl]-4-oxo-1,4-dihydro-1,8-naphthyridin-3-carbonsäure | |

(fortgesetzt)

| Bsp. | IUPAC-Name / Struktur / (Ausbeute) | Analytische Daten |
|---|---|---|
| | 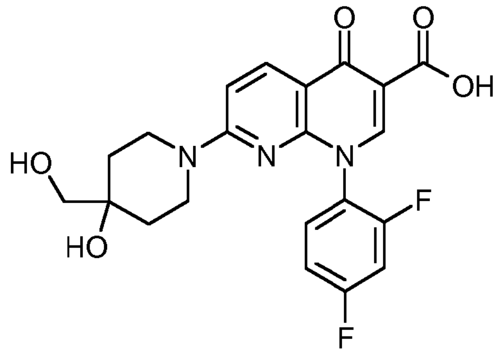 Methode B; (38 % d. Th.) | |
| **59A** | 1-(2,4-Difluorphenyl)-4-oxo-7-(1,3-thiazolidin-3-yl)-1,4-dihydro-1,8-naphthyridin-3-carbonsäure 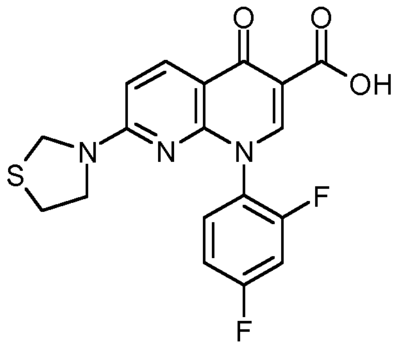 Methode B; (67 % d. Th.) | LC-MS (Methode 3): $R_t$ = 1.84 min MS (ESpos): m/z = 390.0 $[M+H]^+$ |
| **60A** | 1-(2-Chlor-4-fluorphenyl)-7-(dimethylamino)-4-oxo-1,4-dihydro-1,8-naphthyridin-3-carbonsäure 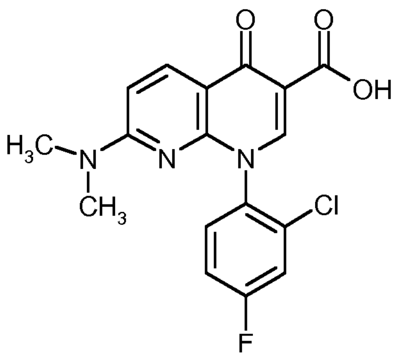 Methode A; (82 % d. Th.) | LC-MS (Methode 1): $R_t$ = 2.16 min MS (ESpos): m/z = 362.1 $[M+H]^+$ |

**Beispiel 61A**

1-(2,4-Difluorphenyl)-4-oxo-7-(2-oxo-1,3-oxazolidin-3-yl)-1,4-dihydro-1,8-naphthyridin-3-carbonsäure

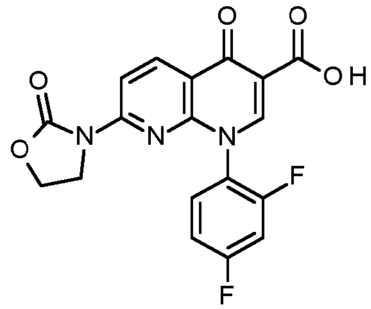

Eine Mischung aus 300 mg (0.67 mmol) 7-Chlor-1-(2,4-difluorphenyl)-4-oxo-1,4-dihydro-1,8-naphthyridin-3-carbonsäureethylester (Darstellung beschrieben in DE 4301246, Beispiel U, S.26), 232 mg (2.7 mmol) 2-Oxazolidinon, 184 mg (1.3 mmol) Kaliumcarbonat, 129 mg (0.68 mmol) Kupfer(I)iodid und 51 mg (0.69 mmol) trans-*N,N'*-Dimethyl-1,2-cyclohexandiamin in 7.5 ml DMF wurde 3 h bei 110°C und anschließend für weitere 13 h bei 23°C gerührt. Anschliessend wurde 127 mg (0.67 mmol) Kupfer(I)iodid und 48 mg (0.66 mmol) trans-*N,N'*-Dimethyl-1,2-cyclohexandiamin zugegeben und weitere 10 h bei 130°C gerührt, das Gemisch filtriert und das Filtrat über präparative HPLC gereinigt (Eluent: Acetonitril/Wasser-Gradient mit 0.1% Ameisensäure). Man erhielt 28 mg (6 % d. Th., Reinheit 59% (HPLC)) der Titelverbindung, die ohne weitere Aufreinigung für die nächste Stufe eingesetzt wurde.
LC-MS (Methode 1): Rt = 2.38 min; m/z = 388.0 $[M+H]^+$.

**Beispiel 62A**

7-(Dimethylamino)-1-(2-fluorphenyl)-4-oxo-1,4-dihydro-1,8-naphthyridin-3-carbonsäure

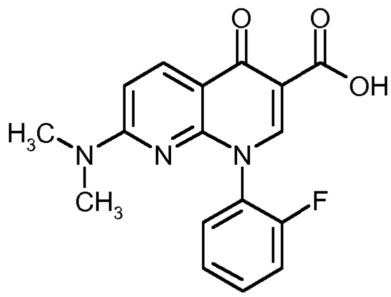

Zu einer Suspension von 1.33 g (3.74 mmol) der Verbindung aus Beispiel 32A in 18.5 ml THF wurden 7.5 ml wässr. Lithiumhydroxid-Lösung (1M, 7.5 mmol) gegeben und die Reaktionsmischung für 3h bei Raumtemperatur gerührt. Anschließend wurde mit 100 ml Wasser verdünnt und der pH-Wert mit 1M wässr. Salzsäure auf pH 1 eingestellt. Der Niederschlag wurde abgesaugt, mit Wasser gewaschen und über Nacht im Vakuumtrockenschrank bei 40°C getrocknet. Es wurden 1.13 g (91 % d. Th., 99% Reinheit) der Titelverbindung erhalten.
$^1$H-NMR (400 MHz, DMSO-d6): δ [ppm] = 15.4 (br. s, 1H), 8.73 (s, 1H), 8.32 (d, 1H), 7.75-7.70 (m, 1H), 7.67-7.61 (m, 1H), 7.53-7.46 (m, 1H), 7.45-7.40 (m, 1H), 7.02 (d, 1H), 2.95 (br. s, 6H).
LC-MS (Methode 3): $R_t$ = 1.62 min; 328 $[M+H]^+$.

**Beispiel 63A**

1-(2,4-Difluorphenyl)-7-[(4*S*)-4-hydroxy-2-oxopyrrolidin-1-yl]-4-oxo-1,4-dihydro-1,8-naphthyridin-3-carbonsäure

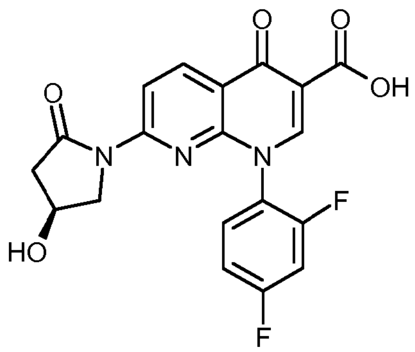

Gemäß AAV2 wurden 2.50 g (7.43 mmol) der Verbindung aus Beispiel 35A mit 750 mg (7.43 mmol) (4*S*)-4-Hydroxypyrrolidin-2-on in Gegenwart von 4.84 g (14.9 mmol) Caesiumcarbonat, 300 mg (1.34 mmol) Palladium(II)acetat und 773 mg (1.34 mmol) Xantphos in 75 ml Dioxan bei 80°C umgesetzt. Das Reaktionsgemisch wurde auf Raumtemperatur abgekühlt und auf 300 ml Wasser gegossen. Es wurde mit 1N wässriger Salzsäure der pH-Wert auf 1 eingestellt und der Niederschlag abgesaugt, mit n-Hexan nachgewaschen und am Hochvakuum getrocknet. Das Rohprodukt wurde mittels Flashchromatographie (Dichlormethan/Methanol-Gradient) gereinigt und 292 mg (6.4% d. Th.; 65% Reinheit) der Titelverbindung erhalten.
LC-MS (Methode 1): $R_t$ = 0.73 min; 402 $[M+H]^+$.

**Beispiel 64A**

1-(2-Chlorphenyl)-7-[(4*S*)-4-hydroxy-2-oxopyrrolidin-1-yl]-4-oxo-1,4-dihydro-1,8-naphthyridin-3-carbonsäure

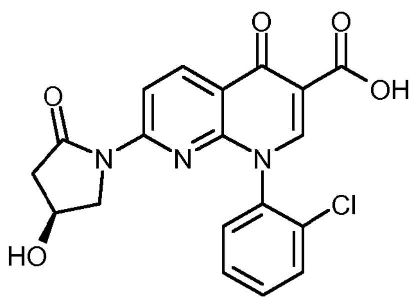

Gemäß AAV2 wurden 500 mg (1.49 mmol) der Verbindung aus Beispiel 34A mit 150 mg (1.49 mmol) (4*S*)-4-Hydroxypyrrolidin-2-on in Gegenwart von 515 mg (3.73 mmol) Kaliumcarbonat, 60.3 mg (269 μmol) Palladium(II)acetat und 311 mg (537 μmol) Xantphos in 15 ml Dioxan bei 90°C umgesetzt. Das Rohprodukt wurde mittels Flashchromatographie (Dichlormethan/Methanol-Gradient) und präparativer HPLC (Säule: Kromasil C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 67.4 mg (11% d. Th.; 99% Reinheit) der Titelverbindung erhalten (als Atropisomerengemisch).
$^1$H-NMR (400 MHz, DMSO-de): δ [ppm] = 14.66 (br. s, 1H), 8.92 (s, 1H), 8.76 (d, 1H), 8.57 (dd, 1H), 7.82-7.75 (m, 2H), 7.70-7.59 (m, 2H), 5.32 (dd, 1H), 4.27-4.20 (m, 1H), 3.60-3.52 (m, 1H), 3.40-3.33 (m, 1H), 2.99-2.88 (m, 1H), 2.40-2.32 (m, 1H).
LC-MS (Methode 3): $R_t$ = 1.30/1.36 min; 400 $[M+H]^+$.

**Beispiel 65A**

7-Chlor-1-(2,4-difluorphenyl)-4-oxo-*N*-(tricyclo[3.3.1.1$^{3,7}$]dec-1-yl)-1,4-dihydro-1,8-naphthyridin-3-carbonsäureamid

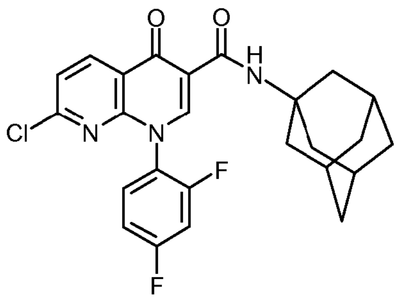

Zu 90 mg (0.27 mmol) der Verbindung aus Beispiel 35A und 68 mg (0.67 mmol) *N*-Methylmorpholin in 3.3 ml DMF wurden bei 0°C 0.54 ml (0.54 mmol) Chlorameisensäureisopropylester (1 M in Toluol) gegeben und dann 1 h bei 0°C gerührt. Dann wurden bei 0°C 53 mg (0.35 mmol) 1-Adamantanamin zugegeben und 2 h bei 20°C gerührt. Das Gemisch wurde über präparative HPLC gereinigt (Eluent: Acetonitril/Wasser-Gradient mit 0.1% Ameisensäure). Man erhielt 48 mg (36 % d. Th.) der Titelverbindung.
LC-MS (Methode 1): $R_t$ = 1.46 min; m/z = 470.2 $[M+H]^+$.

In Analogie zu Beispiel 65A wurden die in Tabelle 3A gezeigten Beispielverbindungen hergestellt, indem die Verbindung aus Beispiel 35A mit den entsprechenden Aminen (bzw. deren Salzen) unter den beschriebenen Reaktionsbedingungen umgesetzt wurden. Abweichungen sind bei den jeweiligen Beispielen angegeben.

Beispielhafte Aufarbeitung der Reaktionsmischung:

Das Reaktionsgemisch wurde anschließend auf Wasser gegeben und mit 1 M wässr. Salzsäure auf pH 1 gestellt. Das Lösungsmittel (Toluol) wurde im Vakuum entfernt und der entstandene Niederschlag abfiltriert, im Vakuum getrocknet. Die Reinigung erfolgte beispielhaft durch Säulenchromatographie (Kieselgel, Cyclohexan → Cyclohexan/Ethylacetat 10:1) oder präparative Dünnschichtchromatographie (Kieselgel, DCM).

Tabelle 3A:

| Bsp. | IUPAC-Name / Struktur / (Ausbeute) | Analytische Daten |
|---|---|---|
| **66A** | *rac*-7-Chlor-1-(2,4-difluorphenyl)-4-oxo-*N*-(1,1,1-trifluorbutan-2-yl)-1,4-dihydro-1,8-naphthyridin-3-carbonsäureamid 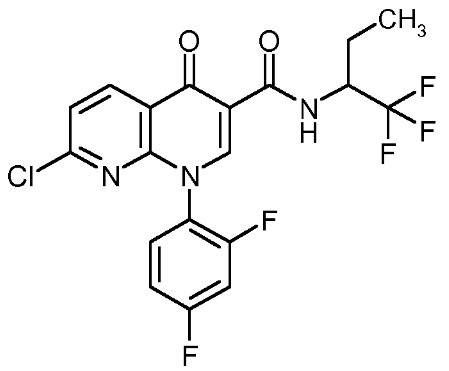 Lösungsmittel: NMP (62 % d. Th.) | LC-MS (Methode 1): $R_t$ = 1.21 min MS (ESpos): m/z = 446.2 $[M+H]^+$ |
| **67A** | 7-Chlor-1-(2,4-difluorphenyl)-4-oxo-N-[(2R)-1,1,1-trifluorbutan-2-yl]-1,4-dihydro-1,8-naphthyridin-3-carbonsäureamid | LC-MS (Methode 1): $R_t$ = 1.16 min MS (ESpos): m/z = 446.1 $[M+H]^+$ |

(fortgesetzt)

| Bsp. | IUPAC-Name / Struktur / (Ausbeute) | Analytische Daten |
|---|---|---|
| | 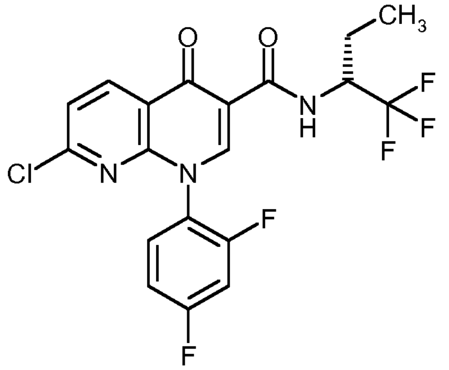 Aufarbeitung: Die Mischung wurde auf Wasser gegeben und mit wässr. 1 M Salzsäure auf pH 1 gestellt. Dann wurde das Toluol im Vakuum entfernt, der Niederschlag abfiltriert, im Vakuum getrocknet und über Kieselgelchromatographie gereinigt (Cyclohexan → Cyclohexan/Ethylacetat 10:1) . (59 % d. Th.) | |
| **68A** | 7-Chlor-1-(2,4-difluorphenyl)-4-oxo-*N*-[(2*S*)-1,1,1-trifluorbutan-2-yl]-1,4-dihydro-1,8-naphthyridin-3-carbonsäureamid 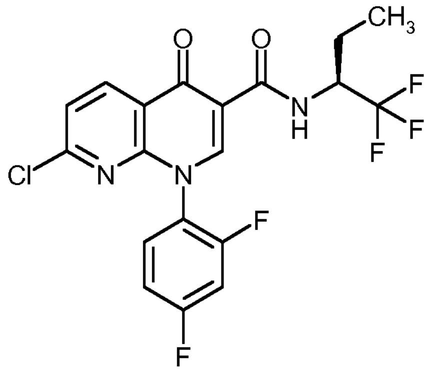 Aufarbeitung: Die Mischung wurde auf Wasser gegeben und mit wässr. 1 M Salzsäure auf pH 1 gestellt. Dann wurde das Toluol im Vakuum entfernt, der Niederschlag abfiltriert, im Vakuum getrocknet und über Kieselgelchromatographie gereinigt (Cyclohexan → Cyclohexan/Ethylacetat 10:1). (70 % d. Th.) | LC-MS (Methode 1): $R_t$ = 1.20 min MS (ESpos): m/z = 446.2 $[M+H]^+$ |
| **69A** | *rac*-7-Chlor-1-(2,4-difluorphenyl)-4-oxo-*N*-(1,1,1-trifluorpropan-2-yl)-1,4-dihydro-1,8-naphthyridin-3 -carbonsäureamid 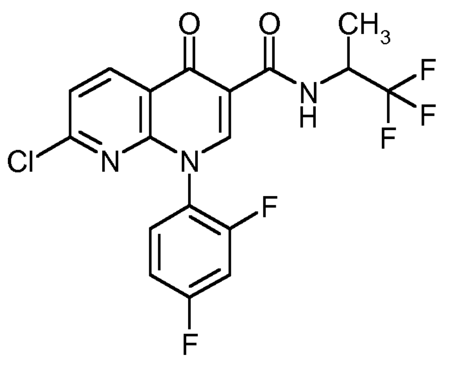 (76 % d. Th.) | LC-MS (Methode 1): $R_t$ = 1.16 min MS (ESpos): m/z = 432.0 $[M+H]^+$ |

(fortgesetzt)

| Bsp. | IUPAC-Name / Struktur / (Ausbeute) | Analytische Daten |
|---|---|---|
| **70A** | 7-Chlor-1-(2,4-difluorphenyl)-*N*-(4-methylbicyclo-[2.2.2]oct-1-yl)-4-oxo-1,4-dihydro-1,8-naphthyridin-3-carbonsäureamid 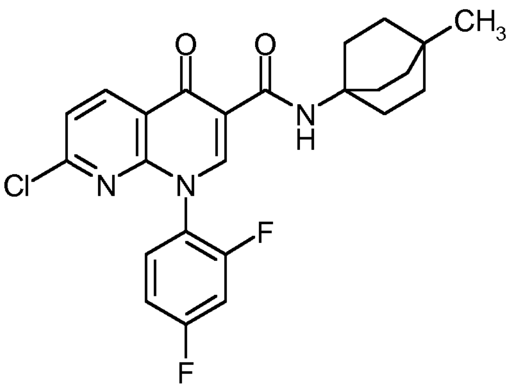 Aufreinigung durch präparative Dünnschichtchromatographie (Kieselgel, DCM) (23 % d. Th.) | LC-MS (Methode 1): $R_t$ = 1.39 min MS (ESpos): m/z = 458.3 $[M+H]^+$ |
| **71A** | 7-Chlor-1-(2,4-difluorphenyl)-*N*-[2-(2,6-difluorphenyl)propan-2-yl]-4-oxo-1,4-dihydro-1,8-naphthyridin-3-carbonsäureamid 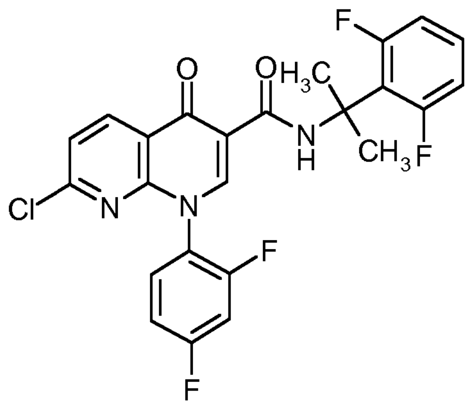 Aufreinigung über präparative Dünnschichtchromatographie (Kieselgel, DCM) (22 % d. Th.) | LC-MS (Methode 1): $R_t$ = 1.26 min MS (ESpos): m/z = 490.3 $[M+H]^+$ |
| **72A** | 7-Chlor-*N*-(2,6-dichlorbenzyl)-1-(2,4-difluorphenyl)-4-oxo-1,4-dihydro-1,8-naphthyridin-3-carbonsäureamid | LC-MS (Methode 1): $R_t$ = 1.24 min MS (ESpos): m/z = 494.1 $[M+H]^+$ |

(fortgesetzt)

| Bsp. | IUPAC-Name / Struktur / (Ausbeute) | Analytische Daten |
|---|---|---|
| | 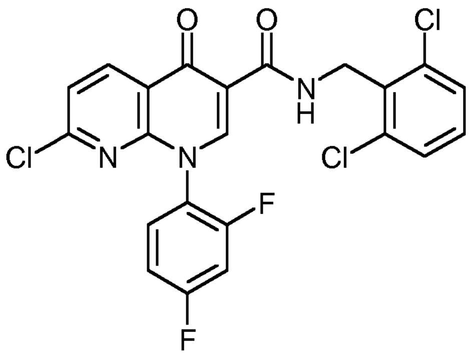 (88 % d. Th.) | |

**Beispiel 73A**

*rac*-7-Chlor-*N*-[1-(2-chlorphenyl)-2,2,2-trifluorethyl] -1 -(2,4-difluorphenyl)-4-oxo-1,4-dihydro-1,8-naphthyridin-3 -carbonsäureamid

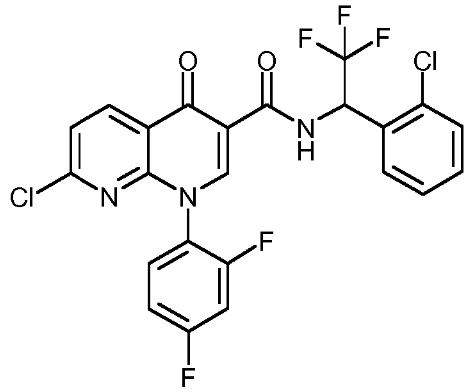

Gemäß AAV1 wurden 1.50 g (4.46 mmol) der Verbindung aus Beispiel 35A mit 1.40 g (6.68 mmol) *rac*-1-(2-Chlorphenyl)-2,2,2-trifluorethanamin in Gegenwart von 1.69 g (4.46 mmol) HATU und 1.09 ml (6.24 mmol) *N*,*N*-Diisopropylethylamin in 45 ml Dimethylformamid umgesetzt. Das Rohprodukt wurde mittels Flashchromatographie (Cyclohexan/Essigsäureethylester-Gradient) gereinigt und 1.73 g (71% d. Th., 96% Reinheit) der Titelverbindung erhalten.
$^1$H-NMR (400 MHz, DMSO-de): δ [ppm] = 11.05 (d, 1H), 8.92 (s, 1H), 8.79 (d, 1H), 7.91-7.75 (m, 1H), 7.77 (d, 1H), 7.68-7.48 (m, 5H), 7.41-7.33 (m, 1H), 6.53-6.42 (m, 1H).
LC-MS (Methode 1): $R_t$ = 1.30 min; 528 [M+H]$^+$.

**Beispiel 74A**

*rac*-7-Chlor-1-(2,4-difluorphenyl)-4-oxo-*N*-[1-(trifluormethoxy)propan-2-yl]-1,4-dihydro-1,8-naphthyridin-3-carbonsäureamid

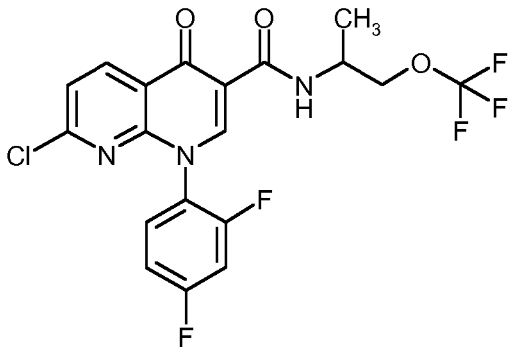

Gemäß AAV1 wurden 2.50 g (7.43 mmol) der Verbindung aus Beispiel 35A mit 1.28 g (6.68 mmol) *rac*-1-(Trifluormethoxy)propan-2-amin in Gegenwart von 3.11g (8.17 mmol) HATU und 1.29ml (7.43 mmol) *N*,*N*-Diisopropylethylamin in 90 ml Dimethylformamid umgesetzt. Nach Reaktionskontrolle über Nacht wurde weitere 1.55 g (4.08 mmol) HATU und 647 µl (3.71 mmol) *N*,*N*-Diisopropylethylamin hinzugegeben und über Nacht bei Raumtemperatur gerührt. Das Rohprodukt wurde mittels Flashchromatographie (Cyclohexan/Essigsäureethylester-Gradient) gereinigt und 2.38 g (69% d. Th., 99% Reinheit) der Titelverbindung erhalten.
$^1$H-NMR (400 MHz, DMSO-$d_6$): δ [ppm] = 9.72 (d, 1H), 8.83 (s, 1H), 8.74 (d, 1H), 7.90-7.82 (m, 1H), 7.73 (d, 1H), 7.67-7.60 (m, 1H), 7.41-7.34 (m,1H), 4.42-4.33 (m, 1H), 4.24-4.15 (m, 2H), 1.26 (d, 3H).
LC-MS (Methode 3): $R_t$ = 2.18 min; 462 $[M+H]^+$.

## Beispiel 75A

7-Chlor-1-(2,4-difluorphenyl)-4-oxo-*N*-[1-phenyl-2-(trifluortnethoxy)ethyl]-1,4-dihydro-1,8-naphthyridin-3 -carbonsäureamid

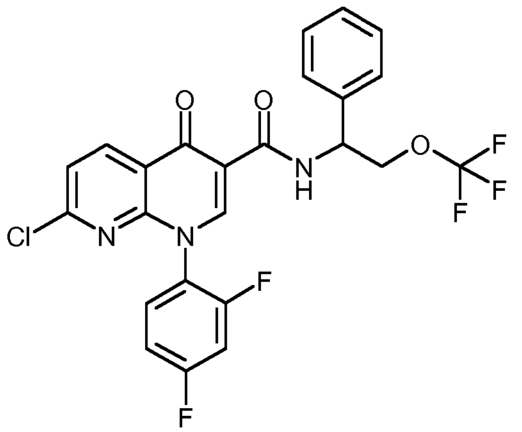

Gemäß AAV1 wurden 1.1g (3.3 mmol) der Verbindung aus Beispiel 35A mit 1.22 g (4.90 mmol) (-)-1-Phenyl-2-(trifluormethoxy)ethanamin Hydrochlorid (97% Reinheit, Drehwert: -21.13° in Methanol c = 0.5300g/100ml, 589nm, 20°C) in Gegenwart von 1.24 g (3.27 mmol) HATU und 1.14 ml (6.53 mmol) *N*,*N*-Diisopropylethylamin in 33 ml Dimethylformamid umgesetzt. Das Rohprodukt wurde mittels Flashchromatographie (Cyclohexan/Essigsäureethylester-Gradient) gereinigt und 880 mg (49% d. Th., 95% Reinheit) der Titelverbindung (nicht-racemisches Gemisch) erhalten.
$^1$H-NMR (400 MHz, DMSO-$d_6$): δ [ppm] = 10.37 (d, 1H), 8.84 (s, 1H), 8.78 (d, 1H), 7.91-7.77 (m, 1H), 7.74 (d, 1H), 7.67-7.59 (m, 1H), 7.51-7.29 (m, 6H), 5.56-5.48 (m, 1H), 4.55-4.41 (m, 2H).
LC-MS (Methode 3): $R_t$ = 2.36 min; 524 $[M+H]^+$.

**Beispiel 76A**

*rac*-7-Chlor-1-(2,4-difluorphenyl)-4-oxo-*N*-[1-(trifluormethoxy)butan-2-yl]-1,4-dihydro-1,8-naphthyridin-3-carbonsäureamid (Racemat)

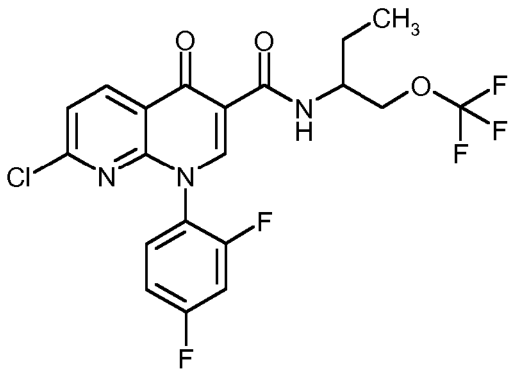

Gemäß AAV1 wurden 100 mg (285 μmol) der Verbindung aus Beispiel 35A (96%-ige Reinheit) mit 82.8 mg (428 μmol) *rac*-1-(Trifluormethoxy)butan-2-amin Hydrochlorid in Gegenwart von 108 mg (285 μmol) HATU und 119 μl (684 μmol) *N*,*N*-Diisopropylethylamin in 3 ml Dimethylformamid umgesetzt. Das Rohprodukt wurde mittels Flashchromatographie (Cyclohexan/Essigsäureethylester-Gradient) gereinigt und 101 mg (75% d. Th., 100% Reinheit) der Titelverbindung erhalten.
$^1$H-NMR (400 MHz, DMSO-$d_6$): δ [ppm] = 9.69 (d, 1H), 8.83 (s, 1H), 8.74 (d, 1H), 7.90-7.83 (m, 1H), 7.73 (d, 1H), 7.67-7.60 (m, 1H), 7.41-7.34 (m, 1H), 4.27-4.13 (m, 3H), 1.76-1.52 (m, 2H), 0.94 (t, 3H).
LC-MS (Methode 1): $R_t$ = 1.25 min; 476 $[M+H]^+$.

**Beispiel 77A**

*rac*-7-Chlor-1-(2,4-difluorphenyl)-*N*-[1-(2-fluorphenyl)ethyl]-4-oxo-1,4-dihydro-1,8-naphthyridin-3-carbonsäureamid

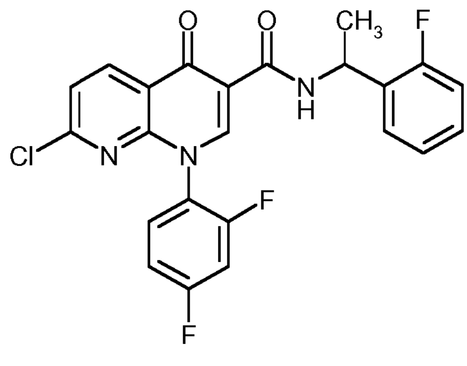

Gemäß AAV1 wurden 100 mg (285 μmol) der Verbindung aus Beispiel 35A (96%-ige Reinheit) mit 59.5 mg (428 μmol) *rac*-1-(2-Fluorphenyl)ethylamin in Gegenwart von 108 mg (285 μmol) HATU und 70 μl (0.40 mmol) *N*,*N*-Diisopropylethylamin in 3 ml Dimethylformamid umgesetzt. Das Rohprodukt wurde mittels Flashchromatographie (Cyclohexan/Essigsäureethylester-Gradient) gereinigt und 97.3 mg (75% d. Th., 100% Reinheit) der Titelverbindung erhalten.
$^1$H-NMR (400 MHz, DMSO-$d_6$): δ [ppm] = 10.12 (d, 1H), 8.79 (s, 1H), 8.75 (d, 1H), 7.89-7.78 (m, 1H), 7.74 (d, 1H), 7.67-7.58 (m, 1H), 7.49-7.41 (m, 1H), 7.40-7.29 (m, 2H), 7.24-7.17 (m, 2H), 5.44-5.34 (m, 1H), 1.52 (d, 3H).
LC-MS (Methode 1): $R_t$ = 1.23 min; 458 $[M+H]^+$.

**Beispiel 78A**

*rac*-7-Chlor-1-(2,4-difluorphenyl)-4-oxo-*N*-(1,1,1-trifluor-3-methoxy-2-methylpropan-2-yl)-1,4-dihydro-1,8-naphthyridin-3-carbonsäureamid

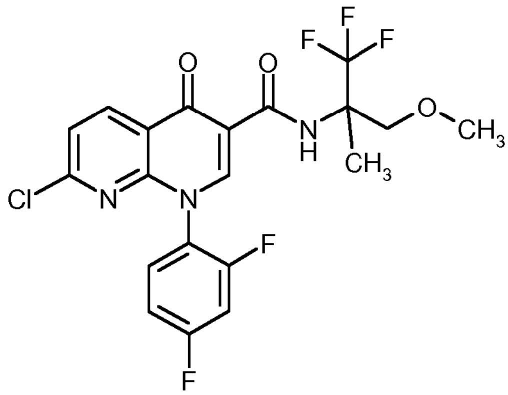

Gemäß AAV 1 wurden 100 mg (285 µmol) der Verbindung aus Beispiel 35A (96%-ige Reinheit) mit 87.2 mg (428 µmol) *rac*-1,1,1-Trifluor-3-methoxy-2-methylpropan-2-amin Hydrochlorid in Gegenwart von 108 mg (285 µmol) HATU und 119 µl (684 µmol) *N*,*N*-Diisopropylethylamin in 3 ml Dimethylformamid umgesetzt. Das Rohprodukt wurde mittels Flashchromatographie (Cyclohexan/Essigsäureethylester-Gradient) gereinigt und 112 mg (82% d. Th., 100% Reinheit) der Titelverbindung erhalten.
$^1$H-NMR (400 MHz, DMSO-$d_6$): δ [ppm] = 10.28 (br. s, 1H), 8.84 (s, 1H), 8.75 (d, 1H), 7.90-7.82 (m, 1H), 7.74 (d, 1H), 7.67-7.60 (m, 1H), 7.41-7.34 (m, 1H), 3.90-3.84 (m, 1H), 3.77-3.67 (m, 1H), 3.36 (s, 3H), 1.64 (s, 3H).
LC-MS (Methode 1): $R_t$ = 1.20 min; 476 $[M+H]^+$.

**Beispiel 79A**

7-Chlor-1-(2,4-difluorphenyl)-4-oxo-*N*-[4-(trifluormethyl)tetrahydro-2*H*-pyran-4-yl]-1,4-dihydro-1,8-naphthyridin-3-carbonsäureamid

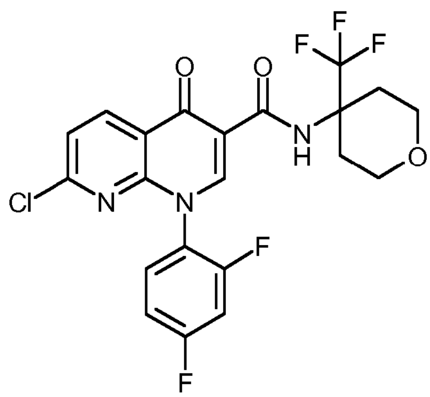

Gemäß AAV1wurden 100 mg (285 µmol) der Verbindung aus Beispiel 35A (96%-ige Reinheit) mit 87.9 mg (428 µmol) 4-(Trifluormethyl)tetrahydro-2*H*-pyran-4-amin Hydrochlorid in Gegenwart von 108 mg (285 µmol) HATU und 119 µl (684 µmol) *N*,*N*-Diisopropylethylamin in 3 ml Dimethylformamid umgesetzt. Das Rohprodukt wurde mittels Flaschromatographie (Cyclohexan/Essigsäureethylester-Gradient) gereinigt und 108 mg (77% d. Th., 100% Reinheit) der Titelverbindung erhalten.
$^1$H-NMR (400 MHz, DMSO-$d_6$): δ [ppm] = 10.08 (s, 1H), 8.87 (s, 1H), 8.76 (d, 1H), 7.90-7.83 (m, 1H), 7.76 (d, 1H), 7.68-7.61 (m, 1H), 7.41-7.34 (m, 1H), 3.95-3.85 (m, 2H), 3.57-3.45 (m, 2H), 2.47-2.39 (m, 1H), 1.95-1.83 (m, 2H).
LC-MS (Methode 1): $R_t$ = 1.17 min; 488 $[M+H]^+$.

**Beispiel 80A**

*rac*-7-Chlor-1-(2,4-difluorphenyl)-*N*-[1-(2,6-difluorphenyl)-2,2,2-trifluorethyl]-4-oxo-1,4-dihydro-1,8-naphthyridin-3 -carbonsäureamid

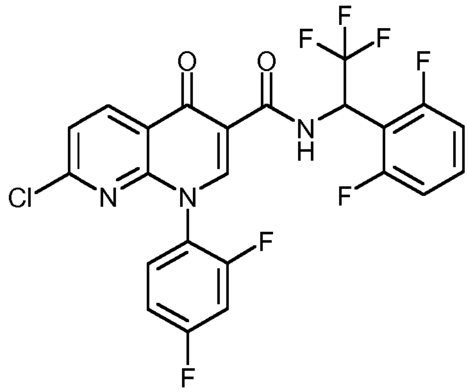

Gemäß AAV1 wurden 3.00 g (8.91 mmol) der Verbindung aus Beispiel 35A mit 1.96 g (9.27 mmol) 1-rac-(2,6-Difluorphenyl)-2,2,2-trifluorethanamin in Gegenwart von 3.39 g (8.91 mmol) HATU und 2.17 ml (12.5 mmol) *N*,*N*-Diisopropylethylamin in 90 ml Dimethylformamid umgesetzt. Das Rohprodukt wurde mittels Flashchromatographie (Cyclohexan/Essigsäureethylester-Gemisch, 5:1) gereinigt und 2.10 g (44% d. Th., 100% Reinheit) der Titelverbindung erhalten.
$^1H$ NMR (400 MHz, DMSO-$d_6$) $\delta$ [ppm] = 11.14 (d, 1H), 8.93 (s, 1H), 8.79 (d, 1H), 7.93-7.75 (m, 1H), 7.76 (d, 1H), 7.68-7.59 (m, 2H), 7.42-7.27 (m, 3H), 6.50-6.38 (m, 1H).
LC-MS (Methode 1): $R_t$ = 1.29 min; 530.1 $[M+H]^+$.

**Beispiel 81A**

7-Chlor-*N*-(2,6-dichlorphenyl)-1-(2,4-difluorphenyl)-4-oxo-1,4-dihydro-1,8-naphthyridin-3-carbonsäureamid

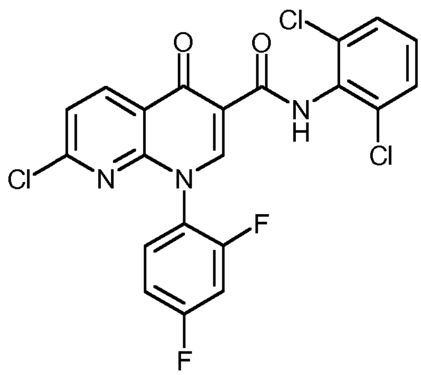

Zu einer Suspension von 1.00 g (2.97 mmol) der Verbindung aus Beispiel 35A in 10 ml THF wurden 0.29 ml (3.3 mmol) Oxalsäuredichlorid und katalytische Mengen Dimethylformamid gegeben. Nach Beendigung der Gasentwicklung wurde die Reaktionsmischung für 1h bei 60°C erhitzt und anschließend auf RT abgekühlt. Alle flüchtigen Komponenten wurden im Vakuum entfernt und der Rückstand in 20 ml DMF aufgenommen. Parallel dazu wurden 481 mg (2.97 mmol) 2,6-Dichloranilin in 10 ml DMF gelöst und mit 119 mg (2.97 mmol) Natriumhydrid (60% in Mineralöl) versetzt. Es wurde 30 min bei RT nachgerührt. Anschließend wurde die obige Lösung zügig hinzugegeben und die Reaktionsmischung über Nacht bei RT gerührt. Die Reaktion wurde durch Zugabe von Wasser und Essigsäureethylester beendet und die Phasen getrennt. Die organische Phase wurde zweimal mit Wasser und mit gesättigter wässriger Natriumchlorid-Lösung gewaschen, über Magnesiumsulfat getrocknet, filtriert und das Lösungsmittel unter reduziertem Druck entfernt. Das Rohprodukt wurde in wenig DCM aufgenommen und mittels Flashchromatographie (Cyclohexan/Essigsäureethylester-Gradient) gereinigt. Es wurden 298 mg (18% d. Th., 88% Reinheit) der Titelverbindung erhalten.
LC-MS (Methode 3): $R_t$ = 2.30 min; 480 [M+H]+.

**Beispiel 82A**

7-Chlor-1-(2-fluorphenyl)-4-oxo-*N*-[(2*R*)-1,1,1-trifluorbutan-2-yl]-1,4-dihydro-1,8-naphthyridin-3-carbonsäureamid

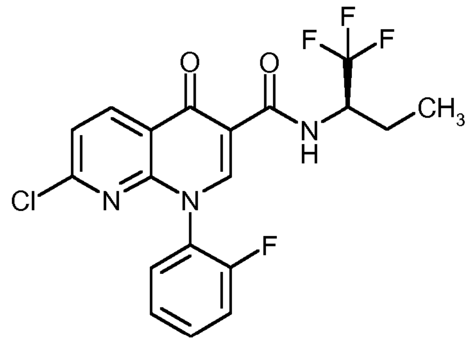

Gemäß AAV1 wurden 250 mg (784 μmol) der Verbindung aus Beispiel 33A mit 192 mg (1.18 mmol) (*R*)-1-Trifluormethylpropylamin Hydrochlorid in Gegenwart von 298 mg (784 μmol) HATU und 410 μl (2.35 mmol) *N*,*N*-Diisopropylethylamin in 8.1 ml Dimethylformamid umgesetzt. Das Rohprodukt wurde mittels Flashchromatographie (Cyclohexan/Essigsäureethylester-Gradient) gereinigt und 139 mg (41% d. Th., 99% Reinheit) der Titelverbindung erhalten.
$^1$H-NMR (400 MHz, DMSO-$d_6$): δ [ppm] = 10.00 (d, 1H), 8.86 (s, 1H), 8.76 (d, 1H), 7.83-7.65 (m, 3H), 7.58-7.51 (m, 1H), 7.50-7.44 (m, 1H), 4.85-4.69 (m, 1H), 1.96-1.82 (m, 1H), 1.75-1.60 (m, 1H), 0.98 (t, 3H).
LC-MS (Methode 1): $R_t$ = 1.17 min; 428 [M+H]+.

**Beispiel 83A**

*tert.*-Butyl-5-[8-(2,4-Difluorphenyl)-5-oxo-6-{[(2*R*)-1,1,1-trifluorbutan-2-yl]carbamoyl}-5,8-dihydro-1,8-naphthyridin-2-yl]-1-oxohexahydropyrrol[3,4-*c*]pyrrol-2(1*H*)-carboxylat (Diastereomerengemisch)

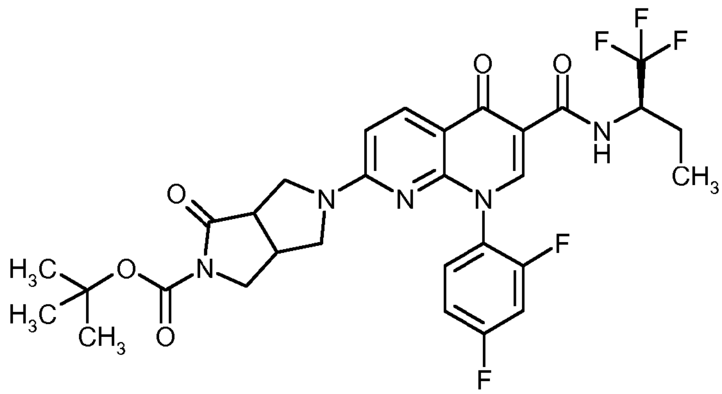

Gemäß AAV3 wurden 200 mg (449 μmol) der Verbindung aus Beispiel 67A mit 128 mg (538 μmol) *rac-tert.*-Butyl-1-oxo-octahydropyrrol[3,4-*c*]pyrrol-2-carboxylat in Gegenwart von 117 μl (673 μmol) *N*,*N*-Diisopropylethylamin in 4.4 ml Dimethylformamid umgesetzt. Das Rohprodukt wurde mit Acetonitril verdünnt und mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 224 mg (79% d. Th., 100% Reinheit) der Titelverbindung erhalten.
$^1$H-NMR (400 MHz, DMSO-$d_6$): δ [ppm] = 10.47 (d, 1H), 8.62 (s, 1H), 8.32 (d, 1H), 7.77-7.86 (m, 1H), 7.54-7.65 (m, 1H), 7.28-7.38 (m, 1H), 6.65-6.90 (br. s, 1H), 4.68-4.80 (m, 1H), 2.97-3.90 (m, 8H), 1.82-1.94 (m, 1H), 1.57-1.70 (m, 1H),

1.43 (s, 9H), 0.96 (t, 3H).
LC-MS (Methode 3): $R_t$ = 2.20 min; 636 $[M+H]^+$.

**Beispiel 84A:**

7-Chlor-1-(2,6-difluorphenyl)-4-oxo-1,4-dihydro-1,8-naphthyridin-3-carbonsäureethylester

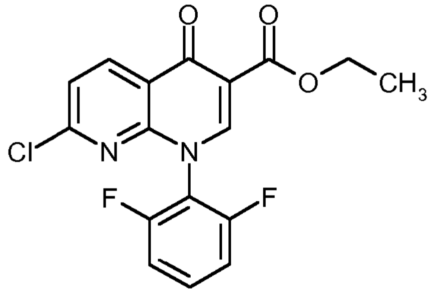

Eine Lösung aus 6.05 g (19.0 mmol) 2-[(2,6-Dichlorpyridin-3-yl)carbonyl]-3-ethoxyacrylsäureethylester (CAS 157373-27-8) und 3.44 g (26.6 mmol) 2,6-Difluoranilin in 30.2 ml DCM wurde mit 23.2 ml (133 mmol) DIPEA versetzt und 4h bei Raumtemperatur gerührt. Es wurde mit 200 ml DCM verdünnt und zweimal mit 75 ml 1M wässriger Salzsäure gewaschen. Die organische Phase wurde über Natriumsulfat getrocknet, filtriert und das Lösungsmittel unter reduziertem Druck entfernt. Der Rückstand wurde mit 40 ml *tert.*-Butylmethylether verrührt und der Niederschlag mit 10 ml *tert.*-Butylmethylether gewaschen. Anschließend wurde der Niederschlag mit 30 ml DCM und 2.63 g (19.0 mmol) Kaliumcarbonat versetzt und über Nacht unter Rückfluss erhitzt. Es wurde auf RT abgekühlt, mit 200 ml DCM verdünnt und zweimal mit 75 ml 1M wässriger Salzsäure gewaschen. Die organische Phase wurde über Magnesiumsulfat getrocknet, filtriert und das Lösungsmittel unter reduziertem Druck entfernt. Der Rückstand wurde in 100 ml Acetonitril und 30 ml DMF aufgenommen und auf 50 °C erwärmt. Bei 50 °C wurden 1.66 g (12.0 mmol) Kaliumcarbonat hinzugegeben und es wurde für 1h nachgerührt. Die Reaktionsmischung wurde auf RT abgekühlt und auf 200 ml wässrige 1M Salzsäure gegossen. Es wurde dreimal mit 150 ml DCM extrahiert. Die vereinigten organischen Phasen wurden über Magnesiumsulfat getrocknet, filtriert und das Lösungsmittel unter reduziertem Druck entfernt. Der Rückstand wurde mit 200 ml Wasser verrührt, der Niederschlag abgesaugt und am Hochvakuum getrocknet. Es wurden 4.19 g (60% d. Th., 100% Reinheit) der Titelverbindung erhalten.
$^1$H-NMR (400 MHz, DMSO-d6): $\delta$ [ppm] = 8.92 (s, 1H), 8.63 (d, 1H), 7.78-7.70 (m, 1H), 7.68 (d, 1H), 7.49-7.43 (m, 2H), 4.25 (q, 2H), 1.28 (t, 3H).
LC-MS (Methode 3): $R_t$ = 1.78 min; 365 $[M+H]^+$.

**Beispiel 85A:**

7-Chlor-1-(2,6-difluorphenyl)-4-oxo-1,4-dihydro-1,8-naphthyridin-3-carbonsäure

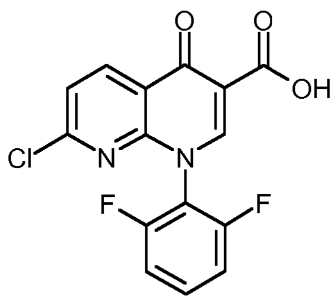

Zu einer Suspension von 3.83 g (10.5 mmol) der Verbindung aus Beispiel 84A in 31.5 ml Wasser wurden nacheinander 31.5 ml konzentrierte Salzsäure und 31.5 ml Tetrahydrofuran gegeben. Die resultierende Suspension

wurde 4h bei 120 °C stark gerührt und im Anschluss auf RT abgekühlt. Es wurde mit 100 ml Wasser verdünnt und der Niederschlag abgesaugt und am Hochvakuum getrocknet. Es wurden 3.39 g (95% d. Th., 98.9% Reinheit) der Titelverbindung erhalten.
$^1$H-NMR (400 MHz, DMSO-$d_6$): δ [ppm] = 13.86 (s, 1H), 9.25 (s, 1H), 8.79 (d, 1H), 7.82 (d, 1H), 7.80-7.72 (m, 1H), 7.51-7.43 (m, 2H).
LC-MS (Methode 3): $R_t$ = 1.74 min; 337 $[M+H]^+$.

**Beispiel 86A:**

7-Chlor-1-(2,6-difluorphenyl)-4-oxo-*N*-[(2*R*)-1,1,1-trifluorbutan-2-yl]-1,4-dihydro-1,8-naphthyridin-3-carbonsäureamid

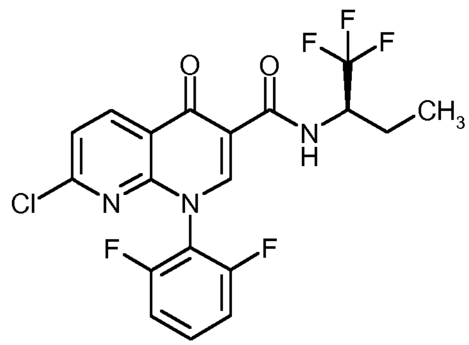

Gemäß AAV1 wurden 500 mg (1.43 mmol) der Verbindung aus Beispiel 85A mit 350 mg (2.14 mmol) (*R*)-1-Trifluormethylpropylamin Hydrochlorid in Gegenwart von 542 mg (1.43 mmol) HATU und 596 µl (3.42 mmol) DIPEA in 14.3 ml DMF umgesetzt. Das Rohprodukt wurde mittels Flashchromatographie (Cyclohexan/Essigsäureethylester-Gradient) gereinigt und 493 mg (77% d. Th., 99% Reinheit) der Titelverbindung erhalten.
$^1$H-NMR (400 MHz, DMSO-$d_6$): δ [ppm] = 9.89 (d, 1H), 9.09 (s, 1H), 8.76 (d, 1H), 7.78 (d, 1H), 7.80-7.71 (m, 1H), 7.50-7.43 (m, 2H), 4.84-4.71 (m, 1H), 1.96-1.84 (m, 1H), 1.75-1.61 (m, 1H), 0.98 (t, 3H).
LC-MS (Methode 3): $R_t$ = 2.30 min; 446 $[M+H]^+$.

**Beispiel 87A:**

*rac*-1-(2,4-Difluorophenyl)-7-(3-hydroxypyrrolidin-1-yl)-4-oxo-1,4-dihydro-1,8-naphthyridin-3-carbonsäureethylester

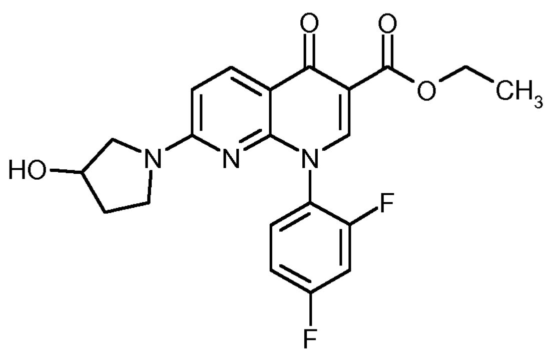

Zu einer Lösung von 3.00 g (8.23 mmol) 7-Chlor-1-(2,4-difluorphenyl)-4-oxo-1,4-dihydro-1,8-naphthyridin-3-carbonsäureethylester (Darstellung beschrieben in DE 4301246, Beispiel U, S.26) in 20.8 ml DMF wurden nacheinander 1.33 ml (16.5 mmol) rac-3-Pyrrolidinol und 5.01 ml (28.8 ml) DIPEA gegeben. Es wurde über Nacht bei RT nachgerührt. Die Reaktion wurde durch Zugabe von Wasser beendet und der Niederschlag abgesaugt, mit Wasser gewaschen und am Hochvakuum getrocknet. Es wurden 3.33 g (97.5% d. Th., 100% Reinheit) der Titelverbindung erhalten.
LC-MS (Methode 1): $R_t$ = 0.83 min; 416 $[M+H]^+$.

**Beispiel 88A:**

*rac*-1-(2,4-Difluorophenyl)-7-(3-hydroxypyrrolidin-1-yl)-4-oxo-1,4-dihydro-1,8-naphthyridin-3-carbonsäure

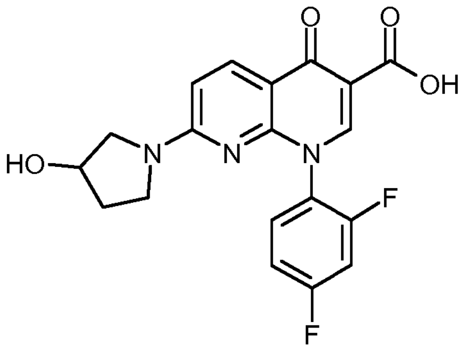

3.30 g (8.02 mmol) der Verbindung aus Beispiel 87Awurden in 25 ml Wasser teilweise gelöst/suspendiert, mit 25 ml konzentrierter Salzsäure versetzt und für 6h unter Rückfluss erhitzt. Die Reaktionsmischung ruhte über das Wochenende bei RT. Der Niederschlag wurde anschließend abgesaugt, mit wässriger 0.5M Salzsäure und Ethanol gewaschen und am Hochvakuum getrocknet. Es wurden 2.14 g (67% d. Th., 97% Reinheit) der Titelverbindung erhalten.
LC-MS (Methode 1): $R_t$ = 0.80 min; 388 $[M+H]^+$.
$^1$H-NMR (400 MHz, DMSO-d6): $\delta$ [ppm] = 8.77 (s, 1H), 8.33-8.27 (m, 1H), 7.85-7.76 (m, 1H), 7.62-7.54 (m, 1H), 7.37-7.29 (m, 1H), 6.88-6.79 (m, 1H), 4.43-3.01 (m, 6H, z.T. unter dem Wasserpeak), 2.07-1.73 (m, 2H).

**Beispiel 89A:**

7-Chlor-4-oxo-1-[2-(trifluormethyl)phenyl]-1,4-dihydro-1,8-naphthyridin-3-carbonsäureethylester

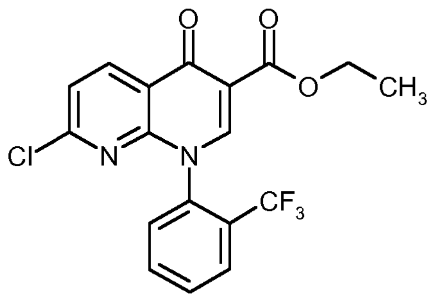

Eine Lösung aus 6.05 g (19.0 mmol) 2-[(2,6-Dichloropyridin-3-yl)carbonyl]-3-ethoxyacrylsäureethylester (CAS 157373-27-8) und 4.59 g (28.5 mmol) 2-Trifluormethylanilin in 30 ml DCM wurde mit 23.2 ml (133 mmol) DIPEA versetzt und 4h bei RT gerührt. Anschließend wurden 2.63 g (19.0 mmol) Kaliumcarbonat zugegeben und über Nacht unter Rückfluss erhitzt. Es wurde mit 400 ml DCM verdünnt und zweimal mit 150 ml 1M wässriger Salzsäure gewaschen. Die organische Phase wurde über Natriumsulfat getrocknet, filtriert und das Lösungsmittel unter reduziertem Druck entfernt. Die erhaltene Suspension wurde mit 40 ml *tert.*-Butylmethylether verrührt, der Niederschlag abgesaugt, mit 10 ml *tert.*-Butylmethylether gewaschen und im Hochvakuum getrocknet. Es wurden 4.21 g (55% d. Th., 99% Reinheit) der Titelverbindung erhalten.
LC-MS (Methode 3): $R_t$ = 1.81 min; 397 $[M+H]^+$.
$^1$H-NMR (400 MHz, DMSO-d6): $\delta$ [ppm] = 8.71 (s, 1H), 8.62 (d, 1H), 8.04-8.00 (m, 1H), 7.99-7.93 (m, 1H), 7.89-7.83 (m, 2H), 7.63 (d, 1H), 4.23 (q, 2H), 1.26 (t, 3H).

**Beispiel 90A:**

7-Chlor-4-oxo-1-[2-(trifluormethyl)phenyl]-1,4-dihydro-1,8-naphthyridin-3-carbonsäure

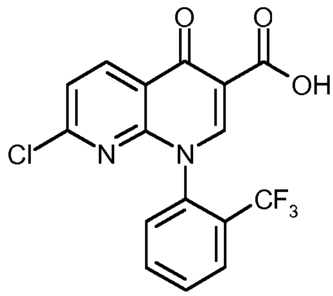

Zu einer Suspension von 4.10 g (10.3 mmol) der Verbindung aus Beispiel 89A in 51 ml THF wurden 20.7 ml wässrige 1M Lithiumhydroxid-Lösung (20.7 mmol) gegeben und die Reaktionsmischung für 3h bei RT gerührt. Anschließend wurde mit 250 ml Wasser verdünnt und der pH-Wert mit 1N wässriger Salzsäure auf pH 1 eingestellt. Der Niederschlag wurde abgesaugt, mit Wasser gewaschen und über Nacht im Vakuumtrockenschrank bei 40°C getrocknet. Es wurden 3.77 g (98% d. Th., 99% Reinheit) der Titelverbindung erhalten.
LC-MS (Methode 3): $R_t$ = 1.84 min; 369 $[M+H]^+$.
$^1$H-NMR (400 MHz, DMSO-$d_6$): $\delta$ [ppm] = 14.03 (br. s, 1H), 9.08 (s, 1H), 8.80 (d, 1H), 8.06-8.01 (m, 1H), 8.00-7.94 (m, 1H), 7.92-7.84 (m, 2H), 7.79 (d, 1H).

**Beispiel 91A:**

7-Chlor-4-oxo-*N*-[(2*R*)-1,1,1-trifluorbutan-2-yl]-1-[2-(trifluormethyl)phenyl]-1,4-dihydro-1,8-naphthyridin-3-carbonsäureamid

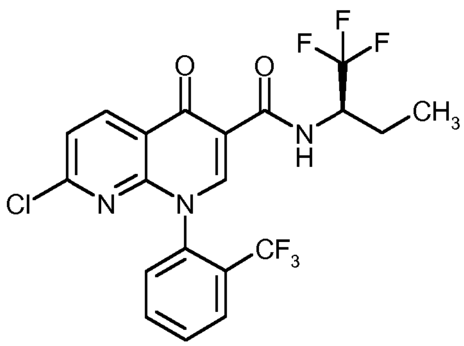

Gemäß AAV1 wurden 250 mg (678 μmol) der Verbindung aus Beispiel 90A mit 166 mg (1.02 mmol) (*R*)-1-Trifluormethylpropylamin Hydrochlorid in Gegenwart von 258 mg (678 μmol) HATU und 354 μl (2.03 mmol) DIPEA in 7 ml DMF umgesetzt. Das Rohprodukt wurde mittels Flashchromatographie (25g, Silica-Kartusche, Fluss: 25 ml/min, Detektion: 220 nm und 270 nm, Cyclohexan/Essigsäureethylester-Gradient (0% Essigsäureethylester, dann 20% Essigsäureethylester, dann 30% Essigsäureethylester) gereinigt. Die beiden Atropisomere wurden getrennt und es wurden 56.6 mg (17% d. Th., 99% Reinheit, Atropisomer 1, Beispiel 92A) und 58.8 mg (18% d. Th., 99% Reinheit, Atropisomer 2, Beispiel 93A) der Titelverbindung als Atropisomere erhalten.

**Beispiel 92A:**

7-Chlor-4-oxo-*N*-[(2*R*)-1,1,1-trifluorbutan-2-yl]-1-[2-(trifluormethyl)phenyl]-1,4-dihydro-1,8-naphthyridin-3-carbonsäureamid (Atropisomer 1)

LC-MS (Methode 3): $R_t$ = 2.32 min; 478 $[M+H]^+$.
$^1$H-NMR (400 MHz, DMSO-$d_6$): δ [ppm] = 9.99 (d, 1H), 8.86 (s, 1H), 8.75 (d, 1H), 8.06-8.02 (m, 1H), 8.00-7.95 (m, 1H), 7.91-7.85 (m, 2H), 7.73 (d, 1H), 4.83-4.70 (m, 1H), 1.96-1.84 (m, 1H), 1.76-1.62 (m, 1H), 0.99 (t, 3H).

**Beispiel 93A:**

7-Chlor-4-oxo-*N*-[(2*R*)-1,1,1-trifluorbutan-2-yl]-1-[2-(trifluormethyl)phenyl]-1,4-dihydro-1,8-naphthyridin-3-carbonsäureamid (Atropisomer 2)

LC-MS (Methode 3): $R_t$ = 2.31 min; 478 $[M+H]^+$.
$^1$H-NMR (400 MHz, DMSO-$d_6$): δ [ppm] = 9.99 (d, 1H), 8.87 (s, 1H), 8.76 (d, 1H), 8.06-8.02 (m, 1H), 8.00-7.95 (m, 1H), 7.91-7.85 (m, 2H), 7.74 (d, 1H), 4.83-4.70 (m, 1H), 1.96-1.83 (m, 1H), 1.74-1.61 (m, 1H), 0.96 (t, 3H).

**Beispiel 94A:**

(3*R*)-2,5-Dioxotetrahydrofuran-3-yltrifluoracetat

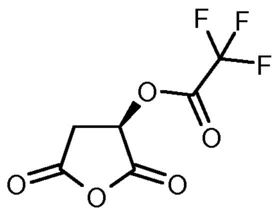

Bei 0°C wurden 5.70 g (42.5 mmol) (2*R*)-2-Hydroxysuccinsäure mit 12.0 ml (85.0 mmol) Trifluoressigsäureanhydrid versetzt und es wurde für 1h bei 0°C und für 2h bei Raumtemperatur nachgerührt. Anschließend wurden alle flüchtigen Komponenten unter reduziertem Druck bei Raumtempertur entfernt. Das Rohprodukt wurde ohne weitere Aufreinigung in Beispiel 95A eingesetzt.

**Beispiel 95A:**

(3*R*)-3-Hydroxy-4-methoxy-4-oxobutansäure

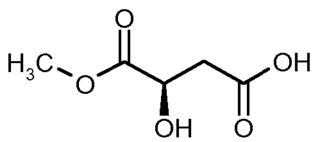

9.02 g (42.5 mmol) der Verbindung aus Beispiel 94A wurden mit 5.17 ml (128 mmol) Methanol versetzt und es wurde für 4h bei Raumtemperatur nachgerührt. Anschließend wurde überschüssiges Methanol unter reduziertem Druck entfernt und der Rückstand aus Diethylether/Cyclohexan umkristallisiert. Der Feststoff wurde am Hochvakuum getrocknet. Es wurden 5.84 g (92.8% d. Th.) der Titelverbindung erhalten.
$^1$H-NMR (400 MHz, DMSO-$d_6$): δ [ppm] = 12.31 (br. s, 1H), 5.68 (br. s, 1H), 4.35 (dd, 1H), 3.63 (s, 3H), 2.63 (dd, 1H), 2.52-2.45 (m, 1H).

**Beispiel 96A:**

(5S)-2-Oxo-1,3-oxazolidin-5-carbonsäuremethylester

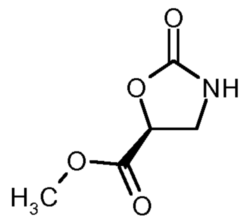

Eine Lösung von 5.84 g (39.4 mmol) der Verbindung aus Beispiel 95A in 159 ml *tert.*-Butanol wurde mit 11.9 g (43.4 mmol) Diphenylphosphorylazid und 6.05 ml (43.4 mmol) Triethylamin versetzt und anschließend für 4h unter Rückfluss erhitzt. Es wurde auf Raumtemperatur abgekühlt und das Lösungsmittel unter reduziertem Druck entfernt. Der Rückstand wurde mittels Flashchromatographie (Essigsäureethylester/Cyclohexan-Gradient) gereinigt. Abschließend wurde aus Essigsäureethylester/Cyclohexan umkrsitallisiert, der Feststoff am Hochvakuum getrocknet und 3.29 g (57.7% d. Th.) der Titelverbindung erhalten.
$^1$H-NMR (400 MHz, DMSO-$d_6$): δ [ppm] = 7.79 (s, 1H), 5.15 (dd, 1H), 3.80-3.74 (m, 1H), 3.73 (s, 3H), 3.52-3.46 (m, 1H).

**Beispiel 97A:**

(3*S*)-2,5-Dioxotetrahydrofuran-3-yl trifluoracetat

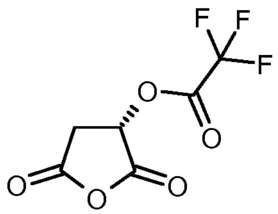

Bei 0°C wurden 5.70 g (42.5 mmol) (2*S*)-2-Hydroxysuccinsäure mit 12.0 ml (85.0 mmol) Trifluoressigsäureanhydrid versetzt und es wurde für 1h bei 0°C und für 2h bei Raumtemperatur nachgerührt. Anschließend wurden alle flüchtigen Komponenten unter reduziertem bei Raumtempertur entfernt. Das Rohprodukt wurde ohne weitere Aufreinigung in Beispiel 98A eingesetzt.

**Beispiel 98A:**

(3*S*)-3-Hydroxy-4-methoxy-4-oxobutansäure

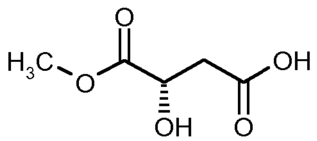

9.02 g (42.5 mmol) der Verbindung aus Beispiel 97A wurden mit 5.17 ml (128 mmol) Methanol versetzt und es wurde für 4h bei Raumtemperatur nachgerührt. Anschließend wurde überschüssiges Methanol unter reduziertem Druck entfernt und der Rückstand aus aus Diethylether/Cyclohexan umkristallisiert. Der Feststoff wurde am Hochvakuum getrocknet. Es wurden 5.95 g (94.5% d. Th.) der Titelverbindung erhalten.
$^1$H-NMR (400 MHz, DMSO-$d_6$): δ [ppm] = 12.31 (br. s, 1H), 5.67 (br. s, 1H), 4.38-4.31 (m, 1H), 3.63 (s, 3H), 2.63 (dd,

1H), 2.52-2.45 (m, 1H).

**Beispiel 99A:**

(5*R*)-2-Oxo-1,3-oxazolidin-5-carbonsäuremethylester

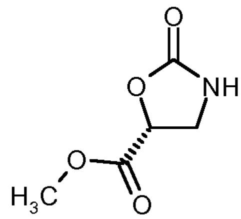

Eine Lösung von 5.95 g (39.4 mmol) der Verbindung aus Beispiel 98A in 160 ml *tert.*-Butanol wurde mit 12.2 g (44.2 mmol) Diphenylphosphorylazid und 6.16 ml (44.2 mmol) Triethylamin versetzt und anschließend für 4h unter Rückfluss erhitzt. Es wurde auf Raumtemperatur abgekühlt und das Lösungsmittel unter reduziertem Druck entfernt. Der Rückstand wurde mittels Flashchromatographie (Essigsäureethylester/Cyclohexan-Gradient) gereinigt. Abschließend wurde aus Essigsäureethylester/Cyclohexan umkrsitallisiert, der Feststoff am Hochvakuum getrocknet und 3.48 g (59.7% d. Th.) der Titelverbindung erhalten.
$^1$H-NMR (400 MHz, DMSO-$d_6$): $\delta$ [ppm] = 7.79 (s, 1H), 5.15 (dd, 1H), 3.80-3.74 (m, 1H), 3.73 (s, 3H), 3.52-3.46 (m, 1H).

**Beispiel 100A:**

7-Chlor-4-oxo-1-(2,4,6-trifluorphenyl)-1,4-dihydro-1,8-naphthyridin-3-carbonsäureethylester

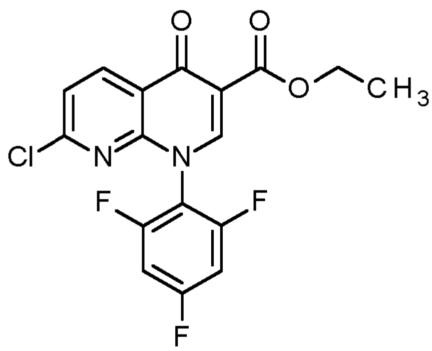

Eine Lösung von 12.1 g (38.0 mmol) 2-[(2,6-Dichlorpyridin-3-yl)carbonyl]-3-ethoxyacrylsäureethylester (CAS 157373-27-8) und 7.83 g (53.2 mmol) 2,4,6-Trifluoranilin in 60.5 ml DCM wurde mit 46.4 ml (266 mmol) DIPEA versetzt und 4h bei RT gerührt. Anschließend wurden 5.26 g (38.0 mmol) Kaliumcarbonat zugegeben und über Nacht unter Rückfluss erhitzt. Es wurde mit 200 ml DCM verdünnt und zweimal mit 150 ml 1M wässriger Salzsäure gewaschen. Die organische Phase wurde über Natriumsulfat getrocknet, filtriert und das Lösungsmittel unter reduziertem Druck entfernt. Die erhaltene Suspension wurde mit 80 ml *tert.*-Butylmethylether verrührt, der Niederschlag abgesaugt, mit 10 ml *tert.*-Butylmethylether gewaschen und im Hochvakuum getrocknet. Es wurden 8.60 g (58% d. Th., 99% Reinheit) der Titelverbindung erhalten.
LC-MS (Methode 1): $R_t$ = 0.97 min; 383 [M+H]$^+$.

**Beispiel 100B:**

7-Chlor-4-oxo-1-(2,4,6-trifluorphenyl)-1,4-dihydro-1,8-naphthyridin-3 -carbonsäure

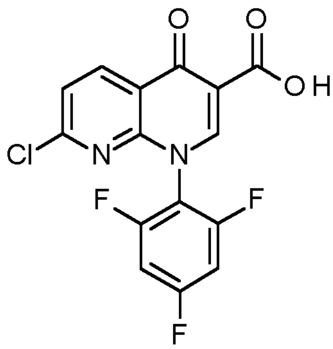

8.60 g (22.5 mmol) 7-Chlor-4-oxo-1-(2,4,6-trifluorphenyl)-1,4-dihydro-1,8-naphthyridin-3-carbonsäure-ethylester (Beispiel 100A) wurden in 67.7 ml Wasser vorgelegt, mit 67.7 ml 36 proz. wässriger Salzsäure und 67.7 ml THF versetzt und 4.5h bei 110°C nachgerührt. Das Reaktionsgemisch wurde auf RT abgekühlt. Der Niederschlag wurde abgesaugt, mit Wasser nachgewaschen und am Hochvakuum getrocknet. Es wurden 7.87 g (98% d. Th., 99% Reinheit) der Titelverbindung erhalten.
LC-MS (Methode 1): $R_t$ = 0.95 min; MS (ESIpos): m/z = 355 $[M+H]^+$.
$^1H$ NMR (400 MHz, DMSO-d6): $\delta$ [ppm] = 13.83 (s, 1H), 9.27 (s, 1H), 8.78 (d, 1H), 7.82 (d, 1H), 7.67-7.59 (m, 2H).

**Beispiel 100C:**

7-Chlor-4-oxo-*N*-[(2*R*)-1,1,1-trifluorbutan-2-yl]-1-(2,4,6-trifluorphenyl)-1,4-dihydro-1,8-naphthyridin-3-carbonsäureamid

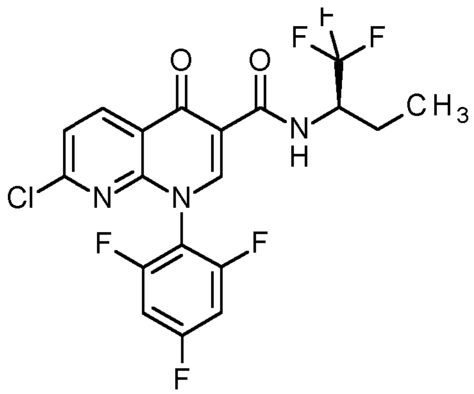

Gemäß AAV1 wurden 1.00 g (2.82 mmol) 7-Chlor-4-oxo-1-(2,4,6-trifluorphenyl)-1,4-dihydro-1,8-naphthyridin-3-carbonsäure (Beispiel 100B) mit 692 mg (4.23 mmol) (2*R*)-1,1,1-Trifluorbutan-2-amin Hydrochlorid in Gegenwart von 1.07 g (2.82 mmol) HATU und 1.18 ml (6.77 mmol) *N,N*-Diisopropylethylamin in 28.3 ml Dimethylformamid umgesetzt. Die Reaktion wurde durch Zugabe von 40 ml Wasser und 60 ml Essigsäureethylester beendet und die Phasen getrennt. Die wässrige Phase wurde dreimal mit 20 ml Essigsäureethylester extrahiert und die vereinigten organischen Phasen mit 40 ml einer Mischung (1:1, v/v) aus gesättigter wässriger Natriumchlorid-Lösung und wässriger 1N Salzsäure gewaschen. Die organische Phase wurde dreimal mit 30 ml gesättigter wässriger Natriumchlorid-Lösung gewaschen, über Natriumsulfat getrocknet, filtriert und das Lösungsmittel unter reduziertem Druck entfernt. Der Rückstand wurde mittels Normalphasenchromatographie (Cyclohexan-Essigsäureethylester Gradient) gereinigt und 1.16 g (88% d. Th., 99% Reinheit) der Titelverbindung erhalten.

$^1$H NMR (400 MHz, DMSO-$d_6$) δ [ppm] = 9.88 (d, 1H), 9.13 (s, 1H), 8.75 (d, 1H), 7.78 (d, 1H), 7.66-7.58 (m, 2H), 4.83-4.72 (m, 1H), 1.95-1.84 (m, 1H), 1.74-1.61 (m, 1H), 0.98 (t, 3H).
LC-MS (Methode 3): $R_t$ = 2.35 min; 464 [M+H]$^+$.

**Beispiel 101A:**

*rac*-7-Chlor-*N*-(1-cyclopropyl-2,2,2-trifluorethyl)-1-(2,4-difluorphenyl)-4-oxo-1,4-dihydro-1,8-naphthyridin-3-carbonsäureamid

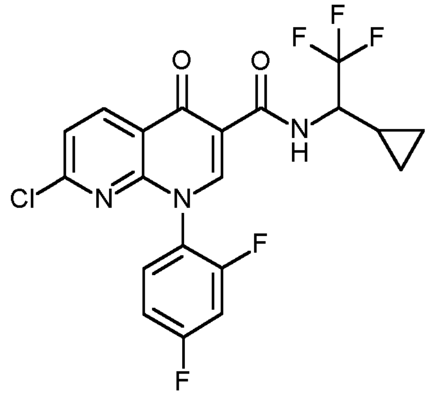

Gemäß AAV1 wurden 500 mg (1.49 mmol) 7-Chlor-4-oxo-1-(2,4-difluorphenyl)-1,4-dihydro-1,8-naphthyridin-3-carbonsäure (Beispiel 35A) mit 391 mg (2.23 mmol) 1-Cyclopropyl-2,2,2-trifluorethanamin Hydrochlorid (Racemat, CAS: 75702-99-7) in Gegenwart von 565 mg (1.49 mmol) HATU und 621 µl (3.56 mmol) *N*,*N*-Diisopropylethylamin in 15 ml Dimethylformamid umgesetzt. Die Reaktion wurde durch Zugabe von 20 ml Wasser und 30 ml Essigsäureethylester beendet und die Phasen getrennt. Die wässrige Phase wurde dreimal mit 10 ml Essigsäureethylester extrahiert und die vereinigten organischen Phasen mit 40 ml einer Mischung (1:1, v/v) aus gesättigter wässriger Natriumchlorid-Lösung und wässriger 1N Salzsäure gewaschen. Die organische Phase wurde dreimal mit 15 ml gesättigter wässriger Natriumchlorid-Lösung gewaschen, über Natriumsulfat getrocknet, filtriert und das Lösungsmittel unter reduziertem Druck entfernt. Der Rückstand wurde mittels Normalphasenchromatographie (Cyclohexan-Essigsäureethylester Gradient) gereinigt und 564 mg (82% d. Th., 99% Reinheit) der Titelverbindung erhalten.
$^1$H NMR (400 MHz, DMSO-$d_6$) δ [ppm] = 10.14 (d, 1H), 8.89 (s, 1H), 8.75 (d, 1H), 7.91-7.81 (m, 1H), 7.76 (d, 1H), 7.68-7.59 (m, 1H), 7.41-7.33 (m, 1H), 4.47-4.33 (m, 1H), 1.29-1.19 (m, 1H), 0.71-0.51 (m, 3H), 0.37-0.28 (m, 1H).
LC-MS (Methode 1): $R_t$ = 1.20 min; 458 [M+H]$^+$.

**Beispiel 102A:**

*rac*-7-Chlor-*N*-(1-cyclopropyl-2,2,2-trifluorethyl)-1-(2,6-difluorphenyl)-4-oxo-1,4-dihydro-1,8-naphthyridin-3-carbonsäureamid

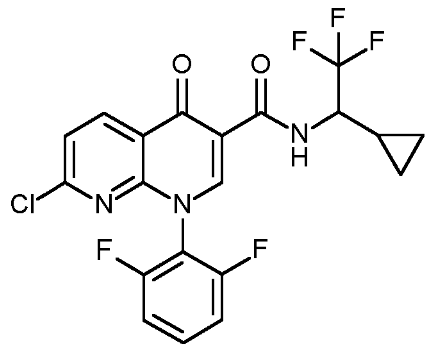

Gemäß AAV1 wurden 1.00 g (2.97 mmol) 7-Chlor-4-oxo-1-(2,6-difluorphenyl)-1,4-dihydro-1,8-naphthyridin-3-carbonsäure (Beispiel 85A) mit 782 mg (4.46 mmol) 1-Cyclopropyl-2,2,2-trifluorethanamin Hydrochlorid (Racemat, CAS: 75702-99-7) in Gegenwart von 1.13g (2.97 mmol) HATU und 1.24 ml (7.13 mmol) *N*,*N*-Diisopropylethylamin in 30 ml Dimethylformamid umgesetzt. Die Reaktion wurde durch Zugabe von 20 ml Wasser und 30 ml Essigsäureethylester beendet und die Phasen getrennt. Die wässrige Phase wurde dreimal mit 10 ml Essigsäureethylester extrahiert und die vereinigten organischen Phasen mit 20 ml einer Mischung (1:1, v/v) aus gesättigter wässriger Natriumchlorid-Lösung und wässriger 1N Salzsäure gewaschen. Die organische Phase wurde dreimal mit 15 ml gesättigter wässriger Natriumchlorid-Lösung gewaschen, über Natriumsulfat getrocknet, filtriert und das Lösungsmittel unter reduziertem Druck entfernt. Der Rückstand wurde mittels Normalphasenchromatographie (Cyclohexan-Essigsäureethylester Gradient) gereinigt und 1.11 g (81% d. Th., 99% Reinheit) der Titelverbindung erhalten.
$^1$H NMR (400 MHz, DMSO-$d_6$) δ [ppm] = 10.05 (d, 1H), 9.08 (s, 1H), 8.77 (d, 1H), 7.81-7.70 (m, 2H), 7.50-7.42 (m, 2H), 4.45-4.33 (m, 1H), 1.30-1.20 (m, 1H), 0.72-0.54 (m, 3H), 0.38-0.30 (m, 1H).
LC-MS (Methode 3): $R_t$ = 2.32 min; 458 $[M+H]^+$.

**Beispiel 103A:**

7-Chlor-*N*-[(1*R*)-1-cyclopropyl-2,2,2-trifluorethyl]-1-(2,6-difluorphenyl)-4-oxo-1,4-dihydro-1,8-naphthyridin-3-carbonsäureamid

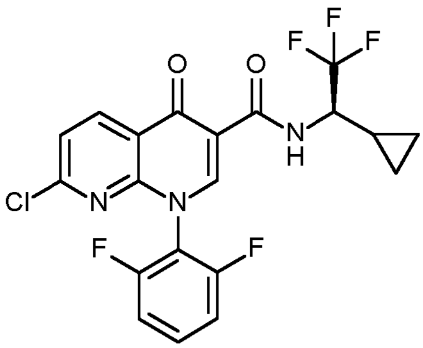

Gemäß AAV1 wurden 1.00 g (2.94 mmol) der Verbindung aus Beispiel 85A mit 774 mg (4.41 mmol) (*R*)-1-Trifluormethylpropylamin Hydrochlorid in Gegenwart von 1.12 g (2.94 mmol) HATU und 1.23 ml (7.06 mmol) DIPEA in 29.5 ml DMF umgesetzt. Die Reaktion wurde durch Zugabe von 40 ml Wasser und 60 ml Essigsäureethylester beendet und die Phasen getrennt. Die wässrige Phase wurde dreimal mit 20 ml Essigsäureethylester extrahiert und die vereinigten organischen Phasen mit 40 ml einer Mischung (1:1, v/v) aus gesättigter wässriger Natriumchlorid-Lösung und wässriger 1N Salzsäure gewaschen. Die organische Phase wurde dreimal mit 30 ml gesättigter wässriger Natriumchlorid-Lösung gewaschen, über Natriumsulfat getrocknet, filtriert und das Lösungsmittel unter reduziertem Druck entfernt. Der Rückstand wurde mittels Normalphasenchromatographie (Cyclohexan-Essigsäureethylester Gradient) gereinigt und 840 mg (62% d. Th., 99% Reinheit) der Titelverbindung erhalten.
$^1$H-NMR (400 MHz, DMSO-$d_6$): δ [ppm] = 10.05 (d, 1H), 9.08 (s, 1H), 8.76 (d, 1H), 7.81-7.70 (m, 2H), 7.50-7.42 (m, 2H), 4.46-4.33 (m, 1H), 1.29-1.18 (m, 1H), 0.73-0.52 (m, 3H), 0.39-0.30 (m, 1H).
LC-MS (Methode 3): $R_t$ = 2.31 min; 458 $[M+H]^+$.

**Beispiel 104A:**

7-Chlor-*N*-[(1*S*)-1-cyclopropyl-2,2,2-trifluorethyl]-1-(2,6-difluorphenyl)-4-oxo-1,4-dihydro-1,8-naphthyridin-3 -carbonsäureamid

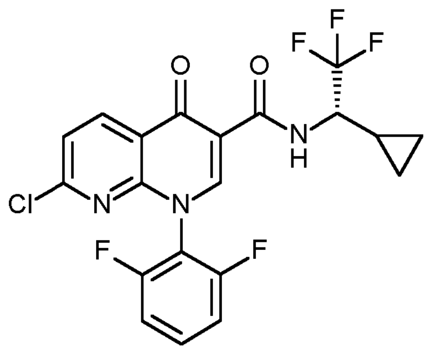

Gemäß AAV1 wurden 350 mg (1.03 mmol) der Verbindung aus Beispiel 85A mit 217 mg (1.24 mmol) (*S*)-1-Trifluormethylpropylamin Hydrochlorid in Gegenwart von 391 mg (1.03 mmol) HATU und 430 µl (2.47 mmol) DIPEA in 10 ml DMF umgesetzt. Der Ansatz wurde auf eine Mischung aus 30 ml Wasser und 5 ml 1N wässrige Salzsäure gegossen und der Niederschlag abgesaugt. Der Niederschlag wurde in wenig Dichlormethan aufgenommen und mittels Normalphasenchromatographie (Cyclohexan-Essigsäureethylester Gradient) gereinigt und 325 mg (69% d. Th., 100% Reinheit) der Titelverbindung erhalten.
$^1$H-NMR (400 MHz, DMSO-$d_6$): δ [ppm] = 10.05 (d, 1H), 9.08 (s, 1H), 8.76 (d, 1H), 7.81-7.70 (m, 2H), 7.50-7.42 (m, 2H), 4.46-4.33 (m, 1H), 1.30-1.20 (m, 1H), 0.72-0.53 (m, 3H), 0.39-0.29 (m, 1H).
LC-MS (Methode 1): $R_t$ = 1.20 min; 458 [M+H]$^+$.

**Beispiel 105A:**

7-Chlor-1-(2-chlor-6-fluorphenyl)-4-oxo-1,4-dihydro-1,8-naphthyridin-3-carbonsäureethylester

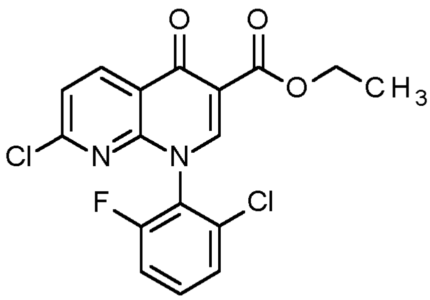

Eine Lösung von 6.05 g (19.0 mmol) 2-[(2,6-Dichlorpyridin-3-yl)carbonyl]-3-ethoxyacrylsäureethylester (CAS 157373-27-8) und 3.88 g (26.6 mmol) 2-Chlor-6-fluoranilin in 30.3 ml Dichlormethan wurde mit 23.2 ml (133 mmol) DIPEA versetzt und 4h bei RT gerührt. Anschließend wurden 2.63 g (19.0 mmol) Kaliumcarbonat zugegeben und über Nacht unter Rückfluss erhitzt. Es wurde mit 200 ml DCM verdünnt und zweimal mit 75 ml 1M wässriger Salzsäure gewaschen. Die organische Phase wurde über Natriumsulfat getrocknet, filtriert und das Lösungsmittel unter reduziertem Druck entfernt. Die erhaltene Suspension wurde mit 40 ml *tert.*-Butylmethylether verrührt, der Niederschlag abgesaugt, mit 10 ml *tert.*-Butylmethylether gewaschen und im Hochvakuum getrocknet. Es wurden 5.70 g (64% d. Th., 81% Reinheit) der Titelverbindung erhalten.
$^1$H-NMR (400 MHz, DMSO-$d_6$): δ [ppm] = 8.88 (s, 1H), 8.64 (d, 1H), 7.76-7.57 (m, 4H), 4.25 (q, 2H), 1.28 (t, 3H).
LC-MS (Methode 3): $R_t$ = 1.86 min; 381 [M+H]$^+$.

**Beispiel 105B:**

7-Chlor-1-(2-chlor-6-fluorphenyl)-4-oxo-1,4-dihydro-1,8-naphthyridin-3-carbonsäure

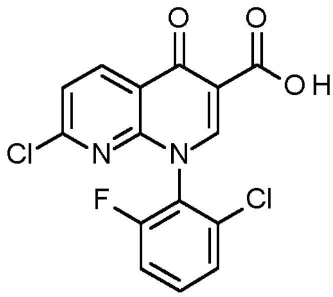

5.70 g (14.9 mmol) 7-Chlor-1-(2-chlor-6-fluorphenyl)-4-oxo-1,4-dihydro-1,8-naphthyridin-3-carbonsäure-ethylester wurden in 45 ml Wasser vorgelegt, mit 45 ml 36 proz. wässriger Salzsäure und 45 ml THF versetzt und 4.5h bei 120°C nachgerührt. Das Reaktionsgemisch wurde auf RT abgekühlt. Der Niederschlag wurde abgesaugt, mit Wasser nachgewaschen und am Hochvakuum getrocknet. Es wurden 4.12 g (77% d. Th., 99% Reinheit) der Titelverbindung erhalten.
$^1$H NMR (400 MHz, DMSO-d6): δ [ppm] = 13.84 (s, 1H), 9.23 (s, 1H), 8.80 (d, 1H), 7.82 (d, 1H), 7.78-7.57 (m, 3H)..
LC-MS (Methode 3): $R_t$ = 1.84 min; MS (ESIpos): m/z = 352.9 $[M+H]^+$.

**Beispiel 105C:**

7-Chlor-1-(2-chlor-6-fluorphenyl)-4-oxo-*N*-[(2*R*)-1,1,1-trifluorbutan-2-yl] -1,4-dihydro-1,8-naphthyridin-3 -carbonsäureamid

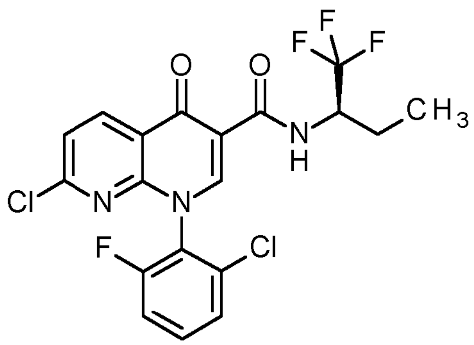

Gemäß AAV1 wurden 1.00 g (2.83 mmol) 7-Chlor-1-(2-chlor-6-fluorphenyl)-4-oxo-1,4-dihydro-1,8-naphthyridin-3-carbonsäure mit 695 mg (4.25 mmol) (2*R*)-1,1,1-Trifluorbutan-2-amin Hydrochlorid in Gegenwart von 1.08 g (2.83 mmol) HATU und 1.18 ml (6.80 mmol) *N*,*N*-Diisopropylethylamin in 28.4 ml Dimethylformamid umgesetzt. Die Reaktion wurde durch Zugabe von 40 ml Wasser und 60 ml Essigsäureethylester beendet und die Phasen getrennt. Die wässrige Phase wurde dreimal mit 20 ml Essigsäureethylester extrahiert und die vereinigten organischen Phasen mit 40 ml einer Mischung (1:1, v/v) aus gesättigter wässriger Natriumchlorid-Lösung und wässriger 1N Salzsäure gewaschen. Die organische Phase wurde dreimal mit 30 ml gesättigter wässriger Natriumchlorid-Lösung gewaschen, über Natriumsulfat getrocknet, filtriert und das Lösungsmittel unter reduziertem Druck entfernt. Der Rückstand wurde mittels Normalphasenchromatographie (Cyclohexan-Essigsäureethylester Gradient) gereinigt und 1.09 g (82% d. Th., 99% Reinheit) der Titelverbindung erhalten.
$^1$H NMR (400 MHz, DMSO-$d_6$) δ [ppm] = 9.90 (d, 1H), 9.07-9.06 (m, 1H), 8.77 (d, 1H), 7.79 (d, 1H), 7.77-7.57 (m, 3H), 4.83-4.71 (m, 1H), 1.96-1.84 (m, 1H), 1.75-1.62 (m, 1H), 1.02-0.95 (m, 3H).
LC-MS (Methode 1): $R_t$ = 1.31 min; 462 $[M+H]^+$.

**Beispiel 106A:**

7-Chlor-1-(2,6-difluorphenyl)-4-oxo-*N*-[(2*S*)-1,1,1-trifluorpropan-2-yl]-1,4-dihydro-1,8-naphthyridin-3-carbonsäureamid

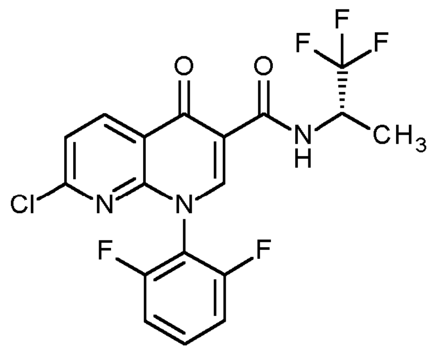

Gemäß AAV1 wurden 880 mg (2.61 mmol) der Verbindung aus Beispiel 85A mit 443 mg (3.92 mmol) (2*S*)-1,1,1-Trifluorpropan-2-amin in Gegenwart von 994 mg (2.61 mmol) HATU und 1.09 ml (6.27 mmol) DIPEA in 26.4 ml DMF umgesetzt. Die Reaktion wurde durch Zugabe von 20 ml Wasser und 30 ml Essigsäureethylester beendet und die Phasen getrennt. Die wässrige Phase wurde dreimal mit 10 ml Essigsäureethylester extrahiert und die vereinigten organischen Phasen mit 20 ml einer Mischung (1:1, v/v) aus gesättigter wässriger Natriumchlorid-Lösung und wässriger 1N Salzsäure gewaschen. Die organische Phase wurde dreimal mit 15 ml gesättigter wässriger Natriumchlorid-Lösung gewaschen, über Natriumsulfat getrocknet, filtriert und das Lösungsmittel unter reduziertem Druck entfernt. Das Rohprodukt wurde mittels Flashchromatographie (Cyclohexan/Essigsäureethylester-Gradient) gereinigt und 773 mg (68% d. Th., 99% Reinheit) der Titelverbindung erhalten.
$^1$H-NMR (400 MHz, DMSO-$d_6$): δ [ppm] = 9.94 (d, 1H), 9.08 (s, 1H), 8.75 (d, 1H), 7.80-7.71 (m, 2H), 7.50-7.43 (m, 2H), 4.99-4.88 (m, 1H), 1.40 (d, 3H).
LC-MS (Methode 3): $R_t$ = 2.19 min; 432 $[M+H]^+$.

**Beispiel 107A:**

7-Chlor-1-(2,4,6-trifluorphenyl)-4-oxo-*N*-[(2*S*)-1,1,1-trifluorpropan-2-yl]-1,4-dihydro-1,8-naphthyridin-3-carbonsäureamid

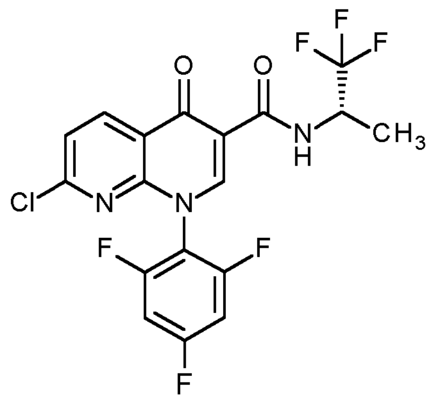

Gemäß AAV1 wurden 1.00 g (2.82 mmol) der Verbindung aus Beispiel 100B mit 478 mg (4.23 mmol) (2*S*)-1,1,1-Trifluorpropan-2-amin in Gegenwart von 1.07 g (2.82 mmol) HATU und 1.18 ml (6.77 mmol) DIPEA in 28.5 ml DMF umgesetzt. Die Reaktion wurde durch Zugabe von 25 ml Wasser und 35 ml Essigsäureethylester beendet und die Phasen getrennt. Die wässrige Phase wurde dreimal mit 15 ml Essigsäureethylester extrahiert und die vereinigten organischen Phasen mit 25 ml einer Mischung (1:1, v/v) aus gesättigter wässriger Natriumchlorid-Lösung und wässriger 1N Salzsäure gewaschen. Die organische Phase wurde dreimal mit 20 ml gesättigter wässriger Natriumchlorid-Lösung gewaschen, über Natriumsulfat getrocknet, filtriert und das Lösungsmittel unter reduziertem Druck entfernt. Das Rohprodukt wurde mittels Flashchromatographie (Cyclohexan/Essigsäureethylester-Gradient) gereinigt und 751 mg (59% d. Th., 99% Reinheit) der Titelverbindung erhalten.
$^1$H-NMR (400 MHz, DMSO-$d_6$): δ [ppm] = 9.93 (d, 1H), 9.13 (s, 1H), 8.75 (d, 1H), 7.78 (d, 1H), 7.66-7.58 (m, 2H), 5.00-4.86 (m, 1H), 1.40 (d, 3H).

LC-MS (Methode 1): $R_t$ = 1.26 min; 450 $[M+H]^+$.

**Beispiel 108A:**

7-Chlor-1-(2-chlor-4,6-difluorphenyl)-4-oxo-1,4-dihydro-1,8-naphthyridin-3-carbonsäureethylester

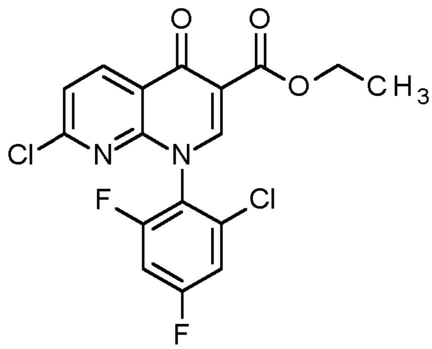

Eine Lösung von 13.8 g (43.4 mmol) 2-[(2,6-Dichlorpyridin-3-yl)carbonyl]-3-ethoxyacrylsäureethylester (CAS 157373-27-8) und 9.93 g (60.7 mmol) 2-Chlor-4,6-difluoranilin in 68.2 ml Dichlormethan wurde mit 52.9 ml (304 mmol) DIPEA versetzt und 4h bei RT gerührt. Anschließend wurden 6.00 g (43.4 mmol) Kaliumcarbonat zugegeben und über Nacht unter Rückfluss erhitzt. Es wurde mit 600 ml DCM verdünnt und zweimal mit 200 ml 1M wässriger Salzsäure gewaschen. Die organische Phase wurde über Natriumsulfat getrocknet, filtriert und das Lösungsmittel unter reduziertem Druck entfernt. Die erhaltene Suspension wurde mit 80 ml *tert.*-Butylmethylether verrührt, der Niederschlag abgesaugt, mit 20 ml *tert.*-Butylmethylether gewaschen und im Hochvakuum getrocknet. Es wurden 15.0 g (72% d. Th., 83% Reinheit) der Titelverbindung erhalten.
LC-MS (Methode 3): $R_t$ = 1.91 min; 399 $[M+H]^+$.

**Beispiel 108B:**

7-Chlor-1-(2-chlor-4,6-difluorphenyl)-4-oxo-1,4-dihydro-1,8-naphthyridin-3-carbonsäure

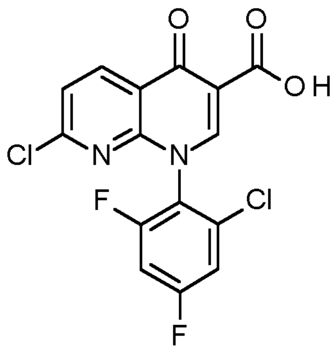

15.0 g (37.6 mmol) 7-Chlor-1-(2-chlor-4,6-difluorphenyl)-4-oxo-1,4-dihydro-1,8-naphthyridin-3-carbon-säureethylester wurden in 131 ml Wasser vorgelegt, mit 131 ml 36 proz. wässriger Salzsäure und 131 ml THF versetzt und 4.5h bei 110°C nachgerührt. Das Reaktionsgemisch wurde auf RT abgekühlt. Der Niederschlag wurde abgesaugt, mit Wasser nachgewaschen und am Hochvakuum getrocknet. Es wurden 10.2 g (72% d. Th., 99% Reinheit) der Titelverbindung erhalten.
$^1$H NMR (400 MHz, DMSO-d6): $\delta$ [ppm] = 13.81 (s, 1H), 9.25 (s, 1H), 8.80 (d, 1H), 7.84-7.73 (m, 3H).
LC-MS (Methode 3): $R_t$ = 1.87 min; MS (ESIpos): m/z = 370.9 $[M+H]^+$.

**Beispiel 108C:**

7-Chlor-1-(2-chlor-4,6-difluorphenyl)-4-oxo-*N*-[{2*R*)-1,1,1-trifluorbutan-2-yl]-1,4-dihydro-1,8-naphthyridin-3 -carbonsäureamid

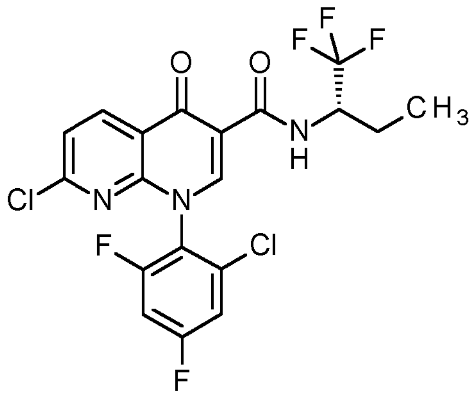

Gemäß AAV1 wurden 1.00 g (2.69 mmol) 7-Chlor-1-(2-chlor-4,6-difluorphenyl)-4-oxo-1,4-dihydro-1,8-naphthyridin-3-carbonsäure mit 513 mg (4.04 mmol) (2*S*)-1,1,1-Trifluorbutan-2-amin Hydrochlorid in Gegenwart von 1.02 g (2.69 mmol) HATU und 657 µl (3.77 mmol) *N,N*-Diisopropylethylamin in 27 ml Dimethylformamid umgesetzt. Die Reaktion wurde durch Zugabe von 40 ml Wasser und 60 ml Essigsäureethylester beendet und die Phasen getrennt. Die wässrige Phase wurde dreimal mit 20 ml Essigsäureethylester extrahiert und die vereinigten organischen Phasen mit 40 ml einer Mischung (1:1, v/v) aus gesättigter wässriger Natriumchlorid-Lösung und wässriger 1N Salzsäure gewaschen. Die organische Phase wurde dreimal mit 30 ml gesättigter wässriger Natriumchlorid-Lösung gewaschen, über Natriumsulfat getrocknet, filtriert und das Lösungsmittel unter reduziertem Druck entfernt. Der Rückstand wurde mittels Normalphasenchromatographie (Cyclohexan-Essigsäureethylester Gradient) gereinigt und 914 mg (71% d. Th., 100% Reinheit) der Titelverbindung erhalten.
$^1$H NMR (400 MHz, DMSO-$d_6$) δ [ppm] = 9.88 (d, 1H), 9.11 (s, 1H), 8.77 (d, 1H), 7.81-7.72 (m, 3H), 4.83-4.71 (m, 1H), 1.96-1.84 (m, 1H), 1.74-1.61 (m, 1H), 1.01-0.94 (m, 3H).
LC-MS (Methode 3): $R_t$ = 2.40 min; 480 [M+H]$^+$.

**Beispiel 109A:**

7-Chlor-1-(2-chlor-4,6-difluorphenyl)-4-oxo-*N*-(4,4,4-trifluor-2-methylbutan-2-yl)-1,4-dihydro-1,8-naphthyridin-3-carbonsäureamid

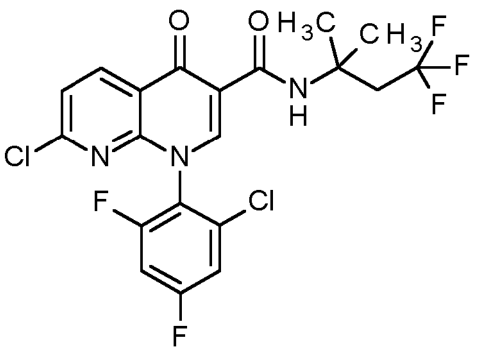

Gemäß AAV1 wurden 255 mg (680 µmol) 7-Chlor-1-(2-chlor-4,6-difluorphenyl)-4-oxo-1,4-dihydro-1,8-naphthyridin-3-carbonsäure mit 181 mg (1.02 mmol) 4,4,4-Trifluor-2-methylbutan-2-amin Hydrochlorid in Gegenwart von 259 mg (680 µmol) HATU und 415 µl (2.38 mmol) *N,N*-Diisopropylethylamin in 7 ml Dimethylformamid umgesetzt. Die

Reaktionsmischung wurde mit 1N wässriger Salzsäure auf pH 1 eingestellt und in mehreren Läufen mittels präparativer HPLC (Säule: Chromatorex C18, 10 μm, 125x30 mm, LM: Acetonitril / 0.05% Ameisensäure-Gradient; (0 bis 3 min 15% Acetonitril bis 15 min. 90% Acetonitril und weitere 3 min. 90% Acetonitril) gereinigt. Es wurden 202 mg (60% d. Th., 100% Reinheit) der Titelverbindung erhalten.

$^1$H NMR (400 MHz, DMSO-$d_6$) δ [ppm] = 9.70 (s, 1H), 9.00 (s, 1H), 8.75 (d, 1H), 7.80-7.72 (m, 3H), 3.03-2.89 (m, 2H), 1.50 (s, 6H).

LC-MS (Methode 3): $R_t$ = 2.41 min; 494 [M+H]$^+$.

**Beispiel 110A:**

7-Chlor-1-(2-chlor-4,6-difluorphenyl)-*N*-[(1*S*)-1-cyclopropyl-2,2,2-trifluorethyl]-4-oxo-1,4-dihydro-1,8-naphthyridin-3 -carbonsäureamid

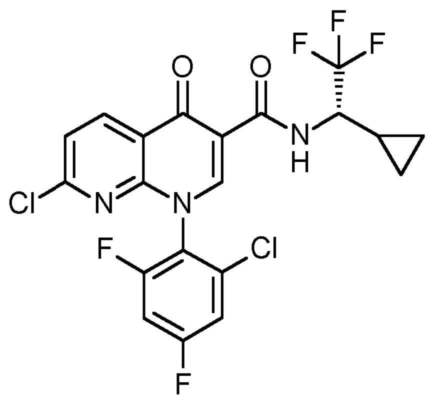

Gemäß AAV1 wurden 600 mg (1.62 mmol) 7-Chlor-1-(2-chlor-4,6-difluorphenyl)-4-oxo-1,4-dihydro-1,8-naphthyridin-3-carbonsäure mit 426 mg (2.43 mmol) (1*S*)-1-Cyclopropyl-2,2,2-trifluorethanamin Hydrochlorid in Gegenwart von 615 mg (1.62 mmol) HATU und 676 μl (3.88 mmol) *N*,*N*-Diisopropylethylamin in 16.2 ml Dimethylformamid umgesetzt. Die Reaktionsmischung wurde in eine Mischung aus 200 ml Wasser und 16 ml 1N wässriger Salzsäure eingerührt und der Niederschlag abgesaugt. Der Niederschlag wurde in wenig Dichlormethan aufgenommen und mittels Normalphasenchromatographie (Cyclohexan-Essigsäureethylester Gradient) gereinigt. Es wurden 633 mg (79% d. Th., 99% Reinheit) der Titelverbindung erhalten.

$^1$H NMR (400 MHz, DMSO-$d_6$) δ [ppm] = 10.05 (d, 1H), 9.11 (s, 1H), 8.78 (d, 1H), 7.81-7.72 (m, 3H), 4.46-4.32 (m, 1H), 1.30-1.19 (m, 1H), 0.71-0.53 (m, 3H), 0.40-0.29 (m, 1H).

LC-MS (Methode 1): $R_t$ = 1.23 min; 492 [M+H]$^+$.

**Beispiel 111A:**

7-Chlor-1-(2-chlor-4,6-difluorphenyl)-4-oxo-*N*-[(2*S*)-1,1,1-trifluorpropan-2-yl]-1,4-dihydro-1,8-naphthyridin-3 -carbonsäureamid

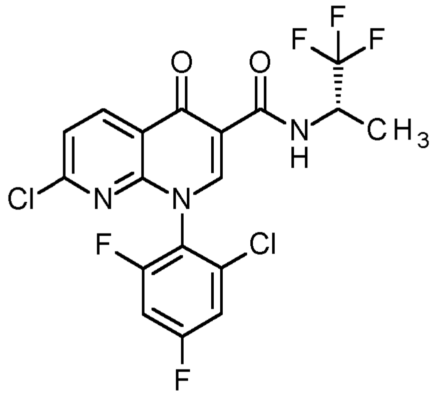

Gemäß AAV1 wurden 5.00 g (13.5 mmol) 7-Chlor-1-(2-chlor-4,6-difluorphenyl)-4-oxo-1,4-dihydro-1,8-naphthyridin-3-carbonsäure mit 1.83 g (16.2 mmol) (2*S*)-1,1,1-Trifluorpropan-2-amin Hydrochlorid in Gegenwart von 5.12 g (13.5 mmol) HATU und 5.63 ml (32.3 mmol) *N*,*N*-Diisopropylethylamin in 135 ml Dimethylformamid umgesetzt. Die Reaktionsmischung wurde in eine Mischung aus 600 ml Wasser und 135 ml 1N wässriger Salzsäure eingerührt und der Niederschlag abgesaugt. Der Niederschlag wurde in 12 ml Dichlormethan aufgenommen und mittels Normalphasenchromatographie (Cyclohexan-Essigsäureethylester Gradient) gereinigt. Es wurden 4.12 g (65% d. Th., 99% Reinheit) der Titelverbindung erhalten.

$^1$H NMR (400 MHz, DMSO-$d_6$) $\delta$ [ppm] = 9.94 (d, 1H), 9.11 (d, 1H), 8.76 (d, 1H), 7.81-7.72 (m, 3H), 4.98-4.86 (m, 1H), 1.42-1.38 (m, 3H).

LC-MS (Methode 1): $R_t$ = 1.21 min; 466 [M+H]$^+$.

**<u>Beispiel 112A:</u>**

7-Chlor-1-(2-chlor-4,6-difluorphenyl)-4-oxo-*N*-[(2S)-1-(trifluormethoxy)propan-2-yl]-1,4-dihydro-1,8-naphthyridin-3-carbonsäureamid

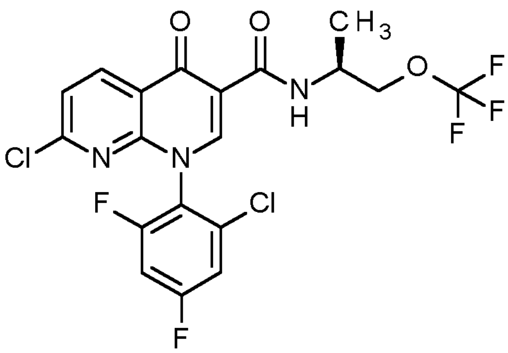

Gemäß AAV1 wurden 264 mg (704 $\mu$mol) 7-Chlor-1-(2-chlor-4,6-difluorphenyl)-4-oxo-1,4-dihydro-1,8-naphthyridin-3-carbonsäure mit 190 mg (1.06 mmol) (2*S*)-1-(Trifluormethoxy)propan-2-amin Hydrochlorid in Gegenwart von 268 mg (704 $\mu$mol) HATU und 294 $\mu$l (1.69 mmol) *N*,*N*-Diisopropylethylamin in 7 ml Dimethylformamid umgesetzt. Die Reaktionsmischung wurde in eine Mischung aus 42 ml Wasser und 6 ml 1N wässriger Salzsäure eingerührt und der Niederschlag abgesaugt. Der Niederschlag wurde in Dichlormethan aufgenommen, die Phasen getrennt und die organische Phase über Magnesiumsulfat getrocknet, filtriert und das Lösungsmittel unter reduziertem Druck entfernt. Der Rückstand wurde mittels Normalphasenchromatographie (Cyclohexan-Essigsäureethylester Gradient) gereinigt und 299 mg (86% d. Th., 100% Reinheit) der Titelverbindung erhalten.

$^1$H NMR (400 MHz, DMSO-$d_6$) $\delta$ [ppm] = 9.76-9.61 (m, 1H), 9.03 (s, 1H), 8.76 (d, 1H), 7.80-7.72 (m, 3H), 4.43-4.31 (m, 1H), 4.24-4.14 (m, 2H), 1.30-1.22 (m, 3H).

LC-MS (Methode 3): $R_t$ = 2.32 min; 496 [M+H]$^+$.

**Beispiel 113A:**

4-Oxo-7-(2-oxoimidazolidin-1-yl)-1-(2,4,6-trifluorphenyl)-1,4-dihydro-1,8-naphthyridin-3-carbonsäure

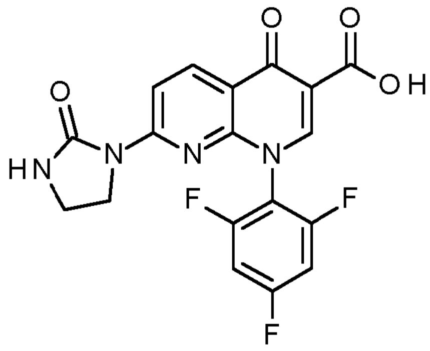

Gemäß AAV2 wurden 15.0 g (42.3 mmol) der Verbindung aus Beispiel 100B mit 25.5 g (296 mmol) Imidazolin-2-on in Gegenwart von 14.6 g (106 mmol) Kaliumcarbonat, 190 mg (846 μmol) Palladium(II)acetat und 979 mg (1.69 mmol) Xantphos in 400 ml 1,4-Dioxan umgesetzt. Es wurde für 2.5 h bei 90°C gerührt und anschließend auf RT abgekühlt. Die Suspension wurde in Wasser eingerührt und mit verdünnter wässriger Salzsäure auf pH2 eingestellt. Der Niederschlag wurde abgesaugt und mit Wasser gewaschen. Der Rückstand wurde in Acetonitril verrührt, abgesaugt, gewaschen und im Hochvakuum getrocknet. Es wurden 15.0 g (88% d. Th.) der Titelverbindung erhalten.
$^1$H-NMR (400 MHz, DMSO-$d_6$): δ [ppm] = 14.7 (s, 1H), 9.20 (s, 1H), 8.63-8.47 (m, 2H), 7.75 (s, 1H), 7.64-7.54 (m, 2H), 3.64-3.55 (m, 2H).
LC-MS (Methode 3): $R_t$ = 1.37 min; 405 $[M+H]^+$.

**Beispiel 114A:**

7-Chlor-1-(2,6-difluorphenyl)-4-oxo-*N*-[(2*S*)-1,1,1-trifluorbutan-2-yl]-1,4-dihydro-1,8-naphthyridin-3-carbonsäureamid

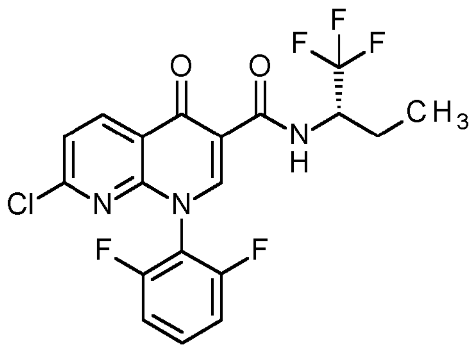

Gemäß AAV1 wurden 1.00 g (2.97 mmol) der Verbindung aus Beispiel 85A mit 566 mg (4.46 mmol) (2*S*)-1,1,1-Trifluorbutan-2-amin in Gegenwart von 1.13 g (2.97 mmol) HATU und 1.24 ml (7.13 mmol) *N*,*N*-Diisopropylethylamin in 30 ml Dimethylformamid umgesetzt. Die Reaktionsmischung wurde mit 20 ml Wasser und 30 ml Essigsäureethylester verdünnt und die Phasen getrennt. Die wässrige Phase wurde dreimal mit 10 ml Essigsäureethylester extrahiert und die vereinigten organischen Phasen mit 20 ml einer Mischung aus 1N wässriger Salzsäure und gesättigter wässriger Ammoniumchlorid-Lösung gewaschen. Anschließend wurde dreimal mit 15 ml gesättigter wässriger Natriumchlorid-Lösung gewaschen, über Natriumsulfat getrocknet, filtriert und das Lösungsmittel unter reduziertem Druck entfernt. Der Rückstand wurde in 10 ml Dichlormethan aufgenommen und mittels Normalphasenchromatographie (Cyclohexan-Essigsäureethylester Gradient) gereinigt. Es wurden 884 mg (66% d. Th., 99% Reinheit) der Titelverbindung erhalten.
$^1$H NMR (400 MHz, DMSO-*$d_6$*) δ [ppm] = 9.89 (d, 1H), 9.09 (s, 1H), 8.76 (d, 1H), 7.80-7.71 (m, 2H), 7.50-7.43 (m, 2H),

4.84-4.70 (m, 1H), 1.96-1.84 (m, 1H), 1.75-1.61 (m, 1H), 0.98 (t, 3H).
LC-MS (Methode 1): $R_t$ = 1.20 min; MS (ESIpos) m/z 446 $[M+H]^+$.

**Beispiel 115A:**

7-Chlor-1-(2,4,6-trifluorphenyl)-4-oxo-*N*-[(2*S*)-1,1,1-trifluorbutan-2-yl]-1,4-dihydro-1,8-naphthyridin-3-carbonsäureamid

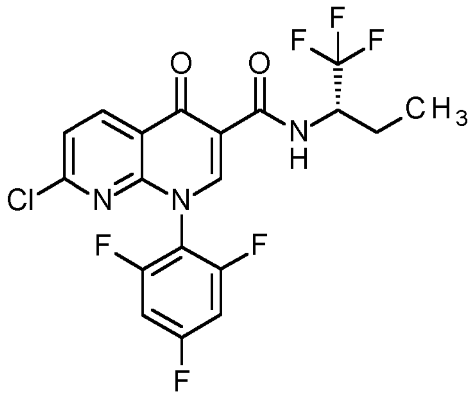

Gemäß AAV1 wurden 2.00 g (5.64 mmol) aus Beispiel 100B mit 1.11 g (6.77 mmol) (2*S*)-1,1,1-Trifluorbutan-2-amin in Gegenwart von 2.14 g (5.64 mmol) HATU und 3.34 ml (19.2 mmol) *N*,*N*-Diisopropylethylamin in 57 ml Dimethylformamid umgesetzt. Die Reaktionsmischung wurde mit 75 ml Wasser und 100 ml Essigsäureethylester verdünnt und die Phasen getrennt. Die wässrige Phase wurde dreimal mit 50 ml Essigsäureethylester extrahiert und die vereinigten organischen Phasen mit 60 ml einer Mischung aus 1N wässriger Salzsäure und gesättigter wässriger Ammoniumchlorid-Lösung gewaschen. Anschließend wurde dreimal mit 20 ml gesättigter wässriger Natriumchlorid-Lösung gewaschen, über Magnesiumsulfat getrocknet, filtriert und das Lösungsmittel unter reduziertem Druck entfernt. Der Rückstand wurde in Dichlormethan aufgenommen und mittels Normalphasenchromatographie (Cyclohexan-Essigsäureethylester Gradient) gereinigt. Es wurden 1.28 g (49% d. Th., 100% Reinheit) der Titelverbindung erhalten.
$^1H$ NMR (400 MHz, DMSO-$d_6$) δ [ppm] = 9.88 (d, 1H), 9.13 (s, 1H), 8.75 (d, 1H), 7.78 (m, 1H), 7.66-7.58 (m, 2H), 4.84-4.71 (m, 1H), 1.96-1.84 (m, 1H), 1.74-1.62 (m, 1H), 0.98 (t, 3H).
LC-MS (Methode 3): $R_t$ = 2.30 min; MS (ESIpos) m/z 464 $[M+H]^+$.

**Beispiel 116A:**

(3*S*,5*R*)-3,5-Dimethylpyrrolidin-3-ol Trifluoressigsäure

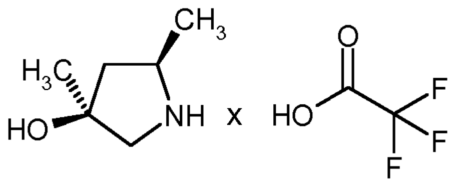

Eine Lösung von 100 mg (464 μmol) *tert.*-Butyl-(2*R*,4*S*)-4-hydroxy-2,4-dimethylpyrrolidin-1-carbamat in 1.5 ml Dichlormethan wurde mit 500 μl (6.49 mmol) Trifluoressigsäure versetzt und für 2 h bei Raumtemperatur gerührt. Das Lösungsmittel wurde unter reduziertem Druck entfernt und der Rückstand dreimal mit 5 ml Dichlormethan coevapuriert. Es wurden 98.6 mg (88%, d. Th. 95% Reinheit) der Titelverbindung erhalten.
$^1H$ NMR (400 MHz, DMSO-$d_6$) δ [ppm] = 9.10 (br. s, 1H), 8.68 (br. s, 1H), 5.22 (br. s, 1H), 3.76-3.63 (m, 1H), 3.10-3.03 (m, 1H), 3.00-2.91 (m, 1H), 2.12 (dd, 1H), 1.62 (ddd, 1H), 1.34 (d, 3H), 1.32 (s, 3H).

**Beispiel 117A:**

7-[(4*S*)-4-Hydroxy-2-oxopyrrolidin-1-yl]-4-oxo-1-(2,4,6-trifluorphenyl)-1,4-dihydro-1,8-naphthyridin-3-carbonsäure

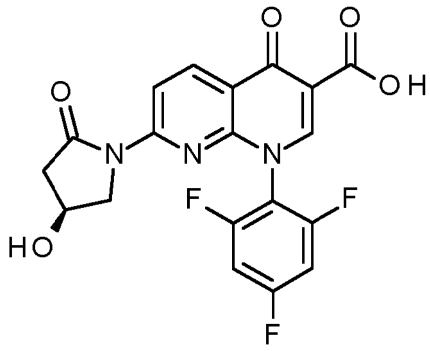

Gemäß AAV2 wurden 50.0 g (141 mmol) der Verbindung aus Beispiel 100B mit 17.1 g (169 mmol) (4*S*)-4-Hydroxypyrolidin-2-on in Gegenwart von 29.2 g (211 mmol) Kaliumcarbonat, 6.33 g (28.2 mmol) Palladium(II)acetat und 16.3 g (28.2 mmol) Xantphos in 1000 ml 1,4-Dioxan bei 80°C für 1.5h umgesetzt. Der Ansatz wurde abgekühlt und auf eine Mischung aus Eiswasser, Salzsäure und Essigsäureethylester ausgerührt. Es wurde über Kieselgur abgesaugt und die organische Phase mit Wasser und gesättigter wässriger Natriumchlorid-Lösung gewaschen, getrocknet und abschließend eingeengt. Der Rückstand wurde mit Acetonitril versetzt, gekühlt, abgesaugt und der Niederschlag mit kaltem Acetonitril gewaschen. Es wurden 48 g (81% d. Th., 97% Reinheit) der Titelverbindung erhalten.
LC-MS (Methode 3): $R_t$ = 1.33 min; MS (ESIpos) m/z 420 $[M+H]^+$.

**Beispiel 118A:**

7-Chlor-1-(2,4,6-trifluorphenyl)-4-oxo-*N*-[(2*S*)-1-(trifluormethoxy)propan-2-yl]-1,4-dihydro-1,8-naphthyridin-3 -carbonsäureamid

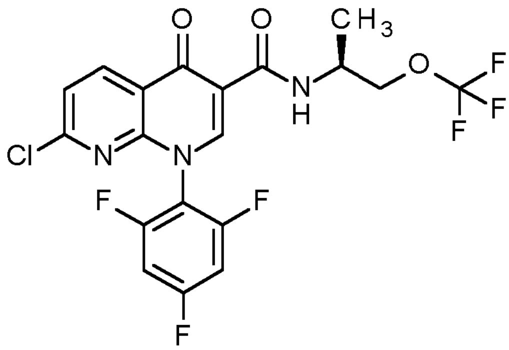

Gemäß AAV1 wurden 250 mg (698 μmol) der Verbindung aus Beispiel 100B mit 188 mg (1.05 mmol) (2*S*)-1-(Trifluormethoxy)propan-2-amin Hydrochlorid in Gegenwart von 265 mg (698 μmol) HATU und 292 μl (1.68 mmol) *N*,*N*-Diisopropylethylamin in 7 ml Dimethylformamid umgesetzt. Die Reaktionsmischung wurde in eine Mischung aus 42 ml Wasser und 6 ml 1N wässriger Salzsäure eingerührt und der Niederschlag abgesaugt. Der Niederschlag wurde in Dichlormethan aufgenommen, die Phasen getrennt und die organische Phase über Magnesiumsulfat getrocknet, filtriert und das Lösungsmittel unter reduziertem Druck entfernt. Der Rückstand wurde mittels Normalphasenchromatographie (Cyclohexan-Essigsäureethylester Gradient) gereinigt und 226 mg (68% d. Th., 100% Reinheit) der Titelverbindung erhalten.
$^1$H NMR (400 MHz, DMSO-$d_6$) δ [ppm] = 9.63 (d, 1H), 9.06 (s, 1H), 8.74 (d, 1H), 7.76 (d, 1H), 7.65-7.57 (m, 2H), 4.43-4.32 (m, 1H), 4.24-4.15 (m, 2H), 1.26 (d, 3H).

LC-MS (Methode 1): $R_t$ = 1.21 min; MS (ESIpos) m/z 480 $[M+H]^+$.

**Beispiel 119A:**

7-Chlor-1-(2-chlor-6-fluorphenyl)-4-oxo-*N*-[(2*S*)-1,1,1-trifluorbutan-2-yl]-1,4-dihydro-1,8-naphthyridin-3 -carbonsäureamid

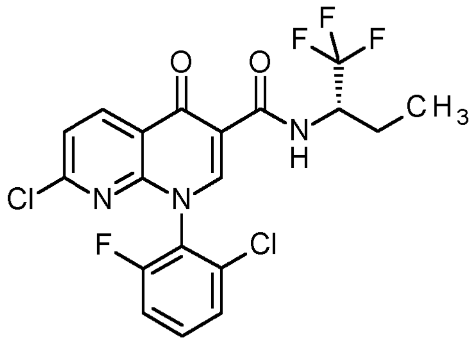

Gemäß AAV1 wurden 500 mg (1.42 mmol) der Verbindung aus Beispiel 105B mit 347 mg (2.12 mmol) (2*S*)-1,1,1-Trifluorbutan-2-amin Hydrochlorid in Gegenwart von 538 mg (1.42 mmol) HATU und 592 µl (3.40 mmol) *N*,*N*-Diisopropylethylamin in 14.2 ml Dimethylformamid umgesetzt. Die Reaktionsmischung wurde in eine Lösung aus 50 ml Wasser und 15 ml 1N wässriger Salzsäure eingerührt und der Niederschlag abgesaugt, mit Wasser gewaschen und getrocknet. Der Niederschlag in 10 ml Dichlormethan gelöst und mittels Normalphasenchromatographie (Cyclohexan-Essigsäureethylester Gradient) gereinigt. Es wurden 496 mg (75% d. Th., 99% Reinheit) der Titelverbindung erhalten.
$^1H$ NMR (400 MHz, DMSO-$d_6$) δ [ppm] = 9.90 (d, 1H), 9.07 (s, 1H), 8.77 (d, 1H), 7.81-7.56 (m, 4H), 4.84-4.70 (m, 1H), 1.96-1.83 (m, 1H), 1.76-1.62 (m, 1H), 1.02-0.94 (m, 3H).
LC-MS (Methode 3): $R_t$ = 2.33 min; 462 $[M+H]^+$.

**Beispiel 120A:**

7-Chlor-1-(2-chlor-6-fluorphenyl)-*N*-[(1*S*)-1-cyclopropyl-2,2,2-trifluorethyl]-4-oxo-1,4-dihydro-1,8-naphthyridin-3-carbonsäureamid

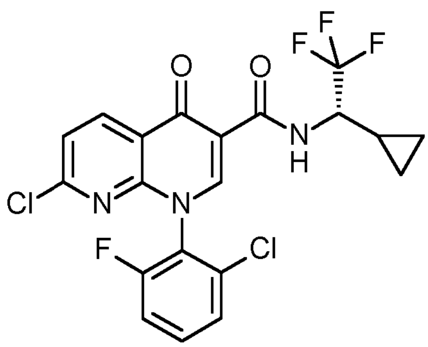

Gemäß AAV1 wurden 250 mg (708 µmol) der Verbindung aus Beispiel 105B mit 186 mg (1.06 mmol) (1S)-1-Cyclopropyl-2,2,2-trifluorethanamin Hydrochlorid in Gegenwart von 269 mg (708 µmol) HATU und 296 µl (1.70 mmol) *N*,*N*-Diisopropylethylamin in 7.1 ml Dimethylformamid umgesetzt. Die Reaktionsmischung wurde in eine Lösung aus 25 ml Wasser und 8 ml 1N wässriger Salzsäure eingerührt und der Niederschlag abgesaugt, mit Wasser gewaschen und getrocknet. Der Niederschlag in 8 ml Dichlormethan gelöst und mittels Normalphasenchromatographie (Cyclohexan-Essigsäureethylester Gradient) gereinigt. Es wurden 250 mg (74% d. Th., 99% Reinheit) der Titelverbindung erhalten.
$^1H$ NMR (400 MHz, DMSO-$d_6$) δ [ppm] = 10.06 (d, 1H), 9.06 (s, 1H), 8.78 (d, 1H), 7.79 (d, 1H), 7.77-7.57 (m, 3H), 4.45-4.32 (m, 1H), 1.31-1.20 (m, 1H), 0.73-0.53 (m, 3H), 0.40-0.30 (m, 1H).

LC-MS (Methode 3): $R_t$ = 2.35 min; 474 $[M+H]^+$.

**Beispiel 121A:**

7-[(3*R*,4*R*)-3,4-Dihydroxypyrrolidin-1-yl]-4-oxo-1-(2,4,6-trifluorphenyl)-1,4-dihydro-1,8-naphthyridin-3-carbonsäure

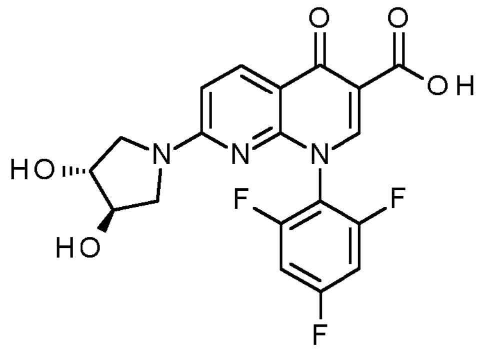

Gemäß AAV3 wurden 10.0 g (28.2 mmol) der Verbindung aus Beispiel 100B mit 4.46 g (31.0 mmol, 97% Reinheit) (3*R*,4*R*)-Pyrrolidin-3,4-diol Hydrochlorid und 17.2 ml (98.7 mmol) *N*,*N*-Diisopropylethylamin in 150 ml Dimethylformamid zur Reaktion gebracht. Es wurde mit 350 ml Wasser, 150 ml 1N wässriger Salzsäure und 250 ml Essigsäureethylester verdünnt. Die Phasen wurden getrennt und die wässrige Phase dreimal mit 250 ml Essigsäureethylester extrahiert. Die vereinigten organischen Phasen wurden zweimal mit 250 ml Phosphat-Pufferlösung (3.52 g Kaliumdihydrogenphosphat, 7.26 g Dinatriumhydrogenphosphat Dihydrat in 1000 ml Wasser, pH 7) und 250 ml gesättigter wässriger Natriumchlorid-Lsg. gewaschen, über Magnesiumsulfat getrocknet, filtriert und auf ein Volumen von ungefähr 100 ml eingeengt. Es wurde langsam 250ml *tert*.-Butylmethylether unter Rühren zugetropft. Der Niederschlag wurde abgesaugt und am Hochvakuum getrocknet. Es wurden 10.8 g (91% d. Th., 100% nach LC-MS) der Titelverbindung erhalten. Nach NMR enthielt das Produkt noch Spuren Essigsäureethylester, wurde aber ohne weitere Aufreinigung eingesetzt.
$^1H$ NMR (400 MHz, DMSO-$d_6$) $\delta$ [ppm] = 15.23 (s, 1H), 9.02 (s, 1H), 8.30 (d, 1H), 7.62-7.54 (m, 2H), 6.87 (d, 1H), 5.26 (d, 1H), 5.17 (d, 1H), 4.06 (br. s, 1H, teilweise unter einer Resonanz von Essigsäureethylester), 3.94 (br. s, 1H), 3.64 (dd, 1H), 3.37 (d, 1H), 3.27 (dd, 1H), 3.08 (d, 1H).
LC-MS (Methode 3): $R_t$ = 1.68 min; m/z = 563 $[M+H]^+$.

**Beispiel 122A:**

1-(2-{[*tert*.-Butyl(dimethyl)silyl]oxy}ethyl)imidazolidin-2-on

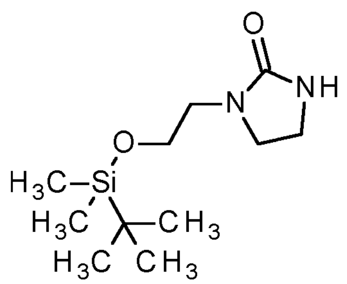

Zu einer Lösung von 1.00 g (7.68 mmol) 1-(2-Hydroxyethyl)imidazolidin-2-on (CAS: 3699-54-5) und 628 mg (9.22 mmol) Imidazol in 7.75 ml Dimethylformamid bei 0°C wurden 1.27 g (8.45 mmol) *tert.* Butyldimethylsilylchlorid gegeben und über Nacht bei Raumtemperatur gerührt. Alle flüchtigen Bestandteile wurden unter reduziertem Druck entfernt und der Rückstand mit 10 ml Wasser versetzt. Es wurde dreimal mit 20 ml Essigsäureethylester extrahiert. Die vereinigten organischen Phasen wurden mit gesättigter wässriger Natriumchlorid-Lösung gewaschen, über Magnesiumsulfat getrocknet, filtriert und das Lösungsmittel unter reduziertem Druck entfernt. Es wurden 1.24 g (66% d. Th.,

100% Reinheit) der Titelverbindung erhalten.
$^1H$ NMR (400 MHz, DMSO-$d_6$) δ [ppm] = 6.24 (br. s, 1H), 3.64 (t, 2H), 3.41-3.36 (m, 2H), 3.22-3.17 (m, 2H), 3.11 (t, 2H), 0.86 (s, 9H), 0.04 (s, 6H).
LC-MS (Methode 3): $R_t$ = 1.78 min; m/z = 245 $[M+H]^+$.

**Beispiel 123A:**

7-[3-(2-Hydroxyethyl)-2-oxoimidazolidin-1-yl]-4-oxo-1-(2,4,6-trifluorphenyl)-1,4-dihydro-1,8-naphthyridin-3 -carbonsäure

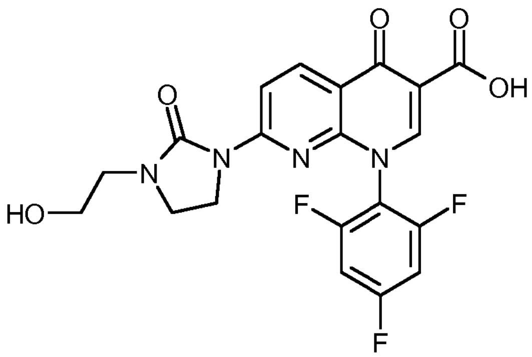

Gemäß allgemeiner Arbeitsvorschrift 2 wurden 250 mg (705 μmol) der Verbindung aus Beispiel 100B mit 190 mg (775 μmol) der Verbindung aus Beispiel 122A in Gegenwart von 244 mg (1.76 mmol) Kaliumcarbonat, 7.9 mg (35 μmol) Palladiumacetat und 41 mg (70 μmol) Xantphos in 10 ml Dioxan für 90 min bei 90°C zur Reaktion gebracht. Die Reaktionsmischung wurde auf 15 ml wässrige 1N Salzsäure und 15 ml gesättigte wässrige Natriumchlorid-Lösung gegossen und nachgerührt. Es wurde zweimal mit 50 ml Essigsäureethylester extrahiert und die vereinigten organischen Phasen eingeengt. Der Rückstand wurde in drei Läufen mittels präp. HPLC (Säule: Chromatorex C18, 10 μm, 125x30 mm, LM: Acetonitril / 0.1 % Ameisensäure-Gradient; 0 bis 5 min. 10% Acetonitril, während 14 min. nach 90% Acetonitril und weitere 4 min. 90% Acetonitril) gereinigt. Die gesammelten Fraktionen des silylierten Intermediats wurden erneut mit 10 ml wässriger 1N Salzsäure versetzt und 30 min bei 40°C gerührt. Anschließend wurde eingeengt und der Rückstand in drei Läufen mittels präp. HPLC (Säule: Chromatorex C18, 10 μm, 125x30 mm, LM: Acetonitril / 0.1 % Ameisensäure-Gradient; 0 bis 5 min. 10% Acetonitril, während 14 min. nach 90% Acetonitril und weitere 4 min. 90% Acetonitril) getrennt. Die Produktfraktionen wurden vereinigt und 269 mg (85% d. Th., 100% Reinheit) der Titelverbindung erhalten.
$^1H$ NMR (400 MHz, DMSO-d6): δ [ppm] = 14.66 (br. s, 1H), 9.20 (s, 1H), 8.61 (d, 1H), 8.51 (d, 1H), 7.64-7.56 (m, 2H), 4.75 (br. s, 1H), 3.58-3.46 (m, 6H), 3.28 (t, 2H).
LC-MS (Methode 3): $R_t$ = 1.18 min; MS (ESIpos): m/z = 449 $[M+H]^+$.

**Beispiel 124A:**

1-(2-{[*tert.*-Butyl(dimethyl)silyl]oxy}ethyl)tetrahydropyrimidin-2(1*H*)-on

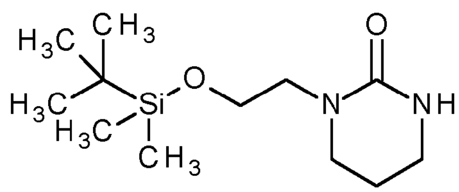

Zu einer Lösung von 600 mg (4.16 mmol) 1-(2-Hydroxyethyl)tetrahydropyrimidin-2(1*H*)-on (DE1121617, 1962) und 690 mg (4.58 mmol) *tert.*-Butyl(chlor)dimethylsilan in 4.2 ml DMF wurde bei 0°C 340 mg (4.99 mmol) Imidazol gegeben. Es wurde für 30 min bei 0°C und über Nacht bei RT nachge-rührt. Anschließend wurden alle flüchtigen Bestandteile unter reduziertem Druck entfernt und der Rückstand mit 10 ml Wasser versetzt und dreimal mit 20 ml

Essigsäureethylester extrahiert. Die vereinigten organischen Phasen wurden mit 30 ml gesättigter wässriger Natriumchlorid-Lösung gewaschen, mit Magnesiumsulfat getrocknet, filtriert und das Lösungsmittel unter reduziertem Druck entfernt. Es wurden 732 mg (68% d. Th., 100% Reinheit) der Titelverbindung erhalten.
$^1$H NMR (400 MHz, DMSO-d6): δ [ppm] = 6.11 (s, 1H), 3.63 (t, 2H), 3.30-3.21 (m, 4H), 3.11-3.04 (m, 2H), 1.80-1.72 (m, 2H), 0.86 (s, 9H), 0.03 (s, 6H).
LC-MS (Methode 3): $R_t$ = 1.83 min; MS (ESIpos): m/z = 259 $[M+H]^+$.

**Beispiel 125A:**

7-[3-(2-Hydroxyethyl)-2-oxotetrahydropyrimidin-1(2*H*)-yl]-4-oxo-1-(2,4,6-trifluorphenyl)-1,4-dihydro-1,8-naphthyridin-3-carbonsäure

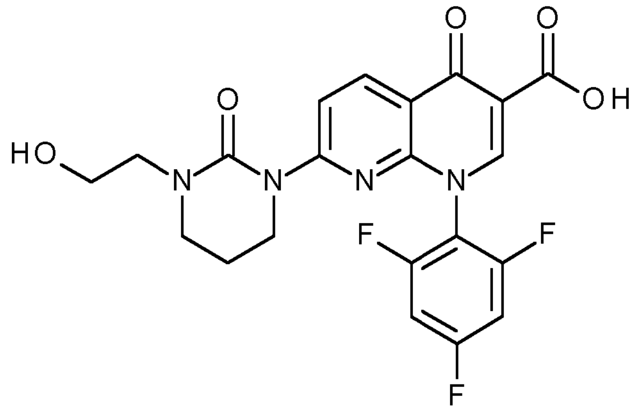

Gemäß allgemeiner Arbeitsvorschrift 2 wurden 250 mg (705 μmol) der Verbindung aus Beispiel 100B mit 166 mg (641 μmol) der Verbindung aus Beispiel 124A in Gegenwart von 221 mg (1.60 mmol) Kaliumcarbonat, 7.2 mg (32 μmol) Palladiumacetat und 37 mg (64 μmol) Xantphos in 6.4 ml Dioxan für 90 min bei 90°C zur Reaktion gebracht. Die Reaktionsmischung wurde mit 15 ml Dioxan verdünnt, auf 15 ml wässrige 1N Salzsäure und 15 ml gesättigte wässrige Natriumchlorid-Lösung gegegossen und bei 40°C nachgerührt. Es wurde dreimal mit 30 ml Essigsäureethylester extrahiert und die vereinigten organischen Phasen eingeengt. Der Rückstand wurde in zwei Läufen mittels präp. HPLC (Säule: Chromatorex C18, 10 μm, 125x30 mm, LM: Acetonitril / 0.1 % Ameisensäure-Gradient; 0 bis 5 min. 10% Acetonitril, während 14 min. nach 90% Acetonitril und weitere 4 min. 90% Acetonitril) gereinigt. Es wurden 110 mg (26% d. Th., 78% Reinheit) der Titelverbindung erhalten.
$^1$H NMR (400 MHz, DMSO-d6): δ [ppm] = 14.61 (br. s, 1H), 9.22 (s, 1H), 8.55 (d, 1H), 8.24 (d, 1H), 7.65-7.57 (m, 2H), 4.71 (t, 1H), 3.58-3.48 (m, 4H), 3.42-3.36 (m, 4H), 1.95-1.86 (m, 2H).
LC-MS (Methode 3): $R_t$ = 1.36 min; MS (ESIpos): m/z = 463 $[M+H]^+$.

**Beispiel 126A:**

7-Chlor-*N*-[(1*S*)-1-cyclopropyl-2,2,2-trifluorethyl]-4-oxo-1-(2,4,6-trifluorphenyl)-1,4-dihydro-1,8-naphthyridin-3 -carbonsäureamid

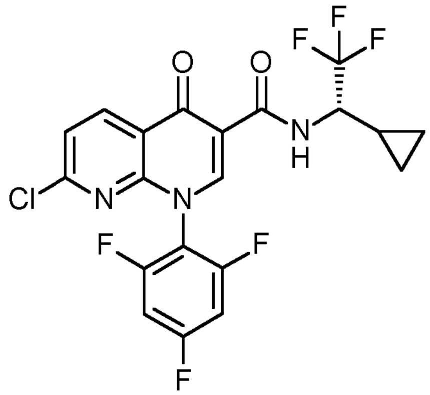

Gemäß AAV1 wurden 2.00 g (5.64 mmol) aus Beispiel 100B mit 1.09 g (6.20 mmol) (1*S*)-1-Cyclopropyl-2,2,2-trifluorethanamin Hydrochlorid in Gegenwart von 2.14 g (5.64 mmol) HATU und 2.36 ml (13.5 mmol) *N,N*-Diisopropylethylamin in 50 ml Dimethylformamid umgesetzt. Es wurde für 5 min bei Raumtemperatur nachgerührt und anschließend die Reaktionslösung auf 20 ml Wasser gegossen. Es wurden 3 ml 1N wässrige Salzsäure hinzugegeben, der Niederschlag abgesaugt und mit Wasser gewaschen. Der Rückstand wurde in 10 ml Dichlormethan aufgenommen und mittels Normalphasenchromatographie (Cyclohexan-Essigsäureethylester Gradient) gereinigt. Es wurden 1.82 g (68% d. Th., 100% Reinheit) der Titelverbindung erhalten.
$^1$H NMR (400 MHz, DMSO-$d_6$) $\delta$ [ppm] = 10.04 (d, 1H), 9.13 (s, 1H), 8.76 (d, 1H), 7.79 (d, 1H), 7.65-7.58 (m, 2H), 4.46-4.33 (m, 1H), 1.30-1.19 (m, 1H), 0.73-0.52 (m, 3H), 0.38-0.29 (m, 1H).
LC-MS (Methode 3): $R_t$ = 2.31 min; MS (ESIpos) m/z 476 $[M+H]^+$.

**Beispiel 127A:**

7-(5-Benzyl-1,1-dioxido-1,2,5-thiadiazolidin-2-yl)-*N*-[(1*S*)-1-cyclopropyl-2,2,2-trifluoroethyl]-4-oxo-1-(2,4,6-trifluorophenyl)-1,4-dihydro-1,8-naphthyridin-3-carbonsäureamid

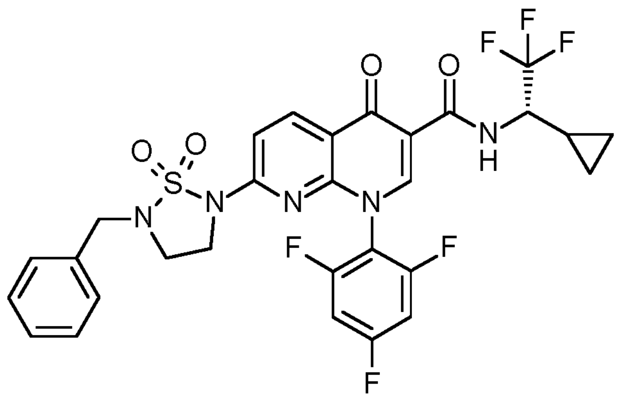

Gemäß AAV2 wurden 100 mg (210 μmol) der Verbindung aus Beispiel 126A mit 53.5 mg (252 μmol) 2-Benzyl-1,2,5-thiadiazolidin 1,1-dioxid in Gegenwart von 43.6 mg (315 μmol) Kaliumcarbonat, 4.7 mg (21 μmol) Palladium(II)acetat und 24 mg (42 μmol) Xantphos in 1.5 ml 1,4-Dioxan zur Reaktion gebracht. Anschließend wurde das Volumen der Mischung unter reduziertem Druck eingeengt, der Rückstand mit 1 ml wässriger 1N Salzsäure und 1 ml Acetonitril aufgenommen und mittels präparativer HPLC (Säule: Chromatorex C18, 10 μm, 125x30 mm, LM: Acetonitril / 0.1 % Ameisensäure-Gradient; 0 bis 5 min. 10% Acetonitril, während 14 min. nach 90% Acetonitril und weitere 4 min. 90% Acetonitril) getrennt. Die Produktfraktionen wurden vereinigt und 104 mg (75% d. Th., 98.5% Reinheit) der Titelverbindung erhalten.
LC-MS (Methode 3): $R_t$ = 2.39 min; MS (ESIpos) m/z 652 $[M+H]^+$.

**Beispiel 128A:**

7-[(2-Aminoethyl)amino]-4-oxo-*N*-[(2*S*)-1,1,1-trifluorbutan-2-yl]-1-(2,4,6-trifluorphenyl)-1,4-dihydro-1,8-naphthyridin-3-carbonsäureamid Hydrochlorid

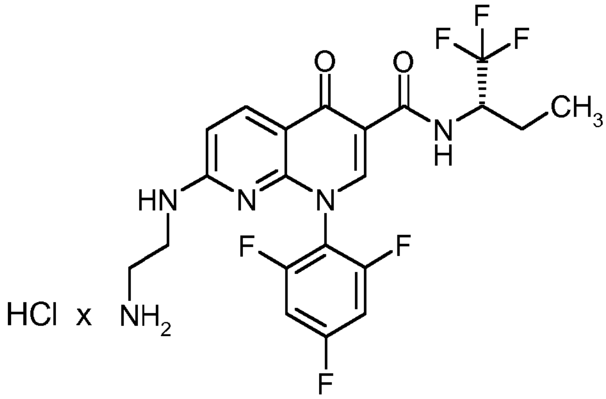

Zu einer Lösung von 500 mg (1.08 mmol) der Verbindung aus Beispiel 115A in 11 ml Dimethylformamid wurden 1.44 ml (21.6 mmol) Ethan-1,2-diamin gegeben. Es wurde für 45 min bei Raumtemperatur nachgerührt. Der Ansatz wurde einrotiert und mit 6 ml wässriger Salzsäure und 4 ml Acetonitril aufgenommen und in zwei Läufen mittels präparativer HPLC (Säule: Chromatorex C18, 10 μm, 125x30 mm, Laufmittel: Acetonitril / 0.1% Ameisensäure-Gradient; 0 bis 5 min. 10% Acetonitril, während 14 min. nach 90% Acetonitril und weitere 4 min. 90% Acetonitril) gereinigt. Es wurden 426 mg (81% d. Th., 100% Reinheit) der Titelverbindung erhalten. Die Ausbeute bezieht sich auf das freie Amin.
$^1$H NMR (400 MHz, DMSO-$d_6$) δ [ppm] = 10.44 (d, 1H), 8.79 (s, 1H), 8.38 (br. s, 1H), 8.30 (s, 1H), 8.19 (d, 1H), 7.58-7.50 (m, 2H), 6.73 (d, 1H), 4.80-4.66 (m, 1H), 3.15-3.03 (m, 2H), 2.71-2.59 (m, 2H), 1.95-1.80 (m, 1H), 1.71-1.56 (m, 1H), 0.96 (t, 3H).
LC-MS (Methode 1): $R_t$ = 0.67 min; MS (ESIpos) m/z 488 $[M+H]^+$.

**Beispiel 129A:**

7-({(2*R*)-2-[(*tert*.-Butoxycarbonyl)amino]propyl}amino)-4-oxo-1-(2,4,6-trifluorphenyl)-1,4-dihydro-1,8-naphthyridin-3-carbonsäureethylester

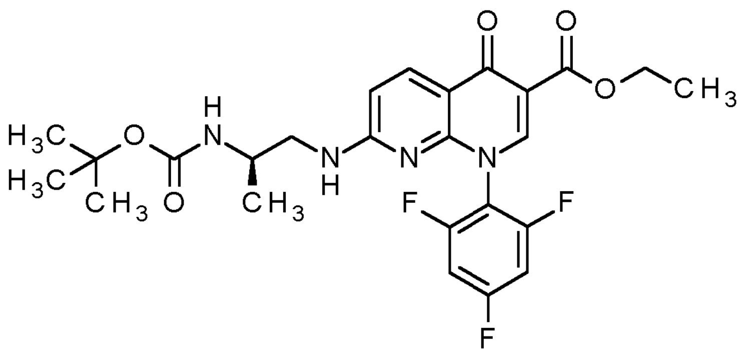

Zu einer Lösung von 1.50 g (3.92 mmol) der Verbindung aus Beispiel 100A in 25 ml Dimethylformamidwurde nacheinander 991 mg (4.70 mmol) tert.-Butyl [(2*R*)-1-aminopropan-2-yl]carbamat Hydrochlorid und 2.39 ml (13.7 mmol) *N,N*-Diisopropylethylamin gegeben. Es wurde über Nacht bei Raumtemperatur und 37h bei 60°C nachgerührt. Anschließend wurde die Reaktionslösung auf 250 ml Wasser gegossen und mit wässriger 1N Salzsäure auf pH 5 eingestellt. Der Niederschlag wurde abgesaugt, mit Wasser gewaschen und im Hochvakuum getrocknet. Es wurden 1.81 g (85% d. Th., 95% Reinheit) der Titelverbindung erhalten.

LC-MS (Methode 3): $R_t$ = 1.82 min; MS (ESIpos) m/z 521 $[M+H]^+$.

**Beispiel 129B:**

7-{[(2*R*)-2-Aminopropyl]amino}-4-oxo-1-(2,4,6-tnfluorphenyl)-1,4-dihydro-1,8-naphthyridin-3-carbonsäureethylester Trifluoracetat

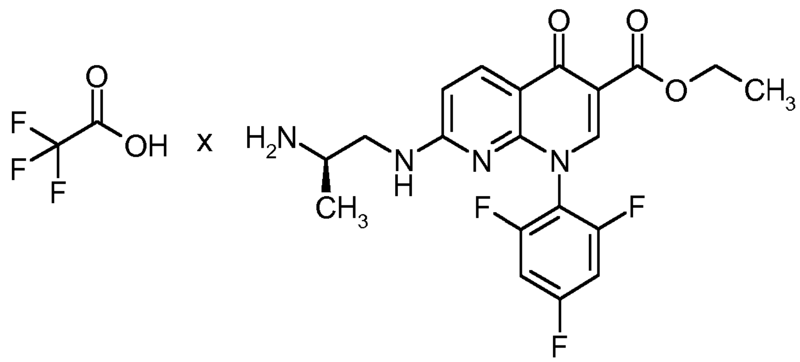

Zu einer Lösung von 1.80 g (3.46 mmol) der Verbindung aus Beispiel 129A in 100 ml Dichlormethan wurden 5.33 ml (69.2 mmol) Trifluoressigsäure gegeben. Es wurde für 2.5h bei Raumtemperatur nachgerührt. Anschließend wurden alle flüchtigen Bestandteile unter reduziertem Druck entfernt und der Rückstand mit Toluol codestilliert und lyophilisiert. Es wurden 2.50 g (quantitativ, 99% Reinheit) der Titelverbindung erhalten.
LC-MS (Methode 3): $R_t$ = 0.92 min; MS (ESIpos) m/z 421 $[M+H]^+$.

**Beispiel 129C:**

7-[(4*R*)-4-Methyl-2-oxoimidazolidin-1-yl]-4-oxo-1-(2,4,6-trifluorphenyl)-1,4-dihydro-1,8-naphthyridin-3-carbonsäureethylester

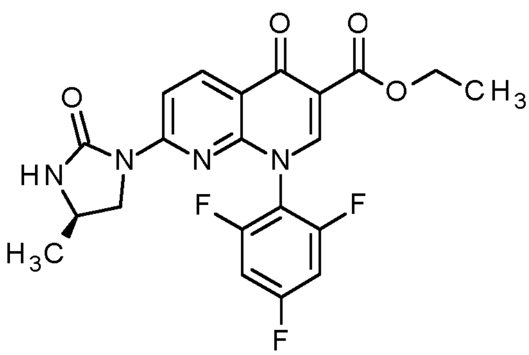

Zu einer Lösung von 2.50 g (4.68 mmol) der Verbindung aus Beispiel 129B in 103 ml Dimethylformamid wurden nacheinander 647 mg (4.68 mmol) Kaliumcarbonat und 1.90 g (11.7 mmol) 1,1'-Carbonyldiimidazol gegeben. Es wurde für 6h bei Raumtemperatur nachgerührt. Die Reaktionslösung wurde auf 600 ml Wasser gegossen und mit 5 ml wässriger 1N Salzsäure versetzt. Der Niederschlag wurde abgesaugt, mit Wasser gewaschen und im Hochvakuum getrocknet. Es wurden 1.20 g (59% d. Th., 99% Reinheit) der Titelverbindung erhalten.
$^1$H NMR (400 MHz, DMSO-$d_6$) $\delta$ [ppm] = 8.79 (s, 1H), 8.44 (d, 1H), 8.34 (d, 1H), 7.76 (s, 1H), 7.63-7.53 (m, 2H), 4.23 (q, 2H), 3.80-3.67 (m, 2H), 3.12-3.02 (m, 1H), 1.28 (t, 3H), 1.12 (d, 3H).
LC-MS (Methode 1): $R_t$ = 0.86 min; MS (ESIpos) m/z 447 $[M+H]^+$.

**Beispiel 129D:**

7-[(4*R*)-4-Methyl-2-oxoimidazolidin-1-yl]-4-oxo-1-(2,4,6-trifluorphenyl)-1,4-dihydro-1,8-naphthyridin-3-carbonsäure

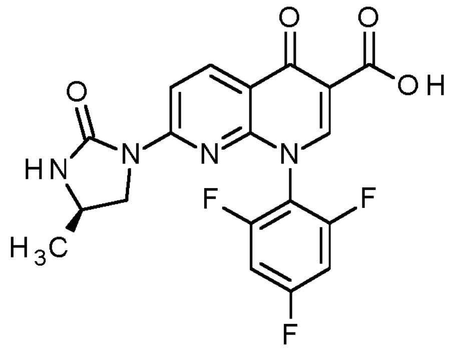

1.19 g (2.67 mmol) der Verbindung aus Beispiel 129C wurden in 8 ml Wasser vorgelegt, mit 8 ml 36 proz. wässriger Salzsäure und 8 ml THF versetzt und 3h bei 110°C nachgerührt. Das Reaktionsgemisch wurde auf RT abgekühlt und mit 100 ml Wasser versetzt. Der Niederschlag wurde abgesaugt, mit Wasser nachgewaschen und am Hochvakuum getrocknet. Es wurden 994 mg (87% d. Th., 97% Reinheit) der Titelverbindung erhalten.
LC-MS (Methode 3): $R_t$ = 1.49 min; MS (ESIpos): m/z = 419 $[M+H]^+$.
$^1$H NMR (400 MHz, DMSO-d6): $\delta$ [ppm] = 14.65 (s, 1H), 9.19 (s, 1H), 8.60 (d, 1H), 8.50 (d, 1H), 7.91 (s, 1H), 7.65-7.55 (m, 2H), 3.81-3.70 (m, 2H), 3.13-3.07 (m, 1H), 1.13 (d, 3H).

**Beispiel 130A:**

7-Chlor-1-(2,6-dichlor-4-fluorphenyl)-4-oxo-1,4-dihydro-1,8-naphthyridin-3-carbonsäureethylester

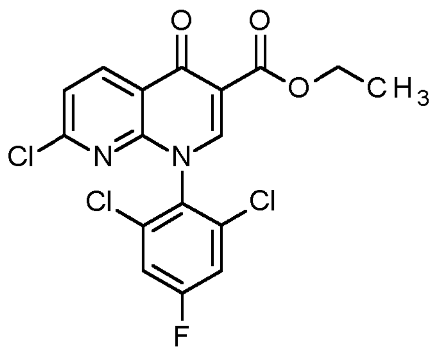

Eine Lösung von 6.07 g (19.1 mmol) 2-[(2,6-Dichlorpyridin-3-yl)carbonyl]-3-ethoxyacryl-säureethylester (CAS 157373-27-8) und 4.81 g (26.7 mmol) 2,6-Dichlor-4-fluoranilin in 30 ml DCM wurde mit 23.3 ml (134 mmol) DIPEA versetzt und 4h bei RT gerührt. Anschließend wurden 2.64 g (19.1 mmol) Kaliumcarbonat zugegeben und über Nacht unter Rückfluss erhitzt. Es wurde mit 200 ml DCM verdünnt und zweimal mit 75 ml 1M wässriger Salzsäure gewaschen. Die organische Phase wurde über Natriumsulfat getrocknet, filtriert und das Lösungsmittel unter reduziertem Druck entfernt. Die erhaltene Suspension wurde mit 40 ml *tert.*-Butylmethylether verrührt, der Niederschlag abgesaugt, mit 10 ml *tert.*-Butylmethylether gewaschen und im Hochvakuum getrocknet. Es wurden 3.81 g (45% d. Th., 94% Reinheit) der Titelverbindung erhalten.
LC-MS (Methode 1): $R_t$ = 1.04 min; MS (ESIpos) m/z 415 $[M+H]^+$.
$^1$H NMR (400 MHz, DMSO-d6): $\delta$ [ppm] = 8.88 (s, 1H), 8.65 (d, 1H), 7.92 (d, 2H), 7.69 (d, 1H), 4.25 (q, 2H), 1.28 (t, 3H).

**Beispiel 130B:**

7-Chlor-1-(2,6-dichlor-4-fluorphenyl)-4-oxo-1,4-dihydro-1,8-naphthyridin-3-carbonsäure

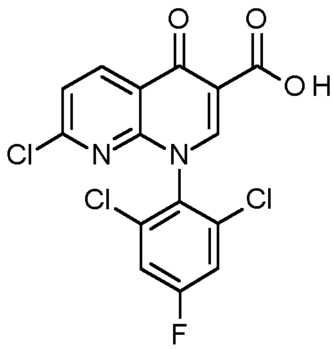

3.81 g (8.62 mmol, 94% Reinheit) der Verbindung aus Beispiel 130A wurden in 38 ml Wasser vorgelegt, mit 38 ml 36 proz. wässriger Salzsäure und 38 ml THF versetzt und 4.5h bei 110°C nachgerührt. Das Reaktionsgemisch wurde auf RT abgekühlt und mit 200 ml Wasser verdünnt. Der Niederschlag wurde abgesaugt, mit Wasser nachgewaschen und am Hochvakuum getrocknet. Es wurden 3.36 g (quantitativ, 100% Reinheit) der Titelverbindung erhalten.
LC-MS (Methode §): $R_t$ = 1.96 min; MS (ESIpos): m/z = 387 $[M+H]^+$.

**Beispiel 130C:**

7-Chlor-*N*-[(1*R*)-1-cyclopropyl-2,2,2-trifluorethyl]-1-(2,6-dichlor-4-fluorphenyl)-4-oxo-1,4-dihydro-1,8-naphthyridin-3 -carbonsäureamid

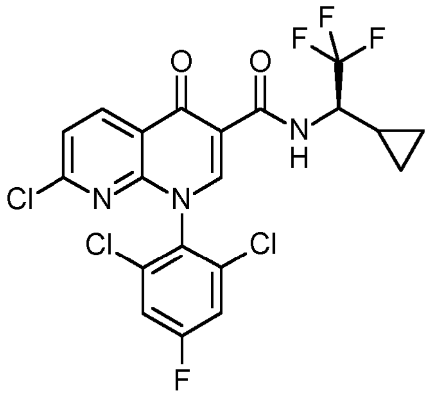

Gemäß AAV1 wurden 460 mg (1.18 mmol) der Verbindung aus Beispiel 130B mit 313 mg (1.76 mmol) (1*R*)-1-Cyclopropyl-2,2,2-trifluorethanamin Hydrochlorid in Gegenwart von 447 mg (1.18 mmol) HATU und 491 µl (2.82 mmol) *N,N*-Diisopropylethylamin in 12 ml Dimethylformamid umgesetzt. Die Reaktion wurde durch Zugabe von 40 ml Wasser und 60 ml Essigsäureethylester beendet und die Phasen getrennt. Die wässrige Phase wurde dreimal mit 20 ml Essigsäureethylester extrahiert und die vereinigten organischen Phasen mit 40 ml einer Mischung (1:1, v/v) aus gesättigter wässriger Natriumchlorid-Lösung und wässriger 1N Salzsäure gewaschen. Die organische Phase wurde dreimal mit 30 ml gesättigter wässriger Natriumchlorid-Lösung gewaschen, über Natriumsulfat getrocknet, filtriert und das Lösungsmittel unter reduziertem Druck entfernt. Der Rückstand wurde mittels Normalphasenchromatographie (Cyclohexan-Essigsäureethylester Gradient) gereinigt und 369 mg (61% d. Th., 99% Reinheit) der Titelverbindung erhalten.
$^1H$ NMR (400 MHz, DMSO-$d_6$) $\delta$ [ppm] = 10.6 (d, 1H), 9.09 (s, 1H), 8.79 (d, 1H), 7.92 (d, 2H), 7.80 (d, 1H), 4.44-4.31 (m, 1H), 1.30-1.20 (m, 1H), 0.73-0.52 (m, 3H), 0.39-0.30 (m, 1H).
LC-MS (Methode 1): $R_t$ = 1.29 min; MS (ESIpos) m/z 464 $[M+H]^+$.

**Beispiel 131A:**

7-Chlor-1-(2,6-dichlor-4-fluorphenyl)-4-oxo-*N*-[(2*R*)-1,1,1-trifluorbutan-2-yl]-1,4-dihydro-1,8-naphthyridin-3-carbonsäureamid

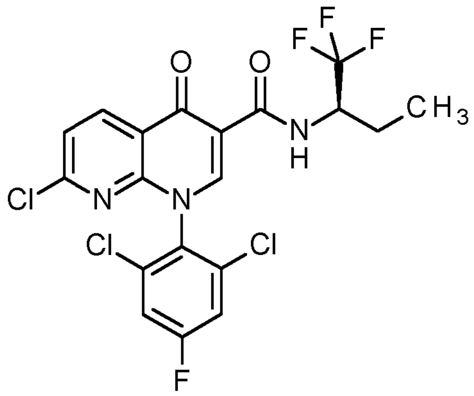

Gemäß AAV1 wurden 1.00 g (2.58 mmol) der Verbindung aus Beispiel 130B mit 633 mg (3.87 mmol) (2*R*)-1,1,1-Trifluorbutan-2-amin Hydrochlorid in Gegenwart von 981 mg (2.58 mmol) HATU und 1.08 ml (6.19 mmol) *N,N*-Diisopropylethylamin in 26 ml Dimethylformamid umgesetzt. Die Reaktion wurde durch Zugabe von 40 ml Wasser und 60 ml Essigsäureethylester beendet und die Phasen getrennt. Die wässrige Phase wurde dreimal mit 20 ml Essigsäureethylester extrahiert und die vereinigten organischen Phasen mit 40 ml einer Mischung (1:1, v/v) aus gesättigter wässriger Natriumchlorid-Lösung und wässriger 1N Salzsäure gewaschen. Die organische Phase wurde dreimal mit 30 ml gesättigter wässriger Natriumchlorid-Lösung gewaschen, über Natriumsulfat getrocknet, filtriert und das Lösungsmittel unter reduziertem Druck entfernt. Der Rückstand wurde mittels Normalphasenchromatographie (Cyclohexan-Essigsäureethylester Gradient) gereinigt und 1.07 g (83% d. Th., 99% Reinheit) der Titelverbindung erhalten.
$^1$H NMR (400 MHz, DMSO-$d_6$) $\delta$ [ppm] = 9.90 (d, 1H), 9.10 (s, 1H), 8.78 (d, 1H), 7.92 (d, 2H), 7.79 (d, 1H), 4.84-4.71 (m, 1H), 1.96-1.84 (m, 1H), 1.75-1.62 (m, 1H), 0.98 (t, 3H).
LC-MS (Methode 1): $R_t$ = 1.28 min; MS (ESIpos) m/z 496 [M+H]$^+$.

**Beispiel 132A:**

7-Chlor-1-(2,6-dichlor-4-fluorphenyl)-4-oxo-*N*-[(2*S*)-1,1,1-trifluorbutan-2-yl]-1,4-dihydro-1,8-naphthyridin-3-carbonsäureamid

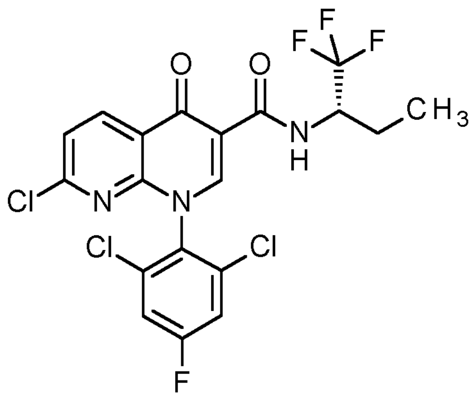

Gemäß AAV1 wurden 900 mg (2.30 mmol) der Verbindung aus Beispiel 130B mit 438 mg (3.45 mmol) (2*S*)-1,1,1-Trifluorbutan-2-amin in Gegenwart von 874 mg (2.30 mmol) HATU und 561 µl (3.22 mmol) *N,N*-Diisopropylethylamin in 23 ml Dimethylformamid umgesetzt. Die Reaktion wurde durch Zugabe von 40 ml Wasser und 60 ml Essigsäureethylester beendet und die Phasen getrennt. Die wässrige Phase wurde dreimal mit 20 ml Essigsäureethylester extrahiert und die vereinigten organischen Phasen mit 40 ml einer Mischung (1:1, v/v) aus gesättigter wässriger Natriumchlorid-Lösung und wässriger 1N Salzsäure gewaschen. Die organische Phase wurde dreimal mit 30 ml gesättigter wässriger Natriumchlorid-Lösung gewaschen, über Natriumsulfat getrocknet, filtriert und das Lösungsmittel unter reduziertem Druck entfernt. Der Rückstand wurde mittels Normalphasenchromatographie (Cyclohexan-Essigsäureethylester Gradient) gereinigt und 425 mg (37% d. Th., 99% Reinheit) der Titelverbindung erhalten.
$^1$H NMR (400 MHz, DMSO-$d_6$) $\delta$ [ppm] = 9.90 (d, 1H), 9.10 (s, 1H), 8.79 (d, 1H), 7.92 (d, 2H), 7.80 (d, 1H), 4.84-4.72 (m, 1H), 1.96-1.84 (m, 1H), 1.76-1.62 (m, 1H), 0.99 (t, 3H).
LC-MS (Methode 1): $R_t$ = 1.28 min; MS (ESIpos) m/z 496 $[M+H]^+$.

**Beispiel 133A:**

1-(2-Chlor-4,6-difluorphenyl)-7-[(4*S*)-4-hydroxy-2-oxopyrrolidin-1-yl]-4-oxo-1,4-dihydro-1,8-naphthyridin-3-carbonsäure

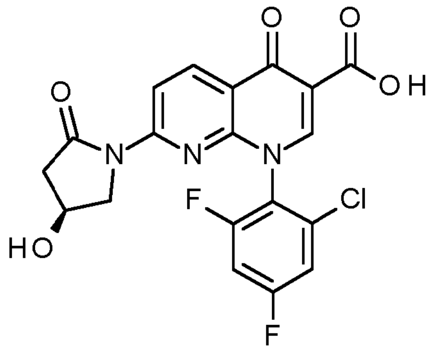

Gemäß AAV2 wurden 1.40 g (3.77 mmol) der Verbindung aus Beispiel 108B mit 458 mg (4.53 mmol) (4*S*)-4-Hydroxypyrolidin-2-on in Gegenwart von 782 mg (5.66 mmol) Kaliumcarbonat, 169 mg (754 µmol) Palladium(II)acetat und 437 mg (754 µmol) Xantphos in 26.8 ml 1,4-Dioxan bei 80°C für 1.5h umgesetzt. Der Ansatz wurde abgekühlt und auf eine Mischung aus Eiswasser, Salzsäure und Essigsäureethylester ausgerührt. Es wurde über Kieselgur abgesaugt und die organische Phase mit Wasser und gesättigter wässriger Natriumchlorid-Lösung gewaschen, getrocknet und abschließend eingeengt. 152 mg des Rückstand wurden mittels präparativer HPLC (Säule: Chromatorex C18, 10 µm, 125x30 mm, Laufmittel: Acetonitril / 0.1% Ameisensäure-Gradient; 0 bis 5 min. 10% Acetonitril, während 14 min. nach 90% Acetonitril und weitere 4 min. 90% Acetonitril) gereinigt und 84.2 mg (5% d. Th., 99% Reinheit) der Titelverbindung erhalten. Die Hauptmenge des Rückstands wurde mit tert.-Butylmethylether im Soxhlet für 22h extrahiert, einrotiert. Der Rückstand wurde mit 3 ml Acetonitril verrührt, der Niederschlag abgesaugt, dreimal mit 0.5 ml Acetonitril gewaschen und im Hochvakuum getrocknet. Es wurden 1.01 g (51% d. Th., 83% Reinheit) der Titelverbindung erhalten.
LC-MS (Methode 1): $R_t$ = 0.76 min; MS (ESIpos) m/z 436 $[M+H]^+$.
$^1$H NMR (400 MHz, DMSO-$d_6$) $\delta$ [ppm] = 14.41 (s, 1H), 9.24 (s, 1H), 8.77 (d, 1H), 8.60 (d, 1H), 7.84-7.74 (m, 2H), 5.35 (d, 1H), 4.32-4.26 (m, 1H), 3.70-3.61 (m, 1H), 3.47-3.38 (m, 1H), 2.95 (dd, 1H), 2.38 (d, 1H).

**Beispiel 134A:**

1-(2-Chlor-4,6-difluorphenyl)-7-[(3*R*,4*R*)-3,4-dihydroxypyrrolidin-1-yl]-4-oxo-1,4-dihydro-1,8-naphthyridin-3-carbonsäure

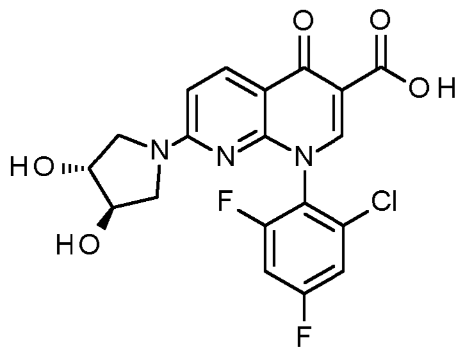

Zu einer Lösung von 150 mg (404 µmol) der Verbindung aus Beispiel 108B in 4 ml Dimethylformamid wurden bei Raumtemperatur 69.8 mg (485 µmol) (3*R*,4*R*)-Pyrrolidin-3,4-diol Hydrochlorid und 246 µl (1.42mmol) *N,N*-Diisopropylethylamin gegeben. Nach vollständigem Umsatz wurde mit 1N wässriger Salzsäure auf pH 1 angesäuert, aufkonzentriert und mittels präp. HPLC (Säule: Chromatorex C18, 10 µm, 125x30 mm, Laufmittel: Acetonitril / 0.05% Ameisensäure-Gradient; 0 bis 3 min. 10% Acetonitril bis 15 min. 90% Acetonitril und weitere 3 min. 90% Acetonitril) gereinigt. Es wurden 161 mg (90% d. Th., 99% Reinheit) der Titelverbindung erhalten.
LC-MS (Methode 3): $R_t$ = 1.21 min; MS (ESIpos) m/z 438 $[M+H]^+$.
$^1H$ NMR (400 MHz, DMSO-$d_6$) δ [ppm] = 15.23 (br. s, 1H), 8.98 (s, 1H), 8.31 (d, 1H), 7.78-7.67 (m, 2H), 6.86 (d, 1H), 5.28-5.23 (m, 1H), 5.20-5.15 (m, 1H), 4.05 (br. s, 1H), 3.93 (br. s, 1H), 3.64 (dd, 1H), 3.37 (d, 1H), 3.27-3.18 (m, 1H), 3.08-2.98 (m, 1H).

**Beispiel 135A:**

7-Chlor-*N*-[(1*R*)-1-cyclopropyl-2,2,2-trifluorethyl]-4-oxo-1-(2,4,6-trifluorphenyl)-1,4-dihydro-1,8-naphthyridin-3-carbonsäureamid

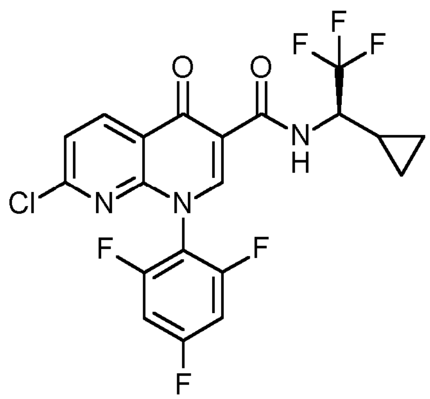

Gemäß AAV1 wurden 500 mg (1.40 mmol) der Verbindung aus Beispiel 100B mit 368 mg (2.09 mmol) (1*R*)-1-Cyclopropyl-2,2,2-trifluorethanamin Hydrochlorid in Gegenwart von 531 mg (1.40 mmol) HATU und 583 µl (3.35 mmol) *N,N*-Diisopropylethylamin in 14 ml Dimethylformamid umgesetzt. Die Reaktion wurde durch Zugabe von 20 ml Wasser und 30 ml Essigsäureethylester beendet und die Phasen getrennt. Die wässrige Phase wurde dreimal mit 20 ml Essigsäureethylester extrahiert und die vereinigten organischen Phasen mit 20 ml einer Mischung (1:1, v/v) aus gesättigter wässriger Natriumchlorid-Lösung und wässriger 1N Salzsäure gewaschen. Die organische Phase wurde dreimal mit 15 ml gesättigter wässriger Natriumchlorid-Lösung gewaschen, über Natriumsulfat getrocknet, filtriert und das Lösungsmittel unter reduziertem Druck entfernt. Der Rückstand wurde in 10 ml Dichlormethan aufgenommen und mittels Normalphasenchromatographie (Cyclohexan-Essigsäureethylester Gradient) gereinigt und 510 mg (76% d. Th., 99% Reinheit) der Titelverbindung erhalten.
$^1H$ NMR (400 MHz, DMSO-$d_6$) δ [ppm] = 10.04 (d, 1H), 9.13 (s, 1H), 8.76 (d, 1H), 7.79 (d, 1H), 7.66-7.58 (m, 2H), 4.43-4.35 (m, 1H), 1.29-1.19 (m, 1H), 0.71-0.52 (m, 3H), 0.37-0.31 (m, 1H).
LC-MS (Methode 1): $R_t$ = 1.23 min; MS (ESIpos) m/z 476 $[M+H]^+$.

**Beispiel 136A:**

7-Chlor-1-(2-chlor-4,6-difluorphenyl)-4-oxo-*N*-[(2*R*)-1,1,1-trifluorbutan-2-yl]-1,4-dihydro-1,8-naphthyridin-3-carbonsäureamid

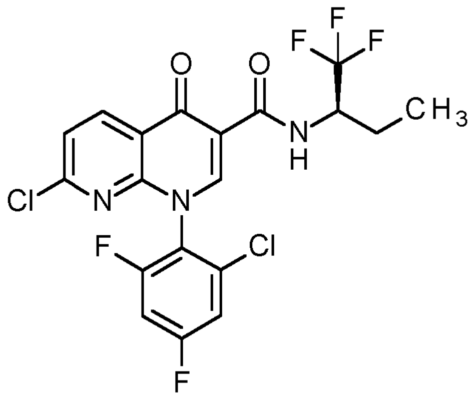

Gemäß AAV1 wurden 1.00 g (2.69 mmol) der Verbindung aus Beispiel 108B mit 661 mg (4.04 mmol) (2*R*)-1,1,1-Trifluorbutan-2-amin Hydrochlorid in Gegenwart von 1.02 g (2.69 mmol) HATU und 1.13 ml (6.47 mmol) *N,N*-Diisopropylethylamin in 27 ml Dimethylformamid umgesetzt. Die Reaktion wurde durch Zugabe von 40 ml Wasser und 60 ml Essigsäureethylester beendet und die Phasen getrennt. Die wässrige Phase wurde dreimal mit 20 ml Essigsäureethylester extrahiert und die vereinigten organischen Phasen mit 40 ml einer Mischung (1:1, v/v) aus gesättigter wässriger Natriumchlorid-Lösung und wässriger 1N Salzsäure gewaschen. Die organische Phase wurde dreimal mit 30 ml gesättigter wässriger Natriumchlorid-Lösung gewaschen, über Magnesiumsulfat getrocknet, filtriert und das Lösungsmittel unter reduziertem Druck entfernt. Der Rückstand wurde in Dichlormethan aufgenommen und mittels Normalphasenchromatographie (Cyclohexan-Essigsäureethylester Gradient) gereinigt und 1.01 g (78% d. Th., 100% Reinheit) der Titelverbindung erhalten.
$^1$H NMR (400 MHz, DMSO-$d_6$) $\delta$ [ppm] = 9.92-9.85 (m, 1H), 9.13-9.08 (m, 1H), 8.80-8.74 (m, 1H), 7.83-7.72 (m, 3H), 4.83-4.72 (m, 1H), 1.95-1.85 (m, 1H), 1.75-1.63 (m, 1H), 1.02-0.94 (m, 3H).
LC-MS (Methode 3): $R_t$ = 2.40 min; MS (ESIpos) m/z 480 $[M+H]^+$.

**Beispiel 137A:**

7-Chlor-4-oxo-*N*-[1-(trifluormethoxy)butan-2-yl]-1-(2,4,6-trifluorphenyl)-1,4-dihydro-1,8-naphthyridin-3-carbonsäureamid (Racemat)

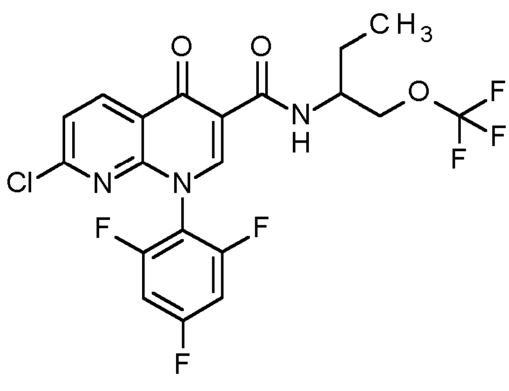

Gemäß AAV1 wurden 200 mg (564 µmol) der Verbindung aus Beispiel 100B mit 120 mg (620 µmol) 1-(Trifluormethoxy)butan-2-amin Hydrochlorid in Gegenwart von 241 mg (564 µmol) HATU und 236 µl (1.35 mmol) *N,N*-Diisopropylethylamin in 5.7 ml Dimethylformamid umgesetzt. Die Reaktion wurde durch Zugabe von 10 ml Wasser und 15

ml Essigsäureethylester beendet und die Phasen getrennt. Die wässrige Phase wurde dreimal mit 20 ml Essigsäureethylester extrahiert und die vereinigten organischen Phasen mit 40 ml einer Mischung (1:1, v/v) aus gesättigter wässriger Natriumchlorid-Lösung und wässriger 1N Salzsäure gewaschen. Die organische Phase wurde dreimal mit 30 ml gesättigter wässriger Natriumchlorid-Lösung gewaschen, über Magnesiumsulfat getrocknet, filtriert und das Lösungsmittel unter reduziertem Druck entfernt. Der Rückstand wurde in Dichlormethan aufgenommen und mittels Normalphasenchromatographie (Cyclohexan-Essigsäureethylester Gradient) gereinigt und 188 mg (67% d. Th., 99% Reinheit) der Titelverbindung erhalten.

$^1$H NMR (400 MHz, DMSO-$d_6$) δ [ppm] = 9.60 (d, 1H), 9.06 (s, 1H), 8.75 (d, 1H), 7.76 (d, 1H), 7.66-7.57 (m, 2H), 4.28-4.14 (m, 3H), 1.76-1.53 (m, 2H), 0.95 (t, 3H).

LC-MS (Methode 1): $R_t$ = 1.23 min; MS (ESIpos) m/z 494 [M+H]$^+$.

**Beispiel 138A:**

7-Chlor-1-(2-chlor-4,6-difluorphenyl)-*N*-[(1*R*)-1-cyclopropyl-2,2,2-trifluorethyl]-4-oxo-1,4-dihydro-1,8-naphthyridin-3-carbonsäureamid

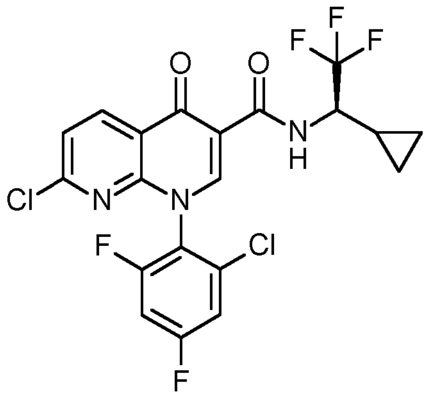

Gemäß AAV1 wurden 640 mg (1.72 mmol) der Verbindung aus Beispiel 108B mit 459 mg (2.59 mmol) (1*R*)-1-Cyclopropyl-2,2,2-trifluorethanamin Hydrochlorid in Gegenwart von 656 mg (1.72 mmol) HATU und 721 µl (4.14 mmol) *N,N*-Diisopropylethylamin in 17.3 ml Dimethylformamid umgesetzt. Die Reaktion wurde durch Zugabe von 40 ml Wasser und 60 ml Essigsäureethylester beendet und die Phasen getrennt. Die wässrige Phase wurde dreimal mit 20 ml Essigsäureethylester extrahiert und die vereinigten organischen Phasen mit 40 ml einer Mischung (1:1, v/v) aus gesättigter wässriger Natriumchlorid-Lösung und wässriger 1N Salzsäure gewaschen. Die organische Phase wurde dreimal mit 30 ml gesättigter wässriger Natriumchlorid-Lösung gewaschen, über Magnesiumsulfat getrocknet, filtriert und das Lösungsmittel unter reduziertem Druck entfernt. Der Rückstand wurde in Dichlormethan aufgenommen und mittels Normalphasenchromatographie (Cyclohexan-Essigsäureethylester Gradient) gereinigt und 635 mg (75% d. Th., 100% Reinheit) der Titelverbindung erhalten.

$^1$H NMR (400 MHz, DMSO-$d_6$) δ [ppm] = 10.04 (d, 1H), 9.11 (s, 1H), 8.77 (d, 1H), 7.82-7.71 (m, 3H), 4.46-4.32 (m, 1H), 1.29-1.19 (m, 1H), 0.73-0.52 (m, 3H), 0.39-0.29 (m, 1H).

LC-MS (Methode 3): $R_t$ = 2.41 min; 492 [M+H]$^+$.

**Beispiel 139A:**

(5*R*)-5-({[*tert.*-Butyl(dimethyl)silyl]oxy}methyl)pyrrolidin-2-on

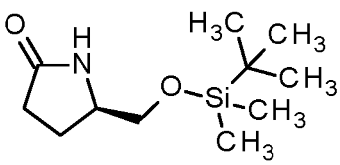

Zu einer Lösung von 1.03 g (8.95 mmol) (5*R*)-5-(Hydroxymethyl)pyrrolidin-2-on und 914 mg (13.4 mmol) Imidazol in 20 ml Dimethylformamid wurde bei 0°C 1.39 g (8.95 mmol) *tert*.-Butyldimethylsilylchlorid gegeben. Es wurde 30 min bei 0°C und über Nacht bei Raumtemperatur nachgerührt. Anschließend wurden alle flüchtigen Bestandteile unter reduziertem Druck entfernt und der Rückstand mit 100 ml Wasser versetzt und dreimal mit 30 ml Essigsäureethylester extrahiert. Die vereinigten organischen Phasen wurden mit 30 ml gesättigter wässriger Natriumchlorid-Lösung gewaschen, über Magnesiumsulfat getrocknet, filtriert und das Lösungsmittel unter reduziertem Druck entfernt. Es wurden 1.56 g (76% d. Th., 100% Reinheit) der Titelverbindung erhalten.
$^1H$ NMR (400 MHz, $CDCl_3$) $\delta$ [ppm] = 5.70 (br. s, 1H), 3.79-3.72 (m, 1H), 3.63 (dd, 1H), 3.44 (dd, 1H), 2.38-2.31 (m, 2H), 2.23-2.12 (m, 1H), 1.78-1.67 (m, 1H), 0.89 (s, 9H), 0.06 (s, 6H).
LC-MS (Methode 1): $R_t$ = 0.97 min; 230 $[M+H]^+$.

**Beispiel 140A:**

*N*-(Cyclopropylmethylen)-2-methylpropane-2-(*R*)-sulfinamid

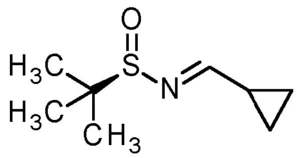

Zu einer Lösung von 1.73 g (14.3 mmol) (*R*)-2-Methylpropan-2-sulfinamid und 2.00 g (28.5 mmol) Cyclopropancarbaldehyd in 85.6 ml Dichlormethan wurden 6.83 g (42.8 mmol) Kupfer(II)sulfat (trocken) gegeben. Es wurde für 18h bei Raumtemperatur nachgerührt und anschließend über 3 cm Celite filtriert und mit Dichlormethan nachgewaschen. Die organische Phase wurde zweimal mit 10 ml einer wässrigen 10%igen Ammoniumchlorid-Lösung gewaschen, über Natriumsulfat getrocknet, filtriert und das Lösungsmittel unter reduziertem Druck entfernt. Es wurden 2.47 g (86% d. Th., 86% Reinheit) der Titelverbindung erhalten.
LC-MS (Methode 3): $R_t$ = 1.37 min; 174 $[M+H]^+$.

**Beispiel 140B:**

*N*-[(1*S*)-1-Cyclopropyl-2,2-difluor-2-(phenylsulfonyl)ethyl]-2-methylpropan-2-(*R*)-sulfinamid

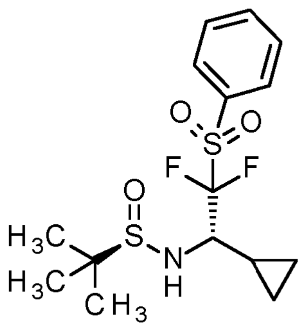

Eine Lösung von 728 mg (4.20 mmol) der Verbindung aus Beispiel 140A und 769 mg (4.00 mmol) Difluormethylphenylsulfon wurde bei -78°C mit 4.8 ml (4.8 mmol, 1M in THF) Lithiumhexamethyldisilazid versetzt und 20 min nachgerührt. Die Reaktion wurde durch Zugabe von 20 ml gesättigter wässriger Natriumchlorid-Lösung beendet und dreimal mit 50 ml Essigsäureethylester extrahiert. Die vereinigten organischen Phasen wurden über Natriumsulfat getrocknet, filtriert und einrotiert. Der Rückstand wurde in 4 ml Dichlormethan gelöst und mittels Normalphasenchromatographie (Cyclohexan-Essigsäureethylester Gradient) gereinigt. Zurück gewonnenes Edukt aus Verbindung 140A wurde in analoger Weise nochmals umgesetzt und die Produktfraktionen vereinigt. Es wurden 897 mg (58%) der Titelverbindung erhalten.
$^1H$ NMR (400 MHz, DMSO-$d_6$) $\delta$ [ppm] = 7.98-7.88 (m, 3H), 7.79-7.73 (m, 2H), 5.80 (d, 1H), 3.43-3.33 (m, 1H), 1.33-1.22

(m, 1H), 0.77-0.48 (m, 4H).
LC-MS (Methode 1): $R_t$ = 0.93 min; MS (ESIpos) m/z 366 $[M+H]^+$.

**Beispiel 140C:**

*N*-[(1*S*)-1-Cyclopropyl-2,2-difluorethyl]-2-methylpropan-2-(*R*)-sulfinamid

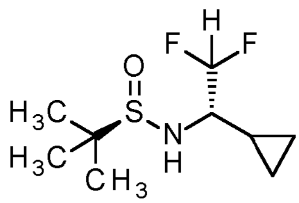

Zu einer Suspension von 650 mg (1.78 mmol) der Verbindung aus Beispiel 140B und 1.79 g (12.6 mmol) Natriumhydrogenphosphat in 23 ml Methanol wurde bei -20°C 4.02 g Natriumamalgam (5% Natrium) gegeben. Es wurde für 4.5h nachgerührt, abdekantiert und alle flüchtigen Bestandteile unter reduziertem Druck entfernt. Es wurden 15 ml gesättigte wässrige Natriumchlorid-Lösung hinzugegeben und dreimal mit 15 ml Essigsäureethylester extrahiert. Die vereinigten organischen Phasen wurden über Natriumsulfat getrocknet, filtriert und das Lösungsmittel unter reduziertem Druck entfernt. Es wurden 397 mg (98% d. Th., 99% Reinheit) der Titelverbindung erhalten.
LC-MS (Methode 1): $R_t$ = 0.79 min; MS (ESIpos) m/z 226 $[M+H]^+$.

**Beispiel 140D:**

(1*S*)-1-Cyclopropyl-2,2-difluorethanamin Hydrochlorid

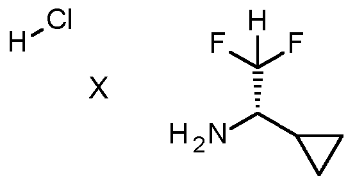

Zu einer Lösung von 396 mg (1.76 mmol) der Verbindung aus Beispiel 140C in 24.8 ml Methanol wurden 6.20 ml (24.8 mmol, 4N in Dioxan) Salzsäure gegeben und für 30 min nachgerührt. Anschließend wurde der Ansatz zur Trockene einrotiert und mit 8 ml Diethylether verrührt, zentrifugiert, dekantiert und der Rückstand im Hochvakuum getrocknet. Es wurden 209 mg (75% d. Th., 99% Reinheit) der Titelverbindung erhalten.
$^1H$ NMR (400 MHz, $D_2O$-$d_2$) $\delta$ [ppm] = 6.28 (t, 1H), 3.03-2.93 (m, 1H), 1.13-1.04 (m, 1H), 0.84-0.76 (m, 2H), 0.62-0.46 (m, 2H).
Drehwert: MeOH, konz. 0.4850 g/ 100 ml, $\lambda$: 365 nm [-15,12°]

**Beispiel 141A:**

*tert.*-Butyl [(2*R*)-1-{[6-{[(1*S*)-1-cyclopropyl-2,2,2-trifluorethyl]carbamoyl}-5-oxo-8-(2,4,6-trifluorphenyl)-5,8-dihydro-1,8-naphthyridin-2-yl]amino}propan-2-yl]carbamat

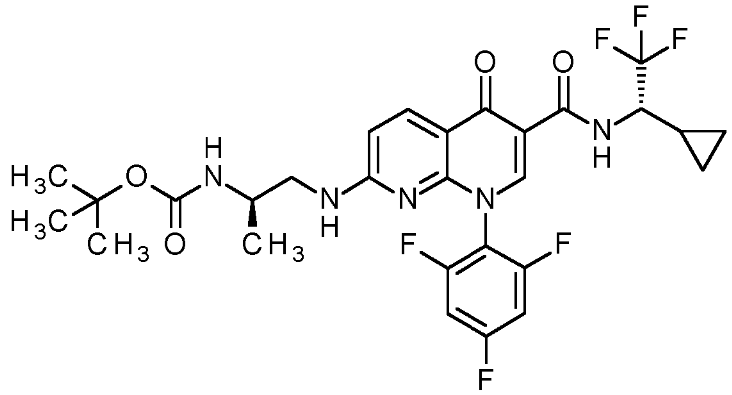

Zu einer Lösung von 150 mg (315 µmol) der Verbindung aus Beispiel 126A in 3.1 ml Dimethylformamid wurde bei Raumtemperatur 93.0 mg (441 µmol) *tert*.-Butyl [(2*R*)-1-Aminopropan-2-yl]carbamat Hydrochlorid und 225 µl (1.29 mmol) *N,N*-Diisopropylethylamin gegeben. Es wurde für 72h nachgerührt. Die Reaktionslösung wurde mit 1 ml Acetonitril und 0.5 ml Wasser verdünnt und mittels präp. HPLC (Säule: Chromatorex C18, 10 µm, 125x30 mm, LM: Acetonitril / 0.1% Ameisensäure-Gradient; 0 bis 5 min. 10% Acetonitril, während 14 min. nach 90% Acetonitril und weitere 4 min. 90% Acetonitril) getrennt. Es wurden 161 mg (83% d. Th., 99% Reinheit) der Titelverbindung erhalten.
LC-MS (Methode 3): $R_t$ = 2.24 min; MS (ESIpos) m/z 614 $[M+H]^+$.

<u>**Beispiel 141B:**</u>

7-{[(2*R*)-2-Aminopropyl]amino}-*N*-[(1*S*)-1-cyclopropyl-2,2,2-trifluorethyl]-4-oxo-1-(2,4,6-trifluorphenyl)-1,4-dihydro-1,8-naphthyridin-3-carbonsäureamid Trifluoressigsäure

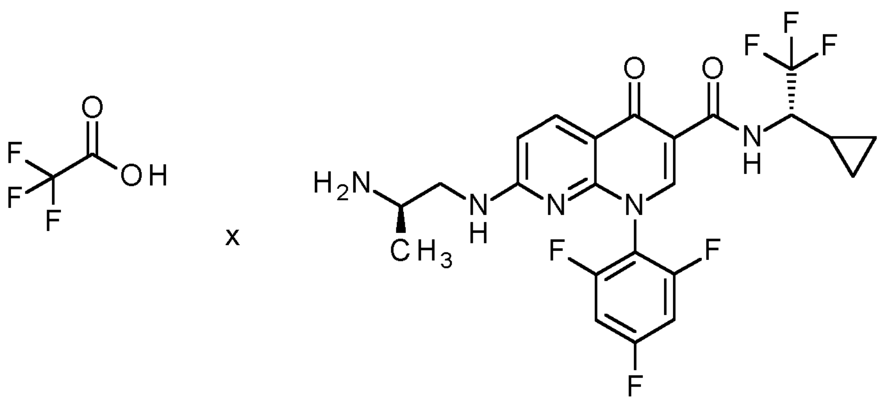

Zu einer Lösung von 166 mg (271 µmol) der Verbindung aus Beispiel 141A in 10 ml Dichlormethan wurden unter Eisbadkühlung 5.00 ml (64.9 mmol) Trifluoressigsäure gegeben. Es wurde für 2h bei Raumtemperatur nachgerührt und anschließend alle flüchtigen Komponenten unter reduziertem Druck entfernt. Der Rückstand wurde mit Toluol kodestilliert und im Hochvakuum getrocknet. Es wurden 170 mg (99% d. Th., 99% Reinheit) der Titelverbindung erhalten.
LC-MS (Methode 3): $R_t$ = 1.32 min; MS (ESIpos) m/z 514 $[M+H]^+$.

<u>**Beispiel 142A:**</u>

*N*-[(1*S*)-1-Cyclopropyl-2,2,2-trifluorethyl]-7-{[(2*S*)-2-hydroxypropyl]amino}-4-oxo-1-(2,4,6-trifluorphenyl)-1,4-dihydro-1,8-naphthyridin-3-carbonsäureamid

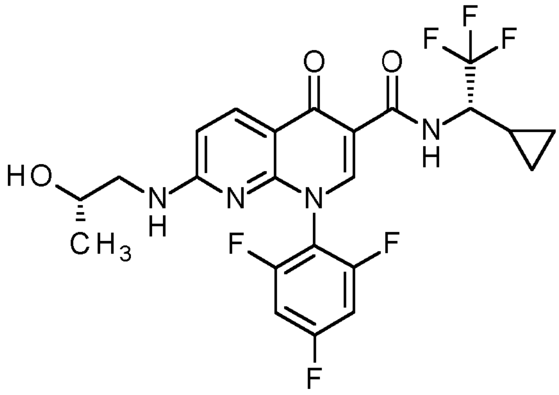

Zu einer Lösung von 150 mg (315 µmol) der Verbindung aus Beispiel 126A in 3.1 ml Dimethylformamid wurde bei Raumtemperatur 33.2 mg (441 µmol) (2*S*)-1-Aminopropan-2-ol und 192 µl (1.10 mmol) *N,N*-Diisopropylethylamin gegeben. Es wurde für 48h nachgerührt. Die Reaktionslösung wurde mit 1 ml Acetonitril, 0.5 ml Wasser und 0.1 ml 1N wässriger Salzsäure verdünnt und mittels präp. HPLC (Säule: Chromatorex C18, 10 µm, 125x30 mm, LM: Acetonitril / 0.1 % Ameisensäure-Gradient; 0 bis 5 min. 10% Acetonitril, während 14 min. nach 90% Acetonitril und weitere 4 min. 90% Acetonitril) getrennt. Es wurden 136 mg (83% d. Th., 99% Reinheit) der Titelverbindung erhalten.
$^1$H-NMR (400 MHz, DMSO-$d_6$) δ [ppm] = 10.60 (d, 1H), 8.77 (s, 1H), 8.21-8.11 (m, 2H), 7.60-7.51 (m, 2H), 6.74 (d, 1H), 4.65 (d, 1H), 4.44-4.32 (m, 1H), 3.62-3.50 (m, 1H), 3.06-2.96 (m, 1H), 2.84-2.75 (m, 1H), 1.25-1.15 (m, 1H), 0.83 (d, 3H), 0.69-0.47 (m, 3H), 0.38-0.30 (m, 1H).
LC-MS (Methode 3): $R_t$ = 1.88 min; MS (ESIpos) m/z 515 $[M+H]^+$.

**Beispiel 143A:**

*tert.*-Butyl [(2*S*)-1-{[6-{[(1*S*)-1-cyclopropyl-2,2,2-trifluorethyl]carbamoyl}-5-oxo-8-(2,4,6-trifluorphenyl)-5,8-dihydro-1,8-naphthyridin-2-yl]amino}propan-2-yl]carbamat

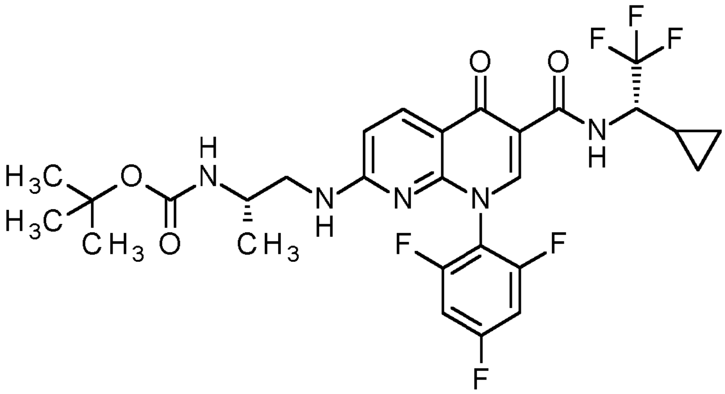

Zu einer Lösung von 150 mg (315 µmol) der Verbindung aus Beispiel 126A in 3.1 ml Dimethylformamid wurde bei Raumtemperatur 93.0 mg (441 µmol) *tert.*-Butyl [(2*S*)-1-Aminopropan-2-yl]carbamat Hydrochlorid und 225 µl (1.29 mmol) *N,N*-Diisopropylethylamin gegeben. Es wurde für 72h nachgerührt. Die Reaktionslösung wurde mit 0.2 ml Acetonitril und 0.5 ml Wasser verdünnt und mittels präp. HPLC (Säule: Chromatorex C18, 10 µm, 125x30 mm, LM: Acetonitril / 0.1% Ameisensäure-Gradient; 0 bis 5 min. 10% Acetonitril, während 14 min. nach 90% Acetonitril und weitere 4 min. 90% Acetonitril) getrennt. Es wurden 174 mg (89% d. Th., 99% Reinheit) der Titelverbindung erhalten.
LC-MS (Methode 3): $R_t$ = 2.22 min; MS (ESIpos) m/z 614 $[M+H]^+$.

**Beispiel 143B:**

7-{[(2*S*)-2-Aminopropyl]amino}-*N*-[(1*S*)-1-cyclopropyl-2,2,2-trifluorethyl]-4-oxo-1-(2,4,6-trifluorphenyl)-1,4-dihydro-1,8-naphthyridin-3-carbonsäureamid Trifluoressigsäure

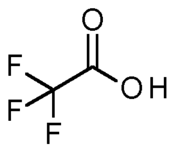

x

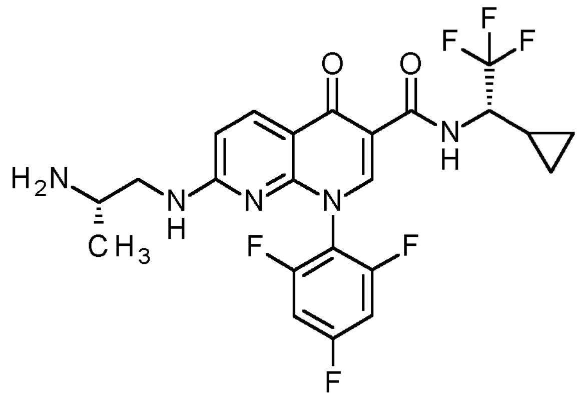

Zu einer Lösung von 174 mg (283 μmol) der Verbindung aus Beispiel 143A in 10 ml Dichlormethan wurden unter Eisbadkühlung 436 μl (5.66 mmol) Trifluoressigsäure gegeben. Es wurde für 3h bei Raumtemperatur nachgerührt und anschließend wurden weitere 10 Äquivalente Trifluoressigsäure hinzugegeben und für eine weitere Stunde bei Raumtemperatur gerührt. Es wurden alle flüchtigen Komponenten unter reduziertem Druck entfernt, der Rückstand zweimal mit 20 ml Toluol kodestilliert und im Hochvakuum getrocknet. Es wurden 185 mg (quantitativ, 100% Reinheit) der Titelverbindung erhalten.
LC-MS (Methode 3): $R_t$ = 1.29 min; MS (ESIpos) m/z 514 $[M+H]^+$.

Beispiel 144A:

*tert.*-Butyl [1-({[6-{[(1*S*)-1-cyclopropyl-2,2,2-trifluorethyl]carbamoyl}-5-oxo-8-(2,4,6-trifluorphenyl)-5,8-dihydro-1,8-naphthyridin-2-yl]amino}methyl)cyclopropyl]carbamat

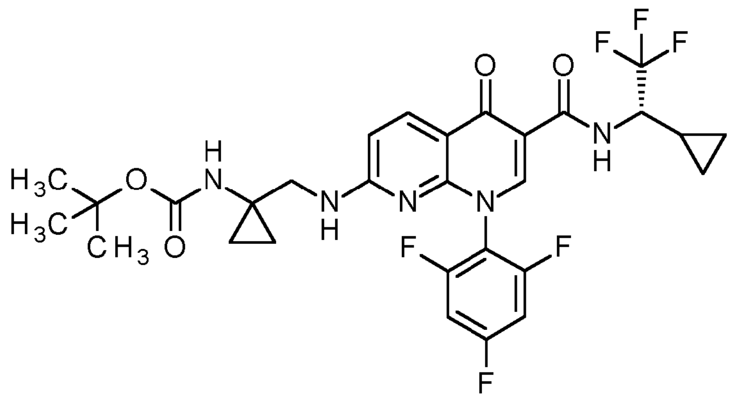

Zu einer Lösung von 150 mg (315 μmol) der Verbindung aus Beispiel 126A in 3.1 ml Dimethylformamid wurde bei Raumtemperatur 98.3 mg (441 μmol) tert.-Butyl [1-(aminomethyl)cyclopropyl]carbamat Hydrochlorid und 225 μl (1.29 mmol) *N,N*-Diisopropylethylamin gegeben. Es wurde für 48h nachgerührt. Die Reaktionslösung wurde mit 0.2 ml Acetonitril und 0.5 ml Wasser verdünnt und mittels präp. HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) getrennt. Es wurden 171 mg (86% d. Th., 99% Reinheit) der Titelverbindung erhalten.
LC-MS (Methode 3): $R_t$ = 2.30 min; MS (ESIpos) m/z 626 $[M+H]^+$.

**Beispiel 144B:**

7-{[(1-Aminocyclopropyl)methyl]amino}-*N*-[(1*S*)-1-cyclopropyl-2,2,2-trifluorethyl]-4-oxo-1-(2,4,6-trifluorphenyl)-1,4-dihydro-1,8-naphthyridin-3-carbonsäureamid Bis(trifluoracetat)

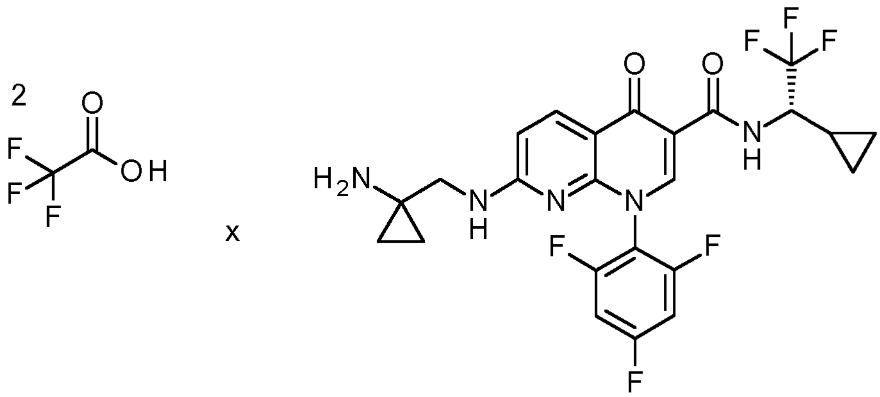

2 x

Zu einer Lösung von 170 mg (272 μmol) der Verbindung aus Beispiel 144A in 7.9 ml Dichlormethan wurden unter Eisbadkühlung 419μl (5.44 mmol) Trifluoressigsäure gegeben. Es wurde für 2.5h bei Raumtemperatur nachgerührt. Es wurden alle flüchtigen Komponenten unter reduziertem Druck entfernt, der Rückstand mit Toluol kodestilliert und abschließend lyophilisiert. Es wurden 185 mg (89% d. Th., 99% Reinheit) der Titelverbindung erhalten.
LC-MS (Methode 3): $R_t$ = 1.31 min; MS (ESIpos) m/z 526 $[M+H]^+$.

**Beispiel 145A:**

*tert.*-Butyl (1-{[6-{[(1*S*)-1-cyclopropyl-2,2,2-trifluorethyl]carbamoyl}-5-oxo-8-(2,4,6-trifluorphenyl)-5,8-dihydro-1,8-naphthyndin-2-yl]amino}-2-methylpropan-2-yl)carbamat

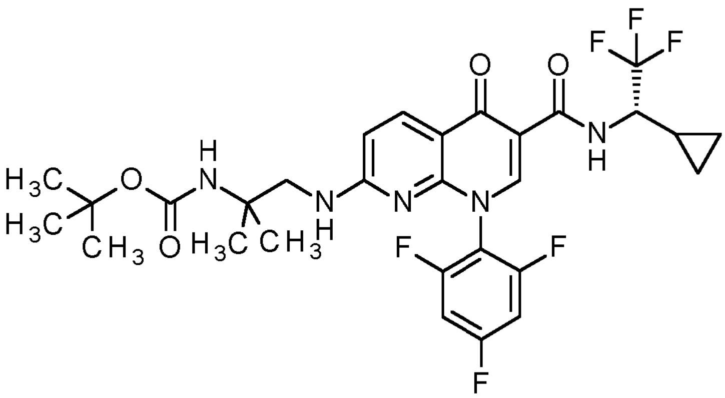

Zu einer Lösung von 150 mg (315 μmol) der Verbindung aus Beispiel 126A in 3.1 ml Dimethylformamid wurde bei Raumtemperatur 99.2 mg (441 μmol) *tert.*-Butyl(1-amino-2-methylpropan-2-yl)carbamat Hydrochlorid und 225 μl (1.29 mmol) *N,N*-Diisopropylethylamin gegeben. Es wurde für 48h nachgerührt. Die Reaktionslösung wurde mit 0.2 ml Acetonitril und 0.5 ml Wasser verdünnt und mittels präp. HPLC (Säule: Chromatorex C18, 10 μm, 125x30 mm, LM: Acetonitril / 0.1% Ameisensäure-Gradient; 0 bis 5 min. 10% Acetonitril, während 14 min. nach 90% Acetonitril und weitere 4 min. 90% Acetonitril) getrennt. Es wurden 174 mg (87% d. Th., 99% Reinheit) der Titelverbindung erhalten.
LC-MS (Methode 3): $R_t$ = 2.40 min; MS (ESIpos) m/z 628 $[M+H]^+$.

**Beispiel 145B:**

7-[(2-Amino-2-methylpropyl)amino]-*N*-[(1*S*)-1-cyclopropyl-2,2,2-trifluorethyl]-4-oxo-1-(2,4,6-trifluorphenyl)-1,4-dihydro-1,8-naphthyridin-3-carbonsäureamid Bis(trifluoracetat)

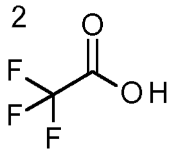

x

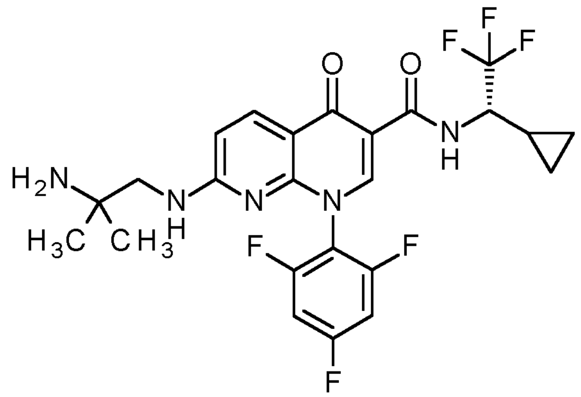

Zu einer Lösung von 172 mg (274 µmol) der Verbindung aus Beispiel 145A in 7.9 ml Dichlormethan wurden unter Eisbadkühlung 422 µl (5.48 mmol) Trifluoressigsäure gegeben. Es wurde für 2.5h bei Raumtemperatur nachgerührt. Es wurden alle flüchtigen Komponenten unter reduziertem Druck entfernt, der Rückstand mit Toluol kodestilliert und abschließend lyophilisiert. Es wurden 185 mg (91% d. Th., 99% Reinheit) der Titelverbindung erhalten.
LC-MS (Methode 3): $R_t$ = 1.33 min; MS (ESIpos) m/z 528 $[M+H]^+$.

**Beispiel 146A:**

7-({(2*R*)-2-[(*tert*.-Butoxycarbonyl)amino]propyl}amino)-4-oxo-1-(2,4,6-trifluorphenyl)-1,4-dihydro-1,8-naphthyridin-3-carbonsäureethylester

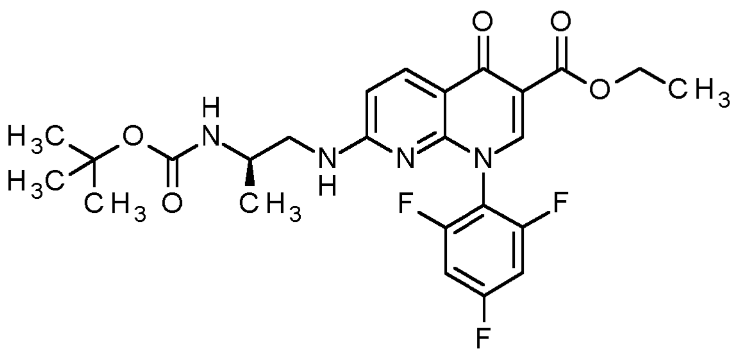

Zu einer Lösung von 1.50 g (3.92 mmol) der Verbindung aus Beispiel 100A in 25 ml Dimethylformamid wurde bei Raumtemperatur 991 mg (4.70 mmol) *tert*.-Butyl [(2*R*)-1-aminopropan-2-yl]carbamat Hydrochlorid und 2.39 ml (13.7 mmol) *N,N*-Diisopropylethylamin gegeben. Es wurde für 12h bei Raumtemperatur und 37h bei 60°C nachgerührt. Die Reaktionslösung wurde auf 250 ml Wasser gegeben und mit 1N wässriger Salzsäure auf pH 5 gestellt. Der Niederschlag wurde abgesaugt, mit Wasser gewaschen und am Hochvakuum getrocknet. Es wurden 1.81 g (85% d. Th., 95% Reinheit) der Titelverbindung erhalten.
LC-MS (Methode 3): $R_t$ = 1.82 min; MS (ESIpos) m/z 521 $[M+H]^+$.

**Beispiel 146B:**

7-{[(2*R*)-2-Aminopropyl]amino}-4-oxo-1-(2,4,6-trifluorphenyl)-1,4-dihydro-1,8-naphthyridin-3-carbonsäureethylester Trifluoracetat

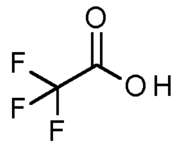

x

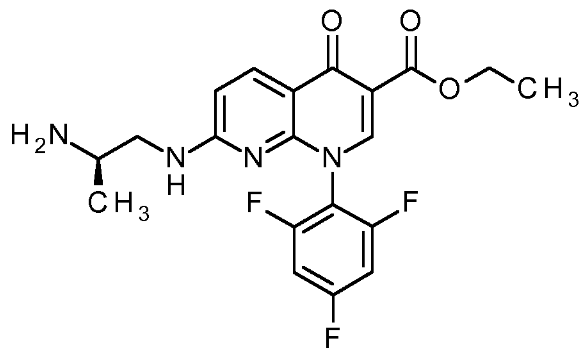

Zu einer Lösung von 1.80 g (3.46 mmol) der Verbindung aus Beispiel 146A in 100 ml Dichlormethan wurden unter Eisbadkühlung 5.33 ml (69.2 mmol) Trifluoressigsäure gegeben. Es wurde für 2.5h bei Raumtemperatur nachgerührt. Es wurden alle flüchtigen Komponenten unter reduziertem Druck entfernt, der Rückstand mit Toluol kodestilliert und abschließend lyophilisiert. Es wurden 2.50 g (quantitativ, 99% Reinheit) der Titelverbindung erhalten.
LC-MS (Methode 3): $R_t$ = 0.92 min; MS (ESIpos) m/z 421 $[M+H]^+$.

**Beispiel 146C:**

7-[(4*R*)-4-Methyl-2-oxoimidazolidin-1-yl]-4-oxo-1-(2,4,6-trifluorphenyl)-1,4-dihydro-1,8-naphthyridin-3-carbonsäureethylester

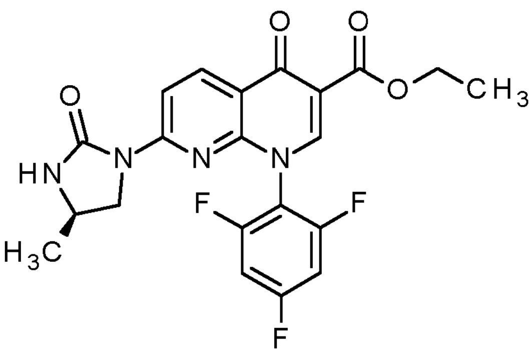

Zu einer Lösung von 2.50 g (4.68 mmol) der Verbindung aus Beispiel 146B in 103 ml Dimethylformamid wurde bei Raumtemperatur 647mg (4.68 mmol) Kaliumcarbonat und 1.89 g (11.7 mmol) 1,1'-Carbonyldiimidazol gegeben. Es wurde 6h nachgerührt. Anschließend wurde die Reaktionslösung auf 600 ml Wasser gegeben, mit 5 ml 1N wässriger Salzsäure versetzt, der Niederschlag abgesaugt, mit Wasser gewaschen und am Hochvakuum getrocknet. Es wurden 1.20 g (57% d. Th., 99% Reinheit) der Titelverbindung erhalten.
LC-MS (Methode 1): $R_t$ = 0.86 min; MS (ESIpos) m/z 447 $[M+H]^+$.

**Beispiel 146D:**

7-[(4*R*)-4-Methyl-2-oxoimidazolidin-1-yl]-4-oxo-1-(2,4,6-trifluorphenyl)-1,4-dihydro-1,8-naphthyridin-3-carbonsäure

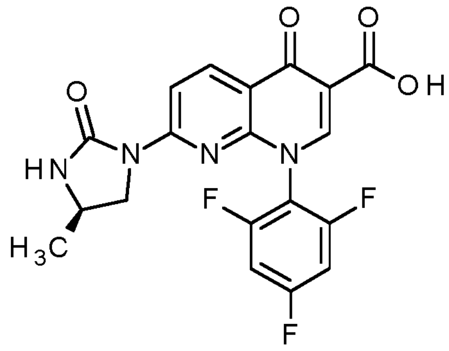

1.19 g (2.67 mmol) der Verbindung aus Beispiel 146C wurden in 8 ml Wasser vorgelegt, mit 8 ml 36 proz. wässriger Salzsäure und 8 ml THF versetzt und 4h bei 110°C nachgerührt. Das Reaktionsgemisch wurde auf RT abgekühlt und mit 100 ml Wasser verdünnt. Der Niederschlag wurde abgesaugt, mit Wasser nachgewaschen und am Hochvakuum getrocknet. Es wurden 995 mg (87% d. Th., 97% Reinheit) der Titelverbindung erhalten.
LC-MS (Methode 3): $R_t$ = 1.49 min; MS (ESIpos): m/z = 419 $[M+H]^+$.
$^1H$ NMR (400 MHz, DMSO-d6): $\delta$ [ppm] = 14.65 (s, 1H), 9.19 (s, 1H), 8.60 (d, 1H), 8.50 (d, 1H), 7.91 (s, 1H), 7.65-7.55 (m, 2H), 3.81-3.70 (m, 2H), 3.14-3.06 (m, 1H), 1.13 (d, 3H).

**Beispiel 147A**

*N*-Benzyl-1,1,1,2,2-pentafluorbutan-3-amin (Racemat)

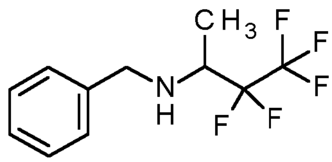

Zu einer Lösung von 2.00 g (12.2 mmol) 3,3,4,4,4-Pentafluorobutan-2-on in 10 ml Dichlormethan wurden bei 0°C 5.40 ml (18.3 mmol) Titantetraisopropoxid und 2.66 ml (24.4 mmol) Benzylamin gegeben. Es wurde 90 min bei RT nachgerührt bevor erneut auf 0°C abgekühlt wurde. Anschließend wurde 2.14 g (34.1 mmol) Natriumcyanoborhydrid, 36 ml Methanol und 3Å Molsieb zugegeben. Es wurde auf RT erwärmt und 2d nachgerührt. Die Reaktionslösung wurde mit wenig Wasser und Essigsäureethylester versetzt und filtriert. Das Filtrat wurde zweimal mit gesättigter wässriger Natriumhydrogencarbonat-Lösung und einmal mit gesättigter wässriger Natriumchlorid-Lösung gewaschen. Die organische Phase wurde über Natriumsulfat getrocknet, filtriert und das Lösungsmittel unter reduziertem Druck entfernt. Der Rückstand wurde zweimal mittels Normalphasenchromatographie (Essigsäureethylester/Cyclohexan 1/20) gereinigt und 1.65 g (48% d. Th., 91% Reinheit) der Titelverbindung erhalten.
LC-MS (Methode 6): $R_t$ = 2.17 min; MS (ESIpos): m/z = 254 $[M+H]^+$.
$^1H$ NMR (500 MHz, DMSO-d6): $\delta$ [ppm] = 7.28-7.36 (m, 4H), 7.20-7.27 (m, 1H), 3.83 (dd, 1H), 3.72 (dd, 1H), 3.22-3.30 (m, 1H), 2.43-2.48 (m, 1H), 1.20 (d, 3H).

**Beispiel 147B**

1,1,1,2,2-Pentafluorbutan-3 -amin-Hydrochlorid (Racemat)

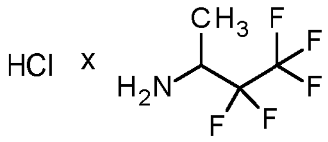

Zu einer Lösung von 1.50 g (5.92 mmol) *N*-Benzyl-1,1,1,2,2-pentafluorpentan-3-amin in 27.4 ml Methanol wurden 150 mg Palladium auf Kohle (10%) gegeben und für 6h bei Normaldruck und Raumtemperatur hydriert. Anschließend wurde die Reaktionsmischung durch einen Milliporefilter filtriert und das Lösungsmittel unter reduziertem Druck entfernt. Die Vorlage mit dem abdestillierten Lösungsmittel wurde anschließend in einen Kolben überführt und mit 4N wässriger Salzsäure in Dioxan versetzt und erneut eingeengt. Der Rückstand wurde mit Diethylether verrührt, der Niederschlag abgesaugt und im Hochvakuum getrocknet. Es wurden 456 mg (39% d. Th., 100% Reinheit) der Titelverbindung erhalten.
$^1$H NMR (500 MHz, DMSO-d6): $\delta$ [ppm] = 9.21 (br. s, 3H), 4.40-4.29 (m, 1H), 1.41 (d, 3H).

**Beispiel 148A:**

3-({[*tert*.-Butyl(dimethyl)silyl]oxy}methyl)pyrrolidin-2-on (Racemat)

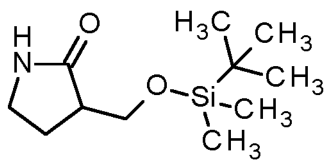

Zu einer Lösung von 110 mg (955 $\mu$mol) 3-(Hydroxymethyl)pyrrolidin-2-on und 97.6 mg (1.43 mmol) Imidazol in 5 ml Dimethylformamid wurde bei 0°C 148 mg (955 $\mu$mol) *tert*.-Butyldimethylsilylchlorid gegeben. Es wurde 30 min bei 0°C und über Nacht bei Raumtemperatur nachgerührt. Anschließend wurden alle flüchtigen Bestandteile unter reduziertem Druck entfernt und der Rückstand mit 10 ml Wasser versetzt und dreimal mit 20 ml Essigsäureethylester extrahiert. Die vereinigten organischen Phasen wurden mit 30 ml gesättigter wässriger Natriumchlorid-Lösung gewaschen, über Magnesiumsulfat getrocknet, filtriert und das Lösungsmittel unter reduziertem Druck entfernt. Es wurden 115 mg (52% d. Th., 100% Reinheit) der Titelverbindung erhalten.
$^1$H NMR (400 MHz, $CDCl_3$) $\delta$ [ppm] = 5.44 (br. s, 1H), 3.84 (dd, 1H), 3.75 (dd, 1H), 3.35-3.22 (m, 2H), 2.48-2.40 (m, 1H), 2.26-2.06 (m, 2H), 0.82 (s, 9H), 0.00 (d, 6H).
LC-MS (Methode 3): $R_t$ = 1.81 min; MS (ESIpos) m/z 230 $[M+H]^+$.

**Beispiel 149A:**

7-[(3*R*)-3-Hydroxy-2-oxopyrrolidin-1-yl]-4-oxo-1-(2,4,6-trifluorphenyl)-1,4-dihydro-1,8-naphthyridin-3-carbonsäure

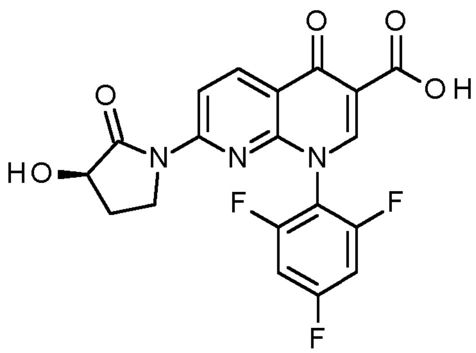

Gemäß AAV2 wurden 270 mg (761 µmol) der Verbindung aus Beispiel 100B mit 92.4 mg (914 µmol) (3*R*)-3-Hydroxypyrrolidin-2-on in Gegenwart von 158 mg (1.14 mmol) Kaliumcarbonat, 17 mg (76 µmol) Palladium(II)acetat und 88.1 mg (152 µmol) Xantphos in 6 ml 1,4-Dioxan bei 80°C für 12h umgesetzt. Der Ansatz wurde nochmals mit Katalysator versetzt und weitere 5h bei 80°C gerührt. Anschließend wurde die Reaktionsmischung auf eine Mischung aus Eiswasser, Salzsäure und Essigsäureethylester ausgerührt. Es wurde über Kieselgur abgesaugt und die organische Phase mit Wasser und gesättigter wässriger Natriumchlorid-Lösung gewaschen, über Natriumsulfat getrocknet, filtriert und eingeengt. Der Rückstand wurde in 6.5 ml Acetonitril und 0.5 ml Wasser gelöst und mittels präp. HPLC (Säule: Chromatorex C18, 10 µm, 125x30 mm, LM: Acetonitril / 0.1% Ameisensäure-Gradient; 0 bis 5 min. 10% Acetonitril, während 14 min. nach 90% Acetonitril und weitere 4 min. 90% Acetonitril) gereinigt. Es wurden 159 mg (49% d. Th., 99% Reinheit) der Titelverbindung erhalten.

$^1$H NMR (400 MHz, DMSO-$d_6$) δ [ppm] = 14.37 (br. s, 1H), 9.25 (s, 1H), 8.77 (d, 1H), 8.60 (d, 1H), 7.66-7.56 (m, 2H), 5.93 (d, 1H), 4.45-4.36 (m, 1H), 3.62-3.53 (m, 1H), 2.38-2.26 (m, 1H), 1.85-1.71 (m, 1H), eine Resonanz teilweise unter dem Wassersignal.

LC-MS (Methode 1): $R_t$ = 0.73 min; MS (ESIpos) m/z 420 $[M+H]^+$.

## Beispiel 151A:

*tert.*-Butyl (5-oxopyrrolidin-3-yl)carbamat (Racemat)

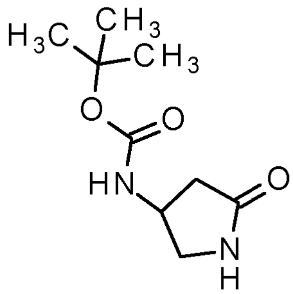

Eine Lösung von 100 mg (732 µmol) 4-Aminopyrrolidin-2-on Hydrochlorid (Racemat) in 1.5 ml Wasser und 3.5 ml Dioxan wurde bei Raumtemperatur mit 185 mg (2.19 mmol) Natriumhydrogencarbonat und 168 mg (769 µmol) Di-*tert.*-butyldicarbonat versetzt. Es wurde über Nacht nachgerührt. Anschließend wurde der Ansatz mit Wasser versetzt und dreimal mit Essigsäureethylester extrahiert. Die vereinigten organischen Phasen wurden über Magnesiumsulfat getrocknet, eingeengt und der Rückstand am Hochvakuum getrocknet. Es wurden 69.2 mg (47% d. Th., 100% Reinheit) der Titelverbindung erhalten.

LC-MS (Methode 1): $R_t$ = 0.48 min; MS (ESIpos): m/z = 201 $[M+H]^+$

## Beispiel 151B:

*tert.*-Butyl {5-oxo-1-[5-oxo-6-{[(2*S*)-1,1,1-trifluorobutan-2-yl]carbamoyl}-8-(2,4,6-trifluorophenyl)-5,8-dihydro-1,8-naphthyridin-2-yl]pyrrolidin-3-yl}carbamat(Diastereomerengemisch)

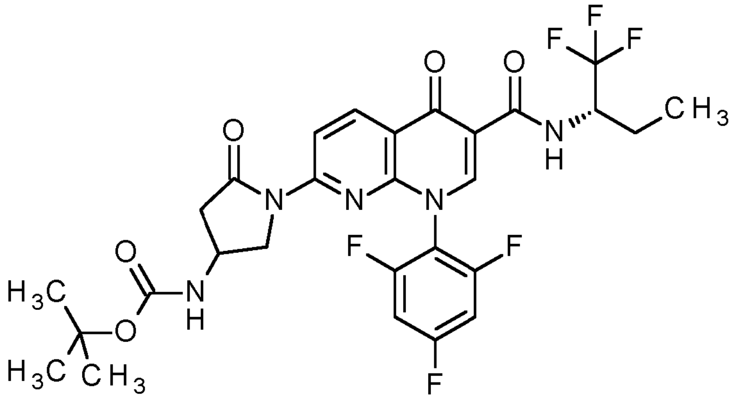

Kaliumcarbonat (17.9 mg, 129 µmol), Palladium(II)acetat (3.87 mg, 17.2 µmol) und 9,9-Dimethyl-4,5-bis-(diphenylphosphino)-xanthen (9.98 mg, 17.2 µmol) wurden in 4.0 ml Dioxan unter Argon für 10 Minuten bei RT gerührt. Dann wurden die Verbindung aus Beispiel 115A (40.0 mg, 86.2 µmol) und die Verbindung aus Beispiel 151A (20.7 mg, 103 µmol) zugegeben und die Mischung für 4 h bei 80°C gerührt. Der Ansatz wurde direct mittels präp. RP-HPLC (Säule: Reprosil 125x30; 10µ, Fluss: 50 mL/min, MeCN/Wasser/0,1%TFA) gereinigt. Die flüchtigen Bestandteile wurden im Vakuum entfernt und der Rückstand im Hochvakuum getrocknet. Man erhielt 30.7 mg (79 % Reinheit, 45 % d. Th.) der Titelverbindung.
LC-MS (Methode 1): $R_t$ = 1.20 min; MS (ESIpos): m/z = 628 $[M+H]^+$.

**Beispiel 151C:**

7-[4-Amino-2-oxopyrrolidin-1-yl]-4-oxo-*N*-[(2*S*)-1,1,1-trifluorbutan-2-yl]-1-(2,4,6-trifluorphenyl)-1,4-dihydro-1,8-naphthyridin-3-carbonsäureamid (Bis)trifluoracetat (Diastereomerengemisch)

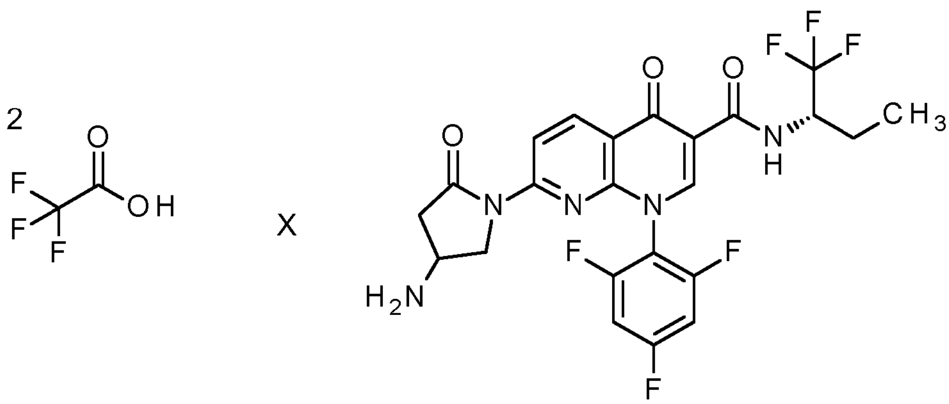

Die Verbindung aus Beispiel 151B (30.7 mg, 79 % Reinheit, 38.5 µmol) wurde in 2.0 ml Dichlormethan gelöst, mit Trifluoressigsäure (150 µl, 1.9 mmol) versetzt und 5 h bei RT gerührt. Die flüchtigen Bestandteile wurden im Vakuum entfernt und der Rückstand mittels präp. RP-HPLC (Säule: Reprosil 125x30; 10µ, Fluss: 50 mL/min, MeCN/Wasser/0,1%TFA) gereinigt. Die flüchtigen Bestandteile wurden im Vakuum entfernt und der Rückstand im Hochvakuum getrocknet. Man erhielt 25.7 mg (95 % Reinheit, 84 % d. Th.) der Titelverbindung.
LC-MS (Methode 1): $R_t$ = 0.72 min; MS (ESIpos): m/z = 528 $[M+H]^+$

**Beispiel 152A:**

*N*-[(5-Oxopyrrolidin-3-yl)methyl]acetamid (Racemat)

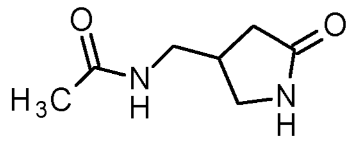

4-(Aminomethyl)pyrrolidin-2-on Hydrochlorid (Racemat) (30.0 mg, 199 μmol) wurde in 1.0 ml Dichlormethan vorgelegt und mit Triethylamin (83 μl, 600 μmol) versetzt. Die Reaktionsmischung wurde mit Acetylchlorid (17 μl, 240 μmol) bei 0°C versetzt und über Nacht bei RT nachgerührt. Die organische Phase wurde einmal mit Wasser gewaschen und über Magnesiumsulfat getrocknet. Die flüchtigen Bestandteile wurden im Vakuum entfernt und der Rückstand im Hochvakuum getrocknet. Man erhielt 11.7 mg der Titelverbindung, die ohne weitere Reinigung sofort in der nächsten Reaktionsstufe eingesetzt wurde.

**Beispiel 153A:**

*N*-Benzyl-1,1,1,2,2-pentafluorpentan-3-amin (Racemat)

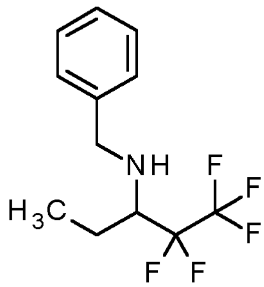

Zu einer Lösung von 2.00 g (11.4 mmol) 1,1,1,2,2-Pentafluorpentan-3-on in 10 ml Dichlormethan wurden bei 0°C 5.03 ml (17.0 mmol) Titantetraisopropoxid und 2.48 ml (22.7 mmol) Benzylamin gegeben. Es wurde 90 min bei RT nachgerührt bevor erneut auf 0°C abgekühlt wurde. Anschließend wurden 2.00 g (31.8 mmol) Natriumcyanoborhydrid, 36 ml Methanol und 3Å Molsieb zugegeben. Es wurde auf RT erwärmt und 2d nachgerührt. Die Reaktionslösung wurde dann mit wenig Wasser und Essigsäureethylester versetzt und filtriert. Das Filtrat wurde zweimal mit gesättigter wässriger Natriumhydrogencarbonat-Lösung und einmal mit gesättigter wässriger Natriumchlorid-Lösung gewaschen. Die organische Phase wurde über Natriumsulfat getrocknet, filtriert und das Lösungsmittel unter reduziertem Druck entfernt. Der Rückstand wurde mittels Normalphasenchromatographie (Essigsäureethylester/Cyclohexan 1/20) gereinigt und 989 mg (25% d. Th., 76% Reinheit) der Titelverbindung erhalten.
LC-MS (Methode 1): $R_t$ = 1.27 min; MS (ESIpos): m/z = 268 $[M+H]^+$
$^1$H NMR (400 MHz, DMSO-d6): δ [ppm] = 7.21-7.36 (m, 5H), 3.73-3.85 (m, 2H), 3.05-3.20 (m, 1H), 1.63-1.75 (m, 1H), 1.49-1.61 (m, 1H), 1.15-1.20 (m, 1H), 0.96 (t, 3H).

**Beispiel 153B:**

1,1,1,2,2-Pentafluorpentan-3 -aminhydrochlorid (Racemat)

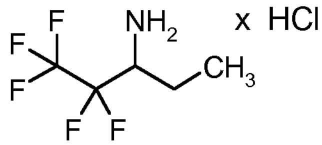

Zu einer Lösung von 980 mg (2.75 mmol, 75% Reinheit) der Verbindung aus Beispiel 153A in 11.3 ml Methanol wurden 75 mg Palladium auf Kohle (10%) gegeben und für 6h bei Normaldruck und Raumtemperatur hydriert. Anschließend wurde die Reaktionsmischung durch einen Milliporefilter filtriert und das Lösungsmittel unter reduziertem Druck

entfernt. Die Vorlage mit dem abdestillierten Lösungsmittel wurde anschließend in einen Kolben überführt und mit 4N wässriger Salzsäure in Dioxan versetzt und erneut eingeengt. Der Rückstand wurde mit Diethylether verrührt, der Niederschlag abgesaugt und im Hochvakuum getrocknet. Es wurden 379 mg (65% d. Th., 100% Reinheit) der Titelverbindung erhalten.
$^1$H NMR (400 MHz, DMSO-d6): δ [ppm] = 8.97 (br. s, 3H), 4.16-4.28 (m, 1H), 1.67-1.94 (m, 2H), 1.05 (t, 3H).

**Beispiel 154A:**

7-Chlor-*N*-(1,1,1,3,3,3-hexafluorpropan-2-yl)-4-oxo-1-(2,4,6-trifluorphenyl)-1,4-dihydro-1,8-naphthyridin-3-carboxamid

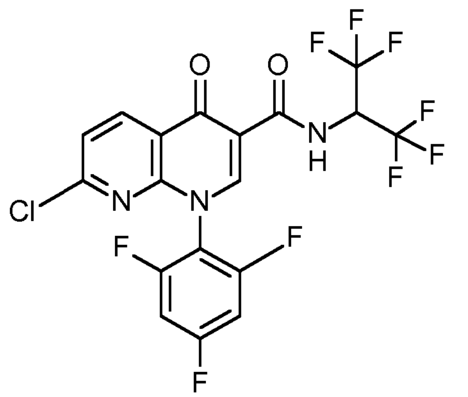

Zu einer Lösung aus 500 mg (1.41 mmol) der Verbindung aus Beispiel 100B, 259 mg (1.55 mmol) 1,1,1,3,3,3-Hexafluorpropan-2-amin und 740 μl (4.20 mmol) DIPEA in 13 ml Essigsäureethylester wurden 3.30 ml (5.60 mmol) 2,4,6-Tripropyl-1,3,5,2,4,6-trioxatriphosphorinan-2,4,6-trioxid (T3P, 50% in DMF) zugetropft. Es wurde über Nacht bei 80°C nachgerührt. Die Reaktionsmischung wurde auf Wasser und Essigsäureethylester gegossen und die Phasen getrennt. Die organische Phase wurde dreimal mit Wasser und einmal mit gesättigter, wässriger Natriumchlorid-Lösung gewaschen, über Natriumsulfat getrocknet, filtriert und das Lösungsmittel unter reduziertem Druck entfernt. Der Rückstand wurde in wenig Acetonitril gelöst, über einen Milliporefilter filtriert und mittels präparativer HPLC (Säule: Chromatorex C18, 10 μm, 125*40 mm, Lösungsmittel: Acetonitril, Wasser, 0.1% Ameisensäure) gereinigt. Die Substanz wurde aus Acetonitril umkristallisiert, abgesaugt, mit etwas Acetonitril nachgewaschen und nachgetrocknet. Es wurden 432 mg (61% d. Th., 100% Reinheit) der Titelverbindung erhalten.
LC-MS (Methode 3): $R_t$ = 2.39 min; MS (ESIpos): m/z = 504 $[M+H]^+$
$^1$H NMR (400 MHz, DMSO-$d_6$): δ ppm = 10.76 (d, 1 H), 9.26 (s, 1 H), 8.78 (d, 1 H), 7.81 (d, 1 H), 7.59 - 7.66 (m, 2 H), 6.36 - 6.47 (m, 1 H).

**Beispiel 155A:**

7-[7-Hydroxy-5-azaspiro[2.4]hept-5-yl]-4-oxo-1-(2,4,6-trifluorphenyl)-1,4-dihydro-1,8-naphthyridin-3-carbonsäure (Racemat)

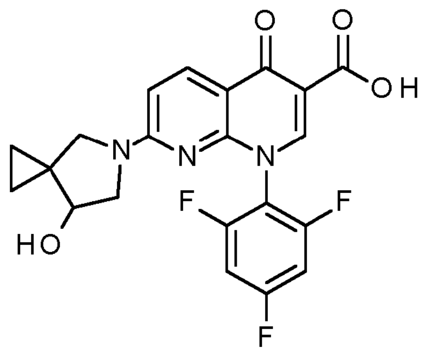

Gemäß AAV3 wurden 500 mg (1.41 mmol) der Verbindung aus Beispiel 100B mit 239 mg (1.55 mmol, 97% Reinheit) 5-Azaspiro[2.4]heptan-7-ol Hydrochlorid in Gegenwart von 860 µl (4.90 mmol) DIPEA in 14 ml DMF zur Reaktion gebracht. Es wurde mit Wasser, 1 M wässriger Salzsäure und Essigsäureethylester verdünnt. Die Phasen wurden getrennt und die wässrige Phase dreimal mit Essigsäureethylester extrahiert. Die vereinigten organischen Phasen wurden mit gesättigter, wässriger Natriumchloridlösung gewaschen, über Natriumsulfat getrocknet, filtriert und das Lösungsmittel unter reduziertem Druck entfernt. Das Rohprodukt wurde mittels präparativer HPLC (Säule: Chromatorex C18, 10 µm, 125*40 mm, Lösungsmittel: Acetonitril, Wasser, 0.1% Ameisensäure) gereinigt und 422 mg (63% d. Th., 90% Reinheit) der Titelverbindung erhalten.

LC-MS (Methode 3): $R_t$ = 1.60 min; MS (ESIpos): m/z = 432 $[M+H]^+$

$^1$H NMR (400 MHz, DMSO-$d_6$): δ ppm = 15.19 (br s, 1 H), 8.99 - 9.04 (m, 1 H), 8.31 (d, 1 H), 7.51 - 7.62 (m, 2 H), 6.89 (d, 0.40 H), 6.76 (d, 0.60 H), 5.04 (br s, 1 H), 3.61 - 3.80 (m, 2 H), 3.13 - 3.53 (m, 2.60 H), 2.89 (d, 0.40 H), 0.78 - 0.87 (m, 1 H), 0.45 - 0.63 (m, 3 H).

## Beispiel 156A

7-[(3*R*,4*S*)-3,4-Dihydroxypyrrolidin-1-yl]-4-oxo-1-(2,4,6-trifluorphenyl)-1,4-dihydro-1,8-naphthyridin-3-carbonsäure

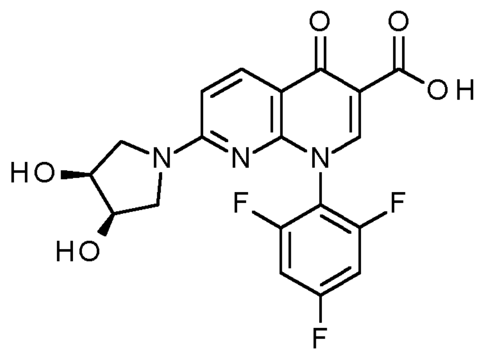

Gemäß AAV3 wurden 500 mg (1.41 mmol) der Verbindung aus Beispiel 100B mit 236 mg (1.69 mmol) (3*R*,4*S*)-Pyrrolidin-3,4-diol-Hydrochlorid in Gegenwart von 860 µl (4.90 mmol) DIPEA in 6.3 ml DMF zur Reaktion gebracht. Es wurde mit Wasser, 1 M wässriger Salzsäure und Essigsäureethylester verdünnt. Die Phasen wurden getrennt und die wässrige Phase dreimal mit Essigsäureethylester extrahiert. Die vereinigten organischen Phasen wurden mit gesättigter, wässriger Natriumchloridlösung gewaschen, über Natriumsulfat getrocknet, filtriert und das Lösungsmittel unter reduziertem Druck entfernt. Das Rohprodukt wurde aus Essigsäureethylester und Cyclohexan kristallisiert, abgesaugt, mit etwas Essigsäureethylester/Cyclohexan nachgewaschen und nachgetrocknet. Es wurden 459 mg (77% d. Th.) der Titelverbindung erhalten.

LC-MS (Methode 3): $R_t$ = 1.19 min; MS (ESIpos): m/z = 422 $[M+H]^+$

$^1$H NMR (400 MHz, DMSO-$d_6$): δ ppm = 15.21 (s, 1 H), 9.02 (s, 1 H), 8.29 (d, 1 H), 7.54 - 7.62 (m, 2 H), 6.84 (d, 1 H), 5.07 (d, 1 H), 4.97 (d, 1 H), 4.10 - 4.20 (m, 1 H), 4.00 - 4.07 (m, 1 H), 3.63 (dd, 1 H), 3.24 (dd, 1 H), 3.01 (dd, 1 H).

**Beispiel 157A**

7-[4-(Methoxycarbonyl)piperazin-1-yl]-4-oxo-1-(2,4,6-trifluorphenyl)-1,4-dihydro-1,8-naphthyridin-3-carbonsäure

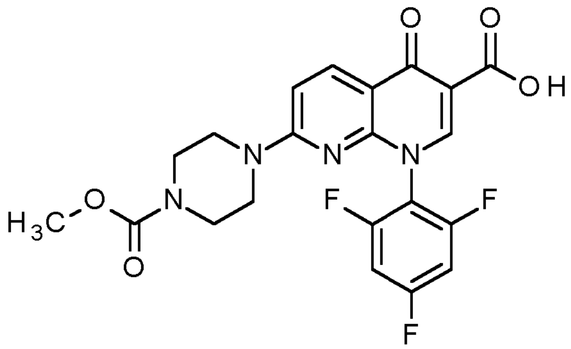

Gemäß AAV3 wurden 500 mg (1.41 mmol) der Verbindung aus Beispiel 100B mit 244 mg (1.69 mmol) Methyl-piperazin-1-carboxylat in Gegenwart von 860 µl (4.90 mmol) DIPEA in 6.3 ml DMF zur Reaktion gebracht. Es wurde mit Acetonitril, etwas Wasser und Ameisensäure verdünnt. Die Substanz wurde mittels präparativer HPLC (Acetonitril / Wasser/ 0.1 % Ameisensäure) gereinigt. Es wurden 380 mg (58% d. Th., 98% Reinheit) der Titelverbindung erhalten.
LC-MS (Methode 3): $R_t$ = 1.63 min; MS (ESIpos): m/z = 463 $[M+H]^+$
$^1H$ NMR (400 MHz, DMSO-$d_6$): δ ppm = 15.06 (br s, 1 H), 9.04 (s, 1 H), 8.35 (d, 1 H), 7.58 (t, 2 H), 7.21 (d, 1 H), 3.61 (s, 3 H), 3.51 - 3.59 (m, 4 H), 3.37 - 3.44 (m, 4 H).

**Beispiel 158A**

Ethyl-4-{[(benzyloxy)carbonyl]amino}-3-oxobutanoat

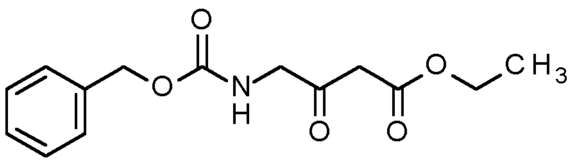

Eine Lösung aus 15.0 g (71.7 mmol) *N*-[(Benzyloxy)carbonyl]glycin in 534 ml THF wurde mit 9.24 g (57.0 mmol) Carbonyldiimidazol (CDI) versetzt und für 2.5h bei RT nachgerührt. Anschließend wurden unter Eisbadkühlung 9.76 g (57.4 mmol) Kalium-3-ethoxy-3-oxopropanoat und 4.95 g (52.0 mmol) Magnesiumchlorid zugegeben. Nach vollständiger Zugabe wurde für 48h bei 50°C nachgerührt. Das Lösungsmittel wurde unter reduziertem Druck entfernt, der Rückstand mit Essigsäureethylester und gesättigter wässriger Ammoniumchlorid-Lösung aufgenommen und die Phasen getrennt. Die organische Phase wurde mit gesättigter wässriger Natriumchlorid-Lösung gewaschen, über Natriumsulfat getrocknet, filtriert und das Lösungsmittel unter reduziertem Druck entfernt. Der Rückstand wurde mittels Normalphasenchromatographie (Essigsäureethylester-Cyclohexan-Gradient) gereinigt und 12.7 g (60% d. Th., 95% Reinheit) der Titelverbindung erhalten.
LC-MS (Methode 1): $R_t$ = 0.83 min; MS (ESIneg): m/z = 278 $[M-H]^-$
$^1H$ NMR (400 MHz, DMSO-d6): δ [ppm] = 7.56 (brt, 1H), 7.25-7.41 (m, 5H), 5.04 (s, 2H), 4.09 (q, 2H), 3.97 (d, 2H), 3.60 (s, 2H), 1.19 (t, 3H).

**Beispiel 158B**

Ethyl-4- {[(benzyloxy)carbonyl]amino} -2-methyl-3 -oxobutanoat (Racemat)

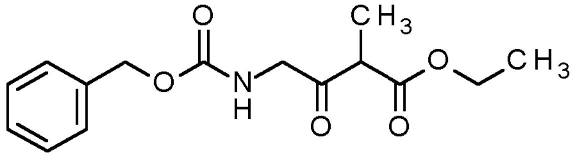

Eine Suspension aus 1.00 g (3.58 mmol) 4-{[(Benzyloxy)carbonyl]amino}-3-oxobutansäureethylester, 669 µl (10.7 mmol) Iodmethan und 990 mg (7.16 mmol) Kaliumcarbonat in 15 ml Aceton wurde für 2h bei 50°C in der Mikrowelle zur Reaktion gebracht. Es wurde für weitere 2h bei 45°C unter Reaktionskontrolle in der Mikrowelle bestrahlt. Die Reaktionsmischung wurde auf Wasser gegossen und dreimal mit Essigsäureethylester extrahiert. Die vereinigten organischen Phasen wurden mit gesättigter wässriger Natriumchlorid-Lösung gewaschen, über Natriumsulfat getrocknet, filtriert und das Lösungsmittel unter reduziertem Druck entfernt. Der Rückstand wurde in wenig Acetonitril gelöst, über einen Milliporefilter filtriert und in zwei Läufen mittels präparativer HPLC (Säule: Chromatorex C18, 10 µm, 125*40 mm, Lösungsmittel: Acetonitril, Wasser, 0.1% Ameisensäure) getrennt. Es wurden 536 mg (51% d. Th., 100% Reinheit) der Titelverbindung erhalten.
LC-MS (Methode 1): $R_t$ = 0.87 min; MS (ESIneg): m/z = 292 $[M-H]^-$
$^1$H NMR (400 MHz, DMSO-d6): δ [ppm] = 7.57 (brt, 1H), 7.24-7.40 (m, 5H), 5.04 (s, 2H), 4.09 (q, 2H), 4.03 (d, 2H), 3.80 (q, 1H), 1.22-1.09 (m, 6H).

**Beispiel 158C**

Ethyl-4- {[(benzyloxy)carbonyl] amino} -3 -hydroxy-2-methylbutanoat (Diastereomerengemisch)

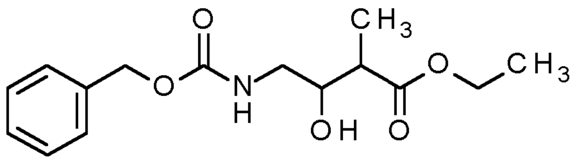

Zu einer Lösung von 533 mg (1.82 mmol) 4-{[(Benzyloxy)carbonyl]amino}-2-methyl-3-oxobutansäureethylester in 9.2 ml Methanol wurden bei -78°C 96.2 mg (2.54 mmol) Natriumborhydrid gegeben. Es wurde langsam unter Reaktionskontrolle auf -15°C erwärmt. Bei -15°C wurde die Reaktion durch Zugabe von gesättigter wässriger Ammoniumchlorid-Lösung beendet. Es wurde dreimal mit Essigsäureethylester extrahiert. Die vereinigten organischen Phasen wurden mit gesättigter wässriger Natriumchlorid-Lösung gewaschen, über Natriumsulfat getrocknet, filtriert und das Lösungsmittel unter reduziertem Druck entfernt. Der Rückstand wurde in etwas Acetonitril aufgenommen und in zwei Läufen mittels präparativer HPLC (Säule: Chromatorex C18, 10 µm, 125*40 mm, Lösungsmittel: Acetonitril, Wasser, 0.1% Ameisensäure) gereinigt. Es wurden 398 mg (74% d. Th., 100% Reinheit) der Titelverbindung erhalten.
LC-MS (Methode 1): $R_t$ = 0.80 min; MS (ESIpos): m/z = 296 $[M+H]^+$
$^1$H NMR (400 MHz, DMSO-d6): δ [ppm] = 7.26-7.45 (m, 5H), 7.20-7.25 (m, 0.3H), 7.11 (br t, 0.7H), 5.01 (s, 2H), 4.90-4.97 (m, 1H), 3.98-4.08 (m, 2H), 3.81-3.88 (m, 0.3H), 3.63-3.71 (m, 0.7H), 3.11-3.20 (m, 0.7H), 2.93-3.07 (m, 1.3H), 2.40-2.49 (m, 1H), 1.17 (t, 3H), 1.00-1.05 (m, 3H).

**Beispiel 158D**

4-Hydroxy-3-methylpyrrolidin-2-on (Diastereomerengemisch)

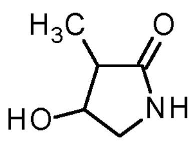

Zu einer Lösung von 397 mg (1.34 mmol) 4-{[(Benzyloxy)carbonyl]amino}-3-hydroxy-2-methylbutansäureethylester in 7.2 ml Methanol wurden 40 mg Palladium auf Kohle (10%) gegeben und für 6h bei Normaldruck und Raumtemperatur hydriert. Anschließend wurde die Reaktionsmischung durch einen Milliporefilter filtriert und das Lösungsmittel unter reduziertem Druck entfernt. Es wurden 211 mg (quantitativ) der Titelverbindung erhalten, welche ohne weitere Aufreinigung im nächsten Schritt eingesetzt wurde.
$^1$H-NMR (400 MHz, DMSO-d6) δ [ppm]: -0.008 (0.80), 0.936 (15.27), 0.954 (16.00), 0.985 (4.68), 1.002 (5.72), 1.010 (9.61), 1.021 (1.34), 1.028 (9.33), 1.038 (1.50), 1.055 (2.69), 1.073 (1.35), 1.158 (4.92), 1.176 (10.33), 1.194 (5.03), 2.004 (1.14), 2.022 (1.44), 2.039 (1.10), 2.225 (1.59), 2.239 (1.71), 2.243 (1.63), 2.257 (1.57), 2.479 (1.15), 2.854 (1.16), 2.868 (1.22), 2.878 (1.29), 2.893 (1.32), 2.958 (1.71), 2.962 (2.91), 2.966 (1.63), 2.984 (1.89), 2.988 (3.22), 2.992 (1.80), 3.317 (5.99), 3.329 (6.86), 3.333 (5.11), 3.336 (4.95), 3.343 (6.27), 3.350 (4.61), 3.355 (6.03), 3.360 (3.76), 3.374 (3.03), 3.377 (2.85), 3.414 (1.25), 3.431 (1.52), 3.449 (1.35), 3.847 (1.23), 3.862 (1.17), 4.018 (1.05), 4.035 (2.89), 4.053 (2.81), 4.071 (0.94), 4.194 (1.14), 4.207 (1.95), 4.219 (1.09), 7.419 (1.14).

**Beispiel 159A**

7-Chlor-*N*-(1,1,1,3,3,3-hexafluor-2-methylpropan-2-yl)-4-oxo-1-(2,4,6-trifluorphenyl)-1,4-dihydro-1,8-naphthyridin-3-carboxamid

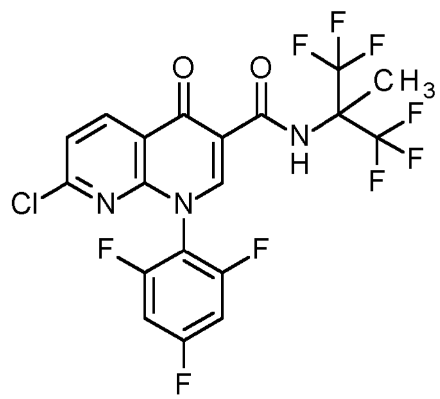

Zu einer Lösung aus 150 mg (423 μmol) der Verbindung aus Beispiel 100B, 91.9 mg (508 μmol) 1,1,1,3,3,3-Hexafluor-2-methylpropan-2-amin und 220 μl (1.30 mmol) DIPEA in 1.6 ml Essigsäureethylester wurden 740 μl (1.30 mmol) 2,4,6-Tripropyl-1,3,5,2,4,6-trioxatriphosphorinan-2,4,6-trioxid (T3P, 50% in DMF) zugetropft. Es wurde über Nacht bei 80°C nachgerührt und noch 370 μl (0.65 mmol) 2,4,6-Tripropyl-1,3,5,2,4,6-trioxatriphosphorinan-2,4,6-trioxid (T3P, 50% in DMF) wurden zugegeben. Die Reaktionsmischung wurde 64 h bei 80°C nachgerührt und das Lösungsmittel wurde anschließend unter reduziertem Druck entfernt. Der Rückstand wurde in Acetonitril, etwas Wasser und Ameisensäure gelöst, über einen Milliporefilter filtriert und mittels präparativer HPLC (Säule: Chromatorex C18, 10 μm, 125*40 mm, Lösungsmittel: Acetonitril, Wasser, 0.1% Ameisensäure) gereinigt. Es wurden 76.3 mg (35% d. Th.) der Titelverbindung erhalten.
LC-MS (Methode 3): $R_t$ = 2.46 min; MS (ESIpos): m/z = 518 $[M+H]^+$
$^1$H NMR (400 MHz, DMSO-$d_6$): δ ppm = 10.96 (s, 1 H), 9.18 (s, 1 H), 8.79 (d, 1 H), 7.80 (d, 1 H), 7.62 (t, 2 H), 2.08 (s, 3 H).

**Beispiel 160A**

7-Chlor-4-oxo-1-(2,4,6-trifluorphenyl)-1,4-dihydro-1,8-naphthyridin-3-carbonylchlorid

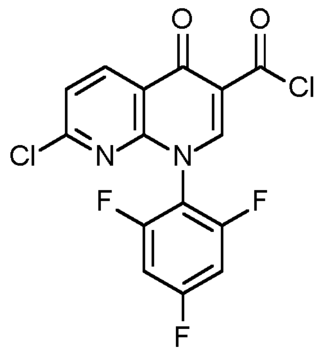

Zu einer Lösung von 800 mg (2.26 mmol) der Verbindung aus Beispiel 100B in 18 ml THF wurden 490 µl (6.70 mmol) Thionylchlorid gegeben und 2h unter Rückfluss nachgerührt und anschließend alle flüchtigen Komponenten unter reduziertem Druck entfernt. Das Rohprodukt wurde ohne weitere Aufarbeitung im nächsten Schritt eingesetzt (quantitative Umsetzung wurde angenommen).

**Beispiel 160B**

7-Chlor-*N*-(2,6-dichlorphenyl)-4-oxo-1-(2,4,6-trifluorphenyl)-1,4-dihydro-1,8-naphthyridin-3-carboxamid

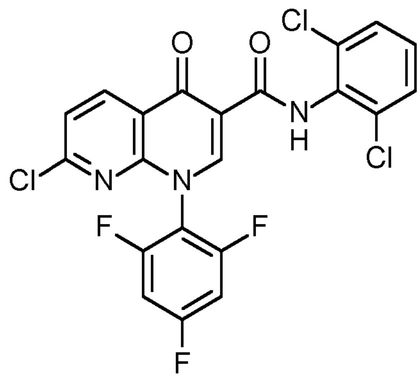

Zu einer Lösung von 840 mg (2.25 mmol) 7-Chlor-4-oxo-1-(2,4,6-trifluorphenyl)-1,4-dihydro-1,8-naphthyridin-3-carbonylchlorid in 47 ml Dichlormethan wurden bei RT 940 µl (6.80 mmol) Triethylamin und 438 mg (2.70 mmol) 2,6-Dichloranilin gegeben. Es wurde für 30 min bei RT und über Nacht bei 50°C nachgerührt. Die Reaktionsmischung wurde eingeengt und in Dichlormethan aufgenommen, zweimal mit 1 M wässriger Salzsäure gewaschen, über Magnesiumsulfat getrocknet, filtriert und das Lösungsmittel unter reduziertem Druck entfernt. Das Rohprodukt wurde mittels Normalphasenchromatographie (Essigsäureethylester/Cyclohexan = 1/1) gereinigt. Es wurden 544 mg (48% d. Th., 99% Reinheit) der Titelverbindung erhalten.
LC-MS (Methode 3): $R_t$ = 2.35 min; MS (ESIpos): m/z = 498 $[M+H]^+$
$^1$H NMR (400 MHz, DMSO-$d_6$): δ ppm = 11.34 (s, 1 H), 9.22 (s, 1 H), 8.81 (d, 1 H), 7.81 (d, 1 H), 7.58 - 7.65 (m, 4 H), 7.36 - 7.43 (m, 1 H).

**Beispiel 161A**

7-(3-Methoxy-3-methylazetidin-1-yl)-4-oxo-1-(2,4,6-trifluorphenyl)-1,4-dihydro-1,8-naphthyridin-3-carbonsäure

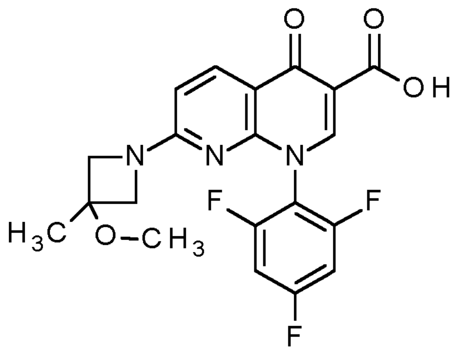

Gemäß AAV3 wurden 91.3 mg (257 µmol) der Verbindung aus Beispiel 100B mit 42.5 mg (309 µmol) 3-Methoxy-3-methylazetidin-Hydrochlorid in Gegenwart von 160 µl (900 µmol) DIPEA in 1.2 ml DMF zur Reaktion gebracht. Die Reaktion wurde durch Zugabe von Acetonitril, etwas Wasser und Ameisensäure beendet, durch einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 72.4 mg (63% d. Th., 93% Reinheit) der Titelverbindung erhalten.
LC-MS (Methode 3): $R_t$ = 1.83 min; MS (ESIpos): m/z = 420 $[M+H]^+$
$^1H$ NMR (400 MHz, DMSO-$d_6$): δ ppm = 15.14 (brs, 1 H), 9.01 (s, 1 H), 8.32 (d, 1 H), 7.52 - 7.60 (m, 2 H), 6.70 (d, 1 H), 3.48 - 4.18 (m, 4 H), 3.16 (s, 3 H), 1.41 (s, 3 H).

**Beispiel 162A**

(3*S*,4*S*)-1-Benzyl-3,4-bis{[tert-butyl(dimethyl)silyl]oxy}pyrrolidin-2,5-dion

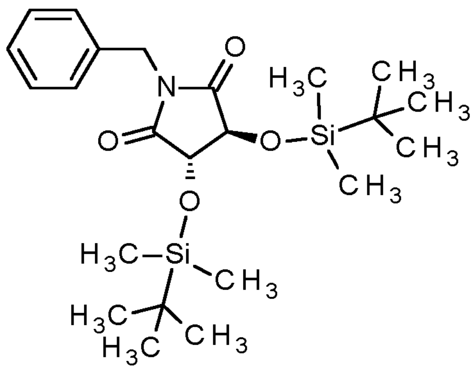

Zu einer Lösung von 1.03 g (4.65 mmol) (3*S*,4*S*)-1-Benzyl-3,4-dihydroxypyrrolidin-2,5-dion und 949 mg (13.9 mmol) Imidazol in 19.2 ml DMF wurden 1.76 g (11.7 mmol) tert-Butyldimethylsilylchlorid zugegeben und die Reaktionsmischung wurde 3 h bei Raumtemperatur nachgerührt. Die Reaktion wurde mit Wasser versetzt und dreimal mit Dichlormethan extrahiert. Die organische Phase wurde mit Wasser gewaschen, über Magnesiumsulfat getrocknet, filtriert und das Lösungsmittel unter reduziertem Druck entfernt. Der Rückstand wurde mittels Normalphasenchromatographie (Essigsäureethylester/Cyclohexan = 1/4) gereinigt. Es wurden 1.57 g (75% d. Th.) der Titelverbindung erhalten.
$^1H$ NMR (400 MHz, DMSO-d6): δ [ppm] = 7.25-7.36 (m, 5H), 4.80 (s, 2H), 4.53 (dd, 2H), 0.91 (s, 18H), 0.17 (s, 6H), 0.13 (s, 6H).

**Beispiel 162B**

(3*R*,4*R*)-1-Benzyl-3,4-bis{[tert-butyl(dimethyl)silyl]oxy}pyrrolidin

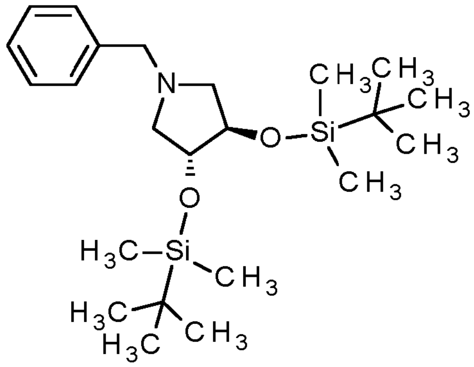

Zu einer Lösung von 1.57 g (3.49 mmol) (3*S*,4*S*)-1-Benzyl-3,4-bis{[*tert*-butyl(dimethyl)silyl]oxy}pyrrolidin-2,5-dion in 11.3 ml THF bei 0°C wurden in 9.1 ml (1.00 M, 9.10 mmol) Boran-Tetrahydrofuran-Komplex zugetropft und die Reaktionsmischung wurde 2.5 h bei Raumtemperatur und 2 h unter Rückfluss nachgerührt. Das Lösungsmittel wurde am Rotationsverdampfer entfernt und der Rückstand wurde in 7 ml Ethanol gelöst. Das Gemisch wurde 21 h unter Rückfluss gerührt. Anschließend wurde das Gemisch am Rotationsverdampfer eingedampft und mit Wasser und Diethylether versetzt. Die organische Phase wurde dreimal mit Diethylether extrahiert. Die vereinten organischen Phasen wurden mit gesättigter, Natriumchlorid-Lösung gewaschen, über Natriumsulfat getrocknet und eingedampft. Der Rückstand wurde mittels Normalphasenchromatographie (Essigsäureethylester-Cyclohexan-Gradient) gereinigt. Es wurden 711 mg (46% d. Th., 95% Reinheit) der Titelverbindung erhalten.
LC-MS (Methode 1): $R_t$ = 1.07 min; MS (ESIpos): m/z = 422 $[M+H]^+$
$^1H$ NMR (400 MHz, $CDCl_3$): δ [ppm] = 7.22-7.35 (m, 5H), 4.07-4.15 (m, 2H), 3.62 (dd, 2H), 2.87 (dd, 2H), 2.43-2.48 (m, 2H), 0.87-0.90 (m, 18H), 0.06 (s, 6H), 0.01-0.05 (m, 6H).

**Beispiel 162C**

(3*R*,4*R*)-3,4-Bis{[tert-butyl(dimethyl)silyl]oxy}pyrrolidin

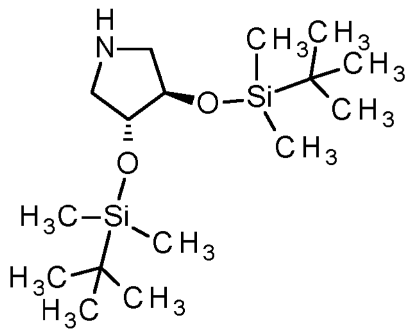

Zu einer Lösung von 711 mg (1.69 mmol) (3*R*,4*R*)-1-Benzyl-3,4-bis{[*tert*-butyl(dimethyl)silyl]oxy}pyrrolidin in 7.7 ml Ethanol wurden 71.1 mg (506 μmol) Palladium(II)hydroxid gegeben und für 2.5 h bei Normaldruck und Raumtemperatur hydriert. Anschließend wurde die Reaktionsmischung durch Kieselgur filtriert und das Lösungsmittel wurde unter reduziertem Druck entfernt. Es wurden 582 mg (quantitativ) der Titelverbindung erhalten, welche ohne weitere Aufreinigung im nächsten Schritt eingesetzt wurde.
$^1H$ NMR (400 MHz, DMSO-d6): δ [ppm] = 4.26-4.39 (m, 1H), 3.90-3.94 (m, 2H), 3.40-3.49 (m, 1H), 2.94-3.01 (m, 2H), 0.85 (s, 18H), 0.05 (s, 6H), 0.04 (s, 6H).

**Beispiel 163A**

Ethyl-4- {[(benzyloxy)carbonyl] amino}-3 -oxopentanoat (Racemat)

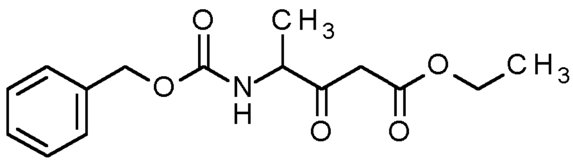

Eine Lösung aus 15.0 g (71.7 mmol) *N*-[(Benzyloxy)carbonyl]-DL-alanin in 200 ml THF wurde mit 3.46 g (21.3 mmol) Carbonyldiimidazol (CDI) versetzt und für 2.5 h bei RT nachgerührt. Anschließend wurden unter Eisbadkühlung 3.63 g (21.3 mmol) Kalium-3-ethoxy-3-oxopropanoat und 1.86 g (19.5 mmol) Magnesiumchlorid zugegeben. Nach vollständiger Zugabe wurde über Nacht bei 50°C nachgerührt. Essigsäureethylester und gesättigter wässriger Ammoniumchlorid-Lösung wurden hinzugefügt und die Phasen getrennt. Die organische Phase wurde mit gesättigter, wässriger Natriumchlorid-Lösung gewaschen, über Natriumsulfat getrocknet, filtriert und das Lösungsmittel unter reduziertem Druck entfernt. Der Rückstand wurde mittels Normalphasenchromatographie (Essigsäureethylester-Cyclohexan-Gradient) gereinigt und 2.90 g (37% d. Th.) der Titelverbindung erhalten.
LC-MS (Methode 3): $R_t$ = 1.63 min; MS (ESIneg): m/z = 292 $[M-H]^-$
$^1H$ NMR (400 MHz, DMSO-$d_6$): δ ppm = 7.74 (br d, 1H), 7.25-7.43 (m, 5H), 5.04 (s, 2H), 4.12-4.21 (m, 1H), 4.00-4.12 (m, 2H), 3.54-3.67 (m, 2H), 1.14-1.22 (m, 6H).

**Beispiel 163B**

Ethyl-4-{[(benzyloxy)carbonyl]amino}-3-hydroxy-pentanoat (Diastereomerengemisch)

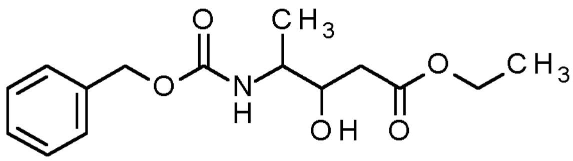

Zu einer Lösung von 1.0 g (3.41 mmol) Ethyl-4-{[(benzyloxy)carbonyl]amino}-3-oxopentanoat in 18 ml Methanol wurden bei 0°C 181 mg (4.77 mmol) Natriumborhydrid gegeben. Es wurde langsam auf RT erwärmt und für 2 h bei RT nachgerührt. Die Reaktion wurde durch Zugabe von gesättigter wässriger Ammoniumchlorid-Lösung beendet. Die organische Phase wurde dreimal mit Essigsäureethylester extrahiert. Die vereinigten organischen Phasen wurden mit gesättigter, wässriger Natriumchlorid-Lösung gewaschen, über Magnesiumsulfat getrocknet, filtriert und das Lösungsmittel unter reduziertem Druck entfernt. Der Rückstand wurde in etwas Acetonitril aufgenommen und mittels präparativer HPLC (Säule: Chromatorex C18, 10 μm, 125*40 mm, Lösungsmittel: Acetonitril, Wasser, 0.1% Ameisensäure) gereinigt. Es wurden 398 mg (40% d. Th.) der Titelverbindung erhalten.
LC-MS (Methode 1): $R_t$ = 0.79 min; MS (ESIpos): m/z = 296 $[M+H]^+$
$^1H$ NMR (400 MHz, DMSO-$d_6$): δ ppm = 7.27-7.39 (m, 5H), 7.08 (br d, 0.75H), 6.97 (br d, 0.25H), 4.97-5.04 (m, 2H), 4.89-4.97 (m, 1H), 4.04 (q, 2H), 3.84-3.90 (m, 0.25H), 3.72-3.80 (m, 0.75H), 3.55-3.65 (m, 0.25H), 3.38-3.50 (m, 0.75H), 2.39-2.47 (m, 2H), 2.16-2.25 (m, 1H), 1.17 (t, 3H), 0.98-1.06 (m, 3H).

**Beispiel 163C**

4-Hydroxy-5-methylpyrrolidin-2-on (Diastereomerengemisch)

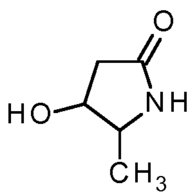

Zu einer Lösung von 398 mg (1.35 mmol) Ethyl-4-{[(benzyloxy)carbonyl]amino}-3-hydroxypentanoat in 6.8 ml Methanol wurden 34 mg Palladium auf Kohle (10%) gegeben und für 5 h bei Normaldruck und Raumtemperatur hydriert. Anschließend wurde die Reaktionsmischung durch einen Milliporefilter filtriert und das Lösungsmittel unter reduziertem Druck entfernt. Es wurden 152 mg (98% d. Th.) der Titelverbindung erhalten, welche ohne weitere Aufreinigung im nächsten Schritt eingesetzt wurde.

$^{1}$H-NMR (400 MHz, DMSO-d6) $\delta$ [ppm]: 0.922 (1.20), 0.938 (1.18), 1.022 (4.00), 1.038 (5.02), 1.054 (16.00), 1.071 (15.05), 1.162 (0.96), 1.180 (1.95), 1.198 (0.96), 1.911 (2.63), 1.922 (2.66), 1.928 (0.88), 1.936 (0.72), 1.953 (3.08), 1.964 (3.16), 1.969 (1.02), 1.977 (0.79), 2.358 (0.76), 2.373 (0.78), 2.399 (0.72), 2.412 (3.05), 2.429 (2.91), 2.454 (2.51), 2.471 (2.81), 3.272 (1.56), 3.274 (1.65), 3.280 (1.80), 3.282 (1.89), 3.288 (2.04), 3.290 (2.15), 3.296 (2.48), 3.298 (2.71), 3.313 (14.07), 3.793 (1.26), 3.801 (1.51), 3.810 (1.48), 3.819 (1.12), 4.038 (0.90), 4.056 (0.89), 4.923 (0.86), 4.934 (0.81), 5.163 (2.76), 5.174 (2.65), 7.588 (1.18).

**Beispiel 164A**

Ethyl-4-{[(benzyloxy)carbonyl]amino}-2,2-dimethyl-3-oxopentanoat (Racemat)

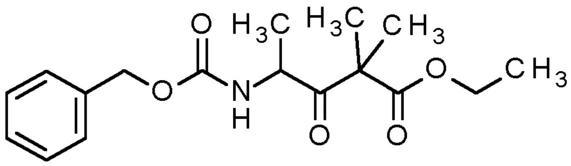

Eine Suspension aus 500 mg (1.70 mmol) Ethyl-4-{[(benzyloxy)carbonyl]amino}-3-oxopentanoat, 320 µl (5.10 mmol) Iodmethan und 471 mg (3.41 mmol) Kaliumcarbonat in 7.2 ml Aceton wurde für 16 h bei 60°C in der Mikrowelle zur Reaktion gebracht. Die Reaktionsmischung wurde auf Wasser gegossen und dreimal mit Essigsäureethylester extrahiert. Die vereinigten organischen Phasen wurden mit gesättigter wässriger Natriumchlorid-Lösung gewaschen, über Natriumsulfat getrocknet, filtriert und das Lösungsmittel unter reduziertem Druck entfernt. Der Rückstand wurde in wenig Acetonitril gelöst, über einen Milliporefilter filtriert und in zwei Läufen mittels präparativer HPLC (Säule: Chromatorex C18, 10 µm, 125*40 mm, Lösungsmittel: Acetonitril, Wasser, 0.1% Ameisensäure) getrennt. Es wurden 260 mg (47% d. Th.) der Titelverbindung erhalten.

LC-MS (Methode 3): $R_t$ = 1.92 min; MS (ESIpos): m/z = 322 $[M+H]^+$

$^{1}$H NMR (400 MHz, DMSO-$d_6$): $\delta$ ppm = 7.65 (br d, 1H), 7.26-7.42 (m, 5H), 5.01 (s, 2H), 4.51 (quint., 1H), 4.06 (q, 2H), 1.35 (s, 3H), 1.29 (s, 3H), 1.11-1.18 (m, 6H).

**Beispiel 164B**

Ethyl-4- {[(benzyloxy)carbonyl] amino} -2,2-dimethyl-3 -hydroxypentanoat (Diastereomer 2)

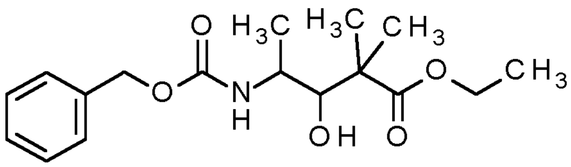

Zu einer Lösung von 260mg (809 µmol) Ethyl-4-{[(benzyloxy)carbonyl]amino}-2,2-dimethyl-3-oxopentanoat in 4.5 ml Methanol wurden bei 0°C 42.9 mg (1.13 mmol) Natriumborhydrid gegeben. Es wurde langsam auf RT erwärmt und für 17 h bei RT nachgerührt. Die Reaktion wurde durch Zugabe von gesättigter, wässriger Ammoniumchlorid-Lösung beendet. Die organische Phase wurde dreimal mit Essigsäureethylester extrahiert. Die vereinigten organischen Phasen wurden mit gesättigter, wässriger Natriumchlorid-Lösung gewaschen, über Magnesiumsulfat getrocknet, filtriert und das Lösungsmittel wurde unter reduziertem Druck entfernt. Der Rückstand wurde in etwas Acetonitril aufgenommen und mittels präparativer HPLC (Säule: Chromatorex C18, 10 µm, 125*40 mm, Lösungsmittel: Acetonitril, Wasser, 0.1% Ameisensäure) gereinigt. Es wurden 64.0 mg (24% d. Th., 100% Reinheit) der Titelverbindung (Diastereomer 2) erhalten.

Es wurden 95.0 mg (34% d. Th., 93% Reinheit) von Diastereomer 1 erhalten.
LC-MS (Methode 3): $R_t$ = 1.71 min; MS (ESIpos): m/z = 324 $[M+H]^+$

**Beispiel 164C**

4-Hydroxy-3,3,5-trimethylpyrrolidin-2-on (Racemat)

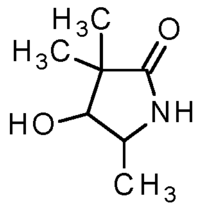

Zu einer Lösung von 64.0 mg (198 µmol) Ethyl-4-{[(benzyloxy)carbonyl]amino}-2,2-dimethyl-3-hydroxypentanoat (Diastereomer 2) in 1.0 ml Methanol wurden 5 mg Palladium auf Kohle (10%) gegeben und für 6 h bei Normaldruck und Raumtemperatur hydriert. Anschließend wurde die Reaktionsmischung durch einen Milliporefilter filtriert und das Lösungsmittel unter reduziertem Druck entfernt. Es wurden 20.0 mg (71% d. Th.) der Titelverbindung erhalten, welche ohne weitere Aufreinigung im nächsten Schritt eingesetzt wurde.
$^1$H NMR (400 MHz, CDCl3): δ ppm = 5.54 (br s, 1H), 3.85-3.92 (m, 2H), 2.62 (s, 1H), 1.27 (d, 3H), 1.20 (s, 3H), 1.19 (s, 3H).

**Beispiel 165A**

7-[(3*R*,4*R*)-3,4-Bis{[*tert*-butyl(dimethyl)silyl]oxy}pyrrolidin-1-yl]-1-(2,6-difluorphenyl)-4-oxo-*N*-[(2*R*)-1,1,1-trifluorbutan-2-yl]-1,4-dihydro-1,8-naphthyridin-3-carboxamid

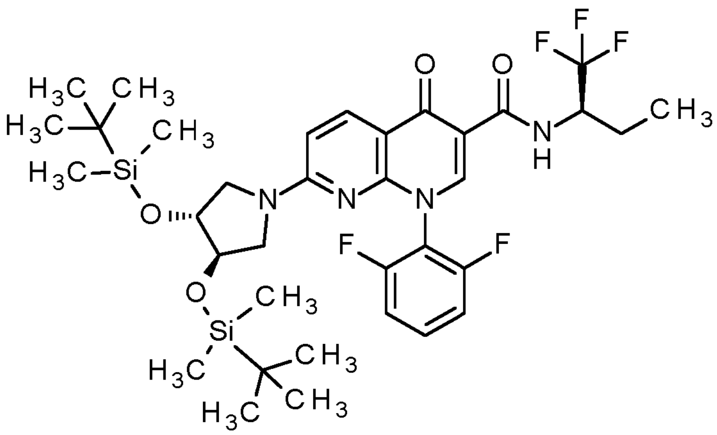

Gemäß AAV3 wurden 75.0 mg (168 µmol) der Verbindung aus Beispiel 86A mit 67.0 mg (202 µmol) der Verbindung aus Beispiel 162C in Gegenwart von 100 µl (590 µmol) DIPEA in 750 µl DMF zur Reaktion gebracht. Das Lösungsmittel wurde unter reduziertem Druck entfernt und das Rohprodukt wurde mittels Normalphasenchromatographie (Essigsäureethylester-Cyclohexan-Gradient) gereinigt. Es wurden 119 mg (95% d. Th., 95% Reinheit) der Titelverbindung erhalten.
$^1$H NMR (400 MHz, DMSO-$d_6$): δ ppm = 10.42 (d, 1 H), 8.78 (s, 1 H), 8.30 (d, 1 H), 7.70 (tt, 1 H), 7.32 - 7.43 (m, 2 H), 6.82 (d, 1 H), 4.69 - 4.80 (m, 1 H), 4.15 - 4.21 (m, 1 H), 4.00 - 4.07 (m, 1 H), 3.68 (br dd, 1 H), 3.21 - 3.29 (m, 2 H), 3.03 - 3.11 (m, 1 H), 1.83 - 1.93 (m, 1 H), 1.58 - 1.70 (m, 1 H), 0.97 (t, 3 H), 0.83 (s, 9 H), 0.79 (s, 9 H), 0.08 (s, 6 H).

**Beispiel 166A**

7-Chlor-1-(2,6-dichlorphenyl)-4-oxo-1,4-dihydro-1,8-naphthyridin-3-carbonsäureethylester

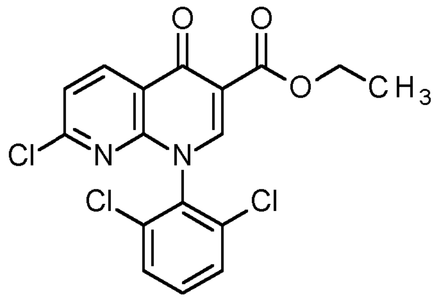

Eine Lösung aus 6.07 g (19.1 mmol) 2-[(2,6-Dichlorpyridin-3-yl)carbonyl]-3-ethoxyacrylsäure-ethylester (CAS 157373-27-8) und 4.33 g (26.7 mmol) 2,6-Dichloranilin in 30 ml DCM wurde mit 23 ml (130 mmol) *N,N*-Diisopropylethylamin versetzt und 4h bei Raumtemperatur gerührt. Anschließend wurden zu der Reaktionsmischung 2.64 g (19.1 mmol) Kaliumcarbonat gegeben und die Reaktion wurde für 4d unter Rückfluss erhitzt. Es wurde auf RT abgekühlt, mit Dichlormethan verdünnt, zweimal mit 1M wässriger Salzsäure und einmal mit gesättigter wässriger Natriumchlorid-Lösung gewaschen. Die organische Phase wurde über Natriumsulfat getrocknet, filtriert und das Lösungsmittel unter reduziertem Druck entfernt. Der Rückstand wurde mit Diethylether verrührt, der Niederschlag abgesaugt und am Hochvakuum getrocknet. Zur Substanz wurden Dichlormethan und Methanol (1:1, v/v) gegeben. Es wurde kurz aufgekocht und der Niederschlag abgesaugt. Die Mutterlauge wurde eingeengt und ausfallender Niederschlag nochmals abgesaugt. Es wurden 2.83 g (37% d. Th., 100% Reinheit) der Titelverbindung erhalten.
LC-MS (Methode 3): $R_t$ = 1.89 min; MS (ESIpos): m/z = 396 $[M+H]^+$
$^1$H NMR (400 MHz, DMSO-$d_6$): $\delta$ ppm = 8.85 (s, 1 H), 8.65 (d, 1 H), 7.77 - 7.82 (m, 2 H), 7.65 - 7.72 (m, 2 H), 4.25 (q, 2 H), 1.28 (t, 3 H).

**Beispiel 166B**

7-Chlor-1-(2,6-dichlorphenyl)-4-oxo-1,4-dihydro-1,8-naphthyridin-3-carbonsäure

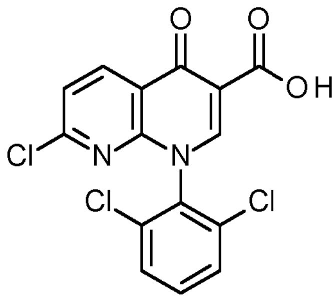

Zu einer Suspension von 2.78 g (7.00 mmol) der Verbindung aus Beispiel 166A in 23 ml Wasser wurden nacheinander 23 ml konzentrierte Salzsäure und 23 ml Tetrahydrofuran gegeben. Die resultierende Suspension wurde 30h bei 120 °C stark gerührt und im Anschluss auf RT abgekühlt. Es wurde mit 150 ml Wasser verdünnt und der Niederschlag abgesaugt und am Hochvakuum getrocknet. Es wurden 2.49 g (96% d. Th., 100% Reinheit) der Titelverbindung erhalten.
LC-MS (Methode 3): $R_t$ = 1.91 min; MS (ESIpos): m/z = 368 $[M+H]^+$
$^1$H NMR (400 MHz, DMSO-$d_6$): $\delta$ ppm = 13.82 (br s, 1 H), 9.22 (s, 1 H), 8.81 (d, 1 H), 7.78 - 7.84 (m, 3 H), 7.70 (dd, 1 H).

**Beispiel 166C**

7-Chlor-1-(2,6-dichlorphenyl)-4-oxo-*N*-[(2*R*)-1,1,1-trifluorbutan-2-yl]-1,4-dihydro-1,8-naphthyridin-3-carboxamid

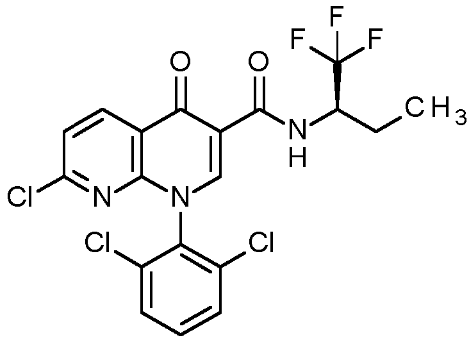

Gemäß AAV1 wurden 400 mg (1.08 mmol) 7-Chlor-1-(2,6-dichlorphenyl)-4-oxo-1,4-dihydro-1,8-naphthyridin-3-carbonsäure mit 266 mg (1.62 mmol) (2*R*)-1,1,1-Trifluorbutan-2-amin-Hydrochlorid in Gegenwart von 494 mg (1.30 mmol) HATU und 570 µl (3.20 mmol) DIPEA in 6.0 ml DMF zur Reaktion gebracht. Es wurde mit Wasser, 1 M wässriger Salzsäure und Essigsäureethylester verdünnt. Die Phasen wurden getrennt und die organische Phase unter reduziertem Druck entfernt. Das Rohprodukt wurde in Acetonitril suspendiert und der Niederschlag (116.5 mg der Titelverbindung) abfiltriert. Die Mutterlauge wurde mittels präparativer HPLC (Säule: Chromatorex C18, 10 µm, 125*40 mm, Lösungsmittel: Acetonitril, Wasser, 0.1% Ameisensäure) gereinigt und kombiniert mit dem Niederschlag insgesamt 304 mg (59% d. Th., 99% Reinheit) der Titelverbindung erhalten.
LC-MS (Methode 3): $R_t$ = 2.40 min; MS (ESIpos): m/z = 478 $[M+H]^+$
$^1$H-NMR (400 MHz, DMSO-$d_6$): δ ppm = 9.91 (d, 1 H), 9.04 (s, 1 H), 8.78 (d, 1 H), 7.77 - 7.83 (m, 3 H), 7.67 - 7.73 (m, 1 H), 4.70 - 4.83 (m, 1 H), 1.85 - 1.95 (m, 1 H), 1.60 - 1.75 (m, 1 H), 0.98 (t, 3 H).

## Beispiel 167A

7-[(3*R*,4*R*)-3,4-Bis{[*tert*-butyl(dimethyl)silyl]oxy}pyrrolidin-1-yl]-1-(2,4-difluorphenyl)-4-oxo-*N*-[(2*R*)-1,1,1-trifluorbutan-2-yl]-1,4-dihydro-1,8-naphthyridin-3-carboxamid

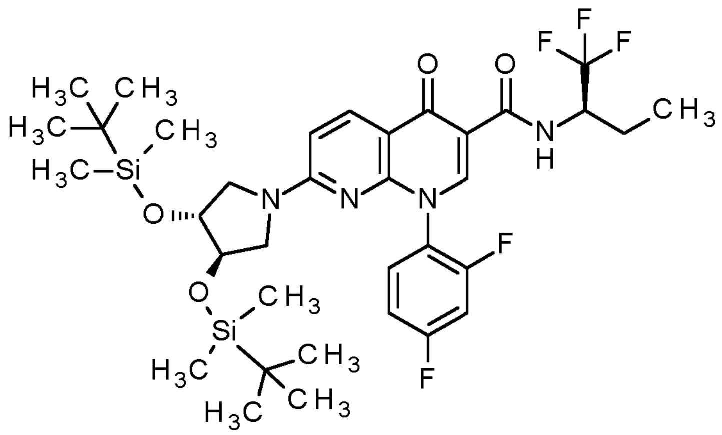

Gemäß allgemeiner Arbeitsvorschrift 3 wurden 100 mg (224 µmol) der Verbindung aus Beispiel 67A mit 89.3 mg (269 µmol) der Verbindung aus Beispiel 162C in Gegenwart von 140 µl (790 µmol) DIPEA in 1.0 ml DMF zur Reaktion gebracht. Das Lösungsmittel wurde unter reduziertem Druck entfernt und das Rohprodukt wurde mittels Normalphasenchromatographie (Essigsäureethylester-Cyclohexan-Gradient) gereinigt. Es wurden 166 mg (quantitativ) der Titelverbindung erhalten.
$^1$H NMR (400 MHz, DMSO-$d_6$): δ ppm = 10.50 (d, 1 H), 8.64 (d, 1 H), 8.29 (d, 1 H), 7.74 - 7.86 (m, 1 H), 7.42 - 7.58 (m, 1 H), 7.26 - 7.35 (m, 1 H), 6.80 (d, 1 H), 4.68 - 4.79 (m, 1 H), 4.18 (br s, 1 H), 4.05 (br s, 1 H), 3.63 - 3.73 (m, 1 H), 3.22 - 3.30 (m, 2 H), 2.95 - 3.17 (m, 1 H), 1.83 - 1.93 (m, 1 H), 1.58 - 1.69 (m, 1 H), 0.96 (t, 3 H), 0.76 - 0.86 (m, 18 H), 0.08 (s, 6 H), -0.03 - 0.05 (m, 6 H).

**Beispiel 168A**

7-(4-Carbamoylpiperazin-1-yl)-4-oxo-1-(2,4,6-trifluorphenyl)-1,4-dihydro-1,8-naphthyridin-3-carbonsäure

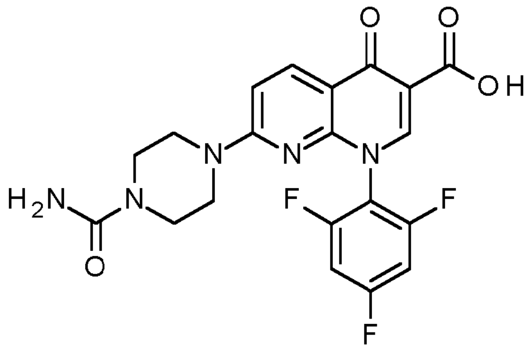

Gemäß allgemeiner Arbeitsvorschrift 3 wurden 500 g (1.41 mmol) 7-Chlor-4-oxo-1-(2,4,6-trifluorphenyl)-1,4-dihydro-1,8-naphthyridin-3-carbonsäure (Beispiel 100B) mit 219 mg (1.69 mmol) Piperazin-1-carboxamid in Gegenwart von 860 µl (4.90 mmol) DIPEA in 6.3 ml DMF zur Reaktion gebracht. Der Niederschlag (358 mg der Titelverbindung) wurde von der Reaktionsmischung abfiltriert und die Mutterlauge wurde mittels präparativer HPLC (Acetonitril / Wasser/ 0,1 % Ameisensäure) gereinigt. Es wurden kombiniert mit dem Niederschlag 418 mg (67% d. Th., 100% Reinheit) der Titelverbindung erhalten.
LC-MS (Methode 1): $R_t$ = 0.70 min; MS (ESIpos): m/z = 448 $[M+H]^+$
$^1H$ NMR (400 MHz, DMSO-$d_6$): δ ppm = 15.09 (br s, 1 H), 9.04 (s, 1 H), 8.34 (d, 1 H), 7.59 (t, 2 H), 7.23 (d, 1 H), 6.04 (s, 2 H), 3.44 - 3.59 (m, 4 H).

**Beispiel 169A**

(5*S*)-3-[6-{[(1*S*)-1-Cyclopropyl-2,2,2-trifluorethyl]carbamoyl}-5-oxo-8-(2,4,6-trifluorphenyl)-5,8-dihydro-1,8-naphthyridin-2-yl]-2-oxo-1,3-oxazolidin-5-carbonylchlorid

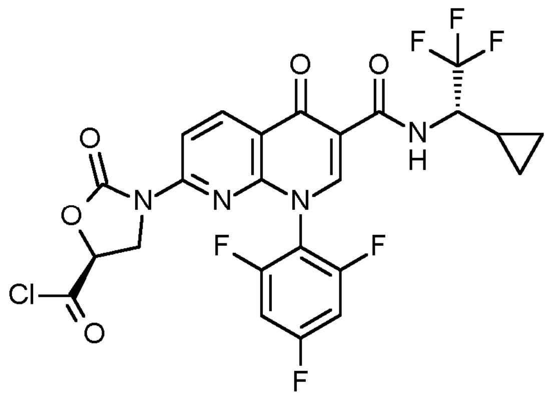

Zu einer Lösung von 317 mg (556 µmol) der Verbindung aus Beispiel 667 in 7.0 ml Dichlormethan wurden 410 µl (5.60 mmol) Thionylchlorid gegeben und 3h unter Rückfluss nachgerührt und noch 820 µl (11.2 mmol) Thionylchlorid wurden hinzugegeben. Die Reaktionsmischung wurde über Nacht unter Rückfluss nachgerührt und anschließend alle flüchtigen Komponenten unter reduziertem Druck entfernt. Das Rohprodukt wurde ohne weitere Aufarbeitung im nächsten Schritt eingesetzt (quantitative Umsetzung wurde angenommen).

**Beispiel 170A**

(5*R*)-3-[6-{[(1*S*)-1-Cyclopropyl-2,2,2-trifluorethyl]carbamoyl}-5-oxo-8-(2,4,6-trifluorphenyl)-5,8-dihydro-1,8-naphthyridin-2-yl]-2-oxo-1,3-oxazolidin-5-carbonylchlorid

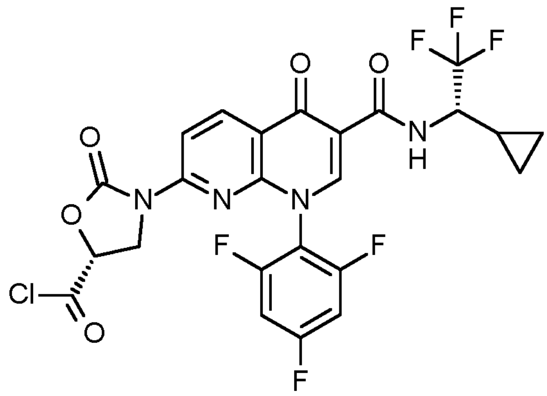

Zu einer Lösung von 440 mg (771 μmol) der Verbindung aus Beispiel 670 in 10 ml Dichlormethan wurden 560 μl (7.70 mmol) Thionylchlorid gegeben und 3h unter Rückfluss nachgerührt und noch 1.12 ml (15.4 mmol) Thionylchlorid wurden hinzugegeben. Die Reaktionsmischung wurde über Nacht unter Rückfluss nachgerührt und anschließend alle flüchtigen Komponenten unter reduziertem Druck entfernt. Das Rohprodukt wurde ohne weitere Aufarbeitung im nächsten Schritt eingesetzt (quantitative Umsetzung wurde angenommen).

**Ausführungsbeispiele:**

**Beispiel 1**

1-(2,4-Difluorphenyl)-7-(3,3-difluorpiperidin-1-yl)-4-oxo-*N*-(tricyclo[3.3.1.1$^{3,7}$]dec-1-yl)-1,4-dihydro-1,8-naphthyridin-3-carbonsäureamid

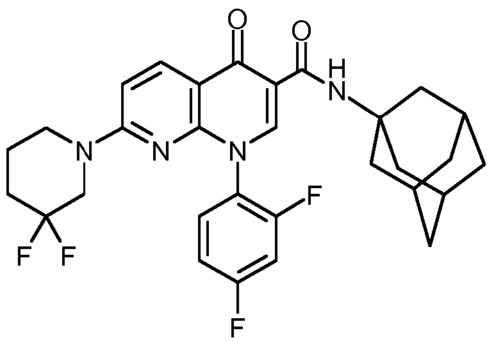

Ein Gemisch aus 80 mg (0.17 mmol) der Verbindung aus Beispiel 65A, 54 mg (0.34 mmol) 3,3-Difluorpiperidin Hydrochlorid und 88 mg (0.68 mmol) DIPEA in 1.5 ml NMP wurde 2 h bei 23°C gerührt. Anschliessend wurde das Gemisch über präparative HPLC gereinigt (Methode 7). Man erhielt 43 mg (45 % d. Th.) der Titelverbindung.
$^1$H-NMR (400 MHz, DMSO-$d_6$) δ [ppm]: -0.008 (1.85), 0.008 (1.65), 1.609 (0.87), 1.620 (1.04), 1.673 (7.07), 2.011 (0.49), 2.029 (0.86), 2.058 (16.00), 2.073 (2.19), 2.366 (0.53), 2.710 (0.50), 3.534 (0.91), 3.546 (1.26), 3.562 (0.86), 3.824 (0.46), 3.841 (0.59), 3.855 (0.75), 3.885 (0.41), 7.185 (1.64), 7.208 (1.68), 7.339 (0.69), 7.348 (0.66), 7.568 (0.46), 7.575 (0.47), 7.590 (0.61), 7.597 (0.72), 7.600 (0.63), 7.616 (0.46), 7.623 (0.45), 7.779 (0.44), 7.795 (0.53), 7.802 (0.90), 7.816 (0.89), 7.823 (0.51), 7.839 (0.42), 8.295 (2.38), 8.318 (2.19), 8.514 (4.49), 9.883 (2.53).
LC-MS (Methode 1): $R_t$ = 1.34 min; m/z = 479.2 $[M+H]^+$.
In Analogie zu Beispiel 1 wurden die in Tabelle 1 gezeigten Beispielverbindungen hergestellt, indem die Verbindung aus Beispiel 65A mit den entsprechenden Aminen (bzw. deren Salzen) unter den beschriebenen Reaktionsbedingungen umgesetzt wurden. Abweichungen sind bei den jeweiligen Beispielen angegeben.

Tabelle 1:

| Bsp. | | Analytische Daten |
|---|---|---|
| **2** | 7-(6,6-Difluor-3-azabicyclo[3.1.0]hex-3-yl)-1-(2,4-difluorphenyl)-4-oxo-*N*-(tricyclo-[3.3.1.1$^{3,7}$]dec-1-yl)-1,4-dihydro-1,8-naphthyridin-3-carbonsäureamid 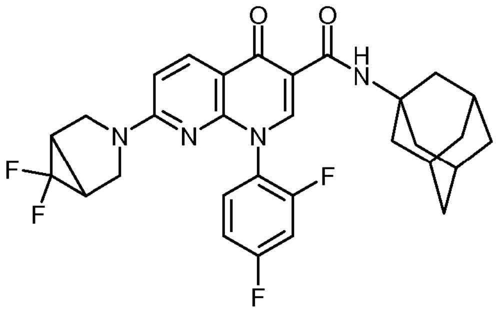 (92 % d. Th.) | LC-MS (Methode 1): $R_t$ = 1.42 min MS (ESpos): m/z = 553.4 $[M+H]^+$ $^1$H-NMR (400 MHz, DMSO-$d_6$): $\delta$ = 1.67 (m, 6 H), 2.06 (m, 9H), 2.58-2.77 (m, 2H, teilweise mit DMSO Signal überlappend), 3.44 (br. s, 2H), 3.80 (br. s, 2H), 6.74 (d, 1H), 7.29-7.36 (m, 1H), 7.53- 7.64 (m, 1H), 7.76-7.84 (m, 1H), 8.30 (d, 1H), 8.50 (s, 1H), 9.91 (br.s, 1H). |
| **3** | 1-(2,4-Difluorphenyl)-7-[(3*R*,4*R*)-4-fluoro-3-hydroxypiperidin-1-yl]-4-oxo-*N*-(tricyclo-[3.3.1.1$^{3,7}$] dec-1-yl)-1,4-dihydro-1,8-naphthyridin-3 -carbonsäureamid 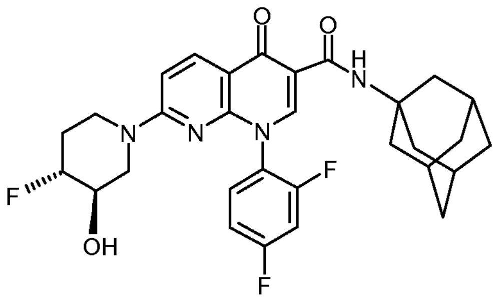 (80 % d. Th.) | LC-MS (Methode 1): $R_t$ = 1.26 min MS (ESpos): m/z = 553.4 $[M+H]^+$ $^1$H-NMR (400 MHz, DMSO-$d_6$): $\delta$ = 1.39-1.55 (m, 1H), 1.68 (s, 6 H), 1.88-2.01 (m, 1H), 2.06 (m, 9H), 2.90- 3.09 (m, 1H), 3.11-3.27 (m, 2H), 3.38-3.51 (m, 2H), 4.36-4.45 (m, 1H), 4.48-4.59 (m, 1H), 5.41-5.49 (m, 2H), 7.11 (d, 1H), 7.29-7.36 (m, 1H), 7.52- 7.62 (m, 1H), 7.77-7.84 (m, 1H), 8.28 (d, 1H), 8.49 (s, 1H), 9.91 (br. s, 1H). |
| **4** | 4-[8-(2,4-Difluorphenyl)-5-oxo-6-(tricyclo-[3.3.1.1$^{3,7}$]dec-1-ylcarbamoyl)-5,8-dihydro-1,8-naphthyridin-2-yl]piperazin-1-carbonsäuremethylester 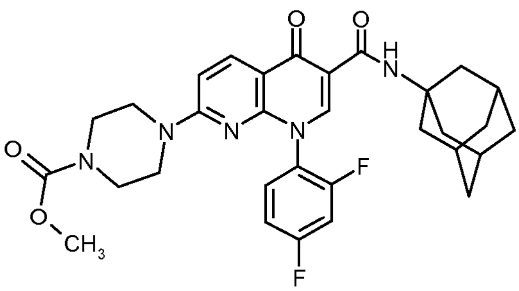 (74 % d. Th.) | LC-MS (Methode 1): $R_t$ = 1.30 min MS (ESpos): m/z = 578.2 $[M+H]^+$ $^1$H-NMR (400 MHz, DMSO-$d_6$) $\delta$ [ppm]: -0.008 (1.17), 0.008 (0.97), 1.157 (1.26), 1.175 (2.57), 1.193 (1.30), 1.672 (7.47), 1.988 (4.65), 2.055 (16.00), 3.360 (1.42), 3.374 (3.07), 3.388 (2.46), 3.493 (2.28), 3.506 (2.93), 3.520 (1.59), 3.603 (13.90), 4.021 (1.08), 4.038 (1.07), 7.070 (2.00), 7.093 (2.02), 7.308 (0.42), 7.325 (0.78), 7.332 (0.76), 7.334 (0.61), 7.344 (0.41), 7.348 (0.43), 7.351 (0.45), 7.354 (0.40), 7.555 (0.52), 7.562 (0.55), 7.578 (0.70), 7.581 (0.76), 7.585 (0.78), 7.589 (0.65), 7.604 (0.54), 7.611 (0.52), 7.761 (0.51), 7.775 (0.61), 7.782 (1.00), 7.797 (1.01), 7.804 (0.56), 7.819 (0.49), 8.297 (2.65), 8.319 (2.43), 8.503 (5.19), 9.897 (2.86). |

(fortgesetzt)

| Bsp. | | Analytische Daten |
|---|---|---|
| **5** | 1-(2,4-Difluorphenyl)-7-(4-fluorpiperidin-1-yl)-4-oxo-*N*-(tricyclo[3.3.1.1$^{3,7}$]dec-1-yl)-1,4-dihydro-1, 8-naphthyridin-3 -carbonsäureamid 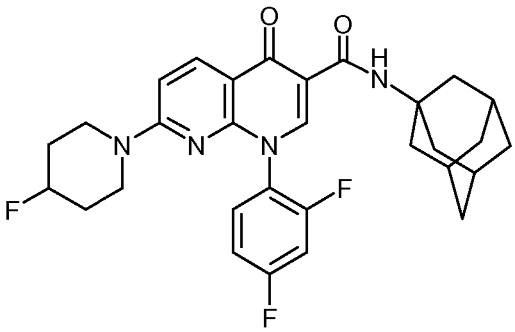 (81 % d. Th.) | LC-MS (Methode 1): $R_t$ = 1.38 min MS (ESpos): m/z = 537.5 $[M+H]^+$ $^1$H-NMR (400 MHz, DMSO-$d_6$) δ [ppm]: 1.670 (8.34), 1.828 (0.58), 2.055 (16.00), 2.365 (0.20), 2.710 (0.19), 3.494 (1.01), 3.602 (0.95), 4.797 (0.41), 4.919 (0.41), 7.124 (1.42), 7.147 (1.47), 7.309 (0.45), 7.330 (0.88), 7.351 (0.49), 7.562 (0.47), 7.584 (0.83), 7.604 (0.48), 7.772 (0.40), 7.794 (0.82), 7.809 (0.82), 7.830 (0.38), 8.272 (1.60), 8.295 (1.52), 8.493 (3.05), 9.909 (2.30) |
| **6** | 7-(3,3-Difluor-4,4-dihydroxypiperidin-1-yl)-1-(2,4-difluorphenyl)-4-oxo-*N*-(tricyclo-[3.3.1.1$^{3,7}$]dec-1-yl)-1,4-dihydro-1,8-naphthyridin-3-carbonsäureamid 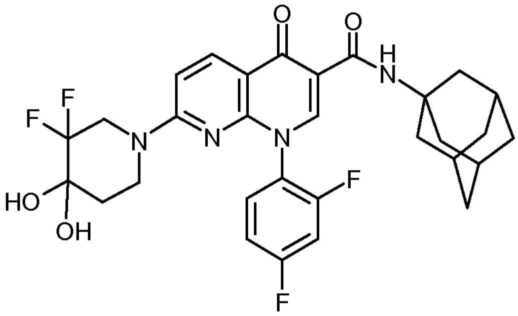 (18 % d. Th.) | LC-MS (Methode 1): $R_t$ = 1.17 min MS (ESpos): m/z = 587.4 $[M+H]^+$ $^1$H-NMR (400 MHz, DMSO-$d_6$) δ [ppm]: 1.003 (5.12), 1.020 (4.58), 1.174 (1.24), 1.671 (8.60), 1.987 (2.21), 2.057 (16.00), 3.560 (1.43), 3.824 (0.84), 6.433 (1.52), 7.197 (1.18), 7.220 (1.24), 7.346 (0.81), 7.594 (0.81), 7.798 (0.93), 7.813 (0.94), 8.191 (1.88), 8.294 (2.21), 8.317 (2.03), 8.516 (4.68), 9.880 (2.69). |
| **7** | 1-(2,4-Difluorphenyl)-7-[(3*R*)-3-fluor-4,4-dihydroxypiperidin-1-yl]-4-oxo-*N*-(tricyclo [3.3.1.1$^{3,7}$]dec-1-yl)-1,4-dihydro-1,8-naphthyridin-3-carbonsäureamid 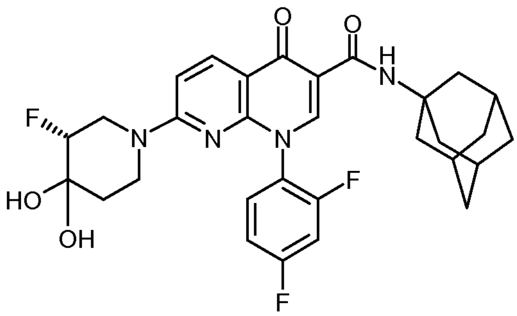 (10 % d. Th.) | LC-MS (Methode 1): $R_t$ = 1.10 min MS (ESpos): m/z = 569.5 $[M+H]^+$ $^1$H-NMR (400 MHz, DMSO-$d_6$) δ [ppm]: 0.976 (1.80), 0.992 (1.66), 1.507 (0.39), 1.537 (0.33), 1.671 (8.13), 2.057 (16.00), 2.328 (0.30), 2.366 (0.39), 2.670 (0.34), 2.711 (0.38), 3.026 (0.31), 3.168 (0.84), 3.934 (0.41), 3.964 (0.41), 4.152 (0.42), 4.229 (0.43), 4.264 (0.69), 5.946 (0.85), 5.959 (0.93), 6.045 (1.02), 7.105 (1.27), 7.128 (1.32), 7.317 (0.44), 7.337 (0.85), 7.354 (0.47), 7.559 (0.44), 7.566 (0.48), 7.585 (0.77), 7.608 (0.46), 7.615 (0.43), 7.782 (0.45), 7.799 (0.65), 7.815 (0.42), 8.209 (0.39), 8.247 (1.41), 8.270 (1.34), 8.486 (2.39), 9.915 (2.26). |

(fortgesetzt)

| Bsp. | | Analytische Daten |
|---|---|---|
| **8** | 1-(2,4-Difluorphenyl)-4-oxo-7-(piperidin-1-yl)-*N*-(tricyclo[3.3.1.1$^{3,7}$]dec-1-yl)-1,4-dihydro-1,8-naphthyridin-3-carbonsäureamid 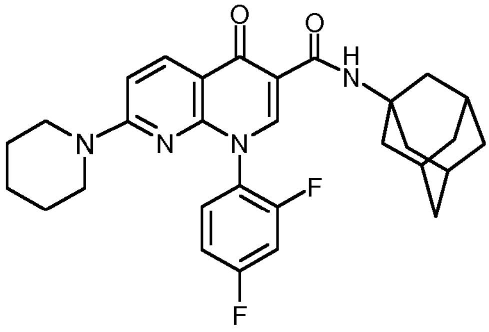 (73 % d. Th.) | LC-MS (Methode 1): $R_t$ = 1.48 min MS (ESpos): m/z = 519.4 $[M+H]^+$ $^1$H-NMR (400 MHz, DMSO-$d_6$) δ [ppm]: -0.001 (14.80), 0.938 (0.69), 0.953 (0.65), 1.427 (2.32), 1.578 (1.22), 1.670 (7.45), 2.054 (16.00), 2.365 (0.41), 2.694 (0.59), 2.709 (0.44), 3.470 (2.58), 7.048 (1.93), 7.071 (1.98), 7.301 (0.42), 7.323 (0.81), 7.343 (0.44), 7.545 (0.51), 7.552 (0.54), 7.574 (0.76), 7.593 (0.52), 7.600 (0.49), 7.760 (0.50), 7.782 (0.99), 7.797 (0.99), 7.819 (0.49), 8.227 (2.51), 8.250 (2.35), 8.466 (5.28), 9.935 (2.69). |
| **9** | *rac*-1-(2,4-Difluorphenyl)-7-(3-fluoro-3-methylpyrrolidin-1-yl)-4-oxo-*N*-(tricyclo-[3.3.1.1$^{3,7}$] dec-1-yl)-1,4-dihydro-1,8-naphthyridin-3-carbonsäureamid 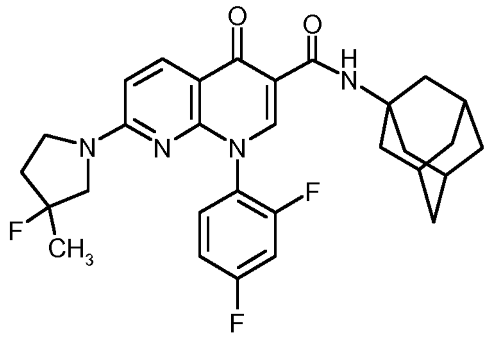 (85 % d. Th.) | LC-MS (Methode 1): $R_t$ = 1.43 min MS (ESpos): m/z = 537.4 $[M+H]^+$ $^1$H-NMR (400 MHz, DMSO-$d_6$) δ [ppm]: -0.150 (0.31), -0.009 (2.72), 0.007 (2.36), 0.146 (0.29), 1.243 (0.52), 1.258 (0.65), 1.272 (0.33), 1.508 (0.80), 1.671 (7.35), 1.987 (0.32), 2.056 (16.00), 2.212 (0.24), 2.322 (0.25), 2.327 (0.34), 2.365 (1.68), 2.518 (1.52), 2.523 (2.13), 2.557 (0.97), 2.560 (0.88), 2.562 (0.73), 2.564 (0.52), 2.567 (0.41), 2.569 (0.47), 2.577 (0.26), 2.580 (0.27), 2.660 (0.25), 2.665 (0.33), 2.669 (0.45), 2.674 (0.29), 2.694 (0.21), 2.709 (1.76), 3.144 (0.22), 3.161 (0.59), 3.174 (0.64), 3.195 (0.19), 3.217 (0.19), 3.380 (0.28), 3.431 (0.22), 3.448 (0.25), 3.459 (0.24), 3.472 (0.21), 3.507 (0.25), 3.681 (0.22), 6.722 (0.27), 7.301 (0.33), 7.322 (0.64), 7.341 (0.38), 7.550 (0.25), 7.573 (0.46), 7.764 (0.29), 7.786 (0.62), 7.800 (0.60), 7.823 (0.27), 8.283 (0.75), 8.305 (0.75), 8.482 (3.76), 9.934 (2.64). |
| **10** | 7-(3-Cyanopiperidin-1-yl)-1-(2,4-difluorphenyl)-4-oxo-*N*-(tricyclo[3.3.1.1$^{3,7}$]dec-1-yl)-1,4-dihydro-1,8-naphthyridin-3-carbonsäureamid 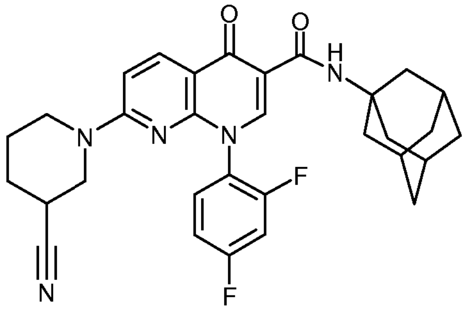 (87 % d. Th.) | LC-MS (Methode 1): $R_t$ = 1.27 min MS (ESpos): m/z = 544.3 $[M+H]^+$ $^1$H-NMR (400 MHz, DMSO-$d_6$) δ [ppm]: 0.936 (1.41), 0.951 (1.34), 1.235 (0.32), 1.475 (0.40), 1.558 (0.43), 1.672 (7.37), 1.862 (0.58), 1.882 (1.03), 1.901 (1.49), 1.920 (1.32), 1.937 (0.61), 2.057 (16.00), 2.155 (1.02), 2.176 (1.43), 2.195 (0.76), 2.327 (0.29), 2.669 (0.31), 2.694 (6.05), 2.961 (0.60), 3.285 (2.06), 3.477 (0.41), 3.561 (0.38), 3.680 (0.35), 3.713 (0.77), 3.743 (0.54), 3.760 (0.53), 7.166 (1.68), 7.189 (1.72), 7.321 (0.53), 7.555 (0.54), 7.575 (0.53), 7.786 (0.49), 7.802 (0.58), 7.822 (0.46), 8.292 (2.36), 8.315 (2.20), 8.514 (3.44), 9.891 (2.51). |

(fortgesetzt)

| Bsp. | | Analytische Daten |
|---|---|---|
| **11** | 1-(2,4-Difluorphenyl)-7-[(2*R*,4*S*)-4-fluoro-2-methylpyrrolidin-1-yl]-4-oxo-*N*-(tricyclo[3.3.1.1$^{3,7}$]dec-1-yl)-1,4-dihydro-1,8-naphthyridin-3-carbonsäureamid 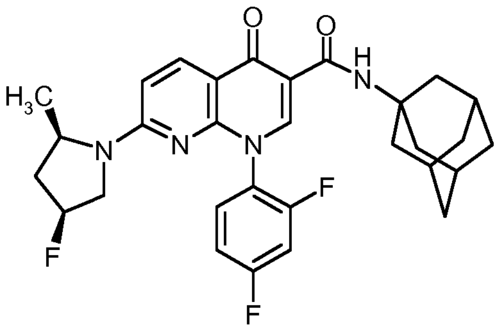 (70 % d. Th.) | LC-MS (Methode 1): $R_t$ = 1.43 min MS (ESpos): m/z = 537.4 $[M+H]^+$ $^1$H-NMR (400 MHz, DMSO-$d_6$) δ [ppm]: -0.150 (0.18), 0.145 (0.20), 0.840 (0.16), 0.889 (0.59), 0.919 (0.47), 0.934 (2.34), 0.951 (2.23), 1.066 (0.31), 1.146 (0.27), 1.234 (0.18), 1.672 (7.79), 1.901 (0.20), 1.959 (0.24), 2.058 (16.00), 2.218 (0.20), 2.327 (0.34), 2.366 (1.39), 2.408 (0.22), 2.427 (0.23), 2.669 (0.35), 2.694 (0.24), 2.709 (1.39), 2.960 (0.19), 3.584 (0.17), 3.915 (0.17), 5.326 (0.24), 5.457 (0.24), 6.737 (0.27), 7.322 (0.66), 7.344 (0.38), 7.569 (0.46), 7.592 (0.47), 7.767 (0.28), 7.788 (0.62), 7.804 (0.62), 7.825 (0.27), 8.281 (1.86), 8.303 (1.78), 8.498 (1.45), 9.940 (2.44). |
| **12** | 7-(4-Carbamoylpiperazin-1-yl)-1-(2,4-difluorphenyl)-4-oxo-*N*-(tricyclo-[3.3.1.1$^{3,7}$]dec-1-yl)-1,4-dihydro-1,8-naphthyridin-3-carbonsäureamid 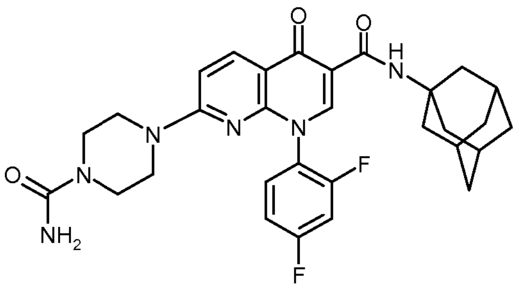 (quant.Ausbeute) | LC-MS (Methode 1): $R_t$ = 1.10 min MS (ESpos): m/z = 563.4 $[M+H]^+$ $^1$H-NMR (400 MHz, DMSO-$d_6$) δ [ppm]: -0.001 (0.96), 1.669 (1.15), 1.861 (0.09), 1.881 (0.29), 1.901 (0.34), 1.916 (0.24), 1.919 (0.30), 1.937 (0.12), 2.055 (2.40), 2.155 (0.34), 2.175 (0.47), 2.195 (0.24), 2.694 (2.11), 3.284 (0.84), 3.302 (1.37), 3.315 (16.00), 3.453 (0.43), 6.021 (0.47), 7.083 (0.28), 7.106 (0.28), 7.314 (0.07), 7.335 (0.12), 7.356 (0.07), 7.560 (0.08), 7.567 (0.09), 7.586 (0.12), 7.608 (0.08), 7.615 (0.08), 7.770 (0.08), 7.785 (0.10), 7.792 (0.15), 7.807 (0.15), 7.814 (0.08), 7.829 (0.07), 8.280 (0.38), 8.303 (0.35), 8.497 (0.81), 9.907 (0.41). |
| **13** | 7-(1,1-Difluor-5-azaspiro[2.4]hept-5-yl)-1-(2,4-difluorphenyl)-4-oxo-*N*-(tricyclo-[3.3.1.1$^{3,7}$]dec-1-yl)-1,4-dihydro-1,8-naphthyridin-3-carbonsäureamid 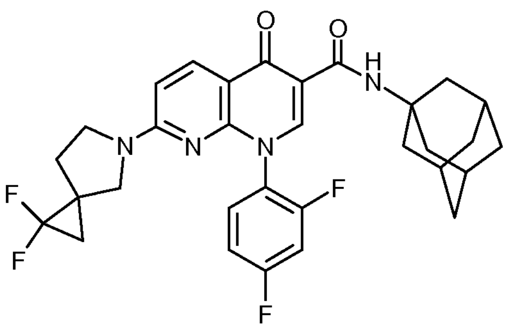 (95 % d. Th.) | LC-MS (Methode 1): $R_t$ = 1.46 min MS (ESpos): m/z = 567.4 $[M+H]^+$ $^1$H-NMR (400 MHz, DMSO-$d_6$) δ [ppm]: -0.150 (0.85), -0.009 (8.08), 0.008 (6.51), 0.146 (0.85), 1.147 (0.31), 1.156 (0.33), 1.174 (0.66), 1.192 (0.37), 1.240 (0.31), 1.258 (0.33), 1.616 (0.81), 1.622 (0.76), 1.636 (0.85), 1.672 (7.46), 1.988 (1.36), 2.056 (16.00), 2.131 (0.39), 2.156 (0.37), 2.177 (0.37), 2.327 (0.66), 2.366 (3.16), 2.608 (0.25), 2.669 (0.74), 2.673 (0.54), 2.694 (1.07), 2.709 (3.12), 3.285 (1.30), 3.461 (0.33), 3.505 (0.29), 4.020 (0.31), 4.038 (0.31), 6.742 (0.48), 6.765 (0.50), 7.293 (0.37), 7.307 (0.76), 7.330 (0.43), 7.526 (0.39), 7.533 (0.37), 7.555 (0.68), 7.575 (0.39), 7.760 (0.35), 7.780 (0.72), 7.796 (0.70), 7.816 (0.33), 8.291 (1.90), 8.313 (1.84), 8.488 (4.22), 9.928 (2.71). |

(fortgesetzt)

| Bsp. | | Analytische Daten |
|---|---|---|
| **14** | *rac*-1-(2,4-Difluorphenyl)-7-(3-hydroxypiperidin-1-yl)-4-oxo-*N*-(tricyclo-[3.3.1.1$^{3,7}$]dec-1-yl)-1,4-dihydro-1,8-naphthyridin-3-carbonsäureamid 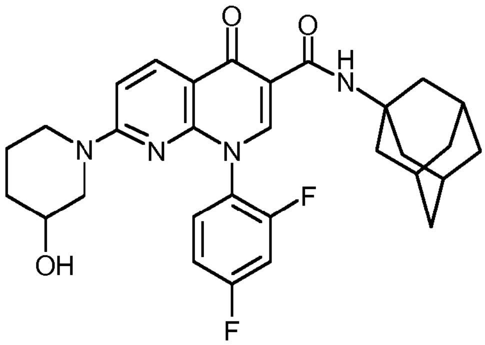 (96 % d. Th.) | LC-MS (Methode 1): $R_t$ = 1.19 min MS (ESpos): m/z = 535.3 $[M+H]^+$ $^1$H-NMR (400 MHz, DMSO-$d_6$) δ [ppm]: -0.009 (1.19), 0.007 (1.22), 0.942 (0.31), 1.263 (0.83), 1.391 (0.47), 1.669 (7.58), 1.803 (0.43), 1.881 (0.20), 1.901 (0.21), 1.920 (0.19), 2.054 (16.00), 2.155 (0.20), 2.176 (0.27), 2.195 (0.16), 2.327 (0.18), 2.366 (0.33), 2.669 (0.23), 2.694 (1.18), 2.709 (0.39), 2.949 (0.25), 3.030 (0.21), 3.057 (0.30), 3.090 (0.23), 3.126 (0.30), 3.154 (0.21), 3.285 (0.44), 3.370 (0.20), 3.429 (0.42), 3.670 (0.32), 3.806 (0.27), 3.839 (0.46), 3.869 (0.25), 4.796 (1.50), 4.808 (1.49), 7.027 (1.79), 7.050 (1.83), 7.296 (0.38), 7.300 (0.42), 7.322 (0.82), 7.339 (0.44), 7.343 (0.46), 7.558 (0.45), 7.757 (0.30), 7.779 (0.66), 7.795 (0.66), 7.816 (0.29), 8.222 (1.38), 8.245 (1.32), 8.464 (4.15), 9.934 (2.51). |
| **15** | 1-(2,4-Difluorphenyl)-7-[(2*S*)-2-(hydroxymethyl) piperidin-1-yl]-4-oxo-*N*-(tricyclo-[3.3.1.1$^{3,7}$]dec-1-yl)-1,4-dihydro-1,8-naphthyridin-3-carbonsäureamid 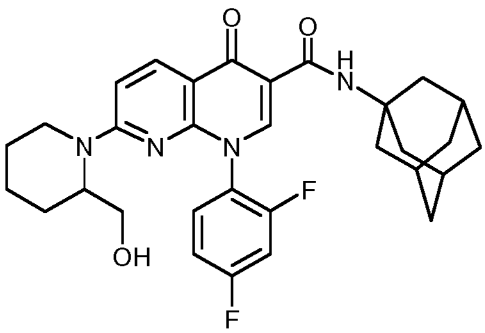 (30 % d. Th.) | LC-MS (Methode 1): $R_t$ = 1.29 min MS (ESpos): m/z = 549.5 $[M+H]^+$ $^1$H-NMR (400 MHz, DMSO-$d_6$) δ [ppm]: -0.150 (0.21), -0.009 (1.84), 0.007 (1.70), 1.236 (0.37), 1.482 (0.76), 1.535 (0.66), 1.567 (0.70), 1.670 (7.53), 1.764 (0.57), 1.796 (0.50), 2.054 (16.00), 2.072 (5.28), 2.365 (0.29), 2.709 (0.31), 2.797 (0.25), 3.502 (0.84), 4.064 (0.44), 4.098 (0.42), 4.216 (0.54), 4.660 (0.70), 7.023 (1.61), 7.046 (1.69), 7.290 (0.40), 7.312 (0.79), 7.332 (0.44), 7.538 (0.42), 7.564 (0.41), 7.751 (0.44), 7.773 (0.89), 7.788 (0.89), 7.810 (0.43), 8.207 (2.03), 8.230 (1.91), 8.457 (4.04), 9.953 (2.51). |
| **16** | 1 -(2,4-Difluorphenyl)-7- [4-fluoro-4-(hydroxymethyl)piperidin-1-yl]-4-oxo-*N*-(tricyclo [3.3.1.1$^{3,7}$]dec-1-yl)-1,4-dihydro-1,8-naphthyridin-3-carbonsäureamid 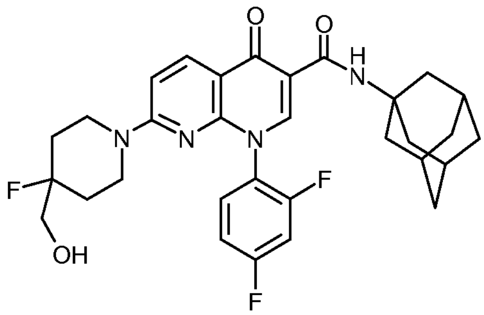 (85 % d. Th.) | LC-MS (Methode 1): $R_t$ = 1.28 min MS (ESpos): m/z = 567.4 $[M+H]^+$ $^1$H-NMR (500 MHz, DMSO-$d_6$) δ [ppm]: -0.004 (1.82), 1.158 (0.27), 1.172 (0.55), 1.187 (0.28), 1.226 (0.04), 1.493 (0.22), 1.517 (0.43), 1.538 (0.34), 1.588 (0.43), 1.608 (0.33), 1.666 (7.42), 1.921 (0.06), 1.986 (1.03), 2.052 (16.00), 2.180 (0.05), 3.171 (0.46), 3.357 (1.33), 3.369 (1.34), 3.397 (1.31), 3.408 (1.34), 3.968 (0.84), 3.989 (0.79), 4.020 (0.26), 4.034 (0.24), 4.049 (0.08), 4.942 (0.95), 4.954 (2.16), 4.965 (0.91), 7.113 (1.93), 7.131 (1.95), 7.305 (0.40), 7.309 (0.43), 7.322 (0.78), 7.326 (0.80), 7.339 (0.43), 7.343 (0.43), 7.552 (0.50), 7.558 (0.53), 7.573 (0.76), 7.576 (0.77), 7.591 (0.51), 7.596 (0.49), 7.771 (0.48), 7.783 (0.58), 7.788 (0.95), 7.800 (0.94), 7.806 (0.54), 7.818 (0.45), 8.266 (2.80), 8.284 (2.54), 8.492 (5.72), 9.913 (2.79). |

(fortgesetzt)

| Bsp. | | Analytische Daten |
|---|---|---|
| **17** | 1-(2,4-Difluorphenyl)-7-[3-(2-hydroxyethyl) piperidin-1-yl]-4-oxo-*N*-(tricyclo-[3.3.1.1$^{3,7}$]dec-1-yl)-1,4-dihydro-1,8-naphthyridin-3 -carbonsäureamid 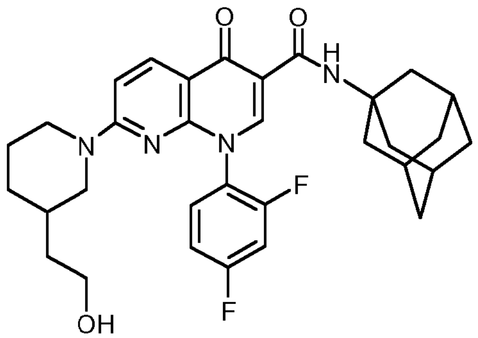 Aufarbeitung: Zugabe von Wasser und dann 1 M wässr. Salzsäure. Der entstandene Niederschlag wurde abfiltriert. (86 % d. Th) | LC-MS (Methode 1): $R_t$ = 1.27 min MS (ESpos): m/z = 563.3 $[M+H]^+$ $^1$H-NMR (400 MHz, DMSO-$d_6$) δ [ppm]: -0.009 (0.83), 0.007 (0.78), 0.935 (3.19), 0.951 (2.99), 1.145 (0.47), 1.172 (0.53), 1.242 (1.02), 1.258 (1.12), 1.447 (0.35), 1.583 (0.44), 1.669 (7.61), 1.741 (0.45), 1.881 (0.30), 1.901 (0.37), 1.920 (0.32), 1.937 (0.14), 2.053 (16.00), 2.155 (0.33), 2.176 (0.46), 2.195 (0.25), 2.327 (0.13), 2.366 (0.20), 2.409 (0.36), 2.426 (0.36), 2.669 (0.16), 2.694 (1.84), 2.709 (0.23), 2.944 (0.40), 2.960 (0.40), 3.284 (0.93), 4.019 (0.87), 4.346 (0.46), 4.359 (0.56), 7.043 (1.77), 7.067 (1.81), 7.298 (0.38), 7.320 (0.75), 7.340 (0.41), 7.549 (0.51), 7.568 (0.52), 7.756 (0.47), 7.771 (0.56), 7.778 (0.91), 7.793 (0.91), 7.799 (0.53), 7.814 (0.44), 8.224 (2.04), 8.246 (1.89), 8.463 (3.15), 9.938 (2.76). |
| **18** | 1-(2,4-Difluorphenyl)-4-oxo-7-(3-oxo-piperazin-1-yl)-*N*-(tricyclo[3.3.1.1$^{3,7}$]dec-1-yl)-1,4-dihydro-1,8-naphthyridin-3-carbonsäureamid 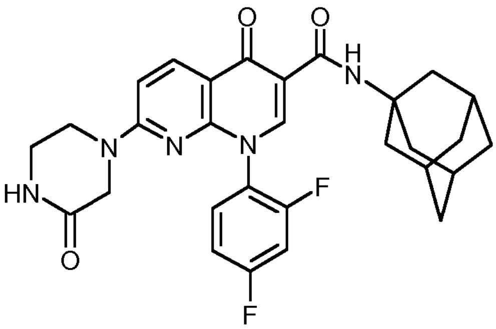 (96 % d. Th.) | LC-MS (Methode 1): $R_t$ = 1.11 min MS (ESpos): m/z = 534.4 $[M+H]^+$ $^1$H-NMR (400 MHz, DMSO-$d_6$) δ [ppm]: -0.013 (0.90), 0.003 (0.81), 0.915 (0.58), 0.930 (4.33), 0.946 (4.10), 1.666 (7.41), 1.877 (0.19), 1.897 (0.25), 1.915 (0.21), 2.052 (16.00), 2.151 (0.23), 2.171 (0.30), 2.191 (0.17), 2.322 (0.13), 2.361 (0.17), 2.386 (0.18), 2.404 (0.48), 2.421 (0.47), 2.439 (0.17), 2.664 (0.14), 2.689 (1.26), 2.705 (0.18), 2.939 (0.26), 2.955 (0.35), 2.972 (0.26), 3.219 (1.44), 3.280 (0.50), 3.631 (1.43), 3.896 (2.24), 7.041 (1.72), 7.064 (1.76), 7.315 (0.41), 7.337 (0.79), 7.358 (0.44), 7.562 (0.50), 7.569 (0.52), 7.591 (0.74), 7.610 (0.52), 7.617 (0.50), 7.773 (0.50), 7.788 (0.58), 7.794 (0.96), 7.809 (0.96), 7.816 (0.54), 7.831 (0.47), 8.138 (1.26), 8.309 (2.58), 8.332 (2.40), 8.503 (4.84), 9.886 (2.76). |

**Beispiel 19**

1-(2,4-Difluorphenyl)-7-[(3*R*)-3-methoxypyrrolidin-1-yl]-*N*-(4-methylbicyclo[2.2.2]oct-1-yl)-4-oxo-1,4-dihydro-1,8-naphthyridin-3-carbonsäureamid

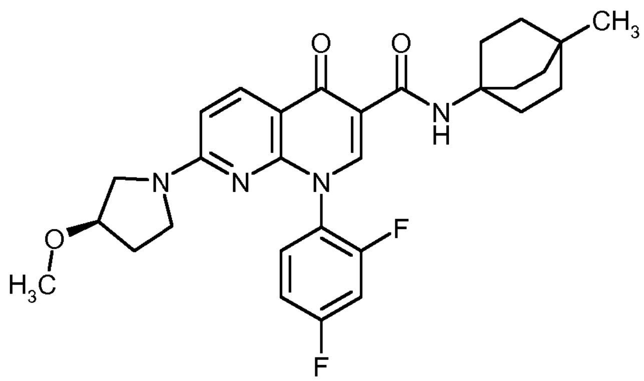

Ein Gemisch aus 100 mg (0.18 mmol) der Verbindung aus Beispiel 70A, 50 mg (0.23 mmol) *(3R)-3*-Methoxypyrrolidintrifluoracetat und 114 mg (0.89 mmol) DIPEA in 3.6 ml NMP wurde für 24 h bei 23°C gerührt. Anschliessend wurde das Gemisch über präparative HPLC gereinigt (Methode 7). Man erhielt 61 mg (66 % d. Th.) der Titelverbindung.
LC-MS (Methode 1): $R_t$ = 1.38 min; m/z = 523.3 $[M+H]^+$.
$^1$H-NMR (400 MHz, DMSO-$d_6$) $\delta$ [ppm]: -0.150 (0.80), -0.009 (7.08), 0.007 (6.82), 0.146 (0.80), 0.786 (16.00), 0.824 (0.73), 0.947 (0.63), 0.965 (0.63), 1.147 (0.53), 1.156 (0.75), 1.174 (1.49), 1.192 (0.80), 1.235 (0.53), 1.447 (4.65), 1.467 (6.04), 1.487 (5.65), 1.893 (5.84), 1.906 (5.88), 1.914 (6.31), 1.933 (5.14), 1.987 (2.90), 2.327 (1.20), 2.366 (2.84), 2.520 (3.49), 2.523 (3.78), 2.525 (3.98), 2.559 (1.63), 2.562 (1.22), 2.569 (0.71), 2.573 (0.55), 2.664 (0.98), 2.669 (1.27), 2.673 (0.90), 2.709 (2.86), 3.203 (3.51), 3.369 (0.53), 3.510 (0.75), 4.020 (0.69), 4.038 (0.75), 4.056 (0.53), 6.713 (4.24), 6.735 (4.29), 7.290 (0.65), 7.309 (1.27), 7.335 (0.75), 7.559 (0.86), 7.747 (0.67), 7.769 (1.33), 7.784 (1.31), 7.805 (0.55), 8.246 (3.49), 8.268 (3.25), 8.465 (8.24), 9.893 (4.82).

In Analogie zu Beispiel 19 wurde die in Tabelle 2 gezeigte Beispielverbindung hergestellt, indem die Verbindung aus Beispiel 70A mit (*R*)-(-)-3-Hydroxypyrrolidin Hydrochlorid umgesetzt wurde.

Tabelle 2:

| Bsp. | | Analytische Daten |
|---|---|---|
| 20 | 1-(2,4-Difluorphenyl)-7-[(3*R*)-3-hydroxypyrrolidin-1-yl] -*N*-(4-methyl-bicyclo [2.2.2]oct-1-yl)-4-oxo-1,4-dihydro-1,8-naphthyridin-3-carbonsäureamid 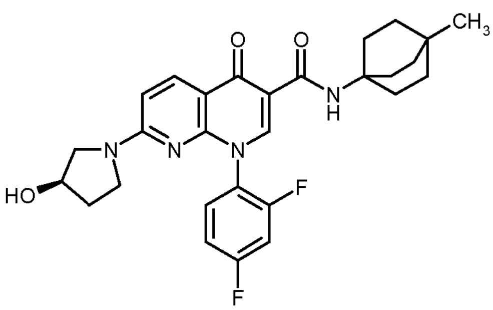 (69 % d. Th.) | LC-MS (Methode 1): $R_t$ = 1.21 min MS (ESpos): m/z = 509.3 $[M+H]^+$ $^1$H-NMR (400 MHz, DMSO-$d_6$) $\delta$ [ppm]: -0.150 (0.42), 0.146 (0.38), 0.786 (12.69), 1.156 (4.38), 1.174 (8.48), 1.192 (4.27), 1.448 (4.66), 1.467 (6.21), 1.487 (5.27), 1.782 (0.40), 1.893 (6.18), 1.914 (6.62), 1.933 (4.80), 1.987 (16.00), 2.327 (0.72), 2.366 (0.96), 2.669 (0.74), 2.710 (1.01), 3.054 (0.40), 3.149 (0.75), 3.498 (0.91), 4.002 (1.32), 4.020 (3.89), 4.038 (3.86), 4.056 (1.25), 4.248 (0.53), 4.370 (0.46), 4.894 (0.37), 4.954 (0.34), 5.007 (0.35), 6.707 (1.01), 7.288 (0.71), 7.309 (1.31), 7.328 (0.77), 7.538 (0.71), 7.560 (1.28), 7.579 (0.66), 7.745 (0.53), 7.765 (1.05), 7.780 (1.03), 7.802 (0.51), 8.237 (1.63), 8.259 (1.67), 8.458 (5.07), 9.902 (4.00). |

## Beispiel 21

1-(2,4-Difluorphenyl)-*N*-[2-(2,6-difluorphenyl)propan-2-yl]-7-[(3R)-3-hydroxypyrrolidin-1-yl]-4-oxo-1,4-dihydro-1,8-naphthyridin-3-carbonsäureamid

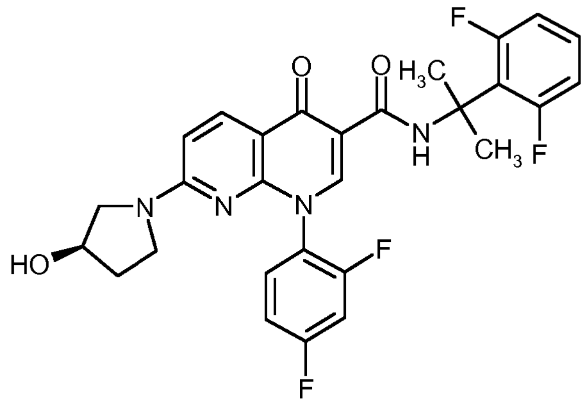

Ein Gemisch aus 400 mg (0.61 mmol) der Verbindung aus Beispiel 71A, 151 mg (1.2 mmol) (*R*)-(-)-3-Hydroxypyrrolidin Hydrochlorid und 396 mg (3.0 mmol) DIPEA in 12.5 ml NMP wurde für 24 h bei 23°C gerührt. Anschliessend wurde das Gemisch im Vakuum eingeengt und über präparative HPLC gereinigt (Methode 7). Man erhielt 201 mg (59 % d. Th.) der Titelverbindung.

LC-MS (Methode 1): $R_t$ = 1.10 min; m/z = 541.3 $[M+H]^+$.

1H-NMR (400 MHz, DMSO-$d_6$) δ [ppm]: -0.009 (2.16), -0.007 (1.87), 0.007 (2.12), 1.156 (3.27), 1.174 (6.61), 1.192 (3.31), 1.824 (16.00), 1.899 (0.99), 1.987 (12.10), 2.327 (0.40), 2.366 (0.62), 2.520 (1.01), 2.523 (1.08), 2.525 (1.05), 2.558 (0.48), 2.560 (0.39), 2.563 (0.35), 2.665 (0.32), 2.669 (0.41), 2.673 (0.33), 2.709 (0.62), 3.047 (0.34), 3.154 (0.68), 3.507 (0.82), 4.002 (0.92), 4.020 (2.74), 4.038 (2.73), 4.055 (0.88), 4.252 (0.52), 4.379 (0.44), 4.892 (0.29), 4.953 (0.32), 5.007 (0.29), 6.733 (0.92), 6.756 (0.59), 6.923 (0.37), 6.935 (2.26), 6.957 (3.14), 6.982 (2.65), 6.995 (0.39), 7.226 (0.48), 7.241 (1.09), 7.247 (1.04), 7.262 (2.30), 7.277 (2.05), 7.283 (2.06), 7.298 (1.07), 7.510 (0.64), 7.515 (0.68), 7.537 (1.19), 7.557 (0.67), 7.563 (0.64), 7.730 (0.45), 7.751 (0.96), 7.767 (0.93), 7.788 (0.41), 8.148 (0.29), 8.274 (1.68), 8.297 (1.63), 8.398 (6.56), 10.700 (4.58).

In Analogie zu Beispiel 21 wurde die in Tabelle 3 gezeigte Beispielverbindung hergestellt, indem die Verbindung aus Beispiel 71A mit (3*R*)-3-Methoxypyrrolidin Trifluoroacetat umgesetzt wurde.

Tabelle 3:

| Bsp. | | Analytische Daten |
|---|---|---|
| **22** | 1-(2,4-Difluorphenyl)-*N*-[2-(2,6-difluorphenyl) propan-2-yl]-7-[(3*R*)-3-methoxypyrrolidin-1-yl]-4-oxo-1,4-dihydro-1,8-naphthyridin-3-carbonsäureamid 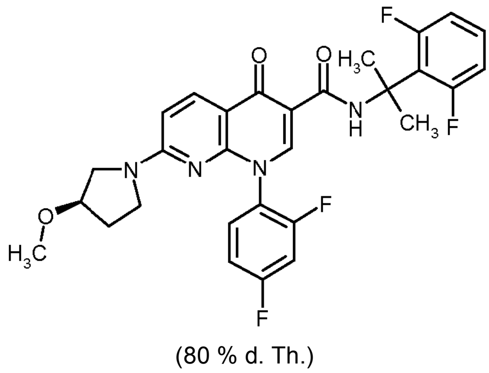 (80 % d. Th.) | LC-MS (Methode 1): $R_t$ = 1.19 min MS (ESpos): m/z = 541.3 $[M+H]^+$ $^1$H-NMR (400 MHz, DMSO-$d_6$) δ [ppm]: - 0.009 (1.27), 0.007 (1.11), 1.156 (4.13), 1.174 (8.37), 1.192 (4.22), 1.824 (11.59), 1.907 (1.33), 1.936 (1.03), 1.987 (16.00), 3.205 (2.05), 3.444 (0.43), 3.526 (0.53), 3.573 (0.41), 4.002 (1.30), 4.020 (3.76), 4.038 (3.78), 4.055 (1.40), 6.739 (2.53), 6.761 (2.58), 6.935 (1.64), 6.957 (2.30), 6.982 (1.93), 7.242 (0.81), 7.248 (0.82), 7.262 (1.61), 7.278 (1.49), 7.283 (1.44), 7.299 (0.73), 7.515 (0.40), 7.538 (0.66), 7.555 (0.46), 7.733 (0.43), 7.755 (0.85), 7.770 (0.86), 8.284 (2.18), 8.306 (2.06), 8.379 (0.40), 8.406 (5.65), 8.426 (0.41), 10.691 (3.02). |

## Beispiel 23

*rac*-4-{8-(2,4-Difluorphenyl)-5-oxo-6-[(1,1,1-trifluorbutan-2-yl)carbamoyl]-5,8-dihydro-1,8-naphthyridin-2-yl}piperazin-1-carbonsäuremethylester

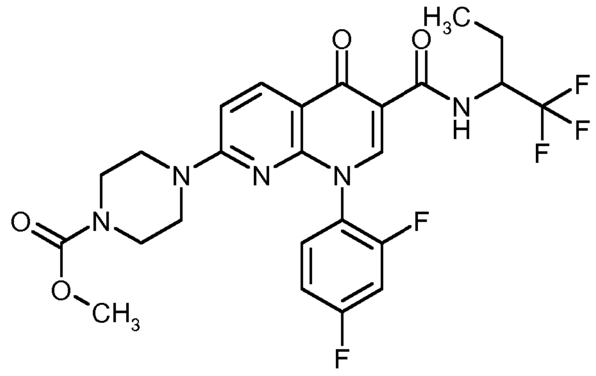

Ein Gemisch aus 100 mg (0.2 mmol) der Verbindung aus Beispiel 66A, 65 mg (0.45 mmol) Piperazin-1-carbonsäuremethylester und 116 mg (0.9 mmol) DIPEA in 4.6 ml NMP wurde 24 h bei 23°C gerührt. Der Ansatz wurde anschliessend mit Wasser verdünnt, mit 1 M wässr. Salzsäure auf pH 7 gebracht und der ausgefallene Feststoff abfiltriert. Der erhaltene Feststoff wurde mit Wasser und Petrolether gewaschen. Man erhielt 98 mg (76 % d. Th.) der Titelverbindung.

LC-MS (Methode 1): $R_t$ = 1.11 min; m/z = 554.2 $[M+H]^+$.

$^1$H-NMR (400 MHz, DMSO-$d_6$) $\delta$ [ppm]: -0.009 (2.77), 0.007 (2.54), 0.946 (2.28), 0.964 (5.05), 0.983 (2.47), 1.156 (1.15), 1.174 (2.29), 1.192 (1.16), 1.615 (0.44), 1.632 (0.54), 1.641 (0.48), 1.650 (0.45), 1.658 (0.53), 1.859 (0.47), 1.868 (0.46), 1.878 (0.54), 1.884 (0.47), 1.894 (0.42), 1.987 (4.10), 2.523 (0.64), 3.381 (3.36), 3.394 (2.76), 3.508 (2.37), 3.519 (3.03), 3.594 (1.16), 3.604 (16.00), 4.020 (0.95), 4.038 (0.96), 4.734 (0.46), 4.754 (0.43), 7.108 (2.25), 7.131 (2.32), 7.312 (0.43), 7.329 (0.82), 7.334 (0.85), 7.350 (0.46), 7.355 (0.46), 7.560 (0.51), 7.567 (0.54), 7.586 (0.80), 7.589 (0.80), 7.609 (0.54), 7.616 (0.52), 7.801 (0.54), 7.811 (0.55), 7.825 (0.53), 8.321 (3.02), 8.344 (2.79), 8.641 (1.92), 8.647 (1.77), 10.431 (1.61), 10.455 (1.56).

660 mg der racemischen Titelverbindung wurde durch chirale HPLC in die Enantiomere getrennt (präparative HPLC: Säule Daicel® Chiralpak AD-H, 5 μm, 250 x 20 mm; Eluent: 85% $CO_2$ / 15% Isopropanol; Fluss 70 ml/min; 40°C, Detektion: 210 nm).

Man erhielt (in der Reihenfolge der Elution von der Säule) 252 mg Enantiomer A $R_t$ = 2.24 min und 230 mg Enantiomer B $R_t$ = 2.51 min.

[Analytische HPLC:Säule SFC Daicel® Chiralpak AD-3, 3ml/min; Eluent A: $CO_2$, Eluent B: Isopropanol Gradient 5% B $\rightarrow$ 50% B]

## Beispiel 24

*ent*-4-{8-(2,4-Difluorphenyl)-5-oxo-6-[(1,1,1-trifluorbutan-2-yl)carbamoyl]-5,8-dihydro-1,8-naphthyridin-2-yl}piperazin-1-carbonsäuremethylester (Enantiomer A)

## Beispiel 25

*ent*-4-{8-(2,4-Difluorphenyl)-5-oxo-6-[(1,1,1-trifluorbutan-2-yl)carbamoyl]-5,8-dihydro-1,8-naphthyridin-2-yl}piperazin-1-carbonsäuremethylester (Enantiomer B)

In Analogie zu Beispiel 23 wurden die in Tabelle 4 gezeigten Beispielverbindungen hergestellt, indem die Verbindung aus Beispiel 66A bzw. 67A mit den entsprechenden Aminen (bzw. deren Salzen) unter den beschriebenen Reaktionsbedingungen umgesetzt wurden. Abweichungen sind bei den jeweiligen Beispielen angegeben.

Tabelle 4:

| Bsp. | | Analytische Daten |
|---|---|---|
| **26** | *rac*-1-(2,4-Difluorphenyl)-7-[3-(hydroxymethyl) azetidin-1-yl]-4-oxo-*N*-[1,1,1-trifluorbutan-2-yl]-1,4-dihydro-1,8-naphthyridin-3 -carbonsäureamid 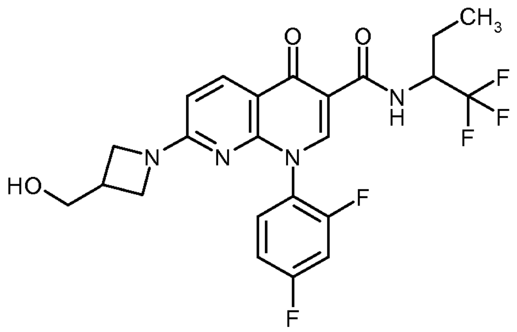 (80 % d. Th.) | LC-MS (Methode 1): $R_t$ = 0.98 min MS (ESpos): m/z = 497.2 $[M+H]^+$ <br> $^1$H-NMR (400 MHz, DMSO-$d_6$) δ [ppm]: - 0.009 (2.48), 0.007 (2.54), 0.943 (7.17), 0.961 (16.00), 0.979 (7.83), 1.156 (2.16), 1.174 (4.31), 1.192 (2.20), 1.592 (1.08), 1.610 (1.44), 1.617 (1.31), 1.627 (1.76), 1.635 (1.60), 1.645 (1.56), 1.652 (1.71), 1.671 (1.29), 1.845 (1.29), 1.855 (1.50), 1.864 (1.51), 1.873 (1.71), 1.880 (1.53), 1.890 (1.34), 1.899 (1.16), 1.908 (1.03), 1.987 (7.76), 2.709 (0.66), 2.722 (0.66), 2.736 (1.22), 2.742 (1.47), 2.756 (2.11), 2.770 (1.55), 2.776 (1.32), 2.791 (0.75), 3.499 (5.39), 3.513 (9.07), 3.527 (5.29), 3.777 (0.78), 4.002 (1.02), 4.020 (2.25), 4.037 (2.22), 4.055 (0.99), 4.703 (0.79), 4.718 (1.35), 4.726 (1.43), 4.747 (1.44), 4.764 (3.32), 4.777 (5.94), 4.790 (2.69), 6.559 (9.51), 6.581 (9.58), 7.280 (1.33), 7.284 (1.45), 7.287 (1.39), 7.301 (2.68), 7.306 (2.81), 7.322 (1.50), 7.327 (1.54), 7.329 (1.42), 7.524 (1.63), 7.530 (1.72), 7.549 (2.56), 7.553 (2.59), 7.572 (1.72), 7.579 (1.66), 7.761 (0.88), 7.782 (1.90), 7.798 (1.86), 7.818 (0.79), 8.254 (9.96), 8.276 (9.63), 8.593 (10.07), 10.486 (5.14), 10.510 (4.93). |
| **27** | 1-(2,4-Difluorphenyl)-7-[(2-fluoroethyl)-amino]-4-oxo-*N*-[(2*R*)-1,1,1-trifluorbutan-2-yl]-1,4-dihydro-1,8-naphthyridin-3-carbonsäureamid 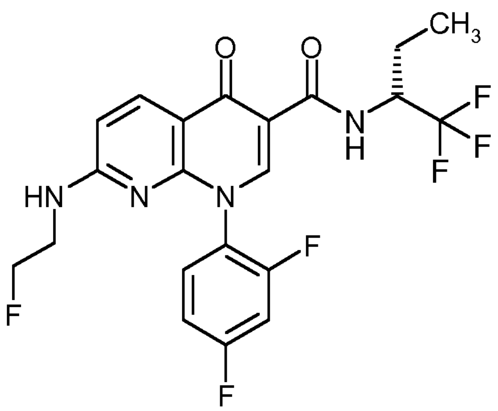 Verbindung aus Bsp. 67A und 2-Fluorethylamin Hydrochlorid (28 % d. Th.) | LC-MS (Methode 1): $R_t$ = 1.08 min MS (ESpos): m/z = 473.3 $[M+H]^+$ <br> $^1$H-NMR (400 MHz, DMSO-$d_6$) δ [ppm]: - 0.010 (1.41), 0.943 (7.22), 0.961 (16.00), 0.980 (7.84), 1.594 (1.08), 1.612 (1.45), 1.619 (1.30), 1.628 (1.78), 1.637 (1.59), 1.647 (1.52), 1.654 (1.72), 1.672 (1.30), 1.846 (1.27), 1.856 (1.47), 1.864 (1.51), 1.875 (1.70), 1.881 (1.52), 1.891 (1.34), 1.900 (1.15), 1.908 (1.18), 3.161 (5.33), 3.174 (5.48), 3.241 (1.95), 4.075 (1.84), 4.088 (1.80), 4.248 (2.71), 4.366 (2.71), 4.727 (1.47), 4.743 (1.38), 6.728 (4.34), 6.750 (4.47), 7.298 (1.44), 7.319 (2.88), 7.336 (1.53), 7.340 (1.58), 7.539 (1.60), 7.546 (1.68), 7.565 (2.66), 7.568 (2.69), 7.587 (1.71), 7.594 (1.66), 7.805 (1.94), 7.813 (1.70), 7.827 (1.93), 8.199 (5.46), 8.221 (5.11), 8.602 (5.92), 10.503 (4.35), 10.527 (4.24). |

(fortgesetzt)

| Bsp. | | Analytische Daten |
|---|---|---|
| **28** | 7-(6,6-Difluor-3-azabicyclo[3.1.0]hex-3-yl)-1-(2,4-difluorphenyl)-4-oxo-*N*-[(2*R*)-1,1,1-trifluorbutan-2-yl]-1,4-dihydro-1,8-naphthyridin-3 -carbonsäureamid 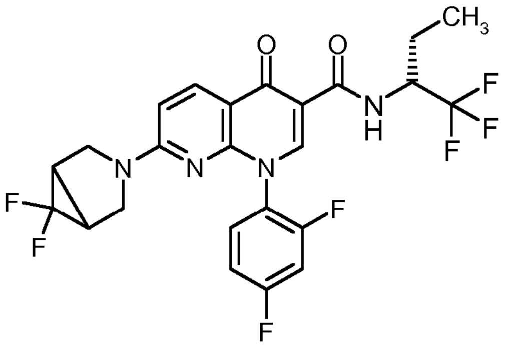 Verbindung aus Bsp. 67A und 6,6-Difluor-3-azabicyclo[3.1.0]hexan Hydrochlorid (81 % d. Th.) | LC-MS (Methode 1): $R_t$ = 1.14 min MS (ESpos): m/z = 529.2 $[M+H]^+$ $^1$H-NMR (400 MHz, DMSO-$d_6$) $\delta$ [ppm]: - 0.009 (5.85), 0.007 (5.33), 0.946 (5.73), 0.965 (12.67), 0.983 (6.30), 1.156 (4.25), 1.174 (8.61), 1.192 (4.36), 1.615 (1.09), 1.623 (0.95), 1.632 (1.35), 1.641 (1.24), 1.650 (1.18), 1.658 (1.35), 1.676 (0.99), 1.849 (1.00), 1.858 (1.19), 1.867 (1.21), 1.877 (1.36), 1.884 (1.23), 1.894 (1.09), 1.987 (16.00), 2.365 (1.97), 2.519 (3.11), 2.522 (3.24), 2.561 (1.42), 2.563 (1.28), 2.623 (1.10), 2.669 (1.19), 2.709 (2.96), 2.730 (1.03), 3.438 (1.56), 3.818 (2.15), 4.002 (1.31), 4.020 (3.85), 4.038 (3.83), 4.055 (1.28), 4.733 (1.15), 4.747 (1.10), 6.772 (5.99), 6.794 (6.14), 7.309 (1.00), 7.329 (1.92), 7.346 (1.08), 7.581 (1.05), 7.815 (1.42), 7.831 (1.36), 8.318 (6.82), 8.340 (6.51), 8.638 (6.35), 10.443 (3.83), 10.467 (3.73). |
| **29** | 7-(1,1-Difluor-5-azaspiro[2.4]hept-5-yl)-1-(2,4-Difluorphenyl)-4-oxo-*N*-[(2*R*)-1,1,1-trifluorbutan-2-yl]-1,4-dihydro-1,8-naphthyridin-3 -carbonsäureamid (Diastereomerengemisch) 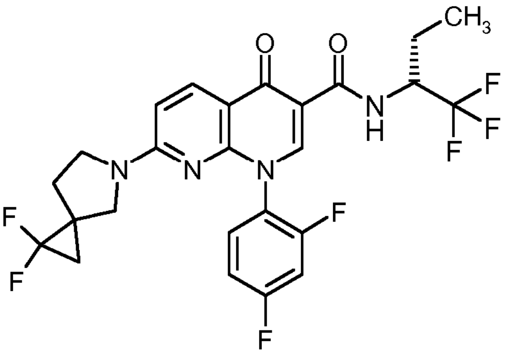 Verbindung aus Bsp. 67A und 1,1-Difluor-5-azaspiro[2.4]heptan Hydrochlorid (74 % d. Th.) | LC-MS (Methode 1): $R_t$ = 1.23 min MS (ESpos): m/z = 543.2 $[M+H]^+$ $^1$H-NMR (400 MHz, DMSO-$d_6$) $\delta$ [ppm]: - 0.150 (2.00), 0.007 (16.00), 0.146 (1.96), 0.948 (5.32), 0.967 (10.79), 0.986 (5.25), 1.156 (3.44), 1.174 (6.39), 1.192 (3.21), 1.237 (0.78), 1.618 (3.03), 1.634 (2.73), 1.860 (1.18), 1.870 (1.26), 1.879 (1.29), 1.896 (1.18), 1.987 (12.34), 2.127 (0.96), 2.327 (1.74), 2.366 (6.87), 2.671 (1.81), 2.710 (6.54), 3.599 (0.92), 4.002 (1.18), 4.020 (2.85), 4.038 (2.85), 4.055 (0.92), 4.735 (1.22), 6.783 (1.18), 7.297 (1.22), 7.316 (2.11), 7.333 (1.18), 7.539 (1.07), 7.558 (1.81), 7.580 (0.96), 7.805 (1.55), 7.819 (1.59), 8.315 (3.92), 8.338 (3.77), 8.629 (5.54), 10.468 (3.21), 10.492 (3.07). |

(fortgesetzt)

| Bsp. | | Analytische Daten |
|---|---|---|
| **30** | 1-(2,4-Difluorphenyl)-7-(3-fluor-3-methylpyrrolidin-1-yl)-4-oxo-*N*-[(2*R*)-1,1,1-trifluorbutan-2-yl]-1,4-dihydro-1,8-naphthyridin-3 -carbonsäureamid (Diastereomerengemisch) 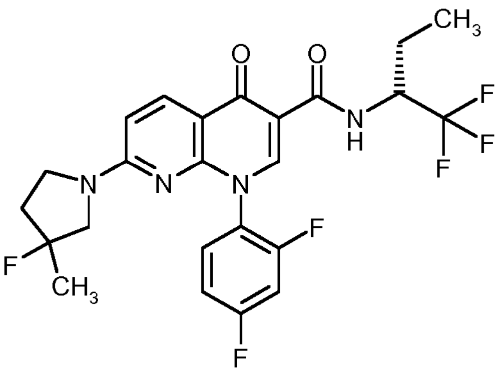 Verbindung aus Bsp. 67A und 3-Fluor-3-methylpyrrolidin *para*-Toluolsulfonsäure Salz (85 % d. Th.) | LC-MS (Methode 1): $R_t$ = 1.26 min MS (ESpos): m/z = 513.3 $[M+H]^+$ $^1$H-NMR (400 MHz, DMSO-$d_6$) $\delta$ [ppm]: - 0.002 (16.00), 0.948 (5.01), 0.967 (11.10), 0.985 (5.46), 1.156 (1.21), 1.173 (2.44), 1.191 (1.23), 1.459 (1.19), 1.511 (2.33), 1.565 (1.27), 1.599 (0.91), 1.617 (1.07), 1.624 (0.94), 1.633 (1.24), 1.642 (1.13), 1.652 (1.06), 1.659 (1.21), 1.677 (0.91), 1.695 (0.26), 1.850 (0.89), 1.860 (1.04), 1.868 (1.07), 1.878 (1.19), 1.885 (1.05), 1.895 (0.94), 1.903 (0.82), 1.913 (0.70), 1.987 (4.81), 2.137 (0.61), 2.366 (0.59), 2.709 (0.59), 3.166 (7.39), 3.532 (0.58), 3.699 (0.58), 4.001 (0.41), 4.019 (1.16), 4.037 (1.20), 4.055 (0.66), 4.077 (1.28), 4.089 (1.19), 4.733 (1.03), 4.752 (0.97), 6.755 (0.65), 6.797 (0.60), 7.305 (0.82), 7.325 (1.62), 7.343 (0.96), 7.575 (1.25), 7.595 (0.87), 7.810 (1.27), 7.826 (1.24), 8.309 (1.63), 8.330 (1.59), 8.625 (5.70), 10.476 (3.51), 10.500 (3.39). |
| **31** | 1-(2,4-Difluorphenyl)-7-[(2*R*,4*S*)-4-fluor-2-methylpyrrolidin-1-yl]-4-oxo-*N*-[(2*R*)-1,1,1-trifluorbutan-2-yl]-1,4-dihydro-1,8-naphthyridin-3 -carbonsäureamid 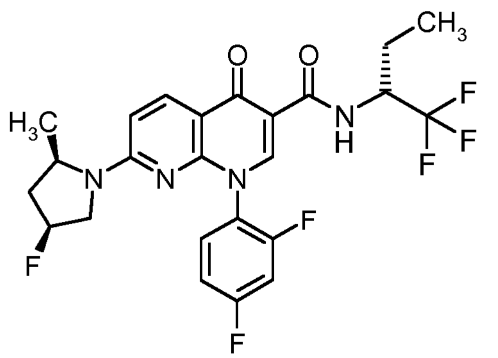 Verbindung aus Bsp. 67A und (2*R*,4*S*)-4-Fluor-2-methylpyrrolidin *para*-Toluolsulfonsäure Salz (70 % d. Th.) | LC-MS (Methode 1): $R_t$ = 1.27 min MS (ESpos): m/z = 513.3 $[M+H]^+$ $^1$H-NMR (400 MHz, DMSO-$d_6$) $\delta$ [ppm]: - 0.150 (0.90), -0.009 (8.02), 0.007 (7.82), 0.145 (0.90), 0.891 (0.90), 0.949 (7.45), 0.968 (16.00), 0.986 (8.02), 1.047 (1.02), 1.133 (0.70), 1.146 (0.90), 1.156 (0.94), 1.174 (1.51), 1.192 (0.98), 1.235 (0.57), 1.600 (1.06), 1.618 (1.43), 1.625 (1.35), 1.635 (1.68), 1.643 (1.60), 1.653 (1.47), 1.660 (1.72), 1.679 (1.35), 1.698 (0.37), 1.851 (1.35), 1.861 (1.51), 1.870 (1.64), 1.880 (1.88), 1.886 (1.72), 1.896 (1.64), 1.904 (1.51), 1.914 (1.43), 1.987 (2.58), 2.226 (0.53), 2.251 (0.53), 2.322 (1.15), 2.327 (1.47), 2.331 (1.19), 2.347 (0.57), 2.365 (6.55), 2.454 (0.45), 2.518 (6.51), 2.564 (2.25), 2.567 (2.13), 2.575 (1.15), 2.585 (0.65), 2.589 (0.49), 2.594 (0.49), 2.596 (0.49), 2.611 (0.41), 2.665 (0.90), 2.669 (1.19), 2.674 (0.86), 2.709 (6.38), 3.161 (2.46), 3.174 (2.70), 3.443 (0.37), 3.456 (0.49), 3.820 (0.53), 3.916 (0.53), 4.002 (0.41), 4.020 (0.57), 4.038 (0.61), 4.061 (0.37), 4.074 (0.70), 4.087 (0.70), 4.712 (0.82), 4.735 (1.39), 4.754 (1.35), 5.331 (0.70), 5.462 (0.70), 6.759 (0.82), 7.307 (1.27), 7.328 (2.58), 7.348 (1.43), 7.574 (1.47), 7.591 (1.64), 7.792 (1.15), 7.813 (2.33), 7.829 (2.25), 7.850 (1.06), 8.306 (7.53), 8.328 (7.12), 8.644 (9.49), 10.479 (5.07), 10.503 (4.87). |

(fortgesetzt)

| Bsp. | | Analytische Daten |
|---|---|---|
| **32** | 1-(2,4-Difluorphenyl)-7-(3-fluorazetidin-1-yl)-4-oxo-*N*-[(2*R*)-1,1,1-trifluorbutan-2-yl]-1,4-dihydro-1,8-naphthyridin-3-carbonsäureamid 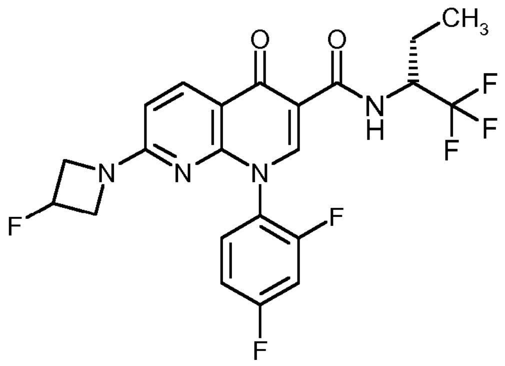 Verbindung aus Bsp. 67A und 3-Fluorazetidin Hydrochlorid (37 % d. Th.) | LC-MS (Methode 1): $R_t$ = 1.19 min MS (ESpos): m/z = 485.3 $[M+H]^+$ $^1$H-NMR (400 MHz, DMSO-$d_6$) δ [ppm]: - 0.013 (4.92), 0.003 (4.10), 0.851 (3.11), 0.869 (6.83), 0.887 (3.72), 0.940 (7.25), 0.959 (16.00), 0.977 (7.76), 1.083 (1.53), 1.103 (2.06), 1.123 (1.81), 1.276 (1.29), 1.295 (2.23), 1.313 (2.25), 1.573 (1.82), 1.592 (2.84), 1.611 (2.42), 1.628 (1.99), 1.636 (1.74), 1.646 (1.61), 1.653 (1.84), 1.671 (1.50), 1.844 (1.34), 1.854 (1.55), 1.863 (1.53), 1.872 (1.73), 1.879 (1.53), 1.889 (1.38), 1.983 (1.34), 4.016 (1.51), 4.033 (1.42), 4.260 (1.47), 4.728 (1.44), 4.748 (1.39), 5.365 (1.42), 5.372 (1.67), 5.379 (1.36), 5.508 (1.35), 5.515 (1.72), 5.522 (1.36), 6.654 (9.23), 6.676 (9.40), 7.289 (1.50), 7.306 (2.80), 7.311 (2.88), 7.328 (1.57), 7.332 (1.64), 7.527 (1.81), 7.534 (1.84), 7.553 (2.73), 7.556 (2.77), 7.576 (1.81), 7.582 (1.71), 7.792 (2.02), 7.808 (1.97), 8.320 (9.70), 8.342 (9.33), 8.629 (10.09), 10.427 (4.92), 10.450 (4.79). |
| **33** | 1-(2,4-Difluorphenyl)-7-[(3*R*,4*R*)-4-fluor-3-hydroxypiperidin-1-yl]-4-oxo-*N*-[(2*R*)-1,1,1-trifluorbutan-2-yl]-1,4-dihydro-1,8-naphthyridin-3 -carbonsäureamid 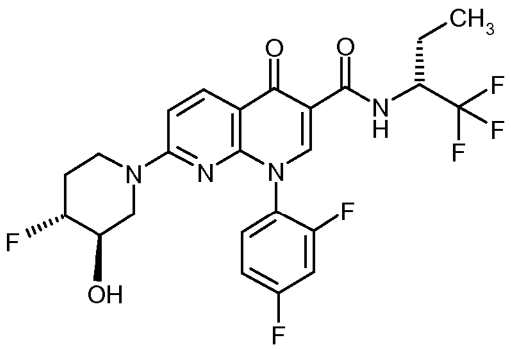 Verbindung aus Bsp. 67A und (4*R*)-Fluor-(3*R*)-piperidinol (HCl-Salz) (75 % d. Th.) | LC-MS (Methode 1): $R_t$ = 1.07 min MS (ESpos): m/z = 529.3 $[M+H]^+$ $^1$H-NMR (500 MHz, DMSO-$d_6$) δ [ppm]: - 0.005 (6.51), 0.949 (7.71), 0.964 (16.00), 0.978 (7.85), 1.483 (1.21), 1.605 (1.14), 1.611 (0.61), 1.620 (1.52), 1.626 (1.37), 1.633 (1.76), 1.640 (1.60), 1.648 (1.52), 1.654 (1.65), 1.668 (1.22), 1.840 (0.49), 1.855 (1.29), 1.862 (1.50), 1.869 (1.53), 1.877 (1.72), 1.882 (1.56), 1.890 (1.39), 1.897 (1.19), 1.905 (1.04), 1.977 (1.18), 1.986 (1.37), 3.044 (0.64), 3.172 (1.08), 3.180 (1.05), 3.207 (0.72), 3.216 (0.73), 3.242 (1.01), 3.262 (0.72), 3.458 (1.12), 3.800 (0.90), 3.879 (0.84), 3.910 (1.36), 3.940 (0.77), 4.427 (0.95), 4.525 (0.97), 4.733 (1.48), 4.745 (1.40), 4.761 (0.80), 5.444 (1.68), 5.454 (2.00), 5.463 (2.46), 5.473 (2.06), 7.144 (6.66), 7.162 (6.72), 7.314 (1.45), 7.331 (2.76), 7.345 (1.54), 7.539 (0.72), 7.561 (1.54), 7.582 (1.53), 7.602 (0.74), 7.811 (1.41), 7.822 (1.95), 7.834 (1.51), 8.289 (3.51), 8.293 (3.42), 8.307 (3.42), 8.311 (3.20), 8.636 (6.64), 10.447 (4.07), 10.466 (3.95). |

(fortgesetzt)

| Bsp. | | Analytische Daten |
|---|---|---|
| **34** | 1-(2,4-Difluorphenyl)-7-[3-fluor-4,4-dihydroxypiperidin-1-yl]-4-oxo-*N*-[(2R)-1,1,1-trifluorbutan-2-yl]-1,4-dihydro-1,8-naphthyridin-3-carbonsäureamid (Diastereomerengemisch) 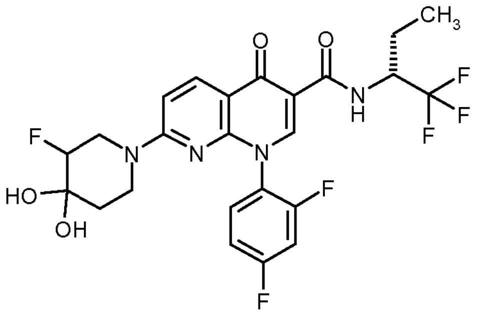 Verbindung aus Bsp. 67A und rac. 3-Fluor-piperidin-4-on Hydrochlorid. Reinigung durch präparative HPLC (Eluent: Acetonitril/Wasser-Gradient mit 0.1% Ameisensäure) . (10 % d. Th.) | LC-MS (Methode 1): $R_t$ = 0.95 min MS (ESpos): m/z = 545.4 $[M+H]^+$ $^1$H-NMR (400 MHz, DMSO-$d_6$) δ [ppm]: - 0.150 (1.60), -0.009 (15.46), 0.007 (13.56), 0.146 (1.68), 0.824 (0.88), 0.841 (2.18), 0.857 (3.04), 0.875 (1.57), 0.946 (7.18), 0.964 (16.00), 0.982 (8.24), 1.052 (0.41), 1.073 (0.52), 1.146 (0.93), 1.235 (1.42), 1.291 (1.44), 1.551 (1.16), 1.597 (1.83), 1.614 (2.50), 1.631 (3.47), 1.641 (3.64), 1.649 (3.77), 1.658 (3.02), 1.675 (1.98), 1.849 (1.38), 1.858 (1.98), 1.867 (2.13), 1.876 (2.22), 1.884 (2.29), 1.893 (1.94), 1.911 (1.25), 1.919 (0.95), 2.327 (2.16), 2.366 (3.80), 2.406 (1.08), 2.669 (2.24), 2.709 (3.13), 2.992 (0.58), 3.173 (8.88), 3.935 (0.80), 4.055 (0.71), 4.087 (0.73), 4.151 (0.60), 4.277 (1.90), 4.387 (0.67), 4.466 (0.56), 4.563 (0.97), 4.745 (1.94), 4.930 (0.37), 5.041 (0.39), 5.960 (1.49), 5.974 (1.79), 6.061 (1.51), 6.271 (1.01), 6.322 (1.08), 7.144 (2.37), 7.155 (2.05), 7.167 (2.67), 7.179 (2.05), 7.320 (1.81), 7.341 (3.47), 7.355 (3.82), 7.378 (2.54), 7.394 (1.83), 7.468 (0.37), 7.565 (1.55), 7.590 (3.13), 7.613 (2.18), 7.817 (2.11), 7.830 (2.35), 7.853 (1.90), 8.271 (2.74), 8.281 (2.72), 8.294 (2.85), 8.303 (2.46), 8.406 (5.13), 8.428 (4.72), 8.630 (5.80), 8.708 (2.98), 8.715 (3.39), 10.386 (2.67), 10.409 (2.46), 10.443 (2.44), 10.451 (2.31), 10.468 (2.50). |
| **35** | 7-(3,3-Difluor-4,4-dihydroxypiperidin-1-yl)-1-(2,4-difluorphenyl)-4-oxo-*N*-[(2*R*)-1,1,1-trifluorbutan-2-yl]-1,4-dihydro-1,8-naphthyridin-3-carbonsäureamid 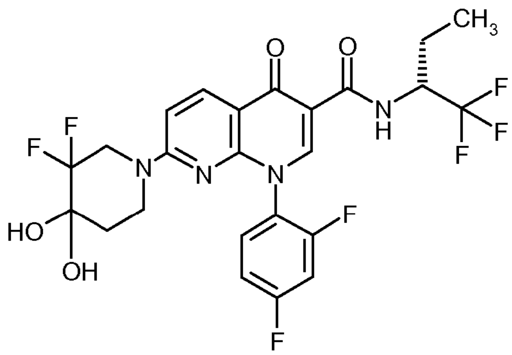 Verbindung aus Bsp. 67A und 3,3-Difluorpiperidin-4-on Hydrochlorid über präparative HPLC gereinigt (Eluent: Acetonitril/Wasser-Gradient mit 0.1% Ameisensäure) . (62 % d. Th.) | LC-MS (Methode 1): $R_t$ = 0.97 min MS (ESpos): m/z = 563.4 $[M+H]^+$ $^1$H-NMR (400 MHz, DMSO-$d_6$) δ [ppm]: - 0.009 (3.57), 0.007 (3.08), 0.878 (1.06), 0.897 (2.32), 0.915 (1.20), 0.946 (7.02), 0.964 (15.55), 0.983 (7.62), 1.156 (3.54), 1.174 (7.22), 1.191 (3.64), 1.599 (1.09), 1.617 (1.49), 1.624 (1.38), 1.634 (1.87), 1.643 (1.90), 1.652 (2.23), 1.660 (3.02), 1.677 (3.45), 1.719 (1.10), 1.725 (1.09), 1.735 (1.10), 1.743 (1.04), 1.753 (0.94), 1.851 (1.22), 1.860 (1.42), 1.869 (1.41), 1.879 (1.61), 1.885 (1.45), 1.896 (1.25), 1.904 (1.15), 1.914 (0.91), 1.987 (13.10), 2.523 (1.19), 3.243 (14.94), 3.563 (2.61), 3.842 (1.83), 4.001 (1.06), 4.019 (3.06), 4.037 (3.03), 4.055 (1.02), 4.736 (1.36), 4.751 (1.28), 5.752 (2.70), 6.432 (16.00), 6.787 (5.50), 7.238 (2.48), 7.253 (2.10), 7.261 (2.70), 7.275 (1.77), 7.329 (1.38), 7.350 (2.70), 7.366 (1.42), 7.371 (1.44), 7.574 (1.36), 7.580 (1.33), 7.599 (2.36), 7.622 (1.39), 7.628 (1.22), 7.818 (1.57), 7.830 (1.84), 7.842 (1.61), 8.320 (5.05), 8.330 (3.54), 8.343 (4.70), 8.353 (3.13), 8.658 (4.55), 8.661 (4.61), 8.665 (4.83), 10.399 (2.31), 10.408 (3.19), 10.424 (2.26), 10.432 (2.93). |

**Beispiel 36**

1-(2,4-Difluorphenyl)-7-(dimethylamino)-4-oxo-*N*-(tricyclo[3.3.1.1$^{3,7}$]dec-1-yl)-1,4-dihydro-1,8-naphthyridin-3-carbonsäureamid

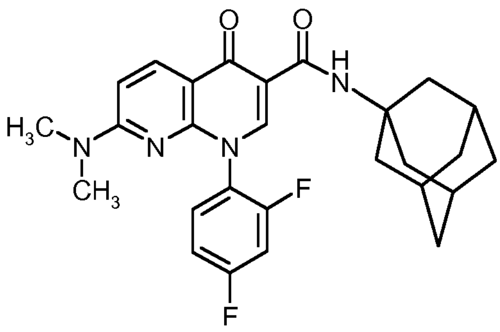

80 mg (0.23 mmol) der Verbindung aus Beispiel 36A wurden in 2.3 ml DMF vorgelegt, mit 106 mg (0.28 mmol) HATU sowie 99 mg (0.77 mmol) DIPEA versetzt und 30 Minuten lang bei 20°C gerührt. Dann wurden 49 mg (0.32 mmol) 1-Adamantanamin zugegeben und 2 Stunden bei 20°C gerührt. Anschliessend wurde das Gemisch über präparative HPLC gereinigt (Eluent: Acetonitril/Wasser-Gradient mit 0.1% Ameisensäure). Man erhielt 63 mg (57 % d. Th.) der Titelverbindung.

LC-MS (Methode 1): $R_t$ = 1.34 min; m/z = 479 [M+H]$^+$.

$^1$H-NMR (400 MHz, DMSO-$d_6$) $\delta$ [ppm]: -0.008 (2.42), 0.009 (2.27), 1.671 (7.70), 2.054 (16.00), 2.076 (1.22), 2.936 (2.35), 6.892 (2.41), 6.915 (2.39), 7.304 (0.41), 7.319 (0.71), 7.327 (0.84), 7.340 (0.41), 7.346 (0.47), 7.350 (0.41), 7.554 (0.53), 7.560 (0.54), 7.575 (0.69), 7.580 (0.79), 7.586 (0.68), 7.602 (0.55), 7.609 (0.52), 7.767 (0.50), 7.781 (0.59), 7.789 (1.00), 7.803 (0.99), 7.810 (0.55), 7.825 (0.46), 8.251 (2.69), 8.275 (2.54), 8.477 (5.26), 9.952 (2.61).

In Analogie zu Beispiel 36 wurden die in Tabelle 5 gezeigten Beispielverbindungen hergestellt, indem die Verbindung aus Beispiel 36A bzw. 60A mit den entsprechenden Aminen (bzw. deren Salzen) unter den beschriebenen Reaktionsbedingungen umgesetzt wurden. Abweichungen sind bei den jeweiligen Beispielen angegeben.

Tabelle 5:

| Bsp. | | Analytische Daten |
|---|---|---|
| **37** | 1-(2,4-Difluorphenyl)-7-(dimethylamino)-*N*-(3-fluortricyclo[3.3.1.1$^{3,7}$ ]dec-1-yl)-4-oxo-1, 4-dihydro-1, 8-naphthyridin-3 -carbonsäureamid 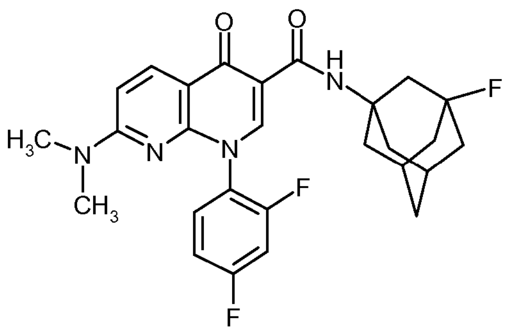 (33 % d. Th.) | LC-MS (Methode 1): $R_t$ = 1.29 min MS (ESpos): m/z = 497.3 [M+H]$^+$ $^1$H-NMR (400 MHz, $CDCl_3$) $\delta$ [ppm]: 0.009 (6.24), 0.017 (0.22), 0.078 (0.31), 1.576 (16.00), 1.631 (0.21), 1.889 (0.30), 1.944 (0.26), 2.015 (0.21), 2.053 (0.18), 2.083 (0.35), 2.137 (0.30), 2.379 (0.60), 2.450 (0.15), 2.795 (0.16), 2.993 (1.92), 3.500 (0.66), 6.656 (0.74), 6.679 (0.75), 7.007 (0.28), 7.022 (0.32), 7.028 (0.30), 7.041 (0.32), 7.047 (0.28), 7.059 (0.15), 7.351 (0.15), 7.372 (0.19), 7.386 (0.20), 7.394 (0.14), 7.409 (0.12), 7.529 (0.15), 8.407 (0.75), 8.429 (0.73), 8.627 (1.17), 10.134 (0.34). |

(fortgesetzt)

| Bsp. | | Analytische Daten |
|---|---|---|
| **38** | 1-(2,4-Difluorphenyl)-*N*-(3,5-difluor-tricyclo [3.3.1.1$^{3,7}$]dec-1-yl)-7-(dimethylamino)-4-oxo-1,4-dihydro-1,8-naphthyridin-3-carbonsäureamid 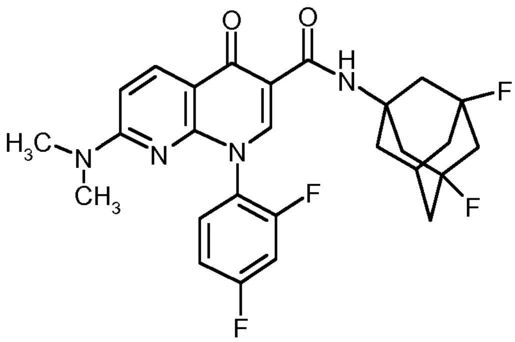 (61 % d. Th.) | LC-MS (Methode 1): $R_t$ = 1.16 min MS (ESpos): m/z = 515.3 [M+H]$^+$ <br> $^1$H-NMR (400 MHz, DMSO-$d_6$) $\delta$ [ppm]: -0.009 (9.96), 0.007 (6.42), 1.803 (11.32), 1.899 (7.42), 2.072 (2.34), 2.138 (3.86), 2.317 (4.06), 2.939 (8.32), 3.161 (10.82), 3.174 (10.98), 4.062 (1.20), 4.075 (3.14), 4.088 (3.06), 6.902 (7.86), 6.925 (7.90), 7.303 (1.50), 7.325 (2.64), 7.341 (1.46), 7.554 (1.88), 7.576 (2.60), 7.595 (1.78), 7.766 (1.76), 7.788 (3.30), 7.803 (3.20), 7.825 (1.56), 8.256 (8.82), 8.279 (8.28), 8.512 (16.00), 10.263 (7.66). |
| **39** | 1-(2,4-Difluorphenyl)-*N*-(4,4-difluor-tricyclo [3.3.1.1$^{3,7}$ ]dec-1-yl)-7-(dimethylamino)-4-oxo-1,4-dihydro-1,8-naphthyridin-3-carbonsäureamid 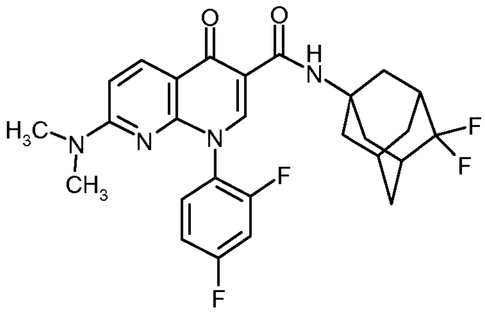 (28 % d. Th.) | LC-MS (Methode 1): $R_t$ = 1.26 min MS (ESpos): m/z = 515.3 [M+H]$^+$ <br> $^1$H-NMR (400 MHz, DMSO-$d_6$) $\delta$ [ppm]: 1.785 (9.36), 2.065 (16.00), 2.277 (8.35), 2.362 (2.47), 2.932 (8.32), 6.892 (5.42), 6.914 (5.67), 7.318 (2.44), 7.571 (2.32), 7.778 (2.38), 8.246 (5.88), 8.269 (5.64), 8.483 (10.54), 10.042 (6.95). |
| **40** | 1-(2-Chlor-4-fluorphenyl)-*N*-(4,4-difluor-tricyclo [3.3.1.1$^{3,7}$]dec-1-yl)-7-(dimethylamino)-4-oxo-1,4-dihydro-1,8-naphthyridin-3 -carbonsäureamid 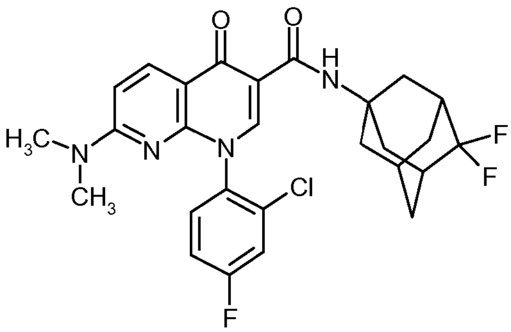 Verbindung aus Bsp. 60A (45 % d. Th.) | LC-MS (Methode 1): $R_t$ = 1.30 min MS (ESpos): m/z = 531.2 [M+H]$^+$ <br> $^1$H-NMR (400 MHz, DMSO-$d_6$) $\delta$ [ppm]: 1.790 (8.79), 2.068 (16.00), 2.280 (7.81), 2.366 (2.24), 2.709 (2.36), 2.906 (6.43), 6.882 (6.22), 6.905 (6.28), 7.473 (2.99), 7.494 (1.91), 7.773 (4.13), 7.787 (4.64), 7.809 (2.78), 8.250 (6.52), 8.272 (6.22), 8.410 (12.62), 10.064 (7.15). |

(fortgesetzt)

| Bsp. | | Analytische Daten |
|---|---|---|
| **41** | 1-(2,4-Difluorphenyl)-7-(dimethylamino)-*N*-(3-methyltricyclo[3.3.1.1$^{3,7}$ ]dec-1-yl)-4-oxo-1, 4-dihydro-1, 8-naphthyridin-3 -carbonsäureamid 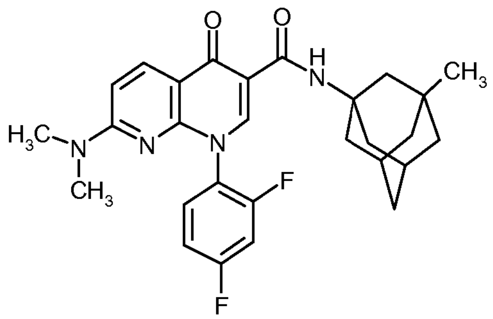 (67 % d. Th.) | LC-MS (Methode 1): $R_t$ = 1.41 min MS (ESpos): m/z = 493.3 $[M+H]^+$ $^1$H-NMR (400 MHz, DMSO-$d_6$) δ [ppm]: 0.836 (16.00), 1.415 (9.74), 1.516 (1.11), 1.546 (1.92), 1.599 (1.99), 1.630 (1.04), 1.761 (7.37), 1.912 (2.13), 1.940 (3.65), 1.999 (3.82), 2.027 (2.14), 2.092 (4.16), 2.366 (0.43), 2.936 (6.28), 6.890 (3.74), 6.912 (3.75), 7.302 (0.93), 7.318 (1.65), 7.339 (0.89), 7.553 (0.98), 7.572 (1.58), 7.595 (0.96), 7.758 (0.93), 7.779 (1.71), 7.794 (1.68), 7.816 (0.79), 8.247 (3.92), 8.269 (3.64), 8.467 (6.71), 9.958 (4.38). |
| **42** | 1-(2,4-Difluorphenyl)-7-(dimethylamino)-*N*-(4-methylbicyclo[2.2.2]oct-1-yl)-4-oxo-1, 4-dihydro-1,8-naphthyridin-3-carbonsäureamid 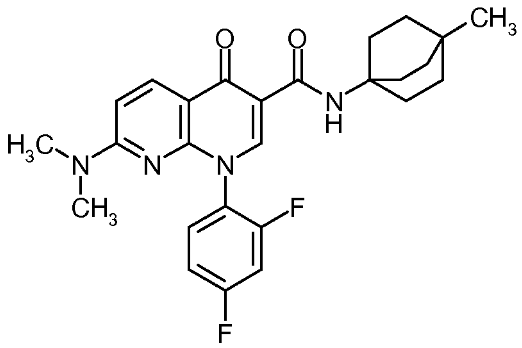 (96 % d. Th.) | LC-MS (Methode 1): $R_t$ = 1.38 min MS (ESpos): m/z = 467.3 $[M+H]^+$ $^1$H-NMR (400 MHz, DMSO-$d_6$) δ [ppm]: -0.014 (1.72), 0.003 (1.72), 0.781 (16.00), 1.442 (4.66), 1.461 (6.05), 1.481 (5.60), 1.888 (5.60), 1.900 (5.04), 1.909 (5.99), 1.928 (4.77), 2.726 (8.77), 2.885 (12.33), 2.928 (4.93), 6.879 (4.49), 6.902 (4.55), 7.285 (0.67), 7.289 (0.74), 7.292 (0.70), 7.306 (1.37), 7.311 (1.42), 7.328 (0.78), 7.332 (0.80), 7.335 (0.72), 7.534 (0.90), 7.541 (0.95), 7.560 (1.32), 7.563 (1.35), 7.582 (0.94), 7.589 (0.92), 7.748 (0.89), 7.763 (1.05), 7.769 (1.78), 7.784 (1.76), 7.791 (1.00), 7.806 (0.87), 7.947 (1.22), 8.238 (4.90), 8.260 (4.64), 8.462 (8.75), 9.879 (4.75). |
| **43** | *N-tert.*-Butyl-1-(2,4-difluorphenyl)-7-(dimethylamino)-4-oxo-1,4-dihydro-1,8-naphthyridin-3 -carbonsäureamid 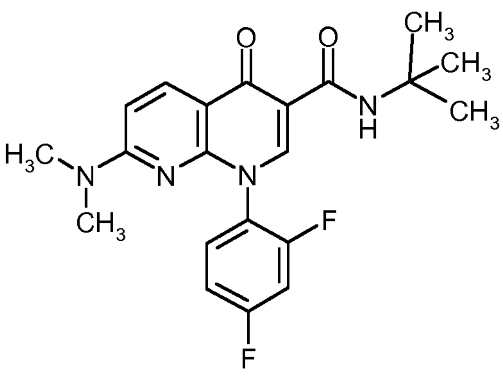 (88 % d. Th.) | LC-MS (Methode 1): $R_t$ = 1.12 min MS (ESpos): m/z = 401.1 $[M+H]^+$ $^1$H-NMR (400 MHz, DMSO-$d_6$) δ [ppm]: -0.009 (0.52), 0.007 (0.34), 1.388 (16.00), 2.523 (0.72), 2.890 (0.41), 2.938 (1.24), 6.894 (1.26), 6.916 (1.25), 7.317 (0.34), 7.321 (0.34), 7.552 (0.25), 7.568 (0.32), 7.571 (0.33), 7.574 (0.33), 7.578 (0.29), 7.759 (0.24), 7.774 (0.29), 7.780 (0.46), 7.795 (0.46), 7.802 (0.26), 8.253 (1.33), 8.276 (1.27), 8.501 (2.21), 10.014 (1.06). |

(fortgesetzt)

| Bsp. | | Analytische Daten |
|---|---|---|
| **44** | 1-(2,4-Difluorphenyl)-7-(dimethylamino)-4-oxo-*N*-[(2*S*)-1,1,1-trifluorpropan-2-yl]-1,4-dihydro-1, 8-naphthyridin-3 -carbonsäureamid 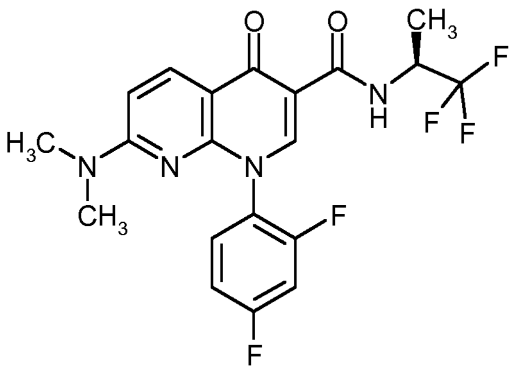 (35 % d. Th.) | LC-MS (Methode 1): $R_t$ = 1.13 min MS (ESpos): m/z = 441.1 $[M+H]^+$ $^{1}$H-NMR (400 MHz, DMSO-$d_6$) $\delta$ [ppm]: -0.150 (1.10), -0.008 (11.13), 0.007 (9.84), 0.146 (1.10), 1.360 (15.85), 1.377 (16.00), 2.365 (0.82), 2.709 (0.88), 2.945 (6.97), 4.860 (1.15), 4.880 (1.78), 4.901 (1.81), 4.919 (1.13), 6.927 (7.86), 6.949 (8.04), 7.306 (1.38), 7.326 (2.83), 7.348 (1.53), 7.558 (1.54), 7.580 (2.58), 7.599 (1.57), 7.808 (1.47), 8.267 (8.65), 8.290 (8.27), 8.607 (6.53), 10.543 (3.31), 10.566 (3.23). |
| **45** | 1-(2,4-Difluorphenyl)-7-(dimethylamino)-4-oxo-*N*-[(2*S*)-1,1,1-trifluorbutan-2-yl]-1,4-dihydro-1,8-naphthyridin-3-carbonsäureamid 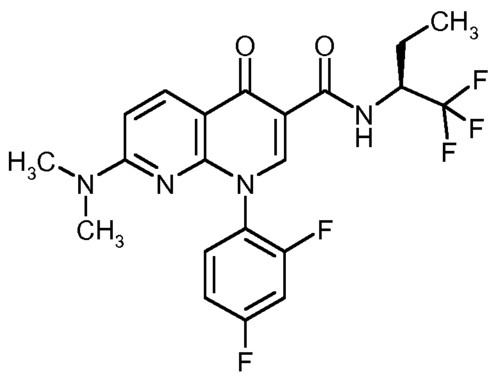 (74 % d. Th.) | LC-MS (Methode 1): $R_t$ = 1.21 min MS (ESpos): m/z = 455.2 $[M+H]^+$ $^{1}$H-NMR (400 MHz, DMSO-$d_6$) $\delta$ [ppm]: -0.009 (4.23), 0.007 (4.21), 0.946 (7.18), 0.965 (16.00), 0.983 (7.72), 1.613 (1.53), 1.630 (1.75), 1.639 (1.51), 1.648 (1.41), 1.655 (1.78), 1.858 (1.43), 1.867 (1.52), 1.876 (1.65), 1.883 (1.44), 2.669 (1.34), 2.947 (6.45), 4.732 (1.34), 4.747 (1.36), 6.929 (9.58), 6.952 (9.93), 7.304 (1.41), 7.327 (2.80), 7.343 (1.55), 7.551 (1.78), 7.558 (1.87), 7.576 (2.74), 7.599 (1.90), 7.606 (1.86), 7.805 (1.90), 7.827 (1.83), 8.275 (11.06), 8.298 (10.59), 8.614 (7.21), 10.490 (4.78), 10.514 (4.65). |
| **46** | *rac-1-*(2,4-Difluorphenyl)-7-(dimethyl-amino)-4-oxo-*N*-[4,4,4-trifluorbutan-2-yl]-1, 4-dihydro-1,8-naphthyridin-3-carbonsäureamid 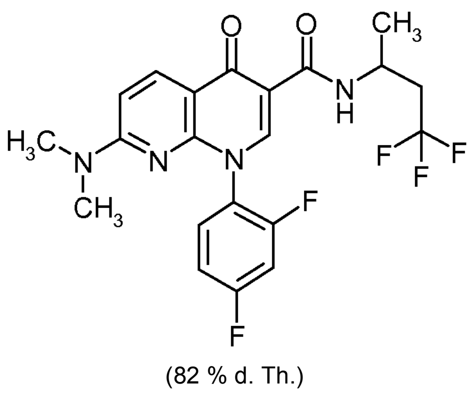 (82 % d. Th.) | LC-MS (Methode 1): $R_t$ = 1.10 min MS (ESpos): m/z = 455.3 $[M+H]^+$ $^{1}$H-NMR (400 MHz, DMSO-$d_6$) $\delta$ [ppm]: 1.272 (15.86), 1.289 (15.96), 2.522 (1.97), 2.560 (2.34), 2.573 (1.89), 2.589 (1.88), 2.602 (1.76), 2.653 (1.74), 2.672 (1.77), 2.939 (10.25), 4.357 (1.92), 4.373 (2.32), 4.388 (1.85), 6.900 (7.38), 6.923 (7.55), 7.317 (2.97), 7.322 (3.10), 7.343 (1.66), 7.546 (1.71), 7.553 (1.80), 7.572 (2.88), 7.594 (1.79), 7.601 (1.69), 7.791 (2.15), 8.252 (8.40), 8.275 (8.03), 8.534 (16.00), 10.126 (4.93), 10.147 (4.79). |

(fortgesetzt)

| Bsp. | | Analytische Daten |
|---|---|---|
| **47** | 1-(2,4-Difluorphenyl)-7-(dimethylamino)-*N*-(4-fluorbicyclo[2.2.2]oct-1-yl)-4-oxo-1,4-dihydro-1,8-naphthyridin-3-carbonsäureamid 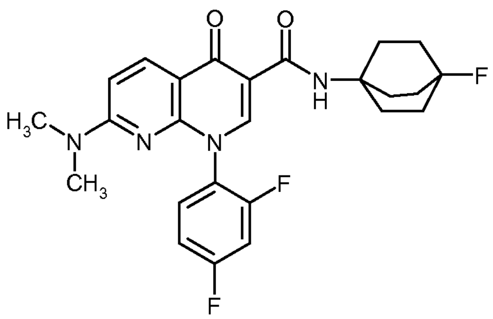 (85 % d. Th.) | LC-MS (Methode 1): $R_t$ = 1.19 min MS (ESpos): m/z = 471.3 $[M+H]^+$ $^1$H-NMR (400 MHz, $CDCl_3$) $\delta$ [ppm]: 0.001 (0.16), 0.017 (0.15), 0.078 (0.09), 1.576 (16.00), 1.935 (0.16), 1.950 (0.30), 1.965 (0.31), 1.976 (0.33), 1.990 (0.21), 2.235 (0.37), 2.247 (0.28), 2.257 (0.35), 2.275 (0.28), 2.634 (0.10), 2.966 (0.08), 2.988 (0.82), 6.651 (0.35), 6.674 (0.36), 7.006 (0.15), 7.022 (0.15), 7.027 (0.14), 7.041 (0.15), 7.046 (0.12), 7.051 (0.09), 7.059 (0.07), 7.343 (0.07), 7.357 (0.08), 7.364 (0.08), 7.377 (0.09), 8.392 (0.37), 8.415 (0.36), 8.607 (0.58), 10.007 (0.15). |
| **48** | *rac*-*N*-[1-(2-Chlorphenyl)-2,2,2-trifluorethyl]-1-(2,4-difluorphenyl)-7-(dimethylamino)-4-oxo-1,4-dihydro-1,8-naphthyridin-3 -carbonsäureamid 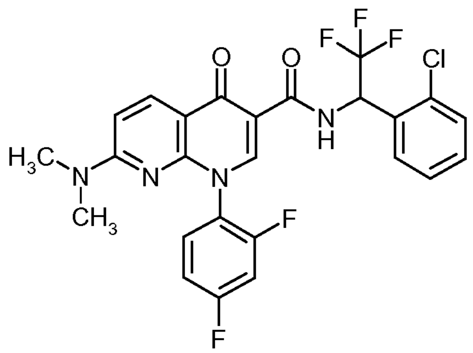 (74 % d. Th.) | LC-MS (Methode 1): $R_t$ = 1.19 min MS (ESpos): m/z = 471.3 $[M+H]^+$ $^1$H-NMR (400 MHz, DMSO-$d_6$) $\delta$ [ppm]: -0.013 (6.67), 0.003 (6.02), 2.322 (0.99), 2.361 (1.40), 2.664 (1.24), 2.705 (1.56), 2.944 (4.69), 6.425 (1.75), 6.446 (2.37), 6.466 (1.61), 6.943 (8.07), 6.966 (8.08), 7.306 (1.62), 7.325 (1.64), 7.471 (1.19), 7.475 (1.31), 7.490 (3.28), 7.494 (3.47), 7.508 (3.26), 7.513 (3.29), 7.525 (2.21), 7.544 (4.34), 7.561 (3.07), 7.594 (7.00), 7.598 (7.38), 7.613 (5.54), 7.617 (4.99), 7.738 (0.97), 7.753 (0.91), 7.812 (0.95), 7.827 (0.96), 8.312 (9.17), 8.335 (8.61), 8.620 (16.00), 11.614 (2.88), 11.637 (2.62). |
| **49** | *N*-(2,6-Dichlorbenzyl)-1-(2,4-difluorphenyl)-7-(dimethylamino)-4-oxo-1,4-dihydro-1,8-naphthyridin-3-carbonsäureamid 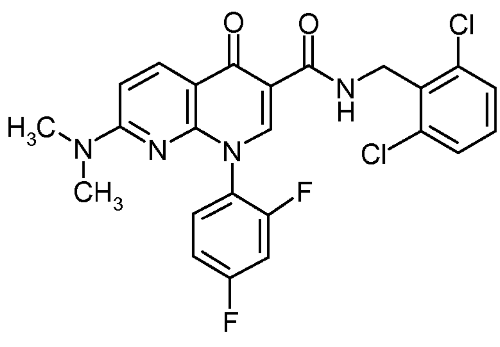 (73 % d.Th.) | LC-MS (Methode 1): $R_t$ = 1.22 min MS (ESpos): m/z = 503.0 $[M+H]^+$ $^1$H-NMR (400 MHz, DMSO-$d_6$) $\delta$ [ppm]: 2.726 (3.73), 2.886 (6.85), 2.927 (11.71), 4.786 (4.36), 4.800 (4.64), 4.812 (4.61), 4.825 (4.28), 6.879 (8.07), 6.902 (8.27), 7.315 (3.57), 7.374 (3.40), 7.395 (6.06), 7.414 (5.77), 7.520 (16.00), 7.540 (12.58), 7.565 (3.42), 7.773 (3.72), 7.788 (3.61), 8.217 (8.38), 8.240 (8.00), 8.565 (14.86), 10.336 (2.96), 10.350 (5.68), 10.362 (2.79). |

(fortgesetzt)

| Bsp. | | Analytische Daten |
|---|---|---|
| **50** | *N*-(2,4-Difluorobenzyl)-1-(2,4-difluorphenyl)-7-(dimethylamino)-4-oxo-1,4-dihydro-1,8-naphthyridin-3-carbonsäureamid 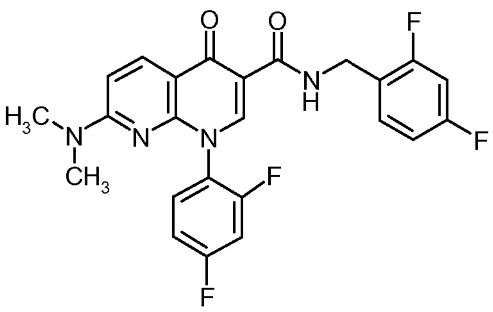 (78 % d. Th.) | LC-MS (Methode 1): $R_t$ = 1.15 min MS (ESpos): m/z = 471.1 $[M+H]^+$ $^1$H-NMR (400 MHz, DMSO-$d_6$) $\delta$ [ppm]: -0.009 (7.30), 0.007 (6.62), 2.939 (8.43), 4.558 (4.82), 4.567 (4.86), 6.898 (7.29), 6.921 (7.44), 7.048 (1.32), 7.054 (1.43), 7.070 (2.82), 7.075 (2.95), 7.091 (1.54), 7.097 (1.58), 7.218 (1.78), 7.225 (1.74), 7.243 (2.60), 7.248 (2.50), 7.268 (1.90), 7.274 (1.76), 7.301 (1.33), 7.315 (2.57), 7.321 (2.64), 7.336 (1.40), 7.341 (1.43), 7.413 (1.64), 7.435 (3.27), 7.451 (3.31), 7.473 (1.48), 7.545 (1.64), 7.552 (1.70), 7.575 (2.53), 7.594 (1.75), 7.601 (1.63), 7.756 (1.66), 7.771 (1.95), 7.778 (3.29), 7.793 (3.27), 7.799 (1.86), 7.814 (1.57), 8.255 (8.00), 8.278 (7.61), 8.555 (16.00), 10.346 (2.21), 10.361 (4.55), 10.376 (2.15). |
| **51** | 1-(2,4-Difluorphenyl)-7-(dimethylamino)-*N*-(2,6-dimethylbenzyl)-4-oxo-1,4-dihydro-1,8-naphthyridin-3-carbonsäureamid 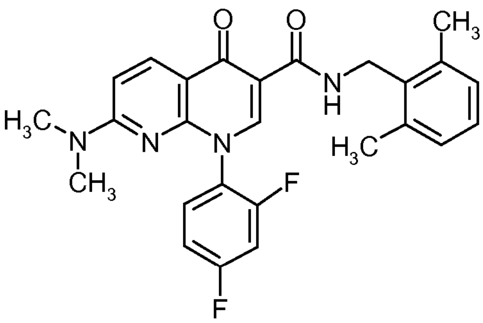 (41 % d. Th.) | LC-MS (Methode 1): $R_t$ = 1.22 min MS (ESpos): m/z = 463.2 $[M+H]^+$ $^1$H-NMR (400 MHz, DMSO-$d_6$) $\delta$ [ppm]: -0.013 (1.23), 0.003 (1.03), 2.068 (0.40), 2.393 (16.00), 2.922 (1.87), 4.526 (0.91), 4.540 (1.40), 4.554 (0.82), 6.869 (1.65), 6.892 (1.65), 7.043 (0.92), 7.060 (2.86), 7.084 (1.56), 7.099 (0.78), 7.106 (0.56), 7.121 (0.34), 7.294 (0.32), 7.311 (0.56), 7.337 (0.29), 7.540 (0.39), 7.547 (0.39), 7.566 (0.55), 7.588 (0.37), 7.595 (0.35), 7.750 (0.38), 7.771 (0.70), 7.786 (0.69), 7.808 (0.32), 8.198 (1.92), 8.221 (1.79), 8.564 (3.61), 10.077 (0.52), 10.090 (0.94), 10.102 (0.43). |
| **52** | 1-(2,4-Difluorphenyl)-*N*-(2,6-difluorphenyl)-7-(dimethylamino)-4-oxo-1,4-dihydro-1,8-naphthyridin-3-carbonsäureamid 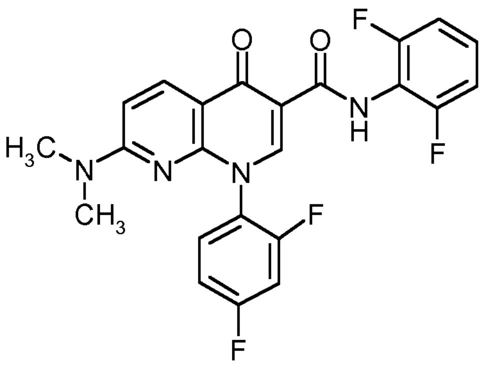 (62 % d. Th.) | LC-MS (Methode 1): $R_t$ = 1.13 min MS (ESpos): m/z = 457.2 $[M+H]^+$ $^1$H-NMR (400 MHz, DMSO-$d_6$) $\delta$ [ppm]: -0.009 (2.92), 0.007 (2.64), 2.962 (5.97), 6.953 (7.45), 6.976 (7.64), 7.188 (4.26), 7.209 (9.81), 7.229 (6.29), 7.328 (2.60), 7.336 (3.06), 7.354 (3.59), 7.374 (3.14), 7.395 (1.60), 7.558 (1.60), 7.565 (1.68), 7.584 (2.45), 7.588 (2.46), 7.607 (1.70), 7.614 (1.64), 7.808 (1.66), 7.823 (1.94), 7.830 (3.26), 7.845 (3.24), 7.852 (1.81), 7.867 (1.58), 8.327 (8.17), 8.350 (7.85), 8.690 (16.00), 11.813 (9.92). |

(fortgesetzt)

| Bsp. | | Analytische Daten |
|---|---|---|
| **53** | 1-(2,4-Difluorphenyl)-*N*-[2-(2,6-difluorphenyl) propan-2-yl]-7-(dimethylamino)-4-oxo-1,4-dihydro-1,8-naphthyridin-3-carbonsäureamid 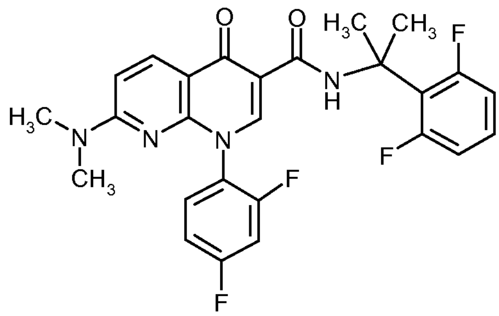 (100 % d. Th.) | LC-MS (Methode 1): $R_t$ = 1.22 min MS (ESpos): m/z = 499.2 $[M+H]^+$ $^1$H-NMR (400 MHz, DMSO-$d_6$) δ [ppm]: -0.013 (8.59), 0.003 (7.77), 1.819 (16.00), 2.934 (4.18), 6.906 (4.62), 6.929 (6.51), 6.953 (3.18), 6.977 (2.76), 7.257 (2.44), 7.278 (2.11), 7.539 (1.48), 7.755 (1.87), 7.770 (1.85), 8.276 (5.32), 8.299 (4.95), 8.402 (10.08), 10.676 (4.51). |
| **54** | 1-(2,4-Difluorphenyl)-7-(dimethylamino)-*N*-(2,6-dimethylphenyl)-4-oxo-1,4-dihydro-1,8-naphthyridin-3-carbonsäureamid 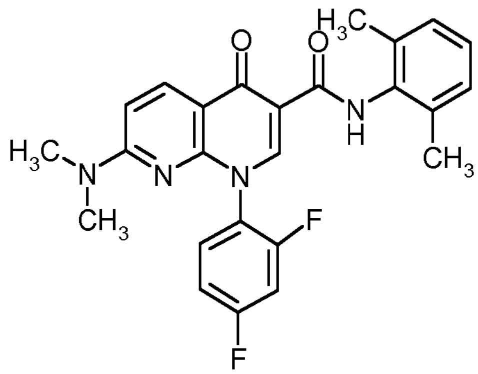 (72 % d. Th.) | LC-MS (Methode 1): $R_t$ = 1.20 min MS (ESpos): m/z = 449.2 $[M+H]^+$ $^1$H-NMR (400 MHz, DMSO-$d_6$) δ [ppm]: -0.001 (3.96), 2.211 (16.00), 2.889 (0.60), 2.962 (1.78), 6.939 (1.47), 6.962 (1.47), 7.114 (6.25), 7.321 (0.66), 7.326 (0.65), 7.580 (0.61), 7.832 (0.70), 7.847 (0.69), 8.339 (1.66), 8.362 (1.57), 8.657 (3.43), 11.593 (1.96). |

**Beispiel 55**

*N*-(2,6-Dichlorphenyl)-1-(2,4-difluorphenyl)-7-(dimethylamino)-4-oxo-1,4-dihydro-1,8-naphthyridin-3-carbonsäureamid

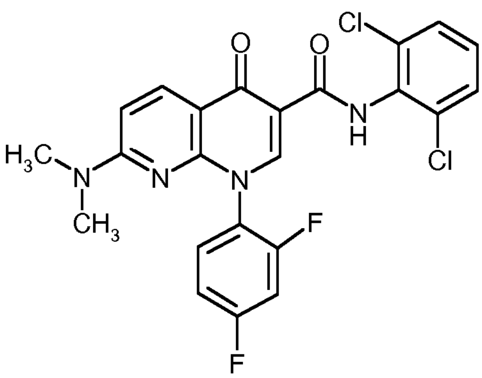

100 mg (0.29 mmol) der Verbindung aus Beispiel 36A wurden in 3 ml DMF vorgelegt, mit 132 mg (0.35 mmol) HATU sowie 119 mg (0.93 mmol) DIPEA versetzt und 30 Minuten lang bei 20°C gerührt. Dann wurde eine Mischung

aus 66 mg (0.4 mmol) 2,6-Dichloranilin und 29 mg (0.72 mmol) NaH (60 proz. in Paraffin) zugegeben und 18 Stunden bei 23°C gerührt. Anschliessend wurde das Gemisch über präparative RP-HPLC gereinigt (Eluent: Acetonitril/Wasser-Gradient mit 0.1% Ameisensäure). Man erhielt 27 mg (19 % d. Th.) der Titelverbindung.
LC-MS (Methode 1): $R_t$ = 1.38 min; m/z = 523.3 $[M+H]^+$.
$^1$H-NMR (400 MHz, DMSO-$d_6$) δ [ppm]: -0.009 (1.63), 0.007 (1.55), 2.689 (15.32), 2.730 (12.82), 2.889 (16.00), 2.965 (4.17), 6.953 (6.16), 6.976 (6.30), 7.328 (2.09), 7.352 (3.89), 7.372 (5.08), 7.392 (4.16), 7.563 (1.58), 7.575 (15.18), 7.586 (2.51), 7.595 (12.02), 7.605 (1.69), 7.837 (1.53), 7.844 (2.57), 7.859 (2.51), 7.951 (1.95), 8.332 (6.93), 8.355 (6.69), 8.685 (13.85), 12.019 (7.76).

## Beispiel 56

*rac-N*-[1-(2,6-Dichlorphenyl)-2,2,2-trifluorethyl]-1-(2,4-Difluorphenyl)-7-(dimethylamino)-4-oxo-1,4-dihydro-1,8-naphthyridin-3-carbonsäureamid

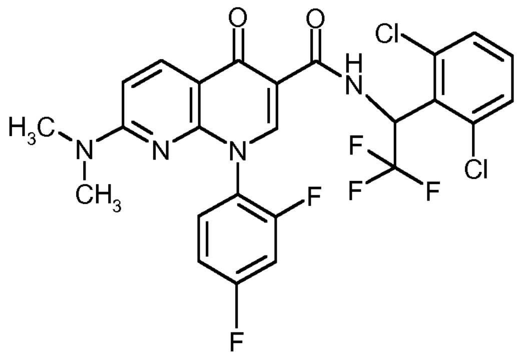

200 mg (0.58 mmol) der Verbindung aus Beispiel 36A und 283 mg (1.2 mmol) 1-(2,6-Dichlorphenyl)-2,2,2-trifluorethanamin wurden in 4 ml DMF vorgelegt, mit 210 mg (1.6 mmol) DIPEA und 422 mg (0.81 mmol) PyBOP versetzt und 40 Minuten lang bei 23°C gerührt. Die Reaktionsmischung wurde dann mit 1 M wässr. Salzsäure auf pH 1 eingestellt, der ausgefallene Feststoff anschließend abgesaugt und mit Wasser und Petrolether gewaschen. Man erhielt 300 mg (86 % d. Th., enth. 0.25 Äq. DMF) der Titelverbindung.
LC-MS (Methode 1): $R_t$ = 1.36 min; m/z = 571.0 $[M+H]^+$.
$^1$H-NMR (400 MHz, DMSO-$d_6$) δ [ppm]: -0.001 (16.00), 0.006 (0.72), 1.242 (0.19), 1.258 (0.19), 1.713 (0.27), 1.722 (0.31), 1.730 (0.67), 1.737 (0.27), 1.746 (0.25), 2.731 (2.00), 2.890 (2.47), 2.946 (0.87), 2.991 (0.45), 3.001 (0.49), 3.007 (0.62), 3.017 (0.60), 3.024 (0.38), 3.034 (0.25), 6.922 (0.96), 6.945 (0.97), 7.028 (0.15), 7.052 (0.20), 7.067 (0.21), 7.310 (0.24), 7.329 (0.27), 7.423 (0.17), 7.443 (0.14), 7.489 (0.45), 7.509 (1.14), 7.529 (0.87), 7.564 (0.36), 7.591 (0.82), 7.611 (0.50), 7.645 (0.69), 7.665 (0.52), 7.735 (0.15), 7.754 (0.15), 7.815 (0.15), 7.830 (0.15), 7.951 (0.33), 8.315 (1.18), 8.338 (1.08), 8.621 (0.72), 11.803 (0.29), 11.822 (0.27).

In Analogie zu Beispiel 56 wurden die in Tabelle 6 gezeigten Beispielverbindungen hergestellt, indem die Verbindung aus Beispiel 36A mit den entsprechenden Aminen (bzw. deren Salzen) unter den beschriebenen Reaktionsbedingungen umgesetzt wurden. Abweichungen sind bei den jeweiligen Beispielen angegeben.

Tabelle 6:

| Bsp. | | Analytische Daten |
|---|---|---|
| **57** | 1-(2,4-Difluorphenyl)-7-(dimethylamino)-4-oxo-*N*-[2-(trifluormethyl)phenyl]-1,4-dihydro-1,8-naphthyridin-3-carbonsäureamid 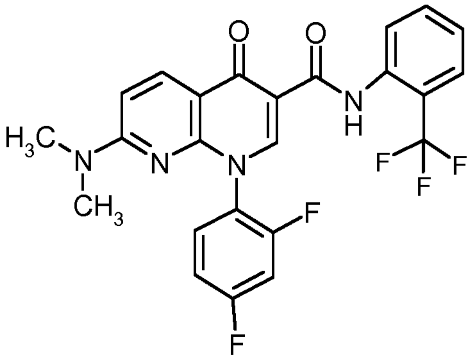 3 d bei 23°C; Aufreinigung durch präparative HPLC (Eluent: Acetonitril/Wasser-Gradient mit 0.1% Ameisensäure). (27 % d. Th.) | LC-MS (Methode 1): $R_t$ = 1.29 min; m/z = 469.3 $[M+H]^+$. $^1$H-NMR (400 MHz, DMSO-$d_6$) $\delta$ [ppm]: - 0.009 (0.87), -0.001 (16.00), 0.005 (0.30), 0.007 (0.48), 2.958 (0.27), 6.938 (0.37), 6.961 (0.36), 7.322 (0.17), 7.343 (0.31), 7.361 (0.18), 7.594 (0.12), 7.673 (0.10), 7.693 (0.17), 7.712 (0.10), 7.746 (0.20), 7.765 (0.18), 7.824 (0.10), 7.830 (0.16), 7.845 (0.16), 8.311 (0.22), 8.331 (0.21), 8.347 (0.44), 8.369 (0.41), 8.724 (0.83), 12.669 (0.40). |
| **58** | *N*-[2-Chlor-6-(trifluormethyl)benzyl]-1-(2,4-difluorphenyl)-7-(dimethylamino)-4-oxo-1,4-dihydro-1,8-naphthyridin-3 -carbonsäureamid 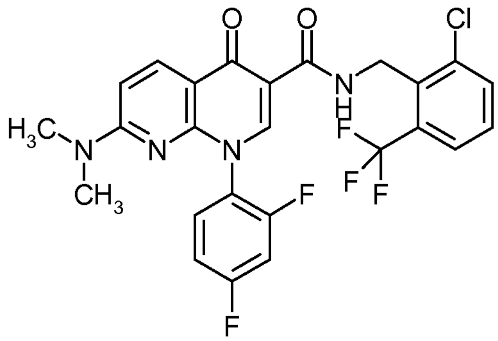 (86 % d. Th.) | LC-MS (Methode 1): $R_t$ = 1.23 min; m/z = 537.3 $[M+H]^+$. $^1$H-NMR (400 MHz, DMSO-$d_6$) $\delta$ [ppm]: 1.244 (3.96), 1.259 (5.15), 1.275 (2.54), 2.889 (3.79), 2.928 (9.26), 4.791 (6.78), 6.873 (6.91), 6.896 (7.09), 7.318 (2.92), 7.323 (3.02), 7.572 (2.88), 7.619 (2.39), 7.639 (5.38), 7.659 (3.27), 7.790 (3.43), 7.805 (3.57), 7.818 (6.03), 7.838 (4.78), 7.905 (5.21), 7.925 (4.46), 8.188 (7.85), 8.211 (7.45), 8.588 (16.00), 10.251 (2.62), 10.264 (5.29), 10.276 (2.47). |
| **59** | 1-(2,4-Difluorphenyl)-7-(dimethylamino)-4-oxo-*N*-[2-(trifluormethyl)benzyl]-1,4-dihydro-1,8-naphthyridin-3-carbonsäureamid 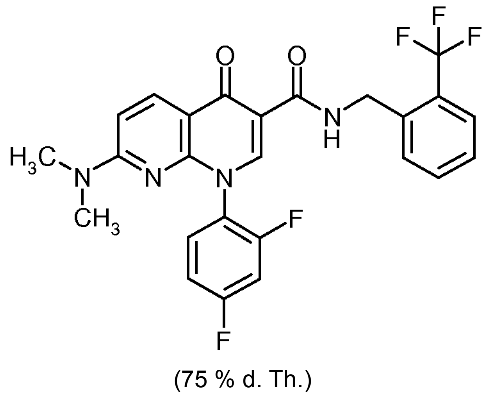 (75 % d. Th.) | LC-MS (Methode 1): $R_t$ = 1.21 min; m/z = 503.4 $[M+H]^+$. $^1$H-NMR (400 MHz, DMSO-$d_6$) $\delta$ [ppm]: - 0.010 (2.01), 0.007 (1.99), 2.940 (7.80), 4.728 (5.35), 4.743 (5.41), 6.899 (7.30), 6.922 (7.44), 7.317 (2.38), 7.322 (2.51), 7.484 (1.65), 7.503 (3.70), 7.522 (2.30), 7.548 (1.63), 7.555 (1.70), 7.573 (2.43), 7.577 (2.54), 7.580 (2.51), 7.588 (3.28), 7.596 (2.10), 7.607 (5.24), 7.657 (2.95), 7.676 (3.89), 7.741 (4.52), 7.761 (4.24), 7.779 (1.88), 7.786 (3.19), 7.801 (3.15), 7.808 (1.76), 8.263 (8.28), 8.286 (7.89), 8.573 (16.00), 10.449 (2.12), 10.464 (4.38), 10.479 (2.00). |

(fortgesetzt)

| Bsp. | | Analytische Daten |
|---|---|---|
| 60 | 1-(2,4-Difluorphenyl)-7-(dimethylamino)-*N*-[2-fluoro-6-(trifluormethyl)benzyl]-4-oxo-1,4-dihydro-1,8-naphthyridin-3-carbonsäureamid 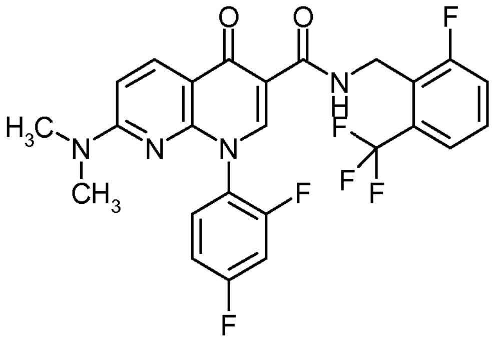 (81 % d. Th.) | LC-MS (Methode 1): $R_t$ = 1.22 min; m/z = 521.3 $[M+H]^+$. $^1$H-NMR (400 MHz, DMSO-$d_6$) $\delta$ [ppm]: 0.007 (3.36), 2.366 (2.57), 2.709 (2.59), 2.730 (0.61), 2.929 (6.92), 4.744 (3.85), 6.878 (6.28), 6.901 (6.36), 7.298 (1.15), 7.320 (2.22), 7.341 (1.24), 7.543 (1.40), 7.550 (1.41), 7.573 (2.16), 7.592 (1.56), 7.599 (1.38), 7.628 (2.72), 7.641 (5.46), 7.657 (16.00), 7.758 (1.32), 7.773 (1.58), 7.779 (2.63), 7.794 (2.62), 7.801 (1.49), 7.816 (1.25), 8.209 (6.65), 8.232 (6.36), 8.571 (11.91), 10.338 (1.95), 10.351 (3.90), 10.364 (1.83). |

**Beispiel 61**

1-(2,4-Difluorphenyl)-7-[(3*S*)-3-fluorpyrrolidin-1-yl]-4-oxo-*N*-(tricyclo[3.3.1.1$^{3,7}$]dec-1-yl)-1,4-dihydro-1,8-naphthyridin-3-carbonsäureamid

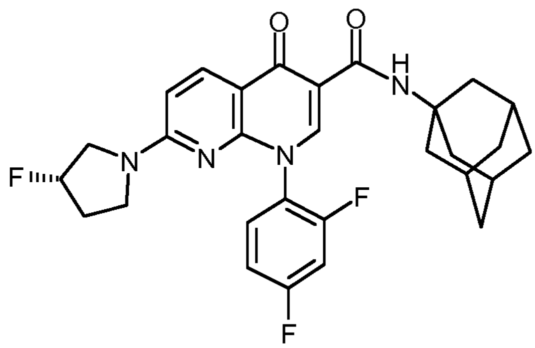

100 mg (0.26 mmol) der Verbindung aus Beispiel 45A wurden in 2.9 ml DMF vorgelegt, mit 117 mg (0.31 mmol) HATU sowie 106 mg (0.82 mmol) DIPEA versetzt und das Gemisch für 30 Minuten lang bei 20°C gerührt. Dann wurden 54 mg (0.36 mmol) 1-Adamantanamin zugegeben und das Gemisch anschließend für 2 Stunden bei 20°C gerührt. Anschliessend wurde das Gemisch über präparative HPLC gereinigt (Eluent: Acetonitril/Wasser-Gradient mit 0.1% Ameisensäure). Man erhielt 103 mg (77 % d. Th.) der Titelverbindung.
LC-MS (Methode 1): $R_t$ = 1.38 min; m/z = 523.3 $[M+H]^+$.
$^1$H-NMR (400 MHz, DMSO-$d_6$) $\delta$ [ppm]: -0.008 (1.49), 0.008 (1.35), 1.157 (2.39), 1.175 (4.83), 1.193 (2.46), 1.356 (0.71), 1.674 (7.25), 1.988 (8.83), 2.058 (16.00), 4.003 (0.70), 4.021 (2.07), 4.038 (2.06), 4.056 (0.68), 6.763 (0.54), 6.785 (0.56), 7.323 (0.65), 7.782 (0.40), 7.790 (0.66), 7.805 (0.66), 8.138 (1.19), 8.292 (1.32), 8.314 (1.29), 8.491 (4.34), 9.938 (2.58).

In Analogie zu Beispiel 61 wurden die in Tabelle 7 gezeigten Beispielverbindungen hergestellt, indem die Verbindung aus Beispiel 45A mit den entsprechenden Aminen (bzw. deren Salzen) unter den beschriebenen Reaktionsbedingungen umgesetzt wurden. Abweichungen sind bei den jeweiligen Beispielen angegeben.

Tabelle 7:

| Bsp. | | Analytische Daten |
|---|---|---|
| **62** | 1-(2,4-Difluorphenyl)-7-[(3*S*)-3-fluor-pyrrolidin-1-yl] -*N*-(3 -fluortricyclo-[3.3.1.1$^{3,7}$]dec-1-yl)-4-oxo-1,4-dihydro-1,8-naphthyridin-3 -carbonsäureamid 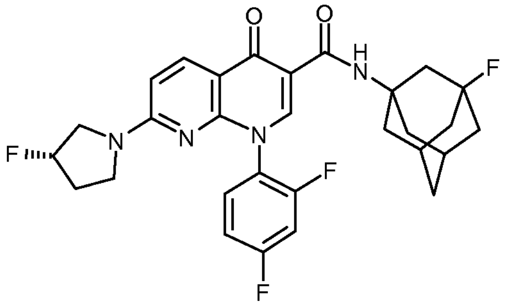 (87 % d. Th.) | LC-MS (Methode 1): $R_t$ = 1.24 min MS (ESpos): m/z = 541.3 $[M+H]^+$ $^1$H-NMR (400 MHz, $CDCl_3$) $\delta$ [ppm]: 0.000 (1.22), 0.016 (1.21), 0.077 (0.68), 1.231 (0.26), 1.296 (1.42), 1.309 (1.45), 1.441 (0.77), 1.592 (16.00), 1.733 (0.25), 1.754 (0.25), 1.889 (1.68), 1.950 (1.39), 2.053 (1.22), 2.083 (2.24), 2.138 (1.82), 2.169 (0.98), 2.324 (0.52), 2.378 (3.30), 2.449 (0.56), 2.794 (0.45), 2.812 (0.99), 2.892 (3.80), 2.965 (4.40), 3.488 (0.35), 3.601 (0.79), 5.242 (0.24), 5.370 (0.24), 6.540 (0.62), 6.561 (0.61), 7.006 (0.79), 7.026 (1.24), 7.046 (1.69), 7.065 (0.86), 7.348 (0.51), 7.368 (0.89), 7.383 (0.88), 7.404 (0.39), 7.528 (0.44), 8.025 (0.47), 8.442 (3.20), 8.464 (3.08), 8.639 (5.60), 10.109 (1.88). |
| **63** | 1-(2,4-Difluorphenyl)-*N*-(4-fluor-bicyclo[2.2.2]oct-1-yl)-7-[(3*S*)-3-fluor-pyrrolidin-1-yl]-4-oxo-1,4-dihydro-1,8-naphthyridin-3 -carbonsäureamid 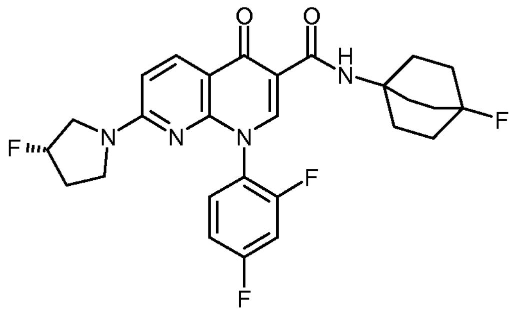 (100 % d. Th.) | LC-MS (Methode 1): $R_t$ = 1.18 min MS (ESpos): m/z = 515.3 $[M+H]^+$ $^1$H-NMR (400 MHz, $CDCl_3$) $\delta$ [ppm]: -0.140 (0.04), 0.001 (0.37), 0.017 (0.29), 0.078 (0.19), 0.155 (0.04), 1.421 (0.08), 1.441 (0.12), 1.576 (16.00), 1.937 (0.28), 1.951 (0.53), 1.967 (0.53), 1.977 (0.58), 1.992 (0.37), 2.132 (0.03), 2.236 (0.64), 2.258 (0.61), 2.276 (0.48), 2.373 (0.04), 2.449 (0.13), 2.794 (0.12), 2.812 (0.13), 2.893 (0.28), 2.965 (0.35), 3.177 (0.03), 3.594 (0.10), 5.243 (0.03), 5.377 (0.03), 6.536 (0.08), 6.556 (0.08), 7.006 (0.17), 7.027 (0.16), 7.046 (0.23), 7.065 (0.11), 7.340 (0.07), 7.361 (0.12), 7.375 (0.12), 7.395 (0.06), 7.529 (0.12), 8.027 (0.04), 8.429 (0.48), 8.451 (0.47), 8.620 (0.81), 9.983 (0.29). |

(fortgesetzt)

| Bsp. | | Analytische Daten |
|---|---|---|
| **64** | 1-(2,4-Difluorphenyl)-7-[(3*S*)-3-fluor-pyrrolidin-1-yl]-4-oxo-*N*-[(2*S*)-1,1,1-trifluorbutan-2-yl]-1,4-dihydro-1,8-naphthyridin-3-carbonsäureamid 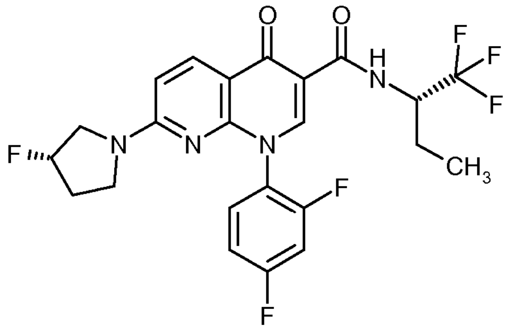 Aufarbeitung: Ausfällen des Produkts mit 1 M wässr. Salzsäure und Wasser und anschließendes Abfiltrieren des Niederschlags. (86 % d. Th.) | LC-MS (Methode 2): $R_t$ = 2.82 min MS (ESpos): m/z = 499.0 $[M+H]^+$ $^1$H-NMR (400 MHz, DMSO-$d_6$) δ [ppm]: - 0.150 (0.56), -0.048 (0.52), -0.042 (0.74), -0.038 (0.83), -0.035 (0.90), -0.033 (0.93), -0.031 (1.01), -0.028 (1.10), -0.026 (1.26), -0.023 (1.40), -0.021 (1.53), -0.018 (1.77), -0.016 (2.10), -0.014 (2.33), -0.012 (3.16), -0.009 (6.86), -0.007 (6.14), -0.006 (7.26), 0.004 (4.31), 0.006 (3.08), 0.007 (4.66), 0.010 (1.10), 0.013 (0.63), 0.015 (0.47), 0.018 (0.40), 0.145 (0.56), 0.950 (7.53), 0.968 (16.00), 0.987 (7.73), 1.146 (0.34), 1.156 (0.33), 1.169 (0.46), 1.174 (0.59), 1.581 (0.39), 1.599 (1.18), 1.617 (1.60), 1.624 (1.41), 1.634 (1.83), 1.643 (1.66), 1.652 (1.56), 1.660 (1.77), 1.678 (1.30), 1.697 (0.38), 1.833 (0.51), 1.852 (1.38), 1.861 (1.57), 1.870 (1.57), 1.880 (1.73), 1.886 (1.53), 1.896 (1.36), 1.904 (1.17), 1.914 (1.01), 1.987 (0.93), 2.166 (0.83), 2.327 (0.76), 2.332 (0.60), 2.366 (0.84), 2.519 (3.20), 2.521 (3.13), 2.523 (3.42), 2.526 (3.67), 2.558 (0.99), 2.560 (0.80), 2.563 (0.66), 2.565 (0.58), 2.568 (0.53), 2.570 (0.50), 2.573 (0.42), 2.575 (0.33), 2.578 (0.33), 2.587 (0.33), 2.665 (0.55), 2.669 (0.69), 2.674 (0.52), 2.709 (0.89), 3.132 (0.37), 3.496 (0.81), 3.690 (0.92), 4.735 (1.51), 4.747 (1.40), 4.771 (0.78), 5.278 (0.44), 5.402 (0.55), 5.510 (0.34), 5.753 (4.85), 6.807 (1.77), 7.324 (2.28), 7.546 (0.78), 7.569 (1.54), 7.587 (1.29), 7.812 (1.76), 7.831 (1.67), 8.136 (0.79), 8.316 (4.26), 8.338 (4.02), 8.631 (10.61), 10.479 (4.79), 10.503 (4.55). |
| **65** | 1-(2,4-Difluorphenyl)-7-[(3*S*)-3-fluor-pyrrolidin-1-yl]-4-oxo-*N*-[1,1,1-trifluor-4-methylpentan-2-yl]-1,4-dihydro-1,8-naphthyridin-3 -carbonsäureamid (Diastereomerengemisch ) 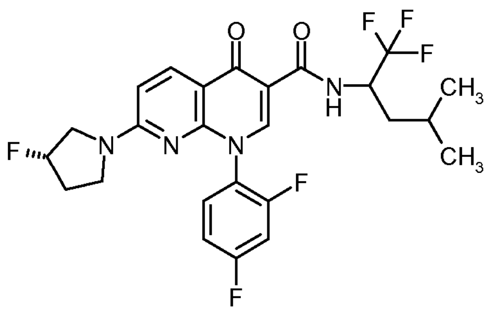 (92 % d. Th.) | LC-MS (Methode 1): $R_t$ = 1.29 min MS (ESpos): m/z = 527.3 $[M+H]^+$ $^1$H-NMR (400 MHz, $CDCl_3$) δ [ppm]: 0.001 (0.58), 0.017 (0.41), 0.078 (0.30), 0.959 (1.73), 0.975 (1.96), 0.984 (3.35), 1.001 (3.17), 1.232 (0.09), 1.379 (0.07), 1.396 (0.06), 1.442 (0.38), 1.459 (0.11), 1.492 (0.12), 1.508 (0.13), 1.518 (0.12), 1.576 (16.00), 1.602 (0.52), 1.611 (0.65), 1.619 (0.37), 1.636 (0.42), 1.645 (0.30), 1.714 (0.32), 1.724 (0.41), 1.742 (0.32), 1.752 (0.52), 1.758 (0.35), 1.768 (0.16), 1.778 (0.40), 1.786 (0.49), 1.795 (0.26), 1.802 (0.22), 1.811 (0.26), 1.821 (0.16), 1.826 (0.16), 1.837 (0.09), 2.069 (0.08), 2.153 (0.08), 2.280 (0.06), 2.384 (0.11), 2.450 (0.21), 2.634 (0.36), 2.795 (0.16), 2.813 (0.23), 2.893 (0.24), 2.966 (0.30), 3.612 (0.30), 4.877 (0.15), 4.895 (0.24), 4.920 (0.24), 4.929 (0.16), 4.939 (0.13), 4.947 (0.10), 5.245 (0.08), 5.379 (0.09), 6.560 (0.22), 7.006 (0.26), 7.040 (0.52), 7.060 (0.68), 7.078 (0.35), 7.280 (0.28), 7.364 (0.21), 7.385 (0.36), 7.399 (0.36), 7.421 (0.17), 7.529 (0.22), 8.450 (1.52), 8.472 (1.49), 8.697 (1.99), 10.362 (0.46), 10.386 (0.46). |

(fortgesetzt)

| Bsp. | | Analytische Daten |
|---|---|---|
| **66** | 1-(2,4-Difluorphenyl)-7-[(3S)-3-fluor-pyrrolidin-1-yl]-4-oxo-*N*-[1,1,1-trifluor-pentan-2-yl]-1,4-dihydro-1,8-naphthyridin-3-carbonsäureamid (Diastereomerengemisch) 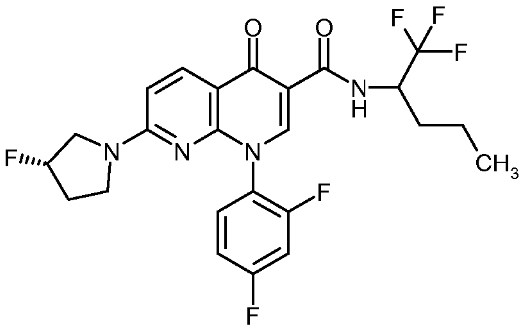 Aufarbeitung: Ausfällen des Produkts mit 1 M wässr. Salzsäure und Wasser und anschließendes Abfiltrieren des Niederschlags. (90 % d. Th.) | LC-MS (Methode 2): $R_t$ = 2.95 min MS (ESpos): m/z = 513.1 $[M+H]^+$ $^1H$-NMR (400 MHz, DMSO-$d_6$) δ [ppm]: - 0.151 (0.40), -0.009 (3.50), 0.007 (3.30), 0.144 (0.41), 0.866 (0.27), 0.894 (7.06), 0.912 (16.00), 0.930 (8.28), 0.998 (0.63), 1.067 (0.57), 1.146 (0.48), 1.168 (0.59), 1.231 (0.25), 1.280 (0.26), 1.299 (0.59), 1.317 (0.99), 1.335 (1.38), 1.355 (2.83), 1.373 (1.58), 1.391 (1.13), 1.410 (0.91), 1.433 (1.07), 1.444 (1.40), 1.464 (1.26), 1.480 (0.73), 1.497 (0.45), 1.594 (0.59), 1.606 (0.60), 1.621 (0.88), 1.629 (1.55), 1.641 (1.14), 1.655 (1.61), 1.667 (1.09), 1.678 (0.85), 1.690 (0.65), 1.739 (0.85), 1.748 (1.00), 1.765 (1.39), 1.772 (1.51), 1.781 (1.19), 1.790 (1.30), 1.796 (0.94), 1.815 (0.53), 1.823 (0.45), 2.182 (0.85), 2.225 (0.67), 2.322 (0.46), 2.326 (0.53), 2.331 (0.40), 2.365 (0.64), 2.522 (1.03), 2.664 (0.37), 2.669 (0.45), 2.689 (0.62), 2.709 (0.66), 2.730 (0.62), 2.889 (0.78), 3.090 (0.45), 3.136 (0.33), 3.486 (0.70), 3.697 (0.77), 4.773 (0.73), 4.797 (1.25), 4.816 (1.27), 4.835 (0.70), 5.283 (0.36), 5.409 (0.46), 5.512 (0.26), 6.804 (1.49), 6.821 (1.46), 7.302 (1.11), 7.324 (2.12), 7.342 (1.19), 7.546 (0.65), 7.566 (1.37), 7.588 (1.15), 7.792 (0.91), 7.813 (1.86), 7.829 (1.85), 7.850 (0.81), 8.311 (3.82), 8.334 (3.68), 8.629 (9.55), 10.474 (4.44), 10.498 (4.28). |
| **67** | 1-(2,4-Difluorphenyl)-7-[(3*S*)-3-fluorpyrrolidin-1-yl]-4-oxo-*N*-(2,2,2-trifluorethyl)-1,4-dihydro-1,8-naphthyridin-3-carbonsäureamid 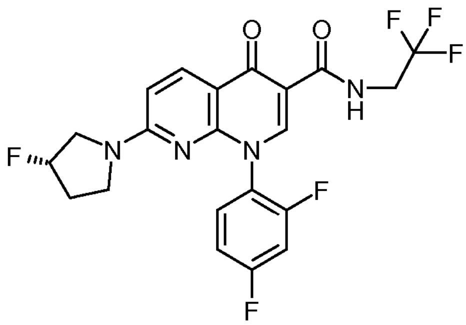 Aufarbeitung: Ausfällen des Produkts mit 1 M wässr. Salzsäure und Wasser und anschließendes Abfiltrieren des Niederschlags. (91 % d. Th.) | LC-MS (Methode 1): $R_t$ = 1.07 min MS (ESpos): m/z = 471.2 $[M+H]^+$ $^1H$-NMR (400 MHz, $CDCl_3$) δ [ppm]: 0.001 (0.23), 0.017 (0.25), 0.079 (0.15), 1.442 (0.09), 1.571 (16.00), 2.156 (0.04), 2.390 (0.06), 2.450 (0.09), 2.794 (0.06), 2.894 (0.08), 2.966 (0.09), 3.608 (0.16), 4.098 (0.11), 4.118 (0.14), 4.137 (0.17), 4.158 (0.14), 4.177 (0.10), 5.243 (0.05), 5.375 (0.05), 6.575 (0.12), 7.006 (0.11), 7.047 (0.27), 7.066 (0.35), 7.086 (0.18), 7.365 (0.11), 7.386 (0.19), 7.400 (0.19), 7.421 (0.08), 7.529 (0.11), 8.460 (0.82), 8.470 (0.06), 8.482 (0.80), 8.492 (0.04), 8.694 (1.41), 8.705 (0.06), 10.563 (0.14), 10.579 (0.25), 10.594 (0.13). |

(fortgesetzt)

| Bsp. | | Analytische Daten |
|---|---|---|
| **68** | 1-(2,4-Difluorphenyl)-7-[(3*S*)-3-fluorpyrrolidin-1-yl]-4-oxo-*N*-[1-(trifluormethyl)cyclopropyl]-1,4-dihydro-1,8-naphthyridin-3-carbonsäureamid 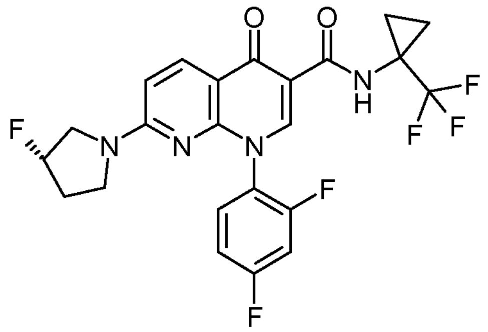 (94 % d. Th.) | LC-MS (Methode 1): $R_t$ = 1.12 min MS (ESpos): m/z = 497.3 $[M+H]^+$ $^1H$-NMR (400 MHz, $CDCl_3$) δ [ppm]: 0.016 (0.30), 0.078 (0.15), 1.234 (0.52), 1.354 (0.11), 1.371 (0.17), 1.391 (1.08), 1.416 (0.12), 1.431 (0.07), 1.441 (0.18), 1.502 (0.05), 1.580 (16.00), 2.059 (0.06), 2.150 (0.06), 2.380 (0.08), 2.450 (0.14), 2.794 (0.10), 2.893 (0.23), 2.965 (0.27), 3.607 (0.23), 5.237 (0.06), 5.368 (0.07), 6.554 (0.18), 6.570 (0.17), 7.006 (0.13), 7.039 (0.36), 7.058 (0.48), 7.078 (0.24), 7.353 (0.15), 7.373 (0.26), 7.388 (0.27), 7.409 (0.13), 7.529 (0.10), 8.438 (0.91), 8.460 (0.88), 8.469 (0.07), 8.677 (1.57), 8.704 (0.07), 10.627 (0.63). |
| **69** | 1-(2,4-Difluorphenyl)-7-[(3*S*)-3-fluorpyrrolidin-1-yl]-4-oxo-*N*-[1,1,1-trifluor-3-methylbutan-2-yl]-1,4-dihydro-1,8-naphthyridin-3-carbonsäureamid (Diastereomerengemisch) 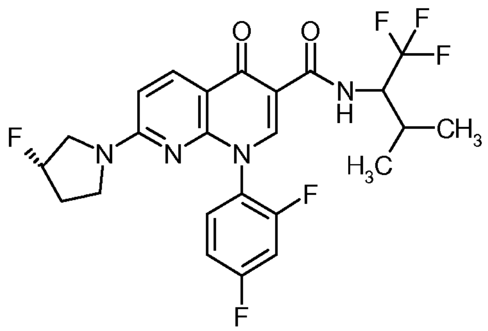 Aufarbeitung: Ausfällen des Produkts mit 1 M wässr. Salzsäure und Wasser und anschließendes Abfiltrieren des Niederschlags. (98 % d. Th.) | LC-MS (Methode 1): $R_t$ = 1.23 min MS (ESpos): m/z = 513.3 $[M+H]^+$ $^1H$-NMR (400 MHz, $CDCl_3$) δ [ppm]: 0.000 (0.84), 0.017 (0.61), 0.078 (0.50), 0.863 (0.16), 0.976 (0.27), 0.995 (0.27), 1.063 (4.55), 1.081 (4.67), 1.138 (4.58), 1.155 (4.44), 1.232 (0.20), 1.267 (0.14), 1.284 (0.14), 1.293 (0.13), 1.441 (0.48), 1.584 (16.00), 2.064 (0.15), 2.089 (0.14), 2.268 (0.22), 2.285 (0.47), 2.295 (0.51), 2.302 (0.62), 2.313 (0.65), 2.320 (0.51), 2.330 (0.50), 2.347 (0.28), 2.384 (0.18), 2.450 (0.34), 2.793 (0.23), 2.812 (0.60), 2.893 (1.00), 2.965 (1.23), 3.609 (0.52), 4.754 (0.11), 4.765 (0.33), 4.775 (0.36), 4.790 (0.46), 4.797 (0.44), 4.811 (0.34), 4.822 (0.32), 5.242 (0.15), 5.372 (0.15), 6.560 (0.39), 7.006 (0.33), 7.044 (0.86), 7.063 (1.17), 7.082 (0.60), 7.366 (0.35), 7.386 (0.62), 7.401 (0.63), 7.422 (0.29), 7.529 (0.30), 8.027 (0.13), 8.480 (2.49), 8.502 (2.40), 8.700 (3.93), 10.580 (0.77), 10.605 (0.76). |
| **70** | 1-(2,4-Difluorphenyl)-7-[(3*S*)-3-fluorpyrrolidin-1-yl]-*N*-(1,1,1,3,3,3-hexafluorpropan-2-yl)-4-oxo-1,4-dihydro-1,8-naphthyridin-3 -carbonsäureamid 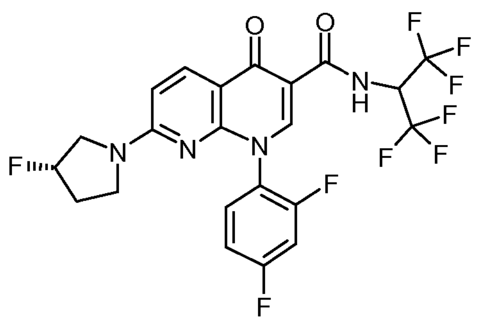 Aufarbeitung: Ausfällen des Produkts mit 1 M wässr. Salzsäure und Wasser und anschließendes Abfiltrieren des Niederschlags. (11 % d. Th.) | LC-MS (Methode 1): $R_t$ = 1.24 min MS (ESpos): m/z = 539.2 $[M+H]^+$ $^1H$-NMR (400 MHz, $CDCl_3$) δ [ppm]: -0.140 (0.11), 0.001 (0.99), 0.017 (0.82), 0.079 (0.21), 0.155 (0.11), 1.265 (0.11), 1.557 (16.00), 2.101 (0.10), 2.152 (0.11), 2.430 (0.12), 2.449 (0.24), 2.794 (0.16), 3.376 (0.10), 3.496 (0.21), 3.505 (0.21), 3.624 (0.35), 5.235 (0.09), 5.371 (0.09), 5.554 (0.15), 5.572 (0.35), 5.579 (0.18), 5.589 (0.44), 5.597 (0.38), 5.607 (0.33), 5.614 (0.46), 5.625 (0.16), 5.632 (0.32), 5.650 (0.13), 6.586 (0.25), 7.006 (0.21), 7.059 (0.55), 7.079 (0.75), 7.098 (0.37), 7.371 (0.24), 7.391 (0.40), 7.407 (0.40), 7.426 (0.17), 7.529 (0.20), 8.469 (1.94), 8.492 (1.89), 8.691 (2.87), 11.305 (0.64), 11.330 (0.63). |

(fortgesetzt)

| Bsp. | | Analytische Daten |
|---|---|---|
| **71** | 1-(2,4-Difluorphenyl)-7-[(3*S*)-3-fluorpyrrolidin-1-yl]-4-oxo-*N*-[1,1,1-trifluorbutan-2-yl]-1,4-dihydro-1,8-naphthyridin-3-carbonsäureamid (Diastereomerengemisch ) 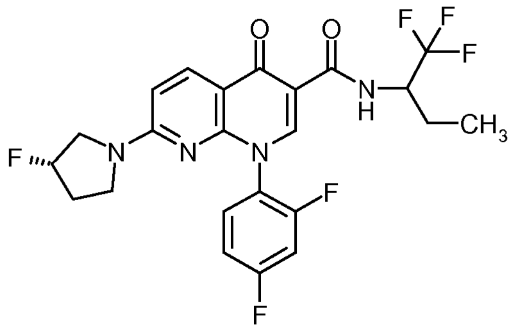 Aufarbeitung: Ausfällen des Produkts mit 1 M wässr. Salzsäure und Wasser und anschließendes Abfiltrieren des Niederschlags. (86 % d. Th.) | LC-MS (Methode 1): $R_t$ = 1.18 min MS (ESpos): m/z = 499.3 $[M+H]^+$ $^1$H-NMR (400 MHz, $CDCl_3$) δ [ppm]: 0.000 (0.22), 0.016 (0.19), 0.078 (0.13), 1.057 (0.57), 1.076 (1.25), 1.094 (0.62), 1.231 (0.04), 1.440 (0.17), 1.457 (0.05), 1.485 (0.06), 1.501 (0.06), 1.511 (0.06), 1.590 (16.00), 1.687 (0.05), 1.705 (0.10), 1.712 (0.05), 1.723 (0.12), 1.731 (0.11), 1.740 (0.15), 1.749 (0.13), 1.759 (0.13), 1.766 (0.15), 1.778 (0.05), 1.784 (0.12), 1.803 (0.04), 1.913 (0.04), 1.932 (0.11), 1.942 (0.12), 1.950 (0.12), 1.961 (0.13), 1.967 (0.11), 1.977 (0.11), 1.986 (0.09), 1.996 (0.09), 2.005 (0.05), 2.014 (0.04), 2.072 (0.03), 2.126 (0.03), 2.399 (0.05), 2.451 (0.08), 2.795 (0.06), 2.812 (0.09), 2.893 (0.08), 2.965 (0.10), 3.611 (0.14), 4.726 (0.05), 4.735 (0.06), 4.750 (0.10), 4.759 (0.10), 4.769 (0.10), 4.778 (0.10), 4.785 (0.07), 4.794 (0.06), 4.804 (0.04), 5.237 (0.04), 5.379 (0.04), 6.565 (0.10), 7.006 (0.10), 7.045 (0.22), 7.064 (0.29), 7.083 (0.15), 7.364 (0.09), 7.385 (0.15), 7.399 (0.15), 7.420 (0.07), 7.529 (0.09), 8.456 (0.66), 8.478 (0.65), 8.694 (0.97), 10.419 (0.20), 10.443 (0.20). |

**Beispiel 72**

1-(2,4-Difluorphenyl)-7-[(3*R*)-3-fluorpyrrolidin-1-yl]-4-oxo-*N*-(tricyclo[3.3.1.1$^{3,7}$]dec-1-yl)-1,4-dihydro-1,8-naphthyridin-3-carbonsäureamid

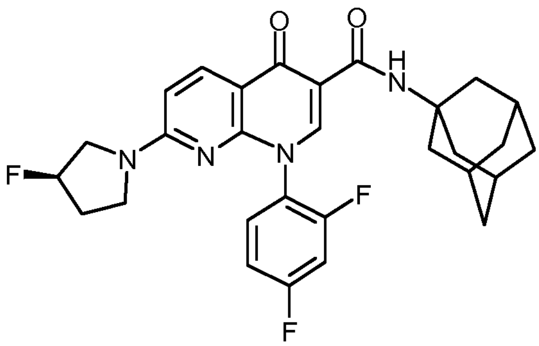

100 mg (0.26 mmol) der Verbindung aus Beispiel 44A wurden in 2.9 ml DMF vorgelegt, mit 117 mg (0.31 mmol) HATU sowie 106 mg (0.82 mmol) DIPEA versetzt und 30 Minuten lang bei 20°C gerührt. Dann wurden 54 mg (0.36 mmol) 1-Adamantanamin zugegeben und 2 Stunden bei 20°C gerührt. Anschliessend wurde das Gemisch direkt über präparative HPLC gereinigt (Eluent: Acetonitril/Wasser-Gradient mit 0.1% Ameisensäure). Man erhielt 103 mg (77 % d. Th.) der Titelverbindung.
LC-MS (Methode 1): $R_t$ = 1.38 min; m/z = 523.3 $[M+H]^+$.
$^1$H-NMR (400 MHz, DMSO-$d_6$) δ [ppm]: -0.150 (0.23), -0.041 (0.10), -0.023 (0.69), -0.018 (0.95), -0.009 (3.93), -0.007 (3.54), 0.006 (1.08), 0.007 (1.84), 0.011 (0.32), 0.013 (0.18), 0.016 (0.13), 0.146 (0.25), 1.156 (1.38), 1.174 (2.79), 1.192 (1.39), 1.234 (0.24), 1.355 (0.69), 1.672 (7.42), 1.825 (0.14), 1.988 (4.88), 2.057 (16.00), 2.182 (0.33), 2.215 (0.29), 2.322 (0.25), 2.327 (0.32), 2.332 (0.22), 2.366 (0.28), 2.519 (1.27), 2.521 (1.30), 2.523 (1.58), 2.558 (0.24), 2.560 (0.20), 2.563 (0.17), 2.565 (0.13), 2.568 (0.14), 2.570 (0.12), 2.573 (0.13), 2.575 (0.11), 2.665 (0.25), 2.669 (0.30), 2.674 (0.22), 2.709 (0.30), 3.161 (1.88), 3.174 (1.86), 3.467 (0.26), 3.675 (0.25), 4.002 (0.39), 4.020 (1.11), 4.038 (1.09), 4.056 (0.43), 4.073 (0.52), 4.086 (0.49), 4.099 (0.18), 5.323 (0.11), 5.412 (0.14), 6.764 (0.58), 6.785 (0.57), 7.300 (0.36), 7.321 (0.67),

7.339 (0.38), 7.565 (0.42), 7.585 (0.38), 7.767 (0.35), 7.789 (0.69), 7.804 (0.68), 7.826 (0.30), 8.149 (0.45), 8.291 (1.37), 8.313 (1.30), 8.490 (4.58), 8.519 (0.13), 9.937 (2.54).

**Beispiel 73**

1-(2,4-Difluorphenyl)-7-(3,3-difluorpyrrolidin-1-yl)-4-oxo-*N*-(tricyclo[3.3.1.1$^{3,7}$]dec-1-yl)-1,4-dihydro-1,8-naphthyridin-3-carbonsäureamid

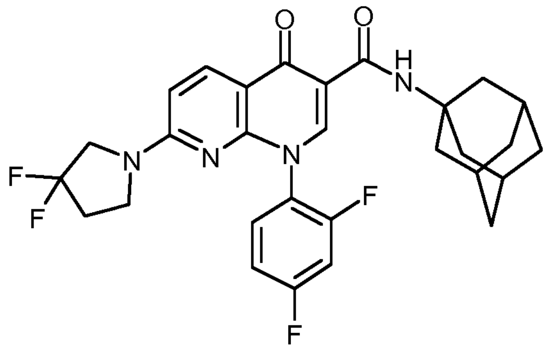

100 mg (0.26 mmol) der Verbindung aus Beispiel 43A wurden in 2.8 ml DMF vorgelegt, mit 112 mg (0.3 mmol) HATU sowie 101 mg (0.79 mmol) DIPEA versetzt und 30 Minuten lang bei 20°C gerührt. Dann wurden 52 mg (0.34 mmol) 1-Adamantanamin zugegeben und 2 Stunden bei 20°C gerührt. Anschliessend wurde das Gemisch über präparative HPLC gereinigt (Eluent: Acetonitril/Wasser-Gradient mit 0.1% Ameisensäure). Man erhielt 76 mg (57 % d. Th.) der Titelverbindung.
LC-MS (Methode 1): $R_t$ = 1.41 min; m/z = 541.3 $[M+H]^+$.
$^1$H-NMR (400 MHz, DMSO-$d_6$) $\delta$ [ppm]: -0.009 (0.75), 0.007 (0.74), 1.232 (0.33), 1.672 (7.44), 2.057 (16.00), 2.365 (0.15), 2.709 (0.15), 3.161 (2.40), 3.174 (2.48), 3.546 (0.24), 4.060 (0.26), 4.073 (0.71), 4.086 (0.69), 4.099 (0.24), 6.791 (0.59), 6.813 (0.61), 7.306 (0.40), 7.328 (0.78), 7.349 (0.43), 7.557 (0.41), 7.563 (0.43), 7.586 (0.69), 7.605 (0.43), 7.612 (0.41), 7.770 (0.49), 7.785 (0.57), 7.791 (0.97), 7.806 (0.96), 7.813 (0.55), 7.828 (0.46), 8.336 (1.86), 8.358 (1.78), 8.519 (4.96), 9.897 (2.61).

In Analogie zu Beispiel 73 wurden die in Tabelle 8 gezeigten Beispielverbindungen hergestellt, indem die Verbindung aus Beispiel 43A mit den entsprechenden Aminen (bzw. deren Salzen) unter den beschriebenen Reaktionsbedingungen umgesetzt wurden. Abweichungen sind bei den jeweiligen Beispielen angegeben.

Tabelle 8:

| Bsp. | | Analytische Daten |
|---|---|---|
| **74** | *rac*-1-(2,4-Difluorphenyl)-7-(3,3-difluorpyrrolidin-1-yl)-4-oxo-*N*-[1,1,1-trifluorbutan-2-yl]-1,4-dihydro-1,8-naphthyridin-3-carbonsäureamid 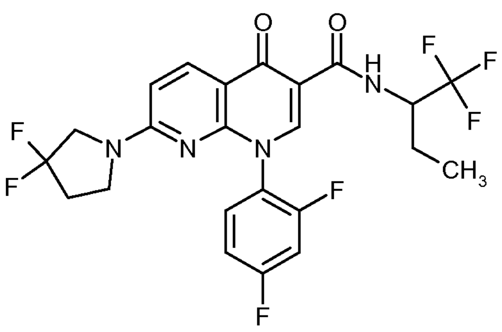 (88 % d. Th.) | LC-MS (Methode 1): $R_t$ = 1.22 min MS (ESpos): m/z = 517.2 [M+H] $^1$H-NMR (400 MHz, $CDCl_3$) $\delta$ [ppm]: 0.079 (1.42), 1.057 (7.39), 1.076 (15.90), 1.095 (7.93), 1.232 (5.14), 1.572 (16.00), 1.705 (1.42), 1.723 (1.66), 1.740 (1.97), 1.766 (1.94), 1.784 (1.50), 1.964 (1.56), 2.450 (2.28), 3.619 (2.76), 4.753 (1.34), 6.541 (2.25), 6.563 (2.32), 7.006 (1.26), 7.045 (2.37), 7.065 (3.90), 7.078 (3.60), 7.360 (1.50), 7.380 (2.31), 7.394 (2.41), 7.529 (1.20), 8.505 (8.37), 8.528 (7.99), 8.712 (12.30), 10.344 (2.52), 10.368 (2.49). |

(fortgesetzt)

| Bsp. | | Analytische Daten |
|---|---|---|
| **75** | 1-(2,4-Difluorphenyl)-7-(3,3-difluorpyrrolidin-1-yl)-4-oxo-*N*-[(2*S*)-1,1,1-trifluorbutan-2-yl]-1,4-dihydro-1,8-naphthyridin-3 -carbonsäureamid 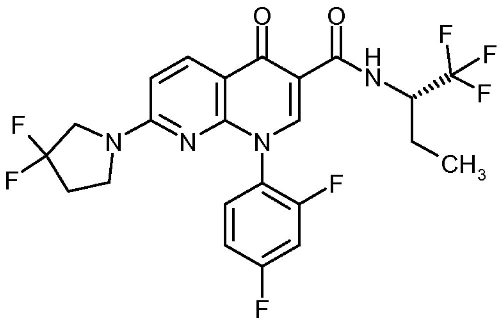 (90 % d. Th.) | LC-MS (Methode 1): $R_t$ = 1.22 min MS (ESpos): m/z = 517.2 $[M+H]^+$ $^1$H-NMR (400 MHz, $CDCl_3$) δ [ppm]: 0.001 (3.23), 0.017 (2.20), 0.079 (1.12), 1.057 (7.43), 1.075 (16.00), 1.094 (7.94), 1.253 (5.33), 1.442 (1.10), 1.587 (6.32), 1.705 (1.64), 1.723 (1.85), 1.730 (1.63), 1.740 (2.10), 1.748 (1.79), 1.758 (1.81), 1.765 (2.06), 1.784 (1.62), 1.935 (1.36), 1.945 (1.43), 1.953 (1.47), 1.963 (1.58), 1.980 (1.25), 2.450 (2.19), 2.471 (2.26), 3.000 (0.76), 3.622 (2.74), 4.761 (1.35), 6.541 (2.20), 6.563 (2.21), 7.006 (1.01), 7.045 (2.38), 7.059 (3.52), 7.065 (3.84), 7.077 (3.64), 7.084 (3.12), 7.359 (1.55), 7.380 (2.28), 7.394 (2.36), 7.416 (1.18), 7.529 (0.88), 8.505 (8.69), 8.527 (8.30), 8.712 (12.29), 10.344 (2.49), 10.368 (2.49). |
| **76** | *rac*-1-(2,4-Difluorphenyl)-7-(3,3-difluorpyrrolidin-1-yl)-4-oxo-*N*-[1,1,1-trifluorpentan-2-yl]-1,4-dihydro-1,8-naphthyridin-3-carbonsäureamid 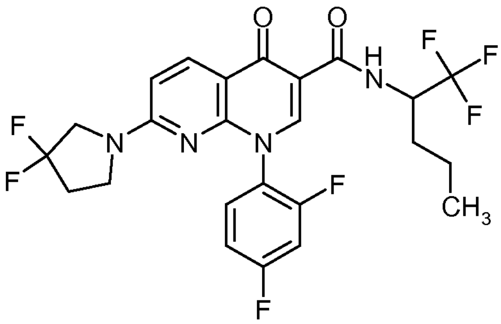 (82 % d. Th.) | LC-MS (Methode 1): $R_t$ = 1.28 min MS (ESpos): m/z = 531.2 $[M+H]^+$ $^1$H-NMR (400 MHz, $CDCl_3$) δ [ppm]: 0.001 (3.68), 0.017 (2.94), 0.079 (1.31), 0.957 (7.45), 0.975 (16.00), 0.994 (8.03), 1.161 (4.06), 1.233 (1.72), 1.442 (2.18), 1.461 (1.88), 1.479 (2.03), 1.497 (1.61), 1.583 (14.64), 1.686 (1.15), 1.721 (1.97), 1.733 (1.47), 1.747 (2.16), 1.759 (1.56), 1.770 (1.26), 1.782 (1.01), 1.841 (1.54), 1.859 (1.34), 2.450 (2.21), 2.470 (2.11), 3.618 (2.48), 4.859 (1.21), 6.539 (2.05), 6.562 (2.05), 7.006 (1.29), 7.043 (2.11), 7.063 (3.49), 7.076 (3.17), 7.358 (1.38), 7.379 (2.09), 7.393 (2.17), 7.415 (1.10), 7.529 (1.23), 8.502 (8.83), 8.524 (8.43), 8.710 (11.39), 10.325 (2.44), 10.349 (2.44). |

## Beispiel 77

1-(2,4-Difluorphenyl)-4-oxo-7-(3,3,4,4-tetrafluorpyrrolidin-1yl)-*N*-(tricyclo[3.3.1.1$^{3,7}$]dec-1-yl)-1,4-dihydro-1,8-naphthyridin-3 -carbonsäureamid

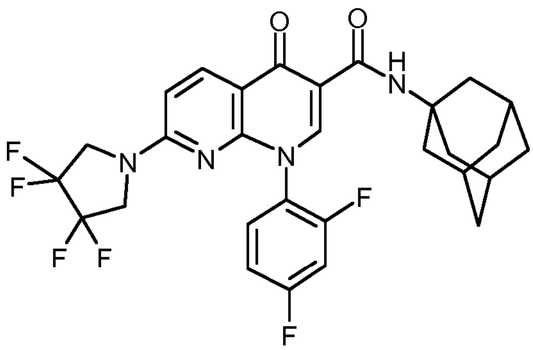

110 mg (0.21 mmol, Reinheit 86%) der Verbindung aus Beispiel 46A wurden in 2.4 ml DMF vorgelegt, mit 97 mg (0.26 mmol) HATU sowie 88 mg (0.68 mmol) DIPEA versetzt und 30 Minuten lang bei 20°C gerührt. Dann wurden 45 mg (0.3 mmol) 1-Adamantanamin zugegeben und 2 Stunden bei 20°C gerührt. Anschliessend wurde das Gemisch

über präparative HPLC gereinigt (Eluent: Acetonitril/Wasser-Gradient mit 0.1% Ameisensäure). Man erhielt 61 mg (50 % d. Th.) der Titelverbindung.
LC-MS (Methode 1): $R_t$ = 1.41 min; m/z = 577.3 $[M+H]^+$.
$^1$H-NMR (400 MHz, DMSO-$d_6$) $\delta$ [ppm]: 0.000 (16.00), 1.235 (0.38), 1.674 (4.18), 2.060 (8.85), 3.161 (0.98), 3.175 (1.04), 4.069 (0.63), 4.083 (0.61), 6.877 (1.25), 6.899 (1.26), 7.337 (0.45), 7.570 (0.31), 7.589 (0.43), 7.612 (0.30), 7.787 (0.29), 7.809 (0.54), 7.823 (0.54), 7.846 (0.27), 8.433 (1.51), 8.455 (1.40), 8.562 (2.73), 9.835 (1.41).

**Beispiel 78**

1-(2,4-Difluorphenyl)-7-(1,1-dioxido-1,3-thiazolidin-3-yl)-4-oxo-*N*-[1,1,1-trifluoro-4-methylpentan-2-yl]-1,4-dihydro-1,8-naphthyridin-3-carbonsäureamid

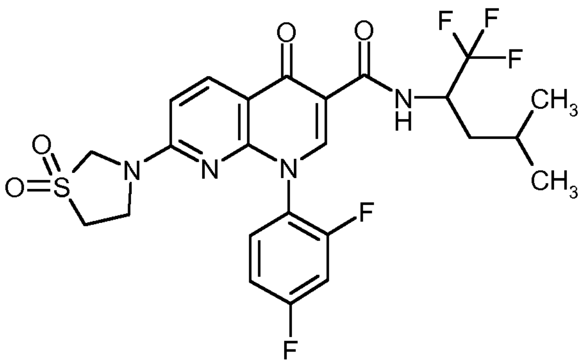

80 mg (0.15 mmol, Reinheit 80%) der Verbindung aus Beispiel 50A wurden in 1.7 ml DMF vorgelegt, mit 70 mg (0.18 mmol) HATU sowie 63 mg (0.49 mmol) DIPEA versetzt und 30 Minuten lang bei 20°C gerührt. Dann wurden 59 mg (0.39 mmol) 1,1,1-Trifluor-4-methylpentan-2-amin Hydrochlorid zugegeben und 2 Stunden bei 20°C gerührt. Dann wurde 1 ml 1 M wässr. Salzsäure und 2 ml Wasser zugegeben und der ausgefallene Niederschlag abfiltriert, mit 2 ml Wasser und 1 ml Petrolether gewaschen und im Hochvakuum getrocknet. Man erhielt 72 mg (84 % d. Th.) der Titelverbindung.
LC-MS (Methode 1): $R_t$ = 1.15 min; m/z = 559.3 $[M+H]^+$.
$^1$H-NMR (400 MHz, $CDCl_3$) $\delta$ [ppm]: -0.140 (0.90), 0.001 (7.91), 0.017 (7.32), 0.079 (2.03), 0.155 (0.80), 0.937 (0.81), 0.957 (6.31), 0.965 (6.32), 0.973 (7.22), 0.982 (7.57), 0.988 (9.90), 1.004 (9.53), 1.233 (1.77), 1.343 (3.73), 1.430 (0.86), 1.442 (2.01), 1.580 (16.00), 1.621 (4.52), 1.647 (2.46), 1.708 (1.59), 1.718 (1.97), 1.737 (1.56), 1.746 (2.37), 1.773 (1.76), 1.799 (1.17), 2.450 (1.53), 2.602 (0.92), 2.794 (1.57), 2.894 (1.25), 2.965 (1.59), 2.999 (0.85), 3.179 (0.78), 3.379 (3.94), 3.397 (8.19), 3.414 (4.27), 3.986 (4.62), 4.003 (8.47), 4.021 (3.86), 4.390 (4.78), 4.893 (0.90), 4.918 (0.94), 6.685 (5.10), 6.708 (5.24), 7.006 (1.92), 7.064 (1.40), 7.084 (3.11), 7.104 (2.61), 7.370 (1.09), 7.385 (1.41), 7.391 (1.53), 7.405 (1.57), 7.427 (0.86), 7.529 (1.90), 8.616 (5.68), 8.639 (5.59), 8.757 (5.70), 10.146 (1.87), 10.170 (1.82).

**Beispiel 79**

1-(2,4-Difluorphenyl)-7-[(2-hydroxyethyl)amino]-4-oxo-*N*-(tricyclo[3.3.1.1$^{3,7}$]dec-1-yl)-1,4-dihydro-1,8-naphthyridin-3-carbonsäureamid

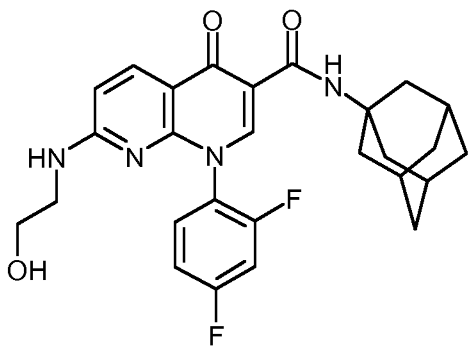

100 mg (0.28 mmol) der Verbindung aus Beispiel 40A wurden in 3.1 ml DMF vorgelegt, mit 126 mg (0.33 mmol) HATU sowie 114 mg (0.89 mmol) DIPEA versetzt und 30 Minuten lang bei 20°C gerührt. Dann wurden 59 mg (0.39 mmol) 1-Adamantanamin zugegeben und 2 Stunden bei 20°C gerührt. Anschliessend wurde das Gemisch über präparative HPLC gereinigt (Eluent: Acetonitril/Wasser-Gradient mit 0.1% Ameisensäure). Man erhielt 77 mg (56 % d. Th.) der Titelverbindung.

LC-MS (Methode 1): $R_t$ = 1.20 min; m/z = 495.3 $[M+H]^+$.

$^1$H-NMR (400 MHz, DMSO-$d_6$) $\delta$ [ppm]: -0.013 (0.84), -0.005 (16.00), 0.003 (1.54), 0.011 (1.70), 1.665 (1.85), 2.047 (3.60), 3.025 (0.21), 3.305 (14.58), 3.320 (2.20), 4.595 (0.20), 6.661 (0.24), 6.684 (0.27), 7.302 (0.21), 7.541 (0.19), 7.760 (0.23), 7.775 (0.25), 7.940 (0.14), 8.132 (0.18), 8.425 (0.71), 9.985 (0.44).

In Analogie zu Beispiel 79 wurden die in Tabelle 9 gezeigten Beispielverbindungen hergestellt, indem die Verbindung aus Beispiel 40A mit den entsprechenden Aminen (bzw. deren Salzen) unter den beschriebenen Reaktionsbedingungen umgesetzt wurden. Abweichungen sind bei den jeweiligen Beispielen angegeben.

Tabelle 9:

| Bsp. | | Analytische Daten |
|---|---|---|
| **80** | 1 -(2,4-Difluorphenyl)-7- [(2-hydroxyethyl) amino]-4-oxo-*N*-[1,1,1-trifluor-4-methylpentan-2-yl]-1,4-dihydro-1,8-naphthyridin-3 -carbonsäureamid 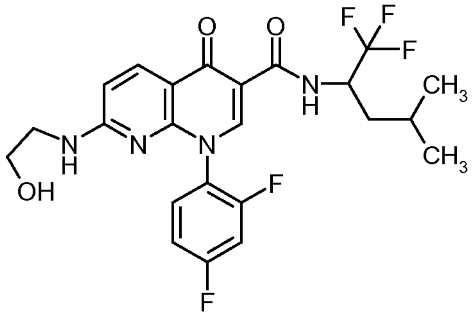 (52 % d. Th.) | LC-MS (Methode 1): $R_t$ = 1.08 min MS (ESpos): m/z = 499.3 $[M+H]^+$ $^1$H-NMR (400 MHz, $CDCl_3$) $\delta$ [ppm]: 0.000 (0.62), 0.009 (16.00), 0.017 (0.54), 0.078 (1.39), 0.953 (1.91), 0.969 (2.16), 0.975 (2.17), 0.981 (3.89), 0.997 (3.76), 1.590 (8.07), 1.635 (1.25), 1.709 (0.55), 1.719 (0.66), 1.737 (0.54), 1.747 (0.83), 1.753 (0.57), 1.779 (0.61), 1.791 (0.40), 1.801 (0.44), 2.449 (0.72), 2.599 (0.31), 2.601 (0.42), 2.604 (0.53), 2.633 (1.92), 2.635 (1.25), 2.637 (0.96), 2.639 (0.83), 2.641 (0.61), 2.644 (0.43), 2.646 (0.29), 2.649 (0.34), 2.794 (0.78), 3.321 (0.51), 3.334 (1.26), 3.345 (1.44), 3.359 (0.71), 3.650 (0.95), 3.662 (1.38), 3.674 (0.74), 4.889 (0.31), 4.913 (0.32), 5.459 (0.38), 5.474 (0.66), 5.488 (0.38), 6.535 (1.92), 6.557 (1.98), 7.006 (0.49), 7.032 (0.37), 7.052 (1.16), 7.073 (1.04), 7.091 (0.32), 7.282 (0.47), 7.285 (0.32), 7.364 (0.46), 7.379 (0.49), 7.386 (0.61), 7.400 (0.59), 7.407 (0.41), 7.421 (0.34), 7.529 (0.50), 8.379 (1.44), 8.401 (1.39), 8.671 (2.40), 10.330 (0.62), 10.353 (0.60). |

(fortgesetzt)

| Bsp. | | Analytische Daten |
|---|---|---|
| **81** | 1-(2,4-Difluorphenyl)-7-[(2-hydroxyethyl) amino]-4-oxo-*N*-[1,1,1-trifluorpentan-2-yl]-1,4-dihydro-1,8-naphthyridin-3-carbonsäureamid 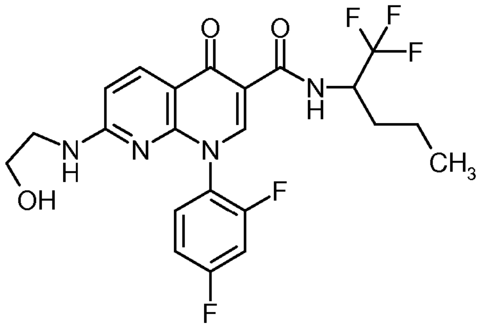 (66 % d. Th.) | LC-MS (Methode 1): $R_t$ = 1.03 min MS (ESpos): m/z = 485.1 $[M+H]^+$ $^1$H-NMR (400 MHz, $CDCl_3$) δ [ppm]: 0.009 (16.00), 0.078 (1.32), 0.953 (2.90), 0.972 (6.47), 0.990 (3.32), 1.268 (0.49), 1.456 (0.71), 1.473 (0.75), 1.592 (5.81), 1.718 (0.94), 1.730 (0.71), 1.744 (0.98), 1.756 (0.76), 1.768 (0.62), 1.828 (0.76), 2.053 (0.81), 2.450 (0.67), 2.795 (0.70), 3.334 (2.18), 3.345 (2.48), 3.359 (1.19), 3.499 (2.54), 3.650 (1.68), 3.662 (2.43), 4.853 (0.54), 5.478 (1.10), 6.535 (3.40), 6.557 (3.46), 7.006 (0.52), 7.034 (0.80), 7.054 (2.29), 7.074 (2.10), 7.092 (0.63), 7.362 (0.56), 7.383 (0.82), 7.397 (0.84), 7.529 (0.52), 8.381 (2.56), 8.403 (2.49), 8.665 (5.40), 10.366 (1.05), 10.390 (1.04). |

**Beispiel 82**

1-(2,4-Difluorphenyl)-7-[(2-hydroxyethyl)(methyl)amino]-4-oxo-*N*-(tricyclo[3.3.1.1$^{3,7}$]dec-1-yl)-1,4-dihydro-1,8-naphthyridin-3 -carbonsäureamid

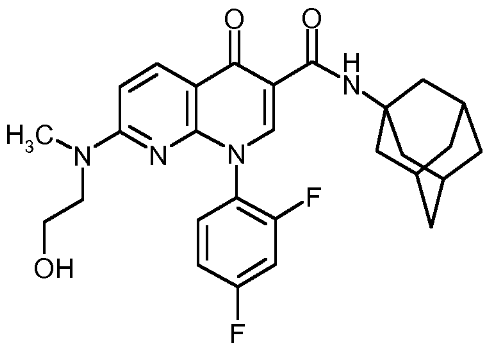

100 mg (0.27 mmol) der Verbindung aus Beispiel 39A wurden in 3 ml DMF vorgelegt, mit 121 mg (0.32 mmol) HATU sowie 110 mg (0.85 mmol) DIPEA versetzt und 30 Minuten lang bei 20°C gerührt. Dann wurden 56 mg (0.37 mmol) 1-Adamantanamin zugegeben und für 2 Stunden bei 20°C gerührt. Anschliessend wurde das Gemisch über präparative HPLC gereinigt (Eluent: Acetonitril/Wasser-Gradient mit 0.1% Ameisensäure). Man erhielt 92 mg (68 % d. Th.) der Titelverbindung.

LC-MS (Methode 1): $R_t$ = 1.19 min; m/z = 509.2 $[M+H]^+$.

$^1$H-NMR (400 MHz, DMSO-$d_6$) δ [ppm]: -0.150 (0.24), -0.023 (0.16), -0.020 (0.15), -0.009 (2.07), 0.007 (1.93), 0.145 (0.23), 1.146 (0.09), 1.168 (0.83), 1.174 (0.20), 1.233 (0.21), 1.270 (0.15), 1.671 (7.32), 1.987 (0.25), 2.055 (16.00), 2.322 (0.13), 2.327 (0.20), 2.365 (0.17), 2.523 (0.40), 2.669 (0.21), 2.674 (0.16), 2.709 (0.18), 3.020 (0.25), 3.162 (2.25), 3.173 (2.27), 4.073 (0.44), 4.087 (0.43), 4.648 (0.12), 6.578 (0.11), 6.903 (0.34), 7.285 (0.37), 7.290 (0.40), 7.306 (0.74), 7.312 (0.77), 7.328 (0.40), 7.332 (0.42), 7.526 (0.38), 7.533 (0.39), 7.556 (0.66), 7.574 (0.40), 7.581 (0.38), 7.747 (0.44), 7.762 (0.54), 7.769 (0.88), 7.784 (0.86), 7.790 (0.51), 7.805 (0.43), 8.143 (1.20), 8.231 (0.74), 8.254 (0.73), 8.473 (5.19), 9.954 (2.53).

**Beispiel 83**

1-(2,4-Difluorphenyl)-7-[(2-fluorethyl)amino]-4-oxo-*N*-(tricyclo[3.3.1.1$^{3,7}$]dec-1-yl)-1,4-dihydro-1,8-naphthyridin-3-carbonsäureamid

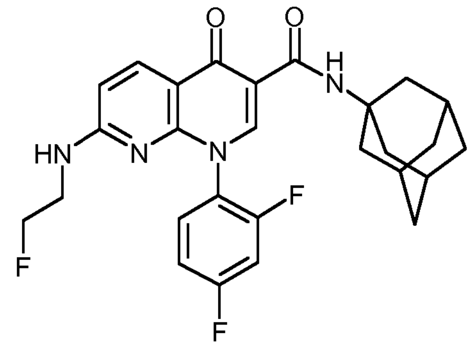

100 mg (0.17 mmol, Reinheit 77%) der Verbindung aus Beispiel 41A wurden in 2.4 ml DMF vorgelegt, mit 96 mg (0.25 mmol) HATU sowie 87 mg (0.68 mmol) DIPEA versetzt und 30 Minuten lang bei 20°C gerührt. Dann wurden 45 mg (0.3 mmol) 1-Adamantanamin zugegeben und 2 Stunden bei 20°C gerührt. Anschliessend wurde das Gemisch über präparative HPLC gereinigt (Eluent: Acetonitril/Wasser-Gradient mit 0.1% Ameisensäure). Man erhielt 92 mg (87 % d. Th.) der Titelverbindung. Weiterhin wurden 11 mg (11 % d. Th.) der Titelverbindung aus Beispiel 84 erhalten (Analytik s. Beispiel 84).
LC-MS (Methode 1): $R_t$ = 1.25 min; m/z = 497.1 $[M+H]^+$.
$^1$H-NMR (400 MHz, DMSO-$d_6$) $\delta$ [ppm]: -0.009 (1.89), 0.007 (1.26), 1.174 (0.32), 1.669 (7.54), 1.988 (0.63), 2.053 (16.00), 4.243 (0.76), 4.362 (0.77), 5.753 (3.13), 6.692 (1.39), 6.714 (1.40), 7.293 (0.46), 7.316 (0.83), 7.336 (0.45), 7.535 (0.54), 7.542 (0.55), 7.564 (0.77), 7.583 (0.54), 7.590 (0.53), 7.763 (0.52), 7.778 (0.73), 7.785 (1.00), 7.800 (1.00), 7.822 (0.48), 8.126 (0.44), 8.173 (1.35), 8.195 (1.26), 8.262 (0.52), 8.459 (4.10), 9.957 (2.41).

**Beispiel 84**

7-(Aziridin-1-yl)-1-(2,4-difluorphenyl)-4-oxo-*N*-(tricyclo[3.3.1..1$^{3,7}$]dec-1-yl)-1,4-dihydro-1,8-naphthyridin-3-carbonsäureamid

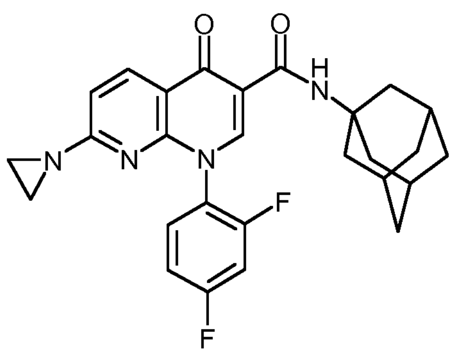

Wie bei der Darstellung der Verbindung aus Beispiel 83 beschrieben, wurden aus 100 mg (0.17 mmol) der Verbindung aus Beispiel 41A, 11 mg (11 % d. Th.) der Titelverbindung erhalten.
LC-MS (Methode 1): $R_t$ = 1.34 min; m/z = 479.2 $[M+H]^+$.
$^1$H-NMR (400 MHz, DMSO-$d_6$) $\delta$ [ppm]: -0.150 (1.66), -0.061 (0.29), -0.009 (16.00), 0.007 (12.85), 0.146 (1.63), 1.147 (0.73), 1.670 (4.93), 2.055 (10.44), 2.322 (1.15), 2.327 (1.55), 2.331 (1.20), 2.365 (1.43), 2.664 (1.49), 2.669 (1.88), 2.709 (1.75), 2.934 (1.81), 3.285 (1.03), 3.460 (0.27), 5.753 (5.19), 6.890 (1.66), 6.912 (1.65), 7.302 (0.31), 7.322 (0.58),

7.342 (0.34), 7.552 (0.36), 7.571 (0.50), 7.600 (0.38), 7.761 (0.39), 7.783 (0.61), 7.799 (0.61), 7.820 (0.29), 8.251 (1.83), 8.274 (1.69), 8.474 (3.26), 9.944 (1.67).

In Analogie zu Beispiel 83 wurden die in Tabelle 10 gezeigten Beispielverbindungen hergestellt, indem die Verbindung aus Beispiel 41A mit den entsprechenden Aminen (bzw. deren Salzen) unter den beschriebenen Reaktionsbedingungen umgesetzt wurden. Abweichungen sind bei den jeweiligen Beispielen angegeben.

Tabelle 10:

| Bsp. | | Analytische Daten |
|---|---|---|
| **85** | *rac*-1-(2,4-Difluorphenyl)-7-[(2-fluorethyl)-amino]-4-oxo-*N*-[1,1,1-trifluorbutan-2-yl]-1,4-dihydro-1,8-naphthyridin-3-carbonsäureamid 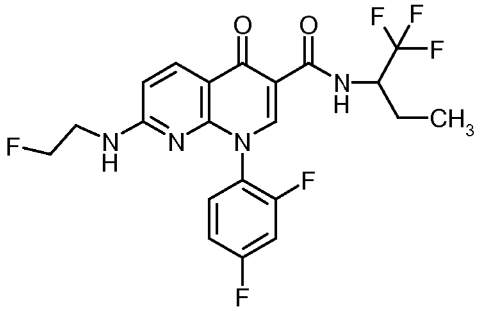 (62 % d. Th.) | LC-MS (Methode 1): $R_t$ = 1.09 min MS (ESpos): m/z = 473.2 $[M+H]^+$ $^1$H-NMR (400 MHz, CDCl3) δ [ppm]: 0.001 (0.93), 0.017 (0.79), 1.054 (1.69), 1.073 (3.70), 1.091 (1.86), 1.570 (16.00), 1.722 (0.35), 1.729 (0.30), 1.739 (0.43), 1.748 (0.36), 1.758 (0.35), 1.765 (0.42), 1.783 (0.35), 1.933 (0.29), 1.943 (0.31), 1.951 (0.33), 1.961 (0.35), 1.969 (0.30), 1.978 (0.28), 3.422 (0.41), 3.434 (0.57), 3.447 (0.46), 3.488 (0.39), 3.501 (0.48), 3.514 (0.36), 4.338 (0.59), 4.455 (0.57), 4.749 (0.32), 4.758 (0.32), 4.768 (0.29), 4.777 (0.30), 5.308 (0.55), 5.325 (0.36), 5.339 (0.64), 5.353 (0.34), 6.551 (2.21), 6.573 (2.25), 7.022 (0.32), 7.029 (0.45), 7.050 (1.36), 7.070 (1.29), 7.091 (0.34), 7.363 (0.34), 7.378 (0.45), 7.385 (0.53), 7.399 (0.54), 7.405 (0.33), 8.408 (1.71), 8.430 (1.64), 8.690 (2.98), 10.367 (0.57), 10.391 (0.56). |
| **86** | *rac*-1-(2,4-Difluorphenyl)-7-[(2-fluorethyl)amino]-4-oxo-*N*-[(2*R*)-1,1,1-trifluor-pentan-2-yl]-1,4-dihydro-1,8-naphthyridin-3-carbonsäureamid 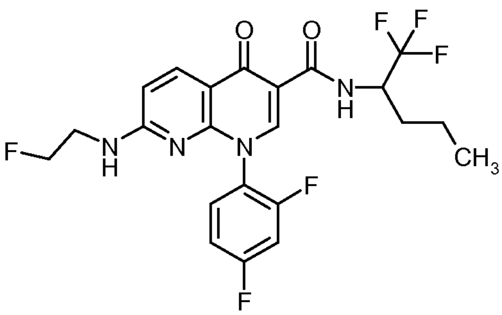 (80 % d. Th.) | LC-MS (Methode 1): $R_t$ = 1.18 min MS (ESpos): m/z = 487.2 $[M+H]^+$ $^1$H-NMR (400 MHz, DMSO-$d_6$) δ [ppm]: 0.890 (4.26), 0.908 (9.54), 0.926 (4.93), 1.156 (4.22), 1.174 (8.57), 1.191 (4.34), 1.293 (0.33), 1.311 (0.65), 1.329 (0.91), 1.348 (1.13), 1.366 (1.05), 1.385 (0.71), 1.403 (0.59), 1.437 (0.98), 1.457 (0.87), 1.589 (0.40), 1.601 (0.41), 1.624 (1.05), 1.636 (0.79), 1.650 (1.11), 1.662 (0.77), 1.673 (0.58), 1.685 (0.45), 1.735 (0.57), 1.744 (0.68), 1.768 (1.06), 1.777 (0.82), 1.786 (0.91), 1.987 (16.00), 3.238 (1.12), 4.001 (1.31), 4.019 (3.90), 4.037 (3.86), 4.055 (1.27), 4.248 (1.63), 4.365 (1.62), 4.787 (0.88), 4.809 (0.90), 6.727 (2.72), 6.750 (2.78), 7.296 (0.91), 7.318 (1.80), 7.339 (0.95), 7.537 (1.01), 7.544 (1.05), 7.563 (1.65), 7.586 (1.05), 7.592 (1.00), 7.788 (0.71), 7.809 (1.46), 7.825 (1.47), 7.846 (0.65), 8.195 (3.34), 8.217 (3.32), 8.601 (4.68), 10.499 (2.74), 10.523 (2.63). |

(fortgesetzt)

| Bsp. | | Analytische Daten |
|---|---|---|
| **87** | 1 -(2,4-Difluorphenyl)-7- [(2-fluorethyl)amino]-4-oxo-*N*-[(2*S*)-1,1,1-trifluor-butan-2-yl]-1,4-dihydro-1,8-naphthyridin-3-carbonsäureamid 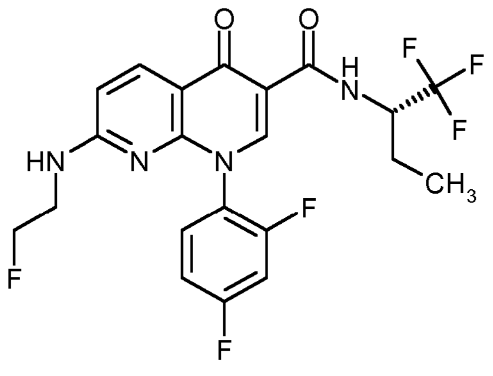 (88 % d. Th.) | LC-MS (Methode 1): $R_t$ = 1.10 min MS (ESpos): m/z = 473.1 $[M+H]^+$ $^1$H-NMR (400 MHz, CDCl3) $\delta$ [ppm]: 0.001 (0.70), 0.017 (0.67), 1.054 (2.82), 1.073 (6.18), 1.091 (3.08), 1.269 (0.84), 1.571 (16.00), 1.704 (0.44), 1.722 (0.57), 1.730 (0.49), 1.739 (0.70), 1.748 (0.60), 1.758 (0.60), 1.765 (0.71), 1.783 (0.56), 1.933 (0.48), 1.943 (0.53), 1.952 (0.56), 1.962 (0.60), 1.969 (0.51), 1.979 (0.47), 2.054 (1.54), 3.422 (0.69), 3.435 (0.96), 3.448 (0.77), 3.488 (0.65), 3.502 (0.81), 3.515 (0.60), 4.338 (0.98), 4.456 (0.97), 4.749 (0.52), 4.758 (0.53), 4.767 (0.50), 4.777 (0.51), 5.322 (0.60), 5.336 (1.10), 5.350 (0.59), 6.551 (3.52), 6.573 (3.59), 7.022 (0.52), 7.029 (0.73), 7.050 (2.28), 7.070 (2.18), 7.091 (0.57), 7.363 (0.60), 7.378 (0.75), 7.385 (0.95), 7.399 (0.92), 7.405 (0.56), 7.420 (0.47), 8.408 (2.76), 8.430 (2.69), 8.690 (4.93), 10.368 (0.96), 10.393 (0.94). |
| **88** | 1 -(2,4-Difluorphenyl)-7- [(2-fluorethyl)amino]-4-oxo-*N*-[1,1,1-trifluor-4-methylpentan-2-yl]-1,4-dihydro-1,8-naphthyridin-3 -carbonsäureamid 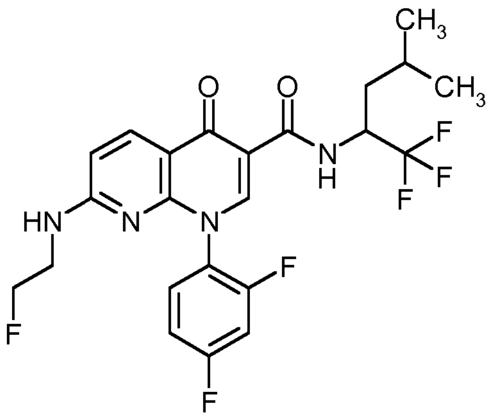 (81 % d. Th.) | LC-MS (Methode 1): $R_t$ = 1.21 min MS (ESpos): m/z = 501.1 $[M+H]^+$ $^1$H-NMR (400 MHz, CDCl3) $\delta$ [ppm]: 0.002 (1.23), 0.018 (0.96), 0.954 (3.69), 0.961 (3.35), 0.970 (4.10), 0.977 (3.90), 0.983 (6.77), 1.000 (6.52), 1.251 (1.45), 1.269 (2.97), 1.287 (1.48), 1.562 (16.00), 1.605 (0.50), 1.613 (0.98), 1.621 (0.57), 1.638 (0.74), 1.711 (0.73), 1.721 (0.95), 1.739 (0.76), 1.749 (1.28), 1.755 (0.82), 1.784 (0.91), 1.793 (0.54), 1.803 (0.60), 2.054 (5.63), 3.422 (0.69), 3.434 (0.94), 3.447 (0.77), 3.487 (0.65), 3.501 (0.78), 3.514 (0.58), 4.123 (1.22), 4.140 (1.19), 4.338 (0.99), 4.455 (0.98), 4.892 (0.54), 4.897 (0.51), 4.916 (0.55), 5.308 (0.65), 5.322 (1.19), 5.336 (0.63), 6.548 (3.79), 6.570 (3.87), 7.024 (0.60), 7.045 (1.87), 7.066 (1.71), 7.089 (0.51), 7.364 (0.78), 7.378 (0.90), 7.385 (1.10), 7.399 (1.12), 7.406 (0.70), 7.420 (0.61), 8.402 (2.82), 8.424 (2.74), 8.693 (4.00), 10.314 (1.02), 10.338 (1.01). |

(fortgesetzt)

| Bsp. | | Analytische Daten |
|---|---|---|
| **89** | 1 -(2,4-Difluorphenyl)-7- [(2-fluorethyl) amino]-*N*-(1,1,1,3,3,3-hexafluorpropan-2-yl)-4-oxo-1,4-dihydro-1,8-naphthyridin-3 -carbonsäureamid 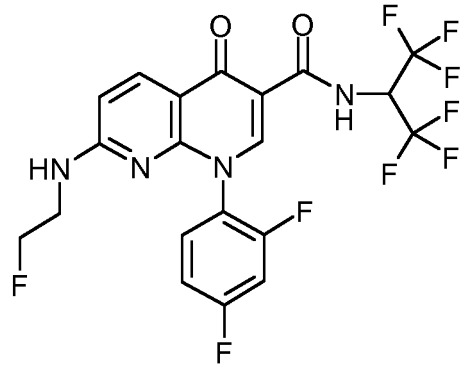 Aufarbeitung: Ausfällen des Produkts aus Wasser / 1 M wässr. Salzsäure und Abfiltrieren des Niederschlags. (49 % d. Th.) | LC-MS (Methode 1): $R_t$ = 1.16 min MS (ESpos): m/z = 513.0 $[M+H]^+$ $^1$H-NMR (400 MHz, DMSO-$d_6$) $\delta$ [ppm]: -0.009 (7.65), 0.007 (6.55), 2.520 (2.86), 2.523 (3.34), 2.525 (3.18), 3.244 (2.42), 4.249 (3.34), 4.367 (3.31), 6.293 (1.94), 6.298 (1.86), 6.317 (2.05), 6.750 (5.02), 6.772 (5.07), 7.302 (2.00), 7.306 (2.17), 7.309 (2.15), 7.322 (3.87), 7.328 (4.05), 7.344 (2.18), 7.348 (2.17), 7.351 (2.07), 7.547 (2.40), 7.554 (2.64), 7.572 (3.49), 7.576 (3.71), 7.595 (2.54), 7.602 (2.55), 7.799 (2.53), 7.814 (3.05), 7.821 (4.81), 7.836 (5.18), 7.843 (2.65), 7.857 (2.68), 8.216 (4.87), 8.239 (4.83), 8.278 (2.50), 8.290 (3.13), 8.705 (16.00), 11.489 (5.43), 11.515 (5.18). |

**Beispiel 90**

1-(2,4-Difluorphenyl)-4-oxo-*N*-(tricyclo[3.3.1.1$^{3,7}$]dec-1-yl)-7-[(2,2,2-trifluorethyl)amino]-1,4-dihydro-1,8-naphthyridin-3-carbonsäureamid

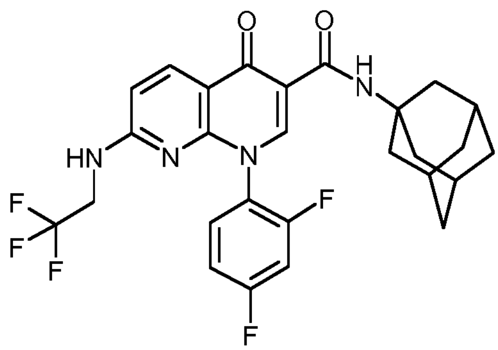

73 mg (0.18 mmol) der Verbindung aus Beispiel 47A wurden in 2 ml DMF vorgelegt, mit 83 mg (0.22 mmol) HATU sowie 76 mg (0.58 mmol) DIPEA versetzt und die Mischung für 30 Minuten bei 20°C gerührt. Dann wurden 39 mg (0.26 mmol) 1-Adamantanamin zugegeben und für 2 Stunden bei 20°C gerührt. Anschliessend wurde das Gemisch über präparative HPLC gereinigt (Eluent: Acetonitril/Wasser-Gradient mit 0.1% Ameisensäure). Man erhielt 86 mg (88 % d. Th.) der Titelverbindung.

LC-MS (Methode 1): $R_t$ = 1.35 min; m/z = 533.3 $[M+H]^+$.

$^1$H-NMR (400 MHz, DMSO-$d_6$) $\delta$ [ppm]: -0.150 (0.27), -0.009 (2.24), 0.007 (2.13), 0.145 (0.26), 1.671 (7.23), 2.056 (16.00), 2.327 (0.23), 2.365 (0.31), 2.669 (0.25), 2.709 (0.31), 3.842 (0.52), 6.782 (1.04), 6.804 (1.05), 7.302 (0.41), 7.323 (0.76), 7.344 (0.42), 7.529 (0.49), 7.536 (0.52), 7.554 (0.73), 7.577 (0.51), 7.584 (0.50), 7.754 (0.48), 7.769 (0.58), 7.776 (0.96), 7.791 (0.95), 7.798 (0.54), 7.813 (0.47), 8.149 (0.31), 8.271 (2.14), 8.293 (2.02), 8.387 (0.42), 8.511 (4.59), 9.896 (2.62).

In Analogie zu Beispiel 90 wurden die in Tabelle 11 gezeigten Beispielverbindungen hergestellt, indem die Verbindung aus Beispiel 47A, bzw. die Verbindung aus Beispiel 55A mit den entsprechenden Aminen (bzw. deren Salzen) unter den beschriebenen Reaktionsbedingungen umgesetzt wurden. Abweichungen sind bei den jeweiligen

Beispielen angegeben.

Tabelle 11:

| Bsp. | | Analytische Daten |
|---|---|---|
| **91** | 1-(2,4-Difluorphenyl)-*N*-(3-fluor-tricyclo[3.3.1.1$^{3,7}$]dec-1-yl)-4-oxo-7-[(2,2,2-trifluorethyl)amino]-1,4-dihydro-1,8-naphthyridin-3-carbonsäureamid 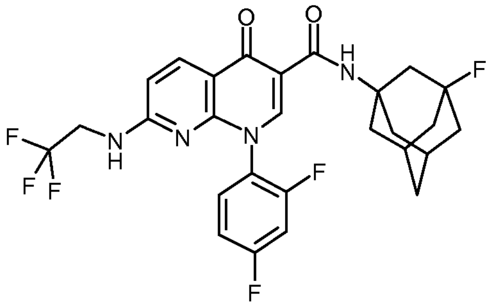 (62 % d. Th.) | LC-MS (Methode 1): $R_t$ = 1.21 min MS (ESpos): m/z = 551.2 $[M+H]^+$ $^1$H-NMR (400 MHz, CDCl3) δ [ppm]: 0.009 (2.02), 0.078 (0.26), 1.250 (0.62), 1.268 (1.29), 1.286 (0.65), 1.576 (16.00), 1.631 (0.40), 1.891 (0.69), 1.940 (0.58), 2.053 (2.80), 2.083 (0.82), 2.132 (0.59), 2.376 (1.07), 2.894 (0.75), 2.966 (0.89), 3.798 (0.25), 3.820 (0.77), 3.837 (0.87), 3.842 (0.82), 3.859 (0.79), 3.881 (0.25), 4.104 (0.18), 4.122 (0.52), 4.140 (0.53), 4.158 (0.17), 5.245 (0.48), 6.600 (1.56), 6.622 (1.58), 7.006 (0.15), 7.024 (0.24), 7.031 (0.41), 7.044 (0.67), 7.051 (0.65), 7.063 (0.74), 7.070 (0.59), 7.076 (0.40), 7.081 (0.37), 7.351 (0.32), 7.365 (0.37), 7.372 (0.42), 7.385 (0.42), 7.394 (0.30), 7.408 (0.24), 7.529 (0.15), 8.465 (1.55), 8.487 (1.50), 8.674 (2.56), 9.991 (0.80). |
| **92** | 1-(2,4-Difluorphenyl)-*N*-(3,5-difluor-tricyclo[3.3.1.1$^{3,7}$]dec-1-yl)-4-oxo-7-[(2,2,2-trifluorethyl)amino]-1,4-dihydro-1,8-naphthyridin-3-carbonsäureamid 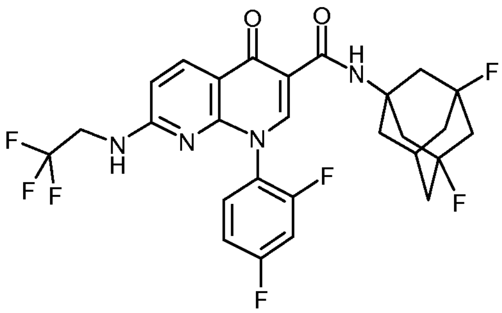 (33 % d. Th.) | LC-MS (Methode 1): $R_t$ = 1.13 min MS (ESpos): m/z = 569.3 $[M+H]^+$ $^1$H-NMR (400 MHz, DMSO-$d_6$) δ [ppm]: - 0.150 (0.90), -0.009 (7.71), 0.007 (7.47), 0.145 (0.97), 1.156 (2.58), 1.174 (5.20), 1.192 (2.65), 1.804 (9.58), 1.902 (6.37), 1.987 (9.51), 2.072 (1.78), 2.112 (1.99), 2.171 (3.19), 2.327 (3.45), 2.448 (1.58), 2.669 (1.05), 2.709 (1.19), 3.161 (15.44), 3.174 (16.00), 3.842 (1.60), 4.020 (2.24), 4.038 (2.24), 4.062 (1.58), 4.075 (4.33), 4.088 (4.28), 4.101 (1.51), 6.795 (2.89), 6.818 (3.02), 7.305 (1.19), 7.326 (2.31), 7.347 (1.31), 7.532 (1.36), 7.538 (1.51), 7.561 (2.24), 7.580 (1.53), 7.586 (1.51), 7.759 (1.41), 7.774 (1.65), 7.781 (2.82), 7.796 (2.84), 7.817 (1.41), 8.279 (6.13), 8.301 (5.84), 8.419 (1.26), 8.551 (13.67), 10.210 (6.91). |
| **93** | 1-(2,4-Difluorphenyl)-4-oxo-7-[(2,2,2-trifluorethyl)amino]-*N*[1,1,1-trifluor-propan-2-yl]-1,4-dihydro-1,8-naphthyridin-3-carbonsäureamid 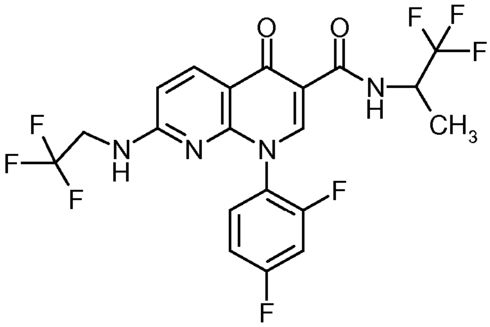 (45 % d. Th.) | LC-MS (Methode 1): $R_t$ = 1.09 min MS (ESpos): m/z = 495.1 $[M+H]^+$ $^1$H-NMR (400 MHz, $CDCl_3$) δ [ppm]: 0.001 (0.87), 0.016 (0.49), 0.017 (0.80), 1.251 (0.34), 1.269 (0.71), 1.287 (0.37), 1.451 (2.80), 1.468 (2.83), 1.566 (16.00), 2.054 (1.26), 2.630 (0.83), 2.634 (0.30), 2.637 (0.20), 3.818 (0.41), 3.827 (0.36), 3.835 (0.40), 3.842 (0.52), 3.849 (0.38), 3.857 (0.36), 3.866 (0.41), 4.122 (0.27), 4.140 (0.27), 4.918 (0.24), 4.937 (0.24), 5.265 (0.36), 6.621 (1.63), 6.643 (1.66), 7.042 (0.21), 7.049 (0.33), 7.065 (0.67), 7.068 (0.60), 7.084 (0.69), 7.093 (0.41), 7.102 (0.30), 7.259 (0.24), 7.261 (0.42), 7.276 (0.51), 7.280 (0.23), 7.382 (0.30), 7.401 (0.29), 8.479 (1.56), 8.501 (1.52), 8.722 (1.17), 10.364 (0.37), 10.388 (0.36). |

(fortgesetzt)

| Bsp. | | Analytische Daten |
|---|---|---|
| **94** | 1-(2,4-Difluorphenyl)-4-oxo-*N*-[(2*R*)-1,1,1-trifluorbutan-2-yl]-7-[(2,2,2-trifluorethyl)amino]-1,4-dihydro-1,8-naphthyridin-3-carbonsäureamid 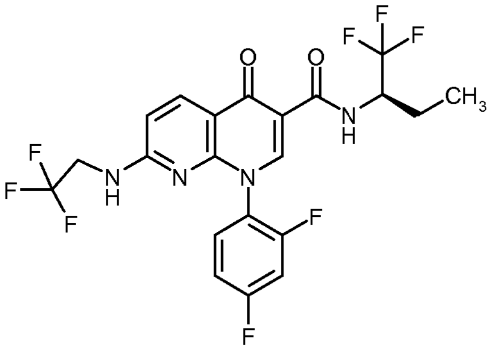 (45 % d. Th.) | LC-MS (Methode 1): $R_t$ = 1.10 min MS (ESpos): m/z = 509.2 $[M+H]^+$ $^1$H-NMR (400 MHz, DMSO-$d_6$) $\delta$ [ppm]: - 0.009 (7.83), 0.007 (6.82), 0.945 (7.20), 0.963 (16.00), 0.982 (7.83), 1.617 (1.49), 1.624 (1.37), 1.634 (1.79), 1.643 (1.63), 1.652 (1.56), 1.659 (1.75), 1.678 (1.35), 1.850 (1.25), 1.860 (1.49), 1.868 (1.53), 1.878 (1.70), 1.885 (1.49), 1.895 (1.30), 1.903 (1.13), 2.523 (2.31), 3.851 (1.98), 3.869 (1.96), 3.892 (1.42), 4.735 (1.44), 4.754 (1.37), 6.819 (3.40), 6.841 (3.45), 7.306 (1.39), 7.328 (2.81), 7.345 (1.53), 7.534 (1.56), 7.542 (1.63), 7.560 (2.64), 7.565 (2.69), 7.583 (1.68), 7.590 (1.63), 7.796 (1.86), 7.805 (1.86), 7.819 (1.84), 8.300 (7.29), 8.322 (6.91), 8.467 (1.63), 8.655 (6.11), 8.662 (5.52), 10.426 (4.88), 10.450 (4.72). |
| **95** | *rac*-1-(2,4-Difluorphenyl)-4-oxo-7-[(2,2,2-trifluorethyl)amino]-*N*-[1,1,1-trifluor-4-methylpentan-2-yl]-1,4-dihydro-1,8-naphthyridin-3-carbonsäureamid 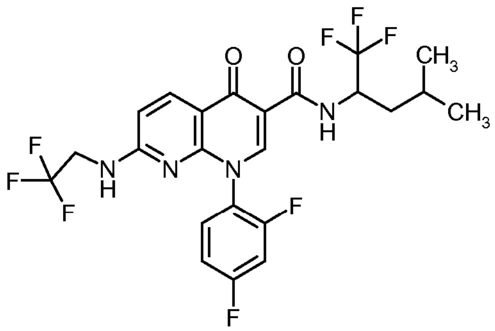 (54 % d. Th.) | LC-MS (Methode 1): $R_t$ = 1.25 min MS (ESpos): m/z = 537.1 $[M+H]^+$ $^1$H-NMR (400 MHz, $CDCl_3$) $\delta$ [ppm]: 0.002 (1.21), 0.018 (0.98), 0.957 (3.49), 0.973 (3.84), 0.984 (6.39), 1.001 (6.32), 1.251 (0.59), 1.269 (1.24), 1.287 (0.60), 1.559 (16.00), 1.609 (0.40), 1.617 (0.79), 1.625 (0.46), 1.643 (0.60), 1.708 (0.56), 1.718 (0.76), 1.736 (0.61), 1.746 (1.03), 1.752 (0.70), 1.775 (0.71), 1.797 (0.39), 2.054 (2.17), 3.818 (0.79), 3.831 (0.85), 3.835 (0.89), 3.841 (0.82), 3.848 (0.86), 3.853 (0.88), 3.857 (0.85), 3.870 (0.78), 4.123 (0.47), 4.141 (0.46), 4.892 (0.42), 4.897 (0.39), 4.917 (0.43), 5.210 (0.48), 5.226 (0.89), 5.242 (0.47), 6.619 (3.06), 6.641 (3.12), 7.044 (0.47), 7.063 (1.28), 7.082 (1.15), 7.369 (0.62), 7.383 (0.69), 7.390 (0.77), 7.404 (0.80), 7.411 (0.52), 7.426 (0.42), 8.478 (2.53), 8.500 (2.48), 8.735 (3.20), 10.235 (0.79), 10.259 (0.78). |
| **96** | 1-(2,4-Difluorphenyl)-4-oxo-7-[(2,2,2-trifluorethyl) amino]-*N*-[1-(trifluormethyl)cyclopentyl]-1,4-dihydro-1,8-naphthyridin-3-carbonsäureamid 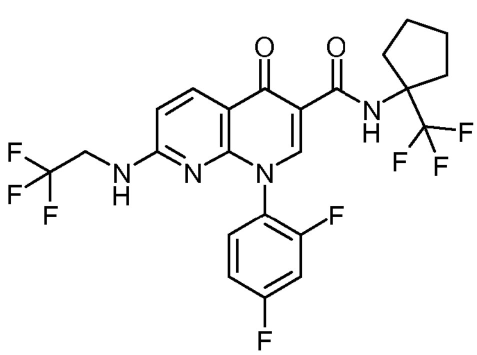 (50 % d. Th.) | LC-MS (Methode 1): $R_t$ = 1.21 min MS (ESpos): m/z = 535.1 $[M+H]^+$ $^1$H-NMR (400 MHz, DMSO-$d_6$) $\delta$ [ppm]: - 0.009 (2.42), 0.007 (1.94), 1.174 (2.34), 1.731 (3.38), 1.749 (4.42), 1.773 (4.61), 1.790 (3.12), 1.987 (4.41), 2.026 (2.74), 2.044 (3.04), 2.061 (3.19), 2.350 (1.72), 2.365 (2.67), 2.384 (2.86), 2.418 (1.36), 3.823 (1.37), 3.847 (1.84), 3.867 (1.87), 6.807 (3.27), 6.829 (3.31), 7.299 (1.38), 7.304 (1.50), 7.321 (2.76), 7.326 (2.90), 7.342 (1.55), 7.347 (1.55), 7.532 (1.76), 7.539 (1.85), 7.557 (2.72), 7.561 (2.71), 7.580 (1.80), 7.587 (1.75), 7.762 (1.74), 7.777 (2.08), 7.784 (3.42), 7.799 (3.34), 7.805 (1.86), 7.820 (1.63), 8.293 (6.84), 8.315 (6.47), 8.454 (1.57), 8.604 (16.00), 10.523 (9.58). |

(fortgesetzt)

| Bsp. | | Analytische Daten |
|---|---|---|
| **97** | 1-(2,4-Difluorphenyl)-*N*-(4-fluorbicyclo-[2.2.2]oct-1-yl)-4-oxo-7-[(2,2,2-trifluorethyl)amino]-1,4-dihydro-1,8-naphthyridin-3-carbonsäureamid 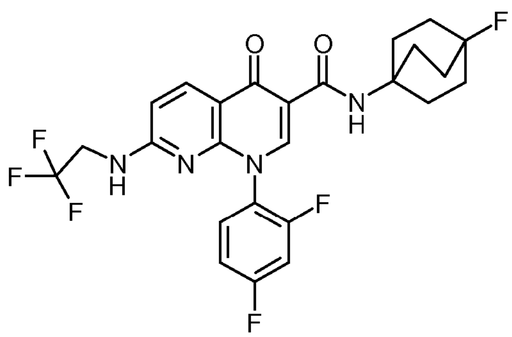 (59 % d. Th.) | LC-MS (Methode 1): $R_t$ = 1.16 min MS (ESpos): m/z = 525.1 $[M+H]^+$ $^1H$-NMR (400 MHz, $CDCl_3$) δ [ppm]: 0.010 (3.66), 0.079 (0.26), 1.233 (0.17), 1.251 (0.35), 1.269 (0.74), 1.287 (0.37), 1.566 (16.00), 1.939 (0.49), 1.953 (0.93), 1.969 (0.95), 1.980 (1.01), 1.993 (0.63), 2.054 (1.38), 2.234 (1.14), 2.246 (0.89), 2.255 (1.03), 2.274 (0.83), 3.811 (0.21), 3.816 (0.49), 3.833 (0.54), 3.838 (0.50), 3.855 (0.48), 4.123 (0.29), 4.140 (0.29), 5.205 (0.26), 6.594 (1.02), 6.616 (1.03), 7.031 (0.24), 7.045 (0.44), 7.051 (0.41), 7.055 (0.26), 7.065 (0.43), 7.070 (0.38), 7.076 (0.25), 7.082 (0.21), 7.344 (0.20), 7.358 (0.22), 7.365 (0.25), 7.378 (0.26), 7.387 (0.19), 8.454 (0.99), 8.476 (0.95), 8.657 (1.56), 9.864 (0.47). |
| **98** | 1-(2,4-Difluorphenyl)-*N*-(4-methyl-bicyclo[2.2.2] oct-1-yl)-4-oxo-7-[(2,2,2-trifluorethyl)amino]-1,4-dihydro-1,8-naphthyridin-3-carbonsäureamid 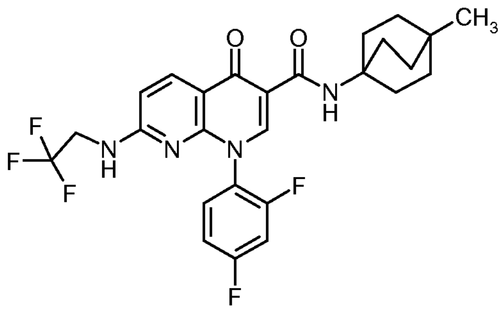 (56 % d. Th.) | LC-MS (Methode 1): $R_t$ = 1.28 min MS (ESpos): m/z = 521.2 $[M+H]^+$ $^1H$-NMR (400 MHz, $CDCl_3$) δ [ppm]: 0.001 (0.28), 0.017 (0.22), 0.816 (1.69), 1.507 (0.44), 1.527 (0.51), 1.547 (0.58), 1.564 (16.00), 2.021 (0.50), 2.041 (0.48), 2.061 (0.42), 3.816 (0.20), 3.832 (0.22), 3.838 (0.20), 3.855 (0.20), 5.174 (0.10), 6.582 (0.44), 6.604 (0.46), 7.006 (0.11), 7.023 (0.10), 7.037 (0.19), 7.044 (0.18), 7.056 (0.19), 7.062 (0.16), 7.342 (0.09), 7.363 (0.11), 7.376 (0.12), 7.399 (0.08), 7.529 (0.10), 8.464 (0.43), 8.486 (0.42), 8.674 (0.69), 9.766 (0.19). |
| **99** | *rac*-1-(2,4-Difluorphenyl)-7-[methyl(2,2,2-trifluorethyl)amino]-4-oxo-*N*-[1,1,1-trifluor-propan-2-yl]-1,4-dihydro-1,8-naphthyridin-3-carbonsäureamid 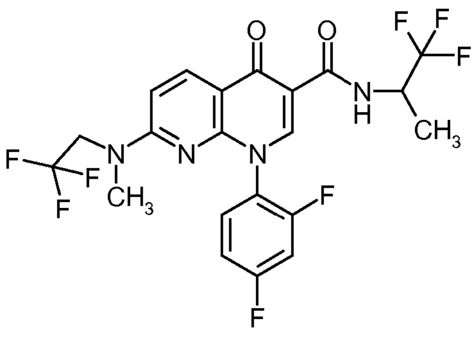 Verbindung aus Beispiel 55A und 1,1,1-Trifluorpropan-2-amin (quant.) | LC-MS (Methode 1): $R_t$ = 1.18 min MS (ESpos): m/z = 509.2 $[M+H]^+$ $^1H$-NMR (400 MHz, DMSO-$d_6$) δ [ppm]: 1.168 (1.57), 1.232 (1.49), 1.269 (1.09), 1.368 (15.88), 1.385 (16.00), 3.094 (6.92), 4.238 (0.99), 4.872 (1.17), 4.892 (1.82), 4.911 (1.83), 4.930 (1.13), 7.099 (1.62), 7.120 (1.64), 7.306 (1.51), 7.311 (1.61), 7.328 (2.88), 7.333 (3.00), 7.349 (1.64), 7.354 (1.69), 7.547 (1.45), 7.570 (2.64), 7.595 (1.43), 7.777 (1.34), 7.792 (1.49), 7.800 (1.54), 7.808 (1.57), 7.823 (1.38), 8.408 (4.61), 8.431 (4.41), 8.692 (6.39), 8.700 (5.51), 10.428 (2.96), 10.451 (2.92). |

(fortgesetzt)

| Bsp. | | Analytische Daten |
|---|---|---|
| 100 | rac-1-(2,4-Difluorphenyl)-7-[methyl(2,2,2-trifluorethyl)amino]-4-oxo-N-[1,1,1-trifluorbutan-2-yl]-1,4-dihydro-1,8-naphthyridin-3-carbonsäureamid 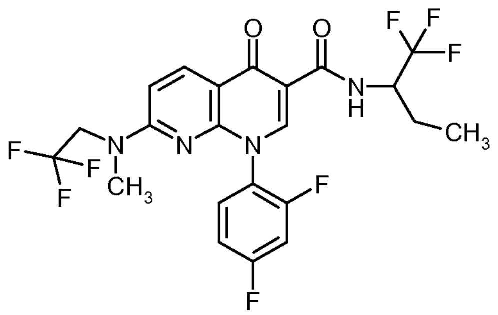 (100 % d. Th.) | LC-MS (Methode 1): Rt = 1.22 min MS (ESpos): m/z = 523.2 [M+H]+ 1H-NMR (400 MHz, DMSO-d6) δ [ppm]: - 0.009 (6.06), 0.007 (4.96), 0.950 (7.55), 0.968 (16.00), 0.987 (7.84), 1.168 (1.87), 1.233 (1.40), 1.269 (1.13), 1.604 (1.17), 1.622 (1.56), 1.630 (1.41), 1.639 (1.87), 1.648 (1.62), 1.657 (1.61), 1.665 (1.81), 1.683 (1.39), 1.854 (1.34), 1.864 (1.54), 1.873 (1.55), 1.883 (1.71), 1.889 (1.50), 1.899 (1.34), 1.908 (1.20), 1.918 (0.98), 2.327 (0.87), 2.365 (1.13), 2.523 (1.71), 2.669 (0.82), 2.709 (1.03), 3.094 (5.27), 4.232 (0.96), 4.718 (0.86), 4.742 (1.43), 4.762 (1.30), 7.102 (1.57), 7.123 (1.59), 7.312 (1.54), 7.333 (2.90), 7.349 (1.59), 7.543 (1.60), 7.550 (1.63), 7.568 (2.65), 7.592 (1.70), 7.598 (1.57), 7.803 (1.81), 7.813 (1.79), 7.827 (1.76), 8.418 (4.92), 8.440 (4.73), 8.699 (6.82), 8.707 (6.06), 10.377 (4.66), 10.401 (4.47). |

Beispiel **101**

1-(2,4-Difluorphenyl)-7-[(2-fluoroethyl)(methyl)amino]-4-oxo-*N*-(tricyclo[3.3.1.1$^{3,7}$]dec-1-yl)-1,4-dihydro-1,8-naphthyridin-3 -carbonsäureamid

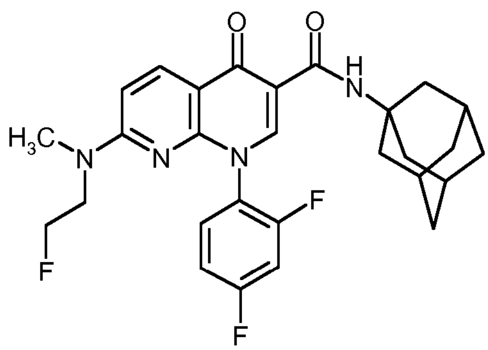

100 mg (0.27 mmol) der Verbindung aus Beispiel 42A wurden in 3 ml DMF vorgelegt, mit 121 mg (0.32 mmol) HATU sowie 110 mg (0.85 mmol) DIPEA versetzt und 30 Minuten lang bei 20°C gerührt. Dann wurden 56 mg (0.37 mmol) 1-Adamantanamin zugegeben und 2 Stunden bei 20°C gerührt. Anschliessend wurde das Gemisch über präparative HPLC gereinigt (Eluent: Acetonitril/Wasser-Gradient mit 0.1% Ameisensäure). Man erhielt 46 mg (34 % d. Th.) der Titelverbindung.

LC-MS (Methode 1): $R_t$ = 1.38 min; m/z = 511.3 $[M+H]^+$.

$^1$H-NMR (400 MHz, DMSO-$d_6$) δ [ppm]: -0.013 (1.34), 0.003 (1.24), 1.229 (0.37), 1.667 (7.41), 1.983 (0.28), 2.052 (16.00), 2.322 (0.18), 2.664 (0.20), 3.021 (0.65), 3.157 (1.10), 3.170 (1.12), 3.612 (0.28), 4.069 (0.29), 4.082 (0.29), 4.328 (0.22), 4.438 (0.22), 5.749 (0.29), 6.938 (0.82), 6.961 (0.82), 7.291 (0.38), 7.295 (0.41), 7.311 (0.76), 7.316 (0.78), 7.331 (0.42), 7.337 (0.42), 7.536 (0.48), 7.543 (0.50), 7.562 (0.74), 7.566 (0.74), 7.585 (0.49), 7.592 (0.47), 7.761 (0.47), 7.776 (0.58), 7.783 (0.93), 7.798 (0.91), 7.804 (0.52), 7.819 (0.43), 8.279 (1.06), 8.302 (1.01), 8.495 (4.56), 9.916 (2.39).

In Analogie zu Beispiel 101 wurden die in Tabelle 12 gezeigten Beispielverbindungen hergestellt, indem die Verbindung aus Beispiel 42A mit den entsprechenden Aminen (bzw. deren Salzen) unter den beschriebenen Reaktionsbedingungen umgesetzt wurden. Abweichungen sind bei den jeweiligen Beispielen angegeben.

Tabelle 12:

| Bsp. | | Analytische Daten |
|---|---|---|
| **102** | 1 -(2,4-Difluorphenyl)-7- [(2-fluorethyl)-(methyl) amino]-*N*-(3-fluortricyclo-[3.3.1.1$^{3,7}$]dec-1-yl)-4-oxo-1,4-dihydro-1,8-naphthyridin-3-carbonsäureamid 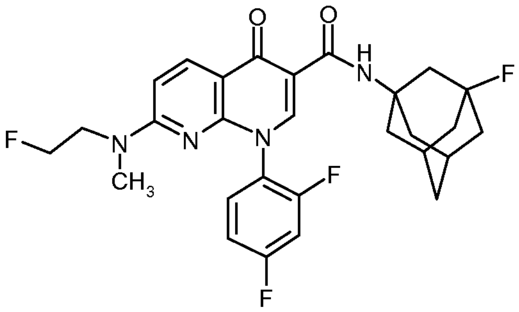 (90 % d. Th.) | LC-MS (Methode 1): $R_t$ = 1.24 min MS (ESpos): m/z = 529.3 $[M+H]^+$ $^1$H-NMR (400 MHz, CDCl3) $\delta$ [ppm]: 0.017 (0.33), 0.078 (0.23), 1.043 (1.05), 1.059 (1.12), 1.232 (0.17), 1.581 (16.00), 1.632 (0.29), 1.890 (0.44), 1.948 (0.36), 2.051 (0.26), 2.081 (0.54), 2.137 (0.36), 2.380 (0.77), 2.450 (0.14), 2.794 (0.14), 2.893 (0.23), 2.965 (0.30), 3.136 (1.37), 3.607 (0.16), 4.304 (0.19), 4.421 (0.19), 6.695 (0.88), 6.718 (0.90), 7.006 (0.36), 7.027 (0.73), 7.047 (0.71), 7.066 (0.19), 7.333 (0.20), 7.347 (0.23), 7.355 (0.27), 7.369 (0.27), 7.529 (0.15), 8.453 (0.81), 8.476 (0.78), 8.636 (1.49), 10.081 (0.46). |
| **103** | 1 -(2,4-Difluorphenyl)-7- [(2-fluorethyl)-(methyl) amino]-4-oxo-*N*-[(2*S*)-1,1,1-trifluorbutan-2-yl]-1,4-dihydro-1,8-naphthyridin-3-carbonsäureamid 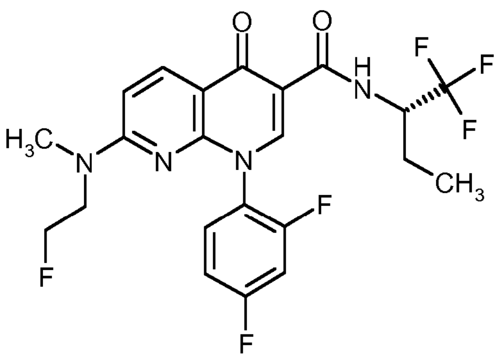 (80 % d. Th.) | LC-MS (Methode 2): $R_t$ = 2.81 min MS (ESpos): m/z = 487.0 $[M+H]^+$ $^1$H-NMR (400 MHz, DMSO-$d_6$) $\delta$ [ppm]: - 0.150 (1.09), -0.047 (1.29), -0.039 (1.70), - 0.036 (1.79), -0.034 (1.99), -0.031 (2.08), - 0.029 (2.31), -0.027 (2.43), -0.024 (2.68), - 0.022 (2.95), -0.019 (3.40), -0.017 (3.89), - 0.014 (4.70), -0.012 (5.89), -0.009 (12.50), -0.007 (10.42), 0.004 (3.50), 0.006 (2.85), 0.007 (6.27), 0.009 (1.14), 0.146 (0.86), 0.948 (7.98), 0.966 (16.00), 0.985 (7.56), 1.146 (0.53), 1.169 (1.42), 1.243 (0.90), 1.598 (1.25), 1.617 (1.59), 1.624 (1.49), 1.633 (1.96), 1.642 (1.69), 1.652 (1.62), 1.659 (1.78), 1.677 (1.33), 1.851 (1.53), 1.860 (1.67), 1.869 (1.65), 1.879 (1.82), 1.885 (1.52), 1.895 (1.39), 1.904 (1.20), 1.913 (1.01), 2.322 (0.87), 2.327 (1.15), 2.332 (0.91), 2.366 (1.73), 2.403 (0.53), 2.416 (0.72), 2.424 (0.89), 2.518 (4.50), 2.521 (4.24), 2.665 (0.61), 2.669 (0.87), 2.674 (0.60), 2.709 (1.31), 3.040 (1.83), 3.324 (1.08), 3.327 (0.74), 3.624 (1.00), 4.334 (0.70), 4.424 (0.70), 4.710 (0.98), 4.726 (1.44), 4.749 (1.41), 6.984 (2.44), 7.007 (2.46), 7.304 (1.49), 7.326 (2.78), 7.342 (1.53), 7.347 (1.53), 7.547 (1.66), 7.554 (1.78), 7.573 (2.58), 7.576 (2.63), 7.595 (1.85), 7.602 (1.70), 7.808 (1.96), 7.816 (1.71), 7.823 (1.76), 7.830 (1.89), 8.309 (3.08), 8.331 (3.03), 8.641 (5.53), 8.647 (5.09), 10.458 (4.33), 10.482 (4.24). |

(fortgesetzt)

| Bsp. | | Analytische Daten |
|---|---|---|
| **104** | *rac*-1-(2,4-Difluorphenyl)-7-[(2-fluorethyl)-(methyl) amino]-4-oxo-*N*-[1,1,1-trifluor-butan-2-yl]-1,4-dihydro-1,8-naphthyridin-3-carbonsäureamid 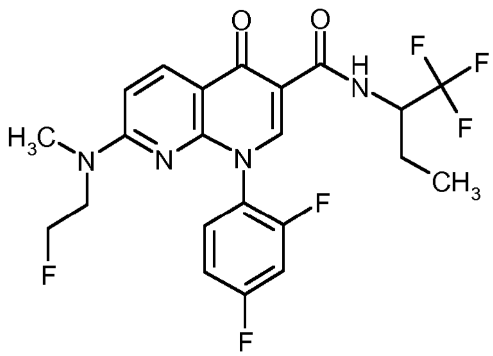 (70 % d. Th.) | LC-MS (Methode 3): $R_t$ = 2.80 min MS (ESpos): m/z = 487.1 $[M+H]^+$ $^1$H-NMR (400 MHz, DMSO-$d_6$) δ [ppm]: - 0.150 (1.38), -0.009 (11.87), 0.007 (11.12), 0.146 (1.29), 0.948 (7.35), 0.966 (16.00), 0.985 (7.63), 1.148 (0.57), 1.169 (1.25), 1.259 (0.90), 1.599 (1.06), 1.617 (1.43), 1.624 (1.37), 1.633 (1.78), 1.642 (1.54), 1.651 (1.62), 1.659 (1.74), 1.677 (1.47), 1.850 (1.25), 1.860 (1.53), 1.868 (1.43), 1.879 (1.72), 1.894 (1.21), 1.914 (1.00), 2.327 (1.59), 2.331 (1.26), 2.365 (2.15), 2.669 (1.51), 2.709 (1.91), 3.034 (1.72), 3.622 (1.03), 4.346 (0.78), 4.427 (0.72), 4.731 (1.51), 4.754 (1.38), 6.984 (2.40), 7.008 (2.40), 7.305 (1.50), 7.320 (2.79), 7.340 (1.60), 7.547 (1.68), 7.554 (1.91), 7.573 (2.75), 7.595 (1.78), 7.602 (1.60), 7.808 (1.96), 7.830 (1.90), 8.309 (3.31), 8.331 (3.10), 8.641 (6.21), 8.647 (5.57), 10.458 (4.46), 10.482 (4.09). |
| **105** | *rac*-1-(2,4-Difluorphenyl)-7-[(2-fluorethyl)-(methyl) amino]-4-oxo-*N*-[1,1,1-trifluor-propan-2-yl]-1,4-dihydro-1,8-naphthyridin-3-carbonsäureamid 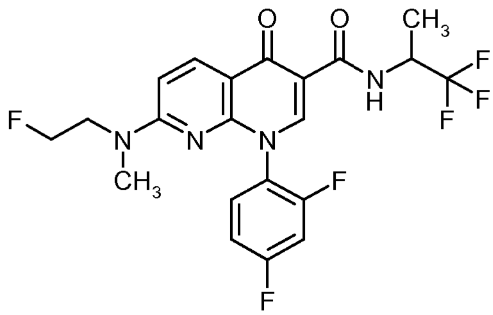 Aufarbeitung: Zugabe von Wasser / 1 M wässr. Salzsäure und Abfiltrieren des Niederschlages. (72 % d. Th.) | LC-MS (Methode 2): $R_t$ = 2.71 min MS (ESpos): m/z = 473.1 $[M+H]^+$ $^1$H-NMR (400 MHz, DMSO-$d_6$) δ [ppm]: - 0.150 (0.75), -0.009 (9.82), 0.007 (6.09), 0.146 (0.75), 1.169 (1.04), 1.242 (1.12), 1.259 (1.01), 1.272 (0.81), 1.322 (0.61), 1.339 (0.97), 1.363 (16.00), 1.381 (15.67), 2.322 (0.61), 2.327 (0.85), 2.331 (0.61), 2.365 (1.09), 2.665 (0.64), 2.669 (0.83), 2.674 (0.59), 2.709 (1.06), 2.890 (0.61), 3.037 (1.81), 3.632 (0.98), 4.338 (0.71), 4.435 (0.72), 4.863 (1.19), 4.883 (1.81), 4.904 (1.75), 4.923 (1.05), 6.982 (2.40), 7.005 (2.44), 7.300 (1.48), 7.304 (1.57), 7.321 (2.73), 7.326 (2.74), 7.343 (1.50), 7.347 (1.52), 7.547 (1.47), 7.554 (1.57), 7.573 (2.53), 7.595 (1.57), 7.602 (1.39), 7.781 (1.24), 7.796 (1.44), 7.804 (1.47), 7.812 (1.50), 7.827 (1.27), 8.300 (3.07), 8.323 (2.86), 8.634 (5.56), 8.639 (4.62), 10.509 (2.89), 10.532 (2.72). |

(fortgesetzt)

| Bsp. | | Analytische Daten |
|---|---|---|
| **106** | 1 -(2,4-Difluorphenyl)-7- [(2-fluorethyl)-(methyl) amino]-4-oxo-*N*-[1,1,1-trifluor-pentan-2-yl]-1,4-dihydro-1,8-naphthyridin-3-carbonsäureamid 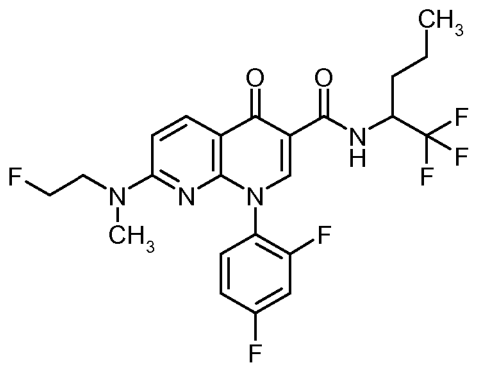 Aufarbeitung: Zugabe von Wasser / 1 M wässr. Salzsäure und Abfiltrieren des Niederschlages. (83 % d. Th.) | LC-MS (Methode 2): $R_t$ = 2.93 min MS (ESpos): m/z = 501.1 $[M+H]^+$ $^1$H-NMR (400 MHz, DMSO-$d_6$) δ [ppm]: - 0.009 (3.94), 0.007 (3.37), 0.881 (0.92), 0.893 (7.30), 0.912 (16.00), 0.930 (8.22), 1.168 (1.40), 1.243 (1.28), 1.259 (1.19), 1.273 (1.01), 1.298 (0.80), 1.316 (1.26), 1.322 (1.31), 1.338 (1.79), 1.353 (1.95), 1.371 (1.81), 1.389 (1.27), 1.409 (1.09), 1.430 (1.25), 1.442 (1.60), 1.462 (1.43), 1.594 (0.71), 1.606 (0.76), 1.621 (1.05), 1.628 (1.78), 1.640 (1.35), 1.654 (1.83), 1.667 (1.25), 1.677 (0.98), 1.690 (0.77), 1.739 (1.00), 1.748 (1.15), 1.765 (1.62), 1.772 (1.74), 1.780 (1.38), 1.790 (1.48), 1.796 (1.09), 2.523 (0.94), 2.890 (0.90), 3.040 (1.77), 3.616 (0.94), 3.626 (0.96), 4.324 (0.71), 4.443 (0.69), 4.773 (0.86), 4.792 (1.43), 4.816 (1.45), 4.834 (0.81), 6.983 (2.47), 7.006 (2.53), 7.298 (1.43), 7.302 (1.51), 7.319 (2.85), 7.324 (2.87), 7.340 (1.55), 7.345 (1.59), 7.545 (1.73), 7.551 (1.83), 7.570 (2.71), 7.574 (2.69), 7.593 (1.86), 7.600 (1.73), 7.790 (1.08), 7.810 (2.21), 7.828 (2.23), 7.847 (0.94), 8.305 (3.19), 8.327 (3.09), 8.641 (6.69), 10.453 (4.58), 10.477 (4.39). |
| **107** | *rac*-1-(2,4-Difluorphenyl)-7-[(2-fluorethyl)-(methyl) amino]-4-oxo-*N*-[1,1,1-trifluor-4-methylpentan-2-yl]-1,4-dihydro-1,8-naphthyridin-3-carbonsäureamid 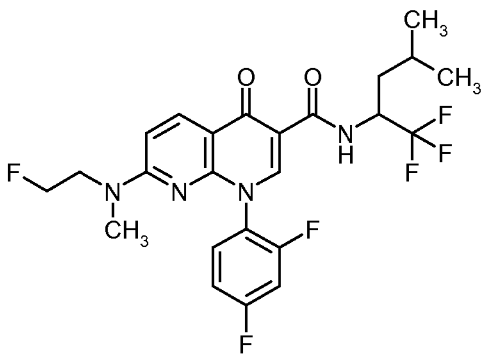 Aufarbeitung: Zugabe von Wasser / 1 M wässr. Salzsäure und Abfiltrieren des Niederschlages. (80 % d. Th.) | LC-MS (Methode 3): $R_t$ = 3.04 min MS (ESpos): m/z = 515.1 $[M+H]^+$ $^1$H-NMR (400 MHz, DMSO-$d_6$) δ [ppm]: - 0.009 (6.42), 0.007 (4.09), 0.883 (13.44), 0.895 (14.22), 0.941 (15.51), 0.956 (16.00), 1.168 (2.55), 1.562 (3.80), 1.589 (3.13), 1.644 (4.16), 1.671 (5.56), 1.698 (2.43), 2.365 (0.82), 2.669 (0.70), 2.709 (0.89), 3.040 (2.45), 3.617 (1.28), 4.438 (0.95), 4.816 (1.94), 6.982 (3.12), 7.005 (3.15), 7.301 (2.00), 7.322 (3.69), 7.338 (1.98), 7.542 (2.15), 7.549 (2.23), 7.568 (3.47), 7.591 (2.21), 7.597 (2.11), 7.794 (1.99), 7.816 (3.88), 7.831 (3.84), 7.853 (1.76), 8.302 (4.18), 8.324 (4.00), 8.648 (13.72), 10.450 (5.71), 10.474 (5.53). |

(fortgesetzt)

| Bsp. | | Analytische Daten |
|---|---|---|
| **108** | 1 -(2,4-Difluorphenyl)-7- [(2-fluorethyl)-(methyl) amino]-4-oxo-*N*-[1,1,1-trifluor-3-methylbutan-2-yl]-1,4-dihydro-1,8-naphthyridin-3-carbonsäureamid 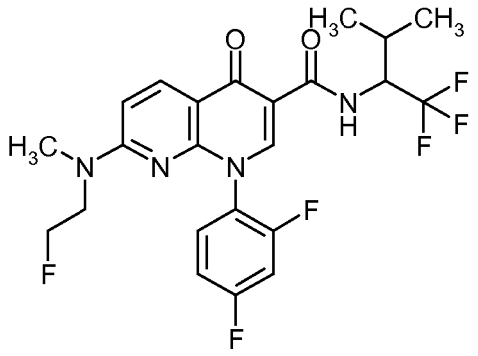 Aufarbeitung: Zugabe von Wasser / 1 M wässr. Salzsäure und Abfiltrieren des Niederschlages. (99 % d. Th.) | LC-MS (Methode 1): $R_t$ = 1.22 min MS (ESpos): m/z = 501.3 $[M+H]^+$ $^1$H-NMR (400 MHz, CDCl3) δ [ppm]: - 0.000 (0.10), 0.016 (0.10), 0.077 (0.05), 1.061 (0.15), 1.079 (0.15), 1.136 (0.13), 1.153 (0.13), 1.587 (16.00), 2.285 (0.01), 2.302 (0.02), 2.312 (0.02), 2.329 (0.01), 2.450 (0.03), 2.794 (0.03), 2.892 (0.13), 2.965 (0.16), 3.144 (0.11), 3.612 (0.01), 3.641 (0.01), 4.309 (0.02), 4.419 (0.02), 4.783 (0.02), 4.806 (0.01), 6.711 (0.09), 6.734 (0.09), 7.006 (0.04), 7.022 (0.02), 7.043 (0.05), 7.063 (0.05), 7.085 (0.01), 7.352 (0.02), 7.366 (0.02), 7.374 (0.03), 7.387 (0.03), 7.394 (0.02), 7.409 (0.01), 7.529 (0.04), 8.025 (0.02), 8.490 (0.08), 8.513 (0.07), 8.697 (0.13), 10.550 (0.02), 10.575 (0.02). |
| **109** | 1 -(2,4-Difluorphenyl)-7- [(2-fluorethyl)-(methyl) amino]-4-oxo-*N*-[1-(trifluormethyl)cyclopropyl]-1,4-dihydro-1,8-naphthyridin-3-carbonsäureamid 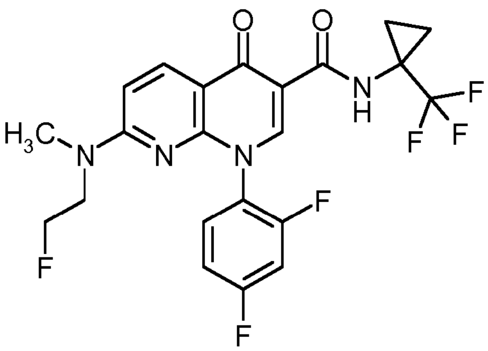 Aufarbeitung: Zugabe von Wasser / 1 M wässr. Salzsäure und Abfiltrieren des Niederschlages. (75 % d. Th.) | LC-MS (Methode 1): $R_t$ = 1.11 min MS (ESpos): m/z = 485.3 $[M+H]^+$ $^1$H-NMR (400 MHz, $CDCl_3$) δ [ppm]: 0.001 (0.18), 0.017 (0.17), 0.078 (0.11), 1.223 (0.13), 1.237 (0.14), 1.267 (0.05), 1.355 (0.04), 1.371 (0.06), 1.392 (0.32), 1.417 (0.04), 1.573 (16.00), 2.449 (0.06), 2.794 (0.06), 2.893 (0.04), 2.965 (0.05), 3.140 (0.36), 3.607 (0.05), 3.620 (0.05), 3.633 (0.05), 4.307 (0.06), 4.419 (0.06), 6.705 (0.26), 6.728 (0.27), 7.006 (0.08), 7.016 (0.07), 7.030 (0.07), 7.039 (0.18), 7.048 (0.04), 7.059 (0.19), 7.080 (0.06), 7.339 (0.06), 7.353 (0.07), 7.361 (0.09), 7.375 (0.09), 7.528 (0.06), 8.449 (0.23), 8.472 (0.22), 8.673 (0.43), 10.596 (0.15). |
| **110** | 1-(2,4-Difluorphenyl)-*N*-(4-fluorbicyclo-[2.2.2]oct-1-yl)-7-[(2-fluorethyl)(methyl)-amino]-4-oxo-1,4-dihydro-1,8-naphthyridin-3-carbonsäureamid 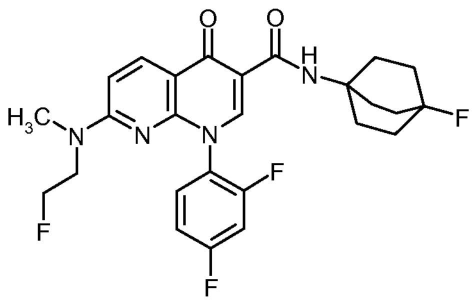 Aufarbeitung: Zugabe von Wasser / 1 M wässr. Salzsäure und Abfiltrieren des Niederschlages. (88 % d. Th.) | LC-MS (Methode 1): $R_t$ = 1.18 min MS (ESpos): m/z = 503.3 $[M+H]^+$ $^1$H-NMR (400 MHz, CDCl3) δ [ppm]: 0.000 (0.20), 0.017 (0.21), 0.078 (0.12), 1.041 (0.05), 1.576 (16.00), 1.937 (0.17), 1.952 (0.31), 1.967 (0.31), 1.978 (0.34), 1.992 (0.22), 2.235 (0.38), 2.246 (0.30), 2.257 (0.35), 2.276 (0.29), 2.450 (0.07), 2.602 (0.03), 2.635 (0.12), 2.639 (0.07), 2.641 (0.05), 2.644 (0.05), 2.794 (0.06), 2.965 (0.03), 3.132 (0.41), 3.565 (0.04), 3.602 (0.05), 3.613 (0.05), 3.627 (0.04), 3.667 (0.04), 4.298 (0.06), 4.415 (0.06), 6.690 (0.33), 6.713 (0.33), 6.998 (0.05), 7.006 (0.14), 7.018 (0.07), 7.026 (0.24), 7.034 (0.05), 7.046 (0.25), 7.053 (0.08), 7.066 (0.06), 7.075 (0.04), 7.256 (0.06), 7.260 (0.13), 7.281 (0.08), 7.290 (0.04), 7.294 (0.03), 7.325 (0.07), 7.339 (0.08), 7.347 (0.10), 7.361 (0.09), 7.382 (0.05), 7.529 (0.08), 8.439 (0.32), 8.462 (0.30), 8.618 (0.60), 9.956 (0.16). |

(fortgesetzt)

| Bsp. | | Analytische Daten |
|---|---|---|
| **111** | 1 -(2,4-Difluorphenyl)-7- [(2-fluorethyl)-(methyl) amino]-*N*-(1,1,1,3,3,3-hexafluorpropan-2-yl)-4-oxo-1,4-dihydro-1,8-naphthyridin-3-carbonsäureamid 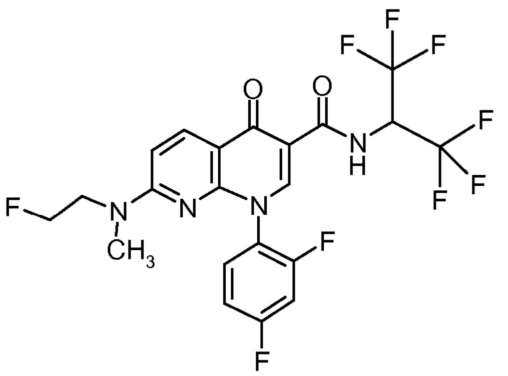 (17 % d. Th.) | LC-MS (Methode 1): $R_t$ = 1.23 min MS (ESpos): m/z = 527.1 $[M+H]^+$ $^1$H-NMR (400 MHz, $CDCl_3$) $\delta$ [ppm]: 0.002 (0.57), 0.018 (0.49), 0.079 (0.13), 1.557 (16.00), 2.450 (0.09), 2.794 (0.09), 3.151 (0.37), 3.613 (0.07), 3.648 (0.07), 4.305 (0.07), 4.432 (0.06), 5.309 (0.16), 5.568 (0.07), 5.586 (0.08), 5.593 (0.07), 5.603 (0.06), 5.611 (0.09), 5.628 (0.07), 6.733 (0.46), 6.756 (0.47), 7.006 (0.13), 7.032 (0.08), 7.039 (0.11), 7.053 (0.09), 7.060 (0.30), 7.081 (0.32), 7.088 (0.10), 7.100 (0.08), 7.357 (0.09), 7.372 (0.11), 7.379 (0.13), 7.394 (0.13), 7.529 (0.14), 8.481 (0.36), 8.503 (0.35), 8.689 (0.72), 11.267 (0.13), 11.293 (0.12). |

**Beispiel 112**

7-[(2,2-Difluorethyl)(methyl)amino]-1-(2,4-difluorphenyl)-4-oxo-*N*(tricyclo[3.3.1.1$^{3,7}$]dec-1-yl)-1,4-dihydro-1,8-naphthyridin-3 -carbonsäureamid

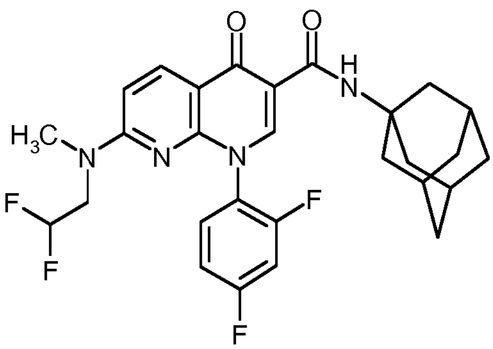

278 mg (0.7 mmol) der Verbindung aus Beispiel 48A wurden in 7.9 ml DMF vorgelegt, mit 321 mg (0.84 mmol) HATU sowie 291 mg (2.3 mmol) DIPEA versetzt und 30 Minuten lang bei 20°C gerührt. Dann wurden 149 mg (1.0 mmol) 1-Adamantanamin zugegeben und 1 Stunde bei 20°C gerührt. Anschliessend wurde 1 ml 1 M wässr. Salzsäure zugegeben und das Gemisch über präparative HPLC gereinigt (Eluent: Acetonitril/Wasser-Gradient mit 0.1% Ameisensäure). Man erhielt 65 mg (18 % d. Th.) der Titelverbindung.
LC-MS (Methode 1): $R_t$ = 1.35 min; m/z = 529.2 $[M+H]^+$.
$^1$H-NMR (400 MHz, DMSO-$d_6$) $\delta$ [ppm]: 1.669 (7.89), 1.946 (0.40), 1.983 (0.31), 2.055 (16.00), 3.077 (0.90), 3.157 (2.88), 3.170 (2.96), 3.725 (0.36), 4.056 (0.31), 4.069 (0.86), 4.082 (0.84), 4.095 (0.30), 6.993 (0.69), 7.016 (0.72), 7.297 (0.40), 7.319 (0.79), 7.336 (0.55), 7.540 (0.45), 7.547 (0.47), 7.569 (0.76), 7.589 (0.47), 7.595 (0.45), 7.770 (0.42), 7.786 (0.53), 7.792 (0.85), 7.807 (0.85), 7.814 (0.51), 7.829 (0.39), 8.339 (1.00), 8.361 (0.98), 8.531 (3.55), 9.880 (2.28).

In Analogie zu Beispiel 112 wurden die in Tabelle 13 gezeigten Beispielverbindungen hergestellt, indem die Verbindung aus Beispiel 48A mit den entsprechenden Aminen (bzw. deren Salzen) unter den beschriebenen Reaktionsbedingungen umgesetzt wurden. Abweichungen sind bei den jeweiligen Beispielen angegeben.

Tabelle 13:

| Bsp. | | Analytische Daten |
|---|---|---|
| **113** | *rac*-7-[(2,2-Difluorethyl)(methyl)amino]-1-(2,4-difluorphenyl)-4-oxo-*N*-[1,1,1-trifluor-propan-2-yl]-1,4-dihydro-1,8-naphthyridin-3-carbonsäureamid 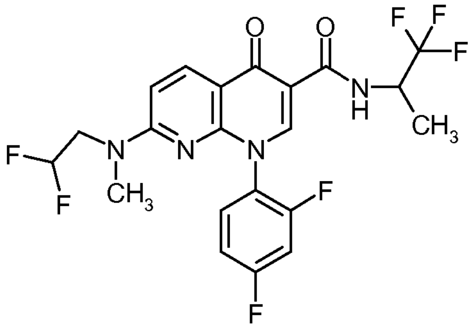 (6 % d. Th.) | LC-MS (Methode 1): $R_t$ = 1.15 min MS (ESpos): m/z = 491.3 [M+H] $^1$H-NMR (500 MHz, DMSO-$d_6$) $\delta$ [ppm]: - 0.120 (1.57), -0.007 (16.00), 0.006 (12.71), 0.116 (1.53), 1.235 (0.81), 1.368 (14.07), 1.382 (14.02), 2.361 (1.27), 2.635 (1.20), 3.101 (1.58), 3.725 (0.75), 4.890 (1.59), 4.907 (1.60), 5.753 (2.66), 7.038 (1.51), 7.311 (1.38), 7.328 (2.64), 7.340 (1.37), 7.559 (1.33), 7.577 (2.44), 7.597 (1.28), 7.790 (1.10), 7.802 (1.56), 7.820 (1.63), 7.833 (1.19), 8.362 (2.37), 8.380 (2.31), 8.672 (5.13), 8.679 (4.44), 10.466 (2.23), 10.483 (2.24). |
| **114** | *rac*-7-[(2,2-Difluorethyl)(methyl)amino]-1-(2,4-difluorphenyl)-4-oxo-*N*-[1,1,1-trifluor-butan-2-yl]-1,4-dihydro-1,8-naphthyridin-3-carbonsäureamid 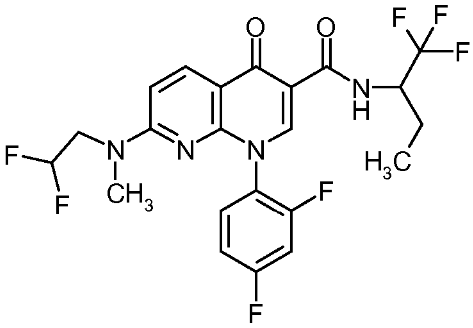 (92 % d. Th.) | LC-MS (Methode 1): $R_t$ = 1.19 min MS (ESpos): m/z = 505.3 $[M+H]^+$ $^1$H-NMR (400 MHz, DMSO-$d_6$) $\delta$ [ppm]: - 0.009 (9.63), 0.007 (5.71), 0.949 (7.95), 0.968 (16.00), 0.986 (7.83), 1.168 (1.61), 1.233 (1.29), 1.602 (1.28), 1.620 (1.68), 1.627 (1.58), 1.637 (1.99), 1.645 (1.79), 1.655 (1.61), 1.662 (1.90), 1.680 (1.38), 1.853 (1.46), 1.862 (1.53), 1.871 (1.72), 1.881 (1.90), 1.897 (1.44), 1.907 (1.21), 1.916 (1.03), 2.327 (1.14), 2.365 (1.46), 2.669 (0.91), 2.709 (1.20), 2.730 (1.72), 2.890 (2.25), 3.091 (5.58), 3.217 (0.83), 3.709 (1.38), 4.739 (1.64), 4.754 (1.48), 7.038 (2.31), 7.060 (2.27), 7.307 (1.80), 7.328 (3.19), 7.344 (1.75), 7.550 (1.92), 7.556 (2.06), 7.576 (3.05), 7.598 (1.88), 7.605 (1.79), 7.792 (1.12), 7.814 (2.41), 7.837 (2.18), 8.368 (3.55), 8.390 (3.37), 8.680 (6.43), 8.686 (5.75), 10.412 (4.34), 10.436 (4.22). |
| **115** | 7-[(2,2-Difluorethyl)(methyl)amino]-1-(2,4-difluorphenyl)-*N*-(1,1,1,3,3,3-hexafluorpropan-2-yl)-4-oxo-1,4-dihydro-1,8-naphthyridin-3-carbonsäureamid 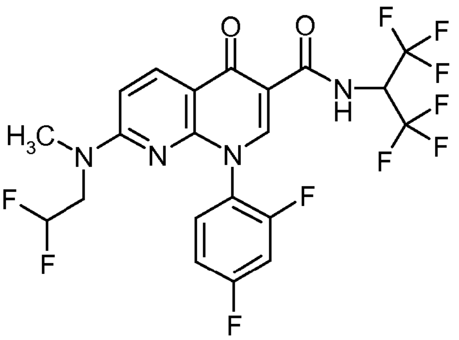 (17 % d. Th.) | LC-MS (Methode 1): $R_t$ = 1.24 min MS (ESpos): m/z = 545.0 $[M+H]^+$ $^1$H-NMR (400 MHz, $CDCl_3$) $\delta$ [ppm]: 1.269 (0.10), 1.557 (16.00), 2.054 (0.15), 3.190 (1.17), 3.597 (0.09), 3.667 (0.09), 5.611 (0.17), 6.766 (0.51), 6.789 (0.52), 7.065 (0.14), 7.083 (0.36), 7.102 (0.33), 7.120 (0.12), 7.350 (0.12), 7.371 (0.15), 7.385 (0.17), 8.536 (0.49), 8.558 (0.47), 8.723 (0.89), 11.191 (0.17), 11.215 (0.18). |

**Beispiel 116**

1-(2,4-Difluorphenyl)-7-[4-hydroxy-4-(hydroxymethyl)piperidin-1-yl]-4-oxo-*N*-(tricyclo[3.3.1.1$^{3,7}$]dec-1-yl)-1,4-dihydro-1,8-naphthyridin-3-carbonsäureamid

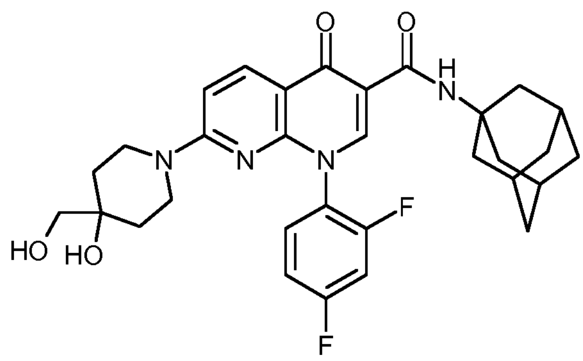

30 mg (0.07 mmol) der Verbindung aus Beispiel 58A wurden in 0.8 ml DMF vorgelegt, mit 32 mg (0.08 mmol) HATU sowie 29 mg (0.22 mmol) DIPEA versetzt und 30 Minuten lang bei 20°C gerührt. Dann wurden 15 mg (0.1 mmol) 1-Adamantanamin zugegeben und 2 Stunden bei 20°C gerührt. Anschliessend wurde das Gemisch über präparative HPLC gereinigt (Eluent: Acetonitril/Wasser-Gradient mit 0.1% Ameisensäure). Man erhielt 6 mg (15 % d. Th.) der Titelverbindung.
LC-MS (Methode 1): $R_t$ = 1.14 min; m/z = 565.2 $[M+H]^+$.
$^1$H-NMR (400 MHz, DMSO-$d_6$) $\delta$ [ppm]: -0.150 (0.51), -0.009 (4.42), 0.007 (3.91), 0.146 (0.50), 1.146 (0.38), 1.234 (0.81), 1.322 (0.81), 1.450 (0.78), 1.670 (7.49), 2.055 (16.00), 2.327 (0.82), 2.365 (1.03), 2.669 (0.87), 2.709 (1.05), 3.083 (0.65), 3.136 (2.56), 3.150 (2.63), 3.899 (0.89), 4.287 (2.59), 4.550 (1.22), 5.753 (5.06), 7.062 (1.93), 7.085 (1.95), 7.302 (0.43), 7.324 (0.82), 7.348 (0.44), 7.553 (0.53), 7.576 (0.76), 7.595 (0.52), 7.602 (0.53), 7.760 (0.50), 7.781 (1.00), 7.796 (1.00), 7.818 (0.47), 8.225 (2.67), 8.248 (2.48), 8.468 (5.33), 9.938 (2.87).

**Beispiel 117**

1-(2,4-Difluorphenyl)-7-[(3*R*)-3-(hydroxymethyl)morpholin-4-yl]-4-oxo-*N*-(tricyclo[3.3.1.1$^{3,7}$]dec-1-yl)-1,4-dihydro-1,8-naphthyridin-3-carbonsäureamid

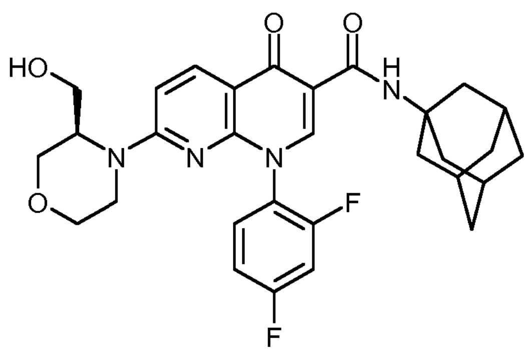

117 mg (0.28 mmol) der Verbindung aus Beispiel 53A wurden in 3.2 ml DMF vorgelegt, mit 128 mg (0.34 mmol) HATU sowie 116 mg (0.9 mmol) DIPEA versetzt und 30 Minuten lang bei 20°C gerührt. Dann wurden 60 mg (0.4 mmol) 1-Adamantanamin zugegeben und 2 Stunden bei 20°C gerührt. Anschliessend wurde 1 ml 1 M wässr. Salzsäure zugegeben und das Gemisch über präparative HPLC gereinigt (Eluent: Acetonitril/Wasser-Gradient mit 0.1% Ameisensäure). Man erhielt 24 mg (15 % d. Th.) der Titelverbindung.
LC-MS (Methode 1): $R_t$ = 1.18 min; m/z = 551.2 $[M+H]^+$.

$^1$H-NMR (400 MHz, $CDCl_3$) δ [ppm]: 0.001 (0.73), 0.017 (0.73), 1.233 (2.05), 1.430 (0.33), 1.562 (16.00), 1.693 (0.38), 1.724 (1.25), 1.745 (1.30), 1.775 (0.35), 2.120 (1.10), 2.180 (3.88), 3.262 (0.20), 3.537 (0.28), 3.564 (0.26), 3.594 (0.26), 3.780 (0.43), 3.949 (0.23), 4.028 (0.38), 4.059 (0.53), 5.309 (0.70), 6.568 (0.24), 6.764 (0.32), 6.787 (0.33), 7.006 (0.26), 7.040 (0.58), 7.060 (0.66), 7.078 (0.27), 7.375 (0.24), 7.529 (0.23), 8.470 (1.03), 8.493 (0.99), 8.650 (0.95), 9.856 (0.49).

## Beispiel 118

*rac*-1-(2,4-Difluorphenyl)-7-[2-(hydroxymethyl)morpholin-4-yl]-4-oxo-*N*-(tricyclo[3.3.1.1$^{3,7}$]dec-1-yl)-1,4-dihydro-1,8-naphthyridin-3-carbonsäureamid

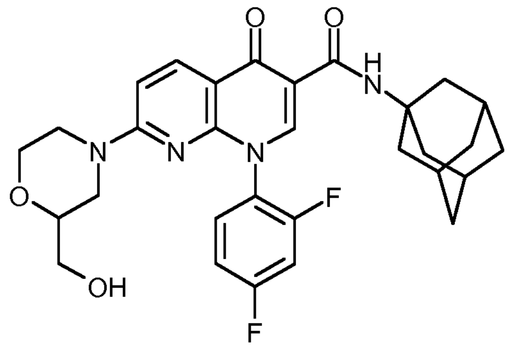

60 mg (0.14 mmol) der Verbindung aus Beispiel 51A wurden in1.6 ml DMF vorgelegt, mit 66 mg (0.17 mmol) HATU sowie 60 mg (0.46 mmol) DIPEA versetzt und 30 Minuten lang bei 20°C gerührt. Dann wurden 30 mg (0.2 mmol) 1-Adamantanamin zugegeben und 2 Stunden bei 20°C gerührt. Anschliessend wurde 1 ml 1 M wässr. Salzsäure zugegeben und das Gemisch über präparative HPLC gereinigt (Eluent: Acetonitril/Wasser-Gradient mit 0.1% Ameisensäure). Man erhielt 28 mg (35 % d. Th.) der Titelverbindung.
LC-MS (Methode 1): $R_t$ = 1.18 min; m/z = 551.2 $[M+H]^+$.
$^1$H-NMR (400 MHz, $CDCl_3$) δ [ppm]: -0.139 (0.05), 0.002 (0.46), 0.018 (0.36), 0.079 (0.09), 0.156 (0.04), 1.233 (0.14), 1.251 (0.34), 1.269 (0.70), 1.287 (0.34), 1.560 (16.00), 1.693 (0.12), 1.724 (0.41), 1.745 (0.42), 1.776 (0.12), 1.830 (0.08), 2.054 (1.26), 2.121 (0.37), 2.181 (1.27), 2.450 (0.04), 2.865 (0.05), 2.886 (0.05), 3.003 (0.05), 3.037 (0.05), 3.568 (0.12), 3.580 (0.12), 3.595 (0.15), 3.609 (0.15), 3.624 (0.10), 3.651 (0.07), 3.907 (0.10), 3.941 (0.14), 3.957 (0.14), 3.986 (0.13), 4.105 (0.10), 4.123 (0.29), 4.140 (0.28), 4.159 (0.09), 6.746 (0.31), 6.769 (0.32), 7.006 (0.14), 7.031 (0.21), 7.051 (0.21), 7.069 (0.07), 7.349 (0.08), 7.364 (0.08), 7.371 (0.11), 7.384 (0.10), 7.392 (0.06), 7.406 (0.05), 7.529 (0.10), 8.469 (0.34), 8.492 (0.33), 8.659 (0.60), 9.865 (0.20).

## Beispiel 119

*rac*-1-(2,4-Difluorphenyl)-7-(3-hydroxy-3-methylpiperidin-1-yl)-4-oxo-*N*-(tricyclo[3.3.1.1$^{3,7}$]dec-1-yl)-1,4-dihydro-1,8-naphthyridin-3-carbonsäureamid

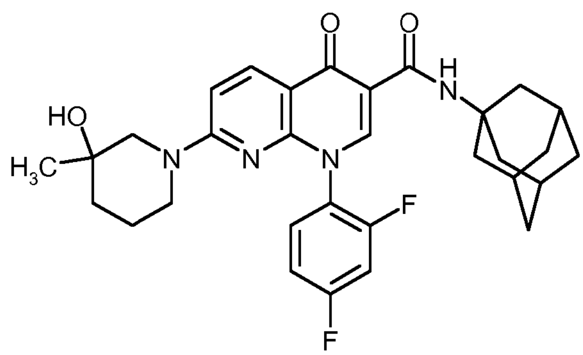

100 mg (0.23 mmol) der Verbindung aus Beispiel 54A wurden in 2.6 ml DMF vorgelegt, mit 103 mg (0.3 mmol) HATU sowie 94 mg (0.73 mmol) DIPEA versetzt und 30 Minuten lang bei 20°C gerührt. Dann wurden 48 mg (0.32 mmol) 1-Adamantanamin zugegeben und 2 Stunden bei 20°C gerührt. Anschliessend wurde mit 1 M wässr. Salzsäure auf pH 7 eingestellt und das Gemisch über präparative HPLC gereinigt (Eluent: Acetonitril/Wasser-Gradient mit 0.1% Ameisensäure). Man erhielt 110 mg (88 % d. Th.) der Titelverbindung.
LC-MS (Methode 1): $R_t$ = 1.28 min; m/z = 549.4 $[M+H]^+$.
$^1$H-NMR (400 MHz, DMSO-$d_6$) δ [ppm]: -0.009 (1.67), 0.007 (1.02), 0.992 (1.31), 1.017 (1.70), 1.086 (0.32), 1.094 (0.32), 1.104 (0.31), 1.146 (0.33), 1.168 (1.13), 1.236 (0.84), 1.269 (0.74), 1.521 (1.16), 1.584 (0.75), 1.615 (0.51), 1.669 (7.59), 1.746 (0.53), 1.826 (0.43), 2.053 (16.00), 2.366 (0.23), 2.523 (0.75), 2.689 (0.26), 2.709 (0.23), 2.730 (1.08), 2.890 (1.46), 3.085 (0.20), 3.150 (0.25), 3.228 (0.50), 3.263 (0.70), 3.407 (0.23), 3.615 (0.34), 4.366 (0.89), 4.379 (1.15), 7.021 (1.80), 7.044 (1.80), 7.305 (0.43), 7.324 (0.76), 7.343 (0.41), 7.547 (0.47), 7.554 (0.49), 7.572 (0.73), 7.595 (0.44), 7.602 (0.39), 7.756 (0.31), 7.772 (0.59), 7.793 (0.57), 8.185 (2.02), 8.208 (1.86), 8.453 (4.93), 8.464 (0.25), 9.935 (0.21), 9.957 (2.41).

## Beispiel 120

7-[(2,2-Difluorethyl)amino]-1-(2,4-difluorphenyl)-4-oxo-*N*-(tricyclo[3.3.1.1$^{3,7}$]dec-1-yl)-1,4-dihydro-1,8-naphthyridin-3-carbonsäureamid

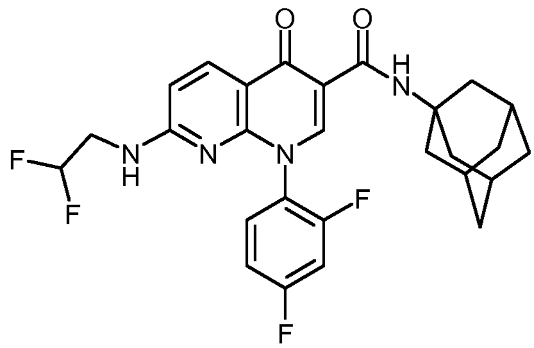

130 mg (0.23 mmol, Reinheit 67%) der Verbindung aus Beispiel 49A wurden in 2.6 ml DMF vorgelegt, mit 105 mg (0.28 mmol) HATU sowie 95 mg (0.73 mmol) DIPEA versetzt und 30 Minuten lang bei 20°C gerührt. Dann wurden 49 mg (0.32 mmol) 1-Adamantanamin zugegeben und 2 Stunden bei 20°C gerührt. Anschliessend wurde 1 ml 1 M wässr. Salzsäure zugegeben und das Gemisch über präparative HPLC gereinigt (Eluent: Acetonitril/Wasser-Gradient mit 0.1% Ameisensäure). Man erhielt 99 mg (84 % d. Th.) der Titelverbindung.
LC-MS (Methode 1): $R_t$ = 1.28 min; m/z = 515.3 $[M+H]^+$.
$^1$H-NMR (400 MHz, $CDCl_3$) δ [ppm]: 0.002 (1.20), 0.018 (1.39), 0.921 (0.24), 0.935 (0.24), 1.557 (16.00), 1.694 (0.36), 1.725 (1.30), 1.746 (1.36), 1.778 (0.36), 2.054 (0.23), 2.122 (1.14), 2.182 (4.00), 3.495 (1.71), 3.507 (1.69), 3.529 (0.20), 3.540 (0.21), 5.195 (0.31), 5.542 (0.21), 5.672 (0.22), 5.683 (0.42), 5.693 (0.20), 5.824 (0.20), 6.561 (1.16), 6.583 (1.18), 7.006 (0.25), 7.033 (0.30), 7.048 (0.53), 7.053 (0.54), 7.067 (0.57), 7.072 (0.46), 7.085 (0.26), 7.259 (0.44), 7.342 (0.24), 7.356 (0.27), 7.363 (0.33), 7.376 (0.36), 7.385 (0.24), 7.398 (0.20), 7.529 (0.25), 8.441 (1.07), 8.463 (1.04), 8.678 (1.99), 9.832 (0.56).

In Analogie zu Beispiel 120 wurden die in Tabelle 14 gezeigten Beispielverbindungen hergestellt, indem die Verbindung aus Beispiel 49A mit den entsprechenden Aminen (bzw. deren Salzen) unter den beschriebenen Reaktionsbedingungen umgesetzt wurden. Abweichungen sind bei den jeweiligen Beispielen angegeben.

Tabelle 14:

| Bsp. | | Analytische Daten |
|---|---|---|
| **121** | *rac*-7-[(2,2-Difluorethyl)amino]-1-(2,4-difluorphenyl)-4-oxo-*N*-[1,1,1-trifluor-propan-2-yl]-1,4-dihydro-1,8-naphthyridin-3-carbonsäureamid 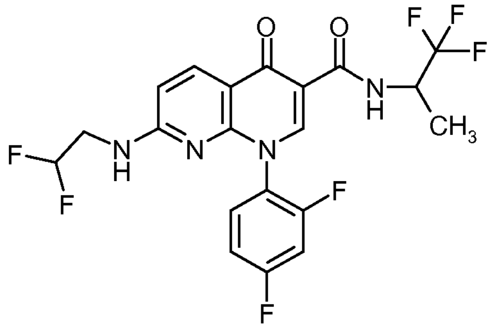 (90 % d. Th.) | LC-MS (Methode 1): $R_t$ = 1.06 min MS (ESpos): m/z = 477.2 $[M+H]^+$ $^1$H-NMR (400 MHz, $CDCl_3$) $\delta$ [ppm]: 0.017 (1.03), 0.079 (0.26), 1.251 (0.34), 1.269 (0.70), 1.287 (0.39), 1.451 (6.74), 1.469 (6.79), 1.568 (16.00), 2.054 (1.31), 2.451 (0.23), 3.499 (0.63), 3.536 (0.64), 4.122 (0.29), 4.140 (0.28), 4.899 (0.41), 4.918 (0.64), 4.937 (0.63), 4.955 (0.39), 5.279 (1.13), 5.309 (0.61), 5.542 (0.54), 5.673 (0.56), 5.683 (1.08), 5.693 (0.54), 5.824 (0.52), 6.593 (3.50), 6.615 (3.54), 7.006 (0.26), 7.049 (0.54), 7.056 (0.78), 7.075 (2.30), 7.094 (2.15), 7.112 (0.69), 7.377 (0.78), 7.398 (0.72), 7.529 (0.24), 8.444 (3.15), 8.466 (3.09), 8.713 (3.35), 10.386 (0.98), 10.410 (0.97). |
| **122** | *rac*-7-[(2,2-Difluorethyl)amino]-1-(2,4-difluorphenyl)-4-oxo-*N*-[1,1,1-trifluorbutan-2-yl]-1,4-dihydro-1,8-naphthyridin-3-carbonsäureamid 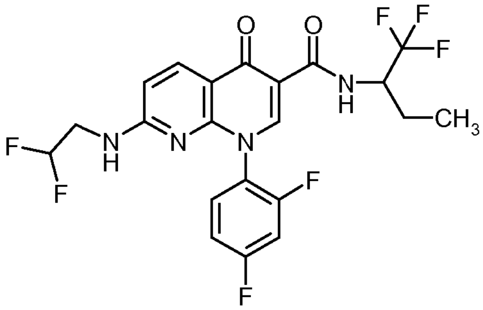 (82 % d. Th.) | LC-MS (Methode 1): $R_t$ = 1.11 min MS (ESpos): m/z = 491.2 $[M+H]^+$ $^1$H-NMR (400 MHz, $CDC_{l3}$) $\delta$ [ppm]: 0.001 (0.67), 0.017 (0.70), 0.079 (0.19), 1.055 (0.55), 1.074 (1.22), 1.092 (0.61), 1.563 (16.00), 1.704 (0.09), 1.723 (0.11), 1.730 (0.10), 1.740 (0.14), 1.748 (0.12), 1.758 (0.12), 1.765 (0.14), 1.784 (0.11), 1.936 (0.09), 1.947 (0.10), 1.954 (0.11), 1.966 (0.11), 1.982 (0.09), 2.449 (0.13), 2.794 (0.14), 3.507 (0.13), 3.538 (0.12), 4.751 (0.11), 4.761 (0.10), 4.769 (0.10), 4.779 (0.11), 5.259 (0.11), 5.272 (0.18), 5.309 (0.26), 5.544 (0.10), 5.674 (0.11), 5.685 (0.21), 5.695 (0.11), 5.825 (0.11), 6.594 (0.73), 6.616 (0.76), 7.006 (0.16), 7.050 (0.10), 7.056 (0.14), 7.076 (0.41), 7.095 (0.35), 7.101 (0.21), 7.113 (0.12), 7.365 (0.12), 7.386 (0.15), 7.400 (0.16), 7.406 (0.12), 7.529 (0.16), 8.450 (0.69), 8.471 (0.67), 8.722 (0.95), 10.309 (0.19), 10.332 (0.19). |
| **123** | 7-[(2,2-Difluorethyl)amino]-1-(2,4-difluorphenyl)-4-oxo-*N*-[(2*R*)-1,1,1-trifluor-butan-2-yl]-1,4-dihydro-1,8-naphthyridin-3-carbonsäureamid 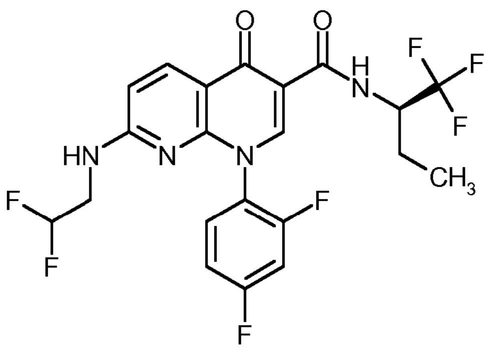 (57 % d. Th.) | LC-MS (Methode 1): $R_t$ = 1.07 min MS (ESpos): m/z = 491.2 $[M+H]^+$ $^1$H-NMR (400 MHz, DMSO-$d_6$) $\delta$ [ppm]: - 0.150 (0.74), -0.009 (10.07), 0.007 (5.25), 0.145 (0.82), 0.944 (7.49), 0.963 (16.00), 0.981 (7.84), 1.156 (1.80), 1.174 (3.56), 1.192 (1.78), 1.597 (1.15), 1.615 (1.63), 1.623 (1.41), 1.632 (1.89), 1.641 (1.58), 1.650 (1.56), 1.658 (1.75), 1.676 (1.37), 1.849 (1.38), 1.859 (1.62), 1.867 (1.55), 1.877 (1.76), 1.883 (1.53), 1.894 (1.40), 1.903 (1.14), 1.913 (0.95), 1.987 (6.41), 2.327 (0.95), 2.365 (0.89), 2.558 (0.69), 2.664 (0.75), 2.669 (1.03), 2.709 (0.91), 3.402 (2.56), 4.020 (1.51), 4.038 (1.49), 4.732 (1.51), 4.746 (1.41), 5.640 (0.74), 5.779 (1.39), 5.922 (0.72), 6.782 (4.45), 6.805 (4.51), 7.305 (1.56), 7.327 (2.88), 7.343 (1.55), 7.549 (1.71), 7.556 (1.80), 7.575 (2.74), 7.598 (1.73), 7.604 (1.59), 7.815 (2.18), 7.824 (1.83), 7.829 (1.78), 7.837 (2.02), 8.254 (6.67), 8.276 (6.39), 8.321 (1.84), 8.641 (5.85), 8.646 (5.13), 10.457 (4.94), 10.481 (4.71). |

(fortgesetzt)

| Bsp. | | Analytische Daten |
|---|---|---|
| **124** | 7-[(2,2-Difluorethyl)amino]-1-(2,4-difluorphenyl)-4-oxo-*N*-[1,1,1-trifluorpentan-2-yl]-1,4-dihydro-1,8-naphthyridin-3-carbonsäureamid 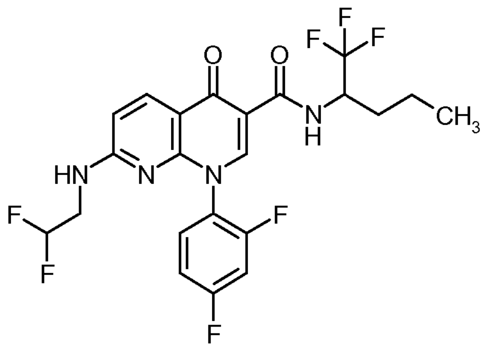 (71 % d. Th.) | LC-MS (Methode 1): $R_t$ = 1.20 min MS (ESpos): m/z = 505.2 $[M+H]^+$ $^1$H-NMR (400 MHz, DMSO-$d_6$) $\delta$ [ppm]: 0.005 (1.90), 0.890 (6.75), 0.909 (15.05), 0.927 (7.74), 1.156 (4.23), 1.173 (8.48), 1.191 (4.30), 1.312 (0.96), 1.329 (1.40), 1.348 (1.72), 1.367 (1.56), 1.385 (1.04), 1.404 (0.88), 1.424 (1.11), 1.437 (1.48), 1.457 (1.31), 1.472 (0.74), 1.592 (0.61), 1.604 (0.65), 1.618 (0.92), 1.627 (1.59), 1.639 (1.22), 1.653 (1.70), 1.664 (1.18), 1.676 (0.88), 1.688 (0.67), 1.737 (0.88), 1.746 (1.04), 1.763 (1.53), 1.769 (1.62), 1.779 (1.26), 1.787 (1.38), 1.812 (0.55), 1.987 (16.00), 3.366 (1.27), 3.403 (2.26), 3.438 (1.27), 4.001 (1.31), 4.019 (3.84), 4.037 (3.80), 4.055 (1.25), 4.771 (0.78), 4.790 (1.36), 4.814 (1.37), 4.832 (0.75), 5.638 (0.66), 5.780 (1.31), 5.921 (0.65), 6.781 (4.18), 6.803 (4.27), 7.299 (1.29), 7.303 (1.36), 7.320 (2.62), 7.325 (2.71), 7.341 (1.46), 7.346 (1.44), 7.546 (1.54), 7.553 (1.57), 7.572 (2.53), 7.576 (2.52), 7.594 (1.60), 7.601 (1.51), 7.797 (1.02), 7.817 (2.15), 7.835 (2.14), 7.854 (0.92), 8.250 (6.07), 8.272 (5.87), 8.319 (1.69), 8.640 (6.16), 10.453 (4.66), 10.477 (4.50). |
| **125** | *rac*-7-[(2,2-Difluorethyl)amino]-1-(2,4-difluorphenyl)-4-oxo-*N*-[1,1,1-trifluoro-4-methylpentan-2-yl]-1,4-dihydro-1,8-naphthyridin-3-carbonsäureamid 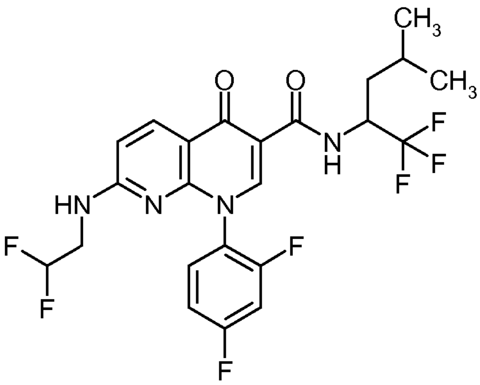 (96 % d. Th.) | LC-MS (Methode 1): $R_t$ = 1.22 min MS (ESpos): m/z = 519.1 $[M+H]^+$ $^1$H-NMR (400 MHz, $CDCl_3$) $\delta$ [ppm]: 0.001 (0.59), 0.017 (0.56), 0.954 (2.22), 0.963 (2.28), 0.970 (2.57), 0.984 (4.16), 1.001 (3.93), 1.251 (1.68), 1.269 (3.42), 1.287 (1.71), 1.568 (16.00), 1.616 (0.64), 1.625 (0.38), 1.643 (0.48), 1.708 (0.48), 1.719 (0.63), 1.737 (0.50), 1.747 (0.87), 1.753 (0.56), 1.766 (0.35), 1.775 (0.61), 1.782 (0.64), 1.790 (0.37), 1.799 (0.40), 2.054 (6.64), 3.502 (0.44), 3.538 (0.43), 3.546 (0.42), 3.553 (0.36), 4.104 (0.50), 4.122 (1.47), 4.140 (1.45), 4.158 (0.48), 4.892 (0.37), 4.897 (0.34), 4.916 (0.37), 5.255 (0.47), 5.270 (0.87), 5.285 (0.44), 5.542 (0.38), 5.673 (0.38), 5.683 (0.74), 5.693 (0.38), 5.824 (0.37), 6.592 (2.49), 6.614 (2.55), 7.051 (0.40), 7.071 (1.29), 7.091 (1.19), 7.109 (0.35), 7.364 (0.49), 7.379 (0.56), 7.386 (0.68), 7.400 (0.67), 7.407 (0.44), 7.421 (0.34), 8.442 (2.11), 8.464 (2.05), 8.725 (2.43), 10.256 (0.68), 10.280 (0.67). |

(fortgesetzt)

| Bsp. | | Analytische Daten |
|---|---|---|
| **126** | 7-[(2,2-Difluorethyl)amino]-1-(2,4-difluorphenyl)-*N*-(1,1,1,3,3,3-hexafluorpropan-2-yl)-4-oxo-1,4-dihydro-1,8-naphthyridin-3-carbonsäureamid 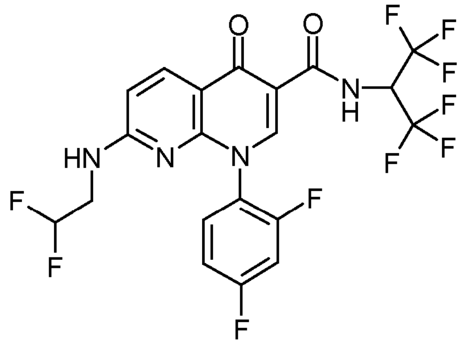 Reinigung über präparative HPLC (Eluent: Acetonitril/Wasser-Gradient mit 0.1% Ameisensäure). (70 % d. Th.) | LC-MS (Methode 1): $R_t$ = 1.17 min MS (ESpos): m/z = 531.2 [M+H]$^+$ $^1$H-NMR (400 MHz, CDCl3) δ [ppm]: 0.002 (1.44), 0.018 (1.22), 1.251 (0.71), 1.269 (1.54), 1.287 (0.74), 1.557 (16.00), 2.054 (2.72), 3.479 (0.46), 3.493 (0.41), 3.501 (0.69), 3.516 (0.89), 3.528 (0.69), 3.537 (0.74), 3.547 (0.81), 3.552 (0.81), 3.563 (0.70), 3.583 (0.40), 3.589 (0.39), 4.123 (0.59), 4.141 (0.59), 5.294 (1.00), 5.309 (1.40), 5.544 (0.71), 5.554 (0.41), 5.566 (0.63), 5.584 (0.82), 5.591 (0.71), 5.601 (0.65), 5.609 (0.84), 5.626 (0.60), 5.674 (0.67), 5.685 (1.33), 5.695 (0.66), 5.825 (0.65), 6.615 (4.34), 6.637 (4.44), 7.065 (0.66), 7.071 (1.01), 7.089 (2.38), 7.096 (1.19), 7.108 (2.23), 7.116 (1.30), 7.126 (0.83), 7.129 (0.72), 7.133 (0.46), 7.136 (0.50), 7.371 (0.88), 7.385 (0.97), 7.393 (1.05), 7.407 (1.17), 7.414 (0.71), 7.428 (0.61), 8.465 (3.85), 8.486 (3.76), 8.720 (6.71), 11.176 (1.18), 11.201 (1.17). |

**Beispiel 127**

*rac*-1-(2,4-Difluorphenyl)-7-[2-(hydroxymethyl)pyrrolidin-1-yl]-4-oxo-*N*-(tricyclo[3.3.1.1$^{3,7}$]dec-1-yl)-1,4-dihydro-1,8-naphthyridin-3-carbonsäureamid

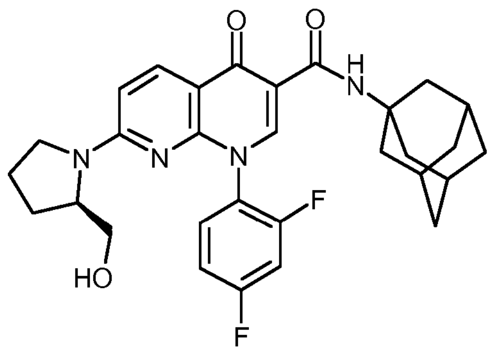

100 mg (0.25 mmol) der Verbindung aus Beispiel 52A wurden in 2.8 ml DMF vorgelegt, mit 114 mg (0.3 mmol) HATU sowie 103 mg (0.8 mmol) DIPEA versetzt und 30 Minuten lang bei 20°C gerührt. Dann wurden 49 mg (0.32 mmol) 1-Adamantanamin zugegeben und 2 Stunden bei 20°C gerührt. Anschliessend wurde 1 ml 1 M wässr. Salzsäure zugegeben und das Gemisch über präparative HPLC gereinigt (Eluent: Acetonitril/Wasser-Gradient mit 0.1% Ameisensäure). Man erhielt 76 mg (57 % d. Th.) der Titelverbindung.
LC-MS (Methode 1): $R_t$ = 1.27 min; m/z = 535.3 [M+H]$^+$.
$^1$H-NMR (400 MHz, DMSO-$d_6$) δ [ppm]: -0.150 (1.22), -0.009 (14.65), 0.007 (10.98), 0.018 (0.98), 0.025 (0.54), 0.058 (0.18), 0.083 (0.13), 0.146 (1.22), 1.146 (0.44), 1.168 (0.21), 1.225 (0.57), 1.243 (2.78), 1.259 (2.59), 1.273 (1.71), 1.355 (0.26), 1.586 (0.40), 1.670 (7.56), 1.752 (0.51), 1.827 (0.87), 1.926 (0.77), 2.055 (16.00), 2.137 (0.14), 2.274 (0.14), 2.322 (0.73), 2.327 (0.95), 2.332 (0.68), 2.365 (1.41), 2.390 (0.22), 2.523 (4.43), 2.559 (0.82), 2.669 (0.94), 2.674 (0.67), 2.689 (0.41), 2.709 (1.31), 2.730 (0.71), 2.890 (1.05), 3.129 (0.49), 3.139 (0.52), 3.147 (0.50), 3.157 (0.47), 3.427 (0.20), 3.466 (0.24), 3.600 (0.16), 3.616 (0.21), 3.625 (0.18), 3.958 (0.15), 4.340 (0.14), 4.462 (0.13), 6.705 (0.26), 6.867 (0.15), 7.278 (0.46), 7.299 (0.76), 7.317 (0.42), 7.547 (0.39), 7.731 (0.37), 7.753 (0.65), 7.769 (0.65), 7.889 (0.12), 7.954 (0.13), 8.019 (0.12), 8.239 (0.99), 8.261 (0.85), 8.471 (2.45), 9.962 (2.29)

**Beispiel 128**

1-(2,4-Difluorphenyl)-4-oxo-7-(2-oxo-1,3-oxazolidin-3-yl)-*N*-(tricyclo[3.3.1.1$^{3,7}$]dec-1-yl)-1,4-dihydro-1,8-naphthyridin-3-carbonsäureamid

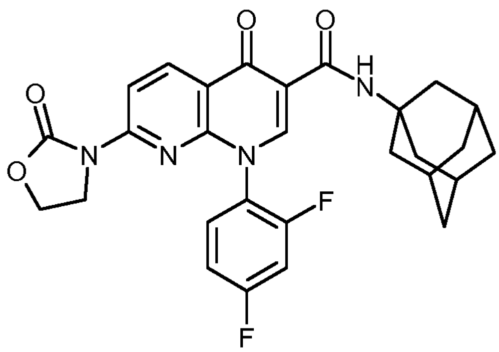

28 mg (0.04 mmol, Reinheit 59% (HPLC)) der Verbindung aus Beispiel 61A wurden in 1 ml DMF vorgelegt, mit 24 mg (0.06 mmol) HATU sowie 18 mg (0.14 mmol) DIPEA versetzt und 30 Minuten lang bei 20°C gerührt. Dann wurden 12 mg (0.08 mmol) 1-Adamantanamin zugegeben und 9 Stunden bei 23°C gerührt. Das Gemisch wurde für 13 h bei RT stehengelassen und anschließend über präparative HPLC gereinigt (Eluent: Acetonitril/Wasser-Gradient mit 0.1% Ameisensäure). Man erhielt 12 mg (54 % d. Th.) der Titelverbindung.
LC-MS (Methode 1): $R_t$ = 1.30 min; m/z = 521.2 $[M+H]^+$.
$^1$H-NMR (400 MHz, DMSO-$d_6$) $\delta$ [ppm]: -0.150 (1.16), -0.009 (10.40), 0.007 (9.44), 0.016 (0.60), 0.021 (0.38), 0.146 (1.19), 1.680 (5.04), 2.072 (16.00), 2.322 (0.58), 2.327 (0.93), 2.331 (0.68), 2.366 (1.10), 2.520 (1.96), 2.523 (2.04), 2.525 (1.76), 2.558 (0.72), 2.560 (0.57), 2.563 (0.46), 2.565 (0.45), 2.660 (0.41), 2.664 (0.63), 2.669 (0.93), 2.674 (0.61), 2.709 (1.14), 3.285 (0.67), 3.711 (0.38), 3.730 (0.79), 3.753 (0.82), 3.771 (0.39), 4.356 (0.52), 4.377 (0.83), 4.385 (0.86), 4.397 (0.50), 4.405 (0.50), 7.353 (0.60), 7.565 (0.42), 7.598 (0.52), 7.613 (0.40), 7.620 (0.40), 7.827 (0.41), 7.842 (0.43), 7.849 (0.71), 7.864 (0.73), 7.871 (0.38), 8.265 (2.34), 8.287 (2.47), 8.678 (2.58), 8.700 (2.77), 8.703 (4.30), 9.720 (1.95).

**Beispiel 129**

1-(2,4-Difluorphenyl)-4-oxo-7-(1,3-thiazolidin-3-yl)-*N*-(tricyclo[3.3.1.1$^{3,7}$]dec-1-yl)-1,4-dihydro-1,8-naphthyridin-3-carbonsäureamid

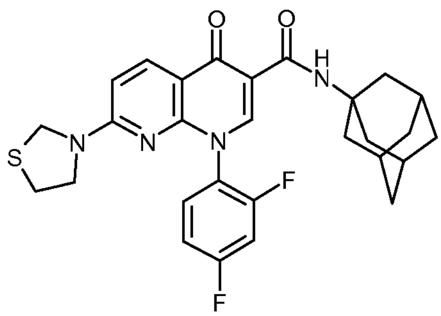

200 mg (0.26 mmol) der Verbindung aus Beispiel 59A wurden in 5.8 ml DMF vorgelegt, mit 235 mg (0.62 mmol) HATU sowie 212 mg (1.64 mmol) DIPEA versetzt und 30 Minuten lang bei 20°C gerührt. Dann wurden 109 mg (0.72 mmol) 1-Adamantanamin zugegeben und 2 Stunden bei 20°C gerührt. Anschliessend wurde das Gemisch über präparative HPLC gereinigt (Eluent: Acetonitril/Wasser-Gradient mit 0.1% Ameisensäure). Man erhielt 130 mg (48 % d. Th.) der Titelverbindung.
LC-MS (Methode 1): $R_t$ = 1.48 min; m/z = 523.2 $[M+H]^+$.
1H-NMR (400 MHz, DMSO-$d_6$) $\delta$ [ppm]: -0.009 (1.08), -0.001 (16.00), 0.007 (0.50), 1.672 (1.96), 1.987 (0.08), 2.058

(4.16), 3.084 (0.32), 3.099 (0.63), 3.115 (0.32), 3.161 (0.22), 3.174 (0.23), 3.614 (0.33), 4.419 (0.44), 6.885 (0.59), 6.907 (0.60), 7.303 (0.12), 7.319 (0.21), 7.346 (0.11), 7.546 (0.14), 7.553 (0.15), 7.572 (0.20), 7.594 (0.14), 7.601 (0.13), 7.775 (0.14), 7.789 (0.17), 7.796 (0.26), 7.811 (0.25), 7.833 (0.12), 8.330 (0.74), 8.353 (0.68), 8.515 (1.27), 9.898 (0.67).

**Beispiel 130**

1-(2,4-Difluorphenyl)-7-(1,1-dioxido-1,3-thiazolidin-3-yl)-4-oxo-*N*-(tricyclo[3.3.1.1$^{3,7}$]dec-1-yl)-1,4-dihydro-1,8-naphthyridin-3-carbonsäureamid

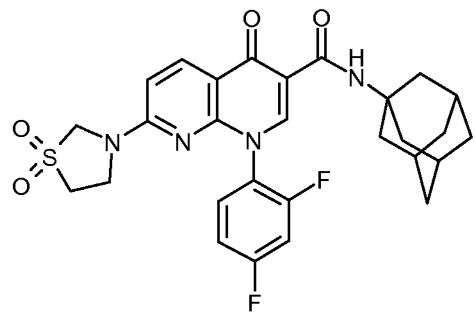

98 mg (0.19 mmol) der Verbindung aus Beispiel 129 wurden in 1.4 ml Dioxan und 0.7 ml Wasser vorgelegt, mit 98 mg (0.56 mmol) Dikaliumhydrogenphosphat sowie 344 mg (0.56 mmol) OXONE® versetzt und füt 8 h bei 23°C gerührt und anschliessend für 13 h stehengelassen. Das Gemisch wurde mit Wasser versetzt, der ausgefallene Feststoff abfiltriert und dieser anschliessend über präparative HPLC gereinigt (Eluent: Acetonitril/Wasser-Gradient mit 0.1% Ameisensäure). Man erhielt 67 mg (64 % d. Th.) der Titelverbindung. Weiterhin wurden 12 mg (12% d.Th.) der Titelverbindung aus Beispiel 131 erhalten (Analytik s. Beispiel 131).
LC-MS (Methode 1): $R_t$ = 1.20 min; m/z = 555.3 $[M+H]^+$.
$^1$H-NMR (400 MHz, DMSO-$d_6$) δ [ppm]: -0.009 (1.95), 0.007 (1.22), 1.673 (7.35), 2.061 (16.00), 2.072 (4.15), 3.526 (1.04), 3.544 (2.15), 3.562 (1.26), 3.799 (0.98), 4.560 (1.05), 6.963 (1.49), 6.985 (1.48), 7.318 (0.45), 7.340 (0.78), 7.361 (0.44), 7.564 (0.55), 7.571 (0.57), 7.590 (0.76), 7.613 (0.53), 7.620 (0.49), 7.791 (0.53), 7.806 (0.64), 7.813 (1.01), 7.828 (0.98), 7.835 (0.55), 7.850 (0.47), 8.420 (2.63), 8.442 (2.44), 8.562 (5.10), 9.841 (2.67).

**Beispiel 131**

1-(2,4-Difluorphenyl)-7-(1-oxido-1,3-thiazolidin-3-yl)-4-oxo-*N*-(tricyclo[3.3.1.1$^{3,7}$]dec-1-yl)-1,4-dihydro-1,8-naphthyridin-3-carbonsäureamid

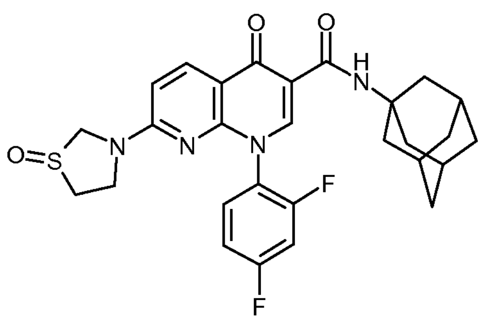

Wie bei der Darstellung der Verbindung aus Beispiel 130 beschrieben, wurden aus 98 mg (0.19 mmol) der Verbindung aus Beispiel 129, 12 mg (12% d.Th.) der Titelverbindung erhalten.
LC-MS (Methode 1): $R_t$ = 1.09 min; m/z = 539.3 $[M+H]^+$.
$^1$H-NMR (400 MHz, DMSO-$d_6$) δ [ppm]: -0.009 (2.26), 0.007 (1.41), 1.026 (1.00), 1.043 (0.87), 1.234 (0.36), 1.673 (7.26), 2.061 (16.00), 2.522 (1.08), 3.072 (0.45), 3.923 (0.56), 4.312 (0.41), 4.622 (0.35), 6.964 (2.06), 6.986 (2.07), 7.338 (0.60), 7.578 (0.45), 7.809 (0.56), 8.177 (2.22), 8.377 (2.66), 8.399 (2.45), 8.534 (4.78), 9.883 (2.63).

## Beispiel 132

1-(2,4-Difluorphenyl)-7-(dimethylamino)-4-oxo-*N*-[(2*R*)-1,1,1-trifluor-3,3-dimethylbutan-2-yl]-1,4-dihydro-1,8-naphthyridin-3-carbonsäureamid

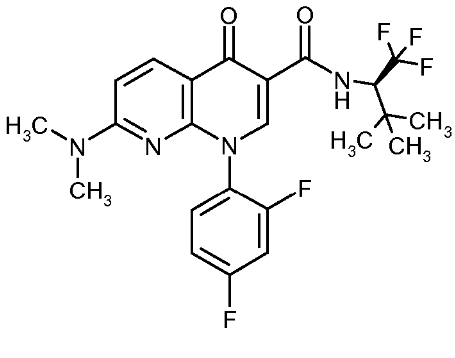

Zu 100 mg (0.29 mmol) der Verbindung aus Beispiel 36A und 73 mg (0.72 mmol) *N*-Methylmorpholin in 3.1 ml DMF wurden bei 0°C 0.58 ml (0.58 mmol) Chlorameisensäureisopropylester (1 M in Toluol) gegeben und 1 h bei 0°C gerührt. Dann wurden bei 0°C 58 mg (0.38 mmol) (*R*)-2,2-Dimethyl-1-trifluormethyl-propylamin zugegeben und 16 Stunden bei 20°C gerührt. Anschliessend wurde das Gemisch eingeengt und über präparative HPLC gereinigt (Eluent: Acetonitril/Wasser-Gradient mit 0.1% Ameisensäure). Man erhielt 20 mg (15 % d. Th.) der Titelverbindung.
LC-MS (Methode 1): $R_t$ = 1.26 min; m/z = 483.2 $[M+H]^+$.
1H-NMR (400 MHz, CDCl3) δ [ppm]: 0.080 (0.08), 1.174 (1.07), 1.266 (0.04), 1.551 (16.00), 3.000 (0.35), 4.651 (0.04), 4.675 (0.05), 4.698 (0.04), 6.669 (0.13), 6.692 (0.13), 7.006 (0.07), 7.042 (0.07), 7.061 (0.07), 7.371 (0.03), 7.385 (0.04), 7.392 (0.05), 7.407 (0.04), 7.528 (0.07), 8.449 (0.12), 8.472 (0.12), 8.688 (0.20), 10.742 (0.04), 10.768 (0.04).

In Analogie zu Beispiel 132 wurden die in Tabelle 15 gezeigten Beispielverbindungen hergestellt, indem die Verbindung aus Beispiel 36A mit den entsprechenden Aminen (bzw. deren Salzen) unter den beschriebenen Reaktionsbedingungen umgesetzt wurden. Abweichungen sind bei den jeweiligen Beispielen angegeben.

Tabelle 15:

| Bsp. | | Analytische Daten |
|---|---|---|
| **133** | 1-(2,4-Difluorphenyl)-7-(dimethylamino)-4-oxo-*N*-[(2*R*)-1,1,1-trifluor-4-methylpentan-2-yl]-1,4-dihydro-1,8-naphthyridin-3-carbonsäureamid 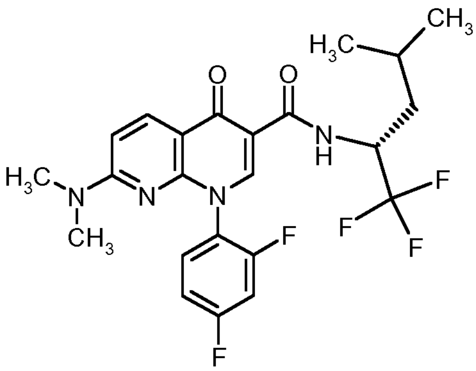 (23 % d. Th.) | LC-MS (Methode 1): $R_t$ = 1.29 min MS (ESpos): m/z = 483.2 $[M+H]^+$ $^1$H-NMR (400 MHz, CDCl3) δ [ppm]: - 0.139 (0.03), 0.156 (0.03), 0.958 (0.19), 0.974 (0.22), 0.983 (0.32), 0.999 (0.28), 1.266 (0.06), 1.551 (16.00), 1.609 (0.06), 1.634 (0.04), 1.713 (0.03), 1.723 (0.04), 1.752 (0.04), 1.787 (0.05), 3.000 (0.35), 4.894 (0.03), 6.671 (0.13), 6.694 (0.13), 7.006 (0.07), 7.038 (0.08), 7.059 (0.07), 7.367 (0.04), 7.388 (0.05), 7.402 (0.05), 7.424 (0.02), 7.528 (0.07), 8.415 (0.13), 8.438 (0.13), 8.684 (0.18), 10.389 (0.04), 10.415 (0.04). |

(fortgesetzt)

| Bsp. | | Analytische Daten |
|---|---|---|
| **134** | 1-(2,4-Difluorphenyl)-7-(dimethylamino)-4-oxo-*N*-[(2*S*)-1,1,1-trifluor-3-methylbutan-2-yl]-1,4-dihydro-1,8-naphthyridin-3-carbonsäureamid 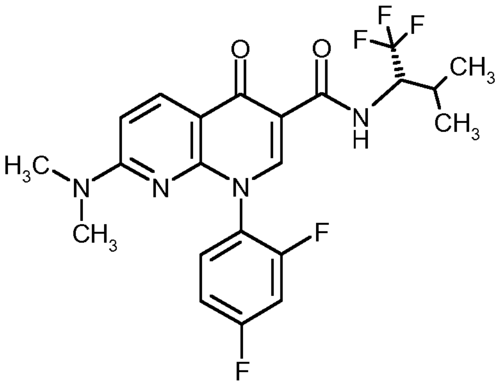 (49 % d. Th.) | LC-MS (Methode 1): $R_t$ = 1.23 min MS (ESpos): m/z = 469.2 $[M+H]^+$ <br> $^1$H-NMR (400 MHz, CDCl3) $\delta$ [ppm]: 0.000 (0.22), 0.017 (0.20), 0.078 (0.13), 1.062 (0.81), 1.079 (0.83), 1.137 (0.70), 1.154 (0.72), 1.265 (0.08), 1.576 (16.00), 2.300 (0.10), 2.310 (0.11), 2.450 (0.07), 2.793 (0.07), 3.000 (1.08), 4.789 (0.08), 6.670 (0.59), 6.693 (0.60), 7.006 (0.09), 7.023 (0.11), 7.042 (0.23), 7.062 (0.21), 7.367 (0.11), 7.382 (0.13), 7.389 (0.14), 7.403 (0.14), 7.425 (0.08), 7.529 (0.09), 8.444 (0.57), 8.467 (0.55), 8.687 (0.79), 10.606 (0.13), 10.632 (0.12). |

## Beispiel 135

1-(2,4-Difluorphenyl)-4-oxo-7-(pyrrolidin-1-yl)-*N*-(tricyclo[3.3.1.1$^{3,7}$]dec-1-yl)-1,4-dihydro-1,8-naphthyridin-3-carbonsäureamid

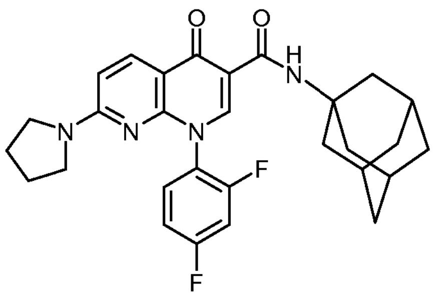

Zu 80 mg (0.22 mmol) der Verbindung aus Beispiel 57A und 54.5 mg (0.54 mmol) N-Methylmorpholin in 2.6 ml DMF wurden bei 0°C 0.43 ml (0.43 mmol) Chlorameisensäureisopropylester (1 M in Toluol) gegeben und 1 h bei 0°C gerührt. Dann wurden bei 0°C 42 mg (0.28 mmol) 1-Adamantanamin zugegeben und 2 Stunden bei 20°C gerührt. Nach 12 h bei 20°C wurde das Gemisch über präparative HPLC gereinigt (Eluent: Acetonitril/Wasser-Gradient mit 0.1% Ameisensäure). Man erhielt 20 mg (19 % d. Th.) der Titelverbindung.
LC-MS (Methode 1): $R_t$ = 1.47 min; m/z = 505.3 $[M+H]^+$.
$^1$H-NMR (400 MHz, DMSO-$d_6$): $\delta$ = 1.67 (m, 6 H), 1.74-1.99 (m, 4H), 2.06 (m, 9H), 2.99 - 3.23 (m, 2H), 3.34-3.48 (m, 2H), 6.70 (d, 1H), 7.28-7.34 (m, 1H), 7.53 - 7.60 (m, 1H), 7.74 - 7.81 (m, 1H), 8.25 (d, 1H), 8.47 (s, 1H), 9.97 (br. s, 1H).

## Beispiel 136

1-(2,4-Difluorphenyl)-7-(morpholin-4-yl)-4-oxo-*N*-(tricyclo[3.3.1.1$^{3,7}$]dec-1-yl)-1,4-dihydro-1,8-naphthyridin-3-carbonsäureamid

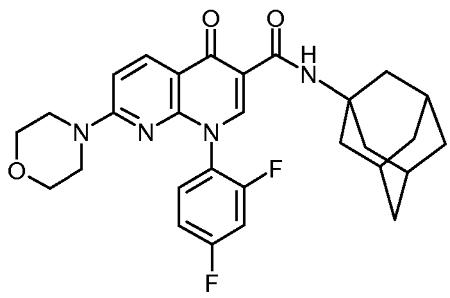

Zu 80 mg (0.17 mmol, Reinheit 83%) der Verbindung aus Beispiel 56A und 43 mg (0.43 mmol) N-Methylmorpholin in 2 ml DMF wurden bei 0°C 0.34 ml (0.34 mmol) Chlorameisensäureisopropylester (1 M in Toluol) gegeben und 1 h bei 0°C gerührt. Dann wurden bei 0°C 34 mg (0.22 mmol) 1-Adamantanamin zugegeben und 2 Stunden bei 20°C gerührt. Das Gemisch wurde über präparative HPLC gereinigt (Eluent: Acetonitril/Wasser-Gradient mit 0.1% Ameisensäure). Man erhielt 29 mg (33 % d. Th.) der Titelverbindung.
LC-MS (Methode 1): $R_t$ = 1.47 min; m/z = 521.2 $[M+H]^+$.
$^1$H-NMR (400 MHz, DMSO-$d_6$) $\delta$ [ppm]: 0.000 (16.00), 1.671 (3.77), 2.055 (7.92), 3.443 (1.72), 3.454 (1.40), 3.574 (1.56), 3.587 (1.91), 7.064 (0.97), 7.087 (0.98), 7.301 (0.21), 7.323 (0.42), 7.340 (0.22), 7.550 (0.28), 7.572 (0.40), 7.592 (0.28), 7.763 (0.25), 7.785 (0.50), 7.800 (0.49), 7.821 (0.23), 8.296 (1.30), 8.318 (1.21), 8.491 (2.61), 9.897 (1.31).

**Beispiel 137**

1-(2,4-Difluorphenyl)-7-(dimethylamino)-4-oxo-*N*-[(2S)-1,1,1-trifluor-4-methylpentan-2-yl]-1,4-dihydro-1,8-naphthyridin-3-carbonsäureamid

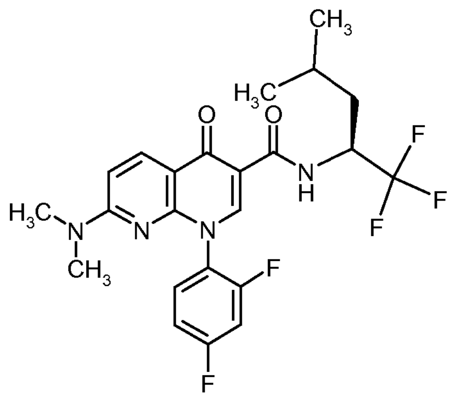

Zu 100 mg (0.29 mmol) der Verbindung aus Beispiel 36A und 73 mg (0.72 mmol) *N*-Methylmorpholin in 3.1 ml DMF wurden bei 0°C 0.58 ml (0.58 mmol) Chlorameisensäureisopropylester (1 M in Toluol) gegeben und 1 h bei 0°C gerührt. Dann wurden bei 0°C 58 mg (0.38 mmol) (*S*)-1,1,1-Trifluor-4-methyl-2-pentylamin zugegeben und 16 Stunden bei 20°C gerührt. Dann wurde das Gemisch über präparative HPLC gereinigt (Eluent: Acetonitril/Wasser-Gradient mit 0.1% Ameisensäure). Man erhielt 104 mg (74 % d. Th.) der Titelverbindung.
LC-MS (Methode 1): $R_t$ = 1.28 min; m/z = 483.2 $[M+H]^+$.
$^1$H-NMR (400 MHz, $CDCl_3$) $\delta$ [ppm]: 0.958 (4.49), 0.975 (5.14), 0.983 (6.83), 0.999 (6.13), 1.266 (0.96), 1.564 (16.00), 1.609 (1.34), 1.635 (0.88), 1.713 (0.67), 1.723 (0.84), 1.751 (1.06), 1.786 (1.05), 2.999 (7.99), 4.919 (0.54), 6.671 (2.93), 6.693 (3.01), 7.019 (0.67), 7.039 (1.67), 7.058 (1.54), 7.367 (0.65), 7.387 (0.97), 7.402 (0.99), 7.424 (0.49), 8.414 (2.85), 8.437 (2.78), 8.683 (4.11), 10.391 (0.96), 10.415 (0.98).

In Analogie zu Beispiel 137 wurden die in Tabelle 16 gezeigten Beispielverbindungen hergestellt, indem die Verbindung aus Beispiel 36A bzw. 60A mit den entsprechenden Aminen (bzw. deren Salzen) unter den beschriebenen Reaktionsbedingungen umgesetzt wurden. Abweichungen sind bei den jeweiligen Beispielen angegeben.

Tabelle 16:

| Bsp. | | Analytische Daten |
|---|---|---|
| **138** | *rac*-1-(2,4-Difluorphenyl)-7-(dimethyl-amino)-4-oxo-*N*-[2,2,2-trifluor-1-(3-methylphenyl)ethyl]-1,4-dihydro-1,8-naphthyridin-3-carbonsäureamid 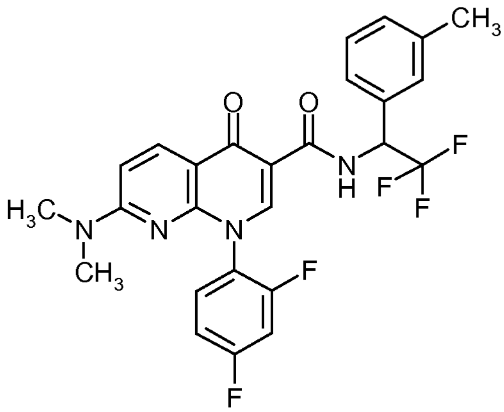 (35 % d. Th.) | LC-MS (Methode 1): $R_t$ = 1.36 min MS (ESpos): m/z = 517.2 $[M+H]^+$ $^1$H-NMR (400 MHz, DMSO-$d_6$) $\delta$ [ppm]: - 0.009 (2.14), 0.007 (1.88), 1.387 (4.58), 2.351 (16.00), 2.522 (0.44), 2.890 (0.83), 2.950 (2.67), 5.962 (0.92), 5.984 (1.25), 6.006 (0.84), 6.942 (4.11), 6.965 (4.18), 7.245 (1.22), 7.262 (1.92), 7.310 (0.79), 7.332 (1.20), 7.355 (7.81), 7.371 (2.23), 7.391 (0.56), 7.553 (0.46), 7.573 (0.79), 7.750 (0.44), 7.769 (0.66), 7.783 (0.53), 7.791 (0.49), 7.819 (0.49), 7.834 (0.47), 8.326 (4.55), 8.349 (4.33), 8.424 (0.68), 8.618 (8.10), 11.425 (2.01), 11.449 (1.94). |
| **139** | *rac*-1-(2,4-Difluorphenyl)-7-(dimethyl-amino)-4-oxo-*N*-[2,2,2-trifluor-1-(4-fluorphenyl)ethyl]-1,4-dihydro-1,8-naphthyridin-3-carbonsäureamid 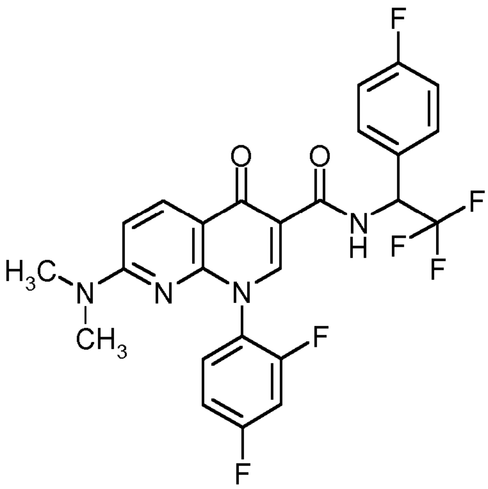 (37 % d. Th.) | LC-MS (Methode 1): $R_t$ = 1.32 min MS (ESpos): m/z = 521.2 $[M+H]^+$ $^1$H-NMR (400 MHz, DMSO-$d_6$) $\delta$ [ppm]: 0.007 (4.07), 2.072 (1.25), 2.951 (5.22), 6.091 (1.85), 6.113 (2.53), 6.134 (1.71), 6.946 (7.80), 6.969 (7.93), 7.308 (6.62), 7.330 (12.46), 7.352 (6.41), 7.574 (1.76), 7.608 (4.02), 7.622 (4.54), 7.641 (3.07), 7.753 (0.93), 7.768 (0.94), 7.818 (1.05), 7.833 (1.00), 8.322 (8.62), 8.345 (8.23), 8.622 (16.00), 11.479 (4.76), 11.503 (4.56) |

(fortgesetzt)

| Bsp. | | Analytische Daten |
|---|---|---|
| **140** | *rac*-1-(2,4-Difluorphenyl)-7-(dimethyl-amino)-4-oxo-*N*-[2,2,2-trifluor-1-(3-fluorphenyl)ethyl]-1,4-dihydro-1,8-naphthyridin-3-carbonsäureamid 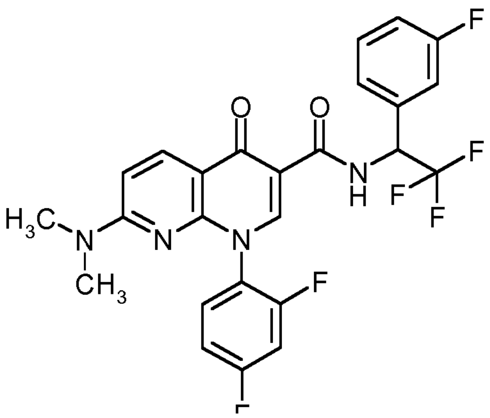 (34 % d. Th.) | LC-MS (Methode 1): $R_t$ = 1.25 min MS (ESpos): m/z = 521.2 $[M+H]^+$ $^1$H-NMR (400 MHz, DMSO-$d_6$) $\delta$ [ppm]: - 0.009 (12.35), 0.004 (4.35), 0.006 (3.57), 0.007 (5.74), 2.072 (2.45), 2.366 (0.89), 2.526 (7.91), 2.669 (0.95), 2.709 (0.99), 2.954 (4.94), 6.134 (1.60), 6.156 (2.15), 6.177 (1.39), 6.950 (7.54), 6.973 (7.35), 7.285 (1.95), 7.299 (3.43), 7.306 (3.99), 7.321 (2.88), 7.326 (2.96), 7.413 (3.90), 7.431 (4.16), 7.528 (2.24), 7.544 (3.21), 7.549 (4.29), 7.564 (4.14), 7.569 (2.98), 7.584 (2.43), 7.753 (0.97), 7.769 (0.91), 7.819 (0.98), 7.835 (0.95), 8.331 (8.79), 8.353 (8.26), 8.627 (16.00), 11.500 (4.07), 11.523 (3.81). |
| **141** | *rac*-1-(2,4-Difluorphenyl)-7-(dimethyl-amino)-4-oxo-*N*-[2,2,2-trifluor-1-(4-methylphenyl)ethyl]-1,4-dihydro-1,8-naphthyridin-3-carbonsäureamid 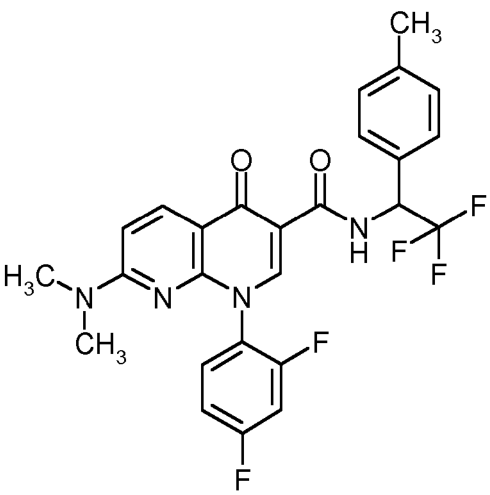 (39 % d. Th.) | LC-MS (Methode 1): $R_t$ = 1.36 min MS (ESpos): m/z = 517.2 $[M+H]^+$ $^1$H-NMR (400 MHz, DMSO-$d_6$) $\delta$ [ppm]: - 0.009 (5.89), 0.007 (3.81), 2.072 (0.71), 2.324 (16.00), 2.523 (1.41), 2.948 (2.94), 5.962 (0.99), 5.983 (1.29), 6.005 (0.83), 6.943 (4.65), 6.966 (4.56), 7.275 (4.04), 7.295 (5.29), 7.326 (0.91), 7.427 (3.90), 7.447 (2.96), 7.572 (0.93), 7.753 (0.49), 7.766 (0.52), 7.819 (0.53), 7.833 (0.52), 8.322 (5.43), 8.345 (5.01), 8.617 (9.31), 11.420 (2.08), 11.443 (1.96). |

(fortgesetzt)

| Bsp. | | Analytische Daten |
|---|---|---|
| **142** | *rac*-1-(2,4-Difluorphenyl)-7-(dimethyl-amino)-4-oxo-*N*-[2,2,2-trifluor-1-(2-methylphenyl)ethyl]-1,4-dihydro-1,8-naphthyridin-3-carbonsäureamid 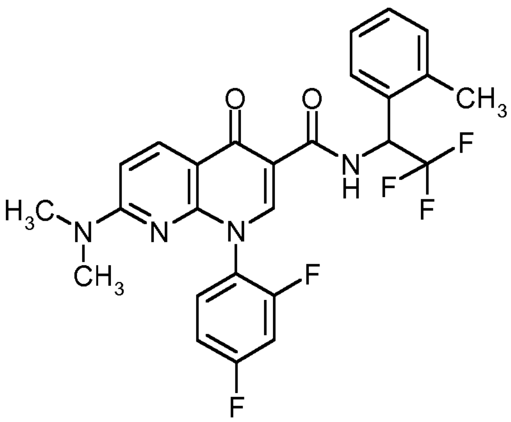 (39 % d. Th.) | LC-MS (Methode 1): $R_t$ = 1.24 min MS (ESpos): m/z = 517.1 $[M+H]^+$ <br> $^1$H-NMR (400 MHz, DMSO-$d_6$) $\delta$ [ppm]: - 0.009 (2.71), 0.007 (2.25), 2.072 (2.19), 2.443 (16.00), 2.523 (0.78), 2.946 (4.02), 3.287 (0.69), 6.149 (1.47), 6.170 (2.06), 6.192 (1.37), 6.937 (6.45), 6.960 (6.55), 7.291 (1.26), 7.297 (1.95), 7.314 (6.35), 7.329 (4.65), 7.346 (2.90), 7.361 (2.05), 7.379 (0.78), 7.460 (1.86), 7.568 (1.13), 7.739 (0.68), 7.755 (0.71), 7.814 (0.75), 7.830 (0.71), 8.307 (7.18), 8.330 (6.80), 8.615 (11.47), 11.459 (3.56), 11.482 (3.44). |
| **143** | *rac*-1-(2,4-Difluorphenyl)-7-(dimethyl-amino)-4-oxo-*N*-(1,1,1-trifluor-3-phenyl-propan-2-yl)-1,4-dihydro-1,8-naphthyridin-3-carbonsäureamid 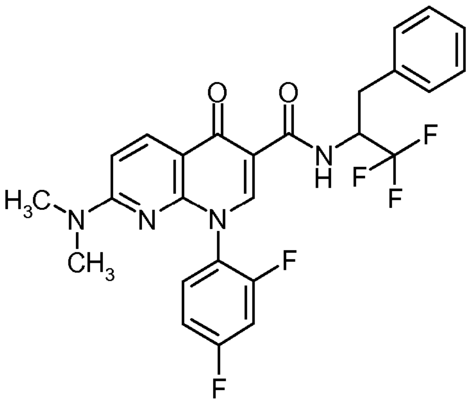 (35 % d. Th.) | LC-MS (Methode 1): $R_t$ = 1.35 min MS (ESpos): m/z = 517.2 $[M+H]^+$ <br> $^1$H-NMR (400 MHz, DMSO-$d_6$) $\delta$ [ppm]: - 0.009 (8.70), 0.007 (3.77), 2.072 (2.06), 2.085 (3.79), 2.730 (7.16), 2.890 (12.47), 2.901 (7.59), 2.936 (13.83), 2.963 (8.00), 3.219 (4.15), 3.246 (3.55), 5.082 (2.18), 6.915 (12.58), 6.938 (12.45), 7.201 (3.83), 7.273 (8.46), 7.310 (10.19), 7.547 (3.56), 7.767 (2.90), 8.268 (16.00), 8.291 (14.62), 8.479 (7.19), 8.489 (5.88), 10.590 (7.45), 10.614 (6.89). |
| **144** | *rac*-1-(2,4-Difluorphenyl)-7-(dimethyl-amino)-4-oxo-*N*-(1,1,1-trifluoropentan-2-yl)-1,4-dihydro-1,8-naphthyridin-3-carbonsäureamid 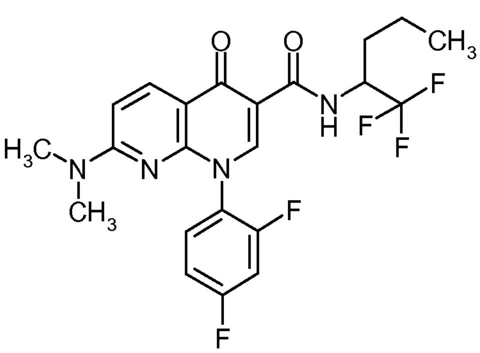 (53 % d. Th.) | LC-MS (Methode 1): $R_t$ = 1.30 min MS (ESpos): m/z = 469.2 $[M+H]^+$ <br> $^1$H-NMR (400 MHz, DMSO-$d_6$) $\delta$ [ppm]: - 0.013 (4.68), 0.003 (4.35), 0.887 (7.06), 0.906 (16.00), 0.924 (8.19), 1.327 (1.40), 1.345 (1.67), 1.365 (1.60), 1.384 (1.10), 1.436 (1.51), 1.620 (1.70), 1.647 (1.76), 1.765 (1.63), 2.942 (6.60), 4.807 (1.43), 6.923 (9.13), 6.946 (9.28), 7.298 (1.43), 7.320 (2.82), 7.341 (1.57), 7.544 (1.76), 7.551 (1.83), 7.570 (2.68), 7.593 (1.82), 7.599 (1.70), 7.803 (2.27), 7.819 (2.25), 8.266 (10.01), 8.289 (9.57), 8.608 (8.67), 10.482 (4.88), 10.505 (4.71). |

(fortgesetzt)

| Bsp. | | Analytische Daten |
|---|---|---|
| **145** | *rac*-1-(2,4-Difluorphenyl)-7-(dimethyl-amino)-4-oxo-*N*-(1,1,1-trifluor-4-methyl-pentan-2-yl)-1,4-dihydro-1,8-naphthyridin-3-carbonsäureamid 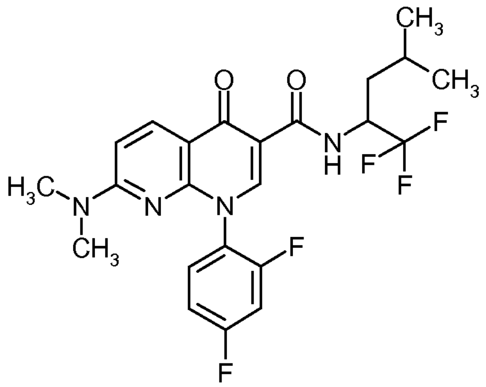 (57 % d. Th.) | LC-MS (Methode 1): $R_t$ = 1.35 min MS (ESpos): m/z = 483.3 $[M+H]^+$ $^1$H-NMR (400 MHz, DMSO-$d_6$) $\delta$ [ppm]: - 0.150 (1.26), -0.009 (16.00), 0.007 (10.57), 0.146 (1.29), 0.880 (5.73), 0.895 (5.90), 0.941 (6.27), 0.957 (6.46), 1.146 (0.73), 1.560 (1.57), 1.587 (1.17), 1.642 (1.65), 1.669 (2.24), 1.696 (1.06), 2.072 (1.51), 2.322 (1.23), 2.327 (1.62), 2.331 (1.26), 2.366 (1.23), 2.562 (0.81), 2.669 (1.73), 2.709 (1.45), 2.946 (3.80), 4.829 (0.84), 6.928 (5.15), 6.950 (5.20), 7.301 (0.87), 7.318 (1.71), 7.344 (0.98), 7.547 (1.01), 7.553 (1.01), 7.574 (1.54), 7.594 (0.90), 7.602 (0.84), 7.792 (0.98), 7.813 (1.79), 7.828 (1.68), 7.850 (0.84), 8.268 (5.62), 8.291 (5.29), 8.619 (7.16), 10.483 (2.69), 10.507 (2.69). |
| **146** | *rac*-1-(2,4-Difluorphenyl)-7-(dimethyl-amino)-4-oxo-*N*-(1,1,1-trifluorbutan-2-yl)-1,4-dihydro-1,8-naphthyridin-3-carbonsäureamid 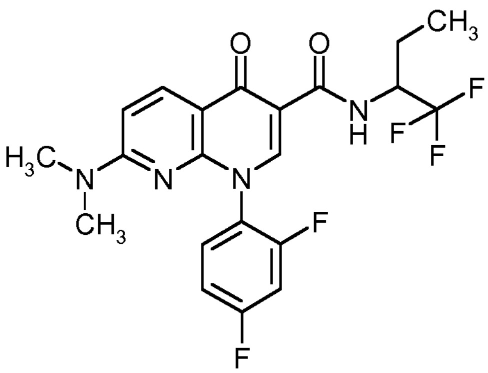 (62 % d. Th.) | LC-MS (Methode 1): $R_t$ = 1.24 min MS (ESpos): m/z = 455.2 $[M+H]^+$ $^1$H-NMR (400 MHz, DMSO-$d_6$) $\delta$ [ppm]: - 0.001 (4.33), 0.946 (7.55), 0.965 (16.00), 0.983 (7.69), 1.595 (1.19), 1.613 (1.56), 1.620 (1.42), 1.630 (1.86), 1.638 (1.67), 1.648 (1.59), 1.655 (1.75), 1.673 (1.41), 1.848 (1.44), 1.858 (1.61), 1.867 (1.61), 1.876 (1.78), 2.947 (7.03), 4.730 (1.52), 6.929 (9.36), 6.951 (9.37), 7.304 (1.59), 7.321 (2.83), 7.347 (1.51), 7.550 (1.86), 7.557 (1.92), 7.576 (2.68), 7.598 (1.83), 7.605 (1.69), 7.804 (1.92), 8.275 (10.57), 8.297 (10.00), 8.614 (6.93), 10.490 (4.90), 10.514 (4.63). |
| **147** | *rac*-1-(2,4-Difluorphenyl)-7-(dimethyl-amino)-4-oxo-*N*-(1,1,1-trifluorpropan-2-yl)-1,4-dihydro-1,8-naphthyridin-3-carbonsäureamid 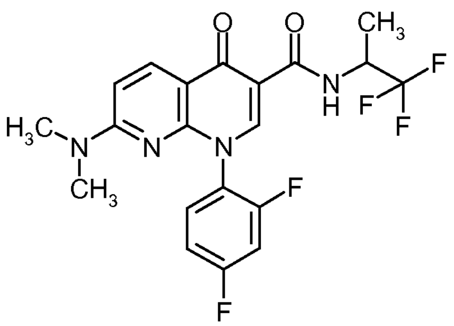 (19 % d. Th.) | LC-MS (Methode 1): $R_t$ = 1.12 min MS (ESpos): m/z = 441.2 $[M+H]^+$ $^1$H-NMR (400 MHz, DMSO-$d_6$) $\delta$ [ppm]: - 0.150 (1.12), -0.020 (3.11), -0.018 (3.55), - 0.016 (4.21), -0.013 (5.41), -0.009 (14.12), 0.006 (5.28), 0.007 (8.66), 0.012 (1.18), 0.145 (1.10), 1.360 (16.00), 1.377 (15.75), 2.520 (2.59), 2.523 (2.85), 2.526 (2.97), 2.890 (1.83), 2.943 (6.81), 4.860 (1.18), 4.880 (1.75), 4.901 (1.76), 4.919 (1.11), 6.927 (8.83), 6.949 (8.89), 7.301 (1.35), 7.305 (1.53), 7.308 (1.40), 7.322 (2.65), 7.327 (2.68), 7.344 (1.47), 7.348 (1.54), 7.351 (1.34), 7.551 (1.61), 7.558 (1.67), 7.576 (2.49), 7.599 (1.58), 7.606 (1.51), 7.777 (1.17), 7.791 (1.44), 7.799 (1.42), 7.808 (1.45), 7.823 (1.18), 8.267 (10.58), 8.290 (9.97), 8.607 (6.25), 10.543 (3.19), 10.566 (3.05). |

(fortgesetzt)

| Bsp. | | Analytische Daten |
|---|---|---|
| 148 | *rac*-*N*-(1-Cyclopropyl-2,2,2-trifluorethyl)-1 - (2,4-difluorphenyl)-7-(dimethylamino)-4-oxo-1,4-dihydro-1,8-naphthyridin-3-carbonsäureamid 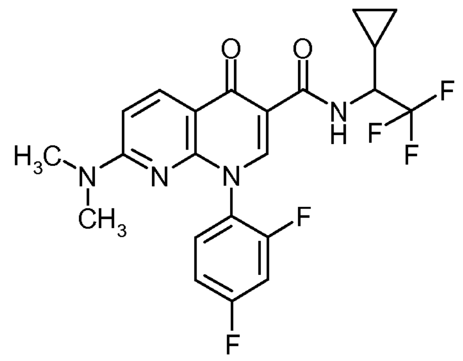 (74 % d. Th.) | LC-MS (Methode 1): $R_t$ = 1.19 min MS (ESpos): m/z = 467.1 $[M+H]^+$ $^1$H-NMR (400 MHz, DMSO-$d_6$) δ [ppm]: - 0.009 (4.53), 0.007 (4.41), 0.327 (2.82), 0.497 (2.60), 0.509 (3.84), 0.521 (3.29), 0.531 (2.72), 0.542 (2.82), 0.562 (3.54), 0.573 (3.26), 0.583 (2.87), 0.593 (2.39), 0.645 (2.81), 0.657 (2.81), 0.667 (2.39), 1.182 (2.22), 1.194 (3.92), 1.203 (2.68), 1.206 (2.53), 1.215 (3.80), 2.072 (3.10), 2.949 (9.68), 4.383 (3.15), 4.405 (3.10), 6.930 (14.26), 6.953 (14.42), 7.302 (2.24), 7.305 (2.12), 7.319 (4.12), 7.324 (4.25), 7.341 (2.27), 7.345 (2.40), 7.348 (2.24), 7.548 (2.78), 7.555 (2.90), 7.574 (4.04), 7.578 (4.12), 7.580 (3.77), 7.597 (2.92), 7.603 (2.87), 7.802 (3.38), 7.818 (3.35), 8.278 (16.00), 8.301 (15.12), 8.603 (14.27), 10.622 (4.05), 10.644 (3.96). |
| **149** | 1-(2,4-Difluorphenyl)-7-(dimethylamino)-*N*-(1,1,1,3,3,3-hexafluoropropan-2-yl)-4-oxo-1,4-dihydro-1,8-naphthyridin-3-carbonsäureamid 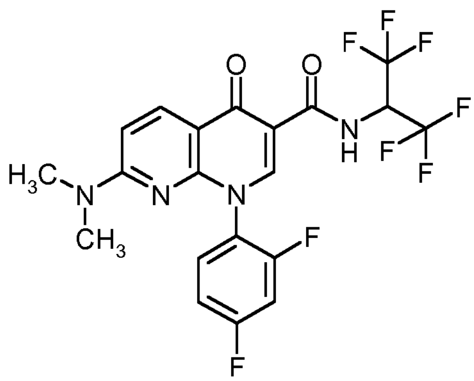 (24 % d. Th.) | LC-MS (Methode 1): $R_t$ = 1.33 min MS (ESpos): m/z = 495.2 $[M+H]^+$ $^1$H-NMR (400 MHz, DMSO-$d_6$) δ [ppm]: - 0.009 (5.51), 0.007 (5.59), 2.322 (1.36), 2.327 (1.80), 2.332 (1.30), 2.366 (1.80), 2.669 (2.09), 2.710 (1.92), 2.956 (4.44), 6.294 (1.45), 6.318 (1.56), 6.955 (8.61), 6.978 (8.77), 7.312 (1.37), 7.334 (2.59), 7.355 (1.58), 7.558 (1.81), 7.565 (1.71), 7.588 (2.61), 7.606 (1.75), 7.613 (1.76), 7.799 (1.84), 7.814 (2.03), 7.820 (3.27), 7.835 (3.25), 7.842 (1.91), 7.857 (1.68), 8.293 (9.07), 8.316 (8.62), 8.716 (16.00), 11.478 (4.79), 11.503 (4.49). |
| **150** | *rac*-1-(2,4-Difluorphenyl)-7-(dimethyl-amino)-4-oxo-*N*-(1,1,1-trifluoro-3-methylbutan-2-yl)-1,4-dihydro-1,8-naphthyridin-3-carbonsäureamid 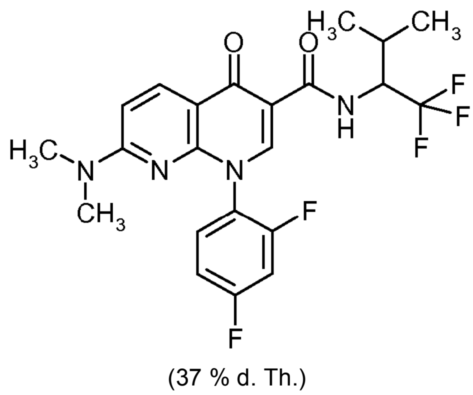 (37 % d. Th.) | LC-MS (Methode 1): $R_t$ = 1.22 min MS (ESpos): m/z = 469.2 $[M+H]^+$ $^1$H-NMR (400 MHz, DMSO-$d_6$) δ [ppm]: - 0.150 (1.70), -0.009 (16.00), 0.007 (14.74), 0.146 (1.81), 0.951 (8.74), 0.968 (8.97), 1.021 (7.68), 1.038 (7.74), 2.245 (1.16), 2.949 (3.60), 4.769 (1.02), 6.929 (5.45), 6.952 (5.59), 7.326 (1.56), 7.557 (1.03), 7.576 (1.53), 7.599 (1.02), 7.812 (1.31), 7.828 (1.28), 8.300 (6.15), 8.323 (5.88), 8.622 (4.12), 10.677 (2.77), 10.702 (2.67). |

(fortgesetzt)

| Bsp. | | Analytische Daten |
|---|---|---|
| **151** | 1-(2,4-Difluorphenyl)-7-(dimethylamino)-4-oxo-*N*-[1-(trifluormethyl)cyclopentyl]-1,4-dihydro-1,8-naphthyridin-3-carbonsäureamid<br>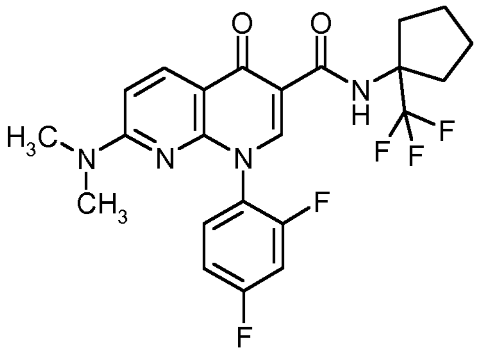<br>(28 % d. Th.) | LC-MS (Methode 1): $R_t$ = 1.33 min MS (ESpos): m/z = 481.2 $[M+H]^+$ $^1$H-NMR (400 MHz, DMSO-$d_6$) δ [ppm]: - 0.001 (16.00), 1.773 (0.24), 2.056 (0.17), 2.366 (0.17), 2.942 (0.38), 6.920 (0.41), 6.942 (0.42), 7.325 (0.15), 7.578 (0.14), 7.791 (0.18), 7.806 (0.18), 8.271 (0.46), 8.294 (0.44), 8.563 (0.84), 10.583 (0.47). |
| **152** | *N*-(Bicyclo[2.2.2]oct-1-yl)-1-(2-chlor-4-fluorphenyl)-7-(dimethylamino)-4-oxo-1,4-dihydro-1,8-naphthyridin-3-carbonsäureamid<br>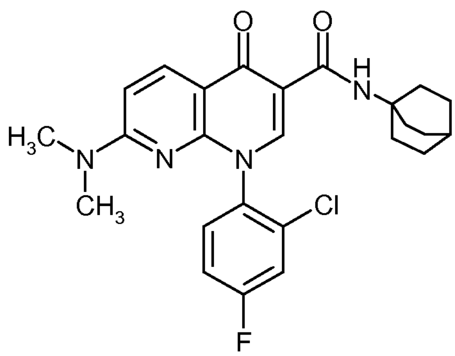<br>Verbindung aus Bsp. 60A und Bicyclo[2.2.2]oct-1-ylamin (34 % d. Th.) | LC-MS (Methode 1): $R_t$ = 1.30 min MS (ESpos): m/z = 469.1 $[M+H]^+$ $^1$H-NMR (400 MHz, DMSO-$d_6$) δ [ppm]: - 0.013 (6.24), 0.003 (6.28), 1.540 (2.38), 1.547 (3.51), 1.555 (3.25), 1.632 (9.41), 1.638 (9.20), 1.650 (8.75), 1.865 (10.16), 1.877 (8.30), 1.887 (10.18), 1.905 (7.68), 2.885 (6.02), 2.900 (7.48), 6.867 (7.56), 6.890 (7.82), 7.456 (3.08), 7.463 (3.36), 7.752 (3.65), 7.759 (6.96), 7.773 (7.07), 7.781 (7.29), 7.795 (3.43), 8.239 (8.11), 8.261 (7.78), 8.386 (16.00), 9.878 (7.48). |
| **153** | 1-(2-Chlor-4-fluorphenyl)-7-(dimethylamino)-4-oxo-*N*-[3-(trifluormethyl)tricyclo $[3.3.1.1^{3,7}]$ dec-1-yl]-1,4-dihydro-1,8-naphthyridin-3-carboxamid<br>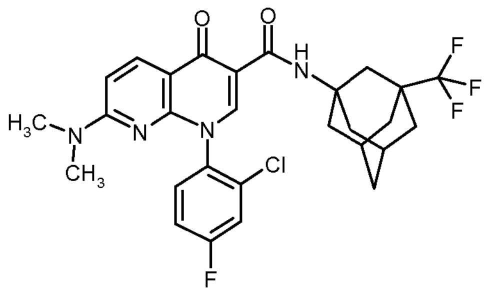<br>Verbindung aus Bsp. 60A und 3-(Trifluormethyl) tricyclo[3.3.1.1$^{3,7}$]decan-1-amin Hydrochlorid (33 % d. Th.) | LC-MS (Methode 1): $R_t$ = 1.39 min MS (ESpos): m/z = 563.1 $[M+H]^+$ $^1$H-NMR (400 MHz, DMSO-$d_6$) δ [ppm]: 1.639 (2.93), 1.671 (4.03), 1.713 (10.59), 1.914 (3.34), 1.945 (4.32), 2.106 (4.81), 2.129 (13.92), 2.231 (5.97), 2.908 (5.71), 6.886 (6.89), 6.909 (6.87), 7.457 (1.72), 7.471 (3.06), 7.478 (3.24), 7.500 (2.00), 7.773 (5.39), 7.796 (5.10), 7.810 (3.24), 8.249 (7.62), 8.271 (7.26), 8.411 (16.00), 10.124 (7.70). |

(fortgesetzt)

| Bsp. | | Analytische Daten |
|---|---|---|
| **154** | 1-(2-Chlor-4-fluorphenyl)-7-(dimethyl-amino)-4-oxo-*N*-(spiro[2.5]oct-1-yl)-1,4-dihydro-1,8-naphthyridin-3-carbonsäureamid 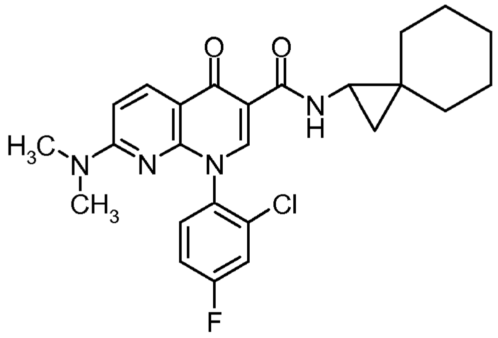 Verbindung aus Bsp. 60A und Spiro[2.5]octan-1-amin (13 % d. Th.) | LC-MS (Methode 1): $R_t$ = 1.36 min MS (ESpos): m/z = 469.2 $[M+H]^+$ $^1$H-NMR (400 MHz, DMSO-$d_6$) δ [ppm]: - 0.009 (5.56), 0.007 (3.50), 0.337 (1.60), 0.350 (3.09), 0.362 (2.78), 0.375 (1.28), 0.725 (1.89), 0.739 (2.80), 0.757 (1.66), 1.269 (1.62), 1.328 (1.59), 1.440 (3.49), 1.470 (6.97), 2.669 (0.55), 2.721 (1.33), 2.732 (2.29), 2.751 (2.17), 2.763 (1.19), 2.909 (6.10), 6.877 (7.56), 6.900 (7.61), 7.445 (1.59), 7.453 (1.80), 7.467 (2.80), 7.474 (2.89), 7.488 (1.78), 7.495 (1.91), 7.764 (3.55), 7.771 (5.42), 7.786 (6.06), 7.793 (5.35), 7.802 (1.98), 7.807 (2.24), 7.824 (1.44), 8.254 (8.13), 8.277 (7.61), 8.437 (0.53), 8.468 (16.00), 10.048 (2.79), 10.062 (2.29). |
| **155** | *N-tert.*-butyl-1-(2-chlor-4-fluorphenyl)-7-(dimethylamino)-4-oxo-1,4-dihydro-1,8-naphthyridin-3-carbonsäureamid 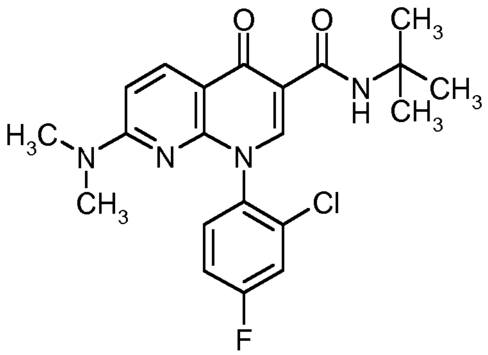 Verbindung aus Bsp. 60A und *tert.*-Butylamin (42 % d. Th.) | LC-MS (Methode 1): $R_t$ = 1.22 min MS (ESpos): m/z = 417.2 $[M+H]^+$ $^1$H-NMR (400 MHz, DMSO-$d_6$) δ [ppm]: - 0.009 (1.09), 0.007 (1.06), 1.387 (16.00), 1.405 (0.38), 2.522 (0.31), 2.908 (1.02), 6.880 (1.21), 6.903 (1.22), 7.445 (0.26), 7.452 (0.29), 7.466 (0.45), 7.473 (0.49), 7.487 (0.30), 7.494 (0.34), 7.763 (0.54), 7.769 (1.06), 7.784 (1.13), 7.791 (1.06), 7.805 (0.53), 8.253 (1.27), 8.275 (1.20), 8.423 (2.46), 10.033 (1.08). |
| **156** | 1-(2-Chlor-4-fluorphenyl)-7-(dimethyl-amino)-*N*-(1-methylcyclohexyl)-4-oxo-1,4-dihydro-1,8-naphthyridin-3-carbonsäureamid 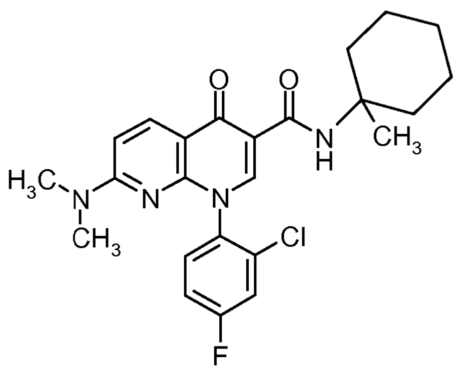 Verbindung aus Bsp. 60A und (1-Methylcyclohexyl) amin Hydrochlorid (22 % d. Th.) | LC-MS (Methode 1): $R_t$ = 1.28 min MS (ESpos): m/z = 457.1 $[M+H]^+$ $^1$H-NMR (400 MHz, DMSO-$d_6$) δ [ppm]: - 0.150 (0.51), -0.009 (4.50), 0.007 (3.96), 0.146 (0.48), 1.225 (0.49), 1.240 (0.53), 1.343 (1.13), 1.382 (16.00), 1.459 (0.75), 1.495 (2.35), 1.505 (2.20), 1.558 (0.57), 2.072 (0.55), 2.105 (0.98), 2.322 (0.45), 2.327 (0.59), 2.331 (0.44), 2.366 (0.57), 2.523 (1.30), 2.664 (0.52), 2.669 (0.68), 2.674 (0.50), 2.709 (0.65), 2.911 (2.70), 6.882 (3.55), 6.905 (3.65), 7.444 (0.76), 7.451 (0.90), 7.465 (1.31), 7.472 (1.48), 7.486 (0.91), 7.493 (1.02), 7.762 (1.52), 7.769 (1.68), 7.776 (1.81), 7.784 (1.75), 7.791 (3.15), 7.798 (1.70), 7.812 (1.59), 8.276 (3.96), 8.299 (3.71), 8.419 (8.02), 10.045 (2.98). |

(fortgesetzt)

| Bsp. | | Analytische Daten |
|---|---|---|
| **157** | 1-(2-Chlor-4-fluorphenyl)-7-(dimethyl-amino)-*N*-(3-ethyltricyclo[3.3.1.1$^{1,7}$]dec-1-yl)-4-oxo-1,4-dihydro-1,8-naphthyridin-3-carbonsäureamid 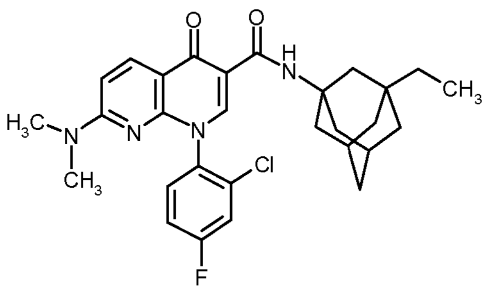 Verbindung aus Bsp. 60A und (3-Ethyl-1-adamantyl)amin Hydrochlorid (43 % d. Th.) | LC-MS (Methode 1): $R_t$ = 1.59 min MS (ESpos): m/z = 523.3 [M+H]$^+$ $^1$H-NMR (400 MHz, DMSO-$d_6$) δ [ppm]: - 0.150 (1.83), -0.009 (16.00), 0.007 (14.17), 0.146 (1.83), 0.766 (3.31), 0.785 (8.94), 0.803 (4.23), 1.125 (1.12), 1.144 (3.75), 1.163 (3.04), 1.182 (0.90), 1.403 (6.87), 1.528 (0.66), 1.560 (1.24), 1.613 (1.23), 1.644 (0.71), 1.742 (5.91), 1.959 (2.01), 2.016 (2.78), 2.044 (1.35), 2.108 (2.98), 2.322 (1.08), 2.326 (1.39), 2.331 (1.04), 2.365 (1.34), 2.664 (1.16), 2.669 (1.54), 2.674 (1.12), 2.709 (1.39), 2.904 (3.63), 6.878 (4.36), 6.901 (4.54), 7.446 (0.93), 7.453 (1.12), 7.467 (1.66), 7.475 (1.90), 7.488 (1.08), 7.495 (1.29), 7.764 (2.14), 7.771 (4.16), 7.785 (4.35), 7.792 (4.13), 7.806 (1.99), 8.248 (5.12), 8.270 (4.82), 8.392 (10.95), 9.982 (4.20). |

## Beispiel 158

1-(2,4-Difluorphenyl)-4-oxo-7-(propan-2-ylamino)-*N*-(tricyclo[3.3.1.1$^{3,7}$]dec-1-yl)-1,4-dihydro-1,8-naphthyridin-3-carbonsäureamid

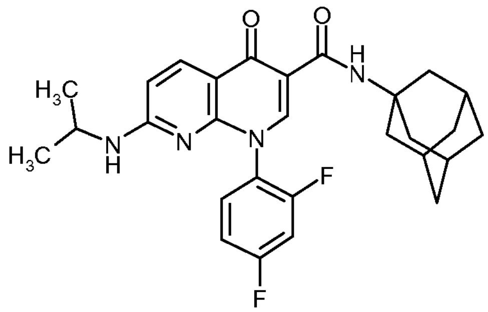

Zu 100 mg (0.28 mmol) der Verbindung aus Beispiel 38A und 70 mg (0.7 mmol) *N*-Methylmorpholin in 2.5 ml DMF wurden bei 0°C 0.56 ml (0.56 mmol) Chlorameisensäureisopropylester (1 M in Toluol) gegeben und 1 h bei 0°C gerührt. Dann wurden bei 0°C 34 mg (0.22 mmol) 1-Adamantanamin zugegeben und 2 Stunden bei 20°C gerührt. Nach 12 h wurde das Gemisch wurde über präparative HPLC gereinigt (Eluent: Acetonitril/Wasser-Gradient mit 0.1% Ameisensäure). Man erhielt 2 mg (2 % d. Th.) der Titelverbindung.
LC-MS (Methode 1): $R_t$ = 1.39 min; m/z = 493.3 [M+H]$^+$.
$^1$H-NMR (400 MHz, $CDCl_3$) δ [ppm]: 1.098 (4.13), 1.117 (4.71), 1.132 (3.93), 1.265 (2.61), 1.567 (12.87), 1.691 (1.63), 1.721 (5.40), 1.745 (5.60), 1.775 (1.65), 2.015 (1.00), 2.117 (5.05), 2.185 (16.00), 3.500 (0.95), 3.693 (0.81), 3.710 (1.29), 3.727 (1.30), 3.743 (0.82), 4.797 (1.15), 4.814 (1.12), 6.410 (2.16), 6.432 (2.22), 6.997 (1.04), 7.015 (2.62), 7.036 (2.60), 7.052 (1.03), 7.336 (0.81), 7.357 (1.29), 7.371 (1.35), 7.393 (0.61), 8.344 (2.01), 8.365 (1.98), 8.624 (6.01), 9.938 (2.22).

In Analogie zu Beispiel 158 wurden die in Tabelle 17 gezeigte Beispielverbindung hergestellt, indem die Verbindung aus Beispiel 38A mit den entsprechenden Aminen (bzw. deren Salzen) unter den beschriebenen Reaktionsbedingungen umgesetzt wurden. Abweichungen sind bei den jeweiligen Beispielen angegeben.

Tabelle 17:

| Bsp. | | Analytische Daten |
|---|---|---|
| **159** | *N*-(Bicyclo[2.2.2]oct-1-yl)-1-(2,4-difluorphenyl)-4-oxo-7-(propan-2-ylamino)-1,4-dihydro-1,8-naphthyridin-3-carbonsäureamid 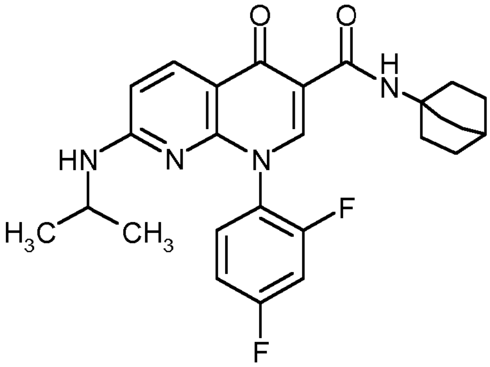 (10 % d. Th.) | LC-MS (Methode 1): $R_t$ = 1.27 min MS (ESpos): m/z = 467.2 [M+H]$^+$ $^1$H-NMR (400 MHz, DMSO-$d_6$) $\delta$ [ppm]: - 0.019 (1.74), -0.016 (2.07), -0.009 (6.78), 0.005 (3.03), 0.007 (4.37), 0.949 (9.89), 0.963 (11.01), 0.995 (13.19), 1.011 (11.80), 1.542 (3.78), 1.549 (5.44), 1.557 (4.95), 1.583 (1.41), 1.633 (15.56), 1.639 (15.24), 1.651 (14.55), 1.818 (3.29), 1.839 (3.03), 1.867 (16.00), 1.878 (13.44), 1.888 (15.97), 1.906 (11.40), 2.072 (2.38), 2.523 (3.78), 2.526 (4.13), 3.497 (1.95), 3.513 (1.94), 6.586 (5.23), 6.608 (5.28), 7.278 (2.03), 7.282 (2.25), 7.285 (2.19), 7.299 (3.89), 7.303 (4.11), 7.321 (2.28), 7.325 (2.35), 7.328 (2.17), 7.336 (2.29), 7.356 (2.15), 7.524 (2.49), 7.531 (2.79), 7.550 (3.94), 7.554 (4.11), 7.572 (2.66), 7.579 (2.48), 7.742 (4.31), 7.757 (3.59), 7.764 (5.12), 7.779 (4.76), 7.786 (2.93), 7.801 (2.21), 8.098 (4.22), 8.120 (4.08), 8.427 (15.57), 8.447 (2.59), 9.912 (9.63), 10.202 (1.52). |

**Beispiel 160**

1-(2,4-Difluorphenyl)-7-(methylamino)-4-oxo-*N*-[3-(trifluormethyl)tricyclo[3.31.1$^{3,7}$]dec-1-yl]-1,4-dihydro-1,8-naphthyridin-3-carbonsäureamid

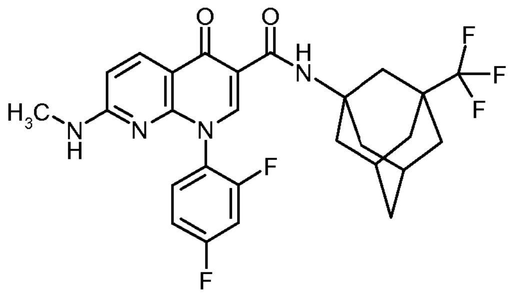

Zu 80 mg (0.24 mmol) der Verbindung aus Beispiel 37A und 61 mg (0.6 mmol) N-Methylmorpholin in 2.2 ml DMF wurden bei 0°C 0.48 ml (0.48 mmol) Chlorameisensäureisopropylester (1 M in Toluol) gegeben und 1 h bei 0°C gerührt. Dann wurden bei 0°C 34 mg (0.22 mmol) 1-(3-Trifluormethyl)adamantanamin Hydrochlorid zugegeben und 2 Stunden bei 20°C gerührt. Nach 12 h wurde das Gemisch wurde über präparative HPLC gereinigt (Eluent: Acetonitril/Wasser-Gradient mit 0.1% Ameisensäure). Man erhielt 57 mg (42 % d. Th.) der Titelverbindung.
LC-MS (Methode 1): $R_t$ = 1.27 min; m/z = 533.1 [M+H]$^+$.
$^1$H-NMR (400 MHz, DMSO-$d_6$) $\delta$ [ppm]: -0.009 (6.50), 0.007 (6.34), 1.639 (3.23), 1.668 (4.16), 1.714 (11.64), 1.915 (4.17), 1.942 (5.42), 2.107 (6.21), 2.128 (16.00), 2.230 (6.76), 3.672 (1.64), 5.753 (4.27), 6.622 (5.65), 6.645 (5.71), 7.295 (1.67), 7.317 (3.10), 7.338 (1.74), 7.538 (2.09), 7.544 (2.19), 7.567 (3.02), 7.586 (2.14), 7.593 (2.09), 7.756 (2.14), 7.778 (4.13), 7.793 (4.26), 7.815 (2.78), 8.141 (1.63), 8.447 (11.87), 10.142 (6.07).

In Analogie zu Beispiel 160 wurden die in Tabelle 18 gezeigten Beispielverbindungen hergestellt, indem die Verbindung aus Beispiel 37A mit den entsprechenden Aminen (bzw. deren Salzen) unter den beschriebenen Reaktionsbedingungen umgesetzt wurden. Abweichungen sind bei den jeweiligen Beispielen angegeben.

Tabelle 18:

| Bsp. | | Analytische Daten |
|---|---|---|
| **161** | 1-(2,4-Difluorphenyl)-*N*-(4,4-difluor-tricyclo [3.3.1.1$^{3,7}$]dec-1-yl)-7-(methyl-amino)-4-oxo-1,4-dihydro-1,8-naphthyridin-3-carbonsäureamid 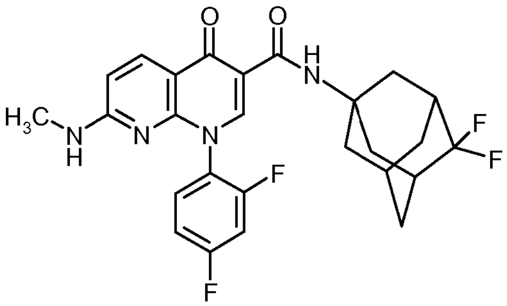 (56 % d. Th.) | LC-MS (Methode 1): $R_t$ = 1.16 min MS (ESpos): m/z = 501.3 $[M+H]^+$ $^1$H-NMR (400 MHz, DMSO-$d_6$) δ [ppm]: -0.005 (16.00), 1.740 (1.03), 1.783 (7.48), 1.820 (1.02), 2.061 (12.90), 2.272 (6.60), 2.297 (4.96), 6.615 (3.10), 6.637 (3.14), 7.287 (1.11), 7.309 (2.02), 7.329 (1.05), 7.530 (1.11), 7.537 (1.20), 7.559 (1.89), 7.578 (1.13), 7.585 (1.13), 7.754 (1.14), 7.769 (1.46), 7.775 (2.28), 7.790 (2.33), 7.797 (1.64), 7.812 (1.67), 8.116 (1.04), 8.136 (1.04), 8.442 (5.93), 10.085 (3.75). |
| **162** | *N*-(Bicyclo[2.2.2]oct-1-yl)-1-(2,4-difluorphenyl)-7-(methylamino)-4-oxo-1,4-dihydro-1,8-naphthyridin-3-carbonsäureamid 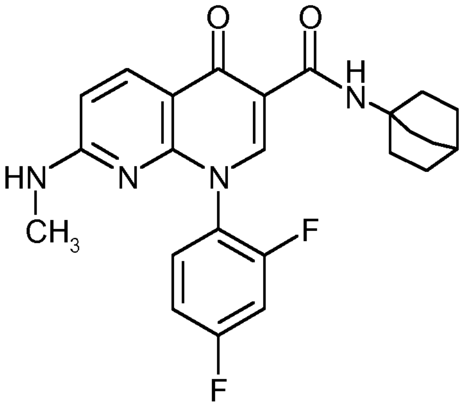 (9 % d. Th.) | LC-MS (Methode 1): $R_t$ = 1.17 min MS (ESpos): m/z = 439.1 $[M+H]^+$ $^1$H-NMR (400 MHz, DMSO-$d_6$) δ [ppm]: 1.546 (5.04), 1.631 (14.89), 1.863 (15.35), 1.885 (16.00), 1.903 (11.48), 2.068 (2.97), 6.605 (5.86), 6.627 (5.97), 7.304 (3.93), 7.531 (2.28), 7.555 (3.73), 7.573 (2.19), 7.768 (4.61), 7.783 (4.99), 7.805 (3.68), 8.111 (2.11), 8.423 (11.13), 9.900 (7.11). |

**Beispiel 163**

*N*-(2,6-Dichlorphenyl)-1-(2,4-difluorphenyl)-4-oxo-7-[(2,2,2-trifluorethyl)amino]-1,4-dihydro-1,8-naphthyridin-3-carbonsäureamid

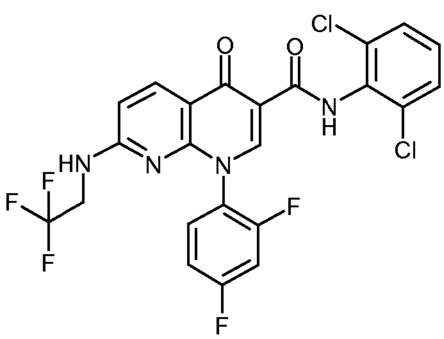

152 mg (0.94 mmol) 2,6-Dichloranilin wurden in 10 ml Dichlormethan gelöst, mit 0.94 ml (0.94 mmol) Trimethylaluminium (1 M Lösung in Toluol) versetzt und eine Stunde lang bei 23°C (unter Argon) gerührt. Dann wurden 200

mg (0.47 mmol) der Verbindung aus Beispiel 30A zugegeben und die Mischung 16 h bei 23°C gerührt. Es wurden 5 ml Wasser zugegeben, die Mischung dann über Kieselgur filtriert, mit Ethylacetat und Methanol gewaschen und die vereinten Eluate im Vakuum eingeengt. Der Rückstand wurde durch präparative HPLC gereinigt (Methode 7). Man erhielt 57 mg (22 % d. Th.) der Titelverbindung.
LC-MS (Methode 1): $R_t$ = 1.11 min; m/z = 543.1 $[M+H]^+$.
$^1$H-NMR (400 MHz, DMSO-$d_6$) $\delta$ [ppm]: -0.031 (2.20), -0.020 (3.38), -0.017 (3.92), -0.009 (13.57), 0.005 (4.57), 0.007 (7.84), 1.156 (3.89), 1.174 (7.63), 1.192 (3.94), 1.249 (2.03), 1.987 (12.97), 2.520 (4.06), 2.523 (4.45), 3.863 (1.67), 3.885 (1.55), 4.020 (3.06), 4.037 (2.99), 6.844 (2.50), 6.866 (2.42), 7.324 (2.25), 7.330 (2.28), 7.355 (4.12), 7.375 (5.28), 7.396 (4.49), 7.540 (1.76), 7.547 (1.79), 7.577 (16.00), 7.589 (2.18), 7.597 (12.49), 7.815 (1.39), 7.830 (1.72), 7.837 (2.62), 7.852 (2.82), 7.859 (1.49), 8.359 (5.18), 8.381 (4.91), 8.500 (1.33), 8.730 (11.18), 11.946 (7.80).

**Beispiel 164**

Methyl-4-{6-[(2,6-dichlorphenyl)carbamoyl]-8-(2,4-difluorphenyl)-5-oxo-5,8-dihydro-1,8-naphthyridin-2-yl}piperazin-1-carboxylat

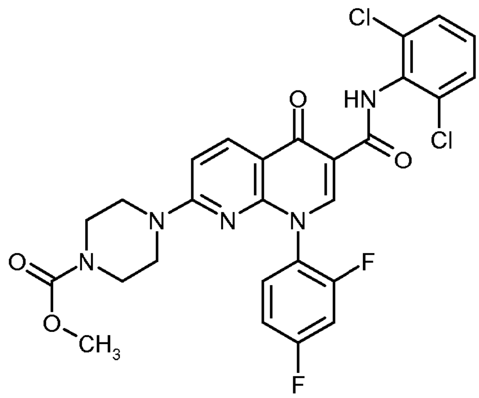

77 mg (0.47 mmol) 2,6-Dichloranilin wurden in Dichlormethan gelöst, mit 0.47 ml (0.47 mmol) Trimethylaluminium (1 M Lösung in Toluol) versetzt und eine Stunde lang bei 23°C (unter Argon) gerührt. Dann wurden 120 mg (0.24 mmol) der Verbindung aus Beispiel 29A zugegeben und die Mischung 16 h bei 23°C gerührt. Die Mischung wurde durch präparative HPLC gereinigt (Methode 7). Man erhielt 80 mg (57 % d. Th.) der Titelverbindung.
LC-MS (Methode 1): $R_t$ = 1.10 min; m/z = 588.1 $[M+H]^+$.
$^1$H-NMR (400 MHz, DMSO-$d_6$) $\delta$ [ppm]: -0.009 (2.56), 0.007 (2.34), 1.156 (2.57), 1.174 (5.27), 1.192 (2.63), 1.987 (9.53), 2.522 (1.04), 2.524 (0.92), 3.394 (3.43), 3.407 (2.81), 3.536 (3.09), 3.610 (16.00), 4.002 (0.76), 4.020 (2.20), 4.038 (2.22), 4.055 (0.75), 7.132 (2.23), 7.155 (2.27), 7.336 (0.99), 7.354 (1.74), 7.375 (2.28), 7.395 (1.92), 7.576 (6.94), 7.597 (6.13), 7.840 (1.12), 7.855 (1.12), 8.381 (3.02), 8.403 (2.84), 8.713 (6.16), 11.950 (3.67).

**Beispiel 165**

1-(2,4-Difluorphenyl)-4-oxo-7-(2-oxopiperidin-1-yl)-*N*-(tricyclo[3.3.1.1$^{3,7}$]dec-1-yl)-1,4-dihydro-1,8-naphthyridin-3-carbonsäureamid

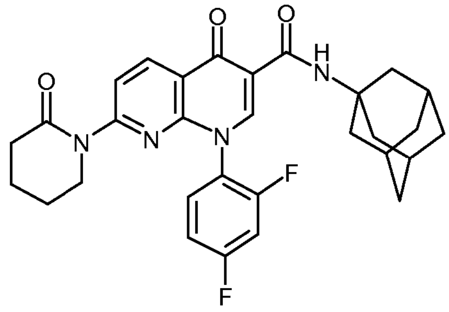

104 mg (0.32 mmol) Cäsiumcarbonat, 5 mg (0.02 mmol) Palladium(II)acetat und 12 mg (0.02 mmol) Xantphos wurden in 5 ml Dioxan unter Argon für 10 Minuten bei 20°C gerührt. Dann wurden 100 mg (0.21 mmol) der Verbindung aus Beispiel 65A und 25 mg (0.26 mmol) δ-Valerolactam zugegeben und die Mischung für 1 h bei 80°C gerührt. Anschliessend wurde das Gemisch auf 30 ml Wasser gegeben und mit 1 M wässr. Salzsäure auf pH 1 gebracht. Der ausgefallene Feststoff wurde abgesaugt, mit Wasser, Petrolether und Acetonitril gewaschen. Der Rückstand wurde dann in DCM gelöst und nach Zugabe von Aktivkohle bei RT gerührt. Die Mischung wurde über Kieselgur filtriert und die flüchtigen Bestandteile anschließend im Vakuum entfernt. Der Rückstand wurde über präparative Dünnschichtchromatographie gereinigt (Eluent: THF; Extraktionsmittel Ethylacetat). Die produkthaltigen Fraktionen wurden im Vakuum eingeengt und der Rückstand anschließend mit 1 M wässr. Salzsäure verrührt. Der Niederschlag wurde dann abfiltriert, mit Wasser, Acetonitril und Ethylacetat gewaschen und im Hochvakuum getrocknet. Man erhielt 12 mg (10 % d. Th.) der Titelverbindung.
LC-MS (Methode 1): $R_t$ = 1.35 min; m/z = 533.5 $[M+H]^+$.
$^1$H-NMR (400 MHz, $CDCl_3$) δ [ppm]: 1.265 (0.20), 1.442 (0.16), 1.567 (16.00), 1.730 (0.51), 1.750 (0.51), 1.852 (0.42), 2.129 (0.45), 2.187 (1.47), 3.597 (0.23), 7.040 (0.12), 7.060 (0.35), 7.079 (0.32), 7.394 (0.12), 7.409 (0.12), 8.176 (0.45), 8.198 (0.48), 8.672 (0.49), 8.694 (0.44), 8.807 (0.77), 9.701 (0.22).

## Beispiel 166

1-(2,4-Difluorphenyl)-4-oxo-7-(2-oxopyrrolidin-1-yl)-*N*-(tricyclo[3.3.1.1$^{3,7}$ ]dec-1-yl)-1,4-dihydro-1,8-naphthyridin-3 -carbonsäureamid

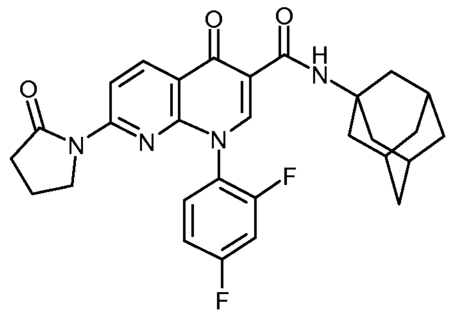

208 mg (0.64 mmol) Cäsiumcarbonat, 10 mg (0.04 mmol) Palladium(II)acetat und 25 mg (0.04 mmol) Xantphos wurden in 5.6 ml Dioxan (unter Argon) 10 Minuten lang bei 20°C gerührt. Dann wurden 200 mg (0.43 mmol) der Verbindung aus Beispiel 65A und 36 mg (0.43 mmol) 2-Pyrrolidinon zugegeben und die Mischung 22 h lang bei 110°C gerührt. Anschliessend wurde das Gemisch filtriert und das Filtrat über präparative HPLC gereinigt (Eluent: Acetonitril/Wasser-Gradient mit 0.1% Ameisensäure). Man erhielt 13 mg (6 % d. Th.) der Titelverbindung.
LC-MS (Methode 1): $R_t$ = 1.33 min; m/z = 519.2 $[M+H]^+$.
$^1$H-NMR (400 MHz, DMSO-$d_6$) δ [ppm]: -0.150 (1.78), -0.009 (16.00), 0.008 (15.04), 0.146 (1.89), 1.146 (0.80), 1.235 (0.55), 1.679 (4.33), 1.956 (0.66), 1.976 (0.50), 2.072 (9.96), 2.322 (1.13), 2.327 (1.68), 2.331 (1.16), 2.365 (1.94), 2.523 (4.23), 2.525 (3.79), 2.558 (2.30), 2.575 (1.51), 2.582 (1.43), 2.595 (0.88), 2.665 (1.49), 2.669 (1.89), 2.674 (1.46), 2.709 (2.20), 3.286 (1.67), 3.426 (0.36), 3.549 (0.83), 3.570 (0.83), 3.587 (0.49), 7.354 (0.52), 7.616 (0.53), 7.642 (0.36), 7.845 (0.57), 7.860 (0.58), 8.464 (1.62), 8.486 (1.97), 8.658 (1.94), 8.680 (1.65), 8.705 (2.97), 9.725 (1.56).
In Analogie zu Beispiel 166 wurden die in Tabelle 19 gezeigten Beispielverbindungen hergestellt, indem die Verbindung aus Beispiel 65A mit den entsprechenden Aminen (bzw. deren Salzen) unter den beschriebenen Reaktionsbedingungen umgesetzt wurden. Abweichungen sind bei den jeweiligen Beispielen angegeben.

Tabelle 19:

| Bsp. | | Analytische Daten |
|---|---|---|
| **167** | 1-(2,4-Difluorphenyl)-7-(1,1-dioxido-1,2-thiazolidin-2-yl)-4-oxo-*N*-(tricyclo-[3.3.1.1$^{3,7}$]dec-1-yl)-1,4-dihydro-1,8-naphthyridin-3-carbonsäureamid 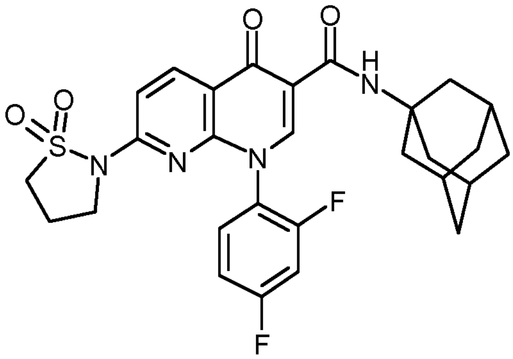 (52 % d.Th.) | LC-MS (Methode 1): $R_t$ = 1.26 min MS (ESpos): m/z = 555.2 $[M+H]^+$ $^1$H-NMR (400 MHz, DMSO-$d_6$) δ [ppm]: - 0.013 (1.19), 0.003 (1.16), 1.672 (7.13), 2.063 (16.00), 2.067 (13.86), 2.091 (0.91), 2.297 (1.24), 2.315 (1.97), 2.332 (1.36), 3.575 (0.95), 3.594 (2.09), 3.606 (2.00), 3.616 (1.55), 3.634 (0.64), 7.320 (0.73), 7.325 (0.76), 7.340 (2.97), 7.362 (2.60), 7.537 (0.49), 7.555 (0.69), 7.559 (0.70), 7.578 (0.50), 7.813 (0.54), 7.820 (0.91), 7.835 (0.91), 7.842 (0.52), 8.612 (2.64), 8.634 (2.52), 8.653 (4.54), 9.730 (2.56). |
| **168** | 1-(2,4-Difluorphenyl)-4-oxo-7-(2-oxoazetidin-1-yl)-*N*-(tricyclo[3.3.1.1$^{3,7}$]dec-1-yl)-1,4-dihydro-1,8-naphthyridin-3-carbonsäureamid 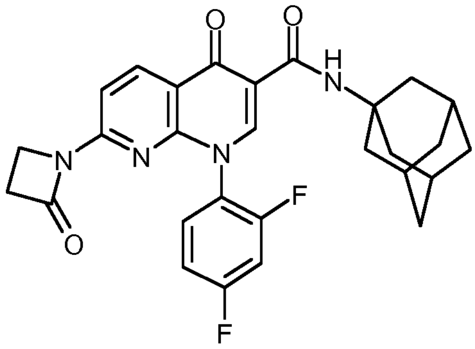 (9 % d. Th.) | LC-MS (Methode 1): $R_t$ = 1.31 min MS (ESpos): m/z = 505.4 $[M+H]^+$ $^1$H-NMR (400 MHz, DMSO-$d_6$) δ [ppm]: 1.156 (0.38), 1.174 (0.71), 1.192 (0.39), 1.234 (0.73), 1.676 (7.37), 1.987 (1.27), 2.067 (16.00), 2.327 (0.39), 2.365 (0.42), 2.669 (0.41), 2.709 (0.40), 3.088 (1.46), 3.100 (1.49), 3.386 (1.28), 4.020 (0.29), 4.038 (0.29), 7.327 (0.44), 7.349 (0.82), 7.366 (0.61), 7.576 (0.50), 7.598 (0.77), 7.618 (0.50), 7.696 (2.27), 7.717 (2.38), 7.810 (0.46), 7.832 (0.88), 7.847 (0.90), 7.869 (0.46), 8.654 (2.35), 8.675 (2.58), 8.681 (4.17), 9.709 (2.41). |
| **169** | 1 -(2,4-Difluorphenyl)-7- [methyl(methylsulfonyl) amino]-4-oxo-*N*-(tricyclo-[3.3.1.1$^{3,7}$]dec-1-yl)-1,4-dihydro-1,8-naphthyridin-3-carbonsäureamid 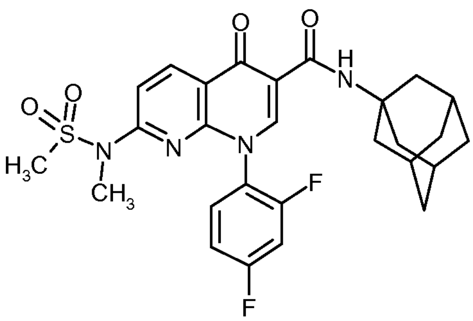 (45 % d. Th.) | LC-MS (Methode 1): $R_t$ = 1.26 min MS (ESpos): m/z = 543.2 $[M+H]^+$ $^1$H-NMR (400 MHz, DMSO-$d_6$) δ [ppm]: - 0.154 (0.08), -0.005 (16.00), 0.141 (0.08), 1.674 (2.57), 2.068 (8.74), 3.036 (4.34), 3.226 (4.18), 7.329 (0.13), 7.351 (0.27), 7.372 (0.14), 7.540 (0.80), 7.562 (0.83), 7.576 (0.17), 7.594 (0.25), 7.617 (0.17), 7.624 (0.16), 7.823 (0.15), 7.845 (0.31), 7.860 (0.31), 7.867 (0.18), 7.881 (0.15), 8.630 (0.81), 8.653 (0.77), 8.699 (1.49), 9.689 (0.87). |

(fortgesetzt)

| Bsp. | | Analytische Daten |
|---|---|---|
| **170** | 1-(2,4-Difluorphenyl)-7-[(4S)-4-hydroxy-2-oxopyrrolidin-1-yl]-4-oxo-*N*-(tricyclo-[3.3.1.1$^{3,7}$]dec-1-yl)-1,4-dihydro-1,8-naphthyridin-3-carbonsäureamid 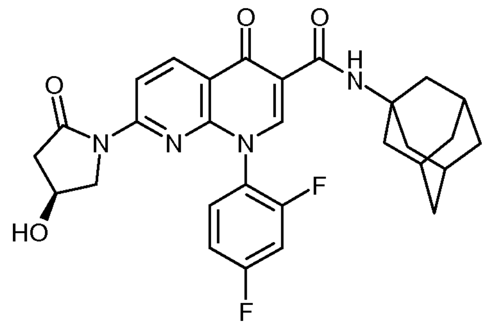 (34 % d. Th.) | LC-MS (Methode 1): $R_t$ = 1.16 min MS (ESpos): m/z = 535.2 $[M+H]^+$ $^1$H-NMR (400 MHz, DMSO-$d_6$) δ [ppm]: - 0.149 (1.21), -0.008 (10.86), 0.008 (9.92), 0.146 (1.23), 1.147 (0.47), 1.676 (8.65), 2.059 (8.89), 2.072 (16.00), 2.327 (1.28), 2.366 (1.28), 2.391 (0.39), 2.524 (2.59), 2.670 (1.06), 2.674 (0.83), 2.710 (1.13), 2.899 (0.30), 2.932 (0.38), 3.288 (1.29), 3.441 (0.36), 3.466 (0.65), 3.499 (0.39), 3.637 (0.32), 3.667 (0.50), 4.284 (0.71), 5.283 (0.52), 5.343 (0.51), 7.368 (0.63), 7.601 (0.62), 7.621 (0.76), 7.649 (0.38), 7.845 (0.52), 7.864 (0.57), 8.365 (0.43), 8.467 (0.57), 8.481 (0.65), 8.503 (0.80), 8.666 (2.59), 8.688 (2.08), 8.704 (4.17), 9.725 (2.21). |
| **171** | 7-(5,5-Difluor-2-oxopiperidin-1-yl)-1-(2,4-difluorphenyl)-4-oxo-*N*-(tricyclo [3.3.1.1$^{3,7}$]-dec-1-yl)-1,4-dihydro-1,8-naphthyridin-3-carbonsäureamid 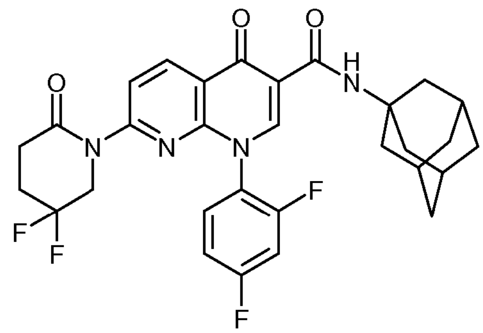 (77% d. Th.) | LC-MS (Methode 1): $R_t$ = 1.31 min MS (ESpos): m/z = 569.3 $[M+H]^+$ H-NMR (400 MHz, DMSO-$d_6$) δ [ppm]: 1.680 (7.72), 2.075 (16.00), 2.366 (1.00), 2.387 (0.85), 2.406 (1.26), 2.424 (0.94), 2.444 (0.66), 2.720 (1.48), 2.738 (2.68), 2.756 (1.18), 3.955 (1.20), 3.987 (2.25), 4.019 (1.16), 7.388 (0.84), 7.641 (0.46), 7.661 (0.78), 7.683 (0.45), 7.857 (0.42), 7.878 (0.82), 7.894 (0.81), 7.915 (0.40), 8.155 (1.85), 8.177 (2.01), 8.670 (2.00), 8.692 (1.84), 8.749 (3.57), 9.679 (2.44). |
| **172** | 1-(2,4-Difluorphenyl)-4-oxo-7-(3-oxomorpholin-4-yl)-*N*-(tricyclo [3.3.1.1$^{3,7}$]dec-1-yl)-1,4-dihydro-1,8-naphthyridin-3-carbonsäureamid 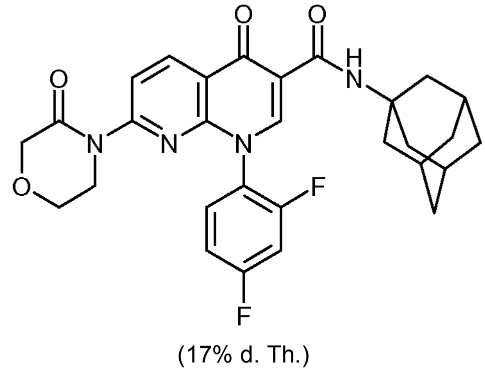 (17% d. Th.) | LC-MS (Methode 1): $R_t$ = 1.28 min MS (ESpos): m/z = 535.2 $[M+H]^+$ $^1$H-NMR (400 MHz, DMSO-$d_6$) δ [ppm]: - 0.150 (1.50), -0.009 (13.61), 0.007 (12.45), 0.146 (1.55), 1.146 (0.70), 1.590 (0.77), 1.681 (7.53), 2.075 (16.00), 2.322 (1.13), 2.327 (1.57), 2.331 (1.05), 2.366 (1.89), 2.522 (3.55), 2.665 (1.16), 2.669 (1.59), 2.673 (1.17), 2.709 (1.95), 3.544 (1.38), 3.558 (2.29), 3.570 (1.65), 3.898 (1.63), 4.277 (3.49), 4.284 (3.49), 7.355 (0.77), 7.605 (0.85), 7.861 (0.97), 7.876 (0.88), 8.339 (2.75), 8.361 (3.02), 8.681 (3.13), 8.703 (2.70), 8.739 (4.78), 9.687 (2.58). |

(fortgesetzt)

| Bsp. | | Analytische Daten |
|---|---|---|
| **173** | *rac*-1-(2,4-Difluorphenyl)-7-[4-hydroxy-2-oxopiperidin-1-yl]-4-oxo-*N*-(tricyclo-[3.3.1.1$^{3,7}$]dec-1-yl)-1,4-dihydro-1,8-naphthyridin-3-carbonsäureamid 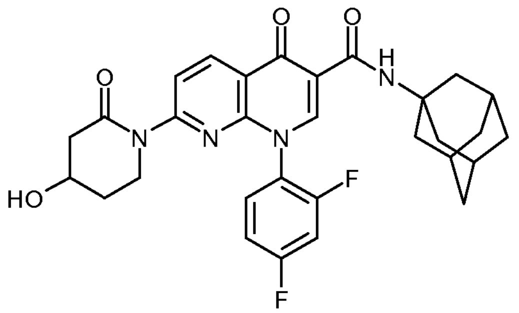 (15% d. Th.) | LC-MS (Methode 1): $R_t$ = 1.12 min MS (ESpos): m/z = 549.3 $[M+H]^+$ $^1$H-NMR (400 MHz, DMSO-$d_6$) δ [ppm]: - 0.150 (1.72), -0.009 (14.59), 0.007 (14.85), 0.083 (0.31), 0.146 (1.85), 1.146 (0.73), 1.156 (0.94), 1.174 (1.95), 1.192 (0.90), 1.237 (0.44), 1.672 (7.27), 1.987 (3.59), 2.059 (16.00), 2.322 (1.28), 2.327 (1.93), 2.365 (2.81), 2.523 (5.04), 2.669 (2.18), 2.673 (1.72), 2.709 (2.92), 3.432 (0.48), 3.682 (0.31), 4.020 (1.01), 4.037 (0.80), 4.056 (0.34), 6.840 (0.42), 7.319 (0.42), 7.337 (0.82), 7.360 (0.57), 7.575 (0.55), 7.606 (0.86), 7.631 (0.52), 7.821 (0.76), 7.837 (0.59), 8.470 (0.46), 8.519 (1.15), 9.805 (0.84), 12.106 (1.28). |

## Beispiel 174

1-(2,4-Difluorphenyl)-7-[(4*S*)-4-hydroxy-2-oxopyrrolidin-1-yl]-4-oxo-*N*-[(2*S*)-1,1,1-trifluorbutan-2-yl]-1,4-dihydro-1,8-naphthyridin-3-carbonsäureamid

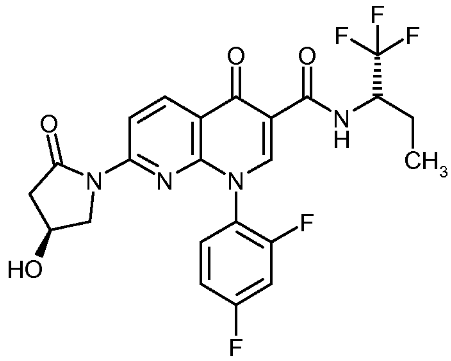

412 mg (1.3 mmol) Cäsiumcarbonat, 34 mg (0.15 mmol) Palladium(II)acetat und 88 mg (0.15 mmol) Xantphos wurden in Dioxan (unter Argonatmosphäre) für 10 Minuten bei 20°C gerührt. Dann wurden 400 mg (0.84 mmol) der Verbindung aus Beispiel 68A und 85 mg (0.84 mmol) (4*S*)-4-Hydroxy-pyrrolidin-2-on zugegeben und die Mischung für 40 min bei 80°C gerührt. Anschliessend wurde das Gemisch auf Wasser gegeben und mit 1 M wässr. Salzsäure auf pH 1 gebracht. Der ausgefallene Feststoff wurde abgesaugt, mit Wasser und Petrolether gewaschen, und anschliessend durch Säulenchromatographie (Kieselgelkartusche; Cyclohexan/ Ethylacetat-Gradient (5:1→ 2:1 →1:1) gereinigt. Man erhielt 169 mg (38 % d. Th.) der Titelverbindung.
LC-MS (Methode 1): $R_t$ = 1.01 min; m/z = 511.3 $[M+H]^+$.
$^1$H-NMR (400 MHz, DMSO-$d_6$) δ [ppm]: -0.001 (16.00), 0.958 (1.16), 0.976 (2.31), 0.995 (1.20), 1.174 (0.23), 1.234 (0.33), 1.565 (0.20), 1.686 (0.37), 1.894 (0.30), 1.987 (0.32), 2.336 (0.30), 2.379 (0.33), 2.943 (0.26), 3.437 (0.27), 3.469 (0.43), 3.503 (0.26), 3.674 (0.30), 4.288 (0.45), 4.764 (0.26), 5.293 (0.38), 5.343 (0.30), 7.373 (0.37), 7.629 (0.42), 7.877 (0.31), 8.516 (0.39), 8.538 (0.45), 8.698 (1.21), 8.720 (1.05), 8.849 (0.86), 10.208 (0.55), 10.232 (0.57).

In Analogie zu Beispiel 174 wurden die in Tabelle 20 gezeigten Beispielverbindungen hergestellt, indem die jeweiligen Verbindungen aus den Beispielen 66A-70A mit den entsprechenden Aminen (bzw. deren Salzen) unter den beschriebenen Reaktionsbedingungen umgesetzt wurden. Abweichungen sind bei den jeweiligen Beispielen angegeben.

Tabelle 20:

| Bsp. | | Analytische Daten |
|---|---|---|
| **175** | 1-(2,4-Difluorphenyl)-7-[(4*S*)-4-hydroxy-2-oxopyrrolidin-1-yl]-4-oxo-*N*-[(2*R*)-1,1,1-trifluorbutan-2-yl]-1,4-dihydro-1,8-naphthyridin-3-carbonsäureamid 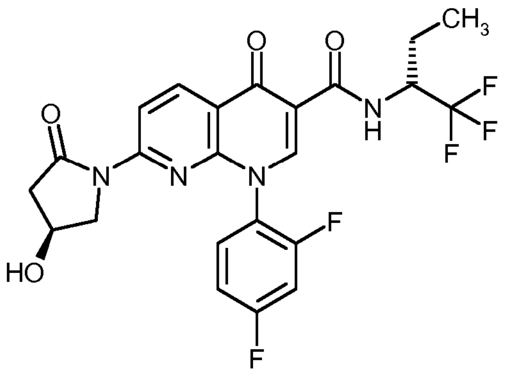 Verbindung aus Bsp. 67A und (4*S*)-4-Hydroxypyrrolidin-2-on (48% d. Th.) | LC-MS (Methode 1): $R_t$ = 0.99 min; m/z = 511.4 $[M+H]^+$. $^1$H-NMR (500 MHz, DMSO-$d_6$) δ [ppm]: - 0.009 (0.77), -0.003 (16.00), 0.004 (0.49), 0.964 (5.50), 0.979 (11.59), 0.994 (5.60), 1.634 (0.80), 1.640 (0.40), 1.649 (1.07), 1.654 (0.94), 1.662 (1.26), 1.669 (1.12), 1.677 (1.07), 1.683 (1.16), 1.697 (0.87), 1.865 (0.40), 1.872 (0.89), 1.879 (1.04), 1.886 (1.06), 1.894 (1.17), 1.899 (1.06), 1.908 (0.94), 1.914 (0.79), 1.922 (0.66), 2.342 (1.02), 2.362 (1.10), 2.376 (1.18), 2.397 (1.08), 2.898 (0.72), 2.910 (0.78), 2.933 (1.41), 2.945 (1.40), 2.967 (0.70), 2.979 (0.64), 3.444 (0.98), 3.468 (1.29), 3.479 (1.06), 3.503 (1.12), 3.632 (0.76), 3.642 (0.89), 3.656 (0.78), 3.667 (1.25), 3.677 (0.91), 3.692 (0.70), 3.701 (0.60), 4.286 (2.23), 4.759 (0.98), 4.765 (1.02), 4.778 (0.96), 4.793 (0.54), 5.294 (0.49), 5.348 (0.44), 5.752 (2.39), 7.354 (0.90), 7.371 (1.72), 7.388 (0.93), 7.608 (1.00), 7.627 (1.78), 7.646 (0.98), 7.869 (1.43), 7.880 (1.22), 7.886 (1.37), 8.503 (1.49), 8.520 (2.77), 8.537 (1.79), 8.700 (8.73), 8.718 (7.10), 8.850 (3.69), 8.857 (3.69), 10.211 (3.77), 10.230 (3.59). |
| **176** | 1-(2,4-Difluorphenyl)-7-[(4*S*)-4-hydroxy-2-oxopyrrolidin-1-yl]-4-oxo-*N*-[1,1,1-trifluorbutan-2-yl]-1,4-dihydro-1,8-naphthyridin-3-carbonsäureamid (Diastereomerengemisch) 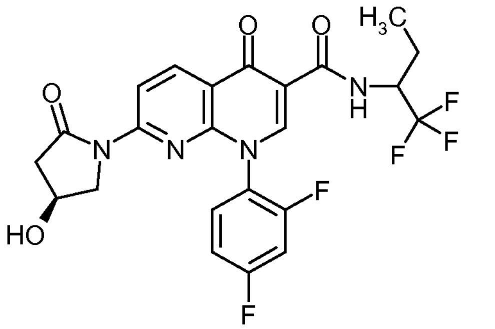 Verbindung aus Bsp. 66A und (4*S*)-4-Hydroxypyrrolidin-2-on; 110°C für 6h (44 % d. Th.) | LC-MS (Methode 1): $R_t$ = 1.01 min; m/z = 511.1 $[M+H]^+$. $^1$H-NMR (400 MHz, DMSO-$d_6$) δ [ppm]: - 0.009 (6.49), 0.007 (6.13), 0.898 (1.89), 0.960 (7.36), 0.978 (15.05), 0.996 (7.39), 1.156 (3.78), 1.174 (7.50), 1.192 (3.75), 1.625 (1.27), 1.643 (1.78), 1.650 (1.47), 1.660 (1.95), 1.669 (1.81), 1.679 (1.62), 1.686 (1.92), 1.704 (1.44), 1.866 (1.43), 1.876 (1.62), 1.885 (1.74), 1.894 (1.69), 1.901 (1.72), 1.908 (1.51), 1.987 (14.10), 2.335 (1.77), 2.357 (1.34), 2.378 (1.74), 2.400 (1.55), 2.906 (1.12), 2.942 (1.40), 3.439 (1.38), 3.470 (2.50), 3.503 (1.70), 3.640 (1.26), 3.664 (1.63), 4.020 (3.12), 4.038 (3.14), 4.280 (2.69), 4.764 (1.63), 4.778 (1.57), 5.287 (2.24), 5.295 (2.18), 5.344 (1.88), 5.354 (1.85), 5.753 (16.00), 7.373 (2.43), 7.607 (1.71), 7.630 (2.72), 7.653 (1.31), 7.870 (1.88), 8.502 (2.22), 8.516 (2.66), 8.524 (2.78), 8.539 (2.90), 8.699 (12.77), 8.721 (10.04), 8.849 (5.98), 8.855 (5.41), 10.209 (4.47), 10.233 (4.26). |
| **177** | *rac*-1-(2,4-Difluorphenyl)-4-oxo-7-(2-oxopyrrolidin-1-yl)-*N*-[1,1,1-trifluorbutan-2-yl]-1,4-dihydro-1,8-naphthyridin-3-carbonsäureamid 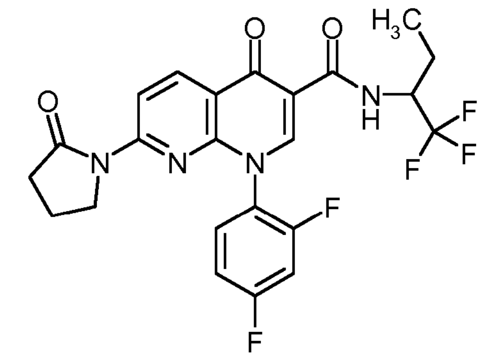 Verbindung aus Bsp. 66A und Pyrrolidin-2-on; 110°C für 6h (10 % d. Th.) | LC-MS (Methode 1): $R_t$ = 1.15 min; m/z = 495.3 $[M+H]^+$. $^1$H-NMR (400 MHz, DMSO-$d_6$) δ [ppm]: - 0.150 (1.83), -0.009 (16.00), 0.007 (15.91), 0.146 (1.93), 0.958 (5.12), 0.977 (11.28), 0.995 (5.59), 1.156 (2.03), 1.174 (3.85), 1.192 (2.09), 1.234 (2.67), 1.642 (1.04), 1.659 (1.22), 1.685 (1.20), 1.703 (0.89), 1.894 (1.21), 1.947 (2.11), 1.962 (2.97), 1.987 (5.90), 2.327 (1.10), 2.366 (1.85), 2.563 (3.59), 2.582 (4.46), 2.590 (4.72), 2.602 (2.58), 2.610 (2.36), 2.669 (1.47), 2.709 (2.18), 3.533 (1.53), 3.550 (2.74), 3.573 (2.86), 3.591 (1.58), 4.020 (1.08), 4.038 (1.03), 4.764 (1.01), 7.339 (0.95), 7.360 (1.97), 7.381 (1.09), 7.598 (1.26), 7.620 (1.88), 7.639 (1.26), 7.646 (1.22), 7.878 (1.35), 8.500 (6.13), 8.522 (7.62), 8.690 (7.51), 8.713 (6.15), 8.850 (3.94), 10.209 (3.35), 10.232 (3.25). |

(fortgesetzt)

| Bsp. | | Analytische Daten |
|---|---|---|
| **178** | 1-(2,4-Difluorphenyl)-4-oxo-7-(2-oxopiperidin-1-yl)-*N*-[(2*R*)-1,1,1-trifluorbutan-2-yl]-1,4-dihydro-1,8-naphthyridin-3-carbonsäureamid 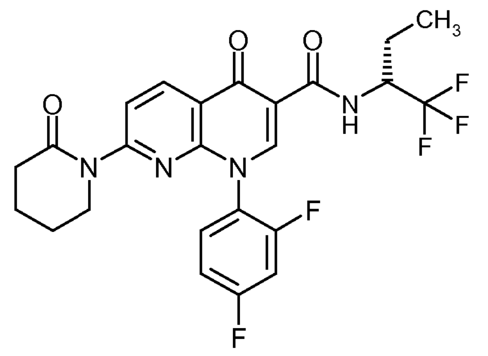 Verbindung aus Bsp. 67A und Piperidin-2-on (35 % d. Th.) | LC-MS (Methode 1): $R_t$ = 1.15 min; m/z = 509.4 $[M+H]^+$. $^1$H-NMR (400 MHz, DMSO-$d_6$) $\delta$ [ppm]: 0.959 (8.20), 0.978 (16.00), 0.996 (7.69), 1.662 (2.25), 1.755 (15.41), 1.894 (1.98), 3.526 (7.02), 4.765 (1.79), 7.342 (1.95), 7.363 (3.27), 7.602 (2.15), 7.624 (3.24), 7.643 (1.92), 7.869 (2.39), 8.133 (8.33), 8.155 (8.74), 8.624 (8.95), 8.646 (8.06), 8.860 (6.39), 10.191 (4.92), 10.214 (4.57). |
| **179** | *rac*-1-(2,4-Difluorphenyl)-4-oxo-7-(2-oxopyrrolidin-1-yl)-*N-[*1,1,1-trifluorpropan-2-yl]-1,4-dihydro-1,8-naphthyridin-3-carbonsäureamid 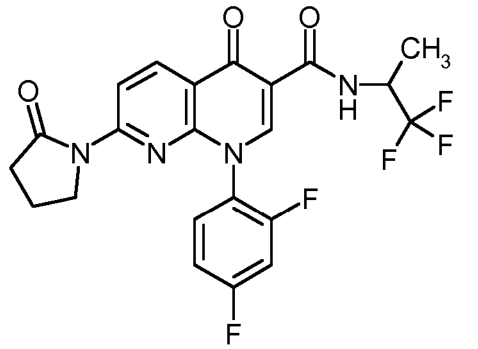 Verbindung aus Bsp. 69A und 2-Pyrrolidinon; 110°C für 6h (15 % d. Th.) | LC-MS (Methode 1): $R_t$ = 1.12 min; m/z = 481.2 $[M+H]^+$. $^1$H-NMR (400 MHz, DMSO-$d_6$) $\delta$ [ppm]: - 0.150 (1.18), -0.009 (10.63), 0.007 (10.36), 0.145 (1.32), 1.381 (15.86), 1.399 (16.00), 1.946 (2.60), 1.960 (3.95), 1.981 (3.04), 2.365 (1.72), 2.561 (4.22), 2.580 (5.74), 2.588 (6.01), 2.601 (3.24), 2.608 (2.87), 2.669 (1.28), 2.709 (1.89), 3.531 (2.16), 3.549 (3.95), 3.570 (3.71), 4.912 (1.69), 4.933 (1.69), 7.339 (1.49), 7.361 (2.84), 7.382 (1.99), 7.598 (1.65), 7.620 (2.46), 7.639 (1.69), 7.855 (1.59), 8.496 (8.78), 8.518 (10.67), 8.681 (11.27), 8.703 (9.08), 8.843 (5.97), 10.258 (3.24), 10.281 (3.07). |
| **180** | *rac*-1-(2,4-Difluorphenyl)-7-(1,1-dioxido-1,2-thiazolidin-2-yl)-4-oxo-*N*-[1,1,1-trifluorbutan-2-yl]-1,4-dihydro-1,8-naphthyridin-3-carbonsäureamid 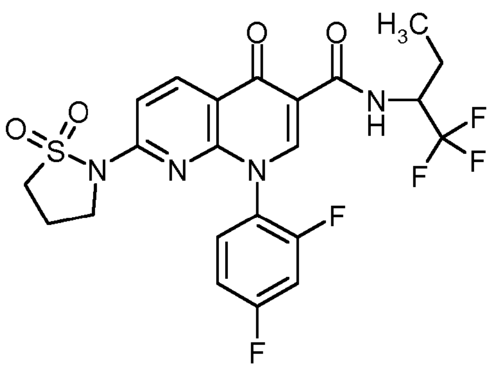 Verbindung aus Bsp. 66A und 1,3-Propansultam; 110°C für 6h (44 % d. Th.) | LC-MS (Methode 1): $R_t$ = 1.09 min; m/z = 531.1 $[M+H]^+$. $^1$H-NMR (400 MHz, DMSO-$d_6$) $\delta$ [ppm]: - 0.009 (4.11), 0.007 (2.26), 0.957 (7.72), 0.975 (16.00), 0.993 (7.78), 1.620 (1.16), 1.638 (1.63), 1.646 (1.74), 1.655 (1.95), 1.663 (1.71), 1.673 (1.65), 1.680 (1.79), 1.698 (1.35), 1.862 (1.46), 1.872 (1.66), 1.881 (1.67), 1.890 (1.83), 1.897 (1.61), 1.907 (1.46), 1.915 (1.14), 1.925 (0.98), 1.987 (1.19), 2.290 (1.50), 2.307 (5.22), 2.325 (8.04), 2.342 (5.26), 2.359 (1.49), 3.593 (4.45), 3.603 (8.33), 3.610 (8.78), 3.619 (8.50), 3.629 (6.96), 3.647 (2.73), 4.757 (1.58), 4.774 (1.48), 7.310 (1.63), 7.326 (2.99), 7.331 (3.06), 7.347 (1.80), 7.352 (1.81), 7.374 (8.75), 7.396 (8.90), 7.537 (1.80), 7.543 (1.86), 7.562 (2.84), 7.566 (2.80), 7.585 (1.80), 7.592 (1.66), 7.818 (1.14), 7.838 (2.25), 7.854 (2.25), 7.875 (1.01), 8.647 (8.78), 8.669 (8.39), 8.804 (8.06), 10.221 (5.01), 10.245 (4.79). |

(fortgesetzt)

| Bsp. | | Analytische Daten |
|---|---|---|
| **181** | 1-(2,4-Difluorphenyl)-7-[(4*S*)-4-hydroxy-2-oxopyrrolidin-1-yl]-*N*-(4-methylbicyclo-[2.2.2]oct-1-yl)-4-oxo-1,4-dihydro-1,8-naphthyridin-3-carbonsäureamid 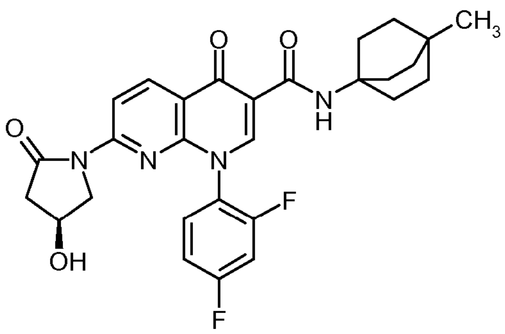 Verbindung aus Bsp. 70A und (4*S*)-4-Hydroxypyrrolidin-2-on; 110°C für 17 h (2 % d. Th.) | LC-MS (Methode 1): $R_t$ = 1.15 min; m/z = 523.4 $[M+H]^+$. $^1$H-NMR (400 MHz, DMSO-$d_6$) δ [ppm]: 0.8 (s, 3H), 1.43-1.52 (m, 6H), 1.89-1.98 (m, 6H), 3.41-3.52 (m, 1H), 3.59-3.72 (m, 1H), 4.24-4.30 (m, 2H), 5.26-5.30 (m, 0.5H), 5.32-5.35 (m, 0.5H), 7.32-7.40 (m, 1H), 7.54-7.66 (m, 1H), 7.77-7.90 (m, 1H), 8.45-8.51 (m, 1H), 8.67 (d, 1H), 8.70 (s, 1H), 9.66 (s, 1H). |

**Beispiel 182**

*N*-(2,6-Dichlorbenzyl)-1-(2,4-difluorphenyl)-7-(1,1-dioxido-1,2-thiazolidin-2-y1)-4-oxo-1,4-dihydro-1,8-naphthyridin-3-carbonsäureamid

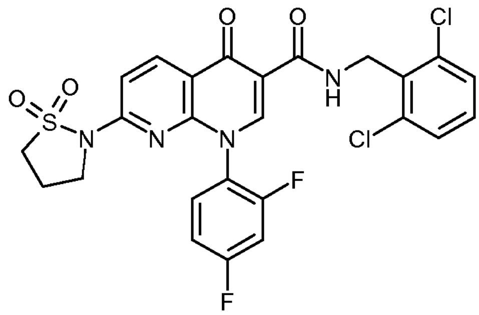

7 mg (0.03 mmol) Palladium(II)acetat und 18 mg (0.03 mmol) Xantphos wurden in 3.6 ml Dioxan unter Argonatmosphäre für 10 Minuten bei 20°C gerührt. Dann wurden 150 mg (0.3 mmol) der Verbindung aus Beispiel 72A, 74 mg (0.6 mmol) 1,3-Propansultam und 148 mg (0.46 mmol) Cäsiumcarbonat zugegeben und die Mischung für 6 h bei 110°C gerührt. Nach dem Abkühlen auf 23°C wurde das Gemisch über präparative HPLC gereinigt (Eluent: Acetonitril/Wasser-Gradient mit 0.1% Ameisensäure). Man erhielt 95 mg (51 % d. Th.) der Titelverbindung.
LC-MS (Methode 1): $R_t$ = 1.15 min; m/z = 579.2 $[M+H]^+$.
$^1$H-NMR (400 MHz, DMSO-$d_6$) δ [ppm]: 1.156 (4.54), 1.174 (8.71), 1.192 (4.39), 1.987 (16.00), 2.297 (4.76), 2.314 (6.89), 2.331 (5.14), 3.577 (4.62), 3.590 (8.77), 3.606 (8.06), 3.634 (2.43), 4.020 (4.03), 4.037 (3.96), 4.815 (3.11), 4.827 (3.16), 4.845 (3.08), 4.859 (2.88), 7.325 (3.46), 7.334 (7.37), 7.356 (6.87), 7.380 (2.99), 7.399 (5.75), 7.420 (4.45), 7.526 (13.43), 7.546 (9.83), 7.555 (3.20), 7.811 (2.87), 7.826 (2.86), 8.595 (6.46), 8.617 (6.25), 8.754 (11.28), 10.157 (2.54), 10.171 (4.84).

In Analogie zu Beispiel 182 wurden die in Tabelle 21 gezeigte Beispielverbindung hergestellt, indem die Verbindung aus Beispiel 72A mit den entsprechenden Aminen (bzw. deren Salzen) unter den beschriebenen Reaktionsbedingungen umgesetzt wurden. Abweichungen sind bei den jeweiligen Beispielen angegeben.

Tabelle 21:

| Bsp. | | Analytische Daten |
|---|---|---|
| **183** | *N*-(2,6-Dichlorbenzyl)-1-(2,4-difluorphenyl)-7-[(4*S*)-4-hydroxy-2-oxopyrrolidin-1-yl]-4-oxo-1,4-dihydro-1,8-naphthyridin-3-carbonsäureamid 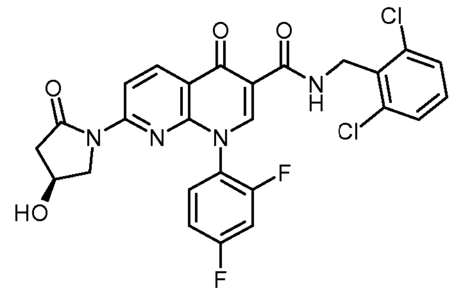 (4 % d. Th.) | LC-MS (Methode 1): $R_t$ = 1.00 min; m/z = 559.1 $[M+H]^+$. $^1$H-NMR (400 MHz, DMSO-$d_6$) $\delta$ [ppm]: 2.83-3.00 (m, 1H), 3.42-3.52 (m, 1H), 3.56-3.73 (m, 1H), 4.24-4.30 (m, 1H), 4.78-4.90 (m, 2H), 5.25-5.29 (m, 0.5H), 5.31-5.36 (m, 0.5H), 7.33-7.43 (m, 2H), 7.54 (d, 1H), 7.56-7.66 (m, 1H), 8.44-8.52 (m, 1H), 8.66 (d, 1H), 8.79 (s, 1H), 10.13-10.19 (m, 1H). |

## Beispiel 184

1-(2,4-Difluorphenyl)-*N*-[2-(2,6-difluorphenyl)propan-2-yl]-4-oxo-7-(2-oxoimidazolidin-1-yl)-1,4-dihydro-1,8-naphthyridin-3-carbonsäureamid

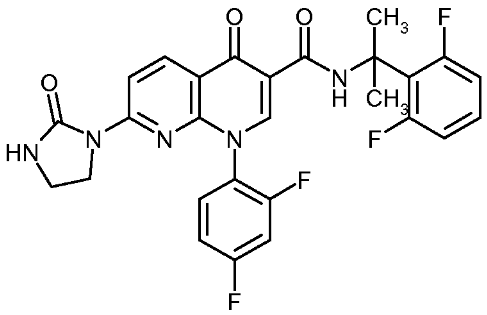

5 mg (0.02 mmol) Palladium(II)acetat und 13 mg (0.02 mmol) Xantphos wurden in 2.7 ml Dioxan unter Argonatmosphäre für 10 Minuten bei 20°C gerührt. Dann wurden 150 mg (0.23 mmol, Reinheit 75%) der Verbindung aus Beispiel 71A, 40 mg (0.46 mmol) 2-Imidazolidinon und 112 mg (0.35 mmol) Cäsiumcarbonat zugegeben und die Mischung für 6 h bei 110°C gerührt. Nach dem Abkühlen auf 23°C wurde das Gemisch über präparative HPLC gereinigt (Eluent: Acetonitril/Wasser-Gradient mit 0.1% Ameisensäure). Man erhielt 45 mg (35 % d. Th.) der Titelverbindung.
LC-MS (Methode 1): $R_t$ = 1.00 min; m/z = 540.2 $[M+H]^+$.
$^1$H-NMR (400 MHz, DMSO-$d_6$) $\delta$ [ppm]: 1.839 (16.00), 1.987 (1.18), 3.534 (1.12), 3.555 (1.83), 3.576 (1.84), 6.942 (2.23), 6.963 (3.18), 6.989 (2.62), 7.248 (1.08), 7.269 (1.75), 7.275 (1.24), 7.284 (1.63), 7.289 (1.21), 7.304 (1.85), 7.556 (1.46), 7.607 (3.76), 7.810 (1.62), 7.825 (1.63), 8.387 (3.80), 8.409 (4.82), 8.546 (4.78), 8.568 (4.25), 8.575 (7.71), 10.534 (4.55).

In Analogie zu Beispiel 184 wurden die in Tabelle 22 gezeigte Beispielverbindung hergestellt, indem die Verbindung aus Beispiel 71A mit den entsprechenden Aminen (bzw. deren Salzen) unter den beschriebenen Reaktionsbedingungen umgesetzt wurden. Abweichungen sind bei den jeweiligen Beispielen angegeben.

Tabelle 22:

| Bsp. | | Analytische Daten |
|---|---|---|
| **185** | 1-(2,4-Difluorphenyl)-*N*-[2-(2,6-difluorphenyl) propan-2-yl]-7-(1,1-dioxido-1,2-thiazolidin-2-yl)-4-oxo-1,4-dihydro-1,8-naphthyridin-3-carbonsäureamid 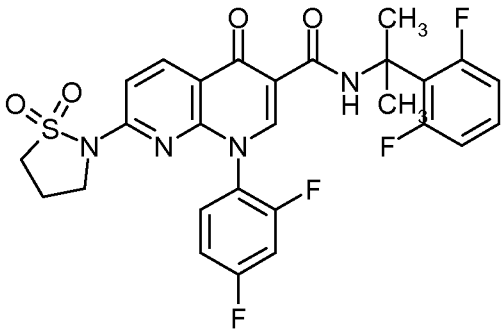 und 1,3-Propansultam (68 % d. Th.) | LC-MS (Methode 1): $R_t$ = 1.16 min MS (ESpos): m/z = 575.3 $[M+H]^+$ $^1$H-NMR (400 MHz, DMSO-$d_6$) $\delta$ [ppm]: 1.157 (4.28), 1.175 (8.38), 1.193 (4.21), 1.842 (10.49), 1.989 (16.00), 2.302 (1.66), 2.319 (2.48), 2.336 (1.72), 3.576 (1.42), 3.595 (2.93), 3.613 (2.62), 3.624 (1.78), 3.642 (0.79), 4.003 (1.40), 4.021 (3.96), 4.039 (3.85), 4.057 (1.24), 6.946 (1.55), 6.967 (2.19), 6.992 (1.69), 7.251 (0.78), 7.256 (0.76), 7.271 (1.60), 7.292 (1.53), 7.309 (0.73), 7.363 (2.74), 7.385 (2.84), 7.507 (0.64), 7.513 (0.65), 7.535 (0.99), 7.555 (0.61), 7.562 (0.57), 7.795 (0.70), 7.801 (1.09), 7.816 (1.08), 7.823 (0.60), 8.597 (5.07), 8.650 (2.78), 8.672 (2.60), 10.470 (2.95). |

**Beispiel 186**

1-(2,4-Difluorphenyl)-4-oxo-7-(2-oxoimidazolidin-1-yl)-*N*-(tricyclo[3.3.11$^{3,7}$]dec-1-yl)-1,4-dihydro-1,8-naphthyridin-3-carbonsäureamid

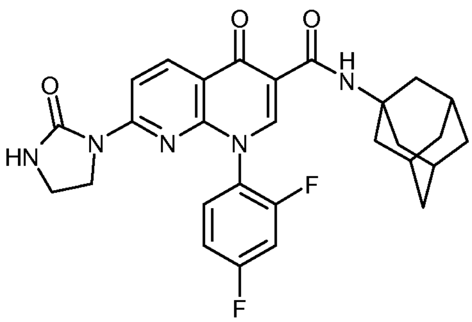

Eine Mischung aus 200 mg (0.4 mmol) der Verbindung aus Beispiel 65A, 147 mg (1.7 mmol) 2-Imidazolidinon, 118 mg (0.9 mmol) Kaliumcarbonat, 83 mg (0.4 mmol) Kupfer(I)iodid und 32 mg (0.4 mmol) trans-*N*,*N*'-Dimethyl-1,2-cyclohexandiamin in 5 ml DMF wurde bei 110°C für 22 h gerührt. Anschliessend wurde das Gemisch filtriert und das Filtrat über präparative HPLC gereinigt (Eluent: Acetonitril/Wasser-Gradient mit 0.1% Ameisensäure). Man erhielt 7 mg (3 % d. Th.) der Titelverbindung.

$^1$H-NMR (400 MHz, DMSO-$d_6$) $\delta$ [ppm]: -0.150 (1.07), -0.009 (9.84), 0.007 (8.65), 0.146 (1.15), 1.677 (7.23), 2.068 (16.00), 2.327 (0.71), 2.365 (0.96), 2.669 (0.75), 2.709 (0.97), 3.563 (0.92), 7.341 (0.77), 7.601 (2.15), 7.836 (0.93), 7.851 (0.93), 8.144 (0.84), 8.368 (2.57), 8.391 (3.21), 8.515 (3.29), 8.537 (2.43), 8.638 (4.84), 9.797 (2.58).

LC-MS (Methode 1): $R_t$ = 1.21 min

MS (ESpos): m/z = 520.2 $[M+H]^+$

**Beispiel 187**

1-(2,4-Difluorphenyl)-7-[4-fluor-2-oxopyrrolidin-1-yl]-4-oxo-*N*-(tricyclo[3.3.1.1$^{3,7}$]dec-1-yl)-1,4-dihydro-1,8-naphthyridin-3-carbonsäureamid

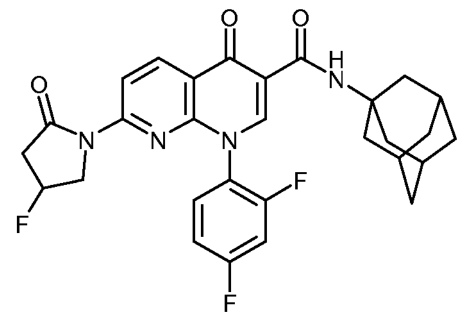

150 mg (0.28 mmol) der Verbindung aus Beispiel 170 wurden in 5 ml DCM bei -78°C tropfenweise mit 143 mg (0.84 mmol) DAST versetzt und 3 Stunden lang bei -78°C gerührt. Dann wurde die Mischung auf 20°C erwärmt und mit gesättigter wässriger Natriumchlorid-Lösung versetzt. Das Produkt wurde mit Essigsäureethylester extrahiert. Das Lösungsmittel wurde im Vakuum entfernt und der Rückstand über präparative HPLC gereinigt (Eluent: Acetonitril/Wasser-Gradient mit 0.1% Ameisensäure). Man erhielt 6 mg (4 % d. Th.) der Titelverbindung.
LC-MS (Methode 1): $R_t$ = 1.31 min; m/z = 537.4 $[M+H]^+$.
$^1$H-NMR (400 MHz, DMSO-$d_6$): $\delta$ = 1.67 (br. s, 6 H), 2.07 (br.s, 9H), 2.73-2.83 (m), 3.7-3.9 (m), 3.94-4.04 (m), 4.48-4.59 (m, 1H), 5.30-5.36 (m), 5.43-5.48 (m), 7.11 (d, 1H), 7.31-7.42 (m, 1H), 7.57 - 7.73 (m, 1H), 7.83 - 7.91 (m, 1H), 8.44-8.49 (m, 1H), 8.69-8.74 (m, 2H), 9.70 (br. s, 1H).

## Beispiel 188

7-[(3*R*)-3-(Difluormethoxy)pyrrolidin-1-yl]-1-(2,4-difluorphenyl)-*N*-[2-(2,6-difluorphenyl)propan-2-yl]-4-oxo-1,4-dihydro-1,8-naphthyridin-3-carbonsäureamid

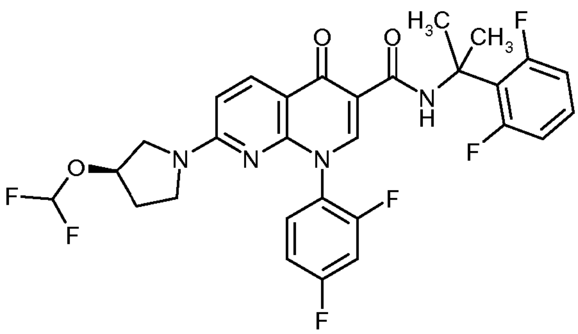

400 mg (0.74 mmol) der Verbindung aus Beispiel 21 und 71 mg (0.37 mmol) Kupfer(I)iodid wurden in 11 ml Acetonitril unter Argonatmosphäre vorgelegt. Zu dieser Mischung wurde bei 55°C eine Lösung von 264 mg (1.48 mmol) 2,2-Difluor-2-(fluorsulfonyl)-essigsäure in 6 ml Acetonitril zugetropft und das Gemisch anschließend für 20 Minuten bei 55°C gerührt. Dann wurden weitere 528 mg (3 mmol) 2,2-Difluor-2-(fluorsulfonyl)-essigsäure in 29 ml Acetonitril zugetropft und das Gemisch anschließend für 6 Stunden bei 55°C gerührt. Das Lösungsmittel wurde im Vakuum entfernt und der Rückstand durch präparative HPLC gereinigt (in drei Portionen; Eluent: Acetonitril/Wasser-Gradient mit 0.1% Ameisensäure). Man erhielt 121 mg (28 % d. Th.) der Titelverbindung.
LC-MS (Methode 1): $R_t$ = 1.24 min; m/z = 591.4 $[M+H]^+$.
$^1$H-NMR (400 MHz, DMSO-$d_6$) $\delta$ [ppm]: 0.006 (0.90), 1.156 (1.80), 1.174 (3.60), 1.192 (1.83), 1.826 (16.00), 1.987 (6.86), 2.147 (0.58), 2.365 (0.31), 2.709 (0.28), 3.493 (0.35), 3.590 (0.46), 4.002 (0.54), 4.020 (1.61), 4.037 (1.60), 4.055 (0.52), 4.844 (0.36), 4.933 (0.27), 6.549 (0.46), 6.739 (0.96), 6.771 (1.40), 6.793 (1.37), 6.936 (2.38), 6.958 (3.19), 6.983 (2.59), 6.995 (0.39), 7.227 (0.45), 7.242 (1.03), 7.248 (1.00), 7.263 (2.26), 7.278 (2.13), 7.283 (2.14), 7.299 (1.07), 7.508 (0.75), 7.514 (0.77), 7.536 (1.28), 7.556 (0.76), 7.563 (0.71), 7.739 (0.59), 7.760 (1.20), 7.776 (1.19), 7.797 (0.54), 8.132 (0.39), 8.314 (2.17), 8.336 (2.09), 8.420 (6.84), 10.672 (4.67).

**Beispiel 189**

1-(2,4-Difluorphenyl)-7-[(3S)-3-fluorpyrrolidin-1-yl]-4-oxo-*N*-[(2*R*)-1,1,1-trifluorbulan-2-yl]-1,4-dihydro-1,8-naphthyridin-3-carbonsäureamid

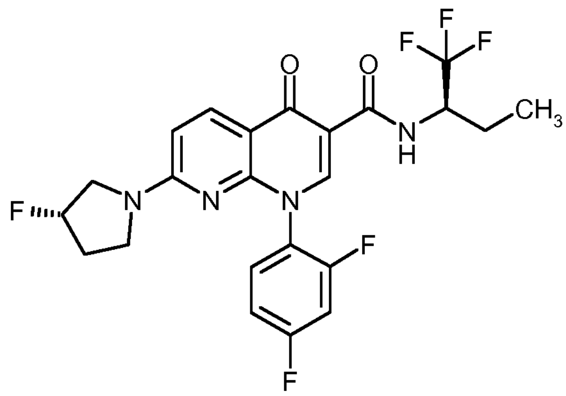

Gemäß AAV3 wurden 100 mg (224 μmol) der Verbindung aus Beispiel 67A mit 34 mg (0.27 mmol) (*S*)-3-Fluorpyrrolidin Hydrochlorid und 0.14 ml (0.79 mmol) *N,N*-Diisopropylethylamin in 1 ml Dimethylformamid zur Reaktion gebracht. Das Rohprodukt wurde mit 0.5 ml Acetonitril verdünnt und mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 97.5 mg (86% d. Th., 99% Reinheit) der Titelverbindung erhalten.

$^1$H-NMR (400 MHz, DMSO-$d_6$): δ [ppm] = 10.49 (d, 1H), 8.63 (s, 1H), 8.33 (d, 1H), 7.86-7.78 (m, 1H), 7.63-7.53 (m, 1H), 7.36-7.29 (m, 1H), 6.85-6.77 (m, 1H), 5.55-5.23 (m, 1H), 4.80-4.67 (m, 1H), 3.85-3.05 (m, 4H), 2.33-1.97 (m, 2H), 1.93-1.82 (m, 1H), 1.70-1.57 (m, 1H), 0.97 (t, 3H).

LC-MS (Methode 1): $R_t$ = 1.22 min; 499 $[M+H]^+$.

**Beispiel 190**

1-(2,4-Difluorphenyl)-7-[(1*R*,5*S*)-6-(methoxymethyl)-3-azabicyclo[3.1.0]hex-3-yl]-4-oxo-*N*-[(2*R*)-1,1,1-trifluorbutan-2-yl]-1,4-dihydro-1,8-naphthyridin-3-carbonsäureamid

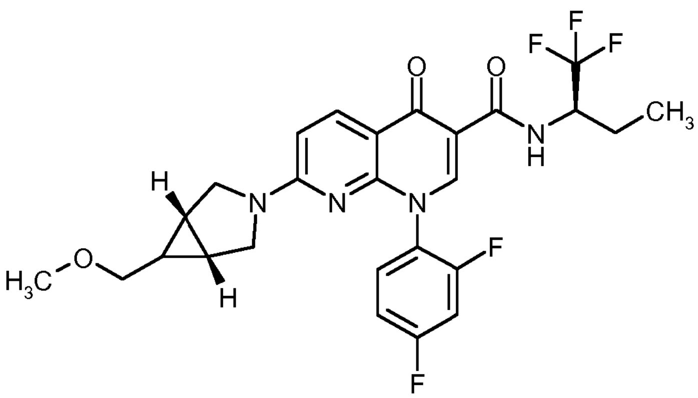

Gemäß AAV3 wurden 100 mg (224 μmol) der Verbindung aus Beispiel 67A mit 44 mg (0.27 mmol) (1*R*,5*S*)-6-(methoxymethyl)-3-azabicyclo[3.1.0]hexan Hydrochlorid und 0.14 ml (0.79 mmol) *N,N*-Diisopropylethylamin in 2.2 ml Dimethylformamid zur Reaktion gebracht. Das Rohprodukt wurde mit Acetonitril verdünnt und mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 107 mg (89% d. Th.,

99% Reinheit) der Titelverbindung erhalten.
$^1$H-NMR (400 MHz, DMSO-d6): δ [ppm] = 10.49 (d, 1H), 8.62 (s, 1H), 8.28 (d, 1H), 7.84-7.76 (m, 1H), 7.64-7.53 (m, 1H), 7.36-7.28 (m, 1H), 6.73 (d, 1H), 4.79-4.66 (m, 1H), 3.73-3.11 (m, 6H), 3.20 (s, 3H), 1.94-1.82 (m, 1H), 1.71-1.50 (m, 3H), 0.96 (t, 3H), 0.85-0.74 (m, 1H).
LC-MS (Methode 3): $R_t$ = 2.29 min; 537 $[M+H]^+$.

**Beispiel 191**

1-(2,4-Difluorphenyl)-7-[3-methoxy-3-methylpyrrolidin-1-yl]-4-oxo-*N*-[(2*R*)-1,1,1-trifluorbutan-2-yl]-1,4-dihydro-1,8-naphthyridin-3-carbonsäureamid (Diastereomerengemisch)

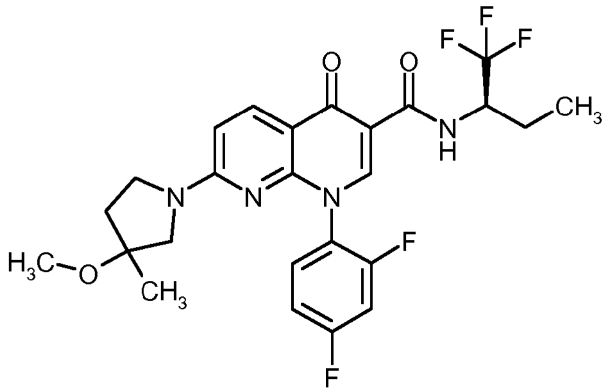

Gemäß AAV3 wurden 100 mg (224 μmol) der Verbindung aus Beispiel 67A mit 43 mg (0.27 mmol) *rac*-3-Methoxy-3-methylpyrrolidin-Hydrochlorid und 0.14 ml (0.79 mmol) *N*,*N*-Diisopropylethylamin in 2.2 ml Dimethylformamid zur Reaktion gebracht. Das Rohprodukt wurde mit Acetonitril verdünnt und mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 105 mg (89% d. Th., 99% Reinheit) der Titelverbindung erhalten.
$^1$H-NMR (400 MHz, DMSO-$d_6$): δ [ppm] = 10.51 (d, 1H), 8.61 (s, 1H), 8.31-8.24 (m, 1H), 7.86-7.76 (m, 1H), 7.64-7.52 (m, 1H), 7.37-7.28 (m, 1H), 6.74 (d, 1H), 4.80-4.67 (m, 1H), 3.63-2.83 (m, 7H), 2.24-2.01 (m, 1H), 1.94-1.57 (m, 3H), 1.35-1.20 (2x s, 3H), 0.97 (t, 3H).
LC-MS (Methode 3): $R_t$ = 2.30 min; 525 $[M+H]^+$.

**Beispiel 192**

1-(2,4-Difluorphenyl)-4-oxo-7-(pyrrolidin-1-yl)-*N*-[(2*R*)-1,1,1-trifluorbutan-2-yl]-1,4-dihydro-1,8-naphthyridin-3-carbonsäureamid

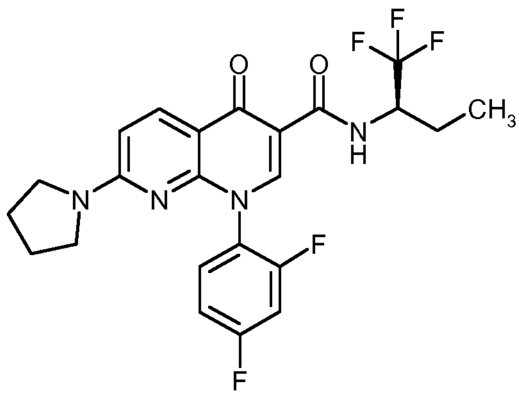

Gemäß AAV1 wurden 100 mg (269 µmol) der Verbindung aus Beispiel 57A mit 66.1 mg (40.4 µmol) (2*R*)-1,1,1-Trifluorbutan-2-amin Hydrochlorid in Gegenwart von 102 mg (269 µmol) HATU und 153 µl (876 µmol) *N*,*N*-Diisopropylethylamin in 2.7 ml Dimethylformamid zur Reaktion gebracht. Nach Aufreinigung mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) wurden 24 mg (19% d. Th., 100% Reinheit) der Titelverbindung erhalten.
$^1$H-NMR (400 MHz, DMSO-$d_6$): δ [ppm] = 10.53 (d, 1H), 8.60 (s, 1H), 8.28 (d, 1H), 7.85-7.75 (m, 1H), 7.61-7.52 (m, 1H), 7.35-7.27 (m, 1H), 6.75 (d, 1H), 4.80-4.68 (m, 1H), 3.51-3.34 (br. s, 2H), 3.21-3.02 (br. s, 2H), 1.99-1.74 (m, 5H), 1.70-1.56 (m, 1H), 0.97 (t, 3H).
LC-MS (Methode 1): $R_t$ = 1.31 min; 481 [M+H]$^+$.

## Beispiel 193

1-(2,4-Difluorphenyl)-7-[(4*S*)-4-methoxy-2-oxopyrrolidin-1-yl]-4-oxo-*N*-[(2*R*)-1,1,1-trifluorbutan-2-yl]-1,4-dihydro-1,8-naphthyridin-3-carbonsäureamid

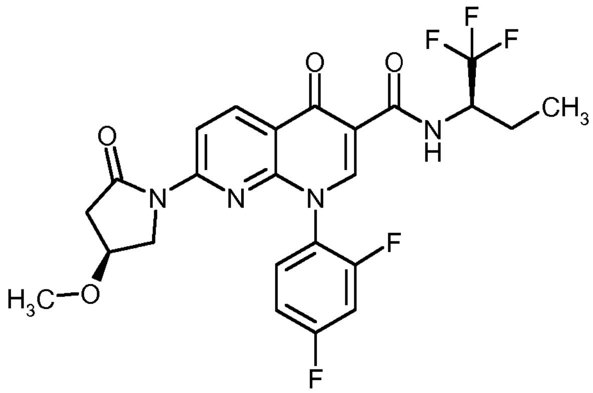

Eine Lösung von 50 mg (98 µmol) der Verbindung aus Beispiel 175 in 1 ml DCM wurde mit 24 µl (0.39 mmol, 4 Äq.) Methyliodid und 270 mg (2.11 mmol, 21.5 Äq.) Silber(I)oxid versetzt und die resultierende Suspension für 1 d bei Raumtemperatur und für 3 d unter Rückfluss gerührt. Anschließend wurde auf RT abgekühlt, filtriert und das Rohprodukt mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 28.4 mg (55% d. Th., 99% Reinheit) der Titelverbindung erhalten.
$^1$H-NMR (400 MHz, DMSO-$d_6$): δ [ppm] = 10.21 (d, 1H), 8.87 (d, 1H), 8.72 (d, 1H), 8.49 (d, 1H), 7.92-7.84 (m, 1H), 7.71-7.59 (m, 1H), 7.42-7.33 (m, 1H), 4.83-4.71 (m, 1H), 4.06-4.00 (m, 1H), 3.74-3.54 (m, 2H), 3.22/3.17 (2x s, 3H), 3.01-2.90 (m, 1H), 2.64-2.56 (m, 1H), 1.96-1.83 (m, 1H), 1.73-1.60 (m, 1H), 0.98 (t, 3H).
LC-MS (Methode 1): $R_t$ = 1.11 min; 525 [M+H]$^+$.

## Beispiel 194

1-(2,4-Difluorphenyl)-7-[3-hydroxy-3-methyl-2-oxopyrrolidin-1-yl]-4-oxo-*N*-[(2*R*)-1,1,1-trifluorbutan-2-yl]-1,4-dihydro-1,8-naphthyridin-3-carbonsäureamid (Diastereomerengemisch)

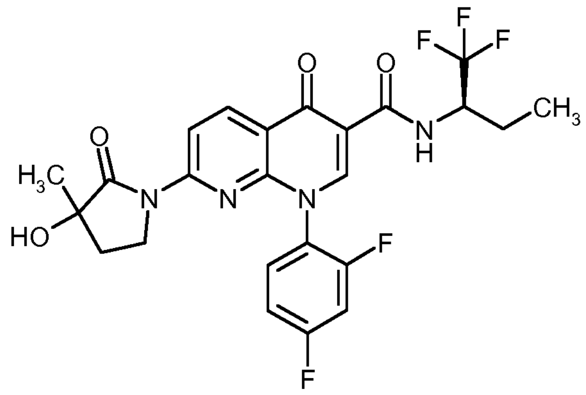

Gemäß AAV2 wurden 163 mg (366 μmol) der Verbindung aus Beispiel 67A mit 42.1 mg (366 μmol) der Verbindung aus Beispiel 2A in Gegenwart von 179 mg (548 μmol) Caesiumcarbonat, 15 mg (66 μmol) Palladium(II)acetat und 38 mg (66 μmol) Xantphos in 8.2 ml Dioxan umgesetzt. Nach Aufreinigung mittels Flashchromatographie (zweimal, Essigsäureethylester/Cyclohexan-Gradient) wurden 153.6 mg (75% d. Th. 94% Reinheit) der Titelverbindung erhalten.
$^1$H-NMR (400 MHz, DMSO-$d_6$): δ [ppm] = 10.21 (d, 1H), 8.87 (s, 1H), 8.74 (d, 1H), 8.54 (d, 1H), 7.92-7.84 (m, 1H), 7.66-7.58 (m, 1H), 7.40-7.30 (m, 1H), 5.72 (d, 1H), 4.84-4.70 (m, 1H), 3.60-3.49 (m, 1H), 3.45-3.34 (m, 1H), 2.06-1.85 (m, 3H), 1.72-1.62 (m, 1H), 1.29/1.27 (2x s, 3H), 0.98 (t, 3H).
LC-MS (Methode 3): $R_t$ = 1.95 min; 525 $[M+H]^+$.

130 mg der Titelverbindung (Diastereomerengemisch) wurde durch chirale HPLC in die Diastereomere getrennt (präparative HPLC: Säule Daicel® Chiralpak AD-H, 5 μm, 250 x 20 mm; Eluent: 85% $CO_2$/ 15% Isopropanol; Fluss 80 ml/min; 40°C, Detektion: 210 nm).
Man erhielt (in der Reihenfolge der Elution von der Säule) 37.2 mg Diastereomer 1 (99% de) $R_t$ = 6.04 min und 32.7 mg (99%de) Diastereomer 2 $R_t$ = 7.33 min.
[Analytische HPLC:Säule SFC Daicel® Chiralpak AD, 3ml/min; 90% $CO_2$ / 10% Isopropanol]

Diastereomer 1 wurde zusätzlich mittels präparativer HPLC (Säule: Chromatorex C18, 10μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 24.6 mg (13% d. Th., 99% Reinheit) der Titelverbindung aus Beispiel 196 erhalten.
Diastereomer 2 wurde zusätzlich mittels präparativer HPLC (Säule: Chromatorex C18, 10μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und es wurden 20.6 mg (11% d. Th., 99% Reinheit) der Titelverbindung aus Beispiel 197 erhalten.

## Beispiel 195

1-(2,4-Difluorphenyl)-7-[3-hydroxy-3-methyl-2-oxopyrrolidin-1-yl]-4-oxo-*N*-[(2*R*)-1,1,1-trifluorbutan-2-yl]-1,4-dihydro-1,8-naphthyridin-3-carbonsäureamid (Diastereomer 1)

$^1$H-NMR (400 MHz, DMSO-$d_6$): δ [ppm] = 10.21 (d, 1H), 8.86 (s, 1H), 8.74 (d, 1H), 8.54 (d, 1H), 7.92-7.84 (m, 1H), 7.67-7.58 (m, 1H), 7.40-7.32 (m, 1H), 5.72 (d, 1H), 4.83-4.71 (m, 1H), 3.60-3.49 (m, 1H), 3.45-3.34 (m, 1H), 2.07-1.84 (m, 3H), 1.74-1.60 (m, 1H), 1.29/1.27 (2x s, 3H), 0.98 (t, 3H).
LC-MS (Methode 1): $R_t$ = 1.04 min; 525 $[M+H]^+$.

## Beispiel 196

1-(2,4-Difluorphenyl)-7-[3-hydroxy-3-methyl-2-oxopyrrolidin-1-yl]-4-oxo-*N*-[(2*R*)-1,1,1-trifluorbutan-2-yl]-1,4-dihydro-1,8-naphthyridin-3-carbonsäureamid (Diastereomer 2)

$^1$H-NMR (400 MHz, DMSO-$d_6$): δ [ppm] = 10.21 (d, 1H), 8.86 (s, 1H), 8.74 (d, 1H), 8.54 (d, 1H), 7.92-7.84 (m, 1H), 7.66-7.59 (m, 1H), 7.39-7.33 (m, 1H), 5.72 (d, 1H), 4.82-4.72 (m, 1H), 3.60-3.49 (m, 1H), 3.45-3.34 (m, 1H), 2.05-1.85 (m, 3H), 1.71-1.59 (m, 1H), 1.29/1.27 (2x s, 3H), 0.98 (t, 3H).
LC-MS (Methode 1): $R_t$ = 1.04 min; 525 $[M+H]^+$.

**Beispiel 197**

1-(2,4-Difluorophenyl)-7-[3-methoxy-3-methyl-2-oxopyrrolidin-1-yl]-4-oxo-*N*-[(2*R*)-1,1,1-trifluorbutan-2-yl]-1,4-dihydro-1,8-naphthyridin-3-carbonsäureamid (Diastereomerengemisch)

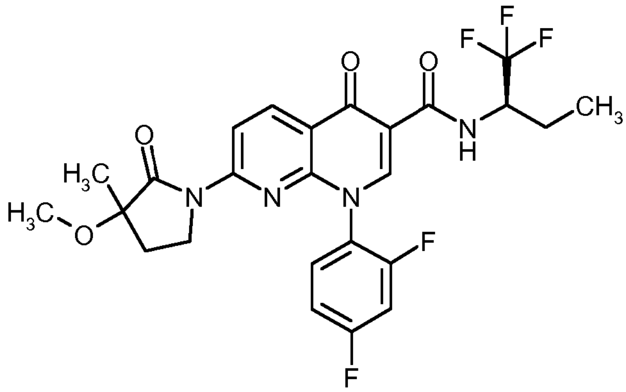

Eine Lösung von 30 mg (57 µmol) des Diastereomerengemisches aus Beispiel 194 in 1 ml DCM wurde mit 28 µl (0.46 mmol, 8 Äq.) Methyliodid und 170 mg (1.37 mmol, 24 Äq.) Silber(I)oxid versetzt und die resultierende Suspension für 3d unter Rückfluss gerührt. Anschließend wurde auf RT abgekühlt, filtriert und 30.0 mg (96% d. Th., 99% Reinheit) der Titelverbindung erhalten.
$^1$H-NMR (400 MHz, DMSO-$d_6$): δ [ppm] = 10.20 (d, 1H), 8.87 (s, 1H), 8.75 (d, 1H), 8.54 (d, 1H), 7.91-7.83 (m, 1H), 7.66-7.58 (m, 1H), 7.41-7.33 (m, 1H), 4.83-4.73 (m, 1H), 3.53-3.39 (m, 2H), 3.21/3.18 (2x s, 3H), 2.29-2.17 (m, 1H), 2.01-2.84 (m, 2H), 1.73-1.61 (m, 1H), 1.33/1.31 (2x s, 3H), 0.98 (t, 3H). LC-MS (Methode 3): $R_t$ = 2.24 min; 539 [M+H]$^+$.
30 mg der Titelverbindung (Diastereomerengemisch) wurde durch chirale HPLC in die Diastereomere getrennt (präparative HPLC: Chiralpak-IF 5 µm 250 x 20 mm; Eluent: 100% Methanol, 0.2% Diethylamin; Temperatur: 30°C; Fluss: 15 ml/min; UV-Detektion: 220 nm).

Man erhielt (in der Reihenfolge der Elution von der Säule) 11 mg Diastereomer 1 (99% de) $R_t$ = 7.26 min und 6 mg (97.4% de) Diastereomer 2 $R_t$ = 8.36 min.

[Analytische HPLC:Säule Chiralpak IF 5 µm 250 x 4.6 mm; Eluent: 100% Methanol, 0.2% Diethylamin; Temperatur: 40°C; Fluss: 1 ml/min; UV-Detektion: 235 nm]

Diastereomer 1 wurde zusätzlich (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 6.4 mg (21% d. Th., 99% Reinheit) der Titelverbindung aus Beispiel 198 erhalten.

Diastereomer 2 wurde zusätzlich mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 3.6 mg (12% d. Th., 99% Reinheit) der Titelverbindung aus Beispiel 199 erhalten.

**Beispiel 198:**

1-(2,4-Difluorophenyl)-7-[3-methoxy-3-methyl-2-oxopyrrolidin-1-yl]-4-oxo-*N*-[(2*R*)-1,1,1-trifluorbutan-2-yl]-1,4-dihydro-1,8-naphthyridin-3-carbonsäureamid (Diastereomer 1)

$^1$H-NMR(400 MHz, DMSO-$d_6$): δ [ppm] = 10.19 (d, 1H), 8.87 (s, 1H), 8.75 (d, 1H), 8.54 (d, 1H), 7.91-7.83 (m, 1H), 7.66-7.58 (m, 1H), 7.39-7.33 (m, 1H), 4.84-4.70 (m, 1H), 3.60-3.38 (m, 2H), 3.20/3.18 (2x s, 3H), 2.28-2.17 (m, 1H), 2.02-1.84 (m, 2H), 1.74-1.60 (m, 1H), 1.33/1.31 (2x s, 3H), 0.98 (t, 3H). LC-MS (Methode 3): $R_t$ = 2.26 min; 539 [M+H]$^+$.

**Beispiel 199:**

1-(2,4-Difluorophenyl)-7-[3-methoxy-3-methyl-2-oxopyrrolidin-1-yl]-4-oxo-*N*-[(2*R*)-1,1,1-trifluorbutan-2-yl]-1,4-dihydro-1,8-naphthyridin-3-carbonsäureamid (Diastereomer 2)

$^1$H-NMR(400 MHz, DMSO-$d_6$): δ [ppm] = 10.19 (d, 1H), 8.87 (s, 1H), 8.75 (d, 1H), 8.54 (d, 1H), 7.91-7.83 (m,

1H), 7.65-7.59 (m, 1H), 7.39-7.33 (m, 1H), 4.83-4.72 (m, 1H), 3.60-3.37 (m, 2H), 3.21/3.18 (2x s, 3H), 2.28-2.17 (m, 1H), 2.01-1.84 (m, 2H), 1.72-1.60 (m, 1H), 1.33/1.31 (2x s, 3H), 0.98 (t, 3H). LC-MS (Methode 3): $R_t$ = 2.24 min; 539 $[M+H]^+$.

**Beispiel 200:**

(3*S*)-1-[8-(2,4-Difluorphenyl)-5-oxo-6-{[(2*R*)-1,1,1-trifluorbutan-2-yl]carbamoyl}-5,8-dihydro-1,8-naphthyridin-2-yl]-5-oxopyrrolidin-3-yl-methylcarbamat

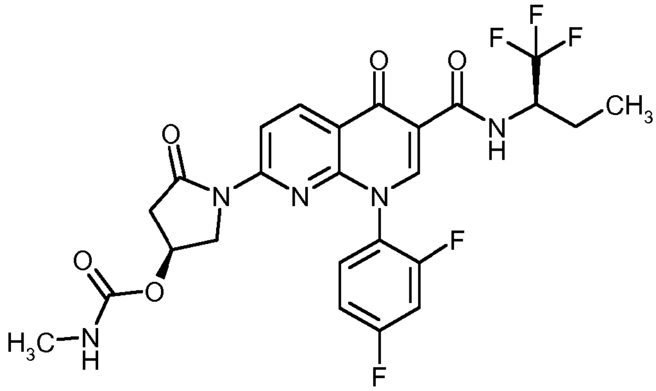

Eine Lösung von 100 mg (196 µmol) der Verbindung aus Beispiel 175 in 4 ml DCM wurde mit 18.3 mg (196 µmol) Methylcarbamoylchlorid, 4.8 mg (39 µmol) 4-Dimethylaminopyridin und 30 µl (0.22 mmol) Triethylamin versetzt und die Mischung über Nacht bei RT gerührt. Das Lösungsmittel wurde dann unter reduziertem Druck entfernt und der Rückstand mittels präp. HPLC (Säule: Kromasil C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 31.9 mg (28% d. Th., 99% Reinheit) der Titelverbindung erhalten.
$^1$H-NMR (400 MHz, DMSO-$d_6$): δ [ppm] = 10.20 (d, 1H), 8.88-8.85 (m, 1H), 8.73 (d, 1H), 8.51-8.45 (m, 1H), 7.91-7.81 (m, 1H), 7.66-7.55 (m, 1H), 7.41-7.32 (m, 1H), 7.22-7.08 (m, 1H), 5.15-5.09 (m, 1H), 4.84-4.71 (m, 1H), 3.89-3.77 (m, 1H), 3.68-3.54 (m, 1H), 3.20-3.09 (m, 1H), 1.96-1.84 (m, 1H), 1.73-1.60 (m, 1H), 0.98 (t, 3H).
LC-MS (Methode 4): $R_t$ = 3.27 min; 568 $[M+H]^+$.

**Beispiel 201:**

*N*-(2,6-Dichlorphenyl)-1-(2,4-difluorphenyl)-7-[(4*S*)-4-hydroxy-2-oxopyrrolidin-1-yl]-4-oxo-1,4-dihydro-1,8-nahthridin-3-carbonsäureamid

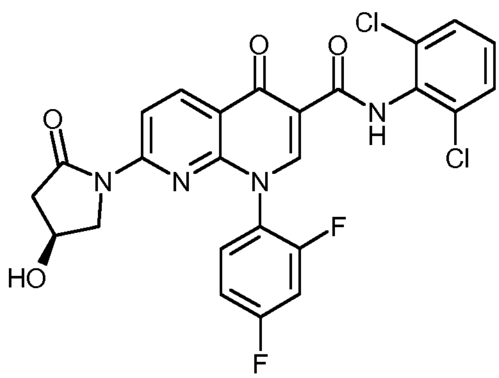

Gemäß AAV2 wurden 100 mg (153 µmol, 73.6% Reinheit) der Verbindung aus Beispiel 81A mit 15.5 mg (153 µmol) (4*S*)-4-Hydroxypyrrolidin-2-on in Gegenwart von 74.8 mg (230 µmol) Caesiumcarbonat, 6.2 mg (28 µmol) Palladium(II)acetat und 16 mg (28 µmol) Xantphos in 2 ml Dioxan zur Reaktion gebracht. Der Rückstand wurde mittels

Flashchromatographie (Cyclohexan/Essigsäureethylester-Gradient) und abschließend durch präparative Dünnschichtchromatographie (Dichlormethan/Methanol = 95/5, 20x20 cm Platten, 1 mm Silica) gereinigt. Die Produktfraktion wurde durch UV-Detektion sichtbar gemacht, abgekratzt und mit Essigsäureethylester vom Kieselgel eluiert. Es wurde über Celite filtriert und das Lösungsmittel unter reduziertem Druck entfernt. Der Rückstand wurde aus Wasser/Acetonitril lyophilisiert und es wurden 27.3 mg (33% d. Th., 100% Reinheit) der Titelverbindung erhalten.
$^1$H-NMR (400 MHz, DMSO-d6): δ [ppm] = 11.7 (s, 1H), 8.92 (s, 1H), 8.77 (d, 1H), 8.57-8.52 (m, 1H), 7.96-7.86 (m, 1H), 7.67-7.57 (m, 3H), 7.42-7.33 (m, 2H), 5.33 (dd, 1H), 4.32-4.25 (m, 1H), 3.74-3.63 (m, 1H), 3.54-3.43 (m, 1H), 3.01-2.89 (m, 1H), 2.43-2.31 (m, 1H).
LC-MS (Methode 1): $R_t$ = 1.03 min; 545 $[M+H]^+$.

**Beispiel 202:**

*N*-(2,6-Dichlorophenyl)-1-(2,4-difluorphenyl)-7-[(3*S*)-3-hydroxy-2-oxopyrrolidin-1-yl]-4-oxo-1,4-dihydro-1, 8-naphthyridin-3 -carbonsäureamid

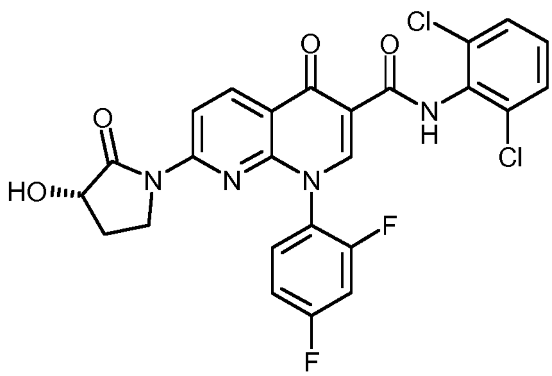

Gemäß AAV2 wurden 150 mg (275 µmol, 88%-ige Reinheit) der Verbindung aus Beispiel 81A mit 27.8 mg (275 µmol) (3*S*)-3-Hydroxypyrrolidin-2-on in Gegenwart von 56.9 mg (412 µmol) Kaliumcarbonat, 6.2 mg (27 µmol) Palladium(II)acetat und 32 mg (55 µmol) Xantphos in 2.8 ml Dioxan zur Reaktion gebracht. Das Rohprodukt wurde zweimal mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 49.2 mg (33% d. Th., 100% Reinheit) der Titelverbindung erhalten.
$^1$H-NMR (400 MHz, DMSO-$d_6$): δ [ppm] = 11.69 (s, 1H), 8.93 (s, 1H), 8.79 (d, 1H), 8.58-8.52 (m, 1H), 7.94-7.85 (m, 1H), 7.66-7.57 (m, 3H), 7.42-7.32 (m, 2H), 5.91 (d, 1H), 4.46-4.34 (m, 1H), 3.64-3.51 (m, 1H), 3.39-3.27 (m, 1H), 2.38-2.27 (m, 1H), 1.86-1.69 (m, 1H).
LC-MS (Methode 3): $R_t$ = 1.83 min; 545 $[M+H]^+$.

**Beispiel 203:**

7-(Dimethylamino)-1-(2-fluorphenyl)-4-oxo-*N*-[(2*S*)-1,1,1-trifluorbutan-2-yl]-1,4-dihydro-1,8-naphthyridin-3-carbonsäureamid

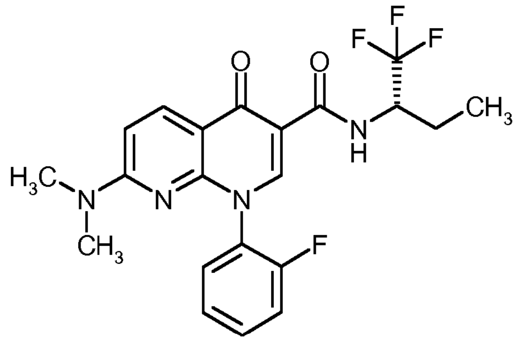

Gemäß AAV1 wurden 100 mg (306 µmol) der Verbindung aus Beispiel 62A mit 58.3 mg (458 µmol) (*S*)-2-Trifluormethylpropylamin in Gegenwart von 116 mg (306 µmol) HATU und 160 µl (917 µmol) *N*,*N*-Diisopropylethylamin in 3.2 ml Dimethylformamid umgesetzt. Die Reaktion wurde durch Zugabe von wässr. 1M Salzsäure und 10 ml Wasser beendet und der Niederschlag abgesaugt. Der Niederschlag wurde mit Wasser gewaschen und über Nacht am Hochvakuum getrocknet. Der Rückstand wurde in 2 ml Dichlormethan aufgenommen und mittels Flashchromatographie (Cyclohexan/Essigsäureethylester-Gradient) gereinigt und 87.4 mg (65% d. Th., 99% Reinheit) der Titelverbindung erhalten.
$^1$H-NMR (400 MHz, DMSO-$d_6$): δ [ppm] = 10.51 (d, 1H), 8.59 (s, 1H), 8.29 (d, 1H), 7.77-7.69 (m, 1H), 7.66-7.59 (m, 1H), 7.52-7.46 (m, 1H), 7.45-7.39 (m, 1H), 6.94 (d, 1H), 4.80-4.66 (m, 1H), 2.93 (br. s, 6H), 1.94-1.82 (m, 1H), 1.71-1.56 (m, 1H), 0.97 (t, 3H).
LC-MS (Methode 3): $R_t$ = 2.18 min; 437 $[M+H]^+$.

**Beispiel 204:**

*rac*-7-(Dimethylamino)-1-(2-fluorphenyl)-4-oxo-*N*- [1,1,1-trifluorbutan-2-yl]-1,4-dihydro-1,8-naphthyridin-3- carbonsäureamid

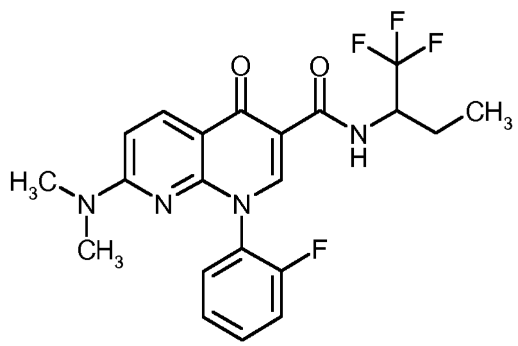

Gemäß AAV1 wurden 100 mg der Verbindung aus Beispiel 62A mit 75.0 mg (458 µmol) *rac*-2-Trifluormethylpropylamin Hydrochlorid in Gegenwart von 116 mg (306 µmol) HATU und 160µl (917 µmol) *N*,*N*-Diisopropylethylamin in 3.2 ml Dimethylformamid umgesetzt. Die Reaktion wurde durch Zugabe von wässr. 1 M Salzsäure und 10 ml Wasser beendet und der Niederschlag abgesaugt. Der Niederschlag wurde mit Wasser gewaschen und über Nacht am Hochvakuum getrocknet. Der Rückstand wurde in 2 ml Dichlormethan aufgenommen und mittels Flashchromatographie (Cyclohexan/Essigsäureethylester-Gradient) gereinigt und 98.6 mg (73% d. Th., 99% Reinheit) der Titelverbindung erhalten.
$^1$H-NMR (400 MHz, DMSO-$d_6$): δ [ppm] = 10.51 (d, 1H), 8.59 (s, 1H), 8.29 (d, 1H), 7.76-7.69 (m, 1H), 7.66-7.59 (m, 1H), 7.52-7.46 (m, 1H), 7.45-7.39 (m, 1H), 6.94 (d, 1H), 4.80-4.68 (m, 1H), 2.93 (br. s, 6H), 1.93-1.84 (m, 1H), 1.69-1.58 (m, 1H), 0.97 (t, 3H).
LC-MS (Methode 3): $R_t$ = 2.14 min; 437 $[M+H]^+$.

**Beispiel 205:**

1-(2,4-Difluorphenyl)-4-oxo-7-(2-oxoazetidin-1-yl)-*N*-[(2*R*)-1,1,1-trifluorbutan-2-yl]-1,4-dihydro-1,8-naphthyridin-3 -carbonsäureamid

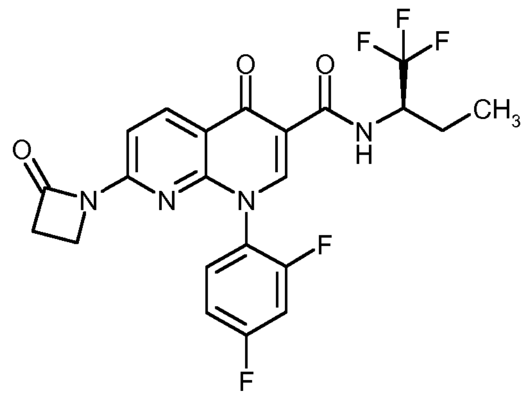

Gemäß AAV2 wurden 100 mg (224 μmol) der Verbindung aus Beispiel 67A mit 15.9 mg (224 μmol) Azetidinon in Gegenwart von 11.6 mg (0.011 mmol) Tris(dibenzylidenaceton)dipalladium-Chloroform-Komplex, 19 mg (34 μmol) Xantphos in 5 ml Toluol bei 90°C zur Reaktion gebracht. Das Rohprodukt wurde mittels Flashchromatographie (Cyclohexan/Essigsäureethylester-Gradient) und präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 18.5 mg (17% d. Th., 99% Reinheit) der Titelverbindung erhalten.
$^1$H-NMR (400 MHz, DMSO-d6): δ [ppm] = 10.20 (d, 1H), 8.83 (s, 1H), 8.69 (d, 1H), 7.90-7.82 (m, 1H), 7.74 (d, 1H), 7.64-7.57 (m, 1H), 7.39-7.31 (m, 1H), 4.83-4.71 (m, 1H), 3.45-3.35 (m, 2H), 3.14-3.07 (m, 2H), 1.95-1.83 (m, 1H), 1.73-1.59 (m, 1H), 0.97 (t, 3H).
LC-MS (Methode 3): $R_t$ = 2.15 min; 481 $[M+H]^+$.

**Beispiel 206:**

*rac*-*N*-[(1*R*)-1-(2-Chlorphenyl)-2,2,2-trifluorethyl] -1 -(2,4-difluorphenyl)-7- [3 -hydroxy-2-oxopiperidin-1 - yl]-4-oxo-1,4-dihydro-1, 8-naphthyridin-3 -carbonsäureamid (Diastereomerengemisch)

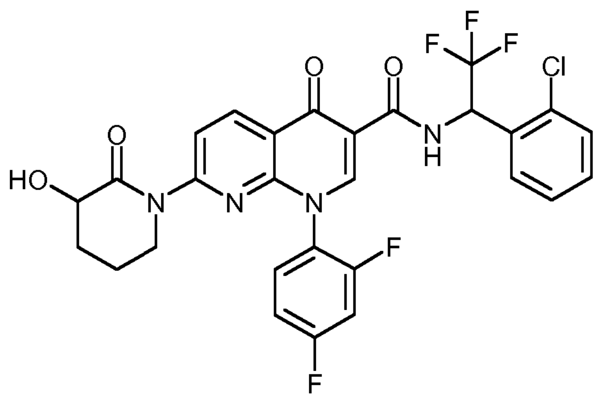

Gemäß AAV2 wurden 150 mg (273 μmol, 96% Reinheit) der Verbindung aus Beispiel 73A mit 31.4 mg (273 μmol) 3-Hydroxy-2-piperidon in Gegenwart von 56.5 mg (409 μmol) Kaliumcarbonat, 6.1 mg (27 μmol) Palladium(II)acetat und 32 mg (55 μmol) Xantphos in 2.7 ml Dioxan zur Reaktion gebracht. Der Rückstand wurde mittels Flashchromatographie (Cyclohexan/Essigsäureethylester-Gradient) und präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90%

Acetonitril und weitere 3 min. 90% Acetonitril) gereinigt. Es wurden 69.8 mg (42% d. Th., 100% Reinheit) der Titelverbindung erhalten. $^{1}$H-NMR (400 MHz, DMSO-$d_6$): δ [ppm] = 11.29 (d, 1H), 8.88 (s, 1H), 8.70 (d, 1H), 8.19-8.10 (m, 1H), 7.93-7.77 (m, 1H), 7.67-7.48 (m, 5H), 7.40-7.31 (m, 1H), 6.53-6.42 (m, 1H), 5.53-5.46 (m, 1H), 4.27-4.18 (m, 1H), 3.71-3.59 (m, 1H), 3.54-3.41 (m, 1H), 2.12-2.02 (m, 1H), 1.84-1.73 (m, 2H), 1.71-1.58 (m, 1H). LC-MS (Methode 3): $R_t$ = 2.18 min; 607 [M+H]$^+$.

58 mg der Titelverbindung (racemisches Diastereomerengemisch) wurde durch chirale HPLC in die enantiomeren Diastereomere getrennt (präparative HPLC: Säule Chiralcel OZ-H 5 µm 250x20 mm; Eluent: 75% Ethanol, 25% Iso-Hexan; Temperatur: 40°C; Fluss: 15 ml/min; UV-Detektion: 220 nm). Man erhielt (in der Reihenfolge der Elution von der Säule) 14 mg Diastereomer 1 (Enantiomer A) (99% de) $R_t$ = 6.63 min, 12 mg (99%de) Diastereomer 2 (Enantiomer A) $R_t$ = 7.71 min, 11 mg (99%de) Diastereomer 1 (Enantiomer B) $R_t$ = 12.9 min und 18 mg (99%de) Diastereomer 2 (Enantiomer B) $R_t$ = 18.3 min.

[Analytische HPLC:Säule Chiralcel OZ-H 5 µm 250x4.6 mm; Eluent: 75% Ethanol, 25% Iso-Hexan; Temperatur: 40°C; Fluss: 1 ml/min; UV-Detektion: 220 nm]

Diastereomer 1 (Enantiomer A) wurde zusätzlich mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 10.4 mg (6.2% d. Th., 99% Reinheit) der Titelverbindung aus Beispiel 207 erhalten.

Diastereomer 2 (Enantiomer A) wurde zusätzlich mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 8.4 mg (5% d. Th., 99% Reinheit) der Titelverbindung aus Beispiel 208 erhalten.

Diastereomer 1 (Enantiomer B) wurde zusätzlich mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 8.7 mg (5% d. Th., 99% Reinheit) der Titelverbindung aus Beispiel 209 erhalten.

Diastereomer 2 (Enantiomer B) wurde zusätzlich mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 11.4 mg (6.8% d. Th., 99% Reinheit) der Titelverbindung aus Beispiel 210 erhalten.

## Beispiel 207:

*N*-[(1*R*)-1-(2-Chlorphenyl)-2,2,2-trifluorethyl] -1 -(2,4-difluorphenyl)-7- [3 -hydroxy-2-oxopiperidin-1 -yl] - 4-oxo-1,4-dihydro-1,8-naphthyridin-3-carbonsäureamid (Diastereomer 1, Enantiomer A)

$^{1}$H-NMR (400 MHz, DMSO-$d_6$): δ [ppm] = 11.29 (d, 1H), 8.88 (s, 1H), 8.70 (d, 1H), 8.18-8.11 (m, 1H), 7.92-7.77 (m, 1H), 7.67-7.48 (m, 5H), 7.40-7.31 (m, 1H), 6.53-6.43 (m, 1H), 5.52-5.47 (m, 1H), 4.26-4.18 (m, 1H), 3.72-3.59 (m, 1H), 3.52-3.41 (m, 1H), 2.11-2.02 (m, 1H), 1.84-1.73 (m, 2H), 1.71-1.58 (m, 1H). LC-MS (Methode 3): $R_t$ = 2.21 min; 607 [M+H]$^+$.

## Beispiel 208:

*N*-[(1*R*)-1-(2-Chlorphenyl)-2,2,2-trifluorethyl]-1-(2,4-difluorphenyl)-7-[3-hydroxy-2-oxopiperidin-1-yl]-4-oxo-1,4-dihydro-1,8-naphthyridin-3-carbonsäureamid (Diastereomer 2, Enantiomer A)

$^{1}$H-NMR (400 MHz, DMSO-$d_6$): δ [ppm] = 11.29 (d, 1H), 8.88 (s, 1H), 8.70 (d, 1H), 8.18-8.11 (m, 1H), 7.91-7.78 (m, 1H), 7.67-7.48 (m, 5H), 7.40-7.31 (m, 1H), 6.53-6.43 (m, 1H), 5.52-5.47 (m, 1H), 4.26-4.18 (m, 1H), 3.72-3.59 (m, 1H), 3.52-3.41 (m, 1H), 2.11-2.02 (m, 1H), 1.84-1.73 (m, 2H), 1.71-1.58 (m, 1H). LC-MS (Methode 3): $R_t$ = 2.21 min; 607 [M+H]$^+$.

## Beispiel 209:

*N*-[(1*R*)-1-(2-Chlorphenyl)-2,2,2-trifluorethyl] -1-(2,4-difluorphenyl)-7-[3-hydroxy-2-oxopiperidin-1-yl]-4-oxo-1,4-dihydro-1,8-naphthyridin-3-carbonsäureamid (Diastereomer 1, Enantiomer B)

$^{1}$H-NMR (400 MHz, DMSO-$d_6$): δ [ppm] = 11.29 (d, 1H), 8.88 (s, 1H), 8.70 (d, 1H), 8.18-8.11 (m, 1H), 7.91-7.78 (m, 1H), 7.67-7.48 (m, 5H), 7.40-7.31 (m, 1H), 6.53-6.43 (m, 1H), 5.52-5.47 (m, 1H), 4.26-4.18 (m, 1H), 3.72-3.59 (m, 1H), 3.52-3.41 (m, 1H), 2.11-2.02 (m, 1H), 1.84-1.73 (m, 2H), 1.71-1.58 (m, 1H). LC-MS (Methode 3): $R_t$ = 2.20 min;

607 [M+H]+.

**Beispiel 210:**

*N*-[(1*R*)-1-(2-Chlorphenyl)-2,2,2-trifluorethyl] -1-(2,4-difluorphenyl)-7-[3-hydroxy-2-oxopiperidin-1-yl]-4-oxo-1,4-dihydro-1,8-naphthyridin-3-carbonsäureamid (Diastereomer 2, Enantiomer B)

$^1$H-NMR (400 MHz, DMSO-$d_6$): δ [ppm] = 11.29 (d, 1H), 8.88 (s, 1H), 8.70 (d, 1H), 8.18-8.11 (m, 1H), 7.92-7.77 (m, 1H), 7.67-7.48 (m, 5H), 7.40-7.31 (m, 1H), 6.53-6.43 (m, 1H), 5.52-5.47 (m, 1H), 4.26-4.18 (m, 1H), 3.72-3.59 (m, 1H), 3.52-3.41 (m, 1H), 2.11-2.02 (m, 1H), 1.84-1.73 (m, 2H), 1.71-1.58 (m, 1H). LC-MS (Methode 3): $R_t$ = 2.21 min; 607 [M+H]+.

**Beispiel 211:**

*N*-[1-(2-Chlorphenyl)-2,2,2-trifluorethyl]-1-(2,4-difluorphenyl)-7-[(4*S*)-4-hydroxy-2-oxopyrrolidin-1-yl]-4-oxo-1,4-dihydro-1,8-naphthyridin-3-carbonsäureamid (Diastereomerengemisch)

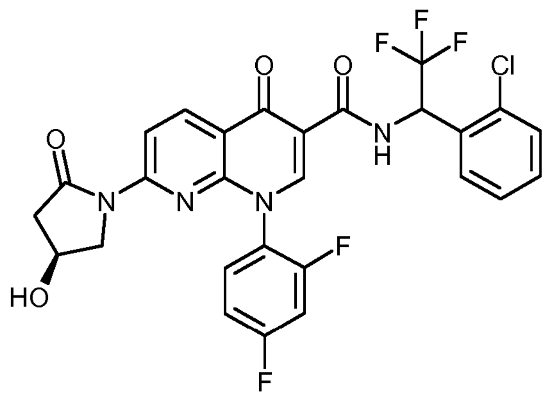

Gemäß AAV2 wurden 750 mg (1.36 mmol, 96% Reinheit) der Verbindung aus Beispiel 73A mit 138 mg (1.36 mmol) (4*S*)-4-Hydroxypyrrolidin-2-on in Gegenwart von 283 mg (2.04 mmol) Kaliumcarbonat, 30.6 mg (136 μmol) Palladium(II)acetat und 158 mg (273 μmol) Xantphos in 14 ml Dioxan zur Reaktion gebracht. Es wurde filtriert, mit Acetonitril nachgewaschen, eingeengt und das Rohprodukt wurde mittels Flashchromatographie (Cyclohexan/Essigsäureethylester-Gradient) gereinigt. Es wurden 555 mg (65% d. Th., 95% Reinheit) der Titelverbindung erhalten.
$^1$H-NMR (400 MHz, DMSO-$d_6$): δ [ppm] = 11.33 (d, 1H), 8.86 (s, 1H), 8.75 (d, 1H), 8.57-8.51 (m, 1H), 7.93-7.76 (m, 1H), 7.67-7.48 (m, 5H), 7.41-7.32 (m, 1H), 6.53-6.42 (m, 1H), 5.37-5.27 (m, 1H), 4.31-4.25 (m, 1H), 3.72-3.61 (m, 1H), 3.51-3.42 (m, 1H), 3.00-2.88 (m, 1H), 2.42-2.31 (m, 1H).
LC-MS (Methode 3): $R_t$ = 2.13 min; 593 [M+H]+.

550 mg der Titelverbindung (Diastereomerengemisch) wurde durch chirale HPLC in die Diastereomere getrennt (präparative HPLC: Säule Chiralpak AZ-H 5 μm 250x20 mm; Eluent: 50% Iso-Propanol, 50% Iso-Hexan; Temperatur: 25°C; Fluss: 15 ml/min; UV-Detektion: 210 nm).

Man erhielt (in der Reihenfolge der Elution von der Säule) 229 mg Diastereomer 1 (99% de) $R_t$ = 0.96 min und 235 mg (99%de) Diastereomer 2 $R_t$ = 1.44 min.

[Analytische HPLC:Säule Chiralcel AZ-3 3 μm 50x4.6 mm; Eluent: 50% Iso-Propanol, 50% Iso-Hexan; Fluss: 1 ml/min; UV-Detektion: 220 nm]

Diastereomer 1 wurde zusätzlich mittels präparativer HPLC (Säule: Chromatorex C18, 10μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 151.6 mg (19% d. Th., 100% Reinheit) der Titelverbindung aus Beispiel 212 erhalten.

Diastereomer 2 wurde zusätzlich mittels präparativer HPLC (Säule: Chromatorex C18, 10μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 151.7 mg (19% d. Th., 100% Reinheit) der Titelverbindung aus Beispiel 213 erhalten.

**Beispiel 212:**

*N*-[1-(2-Chlorphenyl)-2,2,2-trifluorethyl]-1-(2,4-difluorphenyl)-7-[(4*S*)-4-hydroxy-2-oxopyrrolidin-1-yl]-4-oxo-1,4-dihydro-1,8-naphthyridin-3-carbonsäureamid (Diastereomer A)

$^1$H-NMR (400 MHz, DMSO-$d_6$): δ [ppm] = 11.33 (d, 1H), 8.86 (s, 1H), 8.75 (d, 1H), 8.57-8.51 (m, 1H), 7.91-7.78 (m, 1H), 7.67-7.48 (m, 5H), 7.41-7.33 (m, 1H), 6.52-6.42 (m, 1H), 5.32 (dd, 1H), 4.31-4.25 (m, 1H), 3.72-3.61 (m, 1H), 3.51-3.41 (m, 1H), 3.00-2.88 (m, 1H), 2.41-2.31 (m, 1H).
LC-MS (Methode 3): $R_t$ = 2.07 min; 593 $[M+H]^+$.

**Beispiel 213:**

*N*-[1-(2-Chlorphenyl)-2,2,2-trifluorethyl]-1-(2,4-difluorphenyl)-7-[(4*S*)-4-hydroxy-2-oxopyrrolidin-1-yl]-4-oxo-1,4-dihydro-1,8-naphthyridin-3-carbonsäureamid (Diastereomer B)

$^1$H-NMR (400 MHz, DMSO-$d_6$): δ [ppm] = 11.33 (d, 1H), 8.86 (s, 1H), 8.75 (d, 1H), 8.57-8.51 (m, 1H), 7.93-7.76 (m, 1H), 7.67-7.48 (m, 5H), 7.42-7.32 (m, 1H), 6.52-6.42 (m, 1H), 5.32 (dd, 1H), 4.31-4.25 (m, 1H), 3.71-3.61 (m, 1H), 3.52-3.42 (m, 1H), 3.00-2.88 (m, 1H), 2.42-2.32 (m, 1H).
LC-MS (Methode 3): $R_t$ = 2.07 min; 593 $[M+H]^+$.

**Beispiel 214:**

1-(2,4-Difluorphenyl)-*N*-[1-(2,6-difluorphenyl)ethyl]-7-[(4*S*)-4-hydroxy-2-oxopyrrolidin-1-yl]-4-oxo-1,4-dihydro-1, 8-naphthyridin-3 -carbonsäureamid (Diastereomerengemisch)

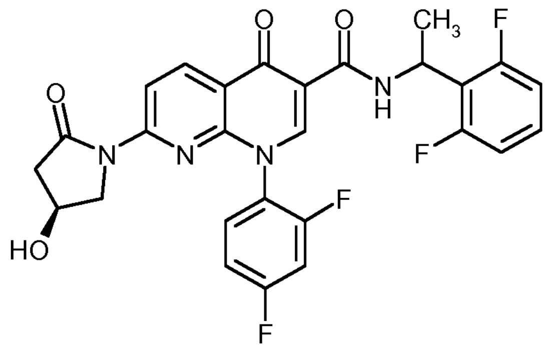

Gemäß AAV1 wurden 90 mg (0.15 mmol, 65% Reinheit) der Verbindung aus Beispiel 63A mit 34.4 mg (219 μmol) *rac*-1-(2,6-Difluorphenyl)ethylamin in Gegenwart von 55.4 mg (146 μmol) HATU und 35.5 μl (204 μmol) *N*,*N*-Diisopropylethylamin in 1.44 ml Dimethylformamid umgesetzt. Das Rohprodukt wurde mittels Flashchromatographie (Cyclohexan/Essigsäureethylester-Gradient) gereinigt und es wurden 51.2 mg (65% d. Th., 100% Reinheit) der Titelverbindung erhalten.
$^1$H-NMR (400 MHz, DMSO-$d_6$): δ [ppm] = 10.54-10.48 (m, 1H), 8.75-8.67 (m, 2H), 8.53-8.46 (m, 1H), 7.89-7.75 (m, 1H), 7.66-7.55 (m, 1H), 7.43-7.30 (m, 2H), 7.16-7.05 (m, 2H), 5.68-5.56 (m, 1H), 5.35-5.26 (m, 1H), 4.31-4.24 (m, 1H), 3.71-3.60 (m, 1H), 3.52-3.40 (m, 1H), 2.99-2.87 (m, 1H), 2.40-2.31 (m, 1H), 1.57 (d, 3H).
LC-MS (Methode 1): $R_t$ = 1.02 min; 541 $[M+H]^+$.

39 mg der Titelverbindung (Diastereomerengemisch) wurde durch chirale HPLC in die Diastereomere getrennt (präparative HPLC: Säule Chiralpak AZ-H 5 μm 250x20 mm; Eluent: 50% Iso-Propanol, 50% Iso-Hexan; Temperatur: 25°C; Fluss: 15 ml/min; UV-Detektion: 210 nm).

Man erhielt (in der Reihenfolge der Elution von der Säule) 14.9 mg Diastereomer 1 (99% de) $R_t$ = 1.14 min und 12.9 mg (99%de) Diastereomer 2 $R_t$ = 1.81 min.

[Analytische HPLC:Säule Chiralcel AZ-3 3 μm 50x4.6 mm; Eluent: 50% Iso-Propanol, 50% Iso-Hexan; Fluss: 1 ml/min; UV-Detektion: 220 nm].

Diastereomer 1 wurde zusätzlich mittels Flashchromatographie (Cyclohexan/Essigsäureethylester-Gradient) gereinigt und 14.3 mg (18% d. Th., 100% Reinheit) der Verbindung aus Beispiel 215 erhalten. Diastereomer 2 wurde

zusätzlich mittels Flashchromatographie (Cyclohexan/Essigsäureethylester-Gradient) gereinigt und 9.8 mg (12% d. Th., 100% Reinheit) der Verbindung aus Beispiel 216 erhalten.

**Beispiel 215:**

1-(2,4-Difluorphenyl)-*N*-[1-(2,6-difluorphenyl)ethyl]-7-[(4*S*)-4-hydroxy-2-oxopyrrolidin-1-yl]-4-oxo-1,4-dihydro-1,8-naphthyridin-3-carbonsäureamid (Diastereomer 1)

$^1$H-NMR (400 MHz, DMSO-$d_6$): $\delta$ [ppm] = 10.54-10.49 (m, 1H), 8.75-8.69 (m, 2H), 8.52-8.47 (m, 1H), 7.87-7.78 (m, 1H), 7.65-7.58 (m, 1H), 7.43-7.32 (m, 2H), 7.15-7.07 (m, 2H), 5.68-5.56 (m, 1H), 5.35-5.25 (m, 1H), 4.31-4.24 (m, 1H), 3.71-3.60 (m, 1H), 3.51-3.41 (m, 1H), 2.99-2.87 (m, 1H), 2.40-2.31 (m, 1H), 1.57 (d, 3H).
LC-MS (Methode 1): $R_t$ = 0.99 min; 541 $[M+H]^+$.

**Beispiel 216:**

1-(2,4-Difluorphenyl)-*N*-[1-(2,6-difluorphenyl)ethyl]-7-[(4*S*)-4-hydroxy-2-oxopyrrolidin-1-yl]-4-oxo-1,4-dihydro-1,8-naphthyridin-3-carbonsäureamid (Diastereomer 2)

$^1$H-NMR (400 MHz, DMSO-$d_6$): $\delta$ [ppm] = 10.54-10.48 (m, 1H), 8.75-8.69 (m, 2H), 8.53-8.47 (m, 1H), 7.90-7.76 (m, 1H), 7.66-7.56 (m, 1H), 7.43-7.31 (m, 2H), 7.16-7.07 (m, 2H), 5.68-5.57 (m, 1H), 5.31 (dd, 1H), 4.31-4.24 (m, 1H), 3.71-3.60 (m, 1H), 3.52-3.41 (m, 1H), 2.99-2.86 (m, 1H), 2.41-2.31 (m, 1H), 1.57 (d, 3H).
LC-MS (Methode 1): $R_t$ = 0.98 min; 541 $[M+H]^+$.

**Beispiel 217:**

*N*-[1-(2-Chlorphenyl)-2,2,2-trifluorethyl]-1-(2,4-difluorphenyl)-7-[(3*S*)-3-fluoropyrrolidin-1-yl]-4-oxo-1,4-dihydro-1,8-naphthyridin-3-carbonsäureamid (Diastereomerengemisch)

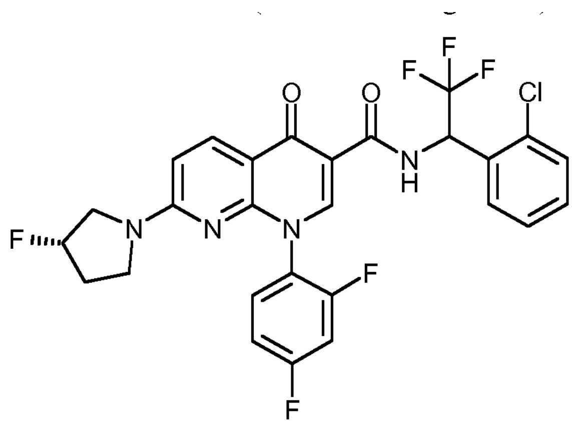

Gemäß AAV3 wurden 150 mg (273 µmol, 96% Reinheit) der Verbindung aus Beispiel 73A mit 41 mg (0.33 mmol) (*S*)-3-Fluorpyrrolidin Hydrochlorid und 0.21 ml (1.2 mmol) *N*,*N*-Diisopropylethylamin in 2.7 ml Dimethylformamid zur Reaktion gebracht. Das Rohprodukt wurde mit Acetonitril verdünnt und mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 142 mg (90% d. Th., 100% Reinheit) der Titelverbindung erhalten.
$^1$H-NMR (400 MHz, DMSO-$d_6$): $\delta$ [ppm] = 11.62 (d, 1H), 8.64 (s, 1H), 8.37 (d, 1H), 7.88-7.70 (m, 1H), 7.65-7.45 (m, 1H), 7.37-7.26 (m, 1H), 6.88-6.77 (m, 1H), 6.50-6.40 (m, 1H), 5.55-5.23 (m, 1H), 3.84-3.06 (m, 4H), 2.34-1.96 (m, 2H).
LC-MS (Methode 3): $R_t$ = 2.40 min; 581 $[M+H]^+$.

**Beispiel 218:**

1-(2,4-Difluorphenyl)-7-[3-hydroxypyrrolidin-1-yl]-4-oxo-*N*-[(2*S*)-1,1,1-trifluorbutan-2-yl]-1,4-dihydro-1,8-naphthyridin-3-carbonsäureamid (Diastereomerengemisch)

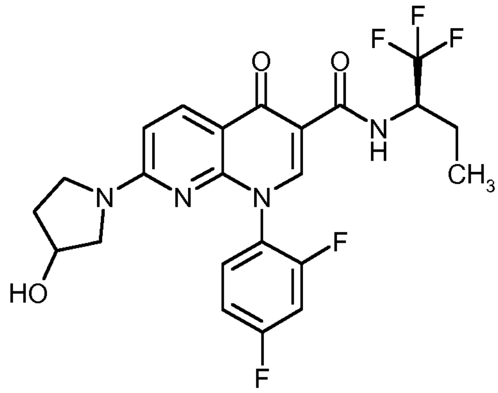

Gemäß AAV1 wurden 250 mg (626 µmol) der Verbindung aus Beispiel 88A mit 154 mg (939 µmol) (2*R*)-1,1,1-Trifluorbutan-2-amin Hydrochlorid in Gegenwart von 238 mg (626 µmol) HATU und 153 µl (876 µmol) *N*,*N*-Diisopropylethylamin in 6.3 ml Dimethylformamid zur Reaktion gebracht. Nach Aufreinigung mittels Flashchromatographie (Cyclohexan/Essigsäureethylester-Gradient) wurden 204 mg (66% d. Th., 100% Reinheit) der Titelverbindung erhalten.
$^1$H-NMR (400 MHz, DMSO-$d_6$): δ [ppm] = 10.53 (d, 1H), 8.61 (s, 1H), 8.28 (d, 1H), 7.86-7.75 (m, 1H), 7.61-7.53 (m, 1H), 7.36-7.28 (m, 1H), 6.80-6.70 (m, 1H), 5.10-4.87 (m, 1H), 4.80-4.67 (m, 1H), 4.43.4.21 (m, 1H), 3.60-3.01 (m, 4H), 2.09-1.56 (m, 4H), 0.97 (t, 3H).
LC-MS (Methode 1): $R_t$ = 1.05 min; 497 $[M+H]^+$.

169.9 mg der Titelverbindung (Diastereomerengemisch) wurde durch chirale HPLC in die Diastereomere getrennt (präparative HPLC: Trennmethode: Säule: Chiralcel OZ-H 5 µm 250x20 mm; Eluent: 25% Iso-Propanol, 75% Iso-Hexan; Temperatur: 30°C; Fluss: 15 ml/min; UV-Detektion: 270 nm).

Man erhielt (in der Reihenfolge der Elution von der Säule) 88 mg Diastereomer 1 (99% de) $R_t$ = 5.35 min und 75 mg (97%de) Diastereomer 2 $R_t$ = 5.91 min.

[Analytische HPLC:Säule Chiralcel OZ-H 5 µm 250x4.6 mm; Eluent: 30% Iso-Propanol, 70% Iso-Hexan; Temperatur: 30°C; Fluss: 1 ml/min; UV-Detektion: 270 nm].

Diastereomer 1 wurde zusätzlich mittels Flashchromatographie (Cyclohexan/Essigsäureethylester-Gradient) gereinigt und 60 mg (19% d. Th., 97% Reinheit) der Titelverbindung aus Beispiel 219 erhalten. Diastereomer 2 wurde zusätzlich mittels Flashchromatographie (Cyclohexan/Essigsäureethylester-Gradient) gereinigt und 46 mg (14% d. Th., 98% Reinheit) der Titelverbindung aus Beispiel 220 erhalten.

**Beispiel 219:**

1-(2,4-Difluorphenyl)-7-[3-hydroxypyrrolidin-1-yl]-4-oxo-*N*-[(2*S*)-1,1,1-trifluorbutan-2-yl]-1,4-dihydro-1,8-naphthyridin-3-carbonsäureamid (Diastereomer 1)

$^1$H-NMR (400 MHz, DMSO-$d_6$): δ [ppm] = 10.52 (d, 1H), 8.61 (s, 1H), 8.28 (d, 1H), 7.85-7.76 (m, 1H), 7.61-7.51 (m, 1H), 7.36-7.27 (m, 1H), 6.80-6.70 (m, 1H), 5.09-4.88 (m, 1H), 4.80-4.67 (m, 1H), 4.42.4.21 (m, 1H), 3.60-2.98 (m, 4H), 2.08-1.55 (m, 4H), 0.97 (t, 3H).
LC-MS (Methode 1): $R_t$ = 1.01 min; 497 $[M+H]^+$.

**Beispiel 220:**

1-(2,4-Difluorphenyl)-7-[3-hydroxypyrrolidin-1-yl]-4-oxo-*N*-[(2*S*)-1,1,1-trifluorbutan-2-yl]-1,4-dihydro-1,8-naphthyridin-3-carbonsäureamid (Diastereomer 2)

$^1$H-NMR (400 MHz, DMSO-$d_6$): δ [ppm] = 10.53 (d, 1H), 8.61 (s, 1H), 8.28 (d, 1H), 7.86-7.76 (m, 1H), 7.61-7.53 (m, 1H), 7.36-7.28 (m, 1H), 6.80-6.71 (m, 1H), 5.08-4.87 (m, 1H), 4.80-4.67 (m, 1H), 4.43.4.22 (m, 1H), 3.58-3.00 (m,

4H), 2.08-1.57 (m, 4H), 0.97 (t, 3H).
LC-MS (Methode 1): $R_t$ = 1.05 min; 497 $[M+H]^+$.

**Beispiel 221:**

1-(2,4-Difluorphenyl)-4-oxo-7-[4-oxohexahydropyrrolo[3,4-*c*]pyrrol-2(1*H*)-yl]-*N*-[(2*R*)-1,1,1-trifluorbutan-2-yl]-1,4-dihydro-1,8-naphthyridin-3-carbonsäureamid (Diastereomerengemisch)

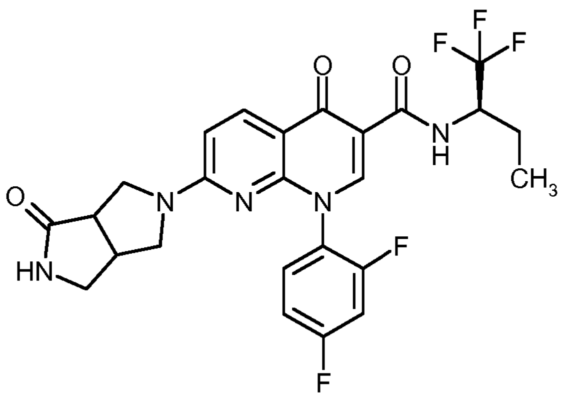

Eine Lösung von 242 mg (381 µmol) des Diastereomerengemisches aus Beispiel 83A in 3 ml THF wurde bei 0°C mit 476 µl (1.90 mmol) Salzsäure (4M in Dioxan) versetzt. Es wurde 30 min bei 0°C und über Nacht bei RT nachgerührt. Das Lösungsmittel wurde unter reduziertem Druck entfernt und der Rückstand mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 168 mg (83% d. Th., 100% Reinheit) der Titelverbindung erhalten.
$^1$H-NMR (400 MHz, DMSO-$d_6$): δ [ppm] = 10.48 (d, 1H), 8.62 (s, 1H), 8.31 (d, 1H), 7.87-7.70 (m, 2H), 7.63-7.52 (m, 1H), 7.37-7.27 (m, 1H), 6.87-6.70 (br. s, 1H), 4.80-4.66 (m, 1H), 3.92-2.88 (br. m, 8H), 1.94-1.81 (m, 1H), 1.70-1.56 (m, 1H), 0.97 (t, 3H).
LC-MS (Methode 3): $R_t$ = 1.74 min; 536 $[M+H]^+$.

167 mg der Titelverbindung (Diastereomerengemisch) wurde durch chirale HPLC in die Diastereomere getrennt (präparative HPLC: Säule Chiralpak AD-H 5 µm 250x20 mm; Eluent: 30% Iso-Propanol, 70% Iso-Hexan; Temperatur: 25°C; Fluss: 20 ml/min; UV-Detektion: 230 nm.)

Man erhielt (in der Reihenfolge der Elution von der Säule) 63.6 mg Diastereomer 1 (99% de) $R_t$ = 3.46 min und 67.9 mg (99%de) Diastereomer 2 $R_t$ = 6.09 min.

[Analytische HPLC:Säule Daicel AD-3 3 µm 50x4.6 mm; Eluent: 30% Iso-Propanol, 70% Iso-Hexan; UV-Detektion: 220 nm].

Diastereomer 1 wurde zusätzlich mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 41 mg (20% d. Th., 100% Reinheit) der Titelverbindung aus Beispiel 222 erhalten.

Diastereomer 2 wurde zusätzlich mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 38 mg (18% d. Th., 100% Reinheit) der Titelverbindung aus Beispiel 223 erhalten.

**Beispiel 222:**

1-(2,4-Difluorphenyl)-4-oxo-7-[4-oxohexahydropyrrolo[3,4-*c*]pyrrol-2(1*H*)-yl]-*N*-[(2*R*)-1,1,1-trifluorbutan-2-yl]-1,4-dihydro-1,8-naphthyridin-3-carbonsäureamid (Diastereomer 1)
$^1$H-NMR (400 MHz, DMSO-$d_6$): δ [ppm] = 10.48 (d, 1H), 8.62 (s, 1H), 8.31 (d, 1H), 7.86-7.73 (m, 2H), 7.63-7.53 (m, 1H), 7.37-7.28 (m, 1H), 6.87-6.71 (br. s, 1H), 4.80-4.68 (m, 1H), 3.93-2.89 (br. m, 8H), 1.94-1.81 (m, 1H), 1.70-1.57 (m, 1H), 0.97 (t, 3H).
LC-MS (Methode 1): $R_t$ = 0.97 min; 536 $[M+H]^+$.

**Beispiel 223:**

1-(2,4-Difluorphenyl)-4-oxo-7-[4-oxohexahydropyrrolo[3,4-*c*]pyrrol-2(1*H*)-yl]-*N*-[(2*R*)-1,1,1-trifluorbutan-2-yl]-1,4-dihydro-1,8-naphthyridin-3-carbonsäureamid (Diastereomer 2)
$^1$H-NMR (400 MHz, DMSO-$d_6$): δ [ppm] = 10.48 (d, 1H), 8.62 (s, 1H), 8.31 (d, 1H), 7.86-7.73 (m, 2H), 7.63-7.54 (m, 1H), 7.37-7.28 (m, 1H), 6.87-6.70 (br. s, 1H), 4.81-4.67 (m, 1H), 3.93-2.89 (br. m, 8H), 1.94-1.82 (m, 1H), 1.70-1.57 (m, 1H), 0.97 (t, 3H).
LC-MS (Methode 1): $R_t$ = 0.97 min; 536 $[M+H]^+$.

**Beispiel 224:**

1-(2,4-Difluorphenyl)-7-[3-hydroxy-2-oxopyrrolidin-1-yl]-4-oxo-*N*-[(2*R*)-1,1,1-trifluorbutan-2-yl]-1,4-dihydro-1, 8-naphthyridin-3 -carbonsäureamid (Diastereomerengemisch)

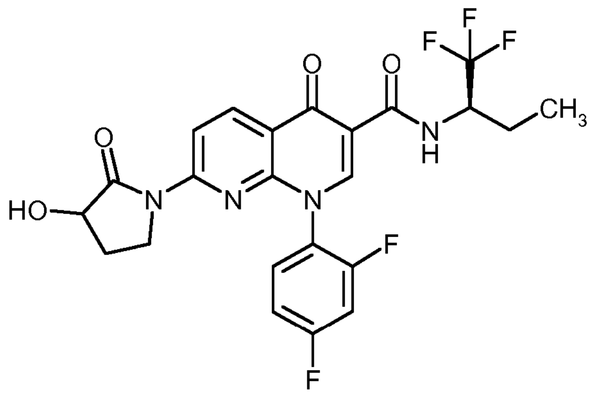

Gemäß AAV2 wurden 150 mg (326 μmol) der Verbindung aus Beispiel 67A (97% Reinheit) mit 33.0 mg (326 μmol) rac-3-Hydroxy-2-pyrrolidin-2-on in Gegenwart von 67.7 mg (490 μmol) Kaliumcarbonat, 7.3 mg (33 μmol) Palladium(II)acetat und 38 mg (65 μmol) Xantphos in 3 ml Dioxan umgesetzt. Das Reaktionsgemisch wurde filtriert, mit Acetonitril nachgewaschen, eingeengt und der Rückstand mittels Flashchromatographie (Cyclohexan/Essigsäureethylester-Gradient) gereinigt. Es wurden 92.5 mg (55% d. Th. 99% Reinheit) der Titelverbindung erhalten.
$^1$H-NMR (400 MHz, DMSO-$d_6$): δ [ppm] = 10.21 (d, 1H), 8.86 (s, 1H), 8.73 (d, 1H), 8.56-8.49 (m, 1H), 7.92-7.82 (m, 1H), 7.67-7.56 (m, 1H), 7.40-7.31 (m, 1H), 5.90 (d, 1H), 4.84-4.70 (m, 1H), 4.44-4.32 (m, 1H), 3.63-3.49 (m, 1H), 3.37-3.23 (m, 1H), 2.37-2.26 (m, 1H), 1.96-1.59 (m, 3H), 0.98 (t, 3H).
LC-MS (Methode 3): $R_t$ = 1.88 min; 511 $[M+H]^+$.

90 mg der Titelverbindung (Diastereomerengemisch) wurde durch chirale HPLC in die Diastereomere getrennt (präparative HPLC: Säule Chiralcel OX-H 5 μm 250x20 mm; Eluent: 40% Ethanol, 60% Iso-Hexan; Temperatur: 30°C; Fluss: 15 ml/min; UV-Detektion: 220 nm)

Man erhielt (in der Reihenfolge der Elution von der Säule) 43 mg Diastereomer 1 (99% de) $R_t$ = 8.76 min und 46 mg (99%de) Diastereomer 2 $R_t$ = 10.65 min.

[Analytische HPLC:Säule Chiralcel OX-H 5 μm 250x4.6 mm; Eluent: 40% Ethanol, 60% Iso-Hexan; Temperatur: 30°C; Fluss: 1 ml/min; UV-Detektion: 220 nm.]

Diastereomer 1 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 36.6 mg (22% d. Th., 100% Reinheit) der Titelverbindung aus Beispiel 225 erhalten.

Diastereomer 2 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 36.7 mg (22% d. Th., 100% Reinheit) der Titelverbindung aus Beispiel 226 erhalten.

**Beispiel 225:**

1-(2,4-Difluorphenyl)-7-[3-hydroxy-2-oxopyrrolidin-1-yl]-4-oxo-*N*-[(2*R*)-1,1,1-trifluorbutan-2-yl]-1,4-dihydro-1,8-naphthyridin-3-carbonsäureamid (Diastereomer 1)
$^1$H-NMR (400 MHz, DMSO-$d_6$): δ [ppm] = 10.21 (d, 1H), 8.86 (s, 1H), 8.73 (d, 1H), 8.56-8.50 (m, 1H), 7.92-7.82 (m, 1H), 7.66-7.57 (m, 1H), 7.40-7.32 (m, 1H), 5.90 (d, 1H), 4.84-4.70 (m, 1H), 4.44-4.33 (m, 1H), 3.63-3.51 (m, 1H), 3.38-3.25 (m, 1H), 2.38-2.26 (m, 1H), 1.96-1.60 (m, 3H), 0.98 (t, 3H).
LC-MS (Methode 3): $R_t$ = 1.86 min; 511 $[M+H]^+$.

**Beispiel 226:**

1-(2,4-Difluorphenyl)-7-[3-hydroxy-2-oxopyrrolidin-1-yl]-4-oxo-*N*-[(2*R*)-1,1,1-trifluorbutan-2-yl]-1,4-dihydro-1,8-naphthyridin-3-carbonsäureamid (Diastereomer 2)
$^1$H-NMR (400 MHz, DMSO-$d_6$): δ [ppm] = 10.21 (d, 1H), 8.86 (s, 1H), 8.73 (d, 1H), 8.56-8.49 (m, 1H), 7.91-7.83 (m, 1H), 7.66-7.57 (m, 1H), 7.39-7.33 (m, 1H), 5.90 (d, 1H), 4.83-4.70 (m, 1H), 4.44-4.33 (m, 1H), 3.63-3.50 (m, 1H), 3.38-3.24 (m, 1H), 2.37-2.26 (m, 1H), 1.96-1.60 (m, 3H), 0.98 (t, 3H).
LC-MS (Methode 3): $R_t$ = 1.87 min; 511 $[M+H]^+$.

**Beispiel 227:**

1-(2,4-Difluorphenyl)-7-[(3*S*)-3-fluorpyrrolidin-1-yl]-4-oxo-*N*-[1-(trifluormethoxy)propan-2-yl]-1,4-dihydro-1,8-naphthyridin-3-carbonsäureamid (Diastereomerengemisch)

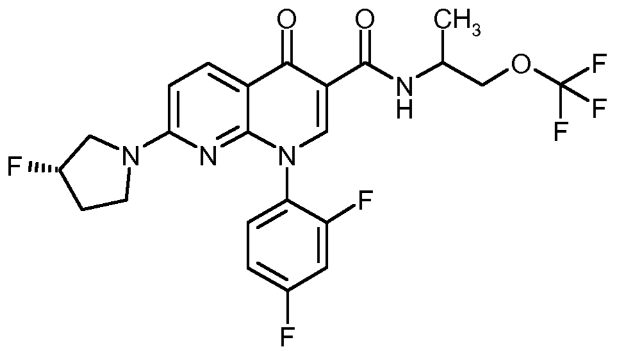

Gemäß AAV3 wurden 150 mg (325 μmol) der Verbindung aus Beispiel 74A mit 48.9 mg (390 μmol) (*S*)-3-Fluorpyrrolidin Hydrochlorid und 0.20 ml (1.1 mmol) *N*,*N*-Diisopropylehtylamin in 1.45 ml Dimethylformamid zur Reaktion gebracht. Das Rohprodukt wurde mit Acetonitril verdünnt und mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 141 mg (84% d. Th., 99% Reinheit) der Titelverbindung erhalten.
$^1$H-NMR (400 MHz, DMSO-$d_6$): δ [ppm] = 10.15 (d, 1H), 8.57 (s, 1H), 8.32 (d, 1H), 7.85-7.74 (m, 1H), 7.64-7.51 (m, 1H), 7.37-7.27 (m, 1H), 6.84-6.74 (m, 1H), 5.56-5.22 (m, 1H), 4.39-4.28 (m, 1H), 4.21-4.11 (m, 2H), 3.88-3.03 (m, 4H), 2.38-1.92 (m, 2H), 1.25 (d, 3H).
LC-MS (Methode 3): $R_t$ = 2.13 min; 515 $[M+H]^+$.
135 mg der Titelverbindung (Diastereomerengemisch) wurde durch chirale HPLC in die Diastereomere getrennt (präparative HPLC: Säule OZ-H 5 μm 250x20 mm; Eluent: 50% Ethanol (mit 2% Diethylamin), 50% Iso-Hexan; Temperatur: 25°C; Fluss: 15 ml/min; UV-Detektion: 210 nm.
Man erhielt (in der Reihenfolge der Elution von der Säule) 50 mg Diastereomer 1 (99% de) $R_t$ = 1.02 min und 57 mg (92.3%de) Diastereomer 2 $R_t$ = 1.33 min.
[Analytische HPLC:Säule Chiralpak OZ-3 3 μm 50x4.6 mm; Eluent: 50% Ethanol (mit 2% Diethylamin), 50% Iso-Hexan; Fluss: 1 ml/min; UV-Detektion: 220 nm.]
Diastereomer 1 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 40.5 mg (24% d. Th., 99% Reinheit) der Titelverbindung aus Beispiel 228 erhalten.
Diastereomer 2 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und

weitere 3 min. 90% Acetonitril) gereinigt und 46.7 mg (28% d. Th., 99% Reinheit) der Titelverbindung aus Beispiel 229 erhalten.

**Beispiel 228:**

1-(2,4-Difluorphenyl)-7-[(3*S*)-3-fluorpyrrolidin-1-yl]-4-oxo-*N*-[1-(trifluormethoxy)propan-2-yl]-1,4-dihydro-1,8-naphthyridin-3-carbonsäureamid (Diastereomer 1)

$^{1}$H-NMR (400 MHz, DMSO-$d_6$): $\delta$ [ppm] = 10.15 (d, 1H), 8.57 (s, 1H), 8.32 (d, 1H), 7.84-7.76 (m, 1H), 7.63-7.52 (m, 1H), 7.37-7.28 (m, 1H), 6.83-6.75 (m, 1H), 5.55-5.24 (m, 1H), 4.39-4.29 (m, 1H), 4.20-4.12 (m, 2H), 3.82-3.05 (m, 4H), 2.35-1.99 (m, 2H), 1.25 (d, 3H).
LC-MS (Methode 3): $R_t$ = 2.09 min; 515 $[M+H]^+$.

**Beispiel 229:**

1-(2,4-Difluorphenyl)-7-[(3*S*)-3-fluorpyrrolidin-1-yl]-4-oxo-*N*-[1-(trifluormethoxy)propan-2-yl]-1,4-dihydro-1,8-naphthyridin-3-carbonsäureamid (Diastereomer 2)

$^{1}$H-NMR (400 MHz, DMSO-$d_6$): $\delta$ [ppm] = 10.15 (d, 1H), 8.57 (s, 1H), 8.32 (d, 1H), 7.84-7.75 (m, 1H), 7.64-7.53 (m, 1H), 7.37-7.27 (m, 1H), 6.84-6.74 (m, 1H), 5.57-5.22 (m, 1H), 4.39-4.29 (m, 1H), 4.21-4.12 (m, 2H), 3.83-3.03 (m, 4H), 2.36-1.92 (m, 2H), 1.25 (d, 3H).
LC-MS (Methode 3): $R_t$ = 2.08 min; 515 $[M+H]^+$.

**Beispiel 230:**

*rac*-1-(2,4-Difluorphenyl)-4-oxo-7-(2-oxopyrrolidin-1-yl)-*N*-[1-(trifluoromethoxy)propan-2-yl]-1,4-dihydro-1,8-naphthyridin-3-carbonsäureamid

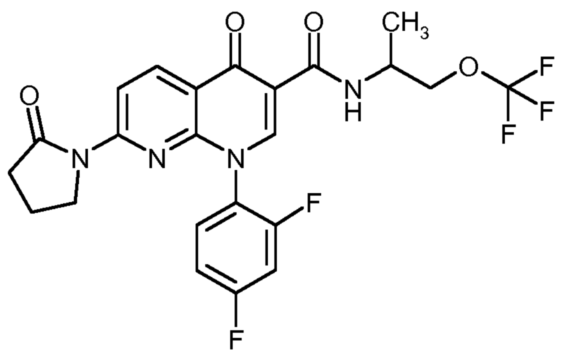

Gemäß AAV2 wurden 150 mg (325 µmol) der Verbindung aus Beispiel 74A mit 27.6 mg (325 µmol) Pyrrolidin-2-on in Gegenwart von 67.3 mg (487 µmol) Kaliumcarbonat, 13 mg (58 µmol) Palladium(II)acetat und 34 mg (58 µmol) Xantphos in 4 ml Dioxan zur Reaktion gebracht. Das Rohprodukt wurde in 3 ml Acetonitril und 0.5 ml Wasser gelöst und mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 3.3 mg (2% d. Th., 99% Reinheit) der Titelverbindung erhalten.
$^{1}$H-NMR (400 MHz, DMSO-$d_6$): $\delta$ [ppm] = 9.92 (d, 1H), 8.78 (s, 1H), 8.69 (d, 1H), 8.49 (d, 1H), 7.89-7.91 (m, 1H), 7.65-7.58 (m, 1H), 7.39-7.33 (m, 1H), 4.41-4.32 (m, 1H), 4.22-4.14 (m, 2H), 3.61-3.50 (m, 2H), 2.00-1.91 (m, 2H), 1.26 (d, 3H).
LC-MS (Methode 3): $R_t$ = 2.04 min; 511 $[M+H]^+$.

**Beispiel 231:**

1-(2,4-Difluorphenyl)-7-(dimethylamino)-4-oxo-*N*-[1-(trifluormethoxy)propan-2-yl]-1,4-dihydro-1,8-naphthyridin-3 -carbonsäureamid

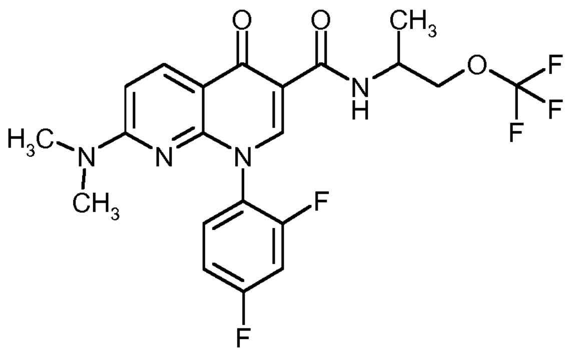

Gemäß AAV1 wurden 100 mg (290 µmol) der Verbindung aus Beispiel 36A mit 78.0 mg (434 µmol) (+)-1-(Trifluormethoxy)propan-2-amin Hydrochlorid (Drehwert: +10°, c = 0.4000g/100ml, MeOH, 20°C) in Gegenwart von 110 mg (290 µmol) HATU und 151 µl (869 µmol) *N*,*N*-Diisopropylethylamin in 3 ml Dimethylformamid zur Reaktion gebracht. Es wurde 1 ml wässrige 1M Salzsäure und 10 ml Wasser zugegeben, der Niederschlag abgesaugt und über Nacht im Hochvakuum getrocknet. Das Rohprodukt wurde mittels Flashchromatographie (Cyclohexan/Essigsäureethylester-Gradient) gereinigt und 80 mg (58% d. Th., 99% Reinheit) der Titelverbindung (nicht-racemisches Gemisch) erhalten.
$^1$H-NMR (400 MHz, DMSO-$d_6$): δ [ppm] = 10.15 (d, 1H), 8.55 (s, 1H), 8.28 (d, 1H), 7.83-7.75 (m, 1H), 7.61-7.54 (m, 1H), 7.35-7.29 (m, 1H), 6.92 (d, 1H), 4.39-4.29 (m, 1H), 4.20-4.12 (m, 2H), 2.94 (br. s, 6H); 1.25 (d, 3H).
LC-MS (Methode 1): $R_t$ = 1.12 min; 471 [M+H]$^+$.

**Beispiel 232:**

1-(2,4-Difluorphenyl)-4-oxo-7-(pyrrolidin-1-yl)-*N*- [1-(trifluormethoxy)propan-2-yl]-1,4-dihydro-1,8-naphthyridin-3-carbonsäureamid

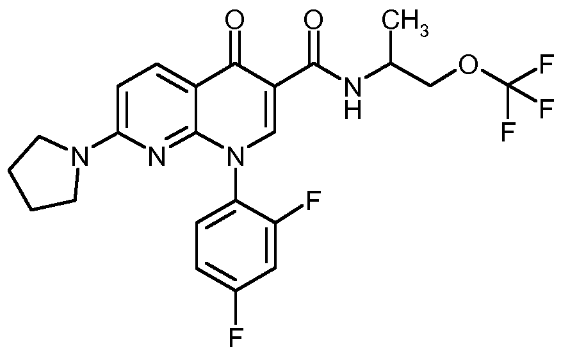

Gemäß AAV1 wurden 100 mg der Verbindung aus Beispiel 57A mit 72.5 mg (404 µmol) (+)-1-(Trifluormethoxy)propan-2-amin Hydrochlorid (Drehwert: +10°, c = 0.4000g/100ml, MeOH, 20°C) in Gegenwart von 102 mg (269 µmol) HATU und 141 µl (808 µmol) *N*,*N*-Diisopropylethylamin in 3 ml Dimethylformamid zur Reaktion gebracht. Es wurde 1 ml wässrige 1M Salzsäure und 10 ml Wasser zugegeben, der Niederschlag abgesaugt und über Nacht im Hochvakuum getrocknet. Das Rohprodukt wurde mittels Flashchromatographie (Cyclohexan/Essigsäureethylester-Gradient) gereinigt und 97 mg (72% d. Th., 99% Reinheit) der Titelverbindung (nicht-racemisches Gemisch) erhalten.
$^1$H-NMR (400 MHz, DMSO-$d_6$): δ [ppm] = 10.17 (d, 1H), 8.54 (s, 1H), 8.27 (d, 1H), 7.82-7.74 (m, 1H), 7.60-7.53 (m, 1H),

7.34-7.28 (m, 1H), 6.73 (d, 1H), 4.39-4.29 (m, 1H), 4.21-4.12 (m, 2H), 3.50-3.35 (br. s, 2H), 3.23-3.02 (br. s, 2H), 2.01-1.73 (m, 4H), 1.25 (d, 3H).
LC-MS (Methode 1): $R_t$ = 1.21 min; 497 $[M+H]^+$.

**Beispiel 233:**

1-(2,4-Difluorphenyl)-7-[(4*S*)-4-hydroxy-2-oxopyrrolidin-1-yl]-4-oxo-*N*-[1-phenyl-2-(trifluormethoxy)ethyl]-1,4-dihydro-1,8-naphthyridin-3-carbonsäureamid

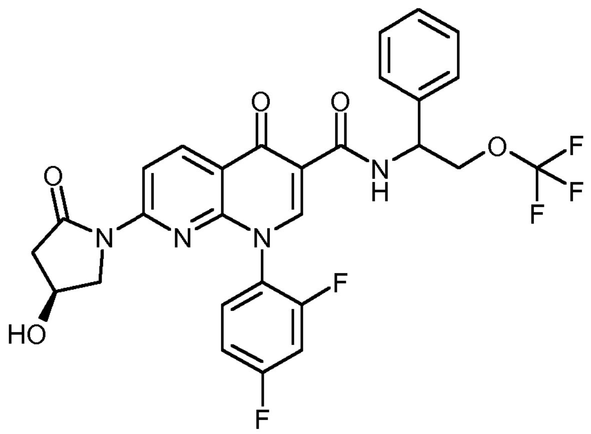

Gemäß AAV2 wurden 200 mg (363 μmol) der Verbindung aus Beispiel 75A (95% Reinheit) mit 36.7 mg (363 μmol) (*S*)-(-)-4-Hydroxy-2-pyrrolidinon in Gegenwart von 75.2 mg (544 μmol) Kaliumcarbonat, 8.1 mg (36 μmol) Palladium(II)acetat und 42 mg (73 μmol) Xantphos in 3.6 ml Dioxan zur Reaktion gebracht. Das Rohprodukt wurde zweimal mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 37.8 mg (18% d. Th., 100% Reinheit) der Titelverbindung (nicht-racemisches Gemisch) erhalten.
$^1$H-NMR (400 MHz, DMSO-$d_6$): δ [ppm] = 10.60 (d, 1H), 8.80 (s, 1H), 8.74 (d, 1H), 8.55-8.48 (m, 1H), 7.90-7.81 (m, 1H), 7.66-7.58 (m, 1H), 7.51-7.29 (m, 6H), 5.55-5.48 (m, 1H), 5.32 (dd, 1H), 4.53-4.40 (m, 2H), 4.31-4.25 (m, 1H), 3.72-3.61 (m, 1H), 3.52-3.42 (m, 1H), 3.00-2.88 (m, 1H), 2.42-2.29 (m, 1H).
LC-MS (Methode 3): $R_t$ = 1.94 min; 589 $[M+H]^+$.

**Beispiel 234:**

1-(2,4-Difluorphenyl)-7-[(4*S*)-4-hydroxy-2-oxopyrrolidin-1-yl]-4-oxo-*N*-[1-(trifluormethoxy)butan-2-yl]-1,4-dihydro-1,8-naphthyridin-3-carbonsäureamid (Diastereomerengemisch)

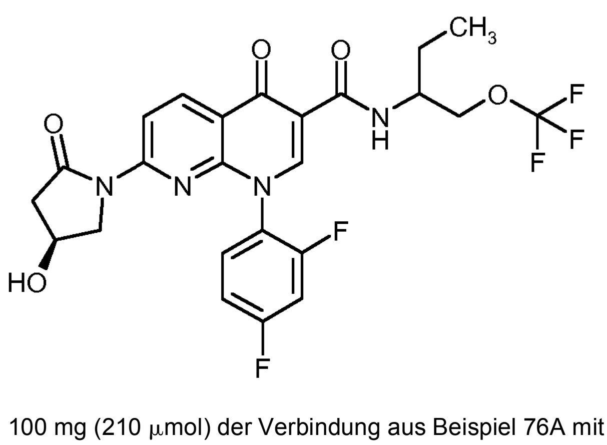

Gemäß AAV2 wurden 100 mg (210 μmol) der Verbindung aus Beispiel 76A mit 21.2 mg (210 μmol) (*S*)-(-)-4-

Hydroxy-2-pyrrolidinon in Gegenwart von 103 mg (315 µmol) Caesiumcarbonat, 8.5 mg (38 µmol) Palladium(II)acetat und 18 mg (31 µmol) Xantphos in 2.1 ml Dioxan zur Reaktion gebracht. Das Rohprodukt wurde mittels Flashchromatographie (Cyclohexan/Essigsäureethylester-Gradient) und präparativer Dünnschichtchromatographie (1 mm Silica-Platten, 20x20 cm, Cyclohexan/Essigsäureethylester = 35/65) gereinigt. Die Produktfraktion wurde durch UV-Detektion sichtbar gemacht, abgekratzt und mit Essigsäureethylester vom Kieselgel eluiert. Es wurde über Celite filtriert und das Lösungsmittel unter reduziertem Druck entfernt. Der Rückstand wurde aus Wasser/Acetonitril lyophilisiert und 17 mg (15% d. Th., 100% Reinheit) der Titelverbindung erhalten.
$^1$H-NMR (400 MHz, DMSO-$d_6$): δ [ppm] = 9.90 (d, 1H), 8.79 (s, 1H), 8.70 (d, 1H), 8.53-8.47 (m, 1H), 7.92-7.81 (m, 1H), 7.67-7.58 (m, 1H), 7.41-7.33 (m, 1H), 5.32 (dd, 1H), 4.31-4.13 (m, 4H), 3.72-3.62 (m, 1H), 3.52-3.43 (m, 1H), 3.00-2.88 (m, 1H), 2.41-2.32 (m, 1H), 1.76-1.53 (m, 2H), 0.95 (t, 3H).
LC-MS (Methode 1): $R_t$ = 0.99 min; 541 $[M+H]^+$.

**Beispiel 235:**

1-(2,4-Difluorphenyl)-*N*-[1-(2-fluorphenyl)ethyl]-7-[(4*S*)-4-hydroxy-2-oxopyrrolidin-1-yl]-4-oxo-1,4-dihydro-1, 8-naphthyridin-3 -carbonsäureamid (Diastereomerengemisch)

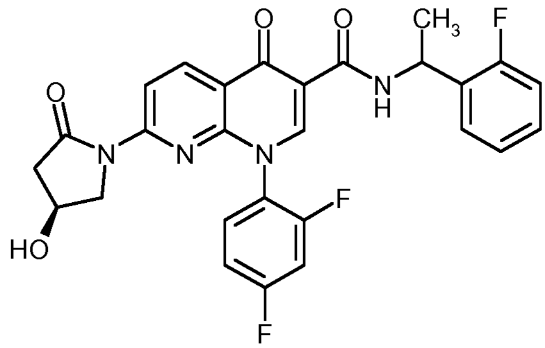

Gemäß AAV2 wurden 97.0 mg (212 µmol) der Verbindung aus Beispiel 77A mit 21.4 mg (212 µmol) (*S*)-(-)-4-Hydroxy-2-pyrrolidinon in Gegenwart von 104 mg (318 µmol) Caesiumcarbonat, 8.6 mg (38 µmol) Palladium(II)acetat und 18 mg (31 µmol) Xantphos in 2 ml Dioxan zur Reaktion gebracht. Das Rohprodukt wurde mittels Flashchromatographie (Cyclohexan/Essigsäureethylester-Gradient) gereinigt und aus Acetonitril/Wasser lyophilisiert. Es wurden 45.8 mg (41% d. Th., 100% Reinheit) der Titelverbindung erhalten.
$^1$H-NMR (400 MHz, DMSO-$d_6$): δ [ppm] = 10.33 (d, 1H), 8.74 (s, 1H), 8.71 (d, 1H), 8.54-8.48 (m, 1H), 7.90-7.78 (m, 1H), 7.66-7.57 (m, 1H), 7.48-7.42 (m, 1H), 7.41-7.29 (m, 2H), 7.24-7.16 (m, 2H), 5.44-5.26 (m, 2H), 4.31-4.24 (m, 1H), 3.72-3.60 (m, 1H), 3.52-3.42 (m, 1H), 2.99-2.87 (m, 1H), 2.42-2.31 (m, 1H), 1.52 (d, 3H).
LC-MS (Methode 1): $R_t$ = 0.97 min; 523 $[M+H]^+$.

**Beispiel 236:**

1-(2,4-Difluorphenyl)-7-[(4*S*)-4-hydroxy-2-oxopyrrolidin-1-yl]-4-oxo-*N*- [1,1,1-trifluor-3-methoxy-2-methylpropan-2-yl]-1,4-dihydro-1,8-naphthyridin-3-carbonsäureamid (Diastereomerengemisch)

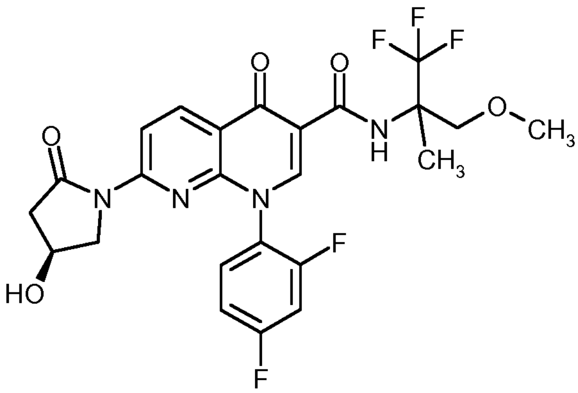

Gemäß AAV2 wurden 110 mg (231 µmol) der Verbindung aus Beispiel 78A mit 23.3 mg (231 µmol) (*S*)-(-)-4-Hydroxy-2-pyrrolidinon in Gegenwart von 96.9 mg (297 µmol) Caesiumcarbonat, 8.0 mg (36 µmol) Palladium(II)acetat und 18 mg (31 µmol) Xantphos in 2.3 ml Dioxan zur Reaktion gebracht. Das Rohprodukt wurde mittels Flashchromatographie (Cyclohexan/Essigsäureethylester-Gradient) und präparativer Dünnschichtchromatographie (1 mm Silica-Platten, 20x20 cm, Dichlormethan/Methanol = 95/5) gereinigt. Die Produktfraktion wurde durch UV-Detektion sichtbar gemacht, abgekratzt und mit Essigsäureethylester vom Kieselgel eluiert. Es wurde über Celite filtriert und das Lösungsmittel unter reduziertem Druck entfernt. Der Rückstand wurde aus Wasser/Acetonitril lyophilisiert und 49 mg (39% d. Th., 100% Reinheit) der Titelverbindung erhalten.
$^1$H-NMR (400 MHz, DMSO-$d_6$): δ [ppm] = 10.50 (br. s, 1H), 8.79 (s, 1H), 8.71 (d, 1H), 8.54-8.48 (m, 1H), 7.91-7.81 (m, 1H), 7.67-7.59 (m, 1H), 7.41-7.33 (m, 1H), 5.32 (dd, 1H), 4.31-4.26 (m, 1H), 3.91-3.84 (m, 1H), 3.77-3.61 (m, 2H), 3.52-3.42 (m, 1H), 3.36 (s, 3H), 2.99-2.88 (m, 1H), 2.41-2.31 (m, 1H), 1.64 (s, 3H).
LC-MS (Methode 1): $R_t$ = 0.96 min; 541 $[M+H]^+$.

**Beispiel 237:**

1-(2,4-Difluorphenyl)-7-[(4*S*)-4-hydroxy-2-oxopyrrolidin-1-yl]-4-oxo-*N*-[4-(trifluormethyl)tetrahydro-2*H*-pyran-4-yl]-1,4-dihydro-1,8-naphthyridin-3-carbonsäureamid

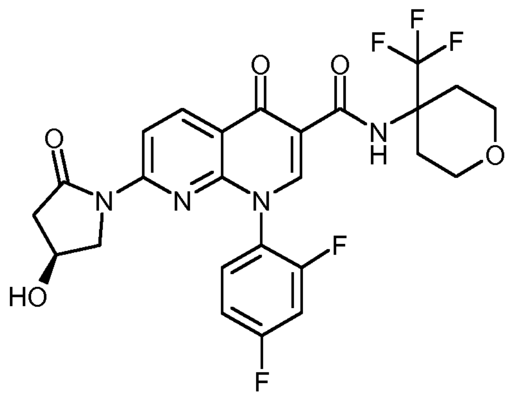

Gemäß AAV2 wurden 106 mg (217 µmol) der Verbindung aus Beispiel 79A mit 21.9 mg (217 µmol) (*S*)-(-)-4-Hydroxy-2-pyrrolidinon in Gegenwart von 106 mg (326 µmol) Caesiumcarbonat, 8.8 mg (39 µmol) Palladium(II)acetat und 23 mg (39 µmol) Xantphos in 2.3 ml Dioxan zur Reaktion gebracht. Das Rohprodukt wurde mittels Flashchromatographie (Cyclohexan/Essigsäureethylester-Gradient) und zweimal mittels präparativer Dünnschichtchromatographie (1 mm Silica-Platten, 20x20 cm, Cyclohexan/Essigsäureethylester = 1/1, dann Dichlormethan/Methanol = 90/10) gereinigt. Die Produktfraktion wurde durch UV-Detektion sichtbar gemacht, abgekratzt und mit Essigsäureethylester bzw. Dichlormethan vom Kieselgel eluiert. Es wurde durch einen Feinfilter filtriert und das Lösungsmittel unter reduziertem Druck entfernt. Der Rückstand wurde aus Wasser/Acetonitril lyophilisiert und 42.8 mg (35% d. Th., 99% Reinheit) der Titelverbindung erhalten.
$^1$H-NMR (400 MHz, DMSO-$d_6$): δ [ppm] = 10.34 (s, 1H), 8.82 (s, 1H), 8.72 (d, 1H), 8.55-8.50 (m, 1H), 7.92-7.83 (m, 1H),

7.67-7.59 (m, 1H), 7.41-7.33 (m, 1H), 5.32 (dd, 1H), 4.31-4.25 (m, 1H), 3.94-3.85 (m, 2H), 3.72-3.62 (m, 1H), 3.58-3.42 (m, 3H), 3.00-2.89 (m, 1H), 2.45-2.31 (m, 2H), 1.95-1.83 (m, 2H).
LC-MS (Methode 4): $R_t$ = 2.83 min; 553 $[M+H]^+$.

**Beispiel 238:**

1-(2,4-Difluorphenyl)-7-[(4*S*)-4-hydroxy-2-oxopyrrolidin-1-yl]-4-oxo-*N*-[3-(trifluormethyl)tetrahydrofuran-3-yl]-1,4-dihydro-1,8-naphthyridin-3-carbonsäureamid (Diastereomerengemisch)

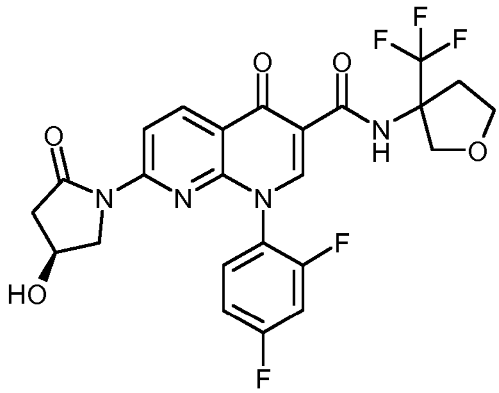

Gemäß AAV1 wurden 82 mg (0.13 mmol, 65% Reinheit) der Verbindung aus Beispiel 63A mit 30.9 mg (199 µmol) *rac*-3-(Trifluormethyl)tetrahydrofuran-3-amin in Gegenwart von 50.5 mg (133 µmol) HATU und 32.0 µl (186 µmol) *N*,*N*-Diisopropylethylamin in 1.3 ml Dimethylformamid umgesetzt. Das Rohprodukt wurde mittels Flashchromatographie (Cyclohexan/Essigsäureethylester-Gradient) gereinigt und 39.8 mg (56% d. Th., 100% Reinheit) der Titelverbindung erhalten.
$^1$H-NMR (400 MHz, DMSO-$d_6$): δ [ppm] = 10.62 (s, 1H), 8.83 (s, 1H), 8.71 (d, 1H), 8.55-8.50 (m, 1H), 7.91-7.81 (m, 1H), 7.67-7.59 (m, 1H), 7.41-7.33 (m, 1H), 5.32 (dd, 1H), 4.34-4.25 (m, 2H), 4.16-4.09 (m, 1H), 4.01-3.93 (m, 1H), 3.92-3.83 (m, 1H), 3.71-3.61 (m, 1H), 3.52-3.41 (m, 1H), 3.00-2.88 (m, 1H), 2.41-2.31 (m, 1H).
LC-MS (Methode 1): $R_t$ = 0.90 min; 539 $[M+H]^+$.

**Beispiel 239:**

1-(2-Fluorphenyl)-7-[(4*S*)-4-hydroxy-2-oxopyrrolidin-1 -yl]-4-oxo-*N*-[(2*R*)-1,1,1-trifluorbutan-2-yl]-1,4-dihydro-1,8-naphthyridin-3-carbonsäureamid

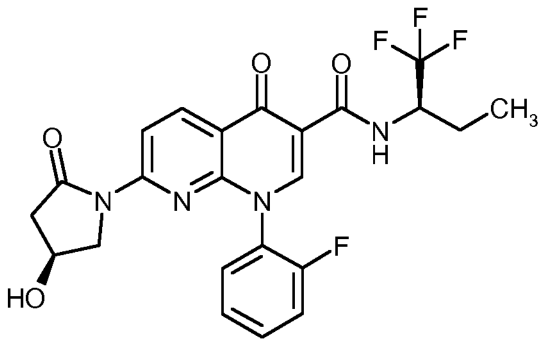

Gemäß AAV2 wurden 130 mg (304 µmol) der Verbindung aus Beispiel 82A mit 30.7 mg (304 µmol) (*S*)-(-)-4-Hydroxy-2-pyrrolidinon in Gegenwart von 149 mg (456 µmol) Caesiumcarbonat, 12 mg (55 µmol) Palladium(II)acetat und 63.3 mg (109 µmol) Xantphos in 6 ml Dioxan zur Reaktion gebracht. Das Rohprodukt wurde mit 3 ml Acetonitril und 0.5 ml Wasser verdünnt und mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 51.3 mg (35% d. Th., 99% Reinheit) der Titelverbindung erhalten.
$^1$H-NMR (400 MHz, DMSO-$d_6$): δ [ppm] = 10.23 (d, 1H), 8.82 (s, 1H), 8.72 (d, 1H), 8.55-8.49 (m, 1H), 7.82-7.76 (m, 1H), 7.72-7.65 (m, 1H), 7.57-7.43 (m, 2H), 5.32 (dd, 1H), 4.84-4.70 (m, 1H), 4.29-4.22 (m, 1H), 3.69-3.57 (m, 1H), 3.50-3.39 (m, 1H), 2.99-2.87 (m, 1H), 2.41-2.30 (m, 1H), 1.96-1.84 (m, 1H), 1.73-1.59 (m, 1H), 0.98 (t, 3H).
LC-MS (Methode 1): $R_t$ = 0.96 min; 493 [M+H]$^+$.

**Beispiel 240:**

1-(2-Chlorphenyl)-7-[(4*S*)-4-hydroxy-2-oxopyrrolidin-1-yl]-4-oxo-*N*-[(2*R*)-1,1,1-trifluorbutan-2-yl]-1,4-dihydro-1,8-naphthyridin-3-carbonsäureamid(Atropisomerengemisch)

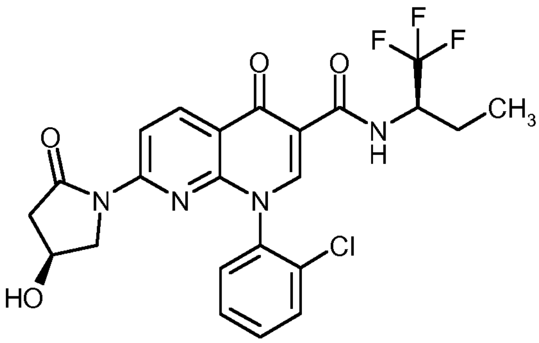

Gemäß AAV1 wurden 30 mg (75 µmol) der Verbindung aus Beispiel 64A mit 18.4 mg (113 µmol) (*R*)-1,1,1-Trifluor-2-butylamin Hydrochlorid in Gegenwart von 29 mg (75 µmol) HATU und 39µl (0.23 mmol) *N,N*-Diisopropylethylamin in 0.8 ml Dimethylformamid zur Reaktion gebracht. Es wurde mit 1 ml Acetonitril und 0.5 ml Wasser verdünnt und die Lösung mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 23 mg (60% d. Th., 99% Reinheit) der Titelverbindung erhalten (als Atropisomerengemisch).
$^1$H-NMR (400 MHz, DMSO-$d_6$): δ [ppm] = 10.25 (d, 1H), 8.75 (d, 1H), 8.72 (d, 1H), 8.51 (dd, 1H), 7.84-7.76 (m, 2H), 7.70-7.59 (m, 2H), 5.31 (dd, 1H), 4.84-4.70 (m, 1H), 4.27-4.20 (m, 1H), 3.61-3.52 (m, 1H), 3.41-3.34 (m, 1H), 2.97-2.87 (m, 1H), 2.40-2.30 (m, 1H), 1.96-1.84 (m, 1H), 1.75-1.60 (m, 1H), 1.02-0.95 (m, 3H).
LC-MS (Methode 1): $R_t$ = 1.00 min; 509 [M+H]$^+$.

**Beispiel 241:**

1-(2,4-Difluorphenyl)-*N*-[1-(2,6-difluorphenyl)-2,2,2-trifluorethyl]-7-[(3*S*)-3-fluorpyrrolidin-1-yl]-4-oxo-1,4-dihydro-1,8-naphthyridin-3-carbonsäureamid (Diastereomerengemisch)

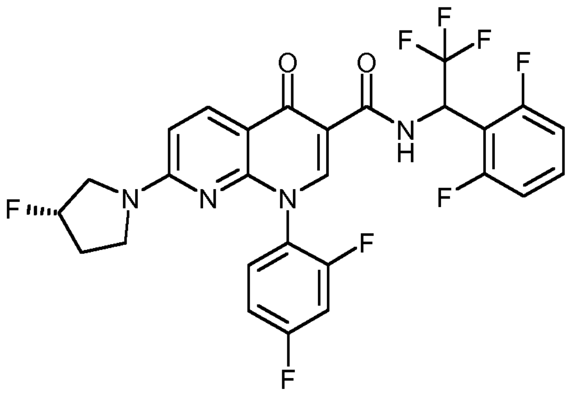

Gemäß AAV3 wurden 150 mg (283 μmol) der Verbindung aus Beispiel 80A mit 43 mg (0.28 mmol) (*S*)-3-Fluorpyrrolidin Hydrochlorid und 0.17 ml (0.99 mmol) *N,N*-Diisopropylethylamin in 1.3 ml Dimethylformamid zur Reaktion gebracht. Das Rohprodukt wurde mittels präparativer HPLC (Säule: Chromatorex C18, 10 μm, 125 x 40 mm, Lösungsmittel: Acetonitril / 0.05% Ameisensäure-Gradient; (0 bis 3 min. 10% Acetonitril bis 40 min. 90% Acetonitril und weitere 3 min. 90% Acetonitril) gereinigt und 119 mg (72% d. Th., 100% Reinheit) der Titelverbindung erhalten.
$^1$H NMR (400 MHz, DMSO-$d_6$) δ [ppm] = 11.61 (d, 1H), 8.65 (s, 1H), 8.36 (d, 1H), 7.89-7.69 (m, 1H), 7.67-7.51 (m, 2H), 7.38-7.25 (m, 3H), 6.82 (br. d, 1H), 6.49-6.38 (m, 1H), 5.57-5.23 (m, 1H), 3.86-3.44 (m, 3H), 3.25-3.01 (m, 1H), 2.32-2.08 (m, 2H).
LC-MS (Methode 1): $R_t$ = 1.31 min; 583 [M+H]$^+$.

131 mg der Titelverbindung (Diastereomerengemisch) wurde durch chirale HPLC in die Diastereomere getrennt (präparative HPLC: Säule Daicel Chiralpak IC 5 μm 250x20 mm; Eluent: 50% iso-Hexan, 50% Ethanol + 0.2% Diethylamin; Temperatur: 40°C; Fluss: 15 ml/min; UV-Detektion: 220 nm.)

Man erhielt (in der Reihenfolge der Elution von der Säule) 57 mg Diastereomer 1 (99% de) $R_t$ = 4.13 min und 58 mg (99%de) Diastereomer 2 $R_t$ = 5.55 min.

[Analytische HPLC: Säule Daicel Chiralpak IC 5 μm 250x4.6 mm; Eluent: 50% iso-Hexan, 50% Ethanol + 0.2% Diethylamin; Temperatur: 40°C; Fluss: 1 ml/min; UV-Detektion: 235 nm.]

## Beispiel 242:

1-(2,4-Difluorphenyl)-*N*-[1-(2,6-difluorphenyl)-2,2,2-trifluorethyl]-7-[(3*S*)-3-fluorpyrrolidin-1-yl]-4-oxo-1,4-dihydro-1,8-naphthyridin-3-carbonsäureamid (Diastereomer 1)

$^1$H NMR (400 MHz, DMSO-$d_6$) δ [ppm] = 11.61 (d, 1H), 8.65 (s, 1H), 8.36 (d, 1H), 7.89-7.69 (m, 1H), 7.68-7.51 (m, 2H), 7.37-7.23 (m, 3H), 6.88-6.73 (m, 1H), 6.50-6.34 (m, 1H), 5.57-5.21 (m, 1H), 3.87-3.34 (m, 3H), 3.22-3.01 (m, 1H), 2.34-1.94 (m, 2H).
LC-MS (Methode 3): $R_t$ = 2.36 min; 583 [M+H]$^+$.

## Beispiel 243:

1-(2,4-Difluorphenyl)-*N*-[1-(2,6-difluorphenyl)-2,2,2-trifluorethyl]-7-[(3*S*)-3-fluorpyrrolidin-1-yl]-4-oxo-1,4-dihydro-1,8-naphthyridin-3-carbonsäureamid (Diastereomer 2)

$^1$H NMR (400 MHz, DMSO-$d_6$) δ [ppm] = 11.61 (d, 1H), 8.65 (s, 1H), 8.36 (d, 1H), 7.90-7.69 (m, 1H), 7.67-7.49 (m, 2H), 7.38-7.26 (m, 3H), 6.82 (br. d, 1H), 6.49-6.37 (m, 1H), 5.58-5.21 (m, 1H), 3.86-3.34 (m, 3H), 3.24-3.00(m, 1H), 2.34-1.97 (m, 2H).
LC-MS (Methode 3): $R_t$ = 2.34 min; 583 [M+H]$^+$.

## Beispiel 244:

1-(2,4-Difluorphenyl)-*N*-[1-(2,6-difluorphenyl)-2,2,2-trifluorethyl]-7-[(4*S*)-4-hydroxy-2-oxopyrrolidin-1-yl]-4-oxo-1,4-dihydro-1,8-naphthyridin-3-carbonsäureamid(Diastereomerengemisch)

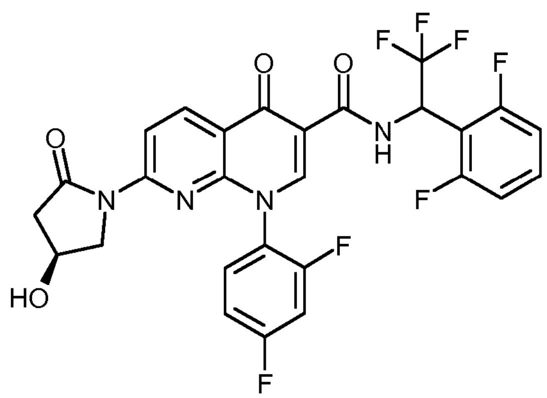

Gemäß AAV2 wurden 200 mg (377 μmol) der Verbindung aus Beispiel 80A mit 45.8 mg (453 μmol) (4*S*)-4-Hydroxypyrrolidin-2-on in Gegenwart von 78.3 mg (566 μmol) Kaliumcarbonat, 17 mg (75 μmol) Palladium(II)acetat und 44 mg (75 μmol) Xantphos in 3.4 ml Dioxan zur Reaktion gebracht. Das Rohprodukt wurde mittels präparativer HPLC (Säule: Chromatorex C18, 10 μm, 125 x 40 mm, Lösungsmittel: Acetonitril / 0.1% Ameisensäure-Gradient; (0 bis 3 min. 10% Acetonitril bis 40 min. 90% Acetonitril und weitere 3 min. 90% Acetonitril) gereinigt und 168 mg (75% d. Th., 100% Reinheit) der Titelverbindung erhalten.
$^1$H NMR (400 MHz, DMSO-$d_6$) δ [ppm] = 11.37 (d, 1H), 8.87 (s, 1H), 8.75 (d, 1H), 8.52 (dd, 1H), 7.94-7.75 (m, 1H), 7.68-7.57 (m, 2H), 7.42-7.26 (m, 3H), 6.50-6.39 (m, 1H), 5.37-5.26 (m, 1H), 4.28 (br. d, 1H), 3.73-3.60 (m, 1H), 3.53-3.40 (m, 1H), 3.01-2.87 (m, 1H), 2.42-2.31 (m, 1H).
LC-MS (Methode 3): $R_t$ = 2.01 min; 595 [M+H]$^+$.

133 mg der Titelverbindung (Diastereomerengemisch) wurde durch chirale HPLC in die Diastereomere getrennt (präparative HPLC: Säule Daicel Chiralpak ID 5 μm 250x20 mm; Eluent: 80% iso-Hexan, 20% Ethanol; Temperatur: 23°C; Fluss: 30 ml/min; UV-Detektion: 220 nm.)

Man erhielt (in der Reihenfolge der Elution von der Säule) 53.9 mg Diastereomer 1 (99% de) $R_t$ = 2.12 min und 52.2 mg (99%de) Diastereomer 2 $R_t$ = 3.03 min.

[Analytische HPLC: Säule Daicel Chiralpak ID-3 3 μm 50x4.6 mm; Eluent: 80% iso-Hexan, 20% Ethanol; Temperatur: 30°C; Fluss: 1 ml/min; UV-Detektion: 220 nm.]

**Beispiel 245:**

1-(2,4-Difluorphenyl)-*N*-[1-(2,6-difluorphenyl)-2,2,2-trifluorethyl]-7-[(4*S*)-4-hydroxy-2-oxopyrrolidin-1-yl]-4-oxo-1,4-dihydro-1,8-naphthyridin-3-carbonsäureamid (Diastereomer 1)

$^1$H NMR (400 MHz, DMSO-$d_6$) δ [ppm] = 11.37 (d, 1H), 8.88 (s, 1H), 8.75 (d, 1H), 8.52 (dd, 1H), 7.92-7.76 (m, 1H), 7.68-7.57 (m, 2H), 7.41-7.25 (m, 3H), 6.50-6.39 (m, 1H), 5.32 (dd, 1H), 4.28 (br. d, 1H), 3.72-3.60 (m, 1H), 3.46 (t, 1H), 3.00-2.87 (m, 1H), 2.37 (dd, 1H).
LC-MS (Methode 3): $R_t$ = 2.01 min; 595 [M+H]$^+$.

**Beispiel 246:**

1-(2,4-Difluorphenyl)-*N*-[1-(2,6-difluorphenyl)-2,2,2-trifluorethyl]-7-[(4*S*)-4-hydroxy-2-oxopyrrolidin-1-yl]-4-oxo-1,4-dihydro-1,8-naphthyridin-3-carbonsäureamid (Diastereomer2)

$^1$H NMR (400 MHz, DMSO-$d_6$) δ [ppm] = 11.37 (dd, 1H), 8.87 (s, 1H), 8.75 (d, 1H), 8.52 (dd, 1H), 7.95-7.75 (m, 1H), 7.68-7.57 (m, 2H), 7.41-7.26 (m, 3H), 6.50-6.38 (m, 1H), 5.32 (dd, 1H), 4.31-4.25 (br. d, 1H), 3.71-3.60 (m, 1H), 3.52-3.41 (m, 1H), 3.00-2.88 (m, 1H), 2.37 (dd, 1H).
LC-MS (Methode 3): $R_t$ = 2.00 min; 595 [M+H]$^+$.

**Beispiel 247:**

1-(2,4-Difluorphenyl)-*N*-[1-(2,6-difluorphenyl)-2,2,2-trifluorethyl]-7-[3-hydroxy-2-oxopiperidin-1-yl]-4-oxo-1,4-dihydro-1,8-naphthyridin-3-carbonsäureamid (Diastereomerengemisch)

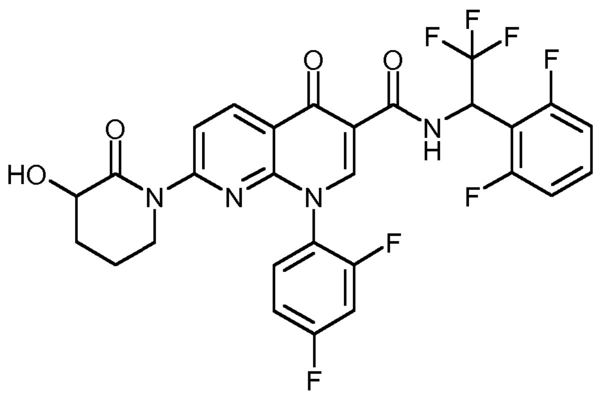

Gemäß AAV2 wurden 150 mg (283 μmol) der Verbindung aus Beispiel 80A mit 39.1 mg (340 μmol) *rac*-3-Hydroxypiperidin-2-on in Gegenwart von 58.7 mg (425 μmol) Kaliumcarbonat, 13 mg (57 μmol) Palladium(II)acetat und 33 mg (57 μmol) Xantphos in 2.5 ml Dioxan zur Reaktion gebracht. Das Rohprodukt wurde mittels präparativer HPLC (Säule: Chromatorex C18, 10 μm, 125 x 40 mm, Lösungsmittel: Acetonitril / 0.1% Ameisensäure-Gradient; (0 bis 3 min. 10% Acetonitril bis 40 min. 90% Acetonitril und weitere 3 min. 90% Acetonitril) gereinigt und 102 mg (59% d. Th., 100% Reinheit) der Titelverbindung erhalten.
$^1$H NMR (400 MHz, DMSO-$d_6$) δ [ppm] = 11.34 (br. d, 1H), 8.89 (s, 1H), 8.70 (d, 1H), 8.17-8.09 (m, 1H), 7.94-7.75 (m, 1H), 7.68-7.56 (m, 2H), 7.41-7.27 (m, 3H), 6.51-6.39 (m, 1H), 5.52-5.46 (m, 1H), 4.27-4.17 (m, 1H), 3.71-3.58 (m, 1H), 3.54-3.40 (m, 1H), 2.12-2.01 (m, 1H), 1.86-1.71 (m, 1H), 1.71-1.57 (m, 1H).
LC-MS (Methode 3): $R_t$ = 2.11 min; 609 $[M+H]^+$.

91.2 mg der Titelverbindung (racemisches Diastereomerengemisch) wurden durch chirale HPLC in die enantiomeren Diastereomere getrennt (präparative HPLC: Säule Daicel Chiralpak AZ-H 5 μm 250x30 mm; Eluent: 50% iso-Hexan, 50% Ethanol; Temperatur: 25°C; Fluss: 50 ml/min; UV-Detektion: 220 nm).

Man erhielt (in der Reihenfolge der Elution von der Säule) 17.8 mg Diastereomer 1 (96.5% de) $R_t$ = 3.19 min, 14.5 mg (95%de) Diastereomer 2 $R_t$ = 4.21 min, 17.4 mg (97%de) Diastereomer 3 $R_t$ = 6.11 min und 14.5 mg (97%de) Diastereomer 4 $R_t$ = 10.80 min.

[Analytische HPLC:Säule : Daicel AZ-3 3 μm 50x4.6 mm; Eluent: 50% iso-Hexan, 50% Ethanol; Temperatur: RT; Fluss: 1 ml/min; UV-Detektion: 220 nm]

## Beispiel 248:

1-(2,4-Difluorphenyl)-*N*-[1-(2,6-difluorphenyl)-2,2,2-trifluorethyl]-7-[3-hydroxy-2-oxopiperidin-1-yl]-4-oxo-1,4-dihydro-1,8-naphthyridin-3-carbonsäureamid (Diastereomer 1)

$^1$H NMR (400 MHz, DMSO-$d_6$) δ [ppm] = 11.34 (br. d, 1H), 8.89 (s, 1H), 8.70 (d, 1H), 8.17-8.10 (m, 1H), 7.93-7.76 (m, 1H), 7.68-7.57 (m, 2H), 7.40-7.27 (m, 3H), 6.51-6.39 (m, 1H), 5.52-5.46 (m, 1H), 4.27-4.18 (m, 1H), 3.71-3.59 (m, 1H), 3.54-3.41 (m, 1H), 2.12-2.01 (m, 1H), 1.83-1.73 (m, 2H), 1.71-1.58 (m, 1H).
LC-MS (Methode 3): $R_t$ = 2.12 min; 609 $[M+H]^+$.

## Beispiel 249:

1-(2,4-Difluorphenyl)-*N*-[1-(2,6-difluorphenyl)-2,2,2-trifluorethyl]-7-[3-hydroxy-2-oxopiperidin-1-yl]-4-oxo-1,4-dihydro-1,8-naphthyridin-3-carbonsäureamid (Diastereomer 2)

$^1$H NMR (400 MHz, DMSO-$d_6$) δ [ppm] = 11.37-11.31 (m, 1H), 8.89 (s, 1H), 8.70 (d, 1H), 8.13 (dd, 1H), 7.94-7.75 (m, 1H), 7.68-7.56 (m, 2H), 7.41-7.26 (m, 3H), 6.51-6.38 (m, 1H), 5.52-5.46 (m, 1H), 4.27-4.16 (m, 1H), 3.72-3.58 (m, 1H), 3.53-3.39 (m, 1H), 2.12-2.01 (m, 1H), 1.83-1.73 (m, 2H), 1.72-1.57 (m, 1H).
LC-MS (Methode 3): $R_t$ = 2.13 min; 609 $[M+H]^+$.

## Beispiel 250:

1-(2,4-Difluorphenyl)-*N*-[1-(2,6-difluorphenyl)-2,2,2-trifluorethyl]-7-[3-hydroxy-2-oxopiperidin-1-yl]-4-oxo-1,4-dihydro-1,8-naphthyridin-3-carbonsäureamid (3. Diastereomer)

$^1$H NMR (400 MHz, DMSO-$d_6$) δ [ppm] = 11.37-11.31 (m, 1H), 8.89 (s, 1H), 8.70 (d, 1H), 8.13 (dd, 1H), 7.94-7.75 (m, 1H), 7.68-7.56 (m, 2H), 7.40-7.26 (m, 3H), 6.51-6.39 (m, 1H), 5.52-5.46 (m, 1H), 4.26-4.17 (m, 1H), 3.72-3.59 (m, 1H), 3.52-3.40 (m, 1H), 2.12-2.01 (m, 1H), 1.84-1.73 (m, 2H), 1.72-1.58 (m, 1H).
LC-MS (Methode 3): $R_t$ = 2.12 min; 609 $[M+H]^+$.

## Beispiel 251:

1-(2,4-Difluorphenyl)-*N*-[1-(2,6-difluorphenyl)-2,2,2-trifluorethyl]-7-[3-hydroxy-2-oxopiperidin-1-yl]-4-oxo-1,4-dihydro-1,8-naphthyridin-3-carbonsäureamid (4. Diastereomer)

$^1$H NMR(400 MHz, DMSO-$d_6$) δ [ppm] = 11.34 (br. d, 1H), 8.89 (s, 1H), 8.70 (d, 1H), 8.17-8.09 (m, 1H), 7.93-7.76 (m, 1H), 7.68-7.57 (m, 2H), 7.40-7.28 (m, 3H), 6.51-6.39 (m, 1H), 5.52-5.46 (m, 1H), 4.27-4.18 (m, 1H), 3.70-3.59 (m, 1H), 3.54-3.41 (m, 1H), 2.12-2.02 (m, 1H), 1.83-1.73 (m, 2H), 1.70-1.58 (m, 1H).
LC-MS (Methode 3): $R_t$ = 2.13 min; 609 $[M+H]^+$.

**Beispiel 252:**

*rac*-1-(2,4-Difluorphenyl)-*N*-[1-(2,6-difluorphenyl)-2,2,2-trifluorethyl]-7-[4-hydroxy-2-oxopiperidin-1-yl]-4-oxo-1,4-dihydro-1,8-naphthyridin-3-carbonsäureamid (Diastereomerengemisch)

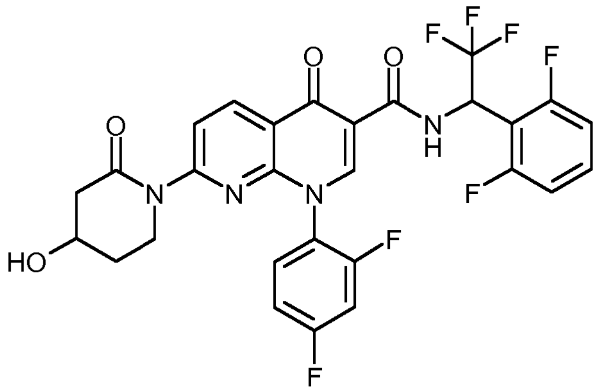

Gemäß AAV2 wurden 150 mg (283 µmol) der Verbindung aus Beispiel 80A mit 39.1 mg (340 µmol) *rac*-4-Hydroxypiperidin-2-on in Gegenwart von 58.7 mg (425 µmol) Kaliumcarbonat, 13 mg (57 µmol) Palladium(II)acetat und 33 mg (57 µmol) Xantphos in 2.5 ml Dioxan zur Reaktion gebracht. Das Rohprodukt wurde mittels präparativer HPLC (Säule: Chromatorex C18, 10 µm, 125 x 40 mm, Lösungsmittel: Acetonitril / 0.1% Ameisensäure-Gradient; (0 bis 3 min. 10% Acetonitril bis 40 min. 90% Acetonitril und weitere 3 min. 90% Acetonitril) gereinigt und 113 mg (65% d. Th., 100% Reinheit) der Titelverbindung erhalten.

$^1$H NMR (400 MHz, DMSO-$d_6$) δ [ppm] = 11.38-11.31 (m, 1H), 8.89 (s, 1H), 8.67 (d, 1H), 8.15-8.08 (m, 1H), 7.94-7.76 (m, 1H), 7.68-7.56 (m, 2H), 7.41-7.24 (m, 3H), 6.51-6.38 (m, 1H), 5.10-5.01 (m, 1H), 4.11-4.01 (m, 1H), 3.69-3.57 (m, 1H), 3.50-3.38 (m, 1H), 2.84-2.72 (m, 1H), 2.46-2.37 (m, 1H), 1.99-1.87 (m, 1H), 1.78-1.66 (m, 1H).

LC-MS (Methode 3): $R_t$ = 2.12 min; 609 [M+H]$^+$.

100 mg der Titelverbindung (racemisches Diastereomerengemisch) wurde durch chirale HPLC in die enantiomeren Diastereomere getrennt (präparative HPLC: Säule Daicel Chiralpak ID 5 µm 250x20 mm; Eluent: 50% iso-Hexan, 50% Ethanol; Temperatur: 40°C; Fluss: 15 ml/min; UV-Detektion: 220 nm).

Man erhielt (in der Reihenfolge der Elution von der Säule) 22 mg Diastereomer 1 (99% de) $R_t$ = 8.70 min, 24 mg (99%de) Diastereomer 2 $R_t$ = 11.80 min, 24 mg (99.5%de) Diastereomer 3 $R_t$ = 6.47 min und 24 mg (99.5%de) Diastereomer 4 $R_t$ = 5.94 min.

[Analytische HPLC:Säule : Daicel Chiralcel OZ-H 5 µm 250x4.6 mm; Eluent: 50% iso-Hexan, 50% Ethanol; Temperatur: 40°C; Fluss: 1 ml/min; UV-Detektion: 220 nm]

**Beispiel 253:**

1-(2,4-Difluorphenyl)-*N*-[1-(2,6-difluorphenyl)-2,2,2-trifluorethyl]-7-[4-hydroxy-2-oxopiperidin-1-yl]-4-oxo-1,4-dihydro-1,8-naphthyridin-3-carbonsäureamid (Diastereomer 1)

$^1$H NMR (400 MHz, DMSO-$d_6$) δ [ppm] = 11.39-11.31 (m, 1H), 8.89 (s, 1H), 8.67 (d, 1H), 8.15-8.09 (m, 1H), 7.94-7.76 (m, 1H), 7.68-7.57 (m, 2H), 7.41-7.27 (m, 3H), 6.51-6.39 (m, 1H), 5.10-5.04 (m, 1H), 4.12-4.01 (m, 1H), 3.69-3.59 (m, 1H), 3.48-3.38 (m, 1H), 2.83-2.72 (m, 1H), 2.47-2.37 (m, 1H), 1.99-1.88 (m, 1H), 1.79-1.67 (m, 1H).

LC-MS (Methode 3): $R_t$ = 1.99 min; 609 [M+H]$^+$.

**Beispiel 254:**

1-(2,4-Difluorphenyl)-*N*-[1-(2,6-difluorphenyl)-2,2,2-trifluorethyl]-7-[4-hydroxy-2-oxopiperidin-1-yl]-4-oxo-1,4-dihydro-1,8-naphthyridin-3-carbonsäureamid (Diastereomer 2)

$^1$H NMR(400 MHz, DMSO-$d_6$) δ [ppm] = 11.34 (br. d, 1H), 8.89 (s, 1H), 8.67 (d, 1H), 8.15-8.09 (m, 1H), 7.93-7.76 (m, 1H), 7.68-7.57 (m, 2H), 7.40-7.26 (m, 3H), 6.50-6.39 (m, 1H), 5.10-5.02 (m, 1H), 4.12-4.01 (m, 1H), 3.68-3.57 (m, 1H), 3.50-3.39 (m, 1H), 2.84-2.72 (m, 1H), 2.47-2.37- (m, 1H), 2.00-1.88 (m, 1H), 1.78-1.66 (m, 1H).

LC-MS (Methode 3): $R_t$ = 2.00 min; 609 $[M+H]^+$.

**Beispiel 255:**

1-(2,4-Difluorphenyl)-*N*-[1-(2,6-difluorphenyl)-2,2,2-trifluorethyl]-7-[4-hydroxy-2-oxopiperidin-1-yl]-4-oxo-1,4-dihydro-1,8-naphthyridin-3-carbonsäureamid (3. Diastereomer)

$^1H$ NMR (400 MHz, DMSO-$d_6$) δ [ppm] = 11.38-11.32 (m, 1H), 8.89 (s, 1H), 8.67 (d, 1H), 8.16-8.07 (m, 1H), 7.95-7.75 (m, 1H), 7.68-7.57 (m, 2H), 7.41-7.26 (m, 3H), 6.51-6.39 (m, 1H), 5.11-5.02 (m, 1H), 4.11-4.01 (m, 1H), 3.69-3.58 (m, 1H), 3.48-3.38 (m, 1H), 2.84-2.72 (m, 1H), 2.48-2.37 (m, 1H), 2.00-1.88 (m, 1H), 1.79-1.67 (m, 1H).
LC-MS (Methode 3): $R_t$ = 1.99 min; 609 $[M+H]^+$.

**Beispiel 256:**

1-(2,4-Difluorphenyl)-*N*-[1-(2,6-difluorphenyl)-2,2,2-trifluorethyl]-7-[4-hydroxy-2-oxopiperidin-1-yl]-4-oxo-1,4-dihydro-1,8-naphthyridin-3-carbonsäureamid (4. Diastereomer)

$^1H$ NMR (400 MHz, DMSO-$d_6$) δ [ppm] = 11.35 (d, 1H), 8.89 (s, 1H), 8.67 (d, 1H), 8.15-8.09 (m, 1H), 7.93-7.77 (m, 1H), 7.68-7.58 (m, 2H), 7.40-7.28 (m, 3H), 6.50-6.39 (m, 1H), 5.10-5.04 (m, 1H), 4.11-4.02 (m, 1H), 3.68-3.58 (m, 1H), 3.50-3.39 (m, 1H), 2.83-2.73 (m, 1H), 2.47-2.37 (m, 1H), 1.99-1.88 (m, 1H), 1.78-1.67 (m, 1H).
LC-MS (Methode 3): $R_t$ = 2.00 min; 609 $[M+H]^+$.

**Beispiel 257:**

(5S)-3-[8-(2,4-difluorphenyl)-5-oxo-6- {[(2R)-1,1,1-trifluorbutan-2-yl]carbamoyl}-5,8-dihydro-1,8-naphthyridin-2-yl]-2-oxo-1,3-oxazolidin-5-carbonsäuremethylester

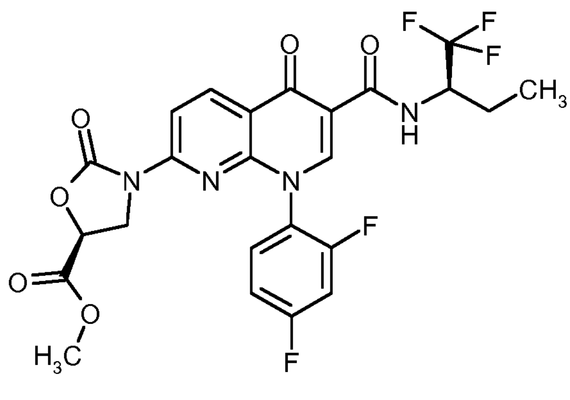

Gemäß AAV2 wurden 100 mg (224 μmol) der Verbindung aus Beispiel 67A mit 39.1 mg (269 μmol) der Verbindung aus Beispiel 96 in Gegenwart von 46.5 mg (269 μmol) Kaliumcarbonat, 10 mg (45 μmol) Palladium(II)acetat und 26 mg (45 μmol) Xantphos in 2 ml 1,4-Dioxan zur Reaktion gebracht. Das Rohprodukt wurde mit Acetonitril und Wasser verdünnt und mittels präparativer HPLC (Säule: Chromatorex C18, 10 μm, 125 x 40 mm, Lösungsmittel: Acetonitril / 0.05% Ameisensäure-Gradient; (0 bis 3 min. 10% Acetonitril bis 40 min. 90% Acetonitril und weitere 3 min. 90% Acetonitril) gereinigt und 101 mg (81% d. Th., 100% Reinheit) der Titelverbindung erhalten.
$^1H$ NMR (400 MHz, DMSO-$d_6$): δ [ppm] = 0.98 (t, 3H), 1.60-1.72 (m, 1H), 1.84-1.95 (m, 1H), 3.73 (d, 3H), 3.77-3.89 (m, 1H), 3.96-4.08 (m, 1H), 4.70-4.83 (m, 1H), 5.23-5.32 (m, 1H), 7.37 (td, 1H), 7.63 (br. t, 1H), 7.82-7.93 (m, 1H), 8.26 (dd, 1H), 8.75 (d, 1H), 8.86 (d, 1H), 10.18 (d, 1H).
LC-MS (Methode 2): $R_t$ = 2.07 min; 555 $[M+H]^+$.

**Beispiel 258:**

(5*S*)-3-[8-(2,4-difluorphenyl)-5-oxo-6-{[(2*S*)-1,1,1-trifluorbutan-2-yl]carbamoyl}-5,8-dihydro-1,8-naphthyridin-2-yl]-2-oxo-1,3-oxazolidin-5-carbonsäuremethylester

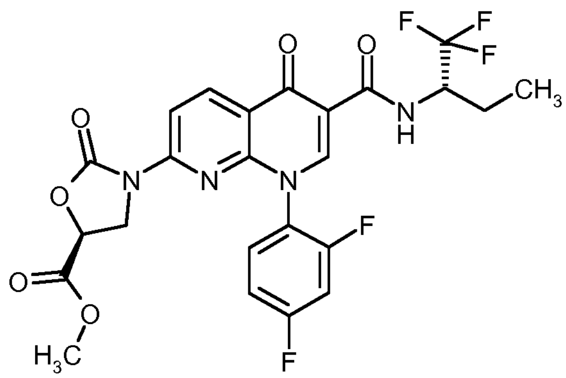

Gemäß AAV2 wurden 500 mg (1.12 mmol, 94% Reinheit) der Verbindung aus Beispiel 68A mit 195 mg (1.35 mmol) der Verbindung aus Beispiel 96 in Gegenwart von 233 mg (1.68 mmol) Kaliumcarbonat, 50.4 mg (224 μmol) Palladium(II)acetat und 130 mg (224 μmol) Xantphos in 10 ml 1,4-Dioxan zur Reaktion gebracht. Das Rohprodukt wurde mit Acetonitril und Wasser verdünnt und mittels präparativer HPLC (Säule: Chromatorex C18, 10 μm, 125 x 40 mm, Lösungsmittel: Acetonitril / 0.05% Ameisensäure-Gradient; (0 bis 3 min. 10% Acetonitril bis 40 min. 90% Acetonitril und weitere 3 min. 90% Acetonitril) gereinigt und 504 mg (81% d. Th., 100% Reinheit) der Titelverbindung erhalten.
$^1$H NMR (400 MHz, DMSO-$d_6$): δ [ppm] = 0.98 (t, 3H), 1.60-1.74 (m, 1H), 1.83-1.97 (m, 1H), 3.73 (d, 3H), 3.77-3.89 (m, 1H), 3.96-4.08 (m, 1H), 4.70-4.85 (m, 1H), 5.22-5.31 (m, 1H), 7.32-7.41 (m, 1H), 7.63 (td, 1H), 7.88 (td, 1H), 8.26 (t, 1H), 8.75 (d, 1H), 8.86 (s, 1H), 10.18 (d, 1H).
LC-MS (Methode 1): $R_t$ = 1.13 min; 555 $[M+H]^+$.

**Beispiel 259:**

(5*R*)-3-[8-(2,4-difluorphenyl)-5-oxo-6-{[(2*S*)-1,1,1-trifluorbutan-2-yl]carbamoyl}-5,8-dihydro-1,8-naphthyridin-2-yl]-2-oxo-1,3-oxazolidin-5-carbonsäuremethylester

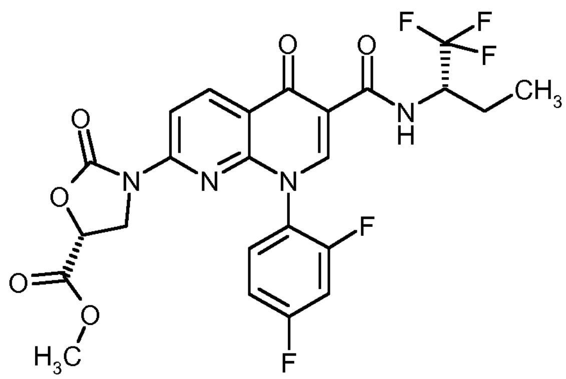

Gemäß AAV2 wurden 500 mg (1.12 mmol, 94% Reinheit) der Verbindung aus Beispiel 68A mit 195 mg (1.35 mmol) der Verbindung aus Beispiel 99 in Gegenwart von 233 mg (1.68 mmol) Kaliumcarbonat, 50.4 mg (224 μmol) Palladium(II)acetat und 130 mg (224 μmol) Xantphos in 10 ml 1,4-Dioxan zur Reaktion gebracht. Das Rohprodukt wurde mit Acetonitril und Wasser verdünnt und mittels präparativer HPLC (Säule: Chromatorex C18, 10 μm, 125 x 40 mm, Lösungsmittel: Acetonitril / 0.05% Ameisensäure-Gradient; (0 bis 3 min. 10% Acetonitril bis 40 min. 90% Acetonitril und weitere 3 min. 90% Acetonitril) gereinigt und 497 mg (80% d. Th., 100% Reinheit) der Titelverbindung erhalten.
$^1$H NMR (400 MHz, DMSO-$d_6$): δ [ppm] = 0.98 (t, 3H), 1.59-1.74 (m, 1H), 1.83-1.96 (m, 1H), 3.73 (d, 3H), 3.77-3.89 (m,

1H), 3.96-4.09 (m, 1H), 4.70-4.84 (m, 1H), 5.23-5.32 (m, 1H), 7.32-7.41 (m, 1H), 7.57-7.68 (m, 1H), 7.82-7.94 (m, 1H), 8.26 (dd, 1H), 8.75 (d, 1H), 10.18 (d, 1H), 8.86 (d, 1H).
LC-MS (Methode 1): $R_t$ = 1.14 min; 555 $[M+H]^+$.

**Beispiel 260:**

*rac*-1-(2,6-Difluorphenyl)-7-[5-(hydroxymethyl)-2-oxo-1,3-oxazolidin-3-yl]-4-oxo-*N*-[(2*R*)-1,1,1-trifluorbutan-2-yl]-1,4-dihydro-1,8-naphthyridin-3-carbonsäureamid

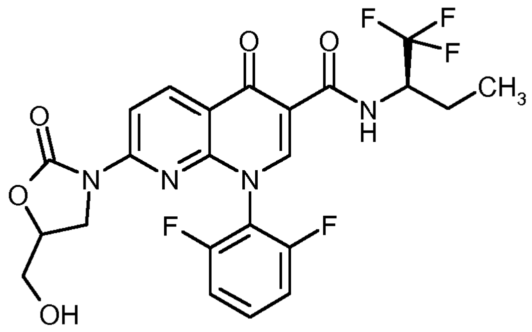

Gemäß AAV2 wurden 40 mg (89.7 µmol, 99% Reinheit) der Verbindung aus Beispiel 86A mit 12.6 mg (108 µmol) *rac*-5-(Hydroxymethyl)-1,3-oxazolidin-2-on in Gegenwart von 18.6 mg (135 µmol) Kaliumcarbonat, 4.0 mg (18 µmol) Palladium(II)acetat und 10 mg (18 µmol) Xantphos in 1 ml 1,4-Dioxan zur Reaktion gebracht. Das Reaktionsgemisch wurde mit Acetonitril und Wasser verdünnt, filtriert und mittels präparativer HPLC (Säule: Chromatorex C18, 10 µm, 125 x 40 mm, Lösungsmittel: Acetonitril / 0.05% Ameisensäure-Gradient; (0 bis 3 min. 10% Acetonitril bis 40 min. 90% Acetonitril und weitere 3 min. 90% Acetonitril) und zusätzlich mittels Flashchromatographie (Cyclohexan/Essigsäureethylester-Gradient) gereinigt und 10 mg (21% d. Th., 100% Reinheit) der Titelverbindung erhalten.
$^1$H NMR (400 MHz, DMSO-$d_6$): δ [ppm] = 0.98 (t, 3H), 1.59-1.75 (m, 1H), 1.83-1.96 (m, 1H), 3.42-3.55 (m, 2H), 3.56-3.66 (m, 1H), 3.75 (t, 1H), 4.62-4.71 (m, 1H), 4.72-4.83 (m, 1H), 5.18 (t, 1H), 7.39-7.50 (m, 2H), 7.69-7.80 (m, 1H), 8.34 (d, 1H), 8.73 (d, 1H), 9.01 (s, 1H), 10.12 (d, 1H).
LC-MS (Methode 2): $R_t$ = 1.88 min; 527 $[M+H]^+$.

**Beispiel 261:**

1-(2,4-Difluorphenyl)-7-(3-hydroxyazetidin-1-yl)-4-oxo-*N*-[(2*R*)-1,1,1-trifluorbutan-2-yl]-1,4-dihydro-1,8-naphthyridin-3-carbonsäureamid

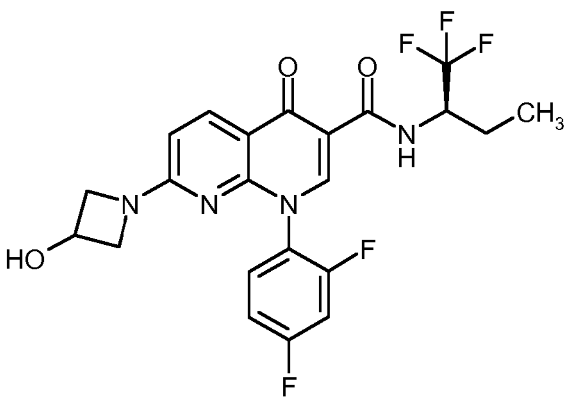

Gemäß AAV3 wurden 50.0 mg (112 µmol) der Verbindung aus Beispiel 67A mit 14.7 mg (135 µmol) 3-Hydroxyazetidin Hydrochlorid und 68 µl (0.39 mmol) *N,N*-Diisopropylamin in 0.5 ml Dimethylformamid zur Reaktion gebracht.

Die Reaktionslösung wurde mit jeweils 0.5 ml Acetonitril und Wasser verdünnt und mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 40 min. 90% Acetonitril und weitere 3 min. 90% Acetonitril) gereinigt. Es wurden 50.7 mg (93% d. Th., 99% Reinheit) der Titelverbindung erhalten.
$^1$H NMR (400 MHz, DMSO-$d_6$): δ [ppm] = 0.96 (t, 3H), 1.57-1.70 (m, 1H), 1.82-1.94 (m, 1H), 3.50-3.81 (br. m, 2H), 3.89-4.32 (br. m, 2H), 4.49-4.57 (m, 1H), 4.67-4.80 (m, 1H), 5.72 (d, 1H), 6.60 (d, 1H), 7.28-7.34 (m, 1H), 7.52-7.60 (m, 1H), 7.75-7.84 (m, 1H), 8.28 (d, 1H), 8.61 (s, 1H), 10.48 (d, 1H).
LC-MS (Methode 1): $R_t$ = 1.00 min; 483 $[M+H]^+$.

**Beispiel 262:**

1-(2,4-Difluorphenyl)-7-[3-hydroxy-3-(trifluormethyl)azetidin-1-yl]-4-oxo-*N*-[(2*R*)-1,1,1-trifluorbutan-2-yl]-1,4-dihydro-1,8-naphthyridin-3-carbonsäureamid

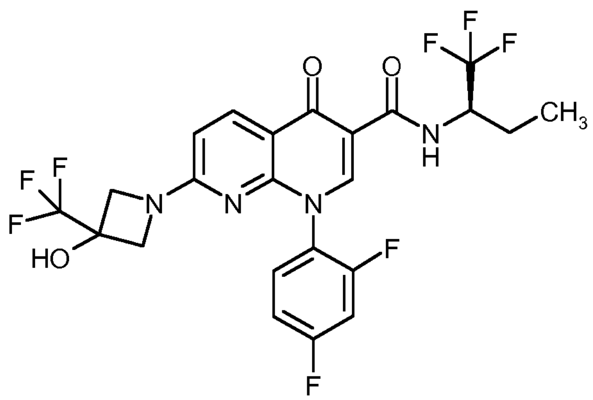

Gemäß AAV3 wurden 50.0 mg (112 μmol) der Verbindung aus Beispiel 67A mit 19.0 mg (135 μmol) 3-(Trifluormethyl)azetidin-3-ol und 68 μl (0.39 mmol) *N,N*-Diisopropylethylamin in 0.5 ml Dimethylformamid zur Reaktion gebracht. Die Reaktionslösung wurde mit 0.5 ml Acetonitril verdünnt und mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 40 min. 90% Acetonitril und weitere 3 min. 90% Acetonitril) gereinigt und 51.9 mg (83% d. Th., 99% Reinheit) der Titelverbindung erhalten.
$^1$H NMR (400 MHz, DMSO-$d_6$): δ [ppm] = 0.97 (t, 3H). 1.57-1.71 (m, 1H), 1.82-1.94 (m, 1H), 3.67-4.51 (br. m, 4H), 4.68-4.81 (m, 1H), 6.75 (d, 1H), 7.29-7.36 (m, 1H), 7.43 (s, 1H), 7.54-7.62 (m, 1H), 7.77-7.86 (m, 1H), 8.38 (d, 1H), 8.66 (s, 1H), 10.42 (d, 1H).
LC-MS (Methode 3): $R_t$ = 2.14 min; 551 $[M+H]^+$.

**Beispiel 263:**

7-[2-(Difluormethyl)morpholin-4-yl]-1-(2,4-difluorphenyl)-4-oxo-*N*-[(2*R*)-1,1,1-trifluorbutan-2-yl]-1,4-dihydro-1,8-naphthyridin-3-carbonsäureamid (Diastereomerengemisch)

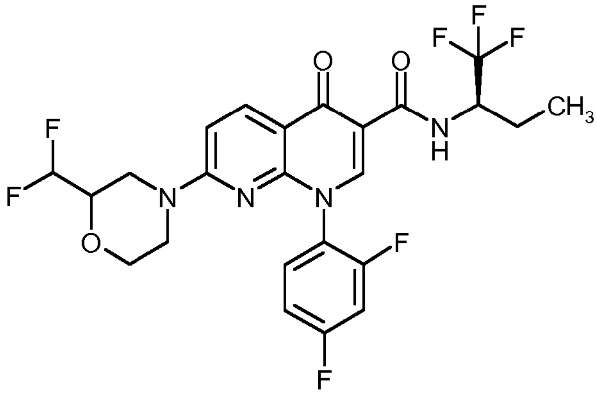

Gemäß AAV3 wurden 100 mg (224 µmol) der Verbindung aus Beispiel 67A mit 36.9 mg (269 µmol) *rac*-2-(Difluormethyl)morpholin und 137 µl (785 µmol) *N,N*-Diisopropylethylamin in 1 ml Dimethylformamid zur Reaktion gebracht. Die Reaktionslösung wurde mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 40 min. 90% Acetonitril und weitere 3 min. 90% Acetonitril) gereinigt und 103 mg (83% d. Th., 99% Reinheit) der Titelverbindung erhalten.
$^1$H NMR (400 MHz, DMSO-$d_6$): δ [ppm] = 0.97 (t, 3H), 1.57-1.71 (m, 1H), 1.83-1.94 (m, 1H), 2.83-2.96 (m, 1H), 3.05-3.16 (m, 1H), 3.48-3.58 (m, 1H), 3.63-3.74 (m, 1H), 3.91-4.04 (m, 2H), 4.07-4.16 (m, 1H), 4.70-4.79 (m, 1H), 6.02 (t, 1H), 7.16 (d, 1H), 7.27-7.35 (m, 1H), 7.48-7.70 (m, 1H), 7.77-7.86 (m, 1H), 8.36 (d, 1H), 8.66-8.69 (m, 1H), 10.43 (d, 1H).
LC-MS (Methode 3): $R_t$ = 2.19 min; 547 $[M+H]^+$.

98 mg der Titelverbindung (Diastereomerengemisch) wurde durch chirale HPLC in die Diastereomere getrennt (präparative HPLC: Säule Daicel Chiralcel OX-H 5 µm 250 x 20 mm; Eluent: 25% Ethanol, 75% Iso-Hexan; Temperatur: 40°C; Fluss: 15 ml/min; UV-Detektion: 220 nm.)

Man erhielt (in der Reihenfolge der Elution von der Säule) 46 mg Diastereomer 1 (99% de) $R_t$ = 6.27 min und 46 mg (99%de) Diastereomer 2 $R_t$ = 7.92 min.

[Analytische HPLC:Säule Chiralcel OX-3 5 µm 50 x 4.6 mm; Eluent: 30% Ethanol, 70% Iso-Hexan; Temperatur: 30°C; Fluss: 1.0 ml/min; UV-Detektion: 220 nm].

Diastereomer 1 (Beispiel 264) wurde zusätzlich mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 38.4 mg (31% d. Th., 99% Reinheit) der Titelverbindung aus Beispiel 264 erhalten.

Diastereomer 2 (Beispiel 265) wurde zusätzlich mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 40.1 mg (32% d. Th., 99% Reinheit) der Titelverbindung aus Beispiel 265 erhalten.

## Beispiel 264:

7-[2-(Difluormethyl)morpholin-4-yl]-1-(2,4-difluorphenyl)-4-oxo-*N*-[(2*R*)-1,1,1-trifluorbutan-2-yl]-1,4-dihydro-1,8-naphthyridin-3-carbonsäureamid (Diastereomer 1)

$^1$H NMR (400 MHz, DMSO-$d_6$): δ [ppm] = 0.97 (t, 3H). 1.57-1.71 (m, 1H), 1.82-1.95 (m, 1H), 2.82-2.97 (m, 1H), 3.04-3.17 (m, 1H), 3.47-3.59 (m, 1H), 3.63-3.75 (m, 1H), 3.91-4.04 (m, 2H), 4.07-4.16 (m, 1H), 4.68-4.81 (m, 1H), 6.02 (t, 1H), 7.16 (d, 1H), 7.27-7.36 (m, 1H), 7.48-7.60 (m, 1H), 7.77-7.85 (m, 1H), 8.36 (d, 1H), 8.67 (d, 1H), 10.43 (d, 1H).
LC-MS (Methode 3): Rt = 2.20 min; 547 $[M+H]^+$.

## Beispiel 265:

7-[2-(Difluormethyl)morpholin-4-yl]-1-(2,4-difluorphenyl)-4-oxo-*N*-[(2*R*)-1,1,1-trifluorbutan-2-yl]-1,4-dihydro-1,8-naphthyridin-3-carbonsäureamid (Diastereomer 2)

$^1$H NMR (400 MHz, DMSO-$d_6$): δ [ppm] = 0.97 (t, 3H), 1.57-1.71 (m, 1H), 1.81-1.95 (m, 1H), 2.82-2.96 (m, 1H), 3.04-3.16 (m, 1H), 3.46-3.58 (m, 1H), 3.62-3.75 (m, 1H), 3.89-4.04 (m, 2H), 4.07-4.16 (m, 1H), 4.67-4.81 (m, 1H), 6.02 (t, 1H), 7.16 (d, 1H), 7.26-7.36 (m, 1H), 7.47-7.60 (m, 1H), 7.77-7.86 (m, 1H), 8.36 (d, 1H), 8.67 (d, 1H), 10.43 (d, 1H).
LC-MS (Methode 3): Rt = 2.20 min; 547 $[M+H]^+$.

**Beispiel 266:**

1-(2,4-Difluorphenyl)-7-[5-methyl-2-oxo-1,3-oxazolidin-3-yl]-4-oxo-*N*-[(2*R*)-1,1,1-trifluorbutan-2-yl]-1,4-dihydro-1,8-naphthyridin-3-carbonsäureamid (Diastereomerengemisch)

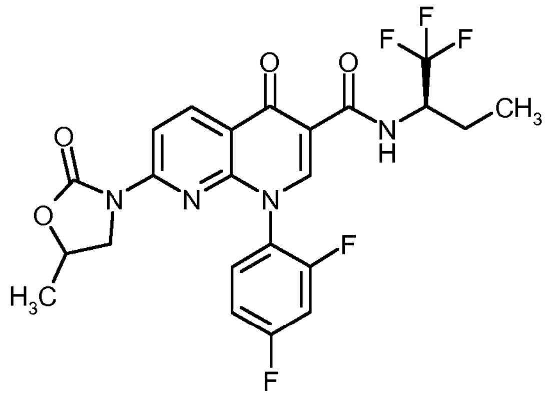

Gemäß AAV2 wurden 100 mg (222 µmol) der Verbindung aus Beispiel 67A mit 26.9 mg (266 µmol) *rac*-5-Methyl-1,3-oxazolidin-2-on in Gegenwart von 36.8 mg (266 µmol) Kaliumcarbonat, 3.5 mg (16 µmol) Palladium(II)acetat und 26 mg (44 µmol) Xantphos in 2 ml 1,4-Dioxan umgesetzt. Das Lösungsmittel wurde unter reduziertem Druck entfernt und der Rückstand in 3 ml Acetonitril und 1 ml DMSO aufgenommen. Der Niederschlag wurde abgesaugt, am Hochvakuum getrocknet und 24.5 mg (21.6% d. Th. 100% Reinheit) der Titelverbindung erhalten.
$^{1}$H-NMR (400 MHz, DMSO-d6): δ [ppm] = 0.98 (t, 3H). 1.31-1.40 (m, 3H), 1.60-1.72 (m, 1H), 1.83-1.93 (m, 1H), 3.83-3.95 (m, 1H), 4.69-4.84 (m, 2H), 7.31-7.39 (m, 1H), 7.55-7.66 (m, 1H), 7.82-7.92 (m, 1H), 8.32 (d, 1H), 8.72 (d, 1H), 8.84 (s, 1H), 10.21 (d, 1H).
LC-MS (Methode 1): $R_t$ = 1.14 min; 511 $[M+H]^+$.

**Beispiel 267:**

1-(2,4-Difluorphenyl)-7-[1-hydroxy-3-azabicyclo[3.1.0]hex-3-yl]-4-oxo-*N*-[(2*R*)-1,1,1-trifluorbutan-2-yl]-1,4-dihydro-1,8-naphthyridin-3-carbonsäureamid (Diastereomerengemisch)

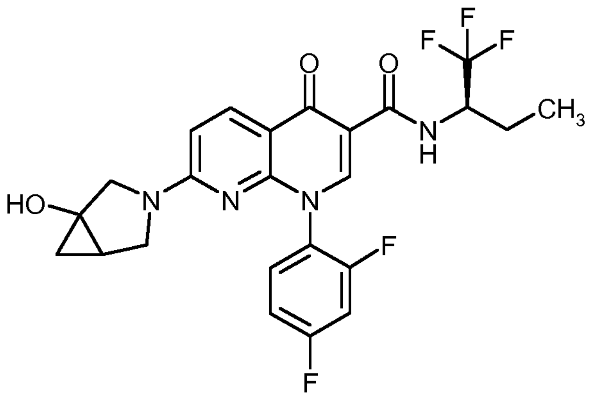

Gemäß AAV3 wurden 50.0 mg (112 µmol) der Verbindung aus Beispiel 67A mit 19.2 mg (135 µmol) *rac*-3-Azabicyclo[3.1.0]hexan-1-ol Hydrochlorid und 68.0 µl (393 µmol) *N,N*-Diisopropylamin in 0.5 ml Dimethylformamid zur Reaktion gebracht. Die Reaktionslösung wurde mit 0.5 ml Acetonitril verdünnt und mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 40 min. 90% Acetonitril und weitere 3 min. 90% Acetonitril) gereinigt und 41.4 mg (72% d. Th., 99% Reinheit) der Titelverbindung erhalten.

$^1H$ NMR (400 MHz, DMSO-$d_6$): δ [ppm] = 0.32-0.50 (m, 1H). 0.89-1.09 (m, 4H), 1.50-1.72 (m, 2H), 1.81-1.94 (m, 1H), 3.04-3.95 (m, 4H), 4.66-4.81 (m, 1H), 6.01 (d, 1H), 6.66-6.82 (m, 1H), 7.27-7.38 (m, 1H), 7.52-7.66 (m, 1H), 7.75-7.86 (m, 1H), 8.28 (d, 1H), 8.62 (s, 1H), 10.48 (d, 1H).
LC-MS (Methode 3): $R_t$ = 1.98 min; 509 $[M+H]^+$.

35 mg der Titelverbindung (Diastereomerengemisch) wurde durch chirale HPLC in die Diastereomere getrennt (präparative HPLC: Säule Daicel Chiralcel OZ-H 5 µm 250x20 mm; Eluent: 25% 2-Propanol, 75% Iso-Hexan; Temperatur: 25°C; Fluss: 15 ml/min; UV-Detektion: 210 nm.)

Man erhielt (in der Reihenfolge der Elution von der Säule) 15.4 mg Diastereomer 1 (100% de) $R_t$ = 1.34 min und 20.1 mg (97% de) Diastereomer 2 $R_t$ = 1.59 min.

[Analytische HPLC: Säule Daicel Chiralpak OZ-3 3 µm 50x4.6 mm; Eluent: 20% Ethanol, 80% Iso-Hexan; Fluß: 1 ml/min; UV-Detektion: 220 nm].

Diastereomer 1 wurde zusätzlich mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 8.8 mg (15% d. Th., 99% Reinheit) der Titelverbindung aus Beispiel 314 erhalten.

Diastereomer 2 wurde zusätzlich mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 8.4 mg (15% d. Th., 99% Reinheit) der Titelverbindung aus Beispiel 315 erhalten.

**Beispiel 268:**

7-[(4*S*)-4-Hydroxy-2-oxopyrrolidin-1-yl]-4-oxo-*N*-[(2*R*)-1,1,1-trifluorbutan-2-yl]-1-[2-(trifluormethyl)phenyl]-1,4-dihydro-1,8-naphthyridin-3-carboxamid (Atropisomerengemisch)

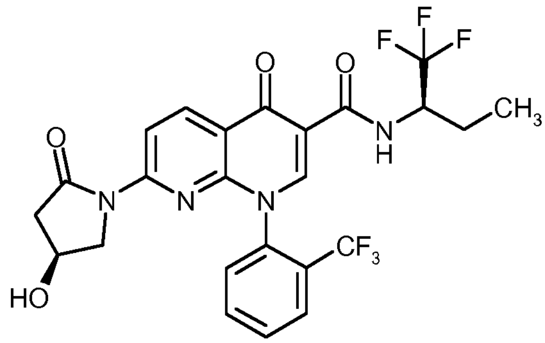

**A:** Gemäß AAV2 wurden 55.0 mg (115 µmol) der Verbindung aus Beispiel 92A mit 11.6 mg (115 µmol) (4*S*)-4-Hydroxypyrolidin-2-on in Gegenwart von 56.3 mg (173 µmol) Cäsiumcarbonat, 4.7 mg (21 µmol) Palladium(II)acetat und 24 mg (41 µmol) Xantphos in 2.3 ml 1,4-Dioxan umgesetzt. Anschließend wurde das Gemisch mit 3 ml Acetonitril und 0.5 ml Wasser verdünnt, filtriert und mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 22 mg (35% d. Th., 99% Reinheit) der Titelverbindung als Gemisch der Atropisomere erhalten.

**B**: In Analogie zu der unter **A** beschriebenen Versuchsdurchführung wurden 56 mg (117 µmol) der Verbindung aus Beispiel 93A mit 11.9 mg (117 µmol) (4*S*)-4-Hydroxypyrolidin-2-on in Gegenwart von 57.3 mg (176 µmol) Cäsiumcarbonat, 4.7 mg (21 µmol) Palladium(II)acetat und 24 mg (41 µmol) Xantphos in 2.3 ml 1,4-Dioxan umgesetzt. Anschließend wurde das Gemisch mit 3 ml Acetonitril und 0.5 ml Wasser verdünnt, filtriert und mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 27 mg (42% d. Th., 99% Reinheit) der Titelverbindung als Gemisch der Atropisomere erhalten.

**C:** Die Gemische der Atropisomere aus **A** und **B** wurden vereinigt und die vereinte Charge dann durch chirale HPLC in die Atropisomere getrennt (präparative HPLC: Säule Daicel Chiralcel OZ-H 5 µm 250x20 mm; Eluent: 30% Ethanol, 70% Iso-Hexan; Temperatur: 25°C; Fluss: 15 ml/min; UV-Detektion: 220 nm.)

Man erhielt (in der Reihenfolge der Elution von der Säule) 22 mg Atropisomer 1 (90% *de*) $R_t$ = 3.99 min und

18 mg (83% *de*) Atropisomer 2 $R_t$ = 4.79 min.

[Analytische HPLC:Säule Daicel Chiralpak AZ-H 5 μm 250x4.6 mm; Eluent: 30% Ethanol, 70% Iso-Hexan mit 0.2% Diethylamin; Temperatur: 50°C; Fluss: 1.0 ml/min; UV-Detektion: 270 nm].

Atropisomer 1 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und es wurden 16.8 mg (26.6% d. Th., 99% Reinheit) der Titelverbindung aus Beispiel 269 erhalten.

Atropisomer 2 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 20.1 mg (32% d. Th., 99% Reinheit) der Titelverbindung aus Beispiel 270 erhalten.

**Beispiel 269:**

7-[(4*S*)-4-Hydroxy-2-oxopyrrolidin-1-yl]-4-oxo-*N*-[(2*R*)-1,1,1-trifluorbutan-2-yl]-1-[2-(trifluoromethyl)phenyl]-1,4-dihydro-1,8-naphthyridin-3-carboxamid (Atropisomer 1)

$^1$H-NMR (400 MHz, DMSO-$d_6$): δ [ppm] = 0.97 (t, 3H), 1.59-1.73 (m, 1H), 1.83-1.95 (m, 1H), 2.32 (d, 1H), 2.93 (dd, 1H), 3.47 (dd, 1H), 4.17-4.23 (m, 1H), 4.70-4.82 (m, 1H), 5.27 (d, 1H), 7.91-7.84 (m, 2H), 7.95-8.00 (m, 1H), 8.02-8.07 (m, 1H), 8.51 (d, 1H), 8.71 (d, 1H), 8.82 (s, 1H), 10.24 (d, 1H).
LC-MS (Methode 3): $R_t$ = 1.88 min; 543 $[M+H]^+$.

**Beispiel 270:**

7-[(4*S*)-4-Hydroxy-2-oxopyrrolidin-1-yl]-4-oxo-*N*-[(2*R*)-1,1,1-trifluorbutan-2-yl]-1-[2-(trifluoromethyl)phenyl]-1,4-dihydro-1,8-naphthyridin-3-carboxamid (Atropisomer 2)

$^1$H-NMR (400 MHz, DMSO-$d_6$): δ [ppm] = 0.99 (t, 3H), 1.61-1.75 (m, 1H), 1.84-1.96 (m, 1H), 2.34 (d, 1H), 2.89 (dd, 1H), 3.25 (d, 1H), 3.47 (dd, 1H), 4.17-4.24 (m, 1H), 4.69-4.83 (m, 1H), 5.29 (d, 1H), 7.82-7.91 (m, 2H), 7.93-7.99 (m, 1H), 8.02-8.07 (m, 1H), 8.49 (d, 1H), 8.71 (d, 1H), 8.81 (s, 1H), 10.23 (d, 1H).
LC-MS (Methode 3): $R_t$ = 1.87 min; 543 $[M+H]^+$.

**Beispiel 271:**

*N*-[(1*R*)-1-Cyclopropyl-2,2,2-trifluorethyl]-1-(2,4-difluorphenyl)-7-[(4*S*)-4-hydroxy-2-oxopyrrolidin-1-yl]-4-oxo-1,4-dihydro-1,8-naphthyridin-3-carbonsäureamid

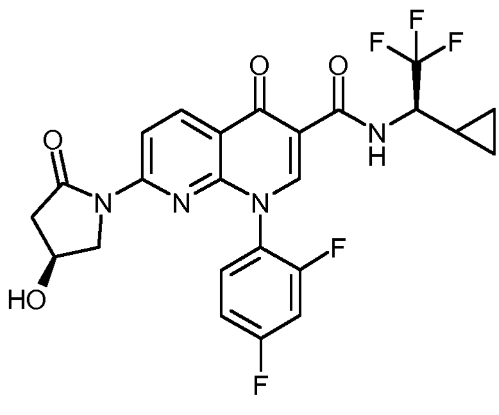

Gemäß AAV1 wurden 74.0 mg (167 μmol, 90.8% Reinheit) der Verbindung aus Beispiel 63A mit 32.3 mg (184 μmol) (*R*)-1-Cyclopropyl-2,2,2-trifluorethanamin Hydrochlorid (J. Med. Chem. 2011, 54, 7334-7349) in Gegenwart von 63.7 mg (167μmol) HATU und 70μl (0.40 mmol) *N,N*-Diisopropylethylamin in 0.9 ml Dimethylformamid zur Reaktion gebracht. Es wurde 1 ml 1M wässrige Salzsäure und 10 ml Wasser hinzugegeben und dreimal mit 10 ml Essigsäureethylester extrahiert. Die vereinigten organischen Phasen wurden über Magnesiumsulfat getrocknet, filtriert und das

Lösungsmittel unter reduziertem Druck entfernt. Der Rückstand wurde in wenig DCM aufgenommen und mittels Flashchromatographie (Cyclohexan/Essigsäureethylester-Gradient) gereinigt. Es wurden 75.3 mg (86% d. Th., 100% Reinheit) der Titelverbindung erhalten.
$^1$H-NMR (400 MHz, DMSO-$d_6$): δ [ppm] = 0.29-0.38 (m, 1H). 0.51-0.71 (m, 3H), 1.18-1.28 (m, 1H), 2.31-2.42 (m, 1H), 2.87-3.00 (m, 1H), 3.42-3.52 (m, 1H), 3.61-3.72 (m, 1H), 4.25-4.32 (m, 1H), 4.34-4.49 (m, 1H), 5.32 (dd, 1H), 7.41-7.33 (m, 1H), 7.59-7.67 (m, 1H), 7.83-7.92 (m, 1H), 8.49-8.55 (m, 1H), 8.71 (d, 1H), 8.84 (s, 1H), 10.36 (dd, 1H).
LC-MS (Methode 3): $R_t$ = 1.89 min; 523 $[M+H]^+$.

**Beispiel 272:**

1-(2,4-Difluorophenyl)-7-[(4*S*)-4-hydroxy-2-oxopyrrolidin-1-yl]-4-oxo-*N*-[2,2,2-trifluor-1-(3-fluorphenyl)ethyl]-1,4-dihydro-1,8-naphthyridin-3-carbonsäureamid (Diastereomerengemisch)

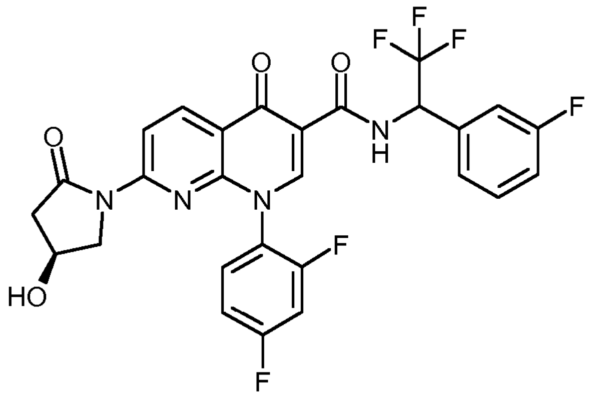

Gemäß AAV1 wurden 170 mg (352 μmol, 83% Reinheit) der Verbindung aus Beispiel 63A mit 102 mg (527 μmol) *rac*-2,2,2-Trifluor-1-(3-fluorphenyl)ethanamin in Gegenwart von 134 mg (352 μmol) HATU und 86 μl (0.49 mmol) *N,N*-Diisopropylethylamin in 3.5 ml Dimethylformamid zur Reaktion gebracht. Es wurde 1 ml 1M wässrige Salzsäure und 10 ml Wasser hinzugegeben und dreimal mit 10 ml Essigsäureethylester extrahiert. Die vereinigten organischen Phasen wurden über Magnesiumsulfat getrocknet, filtriert und das Lösungsmittel unter reduziertem Druck entfernt. Der Rückstand wurde in wenig DCM aufgenommen und mittels Flashchromatographie (Cyclohexan/Essigsäureethylester-Gradient) gereinigt. Es wurden 107 mg (53% d. Th., 100% Reinheit) der Titelverbindung erhalten.
$^1$H-NMR (400 MHz, DMSO-$d_6$): δ [ppm] = 2.31-2.42, 2.88-3.00 (m, 1H), 3.41-3.52 (m, 1H), 3.61-3.72 (m, 1H), 4.25-4.32 (m, 1H), 5.32 (d, 1H), 6.15-6.25 (m, 1H), 7.27-7.49 (m, 4H), 7.52-7.68 (m, 2H), 7.76-7.94 (m, 1H), 8.51-8.57 (m, 1H), 8.77 (d, 1H), 8.87 (s, 1H), 11.21 (d, 1H).
LC-MS (Methode 3): $R_t$ = 2.04 min; 577 $[M+H]^+$.

**Beispiel 273:**

1-(2,4-Difluorphenyl)-7-[(4*S*)-4-hydroxy-2-oxopyrrolidin-1-yl]-4-oxo-*N*-[2,2,2-trifluor-1-(4-fluorphenyl)ethyl]-1,4-dihydro-1,8-naphthyridin-3-carbonsäureamid (Diastereomerengemisch)

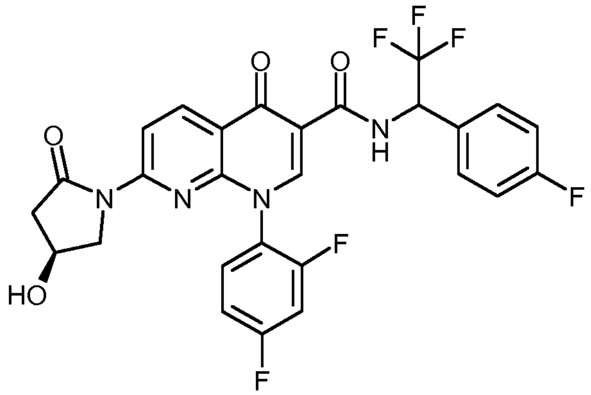

Gemäß AAV1 wurden 170 mg (352 µmol, 83% Reinheit) der Verbindung aus Beispiel 63A mit 81.5 mg (422 µoml) *rac*-2,2,2-Trifluor-1-(4-fluorphenyl)ethanamin in Gegenwart von 134 mg (352 µmol) HATU und 86 µl (0.49 mmol) *N,N*-Diisopropylethylamin in 3.5 ml Dimethylformamid zur Reaktion gebracht. Es wurde 1 ml 1M wässrige Salzsäure und 10 ml Wasser hinzugegeben und dreimal mit 10 ml Essigsäureethylester extrahiert. Die vereinigten organischen Phasen wurden mit ges. wässriger Natriumchlorid-Lösung gewaschen, über Magnesiumsulfat getrocknet, filtriert und das Lösungsmittel unter reduziertem Druck entfernt. Der Rückstand wurde in wenig Dichlormethan aufgenommen und mittels Flashchromatographie (Cyclohexan/Essigsäureethylester-Gradient) gereinigt. Es wurden 98 mg (48% d. Th., 100% Reinheit) der Titelverbindung erhalten.
$^1$H-NMR (400 MHz, DMSO-$d_6$): δ [ppm] = 2.32-2.42 (m, 1H). 2.88-3.00 (m, 1H), 3.42-3.52 (m, 1H), 3.61-3.72 (m, 1H), 4.25-4.32 (m, 1H), 5.32 (d, 1H), 6.09-6.21 (m, 1H), 7.30-7.42 (m, 3H), 7.58-7.68 (m, 3H), 7.77-7.93 (m, 1H), 8.51-8.57 (m, 1H), 8.76 (d, 1H), 8.86 (s, 1H), 11.20 (d, 1H).
LC-MS (Methode 3): $R_t$ = 2.03 min; 577 $[M+H]^+$.

**Beispiel 274:**

*N*-[1-(3-Chlorphenyl)-2,2,2-trifluorethyl]-1-(2,4-difluorphenyl)-7-[(4*S*)-4-hydroxy-2-oxopyrrolidin-1-yl]-4-oxo-1,4-dihydro-1,8-naphthyridin-3-carbonsäureamid (Diastereomerengemisch)

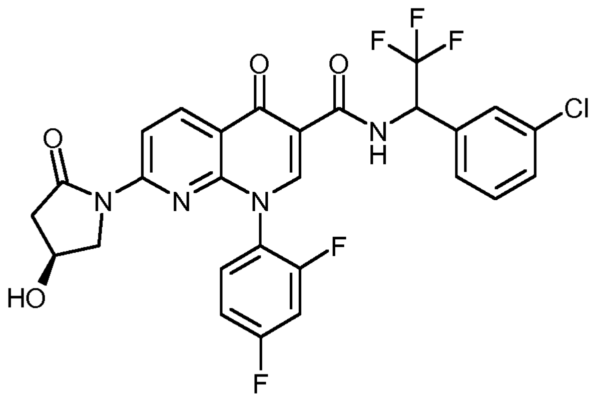

Gemäß AAV1 wurden 170 mg (352 µmol, 83% Reinheit) der Verbindung aus Beispiel 63A mit 88.4 mg (422 µmol) *rac*-1-(3-Chlorphenyl)-2,2,2-trifluoroethanamin in Gegenwart von 134 mg (352 µmol) HATU und 86 µl (0.49 mmol) *N,N*-Diisopropylethylamin in 3.5 ml Dimethylformamid zur Reaktion gebracht. Es wurde 1 ml 1M wässrige Salzsäure und 10 ml Wasser hinzugegeben und dreimal mit 10 ml Essigsäureethylester extrahiert. Die vereinigten organischen Phasen wurden mit ges. wässriger Natriumchlorid-Lösung gewaschen, über Magnesiumsulfat getrocknet, filtriert und das Lösungsmittel unter reduziertem Druck entfernt. Der Rückstand wurde in wenig Dichlormethan aufgenommen und mittels Flashchromatographie (Cyclohexan/Essigsäureethylester-Gradient) gereinigt. Es wurden 127 mg (61% d. Th., 100% Reinheit) der Titelverbindung erhalten.
$^1$H-NMR (400 MHz, DMSO-$d_6$): δ [ppm] = 2.31-2.42 (m, 1H), 2.87-3.01 (m, 1H), 3.42-3.53 (m, 1H), 3.60-3.73 (m, 1H), 4.25-4.32 (m, 1H), 5.32 (d, 1H), 6.15-6.25 (m, 1H), 7.31-7.41 (m, 1H), 7.51-7.71 (m, 5H), 7.77-7.94 (m, 1H), 8.51-8.57

(m, 1H), 8.77 (d, 1H), 8.86 (s, 1H), 11.22 (d, 1H).
LC-MS (Methode 3): $R_t$ = 2.15 min; 593 [M+H]$^+$.

**Beispiel 275:**

1-(2,6-Difluorphenyl)-7-[(4*S*)-4-hydroxy-2-oxopyrrolidin-1-yl]-4-oxo-*N*-[(2*R*)-1,1,1-trifluorbutan-2-yl]-1,4-dihydro-1,8-naphthyridin-3-carbonsäureamid

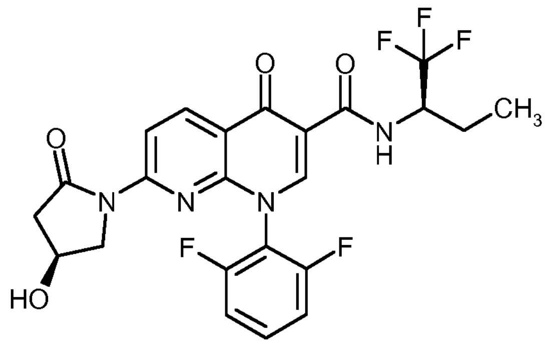

Gemäß AAV2 wurden 100 mg (224 μmol) der Verbindung aus Beispiel 86A mit 22.7 mg (224 μmol) (4*S*)-4-Hydroxypyrolidin-2-on in Gegenwart von 109 mg (336 μmol) Cäsiumcarbonat, 9.1 mg (40 μmol) Palladium(II)acetat und 47 mg (81 μmol) Xantphos in 5 ml 1,4-Dioxan umgesetzt. Anschließend wurde mit 2 ml Acetonitril und 0.5 ml Wasser verdünnt, filtriert und mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 33.2 mg (29% d. Th., 99% Reinheit) der Titelverbindung erhalten.
$^1$H-NMR (400 MHz, DMSO-$d_6$): δ [ppm] = 0.99 (t, 3H). 1.60-1.74 (m, 1H), 1.84-1.96 (m, 1H), 2.37 (d, 1H), 2.93 (dd, 1H), 3.43 (d, 1H), 3.64 (dd, 1H), 4.21-4.30 (m, 1H), 4.70-4.83 (m, 1H), 5.32 (d, 1H), 7.42-7.50 (m, 2H), 7.72-7.81 (m, 1H), 8.54 (d, 1H), 8.72 (d, 1H), 9.02 (s, 1H), 10.13 (d, 1H).
LC-MS (Methode 3): $R_t$ = 1.86 min; 511 [M+H]$^+$.

**Beispiel 276:**

1-(2,6-Difluorophenyl)-7-[3-hydroxy-3-methylpyrrolidin-1-yl]-4-oxo-*N*-[(2*R*)-1,1,1-trifluorbutan-2-yl]-1,4-dihydro-1,8-naphthyridin-3-carbonsäureamid (Diastereomerengemisch)

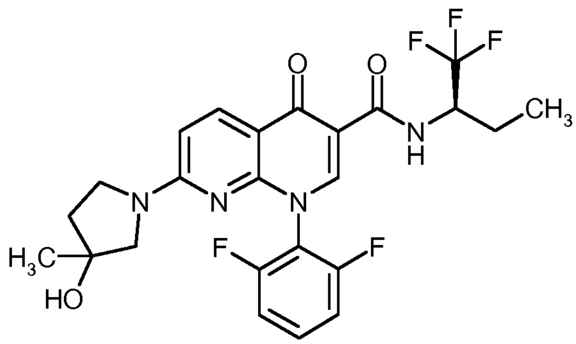

Gemäß AAV3 wurden 100 mg (224 μmol) der Verbindung aus Beispiel 86A mit 41.2 mg (269 μmol, 90% Reinheit) *rac*-3-Methylpyrrolidin-3-ol Hydrochlorid und 137 μl (785 μmol) *N*,*N*-Diisopropylethylamin in 1 ml Dimethylformamid zur Reaktion gebracht. Es wurde mit 0.5 ml Acetonitril verdünnt und das Rohlösung wurde mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 40 min. 90% Acetonitril und weitere 3 min. 90% Acetonitril) gereinigt und es wurden 96.4 mg (83% d. Th., 99% Reinheit) der Titelverbindung erhalten.

$^1H$ NMR (400 MHz, DMSO-$d_6$): δ [ppm] = 0.97 (t, 3H), 1.22/1.31 (2x s, 3H), 1.57-1.72 (m, 1H), 1.72-1.80 (m, 1H), 1.82-1.95 (m, 2H), 2.90/3.09 (2x d, 1H), 3.13-3.21 (m, 1H), 3.23-3.40 (m, 1H, teilweise unter dem DMSO Peak), 3.49-3.59 (m, 1H), 4.67-4.79 (m, 1H), 4.84 (d, 1H), 6.74 (dd, 1H), 7.36-7.46 (m, 2H), 7.65-7.76 (m, 1H), 8.24-8.31 (m, 1H), 8.73 (d, 1H), 10.45 (d, 1H).
LC-MS (Methode 3): $R_t$ = 2.00 min; 511 $[M+H]^+$.

90 mg der Titelverbindung (Diastereomerengemisch) wurde durch chirale HPLC in die Diastereomere getrennt (präparative HPLC: Säule Daicel Chiralpak IE-H 5 μm 250x20 mm; Eluent: 20% Ethanol, 80% Iso-Hexan; Temperatur: 25°C; Fluss: 15 ml/min; UV-Detektion: 220 nm.)

Man erhielt (in der Reihenfolge der Elution von der Säule) 43 mg Diastereomer 1 (99% de) $R_t$ = 6.98 min und 45 mg (94%de) Diastereomer 2 $R_t$ = 7.36 min.

[Analytische HPLC: Säule Chiralpak IE-3 5 μm 250x4.6 mm; Eluent: 25% Ethanol, 75% Iso-Hexan; Temperatur: 30°C; Fluss: 1.0 ml/min; UV-Detektion: 220 nm].

Diastereomer 1 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 35.8 mg (31% d. Th., 99% Reinheit) der Titelverbindung aus Beispiel 276 erhalten.

Diastereomer 2 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 36.0 mg (31% d. Th., 99% Reinheit) der Titelverbindung aus Beispiel 277 erhalten.

**Beispiel 277:**

1-(2,6-Difluorophenyl)-7-[3-hydroxy-3-methylpyrrolidin-1-yl]-4-oxo-*N*-[(2*R*)-1,1,1-trifluorbutan-2-yl]-1,4-dihydro-1,8-naphthyridin-3-carbonsäureamid (Diastereomer 1)

$^1H$ NMR (400 MHz, DMSO-$d_6$): δ [ppm] = 0.97 (t, 3H), 1.22/1.31 (2x s, 3H), 1.58-1.71 (m, 1H), 1.73-1.80 (m, 1H), 1.83-1.94 (m, 2H), 2.91/3.09 (2x d, 1H), 3.13-3.21 (m, 1H), 3.27-3.39 (m, 1H, teilweise unter dem DMSO Peak), 3.51-3.58 (m, 1H), 4.67-4.78 (m, 1H), 4.85 (d, 1H), 6.74 (dd, 1H), 7.37-7.45 (m, 2H), 7.65-7.76 (m, 1H), 8.24-8.31 (m, 1H), 8.74 (d, 1H), 10.45 (d, 1H).
LC-MS (Methode 3): $R_t$ = 2.01 min; 511 $[M+H]^+$.

**Beispiel 278:**

1-(2,6-Difluorophenyl)-7-[3-hydroxy-3-methylpyrrolidin-1-yl]-4-oxo-*N*-[(2*R*)-1,1,1-trifluorbutan-2-yl]-1,4-dihydro-1,8-naphthyridin-3-carbonsäureamid (Diastereomer 2)

$^1H$ NMR (400 MHz, DMSO-$d_6$): δ [ppm] = 0.97 (t, 3H). 1.22/1.31 (2x s, 3H), 1.57-1.71 (m, 1H), 1.73-1.80 (m, 1H), 1.82-1.95 (m, 2H), 2.91/3.09 (2x d, 1H), 3.13-3.21 (m, 1H), 3.26-3.39 (m, 1H, teilweise unter dem DMSO Peak), 3.50-3.57 (m, 1H), 4.67-4.79 (m, 1H), 4.85 (d, 1H), 6.74 (dd, 1H), 7.37-7.46 (m, 2H), 7.65-7.76 (m, 1H), 8.24-8.32 (m, 1H), 8.74 (d, 1H), 10.45 (d, 1H),
LC-MS (Methode 3): $R_t$ = 2.01 min; 511 $[M+H]^+$.

**Beispiel 279:**

1-(2-Chlorphenyl)-*N*-[1-cyclopropyl-2,2,2-trifluorethyl]-7-[(4*S*)-4-hydroxy-2-oxopyrrolidin-1-yl]-4-oxo-1,4-dihydro-1,8-naphthyridin-3-carbonsäureamid (Diastereomerengemisch)

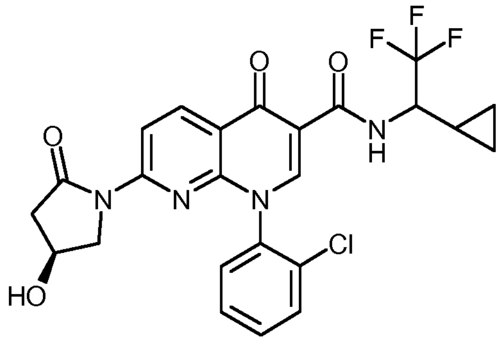

Gemäß AAV1 wurden 30 mg (75 μmol) der Verbindung aus Beispiel 64A mit 19.8 mg (11.3 μmol) *rac*-1-Cyclopropyl-2,2,2-trifluorethanamin Hydrochlorid in Gegenwart von 29 mg (75 μmol) HATU und 39 μl (0.23 mmol) *N*,*N*-Diisopropylethylamin in 0.77 ml Dimethylformamid zur Reaktion gebracht. Es wurde mit 1 ml Acetonitril und 0.5 ml Wasser verdünnt und die Lösung mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 25.8 mg (65% d. Th., 99% Reinheit) der Titelverbindung erhalten.
$^1$H-NMR (400 MHz, DMSO-$d_6$): δ [ppm] = 0.30-0.40 (m, 1H), 0.50-0.71 (m, 3H), 1.18-1.29 (m, 1H), 2.31-2.39 (m, 1H), 2.86-2.97 (m, 1H), 3.33-3.41 (m, 1H), 3.52-3.61 (m, 1H), 4.20-4.26 (m, 1H), 4.35-4.47 (m, 1H), 5.31 (dd. 1H), 7.58-7.70 (m, 2H), 7.76-7.83 (m, 2H), 8.51 (dd, 1H), 8.70-8.75 (m, 2H), 10.38 (dd, 1H).
LC-MS (Methode 1): $R_t$ = 1.02 min; 521 $[M+H]^+$.

**Beispiel 280:**

7-[3-Hydroxy-2-oxopyrrolidin-1-yl]-4-oxo-*N*-[(2*R*)-1,1,1-trifluorbutan-2-yl]-1-(2,4,6-trifluorphenyl)-1,4-dihydro-1,8-naphthyridin-3 -carbonsäureamid (Diastereomerengemisch)

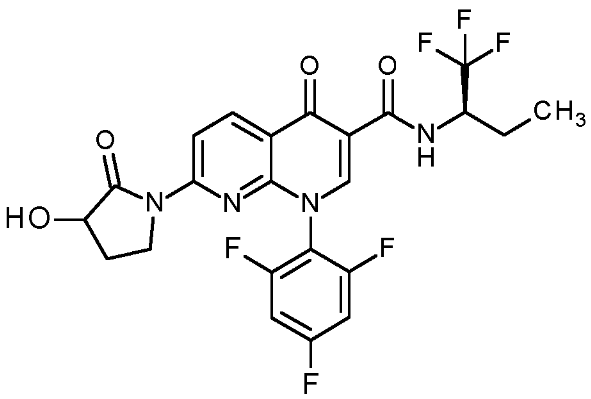

Gemäß AAV2 wurden 150 mg (323 μmol) der Verbindung aus Beispiel 100C mit 32.7 mg (323 μmol) 3-Hydroxypyrolidin-2-on (CAS: 15166-68-4) in Gegenwart von 67.1 mg (485 μmol) Kaliumcarbonat, 13 mg (58 μmol) Palladium(II)acetat und 67.4 mg (116 μmol) Xantphos in 2.97 ml 1,4-Dioxan umgesetzt. Anschließend wurde das Volumen der Mischung unter reduziertem Druck eingeengt und mit 3 ml Acetonitril und 1 ml 1N wässriger Salzsäure verdünnt, filtriert und mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 118.4 mg (69% d. Th., 99% Reinheit) der Titelverbindung erhalten.
$^1$H-NMR (400 MHz, DMSO-$d_6$): δ [ppm] = 10.11 (d, 1H), 9.08 (s, 1H), 8.74 (d, 1H), 8.55 (d, 1H), 7.64-7.56 (m, 2H), 5.91 (d, 1H), 4.83-4.71 (m, 1H), 4.43-4.35 (m, 1H), 3.62-3.54 (m, 1H), 3.38-3.32 (m, 1H), 2.36-2.27 (m, 1H), 1.95-1.60 (m, 3H), 0.98 (t, 3H).
LC-MS (Methode 1): $R_t$ = 1.00 min; 529 $[M+H]^+$.
110 mg der Titelverbindung (Diastereomerengemisch) wurde durch chirale HPLC in die Diastereomere getrennt (präparative HPLC: Säule Daicel Chiralcel OZ-H 5 μm 250x20 mm; Eluent: 20% Ethanol, 80% Iso-Hexan; Temperatur: 23°C; Fluss: 20 ml/min; UV-Detektion: 220 nm.)

Man erhielt (in der Reihenfolge der Elution von der Säule) 41.4 mg Diastereomer 1 (100% de) $R_t$ = 2.27 min und 44.8 mg (93% de) Diastereomer 2 $R_t$ = 2.67 min.

[Analytische HPLC: Säule Chiralcel OZ-3 3 μm; Eluent: 20% Ethanol, 80% Iso-Hexan; Fluss: 1.0 ml/min; UV-Detektion: 220 nm].

Diastereomer 1 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 37.0 mg (22% d. Th., 99% Reinheit) der Titelverbindung aus Beispiel 281 erhalten.

Diastereomer 2 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 39.8 mg (23% d. Th., 99% Reinheit) der Titelverbindung aus Beispiel 282 erhalten.

**Beispiel 281:**

7-[3-Hydroxy-2-oxopyrrolidin-1-yl]-4-oxo-*N*-[(2*R*)-1,1,1-trifluorbutan-2-yl]-1-(2,4,6-trifluorphenyl)-1,4-dihydro-1,8-naphthyridin-3-carbonsäureamid (Diastereomer 1)

$^1$H-NMR (400 MHz, DMSO-$d_6$): δ [ppm] = 10.11 (d, 1H), 9.08 (s, 1H), 8.74 (d, 1H), 8.55 (d, 1H), 7.64-7.56 (m, 2H), 5.91 (d, 1H), 4.82-4.72 (m, 1H), 4.43-4.35 (m, 1H), 3.62-3.54 (m, 1H), 3.38-3.32 (m, 1H), 2.37-2.27 (m, 1H), 1.97-1.61 (m, 3H), 0.98 (t, 3H).
LC-MS (Methode 3): $R_t$ = 1.89 min; 529 $[M+H]^+$.

**Beispiel 282:**

7-[3-Hydroxy-2-oxopyrrolidin-1-yl]-4-oxo-*N*-[(2*R*)-1,1,1-trifluorbutan-2-yl]-1-(2,4,6-trifluorphenyl)-1,4-dihydro-1,8-naphthyridin-3-carbonsäureamid (Diastereomer 2)

$^1$H-NMR (400 MHz, DMSO-$d_6$): δ [ppm] = 10.11 (d, 1H), 9.08 (s, 1H), 8.74 (d, 1H), 8.55 (d, 1H), 7.64-7.56 (m, 2H), 5.91 (d, 1H), 4.84-4.70 (m, 1H), 4.43-4.35 (m, 1H), 3.62-3.53 (m, 1H), 3.38-3.32 (m, 1H), 2.36-2.26 (m, 1H), 1.96-1.60 (m, 3H), 0.98 (t, 3H).
LC-MS (Methode 3): $R_t$ = 1.89 min; 529 $[M+H]^+$.

**Beispiel 283:**

*N*-[(1-Cyclopropyl-2,2,2-trifluorethyl]-1-(2,4-difluorophenyl)-7-[(4*S*)-4-hydroxy-2-oxopyrrolidin-1-yl]-4-oxo-1,4-dihydro-1,8-naphthyridin-3-carbonsäureamid (Diastereomerengemisch)

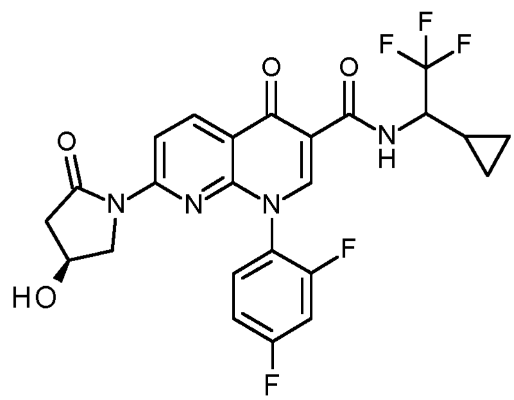

Gemäß AAV2 wurden 560 mg (1.22 mmol) der Verbindung aus Beispiel 101A mit 124 mg (1.22 mmol) (4*S*)-4-Hydroxypyrolidin-2-on (CAS: 68108-18-9) in Gegenwart von 254 mg (1.84 mmol) Kaliumcarbonat, 49.4 mg (220 μmol) Palladium(II)acetat und 255 mg (440 μmol) Xantphos in 11.2 ml 1,4-Dioxan umgesetzt. Anschließend wurde das Volumen der Mischung unter reduziertem Druck eingeengt, der Rückstand mit 1N wässriger Salzsäure angesäuert und Dichlormethan verdünnt. Das Rohprodukt wurde mittels Normalphasenchromatographie (Cyclohexan-Essigsäureethylester Gradient) gereinigt und 316 mg (49% d. Th., 99% Reinheit) der Titelverbindung erhalten.

$^1$H-NMR (400 MHz, DMSO-$d_6$): δ [ppm] = 10.36 (d, 1H), 8.84 (s, 1H), 8.71 (d, 1H), 8.52 (dd, 1H), 7.92-7.82 (m, 1H), 7.66-7.59 (m, 1H), 7.41-7.33 (m, 1H), 5.32 (dd, 1H), 4.48-4.35 (m, 1H), 4.31-4.26 (m, 1H), 3.72-3.61 (m, 1H), 3.52-3.42 (m, 1H), 3.00-2.87 (m, 1H), 2.42-2.32 (m, 1H), 1.29-1.19 (m, 1H), 0.71-0.50 (m, 3H), 0.39-0.30 (m, 1H).

LC-MS (Methode 1): $R_t$ = 1.06 min; 523 [M+H]$^+$.

310 mg der Titelverbindung (Diastereomerengemisch) wurde durch chirale HPLC in die Diastereomere getrennt (präparative HPLC: Säule Daicel Chiralpak IE 5 µm 250x20 mm; Eluent: 50% Ethanol, 50% Iso-Hexan; Temperatur: 25°C; Fluss: 15 ml/min; UV-Detektion: 210 nm.)

Man erhielt (in der Reihenfolge der Elution von der Säule) 145 mg Diastereomer 1 (100% de) $R_t$ = 2.95 min und 128 mg (100% de) Diastereomer 2 $R_t$ = 5.61 min.

[Analytische HPLC: Säule Chiraltek IE-3 3 µm; Eluent: 50% Ethanol, 50% Iso-Hexan; UV-Detektion: 220 nm].

Diastereomer 1 wurde zusätzlich mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 129.7 mg (20% d. Th., 99% Reinheit) der Titelverbindung aus Beispiel 284 erhalten.

Diastereomer 2 wurde zusätzlich mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 112 mg (17% d. Th., 99% Reinheit) der Titelverbindung aus Beispiel 271 erhalten.

**Beispiel 284:**

*N*-[(1*S*)-1-cyclopropyl-2,2,2-trifluoroethyl]-1-(2,4-difluorophenyl)-7-[(4*S*)-4-hydroxy-2-oxopyrrolidin-1-yl]-4-oxo-1,4-dihydro-1,8-naphthyridin-3-carbonsäureamid (Diastereomer 1)

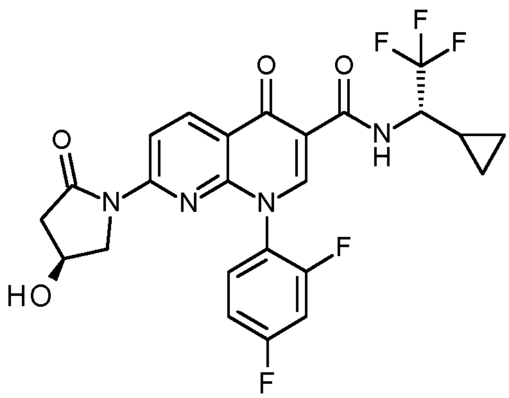

$^1$H-NMR (400 MHz, DMSO-$d_6$): δ [ppm] = 10.11 (d, 1H), 9.08 (s, 1H), 8.74 (d, 1H), 8.55 (d, 1H), 7.64-7.56 (m, 2H), 5.91 (d, 1H), 4.84-4.70 (m, 1H), 4.43-4.35 (m, 1H), 3.62-3.53 (m, 1H), 3.38-3.32 (m, 1H), 2.36-2.26 (m, 1H), 1.96-1.60 (m, 3H), 0.98 (t, 3H).

LC-MS (Methode 3): $R_t$ = 1.89 min; 529 [M+H]$^+$.

**Beispiel 285:**

1-(2,4-Difluorphenyl)-7-[(4*R*)-4-hydroxy-2-oxopyrrolidin-1-yl]-4-oxo-*N*-[(2*S*)-1,1,1-trifluorbutan-2-yl]-1,4-dihydro-1,8-naphthyridin-3-carbonsäureamid

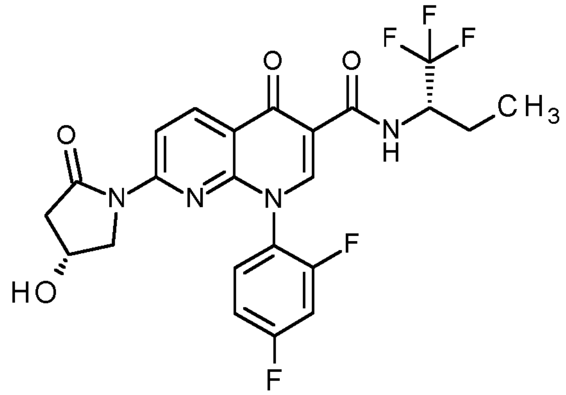

Gemäß AAV2 wurden 140 mg (314 µmol) der Verbindung aus Beispiel 68A mit 38.1 mg (377 µmol) (4*R*)-4-Hydroxypyrolidin-2-on (CAS: 22677-21-0) in Gegenwart von 65.1 mg (471 µmol) Kaliumcarbonat, 7.1 mg (31 µmol) Palladium(II)acetat und 18 mg (31 µmol) Xantphos in 3.1 ml 1,4-Dioxan umgesetzt. Nach Reaktion über Nacht bei 80°C wurden 0.1 Äq. Palladium(II)acetat und 0.1 Äq. Xantphos nachgegeben und weitere 3h gerührt. Anschließend wurde das Volumen der Mischung unter reduziertem Druck eingeengt, der Rückstand mit 0.5 ml Wasser und 3 ml Acetonitril aufgenommen, filtriert und das Rohprodukt wurde mittels präparativer HPLC (Säule: Chromatorex C18, 10 µm, 125x30 mm, LM: Acetonitril / 0.05% Ameisensäure-Gradient; (0 bis 3 min. 10% Acetonitril, bis 35 min. 90% Acetonitril und weitere 3 min. 90% Acetonitril) gereinigt. Es wurden 63.2 mg (39% d. Th., 100% Reinheit) der Titelverbindung erhalten.
$^1$H-NMR (400 MHz, DMSO-$d_6$): δ [ppm] = 10.22 (d, 1H), 8.87-8.83 (m, 1H), 8.71 (d, 1H), 8.52 (dd, 1H), 7.92-7.83 (m, 1H), 7.67-7.58 (m, 1H), 7.41-7.33 (m, 1H), 5.32 (dd, 1H), 4.84-4.70 (m, 1H), 4.32-4.24 (m, 1H), 3.72-3.61 (m, 1H), 3.52-3.42 (m, 1H), 3.00 (m, 1H), 2.41-2.31 (m, 1H), 1.96-1.83 (m, 1H), 1.73-1.59 (m, 1H), 0.98 (t, 3H).
LC-MS (Methode 3): $R_t$ = 1.83 min; 511 $[M+H]^+$.

**Beispiel 286:**

1-(2,4-Difluorphenyl)-4-oxo-7-(2-oxo-2,5-dihydro-1*H*-pyrrol-1-yl)-*N*-[(2*S*)-1,1,1-trifluorbutan-2-yl]-1,4-dihydro-1,8-naphthyridin-3-carbonsäureamid

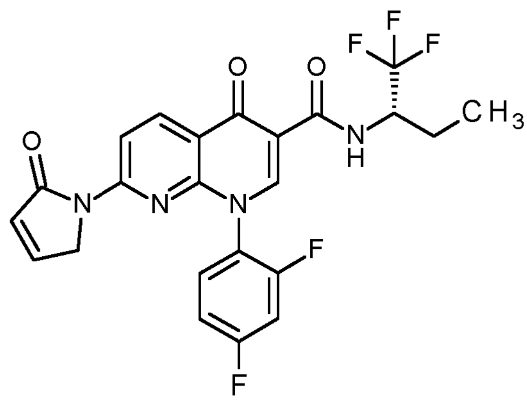

Zu einer Lösung von 47 mg (92 µmol) der Verbindung aus Beispiel 285 in 737 µl Toluol wurden 40 mg (74 µmol) Tetrabutylammonium-triphenyldifluorsilikat und 40.0 µl (230 µmol) Diisopropylethylamin gegeben. Es wurde für 5 min bei Raumtemperatur nachgerührt und danach 61.2 mg (203 µmol) Perfluorbutan-1-sulfonsäurefluorid hinzugegeben. Es wurde für 20 min bei Raumtemperatur nachgerührt und anschließend alle flüchtigen Bestandteile unter reduziertem Druck entfernt. Der Rückstand wurde mit 4 ml Acetonitril und 2 ml Wasser verrührt. Der Niederschlag wurde abgesaugt und im Hochvakuum getrocknet. Es wurden 19.6 mg (37% d. Th., 85% Reinheit) der Titelverbindung erhalten.
$^1$H-NMR (400 MHz, DMSO-$d_6$): δ [ppm] = 10.22 (d, 1H), 8.85 (s, 1H), 8.73 (d, 1H), 8.53 (d, 1H), 7.93-7.84 (m, 1H), 7.64-7.58 (m, 1H), 7.53 (d, 1H), 7.41-7.31 (m, 1H), 6.29 (d, 1H), 4.82-4.71 (m, 1H), 4.24 (s, 2H), 1.96-1.84 (m, 1H), 1.72-1.61 (m, 1H), 0.98 (t, 3H).
LC-MS (Methode 3): $R_t$ = 2.13 min; 493 $[M+H]^+$.

**Beispiel 287:**

*N*-[1-(2-Chlorphenyl)-2,2,2-trifluorethyl]-1-(2-fluorphenyl)-7-[3-hydroxy-2-oxopyrrolidin-1-yl]-4-oxo-1,4-dihydro-1,8-naphthyridin-3-carbonsäureamid (racemisches Diastereomerengemisch)

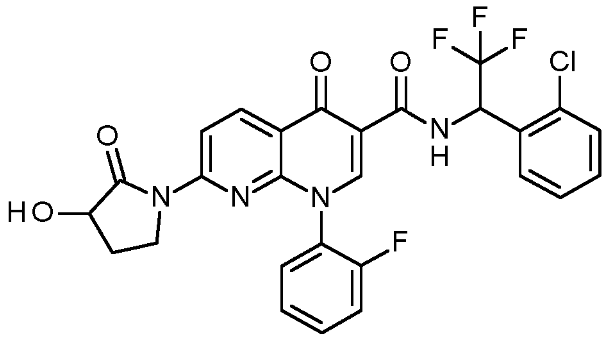

Gemäß AAV1 wurden 75.0 mg (196 µmol) der Verbindung aus Beispiel 33B mit 61.5 mg (293 µmol) *rac*-1-(2-Chlorphenyl)-2,2,2-trifluorethanamin in Gegenwart von 74.4 mg (196 µmol) HATU und 102 µl (587 µmol) *N*,*N*-Diisopropylethylamin in 2 ml Dimethylformamid zur Reaktion gebracht. Es wurde mit 1 ml Acetonitril und 0.5 ml Wasser verdünnt und die Lösung mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 94.5 mg (83% d. Th., 99% Reinheit) der Titelverbindung erhalten.
$^1$H-NMR (400 MHz, DMSO-$d_6$): δ [ppm] = 11.33 (d, 1H), 8.84 (s, 1H), 8.78 (d, 1H), 8.58-8.52 (m, 1H), 7.82-7.41 (m, 8H), 6.53-6.43 (m, 1H), 5.90 (d, 1H), 4.45-4.32 (m, 1H), 3.60-3.46 (m, 1H), 3.36-3.20 (m, 1H teilweise unter dem Wassersignal), 2.34-2.24 (m, 1H), 1.83-1.66 (m, 1H).
LC-MS (Methode 3): $R_t$ = 2.11 min; 575 [M+H]$^+$.

90 mg der Titelverbindung (Diastereomerengemisch) wurde durch zwei chirale HPLC in die Diastereomere getrennt (präparative HPLC: Säule Daicel Chiralcel OX-H 5 µm 250x20 mm; Eluent: 100% Ethanol, Temperatur: 45°C; Fluss: 15 ml/min; UV-Detektion: 220 nm und Säule Daicel Chiralpak IF 5 µm 250x20 mm; Eluent: 35% Ethanol, 65% Iso-Hexan, Temperatur: 45°C; Fluss: 15 ml/min; UV-Detektion: 220 nm). Man erhielt (in der Reihenfolge der Elution von der Säule) 12 mg (Enantiomer 1 von Diastereomer 1, 93% de) $R_t$ = 6.29 min, 15 mg (Enantiomer 1 von Diastereomer 2, 100% de) $R_t$ = 6.93 min, 15 mg (Enantiomer 2 von Diastereomer 2, 80% de) $R_t$ = 10.88 min und 19 mg (Enantiomer 2 von Diastereomer 1, 100% de) $R_t$ = 13.11 min.

[Analytische HPLC: Säule Chiralcel OX-H 5 µm 250x4.6 mm; Eluent: 100% Ethanol; Fluß: 1 ml/min; Temperatur: 45°C; UV-Detektion: 220 nm].

Enantiomer 1 von Diastereomer 1 wurde zusätzlich mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 11.3 mg (10% d. Th., 99% Reinheit) der Titelverbindung aus Beispiel 288 erhalten.

Enantiomer 1 von Diastereomer 2 wurde zusätzlich mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 12.8 mg (11% d. Th., 99% Reinheit) der Titelverbindung aus Beispiel 289 erhalten.

Enantiomer 2 von Diastereomer 2 wurde zusätzlich mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 14.2 mg (13% d. Th., 99% Reinheit) der Titelverbindung aus Beispiel 290 erhalten.

Enantiomer 2 von Diastereomer 1 wurde zusätzlich mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 15.7 mg (14% d. Th., 99% Reinheit) der Titelverbindung aus Beispiel 291 erhalten.

**Beispiel 288:**

$^1$H-NMR (400 MHz, DMSO-$d_6$): δ [ppm] = 11.33 (d, 1H), 8.84 (s, 1H), 8.78 (d, 1H), 8.57-8.57 (m, 1H), 7.82-7.40

(m, 8H), 6.52-6.42 (m, 1H), 5.90 (d, 1H), 4.43-4.32 (m, 1H), 3.60-3.46 (m, 1H), 3.34-3.20 (m, 1H teilweise unter dem Wassersignal), 2.34-2.24 (m, 1H), 1.83-1.66 (m, 1H).
LC-MS (Methode 3): $R_t$ = 2.11 min; 575 $[M+H]^+$.

**Beispiel 289:**

$^1$H-NMR (400 MHz, DMSO-$d_6$): δ [ppm] = 11.33 (d, 1H), 8.84 (s, 1H), 8.78 (d, 1H), 8.57-8.51 (m, 1H), 7.81-7.42 (m, 8H), 6.53-6.42 (m, 1H), 5.90 (d, 1H), 4.44-4.31 (m, 1H), 3.59-3.45 (m, 1H), 3.36-3.19 (m, 1H teilweise unter dem Wassersignal), 2.33-2.23 (m, 1H), 1.83-1.65 (m, 1H).
LC-MS (Methode 3): $R_t$ = 2.14 min; 575 $[M+H]^+$.

**Beispiel 290:**

$^1$H-NMR (400 MHz, DMSO-$d_6$): δ [ppm] = 11.33 (d, 1H), 8.84 (s, 1H), 8.78 (d, 1H), 8.57-8.51 (m, 1H), 7.81-7.41 (m, 8H), 6.53-6.42 (m, 1H), 5.90 (d, 1H), 4.44-4.31 (m, 1H), 3.58-3.46 (m, 1H), 2.34-2.23 (m, 1H), 1.82-1.66 (m, 1H). Eine Protonresonanz unter dem Wassersignal.
LC-MS (Methode 3): $R_t$ = 2.11 min; 575 $[M+H]^+$.

**Beispiel 291:**

$^1$H-NMR (400 MHz, DMSO-$d_6$): δ [ppm] = 11.33 (d, 1H), 8.84 (s, 1H), 8.78 (d, 1H), 8.58-8.52 (m, 1H), 7.82-7.40 (m, 8H), 6.53-6.42 (m, 1H), 5.90 (d, 1H), 4.45-4.32 (m, 1H), 3.60-3.45 (m, 1H), 3.35-3.19 (m, 1H teilweise unter dem Wassersignal), 2.34-2.23 (m, 1H), 1.82-1.67 (m, 1H).
LC-MS (Methode 3): $R_t$ = 2.11 min; 575 $[M+H]^+$.

**Beispiel 292:**

1-(2,6-Difluorphenyl)-7-[3-hydroxy-2-oxopyrrolidin-1-yl]-4-oxo-*N*-[(2*R*)-1,1,1-trifluorbutan-2-yl]-1,4-dihydro-1,8-naphthyridin-3-carbonsäureamid (Diastereomerengemisch)

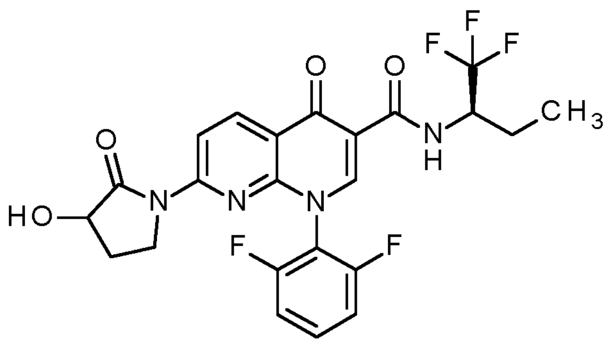

Gemäß AAV2 wurden 150 mg (336 μmol) der Verbindung aus Beispiel 86A mit 34.0 mg (336 μmol) 3-Hydroxypyrolidin-2-on (CAS: 15166-68-4) in Gegenwart von 69.8 mg (505 μmol) Kaliumcarbonat, 14 mg (61 μmol) Palladium(II)acetat und 70.1 mg (121 μmol) Xantphos in 3.09 ml 1,4-Dioxan umgesetzt. Anschließend wurde das Volumen der Mischung unter reduziertem Druck eingeengt und es wurde mit 3 ml Acetonitril verdünnt und mit 1 ml 1N wässriger Salzsäure angesäuert, filtriert und mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 127.3 mg (73% d. Th., 99% Reinheit) der Titelverbindung erhalten.
$^1$H-NMR (400 MHz, DMSO-$d_6$): δ [ppm] = 10.12 (d, 1H), 9.03 (s, 1H), 8.74 (d, 1H), 8.55 (d, 1H), 7.79-7.70 (m, 1H), 7.49-7.41 (m, 2H), 5.90 (d, 1H), 4.83-4.71 (m, 1H), 4.42-4.34 (m, 1H), 3.55-3.49 (m, 1H), 3.33-3.23 (m, 1H teilweise unter dem Wassersignal), 2.34-2.23 (m, 1H), 1.95-1.61 (m, 3H), 0.98 (t, 3H). LC-MS (Methode 3): $R_t$ = 1.87 min; 511 $[M+H]^+$.

120 mg der Titelverbindung (Diastereomerengemisch) wurde durch chirale HPLC in die Diastereomere getrennt (präparative HPLC: Säule Daicel Chiralcel OX-H 5 μm 250x20 mm; Eluent: 45% Ethanol, 55% Iso-Hexan; Temperatur: 23°C; Fluss: 20 ml/min; UV-Detektion: 220 nm.)

Man erhielt (in der Reihenfolge der Elution von der Säule) 52.5 mg Diastereomer 1 (100% de) $R_t$ = 1.13 min und 45.5 mg (94% de) Diastereomer 2 $R_t$ = 1.25 min.

[Analytische HPLC: Säule Daicel Chiralpak OX-3 3 μm 50x4.6 mm; Eluent: 50% Ethanol, 50% Iso-Hexan; Fluß: 1 ml/min; UV-Detektion: 220 nm].

Diastereomer 1 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 40.7 mg (23% d. Th., 99% Reinheit) der Titelverbindung aus Beispiel 293 erhalten.

Diastereomer 2 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 41.1 mg (24% d. Th., 99% Reinheit) der Titelverbindung aus Beispiel 294 erhalten.

**Beispiel 293:**

$^1$H-NMR (400 MHz, DMSO-$d_6$): δ [ppm] = 10.12 (d, 1H), 9.03 (s, 1H), 8.74 (d, 1H), 8.55 (d, 1H), 7.79-7.70 (m, 1H), 7.50-7.41 (m, 2H), 5.90 (d, 1H), 4.84-4.70 (m, 1H), 4.43-4.34 (m, 1H), 3.56-3.48 (m, 1H), 3.34-3.24 (m, 1H teilweise unter dem Wassersignal), 2.33-2.23 (m, 1H), 1.96-1.61 (m, 3H), 0.98 (t, 3H).
LC-MS (Methode 1): $R_t$ = 0.98 min; 511 $[M+H]^+$.

**Beispiel 294:**

$^1$H-NMR (400 MHz, DMSO-$d_6$): δ [ppm] = 10.13 (d, 1H), 9.03 (s, 1H), 8.74 (d, 1H), 8.55 (d, 1H), 7.79-7.70 (m, 1H), 7.50-7.41 (m, 2H), 5.91 (d, 1H), 4.84-4.70 (m, 1H), 4.43-4.34 (m, 1H), 3.56-3.47 (m, 1H), 3.33-3.24 (m, 1H teilweise unter dem Wassersignal), 2.34-2.23 (m, 1H), 1.96-1.60 (m, 3H), 0.99 (t, 3H).
LC-MS (Methode 1): $R_t$ = 0.98 min; 511 $[M+H]^+$.

**Beispiel 295:**

*N*-[1-Cyclopropyl-2,2,2-trifluorethyl]-1-(2,6-difluorphenyl)-7-[(4*S*)-4-hydroxy-2-oxopyrrolidin-1-yl]-4-oxo-1,4-dihydro-1,8-naphthyridin-3-carbonsäureamid (Diastereomerengemisch)

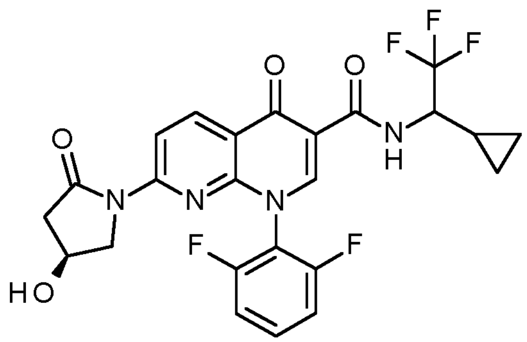

Gemäß AAV2 wurden 100 mg (218 μmol) der Verbindung aus Beispiel 102A mit 22.1 mg (218 μmol) (4*S*)-4-Hydroxypyrolidin-2-on (CAS: 68108-18-9) in Gegenwart von 45.3 mg (328 μmol) Kaliumcarbonat, 8.8 mg (39 μmol) Palladium(II)acetat und 46 mg (79 μmol) Xantphos in 2 ml 1,4-Dioxan umgesetzt. Anschließend wurde das Volumen der Mischung unter reduziertem Druck eingeengt, der Rückstand mit 1N wässriger Salzsäure angesäuert, mit 5 ml Acetonitril verdünnt, filtriert und das Rohprodukt wurde mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 68.9 mg (60% d. Th., 99% Reinheit) der Titelverbindung erhalten.
$^1$H-NMR (400 MHz, DMSO-$d_6$): δ [ppm] = 10.28 (d, 1H), 9.02 (s, 1H), 8.73 (d, 1H), 8.54 (d, 1H), 7.81-7.72 (m, 1H), 7.50-7.42 (m, 2H), 5.33 (d, 1H), 4.47-4.34 (m, 1H), 4.29-4.22 (m, 1H), 3.64 (dd, 1H), 3.43 (d, 1H), 2.93 (dd, 1H), 2.37 (d, 1H), 1.29-1.18 (m, 1H), 0.71-0.51 (m, 3H), 0.40-0.31 (m, 1H).
LC-MS (Methode 1): $R_t$ = 1.05 min; 523 $[M+H]^+$.

65 mg der Titelverbindung (Diastereomerengemisch) wurde durch chirale HPLC in die Diastereomere getrennt (präparative HPLC: Säule Daicel Chiralcel OX-H 250x20 mm; Eluent: 30% Ethanol, 70% Iso-Hexan; Temperatur: 23°C; Fluss: 20 ml/min; UV-Detektion: 220 nm.)

Man erhielt (in der Reihenfolge der Elution von der Säule) 17.3 mg Diastereomer 1 (100% de) $R_t$ = 2.16 min und 17.9 mg (100% de) Diastereomer 2 $R_t$ = 3.39 min.

[Analytische HPLC: Säule Daicel Chiralpak OX-3 3 μm 50x4.6 mm; Eluent: 20% Ethanol, 80% Iso-Hexan; Fluß: 1 ml/min; Temperatur: 30°C; UV-Detektion: 220 nm].

Diastereomer 1 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 16.9 mg (15% d. Th., 99% Reinheit) der Titelverbindung aus Beispiel 296 erhalten.

Diastereomer 2 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 17.7 mg (15% d. Th., 99% Reinheit) der Titelverbindung aus Beispiel 297 erhalten.

## Beispiel 296:

*N*-[(1*R*)-1-cyclopropyl-2,2,2-trifluorethyl]-1-(2,6-difluorphenyl)-7-[(4*S*)-4-hydroxy-2-oxopyrrolidin-1-yl]-4-oxo-1,4-dihydro-1,8-naphthyridin-3-carbonsäureamid (Diastereomer 1)

$^1$H-NMR (400 MHz, DMSO-$d_6$): δ [ppm] = 10.27 (d, 1H), 9.02 (s, 1H), 8.73 (d, 1H), 8.54 (d, 1H), 7.80-7.71 (m, 1H), 7.50-7.42 (m, 2H), 5.33 (d, 1H), 4.46-4.36 (m, 1H), 4.29-4.23 (m, 1H), 3.64 (dd, 1H), 3.43 (d, 1H), 2.93 (dd, 1H), 2.37 (d, 1H), 1.28-1.19 (m, 1H), 0.71-0.52 (m, 3H), 0.40-0.31 (m, 1H).
LC-MS (Methode 3): $R_t$ = 1.84 min; 523 $[M+H]^+$.

Alternativ kann die Titelverbindung auch gemäß AAV2 durch Umsetzung der Verbindung aus Beispiel 103A mit (4*S*)-4-Hydroxypyrolidin-2-on (CAS: 68108-18-9) erhalten werden.

## Beispiel 297:

*N*-[(1*S*)-1-cyclopropyl-2,2,2-trifluorethyl]-1-(2,6-difluorphenyl)-7-[(4*S*)-4-hydroxy-2-oxopyrrolidin-1-yl]-4-oxo-1,4-dihydro-1,8-naphthyridin-3-carbonsäureamid (Diastereomer 2)

$^1$H-NMR (400 MHz, DMSO-$d_6$): δ [ppm] = 10.28 (d, 1H), 9.02 (s, 1H), 8.73 (d, 1H), 8.54 (d, 1H), 7.81-7.71 (m, 1H), 7.50-7.41 (m, 2H), 5.33 (d, 1H), 4.47-4.34 (m, 1H), 4.29-4.22 (m, 1H), 3.64 (dd, 1H), 3.43 (d, 1H), 2.93 (dd, 1H), 2.37 (d, 1H), 1.30-1.18 (m, 1H), 0.71-0.51 (m, 3H), 0.39-0.30 (m, 1H).
LC-MS (Methode 3): $R_t$ = 1.84 min; 523 $[M+H]^+$.

Alternativ kann die Titelverbindung auch gemäß AAV2 durch Umsetzung der Verbindung aus Beispiel 104A mit (4*S*)-4-Hydroxypyrolidin-2-on (CAS: 68108-18-9) erhalten werden.

## Beispiel 298:

*N*-[1-(2-Chlorphenyl)-2,2,2-trifluorethyl]-1-(2,4-difluorphenyl)-7-[(3*S*)-3-hydroxy-2-oxopyrrolidin-1-yl]-4-oxo-1,4-dihydro-1,8-naphthyridin-3-carbonsäureamid (Diastereomerengemisch)

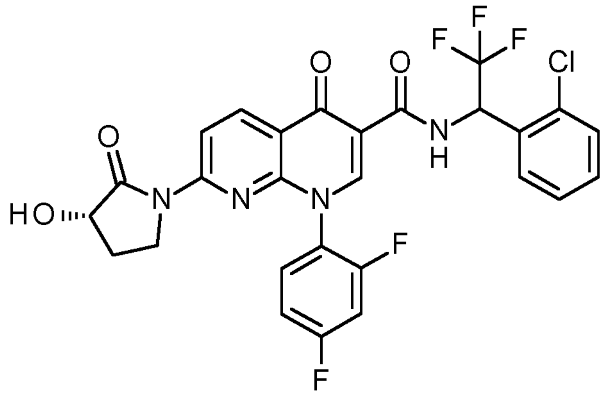

Gemäß AAV2 wurden 439 mg (798 µmol, 96% Reinheit) der Verbindung aus Beispiel 73A mit 99.8 mg (957 µmol) (3*S*)-3-Hydroxypyrolidin-2-on (CAS: 34368-52-0) in Gegenwart von 132 mg (957 µmol) Kaliumcarbonat, 18 mg (80 µmol) Palladium(II)acetat und 92.3 mg (160 µmol) Xantphos in 79 ml 1,4-Dioxan umgesetzt. Anschließend wurde der Ansatz mit 50 ml Wasser versetzt und dreimal mit 30 ml Essigsäureethylester extrahiert. Die wässrige Phase wurde mit 1N wässriger Salzsäure angesäuert und erneut mit Essigsäureethylester extrahiert. Die vereinigten organischen Phasen wurden mit gesättigter wässriger Natriumchlorid-Lösung gewaschen, über Magnesiumsulfat getrocknet, filtriert und mittels Normalphasenchromatographie (Cyclohexan-Essigsäureethylester Gradient) gereinigt. Es wurden 280 mg (59% d. Th., 100% Reinheit) der Titelverbindung erhalten.
$^1$H-NMR (400 MHz, DMSO-$d_6$): δ [ppm] = 11.32 (d, 1H), 8.87 (s, 1H), 8.78 (d, 1H), 8.54 (dd, 1H), 7.92-7.77 (m, 1H), 7.68-7.48 (m, 5H), 7.40-7.31 (m, 1H), 6.53-6.42 (m, 1H), 5.91 (d, 1H), 4.45-4.33 (m, 1H), 3.63-3.50 (m, 1H), 2.36-2.26 (m, 1H), 1.83-1.67 (m, 1H).
LC-MS (Methode 1): $R_t$ = 1.20 min; 593 [M+H]$^+$.

**Beispiel 299:**

*N*-[1-(2-Chlorphenyl)-2,2,2-trifluorethyl]-1-(2,4-difluorphenyl)-7-[(3*R*)-3-hydroxy-2-oxopyrrolidin-1-yl]-4-oxo-1,4-dihydro-1,8-naphthyridin-3-carbonsäureamid (Diastereomerengemisch)

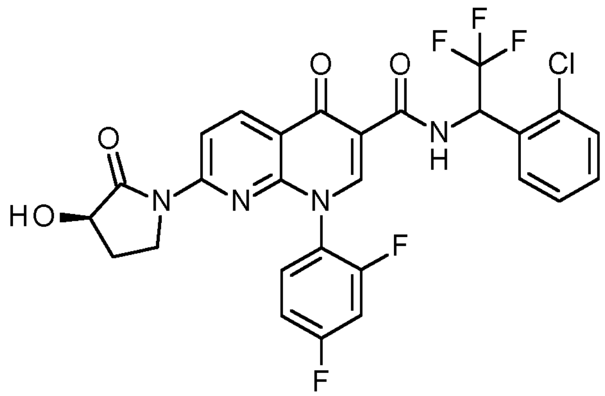

Gemäß AAV2 wurden 606 mg (987 µmol, 86% Reinheit) der Verbindung aus Beispiel 73A mit 123 mg (1.18 mmol) (3*R*)-3-Hydroxypyrolidin-2-on (CAS: 77510-50-0) in Gegenwart von 164 mg (1.18 mmol) Kaliumcarbonat, 22 mg (99 µmol) Palladium(II)acetat und 114 mg (197 µmol) Xantphos in 99 ml 1,4-Dioxan umgesetzt. Nach Reaktion über Nacht wurden weitere 0.1 Äq. Palladium(II)acetat und 0.2 Äq. Xantphos hinzugefügt und für 2.5 h bei 80°C gerührt. Anschließend wurde der Ansatz mit 50 ml Wasser versetzt, mit 1N wässriger Salzsäure angesäuert und dreimal mit 30 ml Essigsäureethylester extrahiert. Die vereinigten organischen Phasen wurden mit gesättigter wässriger Natriumchlorid-Lösung gewaschen, über Magnesiumsulfat getrocknet, filtriert und mittels Normalphasenchromatographie (Cyclohexan-Essigsäureethylester Gradient) gereinigt. Es wurden 284 mg (49% d. Th., 100% Reinheit) der Titelverbindung erhalten.
$^1$H-NMR (400 MHz, DMSO-$d_6$): δ [ppm] = 11.32 (d, 1H), 8.87 (s, 1H), 8.77 (d, 1H), 8.54 (dd, 1H), 7.92-7.76 (m, 1H), 7.67-7.47 (m, 5H), 7.40-7.30 (m, 1H), 6.53-6.41 (m, 1H), 5.91 (d, 1H), 4.45-4.32 (m, 1H), 3.62-3.49 (m, 1H), 2.35-2.27

(m, 1H), 1.84-1.68 (m, 1H).
LC-MS (Methode 4): $R_t$ = 3.69 min; 593 $[M+H]^+$.

**Beispiel 300:**

1-(2,4-Difluorphenyl)-*N*-[1-(2,6-difluorphenyl)-2,2,2-trifluorethyl]-7-[(3*R*)-3-hydroxy-2-oxopyrrolidin-1-yl]-4-oxo-1,4-dihydro-1, 8-naphthyridin-3 -carbonsäureamid (Diastereomerengemisch)

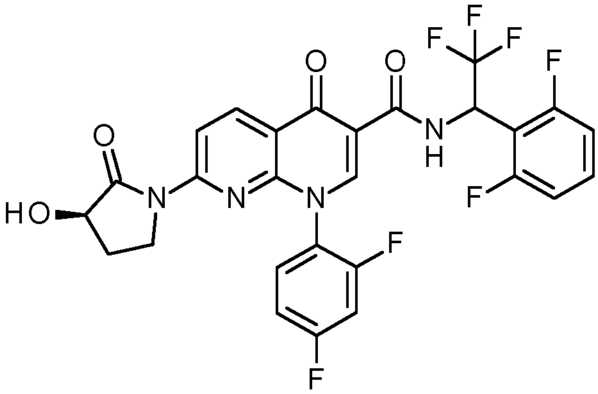

Gemäß AAV2 wurden 428 mg (719 µmol, 89% Reinheit) der Verbindung aus Beispiel 80A mit 89.9 mg (863 µmol) (3*R*)-3-Hydroxypyrolidin-2-on (CAS: 77510-50-0) in Gegenwart von 123 mg (863 µmol) Kaliumcarbonat, 16 mg (72 µmol) Palladium(II)acetat und 83.2 mg (144 µmol) Xantphos in 72 ml 1,4-Dioxan umgesetzt. Anschließend wurde der Ansatz mit 50 ml Wasser versetzt, mit 1N wässriger Salzsäure angesäuert und dreimal mit 30 ml Essigsäureethylester extrahiert. Die vereinigten organischen Phasen wurden mit gesättigter wässriger Natriumchlorid-Lösung gewaschen, über Magnesiumsulfat getrocknet, filtriert und mittels Normalphasenchromatographie (Cyclohexan-Essigsäureethylester Gradient) gereinigt. Es wurden 266 mg (62% d. Th., 100% Reinheit) der Titelverbindung erhalten.
$^1$H-NMR (400 MHz, DMSO-$d_6$): δ [ppm] = 11.36 (d, 1H), 8.89 (s, 1H), 8.77 (d, 1H), 8.56-8.50 (m, 1H), 7.93-7.74 (m, 1H), 7.68-7.57 (m, 2H), 7.40-7.28 (m, 3H), 6.50-6.39 (m, 1H), 5.91 (d, 1H), 4.45-4.32 (m, 1H), 3.62-3.50 (m, 1H), 2.36-2.27 (m, 1H), 1.84-1.70 (m, 1H).
LC-MS (Methode 1): $R_t$ = 1.08 min; 595 $[M+H]^+$.

**Beispiel 301:**

1-(2,4-Difluorphenyl)-7-[4-methyl-2-oxopyrrolidin-1-yl]-4-oxo-*N*-[(2*R*)-1,1,1-trifluorbutan-2-yl]-1,4-dihydro-1,8-naphthyridin-3-carbonsäureamid (Diastereomerengemisch)

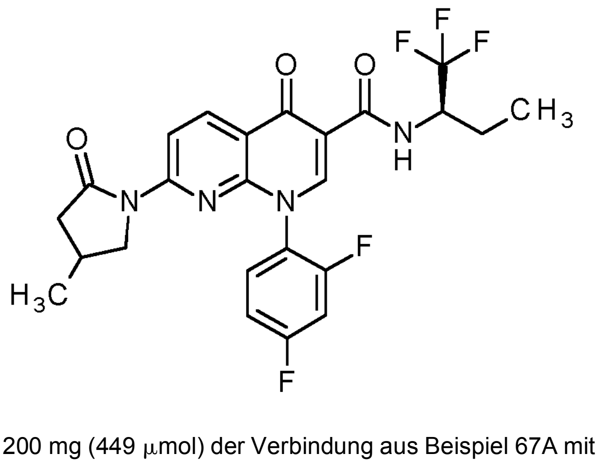

Gemäß AAV2 wurden 200 mg (449 µmol) der Verbindung aus Beispiel 67A mit 49.2 mg (471 µmol) 4-Methyl-2-pyrrolidinon (Racemat) in Gegenwart von 93.0 mg (673 μmol) Kaliumcarbonat, 18 mg (81 μmol) Palladium(II)acetat und 93.5 mg (162 μmol) Xantphos in 4 ml 1,4-Dioxan umgesetzt. Anschließend wurde das Volumen der Mischung unter reduziertem Druck eingeengt, der Rückstand mit 2 ml 1N wässriger Salzsäure und 8 ml Acetonitril aufgenommen und mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 92.9 mg (40% d. Th., 99% Reinheit) der Titelverbindung erhalten.
$^1$H-NMR (400 MHz, DMSO-$d_6$): δ [ppm] = 10.22 (d, 1H), 8.85 (s, 1H), 8.70 (d, 1H), 8.49 (d, 1H), 7.92-7.82 (m, 1H), 7.68-7.59 (m, 1H), 7.40-7.33 (m, 1H), 4.84-4.70 (m, 1H), 3.76-3.65 (m, 1H), 3.18-3.06 (m, 1H), 2.79-2.65 (m, 1H), 2.46-2.23 (m, 2H), 1.95-1.83 (m, 1H), 1.73-1.59 (m, 1H), 1.06-0.94 (m, 6H).
LC-MS (Methode 1): $R_t$ = 1.19 min; 509 [M+H]$^+$.

89 mg der Titelverbindung (Diastereomerengemisch) wurde durch chirale HPLC in die Diastereomere getrennt (präparative HPLC: Säule Daicel Chiralpak AZ-H 5 μm 250x20 mm; Eluent: 25% Ethanol, 75% Iso-Hexan; Temperatur: 25°C; Fluss: 20.2 ml/min; UV-Detektion: 265 nm.)

Man erhielt (in der Reihenfolge der Elution von der Säule) 45.4 mg Diastereomer 1 (100% de) $R_t$ = 3.42 min und 37.1 mg (100% de) Diastereomer 2 $R_t$ = 3.93 min.

[Analytische HPLC: Säule Daicel Chiralpak AZ-3 3 μm 50x4.6 mm; Eluent: 20% Ethanol, 80% Iso-Hexan; Fluß: 1 ml/min; UV-Detektion: 220 nm].

Diastereomer 1 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 33.6 mg (15% d. Th., 99% Reinheit) der Titelverbindung aus Beispiel 302 erhalten.

Diastereomer 2 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 26.9 mg (12% d. Th., 99% Reinheit) der Titelverbindung aus Beispiel 303 erhalten.

**Beispiel 302:**

1-(2,4-Difluorphenyl)-7-[4-methyl-2-oxopyrrolidin-1-yl]-4-oxo-*N*-[(2*R*)-1,1,1-trifluorbutan-2-yl]-1,4-dihydro-1,8-naphthyridin-3-carbonsäureamid (Diastereomer 1)

$^1$H-NMR (400 MHz, DMSO-$d_6$): δ [ppm] = 10.22 (d, 1H), 8.85 (s, 1H), 8.70 (d, 1H), 8.49 (d, 1H), 7.92-7.83 (m, 1H), 7.68-7.59 (m, 1H), 7.41-7.33 (m, 1H), 4.84-4.70 (m, 1H), 3.76-3.65 (m, 1H), 3.18-3.06 (m, 1H), 2.78-2.65 (m, 1H), 2.46-2.23 (m, 2H), 1.96-1.83 (m, 1H), 1.73-1.60 (m, 1H), 1.07-0.94 (m, 6H).
LC-MS (Methode 3): $R_t$ = 2.27 min; 509 [M+H]$^+$.

**Beispiel 303:**

1-(2,4-Difluorphenyl)-7-[4-methyl-2-oxopyrrolidin-1-yl]-4-oxo-*N*-[(2*R*)-1,1,1-trifluorbutan-2-yl]-1,4-dihydro-1,8-naphthyridin-3-carbonsäureamid (Diastereomer 2)

$^1$H-NMR (400 MHz, DMSO-$d_6$): δ [ppm] = 10.22 (d, 1H), 8.85 (s, 1H), 8.70 (d, 1H), 8.49 (d, 1H), 7.92-7.82 (m, 1H), 7.68-7.59 (m, 1H), 7.41-7.32 (m, 1H), 4.83-4.70 (m, 1H), 3.77-3.64 (m, 1H), 3.18-3.07 (m, 1H), 2.78-2.65 (m, 1H), 2.46-2.22 (m, 2H), 1.96-1.84 (m, 1H), 1.73-1.59 (m, 1H), 1.06-0.93 (m, 6H).
LC-MS (Methode 3): $R_t$ = 2.27 min; 509 [M+H]$^+$.

**Beispiel 304:**

1-(2,6-Difluorphenyl)-7-[1-hydroxy-3-azabicyclo[3.1.0]hex-3-yl]-4-oxo-*N*-[(2*R*)-1,1,1-trifluorbutan-2-yl]-1,4-dihydro-1,8-naphthyridin-3-carbonsäureamid (Diastereomerengemisch)

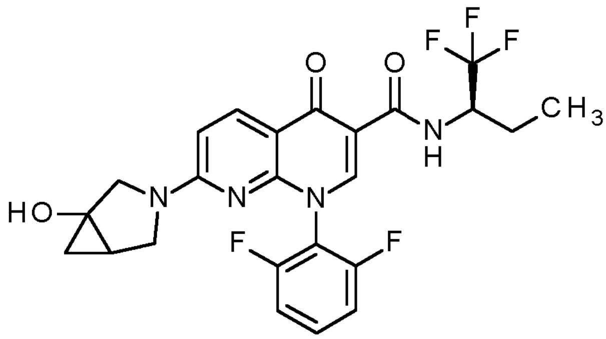

Gemäß AAV3 wurden 240 mg (538 µmol) der Verbindung aus Beispiel 86A mit 80.7 mg (565 µmol) 3-Azabicyclo[3.1.0]hexan-1-ol Hydrochlorid (Racemat, 95% Reinheit) und 328 µl (1.88 mmol) *N*,*N*-Diisopropylethylamin in 2.4 ml Dimethylformamid zur Reaktion gebracht. Das Rohprodukt wurde mit 0.5 ml Acetonitril und 0.5 ml 1N wässriger Salzsäure verdünnt und mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 158.6 mg (57% d. Th., 98.7% Reinheit) der Titelverbindung erhalten.
$^1$H-NMR (400 MHz, DMSO-$d_6$): δ [ppm] = 10.41 (d, 1H), 8.76 (s, 1H), 8.29 (d, 1H), 7.77-7.65 (m, 1H), 7.49-7.37 (m, 2H), 6.83-6.68 (m, 1H), 6.01 (d, 1H), 4.81-4.67 (m, 1H), 3.94-3.83 (m, 0.5H), 3.72-3.60 (m, 0.5H), 3.56-3.39 (m, 1.5H), 3.27-3.18 (m, 0.5H), 3.17-3.02 (m, 1H), 1.94-1.81 (m, 1H), 1.71-1.47 (m, 2H), 1.07-0.92 (m, 4H), 0.48-0.37 (m, 1H).
LC-MS (Methode 3): $R_t$ = 1.99 min; 509 $[M+H]^+$.

150 mg der Titelverbindung (Diastereomerengemisch) wurde durch chirale HPLC in die Diastereomere getrennt (präparative HPLC: Säule Daicel Chiralcel OZ-H 5 µm 250x20 mm; Eluent: 20% Ethanol, 80% Iso-Hexan; Temperatur: 23°C; Fluss: 20 ml/min; UV-Detektion: 220 nm.)

Man erhielt (in der Reihenfolge der Elution von der Säule) 69.0 mg Diastereomer 1 (100% de) $R_t$ = 1.74 min und 50.9 mg (98% de) Diastereomer 2 $R_t$ = 2.48 min.

[Analytische HPLC: Säule Daicel Chiralpak OX-3 3 µm 50x4.6 mm; Eluent: 20% 2-Propanol, 80% Iso-Hexan; Fluß: 1 ml/min; Temperatur: 30°C; UV-Detektion: 220 nm].

Diastereomer 1 wurde zusätzlich mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 48.6 mg (18% d. Th., 99% Reinheit) der Titelverbindung aus Beispiel 305 erhalten.

Diastereomer 2 wurde zusätzlich mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 41.5 mg (15% d. Th., 99% Reinheit) der Titelverbindung aus Beispiel 306 erhalten.

**Beispiel 305:**

1-(2,6-Difluorphenyl)-7-[1-hydroxy-3-azabicyclo[3.1.0]hex-3-yl]-4-oxo-*N*-[(2*R*)-1,1,1-trifluorbutan-2-yl]-1,4-dihydro-1,8-naphthyridin-3-carbonsäureamid (Diastereomer 1)

$^1$H-NMR (400 MHz, DMSO-$d_6$): δ [ppm] = 10.41 (d, 1H), 8.76 (s, 1H), 8.28 (d, 1H), 7.77-7.65 (m, 1H), 7.49-7.36 (m, 2H), 6.84-6.69 (m, 1H), 6.00 (d, 1H), 4.80-4.67 (m, 1H), 3.92-3.82 (m, 0.5H), 3.69-3.60 (m, 0.5H), 3.56-3.39 (m, 1.5H), 3.16-3.02 (m, 1H), 1.94-1.82 (m, 1H), 1.71-1.48 (m, 2H), 1.05-0.90 (m, 4H), 0.47-0.36 (m, 1H).
LC-MS (Methode 3): $R_t$ = 1.96 min; 509 $[M+H]^+$.

**Beispiel 306:**

1-(2,6-Difluorphenyl)-7-[1-hydroxy-3-azabicyclo[3.1.0]hex-3-yl]-4-oxo-*N*-[(2*R*)-1,1,1-trifluorbutan-2-yl]-1,4-dihydro-1,8-naphthyridin-3-carbonsäureamid (Diastereomer 2)

$^1$H-NMR (400 MHz, DMSO-$d_6$): δ [ppm] = 10.41 (d, 1H), 8.76 (s, 1H), 8.28 (d, 1H), 7.77-7.65 (m, 1H), 7.48-7.36 (m, 2H), 6.83-6.68 (m, 1H), 6.00 (d, 1H), 4.80-4.67 (m, 1H), 3.92-3.82 (m, 0.5H), 3.69-3.59 (m, 0.5H), 3.54-3.39 (m, 1.5H), 3.16-3.02 (m, 1H), 1.94-1.81 (m, 1H), 1.71-1.47 (m, 2H), 1.05-0.90 (m, 4H), 0.46-0.38 (m, 1H).
LC-MS (Methode 3): $R_t$ = 1.96 min; 509 $[M+H]^+$.

**Beispiel 307:**

1-(2-Chlor-6-fluorphenyl)-7-[(4*S*)-4-hydroxy-2-oxopyrrolidin-1-yl]-4-oxo-*N*-[(2*R*)-1,1,1-trifluorbutan-2-yl]-1,4-dihydro-1,8-naphthyridin-3-carbonsäureamid

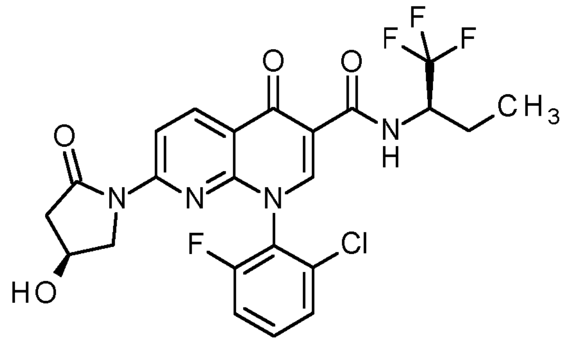

Gemäß AAV2 wurden 100 mg (216 μmol) der Verbindung aus Beispiel 105C mit 21.9 mg (216 μmol) (*S*)-4-Hydroxypyrrolidinon in Gegenwart von 44.8 mg (325 μmol) Kaliumcarbonat, 8.7 mg (39 μmol) Palladium(II)acetat und 45 mg (78 μmol) Xantphos in 1.98 ml 1,4-Dioxan umgesetzt. Anschließend wurde das Volumen der Mischung unter reduziertem Druck eingeengt, der Rückstand mit 1N wässriger Salzsäure angesäuert und 3 ml Acetonitril aufgenommen, filtriert und mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 57.1 mg (50% d. Th., 99% Reinheit) der Titelverbindung erhalten.

$^1$H-NMR (400 MHz, DMSO-$d_6$): δ [ppm] = 10.15 (d, 1H), 8.99 (s, 1H), 8.73 (d, 1H), 8.54 (d, 1H), 7.78-7.56 (m, 3H), 5.32 (d, 1H), 4.82-4.71 (m, 1H), 4.28-4.22 (m, 1H), 3.63-3.55 (m, 1H), 3.41-3.34 (m, 1H), 2.93 (dd, 1H), 2.40-2.31 (m, 1H), 1.95-1.83 (m, 1H), 1.74-1.60 (m, 1H), 1.02-0.95 (m, 3H).

LC-MS (Methode 1): $R_t$ = 1.01 min; 527 $[M+H]^+$.

**Beispiel 308:**

1-(2-Chlor-6-fluorphenyl)-7-[(3*R*)-3-hydroxy-2-oxopyrrolidin-1-yl]-4-oxo-*N*-[(2*R*)-1,1,1-trifluorbutan-2-yl]-1,4-dihydro-1,8-naphthyridin-3-carbonsäureamid

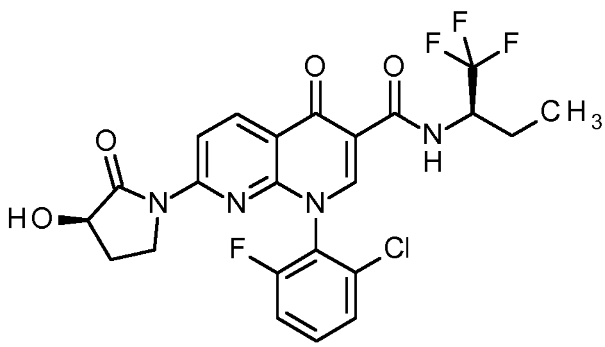

Gemäß AAV2 wurden 100 mg (216 μmol) der Verbindung aus Beispiel 105C mit 21.9 mg (216 μmol) (*R*)-3-Hydroxypyrrolidinon in Gegenwart von 44.8 mg (325 μmol) Kaliumcarbonat, 8.7 mg (39 μmol) Palladium(II)acetat und 45 mg (78 μmol) Xantphos in 1.98 ml 1,4-Dioxan umgesetzt. Anschließend wurde das Volumen der Mischung unter reduziertem Druck eingeengt, der Rückstand mit 1N wässriger Salzsäure angesäuert und 5 ml Acetonitril aufgenommen, filtriert und mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 57.5 mg (58% d. Th., 99% Reinheit) der Titelverbindung erhalten.

$^1$H-NMR (400 MHz, DMSO-$d_6$): δ [ppm] = 10.17-10.11 (m, 1H), 9.00 (s, 1H), 8.75 (d, 1H), 8.54 (d, 1H), 7.76-7.55 (m,

3H), 5.90 (d, 1H), 4.83-4.71 (m, 1H), 4.41-4.33 (m, 1H), 3.51-3.42 (m, 1H), 3.27-3.18 (m, 1H), 2.31-2.22 (m, 1H), 1.96-1.84 (m, 1H), 1.80-1.61 (m, 2H), 1.03-0.94 (m, 1H).
LC-MS (Methode 3): $R_t$ = 1.91 min; 527 $[M+H]^+$.

**Beispiel 309:**

1-(2-Chlor-6-fluorphenyl)-7-[(3*S*)-3-hydroxy-2-oxopyrrolidin-1-yl]-4-oxo-*N*-[(2*R*)-1,1,1-trifluorbutan-2-yl]-1,4-dihydro-1,8-naphthyridin-3-carbonsäureamid

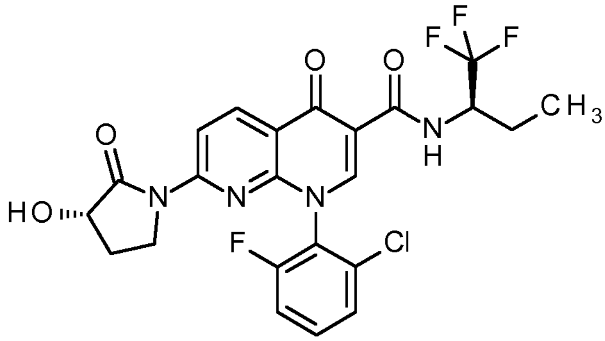

Gemäß AAV2 wurden 100 mg (216 µmol) der Verbindung aus Beispiel 105C mit 21.9 mg (216 µmol) (*S*)-3-Hydroxypyrrolidinon in Gegenwart von 44.8 mg (325 µmol) Kaliumcarbonat, 8.7 mg (39 µmol) Palladium(II)acetat und 45 mg (78 µmol) Xantphos in 1.98 ml 1,4-Dioxan umgesetzt. Anschließend wurde das Volumen der Mischung unter reduziertem Druck eingeengt, der Rückstand mit 1N wässriger Salzsäure angesäuert und 3 ml Acetonitril aufgenommen, filtriert und mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 63.3 mg (55% d. Th., 99% Reinheit) der Titelverbindung erhalten.
$^1$H-NMR (400 MHz, DMSO-$d_6$): δ [ppm] = 10.14 (d, 1H), 9.00 (d, 1H), 8.75 (d, 1H), 8.54 (d, 1H), 7.76-7.54 (m, 3H), 5.90 (d, 1H), 4.83-4.70 (m, 1H), 4.42-4.33 (m, 1H), 3.52-3.41 (m, 1H), 3.28-3.18 (m, 1H), 2.32-2.23 (m, 1H), 1.96-1.84 (m, 1H), 1.80-1.61 (m, 2H), 1.02-0.94 (m, 1H).
LC-MS (Methode 3): $R_t$ = 1.91 min; 527 $[M+H]^+$.

**Beispiel 310:**

1-(2-Chlor-6-fluorphenyl)-7-[1-hydroxy-3-azabicyclo[3.1.0]hex-3-yl]-4-oxo-*N*-[(2*R*)-1,1,1-trifluorbutan-2-yl]-1,4-dihydro-1,8-naphthyridin-3-carbonsäureamid (Diastereomerengemisch)

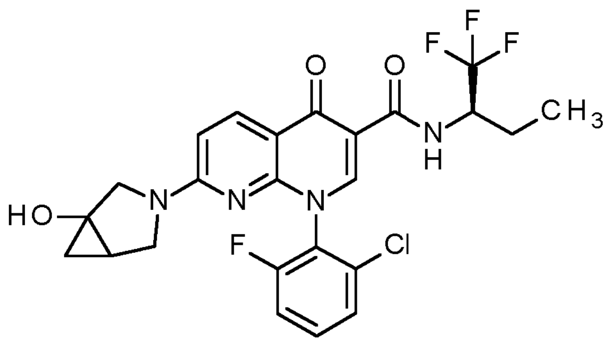

Gemäß AAV3 wurden 100 mg (216 µmol) der Verbindung aus Beispiel 105C mit 35.5 mg (238 µmol) 3-Azabicyclo[3.1.0]hexan-1-ol Hydrochlorid (Racemat, 91% Reinheit) und 132 µl (757 µmol) *N,N*-Diisopropylethylamin in 2 ml Dimethylformamid zur Reaktion gebracht. Das Rohprodukt wurde mit 0.5 ml Acetonitril verdünnt und mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 74.7 mg (66% d. Th., 100% Reinheit) der Titelverbindung erhalten.
$^1$H-NMR (400 MHz, DMSO-$d_6$): δ [ppm] = 10.43 (d, 1H), 8.71 (s, 1H), 8.28 (d, 1H), 7.75-7.50 (m, 3H), 6.83-6.69 (m, 1H), 5.99 (d, 1H), 4.80-4.67 (m, 1H), 3.92-3.82 (m, 0.5H), 3.69-3.60 (m, 0.5H), 3.54-3.37 (m, 1.5H), 3.24-2.97 (m, 1.5H), 1.94-1.82 (m, 1H), 1.72-1.46 (m, 2H), 1.05-0.92 (m, 4H), 0.48-0.35 (m, 1H).
LC-MS (Methode 3): $R_t$ = 2.04 min; 525 $[M+H]^+$.

**Beispiel 311:**

1-(2,6-Difluorphenyl)-7-[1-hydroxy-3-azabicyclo[3.1.0]hex-3-yl]-4-oxo-*N*-[(2*S*)-1,1,1-trifluorpropan-2-yl]-1,4-dihydro-1,8-naphthyridin-3-carbonsäureamid (Diastereomerengemisch)

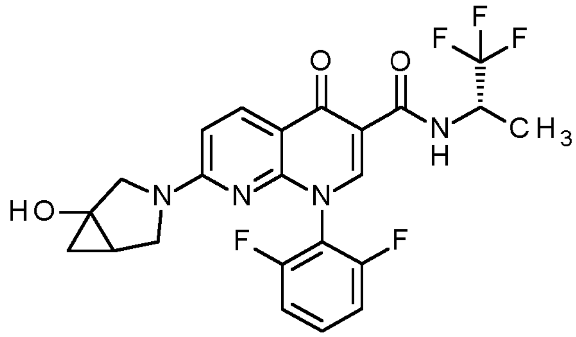

Gemäß AAV3 wurden 100 mg (232 μmol) der Verbindung aus Beispiel 106A mit 37.9 mg (255 μmol) 3-Azabicyclo[3.1.0]hexan-1-ol Hydrochlorid (Racemat, 91% Reinheit) und 141 μl (811 μmol) *N,N*-Diisopropylethylamin in 1 ml Dimethylformamid zur Reaktion gebracht. Das Rohprodukt wurde mit 0.5 ml Acetonitril verdünnt und mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 72.1 mg (63% d. Th., 100% Reinheit) der Titelverbindung erhalten.
$^1$H-NMR (400 MHz, DMSO-$d_6$): δ [ppm] = 10.46 (d, 1H), 8.75 (s, 1H), 8.27 (d, 1H), 7.77-7.65 (m, 1H), 7.48-7.36 (m, 2H), 6.83-6.69 (m, 1H), 6.00 (d, 1H), 4.95-4.81 (m, 1H), 3.91-3.83 (m, 0.5H), 3.69-3.60 (m, 0.5H), 3.55-3.38 (m, 1.5H), 3.26-3.02 (m, 1.5H), 1.69-1.47 (m, 1H), 1.37 (d, 3H), 1.05-0.98 (m, 1H), 0.46-0.39 (m, 1H).
LC-MS (Methode 3): $R_t$ = 1.89 min; 495 $[M+H]^+$.
70 mg der Titelverbindung (Diastereomerengemisch) wurde durch chirale HPLC in die Diastereomere getrennt (präparative HPLC: Säule Daicel Chiralpak AZ-H 5 μm 250x20 mm; Eluent: 15% Ethanol, 85% Iso-Hexan; Temperatur: 25°C; Fluss: 20 ml/min; UV-Detektion: 265 nm.)
Man erhielt (in der Reihenfolge der Elution von der Säule) 34.4 mg Diastereomer 1 (100% de) $R_t$ = 2.29 min und 37.5 mg (100% de) Diastereomer 2 $R_t$ = 2.48 min.
[Analytische HPLC: Säule Daicel Chiralpak AZ-3 3 μm 50x4.6 mm; Eluent: 10% Ethanol, 90% Iso-Hexan; Fluß: 1 ml/min; UV-Detektion: 220 nm].
Diastereomer 1 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 17.3 mg (15% d. Th., 100% Reinheit) der Titelverbindung aus Beispiel 312 erhalten.
Diastereomer 2 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 16.7 mg (15% d. Th., 100% Reinheit) der Titelverbindung aus Beispiel 313 erhalten.

**Beispiel 312:**

1-(2,6-Difluorphenyl)-7-[1-hydroxy-3-azabicyclo[3.1.0]hex-3-yl]-4-oxo-*N*-[(2S)-1,1,1-trifluorpropan-2-yl]-1,4-dihydro-1,8-naphthyridin-3-carbonsäureamid (Diastereomer 1)

$^1$H-NMR (400 MHz, DMSO-$d_6$): δ [ppm] = 10.46 (d, 1H), 8.75 (s, 1H), 8.27 (d, 1H), 7.76-7.65 (m, 1H), 7.48-7.36

(m, 2H), 6.83-6.69 (m, 1H), 6.01 (d, 1H), 4.93-4.83 (m, 1H), 3.91-3.83 (m, 0.5H), 3.69-3.60 (m, 0.5H), 3.54-3.41 (m, 1.5H), 3.25-3.02 (m, 1.5H), 1.68-1.48 (m, 1H), 1.37 (d, 3H), 1.04-0.98 (m, 1H), 0.46-0.39 (m, 1H).
LC-MS (Methode 3): $R_t$ = 1.96 min; 495 $[M+H]^+$.

**Beispiel 313:**

1-(2,6-Difluorphenyl)-7-[1-hydroxy-3-azabicyclo[3.1.0]hex-3-yl]-4-oxo-*N*-[(2S)-1,1,1-trifluorpropan-2-yl]-1,4-dihydro-1,8-naphthyridin-3-carbonsäureamid (Diastereomer 2)

$^1$H-NMR (400 MHz, DMSO-$d_6$): δ [ppm] = 10.46 (d, 1H), 8.75 (s, 1H), 8.27 (d, 1H), 7.76-7.65 (m, 1H), 7.47-7.37 (m, 2H), 6.81-6.69 (m, 1H), 6.00 (d, 1H), 4.93-4.83 (m, 1H), 3.91-3.84 (m, 0.5H), 3.68-3.60 (m, 0.5H), 3.53-3.41 (m, 1.5H), 3.25-3.04 (m, 1.5H), 1.68-1.48 (m, 1H), 1.37 (d, 3H), 1.04-0.99 (m, 1H), 0.47-0.38 (m, 1H).
LC-MS (Methode 3): $R_t$ = 1.88 min; 495 $[M+H]^+$.

**Beispiel 314:**

1-(2,4-Difluorphenyl)-7-[1-hydroxy-3-azabicyclo[3.1.0]hex-3-yl]-4-oxo-*N*-[(2*R*)-1,1,1-trifluorbutan-2-yl]-1,4-dihydro-1,8-naphthyridin-3-carbonsäureamid (Diastereomer 1)

$^1$H NMR (400 MHz, DMSO-$d_6$): δ [ppm] = 10.48 (d, 1H), 8.62 (s, 1H), 8.28 (d, 1H), 7.85-7.75 (m, 1H), 7.65-7.53 (m, 1H), 7.38-7.28 (m, 1H), 6.81-6.66 (m, 1H), 6.00 (d, 1H), 4.80-4.67 (m, 1H), 3.94-3.04 (m, 4H teilweise unter dem Wassersignal), 1.93-1.82 (m, 1H), 1.69-1.51 (m, 2H), 1.07-0.92 (m, 4H), 0.48-0.35 (m, 1H).
LC-MS (Methode 3): $R_t$ = 1.99 min; 509 $[M+H]^+$.

**Beispiel 315:**

1-(2,4-Difluorphenyl)-7-[1-hydroxy-3-azabicyclo[3.1.0]hex-3-yl]-4-oxo-*N*-[(2*R*)-1,1,1-trifluorbutan-2-yl]-1,4-dihydro-1,8-naphthyridin-3-carbonsäureamid (Diastereomer 2)

$^1$H NMR (400 MHz, DMSO-$d_6$): δ [ppm] = 10.48 (d, 1H), 8.62 (s, 1H), 8.28 (d, 1H), 7.86-7.74 (m, 1H), 7.66-7.52 (m, 1H), 7.38-7.27 (m, 1H), 6.81-6.67 (m, 1H), 6.00 (d, 1H), 4.80-4.67 (m, 1H), 3.93-3.06 (m, 4H teilweise unter dem Wassersignal), 1.93-1.81 (m, 1H), 1.71-1.50 (m, 2H), 1.06-0.91 (m, 4H), 0.47-0.35 (m, 1H).
LC-MS (Methode 3): $R_t$ = 1.99 min; 509 $[M+H]^+$.

**Beispiel 316:**

7-[1-Hydroxy-3-azabicyclo[3.1.0]hex-3-yl]-4-oxo-1-(2,4,6-trifluorphenyl)-*N*-[(2*S*)-1,1,1-trifluorpropan-2-yl]-1,4-dihydro-1,8-naphthyridin-3-carbonsäureamid (Diastereomerengemisch)

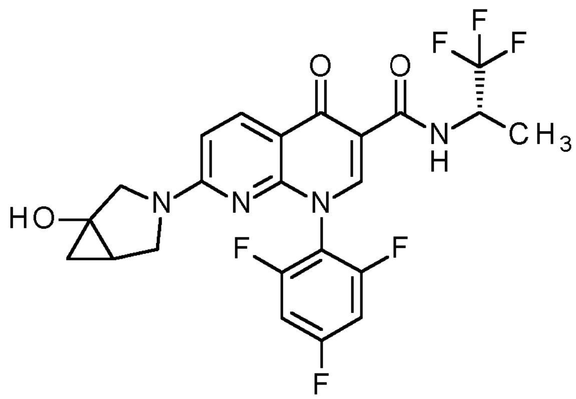

Gemäß AAV3 wurden 150 mg (334 µmol) der Verbindung aus Beispiel 107A mit 54.7 mg (367 µmol) 3-Azabicyclo[3.1.0]hexan-1-ol Hydrochlorid (Racemat, 91% Reinheit) und 203 µl (1.17 mmol) *N,N*-Diisopropylethylamin in 1.5 ml Dimethylformamid zur Reaktion gebracht. Das Rohprodukt wurde mit 0.5 ml Acetonitril verdünnt und mittels

präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 123.8 mg (72% d. Th., 99% Reinheit) der Titelverbindung erhalten.
$^1$H-NMR (400 MHz, DMSO-$d_6$): δ [ppm] = 10.45 (d, 1H), 8.81 (s, 1H), 8.27 (d, 1H), 7.64-7.50 (m, 2H), 6.83-6.68 (m, 1H), 6.02 (d, 1H), 4.95-4.82 (m, 1H), 3.92-3.84 (m, 0.5H), 3.69-3.39 (m, 2H), 3.19-3.08 (m, 1H), 1.69-1.50 (m, 1H), 1.37 (d, 3H), 1.06-0.98 (m, 1H), 0.48-0.39 (m, 1H).
LC-MS (Methode 1): $R_t$ = 1.01 min; 513 [M+H]$^+$.

120 mg der Titelverbindung (Diastereomerengemisch) wurde durch chirale SFC in die Diastereomere getrennt (präparative SFC: Säule Daicel Chiralpak IA 5 μm 250x20 mm; Eluent: 7% Ethanol, 93% Kohlendioxid; Temperatur: 40°C; Fluss: 100 ml/min; UV-Detektion: 210 nm.)

Man erhielt (in der Reihenfolge der Elution von der Säule) 42.0 mg Diastereomer 1 (100% de) $R_t$ = 1.48 min und 47.2 mg (87% de) Diastereomer 2 $R_t$ = 1.57 min.

[Analytische SFC: Säule Daicel Chiralpak IA; Eluent: 10% Ethanol, 90% Kohlendioxid; Fluß: 3 ml/min; UV-Detektion: 220 nm].

Diastereomer 1 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 26.9 mg (16% d. Th., 100% Reinheit) der Titelverbindung aus Beispiel 317 erhalten.

Diastereomer 2 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 38.8 mg (22% d. Th., 100% Reinheit) der Titelverbindung aus Beispiel 318 erhalten.

## Beispiel 317:

7-[1-Hydroxy-3-azabicyclo[3.1.0]hex-3-yl]-4-oxo-1-(2,4,6-trifluorphenyl)-*N*-[(2*S*)-1,1,1-trifluorpropan-2-yl]-1,4-dihydro-1,8-naphthyridin-3-carbonsäureamid (Diastereomer 1)

$^1$H-NMR (400 MHz, DMSO-$d_6$): δ [ppm] = 10.45 (d, 1H), 8.81 (s, 1H), 8.27 (d, 1H), 7.63-7.51 (m, 2H), 6.83-6.68 (m, 1H), 6.01 (d, 1H), 4.95-4.81 (m, 1H), 3.92-3.84 (m, 0.5H), 3.70-3.40 (m, 2H), 3.20-3.08 (m, 1H), 1.69-1.51 (m, 1H), 1.37 (d, 3H), 1.06-0.98 (m, 1H), 0.46-0.39 (m, 1H).
LC-MS (Methode 3): $R_t$ = 1.92 min; 513 [M+H]$^+$.

## Beispiel 318:

7-[1-Hydroxy-3-azabicyclo[3.1.0]hex-3-yl]-4-oxo-1-(2,4,6-trifluorphenyl)-*N*-[(2*S*)-1,1,1-trifluorpropan-2-yl]-1,4-dihydro-1,8-naphthyridin-3-carbonsäureamid (Diastereomer 2)

$^1$H-NMR (400 MHz, DMSO-$d_6$): δ [ppm] = 10.45 (d, 1H), 8.81 (s, 1H), 8.27 (d, 1H), 7.64-7.50 (m, 2H), 6.82-6.69 (m, 1H), 6.02 (d, 1H), 4.94-4.83 (m, 1H), 3.92-3.83 (m, 0.5H), 3.69-3.40 (m, 2H), 3.20-3.09 (m, 1H), 1.69-1.50 (m, 1H), 1.37 (d, 3H), 1.06-0.99 (m, 1H), 0.49-0.39 (m, 1H).
LC-MS (Methode 3): $R_t$ = 1.92 min; 513 [M+H]$^+$.

## Beispiel 319:

*N*-[(1*R*)-1-Cyclopropyl-2,2,2-trifluorethyl]-1-(2,6-difluorphenyl)-7-[1-hydroxy-3-azabicyclo[3.1,0]hex-3-yl]-4-oxo-1,4-dihydro-1,8-naphthyridin-3-carbonsäureamid (Diastereomerengemisch)

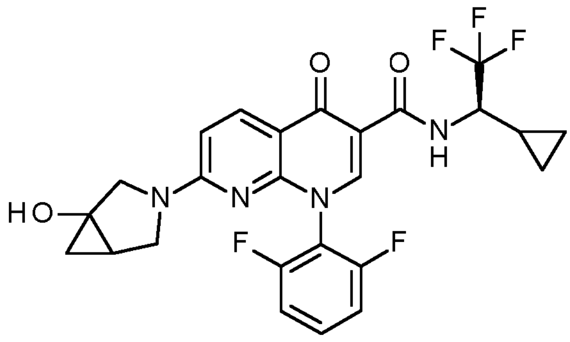

Gemäß AAV3 wurden 150 mg (328 μmol) der Verbindung aus Beispiel 103A mit 53.7 mg (360 μmol) 3-Azabicyclo[3.1.0]hexan-1-ol Hydrochlorid (Racemat, 91% Reinheit) und 200 μl (1.15 mmol) *N,N*-Diisopropylethylamin in 1.5 ml Dimethylformamid zur Reaktion gebracht. Das Rohprodukt wurde mit 5 ml Acetonitril und 0.5 ml 1N wässriger Salzsäure verdünnt, filtriert und mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 119.3 mg (69% d. Th., 98.4% Reinheit) der Titelverbindung erhalten.
$^1$H-NMR (400 MHz, DMSO-$d_6$): δ [ppm] = 10.54 (d, 1H), 8.75 (s, 1H), 8.29 (d, 1H), 7.77-7.65 (m, 1H), 7.47-7.35 (m, 2H), 6.83-6.69 (m, 1H), 6.00 (d, 1H), 4.43-4.31 (m, 1H), 3.92-3.82 (m, 0.5H), 3.70-3.59 (m, 0.5H), 3.55-3.39 (m, 1.5H), 3.27-3.18 (m, 0.5H), 3.16-3.03 (m, 1H), 1.69-1.48 (m, 1H), 1.26-1.14 (m, 1H), 1.05-0.97 (m, 1H), 0.70-0.48 (m, 3H), 0.47-0.39 (m, 1H), 0.37-0.30 (m, 1H).
LC-MS (Methode 3): $R_t$ = 2.01 min; 521 [M+H]$^+$.

**Beispiel 320:**

1-(2,4-Difluorphenyl)-4-oxo-7-(2-oxoimidazolidin-1-yl)-*N*-[(2*R*)-1,1,1-trifluorbutan-2-yl]-1,4-dihydro-1,8-naphthyridin-3-carbonsäureamid

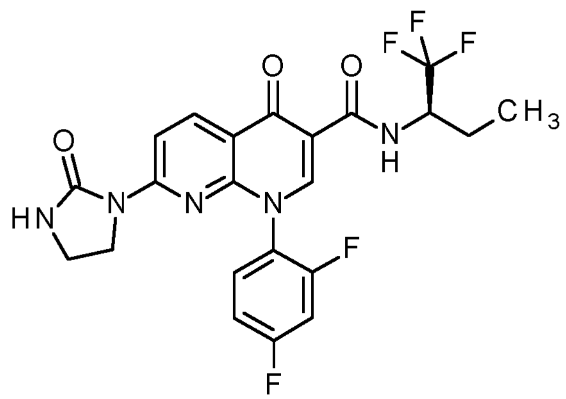

Gemäß AAV2 wurden 300 mg (673 μmol) der Verbindung aus Beispiel 67A mit 64.0 mg (707 μmol) Imidazolidinon in Gegenwart von 140 mg (1.01 mmol) Kaliumcarbonat, 27.2 mg (121 μmol) Palladium(II)acetat und 140 mg (242 μmol) Xantphos in 6 ml 1,4-Dioxan umgesetzt. Anschließend wurde der Ansatz mit 10 ml Essigsäureethylester versetzt, mit 1N wässriger Salzsäure gewaschen und zur Trockne einrotiert. Der Rückstand wurde in 10 ml THF aufgenommen, 250 mg N-Acetylcystein zugegeben und für 30 min bei Raumtemperatur gerührt. Es wurde mit 30 ml Essigsäureethylester verdünnt und mit gesättigter wässriger Natriumhydrogencarbonat-Lösung gewaschen, über Natriumsulfat getrocknet, filtriert und das Lösungsmittel unter reduziertem Druck entfernt. Der Rückstand wurde in 6 ml Acetonitril und 1 ml Wasser verrührt, der Niederschlag abgesaugt und die Mutterlauge mittels präparativer HPLC (Säule: Chromatorex C18, 10 μm, 125 x 30 mm, Lösungsmittel: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 20.2 mg (5.9% d. Th., 97.1% Reinheit) der Titelverbindung erhalten.
$^1$H-NMR(400 MHz, DMSO-$d_6$): δ [ppm] = 10.31 (d, 1H), 8.78 (s, 1H), 8.56 (d, 1H), 8.42 (d, 1H), 7.91-7.82 (m, 1H), 7.66-7.55 (m, 2H), 7.38-7.31 (m, 1H), 4.80-4.72 (m, 1H), 3.65-3.50 (m, 2H), 3.39-3.33 (m, 2H teilweise unter dem

Wassersignal), 1.95-1.82 (m, 1H), 1.72-1.59 (m, 1H), 0.98 (t, 3H).
LC-MS (Methode 3): $R_t$ = 1.89 min; 496 $[M+H]^+$.

**Beispiel 321:**

1-(2,4-Difluorphenyl)-7-(3-methyl-2-oxoimidazolidin-1-yl)-4-oxo-*N*-[(2*R*)-1,1,1-trifluorbutan-2-yl]-1,4-dihydro-1,8-naphthyridin-3 -carbonsäureamid

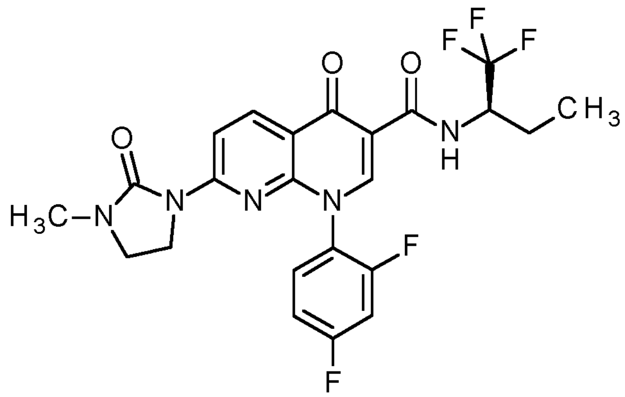

Zu einer Lösung von 20 mg (40 µmol) der Verbindung aus Beispiel 320 in 1 ml 1,2-Dimethoxyethan wurde unter Eisbadkühlung 2.4 mg (61 µmol, 60% in Mineralöl) Natriumhydrid gegeben und für 30 min nachgerührt. Es wurde auf Raumtemperatur erwärmt, 5.0 µl (81 µmol) Iodmethan hinzugegeben und über Nacht bei 80°C gerührt. Anschließend wurde das Volumen der Mischung unter reduziertem Druck eingeengt, der Rückstand in 3 ml Acetonitril, 0.5 ml Wasser und 1 ml DMSO aufgenommen und mittels präparativer HPLC (Säule: Chromatorex C18, 10 µm, 125 x 30 mm, Lösungsmittel: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 5.4 mg (26% d. Th., 99% Reinheit) der Titelverbindung erhalten.
$^1$H-NMR (400 MHz, DMSO-$d_6$): δ [ppm] = 10.31 (d, 1H), 8.78 (s, 1H), 8.57 (d, 1H), 8.43 (d, 1H), 7.91-7.81 (m, 1H), 7.63-7.56 (m, 1H), 7.38-7.31 (m, 1H), 4.81-4.71 (m, 1H), 3.57-3.36 (m, 4H), 2.79 (s, 3H), 1.94-1.84 (m, 1H), 1.72-1.59 (m, 1H), 0.98 (t, 3H).
LC-MS (Methode 1): $R_t$ = 1.16 min; 510 $[M+H]^+$.

**Beispiel 322:**

1-(2-Chlor-4,6-difluorphenyl)-7-[(3*R*,4*R*)-3,4-dihydroxypyrrolidin-1-yl]-4-oxo-*N*-[(2*S*)-1-(trifluormethoxy)propan-2-yl]-1,4-dihydro-1,8-naphthyridin-3-carbonsäureamid

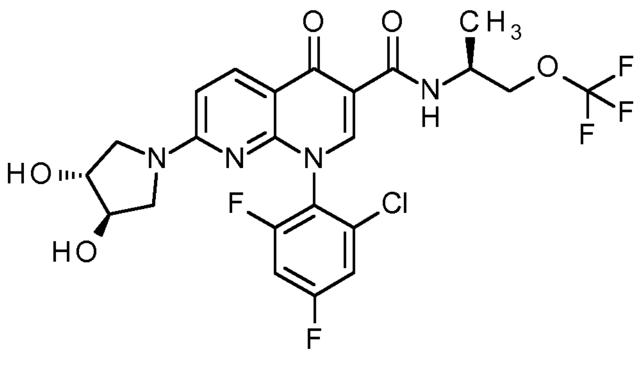

Gemäß AAV3 wurden 51.7 mg (104 µmol) der Verbindung aus Beispiel 112A mit 16.0 mg (115 µmol) (3*R*,4*R*)-Pyrrolidin-3,4-diol Hydrochlorid und 63.5 µl (365 µmol) *N,N*-Diisopropylethylamin in 1 ml Dimethylformamid zur

Reaktion gebracht. Das Rohprodukt wurde mit 2 ml Acetonitril und 1N wässriger Salzsäure angesäuert und mittels präparativer HPLC (Säule: Chromatorex C18, 10 μm, 125 x 30 mm, Lösungsmittel: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 48 mg (82% d. Th., 100% Reinheit) der Titelverbindung erhalten.
$^1$H-NMR (400 MHz, DMSO-$d_6$): δ [ppm] = 10.14-10.09 (m, 1H), 8.66 (s, 1H), 8.27 (d, 1H), 7.75-7.65 (m, 2H), 6.75 (d, 1H), 5.24-5.20 (m, 1H), 5.15-5.11 (m, 1H), 4.38-4.29 (m, 1H), 4.21-4.13 (m, 2H), 4.06-4.02 (m, 1H), 3.93-3.88 (m, 1H), 3.64-3.57 (m, 1H), 3.24-3.16 (m, 1H), 3.06-2.98 (m, 1H), 1.28-1.21 (m, 3H).
LC-MS (Methode 3): $R_t$ = 1.68 min; m/z = 563 $[M+H]^+$.

**Beispiel 323:**

*N*-[(1*S*)-1-Cyclopropyl-2,2,2-trifluorethyl]-4-oxo-7-(2-oxoimidazolidin-1-yl)-1-(2,4,6-trifluorphenyl)-1,4-dihydro-1,8-naphthyridin-3 -carbonsäureamid

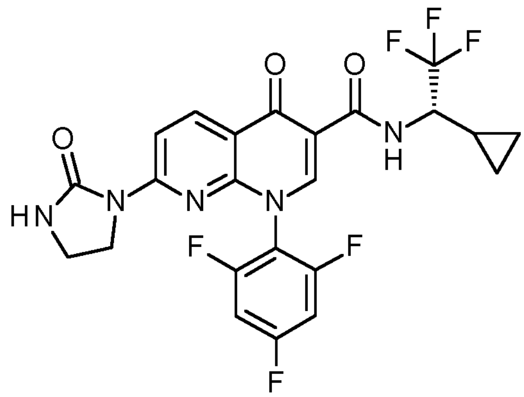

Gemäß AAV1 wurden 15.0 g (37.1 mmol) der Verbindung aus Beispiel 113A mit 7.82 g (44.5 mmol) (1*S*)-1-Cyclopropyl-2,2,2-trifluorethanamin Hydrochlorid in Gegenwart von 16.9 g (44.5 mmol) HATU und 16.2 ml (92.8 mmol) *N,N*-Diisopropylethylamin in 400 ml Dimethylformamid zur Reaktion gebracht. Nach vollständigem Umsatz wurde die Reaktioslösung in Wasser eingerührt und auf pH 3 eingestellt. Es wurde mit Essigsäureethylester extrahiert und die Phasen getrennt. Die organische Phase wurde mit Wasser gewaschen, über Natriumsulfat getrocknet, filtriert und eingeengt. Der Rückstand wurde in Acetonitril verrührt, nach 1h abgesaugt, gewaschen und im Hochvakuum getrocknet. Es wurden 9.3 g (48% d. Th., 99% Reinheit) der Titelverbindung erhalten.
$^1$H-NMR (400 MHz, DMSO-$d_6$): δ [ppm] = 10.36 (d, 1H), 8.99 (s, 1H), 8.57 (d, 1H), 8.44 (d, 1H), 7.67 (s, 1H), 7.62-7.53 (m, 2H), 4.47-4.34 (m, 1H), 3.64-3.56 (m, 2H), 3.39-3.32 (m, 2H), 1.28-1.17 (m, 1H), 0.71-0.50 (m, 3H), 0.39-0.31 (m, 1H).
LC-MS (Methode 3): $R_t$ = 1.94 min; m/z = 526 $[M+H]^+$.

**Beispiel 324:**

7-[3-Hydroxy-3-methylpyrrolidin-1-yl]-4-oxo-*N*-[(2*R*)-1,1,1-trifluorbutan-2-yl]-1-(2,4,6-trifluorphenyl)-1,4-dihydro-1,8-naphthyridin-3-carbonsäureamid (Diastereomerengemisch)

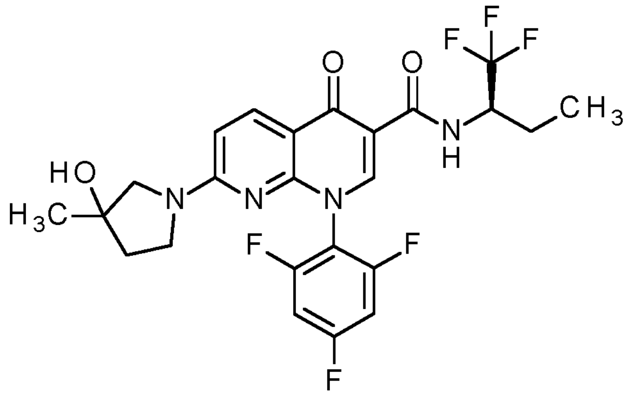

Gemäß AAV3 wurden 100 mg (216 µmol) der Verbindung aus Beispiel 100C und 39.6 mg (90 % Reinheit, 259 µmol) 3-Methylpyrrolidin-3-ol Hydrochlorid in Gegenwart von 130 µl (750 µmol) N,N-Diisopropylethylamin in 1.0 ml DMF zur Reaktion gebracht. Die Reaktionsmischung wurde mit 0.5 ml Acetonitril verdünnt und mittels präparativer HPLC (Säule: Chromatorex C18, 10 µm, 125 x 30 mm, Lösungsmittel: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 93.5 mg (81% d. Th., 99% Reinheit) der Titelverbindung erhalten.

$^1$H-NMR (400 MHz, DMSO-d6) δ [ppm]: -0.008 (2.44), 0.008 (1.51), 0.952 (7.58), 0.970 (16.00), 0.988 (7.79), 1.249 (9.47), 1.323 (10.19), 1.602 (1.14), 1.621 (1.57), 1.627 (1.40), 1.637 (1.85), 1.646 (1.66), 1.655 (1.60), 1.663 (1.71), 1.681 (1.29), 1.795 (2.67), 1.814 (1.90), 1.832 (0.86), 1.850 (1.62), 1.859 (1.90), 1.868 (1.90), 1.878 (2.33), 1.885 (2.43), 1.895 (2.50), 1.904 (2.87), 1.913 (3.39), 1.929 (1.83), 2.941 (1.33), 2.971 (1.75), 3.119 (1.67), 3.150 (1.26), 3.223 (2.27), 3.239 (1.69), 3.288 (2.21), 3.341 (2.64), 3.364 (2.32), 3.391 (0.81), 3.548 (2.35), 3.566 (1.53), 4.732 (1.56), 4.752 (1.44), 4.820 (3.48), 4.893 (3.76), 6.703 (2.03), 6.725 (2.13), 6.762 (1.95), 6.785 (1.84), 7.533 (1.97), 7.554 (3.99), 7.574 (3.36), 8.247 (2.36), 8.270 (4.17), 8.293 (1.97), 8.792 (5.29), 8.799 (5.47), 10.424 (5.03), 10.448 (4.79).

LC-MS Methode 3): $R_t$ = 2.06 min; MS (ESIpos): m/z = 529 [M+H]+

## Beispiel 325:

7-[(4*S*)-4-Hydroxy-2-oxopyrrolidin-1-yl]-4-oxo-*N*-[(2*R*)-1,1,1-trifluorbutan-2-yl]-1-(2,4,6-trifluorphenyl)-1,4-dihydro-1,8-naphthyridin-3-carbonsäureamid

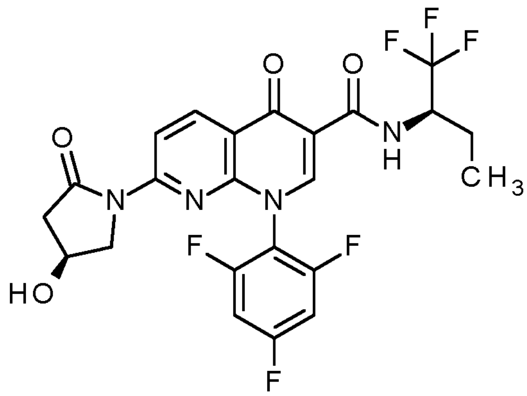

Gemäß AAV2 wurden 100 mg (216 µmol) der Verbindung aus Beispiel 100C mit 26.2 mg (259 µmol) (4*S*)-4-Hydroxypyrrolidin-2-on in Gegenwart von 44.7 mg (323 µmol) Kaliumcarbonat, 8.71 mg (38.8 µmol) Palladium(II)acetat und 44.9 mg (77.6 µmol) Xantphos in 2.0 ml 1,4-Dioxan umgesetzt. Anschließend wurde die Reaktionsmischung eingeengt. Es wurde mit 1N wässriger Salzsäure angesäuert und mit 3 ml Acetonitril versetzt und abfiltriert. Das Filtrat wurde mittels präparativer HPLC (Säule: Chromatorex C18, 10 µm, 125 x 30 mm, Lösungsmittel: Acetonitril / 0.05% Ameisensäure-Gradient; 0 bis 3 min. 10% Acetonitril bis 35 min. 90% Acetonitril und weitere 3 min. 90% Acetonitril) gereinigt und 87.9 mg (76% d. Th., 99% Reinheit) der Titelverbindung erhalten

$^1$H-NMR (400 MHz, DMSO-d6) δ [ppm]: -0.008 (3.32), 0.008 (3.18), 0.965 (7.20), 0.984 (16.00), 1.002 (7.81), 1.632

(1.07), 1.649 (1.52), 1.666 (1.70), 1.675 (1.50), 1.692 (1.78), 1.710 (1.36), 1.866 (1.31), 1.877 (1.46), 1.884 (1.48), 1.895 (1.68), 2.355 (3.56), 2.399 (4.11), 2.711 (0.71), 2.916 (3.44), 2.931 (3.68), 2.959 (3.12), 2.974 (3.03), 3.287 (4.13), 3.463 (3.72), 3.493 (4.43), 3.673 (3.24), 3.686 (3.90), 3.703 (2.95), 3.715 (2.59), 4.290 (2.79), 4.763 (1.42), 5.331 (7.89), 5.340 (7.81), 7.595 (2.27), 7.602 (2.65), 7.617 (4.31), 7.625 (4.51), 7.639 (2.65), 7.645 (2.27), 8.532 (10.26), 8.554 (12.42), 8.707 (12.88), 8.729 (9.97), 9.072 (15.90), 10.103 (5.12), 10.127 (4.94).
LC-MS (Methode 3): Rt = 1.90 min; MS (ESIpos): m/z = 529 [M+H]+

**Beispiel 326:**

*N*-[1-Cyclopropyl-2,2,2-trifluorethyl]-1-(2,6-difluorphenyl)-7-[3-hydroxy-3-methylpyrrolidin-1-yl]-4-oxo-1,4-dihydro-1,8-naphthyridin-3-carbonsäureamid (racemisches Diastereomerengemisch)

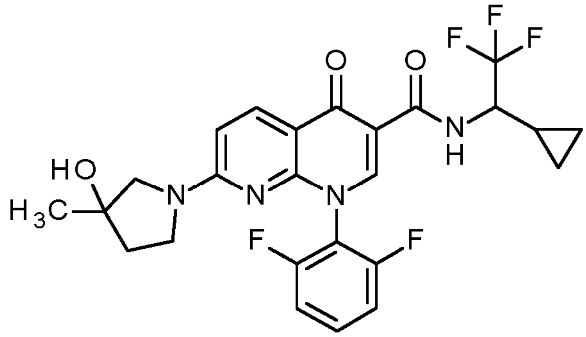

Gemäß AAV3 wurden 150 mg (328 μmol) der Verbindung aus Beispiel 102A und 60.1 mg (90 % Reinheit, 393 μmol) 3-Methylpyrrolidin-3-ol Hydrochlorid in Gegenwart von 200 μl (1.15 mmol) N,N-Diisopropylethylamin in 1.5 ml DMF zur Reaktion gebracht. Die Reaktionsmischung wurde mit 0.5 ml Acetonitril verdünnt und mittels präparativer HPLC (Säule: Chromatorex C18, 10 μm, 125 x 30 mm, Lösungsmittel: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 147 mg (85% d. Th., 99% Reinheit) der Titelverbindung erhalten.
$^1$H-NMR (400 MHz, DMSO-d6) δ [ppm]: -0.008 (2.22), 0.008 (2.01), 0.325 (2.15), 0.336 (3.45), 0.349 (3.49), 0.361 (2.61), 0.373 (1.29), 0.509 (2.38), 0.520 (3.47), 0.532 (3.03), 0.547 (2.85), 0.556 (2.52), 0.568 (3.35), 0.578 (2.96), 0.589 (2.71), 0.599 (2.27), 0.612 (1.41), 0.626 (1.71), 0.637 (1.90), 0.648 (3.05), 0.658 (2.82), 0.663 (2.80), 0.671 (2.75), 0.683 (1.22), 0.693 (0.88), 1.166 (0.72), 1.179 (1.48), 1.187 (2.19), 1.199 (3.96), 1.220 (16.00), 1.240 (2.11), 1.314 (13.09), 1.769 (3.67), 1.786 (2.27), 1.900 (3.26), 1.917 (2.19), 2.891 (1.81), 2.922 (2.43), 3.079 (2.24), 3.107 (1.74), 3.169 (3.01), 3.186 (2.19), 3.289 (3.10), 3.335 (3.65), 3.357 (3.14), 3.385 (1.15), 3.542 (3.08), 4.353 (1.85), 4.374 (3.17), 4.396 (3.12), 4.416 (1.64), 4.806 (4.85), 4.886 (4.93), 6.703 (2.56), 6.724 (2.64), 6.760 (2.57), 6.783 (2.48), 7.386 (4.04), 7.408 (8.42), 7.431 (4.69), 7.694 (2.63), 7.714 (2.48), 7.729 (1.34), 8.258 (2.85), 8.280 (5.59), 8.303 (2.71), 8.724 (5.94), 8.736 (6.08), 10.574 (7.24), 10.598 (6.96).
LC-MS (Methode 1): Rt = 1.14 min; MS (ESIpos): m/z = 523 [M+H]+

**Beispiel 327:**

1-(2,6-Difluorphenyl)-7-[3-hydroxy-3-methylpyrrolidin-1-yl]-4-oxo-*N*-[(2*S*)-1,1,1-trifluorbutan-2-yl]-1,4-dihydro-1,8-naphthyridin-3-carbonsäureamid (Diastereomerengemisch)

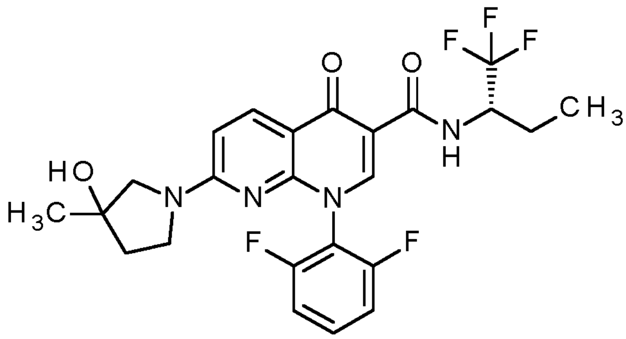

Gemäß AAV3 wurden 150 mg (336 μmol) der Verbindung aus Beispiel 114A und 61.7 mg (90% Reinheit, 404 μmol) 3-Methylpyrrolidin-3-ol Hydrochlorid in Gegenwart von 205 μl (1.18 mmol) *N,N*-Diisopropylethylamin in 1.6 ml DMF zur Reaktion gebracht. Die Reaktionsmischung wurde mit 0.5 ml Acetonitril verdünnt und mittels präparativer HPLC (Säule: Chromatorex C18, 10 μm, 125 x 30 mm, Lösungsmittel: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 148 mg (85% d. Th., 99% Reinheit) der Titelverbindung erhalten.

$^1$H-NMR (400 MHz, DMSO-d6) δ [ppm]: -0.008 (1.74), 0.008 (1.59), 0.953 (7.22), 0.972 (16.00), 0.990 (7.86), 1.221 (9.41), 1.314 (9.55), 1.604 (1.11), 1.622 (1.51), 1.629 (1.32), 1.639 (1.80), 1.647 (1.62), 1.657 (1.53), 1.664 (1.74), 1.683 (1.31), 1.752 (1.35), 1.769 (2.70), 1.786 (1.71), 1.842 (0.74), 1.851 (1.53), 1.861 (1.80), 1.870 (2.02), 1.879 (2.75), 1.886 (2.65), 1.897 (3.47), 1.904 (3.28), 1.915 (2.43), 2.074 (2.15), 2.892 (1.28), 2.922 (1.76), 3.078 (1.63), 3.108 (1.27), 3.171 (2.19), 3.187 (1.57), 3.289 (2.34), 3.335 (2.76), 3.357 (2.27), 3.384 (0.79), 3.541 (2.31), 3.559 (1.53), 4.706 (0.81), 4.730 (1.48), 4.750 (1.39), 4.765 (0.81), 4.806 (3.46), 4.885 (3.51), 6.701 (1.85), 6.723 (1.98), 6.759 (1.91), 6.782 (1.87), 7.389 (2.83), 7.411 (5.95), 7.433 (3.33), 7.680 (1.06), 7.697 (1.88), 7.714 (1.77), 7.734 (0.89), 8.254 (1.99), 8.276 (3.83), 8.299 (1.88), 8.733 (3.67), 8.745 (3.76), 10.440 (4.71), 10.464 (4.56).

LC-MS (Methode 1): $R_t$ = 1.12 min; MS (ESIpos): m/z = 511 [M+H]+

**Beispiel 328:**

1-(2,4-Difluorphenyl)-7-(3-methoxy-3-methylazetidin-1-yl)-4-oxo-*N*-[(2*R*)-1,1,1-trifluorbutan-2-yl]-1,4-dihydro-1,8-naphthyridin-3-carbonsäureamid

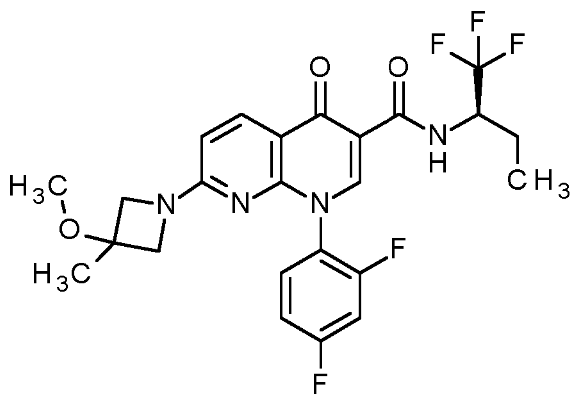

Gemäß AAV3 wurden 80.0 mg (179 μmol) der Verbindung aus Beispiel 67A und 29.6 mg (215 μmol) 3-Methoxy-3-methylazetidin Hydrochlorid in Gegenwart von 110 μl (628 μmol) *N,N*-Diisopropylethylamin in 0.83 ml DMF zur Reaktion gebracht. Die Reaktionsmischung wurde mit 0.5 ml Acetonitril verdünnt und mittels präparativer HPLC (Säule: Chromatorex C18, 10 μm, 125 x 30 mm, Lösungsmittel: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 74.2 mg (80% d. Th., 99% Reinheit) der Titelverbindung erhalten.

$^1$H-NMR (400 MHz, DMSO-d6) δ [ppm]: 0.945 (1.92), 0.964 (4.32), 0.982 (2.11), 1.408 (8.86), 3.155 (16.00), 6.612 (2.62), 6.634 (2.63), 8.293 (2.95), 8.315 (2.81), 8.610 (3.06), 10.461 (1.38), 10.484 (1.33).

LC-MS (Methode 3): $R_t$ = 2.29 min; MS (ESIpos): m/z = 511 [M+H]+

**Beispiel 329:**

1-(2,6-Difluorphenyl)-7-[(4*S*)-4-hydroxy-2-oxopyrrolidin-1-yl]-4-oxo-*N*-[(2*S*)-1,1,1-trifluorpropan-2-yl]-1,4-dihydro-1,8-naphthyridin-3-carbonsäureamid

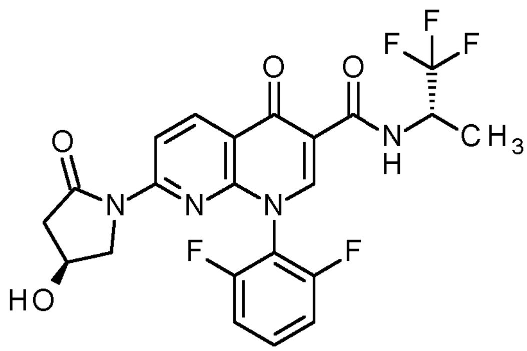

Gemäß AAV2 wurden 100 mg (232 μmol) der Verbindung aus Beispiel 106A mit 25.8 mg (255 μmol) (4*S*)-4-Hydroxypyrrolidin-2-on in Gegenwart von 48.0 mg (347 μmol) Kaliumcarbonat, 5.2 mg (23 μmol) Palladium(II)acetat und 26.6 mg (46.3 μmol) Xantphos in 3.0 ml 1,4-Dioxan umgesetzt. Anschließend wurde die Reaktionsmischung eingeengt. Der Rückstand wurde mit 0.5 ml Wasser und mit 3 ml Acetonitril gelöst und mittels präparativer HPLC (Säule: Chromatorex C18, 10 μm, 125 x 30 mm, Lösungsmittel: Acetonitril / 0.05% Ameisensäure-Gradient; 0 bis 3 min. 10% Acetonitril bis 35 min. 90% Acetonitril und weitere 3 min. 90% Acetonitril) gereinigt und 77.9 mg (68% d. Th., 100% Reinheit) der Titelverbindung erhalten

$^1$H-NMR (400 MHz, DMSO-d6) δ [ppm]: -0.008 (4.64), 0.008 (4.39), 0.146 (0.56), 1.388 (15.95), 1.405 (16.00), 2.074 (1.46), 2.346 (3.39), 2.367 (0.65), 2.389 (3.97), 2.711 (0.60), 2.901 (3.40), 2.916 (3.51), 2.944 (3.10), 2.959 (3.05), 3.288 (3.83), 3.415 (3.37), 3.445 (4.29), 3.615 (3.18), 3.627 (3.85), 3.644 (2.88), 3.656 (2.54), 4.241 (1.11), 4.254 (2.54), 4.262 (2.49), 4.277 (1.01), 4.890 (1.13), 4.913 (1.59), 4.931 (1.71), 4.950 (1.13), 5.322 (7.78), 5.332 (7.60), 7.433 (2.03), 7.445 (2.54), 7.456 (4.16), 7.467 (4.37), 7.478 (2.84), 7.490 (2.29), 7.724 (0.88), 7.740 (1.87), 7.746 (1.94), 7.756 (1.31), 7.762 (3.37), 7.768 (1.31), 7.778 (1.82), 7.783 (1.82), 7.800 (0.83), 8.527 (10.07), 8.549 (12.17), 8.704 (12.31), 8.726 (9.65), 9.018 (12.68), 10.169 (4.80), 10.192 (4.62).

LC-MS (Methode 3): $R_t$ = 1.73 min; MS (ESIpos): m/z = 497 [M+H]+

**Beispiel 330**

7-[(4*S*)-4-Hydroxy-2-oxopyrrolidin-1-yl]-4-oxo-1-(2,4,6-trifluorphenyl)-*N*-[(2*S*)-1,1,1-trifluorpropan-2-yl]-1,4-dihydro-1,8-naphthyridin-3-carbonsäureamid

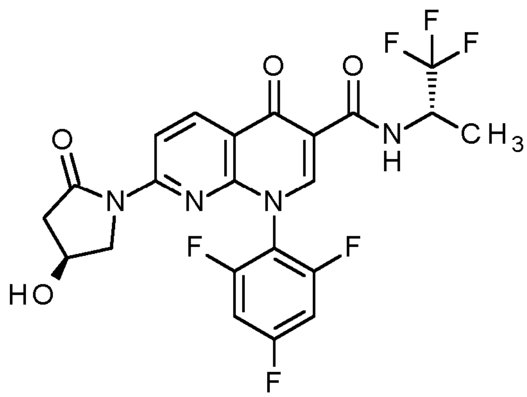

Gemäß AAV2 wurden 300 mg (667 μmol) der Verbindung aus Beispiel 107A mit 74.2 mg (734 μmol) (4*S*)-4-

Hydroxypyrrolidin-2-on in Gegenwart von 138 mg (1.00 mmol) Kaliumcarbonat, 15 mg (67 μmol) Palladium(II)acetat und 27 mg (46 μmol) Xantphos in 8.6 ml 1,4-Dioxan umgesetzt. Anschließend wurde die Reaktionsmischung eingeengt. Anschließend wurde mit 5 ml 1N wässriger Salzsäure angesäuert und der pH-Wert überprüft. Es wurde 140 mg *N*-Acetylcystein zugegeben und für 15 min bei RT nachgerührt. Die Mischung wurde in einen Scheidetrichter gegeben und mit 15 ml gesättigter wässriger Natriumhydrogencarbonat-Lösung und 20 ml Essigsäureethylester verdünnt. Die Phasen wurden getrennt und die wässrige Phase dreimal mit Essigsäureethylester extrahiert. Die vereinigten organischen Phasen wurden über Magnesiumsulfat getrocknet, filtriert und das Lösungsmittel unter reduziertem Druck entfernt. Der Rückstand wurde im 9 ml Acetonitril und 3 ml DMSO gelöst und in vier Läufen mittels präp. HPLC (Säule: Chromatorex C18, 10 μm, 125 x 30 mm, Lösungsmittel: Acetonitril / 0.05% Ameisensäure-Gradient; 0 bis 3 min. 15% Acetonitril bis 35 min. 85% Acetonitril und weitere 3 min. 85% Acetonitril) gereinigt und 208.4 mg (60% d. Th., 99% Reinheit) der Titelverbindung erhalten.

$^1$H-NMR (400 MHz, DMSO-$d_6$): δ [ppm] = 10.17 (d, 1H), 9.07 (s, 1H), 8.71 (d, 1H), 8.54 (d, 1H), 7.66-7.57 (m, 2H), 5.34 (d, 1H), 4.99-4.86 (m, 1H), 4.32-4.26 (m, 1H), 3.69 (dd, 1H), 3.48 (d, 1H), 2.94 (dd, 1H), 2.38 (d, 1H), 1.39 (d, 3H).

LC-MS (Methode 3): $R_t$ = 1.74 min; MS (ESIpos): m/z = 515 $[M+H]^+$

**Beispiel 331**

7-[(2*R*,4*S*)-4-Hydroxy-2,4-dimethylpyrrolidin-1-yl]-4-oxo-*N*-[(2*S*)-1,1,1-trifluorbutan-2-yl]-1-(2,4,6-trifluorphenyl)-1,4-dihydro-1,8-naphthyridin-3-carbonsäureamid

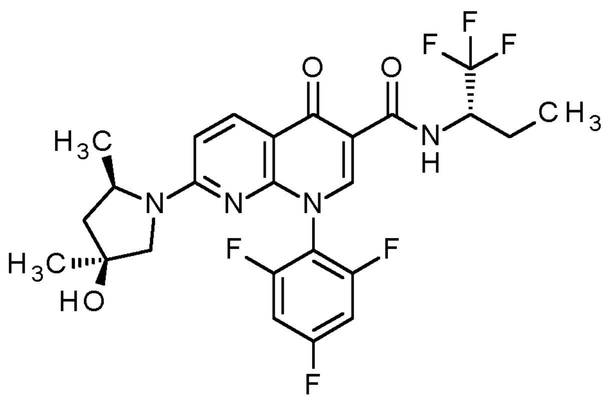

Gemäß AAV3 wurden 30.8 mg (66.4 μmol) der Verbindung aus Beispiel 115A und 24.0 mg (95% Reinheit, 99.6 μmol) der Verbindung aus Beispiel 116A in Gegenwart von 40.0 μl (232 μmol) *N,N*-Di-*iso*-propylethylamin in 640 μl DMF zur Reaktion gebracht. Die Reaktionsmischung wurde mit 0.5 ml Acetonitril verdünnt und mittels präparativer HPLC (Säule: Chromatorex C18, 10 μm, 125 x 30 mm, Lösungsmittel: Acetonitril / 0.05% Ameisensäure-Gradient; 0 bis 3 min. 15% Acetonitril bis 35 min. 85% Acetonitril und weitere 3 min. 85% Acetonitril) gereinigt. Es wurden 30.1 mg (83% d. Th., 99% Reinheit) der Titelverbindung erhalten.

$^1$H-NMR (400 MHz, DMSO-d6) δ [ppm]: -0.150 (0.64), -0.008 (5.62), 0.008 (5.53), 0.146 (0.72), 0.951 (7.19), 0.970 (16.00), 0.988 (8.00), 1.046 (4.51), 1.147 (0.98), 1.271 (7.87), 1.603 (1.19), 1.620 (2.00), 1.628 (2.26), 1.637 (2.64), 1.646 (2.21), 1.655 (2.51), 1.663 (3.02), 1.681 (1.74), 1.850 (1.28), 1.860 (1.57), 1.869 (1.53), 1.879 (1.87), 1.884 (1.57), 1.895 (1.40), 1.904 (1.23), 1.913 (0.98), 2.019 (1.02), 2.328 (0.60), 2.367 (1.02), 2.670 (0.72), 2.710 (1.15), 3.288 (6.51), 3.337 (2.51), 3.483 (1.49), 3.794 (1.11), 4.722 (1.36), 4.877 (2.94), 6.711 (1.53), 7.546 (3.40), 7.569 (6.00), 7.589 (3.23), 8.251 (4.38), 8.273 (4.30), 8.817 (5.11), 10.416 (5.45), 10.440 (5.32).

LC-MS (Methode 3): Rt = 2.13 min; MS (ESIpos): m/z = 543 [M+H]+

**Beispiel 332**

7-[(4*S*)-4-Hydroxy-2-oxopyrrolidin-1-yl]-4-oxo-*N*-[(2*S*)-1,1,1-trifluorbutan-2-yl]-1-(2,4,6-trifluorphenyl)-1,4-dihydro-1,8-naphthyridin-3-carbonsäureanüd

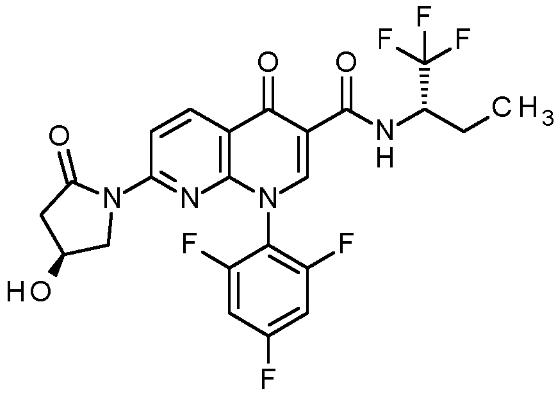

Gemäß AAV1 wurden 20.0 g (47.7 mmol) der Verbindung aus Beispiel 117A mit 9.36 g (57.2 mmol) (2*S*)-1,1,1-Trifluorbutan-2-amin Hydrochlorid in Gegenwart von 27.2 g (71.5 mmol) HATU und 20.8 ml (119 mmol) *N,N*-Diisopropylethylamin in 250 ml Dimethylformamid umgesetzt. Die Reaktionsmischung wurde auf Eiswasser und etwas wässriger Salzsäure ausgerührt, der Niederschlag abgesaugt und mit Wasser gewaschen. Das Rohprodukt wurde mit einem zweiten Ansatz vereinigt, welcher von 2.00 g der Verbindung aus Beispiel 117A in analoger Arbeitsweise ausging. Der vereinigte Rückstand wurde zweimal mittels Normalphasenchromatographie (Dichlormethan-Methanol 95:5, v/v und Petrolether - Essigsäureethylester 1:1, v/v hin zu Dichlormethan-Methanol 9:1, v/v) gereinigt. Abschließend wurde mit *tert.* -Butylmethylether verrührt, der Niederschlag abgesaugt und mit *tert.* -Butylmethylether gewaschen. Es wurden 20.3 g (81% d. Th., 100% Reinheit) der Titelverbindung erhalten.
$^1$H-NMR (400 MHz, DMSO-$d_6$): δ [ppm] = 10.11 (d, 1H), 8.72 (d, 1H), 8.54 (d, 1H), 7.66-7.58 (m, 2H), 5.35-5.31 (m, 1H), 4.83-4.70 (m, 1H), 4.33-4.26 (m, 1H), 3.69 (dd, 1H), 3.47 (d, 1H), 2.95 (dd, 1H), 2.38 (d, 1H), 1.95-1.84 (m, 1H), 1.74-1.60 (m, 1H), 0.98 (t, 3H).
LC-MS (Methode 3): $R_t$ = 1.86 min; 529 $[M+H]^+$.

**Beispiel 333**

1-(2,6-Difluorphenyl)-7-[(4*S*)-4-hydroxy-2-oxopyrrolidin-1-yl]-4-oxo-*N*-[(2*S*)-1,1,1-trifluorbutan-2-yl]-1,4-dihydro-1,8-naphthyridin-3-carbonsäureamid

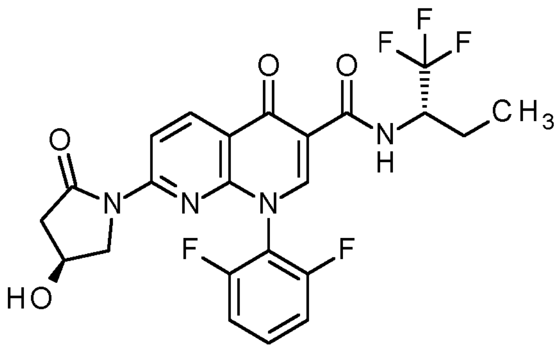

Gemäß AAV2 wurden 150 mg (336 μmol) der Verbindung aus Beispiel 114A mit 34.0 mg (336 μmol) (4*S*)-4-Hydroxypyrrolidin-2-on in Gegenwart von 69.8 mg (505 μmol) Kaliumcarbonat, 13.6 mg (60.6 μmol) Palladium(II)acetat und 70.1 mg (121 μmol) Xantphos in 3.1 ml 1,4-Dioxan umgesetzt. Anschließend wurde die Reaktionsmischung eingeengt. Der Rückstand wurde in 0.5 ml Acetonitril verdünnt und mittels präparativer HPLC (Säule: Chromatorex C18, 10 μm, 125 x 30 mm, Lösungsmittel: Acetonitril / 0.05% Ameisensäure-Gradient; 0 bis 3 min. 15% Acetonitril bis 35 min. 85% Acetonitril und weitere 3 min. 85% Acetonitril) gereinigt. Es wurden 91.7 mg (52% d. Th., 98% Reinheit) der Titelverbindung erhalten.
$^1$H-NMR (400 MHz, DMSO-$d_6$) δ [ppm]: -0.150 (0.56), -0.008 (4.35), 0.008 (4.37), 0.965 (7.27), 0.983 (16.00), 1.002 (7.82), 1.147 (0.70), 1.633 (1.04), 1.651 (1.43), 1.658 (1.32), 1.668 (1.70), 1.677 (1.64), 1.694 (1.72), 1.712 (1.37), 1.868 (1.33), 1.877 (1.66), 1.886 (1.59), 1.896 (1.80), 1.902 (1.61), 1.921 (1.18), 1.931 (1.03), 2.074 (2.86), 2.347 (3.58), 2.390

(4.26), 2.712 (0.60), 2.905 (3.75), 2.920 (3.83), 2.948 (3.25), 2.963 (3.15), 3.288 (4.57), 3.414 (3.64), 3.444 (4.51), 3.619 (3.35), 3.631 (3.95), 3.649 (3.06), 3.661 (2.75), 4.255 (2.75), 4.783 (1.37), 5.322 (7.99), 5.331 (7.85), 7.434 (2.17), 7.447 (2.84), 7.455 (4.53), 7.469 (4.89), 7.479 (3.23), 7.492 (2.57), 7.725 (0.91), 7.741 (2.07), 7.747 (2.07), 7.763 (3.68), 7.779 (1.86), 7.784 (1.95), 7.800 (0.99), 8.530 (10.39), 8.553 (12.44), 8.712 (12.73), 8.734 (9.89), 9.023 (14.97), 10.118 (4.99), 10.142 (4.86).
LC-MS (Methode 3): $R_t$ = 1.85 min; MS (ESIpos): m/z = 511 $[M+H]^+$

**Beispiel 334**

1-(2-Chlor-4,6-difluorphenyl)-7- [(2*R*,4*S*)-4-hydroxy-2,4-dimethylpyrrolidin-1-yl]-4-oxo-*N*-[(2*S*)-1,1,1-trifluorbutan-2-yl]-1,4-dihydro-1,8-naphthyridin-3-carbonsäureamid

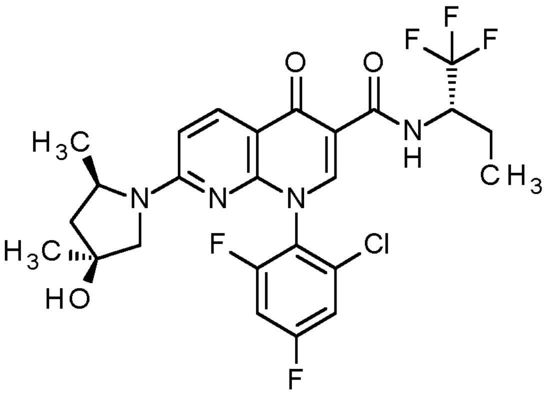

Gemäß AAV3 wurden 19.0 mg (39.7 µmol) der Verbindung aus Beispiel 108C und 10.5 mg (95 % Reinheit, 43.6 µmol) der Verbindung aus Beispiel 116A in Gegenwart von 24.0 µl (140 µmol) *N,N*-Diisopropylethylamin in 1.5 ml DMF zur Reaktion gebracht. Die Reaktionsmischung wurde mit 4 ml Acetonitril und 0.5 ml Wasser verdünnt und mittels präparativer HPLC (Säule: Kromasil C18, 10 µm, 250x20 mm, LM: Acetonitril / 0.05 % Ameisensäure-Gradient; 0 bis 3 min. 10% Acetonitril bis 35 min. 90% Acetonitril und weitere 3 min. 90 % Acetonitril) gereinigt. Es wurden 15.1 mg (67% d. Th., 99% Reinheit) der Titelverbindung erhalten.
$^1$H-NMR (400 MHz, DMSO-d6) δ [ppm]: -0.149 (0.94), -0.008 (16.00), 0.008 (6.41), 0.146 (0.76), 0.949 (3.46), 0.968 (7.24), 0.978 (8.76), 0.996 (5.30), 1.266 (6.27), 1.648 (2.13), 1.886 (1.12), 2.000 (0.86), 2.710 (0.76), 3.288 (13.91), 3.334 (2.74), 3.740 (1.05), 4.733 (1.23), 4.869 (2.34), 6.682 (1.26), 7.712 (2.74), 7.734 (2.92), 8.253 (2.81), 8.275 (2.63), 8.768 (2.70), 10.438 (3.53), 10.462 (3.28).
LC-MS (Methode 3): $R_t$ = 2.16 min; MS (ESIpos): m/z = 559 $[M+H]^+$

**Beispiel 335**

1-(2-Chlor-4,6-difluorphenyl)-7-(3,3-dimethyl-2-oxopyrrolidin-1-yl)-4-oxo-*N*-[(2*S*)-1,1,1-trifluorpropan-2-yl]-1,4-dihydro-1,8-naphthyridin-3-carbonsäureamid

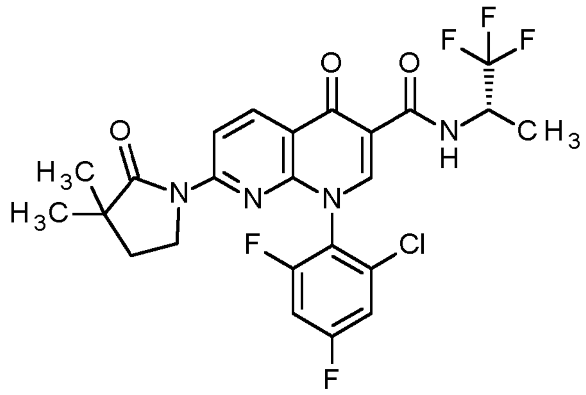

Gemäß AAV2 wurden 50.0 mg (107 µmol) der Verbindung aus Beispiel 111A mit 12.1 mg (107 µmol) 3,3-Dimethylpyrrolidin-2-on in Gegenwart von 22.2 mg (161 µmol) Kaliumcarbonat, 4.3 mg (19 µmol) Palladium(II)acetat und 22.3 mg (38.6 µmol) Xantphos in 980 µl 1,4-Dioxan umgesetzt. Anschließend wurde die Reaktionsmischung mit 0.5 ml 1N wässriger Salzsäure angesäuert und eingeengt. Der Rückstand wurde mittels Normalphasenchromatographie (Cyclohexan-Essigsäureethylester Gradient) gereinigt. Es wurden 34.0 mg (58% d. Th., 99% Reinheit) der Titelverbindung erhalten.
$^1$H-NMR (400 MHz, DMSO-$d_6$) δ [ppm]: -0.008 (1.16), 0.008 (0.85), 1.136 (16.00), 1.385 (2.51), 1.391 (2.58), 1.403 (2.55), 1.409 (2.44), 1.861 (1.36), 1.879 (2.82), 1.896 (1.41), 3.288 (1.97), 3.469 (0.75), 3.487 (1.42), 3.499 (1.18), 7.738 (0.64), 7.745 (0.94), 7.760 (0.98), 7.769 (0.99), 7.781 (0.72), 8.548 (2.50), 8.570 (3.07), 8.704 (3.06), 8.726 (2.32), 9.033 (2.57), 9.037 (2.49), 10.166 (1.26), 10.189 (1.19).
LC-MS (Methode 1): $R_t$ = 1.26 min; MS (ESIpos): m/z = 543 $[M+H]^+$

## Beispiel 336

1-(2-Chlor-4,6-difluorphenyl)-7-(3,3-dimethyl-2-oxopyrrolidin-1-yl)-4-oxo-*N*-[(2*S*-1,1,1-trifluorbutan-2-yl]-1,4-dihydro-1,8-naphthyridin-3-carbonsäureamid

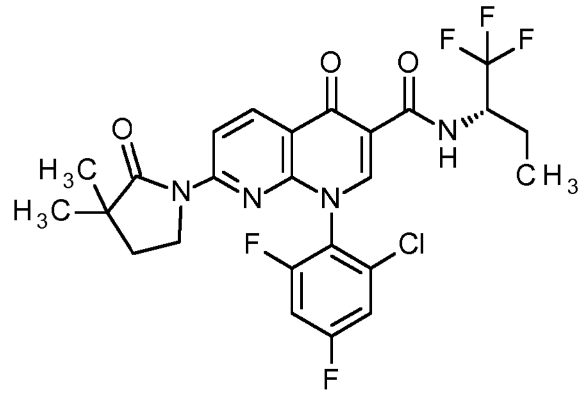

Gemäß AAV2 wurden 50.0 mg (104 µmol) der Verbindung aus Beispiel 108C mit 11.8 mg (104 µmol) 3,3-Dimethylpyrrolidin-2-on in Gegenwart von 21.6 mg (156 µmol) Kaliumcarbonat, 4.2 mg (19 µmol) Palladium(II)acetat und 21.7 mg (37.5 µmol) Xantphos in 950 µl 1,4-Dioxan umgesetzt. Anschließend wurde die Reaktionsmischung mit 0.5 ml wässriger 1N Salzsäure angesäuert und eingeengt. Der Rückstand wurde in 8 ml Dichlormethan gelöst und mittels Normalphasenchromatographie (Cyclohexan-Essigsäureethylester Gradient) gereinigt. Es wurden 33.7 mg (58% d. Th., 99% Reinheit) der Titelverbindung erhalten.
$^1$H-NMR (400 MHz, DMSO-$d_6$) δ [ppm]: -0.008 (0.66), 0.008 (0.63), 0.961 (0.88), 0.971 (1.05), 0.980 (2.05), 0.990 (2.04), 0.998 (1.10), 1.008 (0.96), 1.138 (16.00), 1.863 (1.33), 1.880 (2.84), 1.898 (1.63), 3.288 (1.03), 3.482 (0.71), 3.490 (1.11), 3.500 (1.33), 3.518 (0.64), 7.747 (0.84), 7.763 (0.85), 7.770 (0.93), 8.551 (2.23), 8.574 (2.77), 8.711 (2.67), 8.734 (2.08), 9.040 (3.76), 10.112 (0.77), 10.116 (0.79), 10.136 (0.76), 10.140 (0.74).
LC-MS (Methode 1): $R_t$ = 1.31 min; MS (ESIpos): m/z = 557 $[M+H]^+$

**Beispiel 337**

1-(2,4-Difluorphenyl)-4-oxo-7-(2-oxopyrrolidin-1-yl)-*N*-[(2*R*)-1,1,1-trifluorbutan-2-yl]-1,4-dihydro-1,8-naphthyridin-3-carbonsäureamid

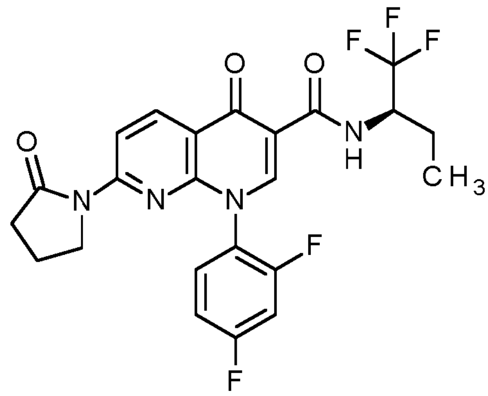

Gemäß AAV2 wurden 100 mg (224 µmol) der Verbindung aus Beispiel 67A mit 21.0 mg (224 µmol) Pyrrolidin-2-on in Gegenwart von 46.5 mg (336 µmol) Kaliumcarbonat, 2.5 mg (11 µmol) Palladium(II)acetat und 13.0 mg (22.4 µmol) Xantphos in 2.0 ml 1,4-Dioxan umgesetzt. Anschließend wurde die Reaktionsmischung mit 0.5 ml wässriger 1N Salzsäure angesäuert und eingeengt. Der Rückstand wurde in 8 ml Dichlormethan gelöst und mittels Normalphasenchromatographie (Cyclohexan-Essigsäureethylester Gradient) gereinigt. Abschließend wurde der Rückstand mit 4 ml Acetonitril, 3 ml Wasser und 2 ml DMSO verrührt, der Niederschlag abgesaugt, mit Wasser gewaschen und im Hochvakuum getrocknet. Es wurden 60.4 mg (52% d. Th., 95% Reinheit) der Titelverbindung erhalten.

$^{1}$H-NMR (400 MHz, DMSO-$d_6$) δ [ppm]: -0.149 (0.63), -0.008 (4.86), 0.008 (4.78), 0.146 (0.63), 0.959 (7.14), 0.978 (16.00), 0.996 (7.84), 1.148 (0.86), 1.175 (0.71), 1.233 (0.86), 1.398 (1.41), 1.625 (1.10), 1.643 (1.41), 1.651 (1.25), 1.660 (1.73), 1.669 (1.57), 1.678 (1.49), 1.686 (1.73), 1.704 (1.33), 1.867 (1.33), 1.876 (1.49), 1.885 (1.57), 1.895 (1.73), 1.901 (1.49), 1.911 (1.41), 1.920 (1.49), 1.930 (1.80), 1.948 (3.06), 1.962 (4.24), 1.988 (3.53), 2.001 (1.18), 2.328 (0.71), 2.366 (1.18), 2.564 (3.69), 2.569 (3.76), 2.582 (5.65), 2.591 (6.12), 2.603 (3.14), 2.610 (2.82), 2.670 (0.71), 2.710 (1.25), 3.289 (7.69), 3.533 (2.04), 3.551 (3.76), 3.574 (3.84), 3.591 (2.04), 4.765 (1.41), 7.343 (1.33), 7.362 (2.75), 7.383 (1.49), 7.591 (1.80), 7.598 (1.88), 7.614 (2.43), 7.617 (2.59), 7.621 (2.67), 7.624 (2.35), 7.640 (1.88), 7.647 (1.80), 7.860 (1.88), 7.880 (1.80), 8.500 (9.25), 8.522 (11.22), 8.690 (11.37), 8.713 (9.18), 8.850 (5.65), 10.209 (4.78), 10.233 (4.63).

LC-MS (Methode 1): $R_t$ = 1.13 min; MS (ESIpos): m/z = 495 $[M+H]^+$

**Beispiel 338**

1-(2-Chlor-4,6-difluorphenyl)-*N*-[(1*S*)-1-cyclopropyl-2,2,2-trifluorethyl]-7-[(4*S*)-4-hydroxy-2-oxopyrrolidin-1-yl]-4-oxo-1,4-dihydro-1,8-naphthyridin-3-carbonsäureamid

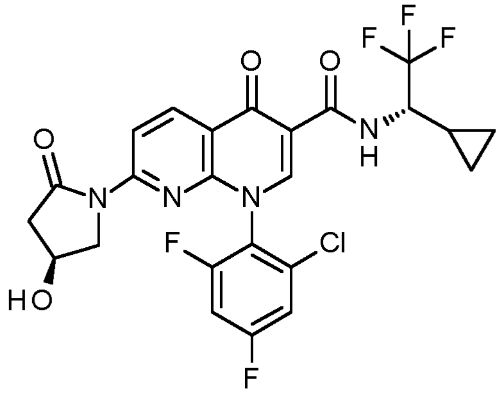

Gemäß AAV2 wurden 100 mg (203 µmol) der Verbindung aus Beispiel 110A mit 22.6 mg (223 µmol) Pyrrolidin-2-on in Gegenwart von 42.1 mg (305 µmol) Kaliumcarbonat, 2.3 mg (10 µmol) Palladium(II)acetat und 11.8 mg (20.3 µmol) Xantphos in 1.8 ml 1,4-Dioxan umgesetzt. Nach vollständiger Umsetzung wurde *N*-Acetylcystein zugeben und für 0.5h bei Raumtemperatur nachgerührt. Es wurde mit 20 ml Essigsäureethylester verdünnt und gegen gesättigte wässrige Natriumhydrogencarbonat-Lösung extrahiert. Die organische Phase wurde eingeengt und in 4 ml Acetonitril und 3 ml Wasser verrührt. Der Niederschlag wurde abgesaugt und im Hochvakuum getrocknet. Die Mutterlauge wurde mittels präparativer HPLC (Säule: Kromasil C18, 10 µm, 250x20 mm, LM: Acetonitril / 0.05 % Ameisensäure-Gradient; 0 bis 3 min. 10% Acetonitril bis 35 min. 90% Acetonitril und weitere 3 min. 90 % Acetonitril) gereinigt und die Produktfraktionen mit dem Niederschlag vereinigt. Abschließend wurden nach Normalphasenchromatographie (Cyclohexan-Essigsäureethylester Gradient) 43.2 mg (36% d. Th., 95% Reinheit) der Titelverbindung erhalten.

$^1$H-NMR (400 MHz, DMSO-$d_6$) δ [ppm] = 10.27 (d, 1H), 9.03 (s, 1H), 8.73 (d, 1H), 8.54 (d, 1H), 7.82-7.72 (m, 2H), 5.33 (d, 1H), 4.46-4.34 (m, 1H), 4.31-4.25 (m, 1H), 3.69-3.60 (m, 1H), 3.46-3.38 (m, 1H), 2.94 (dd, 1H), 2.37 (d, 1H), 1.28-1.19 (m, 1H), 0.71-0.51 (m, 3H), 0.39-0.30 (m, 1H).

LC-MS (Methode 3): $R_t$ = 1.94 min; MS (ESIpos): m/z = 557 $[M+H]^+$

## Beispiel 339

7-[(4*S*)-4-Hydroxy-2-oxopyrrolidin-1-yl]-4-oxo-*N*-[(2*S*)-1-(trifluorrnethoxy)propan-2-yl]-1-(2,4,6-trifluorphenyl)-1,4-dihydro-1,8-naphthyridin-3-carbonsäureamid

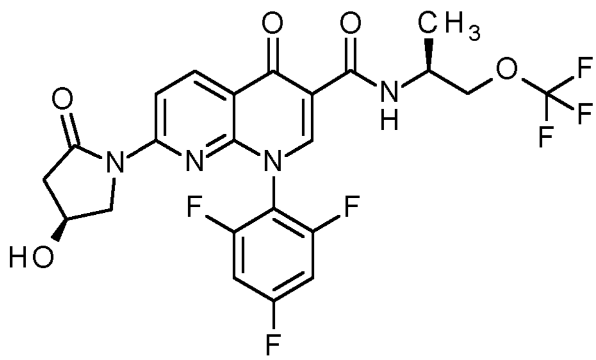

Gemäß AAV2 wurden 80 mg (167 µmol) der Verbindung aus Beispiel 118A mit 18.5 mg (183 µmol) (4*S*)-4-Hydroxypyrrolidin-2-on in Gegenwart von 34.6 mg (250 µmol) Kaliumcarbonat, 1.9 mg (8.3 µmol) Palladium(II)acetat und 9.65 mg (16.7 µmol) Xantphos in 1.7 ml 1,4-Dioxan umgesetzt. Es wurde mit 80 mg *N*-Acetylcystein versetzt und 30 min. bei RT gerührt. Die Reaktionsmischung wurde mit 30 ml Essigsäureethylester versetzt, mit gesättigter wässriger Natriumhydrogencarbonat-Lösung gewaschen, über Natriumsulfat getrocknet, filtriert und eingeengt. Der Rückstand wurde mittels präparativer HPLC (Säule: Chromatorex C18,10 µm, 125x30 mm, LM: Acetonitril / 0.05 % Ameisensäure-Gradient; 0 bis 3 min. 10% Acetonitril bis 35 min. 90% Acetonitril und weitere 3 min. 90% Acetonitril) gereinigt. Es wurden 12.5 mg (14% d. Th., 100% Reinheit) der Titelverbindung erhalten.

$^1$H-NMR (400 MHz, DMSO-d6) δ [ppm]: -0.149 (0.63), -0.008 (5.28), 0.008 (5.09), 0.146 (0.59), 1.258 (15.87), 1.275 (16.00), 2.053 (0.86), 2.353 (2.84), 2.396 (3.32), 2.912 (2.80), 2.926 (2.94), 2.955 (2.59), 2.970 (2.52), 3.291 (2.86), 3.462 (2.92), 3.492 (3.55), 3.670 (2.57), 3.682 (3.15), 3.700 (2.40), 3.712 (2.10), 4.158 (0.69), 4.169 (1.05), 4.183 (4.77), 4.189 (5.49), 4.194 (5.66), 4.201 (5.45), 4.214 (0.90), 4.226 (0.95), 4.296 (2.19), 4.351 (1.26), 4.369 (1.77), 4.386 (1.24), 5.329 (4.44), 5.338 (4.42), 7.588 (1.96), 7.595 (2.23), 7.608 (3.28), 7.618 (3.36), 7.625 (1.89), 7.631 (2.21), 7.638 (1.77), 8.511 (7.76), 8.533 (9.40), 8.693 (9.48), 8.716 (7.55), 8.990 (11.46), 9.825 (4.04), 9.845 (3.93).

LC-MS (Methode 1): $R_t$ = 0.97 min; MS (ESIpos): m/z = 545 $[M+H]^+$

## Beispiel 340

1-(2-Chlor-4,6-difluorphenyl)-4-oxo-7-(2-oxoimidazolidin-1-yl)-*N*-[(2*S*)-1,1,1-trifluorbutan-2-yl]-1,4-dihydro-1,8-naphthyridin-3-carbonsäureamid

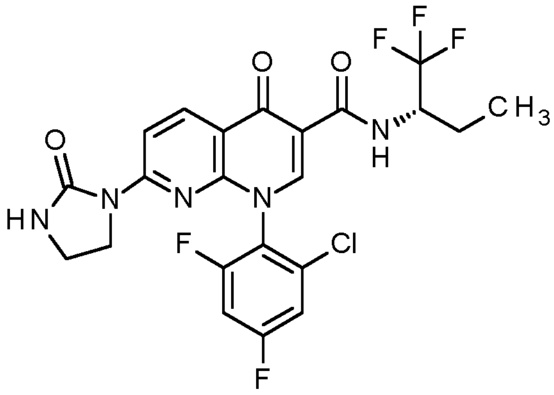

Gemäß AAV2 wurden 100 mg (208 μmol) der Verbindung aus Beispiel 108C mit 89.6 mg (1.04 mmol) Imidazolidin-2-on in Gegenwart von 43.2 mg (312 μmol) Kaliumcarbonat, 2.3 mg (10 μmol) Palladium(II)acetat und 12.0 mg (20.8 μmol) Xantphos in 6.0 ml 1,4-Dioxan umgesetzt. Anschließend wurde mit 0.5 ml 1N wässriger Salzsäure die Reaktionsmischung angesäuert und eingeengt. Der Rückstand wurde in 8 ml Dichlormethan gelöst und mittels Normalphasenchromatographie (Cyclohexan-Essigsäureethylester Gradient) gereinigt. Es wurden 50.0 mg (43% d. Th., 95% Reinheit) der Titelverbindung erhalten.

$^1$H-NMR (400 MHz, DMSO-d6) δ [ppm]: -0.149 (0.90), -0.008 (7.68), 0.008 (7.59), 0.146 (0.93), 0.958 (3.13), 0.968 (3.74), 0.977 (7.30), 0.986 (7.36), 0.995 (3.94), 1.005 (3.45), 1.234 (0.99), 1.398 (0.81), 1.657 (1.22), 1.668 (1.10), 1.676 (1.22), 1.685 (0.93), 1.693 (0.96), 1.872 (1.01), 1.891 (1.16), 2.074 (1.80), 2.367 (0.67), 2.711 (0.70), 3.288 (4.46), 3.340 (6.90), 3.360 (4.38), 3.514 (0.93), 3.523 (2.41), 3.534 (2.67), 3.542 (4.14), 3.553 (3.10), 3.574 (1.74), 4.757 (1.10), 7.663 (6.00), 7.691 (1.28), 7.698 (1.80), 7.713 (1.97), 7.721 (3.16), 7.738 (3.16), 7.744 (3.77), 7.759 (2.26), 8.427 (8.20), 8.449 (10.75), 8.554 (10.61), 8.577 (7.65), 8.959 (16.00), 10.215 (2.72), 10.219 (2.78), 10.239 (2.72), 10.244 (2.64).

LC-MS (Methode 3): $R_t$ = 1.98 min; MS (ESIpos): m/z = 530 $[M+H]^+$

**Beispiel 341**

4-Oxo-7-(2-oxoimidazolidin-1-yl)-1-(2,4,6-trifluorphenyl)-*N*-[(2*S*)-1,1,1-trifluorpropan-2-yl]-1,4-dihydro-1,8-naphthyridin-3-carbonsäureamid

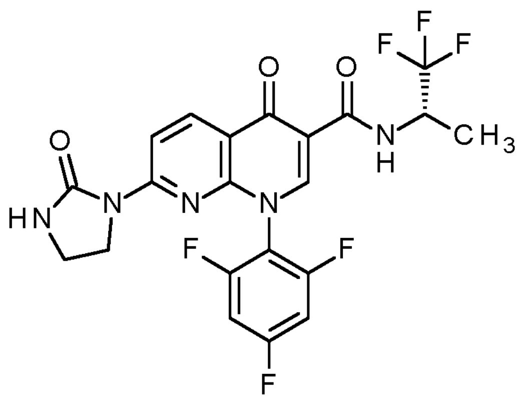

Gemäß AAV2 wurden 51.0 mg (113 μmol) der Verbindung aus Beispiel 107A mit 97.6 mg (1.13 mmol) Imidazolidin-2-on in Gegenwart von 23.5 mg (170 μmol) Kaliumcarbonat, 2.6 mg (11 μmol) Palladium(II)acetat und 13.1 mg (22.7 μmol) Xantphos in 1.1 ml 1,4-Dioxan umgesetzt. Es wurde mit 50 mg N-Acetylcystein versetzt und 30 min. bei RT gerührt. Die Reaktionsmischung wurde mit 30 ml Essigsäureethylester versetzt, mit gesättigter wässriger Natriumhydrogencarbonat-Lösung gewaschen, über Natriumsulfat getrocknet, filtriert und eingeengt. Der Rückstand wurde mittels präparativer HPLC (Säule: Chromatorex C18, 10 μm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 18.4 mg (32% d. Th., 100% Reinheit) der Titelverbindung erhalten.

$^1$H-NMR (400 MHz, DMSO-d6) δ [ppm]: -0.008 (3.27), 0.008 (2.98), 1.381 (15.90), 1.398 (16.00), 2.074 (0.99), 2.367

(1.03), 2.711 (1.03), 3.288 (4.49), 3.329 (4.49), 3.348 (6.38), 3.368 (4.68), 3.576 (5.10), 3.592 (4.33), 3.598 (6.22), 3.616 (3.43), 4.881 (1.12), 4.900 (1.64), 4.923 (1.67), 4.941 (1.06), 7.546 (1.22), 7.554 (4.33), 7.569 (1.92), 7.576 (8.05), 7.584 (1.92), 7.598 (4.42), 7.607 (1.31), 7.667 (6.64), 8.426 (9.14), 8.449 (12.60), 8.541 (12.86), 8.557 (0.90), 8.563 (8.88), 8.993 (12.99), 10.250 (4.75), 10.273 (4.52).
LC-MS (Methode 1): $R_t$ = 1.00 min; MS (ESIpos): m/z = 500 $[M+H]^+$

**Beispiel 342**

*N*-[(1*R*)-1-Cyclopropyl-2,2,2-trifluorethyl]-4-oxo-7-(2-oxoimidazolidin-1-yl)-1-(2,4,6-trifluorphenyl)-1,4-dihydro-1,8-naphthyridin-3-carbonsäureamid

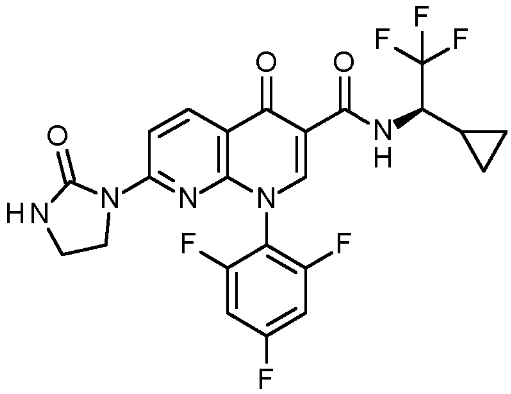

Gemäß AAV1 wurden 150 mg (371 μmol) der Verbindung aus Beispiel 113A mit 78.2 mg (445 μmol) (1*R*)-1-Cyclopropyl-2,2,2-trifluorethanamin Hydrochlorid in Gegenwart von 169 mg (445 μmol) HATU und 160 μl (930 μmol) *N,N*-Diisopropylethylamin in 5.0 ml Dimethylformamid zur Reaktion gebracht. Es wurde mit 1 ml 1N wässriger Salzsäure und 1 ml Dimethylsulfoxid verdünnt und die Lösung mittels präparativer HPLC (Säule: Chromatorex C18, 10 μm, 125 x 30 mm, Lösungsmittel: Acetonitril / 0.05% Ameisensäure-Gradient; 0 bis 3 min. 10% Acetonitril bis 35 min. 90% Acetonitril und weitere 3 min. 90% Acetonitril) gereinigt. Abschließend wurde das Rohprodukt in 20 ml Acetonitril suspendiert und mit 100 ml Wasser verdünnt. Acetonitril wurde unter reduzierten Druck entfernt, der Niederschlag abgesaugt und im Hochvakuum getrocknet. Es wurden 85.3 mg (44% d. Th., 100% Reinheit) der Titelverbindung erhalten.
$^1$H-NMR (400 MHz, DMSO-d6) δ [ppm]: 0.341 (2.94), 0.354 (2.82), 0.576 (3.29), 0.657 (2.82), 1.217 (2.63), 1.238 (2.90), 2.327 (2.08), 2.670 (2.20), 2.710 (1.65), 3.349 (6.98), 3.370 (5.25), 3.578 (5.57), 3.599 (7.10), 3.617 (3.76), 4.395 (2.39), 4.414 (2.55), 7.553 (5.18), 7.575 (9.37), 7.597 (5.22), 7.669 (7.69), 8.430 (8.67), 8.453 (11.45), 8.552 (11.92), 8.575 (8.04), 8.988 (16.00), 10.340 (5.33), 10.363 (5.37).
LC-MS (Methode 1): $R_t$ = 1.03 min; MS (ESIpos): m/z = 526 $[M+H]^+$

**Beispiel 343**

4-Oxo-7-(2-oxoimidazolidin-1-yl)-*N*-[(2*S*)-1,1,1-trifluorbutan-2-yl]-1-(2,4,6-trifluorphenyl)-1,4-dihydro-1,8-naphthyridin-3-carbonsäureamid

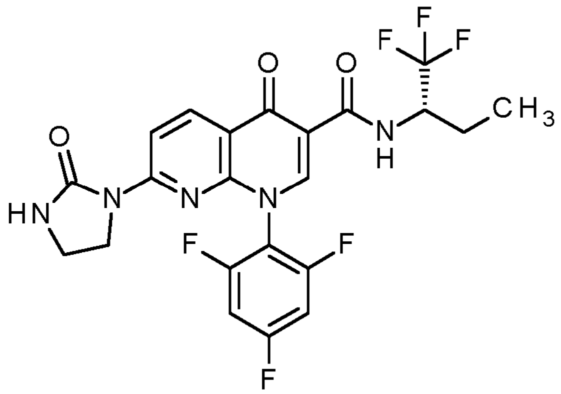

Gemäß AAV2 wurden 100 mg (216 µmol) der Verbindung aus Beispiel 115A mit 186 mg (2.16 mmol) Imidazolidin-2-on in Gegenwart von 44.7 mg (323 µmol) Kaliumcarbonat, 4.8 mg (22 µmol) Palladium(II)acetat und 25.0 mg (43.1 µmol) Xantphos in 2.2 ml 1,4-Dioxan umgesetzt. Es wurde mit 100 mg N-Acetylcystein versetzt und 30 min. bei RT gerührt. Die Reaktionsmischung wurde mit 30 ml Essigsäureethylester versetzt, mit gesättigter wässriger Natriumhydrogencarbonat-Lösung gewaschen, über Magnesiumsulfat getrocknet, filtriert und eingeengt.Der Rückstand wurde mittels präparativer HPLC (Säule: Chromatorex C18, 10 µm, 125x30 mm, LM: Acetonitril / 0.05 % Ameisensäure-Gradient; 0 bis 3 min. 10% Acetonitril bis 35 min. 90% Acetonitril und weitere 3 min. 90% Acetonitril) gereinigt. Noch verunreinigt Produktfraktionen wurden eingeengt und mittels Normalphasenchromatographie (Cyclohexan-Essigsäureethylester Gradient) nachgereinigt. Die Produktfraktionen beider Trennungen wurden vereinigt und 46.6 mg (42% d. Th., 100% Reinheit) der Titelverbindung erhalten.
$^1$H-NMR (400 MHz, DMSO-d6) δ [ppm]: -0.008 (2.95), 0.008 (2.68), 0.960 (7.15), 0.978 (16.00), 0.997 (7.87), 1.148 (0.85), 1.623 (1.07), 1.641 (1.56), 1.648 (1.70), 1.658 (1.74), 1.666 (1.61), 1.676 (1.56), 1.683 (1.70), 1.701 (1.30), 1.862 (1.25), 1.872 (1.52), 1.881 (1.43), 1.890 (1.74), 1.897 (1.52), 1.906 (1.21), 1.915 (1.16), 2.367 (1.39), 2.670 (0.76), 2.710 (1.39), 3.287 (7.51), 3.330 (4.83), 3.350 (6.53), 3.370 (5.05), 3.578 (5.27), 3.600 (6.75), 3.618 (3.80), 4.746 (1.39), 7.556 (4.74), 7.578 (8.45), 7.600 (4.69), 7.668 (7.28), 8.429 (9.97), 8.452 (13.27), 8.549 (13.77), 8.571 (9.34), 8.996 (15.28), 10.199 (5.14), 10.223 (4.87).
LC-MS (Methode 1): $R_t$ = 1.02 min; MS (ESIpos): m/z = 514 $[M+H]^+$

**Beispiel 344**

1-(2,4-Difluorphenyl)-7-(3-ethyl-2-oxotetrahydropyrimidin-1(2*H*)-yl)-4-oxo-*N*-[(2*R*)-1,1,1-trifluorbutan-2-yl]-1,4-dihydro-1,8-naphthyridin-3-carbonsäureamid

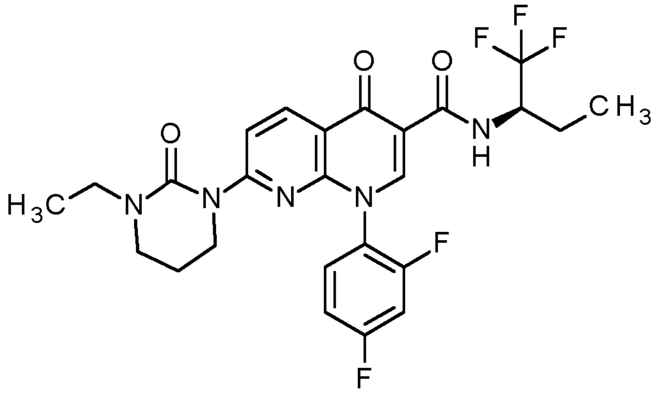

Gemäß AAV2 wurden 200 mg (449 µmol) der Verbindung aus Beispiel 67A mit 63.3 mg (494 µmol) 1-Ethyltetrahydropyrimidin-2(1*H*)-on in Gegenwart von 93.0 mg (673 µmol) Kaliumcarbonat, 5.0 mg (22 µmol) Palladium(II)acetat und 26.0 mg (44.9 µmol) Xantphos in 4.0 ml 1,4-Dioxan umgesetzt. Anschließend wurde die Reaktionsmischung mit wässriger 1N Salzsäure angesäuert und eingeengt. Der Rückstand wurde in 10 ml Dichlormethan gelöst und mittels Normalphasenchromatographie (Cyclohexan-Essigsäureethylester Gradient) gereinigt. Es wurden 191.2 mg (76% d.

Th., 96% Reinheit) der Titelverbindung erhalten.
$^1$H-NMR (400 MHz, DMSO-d6) δ [ppm] = 10.29 (d, 1H), 8.81-8.78 (m, 1H), 8.50 (d, 1H), 8.15 (d, 1H), 7.91-7.83 (m, 1H), 7.65-7.57 (m, 1H), 7.39-7.32 (m, 1H), 4.83-4.70 (m, 1H), 3.58-3.43 (m, 2H), 3.30-3.27 (m, 4H, teilweise unter der Wasser Resonanz), 1.95-1.83 (m, 3H), 1.72-1.60 (m, 1H), 1.07 (t, 3H), 0.98 (t, 3H).
LC-MS (Methode 3): $R_t$ = 2.22 min; MS (ESIpos): m/z = 538 [M+H]$^+$

**Beispiel 345**

*N*-[(1*S*)-1-Cyclopropyl-2,2,2-trifluorethyl]-1-(2,6-difluorphenyl)-7-[(3*R*,4*R*)-3,4-dihydroxypyrrolidin-1-yl]-4-oxo-1,4-dihydro-1,8-naphthyridin-3-carbonsäureamid

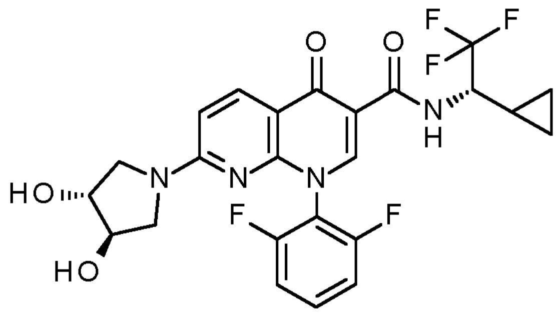

Gemäß AAV3 wurden 50.0 mg (109 µmol) der Verbindung aus Beispiel 104A mit 16.8 mg (120 µmol) (3*R*,4*R*)-Pyrrolidin-3,4-diol Hydrochlorid in Gegenwart von 67.0 µl (380 µmol) *N,N*-Diisopropylethylamin in 2.0 ml Dimethylformamid zur Reaktion gebracht. Die Reaktionsmischung wurde mit wässriger 1M Salzsäure auf pH1 gestellt und mittels präparativer HPLC getrennt (Säule: Chromatorex C18, 10 µm, 125x30 mm, LM: Acetonitril / 0.05 % Ameisensäure-Gradient; 0 bis 3 min. 10% Acetonitril bis 35 min. 90% Acetonitril und weitere 3 min. 90% Acetonitril) gereinigt. Die Produktfraktionen wurden vereinigt und eingeengt. Es wurden 50.9 mg (89% d. Th., 100% Reinheit) der Titelverbindung erhalten.
$^1$H-NMR (400 MHz, DMSO-d6) δ [ppm]: -0.149 (0.90), -0.011 (4.62), -0.008 (11.14), 0.008 (6.98), 0.146 (0.94), 0.316 (0.83), 0.327 (1.80), 0.338 (2.71), 0.350 (2.71), 0.362 (2.01), 0.373 (1.04), 0.498 (0.83), 0.510 (1.94), 0.522 (2.81), 0.534 (2.50), 0.549 (2.22), 0.557 (2.12), 0.569 (2.85), 0.579 (2.36), 0.589 (2.15), 0.600 (1.77), 0.614 (1.15), 0.627 (1.46), 0.637 (1.77), 0.648 (2.46), 0.652 (2.05), 0.658 (2.29), 0.663 (2.22), 0.672 (2.15), 0.680 (1.01), 0.684 (0.94), 0.693 (0.69), 1.179 (1.28), 1.188 (1.77), 1.200 (2.98), 1.208 (2.12), 1.212 (1.84), 1.220 (2.85), 1.232 (1.49), 1.241 (1.04), 2.367 (1.11), 2.710 (1.11), 3.010 (3.12), 3.042 (4.13), 3.174 (2.53), 3.184 (2.81), 3.206 (2.05), 3.216 (1.77), 3.288 (7.05), 3.347 (3.85), 3.592 (2.29), 3.602 (2.53), 3.620 (2.08), 3.630 (1.84), 3.893 (3.68), 4.039 (3.71), 4.356 (1.49), 4.376 (2.39), 4.399 (2.33), 4.419 (1.28), 5.125 (5.48), 5.134 (5.34), 5.219 (5.55), 5.228 (5.24), 6.769 (9.65), 6.792 (9.75), 7.395 (5.17), 7.415 (10.72), 7.437 (5.83), 7.672 (1.15), 7.687 (2.33), 7.693 (2.19), 7.703 (1.70), 7.709 (3.99), 7.714 (1.53), 7.725 (1.98), 7.731 (2.05), 7.746 (0.94), 8.278 (12.08), 8.300 (11.04), 8.742 (16.00), 10.573 (5.93), 10.596 (5.55).
LC-MS (Methode 3): $R_t$ = 1.67 min; MS (ESIpos): m/z = 525 [M+H]$^+$

**Beispiel 346**

1-(2-Chlor-6-fluorphenyl)-7-[(3*R*,4*R*)-3,4-dihydroxypyrrolidin-1-yl]-4-oxo-*N*-[(2*S*)-1,1,1-trifluorbutan-2-yl]-1,4-dihydro-1,8-naphthyridin-3-carbonsäureamid

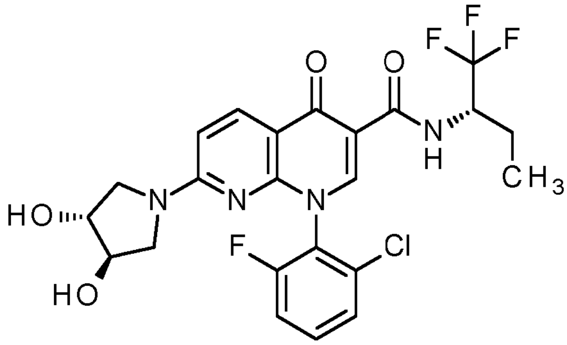

Gemäß AAV3 wurden 50.0 mg (108 µmol) der Verbindung aus Beispiel 119A und 16.6 mg (119 µmol) (3*R*,4*R*)-Pyrrolidin-3,4-diol Hydrochlorid in Gegenwart von 53.0 µl (380 µmol) *N,N*-Diisopropylethylamin in 1.0 ml DMF zur Reaktion gebracht. Die Reaktionsmischung wurde mit wässriger 1M Salzsäure auf pH1 gestellt und mittels präparativer HPLC getrennt (Säule: Chromatorex C18, 10 µm, 125x30 mm, LM: Acetonitril / 0.05 % Ameisensäure-Gradient; 0 bis 3 min. 10% Acetonitril bis 35 min. 90% Acetonitril und weitere 3 min. 90% Acetonitril) gereinigt. Es wurden 46.1 mg (80% d. Th., 99% Reinheit) der Titelverbindung erhalten.

$^1$H-NMR (400 MHz, DMSO-d6) δ [ppm]: -0.008 (1.98), 0.008 (1.84), 0.952 (3.72), 0.963 (4.56), 0.971 (8.66), 0.981 (8.89), 0.989 (4.80), 1.000 (4.09), 1.622 (0.99), 1.633 (1.04), 1.639 (1.46), 1.649 (1.32), 1.657 (1.46), 1.668 (1.08), 1.674 (1.13), 1.854 (1.08), 1.864 (1.27), 1.871 (1.32), 1.883 (1.46), 1.899 (1.18), 1.907 (0.94), 2.367 (1.08), 2.711 (1.08), 2.950 (1.51), 2.980 (2.54), 3.007 (1.98), 3.124 (1.18), 3.133 (1.46), 3.143 (1.32), 3.154 (1.98), 3.165 (1.04), 3.174 (1.04), 3.291 (4.24), 3.339 (4.42), 3.585 (2.02), 3.595 (2.26), 3.612 (1.93), 3.623 (1.69), 3.883 (3.34), 4.034 (3.34), 4.733 (1.36), 4.752 (1.27), 5.126 (3.48), 5.130 (3.91), 5.135 (3.95), 5.222 (3.95), 5.227 (3.86), 6.762 (8.52), 6.784 (8.75), 7.522 (1.08), 7.527 (1.79), 7.531 (1.27), 7.543 (1.79), 7.547 (3.34), 7.551 (2.78), 7.566 (1.51), 7.570 (2.26), 7.575 (1.60), 7.620 (2.26), 7.623 (2.35), 7.627 (1.27), 7.641 (4.80), 7.643 (5.08), 7.647 (2.21), 7.665 (3.01), 7.680 (3.01), 7.686 (3.72), 7.700 (3.76), 7.706 (1.46), 7.721 (1.36), 8.275 (9.88), 8.297 (9.27), 8.695 (16.00), 10.458 (3.29), 10.462 (3.34), 10.482 (3.25), 10.486 (3.20).

LC-MS (Methode 3): $R_t$ = 1.68 min; MS (ESIpos): m/z = 529 $[M+H]^+$

## Beispiel 347

1-(2-Chlor-6-fluorphenyl)-*N*-[(1*S*)-1-cyclopropyl-2,2,2-trifluorethyl]-7-[(3*R*,4*R*)-3,4-dihydroxypyrrolidin-1-yl]-4-oxo-1,4-dihydro-1,8-naphthyridin-3-carbonsäureamid

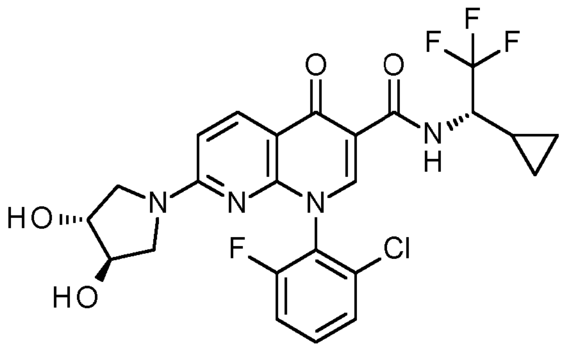

Gemäß AAV3 wurden 50.0 mg (105 µmol) der Verbindung aus Beispiel 120A und 16.2 mg (116 µmol) (3*R*,4*R*)-Pyrrolidin-3,4-diol Hydrochlorid in Gegenwart von 52.0 µl (370 µmol) *N,N*-Diisopropylethylamin in 1.0 ml DMF zur Reaktion gebracht. Die Reaktionsmischung wurde mit wässriger 1N Salzsäure auf pH 1 eingestellt und mittels präparativer HPLC (Säule: Chromatorex C18, 10 µm, 125x30 mm, LM: Acetonitril / 0.05 % Ameisensäure-Gradient; 0 bis 3 min. 10% Acetonitril bis 35 min. 90% Acetonitril und weitere 3 min. 90% Acetonitril) gereinigt. Es wurden 48.3 mg (84% d. Th., 99% Reinheit) der Titelverbindung erhalten.

$^1$H-NMR (400 MHz, DMSO-d6) δ [ppm] = 10.61 (d, 1H), 8.69 (s, 1H), 8.29 (d, 1H), 7.73-7.51 (m, 3H), 6.77 (d, 1H), 5.24-5.20 (m, 1H), 5.14-5.11 (m, 1H), 4.43-4.32 (m, 1H), 4.03 (br. s, 1H), 3.88 (br. s, 1H), 3.64-3.56 (m, 1H), 3.20-3.10

(m, 1H), 3.02-2.93 (m, 1H), 1.26-1.16 (m, 1H), 0.71-0.47 (m, 3H), 0.39-0.29 (m, 1H).
LC-MS (Methode 3): $R_t$ = 1.72 min; MS (ESIpos): m/z = 541 $[M+H]^+$

**Beispiel 348**

1-(2,6-Difluorphenyl)-7-[(3*R*,4*R*)-3,4-dihydroxypyrrolidin-1-yl]-4-oxo-*N*-[(2*S*)-1,1,1-trifluorbutan-2-yl]-1,4-dihydro-1,8-naphthyridin-3-carbonsäureamid

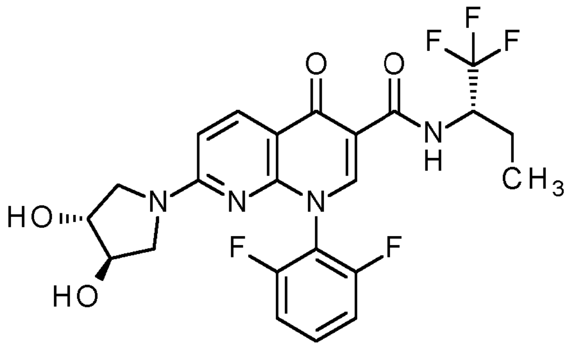

Gemäß AAV3 wurden 50.0 mg (111 µmol) der Verbindung aus Beispiel 114A mit 17.0 mg (122 µmol) (3*R*,4*R*)-Pyrrolidin-3,4-diol Hydrochlorid in Gegenwart von 68.0 µl (390 µmol) *N,N*-Diisopropylethylamin in 1.1 ml Dimethylformamid zur Reaktion gebracht. Die Reaktionsmischung wurde mit 2 ml Acetonitril verdünnt, mit 1N wässriger Salzsäure angesäuert und mittels präparativer HPLC (Säule: Chromatorex C18, 10 µm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 50.9 mg (89% d. Th., 100% Reinheit) der Titelverbindung erhalten.
$^1$H-NMR (500 MHz, DMSO-d6) δ [ppm]: 0.960 (7.49), 0.974 (16.00), 0.989 (7.72), 1.614 (1.16), 1.629 (1.43), 1.634 (1.34), 1.642 (1.76), 1.649 (1.57), 1.657 (1.48), 1.662 (1.62), 1.677 (1.25), 1.859 (1.29), 1.867 (1.48), 1.874 (1.48), 1.882 (1.66), 1.887 (1.48), 1.894 (1.29), 1.902 (1.16), 1.909 (0.97), 3.017 (2.82), 3.043 (3.61), 3.180 (2.13), 3.188 (2.36), 3.205 (1.76), 3.213 (1.57), 3.344 (3.75), 3.596 (1.99), 3.603 (2.22), 3.618 (1.90), 3.626 (1.66), 3.895 (3.24), 4.043 (3.24), 4.735 (1.29), 4.743 (1.20), 5.130 (4.21), 5.137 (4.21), 5.226 (4.16), 5.233 (3.93), 6.771 (8.92), 6.789 (9.02), 7.402 (3.47), 7.419 (6.94), 7.437 (3.61), 7.682 (0.88), 7.694 (1.85), 7.699 (1.90), 7.707 (1.43), 7.712 (3.28), 7.716 (1.29), 7.724 (1.76), 7.729 (1.71), 7.741 (0.79), 8.278 (11.05), 8.296 (10.27), 8.751 (15.72), 10.442 (5.18), 10.462 (4.90).
LC-MS (Methode 1): $R_t$ = 1.63 min; MS (ESIpos): m/z = 513 $[M+H]^+$

**Beispiel 349**

7-[(3*R*,4*R*)-3,4-Dihydroxypyrrolidin-1-yl]-4-oxo-*N*-[(2*R*)-1,1,1-trifluor-3,3-dimethylbutan-2-yl]-1-(2,4,6-trifluorphenyl)-1,4-dihydro-1,8-naphthyridin-3-carbonsäureamid

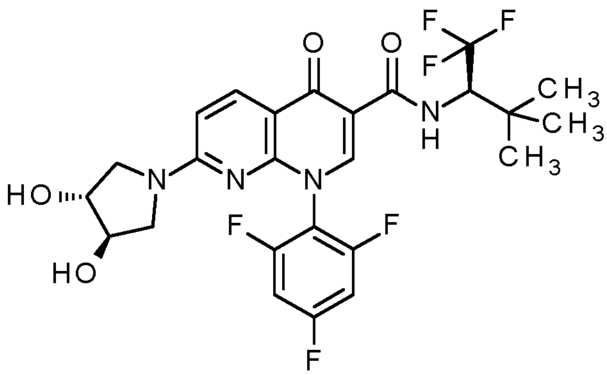

Gemäß AAV1 wurden 55.0 mg (131 μmol) der Verbindung aus Beispiel 121A mit 24.3 mg (157 μmol) (2*R*)-1,1,1-Trifluor-3,3-dimethylbutan-2-amin in Gegenwart von 74.5 mg (196 μmol) HATU und 57.0 μl (330 μmol) *N,N*-Diisopropylethylamin in 2.0 ml Dimethylformamid zur Reaktion gebracht. Es wurde mit 1 ml Wasser und 2 ml Acetonitril verdünnt und die Lösung mittels präparativer HPLC (Säule: Chromatorex C18, 10 μm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 55.9 mg (77% d. Th., 100% Reinheit) der Titelverbindung erhalten.
$^1$H-NMR (400 MHz, DMSO-d6) δ [ppm]: -0.008 (0.77), 0.008 (0.71), 1.090 (16.00), 3.057 (0.73), 3.089 (0.96), 3.241 (0.64), 3.289 (0.78), 3.331 (0.87), 3.360 (0.89), 3.611 (0.58), 3.931 (0.86), 4.050 (0.86), 4.622 (0.74), 5.145 (1.36), 5.154 (1.36), 5.229 (1.36), 5.238 (1.34), 6.771 (2.11), 6.793 (2.16), 7.544 (0.97), 7.567 (1.61), 7.587 (0.93), 8.297 (2.61), 8.319 (2.43), 8.816 (4.01), 10.765 (1.26), 10.790 (1.19).
LC-MS (Methode 3): $R_t$ = 1.86 min; MS (ESIpos): m/z = 559 $[M+H]^+$

**Beispiel 350**

7-[(3*R*,4*R*)-3,4-Dihydroxypyrrolidin-1-yl]-4-oxo-*N*-[1-(trifluormethyl)cyclopentyl]-1-(2,4,6-trifluorphenyl)-1,4-dihydro-1,8-naphthyridin-3-carbonsäureamid

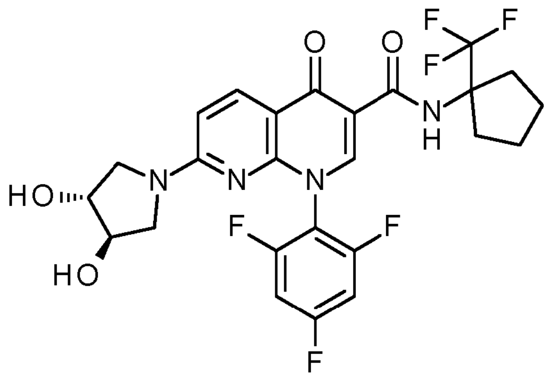

Gemäß AAV1 wurden 55.0 mg (131 μmol) der Verbindung aus Beispiel 121A mit 29.7 mg (157 μmol) 1-(Trifluormethyl)cyclopentanamin Hydrochlorid in Gegenwart von 74.5 mg (196 μmol) HATU und 79.6 μl (460 μmol) *N,N*-Diisopropylethylamin in 2.0 ml Dimethylformamid zur Reaktion gebracht. Es wurde mit 1 ml Wasser und 2 ml Acetonitril verdünnt und die Lösung mittels präparativer HPLC (Säule: Chromatorex C18, 10 μm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 63.4 mg (87% d. Th., 100% Reinheit) der Titelverbindung erhalten.
$^1$H-NMR (400 MHz, DMSO-d6) δ [ppm]: -0.008 (4.57), 0.008 (4.33), 1.708 (1.76), 1.729 (3.67), 1.747 (3.47), 1.765 (3.35), 1.784 (3.71), 1.794 (3.02), 1.803 (3.27), 1.824 (1.96), 2.012 (1.84), 2.029 (3.10), 2.045 (3.63), 2.063 (3.67), 2.073 (16.00), 2.079 (2.12), 2.353 (2.61), 2.367 (4.73), 2.382 (2.90), 2.400 (2.82), 2.711 (1.43), 3.052 (3.14), 3.083 (4.20), 3.225 (2.37), 3.235 (2.73), 3.256 (2.04), 3.267 (1.96), 3.288 (4.53), 3.349 (4.08), 3.593 (2.20), 3.603 (2.57), 3.621 (2.08), 3.632 (1.92), 3.926 (3.76), 4.046 (3.76), 5.135 (5.67), 5.144 (5.76), 5.227 (5.76), 5.236 (5.67), 6.760 (9.55), 6.782 (9.76), 7.541 (2.61), 7.545 (2.94), 7.553 (1.96), 7.561 (5.02), 7.564 (5.02), 7.567 (5.14), 7.576 (2.00), 7.584 (3.02), 7.599 (0.98), 8.263 (11.02), 8.286 (10.49), 8.741 (15.39), 10.513 (11.71).
LC-MS (Methode 3): $R_t$ = 1.83 min; MS (ESIpos): m/z = 557 $[M+H]^+$

**Beispiel 351**

1-(2-Chlor-6-fluorphenyl)-7-[(4*S*)-4-hydroxy-2-oxopyrrolidin-1-yl]-4-oxo-*N*-[(2*S*)-1,1,1-trifluorbutan-2-yl]-1,4-dihydro-1,8-naphthyridin-3-carbonsäureamid

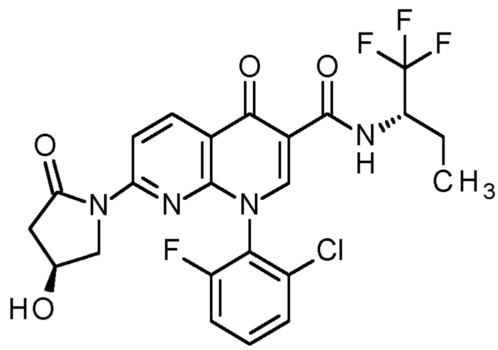

Gemäß AAV2 wurden 100 mg (216 µmol) der Verbindung aus Beispiel 119A mit 24.1 mg (238 µmol) (4*S*)-4-Hydroxypyrrolidin-2-on in Gegenwart von 44.8 mg (325 µmol) Kaliumcarbonat, 2.4 mg (11 µmol) Palladium(II)acetat und 12.5 mg (21.6 µmol) Xantphos in 1.9 ml 1,4-Dioxan umgesetzt. Anschließend wurden 100 mg *N*-Acetylcystein zugegeben und für 0.5 h bei RT nachgerührt. Nach Zugabe von 20 ml Essigsäureethylester wurde gegen gesättigte wässrige Natriumhydrogencarbonat-Lösung extrahiert. Die organische Phase wurde eingeengt und der Rückstand mittels präparativer HPLC (Säule: Chromatorex C18, 10 µm, 125x30 mm, Laufmittel: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 54.1 mg (47% d. Th., 98% Reinheit) der Titelverbindung erhalten.

$^1$H-NMR (500 MHz, DMSO-d6) δ [ppm]: 0.968 (5.41), 0.979 (6.69), 0.983 (11.85), 0.993 (10.59), 0.998 (6.60), 1.008 (4.54), 1.643 (0.88), 1.654 (1.09), 1.657 (1.29), 1.663 (1.13), 1.670 (1.71), 1.674 (1.40), 1.678 (1.37), 1.682 (1.45), 1.685 (1.54), 1.688 (1.55), 1.691 (1.52), 1.696 (1.13), 1.702 (1.30), 1.705 (1.14), 1.717 (0.85), 1.876 (1.21), 1.883 (1.40), 1.886 (1.47), 1.891 (1.56), 1.898 (1.66), 1.902 (1.71), 1.906 (1.44), 1.911 (1.53), 1.918 (1.18), 1.921 (0.99), 1.926 (0.88), 2.076 (0.84), 2.345 (3.82), 2.348 (3.60), 2.380 (4.31), 2.383 (4.02), 2.909 (4.60), 2.920 (4.73), 2.943 (4.34), 2.955 (4.16), 3.354 (3.09), 3.377 (5.05), 3.401 (2.58), 3.572 (2.04), 3.581 (2.57), 3.590 (2.42), 3.595 (2.49), 3.600 (3.01), 3.605 (1.86), 3.614 (2.31), 3.624 (1.88), 4.249 (3.61), 4.767 (1.64), 4.777 (1.52), 5.327 (1.59), 7.572 (1.57), 7.574 (1.63), 7.586 (1.95), 7.589 (3.77), 7.591 (3.44), 7.603 (2.47), 7.607 (3.70), 7.610 (2.16), 7.621 (1.59), 7.624 (1.62), 7.661 (2.54), 7.663 (1.64), 7.675 (4.62), 7.677 (5.15), 7.680 (2.34), 7.691 (4.43), 7.693 (2.60), 7.724 (3.27), 7.736 (3.47), 7.741 (4.77), 7.753 (4.66), 7.758 (2.03), 7.769 (1.86), 8.532 (11.77), 8.550 (13.29), 8.722 (13.08), 8.740 (10.70), 8.796 (0.68), 8.992 (14.83), 8.994 (16.00), 10.136 (3.78), 10.140 (4.00), 10.155 (3.69), 10.159 (3.79).

LC-MS (Methode 1): $R_t$ = 1.00 min; MS (ESIpos): m/z = 527 $[M+H]^+$

## Beispiel 352

1-(2-Chlor-6-fluorphenyl)-*N*-[(1*S*)-1-cyclopropyl-2,2,2-trifluorethyl]-7-[(4*S*)-4-hydroxy-2-oxopyrrolidin-1-yl]-4-oxo-1,4-dihydro-1,8-naphthyridin-3-carbonsäureamid

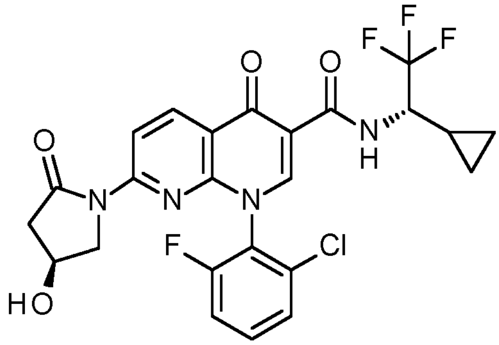

Gemäß AAV2 wurden 100 mg (211 µmol) der Verbindung aus Beispiel 120A mit 23.5 mg (232 µmol) (4*S*)-4-Hydroxypyrrolidin-2-on in Gegenwart von 43.7 mg (316 µmol) Kaliumcarbonat, 2.4 mg (11 µmol) Palladium(II)acetat und 12.2 mg (21.1 µmol) Xantphos in 1.9 ml 1,4-Dioxan umgesetzt. Anschließend wurden 100 mg *N*-Acetylcystein zugegeben und für 0.5 h bei RT nachgerührt. Nach Zugabe von 20 ml Essigsäureethylester wurde gegen gesättigte

wässrige Natriumhydrogencarbonat-Lösung extrahiert. Die organische Phase wurde eingeengt und der Rückstand mittels präparativer HPLC (Säule: Chromatorex C18, 10 µm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 63.8 mg (56% d. Th., 99% Reinheit) der Titelverbindung erhalten.
$^1$H-NMR (400 MHz, DMSO-d6) δ [ppm]: -0.149 (0.89), -0.008 (7.91), 0.008 (7.20), 0.146 (0.94), 0.343 (2.06), 0.356 (2.46), 0.368 (1.92), 0.379 (1.30), 0.549 (2.06), 0.563 (2.91), 0.578 (2.77), 0.588 (2.55), 0.598 (1.92), 0.608 (1.65), 0.622 (1.07), 0.638 (0.85), 0.648 (1.34), 0.659 (1.70), 0.669 (2.10), 0.684 (1.52), 0.689 (1.16), 1.208 (0.89), 1.215 (1.12), 1.227 (2.10), 1.248 (2.06), 1.260 (1.12), 2.337 (3.44), 2.366 (1.65), 2.380 (3.71), 2.670 (0.63), 2.710 (1.52), 2.899 (3.49), 2.914 (3.58), 2.942 (3.13), 2.957 (3.08), 3.287 (5.45), 3.345 (2.32), 3.374 (2.95), 3.398 (1.83), 3.563 (1.34), 3.574 (1.83), 3.582 (2.01), 3.593 (3.22), 3.605 (1.25), 3.611 (1.88), 3.624 (1.56), 4.239 (2.59), 4.382 (1.30), 4.402 (1.79), 4.420 (1.52), 5.318 (7.60), 5.327 (7.37), 7.563 (1.21), 7.567 (1.39), 7.576 (1.07), 7.579 (1.21), 7.587 (2.82), 7.599 (2.06), 7.607 (1.79), 7.610 (1.79), 7.623 (1.25), 7.654 (1.61), 7.665 (2.23), 7.669 (1.70), 7.675 (2.91), 7.686 (4.07), 7.689 (2.46), 7.715 (2.99), 7.729 (3.13), 7.736 (4.07), 7.750 (3.89), 7.757 (1.65), 7.771 (1.47), 8.529 (9.25), 8.551 (10.99), 8.722 (11.22), 8.744 (8.98), 8.984 (16.00), 10.277 (4.83), 10.301 (4.65).
LC-MS (Methode 3): $R_t$ = 1.05 min; MS (ESIpos): m/z = 539 $[M+H]^+$

**Beispiel 353**

7-[(3*R*,4*R*)-3,4-Dihydroxypyrrolidin-1-yl]-4-oxo-*N*-[(2*S*)-1-(trifluormethoxy)propan-2-yl]-1-(2,4,6-trifluorphenyl)-1,4-dihydro-1,8-naphthyridin-3-carbonsäureamid

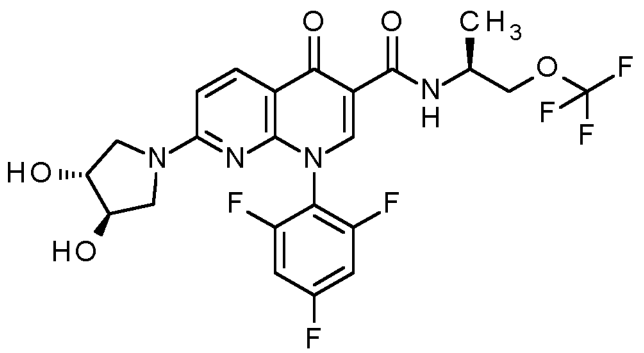

Gemäß AAV3 wurden 50.0 mg (104 µmol) der Verbindung aus Beispiel 118A mit 16.0 mg (115 µmol) (3*R*,4*R*)-Pyrrolidin-3,4-diol Hydrochlorid in Gegenwart von 63.5 µl (365 µmol) *N,N*-Diisopropylethylamin in 1.0 ml Dimethylformamid zur Reaktion gebracht. Die Reaktionsmischung wurde mit 2 ml Acetonitril verdünnt, mit wässrige 1N Salzsäure angesäuert und mittels präparativer HPLC (Säule: Chromatorex C18, 10 µm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 47.9 mg (84% d. Th., 100% Reinheit) der Titelverbindung erhalten
$^1$H-NMR (500 MHz, DMSO-d6) δ [ppm]: 1.244 (16.00), 1.258 (15.83), 2.073 (7.16), 3.057 (2.15), 3.082 (2.68), 3.229 (1.66), 3.237 (1.86), 3.254 (1.49), 3.262 (1.42), 3.338 (3.21), 3.596 (1.49), 3.604 (1.66), 3.618 (1.42), 3.626 (1.26), 3.925 (2.55), 4.047 (2.55), 4.139 (0.89), 4.149 (1.29), 4.159 (4.41), 4.167 (7.16), 4.176 (4.90), 4.186 (1.03), 4.196 (1.09), 4.316 (0.70), 4.325 (1.23), 4.339 (1.62), 4.354 (1.13), 5.137 (3.41), 5.144 (3.38), 5.226 (3.35), 5.234 (3.25), 6.747 (6.92), 6.765 (6.96), 7.539 (1.89), 7.543 (2.09), 7.555 (3.48), 7.561 (3.54), 7.567 (1.62), 7.573 (2.09), 7.578 (1.76), 8.260 (8.68), 8.278 (8.08), 8.722 (12.99), 10.091 (4.07), 10.107 (3.84).
LC-MS (Methode 3): $R_t$ = 1.63 min; MS (ESIpos): m/z = 547 $[M+H]^+$

**Beispiel 354**

1-(2,4-Difluorphenyl)-7-[1-hydroxy-3-azabicyclo[4.1.0]hept-3-yl]-4-oxo-*N*-[(2*R*)-1,1,1-trifluorbutan-2-yl]-1,4-dihydro-1,8-naphthyridin-3-carbonsäureamid (Diastereomerengemisch)

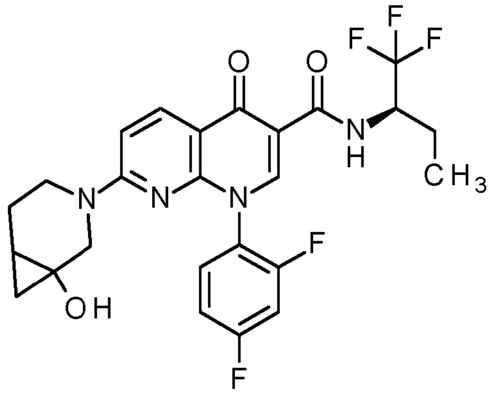

Gemäß AAV3 wurden 50.0 mg (112 μmol) der Verbindung aus Beispiel 67A mit 21.2 mg (95% Reinheit, 135 μmol) 5-Azaspiro[2.4]heptan-7-ol Hydrochlorid in Gegenwart von 68.0μl (390 μmol) *N,N*-Diisopropylethylamin in 0.5 ml Dimethylformamid zur Reaktion gebracht. Die Reaktionsmischung wurde mit 1 ml Acetonitril und 0.1 ml 1N wässrige Salzsäure verdünnt und mittels präparativer HPLC (Säule: Chromatorex C18, 10 μm, 125x30 mm, LM: Acetonitril / 0.1 % Ameisensäure-Gradient; (0 bis 5 min. 10% Acetonitril, während 14 min. nach 90 % Acetonitril und weitere 4 min. 90 % Acetonitril) gereinigt. Es wurden 49.9 mg (84% d. Th., 99% Reinheit) der Titelverbindung erhalten
$^1$H-NMR (400 MHz, DMSO-d6) δ [ppm]: -0.011 (2.18), -0.008 (5.18), 0.008 (3.42), 0.146 (0.60), 0.178 (1.43), 0.204 (1.20), 0.219 (1.62), 0.232 (0.90), 0.666 (1.84), 0.679 (1.99), 0.690 (2.10), 0.704 (1.77), 0.945 (7.51), 0.963 (16.00), 0.982 (7.70), 1.101 (1.92), 1.110 (1.77), 1.119 (1.80), 1.477 (1.01), 1.596 (1.20), 1.614 (1.54), 1.621 (1.43), 1.630 (1.88), 1.639 (1.65), 1.649 (1.58), 1.656 (1.73), 1.674 (1.28), 1.848 (1.35), 1.858 (1.62), 1.867 (1.58), 1.877 (1.77), 1.883 (1.58), 1.893 (1.39), 1.902 (1.24), 1.912 (1.13), 1.966 (1.05), 2.367 (1.28), 2.478 (1.95), 2.519 (3.15), 2.523 (3.64), 2.710 (1.20), 3.103 (0.79), 3.288 (5.07), 3.384 (1.77), 3.399 (1.50), 3.416 (1.50), 4.013 (2.85), 4.046 (2.55), 4.730 (1.46), 4.751 (1.31), 5.603 (2.70), 6.978 (2.74), 7.001 (2.74), 7.309 (1.46), 7.331 (2.63), 7.352 (1.39), 7.544 (1.50), 7.550 (1.58), 7.569 (2.52), 7.573 (2.48), 7.592 (1.46), 7.599 (1.35), 7.805 (1.80), 7.814 (1.69), 7.828 (1.69), 8.267 (10.07), 8.290 (9.31), 8.613 (7.25), 10.467 (5.07), 10.491 (4.77).
LC-MS (Methode 3): $R_t$ = 2.02 min; MS (ESIpos): m/z = 523 $[M+H]^+$

## Beispiel 355

1-(2,4-Difluorphenyl)-7-[7-hydroxy-5-azaspiro[2.4]hept-5-yl]-4-oxo-*N*-[(2*R*)-1,1,1-trifluorbutan-2-yl]-1,4-dihydro-1,8-naphthyridin-3-carbonsäureamid (Diastereomerengemisch)

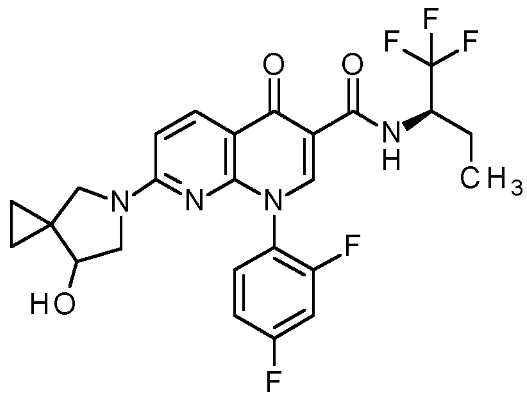

Gemäß AAV3 wurden 50.0 mg (112 μmol) der Verbindung aus Beispiel 67A mit 21.2 mg (95% Reinheit, 135 μmol) 3-Azabicyclo[4.1.0]heptan-1-ol Hydrochlorid in Gegenwart von 68.0μl (393 μmol) *N,N*-Diisopropylethylamin in 0.5 ml Dimethylformamid zur Reaktion gebracht. Die Reaktionsmischung wurde mit 1 ml Acetonitril und 0.1 ml 1N wässrige Salzsäure verdünnt und mittels präparativer HPLC (Säule: Chromatorex C18, 10 μm, 125x30 mm, LM: Acetonitril / 0.1 % Ameisensäure-Gradient; (0 bis 5 min. 10% Acetonitril, während 14 min. nach 90 % Acetonitril und weitere 4 min. 90 % Acetonitril) gereinigt. Es wurden 48.8 mg (82% d. Th., 99% Reinheit) der Titelverbindung erhalten

$^1$H-NMR (400 MHz, DMSO-d6) δ [ppm]: -0.008 (3.32), 0.008 (3.21), 0.470 (1.19), 0.585 (4.12), 0.818 (1.66), 0.949 (7.22), 0.968 (16.00), 0.986 (7.77), 1.598 (1.12), 1.616 (1.44), 1.624 (1.34), 1.633 (1.77), 1.642 (1.63), 1.651 (1.52), 1.659 (1.73), 1.677 (1.30), 1.850 (1.30), 1.860 (1.52), 1.869 (1.52), 1.879 (1.73), 1.885 (1.52), 1.895 (1.30), 1.904 (1.16), 1.913 (0.98), 2.367 (1.23), 2.563 (1.05), 2.711 (1.26), 3.168 (0.87), 3.202 (1.12), 3.228 (1.08), 3.288 (3.36), 3.298 (2.93), 3.334 (1.52), 3.344 (1.34), 3.355 (1.26), 3.624 (1.59), 3.668 (1.08), 3.741 (1.16), 4.734 (1.34), 4.753 (1.30), 4.942 (0.76), 4.987 (0.79), 6.655 (1.48), 6.677 (1.55), 6.776 (0.79), 6.798 (0.76), 7.326 (1.55), 7.571 (1.23), 7.812 (1.52), 8.277 (4.19), 8.300 (4.05), 8.614 (2.96), 10.504 (5.24), 10.528 (5.06).
LC-MS (Methode 3): $R_t$ = 2.05 min; MS (ESIpos): m/z = 523 $[M+H]^+$

**Beispiel 356**

1-(2,4-Difluorphenyl)-7-[4-hydroxy-3,3-dimethylpyrrolidin-1-yl]-4-oxo-*N*-[(2*R*)-1,1,1-trifluorbutan-2-yl]-1,4-dihydro-1,8-naphthyridin-3-carbonsäureamid (Diastereomerengemisch)

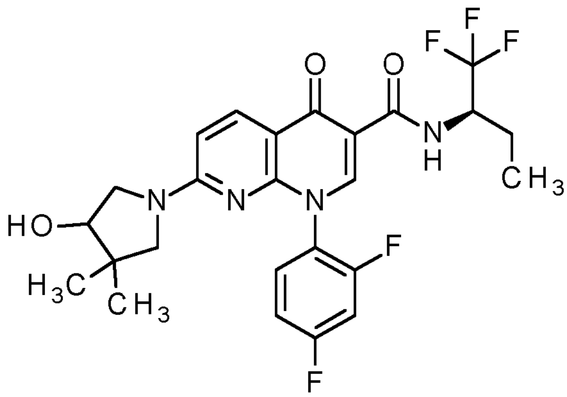

Gemäß AAV3 wurden 50.0 mg (112 μmol) der Verbindung aus Beispiel 67A mit 21.5 mg (95% Reinheit, 135 μmol) 4,4-Dimethylpyrrolidin-3-ol Hydrochlorid in Gegenwart von 68.0ul (393 μmol) *N,N*-Diisopropylethylamin in 0.5 ml Dimethylformamid zur Reaktion gebracht. Die Reaktionsmischung wurde mit 1 ml Acetonitril und 0.1 ml 1N wässrige Salzsäure verdünnt und mittels präparativer HPLC (Säule: Chromatorex C18, 10 μm, 125x30 mm, LM: Acetonitril / 0.1 % Ameisensäure-Gradient; (0 bis 5 min. 10% Acetonitril, während 14 min. nach 90 % Acetonitril und weitere 4 min. 90 % Acetonitril) gereinigt. Es wurden 52.4 mg (88% d. Th., 99% Reinheit) der Titelverbindung erhalten.
$^1$H-NMR (400 MHz, DMSO-d6) δ [ppm] = 10.51 (d, 1H), 8.60 (s, 1H), 8.27 (d, 1H), 7.86-7.76 (m, 1H), 7.63-7.56 (m, 1H), 7.37-7.29 (m, 1H), 6.73 (d, 1H), 5.21-5.00 (m, 1H), 4.81-4.67 (m, 1H), 3.86-3.64 (m, 1.5H), 3.07-2.82 (m, 1.5H), 1.93-1.82 (m, 1H), 1.70-1.57 (m, 1H), 1.04-0.84 (m, 9H), 2H unter dem Wassersignal.
LC-MS (Methode 3): $R_t$ = 2.13 min; MS (ESIpos): m/z = 525 $[M+H]^+$

**Beispiel 357**

7-[(4*S*)-4-Hydroxy-2-oxopyrrolidin-1-yl]-4-oxo-*N*-[(2*R*)-1,1,1-trifluor-3,3-dimethylbutan-2-yl]-1-(2,4,6-trifluorphenyl)-1,4-dihydro-1,8-naphthyridin-3-carbonsäureamid

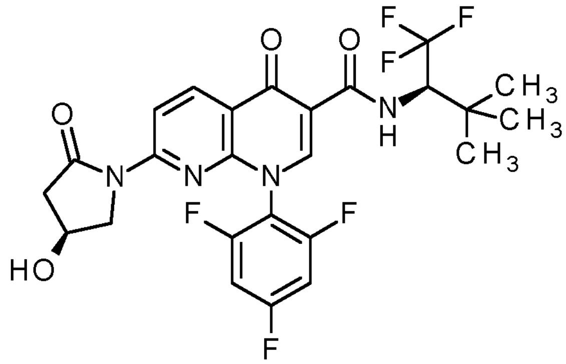

Gemäß AAV1 wurden 50.0 mg (119 μmol) der Verbindung aus Beispiel 117A mit 22.2 mg (143 μmol) (2*R*)-1,1,1-Trifluor-3,3-dimethylbutan-2-amin in Gegenwart von 68.0mg (179 μmol) HATU und 41.5 μl (240 μmol) *N,N*-Diisopropylethylamin in 1.2 ml Dimethylformamid zur Reaktion gebracht. Die Reaktionsmischung wurde mit 2 ml Acetonitril und 1 ml Wasser verdünnt und mittels präparativer HPLC (Säule: Chromatorex C18, 10 μm, 125x30 mm, LM: Acetonitril / 0.1 % Ameisensäure-Gradient; (0 bis 5 min. 10% Acetonitril, während 14 min. nach 90 % Acetonitril und weitere 4 min. 90 % Acetonitril) gereinigt. Es wurden 56.2 mg (84% d. Th., 99% Reinheit) der Titelverbindung erhalten.
$^1$H-NMR (400 MHz, DMSO-d6) δ [ppm]: -0.008 (0.93), 0.008 (0.57), 1.103 (16.00), 2.358 (0.92), 2.401 (1.06), 2.918 (0.90), 2.933 (0.92), 2.961 (0.82), 2.976 (0.78), 3.289 (1.34), 3.465 (0.91), 3.495 (1.09), 3.674 (0.83), 3.686 (0.99), 3.704 (0.76), 3.716 (0.65), 4.291 (0.70), 4.300 (0.67), 4.626 (0.57), 4.651 (0.73), 5.336 (2.14), 5.345 (2.04), 7.596 (0.65), 7.601 (0.72), 7.616 (1.15), 7.624 (1.13), 7.639 (0.68), 8.535 (3.00), 8.557 (3.38), 8.739 (3.49), 8.761 (2.76), 9.087 (3.75), 10.467 (1.27), 10.492 (1.18).
LC-MS (Methode 3): $R_t$ = 2.05 min; MS (ESIpos): m/z = 557 $[M+H]^+$

## Beispiel 358

N-(Bicyclo[1.1.1]pent-1-yl)-7-[(4S)-4-hydroxy-2-oxopyrrolidin-1-yl]-4-oxo-1-(2,4,6-trifluorphenyl)-1,4-dihydro-1,8-naphthyridin-3-carboxamid

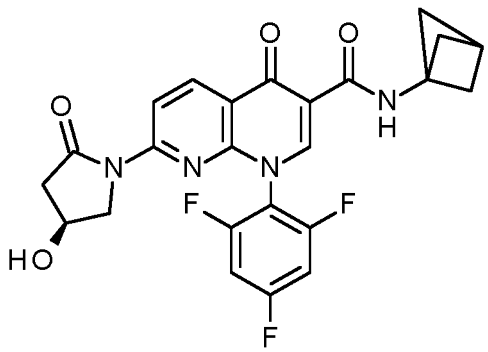

Gemäß AAV1 wurden 45.0 mg (107 μmol) der Verbindung aus Beispiel 117A mit 15.4 mg (129 μmol) Bicyclo[1.1.1]pentan-1-amin Hydrochlorid in Gegenwart von 61.2 mg (161 μmol) HATU und 37.4 μl (210 μmol) *N,N*-Diisopropylethylamin in 2.0 ml Dimethylformamid zur Reaktion gebracht. Die Reaktionsmischung wurde mit 2 ml Acetonitril und 1 ml Wasser verdünnt und mittels präparativer HPLC (Säule: Chromatorex C18, 10 μm, 125x30 mm, LM: Acetonitril / 0.1 % Ameisensäure-Gradient; (0 bis 5 min. 10% Acetonitril, während 14 min. nach 90 % Acetonitril und weitere 4 min. 90 % Acetonitril) gereinigt. Es wurden 31.9 mg (61% d. Th., 100% Reinheit) der Titelverbindung erhalten.
$^1$H-NMR (400 MHz, DMSO-d6) δ [ppm]: 2.113 (16.00), 2.392 (0.81), 2.910 (0.63), 2.925 (0.66), 3.456 (0.70), 3.486 (0.85), 3.678 (0.73), 5.326 (0.89), 5.335 (0.88), 8.505 (1.28), 8.527 (1.65), 8.663 (1.35), 8.665 (1.49), 8.687 (1.16), 8.927 (2.46), 10.002 (1.75).
LC-MS (Methode 3): $R_t$ = 1.76 min; MS (ESIpos): m/z = 485 $[M+H]^+$

**Beispiel 359**

4-Oxo-7-(2-oxoimidazolidin-1-yl)-N-[(2S)-1,1,1-trifluor-3,3-dimethylbutan-2-yl]-1-(2,4,6-trifluorphenyl)-1,4-dihydro-1,8-naphthyridin-3-carboxamid

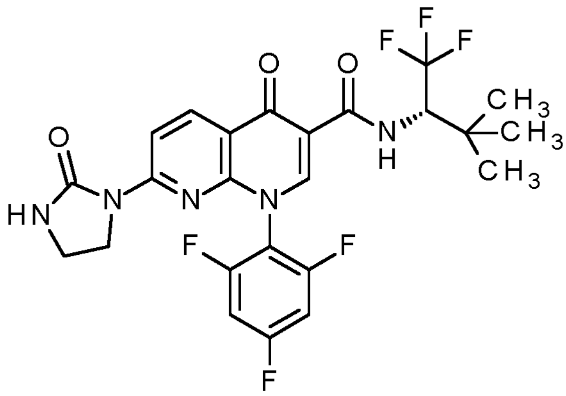

Gemäß AAV1 wurden 30.0 mg (74.2 µmol) der Verbindung aus Beispiel 113A mit 13.8 mg (89.0 µmol) (2*S*)-1,1,1-Trifluor-3,3-dimethylbutan-2-amin in Gegenwart von 33.9 mg (89.0 µmol) HATU und 32.0 µl (190 µmol) *N,N*-Diisopropylethylamin in 1.0 ml Dimethylformamid zur Reaktion gebracht. Die Reaktionsmischung wurde mit 2 ml DMSO , Wasser und 1 ml wässriger 1N Salzsäure verdünnt und mittels präparativer HPLC (Säule: Chromatorex C18, 10 µm, 125x30 mm, LM: Acetonitril / 0.1 % Ameisensäure-Gradient; (0 bis 5 min. 10% Acetonitril, während 14 min. nach 90 % Acetonitril und weitere 4 min. 90 % Acetonitril) gereinigt. Es wurden 31.5 mg (78% d. Th., 99% Reinheit) der Titelverbindung erhalten.

$^1$H-NMR (400 MHz, DMSO-d6) δ [ppm]: 1.098 (16.00), 3.351 (1.68), 3.372 (1.25), 3.581 (1.30), 3.597 (1.13), 3.602 (1.65), 3.621 (0.93), 4.642 (0.74), 7.556 (1.06), 7.578 (1.77), 7.599 (1.05), 7.674 (1.85), 8.434 (2.49), 8.456 (3.12), 8.581 (3.18), 8.603 (2.40), 9.009 (3.62), 10.558 (1.22), 10.584 (1.17).

LC-MS (Methode 3): $R_t$ = 2.12 min; MS (ESIpos): m/z = 542 $[M+H]^+$

**Beispiel 360**

4-Oxo-7-(2-oxoimidazolidin-1-yl)-*N*-[(2*R*)-1,1,1-trifluor-3,3-dimethylbutan-2-yl]-1-(2,4,6-trifluorphenyl)-1,4-dihydro-1,8-naphthyridin-3-carbonsäureamid

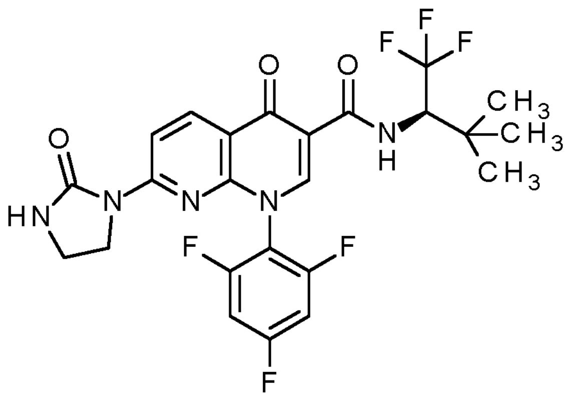

Gemäß AAV1 wurden 30.0 mg (74.2 µmol) der Verbindung aus Beispiel 113A mit 13.8 mg (89.0 µmol) (2*R*)-1,1,1-Trifluor-3,3-dimethylbutan-2-amin in Gegenwart von 33.9 mg (89.0 µmol) HATU und 32.0 µl (190 µmol) *N,N*-Diisopropylethylamin in 1.0 ml Dimethylformamid zur Reaktion gebracht. Die Reaktionsmischung wurde mit 2 ml DMSO, Wasser und 1 ml wässriger 1N Salzsäure verdünnt und mittels präparativer HPLC (Säule: Chromatorex C18, 10 µm,

125x30 mm, LM: Acetonitril / 0.1 % Ameisensäure-Gradient; (0 bis 5 min. 10% Acetonitril, während 14 min. nach 90 % Acetonitril und weitere 4 min. 90 % Acetonitril) gereinigt. Es wurden 33.2 mg (82% d. Th., 99% Reinheit) der Titelverbindung erhalten.
$^1$H-NMR (400 MHz, DMSO-d6) δ [ppm]: -0.008 (0.92), 0.008 (0.92), 1.098 (16.00), 3.351 (1.77), 3.371 (1.30), 3.580 (1.30), 3.596 (1.15), 3.601 (1.65), 3.620 (0.92), 4.641 (0.74), 7.555 (1.03), 7.578 (1.74), 7.599 (1.00), 7.673 (1.83), 8.433 (2.33), 8.455 (2.98), 8.580 (2.95), 8.603 (2.27), 9.009 (3.42), 10.558 (1.21), 10.583 (1.15).
LC-MS (Methode 3): $R_t$ = 2.13 min; MS (ESIpos): m/z = 542 $[M+H]^+$

## Beispiel 361

7-[3-(2-Hydroxyethyl)-2-oxoimidazolidin-1-yl]-4-oxo-*N*-[(2*R*)-1,1,1-trifluor-3,3-dimethylbutan-2-yl]-1-(2,4,6-trifluorphenyl)-1,4-dihydro-1,8-naphthyridin-3-carbonsäureamid

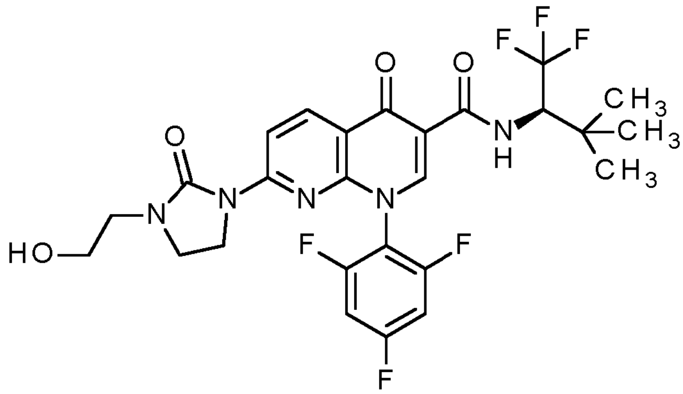

Gemäß AAV1 wurden 30 mg (67 µmol) der Verbindung aus Beispiel 123A mit 12.5 mg (80.3 µmol) (2*R*)-1,1,1-Trifluor-3,3-dimethylbutan-2-amin in Gegenwart von 30.5 mg (80.3 µmol) HATU und 29 µl (0.17 mmol) *N,N*-Diisopropylethylamin in 1.0 ml Dimethylformamid zur Reaktion gebracht. Die Reaktionsmischung wurde mit 2 ml DMSO, Wasser und 1 ml wässriger 1N Salzsäure verdünnt und mittels präparativer HPLC (Säule: Chromatorex C18, 10 µm, 125x30 mm, LM: Acetonitril / 0.1 % Ameisensäure-Gradient; (0 bis 5 min. 10% Acetonitril, während 14 min. nach 90 % Acetonitril und weitere 4 min. 90 % Acetonitril) gereinigt. Es wurden 33.3 mg (85% d. Th., 100% Reinheit) der Titelverbindung erhalten
$^1$H-NMR (400 MHz, DMSO-d6) δ [ppm]: 1.098 (16.00), 3.260 (1.13), 3.275 (2.52), 3.288 (1.60), 3.490 (1.10), 3.505 (2.50), 3.519 (2.79), 3.526 (2.42), 3.533 (2.77), 3.545 (1.40), 4.641 (0.73), 4.727 (0.93), 4.741 (2.09), 4.755 (0.89), 7.558 (1.06), 7.580 (1.87), 7.602 (1.03), 8.437 (2.12), 8.459 (2.61), 8.594 (2.67), 8.616 (1.99), 9.009 (3.66), 10.554 (1.19), 10.579 (1.13).
LC-MS (Methode 3): $R_t$ = 2.03 min; MS (ESIpos): m/z = 586 $[M+H]^+$

## Beispiel 362

7-[3-(2-Hydroxyethyl)-2-oxoimidazolidin-1-yl]-4-oxo-*N*-[(2*S*)-1,1,1-trifluorbutan-2-yl]-1-(2,4,6-trifluorphenyl)-1,4-dihydro-1,8-naphthyridin-3-carbonsäureamid

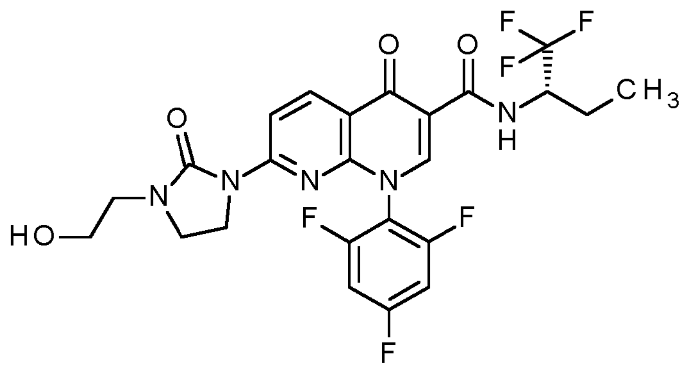

Gemäß AAV1 wurden 30 mg (67 µmol) der Verbindung aus Beispiel 123A mit 13.1 mg (80.3 µmol) (2*S*)-1,1,1-Trifluorbutan-2-amin Hydrochlorid in Gegenwart von 38.2 mg (100 µmol) HATU und 29 µl (0.17 mmol) *N,N*-Diisopropylethylamin in 1.0 ml Dimethylformamid zur Reaktion gebracht. Es wurde mit 1 ml Wasser und 2 ml Acetonitril verdünnt und die Lösung mittels präparativer HPLC (Säule: Chromatorex C18, 10 µm, 125x30 mm, LM: Acetonitril / 0.1% Ameisensäure-Gradient; (0 bis 5 min. 10% Acetonitril, während 14 min. nach 90 % Acetonitril und weitere 4 min. 90 % Acetonitril) gereinigt. Es wurden 33.3 mg (89% d. Th., 100% Reinheit) der Titelverbindung erhalten.
$^1$H-NMR (400 MHz, DMSO-d6) δ [ppm]: -0.008 (2.42), 0.008 (2.11), 0.959 (7.16), 0.978 (16.00), 0.996 (7.76), 1.624 (1.00), 1.641 (1.49), 1.659 (1.80), 1.684 (1.69), 1.702 (1.29), 1.881 (1.58), 1.891 (1.67), 1.915 (1.27), 2.328 (1.22), 2.524 (3.47), 2.670 (1.36), 3.259 (4.78), 3.273 (10.56), 3.287 (6.18), 3.489 (4.40), 3.504 (10.07), 3.518 (11.49), 3.524 (9.87), 3.532 (11.36), 3.543 (5.64), 4.727 (4.56), 4.741 (9.96), 4.755 (5.24), 7.558 (4.78), 7.581 (8.69), 7.603 (4.67), 8.434 (9.07), 8.456 (11.67), 8.563 (12.18), 8.586 (8.51), 8.996 (15.76), 10.195 (5.09), 10.219 (5.00).
LC-MS (Methode 3): $R_t$ = 1.86 min; MS (ESIpos): m/z = 558 $[M+H]^+$

**Beispiel 363**

*N*-[(1*S*)-1-Cyclopropyl-2,2,2-trifluorethyl]-7-[3-(2-hydroxyethyl)-2-oxoimidazolidin-1-yl]-4-oxo-1-(2,4,6-trifluorphenyl)-1,4-dihydro-1,8-naphthyridin-3-carbonsäureamid

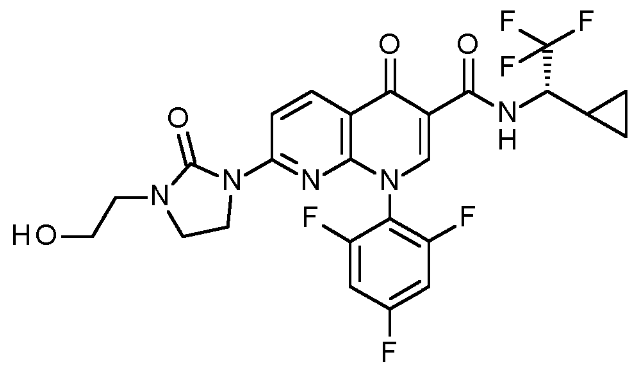

Gemäß AAV1 wurden 30.0 mg (66.9 µmol) der Verbindung aus Beispiel 123A mit 14.1 mg (80.3 µmol) (1*S*)-1-Cyclopropyl-2,2,2-trifluorethanamin Hydrochlorid in Gegenwart von 38.2 mg (100 µmol) HATU und 29 µl (0.17 mmol) *N,N*-Diisopropylethylamin in 1.0 ml Dimethylformamid zur Reaktion gebracht. Es wurde mit 1 ml Wasser und 2 ml Acetonitril verdünnt und die Lösung mittels präparativer HPLC (Säule: Chromatorex C18, 10 µm, 125x30 mm, LM: Acetonitril / 0.1 % Ameisensäure-Gradient; (0 bis 5 min. 10% Acetonitril, während 14 min. nach 90 % Acetonitril und weitere 4 min. 90 % Acetonitril) gereinigt. Es wurden 30.9 mg (81% d. Th., 100% Reinheit) der Titelverbindung erhalten
$^1$H-NMR (400 MHz, DMSO-d6) δ [ppm]: -0.008 (1.99), 0.008 (1.85), 0.331 (2.01), 0.342 (3.30), 0.354 (3.18), 0.365 (2.27), 0.377 (1.33), 0.528 (2.08), 0.540 (3.18), 0.553 (3.20), 0.559 (3.65), 0.564 (2.99), 0.577 (3.84), 0.586 (2.81), 0.596 (2.55), 0.607 (2.06), 0.621 (1.24), 0.635 (1.64), 0.645 (1.73), 0.656 (3.35), 0.659 (2.55), 0.666 (2.60), 0.672 (2.22), 0.677 (2.25), 0.680 (2.04), 0.687 (1.19), 0.692 (1.29), 0.699 (0.77), 1.199 (1.29), 1.207 (1.85), 1.220 (3.11), 1.228 (2.27), 1.240 (3.09), 1.252 (1.68), 1.260 (1.15), 2.328 (0.80), 2.524 (2.85), 2.671 (0.87), 3.260 (5.94), 3.274 (13.33), 3.288 (7.79), 3.463 (1.64), 3.478 (2.60), 3.493 (5.80), 3.498 (5.57), 3.504 (12.91), 3.519 (14.90), 3.524 (13.05), 3.532 (14.29), 3.542 (7.18),

3.547 (6.22), 3.566 (1.82), 4.372 (1.61), 4.393 (2.83), 4.415 (2.78), 4.435 (1.50), 4.726 (4.35), 4.740 (10.06), 4.754 (4.33), 7.547 (1.64), 7.555 (5.61), 7.570 (2.67), 7.578 (10.74), 7.585 (2.90), 7.600 (5.68), 7.608 (1.85), 8.435 (10.50), 8.457 (13.99), 8.567 (13.92), 8.590 (10.32), 8.988 (16.00), 10.336 (6.69), 10.360 (6.41).
LC-MS (Methode 3): $R_t$ = 1.88 min; MS (ESIpos): m/z = 570 $[M+H]^+$

**Beispiel 364**

N-[(1S)-1-Cyclopropyl-2,2,2-trifluorethyl]-7-[3-(2-hydroxyethyl)-2-oxotetrahydropyrimidin-1(2H)-yl]-4-oxo-1-(2,4,6-trifluorphenyl)-1,4-dihydro-1,8-naphthyridin-3-carboxamid

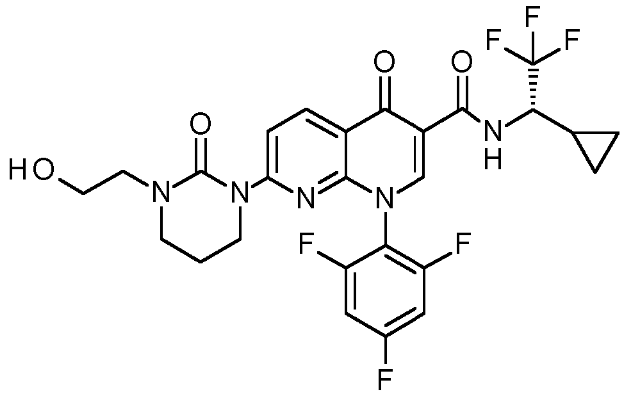

Gemäß AAV1 wurden 54mg (78% Reinheit, 91 µmol) der Verbindung aus Beispiel 125A mit 19.2 mg (109 µmol) (1*S*)-1-Cyclopropyl-2,2,2-trifluorethanamin Hydrochlorid in Gegenwart von 41.6 mg (109 µmol) HATU und 40 µl (0.23 mmol) *N,N*-Diisopropylethylamin in 1.5 ml Dimethylformamid zur Reaktion gebracht. Es wurde mit 1 ml wässriger 1N Salzsäure und 2 ml DMSO verdünnt und die Lösung mittels präparativer HPLC (Säule: Chromatorex C18, 10 µm, 125x30 mm, LM: Acetonitril / 0.1 % Ameisensäure-Gradient; (0 bis 5 min. 10% Acetonitril, während 14 min. nach 90 % Acetonitril und weitere 2 min. 90 % Acetonitril) gereinigt. Es wurden 33.9 mg (63% d. Th., 99% Reinheit) der Titelverbindung erhalten
$^1$H-NMR (400 MHz, DMSO-d6) δ [ppm]: -0.149 (0.60), -0.008 (6.40), 0.008 (4.91), 0.146 (0.64), 0.333 (1.84), 0.343 (3.22), 0.355 (3.03), 0.366 (2.03), 0.378 (1.34), 0.534 (1.96), 0.545 (2.95), 0.560 (3.80), 0.578 (4.09), 0.588 (2.73), 0.599 (2.38), 0.609 (1.93), 0.623 (1.14), 0.637 (1.49), 0.647 (1.71), 0.658 (3.30), 0.668 (2.51), 0.676 (2.16), 0.693 (1.27), 1.187 (0.64), 1.200 (1.27), 1.208 (1.74), 1.220 (2.95), 1.229 (2.16), 1.241 (2.85), 1.253 (1.51), 1.261 (1.09), 1.274 (0.47), 1.878 (1.44), 1.893 (4.09), 1.907 (6.28), 1.922 (4.29), 1.937 (1.56), 2.001 (0.64), 2.323 (1.04), 2.328 (1.44), 2.332 (1.04), 2.366 (0.89), 2.519 (6.57), 2.524 (5.16), 2.665 (1.22), 2.670 (1.64), 2.675 (1.17), 2.710 (1.02), 3.374 (9.15), 3.384 (16.00), 3.398 (9.35), 3.403 (8.04), 3.496 (6.08), 3.511 (8.53), 3.526 (9.23), 3.541 (10.37), 3.555 (9.13), 3.570 (2.98), 4.368 (1.54), 4.389 (2.63), 4.410 (2.60), 4.431 (1.36), 4.695 (3.97), 4.709 (8.81), 4.723 (3.84), 7.571 (5.28), 7.593 (10.15), 7.615 (5.43), 7.623 (1.79), 8.163 (11.66), 8.186 (12.73), 8.500 (12.92), 8.522 (11.44), 9.009 (15.93), 10.318 (6.25), 10.342 (5.98).
LC-MS (Methode 3): $R_t$ = 1.87 min; MS (ESIpos): m/z = 584 $[M+H]^+$

**Beispiel 365**

7-[3-(2-Hydroxyethyl)-2-oxotetrahydropyrimidin-1(2*H*)-yl]-4-oxo-*N*-[(2*S*)-1,1,1-trifluorbutan-2-yl]-1-(2,4,6-trifluorphenyl)-1,4-dihydro-1,8-naphthyridin-3-carbonsäureamid

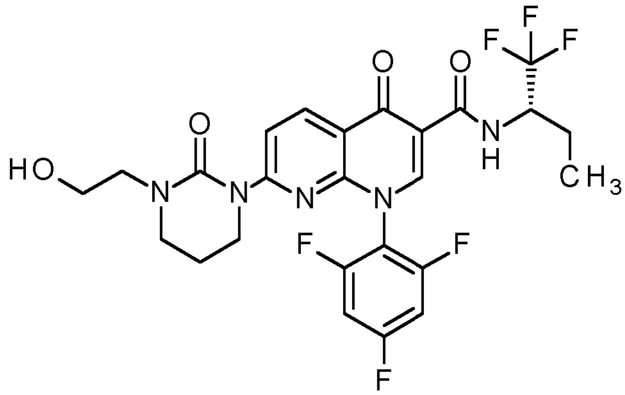

Gemäß AAV1 wurden 54 mg (78% Reinheit, 91 µmol) der Verbindung aus Beispiel 125A mit 13.9 mg (109 µmol) (2*S*)-1,1,1-Trifluorbutan-2-amin in Gegenwart von 41.6 mg (109 µmol) HATU und 56 µl (0.32 mmol) *N,N*-Diisopropylethylamin in 1.5 ml Dimethylformamid zur Reaktion gebracht. Es wurde mit 1 ml wässriger 1N Salzsäure und 2 ml DMSO verdünnt und die Lösung mittels präparativer HPLC (Säule: Chromatorex C18, 10 µm, 125x30 mm, LM: Acetonitril / 0.1 % Ameisensäure-Gradient; (0 bis 5 min. 10% Acetonitril, während 14 min. nach 90 % Acetonitril und weitere 2 min. 90 % Acetonitril) gereinigt. Es wurden 34.3 mg (65% d. Th., 99% Reinheit) der Titelverbindung erhalten
$^1$H-NMR (400 MHz, DMSO-d6) δ [ppm]: -0.008 (2.74), 0.008 (2.26), 0.961 (7.23), 0.980 (16.00), 0.998 (7.82), 1.626 (1.06), 1.644 (1.46), 1.651 (1.28), 1.661 (1.79), 1.670 (1.57), 1.679 (1.50), 1.687 (1.72), 1.705 (1.28), 1.863 (1.46), 1.873 (2.05), 1.882 (2.48), 1.892 (5.19), 1.898 (4.38), 1.908 (6.72), 1.917 (3.98), 1.923 (4.27), 1.938 (1.57), 3.369 (5.52), 3.374 (7.82), 3.384 (13.41), 3.389 (11.76), 3.398 (7.96), 3.404 (6.65), 3.497 (5.19), 3.512 (7.27), 3.527 (8.26), 3.541 (9.39), 3.555 (8.26), 3.570 (2.59), 4.693 (4.02), 4.707 (9.13), 4.721 (4.24), 4.759 (1.42), 4.778 (1.35), 7.566 (1.28), 7.574 (4.42), 7.596 (8.15), 7.618 (4.46), 7.625 (1.39), 8.163 (9.86), 8.185 (10.85), 8.497 (10.85), 8.519 (9.64), 9.017 (13.11), 10.174 (5.19), 10.198 (4.97).
LC-MS (Methode 3): $R_t$ = 1.86 min; MS (ESIpos): m/z = 572 $[M+H]^+$

## Beispiel 366

N-(Bicyclo[1.1.1]pent-1-yl)-7-[(3R,4R)-3,4-dihydroxypyrrolidin-1-yl]-4-oxo-1-(2,4,6-trifluorphenyl)-1,4-dihydro-1,8-naphthyridin-3-carboxamid

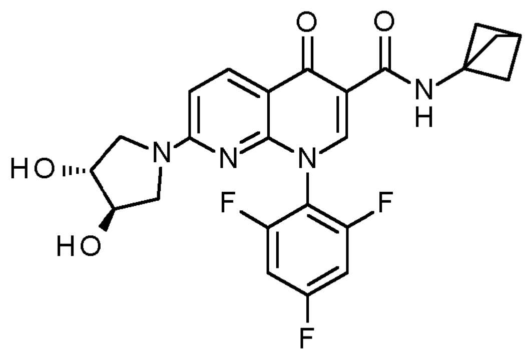

Gemäß AAV1 wurden 50.0 mg (119 µmol) der Verbindung aus Beispiel 121A mit 17.0 mg (142 µmol) Bicyclo[1.1.1]pentan-1-amin Hydrochlorid in Gegenwart von 54.1 mg (142 µmol) HATU und 72 µl (0.42 mmol) *N,N*-Diisopropylethylamin in 1.5 ml Dimethylformamid zur Reaktion gebracht. Es wurde mit 1 ml wässriger 1N Salzsäure und 2 ml DMSO verdünnt und die Lösung mittels präparativer HPLC (Säule: Chromatorex C18, 10 µm, 125x30 mm, LM: Acetonitril / 0.1 % Ameisensäure-Gradient; (0 bis 5 min. 10% Acetonitril, während 14 min. nach 90 % Acetonitril und weitere 2 min. 90 % Acetonitril) gereinigt. Es wurden 48.8 mg (84% d. Th., 99% Reinheit) der Titelverbindung erhalten
$^1$H-NMR (400 MHz, DMSO-d6) δ [ppm]: -0.008 (1.55), 0.008 (1.50), 2.073 (0.58), 2.093 (16.00), 2.476 (3.02), 5.136 (0.77), 5.218 (0.77), 6.738 (1.36), 6.760 (1.37), 7.568 (0.82), 8.230 (1.64), 8.253 (1.57), 8.660 (2.53), 10.283 (1.64).
LC-MS (Methode 1): $R_t$ = 0.90 min; MS (ESIpos): m/z = 487 $[M+H]^+$

**Beispiel 367**

N-(3-Fluorbicyclo[1.1.1]pent-1-yl)-7-[(4S)-4-hydroxy-2-oxopyrrolidin-1-yl]-4-oxo-1-(2,4,6-trifluorphenyl)-1,4-dihydro-1,8-naphthyridin-3-carboxamid

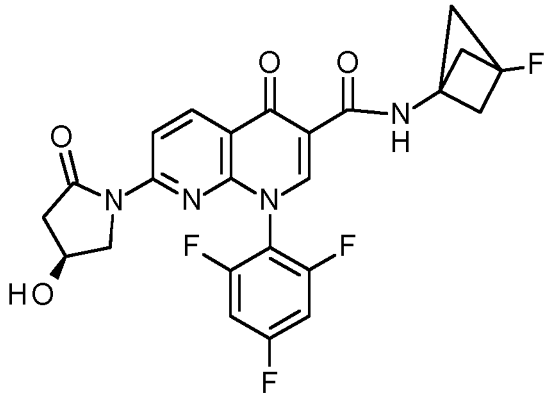

Gemäß AAV1 wurden 50.0 mg (119 μmol) der Verbindung aus Beispiel 117A mit 19.7 mg (143 μmol) 3-Fluorbicyclo[1.1.1]pentan-1-amin Hydrochlorid in Gegenwart von 54.4 mg (143 μmol) HATU und 73 μl (0.42 mmol) *N,N*-Diisopropylethylamin in 1.5 ml Dimethylformamid zur Reaktion gebracht. Es wurde mit 1 ml wässriger 1N Salzsäure und 2 ml DMSO verdünnt und die Lösung mittels präparativer HPLC (Säule: Chromatorex C18, 10 μm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 45.1 mg (68% d. Th., 90% Reinheit) der Titelverbindung erhalten
$^1$H-NMR (400 MHz, DMSO-d6) δ [ppm]: -0.149 (0.64), -0.008 (5.57), 0.008 (5.29), 2.073 (2.43), 2.113 (11.93), 2.328 (1.00), 2.332 (0.86), 2.349 (4.57), 2.393 (5.29), 2.670 (1.07), 2.675 (0.79), 2.911 (4.64), 2.926 (4.86), 2.954 (4.36), 2.969 (4.21), 3.339 (1.79), 3.454 (4.64), 3.484 (5.64), 3.665 (4.14), 3.677 (5.14), 3.695 (4.00), 3.707 (3.50), 4.292 (3.43), 5.324 (5.93), 5.332 (6.43), 7.595 (2.71), 7.601 (3.29), 7.614 (4.93), 7.624 (5.00), 7.637 (3.29), 7.644 (2.64), 8.505 (1.43), 8.513 (11.86), 8.527 (1.93), 8.535 (14.64), 8.665 (1.79), 8.672 (14.64), 8.687 (1.50), 8.694 (11.43), 8.926 (1.64), 8.964 (16.00), 10.003 (1.14), 10.119 (12.29).
LC-MS (Methode 3): $R_t$ = 1.74 min; MS (ESIpos): m/z = 503 $[M+H]^+$

**Beispiel 368**

7-[(3R,4R)-3,4-Dihydroxypyrrolidin-1-yl]-N-(3-fluorbicyclo[1.1.1]pent-1-yl)-4-oxo-1-(2,4,6-trifluorphenyl)-1,4-dihydro-1,8-naphthyridin-3-carboxamid

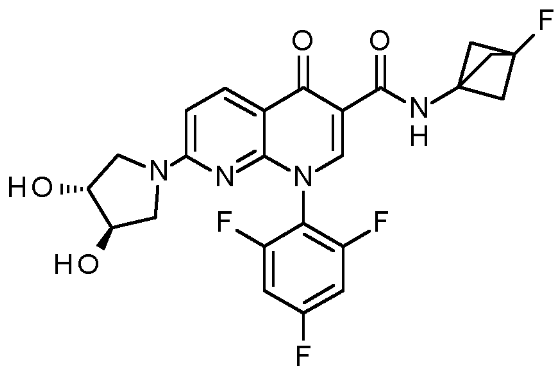

Gemäß AAV1 wurden 50.0 mg (119 μmol) der Verbindung aus Beispiel 121A mit 19.6 mg (142 μmol) 3-Fluorbicyclo[1.1.1]pentan-1-aminhydrochlorid in Gegenwart von 54.1 mg (142 μmol) HATU und 73 μl (0.420 mmol) *N,N*-Diisopropylethylamin in 1.2 ml Dimethylformamid zur Reaktion gebracht. Es wurde mit 1 ml wässriger 1N Salzsäure und 2 ml DMSO verdünnt und die Lösung mittels präparativer HPLC (Säule: Chromatorex C18, 10 μm, 125x30 mm, LM: Acetonitril / 0.05% Ameisensäure-Gradient; 0 bis 3 min. 10% Acetonitril bis 35 min. 90% Acetonitril und weitere 3

min. 90% Acetonitril) gereinigt. Es wurden 50.1 mg (75% d. Th., 90% Reinheit) der Titelverbindung erhalten
$^1$H-NMR (400 MHz, DMSO-d6) δ [ppm]: -0.008 (3.60), 0.008 (3.27), 2.073 (16.00), 2.094 (9.42), 2.476 (2.09), 3.047 (2.50), 3.079 (3.34), 3.222 (2.02), 3.231 (2.25), 3.253 (1.66), 3.264 (1.66), 3.337 (3.32), 3.586 (1.79), 3.595 (2.02), 3.612 (1.63), 3.623 (1.48), 3.922 (3.11), 4.044 (3.06), 5.131 (4.39), 5.139 (4.31), 5.221 (4.39), 5.230 (4.21), 6.738 (0.94), 6.747 (7.09), 6.760 (1.07), 6.770 (7.22), 7.546 (2.58), 7.562 (4.49), 7.569 (4.52), 7.577 (1.68), 7.585 (2.65), 8.231 (1.48), 8.236 (8.55), 8.253 (1.51), 8.258 (8.04), 8.660 (1.38), 8.700 (12.48), 10.283 (0.92), 10.425 (9.08).
LC-MS (Methode 3): $R_t$ = 1.57 min; MS (ESIpos): m/z = 505 $[M+H]^+$

**Beispiel 369**

7-[(3*R*,4*R*)-3,4-Dihydroxypyrrolidin-1-yl]-4-oxo-*N*-[(2*S*)-1,1,1-trifluor-3,3-dimethylbutan-2-yl]-1-(2,4,6-trifluorphenyl)-1,4-dihydro-1,8-naphthyridin-3-carbonsäureamid

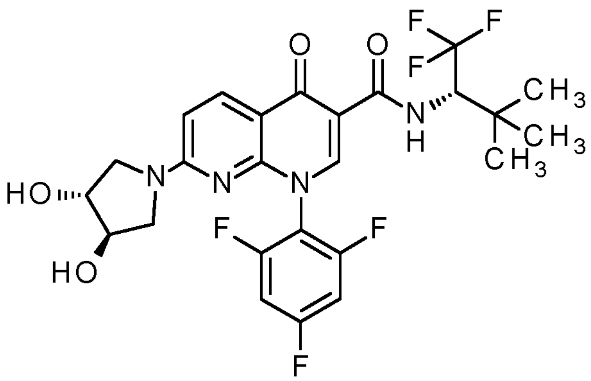

Gemäß AAV1 wurden 30 mg (71 μmol) der Verbindung aus Beispiel 121A mit 13 mg (85 μmol) (2*S*)-1,1,1-Trifluor-3,3-dimethylbutan-2-amin in Gegenwart von 32 mg (85 μmol) HATU und 43 μl (0.25 mmol) *N,N*-Diisopropylethylamin in 1.0 ml Dimethylformamid zur Reaktion gebracht. Es wurde mit 1 ml wässriger 1N Salzsäure und 2 ml DMSO verdünnt und die Lösung mittels präparativer HPLC (Säule: Chromatorex C18, 10 μm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 32.4 mg (81% d. Th., 100% Reinheit) der Titelverbindung erhalten
$^1$H-NMR (400 MHz, DMSO-d6) δ [ppm]: -0.008 (2.17), 0.008 (2.14), 1.089 (16.00), 3.059 (0.72), 3.090 (0.96), 3.241 (0.66), 3.328 (1.22), 3.355 (0.91), 3.610 (0.59), 3.929 (0.86), 4.052 (0.86), 4.621 (0.73), 5.138 (1.04), 5.146 (1.08), 5.236 (1.02), 5.245 (1.02), 6.770 (2.04), 6.792 (2.09), 7.548 (0.63), 7.560 (0.95), 7.570 (0.98), 7.583 (0.64), 8.295 (2.41), 8.318 (2.25), 8.815 (3.51), 10.764 (1.24), 10.790 (1.18).
LC-MS (Methode 3): $R_t$ = 1.88 min; MS (ESIpos): m/z = 559 $[M+H]^+$

**Beispiel 370**

7-[(3*R*,4*R*)-3,4-Dihydroxypyrrolidin-1-yl]-4-oxo-*N*-(4,4,4-trifluor-2-methylbutan-2-yl)-1-(2,4,6-trifluorphenyl)-1,4-dihydro-1,8-naphthyridin-3-carbonsäureamid

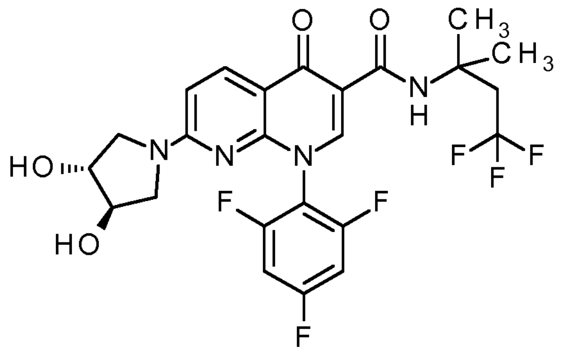

Gemäß AAV1 wurden 1.19 g (2.82 mmol) der Verbindung aus Beispiel 121A mit 601 mg (3.38 mmol) 4,4,4-Trifluor-2-methylbutan-2-amin Hydrochlorid in Gegenwart von 1.29 g (3.38 mmol) HATU und 1.72 ml (9.87 mmol) *N,N*-Diisopropylethylamin in 28 ml Dimethylformamid zur Reaktion gebracht. Der Ansatz wurde mit 40 ml Wasser und 15ml wässriger 1M Salzsäure versetzt und dreimal mit 40 ml Essigsäureethylester extrahiert. Die vereinigten organischen Phasen wurden mit 30ml gesättigter wässriger Natriumchlorid-Lösung gewaschen, getrocknet, eingeengt und der Rückstand mittels Normalphasenchromatographie (Cyclohexan-Essigsäureethylester Gradient) gereinigt. Abschließend wurde das Produkt in 10 ml Acetonitril suspendiert und zusätzlich mit 15 ml *tert.*-Butylmethylether verdünnt. Es wurde für 30 min ausgerührt und anschließend filtriert. Der Niederschlag wurde abgesaugt und im Hocvakuum getrocknet. Es wurden 1.20 g (78% d. Th., 100% Reinheit) der Titelverbindung erhalten

$^1$H-NMR (400 MHz, DMSO-d6) $\delta$ [ppm] = 10.17 (s, 1H), 8.69 (s, 1H), 8.26 (d, 1H), 7.60-7.51 (m, 2H), 6.76 (d, 1H), 5.23 (d, 1H), 5.13 (d, 1H), 4.05 (br. s, 1H), 3.93 (br. s, 1H), 3.61 (dd, 1H), 3.34 (s, 0.5H), 3.25 (dd, 1H), 3.07 (d, 1H), 2.95 (q, 2H), 1.48 (s, 6H).

LC-MS (Methode 3): $R_t$ = 1.71 min; MS (ESIpos): m/z = 545 $[M+H]^+$

**Beispiel 371**

7-[(3*R*,4*R*)-3,4-Dihydroxypyrrolidin-1-yl]-4-oxo-*N*-[3-(trifluormethyl)bicyclo[1.1.1]pent-1-yl]-1-(2,4,6-trifluorphenyl)-1,4-dihydro-1,8-naphthyridin-3-carbonsäureamid

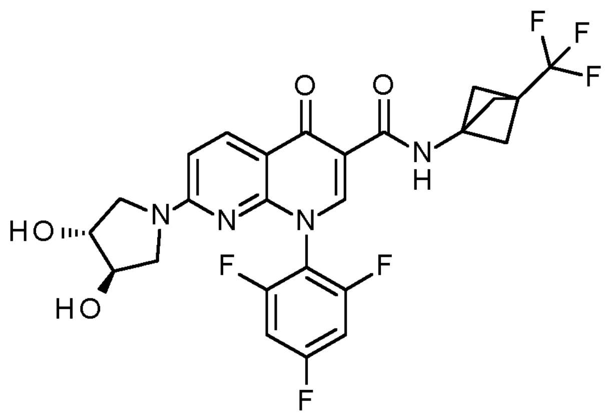

Gemäß AAV1 wurden 30 mg (71 µmol) der Verbindung aus Beispiel 121A mit 17 mg (85 µmol) 3-(Trifluormethyl)bicyclo[1.1.1]pentan-1-amin Hydrochlorid in Gegenwart von 32.5 mg (85.4 µmol) HATU und 43 µl (0.25 mmol) *N,N*-Diisopropylethylamin in 1.0 ml Dimethylformamid zur Reaktion gebracht. Es wurde mit 1 ml wässriger 1N Salzsäure und 2 ml DMSO verdünnt und die Lösung mittels präparativer HPLC (Säule: Chromatorex C18, 10 µm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 27.6 mg (69% d. Th., 99% Reinheit) der Titelverbindung erhalten

$^1$H-NMR (400 MHz, DMSO-d6) $\delta$ [ppm]: -0.008 (1.73), 0.008 (1.57), 2.073 (7.45), 2.356 (16.00), 3.077 (0.74), 3.923 (0.74), 4.043 (0.74), 5.132 (1.08), 5.141 (1.08), 5.222 (1.05), 5.232 (1.05), 6.749 (1.73), 6.771 (1.75), 7.568 (1.12), 8.233 (2.13), 8.256 (2.00), 8.711 (3.43), 10.465 (2.33).

LC-MS (Methode 3): $R_t$ = 1.78 min; MS (ESIpos): m/z = 555 $[M+H]^+$

**Beispiel 372**

7-[(4S)-4-Hydroxy-2-oxopyrrolidin-1-yl]-4-oxo-N-[3-(trifluormethyl)bicyclo[1.1.1]pent-1-yl]-1-(2,4,6-trifluorphenyl)-1,4-dihydro-1,8-naphthyridin-3-carboxamid

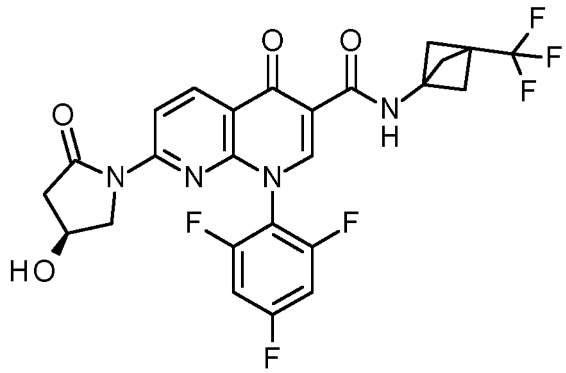

Gemäß AAV1 wurden 30 mg (71 µmol) der Verbindung aus Beispiel 117A mit 17 mg (95% Reinheit, 86 µmol) 3-(Trifluormethyl)bicyclo[1.1.1]pentan-1-amin Hydrochlorid in Gegenwart von 33 mg (86 µmol) HATU und 44 µl (0.25 mmol) *N,N*-Diisopropylethylamin in 1.0 ml Dimethylformamid zur Reaktion gebracht. Es wurde mit 1 ml wässriger 1N Salzsäure und 2 ml DMSO verdünnt und die Lösung mittels präparativer HPLC (Säule: Chromatorex C18, 10 µm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 24.3 mg (61% d. Th., 99% Reinheit) der Titelverbindung erhalten
$^1$H-NMR (400 MHz, DMSO-d6) δ [ppm]: -0.008 (2.13), 0.008 (1.94), 2.349 (0.88), 2.376 (16.00), 2.392 (1.16), 2.911 (0.75), 2.926 (0.78), 2.954 (0.69), 2.969 (0.69), 3.454 (0.78), 3.484 (0.97), 3.666 (0.69), 3.677 (0.85), 3.695 (0.66), 5.324 (1.66), 5.333 (1.66), 7.613 (0.81), 7.622 (0.85), 8.514 (1.85), 8.536 (2.35), 8.671 (2.29), 8.693 (1.78), 8.974 (2.79), 10.159 (2.32).
LC-MS (Methode 3): $R_t$ = 1.95 min; MS (ESIpos): m/z = 553 $[M+H]^+$

**Beispiel 373**

*N*-[(1*S*)-1-Cyclopropyl-2,2,2-trifluorethyl]-7-(1,1-dioxido-1,2,5-thiadiazolidin-2-yl)-4-oxo-1-(2,4,6-trifluorphenyl)-1,4-dihydro-1,8-naphthyridin-3-carbonsäureamid

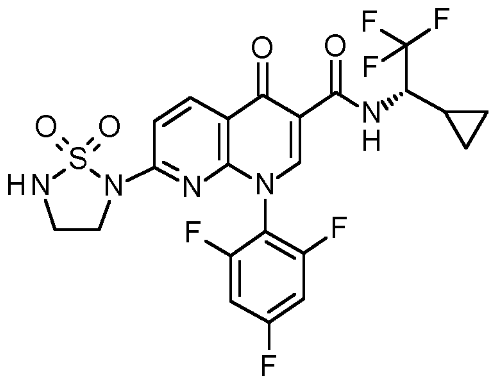

104 mg (159 µmol) der Verbindung aus Beispiel 127A wurden in 10 ml Ethanol gelöst. Es wurden 112 mg (20% Reinheit, 159 µmol) Palladium(II)hydroxid auf Kohle hinzugegeben und über Nacht bei Normaldruck hydriert. Die Reaktionslösung wurde filtriert, das Filtrat wurde eingeengt und mittels präparativer HPLC (Säule: Chromatorex C18,

10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Eine weitere Reingung erfolgte mittels chiraler präparativer HPLC (Säule: Chiralpak IE 5 μm; Fluss: 15 ml/min; Temp.: 35°C; Eluenten: 25% Ethanol, 75% n-Heptan). Es wurden 23.0 mg (25% d. Th., 99% Reinheit) der Titelverbindung erhalten.
$^1$H-NMR (400 MHz, DMSO-d6) δ [ppm]: -0.149 (1.73), -0.008 (16.00), 0.008 (12.76), 0.146 (1.66), 0.341 (2.75), 0.355 (2.60), 0.365 (1.62), 0.377 (1.02), 0.538 (1.62), 0.548 (2.45), 0.562 (3.84), 0.579 (3.61), 0.598 (1.92), 0.609 (1.58), 0.622 (0.98), 0.638 (1.17), 0.647 (1.47), 0.659 (2.56), 0.668 (2.03), 0.680 (1.62), 0.693 (1.05), 1.186 (0.53), 1.198 (1.05), 1.206 (1.43), 1.219 (2.48), 1.239 (2.79), 1.251 (1.51), 1.259 (1.02), 2.327 (1.77), 2.366 (0.83), 2.523 (5.16), 2.665 (1.39), 2.670 (1.88), 2.710 (0.87), 3.457 (3.46), 3.473 (8.24), 3.489 (4.40), 3.752 (4.82), 3.767 (9.04), 3.783 (3.95), 4.368 (1.28), 4.389 (2.22), 4.409 (2.18), 4.429 (1.20), 7.285 (7.98), 7.307 (8.06), 7.513 (4.44), 7.535 (8.43), 7.557 (4.44), 8.037 (4.37), 8.651 (9.56), 8.673 (9.11), 9.002 (13.55), 10.278 (5.12), 10.302 (4.93).
LC-MS (Methode 3): $R_t$ = 1.95 min; MS (ESIpos): m/z = 562 $[M+H]^+$

**Beispiel 374**

*N*-[(1*S*)-1-Cyclopropyl-2,2,2-trifluorethyl]-4-oxo-7-(2-oxo-1,3-oxazolidin-3-yl)-1-(2,4,6-trifluorphenyl)-1,4-dihydro-1,8-naphthyridin-3-carbonsäureamid

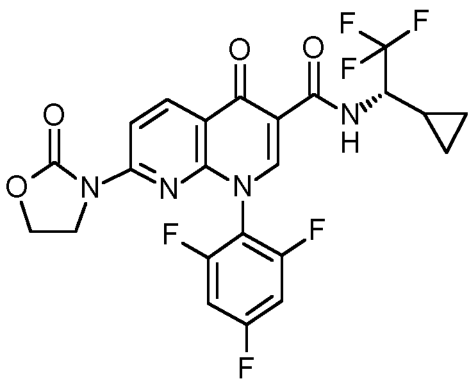

Gemäß AAV2 wurden 50.0 mg (105 μmol) der Verbindung aus Beisiel 126A mit 11.0 mg (126 μmol) 1,3-Oxazolidin-2-on in Gegenwart von 21.8 mg (158 μmol) Kaliumcarbonat, 2.4 mg (11 μmol) Palladium(II)acetat und 12 mg (21 μmol) Xantphos in 0.75 ml 1,4-Dioxan umgesetzt. Anschließend wurde das Volumen der Mischung unter reduziertem Druck eingeengt, der Rückstand mit 1 ml wässriger 1N Salzsäure und 2 ml Acetonitril aufgenommen und mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 33.6 mg (60% d. Th., 99% Reinheit) der Titelverbindung erhalten.
$^1$H-NMR (400 MHz, DMSO-d6) δ [ppm]: -0.008 (1.85), 0.008 (1.34), 0.343 (2.89), 0.357 (2.89), 0.369 (1.83), 0.380 (1.16), 0.541 (1.73), 0.551 (2.65), 0.564 (4.20), 0.582 (4.03), 0.590 (2.61), 0.601 (2.20), 0.611 (1.73), 0.624 (1.06), 0.641 (1.38), 0.650 (1.51), 0.661 (2.74), 0.671 (2.24), 0.682 (1.85), 0.696 (1.19), 1.206 (1.14), 1.215 (1.60), 1.227 (2.57), 1.235 (2.01), 1.248 (2.52), 1.260 (1.47), 1.268 (0.97), 2.073 (1.68), 2.329 (0.69), 2.670 (0.82), 3.745 (4.87), 3.764 (8.60), 3.785 (5.74), 4.372 (7.16), 4.393 (11.23), 4.412 (7.07), 4.437 (1.29), 7.563 (5.18), 7.585 (9.68), 7.608 (5.18), 7.616 (1.64), 8.325 (9.92), 8.347 (10.78), 8.719 (10.89), 8.741 (9.60), 9.063 (16.00), 10.243 (5.50), 10.267 (5.30).
LC-MS (Methode 3): $R_t$ = 2.10 min; MS (ESIpos): m/z = 527 $[M+H]^+$

**Beispiel 375**

7-[3-(2-Hydroxyethyl)-2-oxoimidazolidin-1-yl]-4-oxo-*N*-(4,4,4-trifluor-2-methylbutan-2-yl)-1-(2,4,6-trifluorphenyl)-1,4-dihydro-1,8-naphthyridin-3-carbonsäureamid

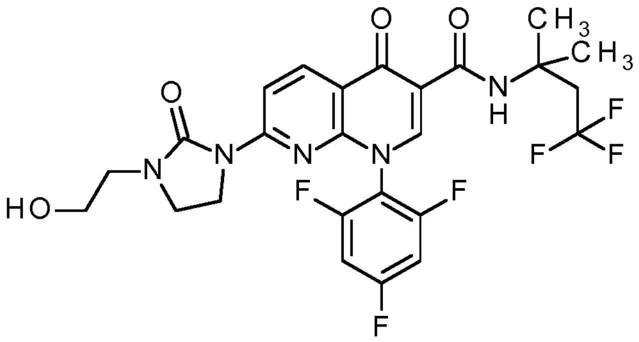

Gemäß AAV1 wurden 30 mg (67 µmol) der Verbindung aus Beispiel 123A mit 14 mg (80 µmol) 4,4,4-Trifluor-2-methylbutan-2-amin Hydrochlorid in Gegenwart von 38.2 mg (100 µmol) HATU und 29.1 µl (170 µmol) *N,N*-Diisopropylethylamin in 2.0 ml Dimethylformamid zur Reaktion gebracht. Es wurde mit 1 ml wässriger 1N Salzsäure, 1 ml Acetonitril und 1 ml DMSO verdünnt und die Lösung mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 29.2 mg (76% d. Th., 100% Reinheit) der Titelverbindung erhalten

$^1$H-NMR (400 MHz, DMSO-d6) δ [ppm]: -0.008 (2.31), 0.008 (1.93), 1.495 (16.00), 2.524 (1.37), 2.670 (0.45), 2.913 (0.72), 2.943 (2.05), 2.973 (1.98), 3.003 (0.60), 3.255 (1.60), 3.270 (3.55), 3.284 (2.02), 3.488 (1.52), 3.501 (3.04), 3.518 (4.32), 3.530 (3.12), 4.739 (1.17), 7.553 (1.55), 7.574 (2.79), 7.596 (1.55), 8.410 (3.01), 8.433 (3.90), 8.542 (4.00), 8.565 (2.88), 8.884 (4.99), 9.980 (3.23).

LC-MS (Methode 3): $R_t$ = 1.89 min; MS (ESIpos): m/z = 572 $[M+H]^+$

## Beispiel 376

4-Oxo-7-(2-oxoimidazolidin-1-yl)-N-(4,4,4-trifluor-2-methylbutan-2-yl)-1-(2,4,6-trifluorphenyl)-1,4-dihydro-1,8-naphthyridin-3-carboxamid

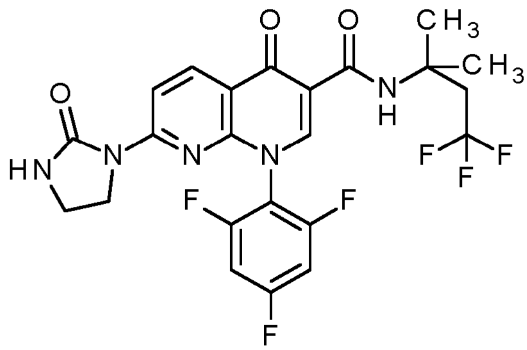

Gemäß AAV1 wurden 50.0 mg (124 µmol) der Verbindung aus Beispiel 113A mit 26.4 mg (148 µmol) 4,4,4-Trifluor-2-methylbutan-2-amin Hydrochlorid in Gegenwart von 56.4 mg (148 µmol) HATU und 53.9µl (309 µmol) *N,N*-Diisopropylethylamin in 1.5 ml Dimethylformamid zur Reaktion gebracht. Es wurde mit 1 ml wässriger 1N Salzsäure und 1 ml DMSO verdünnt und die Lösung mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) und abschließender Normalphasenchromatographie (Cyclohexan-Essigsäureethylester Gradient) gereinigt. Es wurden 35.7 mg (55% d. Th., 100% Reinheit) der Titelverbindung erhalten

$^1$H-NMR (400 MHz, DMSO-d6) δ [ppm]: -0.008 (1.14), 0.008 (1.01), 1.495 (16.00), 2.524 (0.93), 2.913 (0.71), 2.943 (2.10), 2.973 (2.00), 3.003 (0.62), 3.325 (1.57), 3.345 (2.21), 3.365 (1.69), 3.572 (1.77), 3.594 (2.24), 3.612 (1.26), 7.550 (1.45), 7.572 (2.62), 7.594 (1.48), 7.649 (2.43), 8.406 (2.75), 8.429 (3.80), 8.528 (3.71), 8.551 (2.69), 8.884 (4.36), 9.985 (3.18).

LC-MS (Methode 1): $R_t$ = 1.04 min; MS (ESIpos): m/z = 528 $[M+H]^+$

**Beispiel 377**

Methyl- {1-[5-oxo-6-{[(2*S*)-1,1,1-trifluorbutan-2-yl]carbamoyl}-8-(2,4,6-trifluorphenyl)-5,8-dihydro-1,8-naphthyridin-2-yl]imidazolidin-2-yliden}carbamat

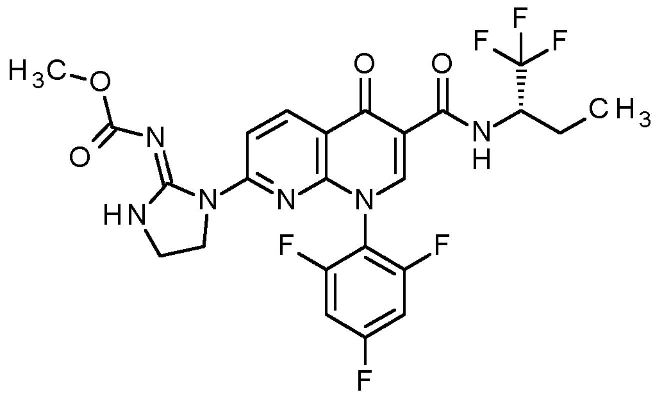

100 mg (205 μmol) der Verbindung aus Beispiel 128A und 57 μl (0.41 mmol) Triethylamin wurden in 2.0 ml Dichlormethan gelöst. Es wurden 32.0 mg (205 μmol) Methyl-(dichlormethylen)carbamat zugegeben und über Nacht bei RT gerührt. Das Reaktionsgemisch wurde mit 20 ml Essigsäureethylester versetzt und dreimal mit gesättigter wässriger Natriumhydrogencarbonat-Lösung gewaschen. Die organische Phase wurde mit gesättigter wässriger Natriumchlorid-Lösung gewaschen, getrocknet und eingeengt. Der Rückstand wurde mittels präparativer HPLC (Säule: Chromatorex C18, 10 μm, 125x30 mm, LM: Acetonitril / 0.1 % Ameisensäure-Gradient; 0 bis 5 min. 10% Acetonitril, während 14 min. nach 90 % Acetonitril und weitere 2 min. 90 % Acetonitril) gereinigt. Es wurden 33.7 mg (29% d. Th., 100% Reinheit) der Titelverbindung erhalten
$^1$H-NMR (400 MHz, DMSO-d6) δ [ppm]: 0.008 (1.62), 0.963 (2.65), 0.981 (5.90), 1.000 (2.87), 1.645 (0.55), 1.662 (0.64), 1.670 (0.54), 1.686 (0.62), 1.705 (0.42), 1.865 (0.50), 1.874 (0.56), 1.883 (0.54), 1.893 (0.64), 1.909 (0.50), 1.928 (0.42), 2.329 (0.42), 2.670 (0.48), 3.520 (0.91), 3.541 (2.07), 3.563 (1.92), 3.611 (16.00), 3.655 (2.02), 3.678 (2.20), 3.697 (0.96), 4.757 (0.55), 7.565 (1.66), 7.587 (3.13), 7.609 (1.67), 8.623 (3.35), 8.646 (3.76), 8.918 (4.03), 8.940 (3.39), 8.964 (1.06), 9.027 (5.59), 10.171 (1.83), 10.194 (1.78).
LC-MS (Methode 1): $R_t$ = 1.07 min; MS (ESIpos): m/z = 571 $[M+H]^+$

**Beispiel 378**

*N*-[(1*S*)-1-Cyclopropyl-2,2,2-trifluorethyl]-7-{[(2*R*)-2-hydroxypropyl]amino}-4-oxo-1-(2,4,6-trifluorphenyl)-1,4-dihydro-1,8-naphthyridin-3-carbonsäureamid

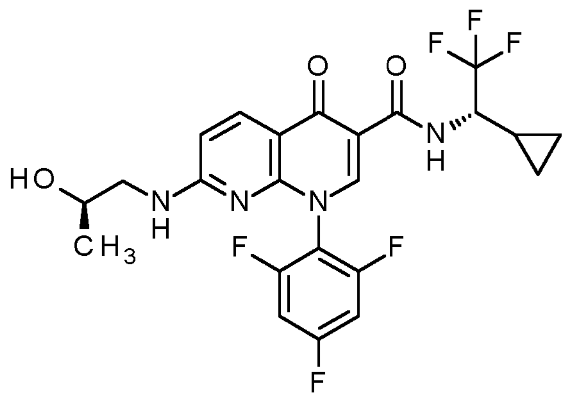

Gemäß AAV3 150 mg (315 μmol) der Verbindung aus Beispiel 126A mit 33.2 mg (441 μmol) (2*R*)-1-Aminop-

ropan-2-ol in Gegenwart von 190 µl (1.10 mmol) *N,N*-Diisopropylethylamin in 3.1 ml Dimethylformamid zur Reaktion gebracht. Die Reaktionsmischung wurde mit 1 ml Acetonitril und 0.5 ml Wasser verdünnt und mittels präparativer HPLC (Säule: Chromatorex C18, 10 µm, 125x30 mm, LM: Acetonitril / 0.1 % Ameisensäure-Gradient; 0 bis 5 min. 10% Acetonitril, während 14 min. nach 90 % Acetonitril und weitere 2 min. 90 % Acetonitril) gereinigt. Es wurden 145 mg (89% d. Th., 99% Reinheit) der Titelverbindung erhalten

$^1$H-NMR (400 MHz, DMSO-d6) δ [ppm]: -0.150 (0.47), -0.008 (4.10), 0.008 (3.55), 0.146 (0.47), 0.307 (1.05), 0.318 (2.37), 0.329 (3.71), 0.341 (3.77), 0.353 (2.84), 0.365 (1.52), 0.490 (0.99), 0.501 (2.60), 0.513 (3.85), 0.525 (3.29), 0.535 (2.80), 0.542 (2.68), 0.549 (2.43), 0.562 (3.79), 0.573 (3.22), 0.583 (2.96), 0.593 (2.41), 0.607 (1.52), 0.622 (1.82), 0.632 (2.17), 0.643 (3.31), 0.653 (3.10), 0.658 (3.00), 0.667 (3.08), 0.675 (1.42), 0.688 (1.03), 0.826 (15.80), 0.842 (16.00), 1.159 (0.79), 1.171 (1.54), 1.179 (2.27), 1.191 (3.91), 1.200 (2.72), 1.212 (3.87), 1.224 (1.99), 1.232 (1.34), 1.244 (0.59), 2.073 (14.17), 2.328 (1.01), 2.333 (0.75), 2.366 (0.79), 2.519 (4.28), 2.524 (3.26), 2.666 (0.79), 2.670 (1.01), 2.710 (0.81), 2.762 (1.12), 2.778 (1.74), 2.795 (2.58), 2.813 (2.31), 2.828 (1.48), 2.984 (1.68), 2.997 (2.51), 3.009 (2.11), 3.029 (1.91), 3.042 (1.40), 3.559 (2.55), 4.330 (0.51), 4.350 (1.93), 4.370 (3.26), 4.392 (3.26), 4.412 (1.74), 4.639 (5.68), 4.650 (5.74), 6.729 (6.67), 6.752 (6.89), 7.535 (6.41), 7.556 (11.17), 7.578 (6.29), 8.137 (6.29), 8.160 (8.19), 8.174 (4.42), 8.188 (2.37), 8.765 (13.93), 10.591 (5.58), 10.615 (5.31).

LC-MS (Methode 1): $R_t$ = 1.00 min; MS (ESIpos): m/z = 515 [M+H]$^+$

**Beispiel 379**

*N*-(2-Cyclopropylpropan-2-yl)-7-[(3*R*,4*R*)-3,4-dihydroxypyrrolidin-1-yl]-4-oxo-1-(2,4,6-trifluorphenyl)-1,4-dihydro-1,8-naphthyridin-3-carbonsäureamid

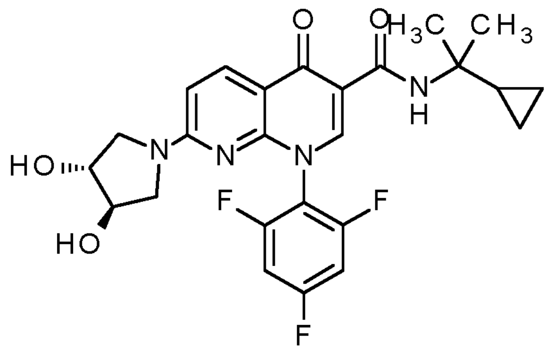

Gemäß AAV1 wurden 30 mg (71 µmol) der Verbindung aus Beispiel 121A mit 8.5 mg (85 µmol) 2-Cyclopropylpropan-2-amin in Gegenwart von 32 mg (85 µmol) HATU und 43 µl (0.25 µmol) *N,N*-Diisopropylethylamin in 1.0 ml Dimethylformamid zur Reaktion gebracht. Es wurde mit 1 ml Wasser und 2 ml DMSO verdünnt und die Lösung mittels präparativer HPLC (Säule: Chromatorex C18, 10 µm, 125x30 mm, LM: Acetonitril / 0.1 % Ameisensäure-Gradient; 0 bis 5 min. 10% Acetonitril, während 14 min. nach 90 % Acetonitril und weitere 2 min. 90 % Acetonitril) gereinigt. Es wurden 21.9 mg (61% d. Th., 100% Reinheit) der Titelverbindung erhalten

$^1$H-NMR (400 MHz, DMSO-d6) δ [ppm]: 0.396 (5.13), 0.414 (4.63), 1.311 (16.00), 2.073 (2.35), 3.049 (0.58), 3.080 (0.80), 3.230 (0.49), 3.253 (0.41), 3.336 (0.74), 3.594 (0.49), 3.921 (0.75), 4.042 (0.75), 5.129 (0.82), 5.221 (0.81), 6.728 (1.58), 6.751 (1.58), 7.536 (0.69), 7.555 (1.16), 7.578 (0.64), 8.256 (1.85), 8.278 (1.73), 8.642 (3.25), 9.938 (2.06).

LC-MS (Methode 1): $R_t$ = 0.89 min; MS (ESIpos): m/z = 503 [M+H]$^+$

**Beispiel 380**

7-(3-Cyan-2-oxotetrahydropyrimidin-1(2H)-yl)-N-[(1S)-1-cyclopropyl-2,2,2-trifluorethyl]-4-oxo-1-(2,4,6-trifluorphenyl)-1,4-dihydro-1,8-naphthyridin-3-carboxamid

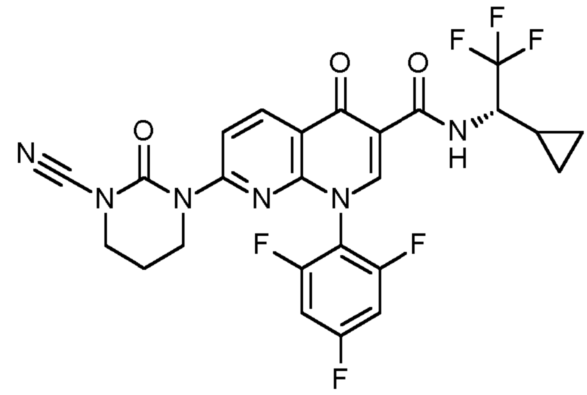

Gemäß AAV2 wurden 100 mg (210 μmol) der Verbindung aus Beispiel 126A mit 28.9 mg (231 μmol) 2-Oxotetrahydropyrimidin-1(2*H*)-carbonitril in Gegenwart von 43.6 mg (315 μmol) Kaliumcarbonat, 4.7 mg (21 μmol) Palladium(II)acetat und 24 mg (42 μmol) Xantphos in 2.1 ml 1,4-Dioxan umgesetzt. Anschließend wurden 2 ml Wasser und 3 ml Acetonitril zugegeben.Es wurde mittels präparativer HPLC (Säule: Chromatorex C18, 10 μm, 125x30 mm, LM: Acetonitril / 0.1 % Ameisensäure-Gradient; 0 bis 5 min. 10% Acetonitril, während 14 min. nach 90 % Acetonitril und weitere 2 min. 90 % Acetonitril) gereinigt. Es wurden 74.0 mg (62% d. Th., 100% Reinheit) der Titelverbindung erhalten.
$^1$H-NMR (400 MHz, DMSO-d6) δ [ppm]: -0.149 (1.15), -0.008 (8.97), 0.008 (8.97), 0.146 (1.12), 0.343 (2.62), 0.356 (2.87), 0.558 (2.55), 0.571 (4.38), 0.584 (3.80), 0.652 (1.63), 0.674 (2.59), 1.232 (2.68), 1.252 (2.40), 2.026 (3.86), 2.040 (5.17), 2.055 (4.09), 2.073 (11.15), 2.328 (1.92), 2.367 (0.93), 2.671 (1.88), 2.710 (0.89), 3.589 (5.05), 3.603 (6.71), 3.617 (5.01), 3.812 (5.43), 3.827 (9.52), 3.841 (5.37), 4.367 (1.31), 4.389 (2.49), 4.409 (2.14), 7.579 (4.89), 7.601 (9.10), 7.624 (5.05), 8.136 (11.08), 8.158 (11.31), 8.695 (11.98), 8.717 (10.57), 9.089 (16.00), 10.195 (5.37), 10.219 (4.89).
LC-MS (Methode 1): $R_t$ = 1.08 min; MS (ESIpos): m/z = 565 $[M+H]^+$

**Beispiel 381**

7-[(4*R*)-4-Methyl-2-oxoimidazolidin-1-yl]-4-oxo-*N*-[(2*S*)-1-(trifluormethoxy)propan-2-yl]-1-(2,4,6-trifluorphenyl)-1,4-dihydro-1,8-naphthyridin-3-carbonsäureamid

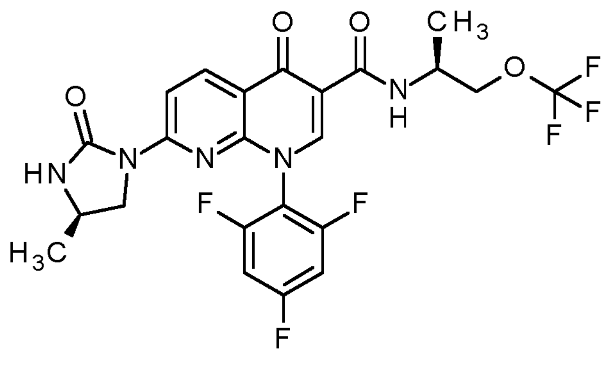

Gemäß AAV1 wurden 100 mg (97 % Reinheit, 232 μmol) der Verbindung aus Beispiel 129D mit 50.0 mg (278 μmol) (2*S*)-1-(Trifluormethoxy)propan-2-amin Hydrochlorid in Gegenwart von 106 mg (278 μmol) HATU und 101 μl (580 μmol) *N,N*-Diisopropylethylamin in 2.3 ml Dimethylformamid zur Reaktion gebracht. Es wurde mit 1 ml 1N wässriger Salzsäure, Wasser und Acetonitril verdünnt und die Lösung mittels präparativer HPLC (Säule: Chromatorex C18, 10 μm, 125x30 mm, LM: Acetonitril / 0.1 % Ameisensäure-Gradient; 0 bis 5 min. 10% Acetonitril, während 14 min. nach 90 % Acetonitril und weitere 2 min. 90 % Acetonitril) gereinigt. Es wurden 69.3 mg (55% d. Th., 100% Reinheit) der Titelverbindung erhalten
$^1$H-NMR (500 MHz, DMSO-d6) δ [ppm]: -0.007 (0.71), 0.007 (0.68), 1.120 (10.98), 1.131 (11.18), 1.233 (0.41), 1.256 (16.00), 1.270 (15.96), 2.516 (2.00), 2.520 (1.59), 2.524 (1.18), 3.084 (1.25), 3.093 (2.58), 3.104 (2.65), 3.112 (1.27), 3.711 (1.38), 3.728 (3.72), 3.745 (3.68), 3.749 (3.29), 3.763 (1.38), 3.780 (0.61), 4.157 (0.78), 4.166 (1.11), 4.177 (4.39), 4.184 (5.76), 4.193 (4.96), 4.203 (0.92), 4.213 (1.01), 4.335 (0.64), 4.345 (1.15), 4.359 (1.55), 4.373 (1.09), 4.383 (0.52),

7.550 (1.03), 7.554 (1.02), 7.569 (4.16), 7.587 (4.18), 7.607 (1.02), 7.808 (5.93), 8.406 (8.66), 8.424 (10.07), 8.538 (10.69), 8.556 (8.06), 8.904 (12.01), 9.902 (3.85), 9.917 (3.70).
LC-MS (Methode 3): $R_t$ = 1.91 min; MS (ESIpos): m/z = 544 $[M+H]^+$

**Beispiel 382**

*N*-(1,1-Difluor-2-methylpropan-2-yl)-7-[3-(2-hydroxyethyl)-2-oxotetrahydropyrimidin-1(2*H*)-yl]-4-oxo-1-(2,4,6-trifluorphenyl)-1,4-dihydro-1,8-naphthyridin-3-carbonsäureamid

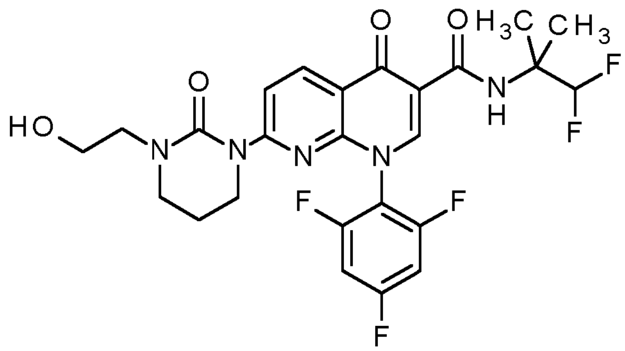

Gemäß AAV1 wurden 25 mg (97% Reinheit, 52 µmol) der Verbindung aus Beispiel 125A mit 9.2 mg (63 µmol) 1,1-Difluor-2-methylpropan-2-amin Hydrochlorid in Gegenwart von 24 mg (63 µmol) HATU und 23 µl (0.13 mmol) *N,N*-Diisopropylethylamin in 1.4 ml Dimethylformamid zur Reaktion gebracht. Es wurde mit 1 ml 1N wässriger Salzsäure, Wasser und Acetonitril verdünnt und die Lösung mittels präparativer HPLC (Säule: Chromatorex C18, 10 µm, 125x30 mm, LM: Acetonitril / 0.1 % Ameisensäure-Gradient; 0 bis 5 min. 10% Acetonitril, während 14 min. nach 90 % Acetonitril und weitere 2 min. 90 % Acetonitril) gereinigt. Es wurden 16.2 mg (56% d. Th., 100% Reinheit) der Titelverbindung erhalten
$^1$H-NMR (500 MHz, DMSO-d6) δ [ppm]: -0.007 (0.86), 0.006 (0.84), 1.451 (16.00), 1.880 (0.39), 1.891 (1.10), 1.904 (1.70), 1.916 (1.18), 1.927 (0.41), 2.515 (2.23), 2.518 (1.70), 2.522 (1.29), 3.368 (1.68), 3.373 (2.29), 3.380 (4.11), 3.385 (3.51), 3.392 (2.47), 3.397 (2.00), 3.494 (1.65), 3.506 (2.23), 3.518 (1.63), 3.528 (1.16), 3.539 (2.88), 3.551 (2.60), 3.563 (0.84), 4.688 (1.27), 4.699 (2.94), 4.710 (1.25), 6.317 (0.84), 6.431 (1.63), 6.544 (0.76), 7.571 (1.39), 7.588 (2.45), 7.606 (1.37), 8.140 (3.64), 8.158 (3.68), 8.484 (3.92), 8.502 (3.43), 8.927 (5.03), 10.099 (3.13).
LC-MS (Methode 3): $R_t$ = 1.80 min; MS (ESIpos): m/z = 554 $[M+H]^+$

**Beispiel 383**

7-[3-(2-Hydroxyethyl)-2-oxotetrahydropyrimidin-1(2*H*)-yl]-4-oxo-*N*-(4,4,4-trifluor-2-methylbutan-2-yl)-1-(2,4,6-trifluorphenyl)-1,4-dihydro-1,8-naphthyridin-3-carbonsäureamid

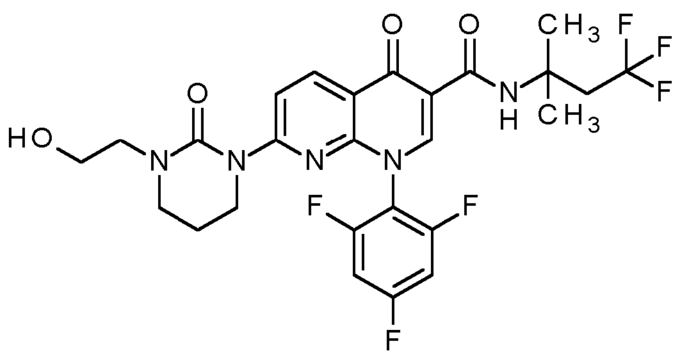

Gemäß AAV1 wurden 19 mg (99% Reinheit, 41 µmol) der Verbindung aus Beispiel 125A mit 8.7 mg (49 µmol)

4,4,4-Trifluor-2-methylbutan-2-amin Hydrochlorid in Gegenwart von 19 mg (49 µmol) HATU und 18 µl (0.10 mmol) *N,N*-Diisopropylethylamin in 1.0 ml Dimethylformamid zur Reaktion gebracht. Es wurde mit 1 ml wässriger 1N Salzsäure, Wasser und Acetonitril verdünnt und die Lösung mittels präparativer HPLC (Säule: Chromatorex C18, 10 µm, 125x30 mm, LM: Acetonitril / 0.1 % Ameisensäure-Gradient; 0 bis 5 min. 10% Acetonitril, während 14 min. nach 90% Acetonitril und weitere 4 min. 90% Acetonitril) gereinigt. Es wurden 9.30 mg (39% d. Th., 100% Reinheit) der Titelverbindung erhalten
$^1$H-NMR (500 MHz, DMSO-d6) δ [ppm]: 1.497 (16.00), 1.880 (0.42), 1.892 (1.17), 1.904 (1.81), 1.916 (1.24), 1.928 (0.45), 2.519 (0.74), 2.523 (0.56), 2.924 (0.69), 2.948 (2.01), 2.972 (1.89), 2.996 (0.57), 3.369 (1.76), 3.374 (2.47), 3.381 (4.30), 3.386 (3.67), 3.392 (2.62), 3.398 (2.12), 3.495 (1.74), 3.507 (2.38), 3.519 (1.71), 3.529 (1.21), 3.541 (2.97), 3.552 (2.69), 3.564 (0.87), 4.689 (1.27), 4.700 (2.92), 4.711 (1.25), 7.569 (1.48), 7.587 (2.61), 7.604 (1.47), 8.136 (3.98), 8.154 (3.98), 8.478 (4.27), 8.496 (3.72), 8.904 (5.42), 9.962 (3.28).
LC-MS (Methode 3): $R_t$ = 1.88 min; MS (ESIpos): m/z = 586 $[M+H]^+$

**Beispiel 384**

*N*-[(1*S*)-1-Cyclopropyl-2,2,2-trifluorethyl]-7-[(2-hydroxyethyl)(methyl)amino]-4-oxo-1-(2,4,6-trifluorphenyl)-1,4-dihydro-1,8-naphthyridin-3-carbonsäureamid

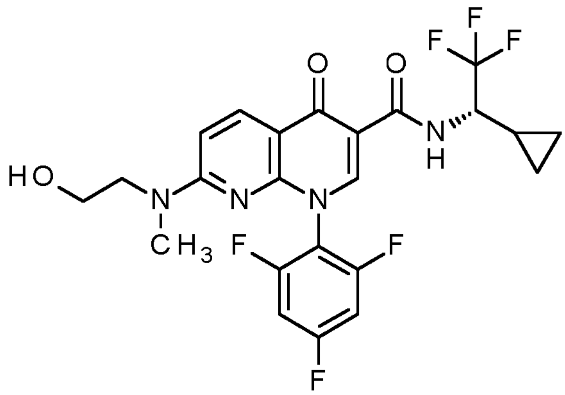

Gemäß AAV3 wurden 50.0 mg (105 µmol) der Verbindung aus Beispiel 126A mit 8.7 mg (116 µmol) 2-(Methylamino)ethanol in Gegenwart von 64 µl (0.37 mmol) *N,N*-Diisopropylethylamin in 0.56 ml Dimethylformamid zur Reaktion gebracht. Die Reaktionsmischung wurde mit 1 ml Acetonitril und 0.2 ml 1N wässriger Salzsäure verdünnt und mittels präparativer HPLC (Säule: Chromatorex C18, 10 µm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 43.6 mg (81% d. Th., 100% Reinheit) der Titelverbindung erhalten
$^1$H-NMR (400 MHz, DMSO-d6) δ [ppm]: 0.313 (1.08), 0.324 (2.62), 0.334 (4.16), 0.346 (4.12), 0.358 (3.20), 0.370 (1.57), 0.498 (1.08), 0.510 (2.93), 0.521 (4.27), 0.534 (3.75), 0.547 (3.80), 0.556 (3.35), 0.567 (4.19), 0.578 (3.62), 0.588 (3.27), 0.599 (2.68), 0.612 (1.63), 0.626 (2.04), 0.636 (2.30), 0.647 (3.74), 0.658 (3.39), 0.663 (3.25), 0.671 (3.20), 0.679 (1.57), 0.692 (1.03), 1.164 (0.78), 1.176 (1.59), 1.184 (2.37), 1.196 (4.08), 1.205 (3.00), 1.217 (3.98), 1.229 (2.16), 1.237 (1.46), 1.249 (0.63), 2.328 (0.79), 2.366 (0.64), 2.670 (0.91), 2.711 (0.79), 2.833 (1.26), 3.123 (2.01), 3.538 (1.66), 4.332 (0.55), 4.352 (2.17), 4.373 (3.82), 4.394 (3.72), 4.414 (1.97), 4.434 (0.48), 4.615 (0.72), 6.961 (1.39), 7.525 (5.57), 7.547 (10.59), 7.569 (5.53), 8.261 (2.60), 8.280 (2.50), 8.805 (16.00), 10.546 (6.57), 10.570 (6.32).
LC-MS (Methode 3): $R_t$ = 1.93 min; MS (ESIpos): m/z = 515 $[M+H]^+$

**Beispiel 385**

*N*-[6-{[(1*S*)-1-Cyclopropyl-2,2,2-trifluorethyl]carbamoyl}-5-oxo-8-(2,4,6-trifluorphenyl)-5,8-dihydro-1,8-naphthyridin-2-yl]-*N*-methylglycin

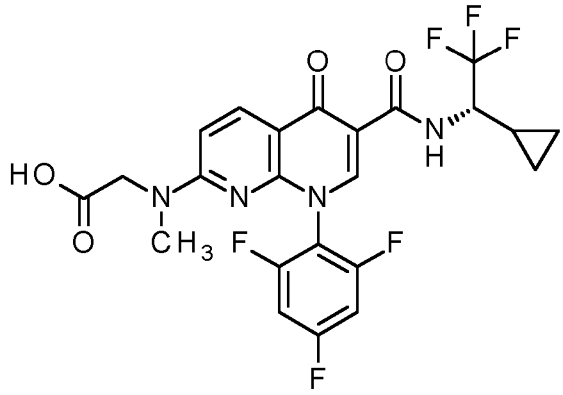

Gemäß AAV3 wurden 50.0 mg (105 μmol) der Verbindung aus Beispiel 126A mit 50.0 mg (325 μmol) Ethyl-N-methylglycinat Hydrochlorid in Gegenwart von 64 μl (0.37 mmol) *N,N*-Diisopropylethylamin in 1.0 ml Dimethylformamid zur Reaktion gebracht. Die Reaktionsmischung wurde mit 1 ml Acetonitril und 0.2 ml wässriger 1N Salzsäure verdünnt und mittels präparativer HPLC (Säule: Chromatorex C18, 10 μm, 125x30 mm, LM: Acetonitril / 0.1 % Ameisensäure-Gradient; 0 bis 5 min. 10% Acetonitril, während 14 min. nach 90% Acetonitril und weitere 4 min. 90% Acetonitril) gereinigt. Es wurden 32.0 mg (56% d. Th., 96% Reinheit) der Titelverbindung erhalten.

$^1$H-NMR (400 MHz, DMSO-d6) δ [ppm] = 12.44 (br. s, 1H), 10.50 (d, 1H), 8.81 (s, 1H), 8.41-8.25 (m, 1H), 7.62-7.35 (m, 2H), 7.08-6.87 (m, 1H), 4.44-4.32 (m, 1H), 3.95 (br. s, 1.4H), 3.15 (br. s, 2.3H), 2.81 (br. s, 0.5H), 1.26-1.16 (m, 1H), 0.70-0.49 (m, 3H), 0.38-0.30 (m, 1H).

LC-MS (Methode 3): $R_t$ = 1.85 min; MS (ESIpos): m/z = 529 $[M+H]^+$

## Beispiel 386

*N*-[(1*R*)-1-Cyclopropyl-2,2,2-trifluorethyl]-7-[(4*S*)-4-hydroxy-2-oxopyrrolidin-1-yl]-4-oxo-1-(2,4,6-trifluorphenyl)-1,4-dihydro-1,8-naphthyridin-3-carbonsäureamid

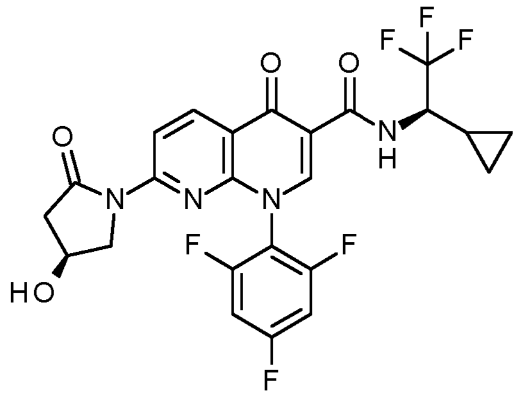

Gemäß AAV1 wurden 11.0 g (26.2 mmol) der Verbindung aus Beispiel 117A mit 5.53 g (31.5mmol) (1*R*)-1-Cyclopropyl-2,2,2-trifluorethanamin Hydrochlorid in Gegenwart von 15.0 g (39.3mmol) HATU und 11.4 ml (65.6 mmol) *N,N*-Diisopropylethylamin in 150 ml Dimethylformamid zur Reaktion gebracht. Es wurde für 1.5h bei Raumtemperatur nachgerührt und anschließend der Ansatz auf Eiswasser mit etwas wässriger Salzsäure ausgerührt. Der Niederschlag wurde abgesaugt und mit Wasser gewaschen. Der Rückstand wurden mittels Normalphasenchromatographie (Petrolether /Essigsäureethylester 1:1 und Dichlormethan/Methanol 9:1) gereinigt. Es wurden 11 g (76% d. Th., 99% Reinheit) der Titelverbindung erhalten.

$^1$H-NMR (400 MHz, DMSO-d6) δ [ppm]: -0.008 (2.46), 0.008 (2.09), 0.332 (1.29), 0.337 (1.43), 0.346 (2.78), 0.358 (2.64), 0.371 (1.67), 0.381 (1.15), 0.541 (1.63), 0.551 (2.52), 0.565 (3.70), 0.582 (3.68), 0.591 (2.39), 0.602 (1.99), 0.612 (1.61), 0.626 (1.01), 0.640 (1.25), 0.649 (1.45), 0.661 (2.70), 0.670 (2.11), 0.685 (1.57), 0.696 (1.07), 1.106 (1.49), 1.175 (1.11), 1.193 (1.07), 1.206 (1.09), 1.214 (1.51), 1.227 (2.50), 1.235 (1.85), 1.247 (2.48), 1.259 (1.37), 1.268 (0.95), 1.989 (2.03), 2.356 (3.72), 2.399 (4.35), 2.731 (0.83), 2.891 (1.09), 2.917 (3.56), 2.931 (3.74), 2.960 (3.30), 2.974 (3.20), 3.463 (3.74),

3.493 (4.55), 3.674 (3.26), 3.685 (4.03), 3.703 (3.10), 3.715 (2.74), 4.290 (2.76), 4.299 (2.78), 4.378 (1.33), 4.398 (2.29), 4.420 (2.21), 4.440 (1.21), 5.330 (9.02), 5.339 (8.94), 7.592 (2.15), 7.600 (2.80), 7.611 (4.09), 7.622 (4.09), 7.635 (2.74), 7.642 (2.07), 8.533 (10.41), 8.556 (12.48), 8.710 (12.66), 8.733 (9.92), 9.064 (16.00), 10.249 (5.43), 10.273 (5.21).
LC-MS (Methode 3): $R_t$ = 1.87 min; MS (ESIpos): m/z = 541 $[M+H]^+$

**Beispiel 387**

*N*-[(1*R*)-1-Cyclopropyl-2,2,2-trifluorethyl]-1-(2,6-dichlor-4-fluorphenyl)-7-[(4*S*)-4-hydroxy-2-oxopyrrolidin-1-yl]-4-oxo-1,4-dihydro-1,8-naphthyridin-3-carbonsäureamid

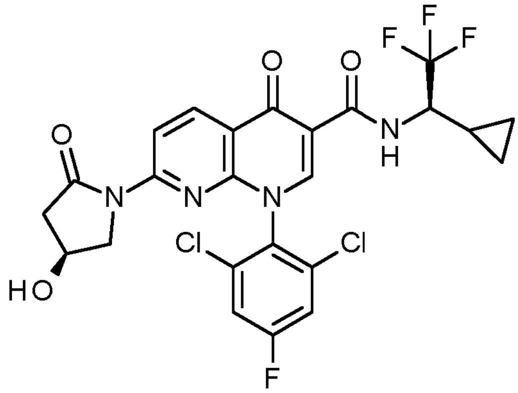

Gemäß AAV2 wurden 150 mg (99 % Reinheit, 292 µmol) der Verbindung aus Beispiel 130C mit 32.5 mg (321 µmol) (4*S*)-4-Hydroxypyrrolidin-2-on in Gegenwart von 60.5 mg (438 µmol) Kaliumcarbonat, 6.6 mg (29 µmol) Palladium(II)acetat und 34 mg (58 µmol) Xantphos in 3.0 ml 1,4-Dioxan umgesetzt. Anschließend wurde mit Acetonitril verdünnt, filtriert und das Lösungsmittel unter reduziertem Druck entfernt. Der Rückstand wurde in 3 ml Acetonitril und 0.5 ml Wasser aufgenommen und mittels präp. HPLC getrennt (Säule: Chromatorex C18, 10 µm, 125x30 mm, LM: Acetonitril / 0.05% Ameisensäure-Gradient; 0 bis 3 min. 10% Acetonitril bis 22 min. 55% Acetonitril, bis 35min 65% ACN, bis 36min und weitere 3 min. 90% Acetonitril) gereinigt und 25 mg (15% d. Th., 97.6% Reinheit) der Titelverbindung erhalten. Anschließend wurden die Mischfraktionen erneut mittels Normalphasenchromatographie (Dichlormethan-Methanol Gradient) gereinigt und weitere 89.2 mg (52% d. Th., 97% Reinheit) der Titelverbindung erhalten.
$^1$H-NMR (400 MHz, DMSO-d6) δ [ppm]: -0.008 (3.21), 0.008 (3.00), 0.343 (0.98), 0.353 (1.81), 0.365 (1.66), 0.554 (1.65), 0.569 (2.02), 0.587 (2.18), 0.597 (1.50), 0.607 (1.29), 0.645 (0.88), 0.655 (0.93), 0.666 (1.86), 0.676 (1.40), 1.214 (0.99), 1.227 (1.61), 1.235 (1.22), 1.248 (1.52), 1.259 (0.91), 2.073 (1.27), 2.341 (2.38), 2.386 (2.72), 2.912 (2.30), 2.927 (2.35), 2.956 (2.17), 2.970 (2.05), 3.287 (2.77), 3.375 (2.46), 3.404 (3.01), 3.597 (2.13), 3.610 (2.53), 3.627 (2.05), 3.640 (1.73), 4.270 (1.84), 4.385 (1.48), 4.406 (1.43), 5.329 (5.75), 5.338 (5.59), 7.906 (1.99), 7.913 (5.03), 7.923 (4.69), 7.929 (3.13), 7.934 (4.95), 7.943 (4.50), 7.950 (2.09), 8.531 (7.38), 8.553 (8.44), 8.723 (8.78), 8.746 (6.86), 9.004 (16.00), 10.285 (3.50), 10.309 (3.47).
LC-MS (Methode 3): $R_t$ = 2.01 min; MS (ESIpos): m/z = 573 $[M+H]^+$

**Beispiel 388**

1-(2,6-Dichlor-4-fluorphenyl)-7-[(4*S*)-4-hydroxy-2-oxopyrrolidin-1-yl]-4-oxo-*N*-[(2*R*)-1,1,1-trifluorbutan-2-yl]-1,4-dihydro-1,8-naphthyridin-3-carbonsäureamid

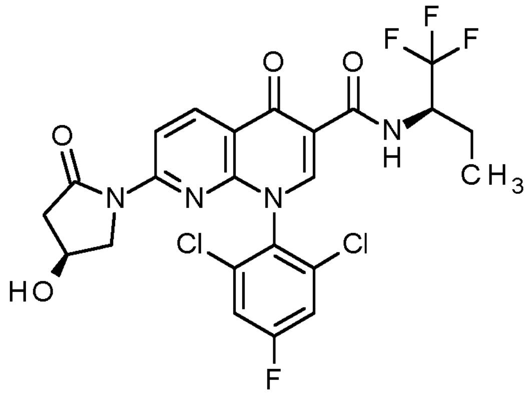

Gemäß AAV2 wurden 150 mg (99% Reinheit, 299 μmol) der Verbindung aus Beispiel 131A mit 33.3 mg (329 μmol) (4*S*)-4-Hydroxypyrrolidin-2-on in Gegenwart von 62.0 mg (448 μmol) Kaliumcarbonat, 6.7 mg (30 μmol) Palladium(II)acetat und 35 mg (60 μmol) Xantphos in 3.3 ml 1,4-Dioxan umgesetzt. Anschließend wurde mit Acetonitril verdünnt, filtriert und das Lösungsmittel unter reduziertem Druck entfernt. Der Rückstand wurde in 3.0 ml Acetonitril und 0.5 ml Wasser aufgenommen und mittels präp. HPLC getrennt (Säule: Chromatorex C18, 10 μm, 125x30 mm, LM: Acetonitril / 0.05% Ameisensäure-Gradient; 0 bis 3 min. 10% Acetonitril bis 22 min. 55% Acetonitril, bis 35 min 65% ACN, bis 36 min und weitere 3 min. 90 % Acetonitril). Die produkthaltigen Fraktionen wurden eingeengt und erneut mittels Normalphasenchromatographie (Dichlormethan-Methanol Gradient) gereinigt. Es wurden 65.2 mg (39% d. Th., 100% Reinheit) der Titelverbindung erhalten.

$^1$H-NMR (500 MHz, DMSO-d6) δ [ppm]: -0.007 (5.14), 0.007 (4.29), 0.971 (5.91), 0.986 (12.43), 1.001 (6.04), 1.648 (0.89), 1.662 (1.16), 1.667 (1.00), 1.676 (1.35), 1.682 (1.22), 1.690 (1.13), 1.696 (1.26), 1.710 (0.96), 1.875 (0.98), 1.883 (1.18), 1.890 (1.18), 1.898 (1.35), 1.903 (1.18), 1.911 (1.05), 1.918 (0.89), 2.347 (2.86), 2.381 (3.23), 2.919 (2.46), 2.931 (2.64), 2.954 (2.35), 2.965 (2.27), 3.286 (5.71), 3.376 (2.94), 3.400 (3.42), 3.603 (2.38), 3.613 (2.88), 3.627 (2.33), 3.637 (2.05), 4.259 (0.98), 4.269 (2.16), 4.278 (2.07), 4.767 (1.09), 4.783 (1.02), 5.328 (6.02), 5.336 (6.02), 5.754 (16.00), 7.912 (2.07), 7.917 (4.66), 7.927 (5.43), 7.934 (5.84), 7.944 (4.19), 7.949 (2.27), 8.531 (7.28), 8.549 (8.15), 8.721 (8.61), 8.739 (6.95), 9.010 (15.72), 10.140 (3.84), 10.159 (3.71).

LC-MS (Methode 3): $R_t$ = 1.97 min; MS (ESIpos): m/z = 561 $[M+H]^+$

## Beispiel 389

1-(2,6-Dichlor-4-fluorphenyl)-7-[(4*S*)-4-hydroxy-2-oxopyrrolidin-1-yl]-4-oxo-*N*-[(2*S*)-1,1,1-trifluorbutan-2-yl]-1,4-dihydro-1,8-naphthyridin-3-carbonsäureamid

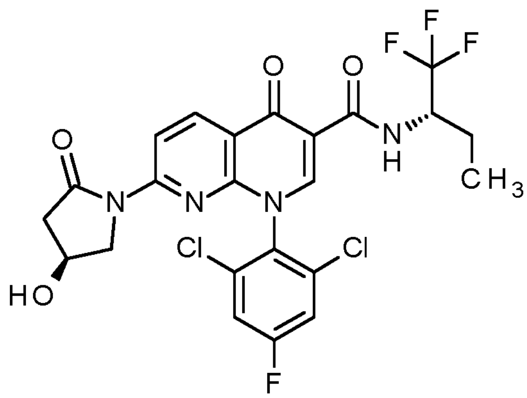

Gemäß AAV2 wurden 150 mg (99 % Reinheit, 299 μmol) der Verbindung aus Beispiel 132A mit 33.3 mg (329 μmol) (4*S*)-4-Hydroxypyrrolidin-2-on in Gegenwart von 62.0 mg (448 μmol) Kaliumcarbonat, 6.7 mg (30 μmol) Palladium(II)acetat und 35 mg (60 μmol) Xantphos in 3.0 ml 1,4-Dioxan umgesetzt. Anschließend wurde mit Acetonitril verdünnt, filtriert und das Lösungsmittel unter reduziertem Druck entfernt. Der Rückstand wurde in 3.0 ml Acetonitril und 0.5 ml Wasser aufgenommen und mittels präp. HPLC getrennt (Säule: Chromatorex C18, 10 μm, 125x30 mm, LM: Acetonitril

/ 0.05% Ameisensäure-Gradient; 0 bis 3 min. 10% Acetonitril bis 22 min. 55% Acetonitril, bis 35 min 65% ACN, bis 36 min und weitere 3 min. 90 % Acetonitril). Die produkthaltigen Fraktionen wurden eingeengt und erneut mittels Normalphasenchromatographie (Dichlormethan-Methanol Gradient) gereinigt. Es wurden 13.0 mg (8% d. Th., 99% Reinheit) der Titelverbindung erhalten.
$^{1}$H-NMR (500 MHz, DMSO-d6) δ [ppm]: -0.120 (1.67), -0.013 (2.76), -0.007 (16.00), 0.007 (13.28), 0.117 (1.63), 0.971 (3.73), 0.986 (7.69), 1.001 (3.73), 1.147 (1.17), 1.236 (1.36), 1.662 (0.74), 1.667 (0.66), 1.675 (0.89), 1.682 (0.82), 1.690 (0.78), 1.695 (0.82), 1.710 (0.58), 1.875 (0.62), 1.882 (0.74), 1.890 (0.78), 1.898 (0.85), 1.911 (0.66), 1.917 (0.58), 2.347 (1.75), 2.358 (0.85), 2.362 (1.05), 2.365 (0.82), 2.381 (1.98), 2.632 (0.74), 2.635 (1.05), 2.639 (0.74), 2.919 (1.55), 2.931 (1.63), 2.953 (1.48), 2.965 (1.40), 3.285 (10.17), 3.376 (1.90), 3.399 (2.10), 3.603 (1.51), 3.613 (1.79), 3.627 (1.44), 3.637 (1.24), 4.277 (1.32), 4.767 (0.70), 5.328 (3.77), 5.335 (3.69), 5.754 (4.97), 7.912 (1.32), 7.917 (2.95), 7.927 (3.42), 7.934 (3.65), 7.944 (2.64), 7.949 (1.44), 8.531 (4.58), 8.548 (5.13), 8.721 (5.40), 8.739 (4.35), 9.010 (10.02), 10.140 (2.37), 10.159 (2.29).
LC-MS (Methode 3): $R_t$ = 1.98 min; MS (ESIpos): m/z = 561 $[M+H]^+$

**Beispiel 390**

1-(2-Chlor-4,6-difluorphenyl)-7-[(2*R*,4*S*)-4-hydroxy-2,4-dimethylpyrrolidin-1-yl]-4-oxo-*N*-[(2*S*)-1,1,1-trifluorpropan-2-yl]-1,4-dihydro-1,8-naphthyridin-3-carbonsäureamid

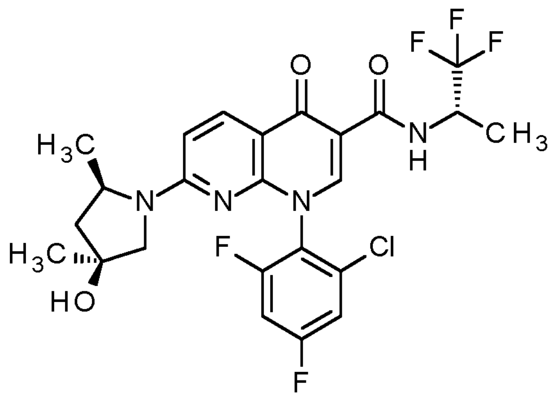

Gemäß AAV3 wurden 18 mg (38 μmol) der Verbindung aus Beispiel 111A mit 10 mg (95 % Reinheit, 41 μmol) der Verbindung aus Beispiel 116A in Gegenwart von 23 μl (0.13 mmol) *N*,*N*-Diisopropylethylamin in 1.5 ml Dimethylformamid zur Reaktion gebracht. Die Reaktionsmischung wurde mit 4 ml Acetonitril und 0.5 ml Wasser verdünnt und mittels präparativer HPLC (Säule: Kromasil C18, 10 μm, 250x20 mm, LM: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 15.9 mg (77% d. Th., 99% Reinheit) der Titelverbindung erhalten.
$^{1}$H-NMR (400 MHz, DMSO-d6) δ [ppm]: -0.149 (0.98), -0.008 (15.59), 0.008 (9.15), 0.146 (0.94), 0.976 (4.35), 1.021 (3.73), 1.267 (12.23), 1.365 (14.65), 1.371 (16.00), 1.382 (14.36), 1.388 (14.61), 1.648 (2.13), 2.001 (1.76), 2.328 (0.90), 2.367 (0.98), 2.711 (1.11), 3.288 (13.09), 3.465 (2.26), 3.752 (1.76), 4.865 (5.87), 4.898 (2.91), 6.702 (2.38), 7.710 (4.76), 7.733 (5.46), 7.749 (3.24), 8.246 (5.91), 8.268 (5.46), 8.761 (5.70), 10.493 (7.30), 10.516 (7.06).
LC-MS (Methode 3): $R_t$ = 2.07 min; MS (ESIpos): m/z = 545 $[M+H]^+$

**Beispiel 391**

1-(2-Chlor-4,6-difluorphenyl)-7-[(3*R*,4*R*)-3,4-dihydroxypyrrolidin-1-yl]-4-oxo-*N*-(4,4,4-trifluor-2-methylbutan-2-yl)-1,4-dihydro-1,8-naphthyridin-3-carbonsäureamid (Atropisomerengemisch)

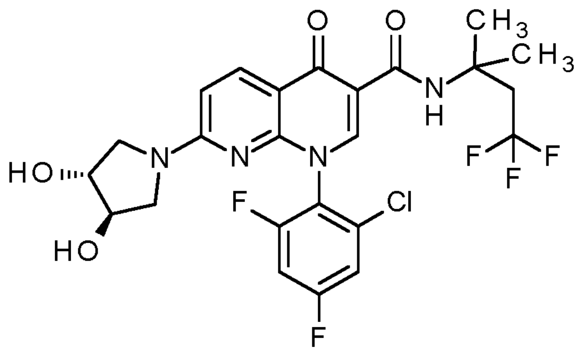

Gemäß AAV3 wurden 100 mg (202 µmol) der Verbindung aus Beispiel 109A mit 33.9 mg (243 µmol) (3*R*,4*R*)-Pyrrolidin-3,4-diol Hydrochlorid in Gegenwart von 123 µl (708 µmol) *N*,*N*-Diisopropylethylamin in 2.0 ml Dimethylformamid zur Reaktion gebracht. Die Reaktionsmischung wurde mit 2.0 ml Acetonitril verdünnt und mit IN wässriger Salzsäure angesäuert. Die Lösung wurde mittels präparativer HPLC (Säule: Chromatorex C18, 10 µm, 125x30 mm, LM: Acetonitril / 0.1 % Ameisensäure-Gradient; 0 bis 5 min. 10% Acetonitril, während 14 min. nach 90% Acetonitril und weitere 4 min. 90% Acetonitril) gereinigt. Es wurden 109 mg (96% d. Th., 100% Reinheit) der Titelverbindung erhalten
LC-MS (Methode 3): $R_t$ = 1.76 min; MS (ESIpos): m/z = 561 $[M+H]^+$
$^1$H-NMR (400 MHz, DMSO-d6) δ [ppm] = 10.19 (s, 1H), 8.63 (s, 1H), 8.26 (d, 1H), 7.76-7.65 (m, 2H), 6.75 (d, 1H), 5.24-5.19 (m, 1H), 5.15-5.10 (m, 1H), 4.04 (br. s, 1H), 3.91 (br. s, 1H), 3.64-3.56 (m, 1H), 3.24-3.15 (m, 1H), 3.06-2.87 (m, 3H), 1.48 (s, 6H).

109 mg der Titelverbindung (Atropisomerengemisch) wurde durch chirale SFC in die Atropisomere getrennt (präparative SFC: Säule Daicel Chiralpak IE 5 µm 250x20 mm; Eluent: 80% iso-Heptan, 20% Ethanol; Temperatur: 35°C; Fluss: 15 ml/min; UV-Detektion: 220 nm).

Man erhielt (in der Reihenfolge der Elution von der Säule) 45.1 mg Atropisomer 1 aus Beispiel 392 (99% de) $R_t$ = 5.56 min und 47.9 mg (99% de) Atropisomer 2 aus Beispiel 393 $R_t$ = 6.17 min.

[Analytische SFC: Säule : Daicel Chiralpak IE 5 µm 250x4.6 mm; Eluent: 75% iso-Hexan, 25% Ethanol; Temperatur: 50°C; Fluss: 1 ml/min; UV-Detektion: 220 nm]

## Beispiel 392

1-(2-Chlor-4,6-difluorphenyl)-7-[(3*R*,4*R*)-3,4-dihydroxypyrrolidin-1-yl]-4-oxo-*N*-(4,4,4-trifluor-2-methylbutan-2-yl)-1,4-dihydro-1,8-naphthyridin-3-carbonsäureamid (Atropisomer 1)

LC-MS (Methode 3): $R_t$ = 1.75 min; MS (ESIpos): m/z = 561 $[M+H]^+$
$^1$H-NMR (400 MHz, DMSO-d6) δ [ppm] = 10.19 (s, 1H), 8.63 (s, 1H), 8.26 (d, 1H), 7.76-7.65 (m, 2H), 6.75 (d, 1H), 5.24-5.19 (m, 1H), 5.15-5.11 (m, 1H), 4.04 (br. s, 1H), 3.91 (br. s, 1H), 3.64-3.56 (m, 1H), 3.23-3.15 (m, 1H), 3.06-2.87 (m, 3H), 1.48 (s, 6H).

## Beispiel 393

1-(2-Chlor-4,6-difluorphenyl)-7-[(3*R*,4*R*)-3,4-dihydroxypyrrolidin-1-yl]-4-oxo-*N*-(4,4,4-trifluor-2-methylbutan-2-yl)-1,4-dihydro-1,8-naphthyridin-3 -carbonsäureamid (Atropisomer 2)

LC-MS (Methode 3): $R_t$ = 1.75 min; MS (ESIpos): m/z = 561 $[M+H]^+$
$^1$H-NMR (400 MHz, DMSO-d6) δ [ppm] = 10.19 (s, 1H), 8.63 (s, 1H), 8.26 (d, 1H), 7.76-7.66 (m, 2H), 6.75 (d, 1H), 5.25-5.19 (m, 1H), 5.15-5.11 (m, 1H), 4.04 (br. s, 1H), 3.91 (br. s, 1H), 3.64-3.56 (m, 1H), 3.25-3.17 (m, 1H), 3.04-2.88 (m, 3H), 1.48 (s, 6H).

## Beispiel 394

1-(2-Chlor-4,6-difluorphenyl)-4-oxo-7-(2-oxoimidazolidin-1-yl)-*N*-(4,4,4-trifluor-2-methylbutan-2-yl)-1,4-dihydro-1,8-naphthyridin-3-carbonsäureamid

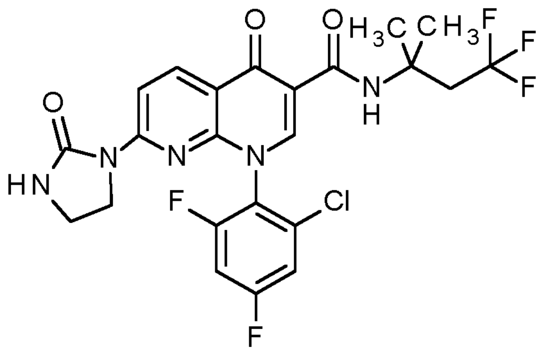

Gemäß AAV2 wurden 30 mg (61 µmol) der Verbindung aus Beispiel 109A mit 54.4 mg (96% Reinheit, 607 µmol) Imidazolidin-2-on in Gegenwart von 13 mg (91 µmol) Kaliumcarbonat, 1.4 mg (6.1 µmol) Palladium(II)acetat und 7.0 mg (12 µmol) Xantphos in 0.6 ml 1,4-Dioxan umgesetzt. Anschließend wurde mit 3.0 ml Acetonitril und 2.0 ml Wasser verdünnt. Es wurde mittels präp. HPLC (Säule: Chromatorex C18, 10 µm, 125x30 mm, LM: Acetonitril / 0.1 % Ameisensäure-Gradient; 0 bis 5 min. 10% Acetonitril, während 14 min. nach 90% Acetonitril und weitere 4 min. 90% Acetonitril) gereinigt. Es wurden 15.6 mg (45% d. Th., 96% Reinheit) der Titelverbindung erhalten.

$^1$H-NMR (400 MHz, DMSO-d6) δ [ppm]: -0.149 (0.53), -0.008 (6.12), 0.008 (4.31), 0.146 (0.52), 1.234 (0.65), 1.259 (0.46), 1.496 (16.00), 2.073 (0.62), 2.323 (0.42), 2.328 (0.55), 2.523 (2.22), 2.665 (0.48), 2.670 (0.59), 2.906 (0.49), 2.918 (0.49), 2.937 (1.31), 2.948 (1.32), 2.966 (1.25), 2.978 (1.28), 2.997 (0.42), 3.008 (0.42), 3.335 (3.40), 3.354 (2.11), 3.516 (1.27), 3.526 (1.37), 3.535 (2.07), 3.546 (1.59), 3.567 (0.89), 7.641 (2.91), 7.685 (0.64), 7.691 (0.86), 7.708 (1.01), 7.715 (1.52), 7.731 (1.58), 7.738 (1.88), 7.753 (1.14), 7.763 (0.78), 8.402 (3.40), 8.424 (4.51), 8.532 (4.47), 8.555 (3.26), 8.836 (6.51), 10.001 (3.82).

LC-MS (Methode 3): $R_t$ = 2.01 min; MS (ESIpos): m/z = 544 $[M+H]^+$

**Beispiel 395**

1-(2-Chlor-4,6-difluorphenyl)-7-[(4*S*)-4-hydroxy-2-oxopyrrolidin-1-yl]-4-oxo-*N*-(4,4,4-trifluor-2-methylbutan-2-yl)-1,4-dihydro-1,8-naphthyridin-3 -carbonsäureamid

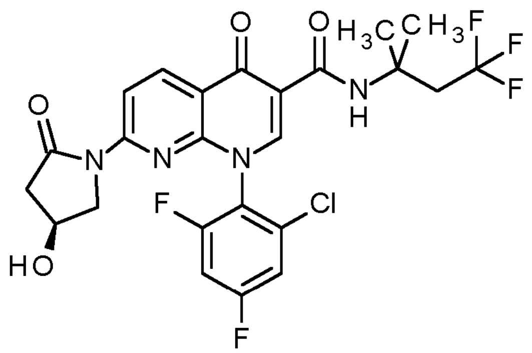

Gemäß AAV2 wurden 30 mg (61 µmol) der Verbindung aus Beispiel 109A mit 6.8 mg (67 µmol) (4*S*)-4-Hydroxypyrrolidin-2-on in Gegenwart von 13 mg (91 µmol) Kaliumcarbonat, 1.4 mg (6.1 µmol) Palladium(II)acetat und 7.0 mg (12 µmol) Xantphos in 0.6 ml 1,4-Dioxan umgesetzt. Anschließend wurde mit 3.0 ml Acetonitril und 2.0 ml Wasser verdünnt. Es wurde mittels präp. HPLC getrennt (Säule: Chromatorex C18, 10 µm, 125x30 mm, LM: Acetonitril / 0.05% Ameisensäure-Gradient; 0 bis 3 min. 15% Acetonitril bis 15 min. 90% Acetonitril und weitere 3 min. 90% Acetonitril) gereinigt. Die Produktfraktionen wurden eingeengt und erneut mittels Normalphasenchromatographie (Essigsäureethylester-Cyclohexan Gradient) gereinigt. Es wurden 14.2 mg (39% d. Th., 93% Reinheit) der Titelverbindung erhalten.

$^1$H-NMR (400 MHz, DMSO-d6) δ [ppm]: -0.149 (0.54), -0.008 (6.50), 0.008 (4.29), 0.146 (0.58), 1.234 (0.80), 1.259 (0.51), 1.501 (16.00), 2.328 (0.70), 2.340 (1.32), 2.383 (1.47), 2.524 (2.20), 2.670 (0.66), 2.905 (1.56), 2.920 (1.76), 2.948 (2.26), 2.963 (1.70), 3.393 (0.71), 3.423 (0.92), 3.443 (0.90), 3.613 (0.70), 3.625 (0.94), 3.632 (0.70), 3.644 (1.25), 3.655 (0.67), 3.662 (0.67), 3.674 (0.50), 4.271 (1.14), 5.318 (3.02), 5.328 (2.85), 7.728 (0.46), 7.743 (0.94), 7.751 (0.83),

7.766 (2.02), 7.777 (1.19), 7.789 (1.98), 7.799 (0.92), 8.507 (3.30), 8.529 (3.96), 8.692 (4.75), 8.714 (3.70), 8.921 (7.11), 9.916 (3.82).
LC-MS (Methode 1): $R_t$ = 1.03 min; MS (ESIpos): m/z = 559 $[M+H]^+$

## Beispiel 396

*N*-(Bicyclo[1.1.1]pent-1-yl)-1-(2-chlor-4,6-difluorphenyl)-7-[(4*S*)-4-hydroxy-2-oxopyrrolidin-1-yl]-4-oxo-1,4-dihydro-1,8-naphthyridin-3-carbonsäureamid

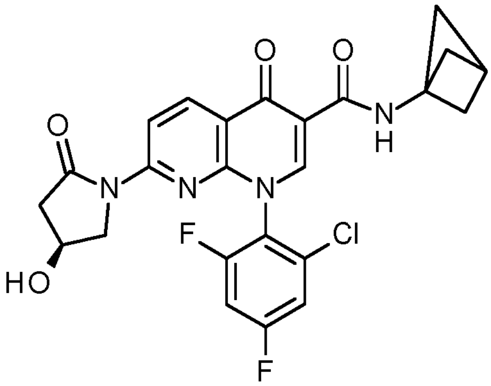

Gemäß AAV1 wurden 40 mg (92 µmol) der Verbindung aus Beispiel 133A mit 13.2 mg (110 µmol) Bicyclo[1.1.1]pentan-1-amin Hydrochlorid in Gegenwart von 41.9 mg (110 µmol) HATU und 56 µl (0.32 mmol) *N*,*N*-Diisopropylethylamin in 0.56 ml Dimethylformamid zur Reaktion gebracht. Es wurde mit 0.1 ml IN wässriger Salzsäure, 1 ml Acetonitril, 0.5 ml DMSO und 0.5 ml Dioxan verdünnt und die Lösung mittels präparativer HPLC (Säule: Chromatorex C18, 10 µm, 125x30 mm, LM: Acetonitril / 0.1 % Ameisensäure-Gradient; 0 bis 5 min. 10% Acetonitril, während 14 min. nach 90% Acetonitril und weitere 4 min. 90% Acetonitril) gereinigt. Es wurden 34.9 mg (75% d. Th., 99% Reinheit) der Titelverbindung erhalten.
$^1$H-NMR (400 MHz, DMSO-d6) δ [ppm]: 2.073 (0.76), 2.114 (16.00), 5.315 (0.76), 5.319 (0.87), 5.324 (0.92), 5.329 (0.77), 7.770 (0.72), 7.793 (0.74), 8.502 (1.41), 8.524 (1.80), 8.671 (1.71), 8.693 (1.35), 8.885 (2.53), 10.018 (1.77).
LC-MS (Methode 1): $R_t$ = 0.98 min; MS (ESIpos): m/z = 501 $[M+H]^+$

## Beispiel 397

1-(2-Chlor-4,6-difluorphenyl)-*N*-(3-fluorbicyclo[1.1.1]pent-1-yl)-7-[(4*S*)-4-hydroxy-2-oxopyrrolidin-1-yl]-4-oxo-1,4-dihydro-1,8-naphthyridin-3 -carbonsäureamid

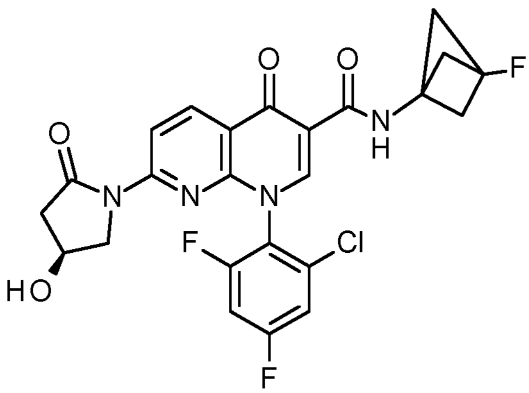

Gemäß AAV1 wurden 40 mg (92 µmol) der Verbindung aus Beispiel 133A mit 15.2 mg (110 µmol) 3-Fluorbicyclo[1.1.1]pentan-1-amin Hydrochlorid in Gegenwart von 41.9 mg (110 μmol) HATU und 56 μl (0.32 mmol) *N,N*-Diisopropylethylamin in 0.56 ml Dimethylformamid zur Reaktion gebracht. Es wurde mit 0.1 ml IN wässriger Salzsäure, 1 ml Acetonitril, 0.5 ml DMSO und 0.5 ml Dioxan verdünnt und die Lösung mittels präparativer HPLC (Säule: Chromatorex C18, 10 μm, 125x30 mm, LM: Acetonitril / 0.1 % Ameisensäure-Gradient; 0 bis 5 min. 10% Acetonitril, während 14 min. nach 90% Acetonitril und weitere 4 min. 90% Acetonitril) gereinigt. Es wurden 28.0 mg (58% d. Th., 99% Reinheit) der Titelverbindung erhalten.
$^1$H-NMR (400 MHz, DMSO-d6) δ [ppm]: -0.008 (2.56), 0.008 (2.56), 2.114 (2.50), 2.339 (3.23), 2.382 (3.75), 2.906 (2.85), 2.920 (2.97), 2.949 (2.58), 2.964 (2.56), 3.389 (2.18), 3.419 (2.79), 3.442 (1.96), 3.611 (1.42), 3.623 (1.89), 3.632 (1.95), 3.643 (2.76), 3.653 (1.35), 3.661 (1.82), 3.674 (1.55), 4.272 (2.70), 4.281 (2.70), 5.317 (5.14), 5.322 (4.81), 5.326 (5.55), 5.331 (4.14), 7.726 (0.85), 7.733 (1.24), 7.741 (0.85), 7.748 (2.16), 7.757 (2.14), 7.763 (1.19), 7.771 (3.91), 7.782 (2.76), 7.793 (4.22), 7.804 (2.14), 7.808 (1.69), 7.815 (1.12), 8.510 (9.03), 8.533 (11.06), 8.678 (10.88), 8.701 (8.50), 8.926 (16.00), 10.135 (10.20).
LC-MS (Methode 1): $R_t$ = 0.96 min; MS (ESIpos): m/z = 519 $[M+H]^+$

**Beispiel 398**

1-(2-Chlor-4,6-difluorphenyl)-7-[(4*S*)-4-hydroxy-2-oxopyrrolidin-1-yl]-4-oxo-*N*-[(2*S*)-1,1,1-trifluorbutan-2-yl]-1,4-dihydro-1,8-naphthyridin-3-carbonsäureamid (Atropisomerengemisch)

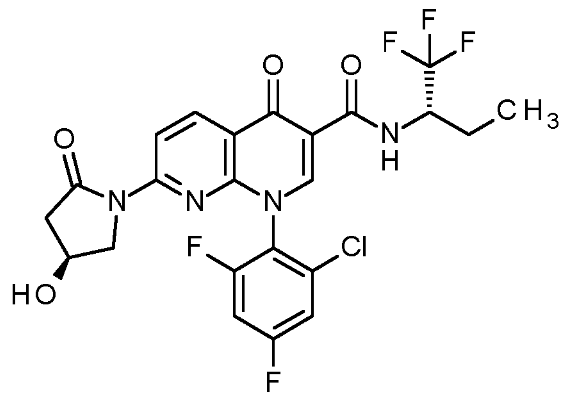

Gemäß AAV1 wurden 2.22 g (5.10 mmol) der Verbindung aus Beispiel 133A mit 1.00 g (6.11 mmol) (2*S*)-1,1,1-Trifluorbutan-2-amin Hydrochlorid in Gegenwart von 2.91 g (7.64 mmol) HATU und 2.22 ml (12.7 mmol) *N,N*-Diisopropylethylamin in 27.8 ml Dimethylformamid zur Reaktion gebracht. Zur Reaktionslösung wurde 200 ml Eiswasser und 20 ml IN wässrige Salzsäure gegeben, der Niederschlag abgesaugt und im Hochvakuum getrocknet. Es wurden 2.66 g (91% d. Th., 95% Reinheit) der Titelverbindung erhalten.
$^1$H-NMR (400 MHz, DMSO-d6) δ [ppm] = 10.13 (d, 1H), 9.04 (s, 1H), 8.72 (d, 1H), 8.54 (d, 1H), 7.82-7.72 (m, 2H), 5.33 (d, 1H), 4.84-4.70 (m, 1H), 4.31-4.25 (m, 1H), 3.69-3.61 (m, 1H), 3.47-3.40 (m, 1H), 2.94 (dd, 1H), 2.37 (d, 1H), 1.95-1.84 (m, 1H), 1.75-1.60 (m, 1H), 1.02-0.93 (m, 3H).
LC-MS (Methode 4): $R_t$ = 3.18/3.20 min; MS (ESIpos): m/z = 545 $[M+H]^+$

2.66 g der Titelverbindung (Atropisomerengemisch) wurde durch chirale SFC in die Atropisomere getrennt (präparative SFC: Säule Daicel Chiralcel OX-H 5 μm 250x30 mm; Eluent: 0.0-5.5 min 74% Kohlendioxid, 26% Acetonitril; 6-12 min 61.1% Kohlendioxid, 38.9% Acetonitril, 12.5-14.0 min 74% Kohlendioxid, 26% Acetonitril; Temperatur: 38°C; Fluss: 180 ml/min; UV-Detektion: 210 nm).

Man erhielt (in der Reihenfolge der Elution von der Säule) 857 mg Atropisomer 1 aus Beispiel 399 (99% de) $R_t$ = 1.00 min und 977 mg (99% de) Atropisomer 2 aus Beispiel 400 $R_t$ = 2.10 min.

[Analytische SFC:Säule : Daicel Chiralpak OX-H 3 μm 100x4.6 mm; Eluent: 80% Kohlendioxid, 20% Acetonitril; Temperatur: 40°C; Fluss: 3 ml/min; UV-Detektion: 210 nm]

Atropisomer 1 wurde abschließend in jeweils 5 ml Essigsäureethylester und Cyclohexan suspendiert, der Niederschlag abfiltriert und 494 mg (17.8% d. Th., 100% Reinheit) der Verbindung aus Beispiel 399 erhalten.

Atropisomer 2 wurde abschließend in jeweils 5 ml Essigsäureethylester und Cyclohexan suspendiert, der Niederschlag abfiltriert und 852 mg (30.7% d. Th., 100% Reinheit) der Verbindung aus Beispiel 400 erhalten. Die Mutterlauge wurde zur Trockene einrotiert und mittels Normalphasenchromatographie (Essigsäureethylester-Cyclohexan Gradient) gereinigt und weiter 30.8 mg (1% d. Th., 100% Reinheit) der Verbindung aus Beispiel 400 erhalten.

**Beispiel 399**

1-(2-Chlor-4,6-difluorphenyl)-7-[(4*S*)-4-hydroxy-2-oxopyrrolidin-1-yl]-4-oxo-*N*-[(2*S*)-1,1,1-trifluorbutan-2-yl]-1,4-dihydro-1,8-naphthyridin-3-carbonsäureamid (Atropisomer 1)

$^1$H-NMR (400 MHz, DMSO-d6) δ [ppm] = 10.13 (d, 1H), 9.04 (s, 1H), 8.72 (d, 1H), 8.54 (d, 1H), 7.83-7.72 (m, 2H), 5.33 (d, 1H), 4.83-4.71 (m, 1H), 4.31-4.25 (m, 1H), 3.66 (dd, 1H), 3.41 (d, 1H), 2.94 (dd, 1H), 2.37 (d, 1H), 1.96-1.83 (m, 1H), 1.74-1.59 (m, 1H), 0.98 (t, 3H).
LC-MS (Methode 1): $R_t$ = 1.00 min; MS (ESIpos): m/z = 545 $[M+H]^+$

**Beispiel 400**

1-(2-Chlor-4,6-difluorphenyl)-7-[(4*S*)-4-hydroxy-2-oxopyrrolidin-1-yl]-4-oxo-*N*-[(2*S*)-1,1,1-trifluorbutan-2-yl]-1,4-dihydro-1,8-naphthyridin-3-carbonsäureamid (Atropisomer 2)

$^1$H-NMR (400 MHz, DMSO-d6) δ [ppm] = 10.13 (d, 1H), 9.04 (s, 1H), 8.73 (d, 1H), 8.54 (d, 1H), 7.81-7.74 (m, 2H), 5.33 (d, 1H), 4.84-4.70 (m, 1H), 4.32-4.24 (m, 1H), 3.64 (dd, 1H), 3.43 (d, 1H), 2.94 (dd, 1H), 2.37 (d, 1H), 1.96-1.83 (m, 1H), 1.75-1.60 (m, 1H), 0.97 (t, 3H).
LC-MS (Methode 1): $R_t$ = 1.01 min; MS (ESIpos): m/z = 545 $[M+H]^+$

**Beispiel 401**

1-(2-Chlor-4,6-difluorphenyl)-7-[(3*R*,4*R*)-3,4-dihydroxypyrrolidin-1-yl]-*N*-(3-fluorbicyclo[1.1.1]pent-1-yl)-4-oxo-1,4-dihydro-1,8-naphthyridin-3-carbonsäureamid

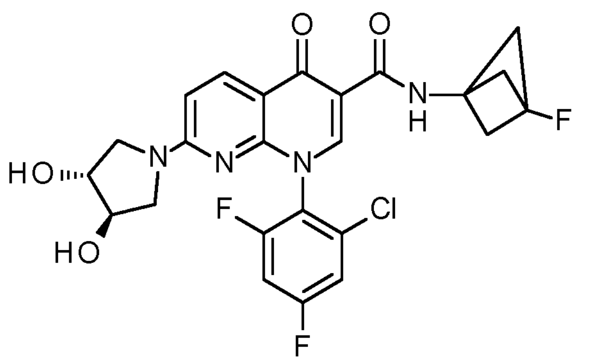

Gemäß AAV1 wurden 50.0 mg (99 % Reinheit, 113 μmol) der Verbindung aus Beispiel 134A mit 18.7mg (136 μmol) 3-Fluorbicyclo[1.1.1]pentan-1-amin Hydrochlorid in Gegenwart von 51.6mg (136 μmol) HATU und 69 μl (0.40 mmol) *N*,*N*-Diisopropylethylamin in 1.2 ml Dimethylformamid zur Reaktion gebracht. Es wurde mit 1 ml IN wässriger Salzsäure, Wasser und 2.0 ml DMSO verdünnt und die Lösung mittels präparativer HPLC (Säule: Chromatorex C18, 10 μm, 125x30 mm, LM: Acetonitril / 0.1 % Ameisensäure-Gradient; 0 bis 5 min. 10% Acetonitril, während 14 min. nach 90% Acetonitril und weitere 4 min. 90% Acetonitril) gereinigt. Es wurden 51.4 mg (86% d. Th., 99% Reinheit) der Titelverbindung erhalten
$^1$H-NMR (400 MHz, DMSO-d6) δ [ppm]: -0.008 (2.40), 0.008 (1.87), 2.073 (14.92), 2.094 (8.32), 2.475 (2.00), 2.988 (1.30), 3.019 (2.12), 3.044 (1.72), 3.178 (1.28), 3.201 (1.88), 3.331 (3.86), 3.587 (2.02), 3.607 (1.63), 3.913 (3.18), 4.039 (3.15), 5.135 (3.79), 5.223 (3.55), 6.731 (0.90), 6.741 (6.39), 6.754 (1.02), 6.763 (6.51), 7.677 (1.08), 7.689 (1.56), 7.700 (2.08), 7.712 (2.36), 7.724 (2.95), 7.753 (2.13), 8.232 (1.47), 8.237 (7.81), 8.259 (7.32), 8.599 (1.82), 8.641 (16.00), 10.305 (0.96), 10.447 (8.86).
LC-MS (Methode 3): $R_t$ = 1.62 min; MS (ESIpos): m/z = 521 $[M+H]^+$

**Beispiel 402**

*N*-(Bicyclo[1.1.1]pent-1-yl)-1-(2-chlor-4,6-difluorphenyl)-7-[(3*R*,4*R*)-3,4-dihydroxypyrrolidin-1-yl]-4-oxo-1,4-dihydro-1,8-naphthyridin-3-carbonsäureamid

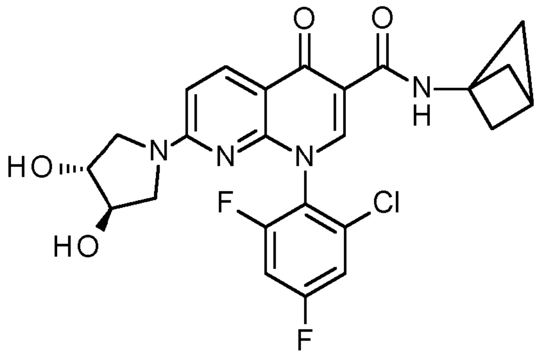

Gemäß AAV1 wurden 50.0 mg (99 % Reinheit, 113 μmol) der Verbindung aus Beispiel 134A mit 16.2 mg (136 μmol) Bicyclo[1.1.1]pentan-1-amin Hydrochlorid in Gegenwart von 51.6 mg (136 μmol) HATU und 69 μl (0.40 mmol) *N*,*N*-Diisopropylethylamin in 1.2 ml Dimethylformamid zur Reaktion gebracht. Es wurde mit 1 ml IN wässriger Salzsäure, Wasser und 2.0 ml DMSO verdünnt und die Lösung mittels präparativer HPLC (Säule: Chromatorex C18, 10 μm, 125x30 mm, LM: Acetonitril / 0.1 % Ameisensäure-Gradient; 0 bis 5 min. 10% Acetonitril, während 14 min. nach 90% Acetonitril und weitere 4 min. 90% Acetonitril) gereinigt. Es wurden 38.4 mg (67% d. Th., 99% Reinheit) der Titelverbindung erhalten
$^1$H-NMR (400 MHz, DMSO-d6) δ [ppm]: 2.073 (2.07), 2.094 (16.00), 2.475 (2.51), 5.132 (0.78), 6.731 (1.37), 6.754 (1.39), 8.232 (1.63), 8.254 (1.55), 8.599 (2.96), 10.305 (1.69).
LC-MS (Methode 3): $R_t$ = 1.64 min; MS (ESIpos): m/z = 503 $[M+H]^+$

**Beispiel 403**

7-[(3*R*,4*R*)-3,4-Dihydroxypyrrolidin-1-yl]-4-oxo-*N*-(1,1,1-trifluor-2-methylpropan-2-yl)-1-(2,4,6-trifluorphenyl)-1,4-dihydro-1,8-naphthyridin-3-carbonsäureamid

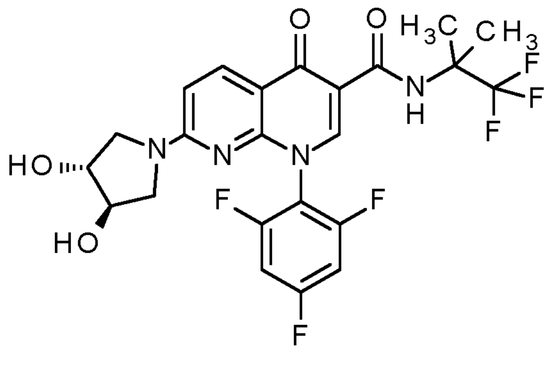

Gemäß AAV1 wurden 100 mg (30% Reinheit, 71.2 μmol) der Verbindung aus Beispiel 121A mit 10.9 mg (85.4 μmol) 1,1,1-Trifluor-2-methylpropan-2-amin in Gegenwart von 32 mg (85 μmol) HATU und 31.0 μl (178 μmol) *N*,*N*-Diisopropylethylamin in 0.72 ml Dimethylformamid zur Reaktion gebracht. Es wurde mit 1 ml IN wässriger Salzsäure, Wasser und 2.0 ml DMSO verdünnt und die Lösung mittels präparativer HPLC (Säule: Chromatorex C18, 10 μm, 125x30 mm, LM: Acetonitril / 0.1 % Ameisensäure-Gradient; 0 bis 5 min. 10% Acetonitril, während 14 min. nach 90% Acetonitril und weitere 4 min. 90% Acetonitril) gereinigt. Es wurden 25.1 mg (66% d. Th., 100% Reinheit) der Titelverbindung erhalten
$^1$H-NMR (400 MHz, DMSO-d6) δ [ppm]: 0.008 (0.97), 1.633 (16.00), 2.073 (1.22), 3.051 (0.77), 3.083 (1.03), 3.225 (0.61), 3.235 (0.68), 3.257 (0.51), 3.266 (0.48), 3.348 (0.96), 3.593 (0.55), 3.602 (0.62), 3.621 (0.51), 3.630 (0.46), 3.925

(0.94), 4.047 (0.94), 5.135 (1.35), 5.143 (1.37), 5.226 (1.35), 5.235 (1.32), 6.759 (1.96), 6.782 (2.01), 7.545 (0.72), 7.565 (1.29), 7.585 (0.73), 8.266 (2.17), 8.289 (2.07), 8.739 (3.38), 10.653 (2.85).
LC-MS (Methode 3): $R_t$ = 1.71 min; MS (ESIpos): m/z = 531 $[M+H]^+$

**Beispiel 404**

*N*-[(1*S*)-1-Cyclopropyl-2,2,2-trifluorethyl]-7-[(4*S*)-4-hydroxy-2-oxopyrrolidin-1-yl]-4-oxo-1-(2,4,6-trifluorphenyl)-1,4-dihydro-1,8-naphthyridin-3-carbonsäureamid

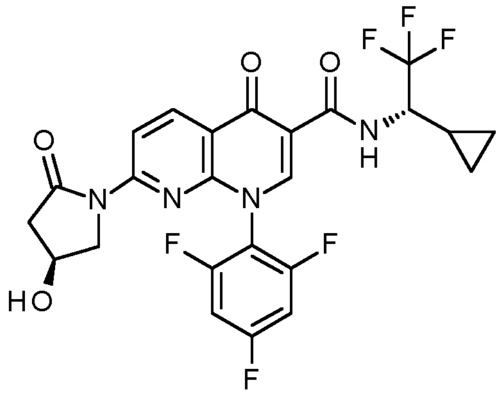

Gemäß AAV2 wurden 100 mg (210 µmol) der Verbindung aus Beispiel 126A mit 23.4 mg (231 µmol) (4*S*)-4-Hydroxypyrrolidin-2-on in Gegenwart von 43.6 mg (315 µmol) Kaliumcarbonat, 2.4 mg (11 µmol) Palladium(II)acetat und 12 mg (21 µmol) Xantphos in 1.9 ml 1,4-Dioxan umgesetzt. Anschließend wurden 100 mg *N*-Acetylcystein zugegeben und 0.5 h bei RT gerührt. Es wurden 20 ml Essigsäureethylester zugesetzt, die organische Phase gegen gesättigte wässrige Natiumhydrogencarbonat-Lösung extrahiert, getrocknet und eingeengt. Der Rückstand wurde mittels präp. HPLC (Säule: Chromatorex C18, 10 µm, 125x30 mm, LM: Acetonitril / 0.05% Ameisensäure-Gradient; 0 bis 3 min. 10% Acetonitril bis 22 min. 55% Acetonitril , bis 35 min 65% Acetonitril, bis 36 min und weitere 3 min. 90 % Acetonitril) gereinigt. Es wurden 60.9 mg (53% d. Th., 99% Reinheit) der Titelverbindung erhalten.
$^1$H-NMR (400 MHz, DMSO-d6) δ [ppm]: -0.149 (1.32), -0.008 (11.84), 0.008 (8.55), 0.146 (1.10), 0.342 (1.75), 0.561 (2.41), 0.578 (2.19), 0.659 (1.53), 1.147 (2.63), 1.226 (1.75), 2.327 (1.75), 2.355 (2.41), 2.366 (5.26), 2.399 (2.63), 2.670 (2.19), 2.710 (5.26), 2.916 (1.97), 2.930 (2.19), 2.959 (1.97), 2.974 (1.75), 3.289 (16.00), 3.461 (2.63), 3.490 (3.07), 3.672 (1.97), 3.684 (2.63), 3.701 (2.19), 3.714 (1.75), 4.286 (1.75), 4.416 (1.53), 5.328 (5.26), 5.338 (5.26), 7.611 (2.63), 8.531 (6.14), 8.554 (7.67), 8.709 (7.67), 8.731 (6.14), 9.062 (8.99), 10.247 (3.29), 10.271 (3.07).
LC-MS (Methode 1): $R_t$ = 1.04 min; MS (ESIpos): m/z = 541 $[M+H]^+$

**Beispiel 405**

*N*-[(1*R*)-1-Cyclopropyl-2,2,2-trifluorethyl]-7-[1-hydroxy-3-azabicyclo[3.1.0]hex-3-yl]-4-oxo-1-(2,4,6-trifluorphenyl)-1,4-dihydro-1,8-naphthyridin-3-carbonsäureamid (Diastereomerengemisch)

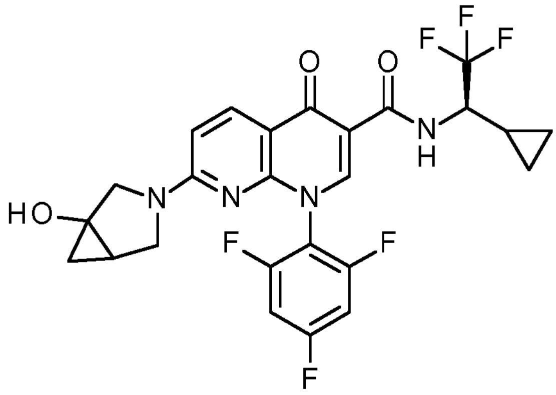

Gemäß AAV3 wurden 150 mg (315 μmol) der Verbindung aus Beispiel 135A mit 51.7 mg (347 μmol, 91% Reinheit) 3-Azabicyclo[3.1.0]hexan-1-ol Hydrochlorid und 192 μl (1.10 mmol) *N*,*N*-Diisopropylethylamin in 1.4 ml Dimethylformamid zur Reaktion gebracht. Die Reaktionslösung wurde mit 4 ml Acetonitril und 0.5 ml IN wässriger Salzsäure verdünnt und mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 40 min. 90% Acetonitril und weitere 3 min. 90% Acetonitril) gereinigt und 118.6 mg (69% d. Th., 99% Reinheit) der Titelverbindung erhalten.
$^1$H NMR (400 MHz, DMSO-$d_6$): δ [ppm] = 10.53 (d, 1H), 8.81 (s, 1H), 8.28 (d, 1H), 7.64-7.50 (m, 2H), 6.84-6.69 (m, 1H), 6.08 (s, 0.5H), 5.96 (s, 0.5H), 4.44-4.32 (m, 1H), 3.93-3.83 (m, 0.5H), 3.70-3.39 (m, 2H), 3.21-3.08 (m, 1H), 1.70-1.51 (m, 1H), 1.26-1.14 (m, 1H), 1.07-0.98 (m, 1H), 0.70-0.29 (m, 5H).
LC-MS (Methode 3): $R_t$ = 2.05 min; MS (ESIpos) m/z 539 $[M+H]^+$.

## Beispiel 406

1-(2-Chlor-4,6-difluorphenyl)-7-[1-hydroxy-3-azabicyclo[3.1.0]hex-3-yl]-4-oxo-*N*-[(2*R*)-1,1,1-trifluorbutan-2-yl]-1,4-dihydro-1,8-naphthyridin-3-carbonsäureamid (Diastereomerengemisch)

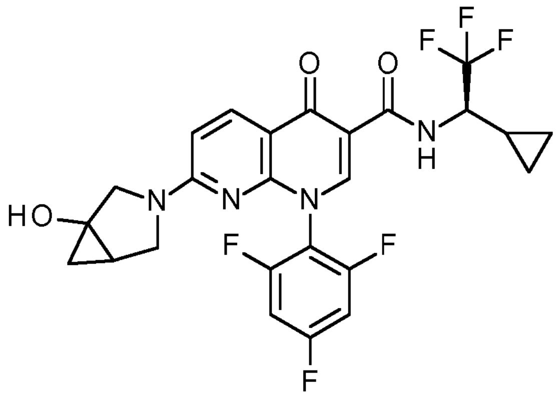

Gemäß AAV3 wurden 150 mg (312 μmol) der Verbindung aus Beispiel 136A mit 51.2 mg (344 μmol, 91% Reinheit) 3-Azabicyclo[3.1.0]hexan-1-ol Hydrochlorid und 190 μl (1.09 mmol) *N*,*N*-Diisopropylethylamin in 3 ml Dimethylformamid zur Reaktion gebracht. Die Reaktionslösung wurde mit 4 ml Acetonitril und 1 ml Wasser verdünnt und mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 40 min. 90% Acetonitril und weitere 3 min. 90% Acetonitril) gereinigt und 104.8 mg (61% d. Th., 99% Reinheit) der Titelverbindung erhalten.
$^1$H NMR (400 MHz, DMSO-$d_6$): δ [ppm] = 10.41 (d, 1H), 8.77 (s, 1H), 8.28 (d, 1H), 7.81-7.64 (m, 2H), 6.83-6.67 (m, 1H), 6.07 (s, 0.5H), 5.95 (s, 0.5H), 4.80-4.67 (m, 1H), 3.93-3.82 (m, 0.5H), 3.71-3.39 (m, 2H), 3.26-3.03 (m, 1.5H), 1.95-1.81 (m, 1H), 1.72-1.50 (m, 2H), 1.07-0.91 (m, 4H), 0.49-0.37 (m, 1H).
LC-MS (Methode 3): $R_t$ = 2.07 min; MS (ESIpos) m/z 543 $[M+H]^+$.

**Beispiel 407**

1-(2-Chlor-4,6-difluorphenyl)-7-[(4*S*)-4-hydroxy-2-oxopyrrolidin-1-yl]-4-oxo-*N*-[(2*R*)-1,1,1-trifluorbutan-2-yl]-1,4-dihydro-1,8-naphthyridin-3-carbonsäureamid

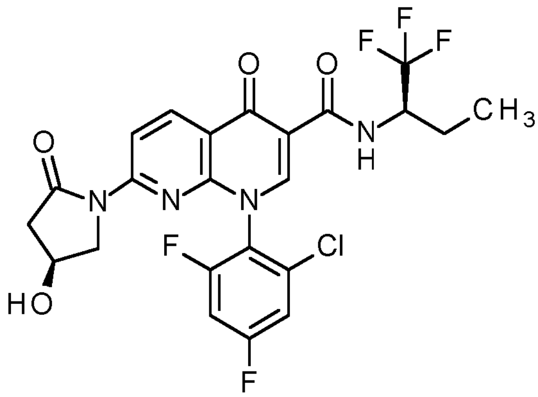

Gemäß AAV2 wurden 150 mg (312 μmol) der Verbindung aus Beispiel 136A mit 34.7 mg (344 μmol) (4*S*)-4-Hydroxypyrrolidin-2-on in Gegenwart von 64.8 mg (469 μmol) Kaliumcarbonat, 7.0 mg (31 μmol) Palladium(II)acetat und 36 mg (62 μmol) Xantphos in 3.1 ml 1,4-Dioxan umgesetzt. Anschließend wurde mit Acetonitril verdünnt, filtriert und eingeengt. Der Rückstand wurde in 3 ml Acetonitril und 0.5 ml Wasser gelöst und zweimal mittels präp. HPLC (Säule: Chromatorex C18, 10 μm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril und Säule: Chromatorex C18, 10 μm, 125x30 mm, LM: Acetonitril / 0.05% Ameisensäure-Gradient; 0 bis 3 min. 10% Acetonitril bis 24min. 60% Acetonitril; bis 35Min 65% Acetonitril, bis 44 min 90% Acetonitril und weitere 12 min 90% Acetonitril) gereinigt. Es wurden 91.2 mg (53% d. Th., 99% Reinheit) der Titelverbindung erhalten.

$^1$H-NMR (400 MHz, DMSO-d6) δ [ppm] = 10.13 (d, 1H), 9.04 (s, 1H), 8.73 (d, 1H), 8.54 (d, 1H), 7.83-7.72 (m, 2H), 5.33 (d, 1H), 4.83-4.71 (m, 1H), 4.31-4.24 (m, 1H), 3.69-3.60 (m, 1H), 3.47-3.38 (m, 1H), 2.94 (dd, 1H), 2.37 (d, 1H), 1.96-1.83 (m, 1H), 1.74-1.61 (m, 1H), 1.03-0.94 (m, 3H).

LC-MS (Methode 1): $R_t$ = 1.07 min; MS (ESIpos): m/z = 545 $[M+H]^+$

**Beispiel 408**

1-(2-Chlor-4,6-difluorphenyl)-7-[1-hydroxy-3-azabicyclo[3.1.0]hex-3-yl]-4-oxo-*N*-[(2*S*)-1,1,1-trifluorpropan-2-yl]-1,4-dihydro-1,8-naphthyridin-3-carbonsäureamid (Diastereomerengemisch)

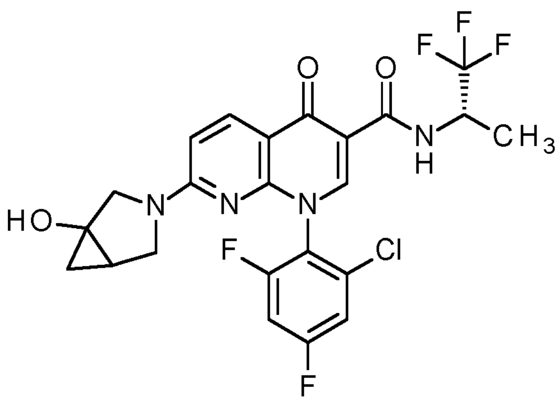

Gemäß AAV3 wurden 150 mg (322 μmol) der Verbindung aus Beispiel 111A mit 52.7 mg (354 μmol, 91% Reinheit) 3-Azabicyclo[3.1.0]hexan-1-ol Hydrochlorid und 196 μl (1.13 mmol) *N*,*N*-Diisopropylethylamin in 3.2 ml Dimethylformamid zur Reaktion gebracht. Die Reaktionslösung wurde mit 4 ml Acetonitril und 0.5 ml Wasser verdünnt und mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 111.8 mg (65% d. Th., 99% Reinheit) der Titelverbindung erhalten.
$^1$H NMR (400 MHz, DMSO-$d_6$): δ [ppm] = 10.46 (d, 1H), 8.76 (s, 1H), 8.27 (d, 1H), 7.80-7.63 (m, 2H), 6.82-6.68 (m, 1H), 6.07 (s, 0.5 H), 5.93 (s, 0.5H), 4.94-4.82 (m, 1H), 3.92-3.81 (m, 0.5H), 3.70-3.39 (m, 2H), 3.26-3.02 (m, 1.5H), 1.70-1.50 (m, 1H), 1.40-1.35 (m, 3H), 1.06-0.99 (m, 1H), 0.47-0.38 (m, 1H).
LC-MS (Methode 3): $R_t$ = 1.98 min; MS (ESIpos) m/z 529 [M+H]$^+$.

## Beispiel 409

1-(2-Chlor-4,6-difluorphenyl)-7-[(4*S*)-4-hydroxy-2-oxopyrrolidin-1-yl]-4-oxo-*N*-[(2*S*)-1,1,1-trifluorpropan-2-yl]-1,4-dihydro-1,8-naphthyridin-3-carbonsäureamid

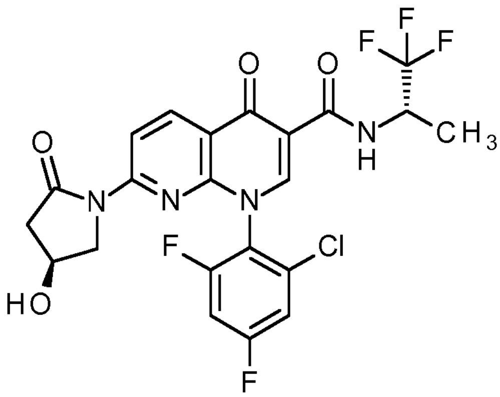

Gemäß AAV2 wurden 150 mg (322 µmol) der Verbindung aus Beispiel 111A mit 35.7 mg (354 µmol) (4*S*)-4-Hydroxypyrrolidin-2-on in Gegenwart von 66.7 mg (483 µmol) Kaliumcarbonat, 7.2 mg (32 µmol) Palladium(II)acetat und 37 mg (64 µmol) Xantphos in 3.2 ml 1,4-Dioxan umgesetzt. Anschließend wurde mit Acetonitril verdünnt, filtriert und eingeengt. Der Rückstand wurde in 3 ml Acetonitril und 0.5 ml Wasser gelöst zweimal mittels präp. HPLC (Säule: Chromatorex C18, 10 µm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril und Säule: Chromatorex C18, 10 µm, 125x30 mm, LM: Acetonitril / 0.05% Ameisensäure-Gradient; 0 bis 3 min. 10% Acetonitril bis 24min. 60% Acetonitril; bis 35Min 65% Acetonitril, bis 44 min 90% Acetonitril und weitere 12 min 90% Acetonitril) gereinigt. Es wurden 55.7 mg (32% d. Th., 98% Reinheit) der Titelverbindung erhalten.
$^1$H-NMR (400 MHz, DMSO-d6) δ [ppm] = 10.18 (d, 1H), 9.04 (d, 1H), 8.72 (d, 1H), 8.54 (d, 1H), 7.82-7.72 (m, 2H), 5.33 (d, 1H), 4.98-4.86 (m, 1H), 4.31-4.24 (m, 1H), 3.68-3.60 (m, 1H), 3.46-3.38 (m, 1H), 2.94 (dd, 1H), 2.37 (d, 1H), 1.42-1.37 (m, 3H).
LC-MS (Methode 1): $R_t$ = 1.01 min; MS (ESIpos): m/z = 531 [M+H]$^+$

## Beispiel 410

7-[(4*S*)-4-Hydroxy-2-oxopyrrolidin-1-yl]-4-oxo-*N*-[1-(trifluormethoxy)butan-2-yl]-1-(2,4,6-trifluorphenyl)-1,4-dihydro-1,8-naphthyridin-3-carbonsäureamid (Diastereomerengemisch)

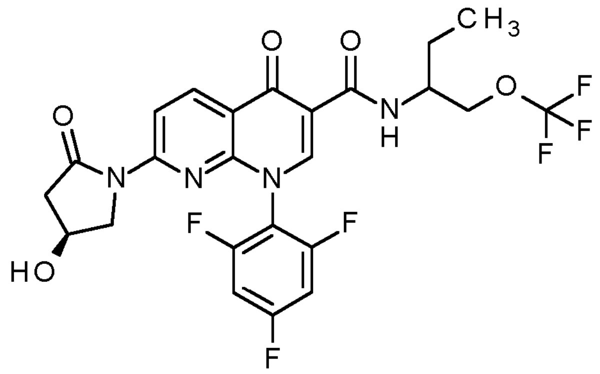

Gemäß AAV2 wurden 188 mg (381 µmol) der Verbindung aus Beispiel 137A mit 38.5 mg (381 µmol) (4*S*)-4-Hydroxypyrrolidin-2-on in Gegenwart von 79.0 mg (572 µmol) Kaliumcarbonat, 15 mg (69 µmol) Palladium(II)acetat und 79.4 mg (137 µmol) Xantphos in 3.8 ml 1,4-Dioxan umgesetzt. Anschließend wurde mit Acetonitril verdünnt, filtriert und eingeengt. Der Rückstand wurde in 3 ml Acetonitril und 0.5 ml Wasser gelöst und mittels präp. HPLC (Säule: Chromatorex C18, 10 µm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 60.2 mg (28% d. Th., 100% Reinheit) der Titelverbindung erhalten.

$^1$H-NMR (400 MHz, DMSO-d6) δ [ppm] = 9.81 (d, 1H), 8.99 (s, 1H), 8.71 (d, 1H), 8.53 (d, 1H), 7.66-7.57 (m, 2H), 5.33 (d, 1H), 4.32-4.15 (m, 4H), 3.69 (dd, 1H), 3.48 (d, 1H), 2.94 (dd, 1H), 2.37 (d, 1H), 1.75-1.54 (m, 2H), 0.95 (t, 3H).

LC-MS (Methode 3): $R_t$ = 1.92 min; MS (ESIpos): m/z = 559 $[M+H]^+$ 60 mg der Titelverbindung (Diastereomerengemisch) wurde durch chirale HPLC in die Diastereomere getrennt (präparative HPLC: Säule Daicel Chiralcel OZ-H 5 µm 250x20 mm; Eluent: 20% Ethanol, 80% Iso-Hexan; Temperatur: 25°C; Fluss: 20 ml/min; UV-Detektion: 220 nm.

Man erhielt (in der Reihenfolge der Elution von der Säule) 10.1 mg Diastereomer 1 (99% de) $R_t$ = 1.77 min und 21 mg (98%de) Diastereomer 2 $R_t$ = 2.56 min.

[Analytische HPLC:Säule Daicel Chiralpak OZ-3 3 µm 50x4.6 mm; Eluent: 20% Ethanol, 80% Iso-Hexan; Fluss: 1 ml/min; UV-Detektion: 220 nm.]

Diastereomer 1 wurde zusätzlich mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 13.6 mg (6.3% d. Th., 99% Reinheit) der Titelverbindung aus Beispiel 411 erhalten.

Diastereomer 2 wurde zusätzlich mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 19.6 mg (9.1% d. Th., 99% Reinheit) der Titelverbindung aus Beispiel 412 erhalten.

## Beispiel 411

7-[(4*S*)-4-Hydroxy-2-oxopyrrolidin-1-yl]-4-oxo-*N*-[1-(trifluormethoxy)butan-2-yl]-1-(2,4,6-trifluorphenyl)-1,4-dihydro-1,8-naphthyridin-3-carbonsäureamid (Diastereomer 1)

$^1$H-NMR (400 MHz, DMSO-d6) δ [ppm] = 9.81 (d, 1H), 8.99 (s, 1H), 8.71 (d, 1H), 8.53 (d, 1H), 7.66-7.57 (m, 2H), 5.33 (d, 1H), 4.32-4.15 (m, 4H), 3.69 (dd, 1H), 3.48 (d, 1H), 2.94 (dd, 1H), 2.37 (d, 1H), 1.76-1.55 (m, 2H), 0.95 (t, 3H).
LC-MS (Methode 3): $R_t$ = 1.90 min; MS (ESIpos): m/z = 559 $[M+H]^+$

## Beispiel 412

7-[(4*S*)-4-Hydroxy-2-oxopyrrolidin-1-yl]-4-oxo-*N*-[1-(trifluormethoxy)butan-2-yl]-1-(2,4,6-trifluorphenyl)-1,4-dihydro-1,8-naphthyridin-3-carbonsäureamid (Diastereomer 2)

$^1$H-NMR (400 MHz, DMSO-d6) δ [ppm] = 9.81 (d, 1H), 8.99 (s, 1H), 8.71 (d, 1H), 8.53 (d, 1H), 7.66-7.57 (m, 2H), 5.33 (d, 1H), 4.32-4.14 (m, 4H), 3.69 (dd, 1H), 3.48 (d, 1H), 2.94 (dd, 1H), 2.37 (d, 1H), 1.75-1.54 (m, 2H), 0.95 (t, 3H).
LC-MS (Methode 3): $R_t$ = 1.89 min; MS (ESIpos): m/z = 559 $[M+H]^+$

**Beispiel 413**

1-(2-Chlor-4,6-difluorphenyl)-7-[1-hydroxy-3-azabicyclo[3.1.0]hex-3-yl]-4-oxo-*N*-[(2*S*)-1,1,1-trifluorbutan-2-yl]-1,4-dihydro-1,8-naphthyridin-3-carbonsäureamid (Diastereomerengemisch)

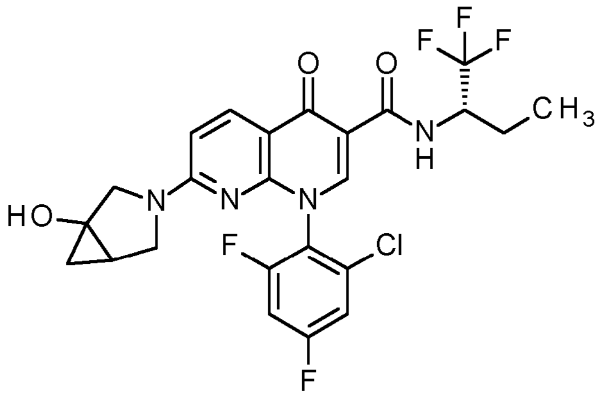

Gemäß AAV3 wurden 150 mg (312 μmol) der Verbindung aus Beispiel 108C mit 51.2 mg (344 μmol, 91% Reinheit) 3-Azabicyclo[3.1.0]hexan-1-ol Hydrochlorid (Racemat) und 190 μl (1.09 mmol) *N*,*N*-Diisopropylethylamin in 3.1 ml Dimethylformamid zur Reaktion gebracht. Die Reaktionslösung wurde mit 1 ml Acetonitril und 0.5 ml Wasser verdünnt und mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 100.5 mg (59% d. Th., 99% Reinheit) der Titelverbindung erhalten.
$^1$H NMR (400 MHz, DMSO-$d_6$): δ [ppm] = 10.42 (d, 1H), 8.76 (s, 1H), 8.28 (d, 1H), 7.80-7.64 (m, 2H), 6.84-6.67 (m, 1H), 6.07 (s, 0.5H), 5.95 (s, 0.5H), 4.80-4.66 (m, 1H), 3.93-3.82 (m, 0.5H), 3.71-3.38 (m, 2H), 3.26-3.02 (m, 1.5H), 1.94-1.82 (m, 1H), 1.72-1.48 (m, 2H), 1.07-0.92 (m, 4H), 0.48-0.37 (m, 1H).
LC-MS (Methode 3): $R_t$ = 2.05 min; MS (ESIpos) m/z 543 $[M+H]^+$.

**Beispiel 414**

1-(2-Chlor-4,6-difluorphenyl)-*N*-[(1*R*)-1-cyclopropyl-2,2,2-trifluorethyl]-7-[(4*S*)-4-hydroxy-2-oxopyrrolidin-1-yl]-4-oxo-1,4-dihydro-1,8-naphthyridin-3-carbonsäureamid

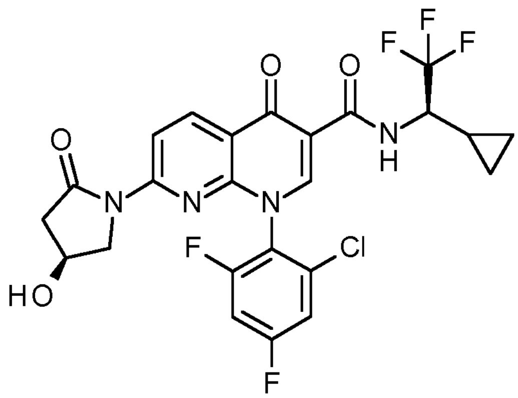

Gemäß AAV2 wurden 150 mg (305 μmol) der Verbindung aus Beispiel 138A mit 30.8 mg (305 μmol) (4*S*)-4-Hydroxypyrrolidin-2-on in Gegenwart von 63.2 mg (457 μmol) Kaliumcarbonat, 12 mg (55 μmol) Palladium(II)acetat und 63.5 mg (110 μmol) Xantphos in 2.8 ml 1,4-Dioxan umgesetzt. Anschließend wurde mit Acetonitril verdünnt, filtriert und eingeengt. Der Rückstand wurde in 3 ml Acetonitril und 0.5 ml Wasser gelöst und zweimal mittels präp. HPLC (Säule: Chromatorex C18, 10 μm, 125x30 mm, LM: Acetonitril / 0.05% Ameisensäure-Gradient; 0 bis 3 min. 10% Acetonitril bis

35 min. 90% Acetonitril und weitere 3 min. 90% Acetonitril und Säule: Chromatorex C18, 10 µm, 125x30 mm, LM: Acetonitril / 0.05% Ameisensäure-Gradient; 0 bis 3 min. 10% Acetonitril bis 22 min. 55% Acetonitril , bis 35 min 65% Acetonitril, weitere 3 min. 90% Acetonitril) gereinigt. Produktfraktionen aus beiden Läufen wurden vereinigt, eingeengt und nochmals mittels Normalphasenchromatographie (Dichlormethan-Methanol Gradient) gereinigt. Es wurden 58 mg (34% d. Th., 100% Reinheit) der Titelverbindung erhalten.
$^1$H-NMR (400 MHz, DMSO-d6) δ [ppm] = 10.27 (d, 1H), 9.03 (s, 1H), 8.73 (d, 1H), 8.54 (d, 1H), 7.82-7.72 (m, 2H), 5.33 (d, 1H), 4.66-4.35 (m, 1H), 4.31-4.25 (m, 1H), 3.69-3.61 (m, 1H), 3.46-3.39 (m, 1H), 2.94 (dd, 1H), 2.37 (d, 1H), 1.28-1.19 (m, 1H), 0.71-0.51 (m, 3H), 0.39-0.31 (m, 1H).
LC-MS (Methode 1): $R_t$ = 1.08 min; MS (ESIpos): m/z = 557 $[M+H]^+$

**Beispiel 415**

1-(2-Chlor-4,6-difluorphenyl)-*N*-[(1*R*)-1-cyclopropyl-2,2,2-trifluorethyl]-7-[1-hydroxy-3-azabicyclo[3.1.0]hex-3-yl]-4-oxo-1,4-dihydro-1, 8-naphthyridin-3 -carbonsäureamid (Diastereomerengemisch)

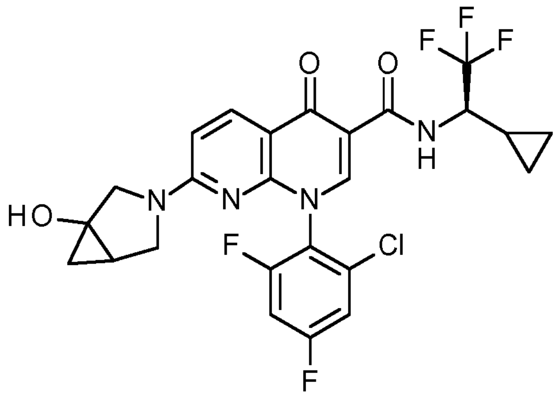

Gemäß AAV3 wurden 150 mg (305 µmol) der Verbindung aus Beispiel 138A mit 49.9 mg (335 µmol, 91% Reinheit) 3-Azabicyclo[3.1.0]hexan-1-ol Hydrochlorid (Racemat) und 186 µl (1.07 mmol) *N*,*N*-Diisopropylethylamin in 1.4 ml Dimethylformamid zur Reaktion gebracht. Die Reaktionslösung wurde mit 4 ml Acetonitril verdünnt, filtriert und mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 112 mg (66% d. Th., 100% Reinheit) der Titelverbindung erhalten.
$^1$H NMR (400 MHz, DMSO-$d_6$): δ [ppm] = 10.55 (d, 1H), 8.76 (s, 1H), 8.29 (d, 1H), 7.80-7.64 (m, 2H), 6.83-6.67 (m, 1H), 6.07 (s, 0.5H), 5.95 (s, 0.5H), 4.45-4.29 (m, 1H), 3.93-3.82 (m, 0.5H), 3.71-3.38 (m, 2H), 3.27-3.01 (m, 1.5H), 1.70-1.49 (m, 1H), 1.26-1.13 (m, 1H), 1.07-0.98 (m, 1H), 0.71-0.29 (m, 5H).
LC-MS (Methode 3): $R_t$ = 2.07 min; MS (ESIpos) m/z 555 $[M+H]^+$.

**Beispiel 416**

7-[1-Hydroxy-3-azabicyclo[3.1.0]hex-3-yl]-4-oxo-*N*-[(2*S*)-1,1,1-trifluorbutan-2-yl]-1-(2,4,6-trifluorphenyl)-1,4-dihydro-1,8-naphthyridin-3-carbonsäureamid (Diastereomerengemisch)

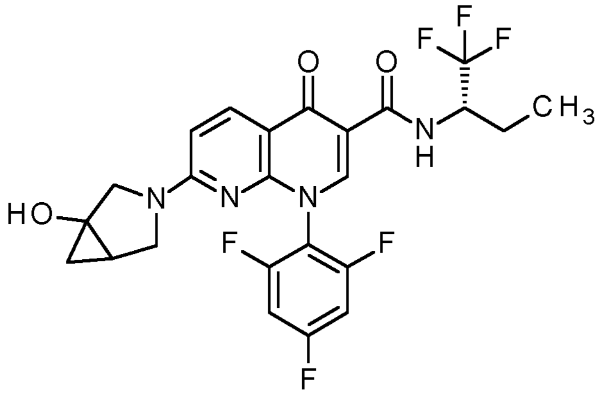

Gemäß AAV3 wurden 150 mg (323 µmol) der Verbindung aus Beispiel 115A mit 53.0 mg (356 µmol, 91% Reinheit) 3-Azabicyclo[3.1.0]hexan-1-ol Hydrochlorid (Racemat) und 197 µl (1.13 mmol) *N*,*N*-Diisopropylethylamin in 1.5 ml Dimethylformamid zur Reaktion gebracht. Die Reaktionslösung wurde mit 0.5 ml Acetonitril verdünnt und mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 116 mg (67% d. Th., 99% Reinheit) der Titelverbindung erhalten.

$^1$H NMR (400 MHz, DMSO-$d_6$): δ [ppm] = 10.40 (d, 1H), 8.82 (s, 1H), 8.27 (d, 1H), 7.65-7.49 (m, 2H), 6.83-6.69 (m, 1H), 6.08 (s, 0.5H), 5.96 (s, 0.5H), 4.80-4.67 (m, 1H), 3.93-3.83 (m, 0.5H), 3.71-3.40 (m, 2H), 3.20-3.07 (m, 1H), 1.94-1.81 (m, 1H), 1.71-1.49 (m, 2H), 1.07-0.91 (m, 4H), 0.49-0.41 (m, 1H).

LC-MS (Methode 3): $R_t$ = 2.03 min; MS (ESIpos) m/z 527 [M+H]$^+$.

## Beispiel 417

1-(2,6-Difluorphenyl)-7-[1-hydroxy-3-azabicyclo[3.1.0]hex-3-yl]-4-oxo-*N*-[(2*S*)-1,1,1-trifluorbutan-2-yl]-1,4-dihydro-1,8-naphthyridin-3-carbonsäureamid (Diastereomerengemisch)

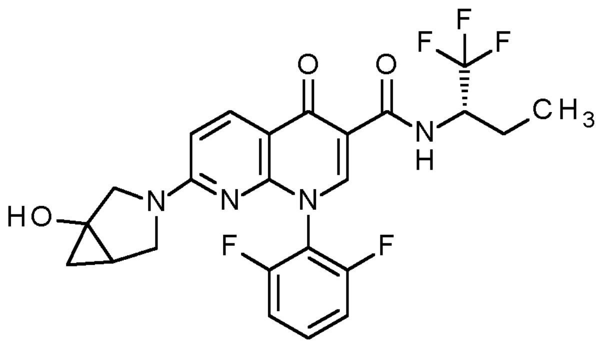

Gemäß AAV3 wurden 100 mg (224 µmol) der Verbindung aus Beispiel 114A mit 50.1 mg (336 µmol, 91% Reinheit) 3-Azabicyclo[3.1.0]hexan-1-ol Hydrochlorid (Racemat) und 137 µl (785 µmol) *N*,*N*-Diisopropylethylamin in 2.2 ml Dimethylformamid zur Reaktion gebracht. Die Reaktionslösung wurde mit 0.5 ml Acetonitril verdünnt und mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 86.4 mg (75% d. Th., 99% Reinheit) der Titelverbindung erhalten.

$^1$H NMR (400 MHz, DMSO-$d_6$): δ [ppm] = 10.41 (d, 1H), 8.76 (s, 1H), 8.28 (d, 1H), 7.77-7.65 (m, 1H), 7.47-7.36 (m, 2H), 6.83-6.68 (m, 1H), 6.07 (s, 0.5H), 5.93 (s, 0.5H), 4.81-4.67 (m, 1H), 3.93-3.81 (m, 0.5H), 3.71-3.39 (m, 2H), 3.26-3.03 (m, 1.5H), 1.93-1.82 (m, 1H), 1.70-1.47 (m, 2H), 1.06-0.92 (m, 4H), 0.48-0.37 (m, 1H).

LC-MS (Methode 3): $R_t$ = 1.99 min; MS (ESIpos) m/z 509 [M+H]$^+$.

**Beispiel 418**

1-(2-Chlor-4,6-difluorphenyl)-7-[3-methyl-2-oxopyrrolidin-1-yl]-4-oxo-*N*-[(2*S*)-1,1,1-trifluorpropan-2-yl]-1,4-dihydro-1,8-naphthyridin-3-carbonsäureamid (Diastereomerengemisch)

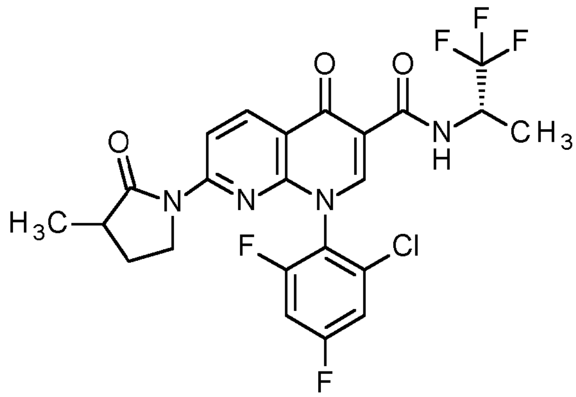

Gemäß AAV2 wurden 100 mg (215 μmol) der Verbindung aus Beispiel 111A mit 23.4 mg (236 μmol) Methylpyrrolidinon (Racemat) in Gegenwart von 44.5 mg (322 μmol) Kaliumcarbonat, 8.7 mg (39 μmol) Palladium(II)acetat und 45 mg (77 μmol) Xantphos in 2 ml 1,4-Dioxan umgesetzt. Anschließend wurde mit 0.5 ml IN wässriger Salzsäure angesäuert und der Ansatz eingeengt. Der Rückstand wurde in 8 ml Dichlormethan gelöst und mittels Normalphasenchromatographie (Essigsäureethylester-Cyclohexan Gradient) gereinigt. Es wurden 78.9 mg (69% d. Th., 99% Reinheit) der Titelverbindung erhalten.

$^{1}$H-NMR (400 MHz, DMSO-d6) δ [ppm] = 10.18 (d, 1H), 9.04-9.02 (m, 1H), 8.71 (d, 1H), 8.55 (d, 1H), 7.80-7.70 (m, 2H), 4.97-4.86 (m, 1H), 3.59-3.49 (m, 1H), 3.44-3.35 (m, 1H), 2.81-2.70 (m, 1H), 2.24-2.15 (m, 1H), 1.67-1.55 (m, 1H), 1.43-1.36 (m, 3H), 1.17-1.11 (m, 3H).

LC-MS (Methode 3): $R_t$ = 2.31 min; MS (ESIpos): m/z = 529 $[M+H]^+$

**Beispiel 419**

1-(2-Chlor-4,6-difluorphenyl)-4-oxo-7-(2-oxoimidazoldin-1-yl)-*N*-[(2*S*)-1,1,1-trifluorpropan-2-yl]-1,4-dihydro-1, 8-naphthyridin-3 -carbonsäureamid

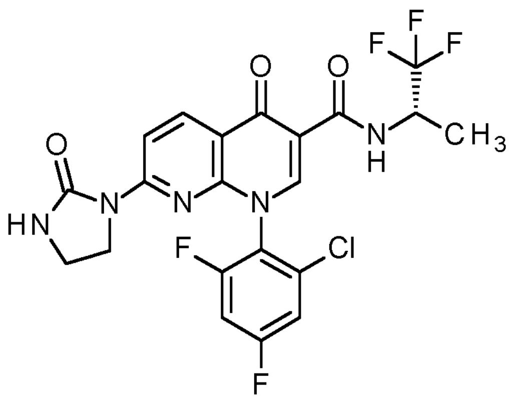

Gemäß AAV2 wurden 100 mg (215 μmol) der Verbindung aus Beispiel 111A mit 92.3 mg (1.07 mmol) Imidazolidin-2-on in Gegenwart von 44.5 mg (322 μmol) Kaliumcarbonat, 2.4 mg (11 μmol) Palladium(II)acetat und 12 mg (21 μmol) Xantphos in 6 ml 1,4-Dioxan umgesetzt. Anschließend wurde mit IN wässriger Salzsäure angesäuert und der Ansatz eingeengt. Der Rückstand wurde in 8 ml Dichlormethan gelöst und mittels Normalphasenchromatographie gereinigt. Es wurden 68.2 mg (59% d. Th., 95% Reinheit) der Titelverbindung erhalten.

$^{1}$H-NMR (400 MHz, DMSO-d6) δ [ppm] = 10.28 (d, 1H), 8.96-8.94 (m, 1H), 8.56 (d, 1H), 8.43 (d, 1H), 7.78-7.64 (m, 3H), 4.97-4.85 (m, 1H), 3.61-3.48 (m, 2H), 1.42-1.35 (m, 3H).
LC-MS (Methode 1): $R_t$ = 1.00 min; MS (ESIpos): m/z = 516 $[M+H]^+$

## Beispiel 420

*N*-[(1*R*)-1-cyclopropyl-2,2,2-trifluorethyl]-7-[4-methyl-2-oxopyrrolidin-1-yl]-4-oxo-1-(2,4,6-trifluorphenyl)-1,4-dihydro-1,8-naphthyridin-3-carbonsäureamid (Diastereomerengemisch)

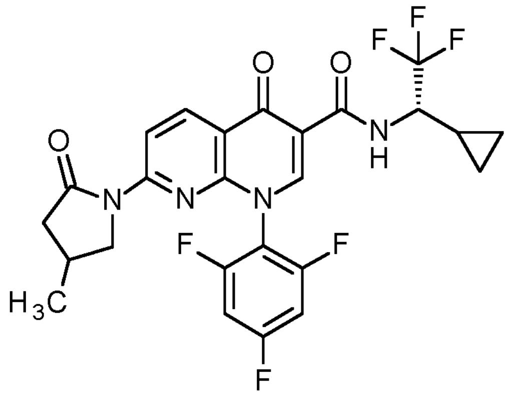

Gemäß AAV2 wurden 200 mg (420 µmol) der Verbindung aus Beispiel 126A mit 45.8 mg (462 µmol) 4-Methyl-2-Pyrrolidinon (Racemat) in Gegenwart von 87.1 mg (631 µmol) Kaliumcarbonat, 4.7 mg (21 µmol) Palladium(II)acetat und 24 mg (42 µmol) Xantphos in 3.7 ml 1,4-Dioxan umgesetzt. Anschließend wurde mit 1N wässriger Salzsäure angesäuert und der Ansatz eingeengt. Der Rückstand wurde in 8 ml Dichlormethan gelöst und mittels Normalphasenchromatographie (Essigsäureethylester-Cyclohexan Gradient) gereinigt. Die Produktfraktionen wurden vereinigt und eingeengt. Der Rückstand wurde zweimal in 10 ml Diethylether verrührt, dekantiert, im Hochvakuum getrocknet und abschließend mittels präparativer HPLC (Säule: Chromatorex C18, 10 µm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt. Es wurden 159.5 mg (70% d. Th., 99% Reinheit) der Titelverbindung erhalten.
$^{1}$H-NMR (400 MHz, DMSO-d6) δ [ppm] = 10.26 (d, 1H), 9.06 (s, 1H), 8.71 (d, 1H), 8.51 (d, 1H), 7.66-7.56 (m, 2H), 4.47-4.35 (m, 1H), 3.72 (dd, 1H), 3.14 (dd, 1H), 2.73 (dd, 1H), 2.48-2.36 (m, 1H), 2.30 (dd, 1H), 1.28-1.18 (m, 1H), 1.03 (d, 3H), 0.71-0.51 (m, 3H), 0.39-0.30 (m, 1H).
LC-MS (Methode 3): $R_t$ = 2.30 min; MS (ESIpos): m/z = 539 $[M+H]^+$

## Beispiel 421

1-(2-Chlor-4,6-difluorphenyl)-7-[(4*S*)-4-hydroxy-2-oxopyrrolidin-1-yl]-4-oxo-*N*-[(2*S*)-1-(trifluormethoxy)propan-2-yl]-1,4-dihydro-1,8-naphthyridin-3-carbonsäureamid

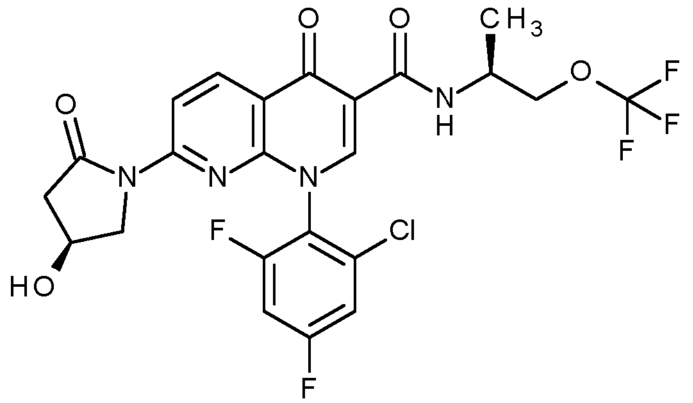

Gemäß AAV2 wurden 80.0 mg (161 µmol) der Verbindung aus Beispiel 112A mit 17.9 mg (177 µmol) 4-(4*S*)-4-Hydroxypyrrolidin-2-on in Gegenwart von 33.4 mg (242 µmol) Kaliumcarbonat, 1.8 mg (8.1 µmol) Palladium(II)acetat und 9.3 mg (16 µmol) Xantphos in 1.6 ml 1,4-Dioxan umgesetzt. Anschließend wurden 80 mg *N*-Acetylcystein zugegeben und 0.5 h bei RT gerührt. Es wurden 30 ml Essigsäureethylester zugesetzt, die organische Phase mit gesättigter wässriger Natiumhydrogencarbonat-Lösung gewaschen, über Natriumsulfat getrocknet, filtriert und eingeengt. Der Rückstand wurde mittels präp. HPLC (Säule: Chromatorex C18, 10 µm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 15.2 mg (17% d. Th., 100% Reinheit) der Titelverbindung erhalten.
$^1$H-NMR (400 MHz, DMSO-d6) δ [ppm] = 9.85 (dd, 1H), 8.95 (s, 1H), 8.71 (d, 1H), 8.52 (d, 1H), 7.82-7.71 (m, 2H), 5.33 (d, 1H), 4.42-4.14 (m, 4H), 3.69-3.60 (m, 1H), 3.46-3.38 (m, 1H), 2.93 (dd, 1H), 2.37 (d, 1H), 1.27 (dd, 3H).
LC-MS (Methode 1): $R_t$ = 0.98 min; MS (ESIpos): m/z = 561 $[M+H]^+$

## Beispiel 422

1-(2-Chlor-4,6-difluorphenyl)-7-[3-methyl-2-oxopyrrolidin-1-yl]-4-oxo-*N*-[(2*S*)-1,1,1-trifluorbutan-2-yl]-1,4-dihydro-1,8-naphthyridin-3-carbonsäureamid (Diastereomerengemisch)

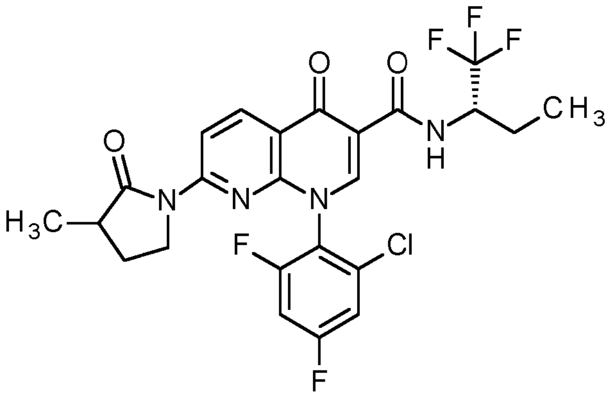

Gemäß AAV2 wurden 100 mg (208 µmol) der Verbindung aus Beispiel 108C mit 22.7 mg (229 µmol) 3-Methylpyrrolidin-2-on (Racemat) in Gegenwart von 43.2 mg (312 µmol) Kaliumcarbonat, 8.4 mg (37 µmol) Palladium(II)acetat und 43 mg (75 µmol) Xantphos in 1.9 ml 1,4-Dioxan umgesetzt. Anschließend wurde mit 0.5 ml IN wässriger Salzsäure angesäuert und der Ansatz eingeengt. Der Rückstand wurde in 5 ml Dichlormethan gelöst und mittels Normalphasenchromatographie (Essigsäureethylester-Cyclohexan Gradient) gereinigt. Es wurden 67.5 mg (59% d. Th., 99% Reinheit) der Titelverbindung erhalten.
$^1$H-NMR (400 MHz, DMSO-d6) δ [ppm] = 10.13 (d, 1H), 9.04 (s, 1H), 8.72 (d, 1H), 8.55 (d, 1H), 7.80-7.70 (m, 2H), 4.84-4.70 (m, 1H), 3.60-3.48 (m, 1H), 3.45-3.36 (m, 1H), 2.82-2.70 (m, 1H), 2.26-2.14 (m, 1H), 1.96-1.83 (m, 1H), 1.75-1.54 (m, 2H), 1.14 (d, 3H), 1.02-0.94 (m, 3H).
LC-MS (Methode 1): $R_t$ = 1.26 min; MS (ESIpos): m/z = 543 $[M+H]^+$ 46 mg der Titelverbindung (Diastereomerengemisch) wurde durch chirale HPLC in die Diastereomere getrennt (präparative HPLC: Säule Daicel Chiralpak IF 5 µm 250x20

mm; Eluent: 15% Ethanol, 85% Iso-Hexan; Temperatur: 30°C; Fluss: 15 ml/min; UV-Detektion: 220 nm.

Man erhielt (in der Reihenfolge der Elution von der Säule) 35 mg Diastereomer 1 (Atropisomerengemisch) $R_t$ = 8.71 min und 16 mg (99% de, Atropisomerengemisch) Diastereomer 2 $R_t$ = 9.96/10.69 min.

[Analytische HPLC:Säule Daicel Chiralpak IC 5 μm 250x4.6 mm; Eluent: 20% Ethanol, 80% Iso-Hexan; Fluss: 1 ml/min; Temp.: 30°C; UV-Detektion: 220 nm.]

Diastereomer 2 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 13.6 mg (12% d. Th., 99% Reinheit) der Titelverbindung aus Beispiel 423 erhalten.

Diastereomer 1 wurde nochmals mittels chiraler präp. HPLC gereinigt (Säule Daicel Chiralpak IC 5 μm 250x20 mm; Eluent: 25% Ethanol, 75% Iso-Hexan; Temperatur: 25°C; Fluss: 15 ml/min; UV-Detektion: 220 nm) und man erhielt (in der Reihenfolge der Elution von der Säule) 10 mg Atropisomer 1 (99% de) $R_t$ = 10.21 min und 11 mg (96% de) Atropisomer 2 $R_t$ = 11.11 min.

Atropisomer 1 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 7.8 mg (6.8% d. Th., 99% Reinheit) der Titelverbindung aus Beispiel 424 erhalten.

Atropisomer 2 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 7.9 mg (6.9% d. Th., 99% Reinheit) der Titelverbindung aus Beispiel 425 erhalten.

## Beispiel 423

1-(2-Chlor-4,6-difluorphenyl)-7-[3-methyl-2-oxopyrrolidin-1-yl]-4-oxo-*N*-[(2*S*)-1,1,1-trifluorbutan-2-yl]-1,4-dihydro-1,8-naphthyridin-3-carbonsäureamid (Diastereomer 2)

$^1$H-NMR (400 MHz, DMSO-d6) δ [ppm] = 10.13 (d, 1H), 9.04 (s, 1H), 8.72 (d, 1H), 8.55 (d, 1H), 7.80-7.70 (m, 2H), 4.84-4.70 (m, 1H), 3.59-3.49 (m, 1H), 3.44-3.35 (m, 1H), 2.82-2.70 (m, 1H), 2.25-2.14 (m, 1H), 1.96-1.84 (m, 1H), 1.75-1.55 (m, 2H), 1.14 (d, 3H), 1.01-0.93 (m, 3H).
LC-MS (Methode 1): $R_t$ = 1.25 min; MS (ESIpos): m/z = 543 $[M+H]^+$

## Beispiel 424

1-(2-Chlor-4,6-difluorphenyl)-7-[3-methyl-2-oxopyrrolidin-1-yl]-4-oxo-*N*-[(2*S*)-1,1,1-trifluorbutan-2-yl]-1,4-dihydro-1,8-naphthyridin-3-carbonsäureamid (Atropisomer 1)

$^1$H-NMR (400 MHz, DMSO-d6) δ [ppm] = 10.13 (d, 1H), 9.04 (s, 1H), 8.72 (d, 1H), 8.55 (d, 1H), 7.81-7.70 (m, 2H), 4.83-4.71 (m, 1H), 3.57-3.49 (m, 1H), 3.45-3.35 (m, 1H), 2.81-2.71 (m, 1H), 2.25-2.14 (m, 1H), 1.95-1.84 (m, 1H), 1.74-1.55 (m, 2H), 1.14 (d, 3H), 1.02-0.94 (m, 3H).
LC-MS (Methode 1): $R_t$ = 1.26 min; MS (ESIpos): m/z = 543 $[M+H]^+$

## Beispiel 425

1-(2-Chlor-4,6-difluorphenyl)-7-[3-methyl-2-oxopyrrolidin-1-yl]-4-oxo-*N*-[(2*S*)-1,1,1-trifluorbutan-2-yl]-1,4-dihydro-1,8-naphthyridin-3-carbonsäureamid (Atropisomer 2)

$^1$H-NMR (400 MHz, DMSO-d6) δ [ppm] = 10.13 (d, 1H), 9.03 (s, 1H), 8.72 (d, 1H), 8.55 (d, 1H), 7.80-7.69 (m, 2H), 4.83-4.70 (m, 1H), 3.60-3.51 (m, 1H), 3.42-3.35 (m, 1H), 2.82-2.70 (m, 1H), 2.25-2.14 (m, 1H), 1.97-1.84 (m, 1H), 1.75-1.55 (m, 2H), 1.14 (d, 3H), 1.03-0.94 (m, 3H).
LC-MS (Methode 1): $R_t$ = 1.26 min; MS (ESIpos): m/z = 543 $[M+H]^+$

## Beispiel 426

1-(2,4-Difluorphenyl)-7-[2-methyl-5-oxopyrrolidin-1-yl]-4-oxo-*N*-[(2*R*)-1,1,1-trifluorbutan-2-yl]-1,4-dihydro-1,8-naphthyridin-3-carbonsäureamid (Diastereomerengemisch)

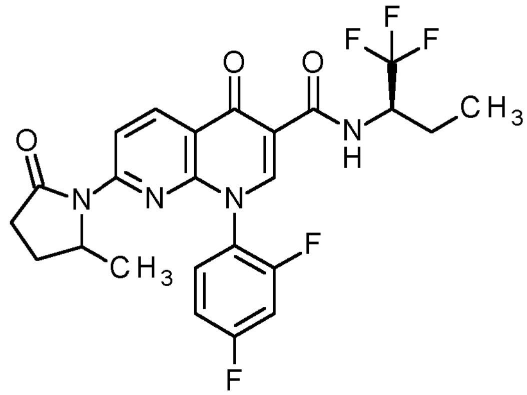

Gemäß AAV2 wurden 200 mg (449 μmol) der Verbindung aus Beispiel 67A mit 48.9 mg (494 μmol) 5-Methylpyrrolidin-2-on (Racemat) in Gegenwart von 93.0 mg (673 μmol) Kaliumcarbonat, 5.0 mg (22 μmol) Palladium(II)acetat und 26 mg (45 μmol) Xantphos in 4.4 ml 1,4-Dioxan umgesetzt. Anschließend wurden 80 mg *N*-Acetylcystein zugegeben und 30 min bei Raumtemperatur nachgerührt. Die Reaktionsmischung wurde mit 30 ml Essigsäureethylester versetzt und mit gesättigter wässriger Natriumhydrogencarbonat-Lösung gewaschen, über Natriumsulfat getrocknet, filtriert und eingeengt. Der Rückstand wurde mittels präp. HPLC (Säule: Chromatorex C18, 10 μm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 120 mg (53% d. Th., 100% Reinheit) der Titelverbindung erhalten.
$^1$H-NMR (400 MHz, DMSO-d6) δ [ppm] = 10.22 (d, 1H), 8.87 (t, 1H), 8.70 (dd, 1H), 8.49 (dd, 1H), 7.93-7.84 (m, 1H), 7.68-7.60 (m, 1H), 7.41-7.33 (m, 1H), 4.83-4.70 (m, 1H), 4.21-4.09 (m, 1H), 2.87-2.73 (m, 1H), 2.24-2.08 (m, 1H), 1.96-1.84 (m, 1H), 1.73-1.59 (m, 2H), 1.07 (d, 1.5H), 0.98 (t, 3H), 0.92 (d, 1.5H).
LC-MS (Methode 1): $R_t$ = 1.17 min; MS (ESIpos): m/z = 509 $[M+H]^+$

120 mg der Titelverbindung (Diastereomerengemisch) wurde durch chirale HPLC in die Diastereomere getrennt (präparative HPLC: Säule Daicel Chiralpak AZ-H 5 μm 250x30 mm; Eluent: 60% Ethanol, 40% Iso-Hexan; Temperatur: 23°C; Fluss: 50 ml/min; UV-Detektion: 220 nm.

Man erhielt (in der Reihenfolge der Elution von der Säule) Diastereomer 1 (99% de) $R_t$ = 1.41 min und Diastereomer 2 (99% de) $R_t$ = 2.24 min.

[Analytische HPLC:Säule Daicel Chiralpak AZ-3 3 μm 50x4.6 mm; Eluent: 50% Ethanol, 50% Iso-Hexan; Fluss: 1 ml/min; UV-Detektion: 220 nm.]

Diastereomer 1 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 41 mg (18% d. Th., 99% Reinheit) der Titelverbindung aus Beispiel 427 erhalten.

Diastereomer 2 wurde zusätzlich mittels präparativer HPLC (Säule: Chromatorex C18, 10 μm, 125 x 30 mm, Lösungsmittel: Acetonitril / 0.05% Ameisensäure-Gradient; (0 bis 3 min. 10% Acetonitril bis 35 min. 90% Acetonitril und weitere 3 min. 90% Acetonitril) gereinigt und 35.6 mg (16% d. Th., 99% Reinheit) der Titelverbindung aus Beispiel 428 erhalten.

## Beispiel 427

1-(2,4-Difluorphenyl)-7-[2-methyl-5-oxopyrrolidin-1-yl]-4-oxo-*N*-[(2*R*)-1,1,1-trifluorbutan-2-yl]-1,4-dihydro-1,8-naphthyridin-3-carbonsäureamid (Diastereomer 1)

$^1$H-NMR (400 MHz, DMSO-d6) δ [ppm] = 10.21 (d, 1H), 8.87 (s, 1H), 8.70 (dd, 1H), 8.49 (dd, 1H), 7.92-7.84 (m, 1H), 7.68-7.60 (m, 1H), 7.40-7.33 (m, 1H), 4.84-4.71 (m, 1H), 4.21-4.09 (m, 1H), 2.87-2.73 (m, 1H), 2.24-2.09 (m, 1H), 1.95-1.83 (m, 1H), 1.73-1.60 (m, 2H), 1.07 (d, 1.5H), 0.98 (t, 3H), 0.92 (d, 1.5H).
LC-MS (Methode 1): $R_t$ = 1.17 min; MS (ESIpos): m/z = 509 $[M+H]^+$

## Beispiel 428

1-(2,4-Difluorphenyl)-7-[2-methyl-5-oxopyrrolidin-1-yl]-4-oxo-*N*-[(2*R*)-1,1,1-trifluorbutan-2-yl]-1,4-dihydro-1,8-naphthyridin-3-carbonsäureamid (Diastereomer 2)

$^1$H-NMR (400 MHz, DMSO-d6) δ [ppm] = 10.22 (d, 1H), 8.87 (d, 1H), 8.70 (dd, 1H), 8.49 (dd, 1H), 7.93-7.83

(m, 1H), 7.67-7.60 (m, 1H), 7.41-7.33 (m, 1H), 4.84-4.70 (m, 1H), 4.21-4.09 (m, 1H), 2.87-2.73 (m, 1H), 2.25-2.06 (m, 1H), 1.96-1.84 (m, 1H), 1.74-1.59 (m, 2H), 1.08 (d, 1.5H), 0.98 (t, 3H), 0.93 (d, 1.5H).
LC-MS (Methode 1): $R_t$ = 1.17 min; MS (ESIpos): m/z = 509 $[M+H]^+$

**Beispiel 429**

1-(2-Chlor-4,6-difluorphenyl)-*N*-[(1*S*)-1-cyclopropyl-2,2,2-trifluorethyl]-7-[4-methyl-2-oxopyrrolidin-1-yl]-4-oxo-1,4-dihydro-1,8-naphthyridin-3-carbonsäureamid (Diastereomerengemisch)

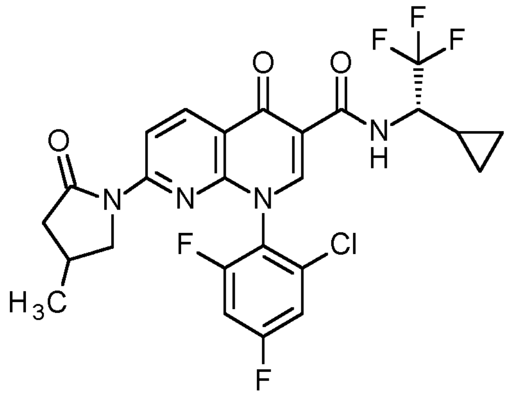

Gemäß AAV2 wurden 200 mg (406 μmol) der Verbindung aus Beispiel 110A mit 44.3 mg (447 μmol) 4-Methylpyrrolidin-2-on (Racemat) in Gegenwart von 84.2 mg (609 μmol) Kaliumcarbonat, 4.6 mg (20 μmol) Palladium(II)acetat und 24 mg (41 μmol) Xantphos in 3.6 ml 1,4-Dioxan umgesetzt. Anschließend wurde mit 1N wässriger Salzsäure angesäuert und der Ansatz eingeengt. Der Rückstand wurde in 8 ml Dichlormethan gelöst und mittels Normalphasenchromatographie (Essigsäureethylester-Cyclohexan Gradient) gereinigt. Es wurden 183 mg (80% d. Th., 99% Reinheit) der Titelverbindung erhalten.
$^1$H-NMR (400 MHz, DMSO-d6) δ [ppm] = 10.27 (d, 1H), 9.03 (s, 1H), 8.71 (d, 1H), 8.51 (d, 1H), 7.81-7.71 (m, 2H), 4.47-4.33 (m, 1H), 3.71-3.62 (m, 1H), 3.13-3.03 (m, 1H), 2.72 (dd, 1H), 2.46-2.36 (m, 1H), 2.29 (dd, 1H), 1.29-1.18 (m, 1H), 1.01 (d, 3H), 0.72-0.51 (m, 3H), 0.40-0.30 (m, 1H).
LC-MS (Methode 3): $R_t$ = 1.26 min; MS (ESIpos): m/z = 555 $[M+H]^+$

**Beispiel 430**

1-(2-Chlor-4,6-difluorphenyl)-7-[(2*R*)-2-(hydroxymethyl)-5-oxopyrrolidin-1-yl]-4-oxo-*N*-[(2S)-1,1,1-trifluorbutan-2-yl]-1,4-dihydro-1,8-naphthyridin-3-carbonsäureamid

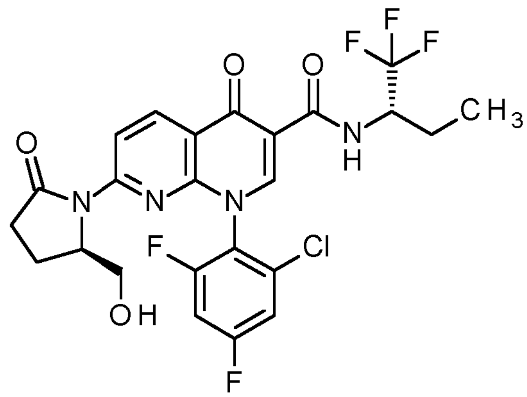

Gemäß AAV2 wurden 95.2 mg (198 μmol) der Verbindung aus Beispiel 108C mit 50.0 mg (218 μmol) der

Verbindung aus Beispiel 139A in Gegenwart von 41.1mg (297 μmol) Kaliumcarbonat, 2.2 mg (10 μmol) Palladium(II)acetat und 11 mg (20 μmol) Xantphos in 2 ml 1,4-Dioxan umgesetzt. Anschließend wurden 100 mg *N*-Acetylcystein zugegeben und 30 min bei Raumtemperatur nachgerührt. Die Reaktionsmischung wurde mit 30 ml Essigsäureethylester versetzt und mit gesättigter wässriger Natriumhydrogencarbonat-Lösung gewaschen, über Magnesiumsulfat getrocknet, filtriert und eingeengt. Der Rückstand wurde mittels präp. HPLC (Säule: Chromatorex C18, 10 μm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril) gereinigt. Die produkthaltigen Farktionen wurden vereinigt und das Lösungsmittel unter reduziertem Druck entfernt. Der Rückstand wurde in 1.5 ml THF aufgenommen und bei Eisbadkühlung mit 57.5 mg (198 μmol) Tris(dimethylamino)sulfur(trimethylsilyldifluorid) versetzt. Es wurde für 1h bei Raumtemperatur nachgerührt. Die Reaktionslösung wurde mit 15ml Wasser versetzt und dreimal mit 15 ml Essigsäureethylester extrahiert. Die vereinigten organischen Phasen wurden mit gesättigter wässriger Natriumchlorid-Lösung gewaschen, über Magnesiumsulfat getrocknet, filtriert und eingeengt. Es wurden 12 mg (10% d. Th., 90% Reinheit) der Titelverbindung erhalten.

$^1$H-NMR (400 MHz, DMSO-d6) δ [ppm] = 10.13 (dd, 1H), 9.05 (d, 1H), 8.71 (d, 1H), 8.59 (d, 1H), 7.78-7.68 (m, 2H), 4.84-4.69 (m, 2H), 4.07-4.00 (m, 1H), 3.51-3.40 (m, 1H), 3.22-3.11 (m, 0.5H), 2.81-2.69 (m, 1H), 2.45-2.34 (m, 0.5H), 2.19-1.83 (m, 3H), 1.75-1.58 (m, 1H), 1.02-0.93 (m, 3H).

LC-MS (Methode 3): $R_t$ = 1.96/1.99 min; MS (ESIpos): m/z = 559 [M+H]$^+$

**Beispiel 431**

1-(2,4-Difluorphenyl)-7-[3-methyl-2-oxopyrrolidin-1-yl]-4-oxo-*N*-[(2*R*)-1,1,1-trifluorbutan-2-yl]-1,4-dihydro-1, 8-naphthyridin-3 -carbonsäureamid (Diastereomerengemisch)

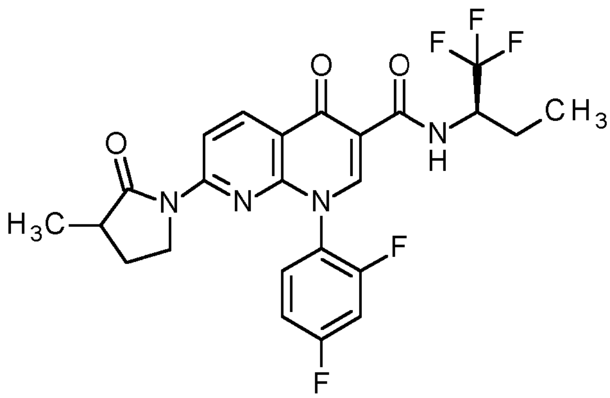

Gemäß AAV2 wurden 150 mg (336 μmol) der Verbindung aus Beispiel 67A mit 36.7 mg (370 μmol) 3-Methylpyrrolidinon in Gegenwart von 69.8 mg (505 μmol) Kaliumcarbonat, 3.8 mg (17 μmol) Palladium(II)acetat und 19 mg (34 μmol) Xantphos in 3.4 ml 1,4-Dioxan umgesetzt. Anschließend wurde das Reaktionsgemisch eingeengt und mit Wasser versetzt und mit 1M wässriger Salzsäure sauer gestellt. Es wurde dreimal mit 20 ml Essigsäureethylester extrahiert. Die vereinigten organischen Phasen wurden über Magnesiumsulfat getrocknet, filtriert und einrotiert. Der Rückstand wurde mittels Normalphasenchromatographie (Essigsäureethylester-Cyclohexan Gradient) gereinigt. Es wurden 113 mg (66% d. Th., 100% Reinheit) der Titelverbindung erhalten.

$^1$H-NMR (400 MHz, DMSO-d6) δ [ppm] = 10.22 (d, 1H), 8.85 (s, 1H), 8.70 (d, 1H), 8.53 (dd, 1H), 7.91-7.82 (m, 1H), 7.66-7.57 (m, 1H), 7.40-7.32 (m, 1H), 4.84-4.70 (m, 1H), 3.66-3.52 (m, 1H), 3.48-3.34 (m, 1H), 2.83-2.68 (m, 1H), 2.26-2.15 (m, 1H), 1.95-1.84 (m, 1H), 1.73-1.52 (m, 2H), 1.17-1.11 (m, 3H), 0.98 (t, 3H).

LC-MS (Methode 3): $R_t$ = 2.32 min; MS (ESIpos): m/z = 509 [M+H]$^+$

**Beispiel 432**

1-(2,4-Difluorphenyl)-7-[4-methyl-2-oxoimidazolidin-1-yl]-4-oxo-*N*-[(2*R*)-1,1,1-trifluorbutan-2-yl]-1,4-dihydro-1,8-naphthyridin-3-carbonsäureamid (Diastereomer 1)

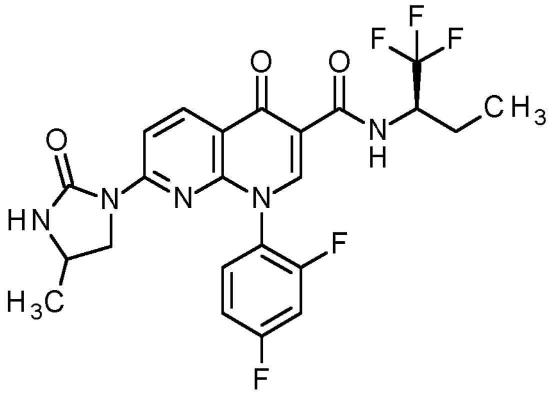

Gemäß AAV2 wurden 250 mg (561 µmol) der Verbindung aus Beispiel 67A mit 61.7 mg (617 µmol) 4-Methylimidazolidin-2-on (Racemat) in Gegenwart von 116 mg (841 µmol) Kaliumcarbonat, 6.3 mg (28 µmol) Palladium(II)acetat und 32 mg (56 µmol) Xantphos in 5 ml 1,4-Dioxan umgesetzt. Anschließend wurden 250 mg *N*-Acetylcystein zugegeben und 30 min bei Raumtemperatur nachgerührt. Die Reaktionsmischung wurde mit 30 ml Essigsäureethylester versetzt und mit gesättigter wässriger Natriumhydrogencarbonat-Lösung gewaschen, über Natriumsulfat getrocknet, filtriert und eingeengt. Der Rückstand wurde in 4 ml Acetonitril aufgenommen und mittels präp. HPLC (Säule: Chromatorex C18, 10 µm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurde weiterhin mittels Normalphasenchromatographie (Dichlormethan-Methanol 95/5) und erneuter präparativer HPLC gereinigt.

35 mg des Rohproduktes (Diastereomeren- und Regioisomerengemisch) wurde durch chirale HPLC in die Regioisomere und Diastereomere getrennt (präparative HPLC: Säule Daicel Chiralpak AD-H 5 µm 250x20 mm; Eluent: 20% Ethanol, 80% Iso-Hexan; Temperatur: 30°C; Fluss: 15 ml/min; UV-Detektion: 220 nm.

[Analytische HPLC:Säule Daicel Chiralpak IB 5 µm 250x4.6 mm; Eluent: 15% Ethanol, 85% Iso-Hexan; Fluss: 1 ml/min; Temp.: 25°C; UV-Detektion: 220 nm.]

Man erhielt (in der Reihenfolge der Elution von der Säule) 10 mg der Titelverbindung (Diastereomer 1 99% de) $R_t$ = 10.21 min, 10 mg Diastereomer 2 aus Beispiel 433 (99% de) $R_t$ = 12.52 min und 4 mg Regiosisomer aus Beispiel 434 (Diastereomerengemisch) Rt = 10.93/14.93 min.

$^1$H-NMR (400 MHz, DMSO-d6) δ [ppm] = 10.31 (d, 1H), 8.78 (s, 1H), 8.56 (d, 1H), 8.41 (d, 1H), 7.90-7.83 (m, 1H), 7.80 (br. s, 1H), 7.65-7.55 (m, 1H), 7.39-7.31 (m, 1H), 4.83-4.69 (m, 1H), 3.81-3.65 (m, 2H), 3.14-3.03 (m, 1H), 1.95-1.84 (m, 1H), 1.72-1.59 (m, 1H), 1.18-1.08 (m, 3H), 0.97 (t, 3H).

LC-MS (Methode 1): $R_t$ = 1.05 min; MS (ESIpos): m/z = 510 $[M+H]^+$

## Beispiel 433

1-(2,4-Difluorphenyl)-7-[4-methyl-2-oxoimidazolidin-1-yl]-4-oxo-*N*-[(2*R*)-1,1,1-trifluorbutan-2-yl]-1,4-dihydro-1,8-naphthyridin-3-carbonsäureamid (Diastereomer 2)

$^1$H-NMR (400 MHz, DMSO-d6) δ [ppm] = 10.31 (d, 1H), 8.78 (s, 1H), 8.56 (d, 1H), 8.41 (d, 1H), 7.91-7.82 (m, 1H), 7.80 (br. s, 1H), 7.66-7.55 (m, 1H), 7.39-7.31 (m, 1H), 4.82-4.70 (m, 1H), 3.80-3.66 (m, 2H), 3.13-3.02 (m, 1H), 1.95-1.84 (m, 1H), 1.72-1.60 (m, 1H), 1.18-1.07 (m, 3H), 0.97 (t, 3H).

LC-MS (Methode 1): $R_t$ = 1.05 min; MS (ESIpos): m/z = 510 $[M+H]^+$

## Beispiel 434

1-(2,4-Difluorphenyl)-7-[5-methyl-2-oxoimidazolidin-1-yl]-4-oxo-*N*-[(2*R*)-1,1,1-trifluorbutan-2-yl]-1,4-dihydro-1, 8-naphthyridin-3 -carbonsäureamid (Diastereomerengemisch)

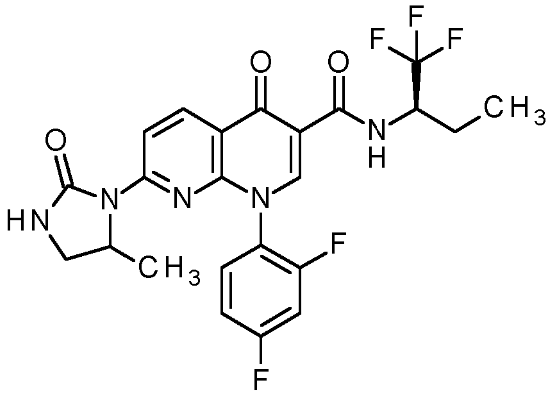

Die Titelverbindung wurde als Regioisomer aus Beispiel 432 erhalten.
$^1$H-NMR (400 MHz, DMSO-d6) δ [ppm] = 10.31 (d, 1H), 8.82-8.78 (m, 1H), 8.55 (d, 1H), 8.42-8.36 (m, 1H), 7.92-7.83 (m, 1H), 7.66-7.57 (m, 2H), 7.39-7.32 (m, 1H), 4.81-4.71 (m, 1H), 4.17-4.04 (m, 1H), 3.56-3.44 (m, 1H), 2.91 (dd, 1H), 1.95-1.83 (m, 1H), 1.72-1.61 (m, 1H), 1.08 (d, 1.5H), 0.98 (t, 3H), 0.92 (d, 1.5H).
LC-MS (Methode 1): $R_t$ = 1.04 min; MS (ESIpos): m/z = 510 $[M+H]^+$

**Beispiel 435**

*N*-[(1*S*)-1-Cyclopropyl-2,2-difluorethyl]-7-[(4*S*)-4-hydroxy-2-oxopyrrolidin-1-yl]-4-oxo-1-(2,4,6-trifluorphenyl)-1,4-dihydro-1,8-naphthyridin-3-carbonsäureamid

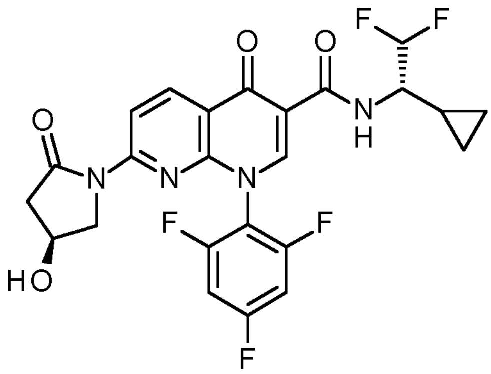

Gemäß AAV1 wurden 40 mg (95 μmol) der Verbindung aus Beispiel 117A mit 16.5 mg (105 μmol) der Verbindung aus Beispiel 140D in Gegenwart von 36 mg (95 μmol) HATU und 40.0 μl (229 μmol) *N,N*-Diisopropylethylamin in 0.55 ml Dimethylformamid zur Reaktion gebracht. Die Reaktionsmischung wurde mit 1 ml wässriger 1N Salzsäure angesäuert, mit 1 ml Acetonitril verdünnt und mittels präparativer HPLC (Säule: Chromatorex C18, 10 μm, 125x30 mm, LM: Acetonitril / 0.1 % Ameisensäure-Gradient; (0 bis 5 min. 10% Acetonitril, während 14 min. nach 90 % Acetonitril und weitere 4 min. 90 % Acetonitril) gereinigt. Es wurden 36.9 mg (72% d. Th., 97.5% Reinheit) der Titelverbindung erhalten.
$^1$H-NMR (400 MHz, DMSO-d6) δ [ppm] = 10.04 (d, 1H), 9.02 (s, 1H), 8.72 (d, 1H), 8.54 (d, 1H), 7.66-7.56 (m, 2H), 6.25 (dt, 1H), 5.33 (d, 1H), 4.32-4.26 (m, 1H), 4.03-3.88 (m, 1H), 3.70 (dd, 1H), 3.48 (d, 1H), 2.94 (dd, 1H), 2.38 (d, 1H), 1.16-1.04 (m, 1H), 0.63-0.41 (m, 3H), 0.37-0.29 (m, 1H).
LC-MS (Methode 3): $R_t$ = 1.74 min; MS (ESIpos): m/z = 523 $[M+H]^+$.

**Beispiel 436**

*N*-[(1*S*)-1-Cyclopropyl-2,2,2-trifluorethyl]-7-[5-methyl-2-oxo-1,3-oxazolidin-3-yl]-4-oxo-1-(2,4,6-trifluorphenyl)-1,4-dihydro-1,8-naphthyridin-3-carbonsäureamid (Diastereomerengemisch)

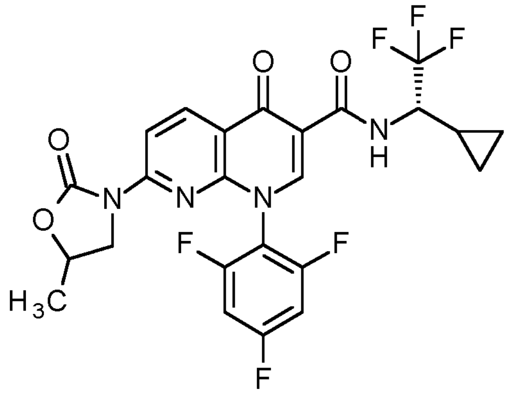

Gemäß AAV2 wurden 50.0 mg (105 µmol) der Verbindung aus Beispiel 126A mit 12.8 mg (126 µmol) 5-Methyl-1,3-oxazolidin-2-on (Racemat) in Gegenwart von 21.8 mg (158 µmol) Kaliumcarbonat, 2.4 mg (11 µmol) Palladium(II)acetat und 12 mg (21 µmol) Xantphos in 0.7 ml 1,4-Dioxan umgesetzt. Anschließend wurde mit 0.1 ml 1N wässriger Salzsäure und 3 ml Acetonitril verdünnt und mittels präp. HPLC (Säule: Chromatorex C18, 10 µm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 40.4 mg (70% d. Th., 99% Reinheit) der Titelverbindung erhalten.
$^1$H-NMR (400 MHz, DMSO-d6) δ [ppm] = 10.25 (d, 1H), 9.05 (s, 1H), 8.73 (d, 1H), 8.34 (d, 1H), 7.64-7.53 (m, 2H), 4.83-4.73 (m, 1H), 4.46-4.34 (m, 1H), 3.91 (dd, 1H), 1.36 (d, 3H), 1.29-1.19 (m, 1H), 0.71-0.52 (m, 3H), 0.39-0.30 (m, 1H).
LC-MS (Methode 3): $R_t$ = 2.18 min; MS (ESIpos): m/z = 541 $[M+H]^+$.

## Beispiel 437

*N*-[(1*S*)-1-Cyclopropyl-2,2,2-trifluorethyl]-7-[(4*R*)-4-methyl-2-oxoimidazolidin-1-yl]-4-oxo-1-(2,4,6-trifluorphenyl)-1,4-dihydro-1,8-naphthyridin-3-carbonsäureamid

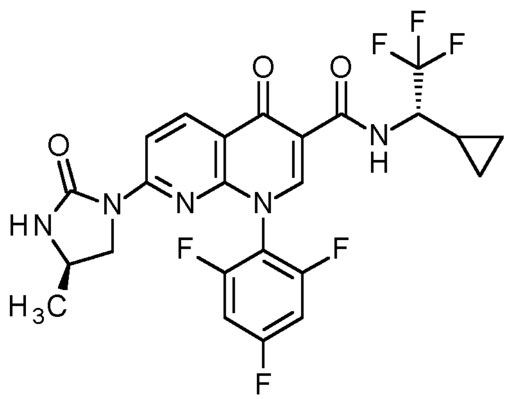

Zu einer Lösung von 168 mg (268 µmol) der Verbindung aus Beispiel 141B in 5.9 ml Dimethylformamid wurde bei Raumtemperatur 37.0 mg (268 µmol) Kaliumcarbonat und 109 mg (699 µmol) 1,1'-Carbonyldiimidazol gegeben. Es wurde für 48h nachgerührt. Anschließend wurde mit 0.1 ml 1N wässriger Salzsäure und 1 ml Acetonitril verdünnt und mittels präp. HPLC (Säule: Chromatorex C18, 10 µm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 122 mg (77% d. Th., 99% Reinheit) der Titelverbindung erhalten.
$^1$H-NMR (400 MHz, DMSO-d6) δ [ppm] = 10.35 (d, 1H), 8.98 (s, 1H), 8.57 (d, 1H), 8.44 (d, 1H), 7.83 (s, 1H), 7.63-7.54 (m, 2H), 4.46-4.34 (m, 1H), 3.81-3.69 (m, 2H), 3.15-3.04 (m, 1H), 1.28-1.17 (m, 1H), 1.12 (d, 3H), 0.71-0.50 (m, 3H), 0.39-0.30 (m, 1H).
LC-MS (Methode 3): $R_t$ = 2.04 min; MS (ESIpos): m/z = 540 $[M+H]^+$.

**Beispiel 438**

*N*-[1-Cyclopropyl-3,3,3-trifluorpropyl]-7-[(3*R*,4*R*)-3,4-dihydroxypyrrolidin-1-yl]-4-oxo-1-(2,4,6-trifluorphenyl)-1,4-dihydro-1,8-naphthyridin-3-carbonsäureamid (Diastereomerengemisch)

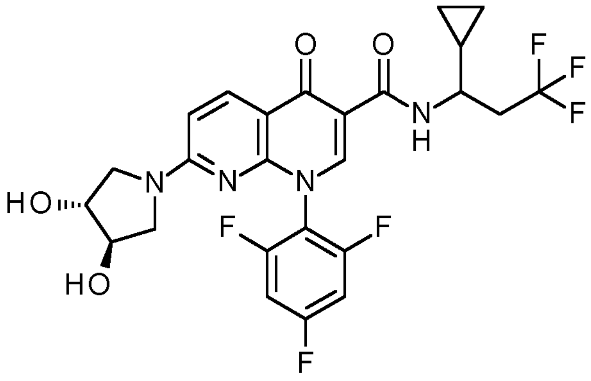

Gemäß AAV1 wurden 100 mg (237 μmol) der Verbindung aus Beispiel 121A mit 54.0 mg (285 μmol) 1-Cyclopropyl-3,3,3-trifluorpropan-1-amin Hydrochlorid (Racemat) in Gegenwart von 108 mg (285 μmol) HATU und 104 μl (593 μmol) *N,N*-Diisopropylethylamin in 2.4 ml Dimethylformamid zur Reaktion gebracht. Es wurde 2 ml 1M wässrige Salzsäure und 2 ml DMSO verdünnt und mittels präp. HPLC (Säule: Chromatorex C18, 10 μm, 125x30 mm, LM: Acetonitril / 0.05% Ameisensäure-Gradient; 0 bis 3 min. 10% Acetonitril bis 15 min. 90% Acetonitril und weitere 3 min. 90% Acetonitril) gereinigt. Es wurden 111 mg (84% d. Th., 100% Reinheit) der Titelverbindung erhalten.
$^1$H-NMR (400 MHz, DMSO-$d_6$): δ [ppm] = 10.14 (d, 1H), 8.70 (s, 1H), 8.27 (d, 1H), 7.60-7.51 (m, 2H), 6.75 (d, 1H), 5.23 (d, 1H), 5.13 (d, 1H), 4.05 (br. s, 1H), 3.92 (br. s, 1H), 3.84-3.73 (m, 1H), 3.65-3.56 (m, 1H), 3.25 (dd, 1H), 3.07 (d, 1H), 2.85-2.59 (m, 2H), 1.21-1.10 (m, 1H), 0.57-0.43 (m, 2H), 0.39-0.24 (m, 2H).
LC-MS (Methode 1): $R_t$ = 0.91 min; 557 [M+H]$^+$.

110 mg der Titelverbindung (Diastereomerengemisch) wurde durch chirale HPLC in die Diastereomere getrennt (präparative HPLC: Säule Daicel Chiralpak AD-H 5 μm 250x20 mm; Eluent: 20% Ethanol, 80% n-Heptan; Temperatur: 23°C; Fluss: 20 ml/min; UV-Detektion: 220 nm.)

Man erhielt (in der Reihenfolge der Elution von der Säule) 38.7 mg Diastereomer 1 (99% de) $R_t$ = 2.49 min und 41.0 mg (92%de) Diastereomer 2 $R_t$ = 3.09 min.

[Analytische HPLC: Säule Daicel IE-3 5 μm 50x4.6 mm; Eluent: 20% Ethanol, 80% Iso-Hexan; UV-Detektion: 220 nm].

Diastereomer 1 wurde zusätzlich mittels präparativer HPLC (Säule: Chromatorex C18, 10 μm, 125x30 mm, LM: Acetonitril / 0.1% Ameisensäure-Gradient; 0 bis 5 min. 10% Acetonitril, während 14 min. nach 90% Acetonitril und weitere 4 min. 90% Acetonitril) gereinigt und 35.4 mg (27% d. Th., 100% Reinheit) der Titelverbindung aus Beispiel 439 erhalten.

Diastereomer 2 wurde zusätzlich mittels präparativer HPLC (Säule: Chromatorex C18, 10 μm, 125x30 mm, LM: Acetonitril / 0.1% Ameisensäure-Gradient; 0 bis 5 min. 10% Acetonitril, während 14 min. nach 90% Acetonitril und weitere 4 min. 90% Acetonitril) gereinigt und 37.1 mg (28% d. Th., 100% Reinheit) der Titelverbindung aus Beispiel 440 erhalten.

**Beispiel 439**

*N*-[1-Cyclopropyl-3,3,3-trifluorpropyl]-7-[(3*R*,4*R*)-3,4-dihydroxypyrrolidin-1-yl]-4-oxo-1-(2,4,6-trifluorphenyl)-1,4-dihydro-1,8-naphthyridin-3-carbonsäurcamid (Diastereomer 1)

$^1$H-NMR (400 MHz, DMSO-$d_6$): δ [ppm] = 10.14 (d, 1H), 8.70 (s, 1H), 8.27 (d, 1H), 7.60-7.51 (m, 2H), 6.76 (d, 1H), 5.23 (d, 1H), 5.14 (d, 1H), 4.05 (br. s, 1H), 3.92 (br. s, 1H), 3.84-3.74 (m, 1H), 3.65-3.57 (m, 1H), 3.25 (dd, 1H), 3.06 (d, 1H), 2.85-2.60 (m, 2H), 1.20-1.10 (m, 1H), 0.57-0.43 (m, 2H), 0.39-0.25 (m, 2H).
LC-MS (Methode 1): $R_t$ = 0.90 min; 557 [M+H]$^+$.

**Beispiel 440**

*N*-[1-Cyclopropyl-3,3,3-trifluorpropyl]-7-[(3*R*,4*R*)-3,4-dihydroxypyrrolidin-1-yl]-4-oxo-1-(2,4,6-trifluorphenyl)-1,4-dihydro-1,8-naphthyridin-3-carbonsäureamid (Diastereomer 2)

$^1$H-NMR (400 MHz, DMSO-$d_6$): δ [ppm] = 10.14 (d, 1H), 8.70 (s, 1H), 8.26 (d, 1H), 7.60-7.51 (m, 2H), 6.76 (d, 1H), 5.23 (d, 1H), 5.14 (d, 1H), 4.05 (br. s, 1H), 3.92 (br. s, 1H), 3.83-3.74 (m, 1H), 3.64-3.57 (m, 1H), 3.25 (dd, 1H), 3.07 (d, 1H), 2.82-2.60 (m, 2H), 1.20-1.10 (m, 1H), 0.57-0.43 (m, 2H), 0.39-0.25 (m, 2H).
LC-MS (Methode 1): $R_t$ = 0.90 min; 557 [M+H]$^+$.

**Beispiel 441**

*N*-[2-Cyclopropyl-1,1,1-trifluorpropan-2-yl]-7-[(3*R*,4*R*)-3,4-dihydroxypyrrolidin-1-yl]-4-oxo-1-(2,4,6-trifluorphenyl)-1,4-dihydro-1,8-naphthyridin-3 -carbonsäureamid (Diastereomerengemisch)

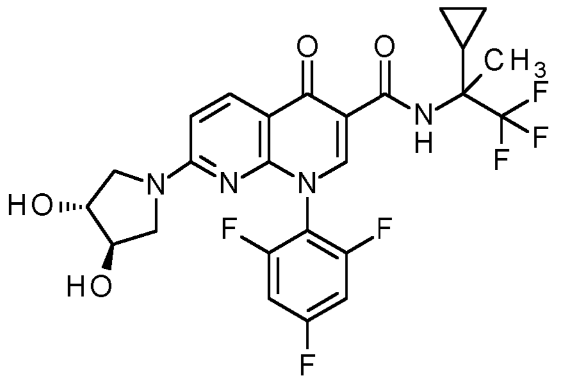

Gemäß AAV1 wurden 100 mg (237 μmol) der Verbindung aus Beispiel 121A mit 36.3 mg (237 μmol) 2-Cyclopropyl-1,1,1-trifluorpropan-2-amin (Racemat) in Gegenwart von 108 mg (285 μmol) HATU und 104 μl (593 μmol) *N,N*-Diisopropylethylamin in 3.3 ml Dimethylformamid zur Reaktion gebracht. Es wurde 1 ml 1M wässrige Salzsäure und 2 ml DMSO verdünnt und mittels präp. HPLC (Säule: Chromatorex C18, 10 μm, 125x30 mm, LM: Acetonitril / 0.05% Ameisensäure-Gradient; 0 bis 3 min. 10% Acetonitril bis 15 min. 90% Acetonitril und weitere 3 min. 90% Acetonitril) gereinigt. Es wurden 71.1 mg (54% d. Th., 100% Reinheit) der Titelverbindung erhalten.
$^1$H-NMR (400 MHz, DMSO-$d_6$): δ [ppm] = 10.38 (s, 1H), 8.73 (s, 1H), 8.28 (d, 1H), 7.61-7.52 (m, 2H), 6.77 (d, 1H), 5.22 (d, 1H), 5.14 (d, 1H), 4.05 (br. s, 1H), 3.92 (br. s, 1H), 3.61 (dd, 1H), 3.25 (dd, 1H), 3.07 (d, 1H), 1.60 (s, 3H), 1.45-1.37 (m, 1H), 0.72-0.66 (m, 1H), 0.59-0.46 (m, 3H).
LC-MS (Methode 1): $R_t$ = 0.96 min; 557 [M+H]$^+$.

70 mg der Titelverbindung (Diastereomerengemisch) wurde durch chirale HPLC in die Diastereomere getrennt (präparative HPLC: Säule Daicel Chiralpak IB 5 μm 250x20 mm; Eluent: 25% Iso-Propanol, 75% n-Heptan; Temperatur: 30°C; Fluss: 15 ml/min; UV-Detektion: 220 nm.)

Man erhielt (in der Reihenfolge der Elution von der Säule) 33.0 mg Diastereomer 1 (99% de) $R_t$ = 4.61 min und 32.0 mg (98.5%de) Diastereomer 2 $R_t$ = 5.24 min.

[Analytische HPLC: Säule Daicel Chiralpak IB 5 μm 250x4.6 mm; Eluent: 30% Iso-Propanol, 70% n-Heptan; Fluss: 1.0 ml/min; Temp.: 35°C; UV-Detektion: 220 nm].

Diastereomer 1 wurde zusätzlich mittels präparativer HPLC (0.1% Ameisensäure; Wasser-Acetonitril Gradient) gereinigt und 29.2 mg (22% d. Th., 100% Reinheit) der Titelverbindung aus Beispiel 442 erhalten.

Diastereomer 2 wurde zusätzlich mittels präparativer HPLC (0.1 % Ameisensäure; Wasser-Acetonitril Gradient) gereinigt und 30.7 mg (23% d. Th., 100% Reinheit) der Titelverbindung aus Beispiel 443 erhalten.

**Beispiel 442**

*N*-[2-Cyclopropyl-1,1,1-trifluorpropan-2-yl]-7-[(3*R*,4*R*)-3,4-dihydroxypyrrolidin-1-yl]-4-oxo-1-(2,4,6-trifluorphenyl)-1,4-dihydro-1,8-naphthyridin-3-carbonsäureamid (Diastereomer 1)

$^{1}$H-NMR (400 MHz, DMSO-$d_6$): δ [ppm] = 10.38 (s, 1H), 8.73 (s, 1H), 8.29 (d, 1H), 7.61-7.51 (m, 2H), 6.77 (d, 1H), 5.23 (d, 1H), 5.14 (d, 1H), 4.05 (br. s, 1H), 3.92 (br. s, 1H), 3.61 (dd, 1H), 3.25 (dd, 1H), 3.07 (d, 1H), 1.60 (s, 3H), 1.45-1.36 (m, 1H), 0.73-0.65 (m, 1H), 0.60-0.45 (m, 3H).
LC-MS (Methode 3): $R_t$ = 1.82 min; 557 $[M+H]^+$.

**Beispiel 443**

*N*-[2-Cyclopropyl-1,1,1-trifluorpropan-2-yl]-7-[(3*R*,4*R*)-3,4-dihydroxypyrrolidin-1-yl]-4-oxo-1-(2,4,6-trifluorphenyl)-1,4-dihydro-1,8-naphthyridin-3-carbonsäureamid (Diastereomer 1)

$^{1}$H-NMR (400 MHz, DMSO-$d_6$): δ [ppm] = 10.38 (s, 1H), 8.73 (s, 1H), 8.29 (d, 1H), 7.61-7.51 (m, 2H), 6.76 (d, 1H), 5.23 (d, 1H), 5.14 (d, 1H), 4.05 (br. s, 1H), 3.93 (br. s, 1H), 3.61 (dd, 1H), 3.25 (dd, 1H), 3.06 (d, 1H), 1.60 (s, 3H), 1.46-1.37 (m, 1H), 0.73-0.64 (m, 1H), 0.61-0.45 (m, 3H).
LC-MS (Methode 3): $R_t$ = 1.82 min; 557 $[M+H]^+$.

**Beispiel 444**

*N*-[(1*S*)-1-Cyclopropyl-2,2,2-trifluorethyl]-7-[(5*R*)-5-methyl-2-oxo-1,3-oxazolidin-3-yl]-4-oxo-1-(2,4,6-trifluorphenyl)-1,4-dihydro-1,8-naphthyridin-3-carbonsäureamid

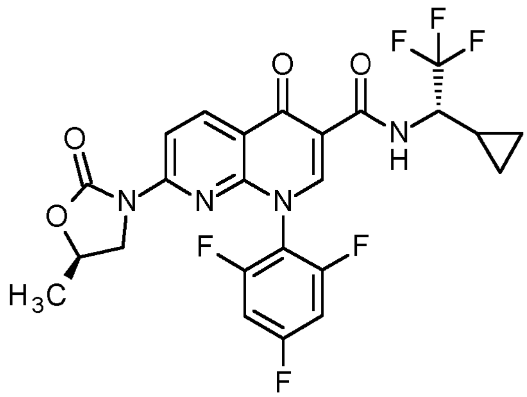

Zu einer Lösung von 137 mg (266 μmol) der Verbindung aus Beispiel 378 in 5.9 ml Dimethylformamid wurde bei Raumtemperatur 108 mg (666 μmol) 1,1'-Carbonyldiimidazol gegeben. Es wurde für 6d nachgerührt. Anschließend wurde mit 0.1 ml 1N wässriger Salzsäure und 1 ml Acetonitril verdünnt und mittels präp. HPLC (Säule: Chromatorex C18, 10 μm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 90 mg (62% d. Th., 99% Reinheit) der Titelverbindung erhalten.
$^{1}$H-NMR (400 MHz, DMSO-d6) δ [ppm] = 10.25 (d, 1H), 9.05 (s, 1H), 8.73 (d, 1H), 8.34 (d, 1H), 7.65-7.53 (m, 2H), 4.83-4.73 (m, 1H), 4.47-4.35 (m, 1H), 3.91 (dd, 1H), 1.37 (d, 3H), 1.29-1.18 (m, 1H), 0.72-0.52 (m, 3H), 0.39-0.31 (m, 1H), eine Resonanz unter dem Wassersignal.
LC-MS (Methode 3): $R_t$ = 2.20 min; MS (ESIpos): m/z = 541 $[M+H]^+$.

**Beispiel 445**

*N*-[(1*S*)-1-Cyclopropyl-2,2,2-trifluorethyl]-7-[(5*S*)-5-methyl-2-oxo-1,3-oxazolidin-3-yl]-4-oxo-1-(2,4,6-trifluorphenyl)-1,4-dihydro-1,8-naphthyridin-3-carbonsäureamid

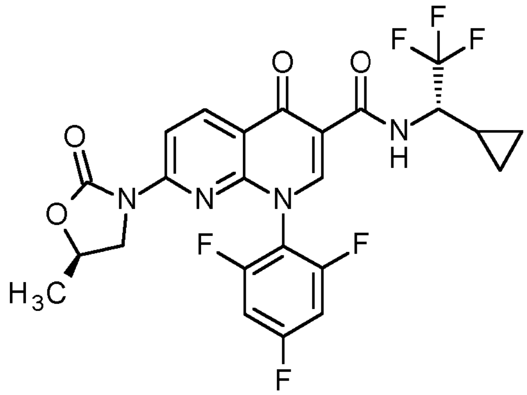

Zu einer Lösung von 114 mg (222 µmol) der Verbindung aus Beispiel 142A in 4.9 ml Dimethylformamid wurde bei Raumtemperatur 126 mg (775 µmol) 1,1'-Carbonyldiimidazol gegeben. Es wurde für 7d nachgerührt. Anschließend wurde mit 0.1 ml 1N wässriger Salzsäure und 1 ml Acetonitril verdünnt und mittels präp. HPLC (Säule: Chromatorex C18, 10 µm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril) gereinigt. Es wurden 45.2 mg (37% d. Th., 99% Reinheit) der Titelverbindung erhalten.
$^1$H-NMR (400 MHz, DMSO-d6) δ [ppm] = 10.25 (d, 1H), 9.05 (s, 1H), 8.73 (d, 1H), 8.34 (d, 1H), 7.64-7.54 (m, 2H), 4.83-4.73 (m, 1H), 4.47-4.34 (m, 1H), 3.91 (dd, 1H), 1.37 (d, 3H), 1.29-1.19 (m, 1H), 0.71-0.52 (m, 3H), 0.39-0.32 (m, 1H), eine Resonanz unter dem Wassersignal.
LC-MS (Methode 1): $R_t$ = 1.18 min; MS (ESIpos): m/z = 541 $[M+H]^+$.

**Beispiel 446**

*N*-[(1*S*)-1-Cyclopropyl-2,2,2-trifluorethyl]-7-[4-ethyl-2-oxotetrahydropyrimidin-1(2*H*)-yl]-4-oxo-1-(2,4,6-trifluorphenyl)-1,4-dihydro-1,8-naphthyridin-3-carbonsäureamid (Diastereomerengemisch)

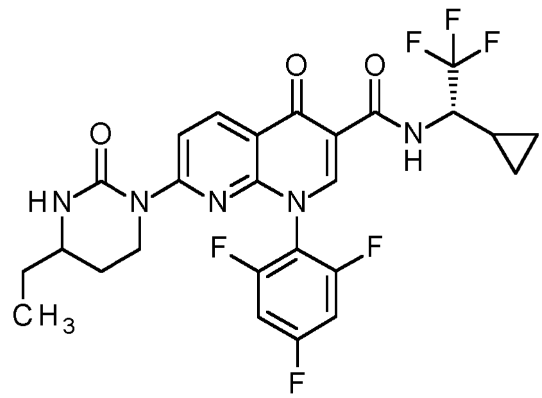

Gemäß AAV2 wurden 100 mg (210 µmol) der Verbindung aus Beispiel 126A mit 29.6 mg (231 µmol) 4-Ethyltetrahydropyrimidin-2(1*H*)-on (Racemat) in Gegenwart von 43.6 mg (315 µmol) Kaliumcarbonat, 4.7 mg (21 µmol) Palladium(II)acetat und 24 mg (42 µmol) Xantphos in 2.1 ml 1,4-Dioxan umgesetzt. Anschließend wurde mit 3 ml Acetonitril und 2 ml Wasser verdünnt und mittels präp. HPLC (Säule: Chromatorex C18, 10 µm, 125x30 mm, LM: Acetonitril / 0.05% Ameisensäure-Gradient; 0 bis 3 min. 15% Acetonitril bis 15 min. 90% Acetonitril und weitere 3 min. 90% Acetonitril) gereinigt. Es wurden 24.3 mg (20% d. Th., 100% Reinheit) der Titelverbindung erhalten.
$^1$H-NMR (400 MHz, DMSO-d6) δ [ppm] = 10.33 (d, 1H), 9.01 (s, 1H), 8.53 (d, 1H), 8.31 (d, 1H), 7.64-7.54 (m, 2H), 7.40 (s, 1H), 4.46-4.34 (m, 1H), 3.73-3.65 (m, 1H), 3.27-3.20 (m, 1H), 2.00-1.92 (m, 1H), 1.61-1.46 (m, 2H), 1.41-1.18 (m, 2H), 0.85 (t, 3H), 0.71-0.51 (m, 3H), 0.39-0.31 (m, 1H).
LC-MS (Methode 3): $R_t$ = 2.15 min; MS (ESIpos): m/z = 568 $[M+H]^+$.
24 mg der Titelverbindung (Diastereomerengemisch) wurde durch chirale HPLC in die Diastereomere getrennt (präparative HPLC: Säule Daicel Chiralpak AZ-H 5 µm 250x30 mm; Eluent: 50% Ethanol, 50% n-Heptan; Temperatur:

23°C; Fluss: 50 ml/min; UV-Detektion: 220 nm.)

Man erhielt (in der Reihenfolge der Elution von der Säule) 5.6 mg Diastereomer 1 (Verbindung 447,99% de, 100% Reinheit) $R_t$ = 1.23 min und 9.9 mg (Verbindung 448, 99%de, 100% Reinheit) Diastereomer 2 $R_t$ = 1.63 min.

[Analytische HPLC: Säule Daicel Chiralpak AZ-3 3 μm 50x4.6 mm; Eluent: 50% Ethanol, 50% iso-Hexan; Fluss: 1.0 ml/min; UV-Detektion: 220 nm].

**Beispiel 447**

*N*-[(1*S*)-1-Cyclopropyl-2,2,2-trifluorethyl]-7-[4-ethyl-2-oxotetrahydropyrimidin-1(2*H*)-yl]-4-oxo-1-(2,4,6-trifluorphenyl)-1,4-dihydro-1,8-naphthyridin-3-carbonsäureamid (Diastereomer 1)

$^1$H-NMR (400 MHz, DMSO-d6) δ [ppm] = 10.33 (d, 1H), 9.01 (s, 1H), 8.53 (d, 1H), 8.31 (d, 1H), 7.64-7.55 (m, 2H), 7.39 (s, 1H), 4.47-4.33 (m, 1H), 3.75-3.64 (m, 1H), 3.28-3.20 (m, 1H), 2.01-1.90 (m, 1H), 1.61-1.45 (m, 2H), 1.41-1.18 (m, 2H), 0.85 (t, 3H), 0.71-0.49 (m, 3H), 0.39-0.30 (m, 1H).
LC-MS (Methode 1): $R_t$ = 1.16 min; MS (ESIpos): m/z = 568 $[M+H]^+$.

**Beispiel 448**

*N*-[(1*S*)-1-Cyclopropyl-2,2,2-trifluorethyl]-7-[4-ethyl-2-oxotetrahydropyrimidin-1(2*H*)-yl]-4-oxo-1-(2,4,6-trifluorphenyl)-1,4-dihydro-1,8-naphthyridin-3-carbonsäureamid (Diastereomer 2)

$^1$H-NMR (400 MHz, DMSO-d6) δ [ppm] = 10.33 (d, 1H), 9.01 (s, 1H), 8.53 (d, 1H), 8.32 (d, 1H), 7.65-7.54 (m, 2H), 7.40 (s, 1H), 4.47-4.33 (m, 1H), 3.74-3.65 (m, 1H), 3.28-3.20 (m, 1H), 2.00-1.91 (m, 1H), 1.61-1.45 (m, 2H), 1.41-1.11 (m, 2H), 0.85 (t, 3H), 0.71-0.50 (m, 3H), 0.39-0.30 (m, 1H).
LC-MS (Methode 1): $R_t$ = 1.16 min; MS (ESIpos): m/z = 568 $[M+H]^+$.

**Beispiel 449**

*N*-[(1*S*)-1-Cyclopropyl-2,2,2-trifluorethyl]-7-[(4*S*)-4-methyl-2-oxoimidazolidin-1-yl]-4-oxo-1-(2,4,6-trifluorphenyl)-1,4-dihydro-1,8-naphthyridin-3-carbonsäureamid

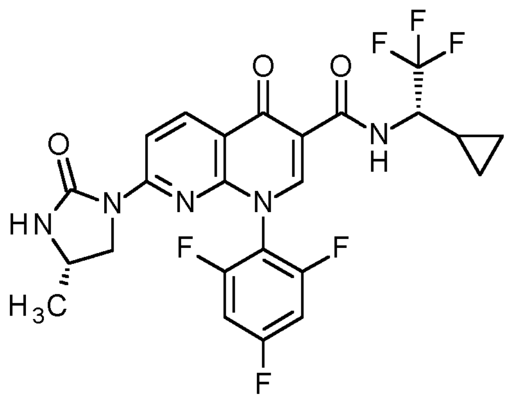

Zu einer Lösung von 184 mg (292 μmol) der Verbindung aus Beispiel 143B in 6.4 ml Dimethylformamid wurde bei Raumtemperatur 40.4 mg (292 μmol) Kaliumcarbonat und 119 mg (731 μmol) 1,1'-Carbonyldiimidazol gegeben. Es wurde für 48h nachgerührt. Anschließend wurde mit 0.2 ml 1N wässriger Salzsäure und 1 ml Acetonitril verdünnt und mittels präp. HPLC (Säule: Chromatorex C18, 10 μm, 125x30 mm, LM: Acetonitril / 0.1% Ameisensäure-Gradient; 0 bis 5 min. 10% Acetonitril, während 14 min. nach 90% Acetonitril und weitere 4 min. 90% Acetonitril) gereinigt. Es wurden 111 mg (70% d. Th., 99% Reinheit) der Titelverbindung erhalten.
$^1$H-NMR (400 MHz, DMSO-d6) δ [ppm] = 10.35 (d, 1H), 8.98 (s, 1H), 8.57 (d, 1H), 8.44 (d, 1H), 7.83 (s, 1H), 7.63-7.53 (m, 2H), 4.47-4.34 (m, 1H), 3.82-3.69 (m, 2H), 3.14-3.05 (m, 1H), 1.28-1.18 (m, 1H), 1.13 (d, 3H), 0.71-0.50 (m, 3H), 0.39-0.30 (m, 1H).
LC-MS (Methode 1): $R_t$ = 1.10 min; MS (ESIpos): m/z = 540 $[M+H]^+$.

**Beispiel 450**

*N*-[(1*S*)-1-Cyclopropyl-2,2,2-trifluorethyl]-4-oxo-7-(5-oxo-4,6-diazaspiro[2.4]hept-6-yl)-1-(2,4,6-trifluorphenyl)-1,4-dihydro-1,8-naphthyridin-3-carbonsäureamid

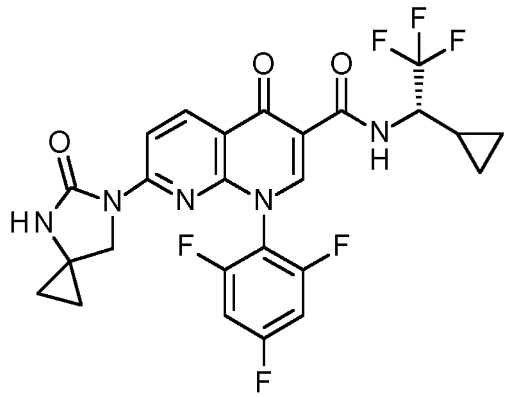

Zu einer Lösung von 183 mg (243 µmol) der Verbindung aus Beispiel 144B in 5.4 ml Dimethylformamid wurde bei Raumtemperatur 67.1 mg (486 µmol) Kaliumcarbonat und 98.5 mg (607 µmol) 1,1'-Carbonyldiimidazol gegeben. Es wurde für 6h nachgerührt. Anschließend wurde mit 0.2 ml 1N wässriger Salzsäure und 1 ml Acetonitril verdünnt und mittels präp. HPLC (Säule: Chromatorex C18, 10 µm, 125x30 mm, Laufmittel: Acetonitril / 0.1% Ameisensäure-Gradient; 0 bis 5 min. 10% Acetonitril, während 14 min. nach 90% Acetonitril und weitere 4 min. 90% Acetonitril) gereinigt. Es wurden 116 mg (86% d. Th., 99% Reinheit) der Titelverbindung erhalten.
$^1$H-NMR (400 MHz, DMSO-d6) δ [ppm] = 10.34 (d, 1H), 8.97 (s, 1H), 8.59 (d, 1H), 8.45 (d, 1H), 7.90 (s, 1H), 7.59-7.50 (m, 2H), 4.47-4.34 (m, 1H), 3.60 (s, 2H), 1.28-1.18 (m, 1H), 0.82-0.50 (m, 7H), 0.39-0.30 (m, 1H).
LC-MS (Methode 3): $R_t$ = 2.08 min; MS (ESIpos): m/z = 552 $[M+H]^+$.

**Beispiel 451**

*N*-[(1*S*)-1-Cyclopropyl-2,2,2-trifluorethyl]-7-(4,4-dimethyl-2-oxoimidazolidin-1-yl)-4-oxo-1-(2,4,6-trifluorphenyl)-1,4-dihydro-1,8-naphthyridin-3-carbonsäureamid

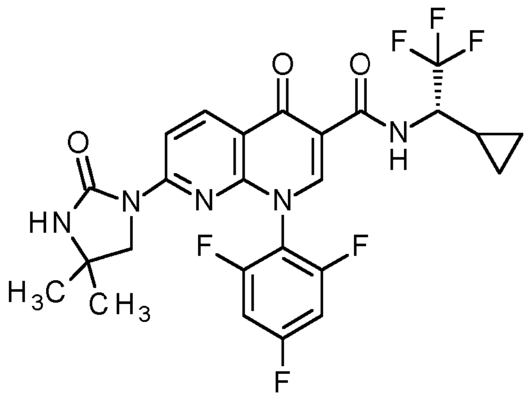

Zu einer Lösung von 190 mg (251 µmol) der Verbindung aus Beispiel 145B in 5.5 ml Dimethylformamid wurde bei Raumtemperatur 69.4mg (502 µmol) Kaliumcarbonat und 102 mg (628 µmol) 1,1'-Carbonyldiimidazol gegeben. Es wurde für 4d nachgerührt. Es wurden jeweils 1 Äquivalent 1,1'-Carbonyldiimidazol und Kaliumcarbonat nachgegeben und für weitere 48h bei Raumtemperatur nachgerührt. Anschließend wurde der Ansatz mit 25 ml 0.5M wässriger Salzsäure und 50 ml Essigsäureethylester verdünnt. Die Phasen wurden getrennt und die wässrige Phase dreimal mit 25 ml Essigsäureethylester extrahiert, über Magnesiumsulfat getrocknet, filtriert und das Lösungsmittel unter reduziertem

Druck entfernt. Der Rückstand wurde in 0.2 ml 1N wässriger Salzsäure und 1 ml Acetonitril aufgenommen und mittels präp. HPLC (Säule: Chromatorex C18, 10 μm, 125x30 mm, LM: Acetonitril / 0.1% Ameisensäure-Gradient; 0 bis 5 min. 10% Acetonitril, während 14 min. nach 90% Acetonitril und weitere 4 min. 90% Acetonitril) gereinigt. Es wurden 39.7 mg (28% d. Th., 99% Reinheit) der Titelverbindung erhalten.
$^1$H-NMR (400 MHz, DMSO-d6) δ [ppm] = 10.35 (d, 1H), 8.98 (s, 1H), 8.57 (d, 1H), 8.44 (d, 1H), 7.89 (s, 1H), 7.64-7.55 (m, 2H), 4.46-4.33 (m, 1H), 1.28-1.14 (m, 7H), 0.70-0.50 (m, 3H), 0.39-0.29 (m, 1H).
LC-MS (Methode 3): $R_t$ = 2.07 min; MS (ESIpos): m/z = 554 $[M+H]^+$.

**Beispiel 452**

*N*-(1,1-Difluor-2-methylpropan-2-yl)-7-[(4*R*)-4-methyl-2-oxoimidazolidin-1-yl]-4-oxo-1-(2,4,6-trifluorphenyl)-1,4-dihydro-1,8-naphthyridin-3-carbonsäureamid

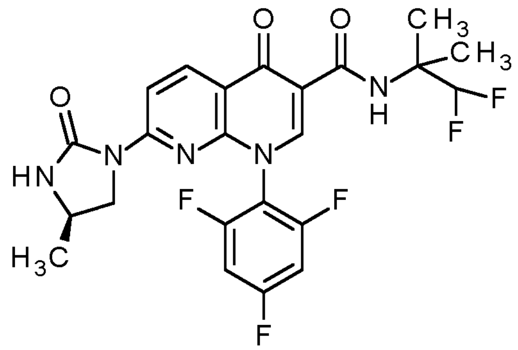

Gemäß AAV1 wurden 100 mg (232 μmol, 97% Reinheit) der Verbindung aus Beispiel 146D mit 40.5 mg (278 μmol) 1,1-Difluor-2-methylpropan-2-amin Hydrochlorid in Gegenwart von 106 mg (278 μmol) HATU und 101 μl (580 μmol) *N,N*-Diisopropylethylamin in 6.2 ml Dimethylformamid zur Reaktion gebracht. Es wurde 1 ml 1M wässrige Salzsäure und Acetonitril verdünnt und mittels präp. HPLC (Chromatorex C18, 10 μm, 125x30 mm, LM: Acetonitril / 0.05% Ameisensäure-Gradient; 0 bis 3 min. 10% Acetonitril bis 15 min. 90% Acetonitril und weitere 3 min. 90% Acetonitril) gereinigt. Es wurden 47 mg (40% d. Th., 100% Reinheit) der Titelverbindung erhalten.
$^1$H-NMR (400 MHz, DMSO-$d_6$): δ [ppm] = 10.12 (s, 1H), 8.90 (s, 1H), 8.55 (d, 1H), 8.42 (d, 1H), 7.81 (s, 1H), 7.62-7.53 (m, 2H), 6.43 (t, 1H), 3.80-3.69 (m, 2H), 3.14-3.05 (m, 1H), 1.45 (s, 6H), 1.12 (d, 3H).
LC-MS (Methode 3): $R_t$ = 1.94 min; 510 $[M+H]^+$.

**Beispiel 453**

7-[(4*R*)-4-Methyl-2-oxoimidazolidin-1-yl]-4-oxo-*N*-[(2*S*)-1-(trifluormethoxy)butan-2-yl]-1-(2,4,6-trifluorphenyl)-1,4-dihydro-1,8-naphthyridin-3-carbonsäureamid (Diastereomerengemisch)

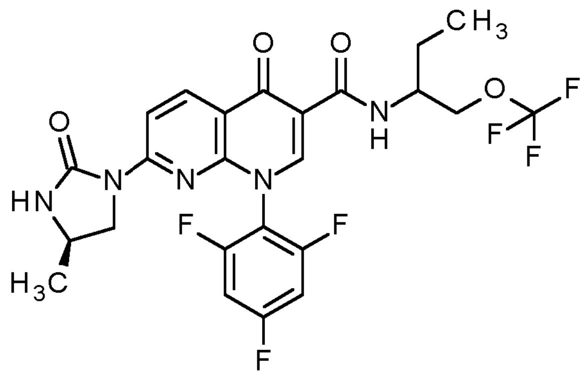

Gemäß AAV1 wurden 100 mg (232 µmol, 97% Reinheit) der Verbindung aus Beispiel 146D mit 53.9 mg (278 µmol) 1-(Trifluormethoxy)butan-2-amin Hydrochlorid in Gegenwart von 106 mg (278 µmol) HATU und 101 µl (580 µmol) *N,N*-Diisopropylethylamin in 2.3 ml Dimethylformamid zur Reaktion gebracht. Der Ansatz wurde mit 1M wässriger Salzsäure auf pH 1 gestellt, mit 20 ml Wasser verdünnt und dreimal mit 20 ml Essigsäureethylester extrahiert. Die vereinigten organischen Phasen wurden mit gesättigter wässriger Natriumchlorid-Lösung gewaschen, über Magnesiumsulfat getrocknet, filtriert und eingeengt. Der Rückstand wurde in Acetonitril, DMSO, Dioxan und THF angelöst. Die unlöslichen Bestandteile wurden abgesaugt und entsprachen der Titelverbindung (109 mg, 84% d. Th., 100% Reinheit). Die Mutterlauge wurde mittels präp. HPLC (Säule: Chromatorex C18, 10 µm, 125x30 mm, LM: Acetonitril / 0.05% Ameisensäure-Gradient; 0 bis 3 min. 10% Acetonitril bis 15 min. 90% Acetonitril und weitere 3 min. 90% Acetonitril) gereinigt. Die Produktfraktionen wurden vereinigt, eingeengt und lyophilisiert. Es wurden 56.8mg (44% d. Th., 100% Reinheit) der Titelverbindung erhalten.
$^1$H-NMR (400 MHz, DMSO-$d_6$): δ [ppm] = 9.89 (d, 1H), 8.91 (s, 1H), 8.55 (d, 1H), 8.42 (d, 1H), 7.82 (s, 1H), 7.64-7.52 (m, 2H), 4.26-4.13 (m, 3H), 3.82-3.69 (m, 2H), 3.15-3.04 (m, 1H), 1.76-1.53 (m, 2H), 1.13 (d, 3H), 0.95 (t, 3H).
LC-MS (Methode 3): $R_t$ = 2.03 min; 558 [M+H]$^+$.

166 mg der Titelverbindung (Diastereomerengemisch) wurde durch chirale HPLC in die Diastereomere getrennt (präparative HPLC: Säule YMC Chiralart Cellulose SA 5 µm 250x30 mm; Eluent: 20% Iso-Propanol, 80% n-Heptan; Temperatur: 30°C; Fluss: 30 ml/min; UV-Detektion: 220 nm.)

Man erhielt (in der Reihenfolge der Elution von der Säule) 27 mg Diastereomer 1 (99% de) $R_t$ = 7.09 min und 28 mg (98%de) Diastereomer 2 $R_t$ = 7.87 min.

[Analytische HPLC: Säule YMC Chiralart Amylose SA 5 µm 250x4.6 mm; Eluent: 20% Iso-Propanol, 80% n-Heptan; Fluss: 1.0 ml/min; Temp.: 30°C; UV-Detektion: 220 nm].

Diastereomer 1 wurde zusätzlich mittels präparativer HPLC (Säule: Chromatorex C18, 10 µm, 125x30 mm, LM: Acetonitril / 0.1% Ameisensäure-Gradient; 0 bis 5 min. 10% Acetonitril, während 14 min. nach 90% Acetonitril und weitere 4 min. 90% Acetonitril) gereinigt und 26.5 mg (20% d. Th., 99% Reinheit) der Titelverbindung aus Beispiel 454 erhalten.

Diastereomer 2 wurde zusätzlich mittels präparativer HPLC (Säule: Chromatorex C18, 10 µm, 125x30 mm, LM: Acetonitril / 0.1% Ameisensäure-Gradient; 0 bis 5 min. 10% Acetonitril, während 14 min. nach 90% Acetonitril und weitere 4 min. 90% Acetonitril) gereinigt und 26.5 mg (20% d. Th., 99% Reinheit) der Titelverbindung aus Beispiel 455 erhalten.

## Beispiel 454

7-[(4*R*)-4-Methyl-2-oxoimidazolidin-1-yl]-4-oxo-*N*-[(2*S*)-1-(trifluormethoxy)butan-2-yl]-1-(2,4,6-trifluorphenyl)-1,4-dihydro-1,8-naphthyridin-3-carbonsäureamid (Diastereomer 1)

$^1$H-NMR (400 MHz, DMSO-$d_6$): δ [ppm] = 9.89 (d, 1H), 8.91 (s, 1H), 8.55 (d, 1H), 8.42 (d, 1H), 7.82 (s, 1H), 7.63-7.53 (m, 2H), 4.25-4.13 (m, 3H), 3.80-3.69 (m, 2H), 3.14-3.05 (m, 1H), 1.75-1.53 (m, 2H), 1.12 (d, 3H), 0.95 (t, 3H).
LC-MS (Methode 3): $R_t$ = 2.04 min; 558 [M+H]$^+$.

## Beispiel 455

7-[(4*R*)-4-Methyl-2-oxoimidazolidin-1-yl]-4-oxo-*N*-[(2*S*)-1-(trifluormethoxy)butan-2-yl]-1-(2,4,6-trifluorphenyl)-1,4-dihydro-1,8-naphthyridin-3-carbonsäureamid (Diastereomer 2)

$^1$H-NMR (400 MHz, DMSO-$d_6$): δ [ppm] = 9.89 (d, 1H), 8.91 (s, 1H), 8.56 (d, 1H), 8.42 (d, 1H), 7.82 (s, 1H), 7.63-7.52 (m, 2H), 4.26-4.14 (m, 3H), 3.81-3.69 (m, 2H), 3.15-3.04 (m, 1H), 1.75-1.53 (m, 2H), 1.13 (d, 3H), 0.95 (t, 3H).
LC-MS (Methode 3): $R_t$ = 2.03 min; 558 [M+H]$^+$.

## Beispiel 456

7-[(4*R*)-4-Methyl-2-oxoimidazolidin-1-yl]-4-oxo-*N*-[(2*S*)-1,1,1-trifluorbutan-2-yl]-1-(2,4,6-trifluorphenyl)-1,4-dihydro-1,8-naphthyridin-3-carbonsäureamid

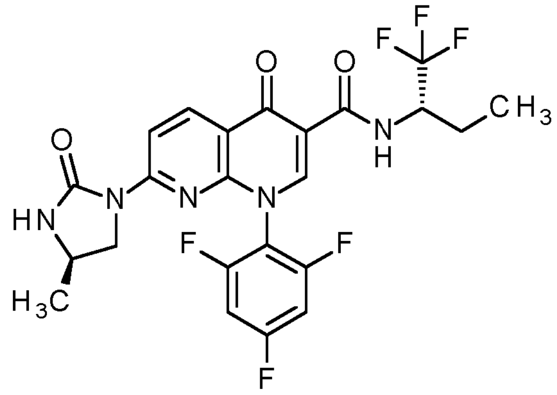

Gemäß AAV1 wurden 100 mg (232 µmol, 97% Reinheit) der Verbindung aus Beispiel 146D mit 45.5 mg (278 µmol) (2*S*)-1,1,1-Trifluorbutan-2-amin Hydrochlorid in Gegenwart von 106 mg (278 µmol) HATU und 101 µl (580 µmol) *N,N*-Diisopropylethylamin in 2.3 ml Dimethylformamid zur Reaktion gebracht. Der Ansatz wurde mit 1 ml 1M wässriger Salzsäure und Acetonitril verdünnt und mittels präp. HPLC (Säule: Chromatorex C18, 10 µm, 125x30 mm, LM: Acetonitril / 0.05% Ameisensäure-Gradient; 0 bis 3 min. 10% Acetonitril bis 15 min. 90% Acetonitril und weitere 3 min. 90% Acetonitril) gereinigt. Die Produktfraktionen wurde vereinigt, eingeengt und lyophilisiert. Es wurden 57.8mg (47% d. Th., 100% Reinheit) der Titelverbindung erhalten.
$^1$H-NMR (400 MHz, DMSO-$d_6$): δ [ppm] = 10.21 (d, 1H), 8.99 (s, 1H), 8.56 (d, 1H), 8.44 (d, 1H), 7.83 (s, 1H), 7.65-7.53 (m, 2H), 4.83-4.68 (m, 1H), 3.81-3.68 (m, 2H), 3.16-3.03 (m, 1H), 1.96-1.82 (m, 1H), 1.74-1.58 (m, 1H), 1.13 (d, 3H), 0.98 (t, 3H).
LC-MS (Methode 3): $R_t$ = 2.00 min; 528 [M+H]$^+$.

**Beispiel 457**

7-[(4*R*)-4-Methyl-2-oxoimidazolidin-1-yl]-4-oxo-*N*-(4,4,4-trifluor-2-methylbutan-2-yl)-1-(2,4,6-trifluorphenyl)-1,4-dihydro-1,8-naphthyridin-3-carbonsäureamid

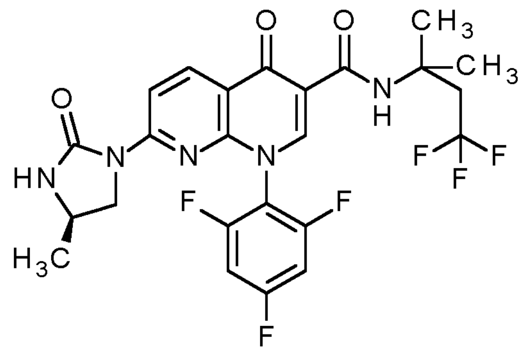

Gemäß AAV1 wurden 100 mg (232 µmol, 97% Reinheit) der Verbindung aus Beispiel 146D mit 20.6 mg (116 µmol) 4,4,4-Trifluor-2-methylbutan-2-amin Hydrochlorid in Gegenwart von 106 mg (278 µmol) HATU und 101 µl (580 µmol) *N,N*-Diisopropylethylamin in 2.3 ml Dimethylformamid zur Reaktion gebracht. Der Ansatz wurde mit 1 ml 1M wässriger Salzsäure und Acetonitril verdünnt und mittels präp. HPLC (Säule: Chromatorex C18, 10 µm, 125x30 mm, LM: Acetonitril / 0.05% Ameisensäure-Gradient; 0 bis 3 min. 10% Acetonitril bis 15 min. 90% Acetonitril und weitere 3 min. 90% Acetonitril) gereinigt. Die Produktfraktionen wurde vereinigt, eingeengt und lyophilisiert. Es wurden 49.8mg (40% d. Th., 100% Reinheit) der Titelverbindung erhalten.
$^1$H-NMR (400 MHz, DMSO-$d_6$): δ [ppm] = 9.98 (s, 1H), 8.88 (s, 1H), 8.54 (d, 1H), 8.41 (d, 1H), 7.81 (s, 1H), 7.64-7.53 (m, 2H), 3.82-3.68 (m, 2H), 3.14-3.05 (m, 1H), 2.96 (q, 2H), 1.49 (s, 6H), 1.13 (d, 3H).
LC-MS (Methode 3): $R_t$ = 2.02 min; 542 [M+H]$^+$.

**Beispiel 458**

7-[(4*R*)-4-Methyl-2-oxoimidazolidin-1-yl]-4-oxo-*N*-[3,3,4,4,4-pentafluorbutan-2-yl]-1-(2,4,6-trifluorphenyl)-1,4-dihydro-1,8-naphthyridin-3-carbonsäureamid (Diastereomerengemisch)

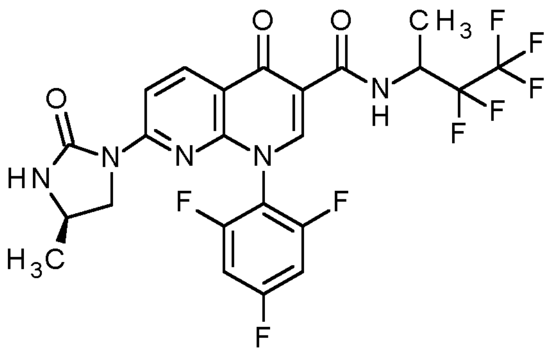

Gemäß AAV1 wurden 100 mg (232 µmol, 97% Reinheit) der Verbindung aus Beispiel 146D mit 53.9 mg (278 µmol) der Verbindung aus Beispiel 147B in Gegenwart von 106 mg (278 µmol) HATU und 101 µl (580 µmol) *N*,*N*-Diisopropylethylamin in 2.3 ml Dimethylformamid zur Reaktion gebracht. Der Ansatz wurde mit 1 ml 1M wässriger Salzsäure und Acetonitril verdünnt und mittels präp. HPLC (Säule: Chromatorex C18, 10 µm, 125x30 mm, LM: Acetonitril / 0.05% Ameisensäure-Gradient; 0 bis 3 min. 10% Acetonitril bis 15 min. 90% Acetonitril und weitere 3 min. 90% Acetonitril) gereinigt. Die Produktfraktionen wurde vereinigt, eingeengt und lyophilisiert. Es wurden 52.6mg (40% d. Th., 100% Reinheit) der Titelverbindung erhalten.
$^1$H-NMR (400 MHz, DMSO-$d_6$): δ [ppm] = 10.33 (d, 1H), 8.99 (s, 1H), 8.55 (d, 1H), 8.44 (d, 1H), 7.83 (s, 1H), 7.63-7.53 (m, 2H), 5.11-4.95 (m, 1H), 3.82-3.68 (m, 2H), 3.14-3.04 (m, 1H), 1.41 (d, 3H), 1.13 (d, 3H).
LC-MS (Methode 3): $R_t$ = 2.05 min; 564 $[M+H]^+$.

50 mg der Titelverbindung (Diastereomerengemisch) wurde durch chirale HPLC in die Diastereomere getrennt (präparative HPLC: Säule Daicel Chiralpak AD-H 5 µm 250x20 mm; Eluent: 30% Ethanol, 70% n-Heptan; Temperatur: 25°C; Fluss: 15 ml/min; UV-Detektion: 210 nm.)

Man erhielt (in der Reihenfolge der Elution von der Säule) 20.8 mg Diastereomer 1 (99% de) $R_t$ = 1.51 min und 20.2 mg (99%de) Diastereomer 2 $R_t$ = 2.09 min.

[Analytische HPLC: Säule Daicel AD-3 3 µm 50x4.6 mm; Eluent: 20% Ethanol, 80% iso-Hexan; UV-Detektion: 220 nm].

Diastereomer 1 wurde zusätzlich mittels präparativer HPLC (Säule: Chromatorex C18, 10 µm, 125x30 mm, LM: Acetonitril / 0.1% Ameisensäure-Gradient; 0 bis 2.5 min. 10% Acetonitril, während 15.5 min. nach 90% Acetonitril und weitere 2 min. 90% Acetonitril) gereinigt und 19.1 mg (15% d. Th., 100% Reinheit) der Titelverbindung aus Beispiel 459 erhalten.

Diastereomer 2 wurde zusätzlich mittels präparativer HPLC (Säule: Chromatorex C18, 10 µm, 125x30 mm, LM: Acetonitril / 0.1% Ameisensäure-Gradient; 0 bis 2.5 min. 10% Acetonitril, während 15.5 min. nach 90% Acetonitril und weitere 2 min. 90% Acetonitril) gereinigt und 11.8 mg (9% d. Th., 100% Reinheit) der Titelverbindung aus Beispiel 460 erhalten.

**Beispiel 459**

7-[(4*R*)-4-Methyl-2-oxoimidazolidin-1-yl]-4-oxo-*N*-[3,3,4,4,4-pentafluorbutan-2-yl]-1-(2,4,6-trifluorphenyl)-1,4-dihydro-1,8-naphthyridin-3-carbonsäureamid (Diastereomer 1)

$^1$H-NMR (400 MHz, DMSO-$d_6$): δ [ppm] = 10.33 (d, 1H), 8.99 (s, 1H), 8.55 (d, 1H), 8.43 (d, 1H), 7.83 (s, 1H), 7.63-7.53 (m, 2H), 5.10-4.95 (m, 1H), 3.80-3.69 (m, 2H), 3.15-3.05 (m, 1H), 1.41 (d, 3H), 1.13 (d, 3H).
LC-MS (Methode 3): $R_t$ = 2.05 min; 564 $[M+H]^+$.

**Beispiel 460**

7-[(4*R*)-4-Methyl-2-oxoimidazolidin-1-yl]-4-oxo-*N*-[3,3,4,4,4-pentafluorbutan-2-yl]-1-(2,4,6-trifluorphenyl)-1,4-dihydro-1,8-naphthyridin-3-carbonsäureamid (Diastereomer 2)

$^1$H-NMR (400 MHz, DMSO-$d_6$): δ [ppm] = 10.33 (d, 1H), 8.99 (s, 1H), 8.55 (d, 1H), 8.44 (d, 1H), 7.83 (s, 1H), 7.64-7.53 (m, 2H), 5.11-4.96 (m, 1H), 3.81-3.69 (m, 2H), 3.14-3.03 (m, 1H), 1.41 (d, 3H), 1.12 (d, 3H).
LC-MS (Methode 3): $R_t$ = 2.05 min; 564 $[M+H]^+$.

**Beispiel 461**

*N*-[(1*S*)-1-Cyclopropyl-2,2,2-trifluorethyl]-7-[4-fluor-4-(hydroxymethyl)piperidin-1-yl]-4-oxo-1-(2,4,6-trifluorphenyl)-1,4-dihydro-1,8-naphthyridin-3-carbonsäureamid

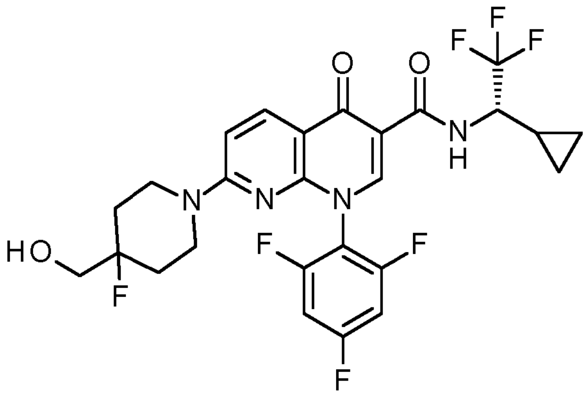

Gemäß AAV3 wurden 100 mg (210 μmol) der Verbindung aus Beispiel 126A mit 39.2 mg (231 μmol) (4-Fluorpiperidin-4-yl)methanol Hydrochlorid und 128 μl (736 μmol) *N,N*-Diisopropylethylamin in 2.1 ml Dimethylformamid zur Reaktion gebracht. Die Reaktionslösung wurde mit Acetonitril und 1 ml 1N wässriger Salzsäure verdünnt und mittels präparativer HPLC (Säule: Chromatorex C18, 10 μm, 125x30 mm, LM: Acetonitril / 0.05% Ameisensäure-Gradient; 0 bis 3 min. 10% Acetonitril, bis 15 min. nach 90% Acetonitril und weitere 3 min. 90% Acetonitril) gereinigt und 102 mg (85% d. Th., 100% Reinheit) der Titelverbindung erhalten.
$^1$H NMR (400 MHz, DMSO-$d_6$): δ [ppm] = 10.51 (d, 1H), 8.82 (s, 1H), 8.30 (d, 1H), 7.61-7.52 (m, 2H), 7.19 (d, 1H), 4.96 (t, 1H), 4.44-4.32 (m, 1H), 4.05-3.93 (m, 2H), 3.39 (dd, 2H), 3.24-3.10 (m, 2H), 1.78-1.47 (m, 4H), 1.26-1.15 (m, 1H), 0.70-0.47 (m, 3H), 0.39-0.29 (m, 1H).
LC-MS (Methode 3): $R_t$ = 2.01 min; MS (ESIpos) m/z 573 $[M+H]^+$.

**Beispiel 462**

*N*-[(1*S*)-1-Cyclopropyl-2,2,2-trifluorethyl]-7-[(2*R*)-2-(hydroxymethyl)piperidin-1-yl]-4-oxo-1-(2,4,6-trifluorphenyl)-1,4-dihydro-1,8-naphthyridin-3-carbonsäureamid

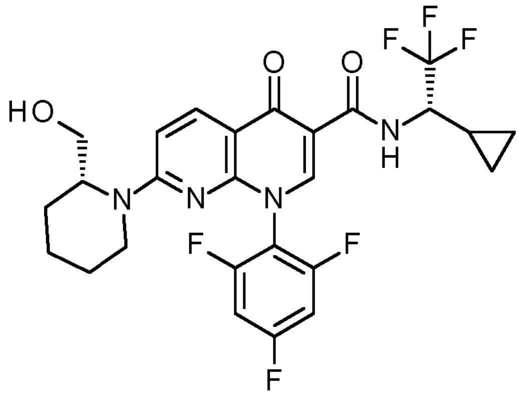

Gemäß AAV3 wurden 100 mg (210 µmol) der Verbindung aus Beispiel 126A mit 35.1 mg (231 µmol) (2*R*)-Piperidin-2-ylmethanol Hydrochlorid und 128 µl (736 µmol) *N,N*-Diisopropylethylamin in 2.1 ml Dimethylformamid zur Reaktion gebracht. Die Reaktionslösung wurde mit Acetonitril und 1 ml 1N wässriger Salzsäure verdünnt und mittels präparativer HPLC (Säule: Chromatorex C18, 10 µm, 125x30 mm, LM: Acetonitril / 0.05% Ameisensäure-Gradient; 0 bis 3 min. 10% Acetonitril, bis 15 min. nach 90% Acetonitril und weitere 3 min. 90% Acetonitril) gereinigt und 74.1 mg (64% d. Th., 100% Reinheit) der Titelverbindung erhalten.
$^1$H NMR (400 MHz, DMSO-$d_6$): δ [ppm] = 10.55 (d, 1H), 8.78 (s, 1H), 8.24 (d, 1H), 7.60-7.48 (m, 2H), 7.10 (d, 1H), 4.74-4.63 (m, 1H), 4.44-4.32 (m, 1H), 4.27-4.18 (m, 1H), 4.13-4.03 (m, 1H), 3.57-3.43 (m, 2H), 2.91-2.73 (m, 1H), 1.82-1.72 (m, 1H), 1.64-1.40 (m, 4H), 1.36-1.14 (m, 2H), 0.70-0.48 (m, 3H), 0.38-0.30 (m, 1H).
LC-MS (Methode 3): $R_t$ = 2.14 min; MS (ESIpos) m/z 555 $[M+H]^+$.

## Beispiel 463

*N*-[(1*S*)-1-Cyclopropyl-2,2,2-trifluorethyl]-7-[2-(hydroxymethyl)morpholin-4-yl]-4-oxo-1-(2,4,6-trifluorphenyl)-1,4-dihydro-1,8-naphthyridin-3-carbonsäureamid (Diastereomerengemisch)

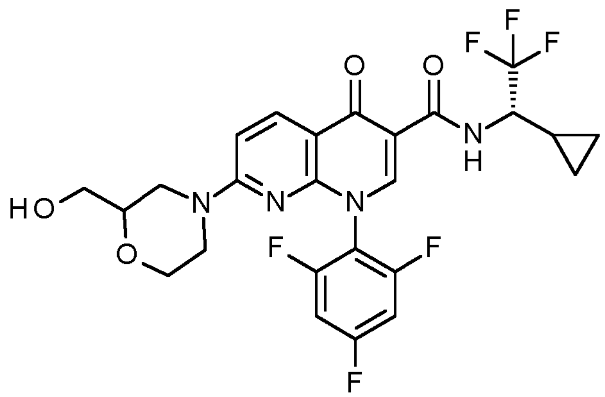

Gemäß AAV3 wurden 100 mg (210 µmol) der Verbindung aus Beispiel 126A mit 37.4 mg (231 µmol, 95% Reinheit) Morpholin-2-ylmethanol Hydrochlorid und 128 µl (736 µmol) *N,N*-Diisopropylethylamin in 2.1 ml Dimethylformamid zur Reaktion gebracht. Die Reaktionslösung wurde mit Acetonitril und 1 ml 1N wässriger Salzsäure verdünnt und mittels präparativer HPLC (Säule: Chromatorex C18, 10 µm, 125x30 mm, LM: Acetonitril / 0.05% Ameisensäure-Gradient; 0 bis 3 min. 10% Acetonitril, bis 15 min. nach 90% Acetonitril und weitere 3 min. 90% Acetonitril) gereinigt und 102 mg (87% d. Th., 100% Reinheit) der Titelverbindung erhalten.
$^1$H NMR (400 MHz, DMSO-$d_6$): δ [ppm] = 10.50 (d, 1H), 8.83 (s, 1H), 8.33 (d, 1H), 7.59-7.48 (m, 2H), 7.12 (d, 1H), 4.78-4.71 (m, 1H), 4.44-4.32 (m, 1H), 4.10 (d, 1H), 3.96 (d, 1H), 3.86 (d, 1H), 3.48-3.37 (m, 2H), 2.98 (br. s, 1H), 2.72 (br. s, 1H), 1.26-1.15 (m, 1H), 0.71-0.48 (m, 3H), 0.39-0.30.
LC-MS (Methode 3): $R_t$ = 1.88 min; MS (ESIpos) m/z 557 $[M+H]^+$.
100 mg der Titelverbindung (Diastereomerengemisch) wurde durch chirale HPLC in die Diastereomere getrennt (präparative HPLC: Säule Daicel Chiralpak ID 5 µm 250x20 mm; Eluent: 20% Iso-Propanol, 80% n-Heptan; Temperatur:

30°C; Fluss: 15 ml/min; UV-Detektion: 220 nm.)

Man erhielt (in der Reihenfolge der Elution von der Säule) 45 mg Diastereomer 1 (99% de) $R_t$ = 13.46 min und 30 mg (99%de) Diastereomer 2 $R_t$ = 14.69 min.

[Analytische HPLC: Säule Daicel Chiralpak ID 5 μm 250x4.6 mm; Eluent: 20% Iso-Propanol, 80% iso-Hexan; Fluss: 1 ml/min; Temp.: 30°C; UV-Detektion: 220 nm].

Diastereomer 1 wurde zusätzlich mittels präparativer HPLC (Säule: Chromatorex C18, 10 μm, 125x30 mm, LM: Acetonitril / 0.1% Ameisensäure-Gradient; 0 bis 5 min. 10% Acetonitril, während 14 min. nach 90% Acetonitril und weitere 4 min. 90% Acetonitril) gereinigt und 40.0 mg (34% d. Th., 99% Reinheit) der Titelverbindung aus Beispiel 464 erhalten.

Diastereomer 2 wurde zusätzlich mittels präparativer HPLC (Säule: Chromatorex C18, 10 μm, 125x30 mm, LM: Acetonitril / 0.1% Ameisensäure-Gradient; 0 bis 5 min. 10% Acetonitril, während 14 min. nach 90% Acetonitril und weitere 4 min. 90% Acetonitril) gereinigt und 24.6 mg (21% d. Th., 99% Reinheit) der Titelverbindung aus Beispiel 465 erhalten.

## Beispiel 464

*N*-[(1*S*)-1-Cyclopropyl-2,2,2-trifluorethyl]-7-[2-(hydroxymethyl)morpholin-4-yl]-4-oxo-1-(2,4,6-trifluorphenyl)-1,4-dihydro-1,8-naphthyridin-3-carbonsäureamid (Diastereomer 1)

$^1$H NMR (400 MHz, DMSO-$d_6$): δ [ppm] = 10.50 (d, 1H), 8.83 (s, 1H), 8.33 (d, 1H), 7.59-7.48 (m, 2H), 7.12 (d, 1H), 4.78-4.72 (m, 1H), 4.44-4.32 (m, 1H), 4.10 (d, 1H), 3.96 (d, 1H), 3.86 (d, 1H), 3.47-3.38 (m, 2H), 2.98 (br. s, 1H), 2.72 (br. s, 1H), 1.26-1.16 (m, 1H), 0.70-0.48 (m, 3H), 0.38-0.30.
LC-MS (Methode 3): $R_t$ = 1.88 min; MS (ESIpos) m/z 557 $[M+H]^+$.

## Beispiel 465

*N*-[(1*S*)-1-Cyclopropyl-2,2,2-trifluorethyl]-7-[2-(hydroxymethyl)morpholin-4-yl]-4-oxo-1-(2,4,6-trifluorphenyl)-1,4-dihydro-1,8-naphthyridin-3-carbonsäureamid (Diastereomer 2)

$^1$H NMR (400 MHz, DMSO-$d_6$): δ [ppm] = 10.50 (d, 1H), 8.83 (s, 1H), 8.33 (d, 1H), 7.59-7.48 (m, 2H), 7.12 (d, 1H), 4.78-4.71 (m, 1H), 4.45-4.32 (m, 1H), 4.10 (d, 1H), 3.96 (d, 1H), 3.86 (d, 1H), 3.48-3.37 (m, 2H), 2.98 (br. s, 1H), 2.72 (br. s, 1H), 1.26-1.15 (m, 1H), 0.71-0.48 (m, 3H), 0.38-0.30.
LC-MS (Methode 3): $R_t$ = 1.88 min; MS (ESIpos) m/z 557 $[M+H]^+$.

## Beispiel 466

*N*-[(1*S*)-1-Cyclopropyl-2,2,2-trifluorethyl]-7-[(3*R*)-3-hydroxy-2-oxopyrrolidin-1-yl]-4-oxo-1-(2,4,6-trifluorphenyl)-1,4-dihydro-1,8-naphthyridin-3-carbonsäureamid

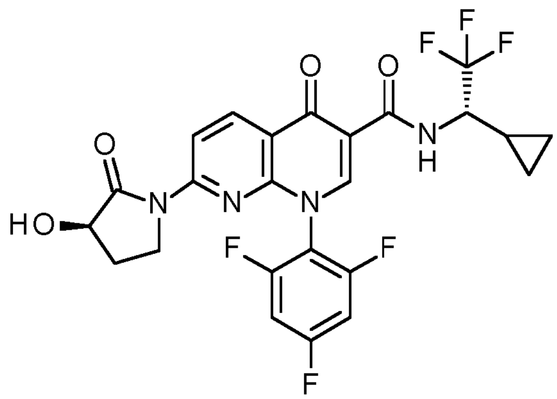

Gemäß AAV2 wurden 100 mg (210 μmol) der Verbindung aus Beispiel 126A mit 23.4 mg (231 μmol) (3*R*)-3-Hydroxypyrrolidin-2-on in Gegenwart von 43.6 mg (315 μmol) Kaliumcarbonat, 4.7 mg (21 μmol) Palladium(II)acetat und 24 mg (42 μmol) Xantphos in 2.1 ml 1,4-Dioxan umgesetzt. Anschließend wurde mit Wasser und Acetonitril verdünnt

und mittels präp. HPLC (Säule: Chromatorex C18, 10 μm, 125x30 mm, LM: Acetonitril / 0.05% Ameisensäure-Gradient; 0 bis 3 min. 15% Acetonitril bis 15 min. 90% Acetonitril und weitere 3 min. 90% Acetonitril) gereinigt. Es wurden 58.1 mg (51% d. Th., 100% Reinheit) der Titelverbindung erhalten.
$^1$H-NMR (400 MHz, DMSO-d6) δ [ppm] = 10.25 (d, 1H), 9.07 (s, 1H), 8.74 (d, 1H), 8.55 (d, 1H), 7.64-7.55 (m, 2H), 5.92 (d, 1H), 4.46-4.34 (m, 2H), 3.63-3.53 (m, 1H), 2.37-2.27 (m, 1H), 1.84-1.71 (m, 1H), 1.29-1.19 (m, 1H), 0.71-0.51 (m, 3H), 0.39-0.30 (m, 1H), eine Resonanz teilweise unter dem Wassersignal.
LC-MS (Methode 3): $R_t$ = 1.88 min; MS (ESIpos): m/z = 541 $[M+H]^+$.

**Beispiel 467**

*N*-[(1*S*)-1-Cyclopropyl-2,2,2-trifluorethyl]-7-[(3*S*)-3-hydroxy-2-oxopyrrolidin-1-yl]-4-oxo-1-(2,4,6-trifluorphenyl)-1,4-dihydro-1,8-naphthyridin-3-carbonsäureamid

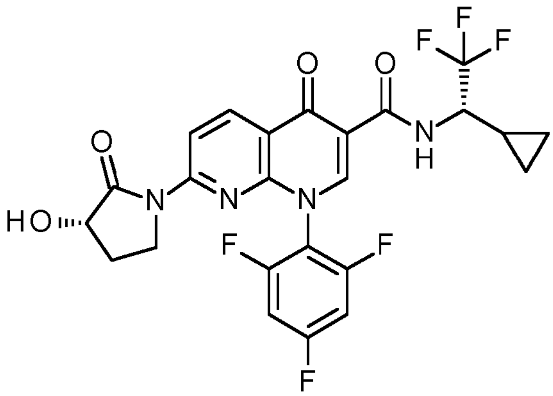

Gemäß AAV2 wurden 100 mg (210 μmol) der Verbindung aus Beispiel 126A mit 23.4 mg (231 μmol) (3*S*)-3-Hydroxypyrrolidin-2-on in Gegenwart von 43.6 mg (315 μmol) Kaliumcarbonat, 4.7 mg (21 μmol) Palladium(II)acetat und 24 mg (42 μmol) Xantphos in 2.1 ml 1,4-Dioxan umgesetzt. Anschließend wurde mit Wasser, Acetonitril verdünnt und mittels präp. HPLC (Säule: Chromatorex C18, 10 μm, 125x30 mm, LM: Acetonitril / 0.05% Ameisensäure-Gradient; 0 bis 3 min. 15% Acetonitril bis 15 min. 90% Acetonitril und weitere 3 min. 90% Acetonitril) gereinigt. Es wurden 62.1 mg (55% d. Th., 100% Reinheit) der Titelverbindung erhalten.
$^1$H-NMR (400 MHz, DMSO-d6) δ [ppm] = 10.25 (d, 1H), 9.07 (s, 1H), 8.74 (d, 1H), 8.55 (d, 1H), 7.64-7.55 (m, 2H), 5.92 (d, 1H), 4.47-4.34 (m, 2H), 3.61-3.54 (m, 1H), 2.38-2.27 (m, 1H), 1.84-1.71 (m, 1H), 1.29-1.18 (m, 1H), 0.71-0.51 (m, 3H), 0.39-0.30 (m, 1H), eine Resonanz teilweise unter dem Wassersignal.
LC-MS (Methode 3): $R_t$ = 1.88 min; MS (ESIpos): m/z = 541 $[M+H]^+$.

**Beispiel 468**

*N*-[(1*S*)-1-Cyclopropyl-2,2,2-trifluorethyl]-7-[3-(hydroxymethyl)-2-oxopyrrolidin-1-yl]-4-oxo-1-(2,4,6-trifluorphenyl)-1,4-dihydro-1,8-naphthyridin-3-carbonsäureamid (Diastereomerengemisch)

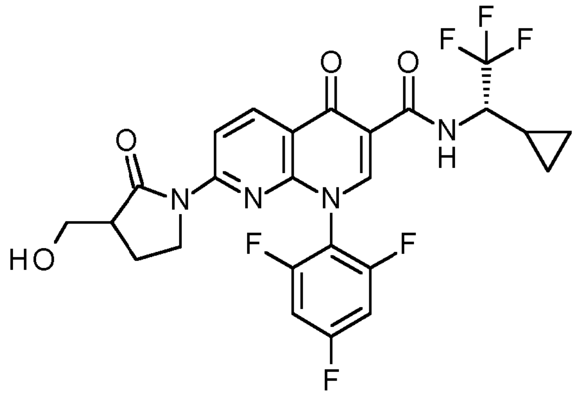

Gemäß AAV2 wurden 57.3 mg (120 µmol) der Verbindung aus Beispiel 126A mit 30.4 mg (133 µmol) der Verbindung aus Beispiel 148A in Gegenwart von 24.9 mg (181 µmol) Kaliumcarbonat, 2.7 mg (12 µmol) Palladium(II)acetat und 14 mg (24 µmol) Xantphos in 3 ml 1,4-Dioxan umgesetzt. Anschließend wurde mit 2 ml Wasser, 3 ml Acetonitril verdünnt und mittels präp. HPLC (Säule: Chromatorex C18, 10 µm, 125x30 mm, LM: Acetonitril / 0.05% Ameisensäure-Gradient; 0 bis 3 min. 15% Acetonitril bis 15 min. 90% Acetonitril und weitere 3 min. 90% Acetonitril) gereinigt. Die Produktfraktionen wurden eingeengt und der Rückstand in 3 ml THF aufgenommen. Die Lösung wurde unter Eisbadkühlung mit 34.9 mg (120 µmol) Tris(dimethylamino)sulfur(trimethylsilyl)difluorid versetzt und 30 min bei 0-5°C und 1h bei Raumtemperatur gerührt. Das Reaktionsgemisch wurde mit 10ml Wasser versetzt und dreimal mit 15ml Essigsäureethylester extrahiert. Die vereinigten organischen Phasen wurden mit gesättigter wässriger Natriumchlorid-Lösung gewaschen, über Magnesiumsulfat getrocknet, filtriert und eingeengt. Der Rückstand wurde mittels präp. HPLC aufgereinigt (Säule: Chromatorex C18, 10 µm, 125x30 mm, LM: Acetonitril / 0.05% Ameisensäure-Gradient; 0 bis 3 min. 10% Acetonitril bis 35 min. 90% Acetonitril und weitere 3 min. 90% Acetonitril). Die Produktfraktionen wurden vereinigt, eingeengt und lyophilisiert. Abschließend wurde das Rohprodukt mittels präparativer Dünnschichtchromatographie (Cyclohexan: Essigsäureethylester, 1:1, v/v) gereinigt. Die Produktbande wurde abgetragen und das Produkt in Dichlormethan/Methanol 9:1 (v/v) abgelöst, filtriert und eingeengt. Der Rückstand wurde in Dichlormethan aufgenommen, über einen Feinfilter filtriert, eingeengt und lyophilisert. Es wurden 5 mg (7% d. Th., 100% Reinheit) der Titelverbindung erhalten.

$^1$H-NMR (400 MHz, DMSO-d6) δ [ppm] = 10.26 (d, 1H), 9.06 (s, 1H), 8.72 (d, 1H), 8.56 (d, 1H), 7.64-7.56 (m, 2H), 4.86 (br. s, 1H), 4.46-4.35 (m, 1H), 3.76-3.70 (m, 1H), 3.63-33.54 (m, 2H), 3.52-3.44 (m, 1H), 2.85-2.78 (m, 1H), 2.15-2.06 (m, 1H), 2.02-1.93 (m, 1H), 1.28-1.19 (m, 2H), 0.70-0.52 (m, 3H), 0.38-0.31 (m, 1H).

LC-MS (Methode 3): $R_t$ = 1.93 min; MS (ESIpos): m/z = 555 $[M+H]^+$.

## Beispiel 469

*N-tert.*-Butyl-7-[(4*S*)-4-hydroxy-2-oxopyrrolidin-1-yl]-4-oxo-1-(2,4,6-trifluorphenyl)-1,4-dihydro-1,8-naphthyridin-3 -carbonsäureamid

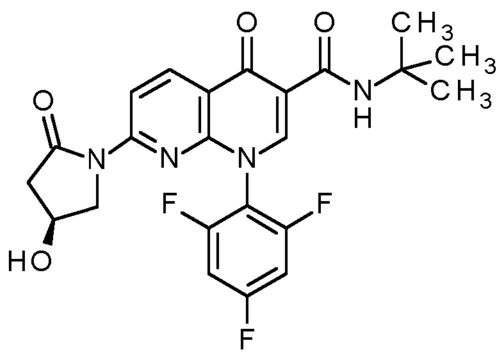

Gemäß AAV1 wurden 50.0 mg (119 µmol) der Verbindung aus Beispiel 117A mit 10.5 mg (143 µmol) *tert.*-Butylamin in Gegenwart von 54.4 mg (143 µmol) HATU und 52.0 µl (298 µmol) *N,N*-Diisopropylethylamin in 2.4 ml Dimethylformamid zur Reaktion gebracht. Der Ansatz wurde mit 0.3 ml 1M wässriger Salzsäure und 1 ml Acetonitril verdünnt und mittels präp. HPLC (Säule: Chromatorex C18, 10 μm, 125x30 mm, LM: Acetonitril / 0.05% Ameisensäure-Gradient; 0 bis 5 min. 10% Acetonitril bis 14 min. 90% Acetonitril und weitere 4 min. 90% Acetonitril) gereinigt. Die Produktfraktionen wurde vereinigt, eingeengt und lyophilisiert. Es wurden 37.1 mg (65% d. Th., 99% Reinheit) der Titelverbindung erhalten.
$^1$H-NMR (400 MHz, DMSO-$d_6$): δ [ppm] = 9.70 (s, 1H), 8.92 (s, 1H), 8.69 (d, 1H), 8.51 (d, 1H), 7.66-7.56 (m, 2H), 5.32 (d, 1H), 4.32-4.25 (m, 1H), 3.69 (dd, 1H), 3.47 (d, 1H), 2.93 (dd, 1H), 2.37 (d, 1H), 1.41 (s, 9H).
LC-MS (Methode 3): $R_t$ = 1.74 min; 475 [M+H]$^+$.

**Beispiel 470**

*N*-[(1*R*)-1-Cyclopropylethyl]-7-[(3*R*,4*R*)-3,4-dihydroxypyrrolidin-1-yl]-4-oxo-1-(2,4,6-trifluorphenyl)-1,4-dihydro-1,8-naphthyridin-3-carbonsäureamid

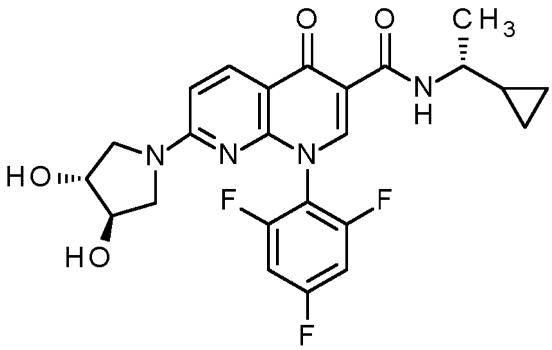

Gemäß AAV1 wurden 50.0 mg (119 μmol) der Verbindung aus Beispiel 121A mit 12.1 mg (142 μmol) (1*R*)-1-Cyclopropylethanamin in Gegenwart von 54.1 mg (142 μmol) HATU und 52.0 μl (297 μmol) *N,N*-Diisopropylethylamin in 2.4 ml Dimethylformamid zur Reaktion gebracht. Der Ansatz wurde mit 0.3 ml 1M wässriger Salzsäure und 1 ml Acetonitril verdünnt und mittels präp. HPLC (Säule: Chromatorex C18, 10 μm, 125x30 mm, LM: Acetonitril / 0.05% Ameisensäure-Gradient; 0 bis 2.5 min. 10% Acetonitril bis 16 min. 90% Acetonitril und weitere 2 min. 90% Acetonitril) gereinigt. Die Produktfraktionen wurde vereinigt, eingeengt und lyophilisiert. Es wurden 38.8 mg (67% d. Th., 100% Reinheit) der Titelverbindung erhalten.
$^1$H-NMR (400 MHz, DMSO-$d_6$): δ [ppm] = 9.95 (d, 1H), 8.67 (s, 1H), 8.26 (d, 1H), 7.60-7.51 (m, 2H), 6.75 (d, 1H), 5.23 (d, 1H), 5.13 (d, 1H), 4.05 (br. s, 1H), 3.92 (br. s, 1H), 3.65-3.47 (m, 2H), 3.25 (dd, 1H), 3.07 (d, 1H), 1.22 (d, 3H), 1.03-0.93 (m, 1H), 0.51-0.38 (m, 2H), 0.33-0.20 (m, 2H).
LC-MS (Methode 3): $R_t$ = 1.50 min; 489 [M+H]$^+$.

**Beispiel 471**

*N*-[1-Cyclobutylethyl]-7-[(4*S*)-4-hydroxy-2-oxopyrrolidin-1-yl]-4-oxo-1-(2,4,6-trifluorphenyl)-1,4-dihdro-18-naphthyridin-3-carbonsäureamid (Diastereomerengemisch)

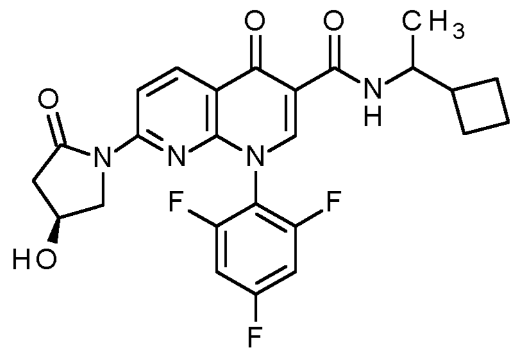

Gemäß AAV1 wurden 50.0 mg (119 µmol) der Verbindung aus Beispiel 117A mit 19.4 mg (143 µmol) 1-Cyclobutylethanamin Hydrochlorid (Racemat) in Gegenwart von 54.4 mg (143 µmol) HATU und 73.0 µl (417 µmol) *N*,*N*-Diisopropylethylamin in 2.4 ml Dimethylformamid zur Reaktion gebracht. Der Ansatz wurde mit 0.3 ml 1M wässriger Salzsäure und 1 ml Acetonitril verdünnt und mittels präp. HPLC (Säule: Chromatorex C18, 10 µm, 125x30 mm, LM: Acetonitril / 0.05% Ameisensäure-Gradient; 0 bis 5 min. 10% Acetonitril bis 14 min. 90% Acetonitril und weitere 4 min. 90% Acetonitril) gereinigt. Die Produktfraktionen wurde vereinigt, eingeengt und lyophilisiert. Es wurden 30.7 mg (51% d. Th., 99% Reinheit) der Titelverbindung erhalten.

$^1$H-NMR (400 MHz, DMSO-$d_6$): δ [ppm] = 9.61 (d, 1H), 8.96 (s, 1H), 8.70 (d, 1H), 8.51 (d, 1H), 7.65-7.56 (m, 2H), 5.36-5.30 (m, 1H), 4.29 (br. s, 1H), 4.07-3.95 (m, 1H), 3.69 (dd, 1H), 3.48 (d, 1H), 2.94 (dd, 1H), 2.47-2.34 (m, 2H), 2.03-1.69 (m, 6H), 1.07 (d, 3H).

LC-MS (Methode 3): $R_t$ = 1.86 min; 501 [M+H]$^+$.

## Beispiel 472

7-[(3*R*,4*R*)-3,4-Dihydroxypyrrolidin-1-yl]-*N*-[(2*S*)-3-methylbutan-2-yl]-4-oxo-1-(2,4,6-trifluorphenyl)-1,4-dihydro-1,8-naphthyridin-3-carbonsäureamid

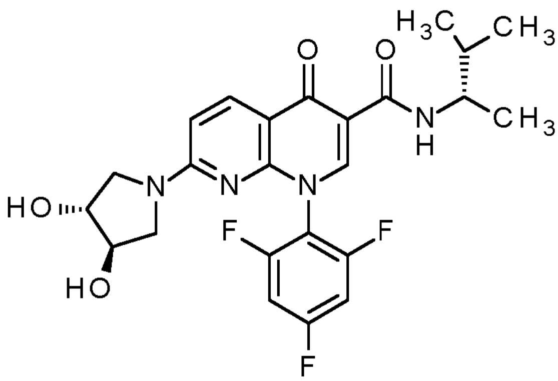

Gemäß AAV1 wurden 50.0 mg (119 µmol) der Verbindung aus Beispiel 121A mit 12.4 mg (142 µmol) (2*S*)-3-Methylbutan-2-amin in Gegenwart von 54.1 mg (142 µmol) HATU und 52.0 µl (297 µmol) *N,N*-Diisopropylethylamin in 2.4 ml Dimethylformamid zur Reaktion gebracht. Der Ansatz wurde mit 0.3 ml 1M wässriger Salzsäure und 1 ml Acetonitril verdünnt und mittels präp. HPLC (Säule: Chromatorex C18, 10 µm, 125x30 mm, LM: Acetonitril / 0.05% Ameisensäure-Gradient; 0 bis 5 min. 10% Acetonitril bis 14 min. 90% Acetonitril und weitere 4 min. 90% Acetonitril) gereinigt. Es wurden 36.0 mg (61% d. Th., 99% Reinheit) der Titelverbindung erhalten.

$^1$H-NMR (400 MHz, DMSO-$d_6$): δ [ppm] = 9.96 (d, 1H), 8.67 (s, 1H), 8.27 (d, 1H), 7.61-7.51 (m, 2H), 6.75 (d, 1H), 5.22 (d, 1H), 5.14 (d, 1H), 4.05 (br. s, 1H), 3.96-3.86 (m, 2H), 3.61 (dd, 1H), 3.25 (dd, 1H), 3.07 (d, 1H), 1.82-1.72 (m, 1H), 1.11 (d, 3H), 0.97-0.88 (m, 6H).

LC-MS (Methode 3): $R_t$ = 1.57 min; 491 [M+H]$^+$.

**Beispiel 473**

7-[(4*S*)-4-Hydroxy-2-oxopyrrolidin-1-yl]-*N*-[(2*R*)-3-methylbutan-2-yl]-4-oxo-1-(2,4,6-trifluorphenyl)-1,4-dihydro-1, 8-naphthyridin-3 -carbonsäureamid

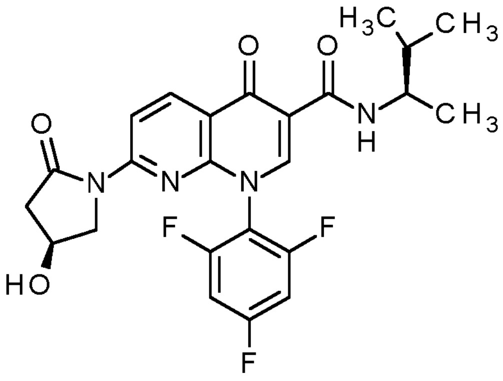

Gemäß AAV1 wurden 50.0 mg (119 µmol) der Verbindung aus Beispiel 117A mit 12.5 mg (143 µmol) (2*R*)-3-Methylbutan-2-amin in Gegenwart von 54.4 mg (143 µmol) HATU und 52.0 µl (298 µmol) *N,N*-Diisopropylethylamin in 2.4 ml Dimethylformamid zur Reaktion gebracht. Der Ansatz wurde mit 0.3 ml 1M wässriger Salzsäure und 1 ml Acetonitril verdünnt und mittels präp. HPLC (Säule: Chromatorex C18, 10 µm, 125x30 mm, LM: Acetonitril / 0.05% Ameisensäure-Gradient; 0 bis 5 min. 10% Acetonitril bis 14 min. 90% Acetonitril und weitere 4 min. 90% Acetonitril) gereinigt. Es wurden 41.7 mg (71% d. Th., 99% Reinheit) der Titelverbindung erhalten.
$^1$H-NMR (400 MHz, DMSO-$d_6$): δ [ppm] = 9.71 (d, 1H), 8.95 (s, 1H), 8.71 (d, 1H), 8.52 (d, 1H), 7.66-7.56 (m, 2H), 5.33 (br. s, 1H), 4.29 (br. s, 1H), 3.99-3.89 (m, 1H), 3.69 (dd, 1H), 3.48 (d, 1H), 2.94 (dd, 1H), 2.37 (d, 1H), 1.85-1.74 (m, 1H), 1.13 (d, 3H), 0.98-0.89 (m, 6H).
LC-MS (Methode 3): $R_t$ = 1.78 min; 489 $[M+H]^+$.

**Beispiel 474**

7-[(3*R*,4*R*)-3,4-Dihydroxypyrrolidin-1-yl]-*N*-(2,4-dimethylpentan-3-yl)-4-oxo-1-(2,4,6-trifluorphenyl)-1,4-dihydro-1,8-naphthyridin-3-carbonsäureamid

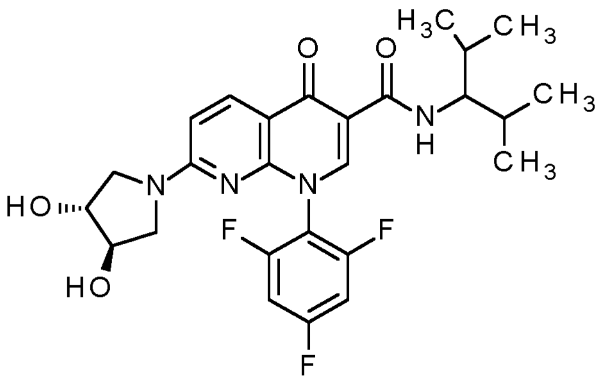

Gemäß AAV1 wurden 50.0 mg (119 µmol) der Verbindung aus Beispiel 121A mit 16.4 mg (142 µmol) 2,4-Dimethylpentan-3-amin in Gegenwart von 54.1 mg (142 µmol) HATU und 52.0 µl (297 µmol) *N,N*-Diisopropylethylamin in 2.4 ml Dimethylformamid zur Reaktion gebracht. Der Ansatz wurde mit 0.3 ml 1M wässriger Salzsäure und 1 ml Acetonitril verdünnt und mittels präp. HPLC (Säule: Chromatorex C18, 10 µm, 125x30 mm, Laufmittel Acetonitril / 0.05% Ameisensäure-Gradient; 0 bis 5 min. 10% Acetonitril bis 14 min. 90% Acetonitril und weitere 4 min. 90% Acetonitril)

gereinigt. Es wurden 37.8 mg (61% d. Th., 99% Reinheit) der Titelverbindung erhalten.
$^1$H-NMR (400 MHz, DMSO-$d_6$): δ [ppm] = 9.85 (d, 1H), 8.69 (s, 1H), 8.29 (d, 1H), 7.61-7.51 (m, 2H), 6.75 (d, 1H), 5.23 (d, 1H), 5.14 (d, 1H), 4.05 (br. s, 1H), 3.93 (br. s, 1H), 3.71-3.56 (m, 2H), 3.25 (dd, 1H), 3.07 (d, 1H), 1.91-1.80 (m, 2H), 0.91-0.85 (m, 12H).
LC-MS (Methode 1): $R_t$ = 0.94 min; 519 $[M+H]^+$.

**Beispiel 475**

*N*-(2,4-Dimethylpentan-3-yl)-7-[(4*S*)-4-hydroxy-2-oxopyrrolidin-1-yl]-4-oxo-1-(2,4,6-trifluorphenyl)-1,4-dihydro-1, 8-naphthyridin-3 -carbonsäureamid

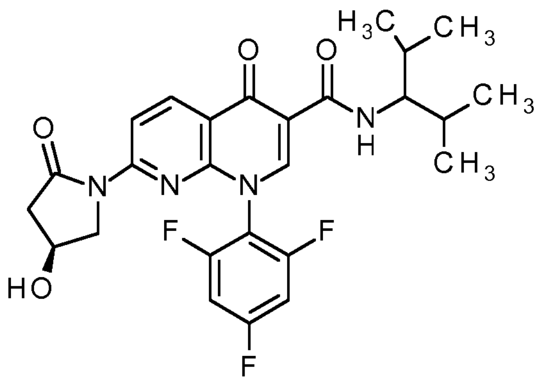

Gemäß AAV1 wurden 50.0 mg (119 μmol) der Verbindung aus Beispiel 117A mit 16.5 mg (143 μmol) 2,4-Dimethylpentan-3-amin in Gegenwart von 54.4 mg (143 μmol) HATU und 52.0 μl (298 μmol) *N,N*-Diisopropylethylamin in 2.4 ml Dimethylformamid zur Reaktion gebracht. Der Ansatz wurde mit 0.3 ml 1M wässriger Salzsäure und 1 ml Acetonitril verdünnt und mittels präp. HPLC (Säule: Chromatorex C18, 10 μm, 125x30 mm, Laufmittel: Acetonitril / 0.05% Ameisensäure-Gradient; 0 bis 5 min. 10% Acetonitril bis 14 min. 90% Acetonitril und weitere 4 min. 90% Acetonitril) gereinigt. Abschließend wurde mittels Normalphasenchromatographie (Cyclohexan-Essigsäureethylester Gradient) aufgereinigt. Es wurden 37.9 mg (61% d. Th., 99% Reinheit) der Titelverbindung erhalten.
$^1$H-NMR (400 MHz, DMSO-$d_6$): δ [ppm] = 9.60 (d, 1H), 8.96 (s, 1H), 8.72 (d, 1H), 8. 52 (d, 1H), 7.66-7.56 (m, 2H), 5.33 (s, 1H), 4.29 (br. s, 1H), 3.73-3.65 (m, 2H), 3.48 (d, 1H), 2.95 (dd, 1H), 2.37 (d, 1H), 1.93-1.81 (m, 2H), 0.94-0.84 (m, 12H).
LC-MS (Methode 3): $R_t$ = 1.99 min; 517 $[M+H]^+$.

**Beispiel 476**

*N*-[1-Cyclobutylethyl]-7-[(3*R*,4*R*)-3,4-dihydroxypyrrolidin-1-yl]-4-oxo-1-(2,4,6-trifluorphenyl)-1,4-dihydro-1, 8-naphthyridin-3 -carbonsäureamid (Diastereomerengemisch)

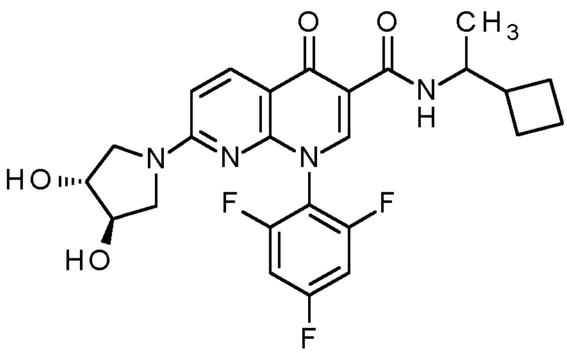

Gemäß AAV1 wurden 50.0 mg (119 µmol) der Verbindung aus Beispiel 121A mit 19.3 mg (142 µmol) 1-Cyclobutylethanamin Hydrochlorid (Racemat) in Gegenwart von 54.1 mg (142 µmol) HATU und 72.0 µl (415 µmol) *N,N*-Diisopropylethylamin in 2.4 ml Dimethylformamid zur Reaktion gebracht. Der Ansatz wurde mit 0.3 ml 1M wässriger Salzsäure und 1 ml Acetonitril verdünnt und mittels präp. HPLC (Säule: Chromatorex C18, 10 µm, 125x30 mm, LM: Acetonitril / 0.05% Ameisensäure-Gradient; 0 bis 5 min. 10% Acetonitril bis 14 min. 90% Acetonitril und weitere 4 min. 90% Acetonitril) gereinigt. Es wurden 40.9 mg (68% d. Th., 99% Reinheit) der Titelverbindung erhalten.
$^1$H-NMR (400 MHz, DMSO-$d_6$): δ [ppm] = 9.86 (d, 1H), 8.68 (s, 1H), 8.27 (d, 1H), 7.60-7.50 (m, 2H), 6.75 (d, 1H), 5.22 (br. s, 1H), 5.13 (br. s, 1H), 4.08-3.89 (m, 3H), 3.65-3.55 (m, 1H), 3.25 (m, 1H), 3.07 (d, 1H), 2.45-2.34 (m, 1H), 2.02-1.67 (m, 6H), 1.05 (d, 3H).
LC-MS (Methode 3): $R_t$ = 1.64 min; 503 [M+H]$^+$.

**Beispiel 477**

*N*-[(1*S*)-1-Cyclopropylethyl]-7-[(3*R*,4*R*)-3,4-dihydroxypyrrolidin-1-yl]-4-oxo-1-(2,4,6-trifluorphenyl)-1,4-dihydro-1,8-naphthyridin-3-carbonsäureamid

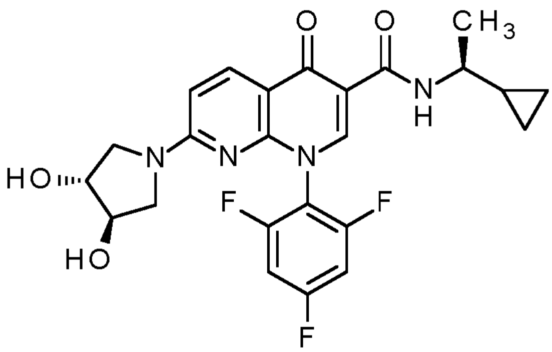

Gemäß AAV1 wurden 50.0 mg (119 µmol) der Verbindung aus Beispiel 121A mit 12.1 mg (142 µmol) (1*S*)-1-Cyclopropylethanamin in Gegenwart von 54.1 mg (142 µmol) HATU und 52.0 µl (297 µmol) *N,N*-Diisopropylethylamin in 2.4 ml Dimethylformamid zur Reaktion gebracht. Der Ansatz wurde mit 0.3 ml 1M wässriger Salzsäure und 1 ml Acetonitril verdünnt und mittels präp. HPLC (Säule: Chromatorex C18, 10 µm, 125x30 mm, LM: Acetonitril / 0.05% Ameisensäure-Gradient; 0 bis 2.5 min. 10% Acetonitril bis 15.5 min. 90% Acetonitril und weitere 2 min. 90% Acetonitril) gereinigt. Es wurden 38.9 mg (66% d. Th., 99% Reinheit) der Titelverbindung erhalten.
$^1$H-NMR (400 MHz, DMSO-$d_6$): δ [ppm] = 9.95 (d, 1H), 8.67 (s, 1H), 8.26 (d, 1H), 7.60-7.51 (m, 2H), 6.75 (d, 1H), 5.22 (br. s, 1H), 5.13 (br. s, 1H), 4.05 (br. s, 1H), 3.93 (br. s, 1H), 3.64-3.48 (m, 2H), 3.25 (m, 1H), 3.07 (d, 1H), 1.22 (d, 3H), 1.03-0.93 (m, 1H), 0.51-0.39 (m, 2H), 0.33-0.20 (m, 2H).
LC-MS (Methode 3): $R_t$ = 1.50 min; 489 [M+H]$^+$.

**Beispiel 478**

*N*-[(1*S*)-1-Cyclopropylethyl]-7-[(4*S*)-4-hydroxy-2-oxopyrrolidin-1-yl]-4-oxo-1-(2,4,6-trifluorphenyl)-1,4-dihydro-1,8-naphthyridin-3-carbonsäureamid

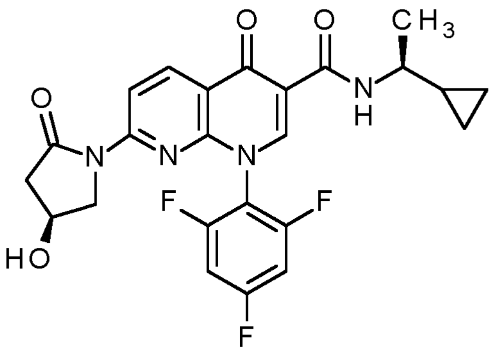

Gemäß AAV1 wurden 50.0 mg (119 µmol) der Verbindung aus Beispiel 117A mit 12.2 mg (143 µmol) (1*S*)-1-Cyclopropylethanamin in Gegenwart von 54.4 mg (143 µmol) HATU und 52.0 µl (298 µmol) *N,N*-Diisopropylethylamin in 2.4 ml Dimethylformamid zur Reaktion gebracht. Der Ansatz wurde mit 0.3 ml 1M wässriger Salzsäure und 1 ml Acetonitril verdünnt und mittels präp. HPLC (Säule: Chromatorex C18, 10 µm, 125x30 mm, LM: Acetonitril / 0.05% Ameisensäure-Gradient; 0 bis 2.5 min. 10% Acetonitril bis 15.5 min. 90% Acetonitril und weitere 2 min. 90% Acetonitril) gereinigt. Es wurden 26.5 mg (46% d. Th., 100% Reinheit) der Titelverbindung erhalten.

$^1$H-NMR (400 MHz, DMSO-$d_6$): δ [ppm] = 9.70 (d, 1H), 8.94 (s, 1H), 8.69 (d, 1H), 8.52 (d, 1H), 7.66-7.56 (m, 2H), 5.33 (d, 1H), 4.33-4.25 (m, 1H), 3.69 (dd, 1H), 3.60-3.44 (m, 2H), 2.94 (dd, 1H), 2.37 (d, 1H), 1.24 (d, 1H), 1.06-0.96 (m, 1H), 0.52-0.40 (m, 2H), 0.35-0.21 (m, 2H).

LC-MS (Methode 3): $R_t$ = 1.70 min; 487 $[M+H]^+$.

## Beispiel 479

*N-tert*.-Butyl-7-[(3*R*,4*R*)-3,4-dihydroxypyrrolidin-1-yl]-4-oxo-1-(2,4,6-trifluorphenyl)-1,4-dihydro-1,8-naphthyridin-3 -carbonsäureamid

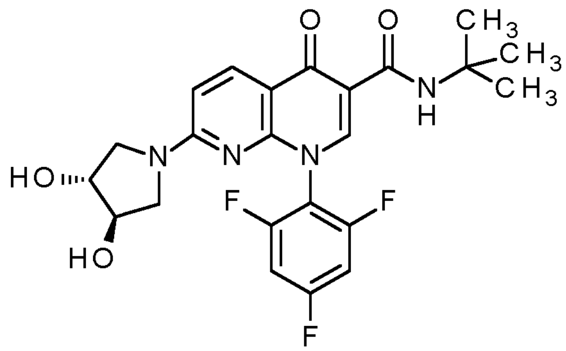

Gemäß AAV1 wurden 50.0 mg (119 µmol) der Verbindung aus Beispiel 121A mit 10.4 mg (142 µmol) 2-Methylpropan-2-amin in Gegenwart von 54.1 mg (142 µmol) HATU und 52.0 µl (297 µmol) *N,N*-Diisopropylethylamin in 2.4 ml Dimethylformamid zur Reaktion gebracht. Der Ansatz wurde mit 0.3 ml 1M wässriger Salzsäure und 1 ml Acetonitril verdünnt und mittels präp. HPLC (Säule: Chromatorex C18, 10 µm, 125x30 mm, LM: Acetonitril / 0.05% Ameisensäure-Gradient; 0 bis 2.5 min. 10% Acetonitril bis 15.5 min. 90% Acetonitril und weitere 2 min. 90% Acetonitril) gereinigt. Es wurden 43.1 mg (76% d. Th., 100% Reinheit) der Titelverbindung erhalten.

$^1$H-NMR (400 MHz, DMSO-$d_6$): δ [ppm] = 9.95 (s, 1H), 8.65 (s, 1H), 8.26 (d, 1H), 7.60-7.51 (m, 2H), 6.74 (d, 1H), 5.22 (d, 1H), 5.13 (d, 1H), 4.04 (br. s, 1H), 3.92 (br. s, 1H), 3.60 (dd, 1H), 3.24 (dd, 1H), 3.06 (d, 1H), 1.39 (s, 9H).

LC-MS (Methode 3): $R_t$ = 1.51 min; 477 $[M+H]^+$.

**Beispiel 480**

*N*-[(1*R*)-1-Cyclopropylethyl]-7-[(4*S*)-4-hydroxy-2-oxopyrrolidin-1-yl]-4-oxo-1-(2,4,6-trifluorphenyl)-1,4-dihydro-1,8-naphthyridin-3-carbonsäureamid

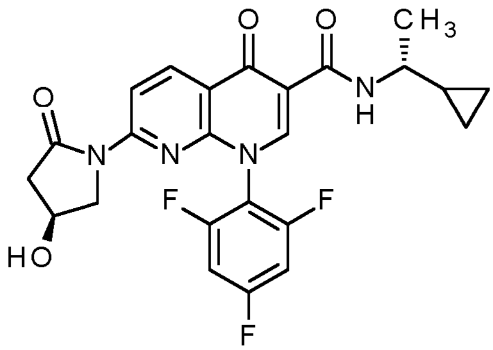

Gemäß AAV1 wurden 50.0 mg (119 µmol) der Verbindung aus Beispiel 117A mit 12.2 mg (143 µmol) (1*R*)-1-Cyclopropylethanamin in Gegenwart von 54.4 mg (143 µmol) HATU und 52.0 µl (298 µmol) *N,N*-Diisopropylethylamin in 2.4 ml Dimethylformamid zur Reaktion gebracht. Der Ansatz wurde mit 0.3 ml 1M wässriger Salzsäure und 1 ml Acetonitril verdünnt und mittels präp. HPLC (Säule: Chromatorex C18, 10 µm, 125x30 mm, LM: Acetonitril / 0.05% Ameisensäure-Gradient; 0 bis 2.5 min. 10% Acetonitril bis 15.5 min. 90% Acetonitril und weitere 2 min. 90% Acetonitril) gereinigt. Es wurden 25.7 mg (44% d. Th., 100% Reinheit) der Titelverbindung erhalten.
$^1$H-NMR (400 MHz, DMSO-$d_6$): δ [ppm] = 9.70 (d, 1H), 8.95 (s, 1H), 8.69 (d, 1H), 8.52 (d, 1H), 7.65-7.57 (m, 2H), 5.33 (d, 1H), 4.32-4.26 (m, 1H), 3.69 (dd, 1H), 3.59-3.44 (m, 2H), 2.94 (dd, 1H), 2.37 (d, 1H), 1.25 (d, 1H), 1.05-0.96 (m, 1H), 0.53-0.40 (m, 2H), 0.35-0.21 (m, 2H).
LC-MS (Methode 3): $R_t$ = 1.70 min; 487 $[M+H]^+$.

**Beispiel 481**

*N*-(2-Cyclopropylpropan-2-yl)-7-[(4*S*)-4-hydroxy-2-oxopyrrolidin-1-yl]-4-oxo-1-(2,4,6-trifluorphenyl)-1,4-dihydro-1,8-naphthyridin-3-carbonsäureamid

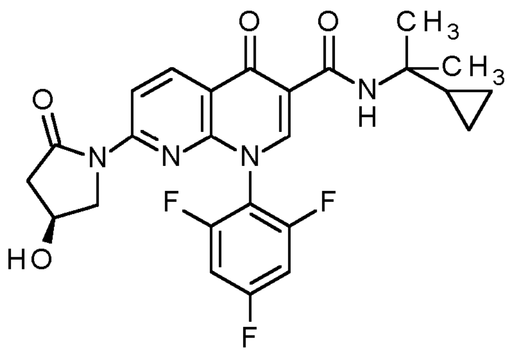

Gemäß AAV1 wurden 50.0 mg (119 µmol) der Verbindung aus Beispiel 117A mit 14.2 mg (143 µmol) 2-Cyclopropylpropan-2-amin in Gegenwart von 54.4 mg (143 µmol) HATU und 52.0 µl (298 µmol) *N,N*-Diisopropylethylamin in 2.4 ml Dimethylformamid zur Reaktion gebracht. Der Ansatz wurde mit 0.3 ml 1M wässriger Salzsäure und 1 ml Acetonitril verdünnt und mittels präp. HPLC (Säule: Chromatorex C18, 10 µm, 125x30 mm, Laufmittel: Acetonitril / 0.05% Ameisensäure-Gradient; 0 bis 5 min. 10% Acetonitril bis 14 min. 90% Acetonitril und weitere 4 min. 90% Acetonitril) gereinigt. Es wurden 41.4 mg (69% d. Th., 99% Reinheit) der Titelverbindung erhalten.
$^1$H-NMR (400 MHz, DMSO-$d_6$): δ [ppm] = 9.71 (s, 1H), 8.92 (s, 1H), 8.70 (d, 1H), 8.51 (d, 1H), 7.66-7.57 (m, 2H), 5.32

(d, 1H), 4.32-4.26 (m, 1H), 3.69 (dd, 1H), 3.48 (d, 1H), 2.94 (dd, 1H), 2.37 (d, 1H), 1.33 (s, 6H), 0.43 (s, 2H), 0.41 (s, 2H). LC-MS (Methode 3): $R_t$ = 1.87 min; 501 $[M+H]^+$.

**Beispiel 482**

7-[(4*S*)-4-Hydroxy-2-oxopyrrolidin-1-yl]-*N*-[(2*S*)-3-methylbutan-2-yl]-4-oxo-1-(2,4,6-trifluorphenyl)-1,4-dihydro-1, 8-naphthyridin-3 -carbonsäureamid

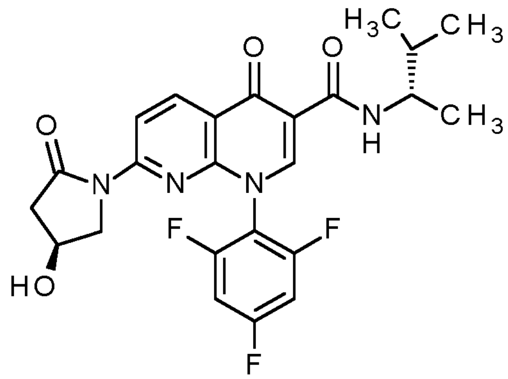

Gemäß AAV1 wurden 50.0 mg (119 µmol) der Verbindung aus Beispiel 117A mit 12.5 mg (143 µmol) (2*S*)-3-Methylbutan-2-amin in Gegenwart von 54.4 mg (143 µmol) HATU und 52.0 µl (298 µmol) *N,N*-Diisopropylethylamin in 2.4 ml Dimethylformamid zur Reaktion gebracht. Der Ansatz wurde mit 0.3 ml 1M wässriger Salzsäure und 1 ml Acetonitril verdünnt und mittels präp. HPLC (Säule: Chromatorex C18, 10 µm, 125x30 mm, LM: Acetonitril / 0.05% Ameisensäure-Gradient; 0 bis 5 min. 10% Acetonitril bis 14 min. 90% Acetonitril und weitere 4 min. 90% Acetonitril) gereinigt. Es wurden 37.4 mg (64% d. Th., 99% Reinheit) der Titelverbindung erhalten.

$^1$H-NMR (400 MHz, DMSO-$d_6$): δ [ppm] = 9.70 (d, 1H), 8.95 (s, 1H), 8.71 (d, 1H), 8.52 (d, 1H), 7.66-7.57 (m, 2H), 5.33 (d, 1H), 4.32-4.26 (m, 1H), 3.99-3.88 (m, 1H), 3.69 (dd, 1H), 3.48 (d, 1H), 2.94 (dd, 1H), 2.37 (d, 1H), 1.84-1.74 (m, 1H), 1.12 (d, 3H), 0.98-0.89 (m, 6H).

LC-MS (Methode 3): $R_t$ = 1.77 min; 489 $[M+H]^+$.

**Beispiel 483**

7-[(3*R*,4*R*)-3,4-Dihydroxypyrrolidin-1-yl]-*N*-[(2*R*)-3-methylbutan-2-yl]-4-oxo-1-(2,4,6-trifluorphenyl)-1,4-dihydro-1,8-naphthyridin-3-carbonsäureamid

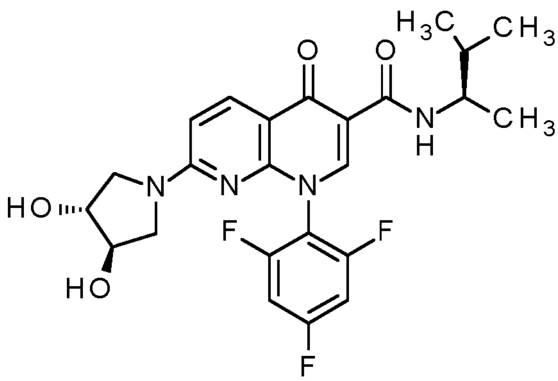

Gemäß AAV1 wurden 50.0 mg (119 µmol) der Verbindung aus Beispiel 121A mit 12.4 mg (142 µmol) (2*R*)-3-Methylbutan-2-amin in Gegenwart von 54.1 mg (142 µmol) HATU und 52.0 µl (297 µmol) *N,N*-Diisopropylethylamin in

2.4 ml Dimethylformamid zur Reaktion gebracht. Der Ansatz wurde mit 0.3 ml 1M wässriger Salzsäure und 1 ml Acetonitril verdünnt und mittels präp. HPLC (0.05% Ameisensäure, Wasser-Acetonitril Gradient) gereinigt. Es wurden 30.1 mg (51% d. Th., 99% Reinheit) der Titelverbindung erhalten.
$^1$H-NMR (400 MHz, DMSO-$d_6$): δ [ppm] = 9.96 (d, 1H), 8.68 (s, 1H), 8.27 (d, 1H), 7.60-7.50 (m, 2H), 6.75 (d, 1H), 5.23 (d, 1H), 5.13 (d, 1H), 4.05 (br. s, 1H), 3.97-3.86 (m, 2H), 3.61 (dd, 1H), 3.25 (dd, 1H), 3.07 (d, 1H), 1.82-1.72 (m, 1H), 1.11 (d, 3H), 0.96-0.86 (m, 6H).
LC-MS (Methode 3): $R_t$ = 1.56 min; 491 $[M+H]^+$.

**Beispiel 484**

7-[(4*S*)-4-Hydroxy-2-oxopyrrolidin-1-yl]-*N*-[2-methylpentan-3-yl]-4-oxo-1-(2,4,6-trifluorphenyl)-1,4-dihydro-1, 8-naphthyridin-3 -carbonsäureamid (Diastereomerengemisch)

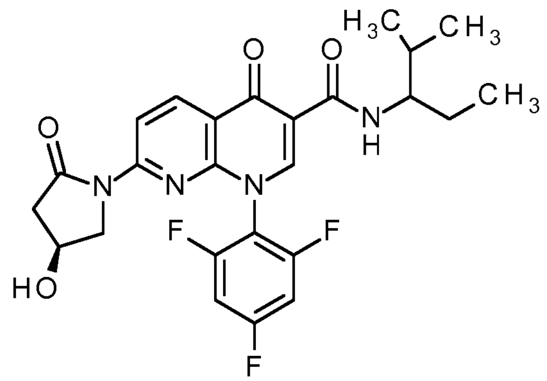

Gemäß AAV1 wurden 100 mg (238 µmol) der Verbindung aus Beispiel 117A mit 39.4 mg (286 µmol) 2-Methylpentan-3-amin Hydrochlorid (Racemat) in Gegenwart von 109 mg (286 µmol) HATU und 145 µl (835 µmol) *N,N*-Diisopropylethylamin in 4.6 ml Dimethylformamid zur Reaktion gebracht. Der Ansatz wurde mit 0.3 ml 1M wässriger Salzsäure und 1 ml Acetonitril verdünnt und mittels präp. HPLC (Säule: Chromatorex C18, 10 µm, 125x30 mm, LM: Acetonitril / 0.05% Ameisensäure-Gradient; 0 bis 5.5 min. 10% Acetonitril bis 34 min. 90% Acetonitril und weitere 7.5 min. 90% Acetonitril) gereinigt. Es wurden 83.4 mg (69% d. Th., 99% Reinheit) der Titelverbindung erhalten.
$^1$H-NMR (400 MHz, DMSO-$d_6$): δ [ppm] = 9.61 (d, 1H), 8.95 (s, 1H), 8.71 (d, 1H), 8.52 (d, 1H), 7.66-7.56 (m, 2H), 5.33 (d, 1H), 4.32-4.26 (m, 1H), 3.87-3.77 (m, 1H), 3.69 (dd, 1H), 3.48 (d, 1H), 2.95 (dd, 1H), 2.37 (d, 1H), 1.89-1.78 (m, 1H), 1.65-1.53 (m, 1H), 1.50-1.37 (m, 1H), 0.96-0.82 (m, 9H).
LC-MS (Methode 3): $R_t$ = 1.89 min; 503 $[M+H]^+$.

80 mg der Titelverbindung (Diastereomerengemisch) wurde durch chirale HPLC in die Diastereomere getrennt (präparative HPLC: Säule Daicel Chiralcel OX-H 5 µm 250x20 mm; Eluent: 25% Ethanol, 75% n-Heptan; Temperatur: 23°C; Fluss: 30 ml/min; UV-Detektion: 260 nm.)

Man erhielt (in der Reihenfolge der Elution von der Säule) 30.7 mg Diastereomer 1 (99% de) $R_t$ = 4.00 min und 31.7 mg (99%de) Diastereomer 2 $R_t$ = 4.99 min.

[Analytische HPLC: Säule Daicel OX-3 50x4.6 mm; Eluent: 20% Ethanol, 80% iso-Hexan; UV-Detektion: 220 nm].

Diastereomer 1 wurde zusätzlich mittels präparativer HPLC (Wasser-Acetonitril Gradient) gereinigt und 29.8 mg (25% d. Th., 99% Reinheit) der Titelverbindung aus Beispiel 485 erhalten.

Diastereomer 2 wurde zusätzlich mittels präparativer HPLC (Wasser-Acetonitril Gradient) gereinigt und 28.6 mg (24% d. Th., 99% Reinheit) der Titelverbindung aus Beispiel 486 erhalten.

**Beispiel 485**

7-[(4*S*)-4-Hydroxy-2-oxopyrrolidin-1-yl]-*N*-[2-methylpentan-3-yl]-4-oxo-1-(2,4,6-trifluorphenyl)-1,4-dihydro-1,8-naphthyridin-3-carbonsäureamid (Diastereomer 1)

$^1$H-NMR (400 MHz, DMSO-$d_6$): δ [ppm] = 9.60 (d, 1H), 8.95 (s, 1H), 8.71 (d, 1H), 8.52 (d, 1H), 7.66-7.56 (m, 2H), 5.33 (d, 1H), 4.32-4.26 (m, 1H), 3.86-3.78 (m, 1H), 3.69 (dd, 1H), 3.48 (d, 1H), 2.94 (dd, 1H), 2.37 (d, 1H), 1.89-1.78

(m, 1H), 1.65-1.53 (m, 1H), 1.50-1.37 (m, 1H), 0.96-0.83 (m, 9H).
LC-MS (Methode 3): $R_t$ = 1.91 min; 503 [M+H]$^+$.

**Beispiel 486**

7-[(4*S*)-4-Hydroxy-2-oxopyrrolidin-1-yl]-*N*-[2-methylpentan-3-yl]-4-oxo-1-(2,4,6-trifluorphenyl)-1,4-dihydro-1,8-naphthyridin-3-carbonsäureamid (Diastereomer 2)

$^1$H-NMR (400 MHz, DMSO-$d_6$): δ [ppm] = 9.60 (d, 1H), 8.95 (s, 1H), 8.71 (d, 1H), 8.52 (d, 1H), 7.66-7.57 (m, 2H), 5.33 (d, 1H), 4.32-4.26 (m, 1H), 3.87-3.78 (m, 1H), 3.69 (dd, 1H), 3.48 (d, 1H), 2.94 (dd, 1H), 2.37 (d, 1H), 1.88-1.78 (m, 1H), 1.65-1.53 (m, 1H), 1.50-1.37 (m, 1H), 0.95-0.84 (m, 9H).
LC-MS (Methode 3): $R_t$ = 1.91 min; 503 [M+H]$^+$.

**Beispiel** 487

7-[(3*R*,4*R*)-3,4-Dihydroxypyrrolidin-1-yl]-*N*-[2-methylpentan-3-yl]-4-oxo-1-(2,4,6-trifluorphenyl)-1,4-dihydro-1, 8-naphthyridin-3 -carbonsäureamid (Diastereomerengemisch)

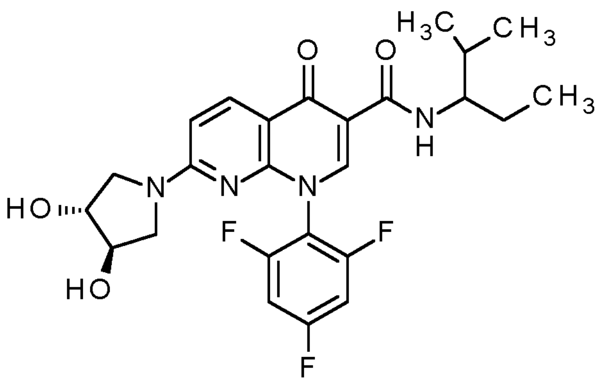

Gemäß AAV1 wurden 100 mg (237 µmol) der Verbindung aus Beispiel 121A mit 39.2 mg (285 µmol) 2-Methylpentan-3-amin Hydrochlorid (Racemat) in Gegenwart von 108 mg (285 µmol) HATU und 145 µl (835 µmol) *N,N*-Diisopropylethylamin in 4.6 ml Dimethylformamid zur Reaktion gebracht. Der Ansatz wurde mit 0.3 ml 1M wässriger Salzsäure und 1 ml Acetonitril verdünnt und mittels präp. HPLC (Säule: Chromatorex C18, 10 µm, 125x30 mm, LM: Acetonitril / 0.05% Ameisensäure-Gradient; 0 bis 5 min. 10% Acetonitril bis 14 min. 90% Acetonitril und weitere 4 min. 90% Acetonitril) gereinigt. Es wurden 80.7 mg (67% d. Th., 99% Reinheit) der Titelverbindung erhalten.
$^1$H-NMR (400 MHz, DMSO-$d_6$): δ [ppm] = 9.86 (d, 1H), 8.68 (s, 1H), 8.28 (d, 1H), 7.60-7.51 (m, 2H), 6.75 (d, 1H), 5.23 (d, 1H), 5.13 (d, 1H), 4.05 (br. s, 1H), 3.92 (br. s, 1H), 3.84.3.75 (m, 1H), 3.61 (dd, 1H), 3.25 (dd, 1H), 3.07 (d, 1H), 1.87-1.76 (m, 1H), 1.63-1.51 (m, 1H), 1.48-1.35 (m, 1H), 0.94-0.84 (m, 9H).
LC-MS (Methode 3): $R_t$ = 1.69 min; 505 [M+H]$^+$.

**Beispiel 488**

7-[(3*R*)-3-Hydroxy-2-oxopyrrolidin-1-yl]-4-oxo-*N*-(1,1,1-trifluor-2-methylpropan-2-yl)-1-(2,4,6-trifluorphenyl)-1,4-dihydro-1,8-naphthyridin-3-carbonsäureamid

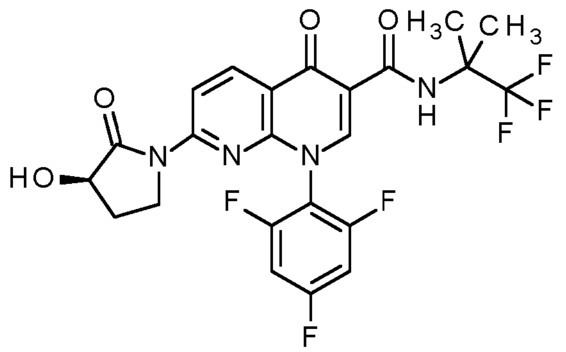

Gemäß AAV1 wurden 63.0 mg (150 µmol) der Verbindung aus Beispiel 149A mit 21.0 mg (165 µmol) 1,1,1-Trifluor-2-methylpropan-2-amin in Gegenwart von 68.6mg (180µmol) HATU und 65.0µl (376 µmol) *N,N*-Diisopropylethylamin in 2.1 ml Dimethylformamid zur Reaktion gebracht. Der Ansatz wurde mit 0.1 ml 1M wässriger Salzsäure und 1 ml Acetonitril verdünnt und mittels präp. HPLC (Säule: Chromatorex C18, 10 µm, 125x30 mm, LM: Acetonitril / 0.05% Ameisensäure-Gradient; 0 bis 5 min. 10% Acetonitril bis 14 min. 90% Acetonitril und weitere 4 min. 90% Acetonitril) gereinigt. Es wurden 64.8 mg (81% d. Th., 99% Reinheit) der Titelverbindung erhalten.
$^1$H-NMR (400 MHz, DMSO-$d_6$): δ [ppm] = 10.34 (s, 1H), 9.02 (s, 1H), 8.74 (d, 1H), 8.54 (d, 1H), 7.64-7.54 (m, 2H), 5.91 (d, 1H), 4.43-4.35 (m, 1H), 3.62-3.54 (m, 1H), 2.38-2.27 (m, 1H), 1.83-1.70 (m, 1H), 1.65 (s, 6H).
LC-MS (Methode 3): $R_t$ = 1.91 min; 529 $[M+H]^+$.

**Beispiel 489**

7-[4,4-Bis(hydroxymethyl)piperidin-1-yl]-N-[(1S)-1-cyclopropyl-2,2,2-trifluorethyl]-4-oxo-1-(2,4,6-trifluorphenyl)-1,4-dihydro-1,8-naphthyridin-3-carboxamid

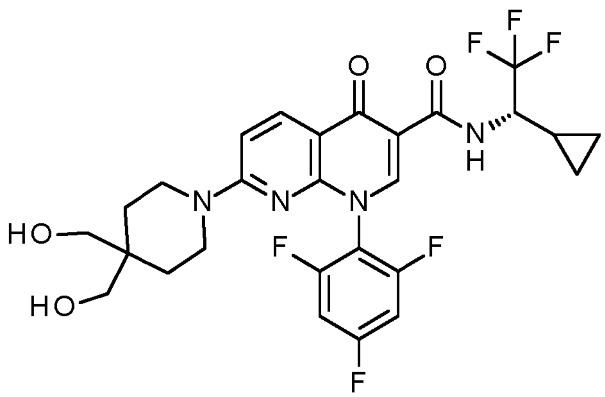

Die Verbindung aus Beispiel 126A (60.0 mg, 126 µmol) wurde in 1.3 ml DMF vorgelegt, mit 4,4-Piperidindiyldimethanol-Hydrochlorid (32 mg, 177 µmol) und *N,N*-Diisopropylethylamin (99 µl, 567 µmol) versetzt und über Nacht bei Raumtemperatur gerührt. Die Reaktionslösung wurde mit Acetonitril/Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden eingedampft, der Rückstand wurde in Dichlormethan gelöst und zweimal mit gesättigter, wässriger Natriumhydrogencarbonatlösung gewaschen. Die vereinigten wässrigen Phasen wurden einmal mit Dichlormethan reextrahiert. Die vereinigten organischen Phasen wurden über Natriumsulfat getrocknet, filtriert und einrotiert. Es wurden 67 mg der Zielverbindung (89% d. Th., Reinheit 98%) erhalten.
LC-MS (Methode 3): $R_t$ = 1.83 min; MS (ESIpos): m/z = 585 $[M+H]^+$
$^1$H-NMR (400 MHz, DMSO-$d_6$) δ [ppm]: -0.008 (0.91), 0.006 (0.40), 0.008 (0.59), 1.334 (0.64), 2.519 (0.77), 2.524 (0.69), 3.276 (1.91), 3.290 (1.96), 3.310 (16.00), 3.475 (0.57), 4.399 (0.74), 4.412 (1.55), 4.426 (0.61), 7.078 (0.69), 7.101 (0.69), 7.542 (0.48), 7.564 (0.88), 7.586 (0.48), 8.244 (0.95), 8.267 (0.87), 8.792 (1.45), 10.539 (0.54), 10.562 (0.51).
In Analogie zu Beispiel 489 wurden die in Tabelle 23 gezeigten Beispielverbindungen hergestellt, indem die jeweiligen Ausgangsverbindungen Bespiel 126A oder Beispiel 115A mit den entsprechenden Aminen (bzw. deren Salzen;

1.2 - 4 Äquivalente) unter den beschriebenen Reaktionsbedingungen (1.5 h bis 18 h bei Raumtemperatur) umgesetzt wurden.

Tabelle 23:

| Bsp. | IUPAC-Name Struktur Eingesetztes Amin Ausbeute | LC-MS Methode Retentionszeit Detektierte Masse NMR-Daten |
|---|---|---|
| **490** | N-[(1S)-1-Cyclopropyl-2,2,2-trifluorethyl]-7-(4-methylpiperazin-1-yl)-4-oxo-1-(2,4,6-trifluorphenyl)-1,4-dihydro-1,8-naphthyridin-3-carboxamid 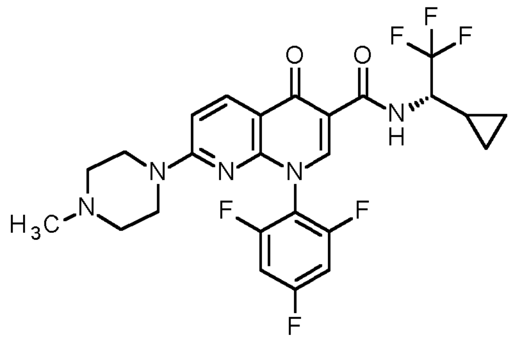 mit 1-Methylpiperazin (84% d. Th.) | LC-MS (Methode 3): $R_t$ = 1.34 min m/z = 540 $[M+H]^+$ $^1$H-NMR (400 MHz, DMSO-$d_6$) $\delta$ [ppm]: 0.008 (2.50), 0.320 (0.77), 0.330 (1.18), 0.343 (1.15), 0.355 (0.91), 0.366 (0.44), 0.511 (0.83), 0.523 (1.21), 0.535 (1.10), 0.547 (1.19), 0.555 (0.93), 0.566 (1.24), 0.577 (1.01), 0.587 (0.92), 0.597 (0.75), 0.611 (0.46), 0.626 (0.65), 0.636 (0.63), 0.647 (1.12), 0.657 (0.98), 0.663 (0.93), 0.670 (0.89), 0.678 (0.44), 1.177 (0.50), 1.185 (0.71), 1.197 (1.20), 1.206 (0.87), 1.217 (1.15), 1.229 (0.69), 1.238 (0.50), 2.073 (0.92), 2.159 (16.00), 2.276 (3.99), 2.288 (5.69), 2.299 (4.00), 2.323 (0.63), 2.328 (0.70), 2.366 (0.42), 2.524 (1.74), 2.670 (0.54), 3.494 (4.95), 4.350 (0.61), 4.371 (1.07), 4.392 (1.03), 4.412 (0.56), 5.754 (1.16), 7.126 (3.15), 7.149 (3.22), 7.543 (2.11), 7.565 (4.02), 7.587 (2.14), 8.284 (3.99), 8.306 (3.73), 8.811 (6.22), 10.498 (2.43), 10.521 (2.35). |
| **491** | N-[(1S)-1-Cyclopropyl-2,2,2-trifluorethyl]-7-{[(2S)-2,3-dihydroxypropyl](methyl)amino}-4-oxo-1-(2,4,6-trifluorphenyl)-1,4-dihydro-1,8-naphthyridin-3-carboxamid 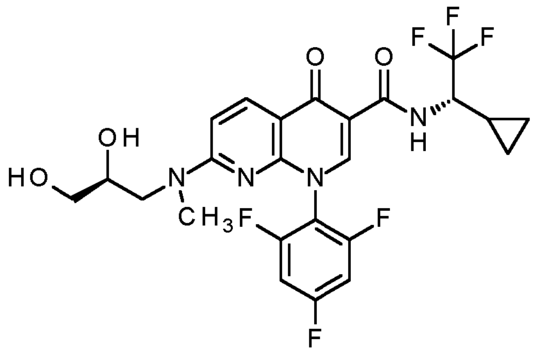 mit (2S)-3-(Methylamino)propan-1,2-diol (74% d. Th.) | LC-MS (Methode 3): $R_t$ = 1.76 min m/z = 545 $[M+H]^+$ $^1$H-NMR (400 MHz, DMSO-$d_6$) $\delta$ [ppm]: -0.149 (0.45), -0.008 (4.12), 0.008 (3.53), 0.146 (0.46), 0.322 (1.80), 0.333 (2.84), 0.346 (2.88), 0.357 (2.23), 0.369 (1.13), 0.509 (1.97), 0.520 (2.93), 0.533 (2.58), 0.547 (2.54), 0.555 (2.30), 0.567 (2.93), 0.577 (2.52), 0.588 (2.25), 0.598 (1.89), 0.612 (1.13), 0.626 (1.39), 0.636 (1.63), 0.646 (2.58), 0.657 (2.36), 0.662 (2.26), 0.671 (2.23), 0.682 (1.10), 0.691 (0.72), 1.163 (0.59), 1.175 (1.21), 1.183 (1.76), 1.195 (3.01), 1.204 (2.17), 1.216 (3.01), 1.228 (1.78), 1.236 (1.60), 2.328 (1.08), 2.366 (0.67), 2.524 (3.51), 2.670 (1.28), 2.710 (0.85), 2.832 (1.95), 3.136 (2.77), 3.371 (1.04), 3.488 (1.45), 3.690 (0.58), 4.352 (1.52), 4.372 (2.82), 4.394 (3.08), 4.414 (2.02), 4.608 (0.82), 4.744 (0.67), 4.937 (0.61), 6.935 (0.85), 7.541 (3.01), 8.273 (1.43), 8.793 (16.00), 10.551 (3.17), 10.575 (3.08). |

(fortgesetzt)

| Bsp. | IUPAC-Name Struktur Eingesetztes Amin Ausbeute | LC-MS Methode Retentionszeit Detektierte Masse NMR-Daten |
|---|---|---|
| **492** | N-[(1S)-1-Cyclopropyl-2,2,2-trifluorethyl]-7-(2-oxa-6-azaspiro[3.3]hept-6-yl)-4-oxo-1-(2,4,6-trifluorphenyl)-1,4-dihydro-1,8-naphthyridin-3-carboxamid 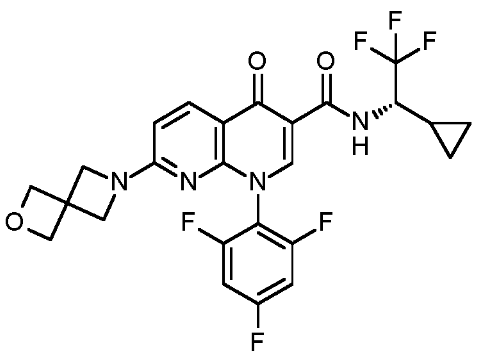 mit 2-Oxa-6-azaspiro[3.3]heptan-Ethandisäure (74% d. Th.) | LC-MS (Methode 3): $R_t$ = 2.06 min m/z = 539 [M+H]+ $^1$H-NMR (400 MHz, DMSO-$d_6$) δ [ppm]: -0.008 (1.90), 0.008 (1.59), 0.314 (0.60), 0.325 (0.95), 0.337 (0.94), 0.348 (0.72), 0.505 (0.67), 0.517 (0.97), 0.530 (0.86), 0.541 (0.95), 0.549 (0.73), 0.560 (0.98), 0.571 (0.79), 0.581 (0.74), 0.591 (0.60), 0.622 (0.51), 0.632 (0.53), 0.642 (0.86), 0.653 (0.77), 0.659 (0.76), 0.666 (0.73), 1.172 (0.40), 1.181 (0.55), 1.193 (1.00), 1.201 (0.71), 1.213 (0.98), 1.225 (0.52), 1.233 (0.47), 2.073 (7.30), 2.328 (0.46), 2.524 (1.43), 2.670 (0.46), 4.348 (0.62), 4.368 (0.92), 4.390 (0.90), 4.410 (0.48), 4.658 (16.00), 5.754 (1.18), 6.593 (3.51), 6.615 (3.56), 7.530 (1.77), 7.552 (3.33), 7.574 (1.80), 8.272 (3.89), 8.294 (3.66), 8.796 (5.72), 10.506 (1.93), 10.530 (1.84). |
| **493** | N-[(1S)-1-Cyclopropyl-2,2,2-trifluorethyl]-7-{[(2S)-2-hydroxypropyl](methyl)amino}-4-oxo-1-(2,4,6-trifluorphenyl)-1,4-dihydro-1,8-naphthyridin-3-carboxamid 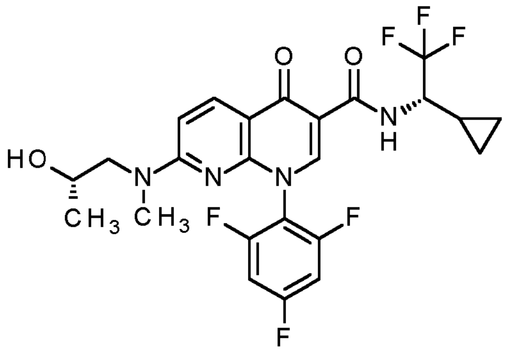 mit (2S)-1-(Methylamino)propan-2-ol (81% d. Th.) | LC-MS (Methode 3): $R_t$ = 2.01 min m/z = 529 [M+H]+ $^1$H-NMR (400 MHz, DMSO-$d_6$) δ [ppm]: -0.149 (0.59), -0.008 (8.10), 0.008 (5.14), 0.146 (0.65), 0.322 (2.70), 0.333 (3.90), 0.345 (3.93), 0.357 (2.97), 0.369 (1.47), 0.508 (2.94), 0.520 (4.26), 0.532 (3.73), 0.546 (3.67), 0.554 (3.38), 0.566 (4.17), 0.576 (3.61), 0.587 (3.29), 0.597 (2.73), 0.611 (1.85), 0.625 (2.23), 0.635 (2.58), 0.646 (3.76), 0.656 (3.58), 0.661 (3.46), 0.670 (3.38), 0.678 (1.94), 0.691 (1.73), 0.737 (4.79), 1.076 (1.32), 1.163 (1.20), 1.175 (2.03), 1.183 (2.70), 1.195 (4.37), 1.204 (3.14), 1.216 (4.23), 1.228 (2.64), 1.236 (2.44), 2.328 (1.41), 2.366 (1.14), 2.524 (8.46), 2.670 (1.73), 2.710 (1.32), 2.834 (2.14), 3.142 (5.58), 3.420 (2.79), 3.602 (1.32), 3.865 (0.47), 4.354 (2.32), 4.375 (3.64), 4.396 (3.29), 4.416 (1.88), 4.631 (1.79), 4.825 (0.50), 5.754 (0.53), 6.927 (1.61), 7.546 (5.55), 7.568 (9.95), 7.590 (5.40), 8.281 (2.08), 8.804 (16.00), 10.546 (4.40), 10.569 (4.20). |
| **494** | N-[(1S)-1-Cyclopropyl-2,2,2-trifluorethyl]-7-{[(2R)-2-hydroxypropyl](methyl)amino}-4-oxo-1-(2,4,6-trifluorphenyl)-1,4-dihydro-1,8-naphthyridin-3-carboxamid | LC-MS (Methode 3): $R_t$ = 2.01 min m/z = 529 [M+H]+ $^1$H-NMR (400 MHz, DMSO-$d_6$) δ [ppm]: -0.149 (0.60), -0.008 (5.76), 0.008 (5.76), 0.146 (0.70), 0.323 (2.44), 0.334 (3.89), 0.346 (3.91), 0.358 (3.04), 0.369 (1.55), 0.510 (2.67), 0.522 (3.96), 0.534 (3.49), 0.547 (3.66), 0.555 (3.17), 0.567 (3.99), 0.578 (3.41), 0.588 (3.17), 0.599 (2.59), |

(fortgesetzt)

| Bsp. | IUPAC-Name Struktur Eingesetztes Amin Ausbeute | LC-MS Methode Retentionszeit Detektierte Masse NMR-Daten |
|---|---|---|
| | 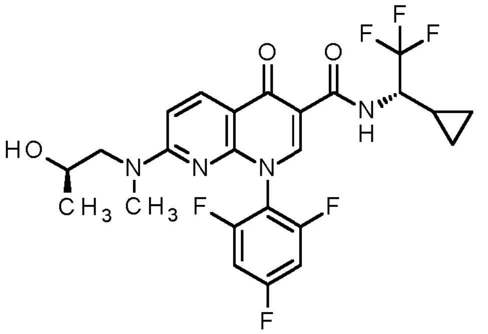<br>mit (2R)-1-(Methylamino)propan-2-ol (75% d. Th.) | 0.612 (1.67), 0.626 (2.02), 0.637 (2.27), 0.647 (3.64), 0.658 (3.29), 0.663 (3.24), 0.671 (3.26), 0.683 (1.82), 0.693 (1.60), 0.742 (4.64), 1.079 (1.25), 1.163 (0.90), 1.175 (1.72), 1.183 (2.52), 1.195 (4.06), 1.204 (2.99), 1.216 (4.04), 1.228 (2.49), 1.236 (2.12), 1.249 (0.85), 2.073 (0.47), 2.328 (1.42), 2.366 (1.05), 2.523 (5.73), 2.670 (1.69), 2.710 (1.17), 2.835 (1.99), 3.140 (5.51), 3.420 (2.77), 3.452 (1.87), 3.606 (1.25), 3.877 (0.45), 4.330 (0.50), 4.350 (2.02), 4.372 (3.59), 4.392 (3.46), 4.413 (1.87), 4.625 (1.72), 4.826 (0.47), 6.925 (1.57), 7.546 (5.38), 7.568 (10.14), 7.590 (5.53), 8.281 (1.94), 8.804 (16.00), 10.546 (4.54), 10.569 (4.46). |
| **495** | 7-{[(2R)-2-Hydroxypropyl](methyl)amino}-4-oxo-N-[(2S)-1,1,1-trifluorbutan-2-yl]-1-(2,4,6-trifluorphenyl)-1,4-dihydro-1,8-naphthyridin-3-carboxamid<br>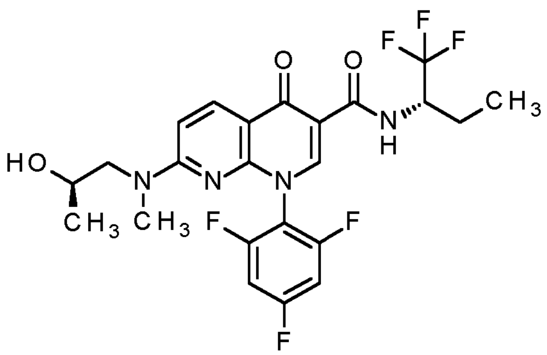<br>mit (2R)-1-(Methylamino)propan-2-ol (84% d. Th.) | LC-MS (Methode 1):<br>$R_t$ = 1.06 min<br>m/z = 517 $[M+H]^+$<br>$^1$H-NMR (400 MHz, DMSO-$d_6$) $\delta$ [ppm]: 0.008 (1.41), 0.744 (2.98), 0.953 (7.32), 0.971 (16.00), 0.989 (8.01), 1.072 (0.79), 1.233 (0.48), 1.603 (1.07), 1.621 (1.44), 1.628 (1.30), 1.638 (1.76), 1.646 (1.56), 1.656 (1.51), 1.663 (1.70), 1.682 (1.28), 1.832 (0.43), 1.851 (1.32), 1.861 (1.53), 1.869 (1.55), 1.879 (1.73), 1.886 (1.56), 1.896 (1.35), 1.904 (1.16), 1.914 (0.98), 2.672 (0.42), 2.837 (1.27), 3.140 (3.52), 3.423 (1.78), 3.455 (1.21), 3.606 (0.79), 4.626 (1.12), 4.732 (1.60), 4.752 (1.51), 6.925 (1.01), 7.549 (3.71), 7.571 (7.04), 7.593 (3.75), 8.277 (1.34), 8.814 (11.16), 10.413 (2.70), 10.437 (2.70). |
| **496** | 7-(4-Methylpiperazin-1-yl)-4-oxo-N-[(2S)-1,1,1-trifluorbutan-2-yl]-1-(2,4,6-trifluorphenyl)-1,4-dihydro-1,8-naphthyridin-3 -carboxamid<br>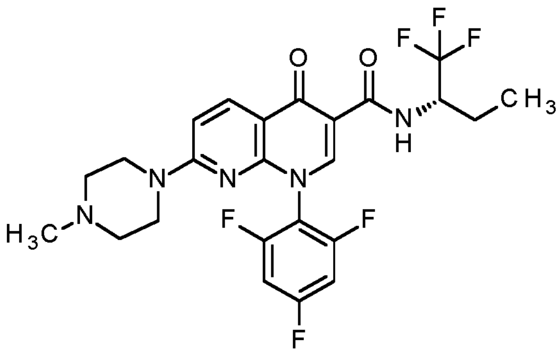<br>mit 1-Methylpiperazin (83% d. Th.) | LC-MS (Methode 3):<br>$R_t$ = 1.29 min<br>m/z = 528 $[M+H]^+$<br>$^1$H-NMR (400 MHz, DMSO-$d_6$) $\delta$ [ppm]: -0.008 (2.46), 0.008 (1.22), 0.949 (3.48), 0.968 (7.30), 0.986 (3.51), 1.602 (0.53), 1.620 (0.71), 1.627 (0.64), 1.637 (0.86), 1.646 (0.74), 1.655 (0.73), 1.662 (0.79), 1.681 (0.59), 1.850 (0.66), 1.859 (0.74), 1.868 (0.76), 1.878 (0.82), 1.884 (0.72), 1.894 (0.63), 1.903 (0.55), 1.913 (0.44), 2.074 (2.23), 2.159 (16.00), 2.275 (4.12), 2.287 (5.55), 2.299 (3.79), 2.324 (0.52), 2.329 (0.50), 2.520 (1.93), 2.524 (1.72), 2.671 (0.41), 3.494 (4.82), 4.732 (0.68), 4.744 (0.64), 7.125 (3.28), 7.148 (3.25), 7.546 (2.19), 7.568 (3.87), 7.590 (2.11), 8.281 (4.40), 8.303 (4.08), 8.820 (6.90), 10.361 (2.35), 10.385 (2.20). |

(fortgesetzt)

| Bsp. | IUPAC-Name Struktur Eingesetztes Amin Ausbeute | LC-MS Methode Retentionszeit Detektierte Masse NMR-Daten |
|---|---|---|
| **497** | 7-{[(2S)-2-Hydroxypropyl](methyl)amino}-4-oxo-N-[(2S)-1,1,1-trifluorbutan-2-yl]-1-(2,4,6-trifluorphenyl)-1,4-dihydro-1,8-naphthyridin-3-carboxamid 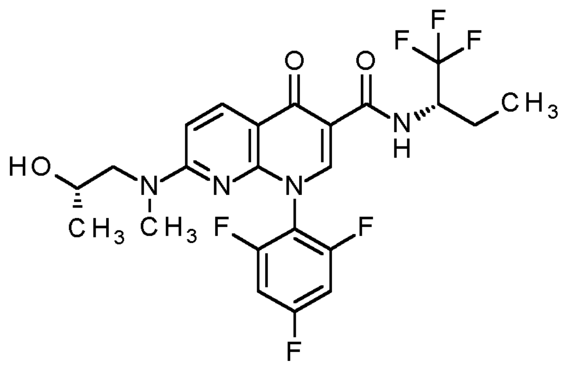 mit (2S)-1-(Methylamino)propan-2-ol (65% d. Th.) | LC-MS (Methode 1): $R_t$ = 1.06 min m/z = 517 $[M+H]^+$ $^1$H-NMR (400 MHz, DMSO-$d_6$) $\delta$ [ppm]: 0.008 (1.19), 0.741 (2.95), 0.951 (7.33), 0.969 (16.00), 0.988 (7.97), 1.075 (0.77), 1.601 (1.08), 1.619 (1.44), 1.626 (1.29), 1.636 (1.75), 1.645 (1.57), 1.655 (1.49), 1.662 (1.69), 1.680 (1.28), 1.832 (0.43), 1.841 (0.55), 1.850 (1.31), 1.860 (1.52), 1.868 (1.53), 1.878 (1.73), 1.885 (1.53), 1.895 (1.33), 1.903 (1.15), 1.913 (0.96), 2.524 (1.06), 2.835 (1.25), 3.140 (3.53), 3.422 (1.68), 3.454 (1.12), 3.610 (0.78), 4.633 (1.10), 4.677 (0.40), 4.708 (0.94), 4.723 (1.49), 4.732 (1.56), 4.752 (1.47), 4.767 (0.90), 6.927 (1.00), 7.550 (3.12), 7.571 (5.75), 7.593 (3.15), 8.279 (1.27), 8.815 (9.96), 10.413 (2.71), 10.437 (2.67). |
| **498** | 7-(2-Oxa-6-azaspiro[3.3]hept-6-yl)-4-oxo-N-[(2S)-1,1,1-trifluorbutan-2-yl]-1-(2,4,6-trifluorphenyl)-1,4-dihydro-1,8-naphthyridin-3-carboxamid 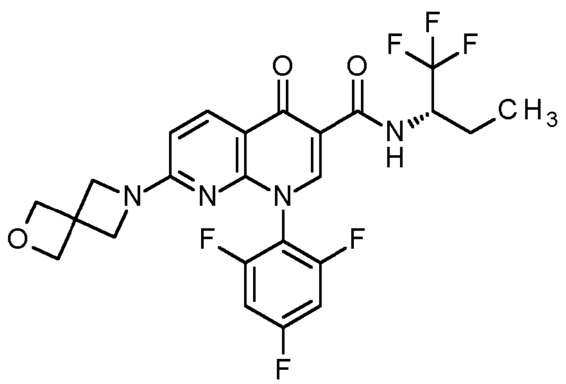 mit 2-Oxa-6-azaspiro[3.3]heptan-Ethandisäure (63% d. Th.) | LC-MS (Methode 1): $R_t$ = 1.09 min m/z = 527 $[M+H]^+$ $^1$H-NMR (400 MHz, DMSO-$d_6$) $\delta$ [ppm]: -0.062 (5.60), -0.008 (0.86), 0.945 (2.72), 0.963 (6.04), 0.981 (2.96), 1.597 (0.40), 1.615 (0.55), 1.622 (0.49), 1.632 (0.65), 1.640 (0.59), 1.650 (0.56), 1.658 (0.65), 1.676 (0.49), 1.846 (0.49), 1.855 (0.56), 1.864 (0.58), 1.874 (0.65), 1.880 (0.58), 1.891 (0.50), 1.899 (0.43), 2.074 (1.54), 4.660 (16.00), 4.727 (0.57), 4.747 (0.55), 6.592 (3.38), 6.614 (3.40), 7.533 (1.68), 7.555 (3.05), 7.577 (1.68), 8.269 (3.76), 8.291 (3.59), 8.806 (5.71), 10.371 (1.89), 10.395 (1.83). |
| **499** | 7-(tert-Butylamino)-4-oxo-N-[(2S)-1,1,1-trifluorbutan-2-yl]-1-(2,4,6-trifluorphenyl)-1,4-dihydro-1,8-naphthyridin-3-carboxamid 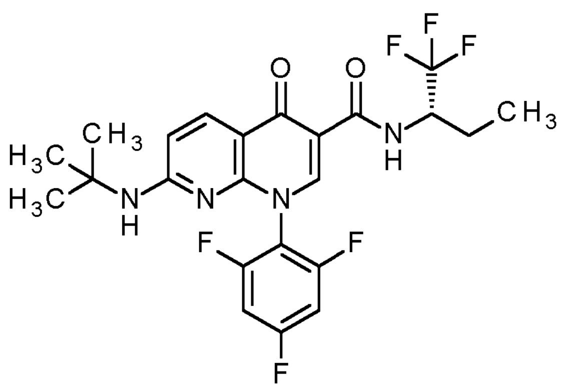 | LC-MS (Methode 3): $R_t$ = 2.30 min m/z = 501 $[M+H]^+$ $^1$H-NMR (400 MHz, DMSO-$d_6$) $\delta$ [ppm]: 0.947 (1.18), 0.966 (2.54), 0.984 (1.25), 1.101 (16.00), 5.756 (1.58), 6.696 (1.28), 6.718 (1.30), 7.564 (0.71), 7.586 (1.32), 7.608 (0.71), 7.706 (1.31), 8.099 (1.42), 8.121 (1.33), 8.793 (2.57), 10.463 (0.78), 10.487 (0.76). |

(fortgesetzt)

| Bsp. | IUPAC-Name Struktur Eingesetztes Amin Ausbeute | LC-MS Methode Retentionszeit Detektierte Masse NMR-Daten |
|---|---|---|
| | mit 2-Methylpropan-2-amin (69% d. Th.) | |
| **500** | 7-[4,4-Bis(hydroxymethyl)piperidin-1-yl]-4-oxo-N-[(2S)-1,1,1-trifluorbutan-2-yl]-1-(2,4,6-trifluorphenyl)-1,4-dihydro-1,8-naphthyridin-3-carboxamid 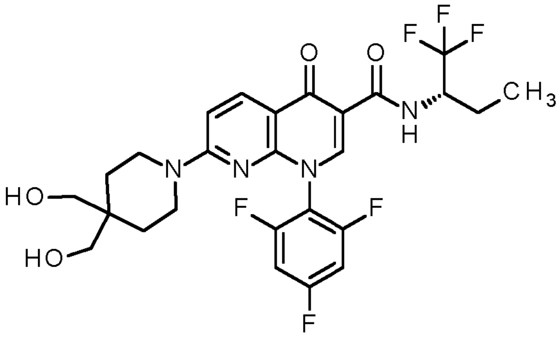 mit Piperidin-4,4-diyldimethanol-Hydrochlorid (71% d. Th.) | LC-MS (Methode 3): $R_t$ = 1.79 min m/z = 573 $[M+H]^+$ $^1$H-NMR (400 MHz, DMSO-$d_6$) $\delta$ [ppm]: -0.149 (0.46), 0.008 (4.07), 0.146 (0.50), 0.949 (6.73), 0.967 (14.80), 0.986 (7.36), 1.236 (0.98), 1.334 (5.59), 1.600 (1.00), 1.618 (1.26), 1.635 (1.55), 1.643 (1.41), 1.660 (1.55), 1.679 (1.26), 1.849 (1.11), 1.858 (1.35), 1.867 (1.39), 1.877 (1.61), 1.894 (1.22), 1.912 (0.94), 2.328 (1.37), 2.366 (0.87), 2.670 (1.39), 2.710 (0.91), 3.276 (15.39), 3.290 (16.00), 3.471 (5.03), 4.291 (1.07), 4.399 (5.14), 4.412 (11.69), 4.425 (4.99), 4.724 (1.35), 4.811 (0.44), 5.754 (1.89), 7.076 (5.44), 7.099 (5.70), 7.127 (0.54), 7.544 (4.24), 7.566 (7.79), 7.588 (4.11), 8.241 (7.03), 8.263 (6.70), 8.291 (0.57), 8.801 (12.73), 8.815 (1.11), 10.404 (4.64), 10.428 (4.22). |
| **501** | 7-(3-Fluorazetidin-1-yl)-4-oxo-N-[(2S)-1,1,1-trifluorbutan-2-yl]-1-(2,4,6-trifluorphenyl)-1,4-dihydro-1,8-naphthyridin-3-carboxamid 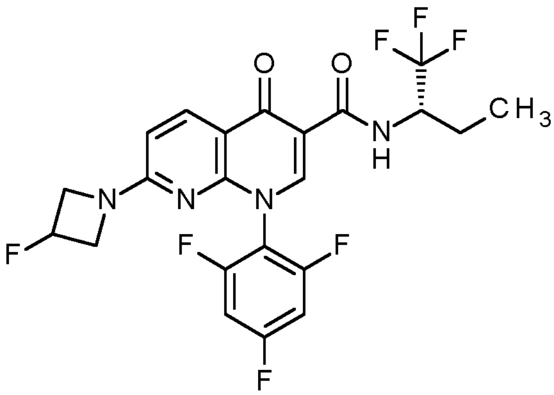 mit 3-Fluorazetidin (56% d. Th.) | LC-MS (Methode 3): $R_t$ = 2.19 min m/z = 503 $[M+H]^+$ $^1$H-NMR (400 MHz, DMSO-$d_6$) $\delta$ [ppm]: -0.149 (0.53), 0.147 (0.78), 0.949 (7.00), 0.968 (16.00), 0.986 (7.76), 1.235 (3.76), 1.398 (2.13), 1.603 (1.09), 1.621 (1.51), 1.639 (1.71), 1.663 (1.60), 1.681 (1.22), 1.868 (1.53), 1.879 (1.71), 1.895 (1.38), 2.073 (0.87), 2.328 (1.18), 2.367 (0.71), 2.670 (1.42), 2.711 (0.73), 3.997 (1.27), 4.276 (1.38), 4.747 (1.51), 5.378 (1.67), 5.522 (1.69), 6.671 (8.96), 6.693 (8.89), 7.524 (4.58), 7.546 (8.47), 7.568 (4.60), 8.318 (9.71), 8.340 (9.11), 8.834 (14.82), 10.344 (4.87), 10.369 (4.64). |

## Beispiel 502

7-[3,3-Bis(hydroxymethyl)azetidin-1-yl]-*N*-[(1*S*)-1-cyclopropyl-2,2,2-trifluorethyl]-4-oxo-1-(2,4,6-trifluorphenyl)-1,4-dihydro-1,8-naphthyridin-3-carboxamid

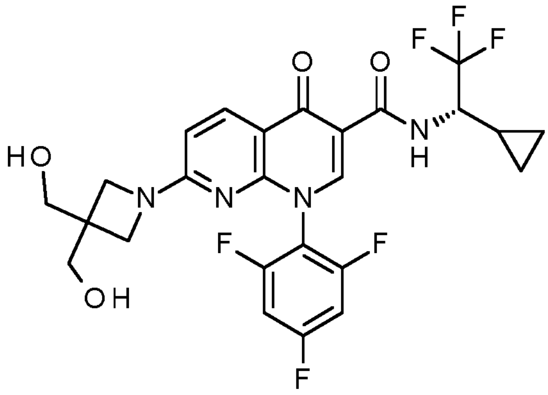

Die Verbindung aus Beispiel 492 (86.0 mg, 160 μmol) wurde in 1 ml Trifluoressigsäure vorgelegt, mit 1 ml Wasser und 1 ml Acetonitril versetzt und über das Wochenende bei Raumtemperatur gerührt. Der Gemisch wurde mittels präparativer HPLC gereinigt (RP18 Säule, Laufmittel: Acetonitril/Wasser-Gradient unter Zusatz von 0.1% TFA) Die Produktfraktionen wurden weitestgehend einrotiert, der Rückstand zweimal mit Dichlormethan extrahiert. Die vereinigten organischen Phasen wurden zweimal mit gesättigter, wässriger Natriumhydrogencarbonatlösung gewaschen. Die vereinigten wässrigen Phasen wurden einmal mit Dichlormethan reextrahiert. Die vereinigten organischen Phasen wurden über Natriumsulfat getrocknet, filtriert und einrotiert. Es wurden 86 mg der Zielverbindung (97% d. Th.) erhalten.
LC-MS (Methode 3): $R_t$ = 1.75 min; MS (ESIpos): m/z = 557 $[M+H]^+$
$^1$H-NMR (400 MHz, DMSO-$d_6$) δ [ppm]: -0.008 (2.85), 0.008 (2.81), 0.319 (1.20), 0.330 (1.93), 0.342 (1.91), 0.354 (1.47), 0.366 (0.73), 0.495 (0.49), 0.507 (1.35), 0.518 (1.98), 0.531 (1.75), 0.544 (1.78), 0.553 (1.55), 0.564 (1.94), 0.575 (1.69), 0.585 (1.52), 0.596 (1.25), 0.609 (0.79), 0.623 (0.98), 0.634 (1.10), 0.645 (1.77), 0.655 (1.61), 0.660 (1.54), 0.668 (1.55), 0.676 (0.78), 0.689 (0.54), 0.835 (0.50), 0.853 (0.53), 1.161 (0.74), 1.173 (1.24), 1.182 (1.63), 1.194 (2.31), 1.202 (1.75), 1.214 (2.20), 1.226 (1.46), 1.234 (1.53), 2.074 (1.23), 2.328 (0.58), 2.367 (0.46), 2.671 (0.59), 2.710 (0.43), 3.471 (16.00), 3.484 (15.15), 3.820 (2.70), 4.349 (0.99), 4.370 (1.73), 4.391 (1.70), 4.412 (0.88), 4.823 (4.75), 4.836 (11.08), 4.850 (4.63), 5.754 (0.48), 6.588 (6.33), 6.610 (6.39), 7.519 (3.46), 7.541 (6.53), 7.564 (3.44), 8.246 (6.51), 8.268 (6.21), 8.777 (10.05), 10.542 (4.05), 10.565 (3.86).

## Beispiel 503

7-[3,3-Bis(hydroxymethyl)azetidin-1-yl]-4-oxo-*N*-[(2*S*)-1,1,1-trifluorbutan-2-yl]-1-(2,4,6-trifluorphenyl)-1,4-dihydro-1,8-naphthyridin-3-carbonsäureamid

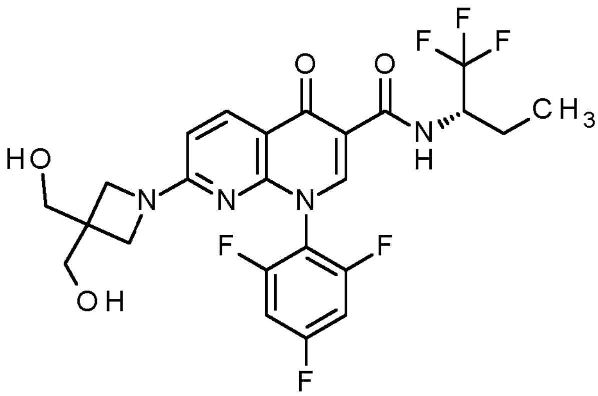

Die Verbindung aus Beispiel 498 (80.0 mg, 150 μmol) wurde in 0.94 ml Trifluoressigsäure vorgelegt, mit 0.94 ml Wasser und 0.94 ml Acetonitril versetzt und 2 Tage bei Raumtemperatur gerührt. Das Reaktionsgemisch wurde eingedampft und mittels präparativer HPLC gereinigt (RP18 Säule, Laufmittel: Acetonitril/Wasser-Gradient unter Zusatz von 0.1% TFA). Die Produktfraktionen wurden weitestgehend einrotiert, der Rückstand zweimal mit Dichlormethan extrahiert. Die vereinigten organischen Phasen wurden zweimal mit gesättigter, wässriger Natriumhydrogencarbonatlösung gewaschen. Die vereinigten wässrigen Phasen wurden einmal mit Dichlormethan reextrahiert. Die vereinigten

organischen Phasen wurden über Natriumsulfat getrocknet, filtriert und eingedampft. Es wurden 63 mg der Zielverbindung (76% d. Th.) erhalten.
LC-MS (Methode 3): $R_t$ = 1.72 min; MS (ESIpos): m/z = 545 $[M+H]^+$

**Beispiel 504**

7-[(4*S*)-4-Hydroxy-2-oxopyrrolidin-1-yl]-4-oxo-*N*-[(2*S*)-1,1,1-trifluor-3,3-dimethylbutan-2-yl]-1-(2,4,6-trifluorphenyl)-1,4-dihydro-1,8-naphthyridin-3-carbonsäureamid

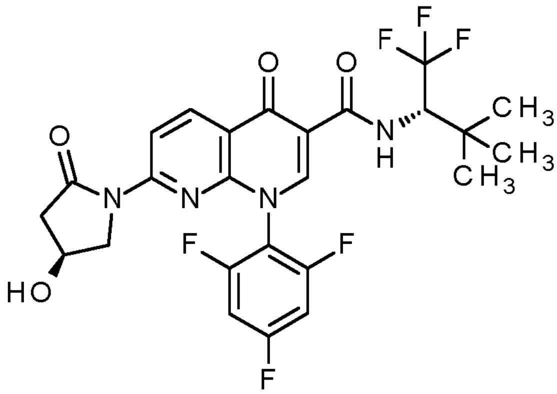

Die Verbindung aus Beispiel 117A (35.0 mg, 83.5 μmol) wurde in 1.0 ml Dimethylformamid vorgelegt und mit HATU (38.1 mg, 100 μmol) und *N*,*N*-Diisopropylethylamin (44 μl, 250 μmol) versetzt. Die Reaktionsmischung wurde 10 min bei RT gerührt und mit (2S)-1,1,1-Trifluor-3,3-dimethylbutan-2-amin (19.4 mg, 125 μmol), in 1.0 ml Dimethylformamid gelöst, versetzt. Der Ansatz wurde 10 min. nachgerührt, mit Acetonitril/Wasser verdünnt, durch einen Spritzenfilter filtriert und mittels präp. RP-HPLC (Säule: Reprosil 125x30; 10μ, Fluss: 50 mL/min, MeCN/Wasser/0,1%TFA) gereinigt. Die flüchtigen Bestandteile wurden im Vakuum entfernt und der Rückstand im Hochvakuum getrocknet. Man erhielt 40.5 mg (100% Reinheit, 87 % d. Th.) der Titelverbindung.
LC-MS (Methode 3): $R_t$ = 2.04 min; MS (ESIpos): m/z = 557 $[M+H]^+$
$^1$H-NMR (400 MHz, DMSO-d6) δ [ppm]: 1.102 (16.00), 2.359 (0.89), 2.403 (1.04), 2.921 (0.87), 2.936 (0.91), 2.964 (0.80), 2.979 (0.79), 3.465 (1.13), 3.495 (1.20), 3.677 (0.80), 3.689 (0.97), 3.707 (0.75), 3.719 (0.65), 4.296 (0.86), 4.652 (0.73), 7.604 (0.76), 7.613 (0.92), 7.627 (0.92), 7.636 (0.77), 8.535 (2.24), 8.557 (2.68), 8.738 (2.64), 8.760 (2.19), 9.086 (3.22), 10.465 (1.20), 10.490 (1.15).

**Beispiel 505**

*N*-[1-Cyclobutyl-2,2,2-trifluorethyl]-7-[(4*S*)-4-hydroxy-2-oxopyrrolidin-1-yl]-4-oxo-1-(2,4,6-trifluorphenyl)-1,4-dihydro-1,8-naphthyridin-3-carbonsäureamid (Diastereomerengemisch)

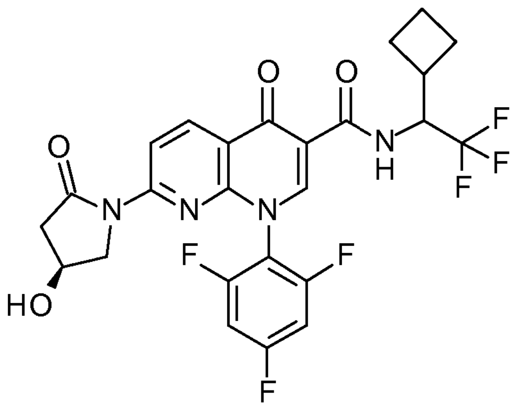

Die Verbindung aus Beispiel 117A (40.0 mg, 95.4 μmol) wurde in 1.0 ml Dimethylformamid vorgelegt und mit HATU (43.5 mg, 114 μmol) und *N,N*-Diisopropylethylamin (49.5 μl, 295 μmol) versetzt. Die Reaktionsmischung wurde 10 min bei RT gerührt und mit *rac*-1-Cyclobutyl-2,2,2-trifluorethanamin Hydrochlorid (27.1 mg, 143 μmol), in 1.0 ml Dimethylformamid und *N,N*-Diisopropylethylamin (16.5 μl, 95 μmol) gelöst, versetzt. Der Ansatz wurde 10 min. nachgerührt, mit Acetonitril/Wasser verdünnt, durch einen Spritzenfilter filtriert und mittels präp. RP-HPLC (Säule: Reprosil 125x30; 10μ, Fluss: 50 mL/min, MeCN/Wasser/0,1%TFA) gereinigt. Die flüchtigen Bestandteile wurden im Vakuum entfernt und der Rückstand im Hochvakuum getrocknet. Man erhielt 42.0 mg (100 % Reinheit, 79 % d. Th.) der Titelverbindung.
LC-MS (Methode 3): $R_t$ = 2.04 min; MS (ESIpos): m/z = 555 $[M+H]^+$
$^1$H-NMR (400 MHz, DMSO-d6) δ [ppm]: -0.008 (3.58), 0.008 (3.53), 1.762 (2.39), 1.789 (0.95), 1.883 (1.62), 1.891 (1.42), 1.904 (3.54), 1.916 (4.79), 1.926 (6.49), 1.933 (5.05), 1.940 (5.04), 1.960 (3.53), 1.990 (3.65), 2.005 (4.84), 2.019 (3.84), 2.040 (1.04), 2.074 (0.76), 2.358 (4.51), 2.402 (5.27), 2.814 (1.33), 2.834 (2.24), 2.857 (1.87), 2.876 (1.04), 2.919 (3.64), 2.933 (3.83), 2.962 (3.27), 2.977 (3.23), 3.467 (4.50), 3.496 (5.56), 3.676 (3.62), 3.688 (4.30), 3.706 (3.25), 3.718 (2.96), 4.297 (3.95), 4.787 (1.50), 4.806 (2.49), 4.829 (2.58), 4.849 (1.47), 5.337 (2.09), 7.597 (2.46), 7.604 (3.04), 7.617 (4.69), 7.626 (4.84), 7.634 (2.83), 7.640 (2.96), 7.647 (2.43), 8.536 (11.34), 8.559 (13.70), 8.728 (13.13), 8.750 (10.68), 9.090 (16.00), 10.272 (5.16), 10.296 (4.99).

25.8 mg der Titelverbindung (Diastereomerengemisch) wurde durch chirale HPLC in die Diastereomere getrennt (präparative HPLC: Säule Daicel Chiralpak IE-H 5 μm 250x20 mm; Eluent: 25% Ethanol, 75% n-Heptan; Fluss: 15 ml/min; UV-Detektion: 220 nm.)

Man erhielt (in der Reihenfolge der Elution von der Säule) 8 mg Diastereomer 1 aus Beispiel 506 (99% de) $R_t$ = 7.20 min und 15 mg (99%de) Diastereomer 2 aus Beispiel 507 $R_t$ = 8.83 min.

[Analytische HPLC: Säule Chiralpak IE-3 5 μm 250x4.6 mm; Eluent: 25% Ethanol, 75% Iso-Hexan; Temperatur: 30°C; Fluss: 1.0 ml/min; UV-Detektion: 220 nm].

## Beispiel 506

*N*-[1-Cyclobutyl-2,2,2-trifluorethyl]-7-[(4*S*)-4-hydroxy-2-oxopyrrolidin-1-yl]-4-oxo-1-(2,4,6-trifluorphenyl)-1,4-dihydro-1,8-naphthyridin-3-carbonsäureamid (Diastereomer 1)

LC-MS (Methode 3): $R_t$ = 2.03 min; MS (ESIpos): m/z = 555 $[M+H]^+$

## Beispiel 507

*N*-[1-Cyclobutyl-2,2,2-trifluorethyl]-7-[(4*S*)-4-hydroxy-2-oxopyrrolidin-1-yl]-4-oxo-1-(2,4,6-trifluorphenyl)-1,4-dihydro-1,8-naphthyridin-3-carbonsäureamid (Diastereomer 2)

LC-MS (Methode 3): $R_t$ = 2.02 min; MS (ESIpos): m/z = 555 $[M+H]^+$

## Beispiel 508

*N*-[2-Cyclopropyl-1,1,1-trifluorpropan-2-yl]-7-[(4*S*)-4-hydroxy-2-oxopyrrolidin-1-yl]-4-oxo-1-(2,4,6-trifluorphenyl)-1,4-dihydro-1,8-naphthyridin-3-carbonsäureamid (Diastereomerengemisch)

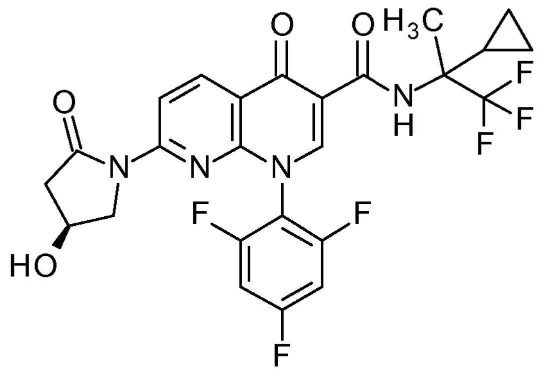

Die Verbindung aus Beispiel 117A (35.0 mg, 83.5 μmol) wurde in 1.0 ml Dimethylformamid vorgelegt und mit HATU (38.1 mg, 100 μmol) und *N*,*N*-Diisopropylethylamin (44 μl, 250 μmol) versetzt. Die Reaktionsmischung wurde 10 min bei RT gerührt und mit *rac*-2-Cyclopropyl-1,1,1-trifluorpropan-2-amin Hydrochlorid (23.7 mg, 125 μmol), in 1.0 ml Dimethylformamid und *N*,*N*-Diisopropylethylamin (15 μl, 83.5 μmol) gelöst, versetzt. Der Ansatz wurde 10 min. nachgerührt, mit Acetonitril/Wasser verdünnt, durch einen Spritzenfilter filtriert und mittels präp. RP-HPLC (Säule: Reprosil 125x30; 10μ, Fluss: 50 mL/min, MeCN/Wasser/0,1%TFA) gereinigt. Die flüchtigen Bestandteile wurden im Vakuum entfernt und der Rückstand im Hochvakuum getrocknet. Man erhielt 31.1 mg (100 % Reinheit, 67 % d. Th.) der Titelverbindung.

LC-MS (Methode 3): $R_t$ = 1.98 min; MS (ESIpos): m/z = 555 $[M+H]^+$

$^1$H-NMR (400 MHz, DMSO-d6) δ [ppm]: -0.149 (0.61), -0.008 (5.62), 0.008 (4.93), 0.146 (0.60), 0.512 (1.29), 0.576 (3.31), 0.597 (3.28), 0.696 (1.33), 1.415 (0.99), 1.422 (0.99), 1.432 (1.47), 1.435 (1.43), 1.444 (1.03), 1.609 (16.00), 2.355 (2.29), 2.398 (2.68), 2.914 (2.19), 2.929 (2.31), 2.957 (2.01), 2.972 (1.93), 3.460 (2.44), 3.490 (2.91), 3.670 (2.01), 3.682 (2.46), 3.700 (1.89), 3.712 (1.65), 4.279 (1.30), 4.292 (2.20), 7.592 (1.30), 7.599 (1.69), 7.611 (2.45), 7.621 (2.55), 7.634 (1.67), 7.642 (1.30), 8.521 (5.65), 8.543 (6.72), 8.717 (6.71), 8.739 (5.41), 8.999 (9.09), 10.088 (6.29).

30 mg der Titelverbindung (Diastereomerengemisch) wurde durch chirale HPLC in die Diastereomere getrennt (präparative HPLC: Säule Daicel IB 250x20 mm; Eluent: 30% Ethanol, 70% n-Heptan; Fluss: 20 ml/min; UV-Detektion: 220 nm.)

Man erhielt (in der Reihenfolge der Elution von der Säule) 2.8 mg Diastereomer 1 aus Beispiel 509 (99% de) $R_t$ = 1.58 min und 3.7 mg (99%de) Diastereomer 2 aus Beispiel 510 $R_t$ = 2.86 min.

[Analytische HPLC: Säule Chiralpak IB-3 3 μm 250x4.6 mm; Eluent: 20% Ethanol, 80% n-Heptan; Temperatur: 30°C; Fluss: 1.0 ml/min; UV-Detektion: 220 nm].

## Beispiel 509

*N*-[2-Cyclopropyl-1,1,1-trifluorpropan-2-yl]-7-[(4*S*)-4-hydroxy-2-oxopyrrolidin-1-yl]-4-oxo-1-(2,4,6-trifluorphenyl)-1,4-dihydro-1,8-naphthyridin-3-carboxamid (Diastereomer 1)

LC-MS (Methode 3): $R_t$ = 1.99 min; MS (ESIpos): m/z = 555 $[M+H]^+$

## Beispiel 510

*N*-[2-Cyclopropyl-1,1,1-trifluorpropan-2-yl]-7-[(4*S*)-4-hydroxy-2-oxopyrrolidin-1-yl]-4-oxo-1-(2,4,6-trifluorphenyl)-1,4-dihydro-1,8-naphthyridin-3-carbonsäureamid (Diastereomer 2)

LC-MS (Methode 3): $R_t$ = 2.00 min; MS (ESIpos): m/z = 555 $[M+H]^+$

## Beispiel 511

7-[(4*S*)-4-Hydroxy-2-oxopyrrolidin-1-yl]-4-oxo-*N*-(4,4,4-trifluor-2-methylbutan-2-yl)-1-(2,4,6-trifluorphenyl)-1,4-dihydro-1,8-naphthyridin-3-carbonsäureamid

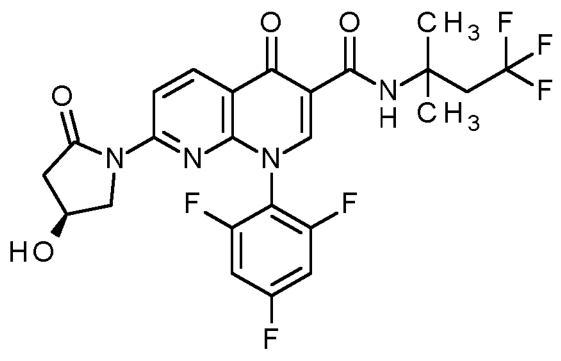

Die Verbindung aus Beispiel 117A (120 mg, 286 μmol) wurde in 4.0 ml Dimethylformamid vorgelegt und mit HATU (131 mg, 343 μmol) und *N*,*N*-Diisopropylethylamin (150 μl, 870 μmol) versetzt. Die Reaktionsmischung wurde

10 min bei RT gerührt und mit 4,4,4-Trifluor-2-methylbutan-2-aminhydrochlorid (1:1) (76.2 mg, 429 μmol), in 1.0 ml Dimethylformamid und *N*,*N*-Diisopropylethylamin (50 μl, 290 μmol) gelöst, versetzt. Der Ansatz wurde 10 min. nachgerührt, mit Acetonitril/Wasser verdünnt, durch einen Spritzenfilter filtriert und mittels präp. RP-HPLC (Säule: Reprosil 125x30; 10μ, Fluss: 50 mL/min, MeCN/Wasser/0,1%TFA) gereinigt. Die flüchtigen Bestandteile wurden im Vakuum entfernt und der Rückstand im Hochvakuum getrocknet. Man erhielt 84.4 mg (100 % Reinheit, 54 % d. Th.) der Titelverbindung.

LC-MS (Methode 3): $R_t$ = 1.89 min; MS (ESIpos): m/z = 543 $[M+H]^+$

$^1$H-NMR (400 MHz, DMSO-d6) δ [ppm]: -0.008 (0.78), 1.500 (16.00), 2.351 (1.25), 2.395 (1.44), 2.912 (1.44), 2.918 (0.93), 2.927 (1.42), 2.948 (2.18), 2.955 (1.57), 2.970 (1.54), 2.978 (2.05), 3.007 (0.63), 3.459 (1.24), 3.489 (1.53), 3.669 (1.11), 3.681 (1.35), 3.699 (1.04), 3.711 (0.90), 4.290 (0.98), 5.325 (0.99), 7.589 (0.69), 7.596 (0.88), 7.609 (1.31), 7.619 (1.31), 7.627 (0.77), 7.632 (0.86), 7.639 (0.68), 8.510 (3.14), 8.533 (3.85), 8.686 (3.77), 8.708 (2.99), 8.963 (4.50), 9.902 (3.32).

**Beispiel 512**

*N*-[1-Cyclopropyl-3,3,3-trifluorpropyl]-7-[(4*S*)-4-hydroxy-2-oxopyrrolidin-1-yl]-4-oxo-1-(2,4,6-trifluorphenyl)-1,4-dihydro-1,8-naphthyridin-3-carbonsäureamid (Diastereomerengemisch)

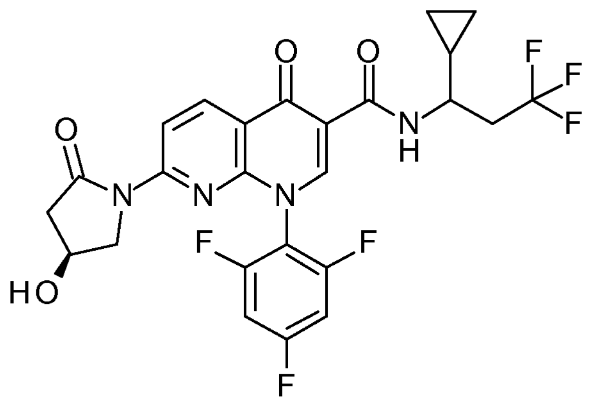

Die Verbindung aus Beispiel 117A (45.0 mg, 107 μmol) wurde in 1.0 ml Dimethylformamid vorgelegt und mit HATU (38.1 mg, 100 μmol) und *N*,*N*-Diisopropylethylamin (57 μl, 321 μmol) versetzt. Die Reaktionsmischung wurde 10 min bei RT gerührt und mit *rac*-1-Cyclopropyl-3,3,3-trifluorpropan-1-amin Hydrochlorid (30.5 mg, 161 μmol), in 1.0 ml Dimethylformamid und *N*,*N*-Diisopropylethylamin (19 μl, 107 μmol) gelöst, versetzt. Der Ansatz wurde 10 min. nachgerührt, mit Acetonitril/Wasser verdünnt, durch einen Spritzenfilter filtriert und mittels präp. RP-HPLC (Säule: Reprosil 125x30; 10μ, Fluss: 50 mL/min, MeCN/Wasser/0,1%TFA) gereinigt. Die flüchtigen Bestandteile wurden im Vakuum entfernt und der Rückstand im Hochvakuum getrocknet. Man erhielt 45.1 mg (100 % Reinheit, 76 % d. Th.) der Titelverbindung.

LC-MS (Methode 3): $R_t$ = 1.85 min; MS (ESIpos): m/z = 555 $[M+H]^+$

$^1$H-NMR (400 MHz, DMSO-d6) δ [ppm]: 0.008 (2.32), 0.295 (1.35), 0.304 (2.35), 0.316 (3.52), 0.327 (3.43), 0.346 (3.20), 0.359 (3.36), 0.370 (2.17), 0.381 (1.35), 0.457 (1.08), 0.466 (2.14), 0.478 (2.89), 0.488 (2.87), 0.498 (2.48), 0.507 (2.64), 0.515 (2.35), 0.528 (3.32), 0.536 (2.78), 0.549 (2.23), 0.557 (1.06), 1.150 (1.06), 1.159 (1.62), 1.170 (2.69), 1.179 (2.12), 1.191 (2.60), 1.203 (1.40), 1.211 (0.93), 2.329 (0.79), 2.352 (4.49), 2.367 (1.13), 2.395 (5.21), 2.520 (3.20), 2.524 (2.93), 2.636 (1.02), 2.648 (1.42), 2.666 (1.65), 2.675 (2.84), 2.687 (1.78), 2.704 (1.87), 2.711 (1.76), 2.715 (1.67), 2.769 (1.58), 2.789 (1.81), 2.798 (1.92), 2.807 (1.26), 2.818 (1.78), 2.828 (1.53), 2.836 (1.08), 2.856 (0.99), 2.913 (3.95), 2.928 (4.08), 2.956 (3.59), 2.971 (3.50), 3.462 (5.84), 3.492 (7.00), 3.672 (4.08), 3.684 (4.85), 3.701 (3.77), 3.714 (3.29), 3.770 (0.95), 3.783 (1.15), 3.792 (2.53), 3.803 (2.57), 3.813 (2.55), 3.825 (2.35), 3.834 (1.04), 3.846 (0.81), 4.279 (2.60), 4.292 (4.36), 4.305 (2.41), 6.947 (1.38), 7.074 (1.47), 7.202 (1.33), 7.586 (2.53), 7.593 (3.14), 7.606 (4.74), 7.616 (4.74), 7.629 (3.11), 7.636 (2.41), 8.515 (9.59), 8.537 (11.96), 8.689 (12.12), 8.711 (9.66), 8.970 (16.00), 9.869 (6.05), 9.890 (5.84).

40 mg der Titelverbindung (Diastereomerengemisch) wurde durch chirale HPLC in die Diastereomere getrennt (präparative HPLC: Säule Daicel Chiralpak IE-H 5 μm 250x20 mm; Eluent: 50% Ethanol, 50% n-Heptan; Temperatur: 25°C; Fluss: 15 ml/min; UV-Detektion: 210 nm.)

Man erhielt (in der Reihenfolge der Elution von der Säule) 13.2 mg Diastereomer 1 (99% de) $R_t$ = 2.50 min und 13.3 mg (95%de) Diastereomer 2 $R_t$ = 2.91 min.

[Analytische HPLC: Säule Chiraltek IE-3 3 μm 250x4.6 mm; Eluent: 25% Ethanol, 75% Iso-Hexan; Temperatur: 30°C; Fluss: 1.0 ml/min; UV-Detektion: 220 nm].

**Beispiel 513**

*N*-[1-Cyclopropyl-3,3,3-trifluorpropyl]-7-[(4*S*)-4-hydroxy-2-oxopyrrolidin-1-yl]-4-oxo-1-(2,4,6-trifluorphenyl)-1,4-dihydro-1,8-naphthyridin-3-carbonsäureamid (Diastereomer 1)

LC-MS (Methode 3): $R_t$ = 1.84 min; MS (ESIpos): m/z = 555 $[M+H]^+$

**Beispiel 514**

*N*-[1-Cyclopropyl-3,3,3-trifluorpropyl]-7-[(4*S*)-4-hydroxy-2-oxopyrrolidin-1-yl]-4-oxo-1-(2,4,6-trifluorphenyl)-1,4-dihydro-1,8-naphthyridin-3-carbonsäureamid (Diastereomer 2)

LC-MS (Methode 3): $R_t$ = 1.84 min; MS (ESIpos): m/z = 555 $[M+H]^+$

**Beispiel 515**

7-[(4*S*)-4-Hydroxy-2-oxopyrrolidin-1-yl]-4-oxo-*N*-[1-(trifluormethyl)cyclobutyl]-1-(2,4,6-trifluorphenyl)-1,4-dihydro-1,8-naphthyridin-3-carbonsäureamid

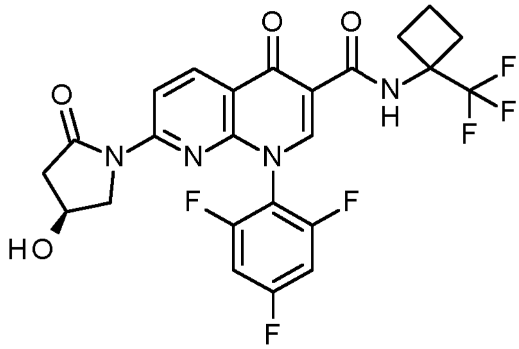

Die Verbindung aus Beispiel 117A (35.0 mg, 83.5 μmol) wurde in 1.0 ml Dimethylformamid vorgelegt und mit HATU (38.1 mg, 100 μmol) und *N,N*-Diisopropylethylamin (44.0 μl, 246 μmol) versetzt. Die Reaktionsmischung wurde 10 min bei RT gerührt und mit 1-(Trifluormethyl)cyclobutanamin Hydrochlorid (22.0 mg, 125 μmol) in 1.0 ml Dimethylformamid und *N,N*-Diisopropylethylamin (15 μl, 84 μmol) gelöst, versetzt. Der Ansatz wurde 10 min. nachgerührt, mit Acetonitril/Wasser verdünnt, durch einen Spritzenfilter filtriert und mittels präp. RP-HPLC (Säule: Reprosil 125x30; 10μ, Fluss: 50 mL/min, MeCN/Wasser/0,1%TFA) gereinigt. Die flüchtigen Bestandteile wurden im Vakuum entfernt und der Rückstand im Hochvakuum getrocknet. Man erhielt 35.8 mg (100 % Reinheit, 79 % d. Th.) der Titelverbindung.
LC-MS (Methode 3): $R_t$ = 1.89 min; MS (ESIpos): m/z = 541 $[M+H]^+$
$^1$H-NMR (400 MHz, DMSO-d6) δ [ppm]: 1.943 (2.33), 1.964 (2.95), 1.985 (2.04), 2.038 (2.61), 2.051 (2.53), 2.073 (5.69), 2.355 (4.96), 2.398 (5.89), 2.561 (6.85), 2.576 (7.95), 2.599 (6.71), 2.622 (4.45), 2.914 (4.08), 2.929 (4.26), 2.958 (3.72), 2.972 (3.66), 3.462 (5.03), 3.491 (6.09), 3.672 (3.95), 3.684 (4.67), 3.702 (3.59), 3.713 (3.21), 4.293 (4.61), 5.335 (4.12), 7.615 (6.03), 8.527 (8.73), 8.549 (10.88), 8.700 (10.72), 8.723 (8.55), 9.016 (16.00), 10.219 (12.50).

**Beispiel 516**

*N*-(1,1-Difluor-2-methylpropan-2-yl)-7-[(4*S*)-4-hydroxy-2-oxopyrrolidin-1-yl]-4-oxo-1-(2,4,6-trifluorphenyl)-1,4-dihydro-1,8-naphthyridin-3-carbonsäureamid

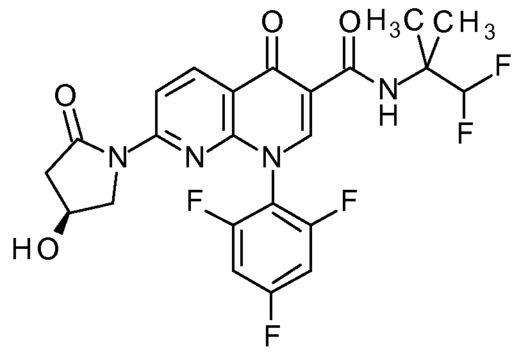

Die Verbindung aus Beispiel 117A (40.0 mg, 95.4 μmol) wurde in 1.0 ml Dimethylformamid vorgelegt und mit HATU (43.5 mg, 114 μmol) und *N,N*-Diisopropylethylamin (49.5 μl, 285 μmol) versetzt. Die Reaktionsmischung wurde 10 min bei RT gerührt und mit 1,1-Difluor-2-methylpropan-2-amin Hydrochlorid (20.8 mg, 143 μmol), in 1.0 ml Dimethylformamid und *N,N*-Diisopropylethylamin (16.5 μl, 95 μmol) gelöst, versetzt. Der Ansatz wurde 10 min. nachgerührt, mit Acetonitril/Wasser verdünnt, durch einen Spritzenfilter filtriert und mittels präp. RP-HPLC (Säule: Reprosil 125x30; 10μ, Fluss: 50 mL/min, MeCN/Wasser/0,1%TFA) gereinigt. Die flüchtigen Bestandteile wurden im Vakuum entfernt und der Rückstand im Hochvakuum getrocknet. Man erhielt 46.4 mg (100 % Reinheit, 95 % d. Th.) der Titelverbindung.
LC-MS (Methode 3): $R_t$ = 1.82 min; MS (ESIpos): m/z = 511 $[M+H]^+$
$^1$H-NMR (400 MHz, DMSO-d6) δ [ppm]: 1.455 (16.00), 2.074 (9.78), 2.353 (1.20), 2.396 (1.40), 2.911 (1.02), 2.926 (1.06), 2.955 (0.93), 2.970 (0.91), 3.459 (1.18), 3.489 (1.46), 3.669 (0.98), 3.681 (1.16), 3.699 (0.89), 3.711 (0.80), 4.292 (1.02), 5.325 (1.11), 5.332 (1.09), 6.289 (0.85), 6.432 (1.58), 6.574 (0.73), 7.591 (0.69), 7.599 (0.86), 7.611 (1.32), 7.621 (1.33), 7.634 (0.86), 7.641 (0.69), 8.517 (2.07), 8.539 (2.63), 8.693 (2.51), 8.715 (2.03), 8.986 (3.49), 10.038 (2.99).

## Beispiel 517

7-[(4*S*)-4-Acetamido-2-oxopyrrolidin-1-yl]-4-oxo-*N*-[(2*S*)-1,1,1-trifluorbutan-2-yl]-1-(2,4,6-trifluorphenyl)-1,4-dihydro-1,8-naphthyridin-3-carbonsäureamid (Diastereomerengemisch)

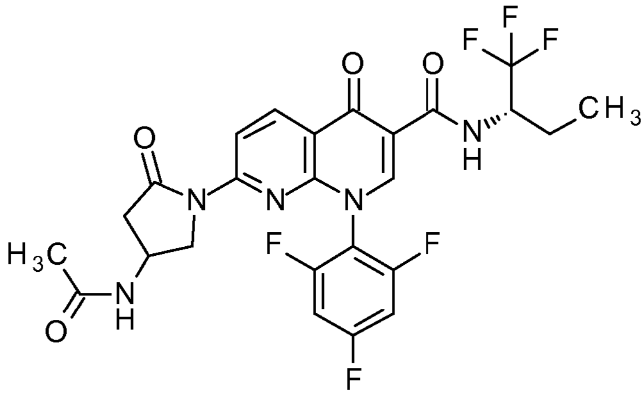

Die Verbindung aus Beispiel 151C (25.7 mg, 34.0 μmol) wurde in 2.0 ml Dichlormethan vorgelegt, und mit Triethylamin (14 μl, 100 μmol) versetzt. Die Reaktionsmischung wurde bei 0°C langsam mit Acetylchlorid (2.9 μl, 41 μmol) versetzt und dann über Nacht bei RT nachgerührt. Die flüchtigen Bestandteile wurden im Vakuum entfernt und der Rückstand mit Acetonitril/Wasser verdünnt und mittels präp. RP-HPLC (Säule: Reprosil 125x30; 10μ, Fluss: 50 mL/min, MeCN/Wasser/0,1%TFA) gereinigt. Die flüchtigen Bestandteile wurden im Vakuum entfernt und der Rückstand im Hochvakuum getrocknet. Man erhielt 16.6 mg (100 % Reinheit, 86 % d. Th.) der Titelverbindung.
LC-MS (Methode 3): $R_t$ = 1.77 min; MS (ESIpos): m/z = 570 $[M+H]^+$
$^1$H-NMR (400 MHz, DMSO-d6) δ [ppm]: 0.964 (3.36), 0.982 (6.75), 1.000 (3.60), 1.633 (0.51), 1.655 (0.80), 1.667 (0.95), 1.691 (0.94), 1.710 (0.66), 1.776 (16.00), 1.876 (0.94), 1.885 (0.96), 1.895 (1.03), 1.911 (0.83), 2.404 (1.58), 2.453 (2.17), 2.969 (1.38), 2.988 (1.50), 3.012 (1.28), 3.032 (1.25), 3.437 (1.58), 3.463 (1.75), 3.777 (1.40), 3.793 (1.73), 3.806 (1.45), 3.823 (1.29), 4.274 (2.02), 4.765 (1.03), 4.776 (1.01), 7.582 (1.88), 7.603 (3.43), 7.625 (1.86), 8.328 (2.08), 8.344

(2.05), 8.510 (2.75), 8.532 (3.34), 8.723 (3.32), 8.745 (2.77), 9.073 (4.94), 10.091 (2.30), 10.115 (2.24).

**Beispiel 518**

7-[4-(Acetamidomethyl)-2-oxopyrrolidin-1-yl]-4-oxo-*N*-[(2*S*)-1,1,1-trifluorbutan-2-yl]-1-(2,4,6-trifluorphenyl)-1,4-dihydro-1,8-naphthyridin-3-carbonsäureamid (Diastereomerengemisch)

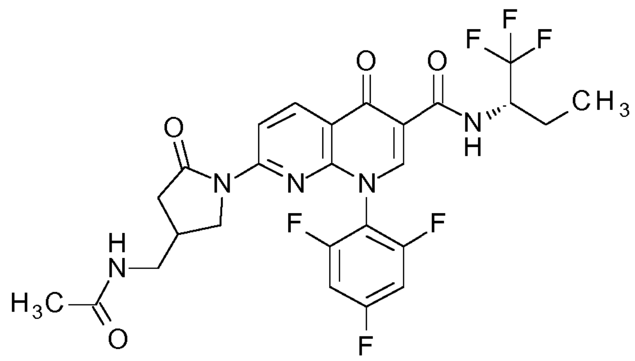

Kaliumcarbonat (12.9 mg, 93.6 μmol), Palladium(II)acetat (2.80 mg, 12.5 μmol) und 9,9-Dimethyl-4,5-bis-(diphenylphosphino)-xanthen (7.22 mg, 12.5 μmol) wurden in 3.0 ml Dioxan unter Argon für 10 Minuten bei RT gerührt. Dann wurden die Verbindung aus 115A (29.0 mg, 62.4 μmol) und die Verbindung aus Beispiel 152A (11.7 mg) zugegeben und die Mischung für 4 h bei 80°C gerührt. Der Ansatz wurde direct mittels präp. RP-HPLC (Säule: Reprosil 125x30; 10μ, Fluss: 50 mL/min, MeCN/Wasser/0,1%TFA) gereinigt. Die flüchtigen Bestandteile wurden im Vakuum entfernt und der Rückstand im Hochvakuum getrocknet. Man erhielt 1.70 mg (100 % Reinheit, 5 % d. Th.) der Titelverbindung.
LC-MS (Methode 1): $R_t$ = 0.97 min; MS (ESIpos): m/z = 584 $[M+H]^+$

**Beispiel 519**

7-[(4*S*)-4-Hydroxy-2-oxopyrrolidin-1-yl]-4-oxo-*N*-[1,1,1,2,2-pentafluorpentan-3-yl]-1-(2,4,6-trifluorphenyl)-1,4-dihydro-1,8-naphthyridin-3-carboxamid (Diastereomerengemisch)

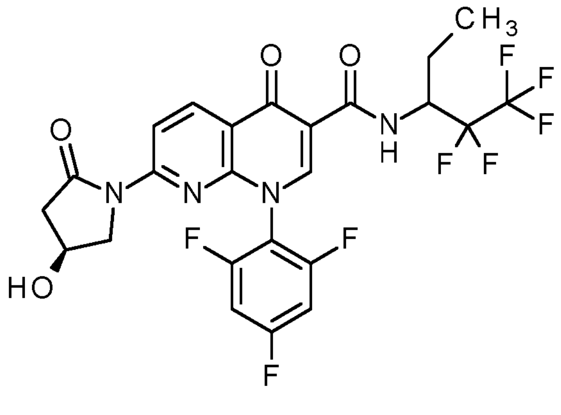

Gemäß AAV1 wurden 1.18 g (2.81 mmol) der Verbindung aus Beispiel 117A mit 300 mg (1.40 mmol) der Verbindung aus Beispiel 153B in Gegenwart von 1.28 g (3.37 mmol) HATU und 2.0 ml (11.0 mmol) DIPEA in 8.0 ml DMF zur Reaktion gebracht. Das Rohprodukt wurde mittels präparativer HPLC (Acetonitril / Wasser / 0,1 % Ameisensäure) gereinigt. Es wurden 528 mg (33% d. Th., 100% Reinheit) der Titelverbindung erhalten.
LC-MS (Methode 3): $R_t$ = 2.00 min; MS (ESIpos): m/z = 579 $[M+H]^+$
$^1H$ NMR (400 MHz, DMSO-$d_6$): δ ppm = 10.16 (d, 1H), 9.08 (s, 1H), 8.72 (d, 1H), 8.54 (d, 1H), 7.56-7.67 (m, 2H), 5.33 (d, 1H), 4.82-4.96 (m, 1H), 4.27-4.32 (m, 1H), 3.69 (dd, 1H), 3.48 (d, 1H), 2.95 (dd, 1H), 2.38 (d, 1H), 1.88-1.99 (m, 1H),

1.62-1.74 (m, 1H), 0.97 (t, 3H).

523 mg der Titelverbindung (Diastereomerengemisch) wurde durch chirale HPLC in die Diastereomere getrennt (präparative HPLC: Säule Daicel Chiralpak IE 5 μm 250x20 mm; Eluent: 80% *n*-Heptan, 20% Iso-Propanol; Temperatur: 30°C; Fluss: 15 ml/min; UV-Detektion: 220 nm.)

Man erhielt (in der Reihenfolge der Elution von der Säule) 249 mg (15% d. Th., 100% Reinheit) Diastereomer 1 aus Beispiel 520 (99% de) Rt = 11.07 min und 241 mg (15% d. Th., 100% Reinheit) Diastereomer 2 aus Beispiel 521 (99% de) Rt = 13.85 min.

[Analytische HPLC: Säule Daicel Chiralpak IE 5 μm 250x4.6 mm; Eluent: 80% Iso-Hexan, 20% Iso-propanol; Temperatur: 30°C; Fluss: 1.0 ml/min; UV-Detektion: 220 nm].

## Beispiel 520

7-[(4*S*)-4-Hydroxy-2-oxopyrrolidin-1-yl]-4-oxo-*N*-[1,1,1,2,2-pentafluorpentan-3-yl]-1-(2,4,6-trifluorphenyl)-1,4-dihydro-1,8-naphthyridin-3-carboxamid (Diastereomer 1)

LC-MS (Methode 1): $R_t$ = 1.08 min; MS (ESIpos): m/z = 579 $[M+H]^+$

$^1H$ NMR (400 MHz, DMSO-$d_6$): δ ppm = 10.16 (d, 1H), 9.08 (s, 1H), 8.72 (d, 1H), 8.54 (d, 1H), 7.56-7.67 (m, 2H), 5.34 (d, 1H), 4.82-4.95 (m, 1H), 4.27-4.32 (m, 1H), 3.69 (dd, 1H), 3.48 (d, 1H), 2.95 (dd, 1H), 2.38 (d, 1H), 1.88-1.98 (m, 1H), 1.62-1.64 (m, 1H), 0.97 (t, 3H).

## Beispiel 521

7-[(4*S*)-4-Hydroxy-2-oxopyrrolidin-1-yl]-4-oxo-*N*-[1,1,1,2,2-pentafluorpentan-3-yl]-1-(2,4,6-trifluorphenyl)-1,4-dihydro-1,8-naphthyridin-3-carboxamid (Diastereomer 2)

LC-MS (Methode 1): $R_t$ = 1.08 min; MS (ESIpos): m/z = 579 $[M+H]^+$

$^1H$ NMR (400 MHz, DMSO-$d_6$): δ ppm = 10.16 (d, 1H), 9.08 (s, 1H), 8.72 (d, 1H), 8.54 (d, 1H), 7.57-7.67 (m, 2H), 5.33 (d, 1H), 4.82-4.96 (m, 1H), 4.27-4.32 (m, 1H), 3.69 (dd, 1H), 3.47 (d, 1H), 2.95 (dd, 1H), 2.38 (d, 1H), 1.88-1.99 (m, 1H), 1.62-1.74 (m, 1H), 0.98 (t, 3H).

## Beispiel 522

7-[(3*R*,4*R*)-3,4-Dihydroxypyrrolidin-1-yl]-4-oxo-*N*-[1,1,1,2,2-pentafluorpentan-3-yl]-1-(2,4,6-trifluorphenyl)-1,4-dihydro-1,8-naphthyridin-3-carboxamid (Diastereomerengemisch)

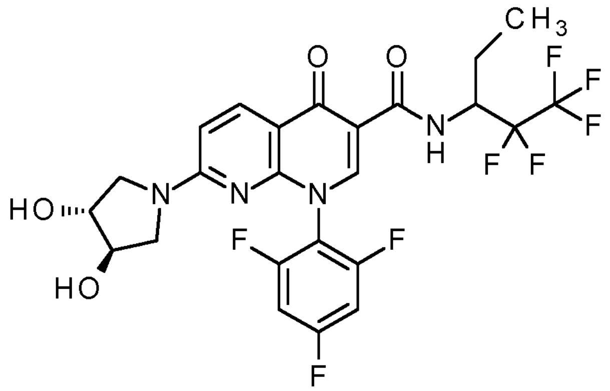

Gemäß AAV1 wurden 947 mg (2.25 mmol) der Verbindung aus Beispiel 121A mit 300 mg (1.40 mmol) der Verbindung aus Beispiel 153B in Gegenwart von 1.03 g (2.70 mmol) HATU und 1.6 ml (9.00 mmol) DIPEA in 6.4 ml DMF zur Reaktion gebracht. Das Rohprodukt wurde mittels präparativer HPLC (Acetonitril / Wasser/ 0,1 % Ameisensäure) gereinigt. Es wurden 477 mg (37% d. Th., 100% Reinheit) der Titelverbindung erhalten.
LC-MS (Methode 3): $R_t$ = 1.83 min; MS (ESIpos): m/z = 581 $[M+H]^+$

$^1H$ NMR (400 MHz, DMSO-$d_6$): δ ppm = 10.48 (d, 1H), 8.81 (s, 1H), 8.28 (d, 1H), 7.53-7.60 (m, 2H), 6.78 (d, 1H), 5.24 (d, 1H), 5.14 (d, 1H), 4.80-4.93 (m, 1H), 4.03-4.07 (m, 1H), 3.90-3.95 (m, 1H), 3.58-3.64 (m, 1H), 3.32-3.37 (m, 1H),

3.21-3.30 (m, 1H), 3.03-3.10 (m, 1H), 1.86-1.97 (m, 1H), 1.60-1.72 (m, 1H), 0.96 (t, 3H).

472 mg der Titelverbindung (Diastereomerengemisch) wurde durch chirale HPLC in die Diastereomere getrennt (präparative HPLC: Säule Daicel Chiralpak IA 5 µm 250x20 mm; Eluent: 85% *n*-Heptan, 15% Iso-Propanol; Temperatur: 30°C; Fluss: 15 ml/min; UV-Detektion: 220 nm.)

Man erhielt (in der Reihenfolge der Elution von der Säule) 203 mg (16% d. Th., 100% Reinheit) Diastereomer 1 aus Beispiel 523 (99% de) Rt = 12.94 min und 209 mg (16% d. Th., 100% Reinheit) Diastereomer 2 aus Beispiel 524 (99% de) Rt = 15.48 min.

[Analytische HPLC: Säule Daicel Chiralpak IA 5 µm 250x4.6 mm; Eluent: 80% Iso-Hexan, 20% Iso-Propanol; Temperatur: 25°C; Fluss: 1.0 ml/min; UV-Detektion: 220nm].

## Beispiel 523

7-[(3*R*,4*R*)-3,4-Dihydroxypyrrolidin-1-yl]-4-oxo-*N*-[1,1,1,2,2-pentafluorpentan-3-yl]-1-(2,4,6-trifluorphenyl)-1,4-dihydro-1,8-naphthyridin-3-carboxamid (Diastereomer 1)

LC-MS (Methode 3): $R_t$ = 1.83 min; MS (ESIpos): m/z = 581 $[M+H]^+$
$^1H$ NMR (400 MHz, DMSO-$d_6$): δ ppm = 10.48 (d, 1H), 8.81 (s, 1H), 8.28 (d, 1H), 7.53-7.60 (m, 2H), 6.78 (d, 1H), 5.23 (d, 1H), 5.14 (d, 1H), 4.80-4.93 (m, 1H), 4.05 (br s, 1H), 3.93 (br s, 1H), 3.62 (dd, 1H), 3.32-3.37 (m, 1H), 3.25 (dd, 1H), 3.07 (d, 1H), 1.87-1.97 (m, 1H), 1.60-1.71 (m, 1H), 0.96 (t, 3H).

## Beispiel 524

7-[(3*R*,4*R*)-3,4-Dihydroxypyrrolidin-1-yl]-4-oxo-*N*-[1,1,1,2,2-pentafluorpentan-3-yl]-1-(2,4,6-trifluorphenyl)-1,4-dihydro-1,8-naphthyridin-3-carboxamid (Diastereomer 2)

LC-MS (Methode 3): $R_t$ = 1.83 min; MS (ESIpos): m/z = 581 $[M+H]^+$
$^1H$ NMR (400 MHz, DMSO-$d_6$): δ ppm = 10.48 (d, 1H), 8.81 (s, 1H), 8.28 (d, 1H), 7.52-7.60 (m, 2H), 6.78 (d, 1H), 5.24 (d, 1H), 5.14 (d, 1H), 4.79-4.93 (m, 1H), 4.05 (br s, 1H), 3.93 (br s, 1H), 3.61 (br dd, 1H), 3.32-3.37 (m, 1H), 3.20-3.29 (m, 1H), 3.07 (br d, 1H), 1.86-1.97 (m, 1H), 1.59-1.72 (m, 1H), 0.96 (t, 3H).

## Beispiel 525

7-[(4*S*)-4-Hydroxy-2-oxopyrrolidin-1-yl]-4-oxo-*N*-[(2*S*)-1,1,1-trifluor-4-methylpentan-2-yl]-1-(2,4,6-trifluorphenyl)-1,4-dihydro-1,8-naphthyridin-3-carboxamid

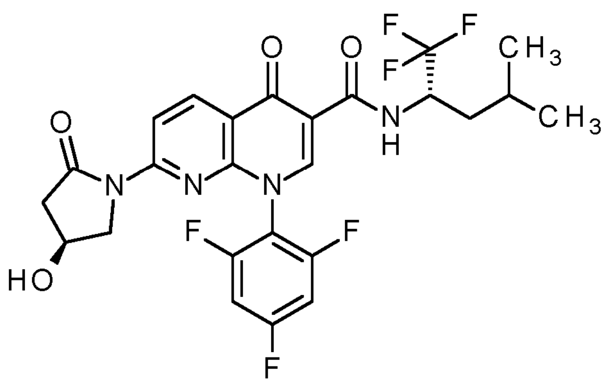

Gemäß AAV1 wurden 50.0 mg (119 µmol) der Verbindung aus Beispiel 117A mit 27.4 mg (143 µmol) (2*S*)-1,1,1-Trifluor-4-methylpentan-2-amin Hydrochlorid in Gegenwart von 54.4 mg (143 µmol) HATU und 62 µl (360 µmol) DIPEA in 460 µl DMF zur Reaktion gebracht. Das Rohprodukt wurde mittels präparativer HPLC (Acetonitril / Wasser/ 0,1 % Ameisensäure) gereinigt. Es wurden 50.3 mg (76% d. Th., 100% Reinheit) der Titelverbindung erhalten.
LC-MS (Methode 3): $R_t$ = 2.10 min; MS (ESIpos): m/z = 557 $[M+H]^+$
$^1H$ NMR (400 MHz, DMSO-$d_6$): δ ppm = 10.10 (d, 1H), 9.07 (s, 1H), 8.71 (d, 1H), 8.54 (d, 1H), 7.58-7.66 (m, 2H), 5.33 (d, 1H), 4.80-4.90 (m, 1H), 4.27-4.31 (m, 1H), 3.69 (dd, 1H), 3.47 (d, 1H), 2.94 (dd, 1H), 2.38 (d, 1H), 1.64-1.75 (m, 2H), 1.54-1.62 (m, 1H), 0.95 (d, 3H), 0.90 (d, 3H).

**Beispiel 526**

7-[(4*S*)-4-Hydroxy-2-oxopyrrolidin-1-yl]-4-oxo-*N*-[1,1,1-trifluor-3-methylbutan-2-yl]-1-(2,4,6-trifluorphenyl)-1,4-dihydro-1,8-naphthyridin-3-carboxamid (Diastereomerengemisch)

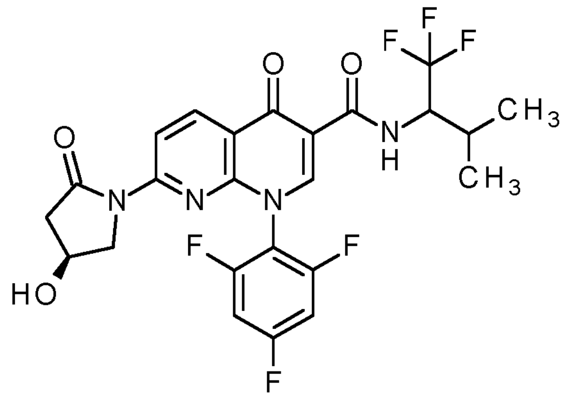

Gemäß AAV1 wurden 80.0 mg (191 µmol) der Verbindung aus Beispiel 117A mit 32.3 mg (229 µmol) 1,1,1-Trifluor-3-methylbutan-2-amin in Gegenwart von 87.1 mg (229 µmol) HATU und 100 µl (570 µmol) DIPEA in 1.4 ml DMF zur Reaktion gebracht. Das Rohprodukt wurde mittels präparativer HPLC (Acetonitril / Wasser/ 0,1 % Ameisensäure) gereinigt. Es wurden 81.5 mg (79% d. Th., 100% Reinheit) der Titelverbindung erhalten.
LC-MS (Methode 3): $R_t$ = 1.97 min; MS (ESIpos): m/z = 543 $[M+H]^+$
$^1H$ NMR (400 MHz, DMSO-$d_6$): δ ppm = 10.32 (d, 1H), 9.08 (s, 1H), 8.74 (d, 1H), 8.54 (d, 1H), 7.58-7.66 (m, 2H), 5.33 (dd, 1H), 4.74-4.84 (m, 1H), 4.27-4.32 (m, 1H), 3.69 (dd, 1H), 3.48 (d, 1H), 2.95 (dd, 1H), 2.38 (d, 1H), 2.22-2.31 (m, 1H), 1.04 (d, 3H), 0.98 (d, 3H).

68.0 mg der Titelverbindung (Diastereomerengemisch) wurde durch chirale HPLC in die Diastereomere getrennt (präparative HPLC: Säule Daicel Chiralcel OX-H 5 µm 250x20 mm; Eluent: 50% *n*-Heptan, 50% Iso-Propanol; Temperatur: 40°C; Fluss: 15 ml/min; UV-Detektion: 220 nm.)

Man erhielt (in der Reihenfolge der Elution von der Säule) 31.0 mg (30% d. Th., 100% Reinheit) Diastereomer 1 aus Beispiel 527 (99% de) Rt = 4.55 min und 31.0 mg (30% d. Th., 100% Reinheit) Diastereomer 2 aus Beispiel 528 (99% de) Rt = 6.48 min.

[Analytische HPLC: Säule Daicel Chiralcel OX-H 5 µm 250x4.6 mm; Eluent: 50% Iso-Hexan, 50% Iso-Propanol; Temperatur: 45°C; Fluss: 1.0 ml/min; UV-Detektion: 220nm].

**Beispiel 527**

7-[(4*S*)-4-Hydroxy-2-oxopyrrolidin-1-yl]-4-oxo-*N*-[1,1,1-trifluor-3-methylbutan-2-yl]-1-(2,4,6-trifluorphenyl)-1,4-dihydro-1,8-naphthyridin-3-carboxamid (Diastereomer 1)

LC-MS (Methode 3): $R_t$ = 1.94 min; MS (ESIpos): m/z = 543 $[M+H]^+$
$^1H$ NMR (400 MHz, DMSO-$d_6$): δ ppm = 10.32 (d, 1H), 9.08 (s, 1H), 8.74 (d, 1H), 8.54 (d, 1H), 7.58-7.66 (m, 2H), 5.34 (d, 1H), 4.74-4.84 (m, 1H), 4.27-4.32 (m, 1H), 3.69 (dd, 1H), 3.48 (br d, 1H), 2.95 (dd, 1H), 2.38 (br d, 1H), 2.22-2.31 (m, 1H), 1.04 (d, 3H), 0.98 (d, 3H).

**Beispiel 528**

7-[(4*S*)-4-Hydroxy-2-oxopyrrolidin-1-yl]-4-oxo-*N*-[1,1,1-trifluor-3-methylbutan-2-yl]-1-(2,4,6-trifluorphenyl)-1,4-dihydro-1,8-naphthyridin-3-carboxamid (Diastereomer 2)

LC-MS (Methode 3): $R_t$ = 1.95 min; MS (ESIpos): m/z = 543 $[M+H]^+$
$^1H$ NMR (400 MHz, DMSO-$d_6$): δ ppm = 10.32 (d, 1H), 9.08 (s, 1H), 8.74 (d, 1H), 8.54 (d, 1H), 7.58-7.66 (m, 2H), 5.33 (d, 1H), 4.74-4.84 (m, 1H), 4.27-4.32 (m, 1H), 3.70 (dd, 1H), 3.48 (br d, 1H), 2.95 (dd, 1H), 2.38 (br d, 1H), 2.22-2.31 (m, 1H), 1.04 (d, 3H), 0.98 (d, 3H).

## Beispiel 529

7-[(4*S*)-4-Hydroxy-2-oxopyrrolidin-1-yl]-4-oxo-*N*-[3,3,4,4,4-pentafluorbutan-2-yl]-1-(2,4,6-trifluorphenyl)-1,4-dihydro-1,8-naphthyridin-3-carboxamid (Diastereomerengemisch)

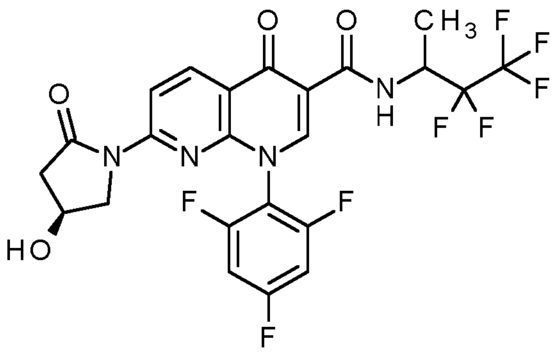

Gemäß AAV1 wurden 500 mg (1.19 mmol) der Verbindung aus Beispiel 117A mit 262 mg (1.31 mmol) der Verbindung 147B in Gegenwart von 544 mg (1.43 mmol) HATU und 830 µl (4.80 mmol) DIPEA in 5.0 ml DMF zur Reaktion gebracht. Das Rohprodukt wurde mittels präparativer HPLC (Acetonitril / Wasser/ 0,1 % Ameisensäure) gereinigt. Es wurden 498 mg (74% d. Th., 100% Reinheit) der Titelverbindung erhalten.
LC-MS (Methode 3): $R_t$ = 1.91 min; MS (ESIpos): m/z = 565 $[M+H]^+$
$^1H$ NMR (400 MHz, DMSO-$d_6$): δ ppm = 10.24 (d, 1H), 9.08 (s, 1H), 8.71 (d, 1H), 8.54 (dd, 1H), 7.58-7.65 (m, 2H), 5.33 (t, 1H), 4.97-5.11 (m, 1H), 4.27-4.32 (m, 1H), 3.66-3.72 (m, 1H), 3.45-3.50 (m, 1H), 2.94 (ddd, 1H), 2.37 (br d, 1H), 1.41 (d, 3H).

492 mg der Titelverbindung (Diastereomerengemisch) wurde durch chirale HPLC in die Diastereomere getrennt (präparative HPLC: Säule Daicel Chiralpak AD-H 5 µm 250x20 mm; Eluent: 80% *n*-Heptan, 20% Iso-Propanol; Temperatur: 23°C; Fluss: 20 ml/min; UV-Detektion: 220 nm.)

Man erhielt (in der Reihenfolge der Elution von der Säule) 200.4 mg (30% d. Th., 100% Reinheit) Diastereomer 1 aus Beispiel 530 (99% de) Rt = 6.05 min und 199 mg (30% d. Th., 100% Reinheit) Diastereomer 2 aus Beispiel 531 (99% de) Rt = 8.82 min.

[Analytische HPLC: Säule Daicel AD-3 3 µm 50x4.6 mm; Eluent: 80% Iso-Hexan, 20% Iso-Propanol; UV-Detektion: 220nm].

## Beispiel 530

7-[(4*S*)-4-Hydroxy-2-oxopyrrolidin-1-yl]-4-oxo-*N*-[3,3,4,4,4-pentafluorbutan-2-yl]-1-(2,4,6-trifluorphenyl)-1,4-dihydro-1,8-naphthyridin-3-carboxamid (Diastereomer 1)

LC-MS (Methode 3): $R_t$ = 1.89 min; MS (ESIpos): m/z = 565 $[M+H]^+$
$^1H$ NMR (400 MHz, DMSO-$d_6$): δ ppm = 10.24 (d, 1H), 9.07 (s, 1H), 8.71 (d, 1H), 8.54 (d, 1H), 7.54-7.69 (m, 2H), 5.34 (d, 1H), 4.98-5.12 (m, 1H), 4.26-4.32 (m, 1H), 3.69 (dd, 1H), 3.48 (br d, 1H), 2.94 (dd, 1H), 2.38 (br d, 1H), 1.41 (d, 3H).

## Beispiel 531

7-[(4*S*)-4-Hydroxy-2-oxopyrrolidin-1-yl]-4-oxo-*N*-[3,3,4,4,4-pentafluorbutan-2-yl]-1-(2,4,6-trifluorphenyl)-1,4-dihydro-1,8-naphthyridin-3-carboxamid (Diastereomer 2)

LC-MS (Methode 3): $R_t$ = 1.90 min; MS (ESIpos): m/z = 565 $[M+H]^+$
$^1H$ NMR (400 MHz, DMSO-$d_6$): δ ppm = 10.24 (d, 1H), 9.08 (s, 1H), 8.71 (d, 1H), 8.54 (d, 1H), 7.58-7.65 (m, 2H), 5.33 (d, 1H), 4.98-5.12 (m, 1H), 4.27-4.32 (m, 1H), 3.69 (dd, 1H), 3.47 (br d, 1H), 2.95 (dd, 1H), 2.37 (br d, 1H), 1.41 (d, 3H).

**Beispiel 532**

*N*-[1-(2-Chlorphenyl)-2,2,2-trifluorethyl]-7-[(4*S*)-4-hydroxy-2-oxopyrrolidin-1-yl]-4-oxo-1-(2,4,6-trifluorphenyl)-1,4-dihydro-1,8-naphthyridin-3-carboxamid (Diastereomerengemisch)

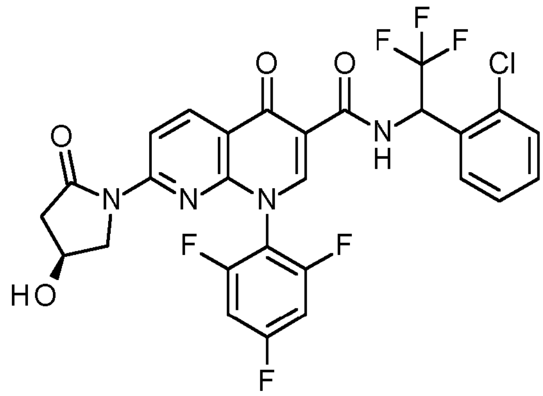

Gemäß AAV1 wurden 50.0 mg (119 µmol) 7-[(4*S*)-4-Hydroxy-2-oxopyrrolidin-1-yl]-4-oxo-1-(2,4,6-trifluorphenyl)-1,4-dihydro-1,8-naphthyridin-3-carbonsäure mit 30.0 mg (143 µmol) 1-(2-Chlorphenyl)-2,2,2-trifluorethanamin in Gegenwart von 54.4 mg (143 µmol) HATU und 62 µl (360 µmol) DIPEA in 460 µl DMF zur Reaktion gebracht. Das Rohprodukt wurde mittels präparativer HPLC (Acetonitril / Wasser/ 0,1 % Ameisensäure) gereinigt. Es wurden 41.9 mg (58% d. Th., 100% Reinheit) der Titelverbindung erhalten.
LC-MS (Methode 3): $R_t$ = 2.14 min; MS (ESIpos): m/z = 611 $[M+H]^+$
$^1$H NMR (400 MHz, DMSO-$d_6$): δ ppm = 11.21 (d, 1H), 9.09 (s, 1H), 8.76 (d, 1H), 8.56 (d, 1H), 7.44-7.70 (m, 6H), 6.42-6.51 (m, 1H), 5.30-5.37 (m, 1H), 4.27-4.32 (m, 1H), 3.65-3.72 (m, 1H), 3.42-3.51 (m, 1H), 2.94 (ddd, 1H), 2.38 (d, 1H).

35.8 mg der Titelverbindung (Diastereomerengemisch) wurde durch chirale HPLC in die Diastereomere getrennt (präparative HPLC: Säule YMC Chiralart Amylose SA 5 µm 250x30 mm; Eluent: 60% *n*-Heptan, 40% Iso-Propanol; Temperatur: 30°C; Fluss: 30 ml/min; UV-Detektion: 220 nm.)

Man erhielt (in der Reihenfolge der Elution von der Säule) 15.0 mg Diastereomer 1 (99% de) Rt = 6.50 min und 15.0 mg (99% de) Diastereomer 2 Rt = 8.62 min.

[Analytische HPLC: Säule YMC Chiralart Amylose SA 5 µm 250x4.6 mm; Eluent: 60% Iso-Hexan, 40% Isopropanol; Temperatur: 30°C; Fluss: 1.0 ml/min; UV-Detektion: 220 nm].

Diastereomer 1 wurde zusätzlich mittels präparativer HPLC (Säule: Chromatorex C18, 10 µm, 125*40 mm, Lösungsmittel: Acetonitril, Wasser, 0.1% Ameisensäure) gereinigt und 13.4 mg (19% d. Th., 100% Reinheit) der Titelverbindung aus Beispiel 533 erhalten.

Diastereomer 2 wurde zusätzlich mittels präparativer HPLC (Säule: Chromatorex C18, 10 µm, 125*40 mm, Lösungsmittel: Acetonitril, Wasser, 0.1% Ameisensäure) gereinigt und 13.2 mg (18% d. Th., 100% Reinheit) der Titelverbindung aus Beispiel 534 erhalten.

**Beispiel 533**

*N*-[1-(2-Chlorphenyl)-2,2,2-trifluorethyl]-7-[(4*S*)-4-hydroxy-2-oxopyrrolidin-1-yl]-4-oxo-1-(2,4,6-trifluorphenyl)-1,4-dihydro-1,8-naphthyridin-3-carboxamid (Diastereomer 1)

LC-MS (Methode 1): $R_t$ = 1.13 min; MS (ESIpos): m/z = 611 $[M+H]^+$
$^1$H NMR (400 MHz, DMSO-$d_6$): δ ppm = 11.21 (d, 1H), 9.09 (s, 1H), 8.76 (d, 1H), 8.56 (d, 1H), 7.46-7.70 (m, 6H), 6.42-6.51 (m, 1H), 5.33 (d, 1H), 4.27-4.32 (m, 1H), 3.69 (dd, 1H), 3.47 (d, 1H), 2.95 (dd, 1H), 2.38 (br d, 1H).

**Beispiel 534**

*N*-[1-(2-Chlorphenyl)-2,2,2-trifluorethyl]-7-[(4*S*)-4-hydroxy-2-oxopyrrolidin-1-yl]-4-oxo-1-(2,4,6-trifluorphenyl)-1,4-dihydro-1,8-naphthyridin-3-carboxamid (Diastereomer 2)

LC-MS (Methode 1): $R_t$ = 1.13 min; MS (ESIpos): m/z = 611 $[M+H]^+$

$^{1}H$ NMR (400 MHz, DMSO-$d_6$): δ ppm = 11.21 (d, 1H), 9.09 (s, 1H), 8.76 (d, 1H), 8.56 (d, 1H), 7.48-7.66 (m, 6H), 6.42-6.51 (m, 1H), 5.34 (d, 1H), 4.27-4.31 (m, 1H), 3.68 (dd, 1H), 3.47 (br d, 1H), 2.94 (dd, 1H), 2.38 (br d, 1H).

**Beispiel 535**

*N*-[(1*S*)-1-Cyclopropyl-2,2,2-trifluorethyl]-7-[(3*S*)-3-methoxypyrrolidin-1-yl]-4-oxo-1-(2,4,6-trifluorphenyl)-1,4-dihydro-1,8-naphthyridin-3-carboxamid

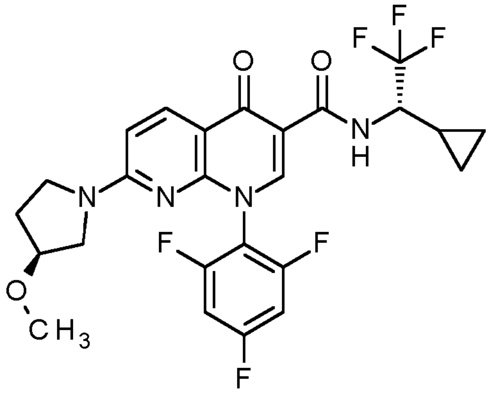

Gemäß AAV3 wurden 60.0 mg (126 μmol) der Verbindung aus Beispiel 126A mit 22.6 mg (164 μmol) (3*S*)-3-Methoxypyrrolidin Hydrochlorid und 99 μl (570 μmol) DIPEA in 700 μl DMF zur Reaktion gebracht. Das Rohprodukt wurde mittels präparativer HPLC (Acetonitril / Wasser/ 0,1 % Ameisensäure) gereinigt. Es wurden 58.2 mg (85% d. Th., 100% Reinheit) der Titelverbindung erhalten.
LC-MS (Methode 3): $R_t$ = 2.27 min; MS (ESIpos): m/z = 541 $[M+H]^+$
$^{1}H$ NMR (400 MHz, DMSO-$d_6$): δ ppm = 10.56 (d, 1H), 8.80 (s, 1H), 8.29 (d, 1H), 7.51-7.61 (m, 2H), 6.79 (d, 1H), 4.33-4.43 (m, 1H), 3.94-4.11 (m, 1H), 3.48-3.60 (m, 1.5H), 3.37-3.47 (m, 0.5H), 3.16-3.28 (m, 4.5H), 3.04-3.14 (m, 0.5H), 1.89-2.15 (m, 2H), 1.16-1.25 (m, 1H), 0.50-0.69 (m, 3H), 0.31-0.38 (m, 1H).

**Beispiel 536**

4-Oxo-7-(2-oxoimidazolidin-1-yl)-*N*-[1,1,1-trifluor-3-methylbutan-2-yl]-1-(2,4,6-trifluorphenyl)-1,4-dihydro-1,8-naphthyridin-3-carboxamid (Racemat)

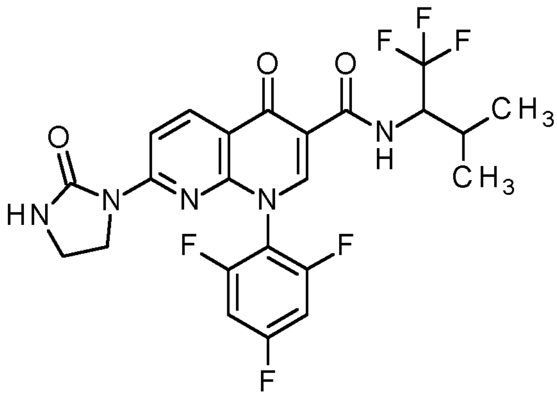

Gemäß AAV1 wurden 80.0 mg (198 μmol) der Verbindung aus Beispiel 113A mit 33.5 mg (237 μmol) 1,1,1-Trifluor-3-methylbutan-2-amin in Gegenwart von 90.3 mg (237 μmol) HATU und 100 μl (590 μmol) DIPEA in 1.4 ml DMF zur Reaktion gebracht. Das Rohprodukt wurde mittels präparativer HPLC (Acetonitril / Wasser/ 0,1 % Ameisensäure) gereinigt. Es wurden 68.7 mg (66% d. Th., 100% Reinheit) der Titelverbindung erhalten.

LC-MS (Methode 3): $R_t$ = 2.03 min; MS (ESIpos): m/z = 528 $[M+H]^+$
$^1H$ NMR (400 MHz, DMSO-$d_6$): δ ppm = 10.41 (d, 1H), 9.00 (s, 1H), 8.58 (d, 1H), 8.44 (d, 1H), 7.67 (s, 1H), 7.58 (t, 2H), 4.73-4.83 (m, 1H), 3.55-3.63 (m, 2H), 3.32-3.38 (m, 2H), 2.21-2.30 (m, 1H), 1.04 (d, 3H), 0.97 (d, 3H).

57.0 mg der Titelverbindung (Racemat) wurde durch chirale HPLC in die Enantiomere getrennt (präparative HPLC: Säule Daicel Chiralpak IE 5 μm 250x20 mm; Eluent: 70% *n*-Heptan, 30% Iso-Propanol; Temperatur: 23°C; Fluss: 20 ml/min; UV-Detektion: 257 nm.)

Man erhielt (in der Reihenfolge der Elution von der Säule) 23.1 mg Enantiomer 1 (99% de) Rt = 14.57 min und 24.4 mg (96% de) Enantiomer 2 Rt = 18.53 min.

[Analytische HPLC: Säule Daicel Chiralpak IF-3 3 μm 50x4.6 mm; Eluent: 80% *n*-Heptan, 20% Iso-propanol; Temperatur: 30°C; Fluss: 1.0 ml/min; UV-Detektion: 255 nm].

Enantiomer 1 wurde zusätzlich mittels präparativer HPLC (Säule: Chromatorex C18, 10 μm, 125*40 mm, Lösungsmittel: Acetonitril, Wasser, 0.1% Ameisensäure) gereinigt und 11.0 mg (11% d. Th., 100% Reinheit) der Titelverbindung aus Beispiel 537 erhalten.

Enantiomer 2 wurde zusätzlich mittels präparativer HPLC (Säule: Chromatorex C18, 10 μm, 125*40 mm, Lösungsmittel: Acetonitril, Wasser, 0.1% Ameisensäure) gereinigt und 12.9 mg (12% d. Th., 100% Reinheit) der Titelverbindung aus Beispiel 538 erhalten.

## Beispiel 537

4-Oxo-7-(2-oxoimidazolidin-1-yl)-*N*-[1,1,1-trifluor-3-methylbutan-2-yl]-1-(2,4,6-trifluorphenyl)-1,4-dihydro-1,8-naphthyridin-3-carboxamid (Enantiomer 1)

LC-MS (Methode 3): $R_t$ = 2.01 min; MS (ESIpos): m/z = 528 $[M+H]^+$
$^1H$ NMR (400 MHz, DMSO-$d_6$): δ ppm = 10.42 (d, 1H), 9.00 (s, 1H), 8.59 (d, 1H), 8.44 (d, 1H), 7.67 (s, 1H), 7.58 (t, 2H), 4.73-4.84 (m, 1H), 3.60 (t, 2H), 3.35 (brt, 2H), 2.21-2.30 (m, 1H), 1.04 (d, 3H), 0.97 (d, 3H).

## Beispiel 538

4-Oxo-7-(2-oxoimidazolidin-1-yl)-*N*-[1,1,1-trifluor-3-methylbutan-2-yl]-1-(2,4,6-trifluorphenyl)-1,4-dihydro-1,8-naphthyridin-3-carboxamid (Enantiomer 2)

LC-MS (Methode 3): $R_t$ = 2.01 min; MS (ESIpos): m/z = 528 $[M+H]^+$
$^1H$ NMR (400 MHz, DMSO-$d_6$): δ ppm = 10.41 (d, 1H), 9.00 (s, 1H), 8.59 (d, 1H), 8.44 (d, 1H), 7.67 (s, 1H), 7.58 (t, 2H), 4.72-4.84 (m, 1H), 3.56-3.63 (m, 2H), 3.31-3.39 (m, 2H), 2.21-2.30 (m, 1H), 1.04 (d, 3H), 0.97 (d, 3H).

## Beispiel 539

*N*-[(1*S*)-1-Cyclopropyl-2,2,2-trifluorethyl]-7-(3-ethyl-2-oxotetrahydropyrimidin-1(2*H*)-yl)-4-oxo-1-(2,4,6-trifluorphenyl)-1,4-dihydro-1,8-naphthyridin-3-carboxamid

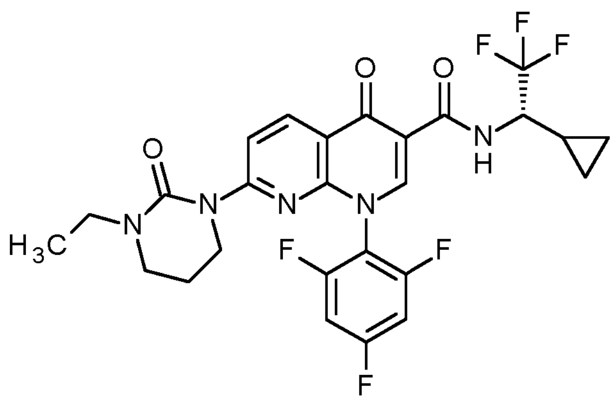

Gemäß AAV2 wurden 60.0 mg (126 μmol) der Verbindung aus Beispiel 126A mit 19.4 mg (151 μmol) 1-Ethyltetrahydropyrimidin-2(1*H*)-on in Gegenwart von 26.1 mg (189 μmol) Kaliumcarbonat, 5.66 mg (25.2 μmol) Palla-

diumacetat und 14.6 mg (25.2 µmol) Xantphos in 600 µl 1,4-Dioxan zur Reaktion gebracht. Das Rohprodukt wurde mittels präparativer HPLC (Acetonitril / Wasser/ 0,1 % Ameisensäure) gereinigt. Es wurden 25.0 mg (35% d. Th., 100% Reinheit) der Titelverbindung erhalten.
LC-MS (Methode 1): $R_t$ = 1.20 min; MS (ESIpos): m/z = 568 $[M+H]^+$
$^1H$ NMR (400 MHz, DMSO-$d_6$): δ ppm = 10.33 (d, 1H), 9.00 (s, 1H), 8.51 (d, 1H), 8.18 (d, 1H), 7.56-7.62 (m, 2H), 4.31-4.44 (m, 1H), 3.51 (t, 2H), 3.32-3.39 (m, 3H), 1.87-1.94 (m, 2H), 1.18-1.27 (m, 1H), 1.08 (t, 3H), 0.53-0.70 (m, 3H), 0.30-0.38 (m, 1H).

**Beispiel 540**

4-Oxo-7-(2-oxoimidazolidin-1-yl)-*N*-[3,3,4,4,4-pentafluorbutan-2-yl]-1-(2,4,6-trifluorphenyl)-1,4-dihydro-1,8-naphthyridin-3-carboxamid (Racemat)

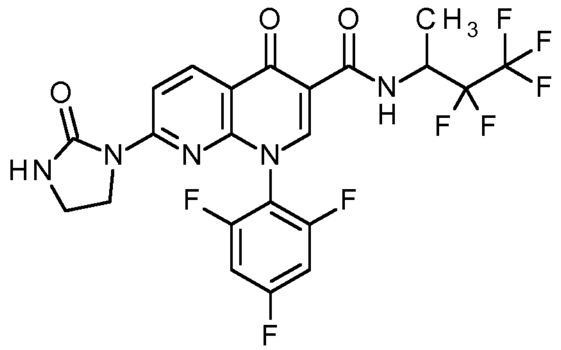

Gemäß AAV1 wurden 500 mg (1.24 mmol) der Verbindung aus Beispie 113A mit 282 mg (1.73 mmol) der Verbindung aus Beispiel 147B in Gegenwart von 564 mg (1.48 mmol) HATU und 650 µl (3.70 mmol) DIPEA in 4.6 ml DMF zur Reaktion gebracht. Das Rohprodukt wurde mittels präparativer HPLC (Acetonitril / Wasser/ 0,1 % Ameisensäure) gereinigt. Es wurden 361 mg (53% d. Th., 100% Reinheit) der Titelverbindung erhalten.
LC-MS (Methode 3): $R_t$ = 1.97 min; MS (ESIpos): m/z = 550 $[M+H]^+$
$^1H$ NMR (400 MHz, DMSO-$d_6$): δ ppm = 10.33 (d, 1H), 9.00 (s, 1H), 8.55 (d, 1H), 8.44 (d, 1H), 7.67 (s, 1H), 7.54-7.61 (m, 2H), 4.97-5.11 (m, 1H), 3.54-3.65 (m, 2H), 3.32-3.38 (m, 2H), 1.41 (d, 3H).

358 mg der Titelverbindung (Racemat) wurde durch chirale SFC in die Enantiomere getrennt (präparative SFC: Säule Daicel Chiralcel OD-H 5 µm 250x20 mm; Eluent: 88% Kohlendioxid, 12% Iso-Propanol; Temperatur: 40°C; Fluss: 80 ml/min; UV-Detektion: 210 nm.)

Man erhielt (in der Reihenfolge der Elution von der Säule) 111 mg (16% d. Th., 100% Reinheit) Enantiomer 1 aus Beispiel 541 (99% ee) Rt = 11.45 min und 124 mg (18% d. Th., 100% Reinheit) Enantiomer 2 aus Beispiel 542 (99% ee) Rt = 13.60 min.

[Analytische SFC: Säule OD; Eluent: 80% Kohlendioxid, 20% Iso-propanol; Fluss: 3.0 ml/min; UV-Detektion: 210 nm].

**Beispiel 541**

4-Oxo-7-(2-oxoimidazolidin-1-yl)-*N*-[3,3,4,4,4-pentafluorbutan-2-yl]-1-(2,4,6-trifluorphenyl)-1,4-dihydro-1,8-naphthyridin-3-carboxamid (Enantiomer 1)

LC-MS (Methode 3): $R_t$ = 1.97 min; MS (ESIpos): m/z = 550 $[M+H]^+$
$^1H$ NMR (400 MHz, DMSO-$d_6$): δ ppm = 10.33 (d, 1H), 9.00 (s, 1H), 8.55 (d, 1H), 8.44 (d, 1H), 7.67 (s, 1H), 7.54-7.61 (m, 2H), 4.97-5.10 (m, 1H), 3.55-3.63 (m, 2H), 3.32-3.38 (m, 2H), 1.41 (d, 3H).

**Beispiel 542**

4-Oxo-7-(2-oxoimidazolidin-1-yl)-*N*-[3,3,4,4,4-pentafluorbutan-2-yl]-1-(2,4,6-trifluorphenyl)-1,4-dihydro-1,8-naphthyridin-3-carboxamid (Enantiomer 2)

LC-MS (Methode 3): $R_t$ = 1.96 min; MS (ESIpos): m/z = 550 $[M+H]^+$
$^1H$ NMR (400 MHz, DMSO-$d_6$): $\delta$ ppm = 10.33 (d, 1H), 9.00 (s, 1H), 8.55 (d, 1H), 8.44 (d, 1H), 7.67 (s, 1H), 7.54-7.61 (m, 2H), 4.97-5.10 (m, 1H), 3.55-3.63 (m, 2H), 3.32-3.38 (m, 2H), 1.41 (d, 3H).

**Beispiel 543**

4-Oxo-7-(2-oxoimidazolidin-1-yl)-*N*-[(2*S*)-1,1,1-trifluor-4-methylpentan-2-yl]-1-(2,4,6-trifluorphenyl)-1,4-dihydro-1,8-naphthyridin-3-carboxamid

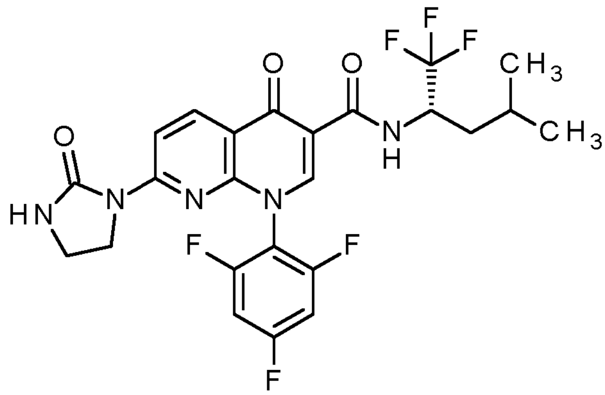

Gemäß AAV1 wurden 26.0 mg (64.3 $\mu$mol) der Verbindung aus Beispiel 113A mit 12.0 mg (77.2 $\mu$mol) (2*S*)-1,1,1-Trifluor-4-methylpentan-2-amin in Gegenwart von 29.3 mg (77.2 $\mu$mol) HATU und 34 $\mu$l (190 $\mu$mol) DIPEA in 250 $\mu$l DMF zur Reaktion gebracht. Das Rohprodukt wurde mittels präparativer HPLC (Acetonitril / Wasser/ 0,1 % Ameisensäure) gereinigt. Es wurden 22.6 mg (65% d. Th., 100% Reinheit) der Titelverbindung erhalten.
LC-MS (Methode 3): $R_t$ = 2.16 min; MS (ESIpos): m/z = 542 $[M+H]^+$
$^1H$ NMR (400 MHz, DMSO-$d_6$): $\delta$ ppm = 10.20 (d, 1H), 9.00 (s, 1H), 8.55 (d, 1H), 8.44 (d, 1H), 7.67 (s, 1H), 7.54-7.61 (m, 2H), 4.78-4.89 (m, 1H), 3.55-3.63 (m, 2H), 3.34-3.40 (m, 2H), 1.54-1.73 (m, 3H), 0.95 (d, 3H), 0.90 (d, 3H).

**Beispiel 544**

7-[(3*R*,4*R*)-3,4-Dihydroxypyrrolidin-1-yl]-4-oxo-*N*-[(2*R*)-1,1,1-trifluor-4-methylpentan-2-yl]-1-(2,4,6-trifluorphenyl)-1,4-dihydro-1,8-naphthyridin-3-carboxamid

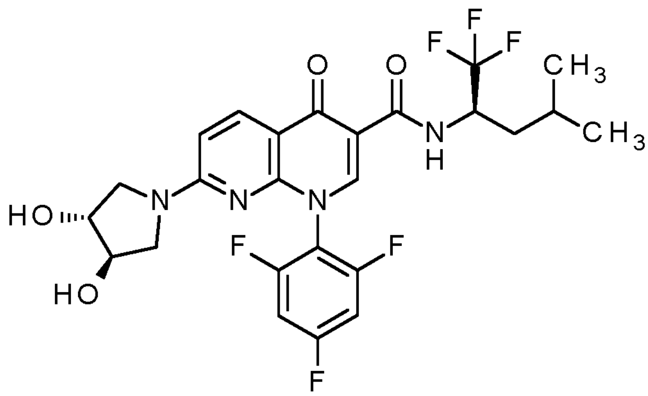

Gemäß AAV1 wurden 50.0 mg (119 µmol) der Verbindung aus Beispiel 121A mit 22.1 mg (142 µmol) (2*R*)-1,1,1-Trifluor-4-methylpentan-2-amin in Gegenwart von 54.1 mg (142 µmol) HATU und 62 µl (360 µmol) DIPEA in 750 µl DMF zur Reaktion gebracht. Das Rohprodukt wurde mittels präparativer HPLC (Acetonitril / Wasser/ 0,1 % Ameisensäure) gereinigt. Es wurden 47.5 mg (67% d. Th., 94% Reinheit) der Titelverbindung erhalten.
LC-MS (Methode 3): $R_t$ = 1.93 min; MS (ESIpos): m/z = 559 $[M+H]^+$
$^1$H NMR (400 MHz, DMSO-$d_6$): δ ppm = 10.42 (d, 1H), 8.81 (s, 1H), 8.27 (d, 1H), 7.53-7.60 (m, 2H), 6.78 (d, 1H), 5.23 (d, 1H), 5.15 (d, 1H), 4.77-4.88 (m, 1H), 4.05 (br s, 1H), 3.93 (br s, 1H), 3.62 (br dd, 1H), 3.34 (br d, 1H), 3.25 (br dd, 1H), 3.07 (br d, 1H), 1.52-1.72 (m, 3H), 0.95 (d, 3H), 0.89 (d, 3H).

## Beispiel 545

7-[(3*R*,4*R*)-3,4-Dihydroxypyrrolidin-1-yl]-*N*-[1,1,1,4,4,4-hexafluorbutan-2-yl]-4-oxo-1-(2,4,6-trifluorphenyl)-1,4-dihydro-1,8-naphthyridin-3-carboxamid (Diastereomerengemisch)

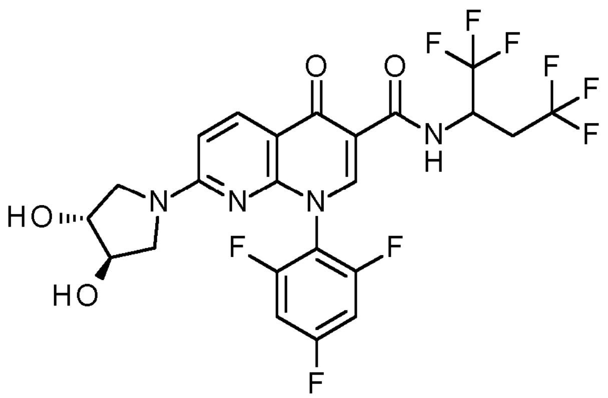

Gemäß AAV1 wurden 40.0 mg (94.9 µmol) der Verbindung aus Beispiel 121A mit 28.9 mg (133 µmol) 1,1,1,4,4,4-Hexafluorbutan-2-amin Hydrochlorid in Gegenwart von 43.3 mg (114 µmol) HATU und 50 µl (280 µmol) DIPEA in 350 µl DMF zur Reaktion gebracht. Das Rohprodukt wurde mittels präparativer HPLC (Acetonitril / Wasser/ 0,1 % Ameisensäure) gereinigt. Es wurden 32.0 mg (58% d. Th., 100% Reinheit) der Titelverbindung erhalten.
LC-MS (Methode 3): $R_t$ = 1.70 min; MS (ESIpos): m/z = 585 $[M+H]^+$
$^1$H NMR (400 MHz, DMSO-$d_6$): δ ppm = 10.71 (d, 1H), 8.83 (s, 1H), 8.27 (d, 1H), 7.53-7.60 (m, 2H), 6.78 (d, 1H), 5.10-5.28 (m, 2H), 4.03-4.06 (m, 1H), 3.91-3.94 (m, 1H), 3.58-3.64 (m, 1H), 3.34-3.39 (m, 1H), 3.20-3.28 (m, 2H), 3.02-3.14 (m, 2H), 2.91-3.01 (m, 1H).

## Beispiel 546

1-(2-Chlor-4,6-difluorphenyl)-*N*-[(1*S*)-1-cyclopropyl-2,2,2-trifluorethyl]-7-[(3*R*,4*R*)-3,4-dihydroxypyrrolidin-1-yl]-4-oxo-1,4-dihydro-1,8-naphthyridin-3-carboxamid (Atropisomerengemisch)

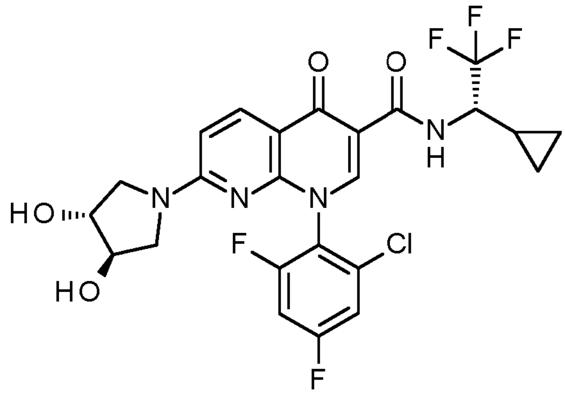

Gemäß AAV3 wurden 50.0 mg (102 µmol) der Verbindung aus Beispiel 110A mit 17.0 mg (122 µmol) (3*R*,4*R*)-Pyrrolidin-3,4-diol Hydrochlorid und 62 µl (360 µmol) DIPEA in 500 µl DMF zur Reaktion gebracht. Das Rohprodukt wurde mittels präparativer HPLC (Acetonitril / Wasser/ 0,1 % Ameisensäure) gereinigt. Es wurden 51.6 mg (91% d. Th., 100% Reinheit) der Titelverbindung erhalten.
LC-MS (Methode 3): $R_t$ = 1.76 min; MS (ESIpos): m/z = 559 $[M+H]^+$
$^1H$ NMR (400 MHz, DMSO-$d_6$): δ ppm = 10.59 (d, 1H), 8.74 (s, 1H), 8.28 (d, 1H), 7.66-7.76 (m, 2H), 6.78 (d, 1H), 5.20-5.26 (m, 1H), 5.12-5.17 (m, 1H), 4.32-4.43 (m, 1H), 4.01-4.08 (m, 1H), 3.88-3.95 (m, 1H), 3.61 (br dd, 1H), 3.33-3.37 (m, 1H), 3.16-3.26 (m, 1H), 2.97-3.08 (m, 1H), 1.14-1.25 (m, 1H), 0.49-0.69 (m, 3H), 0.34 (dt, 1H).

30.0 mg der Titelverbindung (Atropisomerengemisch) wurde durch chirale HPLC in die Atropisomere getrennt (präparative HPLC: Säule Daicel Chiralcel OX-H 5 µm 250x20 mm; Eluent: 75% *n*-Heptan, 25% Iso-Propanol; Temperatur: 40°C; Fluss: 15 ml/min; UV-Detektion: 220 nm.)

Man erhielt (in der Reihenfolge der Elution von der Säule) 14.0 mg (25% d. Th., 100% Reinheit) Atropisomer 1 aus Beispiel 547 (99% de) Rt = 6.08 min und 13.0 mg (23% d. Th., 100% Reinheit) Atropisomer 2 aus Beispiel 548 (99% de) Rt = 8.82 min.

[Analytische HPLC: Säule Daicel Chiralcel OX-H 5 µm 250x4.6 mm; Eluent: 70% Iso-Hexan, 30% Iso-Propanol; Temperatur: 35°C; Fluss: 1.0 ml/min; UV-Detektion: 220nm].

## Beispiel 547

1-(2-Chlor-4,6-difluorphenyl)-*N*-[(1*S*)-1-cyclopropyl-2,2,2-trifluorethyl]-7-[(3*R*,4*R*)-3,4-dihydroxypyrrolidin-1-yl]-4-oxo-1,4-dihydro-1,8-naphthyridin-3-carboxamid (Atropisomer 1)

LC-MS (Methode 3): $R_t$ = 1.77 min; MS (ESIpos): m/z = 559 $[M+H]^+$
$^1H$ NMR (400 MHz, DMSO-$d_6$): δ ppm = 10.59 (d, 1H), 8.74 (s, 1H), 8.28 (d, 1H), 7.66-7.76 (m, 2H), 6.78 (d, 1H), 5.19-5.26 (m, 1H), 5.10-5.19 (m, 1H), 4.33-4.44 (m, 1H), 4.00-4.07 (m, 1H), 3.87-3.96 (m, 1H), 3.61 (br dd, 1H), 3.32-3.36 (m, 1H), 3.17-3.25 (m, 1H), 3.01 (br d, 1H), 1.15-1.28 (m, 1H), 0.49-0.69 (m, 3H), 0.29-0.38 (m, 1H).

## Beispiel 548

1-(2-Chlor-4,6-difluorphenyl)-*N*-[(1*S*)-1-cyclopropyl-2,2,2-trifluorethyl]-7-[(3*R*,4*R*)-3,4-dihydroxypyrrolidin-1-yl]-4-oxo-1,4-dihydro-1,8-naphthyridin-3-carboxamid (Atropisomer 2)

LC-MS (Methode 3): $R_t$ = 1.77 min; MS (ESIpos): m/z = 559 $[M+H]^+$
$^1H$ NMR (400 MHz, DMSO-$d_6$): δ ppm = 10.59 (d, 1H), 8.74 (s, 1H), 8.28 (d, 1H), 7.66-7.77 (m, 2H), 6.78 (d, 1H), 5.19-5.25 (m, 1H), 5.12-5.19 (m, 1H), 4.32-4.42 (m, 1H), 4.04 (br s, 1H), 3.91 (br s, 1H), 3.61 (br dd, 1H), 3.33-3.37 (m, 1H), 3.20 (br dd, 1H), 3.04 (br d, 1H), 1.16-1.26 (m, 1H), 0.50-0.70 (m, 3H), 0.31-0.39 (m, 1H).

## Beispiel 549

7-[(3*R*,4*R*)-3,4-Dihydroxypyrrolidin-1-yl]-4-oxo-*N*-[3,3,4,4,4-pentafluorbutan-2-yl]-1-(2,4,6-trifluorphenyl)-1,4-dihydro-1,8-naphthyridin-3-carboxamid (Diastereomerengemisch)

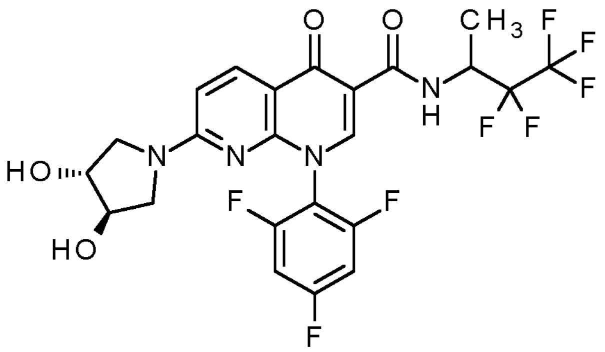

Gemäß AAV1 wurden 500 mg (1.19 mmol) der Verbindung aus Beispiel 121A mit 260 mg (1.31 mmol) der Verbindung aus Beispiel 147B in Gegenwart von 541 mg (1.42 mmol) HATU und 830 µl (4.70 mmol) DIPEA in 5.0 ml DMF zur Reaktion gebracht. Das Rohprodukt wurde mittels präparativer HPLC (Acetonitril / Wasser/ 0,1 % Ameisensäure) gereinigt. Es wurden 399 mg (59% d. Th., 100% Reinheit) der Titelverbindung erhalten.
LC-MS (Methode 1): $R_t$ = 0.93 min; MS (ESIpos): m/z = 567 $[M+H]^+$
$^1$H NMR (500 MHz, DMSO-$d_6$): δ ppm = 10.56 (d, 1H), 8.80 (s, 1H), 8.27 (d, 1H), 7.53-7.59 (m, 2H), 6.78 (d, 1H), 5.23 (t, 1H), 5.14 (t, 1H), 4.96-5.07 (m, 1H), 4.05 (br s, 1H), 3.93 (br s, 1H), 3.58-3.65 (m, 1H), 3.32-3.37 (m, 1H), 3.25 (dt, 1H), 3.07 (br d, 1H), 1.39 (d, 3H).

395 mg der Titelverbindung (Diastereomerengemisch) wurde durch chirale HPLC in die Diastereomere getrennt (präparative HPLC: Säule Daicel Chiralpak AD-H 5 µm 250x20 mm; Eluent: 80% *n*-Heptan, 20% Iso-Propanol; Temperatur: 23°C; Fluss: 20 ml/min; UV-Detektion: 220 nm.)

Man erhielt (in der Reihenfolge der Elution von der Säule) 164.7 mg (24% d. Th., 100% Reinheit) Diastereomer 1 aus Beispiel 550 (99% de) Rt = 4.52 min und 163.7 mg (24% d. Th., 100% Reinheit) Diastereomer 2 aus Beispiel 551 (97% de) Rt = 6.81 min.

[Analytische HPLC: Säule Daicel Chiralpak AD-3 3 µm 50x4.6 mm; Eluent: 80% *n*-Heptan, 20% Iso-Propanol; Fluss: 1.0 ml/min; UV-Detektion: 220nm].

**Beispiel 550**

7-[(3*R*,4*R*)-3,4-Dihydroxypyrrolidin-1-yl]-4-oxo-*N*-[3,3,4,4,4-pentafluorbutan-2-yl]-1-(2,4,6-trifluorphenyl)-1,4-dihydro-1,8-naphthyridin-3-carboxamid (Diastereomer 1)

LC-MS (Methode 3): $R_t$ = 1.72 min; MS (ESIpos): m/z = 567 $[M+H]^+$
$^1$H NMR (400 MHz, DMSO-$d_6$): δ ppm = 10.56 (d, 1H), 8.81 (s, 1H), 8.27 (d, 1H), 7.53-7.60 (m, 2H), 6.78 (d, 1H), 5.23 (d, 1H), 5.15 (d, 1H), 4.95-5.09 (m, 1H), 4.05 (br s, 1H), 3.93 (br s, 1H), 3.62 (dd, 1H), 3.33 (br d, 1H), 3.24 (dd, 1H), 3.07 (br d, 1H), 1.39 (d, 3H).

**Beispiel 551**

7-[(3*R*,4*R*)-3,4-Dihydroxypyrrolidin-1-yl]-4-oxo-*N*-[3,3,4,4,4-pentafluorbutan-2-yl]-1-(2,4,6-trifluorphenyl)-1,4-dihydro-1,8-naphthyridin-3-carboxamid (Diastereomer 2)

LC-MS (Methode 3): $R_t$ = 1.72 min; MS (ESIpos): m/z = 567 $[M+H]^+$
$^1$H NMR (400 MHz, DMSO-$d_6$): δ ppm = 10.56 (d, 1H), 8.81 (s, 1H), 8.27 (d, 1H), 7.52-7.60 (m, 2H), 6.78 (d, 1H), 5.24 (d, 1H), 5.14 (d, 1H), 4.96-5.09 (m, 1H), 4.05 (br s, 1H), 3.93 (br s, 1H), 3.61 (dd, 1H), 3.32-3.37 (m, 1H), 3.25 (dd, 1H), 3.07 (d, 1H), 1.39 (d, 3H).

**Beispiel 552**

7-[(3*R*,4*R*)-3,4-Dihydroxypyrrolidin-1-yl]-4-oxo-*N*-[1,1,1-trifluor-3-methylbutan-2-yl]-1-(2,4,6-trifluorphenyl)-1,4-dihydro-1,8-naphthyridin-3-carboxamid (Diastereomerengemisch)

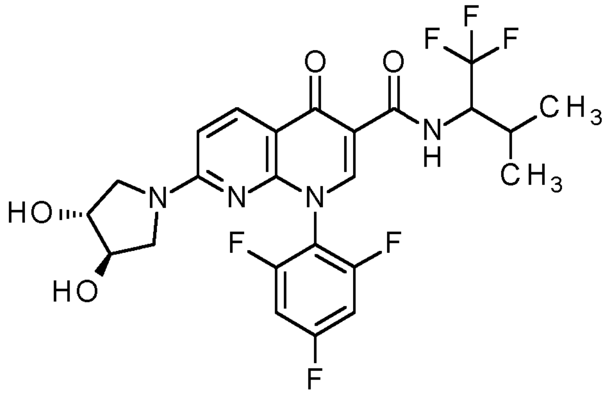

Gemäß AAV1 wurden 80 mg (190 µmol) der Verbindung aus Beispiel 121A mit 32.2 mg (228 µmol) 1,1,1-Trifluor-3-methylbutan-2-amin in Gegenwart von 86.6 mg (228 µmol) HATU und 99 µl (570 µmol) DIPEA in 1.4 ml DMF zur Reaktion gebracht. Das Rohprodukt wurde mittels präparativer HPLC (Acetonitril / Wasser/ 0,1 % Ameisensäure) gereinigt. Es wurden 73.4 mg (71% d. Th., 100% Reinheit) der Titelverbindung erhalten.
LC-MS (Methode 3): $R_t$ = 1.76 min; MS (ESIpos): m/z = 545 $[M+H]^+$
$^1$H NMR (400 MHz, DMSO-$d_6$): δ ppm = 10.62 (d, 1H), 8.81 (s, 1H), 8.30 (d, 1H), 7.57 (br t, 2H), 6.78 (d, 1H), 5.24 (t, 1H), 5.14 (t, 1H), 4.71-4.81 (m, 1H), 4.05 (br s, 1H), 3.93 (br s, 1H), 3.62 (br dd, 1H), 3.32-3.37 (m, 1H), 3.25 (br dd, 1H), 3.07 (br d, 1H), 2.19-2.29 (m, 1H), 1.03 (d, 3H), 0.96 (d, 3H).

51.0 mg der Titelverbindung (Diastereomerengemisch) wurde durch chirale HPLC in die Diastereomere getrennt (präparative HPLC: Säule Daicel Chiralpak IF 5 µm 250x20 mm; Eluent: 80% *n*-Heptan, 20% Iso-Propanol; Temperatur: 23°C; Fluss: 20 ml/min; UV-Detektion: 270 nm.)

Man erhielt (in der Reihenfolge der Elution von der Säule) 7.40 mg Diastereomer 1 (99% de) Rt = 7.55 min und 7.30 mg Diastereomer 2 (96% de) Rt = 9.20 min.

[Analytische HPLC: Säule Daicel Chiralpak IE-3 3 µm 50x4.6 mm; Eluent: 80% *n*-Heptan, 20% Iso-Propanol; Temperatur: 30°C; Fluss: 1.0 ml/min; UV-Detektion: 220nm].

Diastereomer 1 wurde zusätzlich mittels präparativer HPLC (Säule: Chromatorex C18, 10 µm, 125*40 mm, Lösungsmittel: Acetonitril, Wasser, 0.1% Ameisensäure) gereinigt und 2.40 mg (2.3% d. Th., 100% Reinheit) der Titelverbindung aus Beispiel 553 erhalten.

Diastereomer 2 wurde zusätzlich mittels präparativer HPLC (Säule: Chromatorex C18, 10 µm, 125*40 mm, Lösungsmittel: Acetonitril, Wasser, 0.1% Ameisensäure) gereinigt und 2.30 mg (2.2% d. Th., 100% Reinheit) der Titelverbindung aus Beispiel 554 erhalten.

## Beispiel 553

7-[(3*R*,4*R*)-3,4-Dihydroxypyrrolidin-1-yl]-4-oxo-*N*-[1,1,1-trifluor-3-methylbutan-2-yl]-1-(2,4,6-trifluorphenyl)-1,4-dihydro-1,8-naphthyridin-3-carboxamid (Diastereomer 1)

LC-MS (Methode 3): $R_t$ = 1.77 min; MS (ESIpos): m/z = 545 $[M+H]^+$
$^1$H NMR (400 MHz, DMSO-$d_6$): δ ppm = 10.62 (d, 1H), 8.81 (s, 1H), 8.30 (d, 1H), 7.53-7.60 (m, 2H), 6.78 (d, 1H), 5.23 (d, 1H), 5.15 (d, 1H), 4.71-4.82 (m, 1H), 4.05 (br s, 1H), 3.93 (br s, 1H), 3.62 (br dd, 1H), 3.32-3.37 (m, 1H), 3.25 (br dd, 1H), 3.07 (d, 1H), 2.19-2.29 (m, 1H), 1.03 (d, 3H), 0.97 (d, 3H).

## Beispiel 554

7-[(3*R*,4*R*)-3,4-Dihydroxypyrrolidin-1-yl]-4-oxo-*N*-[1,1,1-trifluor-3-methylbutan-2-yl]-1-(2,4,6-trifluorphenyl)-1,4-dihydro-1,8-naphthyridin-3-carboxamid (Diastereomer 2)

LC-MS (Methode 3): $R_t$ = 1.77 min; MS (ESIpos): m/z = 545 $[M+H]^+$
$^1$H NMR (500 MHz, DMSO-$d_6$): δ ppm = 10.62 (br d, 1H), 8.81 (s, 1H), 8.30 (br d, 1H), 7.56 (br t, 2H), 6.78 (br d, 1H), 5.19-5.26 (m, 1H), 5.12-5.19 (m, 1H), 4.72-4.81 (m, 1H), 4.05 (br s, 1H), 3.93 (br s, 1H), 3.57-3.66 (m, 1H), 3.33-3.38 (m, 1H), 3.22-3.27 (m, 1H), 3.07 (br d, 1H), 2.19-2.29 (m, 1H), 1.03 (br d, 3H), 0.97 (br d, 3H).

**Beispiel 555**

*N*-(1,1,1,3,3,3-Hexafluorpropan-2-yl)-7-[(4*S*)-4-hydroxy-2-oxopyrrolidin-1-yl]-4-oxo-1-(2,4,6-trifluorphenyl)-1,4-dihydro-1,8-naphthyridin-3-carboxamid

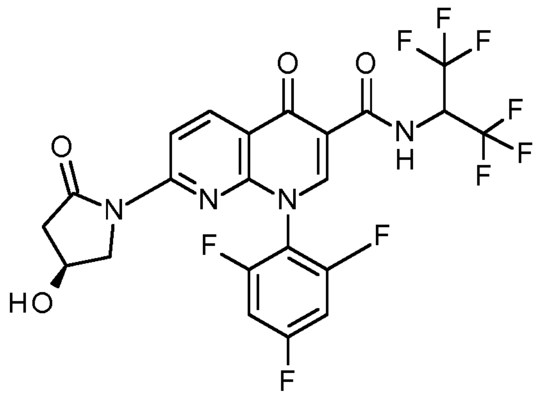

Gemäß AAV2 wurden 60.0 mg (119 μmol) der Verbindung aus Beispiel 154A mit 14.5 mg (143 μmol) (4*S*)-4-Hydroxypyrrolidin-2-on in Gegenwart von 24.7 mg (179 μmol) Kaliumcarbonat, 5.35 mg (23.8 μmol) Palladiumacetat und 13.8 mg (23.8 μmol) Xantphos in 1.2 ml 1,4-Dioxan zur Reaktion gebracht. Das Rohprodukt wurde mittels präparativer HPLC (Acetonitril / Wasser/ 0,1 % Ameisensäure) gereinigt. Es wurden 42.3 mg (62% d. Th., 100% Reinheit) der Titelverbindung erhalten.
LC-MS (Methode 3): $R_t$ = 1.99 min; MS (ESIpos): m/z = 569 $[M+H]^+$
$^1H$ NMR (400 MHz, DMSO-$d_6$): δ ppm = 11.03 (d, 1H), 9.20 (s, 1H), 8.74 (d, 1H), 8.56 (d, 1H), 7.59-7.67 (m, 2H), 6.33-6.43 (m, 1H), 5.34 (d, 1H), 4.27-4.32 (m, 1H), 3.69 (dd, 1H), 3.48 (d, 1H), 2.95 (dd, 1H), 2.38 (d, 1H).

**Beispiel 556**

1-(2,4-Difluorphenyl)-7-[(4*S*)-4-hydroxy-2-oxopyrrolidin-1-yl]-4-oxo-*N*-[1,1,1,2,2-pentafluorpentan-3-yl]-1,4-dihydro-1,8-naphthyridin-3-carboxamid (Diastereomerengemisch)

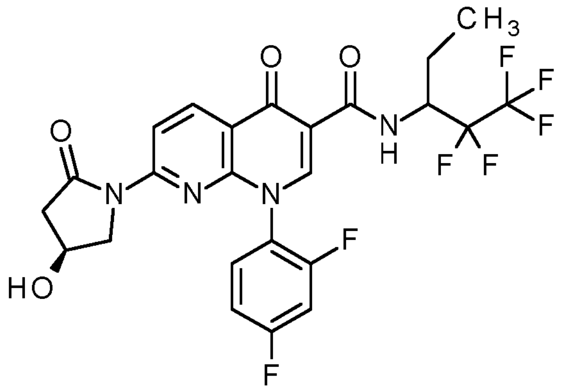

Gemäß AAV1 wurden 100 mg (249 μmol) der Verbindung aus Beispiel 63A mit 63.9 mg (299 μmol) der Verbindung aus Beispiel 153B in Gegenwart von 114 mg (299 μmol) HATU und 170 μl (1000 μmol) DIPEA in 1.0 ml DMF zur Reaktion gebracht. Das Rohprodukt wurde mittels präparativer HPLC (Acetonitril / Wasser/ 0,1 % Ameisensäure) gereinigt. Es wurden 123 mg (88% d. Th., 100% Reinheit) der Titelverbindung erhalten.
LC-MS (Methode 3): $R_t$ = 1.96 min; MS (ESIpos): m/z = 561 $[M+H]^+$
$^1H$ NMR (400 MHz, DMSO-$d_6$): δ ppm = 10.27 (d, 1H), 8.86 (d, 1H), 8.71 (d, 1H), 8.52 (br dd, 1H), 7.83-7.94 (m, 1H), 7.63 (br t, 1H), 7.37 (br t, 1H), 5.27-5.37 (m, 1H), 4.82-4.96 (m, 1H), 4.25-4.31 (m, 1H), 3.66 (td, 1H), 3.47 (brt, 1H),

2.88-3.00 (m, 1H), 2.37 (br dd, 1H), 1.88-1.99 (m, 1H), 1.62-1.74 (m, 1H), 0.97 (br t, 3H).

95.0 mg der Titelverbindung (Diastereomerengemisch) wurde durch chirale HPLC in die Diastereomere getrennt (präparative HPLC: Säule Daicel Chiralpak IE 5 µm 250x20 mm; Eluent: 70% *n*-Heptan, 30% Iso-Propanol; Temperatur: 23°C; Fluss: 20 ml/min; UV-Detektion: 220 nm.)

Man erhielt (in der Reihenfolge der Elution von der Säule) 35.1 mg (25% d. Th., 100% Reinheit) Diastereomer 1 aus Beispiel 557 (99% de) Rt = 7.05 min und 34.9 mg (25% d. Th., 100% Reinheit) Diastereomer 2 aus Beispiel 558 (99% de) Rt = 9.72 min.

[Analytische HPLC: Säule Daicel Chiralpak IE-3 3 µm 50x4.6 mm; Eluent: 80% *n*-Heptan, 20% Iso-Propanol; Temperatur: 30°C; Fluss: 1.0 ml/min; UV-Detektion: 220nm].

## Beispiel 557

1-(2,4-Difluorphenyl)-7-[(4*S*)-4-hydroxy-2-oxopyrrolidin-1-yl]-4-oxo-*N*-[1,1,1,2,2-pentafluorpentan-3-yl]-1,4-dihydro-1,8-naphthyridin-3-carboxamid (Diastereomer 1)

LC-MS (Methode 3): $R_t$ = 1.97 min; MS (ESIpos): m/z = 561 $[M+H]^+$
$^1H$ NMR (400 MHz, DMSO-$d_6$): δ ppm = 10.27 (d, 1H), 8.86 (d, 1H), 8.71 (d, 1H), 8.52 (dd, 1H), 7.83-7.94 (m, 1H), 7.59-7.66 (m, 1H), 7.33-7.40 (m, 1H), 5.28-5.37 (m, 1H), 4.83-4.96 (m, 1H), 4.26-4.31 (m, 1H), 3.66 (td, 1H), 3.47 (brt, 1H), 2.89-2.99 (m, 1H), 2.32-2.41 (m, 1H), 1.88-1.98 (m, 1H), 1.62-1.74 (m, 1H), 0.97 (t, 3H).

## Beispiel 558

1-(2,4-Difluorphenyl)-7-[(4*S*)-4-hydroxy-2-oxopyrrolidin-1-yl]-4-oxo-*N*-[1,1,1,2,2-pentafluorpentan-3-yl]-1,4-dihydro-1,8-naphthyridin-3-carboxamid (Diastereomer 2)

LC-MS (Methode 3): $R_t$ = 1.97 min; MS (ESIpos): m/z = 561 $[M+H]^+$
$^1H$ NMR (400 MHz, DMSO-$d_6$): δ ppm = 10.27 (d, 1H), 8.86 (d, 1H), 8.71 (d, 1H), 8.52 (dd, 1H), 7.84-7.92 (m, 1H), 7.63 (br t, 1H), 7.37 (br t, 1H), 5.18-5.43 (m, 1H), 4.82-4.96 (m, 1H), 4.28 (br t, 1H), 3.59-3.72 (m, 1H), 3.42-3.52 (m, 1H), 2.94 (ddd, 1H), 2.32-2.42 (m, 1H), 1.88-1.99 (m, 1H), 1.62-1.74 (m, 1H), 0.97 (t, 3H).

## Beispiel 559

7-[(3*R*,4*R*)-3,4-Dihydroxypyrrolidin-1-yl]-4-oxo-*N*-[(2*S*)-1,1,1-trifluor-4-methylpentan-2-yl]-1-(2,4,6-trifluorphenyl)-1,4-dihydro-1,8-naphthyridin-3-carboxamid

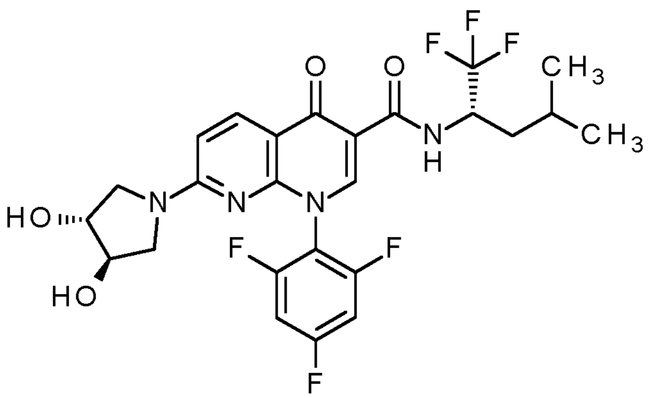

Gemäß AAV1 wurden 50.0 mg (119 µmol) der Verbindung aus Beispiel 121A mit 22.1 mg (142 µmol) (2*S*)-1,1,1-Trifluor-4-methylpentan-2-amin in Gegenwart von 54.1 mg (142 µmol) HATU und 62 µl (360 µmol) DIPEA in 750 µl DMF zur Reaktion gebracht. Das Rohprodukt wurde mittels präparativer HPLC (Acetonitril / Wasser/ 0,1 % Ameisensäure) gereinigt. Es wurden 47.5 mg (72% d. Th., 100% Reinheit) der Titelverbindung erhalten.
LC-MS (Methode 3): $R_t$ = 1.93 min; MS (ESIpos): m/z = 559 $[M+H]^+$
$^1H$ NMR (400 MHz, DMSO-$d_6$): δ ppm = 10.42 (d, 1H), 8.81 (s, 1H), 8.27 (d, 1H), 7.52-7.60 (m, 2H), 6.78 (d, 1H), 5.24 (d, 1H), 5.14 (d, 1H), 4.77-4.88 (m, 1H), 4.05 (br s, 1H), 3.93 (br s, 1H), 3.61 (br dd, 1H), 3.34 (br d, 1H), 3.25 (br dd,

1H), 3.07 (br d, 1H), 1.52-1.72 (m, 3H), 0.95 (d, 3H), 0.89 (d, 3H).

**Beispiel 560**

*N*-[1-(2-Chlorphenyl)-2,2,2-trifluorethyl]-7-[(3*R*,4*R*)-3,4-dihydroxypyrrolidin-1-yl]-4-oxo-1-(2,4,6-trifluorphenyl)-1,4-dihydro-1,8-naphthyridin-3-carboxamid (Diastereomerengemisch)

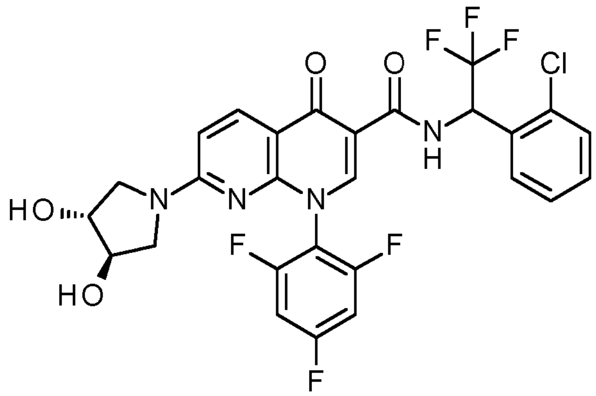

Gemäß AAV1 wurden 50.0 mg (119 μmol) der Verbindung aus Beispiel 121A mit 29.8 mg (142 μmol) 1-(2-Chlorphenyl)-2,2,2-trifluorethanamin in Gegenwart von 54.1 mg (142 μmol) HATU und 62 μl (360 μmol) DIPEA in 460 μl DMF zur Reaktion gebracht. Das Rohprodukt wurde mittels präparativer HPLC (Acetonitril / Wasser/ 0,1 % Ameisensäure) gereinigt. Es wurden 44.9 mg (61% d. Th., 100% Reinheit) der Titelverbindung erhalten.
LC-MS (Methode 3): $R_t$ = 1.97 min; MS (ESIpos): m/z = 613 $[M+H]^+$
$^1H$ NMR (400 MHz, DMSO-$d_6$): δ ppm = 11.56 (d, 1H), 8.83 (s, 1H), 8.32 (d, 1H), 7.48-7.64 (m, 6H), 6.80 (d, 1H), 6.40-6.49 (m, 1H), 5.08-5.30 (m, 2H), 4.03-4.07 (m, 1H), 3.90-3.95 (m, 1H), 3.58-3.65 (m, 1H), 3.03-3.10 (m, 1H), 0.97-1.04 (m, 2H).

37.6 mg der Titelverbindung (Diastereomerengemisch) wurde durch chirale HPLC in die Diastereomere getrennt (präparative HPLC: Säule Daicel Chiralcel OX-H 5 μm 250x20 mm; Eluent: 45% *n*-Heptan, 55% Iso-Propanol; Temperatur: 40°C; Fluss: 15 ml/min; UV-Detektion: 220 nm.)

Man erhielt (in der Reihenfolge der Elution von der Säule) 14.0 mg (19% d. Th., 100% Reinheit) Diastereomer 1 aus Beispiel 561 (99% de) Rt = 4.83 min und 15.0 mg (20% d. Th., 100% Reinheit) Diastereomer 2 aus Beispiel 562 (99% de) Rt = 6.63 min.

[Analytische HPLC: Säule Daicel Chiralcel OX-H 5 μm 250x4.6 mm; Eluent: 50% Iso-Hexan, 50% Iso-Propanol; Temperatur: 45°C; Fluss: 1.0 ml/min; UV-Detektion: 220nm].

**Beispiel 561**

*N*-[1-(2-Chlorphenyl)-2,2,2-trifluorethyl]-7-[(3*R*,4*R*)-3,4-dihydroxypyrrolidin-1-yl]-4-oxo-1-(2,4,6-trifluorphenyl)-1,4-dihydro-1,8-naphthyridin-3-carboxamid (Diastereomer 1)

LC-MS (Methode 3): $R_t$ = 1.95 min; MS (ESIpos): m/z = 613 $[M+H]^+$
$^1H$ NMR (400 MHz, DMSO-$d_6$): δ ppm = 11.56 (d, 1H), 8.83 (s, 1H), 8.32 (d, 1H), 7.48-7.64 (m, 6H), 6.80 (d, 1H), 6.40-6.49 (m, 1H), 5.23 (d, 1H), 5.15 (d, 1H), 4.05 (br s, 1H), 3.90-3.95 (m, 1H), 3.62 (dd, 1H), 3.33-3.38 (m, 1H), 3.24 (br dd, 1H), 3.07 (br d, 1H).

**Beispiel 562**

*N*-[1-(2-Chlorphenyl)-2,2,2-trifluorethyl]-7-[(3*R*,4*R*)-3,4-dihydroxypyrrolidin-1-yl]-4-oxo-1-(2,4,6-trifluorphenyl)-1,4-dihydro-1,8-naphthyridin-3-carboxamid (Diastereomer 2)

LC-MS (Methode 3): $R_t$ = 1.96 min; MS (ESIpos): m/z = 613 $[M+H]^+$
$^1H$ NMR (400 MHz, DMSO-$d_6$): δ ppm = 11.56 (d, 1H), 8.83 (s, 1H), 8.32 (d, 1H), 7.48-7.64 (m, 6H), 6.80 (d, 1H), 6.40-6.49 (m, 1H), 5.24 (d, 1H), 5.14 (d, 1H), 4.05 (br s, 1H), 3.93 (br s, 1H), 3.62 (br dd, 1H), 3.33-3.38 (m, 1H), 3.25

(dd, 1H), 3.06 (d, 1H).

**Beispiel 563**

*N*-[1-(2-Chlorphenyl)-2,2,2-trifluorethyl]-4-oxo-7-(2-oxoimidazolidin-1-yl)-1-(2,4,6-trifluorphenyl)-1,4-dihydro-1,8-naphthyridin-3-carboxamid (Racemat)

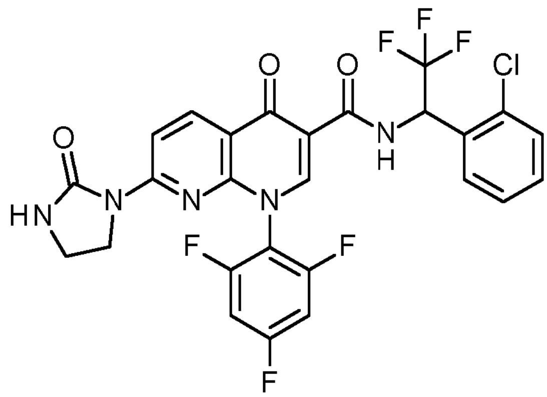

Gemäß AAV1 wurden 50.0 mg (124 μmol) 4-Oxo-7-(2-oxoimidazolidin-1-yl)-1-(2,4,6-trifluorphenyl)-1,4-dihydro-1,8-naphthyridin-3-carbonsäure (Beispiel 113A) mit 31.1 mg (148 μmol) 1-(2-Chlorphenyl)-2,2,2-trifluorethanamin in Gegenwart von 56.4 mg (148 μmol) HATU und 65 μl (370 μmol) DIPEA in 480 μl DMF zur Reaktion gebracht. Das Rohprodukt wurde mittels präparativer HPLC (Acetonitril / Wasser/ 0,1 % Ameisensäure) gereinigt. Es wurden 50.6 mg (69% d. Th., 100% Reinheit) der Titelverbindung erhalten.
LC-MS (Methode 1): $R_t$ = 1.16 min; MS (ESIpos): m/z = 596 $[M+H]^+$
$^1H$ NMR (400 MHz, DMSO-$d_6$): δ ppm = 11.32 (d, 1H), 9.01 (s, 1H), 8.60 (d, 1H), 8.46 (d, 1H), 7.48-7.69 (m, 7H), 6.41-6.50 (m, 1H), 3.55-3.64 (m, 2H), 3.34-3.38 (m, 2H).

40.4 mg der Titelverbindung (Racemat) wurde durch chirale HPLC in die Enantiomere getrennt (präparative HPLC: Säule Daicel Chiralpak AZ-H 5 μm 250x20 mm; Eluent: 25% *n*-Heptan, 75% Iso-Propanol; Temperatur: 25°C; Fluss: 15 ml/min; UV-Detektion: 210 nm.)

Man erhielt (in der Reihenfolge der Elution von der Säule) 19.3 mg Enantiomer 1 (99% ee) Rt = 8.40 min und 16.8 mg Enantiomer 2 (99% ee) Rt = 17.95 min.

[Analytische HPLC: Säule Chiraltek AZ-3 3 μm; Eluent: 50% Iso-Hexan, 50% Iso-propanol; UV-Detektion: 220 nm].

Enantiomer 1 wurde zusätzlich mittels präparativer HPLC (Säule: Chromatorex C18, 10 μm, 125*40 mm, Lösungsmittel: Acetonitril, Wasser, 0.1% Ameisensäure) gereinigt und 11.5 mg (16% d. Th., 97% Reinheit) der Titelverbindung aus Beispiel 564 erhalten.

Enantiomer 2 wurde zusätzlich mittels präparativer HPLC (Säule: Chromatorex C18, 10 μm, 125*40 mm, Lösungsmittel: Acetonitril, Wasser, 0.1% Ameisensäure) gereinigt und 9.30 mg (13% d. Th., 100% Reinheit) der Titelverbindung aus Beispiel 565 erhalten.

**Beispiel 564**

*N*-[1-(2-Chlorphenyl)-2,2,2-trifluorethyl]-4-oxo-7-(2-oxoimidazolidin-1-yl)-1-(2,4,6-trifluorphenyl)-1,4-dihydro-1,8-naphthyridin-3-carboxamid (Enantiomer 1)

LC-MS (Methode 4): $R_t$ = 3.81 min; MS (ESIpos): m/z = 596 $[M+H]^+$
$^1H$ NMR (500 MHz, DMSO-$d_6$): δ ppm = 11.32 (d, 1H), 9.01 (s, 1H), 8.60 (d, 1H), 8.46 (d, 1H), 7.49-7.68 (m, 7H), 6.42-6.49 (m, 1H), 3.56-3.62 (m, 2H), 3.33-3.37 (m, 2H).

**Beispiel 565**

*N*-[1-(2-Chlorphenyl)-2,2,2-trifluorethyl]-4-oxo-7-(2-oxoimidazolidin-1-yl)-1-(2,4,6-trifluorphenyl)-1,4-dihydro-1,8-naphthyridin-3-carboxamid (Enantiomer 2)

LC-MS (Methode 3): $R_t$ = 2.17 min; MS (ESIpos): m/z = 596 $[M+H]^+$
$^1H$ NMR (500 MHz, DMSO-$d_6$): δ ppm = 11.32 (d, 1H), 9.01 (s, 1H), 8.60 (d, 1H), 8.46 (d, 1H), 7.49-7.68 (m, 7H), 6.42-6.49 (m, 1H), 3.56-3.63 (m, 2H), 3.32-3.37 (m, 2H).

**Beispiel 566**

1-(2-Chlor-4,6-difluorphenyl)-7-[(3*R*,4*R*)-3,4-dihydroxypyrrolidin-1-yl]-4-oxo-*N*-[(2*S*)-1,1,1-trifluorbutan-2-yl]-1,4-dihydro-1,8-naphthyridin-3-carboxamid (Atropisomerengemisch)

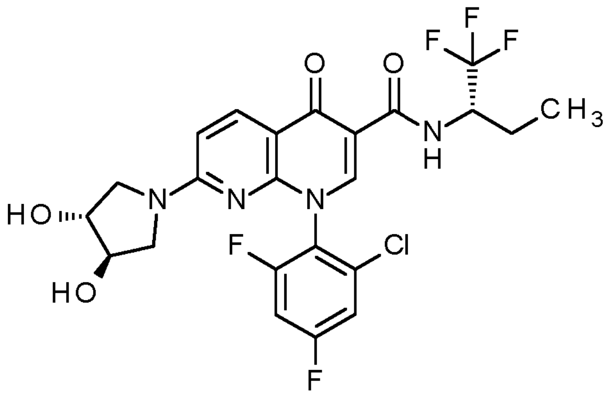

Gemäß AAV3 wurden 50.0 mg (104 µmol) der Verbindung aus Beispiel 108C mit 17.4 mg (125 µmol) (3*R*,4*R*)-Pyrrolidin-3,4-diol Hydrochlorid und 63 µl (360 µmol) DIPEA in 500 µl DMF zur Reaktion gebracht. Das Rohprodukt wurde mittels präparativer HPLC (Acetonitril / Wasser/ 0,1 % Ameisensäure) gereinigt. Es wurden 50.0 mg (88% d. Th., 100% Reinheit) der Titelverbindung erhalten.
LC-MS (Methode 3): $R_t$ = 1.72 min; MS (ESIpos): m/z = 547 $[M+H]^+$
$^1H$ NMR (400 MHz, DMSO-$d_6$): δ ppm = 10.46 (dd, 1H), 8.75 (s, 1H), 8.28 (d, 1H), 7.67-7.76 (m, 2H), 6.77 (d, 1H), 5.23 (br d, 1H), 5.14 (d, 1H), 4.68-4.79 (m, 1H), 4.04 (br s, 1H), 3.92 (br s, 1H), 3.61 (br dd, 1H), 3.32-3.37 (m, 1H), 3.17-3.25 (m, 1H), 3.02 (br dd, 1H), 1.83-1.93 (m, 1H), 1.59-1.71 (m, 1H), 0.94-1.00 (m, 3H).
30.0 mg der Titelverbindung (Atropisomerengemisch) wurde durch chirale HPLC in die Atropisomere getrennt (präparative HPLC: Säule Daicel Chiralcel OX-H 5 µm 250x20 mm; Eluent: 75% *n*-Heptan, 25% Iso-Propanol; Temperatur: 40°C; Fluss: 15 ml/min; UV-Detektion: 220 nm.)
Man erhielt (in der Reihenfolge der Elution von der Säule) 13.0 mg (23% d. Th., 100% Reinheit) Atropisomer 1 aus Beispiel 567 (99% de) Rt = 6.73 min und 10.0 mg (18% d. Th., 100% Reinheit) Atropisomer 2 aus Beispiel 568 (99% de) Rt = 11.48 min.
[Analytische HPLC: Säule Daicel Chiralcel OX-H 5 µm 250x4.6 mm; Eluent: 70% Iso-Hexan, 30% Iso-Propanol; Temperatur: 35°C; Fluss: 1.0 ml/min; UV-Detektion: 220nm].

**Beispiel 567**

1-(2-Chlor-4,6-difluorphenyl)-7-[(3*R*,4*R*)-3,4-dihydroxypyrrolidin-1-yl]-4-oxo-*N*-[(2*S*)-1,1,1-trifluorbutan-2-yl]-1,4-dihydro-1,8-naphthyridin-3-carboxamid (Atropisomer 1)

LC-MS (Methode 3): $R_t$ = 1.73 min; MS (ESIpos): m/z = 547 $[M+H]^+$
$^1H$ NMR (400 MHz, DMSO-$d_6$): δ ppm = 10.46 (d, 1H), 8.75 (s, 1H), 8.28 (d, 1H), 7.67-7.76 (m, 2H), 6.77 (d, 1H), 5.23 (d, 1H), 5.14 (d, 1H), 4.68-4.80 (m, 1H), 4.04 (br s, 1H), 3.92 (br s, 1H), 3.61 (dd, 1H), 3.32-3.37 (m, 1H), 3.22 (br dd, 1H), 3.01 (d, 1H), 1.82-1.94 (m, 1H), 1.58-1.70 (m, 1H), 0.97 (t, 3H).

**Beispiel 568**

1-(2-Chlor-4,6-difluorphenyl)-7-[(3*R*,4*R*)-3,4-dihydroxypyrrolidin-1-yl]-4-oxo-*N*-[(2*S*)-1,1,1-trifluorbutan-2-yl]-1,4-dihydro-1,8-naphthyridin-3-carboxamid (Atropisomer 2)

LC-MS (Methode 3): $R_t$ = 1.73 min; MS (ESIpos): m/z = 547 $[M+H]^+$
$^1H$ NMR (400 MHz, DMSO-$d_6$): $\delta$ ppm = 10.45 (d, 1H), 8.75 (s, 1H), 8.28 (d, 1H), 7.68-7.75 (m, 2H), 6.77 (d, 1H), 5.23 (d, 1H), 5.14 (d, 1H), 4.68-4.79 (m, 1H), 4.04 (br s, 1H), 3.87-3.94 (m, 1H), 3.57-3.65 (m, 1H), 3.32-3.38 (m, 1H), 3.20 (br dd, 1H), 3.04 (br d, 1H), 1.83-1.93 (m, 1H), 1.59-1.71 (m, 1H), 0.98 (t, 3H).

**Beispiel 569**

7-[7-Hydroxy-5-azaspiro[2.4]hept-5-yl]-4-oxo-*N*-(4,4,4-trifluor-2-methylbutan-2-yl)-1-(2,4,6-trifluorphenyl)-1,4-dihydro-1,8-naphthyridin-3-carboxamid (Racemat)

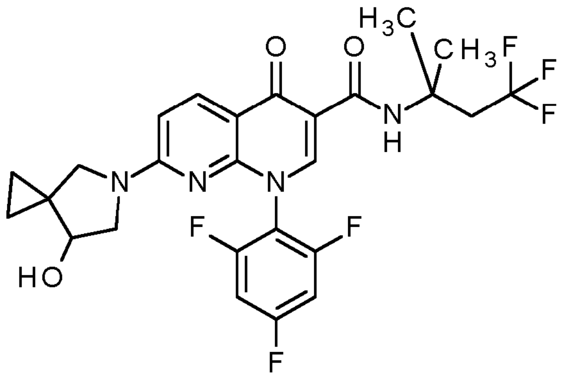

Gemäß AAV1 wurden 80.0 mg (185 $\mu$mol) der Verbindung aus Beispiel 155A mit 39.5 mg (223 $\mu$mol) 4,4,4-Trifluor-2-methylbutan-2-amin Hydrochlorid in Gegenwart von 84.6 mg (223 $\mu$mol) HATU und 97 $\mu$l (560 $\mu$mol) DIPEA in 890 $\mu$l DMF zur Reaktion gebracht. Das Rohprodukt wurde mittels präparativer HPLC (Acetonitril / Wasser/ 0,1 % Ameisensäure) gereinigt. Es wurden 80.9 mg (79% d. Th., 100% Reinheit) der Titelverbindung erhalten.
LC-MS (Methode 4): $R_t$ = 3.60 min; MS (ESIpos): m/z = 555 $[M+H]^+$
$^1H$ NMR (400 MHz, DMSO-$d_6$): $\delta$ ppm = 10.16 (s, 1H), 8.66-8.71 (m, 1H), 8.27 (d, 1H), 7.49-7.60 (m, 2H), 6.78 (br d, 0.40H), 6.66 (br d, 0.60H), 5.06 (br s, 0.40H), 4.97 (br d, 0.60H), 3.59-3.78 (m, 2H), 3.36-3.49 (m, 1H), 3.10-3.26 (m, 2H), 2.95 (q, 2H), 1.48 (s, 6H), 0.82 (br t, 1H), 0.54-0.62 (m, 2H), 0.48 (br s, 1H).

76.6 mg der Titelverbindung (Racemat) wurde durch chirale HPLC in die Enantiomere getrennt (präparative HPLC: Säule YMC Chiralart Amylose SA 5 $\mu$m 250x30 mm; Eluent: 80% *n*-Heptan, 20% Ethanol; Temperatur: 25°C; Fluss: 30 ml/min; UV-Detektion: 220 nm.)

Man erhielt (in der Reihenfolge der Elution von der Säule) 30.0 mg (29% d. Th., 100% Reinheit) Enantiomer 1 aus Beispiel 570 (99% ee) Rt = 9.31 min und 32.0 mg (31% d. Th., 100% Reinheit) Enantiomer 2 aus Beispiel 571 (99% ee) Rt = 11.08 min.

[Analytische HPLC: Säule YMC Chiralart Amylose SA 5 $\mu$m 250x4.6 mm; Eluent: 80% Iso-Hexan, 20% Ethanol; Temperatur: 25°C; Fluss: 1.0 ml/min; UV-Detektion: 220nm].

**Beispiel 570**

7-[7-Hydroxy-5-azaspiro[2.4]hept-5-yl]-4-oxo-*N*-(4,4,4-trifluor-2-methylbutan-2-yl)-1-(2,4,6-trifluorphenyl)-1,4-dihydro-1,8-naphthyridin-3-carboxamid (Enantiomer 1)

LC-MS (Methode 1): $R_t$ = 1.12 min; MS (ESIpos): m/z = 555 $[M+H]^+$
$^1H$ NMR (500 MHz, DMSO-$d_6$): $\delta$ ppm = 10.16 (s, 1H), 8.66-8.70 (m, 1H), 8.27 (d, 1H), 7.49-7.59 (m, 2H), 6.78 (br d, 0.40H), 6.66 (br d, 0.60H), 5.02-5.08 (m, 0.40H), 4.97 (br d, 0.60H), 3.60-3.78 (m, 2H), 3.31-3.47 (m, 2H), 3.20 (br dd, 1H), 2.95 (q, 2H), 1.48 (s, 6H), 0.77-0.87 (m, 1H), 0.53-0.62 (m, 2H), 0.48 (br s, 1H).

**Beispiel 571**

7-[7-Hydroxy-5-azaspiro[2.4]hept-5-yl]-4-oxo-*N*-(4,4,4-trifluor-2-methylbutan-2-yl)-1-(2,4,6-trifluorphenyl)-1,4-dihydro-1,8-naphthyridin-3-carboxamid (Enantiomer 2)

LC-MS (Methode 1): $R_t$ = 1.12 min; MS (ESIpos): m/z = 555 $[M+H]^+$
$^1$H NMR (500 MHz, DMSO-$d_6$): δ ppm = 10.16 (s, 1H), 8.66-8.70 (m, 1H), 8.27 (d, 1H), 7.49-7.59 (m, 2H), 6.78 (br d, 0.40H), 6.66 (br d, 0.60H), 5.07 (br s, 0.40H), 4.97 (br s, 0.60H), 3.60-3.77 (m, 2H), 3.33-3.47 (m, 2H), 3.20 (br dd, 1H), 2.95 (q, 2H), 1.48 (s, 6H), 0.77-0.86 (m, 1H), 0.54-0.62 (m, 2H), 0.45-0.54 (m, 1H).

**Beispiel 572**

7-[(3*R*,4*R*)-3,4-Dihydroxypyrrolidin-1-yl]-*N*-(1,1,1,3,3,3-hexafluorpropan-2-yl)-4-oxo-1-(2,4,6-trifluorphenyl)-1,4-dihydro-1,8-naphthyridin-3-carboxamid

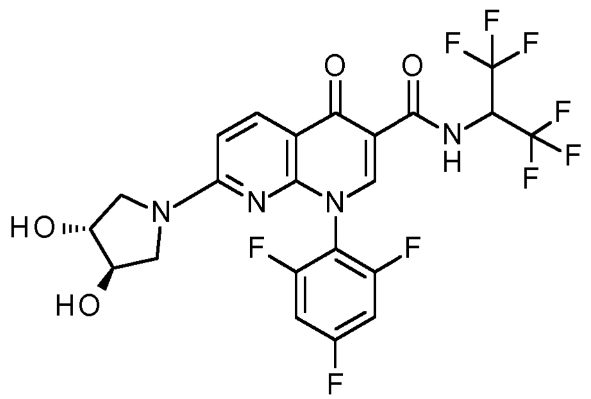

Gemäß AAV3 wurden 60.0 mg (119 µmol) der Verbindung aus Beispiel 154A mit 20.0 mg (143 µmol) (3*R*,4*R*)-Pyrrolidin-3,4-diol Hydrochlorid und 73 µl (420 µmol) DIPEA in 600 µl DMF zur Reaktion gebracht. Das Rohprodukt wurde mittels präparativer HPLC (Acetonitril / Wasser/ 0,1 % Ameisensäure) gereinigt. Es wurden 58.9 mg (87% d. Th., 100% Reinheit) der Titelverbindung erhalten.
LC-MS (Methode 3): $R_t$ = 1.83 min; MS (ESIpos): m/z = 571 $[M+H]^+$
$^1$H NMR (400 MHz, DMSO-$d_6$): δ ppm = 11.41 (d, 1H), 8.94 (s, 1H), 8.30 (d, 1H), 7.53-7.62 (m, 2H), 6.81 (d, 1H), 6.24-6.37 (m, 1H), 5.24 (d, 1H), 5.15 (d, 1H), 4.05 (br s, 1H), 3.93 (br s, 1H), 3.62 (br dd, 1H), 3.33-3.39 (m, 1H), 3.20-3.28 (m, 1H), 3.07 (br d, 1H).

**Beispiel 573**

7-[(3*R*,4*S*)-3,4-Dihydroxypyrrolidin-1-yl]-4-oxo-*N*-[(2*S*)-1,1,1-trifluor-4-methylpentan-2-yl]-1-(2,4,6-trifluorphenyl)-1,4-dihydro-1,8-naphthyridin-3-carboxamid

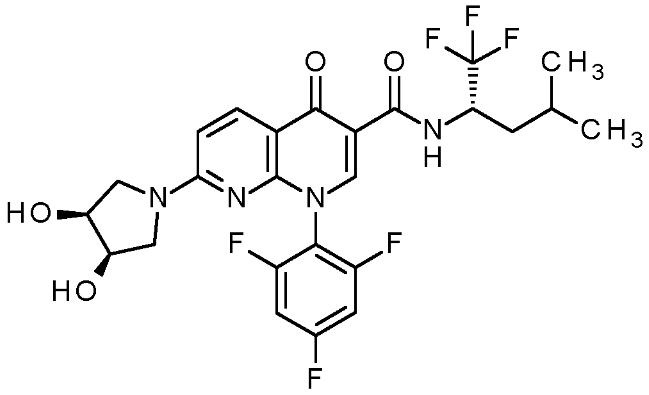

Gemäß AAV1 wurden 60.0 mg (142 μmol) der Verbindung aus Beispiel 156A mit 26.5 mg (171 μmol) (2*S*)-1,1,1-Trifluor-4-methylpentan-2-amin in Gegenwart von 65.0 mg (171 μmol) HATU und 74 μl (430 μmol) DIPEA in 1.2 ml DMF zur Reaktion gebracht. Das Rohprodukt wurde mittels präparativer HPLC (Acetonitril / Wasser/ 0,1 % Ameisensäure) gereinigt. Es wurden 46.5 mg (58% d. Th., 100% Reinheit) der Titelverbindung erhalten.
LC-MS (Methode 3): $R_t$ = 1.97 min; MS (ESIpos): m/z = 559 $[M+H]^+$
$^1$H NMR (400 MHz, DMSO-$d_6$): δ ppm = 10.41 (br d, 1H), 8.81 (s, 1H), 8.27 (d, 1H), 7.52-7.61 (m, 2H), 6.76 (d, 1H), 4.99-5.07 (m, 1H), 4.94 (br d, 1H), 4.76-4.88 (m, 1H), 4.09-4.17 (m, 1H), 3.99-4.07 (m, 1H), 3.56-3.64 (m, 1H), 3.18-3.28 (m, 2H), 2.96-3.05 (m, 1H), 1.62-1.72 (m, 2H), 1.52-1.62 (m, 1H), 0.95 (br d, 3H), 0.89 (br d, 3H).

## Beispiel 574

7-[(3*R*,4*S*)-3,4-Dihydroxypyrrolidin-1-yl]-4-oxo-*N*-[(2*R*)-1,1,1-trifluor-4-methylpentan-2-yl]-1-(2,4,6-trifluorphenyl)-1,4-dihydro-1,8-naphthyridin-3-carboxamid

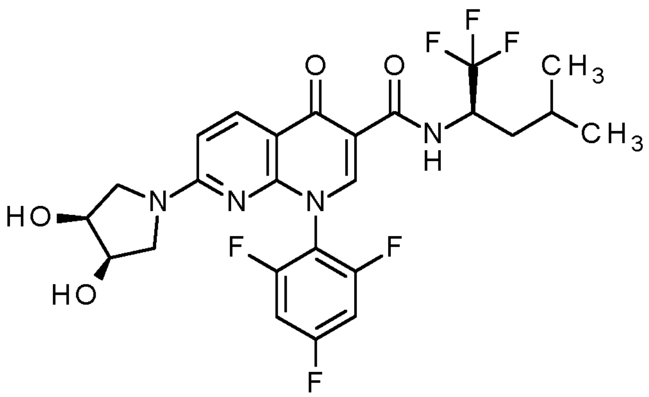

Gemäß AAV1 wurden 60.0 mg (142 μmol) der Verbindung aus Beispiel 156A mit 26.5 mg (171 μmol) (2*R*)-1,1,1-Trifluor-4-methylpentan-2-amin in Gegenwart von 65.0 mg (171 μmol) HATU und 74 μl (430 μmol) DIPEA in 1.2 ml DMF zur Reaktion gebracht. Das Rohprodukt wurde mittels präparativer HPLC (Acetonitril / Wasser/ 0,1 % Ameisensäure) gereinigt. Es wurden 18.2 mg (23% d. Th., 100% Reinheit) der Titelverbindung erhalten.
LC-MS (Methode 3): $R_t$ = 1.97 min; MS (ESIpos): m/z = 559 $[M+H]^+$
$^1$H NMR (400 MHz, DMSO-$d_6$): δ ppm = 10.33-10.50 (m, 1H), 8.81 (br s, 1H), 8.19-8.34 (m, 1H), 7.46-7.66 (m, 2H), 6.68-6.84 (m, 1H), 4.78-5.07 (m, 3H), 3.94-4.21 (m, 2H), 3.53-3.66 (m, 1H), 2.95-3.08 (m, 1H), 1.49-1.76 (m, 3H), 0.82-1.02 (m, 6H).

**Beispiel 575**

Methyl-4-{5-oxo-6-[(4,4,4-trifluor-2-methylbutan-2-yl)carbamoyl]-8-(2,4,6-trifluorphenyl)-5,8-dihydro-1,8-naphthyridin-2-yl}piperazin-1-carboxylat

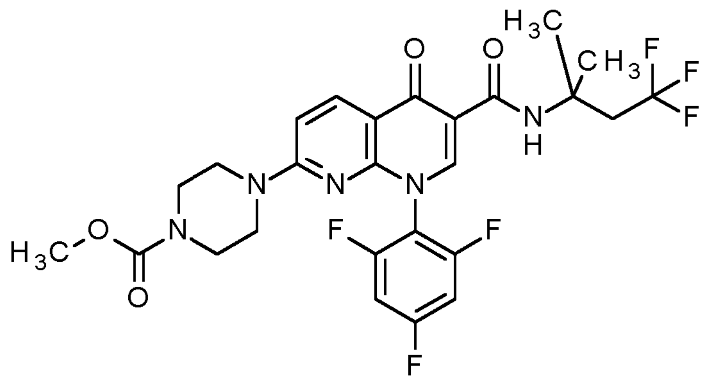

Gemäß AAV1 wurden 70.0 mg (151 µmol) der Verbindung aus Beispiel 157A mit 32.3 mg (182 µmol) 4,4,4-Trifluor-2-methylbutan-2-amin Hydrochlorid in Gegenwart von 69.1 mg (182 µmol) HATU und 110 µl (610 µmol) DIPEA in 580 µl DMF zur Reaktion gebracht. Das Rohprodukt wurde mittels präparativer HPLC (Acetonitril / Wasser/ 0,1 % Ameisensäure) gereinigt. Es wurden 61.1 mg (69% d. Th., 100% Reinheit) der Titelverbindung erhalten.
LC-MS (Methode 3): $R_t$ = 2.14 min; MS (ESIpos): m/z = 586 $[M+H]^+$
$^1H$ NMR (400 MHz, DMSO-$d_6$): δ ppm = 10.10 (s, 1H), 8.72 (s, 1H), 8.31 (d, 1H), 7.56 (t, 2H), 7.11 (d, 1H), 3.60 (s, 3H), 3.48-3.55 (m, 4H), 3.36-3.43 (m, 4H), 2.95 (q, 2H), 1.48 (s, 6H).

**Beispiel 576**

7-[(3*R*,4*R*)-3,4-Dihydroxypyrrolidin-1-yl]-4-oxo-*N*-[1-(trifluormethoxy)butan-2-yl]-1-(2,4,6-trifluorphenyl)-1,4-dihydro-1,8-naphthyridin-3-carboxamid (Diastereomerengemisch)

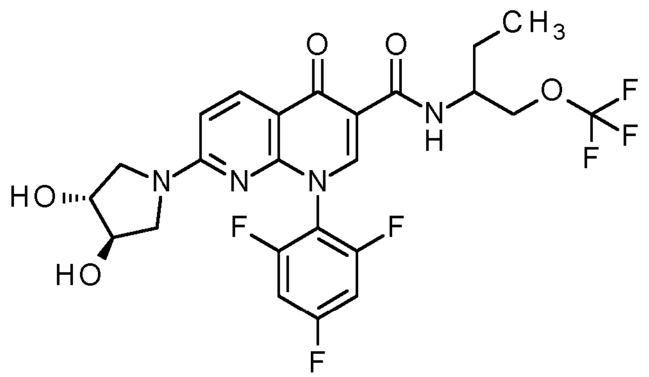

Gemäß AAV1 wurden 500 mg (1.19 mmol) der Verbindung aus Beispiel 121A mit 276 mg (1.42 mmol) 1-(Trifluormethoxy)butan-2-amin Hydrochlorid in Gegenwart von 541 mg (1.42 mmol) HATU und 830 µl (4.70 mmol) DIPEA in 5.0 ml DMF zur Reaktion gebracht. Das Rohprodukt wurde mittels präparativer HPLC (Acetonitril / Wasser/ 0,1 % Ameisensäure) gereinigt. Es wurden 389 mg (58% d. Th., 100% Reinheit) der Titelverbindung erhalten.
LC-MS (Methode 1): $R_t$ = 0.94 min; MS (ESIpos): m/z = 561 $[M+H]^+$
$^1H$ NMR (400 MHz, DMSO-$d_6$): δ ppm = 10.08 (br d, 1H), 8.72 (s, 1H), 8.27 (d, 1H), 7.52-7.60 (m, 2H), 6.76 (d, 1H), 5.23 (d, 1H), 5.13 (d, 1H), 4.13-4.23 (m, 3H), 4.05 (br s, 1H), 3.92 (br s, 1H), 3.61 (br dd, 1H), 3.32-3.36 (m, 1H), 3.25 (br dd, 1H), 3.07 (br d, 1H), 1.53-1.73 (m, 2H), 0.94 (t, 3H).

385 mg der Titelverbindung (Diastereomerengemisch) wurde durch chirale HPLC in die Diastereomere getrennt (präparative HPLC: Säule Daicel Chiralcel OZ-H 5 µm 250x20 mm; Eluent: 80% *n*-Heptan, 20% Iso-Propanol; Temperatur: 23°C; Fluss: 20 ml/min; UV-Detektion: 220 nm.)

Man erhielt (in der Reihenfolge der Elution von der Säule) 119.4 mg Diastereomer 1 (99% de) Rt = 5.13 min und 96.4 mg Diastereomer 2 (95% de) Rt = 6.74 min.

[Analytische HPLC: Säule Daicel OZ-3 3 µm 50x4.6 mm; Eluent: 80% Iso-Hexan, 20% Iso-propanol; UV-Detektion: 220 nm].

Diastereomer 1 wurde zusätzlich mittels präparativer HPLC (Säule: Chromatorex C18, 10 µm, 125*40 mm, Lösungsmittel: Acetonitril, Wasser, 0.1% Ameisensäure) gereinigt. Die isolierte Substanz enthielt Verunreinigungen und wurde weiter aus Acetonitril umkristallisiert, abgesaugt, mit etwas Acetonitril nachgewaschen und nachgetrocknet. Es wurden 89.3 mg (13% d. Th., 100% Reinheit) der Titelverbindung aus Beispiel 577 erhalten.

Diastereomer 2 wurde zusätzlich mittels präparativer HPLC (Säule: Chromatorex C18, 10 µm, 125*40 mm, Lösungsmittel: Acetonitril, Wasser, 0.1% Ameisensäure) gereinigt. Die isolierte Substanz enthielt Verunreinigungen und wurde weiter aus Acetonitril umkristallisiert, abgesaugt, mit etwas Acetonitril nachgewaschen und nachgetrocknet. Es wurden 75.5 mg (11% d. Th., 100% Reinheit) der Titelverbindung aus Beispiel 578 erhalten.

## Beispiel 577

7-[(3*R*,4*R*)-3,4-Dihydroxypyrrolidin-1-yl]-4-oxo-*N*-[1-(trifluormethoxy)butan-2-yl]-1-(2,4,6-trifluorphenyl)-1,4-dihydro-1,8-naphthyridin-3-carboxamid (Diastereomer 1)

LC-MS (Methode 1): $R_t$ = 0.92 min; MS (ESIpos): m/z = 561 $[M+H]^+$
$^1$H NMR (400 MHz, DMSO-$d_6$): δ ppm = 10.04-10.11 (m, 1H), 8.72 (s, 1H), 8.27 (d, 1H), 7.52-7.60 (m, 2H), 6.76 (d, 1H), 5.23 (d, 1H), 5.13 (d, 1H), 4.13-4.23 (m, 3H), 4.05 (br s, 1H), 3.92 (br s, 1H), 3.61 (br dd, 1H), 3.32-3.37 (m, 1H), 3.25 (br dd, 1H), 3.07 (br d, 1H), 1.54-1.73 (m, 2H), 0.94 (t, 3H).

## Beispiel 578

7-[(3*R*,4*R*)-3,4-Dihydroxypyrrolidin-1-yl]-4-oxo-*N*-[1-(trifluormethoxy)butan-2-yl]-1-(2,4,6-trifluorphenyl)-1,4-dihydro-1,8-naphthyridin-3-carboxamid (Diastereomer 2)

LC-MS (Methode 1): $R_t$ = 0.92 min; MS (ESIpos): m/z = 561 $[M+H]^+$
$^1$H NMR (400 MHz, DMSO-$d_6$): δ ppm = 10.08 (br d, 1H), 8.72 (s, 1H), 8.27 (d, 1H), 7.52-7.60 (m, 2H), 6.76 (d, 1H), 5.22 (d, 1H), 5.13 (d, 1H), 4.13-4.23 (m, 3H), 4.05 (br s, 1H), 3.89-3.96 (m, 1H), 3.61 (br dd, 1H), 3.32-3.37 (m, 1H), 3.25 (br dd, 1H), 3.07 (br d, 1H), 1.53-1.73 (m, 2H), 0.94 (t, 3H).

## Beispiel 579

*N*-[(1*S*)-1-Cyclopropyl-2,2,2-trifluorethyl]-7-[4-hydroxy-3-methyl-2-oxopyrrolidin-1-yl]-4-oxo-1-(2,4,6-trifluorphenyl)-1,4-dihydro-1,8-naphthyridin-3-carboxamid (Diastereomerengemisch)

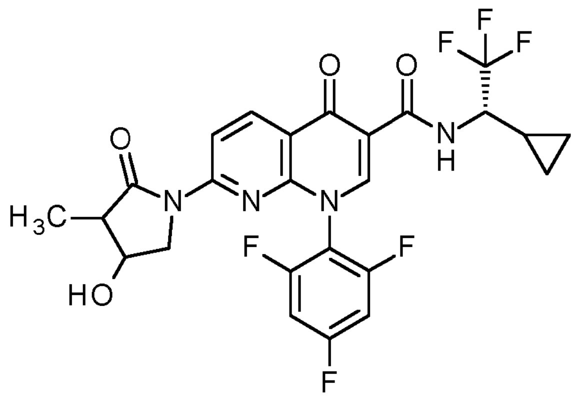

Gemäß AAV2 wurden 130 mg (273 µmol) der Verbindung aus Beispiel 126A mit 37.8 mg (328 µmol) der Verbindung 158D in Gegenwart von 56.6 mg (410 µmol) Kaliumcarbonat, 12.3 mg (54.6 µmol) Palladiumacetat und 31.6 mg (54.6 µmol) Xantphos in 2.4 ml 1,4-Dioxan zur Reaktion gebracht. Das Rohprodukt wurde mittels präparativer

HPLC (Acetonitril / Wasser/ 0,1 % Ameisensäure) gereinigt. Die isolierte Substanz enthielt Verunreinigungen und wurde weiter aus Acetonitril umkristallisiert, abgesaugt, mit etwas Acetonitril nachgewaschen und nachgetrocknet. Es wurden 79.6 mg (51% d. Th., 97% Reinheit) der Titelverbindung erhalten.
LC-MS (Methode 1): $R_t$ = 1.06 min; MS (ESIpos): m/z = 555 $[M+H]^+$
$^1$H NMR (400 MHz, DMSO-$d_6$): δ ppm = 10.22-10.30 (m, 1H), 9.06 (s, 1H), 8.71 (d, 1H), 8.51-8.58 (m, 1H), 7.57-7.66 (m, 2H), 5.50 (d, 0.20H), 5.24 (d, 0.80H), 4.40 (sxt, 1H), 4.19 (q, 0.80H), 3.90-3.96 (m, 0.20H), 3.77 (dd, 0.20H), 3.56-3.64 (m, 0.80H), 3.47-3.53 (m, 0.80H), 3.25 (dd, 0.20H), 2.80-2.88 (m, 0.80H), 1.18-1.28 (m, 1H), 1.14 (d, 0.60H), 1.08 (d, 2.40H), 0.52-0.70 (m, 3H), 0.30-0.39 (m, 1H).

## Beispiel 580

4-Oxo-7-(2-oxoimidazolidin-1-yl)-*N*-[(2*R*)-1,1,1-trifluor-4-methylpentan-2-yl]-1-(2,4,6-trifluorphenyl)-1,4-dihydro-1,8-naphthyridin-3-carboxamid

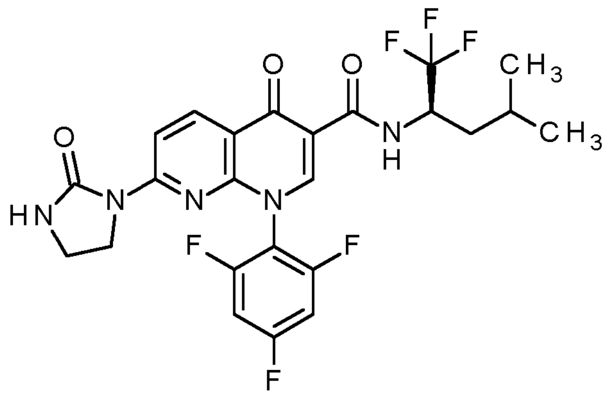

Gemäß AAV1 wurden 50.0 mg (124 μmol) der Verbindung aus Beispiel 113A mit 23.0 mg (148 μmol) (2*R*)-1,1,1-Trifluor-4-methylpentan-2-amin in Gegenwart von 56.4 mg (148 μmol) HATU und 65 μl (370 μmol) DIPEA in 480 μl DMF zur Reaktion gebracht. Das Rohprodukt wurde mittels präparativer HPLC (Acetonitril / Wasser/ 0,1 % Ameisensäure) gereinigt. Es wurden 36.2 mg (54% d. Th., 100% Reinheit) der Titelverbindung erhalten.
LC-MS (Methode 3): $R_t$ = 2.16 min; MS (ESIpos): m/z = 542 $[M+H]^+$
$^1$H NMR (400 MHz, DMSO-$d_6$): δ ppm = 10.20 (d, 1H), 9.00 (s, 1H), 8.55 (d, 1H), 8.44 (d, 1H), 7.67 (s, 1H), 7.57 (t, 2H), 4.80-4.89 (m, 1H), 3.56-3.62 (m, 2H), 3.32-3.38 (m, 2H), 1.64-1.74 (m, 2H), 1.53-1.62 (m, 1H), 0.95 (d, 3H), 0.90 (d, 3H).

## Beispiel 581

*N*-(1,1,1,3,3,3-Hexafluor-2-methylpropan-2-yl)-4-oxo-7-(2-oxoimidazolidin-1-yl)-1-(2,4,6-trifluorphenyl)-1,4-dihydro-1,8-naphthyridin-3-carboxamid

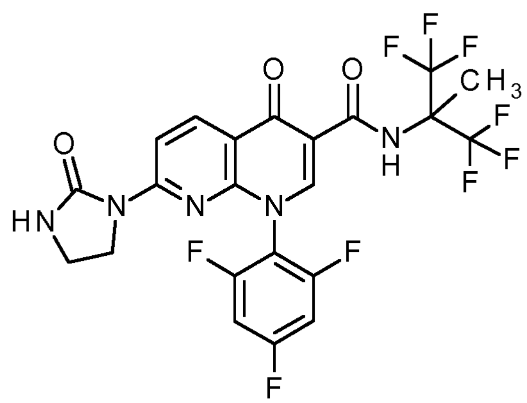

Gemäß AAV2 wurden 22.0 mg (42.5 µmol) der Verbindung aus Beispiel 159A mit 36.6 mg (425 µmol) Imidazolidin-2-on in Gegenwart von 8.81 mg (63.7 µmol) Kaliumcarbonat, 1.91 mg (8.50 µmol) Palladiumacetat und 4.92 mg (8.50 µmol) Xantphos in 430 µl 1,4-Dioxan zur Reaktion gebracht. Das Rohprodukt wurde mittels präparativer HPLC (Acetonitril / Wasser/ 0,1 % Ameisensäure) gereinigt. Es wurden 5.30 mg (22% d. Th., 100% Reinheit) der Titelverbindung erhalten.
LC-MS (Methode 3): $R_t$ = 2.12 min; MS (ESIpos): m/z = 568 $[M+H]^+$
$^1H$ NMR (400 MHz, DMSO-$d_6$): δ ppm = 11.36 (s, 1H), 9.04 (s, 1H), 8.59 (d, 1H), 8.45 (d, 1H), 7.69 (s, 1H), 7.58 (t, 2H), 3.53-3.65 (m, 2H), 3.32-3.38 (m, 2H), 2.07 (s, 3H).

## Beispiel 582

*N*-[(1*S*)-1-Cyclopropyl-2,2,2-trifluorethyl]-7-[*trans*-3-hydroxy-4-methoxypyrrolidin-1-yl]-4-oxo-1-(2,4,6-trifluorphenyl)-1,4-dihydro-1,8-naphthyridin-3-carboxamid (Diastereomerengemisch)

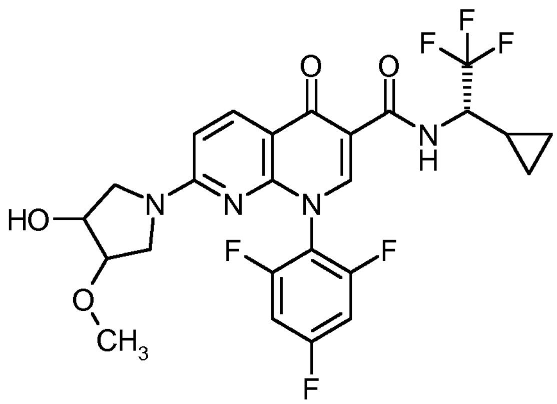

Gemäß AAV3 wurden 150 mg (315 µmol) der Verbindung aus Beispiel 126A mit 44.3 mg (378 µmol) 4-Methoxypyrrolidin-3-ol und 160 µl (950 µmol) DIPEA in 1.3 ml DMF zur Reaktion gebracht. Das Rohprodukt wurde mittels präparativer HPLC (Acetonitril / Wasser/ 0,1 % Ameisensäure) gereinigt. Es wurden 147 mg (84% d. Th., 100% Reinheit) der Titelverbindung erhalten.
LC-MS (Methode 3): $R_t$ = 1.95 min; MS (ESIpos): m/z = 557 $[M+H]^+$
$^1H$ NMR (400 MHz, DMSO-$d_6$): δ ppm = 10.56 (d, 1H), 8.80 (s, 1H), 8.29 (d, 1H), 7.52-7.60 (m, 2H), 6.80 (t, 1H), 5.35 (br s, 0.45H), 5.25 (br s, 0.55H), 4.33-4.44 (m, 1H), 4.27 (br s, 0.45H), 4.14 (br s, 0.55H), 3.79 (br s, 0.55H), 3.69 (br s, 0.45H), 3.50-3.64 (m, 1.55H), 3.35-3.40 (m, 0.45H), 3.26 (br d, 3.45H), 3.19 (br d, 1H), 3.05-3.12 (m, 0.55H), 1.16-1.25 (m, 1H), 0.50-0.69 (m, 3H), 0.31-0.38 (m, 1H).

138 mg der Titelverbindung (Diastereomerengemisch) wurde durch chirale HPLC in die Diastereomere getrennt (präparative HPLC: Säule Daicel Chiralpak IA 5 µm 250x20 mm; Eluent: 85% *n*-Heptan, 15% Ethanol; Temperatur: 25°C; Fluss: 15 ml/min; UV-Detektion: 210 nm.)

Man erhielt (in der Reihenfolge der Elution von der Säule) 62.7 mg (36% d. Th., 100% Reinheit) Diastereomer 1 aus Beispiel 583 (96% de) Rt = 12.53 min und 59.6 mg (34% d. Th., 100% Reinheit) Diastereomer 2 aus Beispiel 584 (96% de) Rt = 14.78 min.

[Analytische HPLC: Säule Daicel IA-3 3 µm 50x4.6 mm; Eluent: 80% Iso-Hexan, 20% Ethanol; UV-Detektion: 220nm].

## Beispiel 583

*N*-[(1S)-1-Cyclopropyl-2,2,2-trifluorethyl]-7-[*trans*-3-hydroxy-4-methoxypyrrolidin-1-yl]-4-oxo-1-(2,4,6-trifluorphenyl)-1,4-dihydro-1,8-naphthyridin-3-carboxamid (Diastereomer 1)

LC-MS (Methode 3): $R_t$ = 1.96 min; MS (ESIpos): m/z = 557 $[M+H]^+$
$^1H$ NMR (400 MHz, DMSO-$d_6$): δ ppm = 10.55 (d, 1H), 8.80 (s, 1H), 8.29 (d, 1H), 7.51-7.61 (m, 2H), 6.80 (br t, 1H), 5.30-5.37 (m, 0.45H), 5.22-5.30 (m, 0.55H), 4.33-4.44 (m, 1H), 4.22-4.31 (m, 0.45H), 4.11-4.20 (m, 0.55H), 3.73-3.82 (m, 0.55H), 3.66-3.71 (m, 0.45H), 3.49-3.64 (m, 1.55H), 3.35-3.41 (m, 0.45H), 3.26 (br d, 3.45H), 3.15-3.22 (m, 1H), 3.06-3.13 (m, 0.55H), 1.15-1.27 (m, 1H), 0.49-0.69 (m, 3H), 0.29-0.37 (m, 1H).

**Beispiel 584**

*N*-[(1*S*)-1-Cyclopropyl-2,2,2-trifluorethyl]-7-[*trans*-3-hydroxy-4-methoxypyrrolidin-1-yl]-4-oxo-1-(2,4,6-trifluorphenyl)-1,4-dihydro-1,8-naphthyridin-3-carboxamid (Diastereomer 2)

LC-MS (Methode 3): $R_t$ = 1.96 min; MS (ESIpos): m/z = 557 $[M+H]^+$
$^1$H NMR (400 MHz, DMSO-$d_6$): δ ppm = 10.56 (d, 1H), 8.80 (s, 1H), 8.29 (d, 1H), 7.52-7.60 (m, 2H), 6.80 (br t, 1H), 5.23-5.39 (m, 1H), 4.33-4.43 (m, 1H), 4.25-4.30 (m, 0.45H), 4.14 (br s, 0.55H), 3.75-3.82 (m, 0.55H), 3.66-3.71 (m, 0.45H), 3.50-3.64 (m, 1.55H), 3.35-3.41 (m, 0.45H), 3.26 (br d, 3.45H), 3.19 (br d, 1H), 3.06-3.14 (m, 0.55H), 1.16-1.25 (m, 1H), 0.50-0.69 (m, 3H), 0.31-0.37 (m, 1H).

**Beispiel 585**

*N*-(2,6-Dichlorphenyl)-7-[(3*R*,4*R*)-3,4-dihydroxypyrrolidin-1-yl]-4-oxo-1-(2,4,6-trifluorphenyl)-1,4-dihydro-1,8-naphthyridin-3-carboxamid

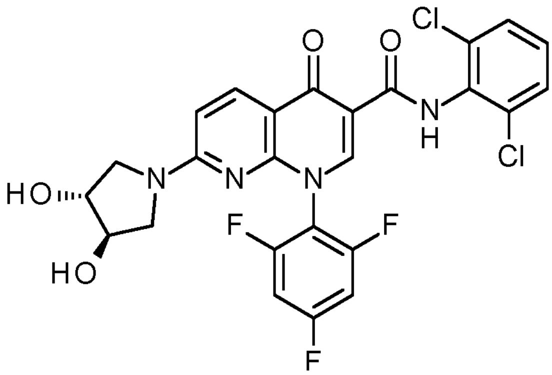

Gemäß AAV3 wurden 60.0 mg (120 μmol) der Verbindung aus Beispiel 160B mit 20.2 mg (144 μmol) (3*R*,4*R*)-Pyrrolidin-3,4-diol Hydrochlorid und 73 μl (420 μmol) DIPEA in 540 μl DMF zur Reaktion gebracht. Das Rohprodukt wurde mittels präparativer HPLC (Acetonitril / Wasser/ 0,1 % Ameisensäure) gereinigt. Es wurden 53.3 mg (76% d. Th., 98% Reinheit) der Titelverbindung erhalten.
LC-MS (Methode 1): $R_t$ = 0.91 min; MS (ESIpos): m/z = 565 $[M+H]^+$
$^1$H NMR (400 MHz, DMSO-$d_6$): δ ppm = 11.95 (s, 1H), 8.89 (s, 1H), 8.34 (d, 1H), 7.48-7.65 (m, 4H), 7.38 (t, 1H), 6.81 (d, 1H), 5.25 (d, 1H), 5.16 (d, 1H), 4.06 (br s, 1H), 3.94 (br s, 1H), 3.63 (br dd, 1H), 3.33-3.39 (m, 1H), 3.23-3.29 (m, 1H), 3.09 (br d, 1H).

**Beispiel 586**

*N*-[1,1,1,4,4,4-Hexafluorbutan-2-yl]-4-oxo-7-(2-oxoimidazolidin-1-yl)-1-(2,4,6-trifluorphenyl)-1,4-dihydro-1,8-naphthyridin-3-carboxamid (Racemat)

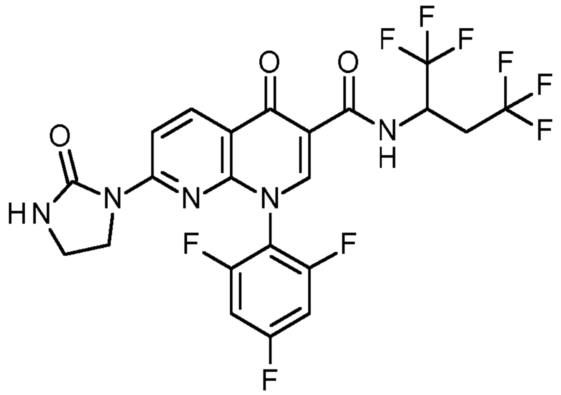

Gemäß AAV1 wurden 40.0 mg (89.4 µmol, 90% Reinheit) der Verbindung aus Beispiel 113A mit 27.2 mg (125 µmol) 1,1,1,4,4,4-Hexafluorbutan-2-amin Hydrochlorid in Gegenwart von 40.8 mg (107 µmol) HATU und 47 µl (270 µmol) DIPEA in 330 µl DMF zur Reaktion gebracht. Das Rohprodukt wurde mittels präparativer HPLC (Acetonitril / Wasser/ 0,1 % Ameisensäure) gereinigt. Es wurden 33.3 mg (66% d. Th., 100% Reinheit) der Titelverbindung erhalten.
LC-MS (Methode 3): $R_t$ = 1.90 min; MS (ESIpos): m/z = 568 $[M+H]^+$
$^1H$ NMR (400 MHz, DMSO-$d_6$): δ ppm = 10.47 (d, 1H), 9.02 (s, 1H), 8.55 (d, 1H), 8.44 (d, 1H), 7.67 (s, 1H), 7.54-7.61 (m, 2H), 5.21-5.32 (m, 1H), 3.60 (t, 2H), 3.33-3.38 (m, 2H), 3.07-3.20 (dt, 1H), 2.90-3.03 (m, 1H).

25.4 mg der Titelverbindung (Racemat) wurde durch chirale HPLC in die Enantiomere getrennt (präparative HPLC: Säule Daicel Chiralpak IF 5 µm 250x20 mm; Eluent: 80% *n*-Heptan, 20% Ethanol; Temperatur: 25°C; Fluss: 15 ml/min; UV-Detektion: 220 nm.)

Man erhielt (in der Reihenfolge der Elution von der Säule) 10.0 mg (20% d. Th., 100% Reinheit) Enantiomer 1 aus Beispiel 587 (99% ee) Rt = 12.96 min und 9.00 mg (18% d. Th., 100% Reinheit) Enantiomer 2 aus Beispiel 588 (98% ee) Rt = 14.19 min.

[Analytische HPLC: Säule Daicel Chiralpak IF 5 µm 250x4.6 mm; Eluent: 80% Iso-Hexan, 20% Ethanol; Temperatur: 30°C; Fluss: 1.0 ml/min; UV-Detektion: 220nm].

## Beispiel 587

*N*-[1,1,1,4,4,4-Hexafluorbutan-2-yl]-4-oxo-7-(2-oxoimidazolidin-1-yl)-1-(2,4,6-trifluorphenyl)-1,4-dihydro-1,8-naphthyridin-3-carboxamid (Enantiomer 1)

LC-MS (Methode 3): $R_t$ = 1.91 min; MS (ESIpos): m/z = 568 $[M+H]^+$
$^1H$ NMR (400 MHz, DMSO-$d_6$): δ ppm = 10.47 (d, 1H), 9.02 (s, 1H), 8.55 (d, 1H), 8.44 (d, 1H), 7.67 (br s, 1H), 7.58 (t, 2H), 5.21-5.33 (m, 1H), 3.56-3.63 (m, 2H), 3.32-3.38 (m, 2H), 3.08-3.20 (m, 1H), 2.90-3.03 (m, 1H).

## Beispiel 588

*N*-[1,1,1,4,4,4-Hexafluorbutan-2-yl]-4-oxo-7-(2-oxoimidazolidin-1-yl)-1-(2,4,6-trifluorphenyl)-1,4-dihydro-1,8-naphthyridin-3-carboxamid (Enantiomer 2)

LC-MS (Methode 3): $R_t$ = 1.92 min; MS (ESIpos): m/z = 568 $[M+H]^+$
$^1H$ NMR (400 MHz, DMSO-$d_6$): δ ppm = 10.47 (d, 1H), 9.02 (s, 1H), 8.55 (d, 1H), 8.44 (d, 1H), 7.67 (s, 1H), 7.54-7.61 (m, 2H), 5.23-5.30 (m, 1H), 3.57-3.62 (m, 2H), 3.32-3.37 (m, 2H), 3.09-3.20 (m, 1H), 2.90-3.02 (m, 1H).

## Beispiel 589

7-[(3*R*,4*R*)-3,4-Dihydroxypyrrolidin-1-yl]-4-oxo-*N*-[(2*S*)-1,1,1-trifluorbutan-2-yl]-1-(2,4,6-trifluorphenyl)-1,4-dihydro-1,8-naphthyridin-3-carboxamid

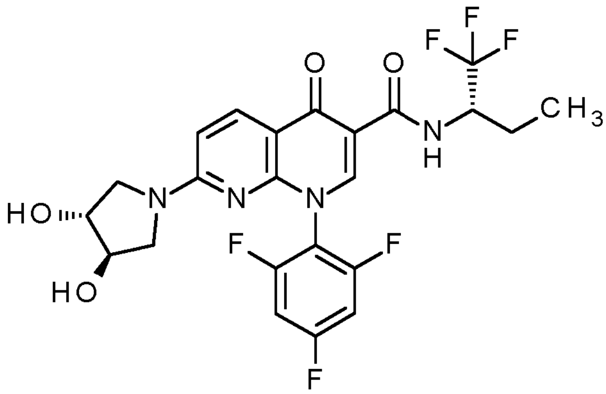

Gemäß AAV3 wurden 50.0 mg (108 µmol) der Verbindung aus Beispiel 115A mit 18.1 mg (129 µmol) (3*R*,4*R*)-Pyrrolidin-3,4-diol Hydrochlorid und 66 µl (380 µmol) DIPEA in 500 µl DMF zur Reaktion gebracht. Das Rohprodukt wurde mittels präparativer HPLC (Acetonitril / Wasser/ 0,1 % Ameisensäure) gereinigt. Es wurden 43.1 mg (75% d. Th., 100% Reinheit) der Titelverbindung erhalten.
LC-MS (Methode 3): $R_t$ = 1.70 min; MS (ESIpos): m/z = 531 $[M+H]^+$
$^1H$ NMR (500 MHz, DMSO-$d_6$): δ ppm = 10.43 (d, 1H), 8.81 (s, 1H), 8.28 (d, 1H), 7.53-7.60 (m, 2H), 6.78 (d, 1H), 5.24 (d, 1H), 5.14 (d, 1H), 4.69-4.78 (m, 1H), 4.05 (br s, 1H), 3.93 (br s, 1H), 3.62 (br dd, 1H), 3.33-3.37 (m, 1H), 3.25 (br dd, 1H), 3.07 (br d, 1H), 1.83-1.92 (m, 1H), 1.59-1.69 (m, 1H), 0.97 (t, 3H).

**Beispiel 590**

*N*-[(1*S*)-1-Cyclopropyl-2,2,2-trifluorethyl]-7-[(3*R*,4*R*)-3,4-dihydroxypyrrolidin-1-yl]-4-oxo-1-(2,4,6-trifluorphenyl)-1,4-dihydro-1,8-naphthyridin-3-carboxamid

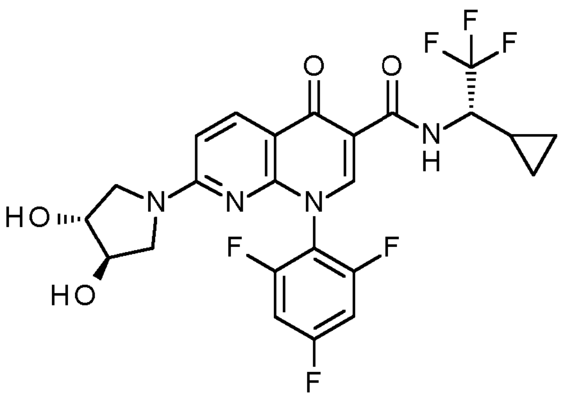

Gemäß AAV3 wurden 50.0 mg (105 µmol) der Verbindung aus Beispiel 126A mit 17.6 mg (126 µmol) (3*R*,4*R*)-Pyrrolidin-3,4-diol Hydrochlorid und 64 µl (370 µmol) DIPEA in 500 µl DMF zur Reaktion gebracht. Das Rohprodukt wurde mittels präparativer HPLC (Acetonitril / Wasser/ 0,1 % Ameisensäure) gereinigt. Es wurden 45.5 mg (80% d. Th., 100% Reinheit) der Titelverbindung erhalten.
LC-MS (Methode 3): $R_t$ = 1.73 min; MS (ESIpos): m/z = 543 $[M+H]^+$
$^1H$ NMR (400 MHz, DMSO-$d_6$): δ ppm = 10.57 (d, 1H), 8.80 (s, 1H), 8.28 (d, 1H), 7.52-7.60 (m, 2H), 6.78 (d, 1H), 5.23 (d, 1H), 5.14 (d, 1H), 4.33-4.44 (m, 1H), 4.04-4.07 (m, 1H), 3.93 (br s, 1H), 3.62 (br dd, 1H), 3.32-3.37 (m, 1H), 3.25 (br dd, 1H), 3.07 (br d, 1H), 1.19-1.24 (m, 1H), 0.50-0.69 (m, 3H), 0.30-0.38 (m, 1H).

**Beispiel 591**

7-[(3*R*,4*R*)-3,4-Dihydroxypyrrolidin-1-yl]-*N*-(1,1,1,3,3,3-hexafluor-2-methylpropan-2-yl)-4-oxo-1-(2,4,6-trifluorphenyl)-1,4-dihydro-1,8-naphthyridin-3-carboxamid

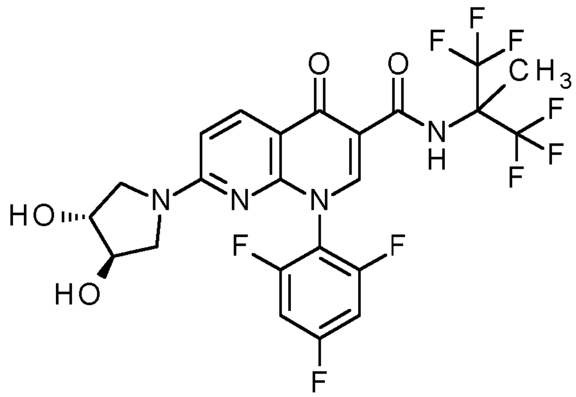

Gemäß AAV3 wurden 25.0 mg (48.3 μmol) der Verbindung aus Beispiel 159A mit 8.09 mg (57.9 μmol) (3*R*,4*R*)-Pyrrolidin-3,4-diol Hydrochlorid und 29 μl (170 μmol) DIPEA in 220 μl DMF zur Reaktion gebracht. Das Rohprodukt wurde mittels präparativer HPLC (Acetonitril / Wasser/ 0,1 % Ameisensäure) gereinigt. Es wurden 25.7 mg (91% d. Th., 100% Reinheit) der Titelverbindung erhalten.
LC-MS (Methode 3): $R_t$ = 1.91 min; MS (ESIpos): m/z = 585 $[M+H]^+$
$^1$H NMR (400 MHz, DMSO-$d_6$): δ ppm = 11.61 (s, 1H), 8.84 (s, 1H), 8.30 (d, 1H), 7.54-7.61 (m, 2H), 6.80 (d, 1H), 5.24 (d, 1H), 5.15 (d, 1H), 4.03-4.07 (m, 1H), 3.90-3.95 (m, 1H), 3.62 (dd, 1H), 3.33-3.39 (m, 1H), 3.25 (dd, 1H), 3.07 (d, 1H), 2.06 (br s, 3H).

**Beispiel 592**

*N*-(2,6-Dichlorphenyl)-7-[(4*S*)-4-hydroxy-2-oxopyrrolidin-1-yl]-4-oxo-1-(2,4,6-trifluorphenyl)-1,4-dihydro-1,8-naphthyridin-3-carboxamid

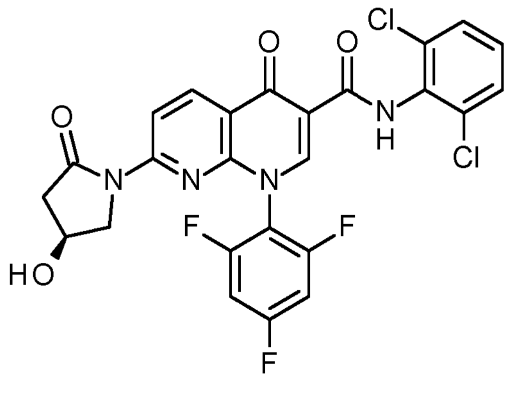

Gemäß AAV2 wurden 60.0 mg (120 μmol) der Verbindung aus Beispiel 160B mit 19.9 mg (144 μmol) (4*S*)-4-Hydroxypyrrolidin-2-on Hydrochlorid in Gegenwart von 24.9 mg (180 μmol) Kaliumcarbonat, 5.40 mg (24.1 μmol) Palladiumacetat und 13.9 mg (24.1 μmol) Xantphos in 1.1 ml 1,4-Dioxan zur Reaktion gebracht. Das Rohprodukt wurde mittels präparativer HPLC (Acetonitril / Wasser/ 0,1 % Ameisensäure) gereinigt. Die Substanz wurde aus Acetonitril umkristallisiert, abgesaugt, mit etwas Acetonitril nachgewaschen und nachgetrocknet. Die Substanz wurde mit Verunreinigungen isoliert und wurde weiter anschließend mittels Normalphasenchromatographie gereinigt (Eluent: Cyclohexan-Essigsäureethylester-Gradient). Es wurden 6.20 mg (9% d. Th., 100% Reinheit) der Titelverbindung erhalten.
LC-MS (Methode 1): $R_t$ = 1.00 min; MS (ESIpos): m/z = 563 $[M+H]^+$

$^1H$ NMR (400 MHz, DMSO-$d_6$): δ ppm = 11.60 (s, 1H), 9.16 (s, 1H), 8.78 (d, 1H), 8.57 (d, 1H), 7.58-7.66 (m, 4H), 7.39 (t, 1H), 5.35 (br s, 1H), 4.22-4.36 (m, 1H), 3.71 (dd, 1H), 3.49 (d, 1H), 2.96 (dd, 1H).

**Beispiel 593**

4-{6-[(2,6-dichlorphenyl)carbamoyl]-5-oxo-8-(2,4,6-trifluorphenyl)-5,8-dihydro-1,8-naphthyridin-2-yl}piperazin-1-carbonsäuremethylester

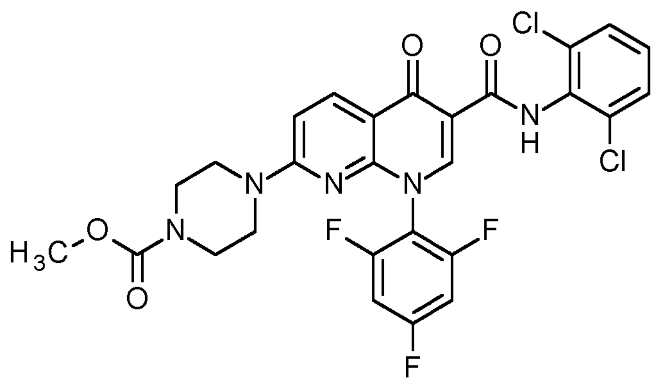

Gemäß AAV3 wurden 60.0 mg (120 μmol) der Verbindung aus Beispiel 160B mit 20.8 mg (144 μmol) Methylpiperazin-1-carboxylat und 73 μl (420 μmol) DIPEA in 540 μl DMF zur Reaktion gebracht. Das Rohprodukt wurde mittels präparativer HPLC (Acetonitril / Wasser/ 0,1 % Ameisensäure) gereinigt. Es wurden 40.3 mg (55% d. Th., 100% Reinheit) der Titelverbindung erhalten.
LC-MS (Methode 3): $R_t$ = 2.14 min; MS (ESIpos): m/z = 606 $[M+H]^+$
$^1H$ NMR (400 MHz, DMSO-$d_6$): δ ppm = 11.86 (s, 1H), 8.93 (s, 1H), 8.39 (d, 1H), 7.54-7.61 (m, 4H), 7.38 (t, 1H), 7.16 (d, 1H), 3.61 (s, 3H), 3.50-3.58 (m, 4H), 3.38-3.46 (m, 4H).

**Beispiel 594**

4-[6-{[(1*R*)-1-cyclopropyl-2,2,2-trifluorethyl]carbamoyl}-5-oxo-8-(2,4,6-trifluorphenyl)-5,8-dihydro-1,8-naphthyridin-2-yl]piperazin-1-carbonsäuremethylester

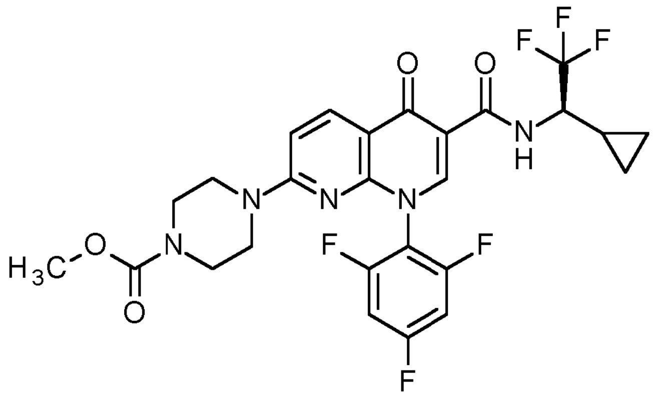

Gemäß AAV1 wurden 70.0 mg (151 μmol) der Verbindung aus Beispiel 157A mit 31.9 mg (182 μmol) (1*R*)-1-Cyclopropyl-2,2,2-trifluorethanamin Hydrochlorid in Gegenwart von 69.1 mg (182 μmol) HATU und 110 μl (610 μmol) DIPEA in 580 μl DMF zur Reaktion gebracht. Das Rohprodukt wurde mittels präparativer HPLC (Acetonitril / Wasser/ 0,1 % Ameisensäure) gereinigt. Es wurden 50.9 mg (58% d. Th., 100% Reinheit) der Titelverbindung erhalten.
LC-MS (Methode 3): $R_t$ = 2.13 min; MS (ESIpos): m/z = 584 $[M+H]^+$
$^1H$ NMR (400 MHz, DMSO-$d_6$): δ ppm = 10.49 (d, 1H), 8.83 (s, 1H), 8.33 (d, 1H), 7.53-7.60 (m, 2H), 7.14 (d, 1H),

4.33-4.43 (m, 1H), 3.61 (s, 3H), 3.50-3.57 (m, 4H), 3.36-3.43 (m, 4H), 1.16-1.25 (m, 1H), 0.50-0.69 (m, 3H), 0.31-0.37 (m, 1H).

**Beispiel 595**

Methyl-4-[5-oxo-6- {[(2*S*)-1,1,1-trifluorbutan-2-yl]carbamoyl}-8-(2,4,6-trifluorphenyl)-5,8-dihydro-1,8-naphthyridin-2-yl]piperazin-1-carboxylat

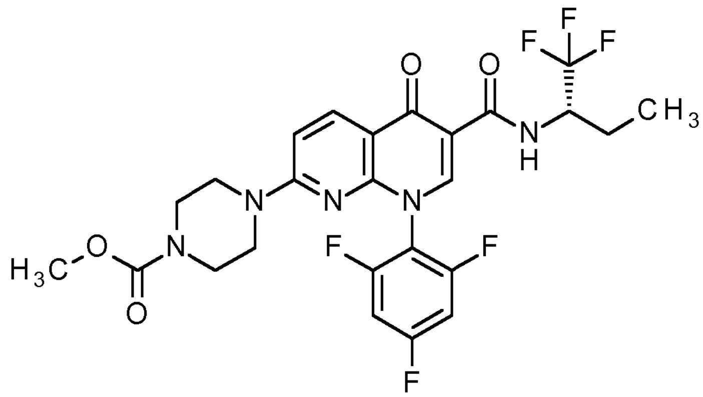

Gemäß AAV1 wurden 70.0 mg (151 μmol) der Verbindung aus Beispiel 157A mit 29.7 mg (182 μmol) (2*S*)-1,1,1-Trifluorbutan-2-amin Hydrochlorid in Gegenwart von 69.1 mg (182 μmol) HATU und 79 μl (450 μmol) DIPEA in 580 μl DMF zur Reaktion gebracht. Das Rohprodukt wurde mittels präparativer HPLC (Acetonitril / Wasser/ 0,1 % Ameisensäure) gereinigt. Es wurden 46.5 mg (54% d. Th., 100% Reinheit) der Titelverbindung erhalten.
LC-MS (Methode 3): $R_t$ = 2.11 min; MS (ESIpos): m/z = 572 $[M+H]^+$
$^1H$ NMR (400 MHz, DMSO-$d_6$): δ ppm = 10.36 (d, 1H), 8.84 (s, 1H), 8.33 (d, 1H), 7.57 (t, 2H), 7.14 (d, 1H), 4.69-4.79 (m, 1H), 3.61 (s, 3H), 3.48-3.58 (m, 4H), 3.36-3.44 (m, 4H), 1.83-1.92 (m, 1H), 1.59-1.69 (m, 1H), 0.97 (t, 3H).

**Beispiel 596**

4-Oxo-7-(2-oxoimidazolidin-1-yl)-*N*-[1-(trifluormethyl)cyclobutyl]-1-(2,4,6-trifluorphenyl)-1,4-dihydro-1,8-naphthyridin-3-carboxamid

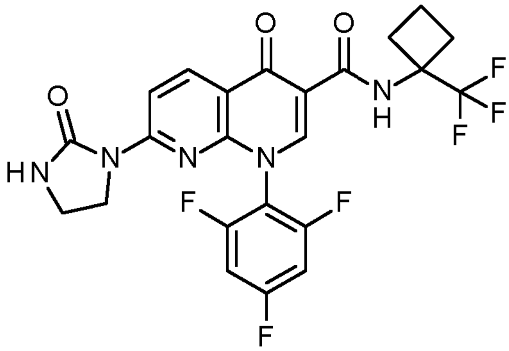

Gemäß AAV1 wurden 50.0 mg (124 μmol) der Verbindung aus Beispiel 113A mit 26.1 mg (148 μmol) 1-(Trifluormethyl)cyclobutanamin Hydrochlorid in Gegenwart von 56.4 mg (148 μmol) HATU und 65 μl (370 μmol) DIPEA in 480 μl DMF zur Reaktion gebracht. Das Rohprodukt wurde mittels präparativer HPLC (Acetonitril / Wasser/ 0,1 % Ameisensäure) gereinigt. Es wurden 41.6 mg (64% d. Th., 100% Reinheit) der Titelverbindung erhalten.
LC-MS (Methode 3): $R_t$ = 1.97 min; MS (ESIpos): m/z = 526 $[M+H]^+$
$^1H$ NMR (400 MHz, DMSO-$d_6$): δ ppm = 10.31 (s, 1H), 8.94 (s, 1H), 8.55 (d, 1H), 8.43 (d, 1H), 7.66 (s, 1H), 7.58 (t, 2H),

3.56-3.63 (m, 2H), 3.32-3.38 (m, 2H), 2.54-2.66 (m, 4H), 1.90-2.09 (m, 2H).

**Beispiel 597**

*N*-(2,6-Dichlorphenyl)-1-(2,4-difluorphenyl)-7-[1-hydroxy-3-azabicyclo[3.1.0]hex-3-yl]-4-oxo-1,4-dihydro-1,8-naphthyridin-3-carboxamid (Racemat)

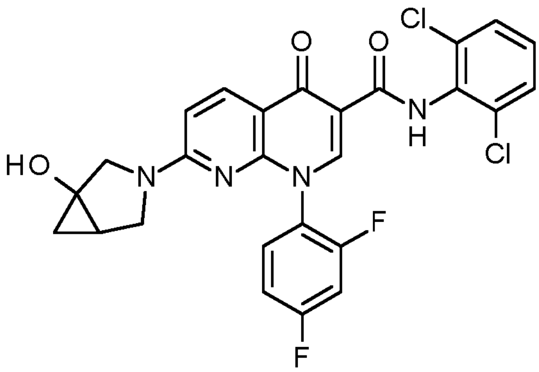

Gemäß AAV3 wurden 90.0 mg (187 µmol) der Verbindung aus Beispiel 81A mit 30.5 mg (225 µmol) 3-Azabicyclo[3.1.0]hexan-1-ol Hydrochlorid und 110 µl (660 µmol) DIPEA in 900 µl DMF zur Reaktion gebracht. Das Rohprodukt wurde mittels präparativer HPLC (Acetonitril / Wasser/ 0,1 % Ameisensäure) gereinigt. Es wurden 62.1 mg (61% d. Th., 100% Reinheit) der Titelverbindung erhalten.
LC-MS (Methode 3): $R_t$ = 2.02 min; MS (ESIpos): m/z = 543 $[M+H]^+$
$^1$H NMR (400 MHz, DMSO-$d_6$): δ ppm = 11.99 (s, 1H), 8.69 (s, 1H), 8.34 (d, 1H), 7.78-7.89 (m, 1H), 7.54-7.65 (m, 3H), 7.29-7.40 (m, 2H), 6.70-6.83 (m, 1H), 5.90-6.13 (m, 1H), 3.79-3.95 (m, 0.50H), 3.37-3.75 (m, 2.50H), 3.08-3.23 (m, 1H), 1.52-1.70 (m, 1H), 0.95-1.08 (m, 1H), 0.37-0.50 (m, 1H).

**Beispiel 598**

7-(3-Methoxy-3-methylazetidin-1-yl)-4-oxo-*N*-[(2*S*)-1,1,1-trifluor-3,3-dimethylbutan-2-yl]-1-(2,4,6-trifluorphenyl)-1,4-dihydro-1,8-naphthyridin-3-carboxamid

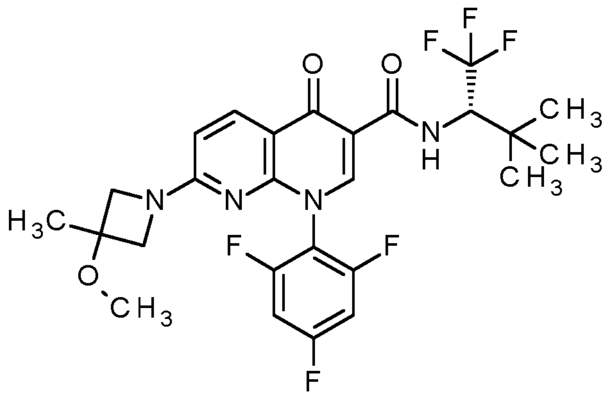

Gemäß AAV1 wurden 24.1 mg (57.5 µmol) der Verbindung aus Beispiel 161A mit 13.2 mg (69.0 µmol) (2*S*)-1,1,1-Trifluor-3,3-dimethylbutan-2-amin Hydrochlorid in Gegenwart von 26.3 mg (69.0 µmol) HATU und 30 µl (170 µmol) DIPEA in 230 µl DMF zur Reaktion gebracht. Das Rohprodukt wurde mittels präparativer HPLC (Acetonitril / Wasser/ 0,1 % Ameisensäure) gereinigt. Es wurden 23.2 mg (72% d. Th., 100% Reinheit) der Titelverbindung erhalten.
LC-MS (Methode 3): $R_t$ = 2.47 min; MS (ESIpos): m/z = 557 $[M+H]^+$
$^1$H NMR (400 MHz, DMSO-$d_6$): δ ppm = 10.73 (d, 1H), 8.81 (s, 1H), 8.33 (d, 1H), 7.54 (t, 2H), 6.64 (d, 1H), 4.62 (quint,

1H), 3.47-4.12 (m, 4H), 3.16 (s, 3H), 1.41 (s, 3H), 1.08 (s, 9H).

**Beispiel 599**

*N*-(1,1,1,3,3,3-Hexafluorpropan-2-yl)-4-oxo-7-(2-oxoimidazolidin-1-yl)-1-(2,4,6-trifluorphenyl)-1,4-dihydro-1,8-naphthyridin-3-carboxamid

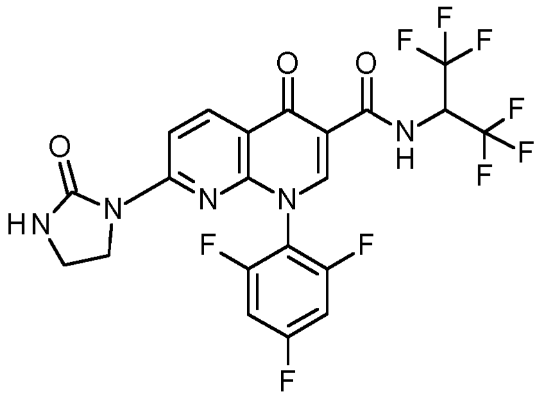

Gemäß AAV2 wurden 60.0 mg (119 µmol) der Verbindung aus Beispiel 154A mit 103 mg (1.19 mmol) Imidazolidin-2-on in Gegenwart von 24.7 mg (179 µmol) Kaliumcarbonat, 5.35 mg (23.8 µmol) Palladiumacetat und 13.8 mg (23.8 µmol) Xantphos in 1.2 ml 1,4-Dioxan zur Reaktion gebracht. Das Rohprodukt wurde mittels präparativer HPLC (Acetonitril / Wasser/ 0,1 % Ameisensäure) gereinigt. Es wurden 34.4 mg (52% d. Th., 100% Reinheit) der Titelverbindung erhalten.
LC-MS (Methode 3): $R_t$ = 2.05 min; MS (ESIpos): m/z = 554 $[M+H]^+$
$^1H$ NMR (400 MHz, DMSO-$d_6$): δ ppm = 11.15 (d, 1H), 9.12 (s, 1H), 8.58 (d, 1H), 8.46 (d, 1H), 7.69 (s, 1H), 7.59 (t, 2H), 6.33-6.40 (m, 1H), 3.57-3.62 (m, 2H), 3.33-3.38 (m, 2H).

**Beispiel 600**

*N*-[(1*S*)-1-Cyclopropyl-2,2,2-trifluorethyl]-7-[7-hydroxy-5-azaspiro[2.4]hept-5-yl]-4-oxo-1-(2,4,6-trifluorphenyl)-1,4-dihydro-1,8-naphthyridin-3-carboxamid (Diastereomerengemisch)

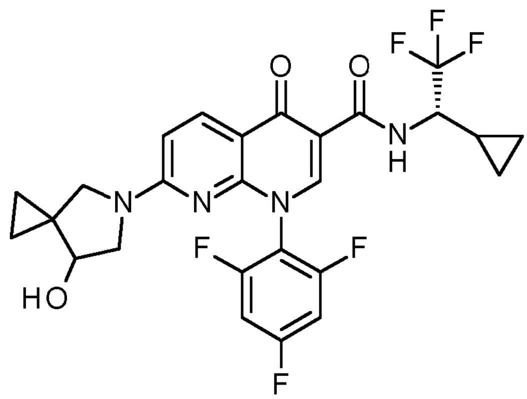

Gemäß AAV1 wurden 80.0 mg (185 µmol) der Verbindung aus Beispiel 155A mit 39.1 mg (223 µmol) (1*S*)-1-Cyclopropyl-2,2,2-trifluorethanamin Hydrochlorid in Gegenwart von 84.6 mg (223 µmol) HATU und 97 µl (560 µmol) DIPEA in 890 µl DMF zur Reaktion gebracht. Das Rohprodukt wurde mittels präparativer HPLC (Acetonitril / Wasser/ 0,1 % Ameisensäure) gereinigt. Es wurden 67.6 mg (66% d. Th., 100% Reinheit) der Titelverbindung erhalten.
LC-MS (Methode 4): $R_t$ = 3.62 min; MS (ESIpos): m/z = 553 $[M+H]^+$

$^{1}H$ NMR (400 MHz, DMSO-$d_6$): δ ppm = 10.56 (d, 1H), 8.77-8.82 (m, 1H), 8.29 (d, 1H), 7.46-7.64 (m, 2H), 6.81 (br d, 0.40H), 6.69 (br d, 0.60H), 4.95-5.11 (m, 1H), 4.33-4.43 (m, 1H), 3.59-3.78 (m, 2H), 3.40-3.48 (m, 1.60H), 3.13-3.27 (m, 1H), 2.89 (s, 0.40H), 1.16-1.25 (m, 1H), 0.77-0.86 (m, 1H), 0.46-0.69 (m, 6H), 0.29-0.38 (m, 1H).

59.0 mg der Titelverbindung (Diastereomerengemisch) wurde durch chirale HPLC in die Diastereomere getrennt (präparative HPLC: Säule YMC Chiralart Amylose SA 5 μm 250x30 mm; Eluent: 80% *n*-Heptan, 20% Ethanol; Temperatur: 25°C; Fluss: 30 ml/min; UV-Detektion: 220 nm.)

Man erhielt (in der Reihenfolge der Elution von der Säule) 23.0 mg (22% d. Th., 100% Reinheit) Diastereomer 1 aus Beispiel 601 (99% de) Rt = 9.88 min und 22.0 mg (21% d. Th., 100% Reinheit) Diastereomer 2 aus Beispiel 602 (99% de) Rt = 12.57 min.

[Analytische HPLC: Säule YMC Chiralart Amylose SA 5 μm 250x4.6 mm; Eluent: 80% Iso-Hexan, 20% Ethanol; Temperatur: 30°C; Fluss: 1.0 ml/min; UV-Detektion: 220nm].

**Beispiel 601**

*N*-[(1*S*)-1-Cyclopropyl-2,2,2-trifluorethyl]-7-[7-hydroxy-5-azaspiro[2.4]hept-5-yl]-4-oxo-1-(2,4,6-trifluorphenyl)-1,4-dihydro-1,8-naphthyridin-3-carboxamid (Diastereomer 1)

LC-MS (Methode 3): $R_t$ = 2.08 min; MS (ESIpos): m/z = 553 $[M+H]^+$
$^{1}H$ NMR (400 MHz, DMSO-$d_6$): δ ppm = 10.56 (d, 1H), 8.77-8.82 (m, 1H), 8.29 (d, 1H), 7.46-7.64 (m, 2H), 6.81 (br d, 0.40H), 6.69 (br d, 0.60H), 5.07 (br d, 0.40H), 4.98 (br d, 0.60H), 4.33-4.44 (m, 1H), 3.59-3.79 (m, 2H), 3.31-3.47 (m, 1.60H), 3.14-3.27 (m, 1H), 2.88 (br d, 0.40H), 1.14-1.28 (m, 1H), 0.76-0.88 (m, 1H), 0.46-0.69 (m, 6H), 0.29-0.39 (m, 1H).

**Beispiel 602**

*N*-[(1*S*)-1-Cyclopropyl-2,2,2-trifluorethyl]-7-[7-hydroxy-5-azaspiro[2.4]hept-5-yl]-4-oxo-1-(2,4,6-trifluorphenyl)-1,4-dihydro-1,8-naphthyridin-3-carboxamid (Diastereomer 2)

LC-MS (Methode 3): $R_t$ = 2.08 min; MS (ESIpos): m/z = 553 $[M+H]^+$
$^{1}H$ NMR (400 MHz, DMSO-$d_6$): δ ppm = 10.56 (d, 1H), 8.77-8.82 (m, 1H), 8.29 (d, 1H), 7.49-7.60 (m, 2H), 6.81 (br d, 0.40H), 6.69 (br d, 0.60H), 5.08 (br d, 0.40H), 4.98 (br d, 0.60H), 4.33-4.43 (m, 1H), 3.59-3.79 (m, 2H), 3.31-3.49 (m, 1.60H), 3.21 (br dd, 1H), 2.83-2.93 (m, 0.40H), 1.16-1.26 (m, 1H), 0.76-0.87 (m, 1H), 0.43-0.71 (m, 6H), 0.29-0.39 (m, 1H).

**Beispiel 603**

4-[6-{[(1*S*)-1-cyclopropyl-2,2,2-trifluorethyl]carbamoyl}-5-oxo-8-(2,4,6-trifluorphenyl)-5,8-dihydro-1,8-naphthyridin-2-yl]piperazin-1-carbonsäuremethylester

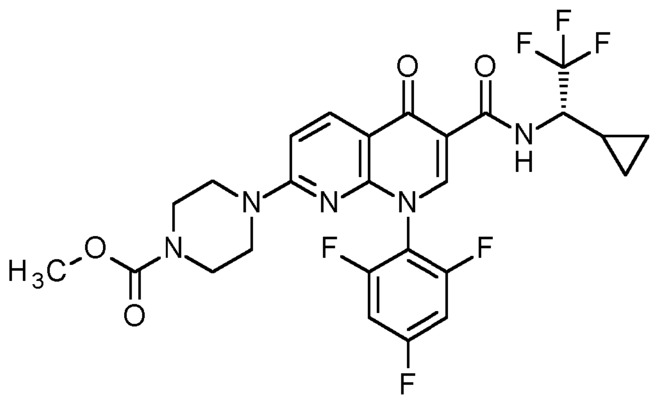

Gemäß AAV1 wurden 70.0 mg (151 μmol) der Verbindung aus Beispiel 157A mit 31.9 mg (182 μmol) (1*S*)-1-Cyclopropyl-2,2,2-trifluorethanamin Hydrochlorid in Gegenwart von 69.1 mg (182 μmol) HATU und 79 μl (450 μmol) DIPEA in 580 μl DMF zur Reaktion gebracht. Das Rohprodukt wurde mittels präparativer HPLC (Acetonitril / Wasser/ 0,1 % Ameisensäure) gereinigt. Es wurden 47.9 mg (54% d. Th., 100% Reinheit) der Titelverbindung erhalten.
LC-MS (Methode 3): $R_t$ = 2.13 min; MS (ESIpos): m/z = 584 $[M+H]^+$

$^1H$ NMR (400 MHz, DMSO-$d_6$): δ ppm = 10.49 (d, 1H), 8.83 (s, 1H), 8.33 (d, 1H), 7.53-7.60 (m, 2H), 7.14 (d, 1H), 4.33-4.43 (m, 1H), 3.61 (s, 3H), 3.49-3.57 (m, 4H), 3.36-3.44 (m, 4H), 1.16-1.25 (m, 1H), 0.50-0.69 (m, 3H), 0.30-0.38 (m, 1H).

## Beispiel 604

*N*-[(1*S*)-1-Cyclopropyl-2,2,2-trifluorethyl]-4-oxo-7-(2-oxotetrahydropyrimidin-1(2*H*)-yl)-1-(2,4,6-trifluorphenyl)-1,4-dihydro-1,8-naphthyridin-3 -carboxamid

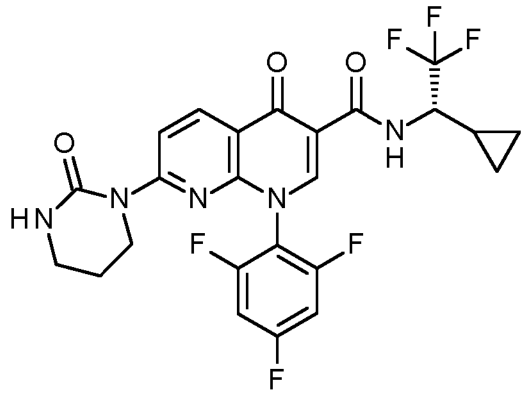

Gemäß AAV2 wurden 60.0 mg (126 μmol) der Verbindung aus Beispiel 126A mit 126 mg (1.26 mmol) Tetrahydropyrimidin-2(1*H*)-on in Gegenwart von 26.1 mg (189 μmol) Kaliumcarbonat, 5.66 mg (25.2 μmol) Palladiumacetat und 14.6 mg (25.2 μmol) Xantphos in 1.2 ml 1,4-Dioxan zur Reaktion gebracht. Das Rohprodukt wurde mittels präparativer HPLC (Acetonitril / Wasser/ 0,1 % Ameisensäure) gereinigt. Es wurden 46.0 mg (68% d. Th., 100% Reinheit) der Titelverbindung erhalten.
LC-MS (Methode 3): $R_t$ = 1.94 min; MS (ESIpos): m/z = 540 $[M+H]^+$
$^1H$ NMR (400 MHz, DMSO-$d_6$): δ ppm = 10.33 (d, 1H), 9.01 (s, 1H), 8.52 (d, 1H), 8.30 (d, 1H), 7.60 (t, 2H), 7.38-7.41 (m, 1H), 4.35-4.45 (m, 1H), 3.50 (brt, 2H), 3.15 (td, 2H), 1.80-1.88 (m, 2H), 1.19-1.27 (m, 1H), 0.52-0.70 (m, 3H), 0.31-0.38 (m, 1H).

## Beispiel 605

*N*-[(1*R*)-1-Cyclopropyl-2,2,2-trifluorethyl]-7-(3-methoxy-3-methylazetidin-1-yl)-4-oxo-1-(2,4,6-trifluorphenyl)-1,4-dihydro-1,8-naphthyridin-3-carboxamid

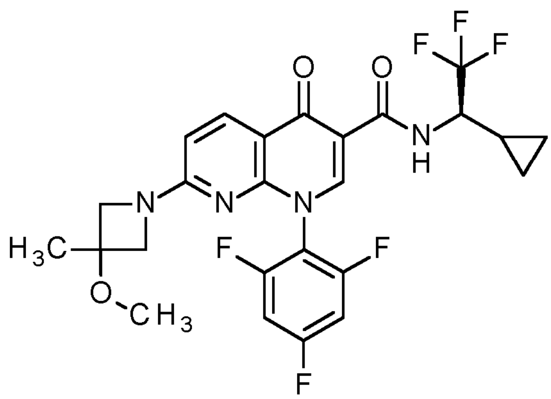

Gemäß AAV1 wurden 24.0 mg (57.2 μmol) der Verbindung aus Beispiel 161A mit 12.1 mg (68.7 μmol) (1*R*)-1-Cyclopropyl-2,2,2-trifluorethanamin Hydrochlorid in Gegenwart von 26.1 mg (68.7 μmol) HATU und 30 μl (170 μmol)

DIPEA in 230 µl DMF zur Reaktion gebracht. Das Rohprodukt wurde mittels präparativer HPLC (Acetonitril / Wasser/ 0,1 % Ameisensäure) gereinigt. Es wurden 23.9 mg (77% d. Th., 100% Reinheit) der Titelverbindung erhalten.
LC-MS (Methode 3): $R_t$ = 2.32 min; MS (ESIpos): m/z = 541 $[M+H]^+$
$^1H$ NMR (400 MHz, DMSO-$d_6$): δ ppm = 10.52 (d, 1H), 8.80 (s, 1H), 8.30 (d, 1H), 7.50-7.58 (m, 2H), 6.64 (d, 1H), 4.33-4.43 (m, 1H), 3.50-4.11 (m, 3H), 3.16 (s, 3H), 1.41 (s, 3H), 1.16-1.25 (m, 1H), 0.49-0.69 (m, 3H), 0.30-0.37 (m, 1H).

**Beispiel 606**

*N*-[1,1,1,4,4,4-Hexafluorbutan-2-yl]-7-[(4*S*)-4-hydroxy-2-oxopyrrolidin-1-yl]-4-oxo-1-(2,4,6-trifluorphenyl)-1,4-dihydro-1,8-naphthyridin-3-carboxamid (Diastereomerengemisch)

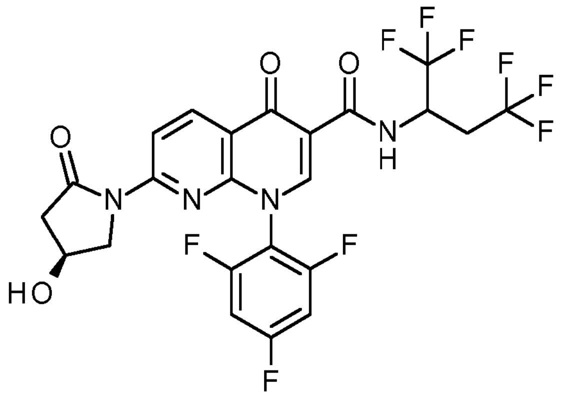

Gemäß AAV1 wurden 40.0 mg (95.4 µmol) der Verbindung aus Beispiel 117A mit 29.1 mg (134 µmol) 1,1,1,4,4,4-Hexafluorbutan-2-amin Hydrochlorid in Gegenwart von 43.5 mg (114 µmol) HATU und 50 µl (290 µmol) DIPEA in 350 µl DMF zur Reaktion gebracht. Das Rohprodukt wurde mittels präparativer HPLC (Acetonitril / Wasser/ 0,1 % Ameisensäure) gereinigt. Es wurden 36.5 mg (66% d. Th., 100% Reinheit) der Titelverbindung erhalten.
LC-MS (Methode 1): $R_t$ = 1.00 min; MS (ESIpos): m/z = 583 $[M+H]^+$
$^1H$ NMR (400 MHz, DMSO-$d_6$): δ ppm = 10.37 (d, 1H), 9.09 (s, 1H), 8.71 (d, 1H), 8.55 (d, 1H), 7.58-7.66 (m, 2H), 5.21-5.37 (m, 2H), 4.29 (br s, 1H), 3.69 (dd, 1H), 3.47 (d, 1H), 3.11-3.22 (m, 1H), 2.90-3.03 (m, 2H), 2.38 (br d, 1H).

36.0 mg der Titelverbindung (Diastereomerengemisch) wurde durch chirale HPLC in die Diastereomere getrennt (präparative HPLC: Säule Daicel Chiralcel OX-H 5 µm 250x20 mm; Eluent: 80% *n*-Heptan, 20% Iso-Propanol; Temperatur: 23°C; Fluss: 20 ml/min; UV-Detektion: 220 nm.)

Man erhielt (in der Reihenfolge der Elution von der Säule) 10.50 mg (19% d. Th., 100% Reinheit) Diastereomer 1 aus Beispiel 607 (99% de) Rt = 6.32 min und 6.90 mg (12% d. Th., 100% Reinheit) Diastereomer 2 aus Beispiel 608 (94% de) Rt = 7.62 min.

[Analytische HPLC: Säule Daicel OX-3 3 µm 50x4.6 mm; Eluent: 80% Iso-Hexan, 20% Iso-Propanol; UV-Detektion: 220nm].

**Beispiel 607**

*N*-[1,1,1,4,4,4-Hexafluorbutan-2-yl]-7-[(4*S*)-4-hydroxy-2-oxopyrrolidin-1-yl]-4-oxo-1-(2,4,6-trifluorphenyl)-1,4-dihydro-1,8-naphthyridin-3-carboxamid (Diastereomer 1)

LC-MS (Methode 3): $R_t$ = 1.86 min; MS (ESIpos): m/z = 583 $[M+H]^+$
$^1H$ NMR (400 MHz, DMSO-$d_6$): δ ppm = 10.37 (d, 1H), 9.09 (s, 1H), 8.71 (d, 1H), 8.54 (d, 1H), 7.58-7.66 (m, 2H), 5.22-5.34 (m, 1H), 4.29 (br t, 1H), 3.69 (dd, 1H), 3.44-3.52 (m, 2H), 3.10-3.23 (m, 1H), 2.90-3.01 (m, 2H), 2.38 (d, 1H).

**Beispiel 608**

*N*-[1,1,1,4,4,4-Hexafluorbutan-2-yl]-7-[(4*S*)-4-hydroxy-2-oxopyrrolidin-1-yl]-4-oxo-1-(2,4,6-trifluorphenyl)-1,4-dihydro-1,8-naphthyridin-3-carboxamid (Diastereomer 2)

LC-MS (Methode 3): $R_t$ = 1.85 min; MS (ESIpos): m/z = 583 $[M+H]^+$

$^1H$ NMR (400 MHz, DMSO-$d_6$): δ ppm = 10.37 (d, 1H), 9.09 (s, 1H), 8.71 (d, 1H), 8.55 (d, 1H), 7.57-7.66 (m, 2H), 5.21-5.34 (m, 1H), 4.27-4.31 (m, 1H), 3.69 (dd, 1H), 3.11-3.22 (m, 1H), 2.91-3.02 (m, 2H), 2.38 (br d, 1H).

**Beispiel 609**

4-Oxo-7-(2-oxoimidazolidin-1-yl)-*N*-[1-(trifluormethoxy)butan-2-yl]-1-(2,4,6-trifluorphenyl)-1,4-dihydro-1,8-naphthyridin-3-carboxamid (Racemat)

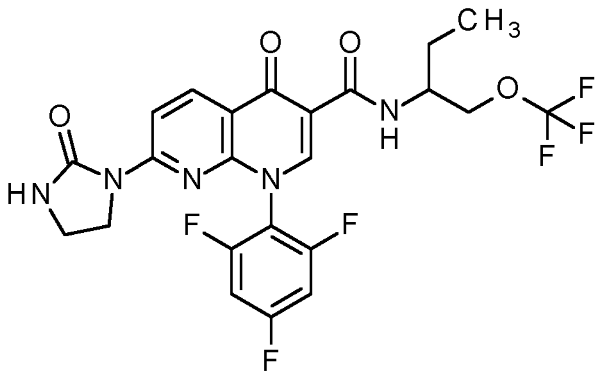

Gemäß AAV1 wurden 300 mg (742 μmol) der Verbindung aus Beispiel 113A mit 201 mg (1.04 mmol) 1-(Trifluormethoxy)butan-2-amin Hydrochlorid in Gegenwart von 339 mg (890 μmol) HATU und 390 μl (2.20 mmol) DIPEA in 2.8 ml DMF zur Reaktion gebracht. Das Rohprodukt wurde mittels präparativer HPLC (Acetonitril / Wasser/ 0,1 % Ameisensäure) gereinigt. Es wurden 224 mg (52% d. Th., 94% Reinheit) der Titelverbindung erhalten.
LC-MS (Methode 3): $R_t$ = 1.92 min; MS (ESIpos): m/z = 544 $[M+H]^+$
$^1H$ NMR (400 MHz, DMSO-$d_6$): δ ppm = 9.89 (br d, 1H), 8.92 (s, 1H), 8.55 (d, 1H), 8.42 (d, 1H), 7.65 (s, 1H), 7.53-7.63 (m, 2H), 4.15-4.25 (m, 3H), 3.54-3.64 (m, 2H), 3.32-3.38 (m, 2H), 1.55-1.74 (m, 2H), 0.95 (t, 3H).

216 mg der Titelverbindung (Racemat) wurde durch chirale HPLC in die Enantiomere getrennt (präparative HPLC: Säule YMC Chiralart Cellulose SB 5 μm 250x20 mm; Eluent: 70% *n*-Heptan, 30% Iso-Propanol; Temperatur: 30°C; Fluss: 15 ml/min; UV-Detektion: 220 nm.)

Man erhielt (in der Reihenfolge der Elution von der Säule) 91.0 mg (21% d. Th., 100% Reinheit) Enantiomer 1 aus Beispiel 610 (99% ee) Rt = 12.46 min und 88.0 mg (20% d. Th., 100% Reinheit) Enantiomer 2 aus Beispiel 611 (98% ee) Rt = 14.51 min.

[Analytische HPLC: Säule YMC Chiralart Amylose SB 5 μm 250x4.6 mm; Eluent: 70% Iso-Hexan, 30% Iso-Propanol; Temperatur: 40°C; Fluss: 1.0 ml/min; UV-Detektion: 220nm].

**Beispiel 610**

4-Oxo-7-(2-oxoimidazolidin-1-yl)-*N*-[1-(trifluormethoxy)butan-2-yl]-1-(2,4,6-trifluorphenyl)-1,4-dihydro-1,8-naphthyridin-3-carboxamid (Enantiomer 1)

LC-MS (Methode 3): $R_t$ = 1.94 min; MS (ESIpos): m/z = 544 $[M+H]^+$
$^1H$ NMR (400 MHz, DMSO-$d_6$): δ ppm = 9.89 (br d, 1H), 8.92 (s, 1H), 8.55 (d, 1H), 8.42 (d, 1H), 7.65 (s, 1H), 7.54-7.61 (m, 2H), 4.15-4.24 (m, 3H), 3.55-3.63 (m, 2H), 3.32-3.38 (m, 2H), 1.55-1.74 (m, 2H), 0.95 (t, 3H).

**Beispiel 611**

4-Oxo-7-(2-oxoimidazolidin-1-yl)-*N*-[1-(trifluormethoxy)butan-2-yl]-1-(2,4,6-trifluorphenyl)-1,4-dihydro-1,8-naphthyridin-3-carboxamid (Enantiomer 2)

LC-MS (Methode 3): $R_t$ = 1.94 min; MS (ESIpos): m/z = 544 $[M+H]^+$
$^1H$ NMR (400 MHz, DMSO-$d_6$): δ ppm = 9.89 (br d, 1H), 8.92 (s, 1H), 8.55 (d, 1H), 8.42 (d, 1H), 7.65 (s, 1H), 7.53-7.62 (m, 2H), 4.15-4.24 (m, 3H), 3.55-3.63 (m, 2H), 3.32-3.38 (m, 2H), 1.54-1.74 (m, 2H), 0.95 (t, 3H).

**Beispiel 612**

7-[(3*R*,4*S*)-3,4-Dihydroxypyrrolidin-1-yl]-4-oxo-*N*-(4,4,4-trifluor-2-methylbutan-2-yl)-1-(2,4,6-trifluorhenl-1,4-dihdro-1,8-nahthridin-3-carboxamid

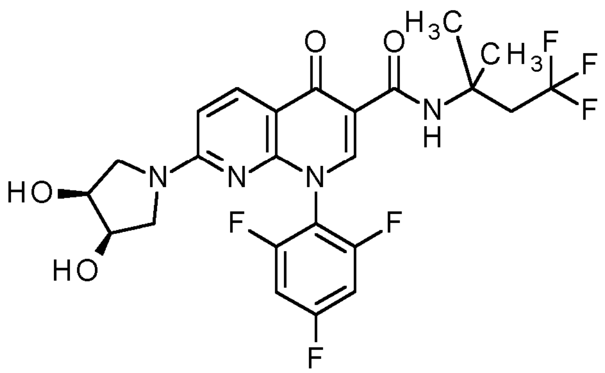

Gemäß AAV1 wurden 60.0 mg (142 µmol) der Verbindung aus Beispiel 156A mit 30.3 mg (171 µmol) 4,4,4-Trifluor-2-methylbutan-2-amin Hydrochlorid in Gegenwart von 65.0 mg (171 µmol) HATU und 99 µl (570 µmol) DIPEA in 1.2 ml DMF zur Reaktion gebracht. Das Rohprodukt wurde mittels präparativer HPLC (Acetonitril / Wasser/ 0,1 % Ameisensäure) gereinigt. Es wurden 56.0 mg (72% d. Th., 100% Reinheit) der Titelverbindung erhalten.
LC-MS (Methode 3): $R_t$ = 1.75 min; MS (ESIpos): m/z = 545 $[M+H]^+$
$^1H$ NMR (400 MHz, DMSO-$d_6$): δ ppm = 10.16 (s, 1H), 8.69 (s, 1H), 8.25 (d, 1H), 7.52-7.60 (m, 2H), 6.73 (d, 1H), 5.03 (d, 1H), 4.93 (d, 1H), 4.09-4.17 (m, 1H), 3.99-4.07 (m, 1H), 3.55-3.63 (m, 1H), 3.19-3.30 (m, 2H), 2.89-3.04 (m, 3H), 1.48 (s, 6H).

**Beispiel 613**

*N*-(2,6-Dichlorphenyl)-4-oxo-7-(2-oxoimidazolidin-1-yl)-1-(2,4,6-trifluorphenyl)-1,4-dihydro-1,8-naphthyridin-3 -carboxamid

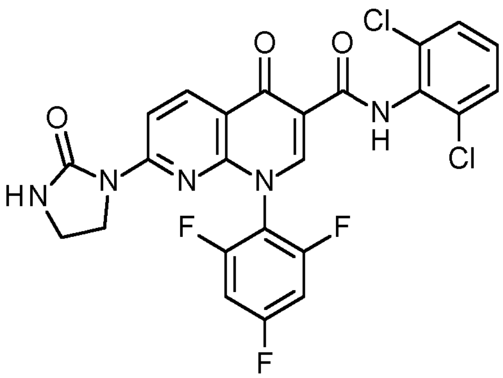

Gemäß AAV2 wurden 60.0 mg (120 µmol) der Verbindung aus Beispiel 160B mit 147 mg (1.20 mmol) Imidazolidin-2-on Hydrochlorid in Gegenwart von 24.9 mg (180 µmol) Kaliumcarbonat, 5.40 mg (24.1 µmol) Palladiumacetat und 13.9 mg (24.1 µmol) Xantphos in 1.1 ml 1,4-Dioxan zur Reaktion gebracht. Das Rohprodukt wurde mittels präparativer HPLC (Acetonitril / Wasser/ 0,1 % Ameisensäure) gereinigt. Die Substanz wurde mit Verunreinigungen isoliert und wurde weiter aus Acetonitril umkristallisiert, abgesaugt, mit etwas Acetonitril nachgewaschen und nachgetrocknet. Es wurden 38.5 mg (58% d. Th., 100% Reinheit) der Titelverbindung erhalten.
LC-MS (Methode 3): $R_t$ = 1.95 min; MS (ESIpos): m/z = 548 $[M+H]^+$
$^1H$ NMR (400 MHz, DMSO-$d_6$): δ ppm = 11.70 (s, 1H), 9.08 (s, 1H), 8.62 (d, 1H), 8.47 (d, 1H), 7.68 (s, 1H), 7.54-7.63

(m, 4H), 7.39 (t, 1H), 3.57-3.65 (m, 2H), 3.33-3.39 (m, 2H).

**Beispiel 614**

*N*-(1,1-Difluor-2-methylpropan-2-yl)-4-oxo-7-(2-oxoimidazolidin-1-yl)-1-(2,4,6-trifluorphenyl)-1,4-dihydro-1,8-naphthyridin-3-carboxamid

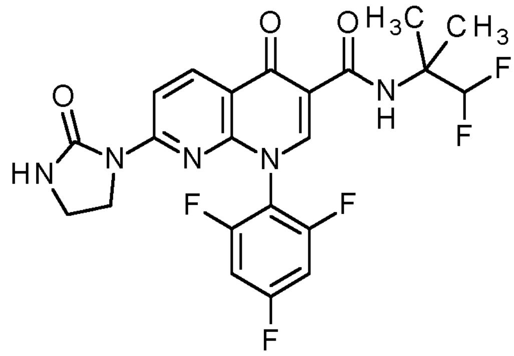

Gemäß AAV1 wurden 50.0 mg (124 µmol) der Verbindung aus Beispiel 113A mit 25.2 mg (173 µmol) 1,1-Difluor-2-methylpropan-2-amin Hydrochlorid in Gegenwart von 56.4 mg (148 µmol) HATU und 65 µl (370 µmol) DIPEA in 460 µl DMF zur Reaktion gebracht. Das Rohprodukt wurde mittels präparativer HPLC (Acetonitril / Wasser/ 0,1 % Ameisensäure) gereinigt. Es wurden 34.9 mg (57% d. Th., 100% Reinheit) der Titelverbindung erhalten.
LC-MS (Methode 3): $R_t$ = 1.86 min; MS (ESIpos): m/z = 496 $[M+H]^+$
$^1$H NMR (400 MHz, DMSO-$d_6$): δ ppm = 10.12 (s, 1H), 8.91 (s, 1H), 8.55 (d, 1H), 8.42 (d, 1H), 7.65 (s, 1H), 7.53-7.61 (m, 2H), 6.43 (t, 1H), 3.53-3.65 (m, 2H), 3.32-3.38 (m, 2H), 1.45 (s, 6H).

**Beispiel 615**

*N*-[(1*S*)-1-Cyclopropyl-2,2,2-trifluorethyl]-7-[*trans*-3,4-dihydroxypiperidin-1-yl]-4-oxo-1-(2,4,6-trifluorphenyl)-1,4-dihydro-1,8-naphthyridin-3-carboxamid (Diastereomerengemisch)

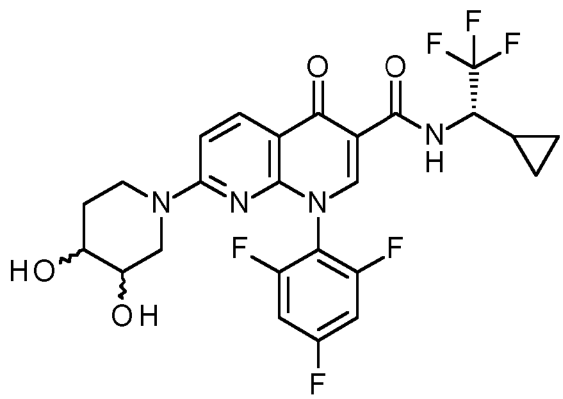

Gemäß AAV3 wurden 150 mg (315 µmol) der Verbindung aus Beispiel 126A mit 58.1 mg (378 µmol) Piperidin-3,4-diol Hydrochlorid und 160 µl (950 µmol) DIPEA in 1.3 ml DMF zur Reaktion gebracht. Das Rohprodukt wurde mittels präparativer HPLC (Acetonitril / Wasser/ 0,1 % Ameisensäure) gereinigt. Es wurden 153 mg (87% d. Th., 100% Reinheit) der Titelverbindung erhalten.
LC-MS (Methode 4): $R_t$ = 2.95 min; MS (ESIpos): m/z = 557 $[M+H]^+$
$^1$H NMR (400 MHz, DMSO-$d_6$): δ ppm = 10.53 (d, 1H), 8.80 (s, 1H), 8.22-8.28 (m, 1H), 7.52-7.60 (m, 2H), 7.12 (d, 1H), 4.98 (br s, 1H), 4.86 (br s, 1H), 4.33-4.43 (m, 1H), 3.75-3.87 (m, 1H), 3.56-3.75 (m, 1H), 3.39-3.47 (m, 1H), 3.10-3.27

(m, 3H), 1.72-1.82 (m, 1H), 1.16-1.27 (m, 2H), 0.49-0.69 (m, 3H), 0.31-0.37 (m, 1H).

142 mg der Titelverbindung (Diastereomerengemisch) wurde durch chirale HPLC in die Diastereomere getrennt (präparative SFC: Säule Chiralpak AD 250x20 mm; Eluent: 85% Kohlendioxid, 15% Ethanol; Temperatur: 40°C; Fluss: 80 ml/min; UV-Detektion: 210 nm.)

Man erhielt (in der Reihenfolge der Elution von der Säule) 63.80 mg (15% d. Th., 100% Reinheit) Diastereomer 1 aus Beispiel 616 (99% de) Rt = 7.56 min und 62.10 mg (18% d. Th., 100% Reinheit) Diastereomer 2 aus Beispiel 617 (96% de) Rt = 9.25 min.

[Analytische SFC: Säule AD; Eluent: 80% Kohlendioxid, 20% Ethanol; Fluss: 3.0 ml/min; UV-Detektion: 210nm].

## Beispiel 616

*N*-[(1*S*)-1-Cyclopropyl-2,2,2-trifluorethyl]-7-[*trans*-3,4-dihydroxypiperidin-1-yl]-4-oxo-1-(2,4,6-trifluorphenyl)-1,4-dihydro-1,8-naphthyridin-3-carboxamid (Diastereomer 1)

LC-MS (Methode 1): $R_t$ = 0.96 min; MS (ESIpos): m/z = 557 $[M+H]^+$
$^1H$ NMR (600 MHz, DMSO-$d_6$): δ ppm = 10.54 (d, 1H), 8.80 (s, 1H), 8.26 (d, 1H), 7.54-7.60 (m, 2H), 7.12 (d, 1H), 4.96-5.03 (m, 1H), 4.88 (d, 1H), 4.34-4.41 (m, 1H), 3.76-3.84 (m, 1H), 3.52-3.74 (m, 1H), 3.39-3.46 (m, 1H), 3.05-3.29 (m, 2H), 1.71-1.82 (m, 1H), 1.17-1.26 (m, 2H), 0.63-0.68 (m, 1H), 0.51-0.60 (m, 2H), 0.31-0.35 (m, 1H).

## Beispiel 617

*N*-[(1*S*)-1-Cyclopropyl-2,2,2-trifluorethyl]-7-[*trans*-3,4-dihydroxypiperidin-1-yl]-4-oxo-1-(2,4,6-trifluorphenyl)-1,4-dihydro-1,8-naphthyridin-3-carboxamid (Diastereomer 2)

LC-MS (Methode 1): $R_t$ = 0.96 min; MS (ESIpos): m/z = 557 $[M+H]^+$
$^1H$ NMR (600 MHz, DMSO-$d_6$): δ ppm = 10.54 (d, 1H), 8.80 (s, 1H), 8.26 (d, 1H), 7.54-7.60 (m, 2H), 7.12 (d, 1H), 5.00 (br d, 1H), 4.88 (d, 1H), 4.34-4.41 (m, 1H), 3.77-3.84 (m, 1H), 3.68 (br s, 1H), 3.38-3.48 (m, 2H), 3.18-3.26 (m, 2H), 1.76 (br s, 1H), 1.21-1.28 (m, 1H), 1.16-1.26 (m, 1H), 0.63-0.68 (m, 1H), 0.51-0.60 (m, 2H), 0.33 (br dd, 1H).

## Beispiel 618

1-(2,6-Difluorphenyl)-7-[(3*R*,4*R*)-3,4-dihydroxypyrrolidin-1-yl]-4-oxo-*N*-[(2*R*)-1,1,1-trifluorbutan-2-yl]-1,4-dihydro-1,8-naphthyridin-3-carboxamid

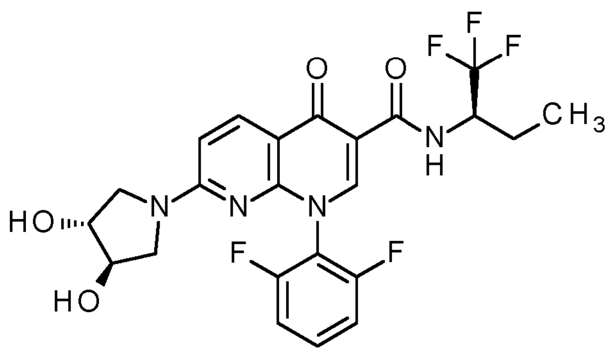

Zu einer Lösung von 115 mg (155 μmol) der Verbindung aus Beispiel 165A in 1.3 ml THF wurden 350 μl (1.0 M in THF, 350 μmol) Tetra-n-butylammoniumfluorid gegeben und die Reaktionsmischung für 2h bei Raumtemperatur nachgerührt. Das Lösungsmittel wurde unter reduziertem Druck entfernt und der Rückstand wurde mittels Normalphasenchromatographie (Essigsäureethylester-Cyclohexan-Gradient) gereinigt. Die Substanz wurde mit Verunreinigungen isoliert und wurde weiter mittels präparativer HPLC (Acetonitril / Wasser/ 0.1% Ameisensäure) gereinigt. Es wurden 26 mg (33% d. Th., 100% Reinheit) der Titelverbindung erhalten.
LC-MS (Methode 3): $R_t$ = 1.63 min; MS (ESIpos): m/z = 513 $[M+H]^+$
$^1H$ NMR (400 MHz, DMSO-$d_6$): δ ppm = 10.45 (d, 1H), 8.75 (s, 1H), 8.28 (d, 1H), 7.67-7.75 (m, 1H), 7.42 (t, 2H), 6.78 (d, 1H), 5.22 (d, 1H), 5.13 (d, 1H), 4.68-4.79 (m, 1H), 4.04 (br s, 1H), 3.89 (br s, 1H), 3.57-3.64 (m, 1H), 3.32-3.37 (m,

1H), 3.19 (dd, 1H), 3.02 (d, 1H), 1.83-1.93 (m, 1H), 1.59-1.70 (m, 1H), 0.97 (t, 3H).

**Beispiel 619**

7-[7-Hydroxy-5-azaspiro[2.4]hept-5-yl]-4-oxo-*N*-[(2*S*)-1,1,1-trifluorbutan-2-yl]-1-(2,4,6-trifluorphenyl)-1,4-dihydro-1,8-naphthyridin-3-carboxamid (Diastereomerengemisch)

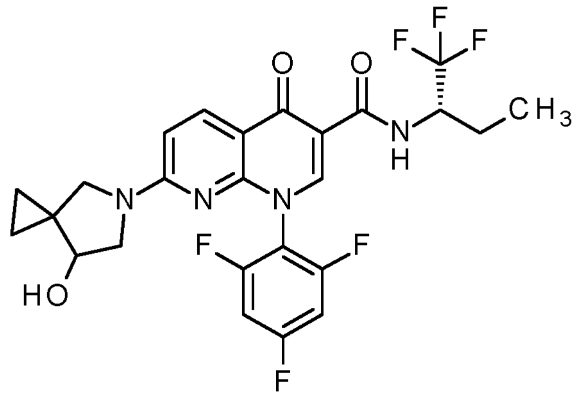

Gemäß AAV1 wurden 80.0 mg (185 μmol) der Verbindung aus Beispiel 155A mit 36.4 mg (223 μmol) (2*S*)-1,1,1-Trifluorbutan-2-amin Hydrochlorid in Gegenwart von 84.6 mg (223 μmol) HATU und 97 μl (560 μmol) DIPEA in 890 μl DMF zur Reaktion gebracht. Das Rohprodukt wurde mittels präparativer HPLC (Acetonitril / Wasser/ 0.1% Ameisensäure) gereinigt. Es wurden 90.2 mg (90% d. Th., 100% Reinheit) der Titelverbindung erhalten.
LC-MS (Methode 4): $R_t$ = 3.57 min; MS (ESIpos): m/z = 541 $[M+H]^+$
$^1H$ NMR (400 MHz, DMSO-$d_6$): δ ppm = 10.43 (d, 1H), 8.77-8.82 (m, 1H), 8.28 (d, 1H), 7.49-7.61 (m, 2H), 6.80 (br d, 0.40H), 6.69 (d, 0.60H), 5.04-5.11 (m, 0.40H), 4.95-5.02 (m, 0.60H), 4.68-4.78 (m, 1H), 3.61-3.78 (m, 2H), 3.34-3.49 (m, 1.60H), 3.14-3.26 (m, 1H), 2.84-2.92 (m, 0.40H), 1.83-1.92 (m, 1H), 1.58-1.70 (m, 1H), 0.97 (t, 3H), 0.77-0.86 (m, 1H), 0.44-0.63 (m, 3H).

78.7 mg der Titelverbindung (Diastereomerengemisch) wurde durch chirale HPLC in die Diastereomere getrennt (präparative HPLC: Säule Daicel Chiralpak IA 5 μm 250x20 mm; Eluent: 80% *n*-Heptan, 20% Ethanol; Temperatur: 25°C; Fluss: 15 ml/min; UV-Detektion: 210 nm.)

Man erhielt (in der Reihenfolge der Elution von der Säule) 33.4 mg (33% d. Th., 100% Reinheit) Diastereomer 1 aus Beispiel 620 (99% de) Rt = 8.04 min und 34.5 mg (34% d. Th., 100% Reinheit) Diastereomer 2 aus Beispiel 621 (98% de) Rt = 9.82 min.

[Analytische HPLC: Säule Daicel IA-3 3 μm 50x4.6 mm; Eluent: 80% Iso-Hexan, 20% Ethanol; UV-Detektion: 220nm].

**Beispiel 620**

7-[7-Hydroxy-5-azaspiro[2.4]hept-5-yl]-4-oxo-*N*-[(2*S*)-1,1,1-trifluorbutan-2-yl]-1-(2,4,6-trifluorphenyl)-1,4-dihydro-1,8-naphthyridin-3-carboxamid (Diastereomer 1)

LC-MS (Methode 1): $R_t$ = 1.10 min; MS (ESIpos): m/z = 541 $[M+H]^+$
$^1H$ NMR (400 MHz, DMSO-$d_6$): δ ppm = 10.43 (d, 1H), 8.78-8.83 (m, 1H), 8.28 (d, 1H), 7.49-7.61 (m, 2H), 6.81 (br d, 0.40H), 6.69 (br d, 0.60H), 4.93-5.13 (m, 1H), 4.68-4.79 (m, 1H), 3.60-3.78 (m, 2H), 3.32-3.48 (m, 1.60H), 3.14-3.27 (m, 1H), 2.88 (br d, 0.40H), 1.83-1.93 (m, 1H), 1.58-1.70 (m, 1H), 0.97 (t, 3H), 0.76-0.88 (m, 1H), 0.44-0.63 (m, 3H).

**Beispiel 621**

7-[7-Hydroxy-5-azaspiro[2.4]hept-5-yl]-4-oxo-*N*-[(2*S*)-1,1,1-trifluorbutan-2-yl]-1-(2,4,6-trifluorphenyl)-1,4-dihydro-1,8-naphthyridin-3-carboxamid (Diastereomer 2)

LC-MS (Methode 1): $R_t$ = 1.10 min; MS (ESIpos): m/z = 541 $[M+H]^+$
$^1H$ NMR (400 MHz, DMSO-$d_6$): δ ppm = 10.43 (d, 1H), 8.78-8.83 (m, 1H), 8.28 (d, 1H), 7.49-7.61 (m, 2H), 6.81 (br d,

0.40H), 6.69 (br d, 0.60H), 4.92-5.19 (m, 1H), 4.68-4.79 (m, 1H), 3.59-3.78 (m, 2H), 3.33-3.48 (m, 1.60H), 3.14-3.27 (m, 1H), 2.88 (br d, 0.40H), 1.83-1.93 (m, 1H), 1.58-1.70 (m, 1H), 0.97 (t, 3H), 0.76-0.88 (m, 1H), 0.44-0.63 (m, 3H).

**Beispiel 622**

1-(2,4-Difluorphenyl)-7-[(4*S*)-4-hydroxy-2-oxopyrrolidin-1-yl]-4-oxo-*N*-[3,3,4,4,4-pentafluorbutan-2-yl]-1,4-dihydro-1,8-naphthyridin-3-carboxamid (Diastereomerengemisch)

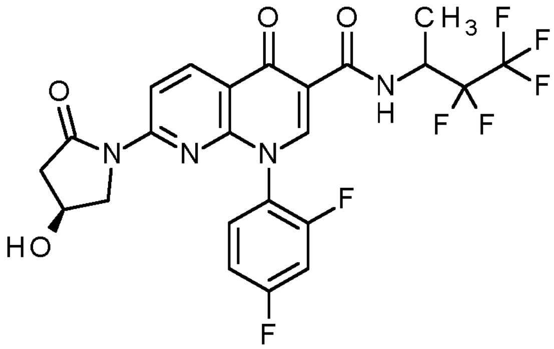

Gemäß AAV1 wurden 150 mg (374 μmol) der Verbindung aus Beispiel 63A mit 89.5 mg (449 μmol) 3,3,4,4,4-Pentafluorbutan-2-amin Hydrochlorid in Gegenwart von 171 mg (449 μmol) HATU und 260 μl (1.50 mmol) DIPEA in 1.5 ml DMF zur Reaktion gebracht. Das Rohprodukt wurde mittels präparativer HPLC (Acetonitril / Wasser/ 0,1 % Ameisensäure) gereinigt. Es wurden 177 mg (87% d. Th., 100% Reinheit) der Titelverbindung erhalten.
LC-MS (Methode 1): $R_t$ = 1.03 min; MS (ESIpos): m/z = 547 $[M+H]^+$
$^1H$ NMR (400 MHz, DMSO-$d_6$): δ ppm = 10.35 (br d, 1H), 8.85 (d, 1H), 8.70 (d, 1H), 8.52 (dd, 1H), 7.81-7.93 (m, 1H), 7.63 (br t, 1H), 7.37 (br t, 1H), 5.28-5.36 (m, 1H), 4.98-5.11 (m, 1H), 4.26-4.31 (m, 1H), 3.62-3.71 (m, 1H), 3.42-3.51 (m, 1H), 2.89-2.99 (m, 1H), 2.32-2.41 (m, 1H), 1.41 (d, 3H).

167 mg der Titelverbindung (Diastereomerengemisch) wurde durch chirale HPLC in die Diastereomere getrennt (präparative HPLC: Säule Chiralpak AD-H 5 μm 250x30 mm; Eluent: 80% *n*-Heptan, 20% Iso-Propanol; Temperatur: 23°C; Fluss: 50 ml/min; UV-Detektion: 220 nm.)

Man erhielt (in der Reihenfolge der Elution von der Säule) 74.1 mg Diastereomer 1 (99% de) Rt = 6.33 min und 71.3 mg Diastereomer 2 (95% de) Rt = 9.54 min.

[Analytische HPLC: Säule Daicel AD-3 3 μm 50x4.6 mm; Eluent: 80% Iso-Hexan, 20% Iso-propanol; UV-Detektion: 220 nm].

Diastereomer 1 wurde zusätzlich mittels präparativer HPLC (Säule: Chromatorex C18, 10 μm, 125*40 mm, Lösungsmittel: Acetonitril, Wasser, 0.1% Ameisensäure) gereinigt und 60.3 mg (30% d. Th., 100% Reinheit) der Titelverbindung aus Beispiel 623 erhalten.

Diastereomer 2 wurde zusätzlich mittels präparativer HPLC (Säule: Chromatorex C18, 10 μm, 125*40 mm, Lösungsmittel: Acetonitril, Wasser, 0.1% Ameisensäure) gereinigt und 60.2 mg (30% d. Th., 100% Reinheit) der Titelverbindung aus Beispiel 624 erhalten.

**Beispiel 623**

1-(2,4-Difluorphenyl)-7-[(4*S*)-4-hydroxy-2-oxopyrrolidin-1-yl]-4-oxo-*N*-[3,3,4,4,4-pentafluorbutan-2-yl]-1,4-dihydro-1,8-naphthyridin-3-carboxamid (Diastereomer 1)

LC-MS (Methode 3): $R_t$ = 1.88 min; MS (ESIpos): m/z = 547 $[M+H]^+$
$^1H$ NMR (400 MHz, DMSO-$d_6$): δ ppm = 10.35 (d, 1H), 8.83-8.87 (m, 1H), 8.70 (d, 1H), 8.51 (dd, 1H), 7.80-7.93 (m, 1H), 7.63 (br t, 1H), 7.37 (br t, 1H), 5.28-5.37 (m, 1H), 4.98-5.11 (m, 1H), 4.26-4.31 (m, 1H), 3.66 (td, 1H), 3.47 (brt, 1H), 2.88-2.99 (m, 1H), 2.32-2.42 (m, 1H), 1.41 (d, 3H).

**Beispiel 624**

1-(2,4-Difluorphenyl)-7-[(4*S*)-4-hydroxy-2-oxopyrrolidin-1-yl]-4-oxo-*N*-[3,3,4,4,4-pentafluorbutan-2-yl]-1,4-dihydro-1,8-naphthyridin-3-carboxamid (Diastereomer 2)

LC-MS (Methode 3): $R_t$ = 1.88 min; MS (ESIpos): m/z = 547 $[M+H]^+$
$^1H$ NMR (400 MHz, DMSO-$d_6$): δ ppm = 10.35 (br dd, 1H), 8.85 (d, 1H), 8.70 (d, 1H), 8.52 (dd, 1H), 7.82-7.92 (m, 1H), 7.62 (br t, 1H), 7.37 (br t, 1H), 5.27-5.36 (m, 1H), 4.98-5.12 (m, 1H), 4.26-4.31 (m, 1H), 3.62-3.72 (m, 1H), 3.46 (brt, 1H), 2.89-3.00 (m, 1H), 2.37 (br dd, 1H), 1.41 (d, 3H).

**Beispiel 625**

7-[(3*R*,4*R*)-3,4-Dihydroxypyrrolidin-1-yl]-4-oxo-*N*-[1-(trifluormethyl)cyclobutyl]-1-(2,4,6-trifluorphenyl)-1,4-dihydro-1,8-naphthyridin-3-carboxamid

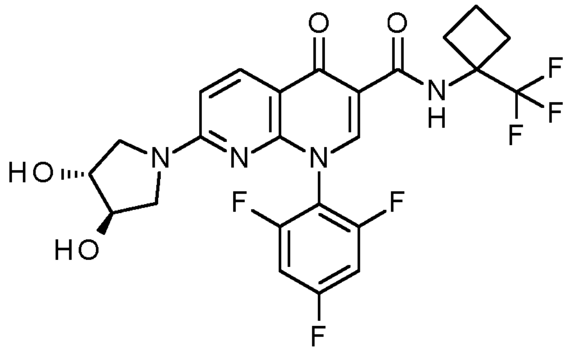

Gemäß AAV1 wurden 50.0 mg (119 μmol) der Verbindung aus Beispiel 121A mit 25.0 mg (142 μmol) 1-(Trifluormethyl)cyclobutanamin Hydrochlorid in Gegenwart von 54.1 mg (142 μmol) HATU und 62 μl (360 μmol) DIPEA in 460 μl DMF zur Reaktion gebracht. Das Rohprodukt wurde mittels präparativer HPLC (Acetonitril / Wasser/ 0,1 % Ameisensäure) gereinigt. Es wurden 48.7 mg (76% d. Th., 100% Reinheit) der Titelverbindung erhalten.
LC-MS (Methode 3): $R_t$ = 1.73 min; MS (ESIpos): m/z = 543 $[M+H]^+$
$^1H$ NMR (400 MHz, DMSO-$d_6$): δ ppm = 10.54 (s, 1H), 8.75 (s, 1H), 8.27 (d, 1H), 7.53-7.60 (m, 2H), 6.77 (d, 1H), 5.10-5.31 (m, 2H), 4.05 (br s, 1H), 3.93 (br s, 1H), 3.57-3.64 (m, 1H), 3.33-3.37 (m, 2H), 3.21-3.28 (m, 2H), 3.07 (br d, 1H), 2.56-2.65 (m, 2H), 1.90-2.06 (m, 2H).

**Beispiel 626**

*N*-(Bicyclo[1.1.1]pent-1-yl)-7-[7-hydroxy-5-azaspiro[2.4]hept-5-yl]-4-oxo-1-(2,4,6-trifluorphenyl)-1,4-dihydro-1,8-naphthyridin-3-carboxamid (Racemat)

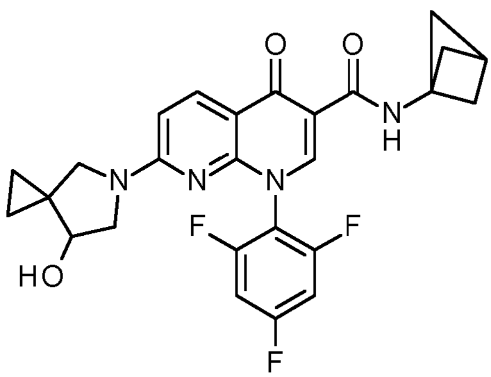

Gemäß AAV1 wurden 80.0 mg (119 µmol) der Verbindung aus Beispiel 155A mit 26.6 mg (223 µmol) Bicyclo[1.1.1]pentan-1-amin Hydrochlorid in Gegenwart von 84.6 mg (223 µmol) HATU und 97 µl (560 µmol) DIPEA in 890 µl DMF zur Reaktion gebracht. Das Rohprodukt wurde mittels präparativer HPLC (Acetonitril / Wasser/ 0.1% Ameisensäure) gereinigt. Es wurden 57.8 mg (63% d. Th., 100% Reinheit) der Titelverbindung erhalten.
LC-MS (Methode 4): $R_t$ = 3.34 min; MS (ESIpos): m/z = 497 $[M+H]^+$
$^1H$ NMR (400 MHz, DMSO-$d_6$): δ ppm = 10.28 (s, 1H), 8.63-8.68 (m, 1H), 8.25 (d, 1H), 7.49-7.61 (m, 2H), 6.78 (br d, 0.40H), 6.66 (br d, 0.60H), 5.02-5.11 (m, 0.40H), 4.95-5.02 (m, 0.60H), 3.58-3.78 (m, 2H), 3.33-3.47 (m, 2H), 3.11-3.25 (m, 1H), 2.87 (br d, 0.40H), 2.09 (s, 6H), 0.77-0.87 (m, 1H), 0.52-0.62 (m, 2H), 0.43-0.52 (m, 1H).

53.4 mg der Titelverbindung (Racemat) wurde durch chirale HPLC in die Enantiomere getrennt (präparative HPLC: Säule YMC Chiralart Amylose SA 5 µm 250x30 mm; Eluent: 85% *n*-Heptan, 15% Ethanol; Temperatur: 25°C; Fluss: 30 ml/min; UV-Detektion: 220 nm.)

Man erhielt (in der Reihenfolge der Elution von der Säule) 22.0 mg (24% d. Th., 100% Reinheit) Enantiomer 1 aus Beispiel 627 (99% ee) Rt = 15.56 min und 23.0 mg (25% d. Th., 100% Reinheit) Enantiomer 2 aus Beispiel 628 (99% ee) Rt = 18.15 min.

[Analytische HPLC: Säule YMC Chiralart Amylose SA 5 µm 250x4.6 mm; Eluent: 80% Iso-Hexan, 20% Ethanol; Temperatur: 25°C; Fluss: 1.0 ml/min; UV-Detektion: 220nm].

## Beispiel 627

*N*-(Bicyclo[1.1.1]pent-1-yl)-7-[7-hydroxy-5-azaspiro[2.4]hept-5-yl]-4-oxo-1-(2,4,6-trifluorphenyl)-1,4-dihydro-1,8-naphthyridin-3-carboxamid (Enantiomer 1)

LC-MS (Methode 3): $R_t$ = 1.99 min; MS (ESIpos): m/z = 497 $[M+H]^+$
$^1H$ NMR (400 MHz, DMSO-$d_6$): δ ppm = 10.28 (s, 1H), 8.63-8.68 (m, 1H), 8.25 (d, 1H), 7.49-7.61 (m, 2H), 6.77 (br d, 0.40H), 6.66 (br d, 0.60H), 5.02-5.09 (m, 0.40H), 4.94-5.02 (m, 0.60H), 3.60-3.77 (m, 2H), 3.31-3.47 (m, 1.60H), 3.14-3.25 (m, 1H), 2.87 (d, 0.40H), 2.09 (s, 6H), 0.77-0.87 (m, 1H), 0.52-0.62 (m, 2H), 0.43-0.52 (m, 1H).

## Beispiel 628

*N*-(Bicyclo[1.1.1]pent-1-yl)-7-[7-hydroxy-5-azaspiro[2.4]hept-5-yl]-4-oxo-1-(2,4,6-trifluorphenyl)-1,4-dihydro-1,8-naphthyridin-3-carboxamid (Enantiomer 2)

LC-MS (Methode 3): $R_t$ = 1.99 min; MS (ESIpos): m/z = 497 $[M+H]^+$
$^1H$ NMR (400 MHz, DMSO-$d_6$): δ ppm = 10.28 (s, 1H), 8.63-8.68 (m, 1H), 8.25 (d, 1H), 7.49-7.61 (m, 2H), 6.78 (br d, 0.40H), 6.66 (br d, 0.60H), 5.02-5.09 (m, 0.40H), 4.97 (br d, 0.60H), 3.59-3.78 (m, 2H), 3.32-3.47 (m, 1.60H), 3.13-3.25 (m, 1H), 2.87 (br d, 0.40H), 2.09 (s, 6H), 0.77-0.87 (m, 1H), 0.44-0.63 (m, 3H).

## Beispiel 629

*N*-[(1*R*)-1-Cyclopropyl-2,2,2-trifluorethyl]-1-(2,6-difluorphenyl)-4-oxo-7-(2-oxoimidazolidin-1-yl)-1,4-dihydro-1,8-naphthyridin-3-carboxamid

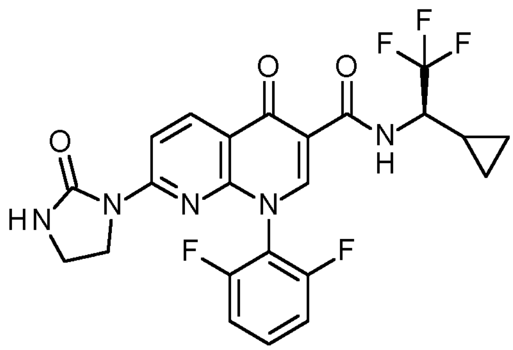

Gemäß AAV2 wurden 60.0 mg (131 µmol) der Verbindung aus Beispiel 103A mit 113 mg (1.31 mmol) Imidazolidin-2-on in Gegenwart von 27.2 mg (197 μmol) Kaliumcarbonat, 5.88 mg (26.2 μmol) Palladiumacetat und 15.2 mg (26.2 μmol) Xantphos in 1.2 ml 1,4-Dioxan zur Reaktion gebracht. Das Rohprodukt wurde mittels präparativer HPLC (Acetonitril / Wasser/ 0.1% Ameisensäure) gereinigt. Die Substanz wurde mit Verunreinigungen isoliert und wurde weiter aus Acetonitril umkristallisiert, abgesaugt, mit etwas kaltem Acetonitril nachgewaschen und nachgetrocknet. Es wurden 39.6 mg (60% d. Th., 100% Reinheit) der Titelverbindung erhalten.
LC-MS (Methode 3): $R_t$ = 1.91 min; MS (ESIpos): m/z = 508 $[M+H]^+$
$^1H$ NMR (400 MHz, DMSO-$d_6$): δ ppm = 10.36 (d, 1H), 8.94 (s, 1H), 8.57 (d, 1H), 8.44 (d, 1H), 7.68-7.76 (m, 1H), 7.66 (br s, 1H), 7.43 (t, 2H), 4.35-4.45 (m, 1H), 3.50-3.58 (m, 2H), 3.32-3.36 (m, 2H), 1.18-1.27 (m, 1H), 0.51-0.70 (m, 3H), 0.32-0.39 (m, 1H).

**Beispiel 630**

*N*-[(1S)-1-Cyclopropyl-2,2,2-trifluorethyl]-7-[(3*R*,4*S*)-3,4-dihydroxypyrrolidin-1-yl]-4-oxo-1-(2,4,6-trifluorphenyl)-1,4-dihydro-1,8-naphthyridin-3-carboxamid

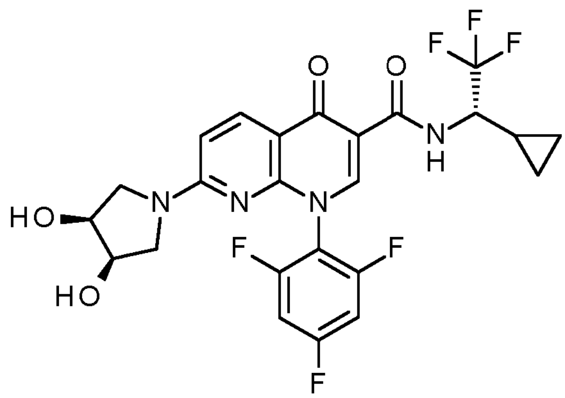

Gemäß AAV3 wurden 60.0 mg (126 μmol) der Verbindung aus Beispiel 126A mit 21.1 mg (151 μmol) (3*R*,4*S*)-Pyrrolidin-3,4-diol Hydrochlorid und 77 μl (440 μmol) DIPEA in 600 μl DMF zur Reaktion gebracht. Das Rohprodukt wurde mittels präparativer HPLC (Acetonitril / Wasser/ 0,1 % Ameisensäure) gereinigt. Es wurden 61.2 mg (89% d. Th., 100% Reinheit) der Titelverbindung erhalten.
LC-MS (Methode 3): $R_t$ = 1.77 min; MS (ESIpos): m/z = 543 $[M+H]^+$
$^1H$ NMR (400 MHz, DMSO-$d_6$): δ ppm = 10.56 (d, 1H), 8.80 (s, 1H), 8.28 (d, 1H), 7.52-7.60 (m, 2H), 6.76 (d, 1H), 5.04 (d, 1H), 4.94 (d, 1H), 4.33-4.43 (m, 1H), 4.10-4.16 (m, 1H), 3.99-4.05 (m, 1H), 3.60 (br dd, 1H), 3.20-3.30 (m, 2H), 2.97-3.04 (m, 1H), 1.16-1.25 (m, 1H), 0.49-0.68 (m, 3H), 0.29-0.37 (m, 1H).

**Beispiel 631**

*N*-(2,6-Dichlorphenyl)-1-(2,4-difluorphenyl)-7-[(3*R*,4*R*)-3,4-dihydroxypyrrolidin-1-yl]-4-oxo-1,4-dihydro-1,8-naphthyridin-3-carboxamid

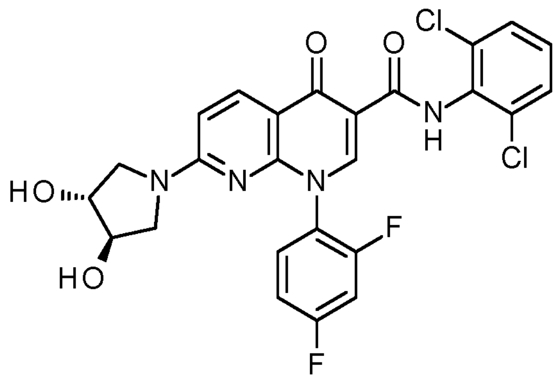

Gemäß AAV3 wurden 40.0 mg (83.2 µmol) der Verbindung aus Beispiel 81A mit 13.9 mg (99.9 µmol) (3*R*,4*R*)-Pyrrolidin-3,4-diol Hydrochlorid und 51 µl (290 µmol) DIPEA in 400 µl DMF zur Reaktion gebracht. Das Rohprodukt wurde mittels präparativer HPLC (Acetonitril / Wasser/ 0,1 % Ameisensäure) gereinigt. Es wurden 31.2 mg (68% d. Th., 100% Reinheit) der Titelverbindung erhalten.
LC-MS (Methode 3): $R_t$ = 1.68 min; MS (ESIpos): m/z = 547 $[M+H]^+$
$^1$H NMR (400 MHz, DMSO-$d_6$): δ ppm = 12.04 (s, 1H), 8.68 (s, 1H), 8.34 (d, 1H), 7.85 (td, 1H), 7.55-7.61 (m, 2H), 7.30-7.40 (m, 2H), 6.78 (d, 1H), 5.07-5.29 (m, 2H), 4.01-4.09 (m, 1H), 3.93 (br s, 1H), 3.57-3.66 (m, 1H), 3.16-3.28 (m, 2H), 3.03-3.14 (m, 1H).

**Beispiel 632**

7-[(4*S*)-4-Hydroxy-2-oxopyrrolidin-1-yl]-4-oxo-*N*-[1-(2,2,2-trifluorethyl)cyclopropyl]-1-(2,4,6-trifluorphenyl)-1,4-dihydro-1,8-naphthyridin-3-carboxamid

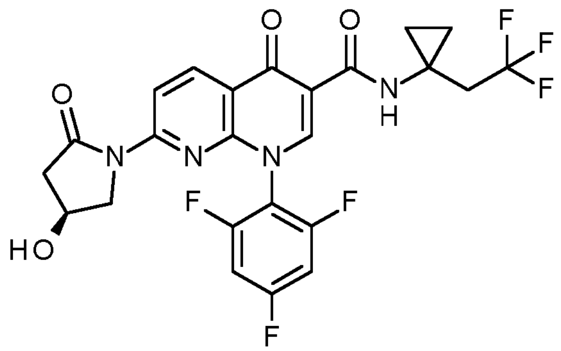

Gemäß AAV1 wurden 23.7 mg (56.4 µmol) der Verbindung aus Beispiel 117A mit 10.4 mg (59.2 µmol) 1-(2,2,2-Trifluorethyl)cyclopropanamin Hydrochlorid in Gegenwart von 25.7 mg (67.7 µmol) HATU und 39 µl (500 µmol) DIPEA in 500 µl DMF zur Reaktion gebracht. Das Rohprodukt wurde mittels präparativer HPLC (Acetonitril / Wasser/ 0,1 % Ameisensäure) gereinigt. Es wurden 20.1 mg (66% d. Th., 100% Reinheit) der Titelverbindung erhalten.
LC-MS (Methode 3): $R_t$ = 1.72 min; MS (ESIpos): m/z = 541 $[M+H]^+$
$^1$H NMR (400 MHz, DMSO-$d_6$): δ ppm = 9.99 (s, 1H), 8.97 (s, 1H), 8.67 (d, 1H), 8.51 (d, 1H), 7.57-7.65 (m, 2H), 5.32 (d, 1H), 4.26-4.31 (m, 1H), 3.68 (dd, 1H), 3.47 (d, 1H), 2.94 (dd, 1H), 2.68 (q, 2H), 2.37 (d, 1H), 0.86-0.95 (m, 4H).

**Beispiel 633**

Methyl-4-[6-(bicyclo[1.1.1]pent-1-ylcarbamoyl)-5-oxo-8-(2,4,6-trifluorphenyl)-5,8-dihydro-1,8-naphthyridin-2-yl]piperazin-1-carboxylat

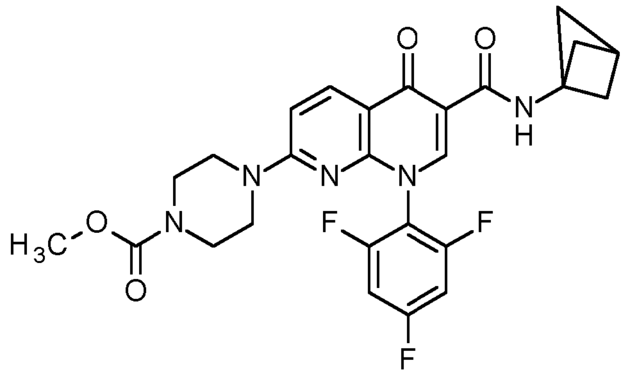

Gemäß AAV1 wurden 70.0 mg (151 μmol) der Verbindung aus Beispiel 157A mit 21.7 mg (182 μmol) Bicyclo[1.1.1]pentan-1-amin Hydrochlorid in Gegenwart von 69.1 mg (182 μmol) HATU und 79 μl (450 μmol) DIPEA in 580 μl DMF zur Reaktion gebracht. Das Rohprodukt wurde mittels präparativer HPLC (Acetonitril / Wasser/ 0,1 % Ameisensäure) gereinigt. Es wurden 4.80 mg (6% d. Th., 100% Reinheit) der Titelverbindung erhalten.
LC-MS (Methode 3): $R_t$ = 2.05 min; MS (ESIpos): m/z = 528 $[M+H]^+$
$^1H$ NMR (400 MHz, DMSO-$d_6$): δ ppm = 10.22 (s, 1H), 8.69 (s, 1H), 8.29 (d, 1H), 7.57 (t, 2H), 7.11 (d, 1H), 3.60 (s, 3H), 3.47-3.56 (m, 4H), 3.33-3.43 (m, 4H), 2.09 (s, 6H).

**Beispiel 634**

*N*-(1,1-Difluor-2-methylpropan-2-yl)-7-[(3*R*,4*R*)-3,4-dihydroxypyrrolidin-1-yl]-4-oxo-1-(2,4,6-trifluorphenyl)-1,4-dihydro-1,8-naphthyridin-3-carboxamid

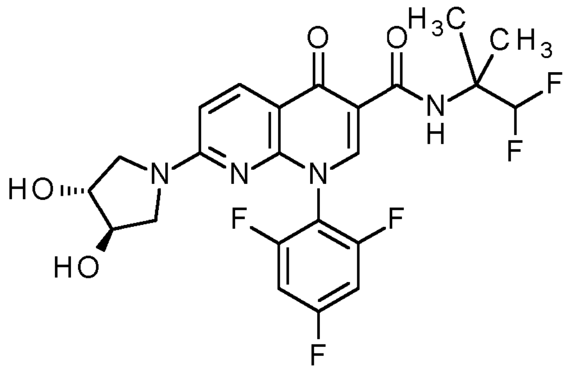

Gemäß AAV1 wurden 30.0 mg (119 μmol) der Verbindung aus Beispiel 121A mit 12.4 mg (85.4 μmol) 1,1-Difluor-2-methylpropan-2-amin Hydrochlorid in Gegenwart von 32.5 mg (85.4 μmol) HATU und 37 μl (210 μmol) DIPEA in 340 μl DMF zur Reaktion gebracht. Das Rohprodukt wurde mittels präparativer HPLC (Acetonitril / Wasser/ 0,1 % Ameisensäure) gereinigt. Es wurden 10.3 mg (28% d. Th., 100% Reinheit) der Titelverbindung erhalten.
LC-MS (Methode 3): $R_t$ = 1.61 min; MS (ESIpos): m/z = 513 $[M+H]^+$
$^1H$ NMR (400 MHz, DMSO-$d_6$): δ ppm = 10.32 (s, 1H), 8.71 (s, 1H), 8.26 (d, 1H), 7.52-7.60 (m, 2H), 6.76 (d, 1H), 6.43 (t, 1H), 5.23 (d, 1H), 5.13 (d, 1H), 4.04 (br s, 1H), 3.88-3.97 (m, 1H), 3.61 (br dd, 1H), 3.32-3.36 (m, 1H), 3.21-3.27 (m, 1H), 3.06 (br d, 1H), 1.43 (s, 6H).

**Beispiel 635**

7-[(3*R*,4*R*)-3,4-Dihydroxypyrrolidin-1-yl]-4-oxo-*N*-[1-(2,2,2-trifluorethyl)cyclopropyl]-1-(2,4,6-trifluorphenyl)-1,4-dihydro-1,8-naphthyridin-3-carboxamid

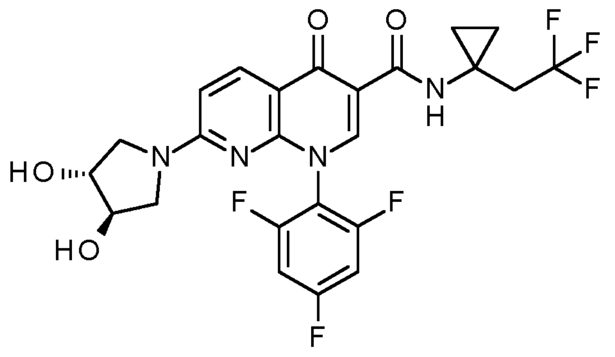

Gemäß AAV1 wurden 23.8 mg (56.4 μmol) der Verbindung aus Beispiel 121A mit 10.4 mg (59.2 μmol) 1-(2,2,2-Trifluorethyl)cyclopropanamin Hydrochlorid in Gegenwart von 25.7 mg (67.7 μmol) HATU und 39 μl (230 μmol) DIPEA in 500 μl DMF zur Reaktion gebracht. Das Rohprodukt wurde mittels präparativer HPLC (Acetonitril / Wasser/ 0,1 % Ameisensäure) gereinigt. Es wurden 18.9 mg (62% d. Th., 100% Reinheit) der Titelverbindung erhalten.
LC-MS (Methode 3): $R_t$ = 1.53 min; MS (ESIpos): m/z = 543 $[M+H]^+$
$^1$H NMR (400 MHz, DMSO-$d_6$): δ ppm = 10.25 (s, 1H), 8.70 (s, 1H), 8.24 (d, 1H), 7.52-7.59 (m, 2H), 6.75 (d, 1H), 5.22 (d, 1H), 5.13 (d, 1H), 4.02-4.06 (m, 1H), 3.90-3.94 (m, 1H), 3.57-3.63 (m, 1H), 3.32-3.35 (m, 1H), 3.21-3.27 (m, 1H), 3.06 (br d, 1H), 2.62-2.71 (m, 2H), 0.84-0.93 (m, 4H).

**Beispiel 636**

7-(4-Carbamoylpiperazin-1-yl)-*N*-(2,6-dichlorphenyl)-4-oxo-1-(2,4,6-trifluorphenyl)-1,4-dihydro-1,8-nahthridin-3-carboxamid

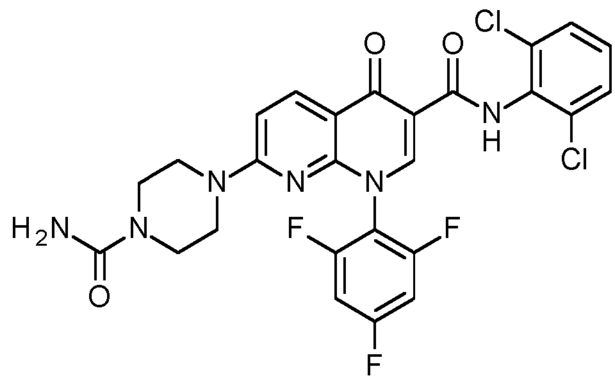

Gemäß AAV3 wurden 60.0 mg (120 μmol) der Verbindung aus Beispiel 160B mit 18.6 mg (144 μmol) Piperazin-1-carboxamid und 73 μl (420 μmol) DIPEA in 540 μl DMF zur Reaktion gebracht. Das Rohprodukt wurde mittels präparativer HPLC (Acetonitril / Wasser/ 0,1 % Ameisensäure) gereinigt. Es wurden 44.1 mg (62% d. Th., 100% Reinheit) der Titelverbindung erhalten.
LC-MS (Methode 3): $R_t$ = 1.73 min; MS (ESIpos): m/z = 591 $[M+H]^+$
$^1$H NMR (400 MHz, DMSO-$d_6$): δ ppm = 11.88 (s, 1H), 8.92 (s, 1H), 8.37 (d, 1H), 7.55-7.62 (m, 4H), 7.35-7.41 (m, 1H), 7.18 (d, 1H), 6.04 (s, 2H), 3.43-3.54 (m, 4H), 3.32-3.38 (m, 4H).

**Beispiel 637**

1-(2,6-Difluorphenyl)-4-oxo-7-(2-oxoimidazolidin-1-yl)-*N*-[(2*S*)-1,1,1-trifluorbutan-2-yl]-1,4-dihydro-1,8-naphthyridin-3-carboxamid

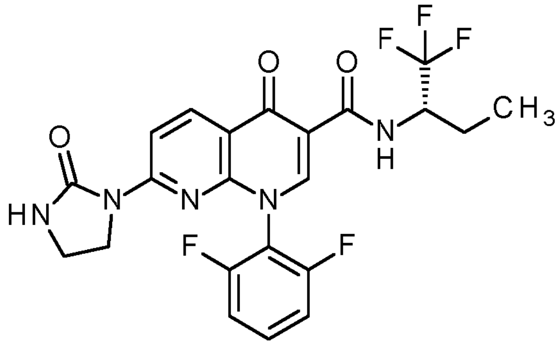

Gemäß AAV2 wurden 60.0 mg (135 µmol) der Verbindung aus Beispiel 114A mit 116 mg (1.35 mmol) Imidazolidin-2-on in Gegenwart von 27.9 mg (202 µmol) Kaliumcarbonat, 6.04 mg (26.9 µmol) Palladiumacetat und 15.6 mg (26.9 µmol) Xantphos in 1.2 ml 1,4-Dioxan zur Reaktion gebracht. Das Rohprodukt wurde mittels präparativer HPLC (Acetonitril / Wasser/ 0.1% Ameisensäure) gereinigt. Die Substanz wurde mit Verunreinigungen isoliert und wurde weiter aus Acetonitril umkristallisiert, abgesaugt, mit etwas Acetonitril nachgewaschen und nachgetrocknet. Es wurden 33.5 mg (50% d. Th., 100% Reinheit) der Titelverbindung erhalten.
LC-MS (Methode 3): $R_t$ = 1.88 min; MS (ESIpos): m/z = 496 $[M+H]^+$
$^1$H NMR (400 MHz, DMSO-$d_6$): δ ppm = 10.23 (d, 1H), 8.95 (s, 1H), 8.57 (d, 1H), 8.44 (d, 1H), 7.68-7.76 (m, 1H), 7.65 (s, 1H), 7.43 (t, 2H), 4.70-4.81 (m, 1H), 3.51-3.57 (m, 2H), 3.32-3.36 (m, 2H), 1.84-1.94 (m, 1H), 1.61-1.72 (m, 1H), 0.98 (t, 3H).

## Beispiel 638

1-(2,6-Difluorphenyl)-4-oxo-7-(2-oxoimidazolidin-1-yl)-*N*-[(2*R*)-1,1,1-trifluorbutan-2-yl]-1,4-dihydro-1,8-naphthyridin-3-carboxamid

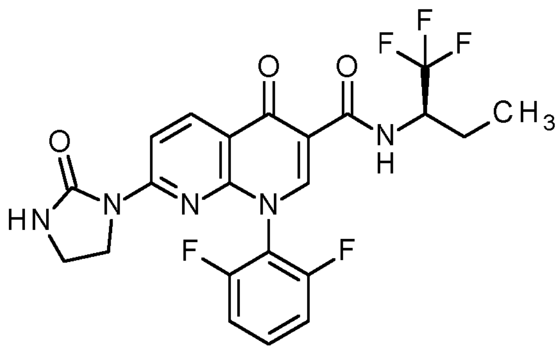

Gemäß AAV2 wurden 60.0 mg (135 µmol) der Verbindung aus Beispiel 86A mit 116 mg (1.35 mmol) Imidazolidin-2-on in Gegenwart von 27.9 mg (202 µmol) Kaliumcarbonat, 6.04 mg (26.9 µmol) Palladiumacetat und 15.6 mg (26.9 µmol) Xantphos in 1.2 ml 1,4-Dioxan zur Reaktion gebracht. Das Rohprodukt wurde mittels präparativer HPLC (Acetonitril / Wasser/ 0,1 % Ameisensäure) gereinigt. Die Substanz wurde mit Verunreinigungen isoliert und wurde weiter aus Acetonitril umkristallisiert, abgesaugt, mit etwas kaltem Acetonitril nachgewaschen und nachgetrocknet. Es wurden 27.5 mg (41% d. Th., 100% Reinheit) der Titelverbindung erhalten.
LC-MS (Methode 3): $R_t$ = 1.88 min; MS (ESIpos): m/z = 496 $[M+H]^+$
$^1$H NMR (600 MHz, DMSO-$d_6$): δ ppm = 10.23 (d, 1H), 8.95 (s, 1H), 8.56 (d, 1H), 8.44 (d, 1H), 7.69-7.76 (m, 1H), 7.67 (s, 1H), 7.43 (t, 2H), 4.72-4.80 (m, 1H), 3.49-3.58 (m, 2H), 3.33-3.35 (m, 1H), 3.31-3.33 (m, 1H), 1.86-1.93 (m, 1H), 1.62-1.70 (m, 1H), 0.98 (t, 3H).

**Beispiel 639**

1-(2,4-Difluorphenyl)-7-[(3*R*,4*S*)-3,4-dihydroxypyrrolidin-1-yl]-4-oxo-*N*-[(2*R*)-1,1,1-trifluorbutan-2-yl]-1,4-dihydro-1,8-naphthyridin-3-carboxamid

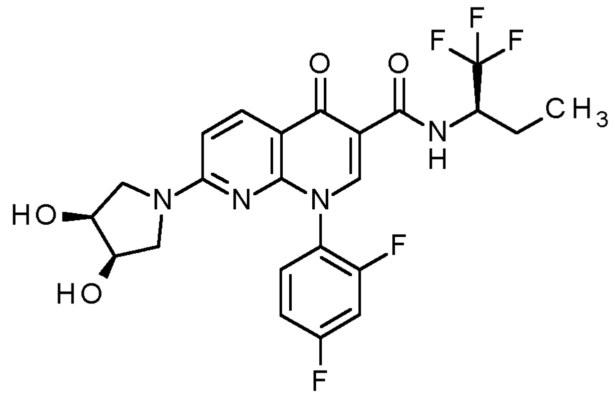

Gemäß AAV3 wurden 100 mg (224 µmol) der Verbindung aus Beispiel 67A mit 37.6 mg (269 µmol) (3*R*,4*S*)-Pyrrolidin-3,4-diol Hydrochlorid und 140 µl (790 µmol) DIPEA in 1.0 ml DMF zur Reaktion gebracht. Das Rohprodukt wurde mittels präparativer HPLC (Acetonitril / Wasser/ 0,1 % Ameisensäure) gereinigt. Es wurden 105 mg (91% d. Th., 100% Reinheit) der Titelverbindung erhalten.
LC-MS (Methode 3): $R_t$ = 1.71 min; MS (ESIpos): m/z = 513 $[M+H]^+$
$^1H$ NMR (400 MHz, DMSO-$d_6$): δ ppm = 10.51 (d, 1H), 8.61 (s, 1H), 8.28 (d, 1H), 7.76-7.85 (m, 1H), 7.58 (br t, 1H), 7.33 (br t, 1H), 6.74 (d, 1H), 5.02 (br dd, 1H), 4.89-4.96 (m, 1H), 4.67-4.79 (m, 1H), 4.13 (br s, 1H), 3.97-4.07 (m, 1H), 3.53-3.65 (m, 1H), 3.16-3.30 (m, 2H), 2.93-3.09 (m, 1H), 1.83-1.93 (m, 1H), 1.58-1.69 (m, 1H), 0.97 (t, 3H).

**Beispiel 640**

1-(2,4-Difluorphenyl)-7-[3-hydroxy-2-methyl-5-oxopyrrolidin-1-yl]-4-oxo-*N*-[(2*R*)-1,1,1-trifluorbutan-2-yl]-1,4-dihydro-1,8-naphthyridin-3-carboxamid (Diastereomerengemisch)

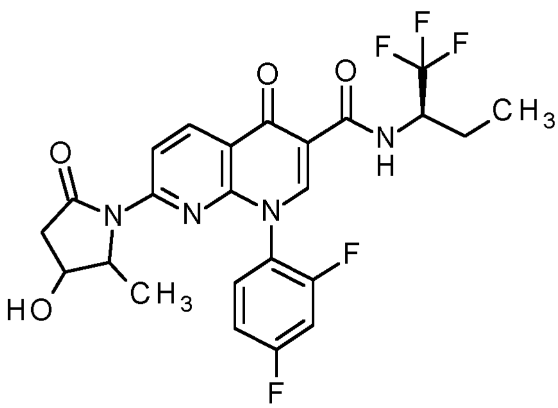

Gemäß AAV2 wurden 60.0 mg (135 µmol) der Verbindung aus Beispiel 67A mit 18.6 mg (162 µmol) der Verbindung aus Beispiel 163C in Gegenwart von 27.9 mg (202 µmol) Kaliumcarbonat, 6.04 mg (26.9 µmol) Palladiumacetat und 15.6 mg (26.9 µmol) Xantphos in 1.0 ml 1,4-Dioxan zur Reaktion gebracht. Das Rohprodukt wurde mittels präparativer HPLC (Acetonitril / Wasser/ 0,1 % Ameisensäure) gereinigt. Die Substanz wurde mit Verunreinigungen isoliert und wurde weiter anschließend mittels Normalphasenchromatographie (Eluent: Cyclohexan-Essigsäureethylester-Gradient). Es wurden 21.6 mg (31% d. Th., 100% Reinheit) der Titelverbindung erhalten.
LC-MS (Methode 3): $R_t$ = 1.90 min; MS (ESIpos): m/z = 525 $[M+H]^+$

$^1H$ NMR (400 MHz, DMSO-$d_6$): δ ppm = 10.17-10.27 (m, 1H), 8.84-8.90 (m, 1H), 8.67-8.73 (m, 1H), 8.54 (t, 0.80H), 8.44 (t, 0.20H), 7.84-7.94 (m, 1H), 7.59-7.69 (m, 1H), 7.33-7.42 (m, 1H), 5.31-5.41 (m, 1H), 4.71-4.83 (m, 1H), 3.88-4.13 (m, 2H), 3.05-3.17 (m, 1H), 2.28 (br dd, 1H), 1.83-1.95 (m, 1H), 1.60-1.73 (m, 1H), 0.93-1.05 (m, 5H), 0.84-0.90 (m, 1H).

**Beispiel 641**

*N*-(Bicyclo[1.1.1]pent-1-yl)-7-(3-methoxy-3-methylazetidin-1-yl)-4-oxo-1-(2,4,6-trifluorphenyl)-1,4-dihydro-1,8-naphthyridin-3-carboxamid

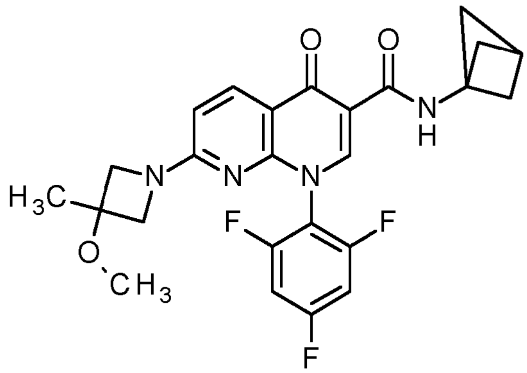

Gemäß AAV1 wurden 24.1 mg (57.5 μmol) der Verbindung aus Beispiel 161A mit 8.26 mg (69.0 μmol) Bicyclo[1.1.1]pentan-1-amin Hydrochlorid in Gegenwart von 26.3 mg (69.0 μmol) HATU und 30 μl (170 μmol) DIPEA in 230 μl DMF zur Reaktion gebracht. Das Rohprodukt wurde mittels präparativer HPLC (Acetonitril / Wasser/ 0,1 % Ameisensäure) gereinigt. Es wurden 14.0 mg (50% d. Th., 100% Reinheit) der Titelverbindung erhalten.
LC-MS (Methode 1): $R_t$ = 1.17 min; MS (ESIpos): m/z = 485 $[M+H]^+$
$^1H$ NMR (400 MHz, DMSO-$d_6$): δ ppm = 10.24 (s, 1H), 8.66 (s, 1H), 8.26 (d, 1H), 7.51-7.58 (m, 2H), 6.61 (d, 1H), 3.53-4.02 (m, 4H), 3.15 (s, 3H), 2.09 (s, 6H), 1.41 (s, 3H).

**Beispiel 642**

*N*-[(1*S*)-1-Cyclopropyl-2,2,2-trifluorethyl]-7-[(3*S*,4*S*)-3,4-dihydroxypyrrolidin-1-yl]-4-oxo-1-(2,4,6-trifluorphenyl)-1,4-dihydro-1,8-naphthyridin-3-carboxamid

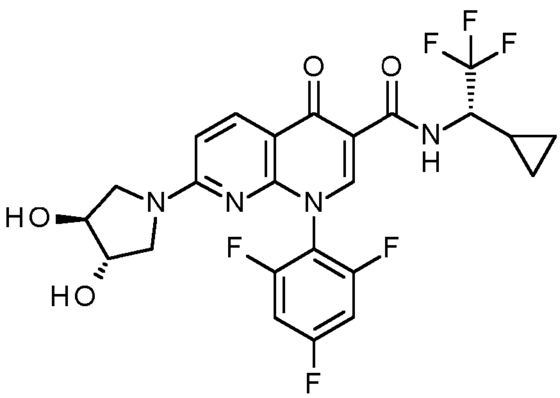

Gemäß AAV3 wurden 60.0 mg (126 μmol) der Verbindung aus Beispiel 126A mit 15.6 mg (151 μmol) (3*S*,4*S*)-Pyrrolidin-3,4-diol und 55 μl (320 μmol) DIPEA in 600 μl DMF zur Reaktion gebracht. Das Rohprodukt wurde mittels präparativer HPLC (Acetonitril / Wasser/ 0,1 % Ameisensäure) gereinigt. Es wurden 60.6 mg (89% d. Th., 100% Reinheit) der Titelverbindung erhalten.
LC-MS (Methode 3): $R_t$ = 1.72 min; MS (ESIpos): m/z = 543 $[M+H]^+$

$^{1}$H NMR (400 MHz, DMSO-$d_6$): δ ppm = 10.57 (d, 1H), 8.80 (s, 1H), 8.28 (d, 1H), 7.56 (br t, 2H), 6.78 (d, 1H), 5.23 (d, 1H), 5.14 (d, 1H), 4.33-4.43 (m, 1H), 4.05 (br s, 1H), 3.93 (br s, 1H), 3.62 (br dd, 1H), 3.32-3.38 (m, 1H), 3.25 (br dd, 1H), 3.07 (br d, 1H), 1.16-1.25 (m, 1H), 0.50-0.69 (m, 3H), 0.31-0.38 (m, 1H).

## Beispiel 643

1-(2,6-Dichlor-4-fluorphenyl)-7-[(3*R*,4*R*)-3,4-dihydroxypyrrolidin-1-yl]-4-oxo-*N*-[(2*R*)-1,1,1-trifluorbutan-2-yl]-1,4-dihydro-1,8-naphthyridin-3-carboxamid

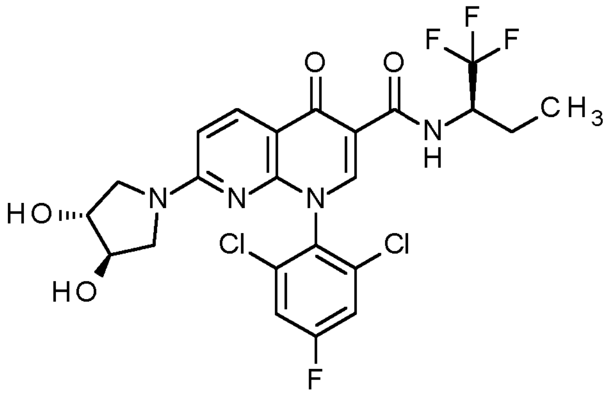

Gemäß AAV3 wurden 60.0 mg (121 μmol) der Verbindung aus Beispiel 131A mit 20.2 mg (145 μmol) (3*R*,4*R*)-Pyrrolidin-3,4-diol Hydrochlorid und 74 μl (420 μmol) DIPEA in 540 μl DMF zur Reaktion gebracht. Das Rohprodukt wurde mittels präparativer HPLC (Acetonitril / Wasser/ 0,1 % Ameisensäure) gereinigt. Es wurden 56.8 mg (83% d. Th., 100% Reinheit) der Titelverbindung erhalten.
LC-MS (Methode 3): $R_t$ = 1.78 min; MS (ESIpos): m/z = 563 $[M+H]^+$
$^{1}$H NMR (400 MHz, DMSO-$d_6$): δ ppm = 10.48 (d, 1H), 8.70 (s, 1H), 8.28 (d, 1H), 7.88 (dq, 2H), 6.77 (d, 1H), 5.23 (br d, 1H), 5.15 (br d, 1H), 4.68-4.79 (m, 1H), 4.04 (br s, 1H), 3.91 (br s, 1H), 3.61 (br dd, 1H), 3.33-3.38 (m, 1H), 3.18 (br dd, 1H), 2.99 (br d, 1H), 1.83-1.93 (m, 1H), 1.59-1.71 (m, 1H), 0.98 (t, 3H).

## Beispiel 644

*N*-[(1*S*)-1-Cyclopropyl-2,2,2-trifluorethyl]-1-(2,6-difluorphenyl)-4-oxo-7-(2-oxoimidazolidin-1-yl)-1,4-dihydro-1,8-naphthyridin-3-carboxamid

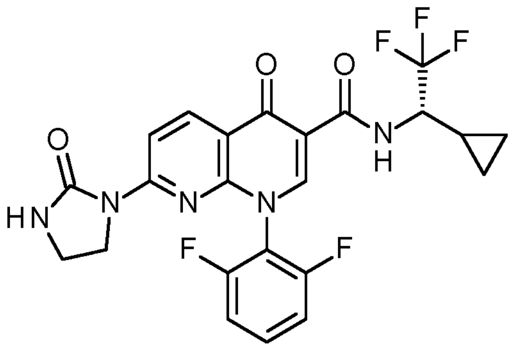

Gemäß AAV2 wurden 60.0 mg (131 μmol) der Verbindung aus Beispiel 104A mit 113 mg (1.31 mmol) Imidazolidin-2-on in Gegenwart von 27.2 mg (197 μmol) Kaliumcarbonat, 5.88 mg (26.2 μmol) Palladiumacetat und 15.2 mg (26.2 μmol) Xantphos in 1.2 ml 1,4-Dioxan zur Reaktion gebracht. Das Rohprodukt wurde mittels präparativer HPLC (Acetonitril / Wasser/ 0,1 % Ameisensäure) gereinigt. Die Substanz wurde mit Verunreinigungen isoliert und wurde

weiter aus Acetonitril umkristallisiert, abgesaugt, mit etwas kaltem Acetonitril nachgewaschen und nachgetrocknet. Es wurden 34.7 mg (52% d. Th., 100% Reinheit) der Titelverbindung erhalten.
LC-MS (Methode 3): $R_t$ = 1.91 min; MS (ESIpos): m/z = 508 $[M+H]^+$
$^1H$ NMR (400 MHz, DMSO-$d_6$): δ ppm = 10.36 (d, 1H), 8.94 (s, 1H), 8.57 (d, 1H), 8.44 (d, 1H), 7.68-7.76 (m, 1H), 7.65 (s, 1H), 7.43 (t, 2H), 4.35-4.45 (m, 1H), 3.50-3.58 (m, 2H), 3.31-3.36 (m, 2H), 1.18-1.27 (m, 1H), 0.51-0.70 (m, 3H), 0.32-0.39 (m, 1H).

**Beispiel 645**

1-(2,6-Dichlorphenyl)-7-[(4*S*)-4-hydroxy-2-oxopyrrolidin-1-yl]-4-oxo-*N*-[(2*R*)-1,1,1-trifluorbutan-2-yl]-1,4-dihydro-1,8-naphthyridin-3-carboxamid

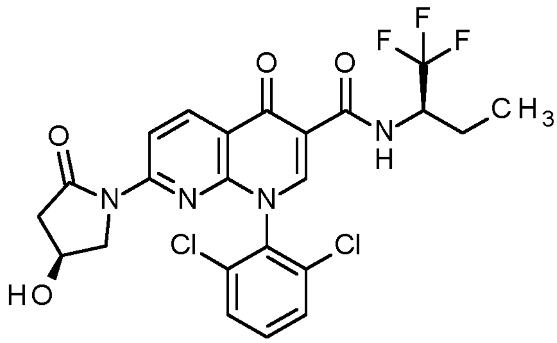

Gemäß AAV2 wurden 60.0 mg (125 µmol) der Verbindung aus Beispiel 166C mit 15.2 mg (150 µmol) (4*S*)-4-Hydroxypyrrolidin-2-on in Gegenwart von 26.0 mg (188 µmol) Kaliumcarbonat, 5.63 mg (25.1 µmol) Palladiumacctat und 14.5 mg (25.1 µmol) Xantphos in 1.0 ml 1,4-Dioxan zur Reaktion gebracht. Das Rohprodukt wurde mittels präparativer HPLC (Acetonitril / Wasser/ 0,1 % Ameisensäure) gereinigt. Es wurden 5.00 mg (7% d. Th., 100% Reinheit) der Titelverbindung erhalten.
LC-MS (Methode 3): $R_t$ = 1.94 min; MS (ESIpos): m/z = 543 $[M+H]^+$
$^1H$ NMR (500 MHz, DMSO-$d_6$): δ ppm = 10.16 (d, 1H), 8.95 (s, 1H), 8.73 (d, 1H), 8.54 (d, 1H), 7.78-7.84 (m, 2H), 7.70 (t, 1H), 5.33 (d, 1H), 4.73-4.82 (m, 1H), 4.22-4.26 (m, 1H), 3.56 (dd, 1H), 3.32-3.36 (m, 1H), 2.93 (dd, 1H), 2.35 (br d, 1H), 1.86-1.94 (m, 1H), 1.63-1.73 (m, 1H), 0.99 (t, 3H).

**Beispiel 646**

1-(2,4-Difluorphenyl)-7-[(3*R*,4*R*)-3,4-dihydroxypyrrolidin-1-yl]-4-oxo-*N*-[(2*R*)-1,1,1-trifluorbutan-2-yl]-1,4-dihydro-1,8-nahthyridin-3-carboxamid

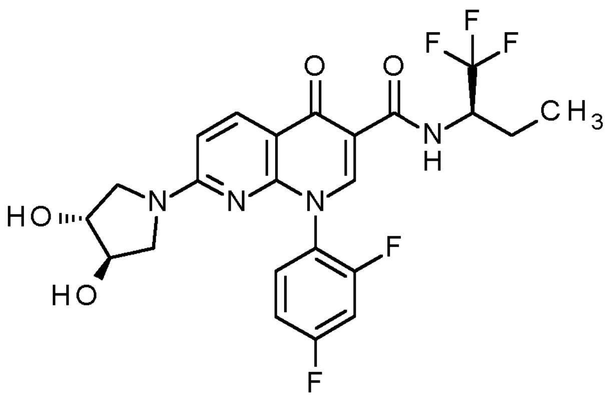

Zu einer Lösung von 170 mg (229 µmol) der Verbindung aus 167A in 1.9 ml THF wurden 500 µl (1.0 M in THF,

500 µmol) Tetra-n-butylammoniumfluorid gegeben und die Reaktionsmischung für 2h bei Raumtemperatur nachgerührt. Das Lösungsmittel wurde unter reduziertem Druck entfernt und der Rückstand mittels präparativer HPLC (Acetonitril / Wasser/ 0,1 % Ameisensäure) gereinigt. Es wurden 42.0 mg (36% d. Th., 100% Reinheit) der Titelverbindung erhalten.
LC-MS (Methode 3): $R_t$ = 1.65 min; MS (ESIpos): m/z = 513 $[M+H]^+$
$^1$H NMR (400 MHz, DMSO-$d_6$): δ ppm = 10.52 (d, 1H), 8.61 (s, 1H), 8.28 (d, 1H), 7.73-7.85 (m, 1H), 7.52-7.62 (m, 1H), 7.25-7.37 (m, 1H), 6.76 (d, 1H), 5.08-5.26 (m, 2H), 4.67-4.79 (m, 1H), 4.04 (br s, 1H), 3.92 (br s, 1H), 3.55-3.65 (m, 1H), 3.16-3.28 (m, 2H), 3.00-3.14 (m, 1H), 1.80-1.94 (m, 1H), 1.56-1.70 (m, 1H), 0.97 (t, 3H).

**Beispiel 647**

*N*-[(1*R*)-1-Cyclopropyl-2,2,2-trifluorethyl]-1-(2,6-dichlor-4-fluorphenyl)-4-oxo-7-(2-oxoimidazolidin-1-yl)-1,4-dihydro-1,8-naphthyridin-3-carboxamid

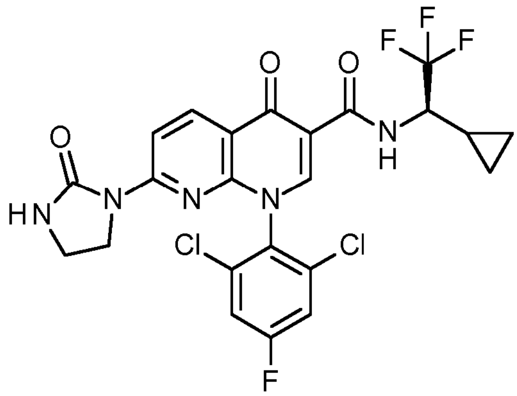

Gemäß AAV2 wurden 60.0 mg (118 µmol) der Verbindung aus Beispiel 130C mit 102 mg (1.18 mmol) Imidazolidin-2-on in Gegenwart von 24.5 mg (177 µmol) Kaliumcarbonat, 5.30 mg (23.6 µmol) Palladiumacetat und 13.6 mg (23.6 µmol) Xantphos in 1.1 ml 1,4-Dioxan zur Reaktion gebracht. Das Rohprodukt wurde mittels präparativer HPLC (Acetonitril / Wasser/ 0,1 % Ameisensäure) gereinigt. Die Substanz wurde mit Verunreinigungen isoliert und wurde weiter aus Acetonitril umkristallisiert, abgesaugt, mit etwas Acetonitril nachgewaschen und nachgetrocknet. Es wurden 33.9 mg (51% d. Th., 100% Reinheit) der Titelverbindung erhalten.
LC-MS (Methode 3): $R_t$ = 2.05 min; MS (ESIpos): m/z = 558 $[M+H]^+$
$^1$H NMR (400 MHz, DMSO-$d_6$): δ ppm = 10.39 (d, 1H), 8.91 (s, 1H), 8.57 (d, 1H), 8.44 (d, 1H), 7.89 (d, 2H), 7.66 (s, 1H), 4.34-4.44 (m, 1H), 3.46-3.56 (m, 2H), 3.32-3.37 (m, 2H), 1.18-1.27 (m, 1H), 0.52-0.70 (m, 3H), 0.32-0.39 (m, 1H).

**Beispiel 648**

1-(2,6-Difluorphenyl)-7-[(3*R*,4*S*)-3,4-dihydroxypyrrolidin-1-yl]-4-oxo-*N*-[(2*R*)-1,1,1-trifluorbutan-2-yl]-1,4-dihydro-1,8-naphthyridin-3-carboxamid

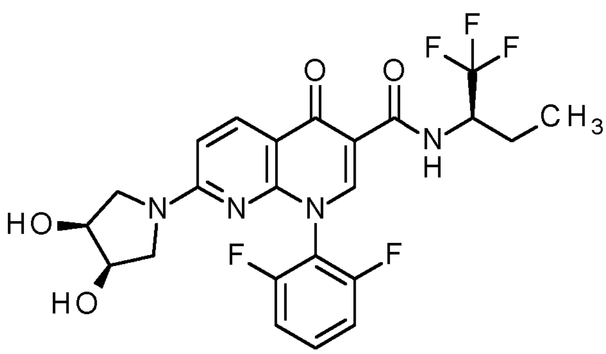

Gemäß AAV3 wurden 40.0 mg (89.7 µmol) der Verbindung aus Beispiel 86A mit 15.0 mg (108 µmol) (3*R*,4*S*)-Pyrrolidin-3,4-diol Hydrochlorid und 55 µl (310 µmol) DIPEA in 410 µl DMF zur Reaktion gebracht. Das Rohprodukt wurde mittels präparativer HPLC (Acetonitril / Wasser/ 0,1 % Ameisensäure) gereinigt. Es wurden 20.0 mg (43% d. Th., 100% Reinheit) der Titelverbindung erhalten.
LC-MS (Methode 3): $R_t$ = 1.72 min; MS (ESIpos): m/z = 513 $[M+H]^+$
$^1H$ NMR (400 MHz, DMSO-$d_6$): δ ppm = 10.44 (d, 1H), 8.75 (s, 1H), 8.28 (d, 1H), 7.67-7.75 (m, 1H), 7.38-7.45 (m, 2H), 6.76 (d, 1H), 5.03 (d, 1H), 4.91 (d, 1H), 4.68-4.78 (m, 1H), 4.08-4.15 (m, 1H), 3.97-4.05 (m, 1H), 3.56-3.63 (m, 1H), 3.24-3.30 (m, 1H), 3.14-3.22 (m, 1H), 2.92-3.01 (m, 1H), 1.83-1.93 (m, 1H), 1.58-1.70 (m, 1H), 0.97 (t, 3H).

## Beispiel 649

1-(2,6-Dichlor-4-fluorphenyl)-4-oxo-7-(2-oxoimidazolidin-l-yl)-*N*-[(2*R*)-1,1,1-trifluorbutan-2-yl]-1,4-dihydro-1,8-naphthyridin-3-carboxamid

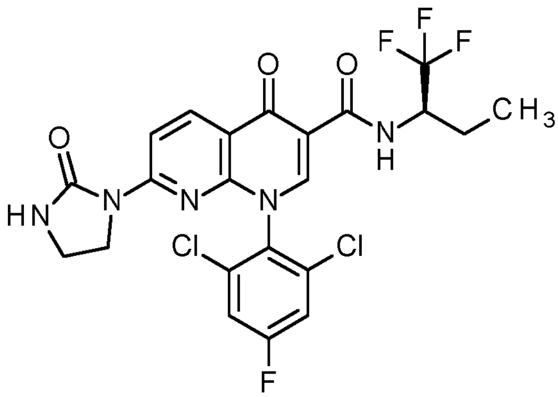

Gemäß AAV2 wurden 60.0 mg (121 µmol) der Verbindung aus Beispiel 131A mit 104 mg (1.21 mmol) Imidazolidin-2-on in Gegenwart von 25.0 mg (181 µmol) Kaliumcarbonat, 5.42 mg (24.2 µmol) Palladiumacetat und 14.0 mg (24.2 µmol) Xantphos in 1.1 ml 1,4-Dioxan zur Reaktion gebracht. Das Rohprodukt wurde mittels präparativer HPLC (Acetonitril / Wasser/ 0,1 % Ameisensäure) gereinigt. Die Substanz wurde mit Verunreinigungen isoliert und wurde weiter aus Acetonitril umkristallisiert, abgesaugt, mit etwas Acetonitril nachgewaschen und nachgetrocknet. Es wurden 24.3 mg (37% d. Th., 100% Reinheit) der Titelverbindung erhalten.
LC-MS (Methode 3): $R_t$ = 2.03 min; MS (ESIpos): m/z = 546 $[M+H]^+$
$^1H$ NMR (400 MHz, DMSO-$d_6$): δ ppm = 10.25 (d, 1H), 8.92 (s, 1H), 8.57 (d, 1H), 8.43 (d, 1H), 7.89 (d, 2H), 7.66 (s, 1H), 4.71-4.82 (m, 1H), 3.47-3.55 (m, 2H), 3.32-3.37 (m, 2H), 1.84-1.94 (m, 1H), 1.61-1.73 (m, 1H), 0.98 (t, 3H).

## Beispiel 650

7-(4-Carbamoylpiperazin-1-yl)-*N*-[(1*R*)-1-cyclopropyl-2,2,2-trifluorethyl]-4-oxo-1-(2,4,6-trifluorphenyl)-1,4-dihydro-1,8-naphthyridin-3-carboxamid

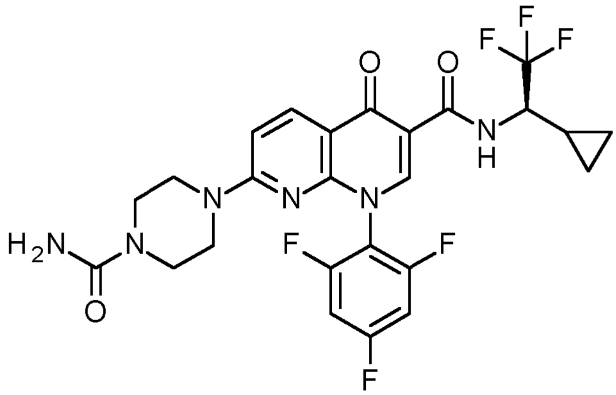

Gemäß AAV1 wurden 80.0 mg (179 µmol) der Verbindung aus Beispiel 168A mit 37.7 mg (215 µmol) (1*R*)-1-Cyclopropyl-2,2,2-trifluorethanamin Hydrochlorid in Gegenwart von 81.6 mg (215 µmol) HATU und 120 µl (720 µmol) DIPEA in 690 µl DMF zur Reaktion gebracht. Das Rohprodukt wurde mittels präparativer HPLC (Acetonitril / Wasser/ 0,1 % Ameisensäure) gereinigt. Es wurden 74.1 mg (73% d. Th., 100% Reinheit) der Titelverbindung erhalten.
LC-MS (Methode 3): $R_t$ = 1.74 min; MS (ESIpos): m/z = 569 $[M+H]^+$
$^1H$ NMR (400 MHz, DMSO-$d_6$): δ ppm = 10.50 (d, 1H), 8.83 (s, 1H), 8.32 (d, 1H), 7.54-7.61 (m, 2H), 7.15 (d, 1H), 6.03 (s, 2H), 4.33-4.43 (m, 1H), 3.44-3.52 (m, 4H), 3.31-3.35 (m, 4H), 1.16-1.25 (m, 1H), 0.50-0.69 (m, 3H), 0.30-0.38 (m, 1H).

**Beispiel 651**

*N*-(2,6-Dichlorphenyl)-1-(2,4-difluorphenyl)-7-[(3*R*,4*S*)-3,4-dihydroxypyrrolidin-1-yl]-4-oxo-1,4-dihydro-1,8-naphthyridin-3-carboxamid

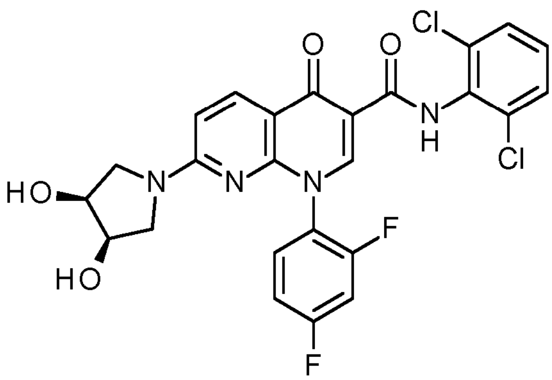

Gemäß AAV3 wurden 40.0 mg (83.2 µmol) der Verbindung aus Beispiel 81A mit 13.9 mg (99.9 µmol) (3*R*,4*S*)-Pyrrolidin-3,4-diol Hydrochlorid und 51 µl (290 µmol) DIPEA in 400 µl DMF zur Reaktion gebracht. Das Rohprodukt wurde mittels präparativer HPLC (Acetonitril / Wasser/ 0,1 % Ameisensäure) gereinigt. Es wurden 32.8 mg (72% d. Th., 100% Reinheit) der Titelverbindung erhalten.
LC-MS (Methode 3): $R_t$ = 1.74 min; MS (ESIpos): m/z = 547 $[M+H]^+$
$^1H$ NMR (400 MHz, DMSO-$d_6$): δ ppm = 12.03 (s, 1H), 8.68 (s, 1H), 8.33 (d, 1H), 7.80-7.89 (m, 1H), 7.53-7.63 (m, 3H), 7.30-7.40 (m, 2H), 6.76 (d, 1H), 4.99-5.07 (m, 1H), 4.91-4.98 (m, 1H), 4.10-4.18 (m, 1H), 3.98-4.09 (m, 1H), 3.55-3.66 (m, 1H), 3.17-3.29 (m, 2H), 2.95-3.09 (m, 1H).

**Beispiel 652**

1-(2,6-Dichlorphenyl)-7-[1-hydroxy-3-azabicyclo[3.1.0]hex-3-yl]-4-oxo-*N*-[(2*R*)-1,1,1-trifluorbutan-2-yl]-1,4-dihydro-1,8-naphthyridin-3-carboxamid (Diastereomerengemisch)

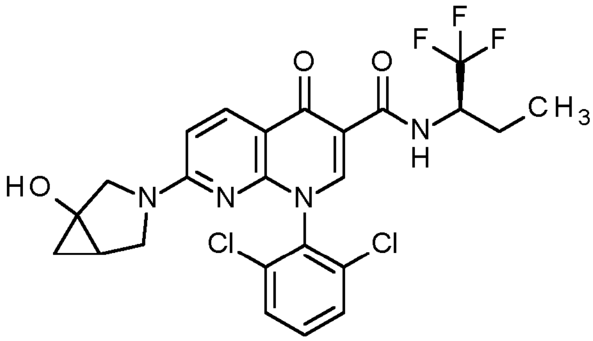

Gemäß AAV3 wurden 72.7 mg (152 µmol) der Verbindung aus Beispiel 166C mit 27.2 mg (182 µmol, 91% Reinheit) 3-Azabicyclo[3.1.0]hexan-1-ol Hydrochlorid und 93 µl (530 µmol) DIPEA in 640 µl DMF zur Reaktion gebracht. Das Rohprodukt wurde mittels präparativer HPLC (Acetonitril / Wasser/ 0,1 % Ameisensäure) gereinigt. Es wurden 62.6 mg (76% d. Th., 100% Reinheit) der Titelverbindung erhalten.

LC-MS (Methode 1): $R_t$ = 1.09 min; MS (ESIpos): m/z = 541 $[M+H]^+$

$^1H$ NMR (400 MHz, DMSO-$d_6$): δ ppm = 10.45 (d, 1H), 8.65 (s, 1H), 8.29 (d, 1H), 7.72-7.81 (m, 2H), 7.61-7.72 (m, 1H), 6.69-6.81 (m, 1H), 5.87-6.08 (m, 1H), 4.68-4.79 (m, 1H), 3.82-3.90 (m, 0.40H), 3.58-3.70 (m, 0.60H), 3.35-3.55 (m, 1.60H), 3.13-3.21 (m, 0.40H), 2.96-3.10 (m, 1H), 1.83-1.93 (m, 1H), 1.48-1.71 (m, 2H), 0.92-1.04 (m, 4H), 0.36-0.44 (m, 1H).

**Beispiel 653**

*N*-(Bicyclo[1.1.1]pent-1-yl)-4-oxo-7-(2-oxoimidazolidin-1-yl)-1-(2,4,6-trifluorphenyl)-1,4-dihydro-1,8-naphthyridin-3-carboxamid

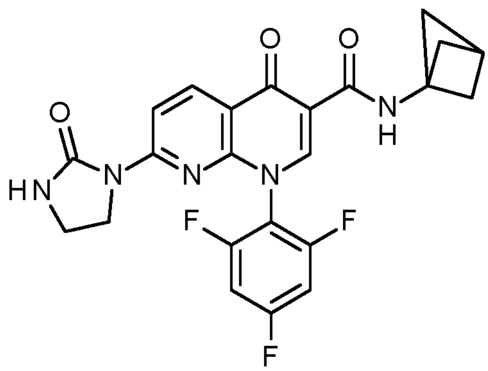

Gemäß AAV1 wurden 50.0 mg (124 µmol) der Verbindung aus Beispiel 113A mit 17.7 mg (148 µmol) Bicyclo[1.1.1]pentan-1-amin Hydrochlorid in Gegenwart von 56.4 mg (148 µmol) HATU und 86 µl (490 µmol) DIPEA in 750 µl DMF zur Reaktion gebracht. Das Rohprodukt wurde mittels präparativer HPLC (Acetonitril / Wasser/ 0,1 % Ameisensäure) gereinigt. Es wurden 36.5 mg (63% d. Th., 100% Reinheit) der Titelverbindung erhalten.

LC-MS (Methode 3): $R_t$ = 1.84 min; MS (ESIpos): m/z = 470 $[M+H]^+$

$^1H$ NMR (400 MHz, DMSO-$d_6$): δ ppm = 10.08 (s, 1 H), 8.85 (s, 1 H), 8.52 (d, 1 H), 8.41 (d, 1 H), 7.65 (s, 1 H), 7.57 (t, 2 H), 3.55 - 3.62 (m, 2 H), 3.32 - 3.37 (m, 2 H), 2.11 (s, 6H).

**Beispiel 654**

1-(2,6-Dichlor-4-fluorphenyl)-4-oxo-7-(2-oxoimidazolidin-1-yl)-*N*-[(2*S*)-1,1,1-trifluorbutan-2-yl]-1,4-dihydro-1,8-naphthyridin-3-carboxamid

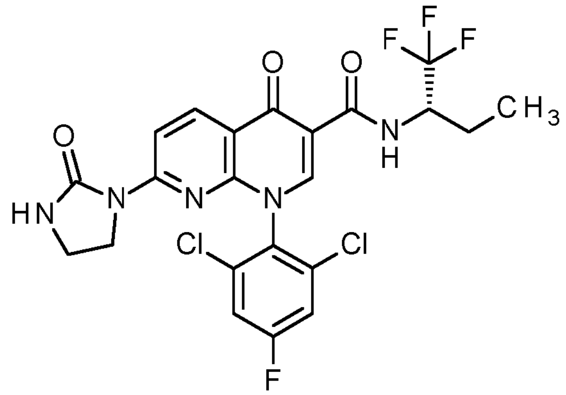

Gemäß AAV2 wurden 60.0 mg (121 μmol) der Verbindung aus Beispiel 132A mit 104 mg (1.21 mmol) Imidazolidin-2-on in Gegenwart von 25.0 mg (181 μmol) Kaliumcarbonat, 5.42 mg (24.2 μmol) Palladiumacetat und 14.0 mg (24.2 μmol) Xantphos in 1.1 ml 1,4-Dioxan zur Reaktion gebracht. Das Rohprodukt wurde mittels präparativer HPLC (Acetonitril / Wasser/ 0,1 % Ameisensäure) gereinigt. Die Substanz wurde mit Verunreinigungen isoliert und wurde weiter aus Acetonitril umkristallisiert, abgesaugt, mit etwas Acetonitril nachgewaschen und nachgetrocknet. Es wurden 46.6 mg (71% d. Th., 100% Reinheit) der Titelverbindung erhalten.
LC-MS (Methode 3): $R_t$ = 2.03 min; MS (ESIpos): m/z = 546 $[M+H]^+$
$^1H$ NMR (400 MHz, DMSO-$d_6$): δ ppm = 10.25 (d, 1 H), 8.92 (s, 1 H), 8.57 (d, 1 H), 8.43 (d, 1 H), 7.89 (d, 2 H), 7.66 (s, 1 H), 4.70 - 4.81 (m, 1 H), 3.47 - 3.55 (m, 2 H), 3.32 - 3.37 (m, 2 H), 1.84 - 1.95 (m, 1 H), 1.61 - 1.73 (m, 1 H), 0.98 (t, 3H).

## Beispiel 655

*N*-(3,3-Difluor-1-methylcyclobutyl)-7-[(3*R*,4*R*)-3,4-dihydroxypyrrolidin-1-yl]-4-oxo-1-(2,4,6-trifluorphenyl)-1,4-dihydro-1,8-naphthyridin-3-carboxamid

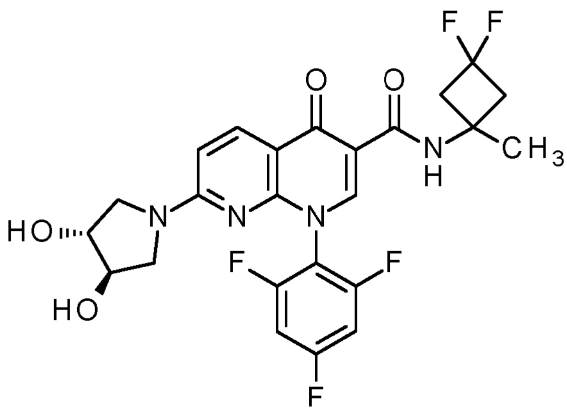

Gemäß AAV1 wurden 50.0 mg (119 μmol) der Verbindung 121A mit 22.4 mg (142 μmol) 3,3-Difluor-1-methylcyclobutanamin Hydrochlorid in Gegenwart von 54.1 mg (142 μmol) HATU und 83 μl (470 μmol) DIPEA in 750 μl DMF zur Reaktion gebracht. Das Rohprodukt wurde mittels präparativer HPLC (Acetonitril / Wasser/ 0,1 % Ameisensäure) gereinigt. Es wurden 39.6 mg (64% d. Th., 100% Reinheit) der Titelverbindung erhalten.
LC-MS (Methode 3): $R_t$ = 1.59 min; MS (ESIpos): m/z = 525 $[M+H]^+$
$^1H$ NMR (400 MHz, DMSO-$d_6$): δ ppm = 10.28 (s, 1 H), 8.70 (s, 1 H), 8.26 (d, 1 H), 7.53 - 7.60 (m, 2 H), 6.76 (d, 1 H), 5.23 (d, 1 H), 5.14 (d, 1 H), 4.04 (br s, 1 H), 3.92 (br s, 1 H), 3.61 (br dd, 1 H), 3.32 - 3.36 (m, 1 H), 3.24 (br dd, 1 H), 2.97 - 3.10 (m, 3 H), 2.70 (td, 2 H), 1.55 (s, 3 H).

**Beispiel 656**

*N*-(3,3-Difluor-1-methylcyclobutyl)-4-oxo-7-(2-oxoimidazolidin-1-yl)-1-(2,4,6-trifluorphenyl)-1,4-dihydro-1,8-naphthyridin-3-carboxamid

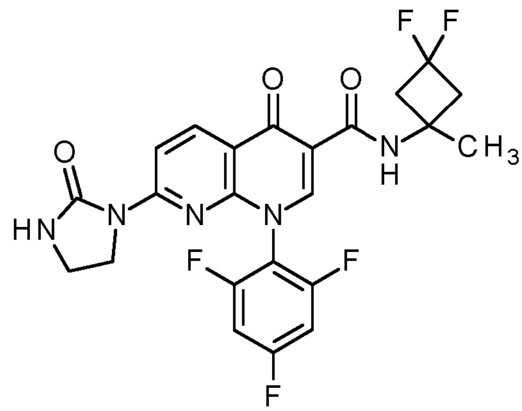

Gemäß AAV1 wurden 50.0 mg (124 µmol) der Verbindung aus Beispiel 113A mit 23.4 mg (148 µmol) 3,3-Difluor-1-methylcyclobutanamin Hydrochlorid in Gegenwart von 56.4 mg (148 µmol) HATU und 86 µl (490 µmol) DIPEA in 750 µl DMF zur Reaktion gebracht. Das Rohprodukt wurde mittels präparativer HPLC (Acetonitril / Wasser/ 0,1 % Ameisensäure) gereinigt. Es wurden 37.9 mg (60% d. Th., 100% Reinheit) der Titelverbindung erhalten.
LC-MS (Methode 3): $R_t$ = 1.83 min; MS (ESIpos): m/z = 508 $[M+H]^+$
$^1H$ NMR (400 MHz, DMSO-$d_6$): δ ppm = 10.08 (s, 1 H), 8.89 (s, 1 H), 8.54 (d, 1 H), 8.42 (d, 1 H), 7.65 (s, 1 H), 7.57 (t, 2 H), 3.55 - 3.62 (m, 2 H), 3.32 - 3.38 (m, 2 H), 2.98 - 3.10 (m, 2 H), 2.68 - 2.77 (m, 2 H), 1.57 (s, 3 H).

**Beispiel 657**

1-(2,4-Difluorphenyl)-7-[4-hydroxy-3,3,5-trimethyl-2-oxopyrrolidin-1-yl]-4-oxo-*N*-[(2*R*)-1,1,1-trifluorbutan-2-yl]-1,4-dihydro-1,8-naphthyridin-3-carboxamid (Diastereomerengemisch)

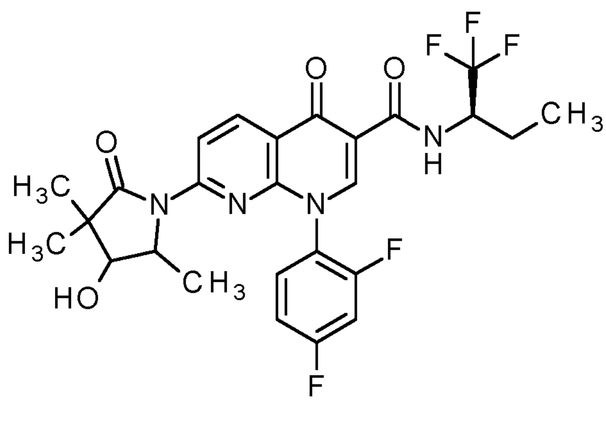

Gemäß AAV2 wurden 31.1 mg (69.8 µmol) der Verbindung aus Beispiel 67A mit 12.0 mg (83.8 µmol) der Verbindung aus Beispiel 164C in Gegenwart von 14.5 mg (105 µmol) Kaliumcarbonat, 3.14 mg (14.0 µmol) Palladiumacetat und 8.08 mg (14.0 µmol) Xantphos in 620 µl 1,4-Dioxan zur Reaktion gebracht. Das Rohprodukt wurde mittels präparativer HPLC (Acetonitril / Wasser/ 0,1 % Ameisensäure) gereinigt. Es wurden 20.0 mg (52% d. Th., 100% Reinheit) der Titelverbindung erhalten.
LC-MS (Methode 4): $R_t$ = 3.72 min; MS (ESIpos): m/z = 553 $[M+H]^+$
$^1H$ NMR (400 MHz, DMSO-$d_6$): δ ppm = 10.21 (br d, 1 H), 8.84 - 8.89 (m, 1 H), 8.70 (dd, 1 H), 8.49 (dd, 1 H), 7.84 - 7.93 (m, 1 H), 7.49 - 7.75 (m, 1 H), 7.29 - 7.41 (m, 1 H), 5.40 (d, 1 H), 4.71 - 4.83 (m, 1 H), 4.03 - 4.13 (m, 1 H), 3.93 - 4.03

(m, 1 H), 1.82 - 1.95 (m, 1 H), 1.60 - 1.73 (m, 1 H), 0.85 - 1.11 (m, 12 H).

**Beispiel 658**

7-[(4*S*)-4-Hydroxy-2-oxopyrrolidin-1-yl]-4-oxo-*N*-[(2*R*)-1,1,1-trifluor-4-methylpentan-2-yl]-1-(2,4,6-trifluorphenyl)-1,4-dihydro-1,8-naphthyridin-3-carboxamid

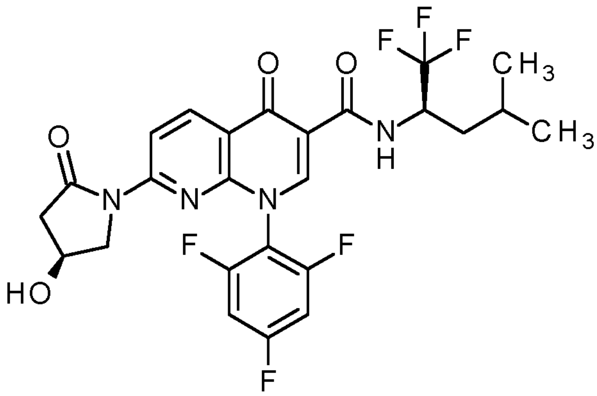

Gemäß AAV1 wurden 50.0 mg (119 μmol) der Verbindung aus Beispiel 117A mit 27.4 mg (143 μmol) (2*R*)-1,1,1-Trifluor-4-methylpentan-2-amin Hydrochlorid in Gegenwart von 54.4 mg (143 μmol) HATU und 62 μl (360 μmol) DIPEA in 460 μl DMF zur Reaktion gebracht. Das Rohprodukt wurde mittels präparativer HPLC (Acetonitril / Wasser/ 0,1 % Ameisensäure) gereinigt. Es wurden 42.5 mg (62% d. Th., 97% Reinheit) der Titelverbindung erhalten.
LC-MS (Methode 3): $R_t$ = 2.10 min; MS (ESIpos): m/z = 557 $[M+H]^+$
$^1H$ NMR (400 MHz, DMSO-$d_6$): δ ppm = 10.10 (d, 1 H), 9.08 (s, 1 H), 8.71 (d, 1 H), 8.54 (d, 1 H), 7.58 - 7.65 (m, 2 H), 5.34 (d, 1 H), 4.81 - 4.89 (m, 1 H), 4.27 - 4.31 (m, 1 H), 3.69 (dd, 1 H), 3.48 (d, 1 H), 2.94 (dd, 1 H), 2.38 (br d, 1 H), 1.65 - 1.74 (m, 2 H), 1.54 - 1.62 (m, 1 H), 0.95 (d, 3 H), 0.90 (d, 3 H).

**Beispiel 659**

*N*-[(1S)-1-Cyclopropyl-2,2,2-trifluorethyl]-7-[5-(hydroxymethyl)-2-oxo-1,3-oxazolidin-3-yl]-4-oxo-1-(2,4,6-trifluorphenyl)-1,4-dihydro-1,8-naphthyridin-3-carboxamid (Diastereomerengemisch)

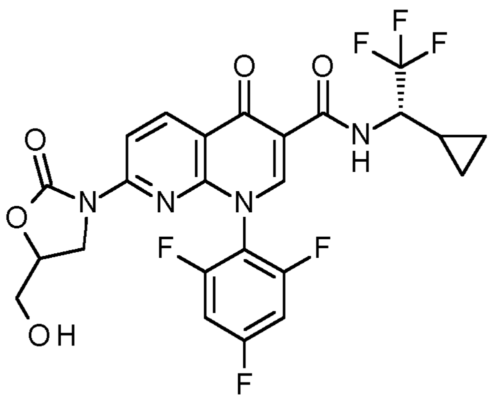

Gemäß AAV2 wurden 150.0 mg (315 μmol) der Verbindung aus Beispiel 126A mit 44.3 mg (378 μmol) 5-(Hydroxymethyl)-1,3-oxazolidin-2-on (Racemat) in Gegenwart von 65.4 mg (473 μmol) Kaliumcarbonat, 14.2 mg (63.1 μmol) Palladiumacetat und 36.5 mg (63.1 μmol) Xantphos in 1.5 ml 1,4-Dioxan zur Reaktion gebracht. Das Rohprodukt wurde mittels präparativer HPLC (Acetonitril / Wasser/ 0,1 % Ameisensäure) gereinigt. Es wurden 17.4 mg (10% d. Th., 100% Reinheit) der Titelverbindung erhalten.

LC-MS (Methode 3): $R_t$ = 1.92 min; MS (ESIpos): m/z = 557 $[M+H]^+$
$^1H$ NMR (400 MHz, DMSO-$d_6$): δ ppm = 10.53 (d, 1 H), 8.81 (s, 1 H), 8.32 - 8.44 (m, 1 H), 8.24 (d, 1 H), 7.50 - 7.59 (m, 2 H), 6.78 (br d, 1 H), 4.67 - 4.78 (m, 1 H), 4.33 - 4.44 (m, 2 H), 4.02 (br t, 1 H), 3.32 - 3.46 (m, 2 H), 1.16 - 1.25 (m, 1 H), 0.49 - 0.69 (m, 3 H), 0.30 - 0.37 (m, 1 H).

**Beispiel 660**

*N*-[(1*S*)-1-Cyclopropyl-2,2,2-trifluorethyl]-7-[(2*S*)-2-(hydroxymethyl)pyrrolidin-1-yl]-4-oxo-1-(2,4,6-trifluorphenyl)-1,4-dihydro-1,8-naphthyridin-3-carboxamid

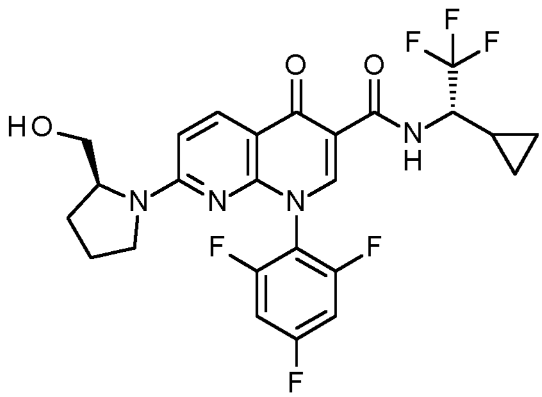

Gemäß AAV3 wurden 100 mg (210 μmol) der Verbindung aus Beispiel 126A mit 25.5 mg (250 μmol) (2*S*)-Pyrrolidin-2-ylmethanol und 110 μl (630 μmol) DIPEA in 850 μl DMF zur Reaktion gebracht. Das Rohprodukt wurde mittels präparativer HPLC (Acetonitril / Wasser/ 0,1 % Ameisensäure) gereinigt. Es wurden 93.6 mg (82% d. Th., 100% Reinheit) der Titelverbindung erhalten.
LC-MS (Methode 3): $R_t$ = 2.05 min; MS (ESIpos): m/z = 541 $[M+H]^+$
$^1H$ NMR (400 MHz, DMSO-$d_6$): δ ppm = 10.57 (d, 1 H), 8.80 (s, 1 H), 8.28 (br d, 1 H), 7.46 - 7.57 (m, 2 H), 6.72 - 6.97 (m, 1 H), 4.85 - 4.94 (m, 0.40 H), 4.42 - 4.50 (m, 0.60 H), 4.31 - 4.42 (m, 1 H), 3.94 - 4.04 (br d, 0.40 H), 3.66 - 3.76 (m, 0.60 H), 3.34 - 3.54 (m, 2 H), 3.05 - 3.28 (m, 2 H), 1.75 - 2.07 (m, 4 H), 1.16 - 1.25 (m, 1 H), 0.49 - 0.69 (m, 3 H), 0.30 - 0.37 (m, 1 H).

**Beispiel 661**

*N*-[(1*S*)-1-Cyclopropyl-2,2,2-trifluorethyl]-7-[(2*R*)-2-(hydroxymethyl)pyrrolidin-1-yl]-4-oxo-1-(2,4,6-trifluorphenyl)-1,4-dihydro-1,8-naphthyridin-3-carboxamid

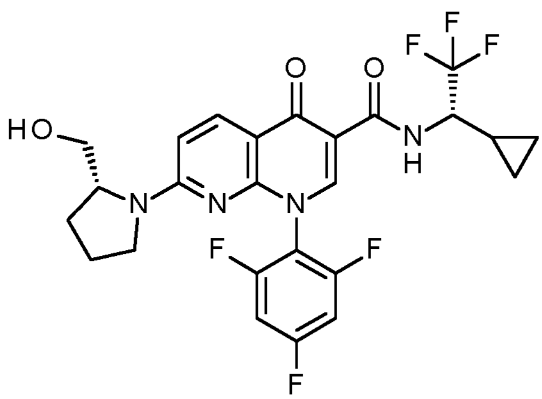

Gemäß AAV3 wurden 100 mg (210 μmol) der Verbindung aus Beispiel 126A mit 29.8 mg (294 μmol) (2*R*)-Pyr-

rolidin-2-ylmethanol und 110 µl (630 µmol) DIPEA in 850 µl DMF zur Reaktion gebracht. Das Rohprodukt wurde mittels präparativer HPLC (Acetonitril / Wasser/ 0,1 % Ameisensäure) gereinigt. Es wurden 109 mg (96% d. Th., 100% Reinheit) der Titelverbindung erhalten.
LC-MS (Methode 3): $R_t$ = 2.06 min; MS (ESIpos): m/z = 541 $[M+H]^+$
$^1H$ NMR (400 MHz, DMSO-$d_6$): δ ppm = 10.57 (br d, 1 H), 8.80 (s, 1 H), 8.28 (br d, 1 H), 7.46 - 7.58 (m, 2 H), 6.85 - 6.98 (m, 0.40 H), 6.72 - 6.85 (m, 0.60 H), 4.83 - 4.96 (m, 0.40 H), 4.43 - 4.50 (m, 0.60 H), 4.32 - 4.43 (m, 1 H), 3.94 - 4.04 (m, 0.40 H), 3.66 - 3.78 (m, 0.60 H), 3.34 - 3.55 (m, 2 H), 3.03 - 3.27 (m, 2 H), 1.76 - 2.06 (m, 4 H), 1.15 - 1.26 (m, 1 H), 0.50 - 0.69 (m, 3 H), 0.31 - 0.38 (m, 1 H).

## Beispiel 662

*N*-[(1*S*)-1-Cyclopropyl-2,2,2-trifluorethyl]-7-(3-hydroxyazetidin-1-yl)-4-oxo-1-(2,4,6-trifluorphenyl)-1,4-dihydro-1,8-naphthyridin-3-carboxamid

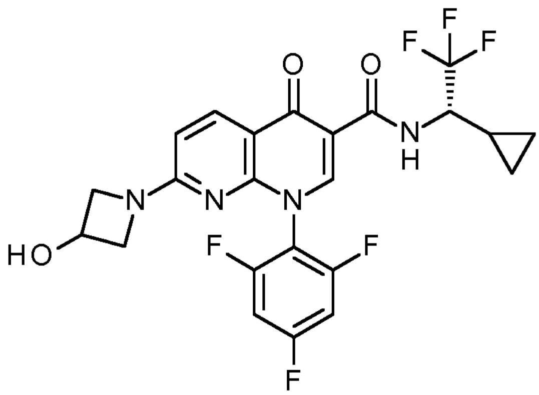

Gemäß AAV3 wurden 200 mg (420 µmol) der Verbindung aus Beispiel 126A mit 64.5 mg (589 µmol) Azetidin-3-ol Hydrochlorid und 220 µl (1.30 mmol) DIPEA in 1.7 ml DMF zur Reaktion gebracht. Das Rohprodukt wurde mittels präparativer HPLC (Acetonitril / Wasser/ 0,1 % Ameisensäure) gereinigt. Es wurden 180 mg (83% d. Th., 100% Reinheit) der Titelverbindung erhalten.
LC-MS (Methode 3): $R_t$ = 1.91 min; MS (ESIpos): m/z = 513 $[M+H]^+$
$^1H$ NMR (400 MHz, DMSO-$d_6$): δ ppm = 10.53 (d, 1 H), 8.79 (s, 1 H), 8.28 (d, 1 H), 7.50 - 7.58 (m, 2 H), 6.61 (d, 1 H), 5.73 (d, 1 H), 4.49 - 4.57 (m, 1 H), 4.34 - 4.42 (m, 1 H), 3.89 - 4.30 (m, 2 H), 3.47 - 3.85 (m, 2 H), 1.16 - 1.25 (m, 1 H), 0.49 - 0.69 (m, 3 H), 0.30 - 0.37 (m, 1 H).

## Beispiel 663

*N*-[(1*S*)-1-Cyclopropyl-2,2,2-trifluorethyl]-7-[4-hydroxy-4-(hydroxymethyl)piperidin-1-yl]-4-oxo-1-(2,4,6-trifluorphenyl)-1,4-dihydro-1,8-naphthyridin-3-carboxamid

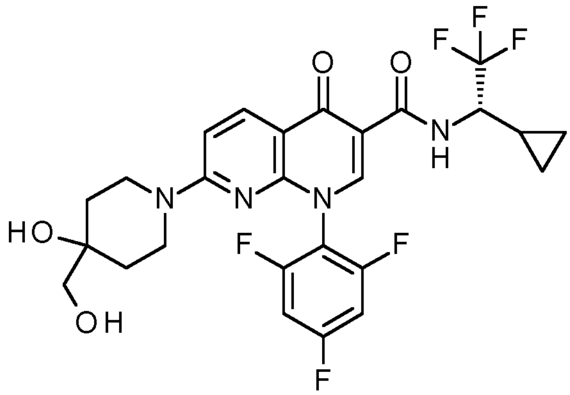

Gemäß AAV3 wurden 100 mg (210 µmol) der Verbindung aus Beispiel 126A mit 38.6 mg (294 µmol) 4-(Hydroxymethyl)piperidin-4-ol und 110 µl (630 µmol) DIPEA in 850 µl DMF zur Reaktion gebracht. Das Rohprodukt wurde mittels präparativer HPLC (Acetonitril / Wasser/ 0,1 % Ameisensäure) gereinigt. Es wurden 78.7 mg (66% d. Th., 100% Reinheit) der Titelverbindung erhalten.
LC-MS (Methode 3): $R_t$ = 1.80 min; MS (ESIpos): m/z = 571 $[M+H]^+$
$^1H$ NMR (400 MHz, DMSO-$d_6$): δ ppm = 10.54 (d, 1 H), 8.80 (s, 1 H), 8.26 (d, 1 H), 8.16 (s, 1 H), 7.56 (t, 2 H), 7.14 (d, 1 H), 4.47 - 4.68 (m, 1 H), 4.23 - 4.47 (m, 2 H), 3.93 (br d, 2 H), 3.15 (s, 2 H), 1.27 - 1.52 (m, 4 H), 1.16 - 1.25 (m, 1 H), 0.49 - 0.69 (m, 3 H), 0.34 (dq, 1 H).

## Beispiel 664

7-[Bis(2-hydroxyethyl)amino]-*N*-[(1*S*)-1-cyclopropyl-2,2,2-trifluorethyl]-4-oxo-1-(2,4,6-trifluorphenyl)-1,4-dihydro-1,8-naphthyridin-3-carboxamid

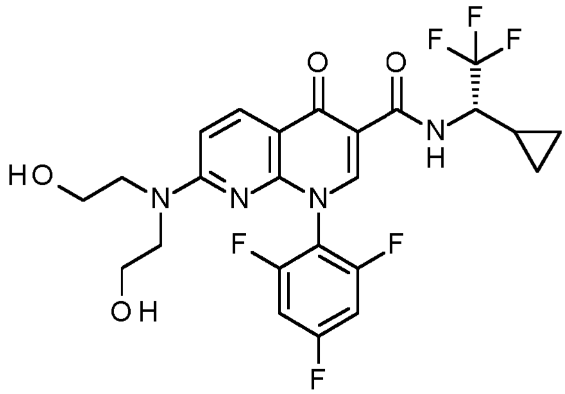

Gemäß AAV3 wurden 100 mg (210 µmol) der Verbindung aus Beispiel 126A mit 30.9 mg (294 µmol) 2,2'-Iminodiethanol und 110 µl (630 µmol) DIPEA in 850 µl DMF zur Reaktion gebracht. Das Rohprodukt wurde mittels präparativer HPLC (Acetonitril / Wasser/ 0,1 % Ameisensäure) gereinigt. Es wurden 87.5 mg (76% d. Th., 100% Reinheit) der Titelverbindung erhalten.
LC-MS (Methode 3): $R_t$ = 1.71 min; MS (ESIpos): m/z = 545 $[M+H]^+$
$^1H$ NMR (400 MHz, DMSO-$d_6$) δ ppm 10.56 (d, 1 H), 8.81 (s, 1 H), 8.25 (d, 1 H), 7.50 - 7.57 (m, 2 H), 7.01 (d, 1 H), 4.72 - 4.90 (m, 1 H), 4.54 - 4.72 (m, 1 H), 4.33 - 4.43 (m, 1 H), 3.52 - 3.68 (m, 4 H), 3.18 - 3.29 (m, 2 H), 1.16 - 1.25 (m, 1 H), 0.50 - 0.69 (m, 3 H), 0.30 - 0.38 (m, 1 H).

**Beispiel 665**

*N*-[(1*S*)-1-Cyclopropyl-2,2,2-trifluorethyl]-7-[3-hydroxy-3-methylpiperidin-1-yl]-4-oxo-1-(2,4,6-trifluorphenyl)-1,4-dihydro-1,8-naphthyridin-3-carboxamid (Diastereomerengemisch)

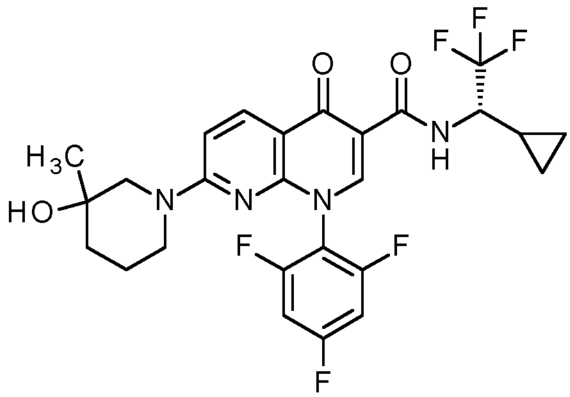

Gemäß AAV3 wurden 200 mg (420 μmol) der Verbindung aus Beispiel 126A mit 67.8 mg (589 μmol) 3-Methylpiperidin-3-ol und 220 μl (1.3 mmol) DIPEA in 1.7 ml DMF zur Reaktion gebracht. Das Rohprodukt wurde mittels präparativer HPLC (Acetonitril / Wasser/ 0,1 % Ameisensäure) gereinigt. Es wurden 220 mg (94% d. Th., 100% Reinheit) der Titelverbindung erhalten.
LC-MS (Methode 3): $R_t$ = 2.11 min; MS (ESIpos): m/z = 555 $[M+H]^+$
$^1H$ NMR (400 MHz, DMSO-$d_6$): δ ppm = 10.56 (s, 1 H), 8.78 (s, 1 H), 8.22 (d, 1 H), 7.53 - 7.60 (m, 2 H), 7.09 (d, 1 H), 4.33 - 4.44 (m, 2 H), 3.34 - 3.80 (m, 2 H), 1.45 - 1.68 (m, 3 H), 1.28 - 1.42 (m, 1 H), 1.15 - 1.25 (m, 1 H), 0.90 - 1.09 (m, 3 H), 0.49 - 0.69 (m, 3 H), 0.29 - 0.37 (m, 1 H).

**Beispiel 666**

Methyl-(5*S*)-3-[6-{[(1*S*)-1-cyclopropyl-2,2,2-trifluorethyl]carbamoyl}-5-oxo-8-(2,4,6-trifluorphenyl)-5,8-dihydro-1,8-naphthyridin-2-yl]-2-oxo-1,3-oxazolidin-5-carboxylat

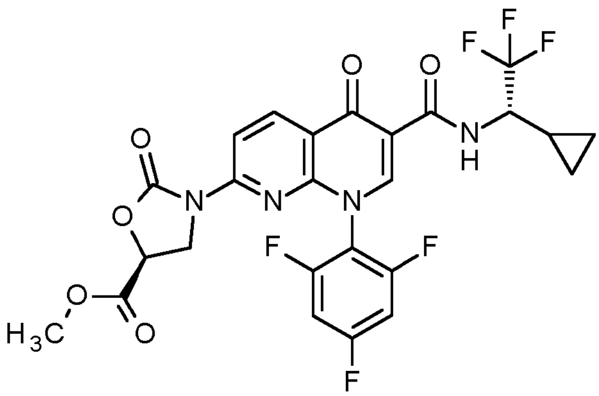

Gemäß AAV2 wurden 1.00 g (2.10 mmol) der Verbindung aus Beispiel 126A mit 366 mg (2.52 mmol) Methyl-(5*S*)-2-oxo-1,3-oxazolidin-5-carboxylat in Gegenwart von 436 mg (3.15 mmol) Kaliumcarbonat, 94.4 mg (420 μmol) Palladiumacetat und 243 mg (420 μmol) Xantphos in 10 ml 1,4-Dioxan zur Reaktion gebracht. Das Rohprodukt wurde mittels präparativer HPLC (Acetonitril / Wasser/ 0,1 % Ameisensäure) gereinigt. Die Substanz wurde mit Verunreinigungen isoliert und wurde weiter aus Acetonitril umkristallisiert, abgesaugt und nachgetrocknet. Es wurden 515 mg (42% d. Th., 100% Reinheit) der Titelverbindung erhalten.
LC-MS (Methode 3): $R_t$ = 2.11 min; MS (ESIpos): m/z = 585 $[M+H]^+$

$^{1}H$ NMR (400 MHz, DMSO-$d_6$): δ ppm = 10.23 (d, 1 H), 9.07 (s, 1 H), 8.76 (d, 1 H), 8.29 (d, 1 H), 7.57 - 7.65 (m, 2 H), 5.27 (dd, 1 H), 4.35 - 4.45 (m, 1 H), 4.04 (t, 1 H), 3.85 (dd, 1 H), 3.73 (s, 3 H), 1.19 - 1.28 (m, 1 H), 0.53 - 0.70 (m, 3 H), 0.31 - 0.39 (m, 1 H).

## Beispiel 667

(5*S*)-3-[6-{[(1*S*)-1-Cyclopropyl-2,2,2-trifluorethyl]carbamoyl}-5-oxo-8-(2,4,6-trifluorphenyl)-5,8-dihydro-1,8-naphthyridin-2-yl]-2-oxo-1,3-oxazolidin-5-carbonsäure

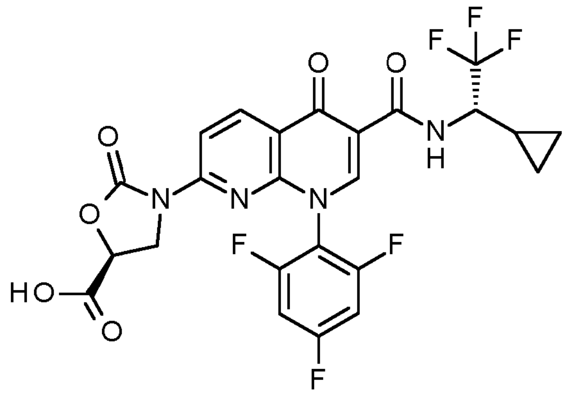

Eine Lösung von 470 mg (804 μmol) der Verbindung aus Beispiel 666 in 21.4 ml einer Mischung aus THF und Wasser (3.1, v/v) wurde bei 0°C mit 33.7 mg (804 μmol) Lithiumhydroxidmonohydrat in Wasser gelöst versetzt. Es wurde 1h bei 0°C nachgerührt. Anschließend wurde die Reaktionslösung auf Wasser mit wenig 1N wässriger Salzsäure gegeben und dreimal mit Essigsäureethylester extrahiert. Die vereinigten organischen Phasen wurden mit gesättigter wässriger Natriumchlorid-Lösung gewaschen und über Natriumsulfat getrocknet. Das Lösemittel wurde unter reduziertem Druck entfernt, die Substanz aus etwas Acetonitril umkristallisiert und am Hochvakuum getrocknet. Es wurden 363 mg (79% d. Th., 100% Reinheit) der Titelverbindung erhalten.

LC-MS (Methode 3): $R_t$ = 1.75 min; MS (ESIpos): m/z = 571 $[M+H]^+$

$^{1}H$ NMR (400 MHz, DMSO-$d_6$): δ ppm = 13.73 (br s, 1 H), 10.23 (d, 1 H), 9.07 (s, 1 H), 8.75 (d, 1 H), 8.29 (d, 1 H), 7.57 - 7.65 (m, 2 H), 5.16 (dd, 1 H), 4.35 - 4.45 (m, 1 H), 4.04 (t, 1 H), 3.77 (dd, 1 H), 1.19 - 1.28 (m, 1 H), 0.52 - 0.70 (m, 3 H), 0.31 - 0.38 (m, 1 H).

## Beispiel 668

7-[(5*S*)-5-Carbamoyl-2-oxo-1,3-oxazolidin-3-yl]-*N*-[(1*S*)-1-cyclopropyl-2,2,2-trifluorethyl]-4-oxo-1-(2,4,6-trifluorphenyl)-1,4-dihydro-1,8-naphthyridin-3-carboxamid

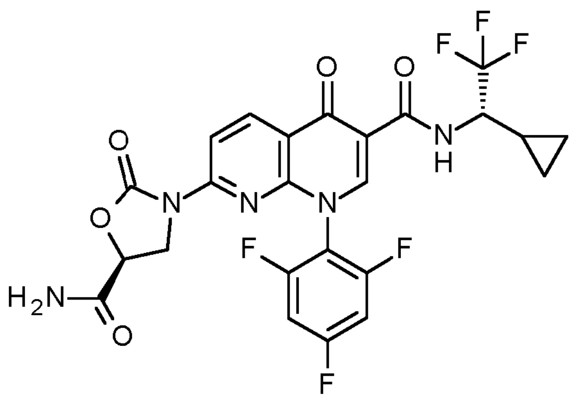

Zu einer Lösung von 327 mg (556 µmol) der Verbindung aus Beispiel 169A in 12 ml THF wurden 11 ml (0.50 M in Dioxan, 5.60 mmol) Ammoniak zugetropft und die Reaktionsmischung für 1h bei Raumtemperatur nachgerührt. Das Lösungsmittel wurde unter reduziertem Druck entfernt und der Rückstand mittels präparativer HPLC (Acetonitril / Wasser/ 0.1% Ameisensäure) gereinigt. Es wurden 311 mg (98% d. Th., 100% Reinheit) der Titelverbindung erhalten.
LC-MS (Methode 3): $R_t$ = 1.80 min; MS (ESIpos): m/z = 570 $[M+H]^+$
$^1H$ NMR (400 MHz, DMSO-$d_6$): δ ppm = 10.23 (d, 1 H), 9.06 (s, 1 H), 8.75 (d, 1 H), 8.31 (d, 1 H), 7.83 (s, 1 H), 7.54 - 7.65 (m, 3 H), 4.99 (dd, 1 H), 4.35 - 4.46 (m, 1 H), 3.99 (t, 1 H), 3.67 (dd, 1 H), 1.19 - 1.28 (m, 1 H), 0.53 - 0.70 (m, 3 H), 0.31 - 0.39 (m, 1 H).

## Beispiel 669

Methyl-(5*R*)-3-[6-{[(1*S*)-1-cyclopropyl-2,2,2-trifluorethyl]carbamoyl}-5-oxo-8-(2,4,6-trifluorphenyl)-5,8-dihydro-1,8-naphthyridin-2-yl]-2-oxo-1,3-oxazolidin-5-carboxylat

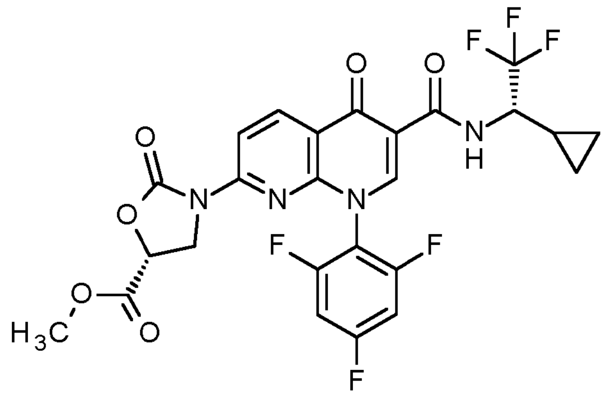

Gemäß AAV2 wurden 1.00 g (2.10 mmol) der Verbindung aus Beispiel 126A mit 366 mg (2.52 mmol) Methyl-(5*R*)-2-oxo-1,3-oxazolidin-5-carboxylat in Gegenwart von 436 mg (3.15 mmol) Kaliumcarbonat, 94.4 mg (420 µmol) Palladiumacetat und 243 mg (420 µmol) Xantphos in 10 ml 1,4-Dioxan zur Reaktion gebracht. Das Rohprodukt wurde mittels präparativer HPLC (Acetonitril / Wasser/ 0,1 % Ameisensäure) gereinigt. Die Substanz wurde mit Verunreinigungen isoliert und wurde weiter aus Acetonitril umkristallisiert, abgesaugt und nachgetrocknet. Es wurden 631 mg (51% d. Th., 99% Reinheit) der Titelverbindung erhalten.
LC-MS (Methode 3): $R_t$ = 2.11 min; MS (ESIpos): m/z = 585 $[M+H]^+$
$^1H$ NMR (400 MHz, DMSO-$d_6$): δ ppm = 10.22 (d, 1 H), 9.07 (s, 1 H), 8.76 (d, 1 H), 8.28 (d, 1 H), 7.58 - 7.65 (m, 2 H), 5.27 (dd, 1 H), 4.35 - 4.45 (m, 1 H), 4.04 (t, 1 H), 3.85 (dd, 1 H), 3.73 (s, 3 H), 1.19 - 1.28 (m, 1 H), 0.53 - 0.70 (m, 3 H), 0.31 - 0.38 (m, 1 H).

## Beispiel 670

(5*R*)-3-[6-{[(1*S*)-1-Cyclopropyl-2,2,2-trifluorethyl]carbamoyl}-5-oxo-8-(2,4,6-trifluorphenyl)-5,8-dihydro-1,8-naphthyridin-2-yl]-2-oxo-1,3-oxazolidin-5-carbonsäure

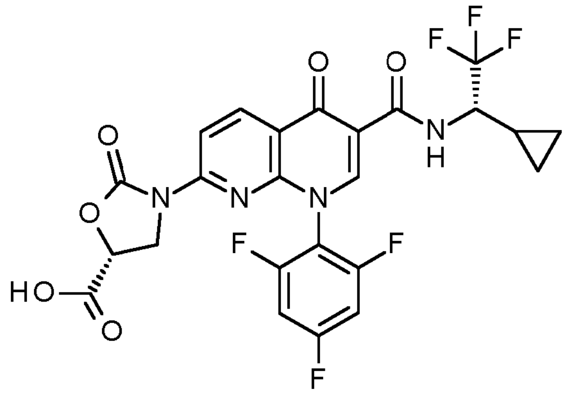

Eine Lösung von 589 mg (1.01 mmol) der Verbindung aus Beispiel 669 in 26.8 ml einer Mischung aus THF und Wasser (3.1, v/v) wurde bei 0°C mit 42.3 mg (1.01 mmol) Lithiumhydroxidmonohydrat in Wasser gelöst versetzt. Es wurde 1h bei 0°C nachgerührt. Anschließend wurde die Reaktionslösung auf Wasser mit wenig 1N wässriger Salzsäure gegeben und dreimal mit Essigsäureethylester extrahiert. Die vereinigten organischen Phasen wurden mit etwas gesättigter wässriger Natriumchlorid-Lösung gewaschen und über Natriumsulfat getrocknet. Das Lösemittel wurde unter reduziertem Druck entfernt, die Substanz aus etwas Acetonitril umkristallisiert und am Hochvakuum getrocknet. Es wurden 485 mg (84% d. Th., 100% Reinheit) der Titelverbindung erhalten.
LC-MS (Methode 3): $R_t$ = 1.75 min; MS (ESIpos): m/z = 571 $[M+H]^+$
$^1$H NMR (400 MHz, DMSO-$d_6$): δ ppm = 13.73 (br s, 1 H), 10.23 (d, 1 H), 9.07 (s, 1 H), 8.75 (d, 1 H), 8.29 (d, 1 H), 7.58 - 7.65 (m, 2 H), 5.16 (dd, 1 H), 4.35 - 4.45 (m, 1 H), 4.04 (t, 1 H), 3.77 (dd, 1 H), 1.19 - 1.28 (m, 1 H), 0.53 - 0.70 (m, 3 H), 0.31 - 0.38 (m, 1 H).

**Beispiel 671**

7-[(5*R*)-5-Carbamoyl-2-oxo-1,3-oxazolidin-3-yl]-*N*-[(1*S*)-1-cyclopropyl-2,2,2-trifluorethyl]-4-oxo-1-(2,4,6-trifluorphenyl)-1,4-dihydro-1,8-naphthyridin-3-carboxamid

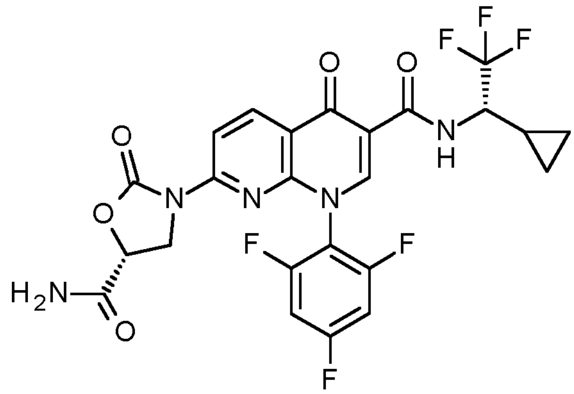

Zu einer Lösung von 454 mg (771 μmol) der Verbindung aus Beispiel 170A in 18 ml THF wurden 15 ml (0.50 M in Dioxan, 7.70 mmol) Ammoniak zugetropft und die Reaktionsmischung für 1h bei Raumtemperatur nachgerührt. Das Lösungsmittel wurde unter reduziertem Druck entfernt und der Rückstand mittels präparativer HPLC (Acetonitril / Wasser/ 0.1% Ameisensäure) gereinigt. Es wurden 421 mg (96% d. Th., 100% Reinheit) der Titelverbindung erhalten.
LC-MS (Methode 3): $R_t$ = 1.79 min; MS (ESIpos): m/z = 570 $[M+H]^+$
$^1$H NMR (400 MHz, DMSO-$d_6$) δ ppm 10.23 (d, 1 H), 9.06 (s, 1 H), 8.75 (d, 1 H), 8.31 (d, 1 H), 7.83 (s, 1 H), 7.54 - 7.65 (m, 3 H), 4.99 (dd, 1 H), 4.35 - 4.45 (m, 1 H), 3.99 (t, 1 H), 3.67 (dd, 1 H), 1.19 - 1.28 (m, 1 H), 0.53 - 0.70 (m, 3 H), 0.31 - 0.38 (m, 1 H).

## B. Bewertung der pharmakologischen Wirksamkeit

Die pharmakologische Aktivität der erfindungsgemäßen Verbindungen kann durch *in vitro*- und *in vivo*-Untersuchungen, wie sie dem Fachmann bekannt sind, nachgewiesen werden. Die nachfolgenden Anwendungsbeispiele beschreiben die biologische Wirkung der erfindungsgemäßen Verbindungen, ohne die Erfindung auf diese Beispiele zu beschränken.

## Abkürzungen und Akronyme:

| | |
|---|---|
| $B_{Max}$ | Anzahl spezifischer Bindungstellen des Radioliganden |
| CAFTY | calcium free tyrode |
| CHO | chinese hamster ovary |
| CRE | cAMP-responsive element |
| DMEM | Dulbecco's modified eagle medium |
| DMSO | Dimethylsulfoxid |
| FCS | fötales Kälberserum |
| FRET | fluorescence resonance energy transfer |
| GIRK1/4 | G-protein-coupled inward rectifier potassium channel, member 1/4 |
| HEPES | Hydroxyethylpiperazin-Ethansulfonsäure |
| HTRF | homogeneous time resolved fluorescence |
| $K_d$ | Gleichgewichtsdissoziationskonstante |
| $K_i$ | Gleichgewichtsinhibitor-Konstante |
| $k_{off}$ | Dissoziationsrate |
| $k_{on}$ | Assoziationsrate |
| nM | nano-molar |
| MEM | minimum essential medium |
| µL | Mikro-Liter |
| µM | mikro-molar |
| mL | milli-Liter |
| mM | milli-molar |
| mtClytin | mitochondriales Clytin |
| min | Minuten |
| NMS | N-Me-Scopolamin |
| PAM | positiv allosterischer Modulator |
| PEI | Polyethylenimin |
| Pen/Strep | Penicillin/Streptomycin |
| sec | Sekunden |

•

## B-1. Funktioneller M2-GIRK1/4-Aktivierungstest

Sowohl die Aktivierung des M2-Rezeptors durch orthosterische Agonisten allein als auch die allosterische Verstärkung der Orthoster-induzierten Aktivierung durch positiv allosterische Modulatoren (PAMs) kann mittels eines zellbasierten funktionellen GIRKl/4-Aktivitätstest bestimmt werden. Die Bindung von orthosterischen Agonisten (endogener Ligand: Acetylcholin) an den M2-Rezeptor führt zu einer Rezeptoraktivierung bzw. Konformationsänderung des Rezeptors im Sinne einer Gleichgewichtsverschiebung zugunsten der aktiven Rezeptorkonformation. Die Bindung der orthosterischen Agonisten an den M2-Rezeptor und damit dessen Aktivierung kann durch positive allosterische Modulatoren, welche nicht an die orthosterische Bindungsstelle der Agonisten, sondern an eine separate allosterische Bindungsstelle binden, verstärkt werden.

Die Agonisten-induzierte Konformationsänderung des M2-Rezeptors hat eine Gai-Proteinaktivierung zur Folge. Die Aktivierung der Ga-Untereinheit wiederum führt zu einer Dissoziation und damit Freisetzung der Gβγ-Untereinheiten von der Ga-Untereinheit und der Aktivierung separater nachgeschalteter Signaltransduktionskaskaden. Der freigesetzte heterodimere Gβγ-Komplex bindet an den GIRK1/4 Kalium-Kanal und induziert eine ligandengesteuerte Kanalaktivierung bzw. -öffnung (Reuveny et al., Nature, July 1994, 370, 143-146). Unter physiologischen Bedingungen kommt es dann zu einem selektiven Ausstrom von Kalium aus der Zelle entlang des elektrochemischen Gradienten. Der Export positiver Ladung führt zu einer Erniedrigung des Transmembranpotentials und damit zu einer Hyperpolarisation der Zelle. Das

Ausmaß der Hyperpolarisation kann daher als Maß für die Aktivierung des M2-Rezeptors angesehen werden.

Als Testzelle dient eine rekombinante CHO-DUKX-Zelllinie, welche mit cDNA kodierend für den humanen M2-Rezeptor sowie mit cDNA kodierend für beide GIRK1/4-Untereinheiten stabil transfiziert wurde (CHO-DUKX-M2-GIRK). Die Bestimmung des Transmembranpotentials bzw. der relativen Änderungen des Transmembranpotentials in Abhängigkeit von Substanzzugabe bzw. M2-Aktivierung erfolgt mittels eines spannungssensitiven Farbstoffes (FLIPR Membrane Potential Assay Kit Blue, Molecular Devices # R8034) und der Messung der Zellfluoreszenz unter Verwendung eines proprietären Fluoreszenz-Imaging-Messgerätes.

### B-1.1. Bestimmung der allosterischen Potenz der Testsubstanzen ($EC_{50}$-Wert)

Die Testsubstanzen werden in Dimethylsulfoxid (DMSO) mit einer Konzentration von 10 mM gelöst und für eine 10 Punkt-Dosis-Wirkungsanalyse in Schritten von 1:3.16 seriell in DMSO verdünnt. Entsprechend der gewünschten Testkonzentrationen werden die Substanzen in Beladungspuffer (Zusammensetzung: 0,6 ml FLIPR Membrane Potential Assay Kit Blue (10 mg/ml), 0,6 ml Brilliant Black (10 mg/ml), 2 mM $CaCl_2$ und 2 mM KCl ad 50 ml Natrium-Gluconat-Tyrode (PAA, #T21-155)) vorverdünnt.

Die in MEM alpha-Medium (supplementiert mit 10% FCS, 2% Glutamax, 1 mg/ml Geniticin) kultivierten Reporter-Zellen wurden mit 2000 Zellen (Messung nach 48 h) bzw. 4000 Zellen (Messung nach 24 h) in 30 μl pro 384 well in μCLEAR/schwarz Greiner-Zellkulturplatten (#781092) ausgesät und 24 h bzw. 48 h bei 37°C inkubiert. Das Aussaatmedium bestand aus MEM alpha-Medium (supplementiert mit 5% FCS, 2% Glutamax, kein Geniticin).

Für die jeweilige Messung wurde das Medium entfernt und die Zellen für mindestens 6 min bei Raumtemperatur mit dem spannungssensitiven Farbstoff beladen (30 μl Beladungspuffer pro 384 Well). Anschließend erfolgte in einer ersten Messung die Bestimmung der Fluoreszenz für das Ruhe-Transmembranpotential für einen Zeitraum von 5 sec. Hiernach erfolgte die Zugabe von je 10 μl der in Beladungspuffer verdünnten Testsubstanzen, gefolgt von einer zweiten Messung zur Bestimmung des Transmembranpotentials für einen Zeitraum von 50 sec. Schließlich wurden die Zellen mit 10 μl Agonistenlösung (Acetylcholin gelöst in Beladungspuffer) versetzt. Acetylcholin wurde mit der dem $EC_{20}$-Wert entsprechenden Konzentration eingesetzt, die in einem Vortest bestimmt wurde. Die M2-vermittelte GIRK1/4-Aktivierung bzw. Hyperpolarisation wurde dann in einer dritten Messung über einen Zeitraum von 60 sec verfolgt. Der $EC_{50}$-Wert (Maß der allosterischen Potenz der Testverbindung) und die Effizienz (Maß der Verstärkung des Acetylcholin-Effektes bei einer $EC_{20}$-Acetylcholin-Konzentration) wurden mit Hilfe einer 4-Parameter-Logistik-Funktion (Hill-Funktion) ermittelt.

### B-1.2. Bestimmung der positiven Kooperativität (α-Faktor)

Die Testsubstanzen wurden in DMSO mit einer Konzentration von 10 mM gelöst und für eine 10 Punkt-Dosis-Wirkungsanalyse in Schritten von 1:3.16 seriell in DMSO verdünnt. Entsprechend der gewünschten Testkonzentrationen wurden die Substanzen in Beladungspuffer (s.o.) vorverdünnt.

Die in MEM alpha-Medium (supplementiert mit 10% FCS, 2% Glutamax, 1mg/ml Geniticin) kultivierten Reporter-Zellen werden mit 2000 Zellen (Messung nach 48 h) bzw. 4000 Zellen (Messung nach 24 h) in 30 μl pro 384 well in μCLEAR/schwarz Greiner-Zellkulturplatten (#781092) ausgesät und 24 h bzw. 48 h bei 37°C inkubiert. Das Aussaatmedium bestand aus MEM alpha-Medium (supplementiert mit 5% FCS, 2% Glutamax, kein Geniticin).

Für die jeweilige Messung wurde das Medium entfernt und die Zellen für mindestens 6 min bei Raumtemperatur mit dem spannungssensitiven Farbstoff beladen (30 μl Beladungspuffer pro 384 well). Anschließend erfolgt in einer ersten Messung die Bestimmung des Ruhe-Transembranpotentials für einen Zeitraum von 5 sec in 1 sec-Inkrementen. Hiernach erfolgt die Zugabe von je 10 μl der in Beladungspuffer verdünnten Testsubstanzen, gefolgt von einer zweiten Messung zur Bestimmung des Transmembranpotentials für einen Zeitraum von 50 sec in 1 sec-Inkrementen.

Schließlich werden die Zellen mit 10 μl Agonistenlösung (Acetylcholin gelöst in Beladungspuffer) versetzt. Im Gegensatz zur $EC_{50}$-Bestimmung der Testsubstanzen (siehe B-1.1) wird dabei aber nicht nur eine Acetylcholin-Konzentration eingesetzt, sondern jede Konzentration der Testsubstanz wird mit einer Acetylcholin-8 Punkt-Dosis-Wirkungskurve kombiniert. Für die Acetylcholin-Verdünnungsreihe wird der Agonist beginnend mit einer maximalen Endkonzentration von 3 μM in Schritten von 1:3.16 seriell in Beladungspuffer entsprechend der gewünschten Endkonzentrationen vorverdünnt. Die M2-vermittelte GIRK1/4-Aktivierung bzw. Hyperpolarisation wird dann in einer dritten Messung über einen Zeitraum von 60 sec in 1 sec-Inkrementen verfolgt. Die Verschiebung der Acetylcholin-Dosis-Wirkungskurve in Anwesenheit zunehmender Konzentrationen der Testsubstanz wird mittels GraphPad PRISM (Allosteric $EC_{50}$ shift) analysiert und quantifiziert. Der ermittelte α-Faktor ist dabei ein Maß für die Stärke und Richtung des allosterischen Effektes. α-Werte > 1 spiegeln eine Erniedrigung des $EC_{50}$-Wertes bzw. eine Erhöhung der Potenz des Agonisten (Acetylcholin) in Anwesenheit von Alloster wider und bedeuten damit eine positive Kooperativität zwischen Orthoster (Acetylcholin) und Alloster (Testsubstanz). Eine positive Kooperativität ist das Kennzeichen eines positiven allosterischen Modulators. Umgekehrt sind α -Werte < 1 bezeichnend für eine negative Kooperativität zwischen Orthoster und Alloster und kennzeichnen damit negative allosterische Modulatoren. α-Werte = 1 bedeuten keine Kooperativität zwischen Orthoster und Alloster, d.h. die Bindungsaffinitäten von Orthoster und Alloster an den Rezeptor beeinflussen sich nicht wechselseitig. Je größer der Betrag des α-Wertes ist, umso größer ist das Ausmaß der Kooperativität zwischen Orthoster und Alloster.

In der folgenden Tabelle 1 sind für individuelle Ausführungsbeispiele die so ermittelten $EC_{50}$- und Effizienz-Werte sowie die α-Werte aus diesem Assay aufgeführt (zum Teil als Mittelwerte aus mehreren unabhängigen Einzelbestimmungen):

**Tabelle 1**

| Bsp. Nr. | Rezeptor-aktivität $EC_{50}$ [µmol/L] | Effizienz [%] | Koopera-tivität (alpha-Faktor) |
|---|---|---|---|
| 1 | 0.014 | 97.5 | 11 |
| 2 | 0.017 | 100.0 | 24 |
| 3 | 0.017 | 99.8 | 30 |
| 4 | 0.024 | 99.8 | 30 |
| 5 | 0.031 | 98.7 | |
| 6 | 0.034 | 98.8 | 15 |

| Bsp. Nr. | Rezeptor-aktivität $EC_{50}$ [µmol/L] | Effizienz [%] | Koopera-tivität (alpha-Faktor) |
|---|---|---|---|
| 7 | 0.034 | 100.0 | 34 |
| 8 | 0.038 | 98.0 | |
| 9 | 0.040 | 96.5 | 11 |
| 10 | 0.060 | 98.5 | 12 |
| 11 | 0.061 | 99.3 | 10 |
| 12 | 0.064 | 95.0 | |

| Bsp. Nr. | Rezeptor-aktivität $EC_{50}$ [µmol/L] | Effizienz [%] | Koopera-tivität (alpha-Faktor) |
|---|---|---|---|
| 13 | 0.071 | 95.8 | 6 |
| 14 | 0.082 | 97.7 | |
| 15 | 0.085 | 90.5 | 8 |
| 16 | 0.090 | 97.3 | |
| 17 | 0.159 | 97.3 | |
| 18 | 0.117 | 97.3 | |
| 19 | 0.058 | 95.0 | 12 |
| 20 | 0.042 | 98.4 | 34 |
| 21 | 0.145 | 100.0 | |
| 22 | 0.131 | 97.7 | |
| 23 | 0.096 | 99.3 | 20 |
| 24 | 0.063 | 100.0 | 25 |
| 25 | 0.084 | 100.0 | 20 |
| 26 | 0.164 | 97.0 | |
| 27 | 0.089 | 97.7 | 20 |
| 28 | 0.023 | 100.0 | 25 |
| 29 | 0.112 | 95.3 | 4 |
| 30 | 0.095 | 93.3 | 11 |
| 31 | 0.040 | 97.0 | 13 |
| 32 | 0.173 | 93.5 | |
| 33 | 0.091 | 96.7 | 20 |
| 34 | 0.095 | 99.7 | |
| 35 | 0.062 | 98.8 | 24 |
| 36 | 0.019 | 96.1 | 16 |
| 37 | 0.023 | 96.3 | |
| 38 | 0.041 | 100.0 | 14 |
| 39 | 0.088 | 92.1 | 14 |

| Bsp. Nr. | Rezeptor-aktivität $EC_{50}$ [µmol/L] | Effizienz [%] | Koopera-tivität (alpha-Faktor) |
|---|---|---|---|
| 40 | 0.106 | 94.2 | |
| 41 | 0.039 | 99.4 | |
| 42 | 0.017 | 94.5 | |
| 43 | 0.141 | 96.6 | |
| 44 | 0.111 | 98.3 | 12 |
| 45 | 0.028 | 99.7 | |
| 46 | 0.127 | 98.7 | |
| 47 | 0.043 | 100.0 | |
| 48 | 0.110 | 94.8 | |
| 49 | 0.089 | 86.0 | |
| 50 | 0.158 | 95.0 | |
| 51 | 0.100 | 95.0 | |
| 52 | 0.104 | 98.5 | |
| 53 | 0.058 | 100.0 | |
| 54 | 0.085 | 98.3 | |
| 55 | 0.036 | 99.8 | 25 |
| 56 | 0.056 | 100.0 | 15 |
| 57 | 0.082 | 93.8 | 7 |
| 58 | 0.077 | 100.0 | 9 |
| 59 | 0.094 | 97.0 | 11 |
| 60 | 0.155 | 100.0 | 8 |
| 61 | 0.016 | 98.2 | |
| 62 | 0.017 | 95.5 | |
| 63 | 0.040 | 88.0 | |
| 64 | 0.012 | 89.7 | 28 |
| 65 | 0.007 | 93.7 | 36 |
| 66 | 0.010 | 92.0 | |

| Bsp. Nr. | Rezeptor-aktivität $EC_{50}$ [µmol/L] | Effizienz [%] | Kooperativität (alpha-Faktor) |
|---|---|---|---|
| 67 | 0.138 | 78.0 | |
| 68 | 0.058 | 97.5 | |
| 69 | 0.013 | 99.5 | |
| 70 | 0.076 | 97.4 | 29 |
| 71 | 0.039 | 95.0 | 30 |
| 72 | 0.053 | 94.0 | |
| 73 | 0.073 | 91.9 | |
| 74 | 0.058 | 87.0 | |
| 75 | 0.073 | 87.0 | |
| 76 | 0.069 | 96.0 | |
| 77 | 0.666 | 100.0 | |
| 78 | 0.079 | 94.3 | |
| 79 | 0.144 | 92.2 | |
| 80 | 0.073 | 94.0 | |
| 81 | 0.102 | 89.7 | |
| 82 | 0.060 | 100.0 | |
| 83 | 0.110 | 88.9 | |
| 84 | 0.077 | 100.0 | |
| 85 | 0.118 | 98.5 | 26 |
| 86 | 0.108 | 94.5 | |
| 87 | 0.129 | 94.0 | 26 |
| 88 | 0.042 | 95.0 | |
| 89 | 0.413 | 96.6 | 20 |
| 90 | 0.298 | 94.7 | 11 |
| 91 | 0.058 | 90.5 | |
| 92 | 0.174 | 95.5 | |
| 93 | 0.213 | 85.7 | 8 |
| 94 | 0.164 | 98.7 | |
| 95 | 0.144 | 90.0 | |
| 96 | 0.134 | 92.3 | |
| 97 | 0.209 | 95.0 | 9 |
| 98 | 0.061 | 97.4 | 14 |
| 99 | 0.182 | 92.7 | |
| 100 | 0.143 | 96.3 | |
| 101 | 0.026 | 76.8 | |
| 102 | 0.031 | 94.0 | |
| 103 | 0.050 | 95.7 | |
| 104 | 0.028 | 96.0 | 15 |
| 105 | 0.146 | 97.7 | |
| 106 | 0.032 | 100.0 | |
| 107 | 0.057 | 91.8 | 14 |
| 108 | 0.024 | 95.0 | |
| 109 | 0.187 | 95.0 | |
| 110 | 0.062 | 98.0 | |
| 111 | 0.035 | 100.0 | 26 |
| 112 | 0.029 | 85.5 | |
| 113 | 0.141 | 91.0 | |
| 114 | 0.098 | 93.3 | |
| 115 | 0.073 | 91.0 | |
| 116 | 0.059 | 93.7 | |
| 117 | 0.089 | 90.5 | |
| 118 | 0.091 | 91.0 | |
| 119 | 0.090 | 94.3 | |
| 120 | 0.062 | 86.5 | |

| Bsp. Nr. | Rezeptor-aktivität $EC_{50}$ [µmol/L] | Effizienz [%] | Kooperativität (alpha-Faktor) |
|---|---|---|---|
| 121 | 0.295 | 88.3 | 8 |
| 122 | 0.094 | 87.7 | |
| 123 | 0.115 | 97.7 | 14 |
| 124 | 0.155 | 88.5 | |
| 125 | 0.116 | 93.0 | |
| 126 | 0.180 | 81.0 | |
| 127 | 0.077 | 86.5 | |
| 128 | 0.057 | 91.7 | 18 |
| 129 | 0.045 | 88.1 | |
| 130 | 0.113 | 90.0 | |
| 131 | 0.157 | 89.8 | |
| 132 | 0.010 | 98.0 | 7 |
| 133 | 0.019 | 94.0 | |
| 134 | 0.032 | 92.5 | |
| 135 | 0.017 | 91.0 | 8 |
| 136 | 0.024 | 81.5 | |
| 137 | 0.016 | 97.0 | |
| 138 | 0.068 | 100.0 | |
| 139 | 0.065 | 100.0 | |
| 140 | 0.068 | 99.0 | |
| 141 | 0.083 | 99.4 | |
| 142 | 0.110 | 100.0 | |
| 143 | 0.037 | 95.5 | |
| 144 | 0.032 | 92.7 | |
| 145 | 0.030 | 100.0 | |
| 146 | 0.026 | 95.8 | |
| 147 | 0.082 | 94.9 | |

| Bsp. Nr. | Rezeptor-aktivität $EC_{50}$ [µmol/L] | Effizienz [%] | Kooperativität (alpha-Faktor) |
|---|---|---|---|
| 148 | 0.041 | 94.9 | |
| 149 | 0.085 | 100.0 | |
| 150 | 0.072 | 90.5 | |
| 151 | 0.023 | 86.4 | |
| 152 | 0.010 | 97.0 | |
| 153 | 0.104 | 100.0 | |
| 154 | 0.158 | 97.6 | |
| 155 | 0.173 | 97.7 | |
| 156 | 0.086 | 84.7 | |
| 157 | 0.067 | 100.0 | |
| 158 | 0.184 | 100.0 | |
| 159 | 0.117 | 88.9 | |
| 160 | 0.085 | 92.5 | |
| 161 | 0.137 | 99.5 | |
| 162 | 0.038 | 93.2 | |
| 163 | 0.173 | 92.8 | 13 |
| 164 | 0.031 | 97.3 | 21 |
| 165 | 0.011 | 40.5 | |
| 166 | 0.014 | 99.0 | |
| 167 | 0.047 | 95.0 | |
| 168 | 0.060 | 95.0 | 13 |
| 169 | 0.101 | 79.5 | |
| 170 | 0.019 | 96.5 | |
| 171 | 0.030 | 90.5 | |
| 172 | 0.083 | 87.3 | |
| 173 | 0.088 | 100.0 | |
| 174 | 0.113 | 98.6 | 25 |

| Bsp. Nr. | Rezeptor-aktivität $EC_{50}$ [µmol/L] | Effizienz [%] | Koopera-tivität (alpha-Faktor) | Bsp. Nr. | Rezeptor-aktivität $EC_{50}$ [µmol/L] | Effizienz [%] | Koopera-tivität (alpha-Faktor) |
|---|---|---|---|---|---|---|---|
| 175 | 0.069 | 97.3 | 33 | 202 | 0.089 | 93.3 | 18 |
| 176 | 0.095 | 94.1 | 28 | 203 | 0.062 | 92.5 | 8 |
| 177 | 0.138 | 82.4 | 5 | 204 | 0.034 | 92.5 | 8 |
| 178 | 0.197 | 81.3 | | 205 | 0.105 | 85.0 | 4 |
| 179 | 0.169 | 63.3 | | 206 | 0.107 | 95.8 | 14 |
| 180 | 0.115 | 96.6 | | 207 | 0.035 | 100.0 | 18 |
| 181 | 0.059 | 94.5 | 36 | 208 | 0.115 | 97.5 | 23 |
| 182 | 0.182 | 94.8 | | 209 | 0.163 | 94.7 | |
| 183 | 0.166 | 98.0 | | 210 | 0.285 | 93.5 | |
| 184 | 0.129 | 99.0 | | 211 | 0.021 | 94.8 | 35 |
| 185 | 0.156 | 97.6 | | 212 | 0.041 | 99.4 | 54 |
| 186 | 0.030 | 94.0 | | 213 | 0.019 | 99.6 | 55 |
| 187 | 0.098 | 93.5 | 10 | 214 | 0.107 | 97.0 | 43 |
| 188 | 0.113 | 98.5 | 9 | 215 | 0.178 | 100.0 | |
| 189 | 0.023 | 90.5 | 24 | 216 | 0.089 | 100.0 | 35 |
| 190 | 0.046 | 93.7 | 12 | 217 | 0.036 | 96.3 | 18 |
| 191 | 0.060 | 85.3 | 7 | 218 | 0.153 | 93.5 | 33 |
| 192 | 0.054 | 91.7 | 9 | 219 | 0.106 | 97.0 | 33 |
| 193 | 0.036 | 100.0 | 50 | 220 | 0.116 | 93.0 | 29 |
| 194 | 0.140 | 96.0 | 16 | 221 | 0.106 | 97.5 | 24 |
| 195 | 0.343 | 96.3 | | 222 | 0.200 | 100.0 | |
| 196 | 0.137 | 98.0 | | 223 | 0.084 | 99.0 | 30 |
| 197 | 0.120 | 92.0 | 10 | 224 | 0.157 | 97.5 | 31 |
| 198 | 0.113 | 96.0 | 13 | 225 | 0.363 | 99.7 | 33 |
| 199 | 0.263 | 96.0 | 15 | 226 | 0.083 | 100.0 | 35 |
| 200 | 0.293 | 83.3 | | 227 | 0.073 | 89.5 | 14 |
| 201 | 0.039 | 94.8 | 39 | 228 | 0.051 | 98.7 | 12 |

| Bsp. Nr. | Rezeptor-aktivität $EC_{50}$ [µmol/L] | Effizienz [%] | Koopera-tivität (alpha-Faktor) |
|---|---|---|---|
| 229 | 0.052 | 100.0 | 13 |
| 230 | 0.203 | 66.3 | |
| 231 | 0.120 | 95.4 | 12 |
| 232 | 0.097 | 91.8 | 8 |
| 233 | 0.057 | 100.0 | 41 |
| 234 | 0.075 | 89.8 | 28 |
| 235 | 0.207 | 98.3 | |
| 236 | 0.860 | 100.0 | |
| 237 | 0.751 | 100.0 | |
| 238 | 1.640 | 92.3 | |
| 239 | 0.112 | 99.3 | 30 |
| 240 | 0.110 | 90.7 | 10 |
| 241 | 0.021 | 95.8 | 18 |
| 242 | 0.090 | 96.7 | 10 |
| 243 | 0.015 | 98.0 | 33 |
| 244 | 0.022 | 93.7 | 58 |
| 245 | 0.112 | 98.0 | 42 |
| 246 | 0.023 | 100.0 | 53 |
| 247 | 0.160 | 93.0 | |
| 248 | 0.330 | 91.7 | |
| 249 | 0.505 | 89.0 | |
| 250 | 0.101 | 95.5 | 27 |
| 251 | 0.163 | 100.0 | |
| 252 | 0.109 | 95.5 | |
| 253 | 0.483 | 100.0 | |
| 254 | 0.337 | 94.0 | |
| 255 | 0.083 | 96.7 | 31 |

| Bsp. Nr. | Rezeptor-aktivität $EC_{50}$ [µmol/L] | Effizienz [%] | Koopera-tivität (alpha-Faktor) |
|---|---|---|---|
| 256 | 0.147 | 95.0 | |
| 257 | 0.138 | 100.0 | |
| 258 | 0.147 | 99.3 | 30 |
| 259 | 0.560 | 94.5 | 14 |
| 260 | 0.280 | 96.5 | |
| 261 | 0.075 | 95.7 | 13 |
| 262 | 0.072 | 89.0 | 27 |
| 263 | 0.440 | 68.5 | |
| 264 | 0.530 | 76.3 | |
| 265 | 23.700 | 100.0 | |
| 266 | 0.743 | 100.0 | |
| 267 | 0.024 | 100.0 | 28 |
| 269 | 0.210 | 98.0 | |
| 270 | 1.600 | 94.0 | |
| 271 | 0.040 | 91.0 | 43 |
| 272 | 0.033 | 98.0 | 55 |
| 273 | 0.044 | 100.0 | |
| 274 | 0.049 | 99.8 | |
| 275 | 0.018 | 92.8 | 38 |
| 276 | 0.080 | 100.0 | 28 |
| 277 | 0.078 | 98.5 | |
| 278 | 0.077 | 97.5 | |
| 279 | 0.066 | 92.5 | 34 |
| 280 | 0.044 | 96.5 | 24 |
| 281 | 0.034 | 100.0 | 44 |
| 282 | 0.021 | 99.5 | 32 |
| 283 | 0.040 | 97.0 | |

| Bsp. Nr. | Rezeptor-aktivität $EC_{50}$ [µmol/L] | Effizienz [%] | Koopera-tivität (alpha-Faktor) |
|---|---|---|---|
| 284 | 0.061 | 97.8 | 41 |
| 285 | 0.598 | 79.9 | 23 |
| 286 | 0.094 | 40.0 | 3 |
| 287 | 0.145 | 94.8 | 24 |
| 288 | 0.040 | 84.0 | 12 |
| 289 | 0.061 | 94.3 | |
| 290 | 0.312 | 100.0 | |
| 291 | 0.673 | 99.5 | |
| 292 | 0.059 | 90.5 | |
| 293 | 0.073 | 88.5 | |
| 294 | 0.065 | 92.5 | 31 |
| 295 | 0.021 | 100.0 | 71 |
| 296 | 0.028 | 97.4 | 49 |
| 297 | 0.037 | 97.3 | 47 |
| 298 | 0.074 | 94.0 | |
| 299 | 0.056 | 92.0 | |
| 300 | 0.054 | 95.0 | |
| 301 | 0.030 | 95.5 | 12 |
| 302 | 0.017 | 96.0 | |
| 303 | 0.060 | 95.0 | |
| 304 | 0.009 | 93.0 | 24 |
| 305 | 0.012 | 98.5 | 23 |
| 306 | 0.052 | 98.5 | 21 |
| 307 | 0.014 | 99.5 | 36 |
| 308 | 0.030 | 100.0 | |
| 309 | 0.040 | 96.0 | |
| 310 | 0.014 | 94.5 | 21 |

| Bsp. Nr. | Rezeptor-aktivität $EC_{50}$ [µmol/L] | Effizienz [%] | Koopera-tivität (alpha-Faktor) |
|---|---|---|---|
| 311 | 0.025 | 95.0 | 20 |
| 312 | 0.072 | 97.0 | |
| 313 | 0.071 | 80.0 | |
| 314 | 0.027 | 100.0 | 24 |
| 315 | 0.038 | 96.3 | 25 |
| 316 | 0.028 | 97.0 | |
| 317 | 0.026 | 88.0 | |
| 318 | 0.014 | 86.0 | |
| 319 | 0.025 | 95.5 | |
| 320 | 0.130 | 90.0 | 6 |
| 321 | 0.043 | 58.5 | |
| 322 | 0.040 | 96.0 | |
| 323 | 0.014 | 94.6 | 16 |
| 324 | 0.034 | 97.3 | |
| 325 | 0.007 | 93.0 | 56 |
| 326 | 0.047 | 100.0 | |
| 327 | 0.069 | 95.5 | |
| 328 | 0.079 | 100.0 | |
| 329 | 0.076 | 95.0 | 16 |
| 330 | 0.041 | 93.3 | 29 |
| 331 | 0.033 | 96.0 | 7 |
| 332 | 0.020 | 99.2 | 37 |
| 333 | 0.031 | 94.5 | 28 |
| 334 | 0.007 | 84.0 | |
| 335 | 0.023 | 94.5 | 7 |
| 336 | 0.012 | 92.0 | 13 |
| 337 | 0.036 | 68.0 | |

| Bsp. Nr. | Rezeptor-aktivität $EC_{50}$ [µmol/L] | Effizienz [%] | Koopera-tivität (alpha-Faktor) |
|---|---|---|---|
| 338 | 0.007 | 99.0 | 24 |
| 339 | 0.044 | 94.0 | 26 |
| 340 | 0.010 | 100.0 | 22 |
| 341 | 0.056 | 86.0 | |
| 342 | 0.011 | 93.7 | 13 |
| 343 | 0.025 | 90.5 | |
| 344 | 0.054 | 95.5 | |
| 345 | 0.059 | 100.0 | |
| 346 | 0.050 | 96.0 | |
| 347 | 0.034 | 92.5 | |
| 348 | 0.061 | 100.0 | |
| 349 | 0.004 | 100.0 | 52 |
| 350 | 0.006 | 98.5 | 42 |
| 351 | 0.017 | 100.0 | |
| 352 | 0.017 | 100.0 | |
| 353 | 0.050 | 99.0 | |
| 354 | 0.038 | 93.0 | |
| 355 | 0.085 | 99.5 | |
| 356 | 0.094 | 92.5 | |
| 357 | 0.004 | 100.0 | 63 |
| 358 | 0.024 | 95.0 | 31 |
| 359 | 0.013 | 98.5 | |
| 360 | 0.006 | 100.0 | 36 |
| 361 | 0.017 | 100.0 | |
| 362 | 0.077 | 97.0 | 27 |
| 363 | 0.045 | 95.0 | 28 |
| 364 | 0.016 | 93.0 | 25 |
| 365 | 0.021 | 97.0 | 20 |
| 366 | 0.035 | 100.0 | 36 |
| 367 | 0.100 | 93.0 | |
| 368 | 0.091 | 100.0 | |
| 369 | 0.008 | 95.0 | 39 |
| 370 | 0.014 | 97.2 | 32 |
| 371 | 0.018 | 97.5 | 36 |
| 372 | 0.020 | 100.0 | 34 |
| 373 | 0.089 | 100.0 | |
| 374 | 0.049 | 60.0 | |
| 375 | 0.040 | 96.0 | |
| 376 | 0.016 | 93.0 | |
| 377 | 0.048 | 99.0 | 34 |
| 378 | 0.081 | 76.8 | |
| 379 | 0.035 | 95.5 | |
| 380 | 0.046 | 88.0 | |
| 381 | 0.023 | 87.0 | |
| 382 | 0.072 | 91.0 | |
| 383 | 0.018 | 85.5 | |
| 384 | 0.028 | 88.0 | 22 |
| 385 | 0.068 | 72.5 | 11 |
| 386 | 0.009 | 97.8 | 39 |
| 387 | 0.042 | 95.5 | |
| 388 | 0.052 | 89.0 | |
| 389 | 0.038 | 85.5 | |
| 390 | 0.014 | 88.0 | |
| 391 | 0.008 | 97.0 | |

| Bsp. Nr. | Rezeptor-aktivität $EC_{50}$ [µmol/L] | Effizienz [%] | Kooperativität (alpha-Faktor) |
|---|---|---|---|
| 392 | 0.006 | 100.0 | |
| 393 | 0.125 | 100.0 | |
| 394 | 0.007 | 100.0 | |
| 395 | 0.007 | 100.0 | |
| 396 | 0.017 | 92.0 | 29 |
| 397 | 0.032 | 90.0 | |
| 398 | 0.010 | 100.0 | 26 |
| 399 | 0.040 | 96.5 | 11 |
| 400 | 0.005 | 100.0 | 32 |
| 401 | 0.041 | 95.5 | |
| 402 | 0.025 | 97.5 | 18 |
| 403 | 0.032 | 100.0 | |
| 404 | 0.007 | 98.3 | 35 |
| 405 | 0.008 | 95.5 | |
| 406 | 0.009 | 88.0 | |
| 407 | 0.007 | 98.5 | 32 |
| 408 | 0.009 | 94.0 | |
| 409 | 0.019 | 99.5 | 22 |
| 410 | 0.009 | 87.0 | 36 |
| 411 | 0.021 | 100.0 | |
| 412 | 0.016 | 98.0 | 34 |
| 413 | 0.010 | 100.0 | |
| 414 | 0.009 | 100.0 | 40 |
| 415 | 0.006 | 96.0 | |
| 416 | 0.008 | 92.0 | |
| 417 | 0.024 | 100.0 | |
| 418 | 0.017 | 89.0 | 7 |

| Bsp. Nr. | Rezeptor-aktivität $EC_{50}$ [µmol/L] | Effizienz [%] | Kooperativität (alpha-Faktor) |
|---|---|---|---|
| 419 | 0.046 | 91.5 | |
| 420 | 0.016 | 97.5 | |
| 421 | 0.048 | 100.0 | |
| 422 | 0.006 | 79.0 | 16 |
| 423 | 3.150 | 53.5 | |
| 424 | 0.007 | 99.0 | 15 |
| 425 | 0.005 | 90.5 | 9 |
| 426 | 0.048 | 92.0 | |
| 427 | 0.019 | 91.5 | |
| 428 | 0.072 | 86.0 | |
| 429 | 0.016 | 99.0 | |
| 430 | 0.061 | 90.0 | |
| 431 | 0.092 | 98.0 | |
| 432 | 0.040 | 94.0 | 26 |
| 433 | 0.170 | 100.0 | |
| 434 | 0.061 | 92.0 | |
| 435 | 0.084 | 100.0 | |
| 436 | 0.081 | 94.0 | |
| 437 | 0.009 | 98.4 | 29 |
| 438 | 0.037 | 99.5 | |
| 439 | 0.020 | 100.0 | 35 |
| 440 | 0.023 | 98.5 | |
| 441 | 0.015 | 100.0 | |
| 442 | 0.010 | 100.0 | |
| 443 | 0.009 | 100.0 | |
| 444 | 0.051 | 100.0 | |
| 445 | 0.096 | 97.5 | |

| Bsp. Nr. | Rezeptor-aktivität $EC_{50}$ [µmol/L] | Effizienz [%] | Koopera-tivität (alpha-Faktor) |
|---|---|---|---|
| 446 | 0.050 | 85.0 | 21 |
| 447 | 0.024 | 93.0 | |
| 448 | 0.019 | 97.0 | 22 |
| 449 | 0.015 | 91.0 | 34 |
| 450 | 0.019 | 78.5 | |
| 451 | 0.008 | 91.0 | 21 |
| 452 | 0.013 | 100.0 | 25 |
| 453 | 0.011 | 98.5 | |
| 454 | 0.007 | 100.0 | 28 |
| 455 | 0.010 | 100.0 | 17 |
| 456 | 0.009 | 92.0 | 32 |
| 457 | 0.004 | 82.0 | 36 |
| 458 | 0.003 | 100.0 | |
| 459 | 0.004 | 95.0 | |
| 460 | 0.005 | 98.0 | |
| 461 | 0.039 | 89.0 | |
| 462 | 0.018 | 88.0 | 17 |
| 463 | 0.054 | 89.0 | |
| 464 | 0.128 | 82.5 | |
| 465 | 0.076 | 89.0 | |
| 466 | 0.026 | 100.0 | |
| 467 | 0.042 | 97.5 | |
| 468 | 0.023 | 98.5 | |
| 469 | 0.020 | 100.0 | |
| 470 | 0.104 | 98.5 | |
| 471 | 0.010 | 89.0 | |
| 472 | 0.029 | 90.0 | |

| Bsp. Nr. | Rezeptor-aktivität $EC_{50}$ [µmol/L] | Effizienz [%] | Koopera-tivität (alpha-Faktor) |
|---|---|---|---|
| 473 | 0.031 | 100.0 | |
| 474 | 0.005 | 100.0 | 44 |
| 475 | 0.002 | 100.0 | 68 |
| 476 | 0.018 | 98.0 | |
| 477 | 0.073 | 89.0 | |
| 478 | 0.024 | 100.0 | |
| 479 | 0.038 | 100.0 | |
| 480 | 0.046 | 100.0 | |
| 481 | 0.010 | 100.0 | |
| 482 | 0.013 | 98.5 | |
| 483 | 0.054 | 92.5 | |
| 484 | 0.006 | 99.5 | 44 |
| 485 | 0.006 | 100.0 | |
| 486 | 0.004 | 100.0 | |
| 487 | 0.012 | 96.5 | |
| 488 | 0.024 | 96.0 | 53 |
| 489 | 0.027 | 100.0 | 23 |
| 490 | 0.103 | 91.5 | |
| 491 | 0.106 | 85.5 | |
| 492 | 0.044 | 97.5 | |
| 493 | 0.016 | 94.5 | 14 |
| 494 | 0.028 | 78.0 | 20 |
| 495 | 0.031 | 99.5 | |
| 496 | 0.067 | 89.0 | |
| 497 | 0.029 | 92.0 | |
| 498 | 0.060 | 83.5 | |
| 499 | 0.063 | 96.0 | |

| Bsp. Nr. | Rezeptor-aktivität $EC_{50}$ [µmol/L] | Effizienz [%] | Koopera-tivität (alpha-Faktor) |
|---|---|---|---|
| 500 | 0.076 | 100.0 | |
| 501 | 0.058 | 98.0 | |
| 502 | 0.030 | 91.0 | |
| 503 | 0.028 | 88.5 | 24 |
| 504 | 0.004 | 100.0 | 47 |
| 505 | 0.006 | 100.0 | |
| 506 | 0.007 | 100.0 | 57 |
| 507 | 0.006 | 98.0 | 37 |
| 508 | 0.007 | 93.5 | |
| 509 | 0.007 | 88.0 | |
| 510 | 0.006 | 94.0 | 46 |
| 511 | 0.009 | 100.0 | 30 |
| 512 | 0.010 | 100.0 | |
| 513 | 0.010 | 91.0 | |
| 514 | 0.010 | 99.0 | 30 |
| 515 | 0.012 | 99.3 | 37 |
| 516 | 0.020 | 100.0 | 35 |
| 517 | 0.085 | 88.5 | |
| 518 | 0.470 | 99.5 | |
| 519 | 0.003 | 96.7 | |
| 520 | 0.002 | 100.0 | 36 |
| 521 | 0.002 | 100.0 | 72 |
| 522 | 0.004 | 92.8 | |
| 523 | 0.001 | 100.0 | 41 |
| 524 | 0.002 | 100.0 | 60 |
| 525 | 0.005 | 100.0 | |
| 526 | 0.009 | 100.0 | |

| Bsp. Nr. | Rezeptor-aktivität $EC_{50}$ [µmol/L] | Effizienz [%] | Koopera-tivität (alpha-Faktor) |
|---|---|---|---|
| 527 | 0.005 | 100.0 | 61 |
| 528 | 0.006 | 100.0 | 66 |
| 529 | 0.006 | 96.0 | |
| 530 | 0.006 | 98.7 | 44 |
| 531 | 0.009 | 97.0 | |
| 532 | 0.018 | 92.0 | |
| 533 | 0.024 | 100.0 | |
| 534 | 0.006 | 100.0 | |
| 535 | 0.007 | 100.0 | 27 |
| 536 | 0.016 | 99.5 | |
| 537 | 0.010 | 92.0 | 31 |
| 538 | 0.007 | 100.0 | 40 |
| 539 | 0.007 | 86.5 | 21 |
| 540 | 0.009 | 90.0 | |
| 541 | 0.007 | 92.2 | 31 |
| 542 | 0.019 | 98.5 | 36 |
| 543 | 0.008 | 100.0 | 28 |
| 544 | 0.008 | 98.0 | |
| 545 | 0.008 | 99.0 | |
| 546 | 0.010 | 100.0 | |
| 547 | 0.120 | 90.0 | |
| 548 | 0.009 | 100.0 | 37 |
| 549 | 0.009 | 100.0 | |
| 550 | 0.009 | 100.0 | 34 |
| 551 | 0.018 | 100.0 | |
| 552 | 0.014 | 96.0 | |
| 553 | 0.013 | 97.5 | 49 |

| Bsp. Nr. | Rezeptor-aktivität $EC_{50}$ [µmol/L] | Effizienz [%] | Koopera-tivität (alpha-Faktor) |
|---|---|---|---|
| 554 | 0.010 | 94.5 | 54 |
| 555 | 0.010 | 100.0 | |
| 556 | 0.010 | 100.0 | |
| 557 | 0.011 | 100.0 | 44 |
| 558 | 0.022 | 100.0 | |
| 559 | 0.010 | 99.0 | |
| 560 | 0.017 | 100.0 | |
| 561 | 0.004 | 95.0 | 62 |
| 562 | 0.010 | 100.0 | 40 |
| 563 | 0.020 | 100.0 | |
| 564 | 0.042 | 89.0 | |
| 565 | 0.010 | 99.5 | |
| 566 | 0.016 | 96.5 | |
| 567 | 0.140 | 80.0 | |
| 568 | 0.011 | 100.0 | |
| 569 | 0.049 | 100.0 | |
| 570 | 0.056 | 100.0 | |
| 571 | 0.012 | 94.0 | 33 |
| 572 | 0.012 | 100.0 | |
| 573 | 0.012 | 100.0 | 57 |
| 574 | 0.012 | 100.0 | 36 |
| 575 | 0.013 | 96.0 | |
| 576 | 0.013 | 99.5 | |
| 577 | 0.020 | 100.0 | |
| 578 | 0.017 | 100.0 | 29 |
| 579 | 0.014 | 97.5 | |
| 580 | 0.014 | 100.0 | |

| Bsp. Nr. | Rezeptor-aktivität $EC_{50}$ [µmol/L] | Effizienz [%] | Koopera-tivität (alpha-Faktor) |
|---|---|---|---|
| 581 | 0.014 | 98.0 | |
| 582 | 0.014 | 100.0 | |
| 583 | 0.019 | 100.0 | |
| 584 | 0.016 | 98.0 | |
| 585 | 0.014 | 95.5 | |
| 586 | 0.015 | 100.0 | |
| 587 | 0.014 | 93.5 | |
| 588 | 0.009 | 100.0 | |
| 589 | 0.015 | 100.0 | 34 |
| 590 | 0.015 | 100.0 | 43 |
| 591 | 0.016 | 100.0 | |
| 592 | 0.016 | 99.5 | |
| 593 | 0.017 | 89.5 | 29 |
| 594 | 0.018 | 100.0 | |
| 595 | 0.019 | 94.0 | 36 |
| 596 | 0.021 | 98.5 | 19 |
| 597 | 0.021 | 95.5 | |
| 598 | 0.021 | 100.0 | |
| 599 | 0.023 | 90.0 | |
| 600 | 0.028 | 96.9 | |
| 601 | 0.073 | 100.0 | |
| 602 | 0.023 | 91.5 | |
| 603 | 0.025 | 89.5 | |
| 604 | 0.025 | 93.5 | |
| 605 | 0.025 | 98.0 | |
| 606 | 0.027 | 98.0 | |
| 607 | 0.005 | 100.0 | 32 |

| Bsp. Nr. | Rezeptor-aktivität $EC_{50}$ [µmol/L] | Effizienz [%] | Kooperativität (alpha-Faktor) |
|---|---|---|---|
| 608 | 0.022 | 100.0 | |
| 609 | 0.028 | 99.0 | |
| 610 | 0.011 | 100.0 | |
| 611 | 0.017 | 90.0 | |
| 612 | 0.029 | 100.0 | |
| 613 | 0.033 | 95.5 | |
| 614 | 0.036 | 94.5 | |
| 615 | 0.053 | 99.3 | |
| 616 | 0.037 | 90.5 | |
| 617 | 0.096 | 98.0 | |
| 618 | 0.039 | 99.0 | 30 |
| 619 | 0.040 | 88.6 | |
| 620 | 0.047 | 100.0 | |
| 621 | 0.010 | 100.0 | 44 |
| 622 | 0.043 | 93.0 | |
| 623 | 0.067 | 97.0 | |
| 624 | 0.140 | 96.5 | |
| 625 | 0.043 | 100.0 | |
| 626 | 0.044 | 93.5 | |
| 627 | 0.140 | 83.5 | |
| 628 | 0.032 | 89.5 | |
| 629 | 0.044 | 100.0 | |
| 630 | 0.048 | 100.0 | |
| 631 | 0.049 | 86.0 | 23 |
| 632 | 0.049 | 100.0 | |
| 633 | 0.052 | 93.8 | |
| 634 | 0.054 | 100.0 | 48 |

| Bsp. Nr. | Rezeptor-aktivität $EC_{50}$ [µmol/L] | Effizienz [%] | Kooperativität (alpha-Faktor) |
|---|---|---|---|
| 635 | 0.059 | 100.0 | |
| 636 | 0.060 | 98.1 | |
| 637 | 0.060 | 93.0 | |
| 638 | 0.063 | 100.0 | |
| 639 | 0.068 | 96.5 | 25 |
| 640 | 0.069 | 91.3 | |
| 641 | 0.071 | 100.0 | |
| 642 | 0.072 | 100.0 | |
| 643 | 0.073 | 92.0 | |
| 644 | 0.074 | 85.0 | |
| 645 | 0.074 | 83.0 | |
| 646 | 0.074 | 100.0 | 30 |
| 647 | 0.077 | 92.0 | |
| 648 | 0.081 | 96.5 | 49 |
| 649 | 0.083 | 94.0 | |
| 650 | 0.083 | 100.0 | |
| 651 | 0.086 | 95.0 | |
| 652 | 0.089 | 80.0 | |
| 653 | 0.089 | 86.5 | |
| 654 | 0.093 | 84.0 | |
| 655 | 0.097 | 98.0 | |
| 656 | 0.100 | 91.5 | |
| 657 | 0.100 | 85.3 | |
| 658 | 0.006 | 100.0 | |
| 659 | 0.081 | 98.0 | |
| 660 | 0.102 | 84.5 | |
| 661 | 0.048 | 95.5 | |

| Bsp. Nr. | Rezeptor-aktivität $EC_{50}$ [µmol/L] | Effizienz [%] | Kooperativität (alpha-Faktor) |
|---|---|---|---|
| 662 | 0.038 | 100.0 | |
| 663 | 0.030 | 90.5 | |
| 664 | 0.100 | 98.0 | |
| 665 | 0.073 | 92.5 | |
| 666 | 0.022 | 100.0 | 60 |
| 667 | 3.850 | 100.0 | |

| Bsp. Nr. | Rezeptor-aktivität $EC_{50}$ [µmol/L] | Effizienz [%] | Kooperativität (alpha-Faktor) |
|---|---|---|---|
| 668 | 0.089 | 100.0 | |
| 669 | 0.106 | 84.9 | |
| 670 | 1.450 | 66.5 | |
| 671 | 0.100 | 94.0 | |

**B-2. Funktioneller Ca2+-Freisetzungstest mittels M2-Gα16-Reporterzellen**

Eine potentiell agonistische wie auch die positiv allosterische Wirkung der Testsubstanzen auf den M2-Rezeptor kann anhand eines funktionellen $Ca^{2+}$-Freisetzungstests bestimmt werden. Die Aktivierung des M2-Rezeptors durch Bindung orthosterischer Agonisten (Acetylcholin) oder anderer Substanzen mit einem agonistischen Effekt führt zu einer Konformationsänderung des Rezeptors, welche im endogenen Zustand eine Gai-Protein-Aktivierung zur Folge hat. Durch Kopplung des M2-Rezeptors an das exogen exprimierte promiskuitive Gaq-Protein Gα16 kommt es jedoch zu einer Gα16-Proteinaktivierung nach Aktivierung des M2-Rezeptors, welche - über eine nachgeschaltete Signaltransduktionskaskade - eine intrazelluläre $Ca^{2+}$-Freisetzung bedingt. Das Ausmaß der intrazellulären $Ca^{2+}$-Mobilisierung kann daher in diesem Fall als Maß für die Aktivierung des M2-Rezeptors angesehen werden.

Als Testzelle dient eine rekombinante CHO-Zelllinie, welche mit cDNA kodierend für den humanen M2-Rezeptor und das Gal6-Protein sowie mit cDNA kodierend für das mitochondrial exprimierte Photoprotein Clytin (mtClytin) stabil transfiziert wurde (CHO mtClytin Gα16 M2). Die Bestimmung der intrazellulären $Ca^{2+}$-Freisetzung in Abhängigkeit von Substanzzugabe bzw. M2-Aktivierung erfolgt mittels eines $Ca^{2+}$-sensitiven Farbstoffes (Fluo-8) und der Messung der Zellfluoreszenz unter Verwendung eines $FLIPR^{TETRA}$-Instrumentes (Molecular Devices).

**B-2.1. Agonismus-Assay**

Die Testsubstanzen werden in DMSO mit einer Konzentration von 10 mM gelöst und für eine 10 Punkt-Dosis-Wirkungsanalyse in Schritten von 1:3.16 seriell in DMSO verdünnt. Entsprechend der gewünschten Testkonzentrationen werden die Substanzen in Fluo-8-Puffer (Zusammensetzung pro 100 ml: 500 µl Probenecid, 2 ml Brilliant Black (20 mg/ml), 440 µl Fluo-8, 2 mM $CaCl_2$ ad 100 ml CAFTY-Tyrode (130 mM NaCl, 5 mM KCl, 20 mM HEPES, 1 mM $MgCl_2$, 5 mM $NaHCO_3$, pH 7.4)) vorverdünnt.

Die in MEM alpha-Medium (supplementiert mit 10% FCS, 2% Glutamax) kultivierten Reporter-Zellen wurden mit 3000 Zellen in 30 µl-Aussaatmedium pro 384 Well in µCLEAR/schwarz Greiner-Zellkulturplatten (#781092) ausgesät und 24 h bei 37°C inkubiert. Das Aussaatmedium besteht aus MEM alpha-Medium (supplementiert mit 5% FCS, 2% Glutamax). Für die jeweilige Messung wird das Medium entfernt und die Zellen nach Zugabe von je 20 µl Fluo-8-Puffer pro 384 Well für 1h 37°C im Brutschrank inkubiert. Nach Zugabe von je 10 µl pro 384 Well der vorverdünnten Testsubstanzen erfolgt die Messung der Zellfluoreszenz für einen Zeitraum von 5 min in 1 sec-Inkrementen. Das relative Maß der maximalen Aktivierung des M2-Rezeptors durch die jeweiligen Testsubstanzen wird durch Normierung des Testsignals auf das der $E_{Max}$-Konzentration von Acetylcholin (3µM) entsprechende Signal berechnet.

**B-2.2. Bestimmung des positiv allosterischen Modulator-Effektes**

Um die positive Kooperativität der Testsubstanzen in Bezug auf die Acetylcholin-vermittelte M2-Rezeptoraktivierung bestimmen zu können, wird anschließend Referenzagonist (Acetylcholin) für eine vollständige Dosis-Wirkungsanalyse hinzugefügt. Hierzu wird Acetylcholin beginnend mit einer maximalen finalen Konzentration von 1 µM in Schritten

von 1:3.16 seriell in Fluo-8-Puffer verdünnt. Nach Zugabe von je 10 µl Agonistenlösung pro 384 Well erfolgt wiederum die Messung der Zellfluoreszenz für einen Zeitraum von 5 min in 1 sec-Inkrementen. Dabei wird dieselbe Assay-Platte verwendet wie unmittelbar zuvor für den M2-Agonismus-Assay. Die Verschiebung der Acetylcholin-Dosis-Wirkungskurve in Anwesenheit zunehmender Konzentrationen der Testsubstanz wird mittels GraphPad PRISM (Allosteric $EC_{50}$ shift) analysiert und quantifiziert (s.o.).

## B-3. Selektivitätstest gegenüber humanen muskarinischen Acetylcholin-Rezeptoren

Eine potentiell agonistische Wirkung wie auch positiv allosterische Wirkung der Testsubstanzen auf anderen humanen muskarinischen Acetylcholin-Rezeptoren kann in einem in funktionellen $Ca^{2+}$-Freisetzungstests bestimmt werden (Eurofins; GPCRProfiler® Services in agonistic and allosteric mode for Mx Receptors; cat#: HTS600GPCR).
Als Testzellen dienen mit dem jeweiligen Rezeptor transfizierten Chem-1- oder Chem-4-Zelllinien (ChemiScreen™ M1 Calcium-Optimized FLIPR Cell Lines, Eurofins; M1: HTS044C; ChemiScreen™ Calcium-Optimized Stable Cell Line Human Recombinant M2 Muscarininc Acetylcholine Receptor, Eurofins; M2: HTS115C; ChemiScreen™ Human Recombinant M3 Muscarinic Acetylcholine Receptor Calcium-Optimized Stable Cell Line, Eurofins; M3: HTS116C; ChemiScreen™ Human Recombinant M4 Muscarinic Acetylcholine Receptor Calcium-Optimized Stable Cell Line, Eurofins; M4: HTS117C; ChemiScreen™ M5 Calcium-Optimized FLIPR Cell Lines, Eurofins; M5: HTS075C). Der Substanztest wird mit einem $FLIPR^{TETRA}$-Instrument (Molecular Devices) durchgeführt.

### B-3.1. Agonismus-Assay

Um einen potentiellen agonistischen Effekt der Testsubstanzen zu ermitteln, werden die jeweiligen Testsubstanzen mit einer finalen Testkonzentration von 10 µM oder 1 µM zugefügt. Die $Ca^{2+}$-Freisetzung bzw. Zellfluoreszenz wird über einen Zeitraum von 180 sec gemessen. Als Positivkontrolle zur Normierung des Substanzeffektes auf die Rezeptoraktivierung wird eine dem $E_{Max}$-Wert entsprechende Konzentration von Acetylcholin eingesetzt.
Nach Beendigung des Agonismus-Assay wird die Assay-Platte bei 25°C für 7 min inkubiert. Nach der Inkubationsperiode wird der positiv allosterische Modulator-Assay initialisiert.

### B-3.2. Allosterischer Modulator-Assay

Um eine positiv oder negativ allosterische Wirkung der Testsubstanzen auf andere humane muskarinische Acetylcholinrezeptoren sowie den M2-Rezeptor selbst zu untersuchen, wird jede Substanzkonzentration mit einer Acetylcholin-8 Punkt-Dosis-Wirkungskurve kombiniert. Nach Zugabe von Agonistenlösung erfolgt wiederum die Messung der Zellfluoreszenz für einen Zeitraum von 180 sec. Die Verschiebung der Acetylcholin-Dosis-Wirkungskurve (maximale Verschiebung des $EC_{50}$-Wertes von Acetylcholin) wird mittels GraphPad PRISM (Sigmoidal dose-response (variable slope) - $EC_{50}$) analysiert und quantifiziert. Abschließend werden Quotienten der allosterischen Verschiebung für den M2-Rezeptor und M4-Rezeptor gebildet, die ihrerseits als Maß für die jeweilige Selektivität fungieren.
In den folgenden Tabellen 2 und 3 sind für individuelle Ausführungsbeispiele die so ermittelten Quotienten anhand ausgewählter Moleküle aus diesem Assay aufgeführt:

**Tabelle 2**

| Bsp.-Nr | $EC_{50}$ AChM2 [nM] | $EC_{50}$ AChM4 [nM] | Konzentration [µM] | Shift $EC_{50}$ M2 | Shift $EC_{50}$ M4 | Selektivität (Shift $EC_{50}$ M2 / Shift $EC_{50}$ M4) |
|---|---|---|---|---|---|---|
| Ref. (LY2119620) | 440 | 100 | 10 | 46 | 56 | 0.8 |
| Ref. (LY2119620) | 440 | 100 | 1 | 24 | 83 | 0.3 |
| 175 | 440 | 100 | 10 | 76 | 0.9 | 83 |
| 175 | 440 | 100 | 1 | 21 | 0.7 | 29 |
| 323 | 323 | 110 | 10 | 40 | 0.9 | 44 |
| 323 | 350 | 110 | 1 | 48 | 0.9 | 52 |
| 590 | 350 | 110 | 10 | 39 | 1.2 | 33 |

(fortgesetzt)

| Bsp.-Nr | $EC_{50}$ AChM2 [nM] | $EC_{50}$ AChM4 [nM] | Konzentration [µM] | Shift $EC_{50}$ M2 | Shift $EC_{50}$ M4 | Selektivität (Shift $EC_{50}$ M2 / Shift $EC_{50}$ M4) |
|---|---|---|---|---|---|---|
| 590 | 350 | 110 | 1 | 7 | 1.1 | 7 |

Insbesondere lässt Tabelle 2 erkennen, dass die Selektivität des zuvor besprochenen Moleküls LY2119620 klar zugunsten M4 tendiert, während die Selektivität der erfindungsgemäßen Moleküle ausgewogen ist oder auf der Seite von M2 liegt.

**Tabelle 3**

| Bsp.-Nr | $EC_{50}$ AChM2 [nM] | $EC_{50}$ AChM4 [nM] | Konzentration [µM] | Shift $EC_{50}$ M2 | Shift $EC_{50}$ M1 | Selektivität (Shift $EC_{50}$ M2 / Shift $EC_{50}$ M1) |
|---|---|---|---|---|---|---|
| Ref. (LY2119620) | 18 | 100 | 10 | 46 | 11 | 4 |
| 175 | 18 | 100 | 10 | 76 | 0.3 | 287 |
| 323 | 18 | 100 | 10 | 40 | 1.3 | 31 |
| 590 | 18 | 100 | 10 | 39 | 1.3 | 30 |

Tabelle 3 lässt erkennen, dass die erfindungsgemäßen Moleküle eine sehr viel größere Selektivität zu M2 im Vergleich zu M1 haben als das zuvor besprochene Molekül LY2119620.

## B-4. In vitro M2 PAM Gi-Assay

Für die Charakterisierung von Testsubstanzen auf positiv allosterische Modulation des humanen M2 Rezeptors wird die Carbachol induzierte Hemmung des cAMP Anstieg durch Forskolin in rekombinant M2 Rezeptor-exprimierenden CHO Zellen gemessen, die zusätzlich ein Luciferase Gen unter der Kontrolle eine cAMP responsiven Elementes (CRE) exprimieren: 3000 Zellen in 25 µl Vollmedium (DMEM F12 PAN Medium, 10 % FCS, 1.35 mM Na-Pyruvat, 20 mM Hepes, 4mM Glutamax, 2 % Natrium Bicarbonat, 1 % Pen/Strep, 1 % 100x non-essential amino acids) werden je Loch einer 384 Multititerplatte (Greiner, TC Platte, schwarz mit klarem Boden) ausgesät und bei 37°C, 5 % $CO_2$ für 24 Stunden inkubiert. Vor der Messung wird das Medium durch 30 µl Messmedium (Optimem) ersetzt und bei 37°C, 5 % $CO_2$ für 10 Minuten inkubiert. Die Testsubstanz wird in DMSO in verschiedenen Konzentrationen (Startkonzentration 10 mM, Verdünnungsfaktor 3.16) als Dosiswirkungskurve vorbereitet und 1:50 mit calciumfreier Tyrode, 2 mM $CaCl_2$, 0.01% BSA vorverdünnt. 10 µl der vorverdünnten Substanzlösung werden zu den Zellen gegeben und bei 37°C, 5 % $CO_2$ für 10 Minuten inkubiert. Der M2 Rezeptor wird durch Zugabe von 10 µl Carbachol in verschiedenen Konzentrationen in calciumfreier Tyrode, 2 mM $CaCl_2$ aktiviert und bei 37°C, 5 % $CO_2$ für 5 Minuten inkubiert. Die Adenylylcyclase wird durch Zugabe von 10 µl 1 µM (finale Konzentration) Forskolin in calciumfreier Tyrode, 2 mM $CaCl_2$ aktiviert und bei 37°C, 5 % $CO_2$ für 5 Stunden inkubiert. Nach Entfernen des Zellüberstandes und Zugabe von 20 µl Luci/Triton Puffer (1:1) wurde die Lumineszenz in einem Luminometer für 60 Sekunden bestimmt.

Calciumfreie Tyrode: 130 mM NaCl, 5 mM KCl, 20 mM HEPES, 1 mM $MgCl_2$, 4.8 mM $NaHCO_3$, pH 7.4 Luci/Triton Puffer (1:1): Luci-Puffer (20mM Tricine, pH 7.8, 2,67 mM Magnesiumsulfat, 0.1 mM EDTA, 4 mM DTT, 270 µM Coenzym A, 470 µM D-Luciferin, 530 µM ATP) 1:1 mit Triton-Puffer (25 mM Tris wässr. Salzsäure, pH 7.8, 25 mM $Na_2HPO_4$, 2mM Dithiothreitol, 3 % Triton X-100, 10 % Glycerin) gemischt.

Der $EC_{50}$-Wert wurde mit Hilfe einer 4-Parameter-Logistik-Funktion (Hill-Funktion) ermittelt.

## B-5. Kompetitiver Bindungstest für humane M2- und M4-Rezeptoren

Die direkte Bindung der Testsubstanzen an den M2-Rezeptor sowie die Verstärkung der Bindung (Erhöhung der Affinität) des natürlichen Agonisten Acetylcholin an den M2-Rezeptor in Anwesenheit der Testsubstanzen (positiv allosterischer Effekt) wird mittels eines FRET-basierten Bindungsassays (HTRF Tag-lite® Bindungsassay, Cisbio) bestimmt. Zur Kontrolle der Selektivität wird die Bindung der Testsubstanzen an den strukturverwandten M4-Rezeptor analog untersucht. Der HTRF Tag-lite® Assay ist ein homogener Bindungsassay und beruht auf der kompetitiven Bindung

eines fluoreszenten Liganden (Sonde) und der unmarkierten Testsubstanz an den Rezeptor, welcher auf lebenden Zellen exprimiert ist. Der Rezeptor ist seinerseits mit einem fluoreszenten Donor-Farbstoff (Terbium-Kryptat) derivatisiert, so dass nach Anregung des Donor-Farbstoffes ein FRET-Signal zwischen Rezeptor und Sonde (Akzeptor) entsteht, wenn die Sonde an den Rezeptor gebunden ist. Als Akzeptor-Sonde wurde ein Telenzepin-Derivat eingesetzt, welches mit einem HTRF Fluoreszenz-Farbstoff konjugiert ist (red ligand; L0040RED). Die Sonde bindet daher in der konservierten orthosterischen Bindungsstelle sowohl des M2- als auch des M4-Rezeptors. Die allosterische Bindungsstelle des M2-Rezeptors wurde röntgenkristallographisch charakterisiert und wird direkt oberhalb der orthosterischen Bindungstasche postuliert (Kruse et al., Nature, 2013, 504, 101-106). Sowohl die Bindung von unmarkierten orthosterischen Agonisten (Acetylcholin) an die orthosterische Bindungsstelle als auch die Bindung von allosterischen Modulatoren (Testsubstanzen) an die allosterische Bindungsstelle führt daher zu einer konzentrationsabhängigen kompetitiven Verdrängung der Sonde und damit einer Abnahme des FRET-basierten Fluoreszenzsignals.

Alle Bindungstests werden auf weißen 384 Mikrotiterplatten (small-volume) in einem Gesamtvolumen von 20 μl durchgeführt. Die HTRF-Messungen werden mit einem PHERAstar-Instrument (BMG Labtech) vorgenommen. Für den muskarinischen M2- oder M4-Rezeptor-Bindungstest werden SNAPed-M2-exprimierende Zellen (C1TT1M2) oder SNAPed-M4-exprimierende Zellen (C1TT1M4) verwendet, welche mit einem Donor-Fluorophor (Lumi4Tb; CELLCUST) markiert wurden. Die Zellen werden mit der Akzeptor-Sonde in Tag-lite Bindungspuffer (LABMED) in Anwesenheit von Testsubstanz bzw. Acetylcholin inkubiert. Anschließend wird das Fluoreszenz-Signal bei Wellenlängen von 665 nm und 620 nm gemessen und der HTRF-Quotient (Signal bei 665 nm / Signal bei 620 nm) bestimmt. Das relative spezifische Signal wird durch Subtraktion des HTRF-Quotienten der Negativkontrolle (nur Tag-lite Puffer ohne Sonde) ermittelt.

### B-5.1. Bindung der Testsubstanzen

Um die Bindung der Testsubstanzen an den M2- oder M4-Rezeptor in Abwesenheit von orthosterischem Agonisten zu bestimmen, wird eine Dosis-Wirkungsanalyse der Testsubstanzen im kompetitiven Format des M2-Tag-lite®- oder M4-Tag-lite®-Bindungsassays vorgenommen. Die Testsubstanzen werden in DMSO mit einer Konzentration von 10 mM gelöst und für eine Dosis-Wirkungsanalyse in Schritten von 1:3.16 seriell in DMSO verdünnt. Die maximale Testkonzentration entspricht 10 μM. Die molare Konzentration der Testsubstanz, die eine halbmaximale Reduktion des HTRF-Signals in Bezug auf das maximale und verbleibende HTRF-Signal bei höchster Substanzkonzentration bewirkte ($EC_{50}$-Wert der Bindung), wird mittels GraphPad PRISM (Sigmoidal dose response) ermittelt. Zugleich wird die Stärke des Kompetitionseffektes bestimmt, indem die maximale Abnahme des spezifischen HTRF-Signals bei höchster Substanzkonzentration berechnet wird (% max. Kompetition).

### B-5.2. Bindung der Testsubstanzen im allosterischen Modus

Um die allosterische Modulation des M2-Rezeptors durch die Testverbindungen zu untersuchen, wird zum einen eine Dosis-Wirkungsanalyse der Testsubstanzen im kompetitiven Format des M2-Tag-lite®-oder M4-Tag-lite®-Bindungsassays in Anwesenheit einer dem $EC_{20}$-Wert entsprechenden Konzentration von Acetylcholin vorgenommen, welche in einer separaten 11 Punkt-Acetylcholin-Dosis-Wirkungsanalyse bestimmt wird (3 μM). Die Testsubstanzen werden in DMSO mit einer Konzentration von 10 mM gelöst und für eine 10 Punkt-Dosis-Wirkungsanalyse in Schritten von 1:3.16 seriell in DMSO verdünnt. Die maximale Testkonzentration entspricht 10 μM. Die molare Konzentration der Testsubstanz, die eine halbmaximale Reduktion des HTRF-Signals in Bezug auf das maximale und verbleibende HTRF-Signal bei höchster Substanzkonzentration in Anwesenheit einer dem EC20-Wert entsprechenden Acetylcholin-Konzentration bewirkt ($EC_{50}$-Wert der Bindung), wird mittels GraphPad PRISM (Sigmoidal dose response) ermittelt. Zugleich wird die Stärke des Kompetitionseffektes bestimmt, indem die maximale Abnahme des spezifischen HTRF-Signals bei höchster Substanzkonzentration berechnet wird (% max. Kompetition).

Um die Verstärkung der Bindung von Acetylcholin an den M2- oder M4-Rezeptor zu untersuchen, wurde zusätzlich zum anderen eine 11 Punkt-Dosis-Wirkungsanalyse von Acetylcholin im kompetitiven Format des M2-Tag-lite®- oder M4-Tag-lite®-Bindungsassays in Abwesenheit oder in Anwesenheit von 1 μM oder 10 μM Testsubstanzvorgenommen. Die Verschiebung der Acetylcholin-Dosis-Wirkungskurve (maximale Verschiebung des EC50-Wertes von Acetylcholin) wurde mittels GraphPad PRISM (Sigmoidal dose-response) analysiert und quantifiziert.

### B-6. Radioligand-Bindungstests für humane M2-Rezeptoren

Der allosterische Wirkmechanismus der Testsubstanzen kann mittels verschiedener Radioligand-Bindungstests näher untersucht und quantifiziert werden. Die Bindung des Allosters an die allosterische Bindungsstelle des M2-Rezeptors hat im Falle einer positiven Kooperativität eine Erhöhung der Bindungaffinität des orthosterischen Liganden für den M2-Rezeptor zur Folge. Die Erhöhung der Bindungsaffinität des orthosterischen Liganden durch den Alloster im ternären Komplex bestehend aus Orthoster, Alloster und M2-Rezeptor wiederum ist auf eine Modulation der Bindungskinetiken des Orthosters zurückzuführen. Dabei kann der Alloster die Assoziations- und/oder Dissoziationsgeschwindigkeit des Orthosters am M2-Rezeptor verändern. Eine Erniedrigung der Dissoziationsgeschwindigkeit spiegelt dabei eine Stabilisierung des ternären Komplexes wieder und geht daher auch mit einer Erniedrigung der Dissoziationskonstante des orthosterischen Liganden unter Gleichgewichtsbedingungen einher (Lazareno, Determination of Allosteric Interactions Using Radioligand-Binding Techniques in Methods in Molecular Biology, vol. 259, Receptor Signal Transduction Protocols, 2nd ed.; Kostenis and Mohr, Trends Pharmacol. Sci. 1996, 17(8), 280-283).

**B-6.1. Radioligand-Bindungstest unter Gleichgewichtsbedingungen**

Um den Einfluss der Testsubstanzen auf die Bindungsaffinität von orthosterischen Agonisten für den M2-Rezeptor zu überprüfen und zu quantifizieren, kann ein Radioligand-Bindungstest unter Gleichgewichtsbedingungen durchgeführt werden. Dabei wird die Bindung des radioaktiv markierten M2-Rezeptor-Agonisten $^3$H-Oxotremorin-M an den M2-Rezeptor für unterschiedliche $^3$H-Oxotremorin-M-Konzentrationen im Bindungsgleichgewicht gemessen (Croy et al., Mol. Pharmacol. 2014, 86, 106-115). Basierend auf der spezifisch gebundenen Menge von radioaktivem Agonisten an den M2-Rezeptor in Abhängigkeit von der Agonisten-Konzentration (graphisch dargestellt als sog. Langmuir-Isotherme) kann dann zum einen die Gleichgewichtsdissoziationskonstante $K_d$ des Agonisten als quantitatives Maß seiner Bindungsaffinität für den M2-Rezeptor sowie zum anderen die Konzentration bzw. Anzahl spezifischer Bindungstellen des Radioliganden (Agonisten) $B_{max}$ in Abwesenheit oder Anwesenheit unterschiedlicher Konzentrationen der Testsubstanzen (positiven allosterischen Modulatoren) berechnet werden (Hulme and Trevethick, Brit. J. Pharmacol. 2010, 161, 1219-1237).

Der Radioligandenbindungsassay für den M2 Rezeptor (Euroscreen, FAST-0261B) wird mittels $^3$H-markiertem Oxotremorin-M (NET671) als Agonisten durchgeführt. Die Agonistenbindung an den M2-Rezeptor wird auf 96 Mikrotiterplatten (Master Block, Greiner, 786201) in Bindungspuffer (Natrium-/Kalium-Phosphat-Pufer, pH 7,4) in Triplikaten vorgenommen. Hierzu werden pro Ansatz M2-Membranextrakte (20 µg Protein / 96 Well) mit verschiedenen Konzentrationen von radioaktiv markiertem Agonisten (0,2 - 100 nM) allein oder in Anwesenheit von 1 µM oder 10 µM Testsubstanz oder Bindungspuffer allein in einem Gesamtvolumen von 0,1 mL für 60 min bei 37°C inkubiert. Die nichtspezifische Bindung von $^3$H-markiertem Oxotremorin-M an die Membran wird durch Co-Inkubation mit N-Me-Scopolamin (NMS), einem orthosterischen Antagonisten des M2-Rezeptors, in 200-fachem Überschuss bestimmt. Anschließend werden die Proben zum Stoppen der Bindungsreaktion über GF/C Filter (Perkin Elmer, 6005174), welche zuvor mit 0,5 %-iger Polyethylenimin- (PEI-) Lösung benetzt wurden, für 2 h bei Raumtemperatur filtriert. Die Filter werden sechsmal mit je 0,5 mL eiskaltem Waschpuffer (10 mM Natrium-/Kalium-Phosphat-Puffer, pH 7.4) gewaschen und anschließend je 50 µL Microscint 20 Szintillationslösung (Packard) pro Ansatz zugegeben. Die Proben werden dann für 15 min auf einem Orbitalschüttler inkubiert, bevor die Messung der Radioaktivität mittels eines Top-Count™-Gerätes (1 min / Well) vorgenommen wird.

Die Testsubstanzen werden in DMSO mit einer Konzentration von 10 mM gelöst und entsprechend der finalen Testkonzentration weiter in DMSO verdünnt, um eine 100-fache Verdünnung der eingesetzten DMSO-Lösung in Bindungspuffer zu erhalten.

Der $K_d$-Wert sowie der $B_{max}$-Wert von $^3$H-Oxotremorin-M für den M2-Rezeptor werden mit Hilfe eines "one-site" spezifischen Bindungsmodells bestimmt (Croy et al., Mol. Pharmacol. 2014, 86, 106-115).

**B-6.2. Kompetitiver Radioligand-Bindungstest unter Gleichgewichtsbedingungen**

Um den Einfluss der Testsubstanzen auf die Bindungsaffinität von orthosterischen Agonisten für den M2-Rezeptor weitergehend zu überprüfen und zu quantifizieren, wird zusätzlich ein kompetitiver Radioligand-Bindungstest unter Gleichgewichtsbedingungen durchgeführt. Hierbei wird die Bindung des antagonistischen Radioliganden $^3$H-N-Me-Scopolamin ($^3$H-NMS) an den M2-Rezeptor in Abwesenheit oder Anwesenheit unterschiedlicher Konzentrationen des nicht radioaktiv markierten Agonisten Oxotremorin-M bestimmt (Croy et al., Mol. Pharmacol. 2014, 86, 106-115; Schober et al., Mol. Pharmacol. 2014, 86, 116-123). Die radioaktiv markierte Sonde (Antagonist) und der unmarkierte Agonist kompetieren um die Bindung an die orthosterische Bindungsstelle des M2-Rezeptors. Die Fähigkeit zur Verdrängung der radioaktiv markierten Sonde dient daher als Maß für die Bindungsaffinität des Agonisten für den Rezeptor und kann gemäß der Cheng-Prusoff-Gleichung als Gleichgewichtsinhibitor-Konstante ($K_i$-Wert) quantifiziert werden (Cheng and Prusoff, Biochem. Pharmacol. 1973, 22(23), 3099-3108). Um den allosterischen Effekt der Testsubstanzen weitergehend zu untersuchen, wird der Einfluss der Testsubstanzen auf den $K_i$-Wert von Oxotremorin-M bestimmt.

Der Antagonisten-Inhibitionsbindungsassay für den M2-Rezeptor (Euroscreen, FAST-0261B) wird auf 96 Mikrotiterplatten (Master Block, Greiner, 786201) in Bindungspuffer (50 mM Tris-Puffer pH 7.4, 1 mM EDTA, 10 µg/ml Saponin) mit $^3$H-NMS als M2 Rezeptor-Antagonisten durchgeführt. Zur Einstellung des Bindungsgleichewichts werden pro Ansatz M2-Membranextrakte (20 µg Protein / 96 Well) mit einer definierten Konzentration von radioaktiv markiertem Antagonisten (0,5 nM) allein oder in Anwesenheit von unterschiedlichen Konzentrationen von unmarkierten Agonisten

(Oxotremorin-M; 0,001nM bis 1 mM) mit oder ohne 1 μM oder 10 μM Testsubstanz oder Bindungspuffer allein in einem Gesamtvolumen von 0,1 mL für 2 h bei 25°C inkubiert. Die nichtspezifische Bindung von $^3$H-markiertem NMS an die Membran wird durch Co-Inkubation mit nicht radioaktiv markiertem Acetylcholin in 200-fachem Überschuss bestimmt. Anschließend werden die Proben zum Stoppen der Bindungsreaktion über GF/C Filter (Perkin Elmer, 6005174), welche zuvor mit 0,5 %-iger PEI-Lösung benetzt wurden, für 2 h bei Raumtemperatur filtriert. Die Filter werden sechsmal mit je 0,5 mL eiskaltem Waschpuffer (10 mM Natrium-/Kalium-Phosphat-Puffer, pH 7.4) gewaschen und anschließend je 50 μL Microscint 20 Szintillationslösung (Packard) pro Ansatz zugegeben. Die Proben wurden dann für 15 min auf einem Orbitalschüttler inkubiert, bevor die Messung der Radioaktivität mittels eines TopCount™-Gerätes (1 min / Well) vorgenommen wird.

Die Testsubstanzen werden in DMSO mit einer Konzentration von 10 mM gelöst und entsprechend der finalen Testkonzentration weiter in DMSO verdünnt, um eine 100-fache Verdünnung der eingesetzten DMSO-Lösung in Bindungspuffer zu erhalten.

Die $K_i$-Werte in An- oder Abwesenheit von Testsubstanz werden mit Hilfe der Cheng-Prusoff-Gleichung quantifiziert (Cheng and Prusoff, Biochem. Pharmacol. 1973, 22(23), 3099-3108). Dabei werden die $IC_{50}$-Werte der Substanzen gemäß einer vier Parameter enthaltenden logistischen Gleichung ermittelt und der $K_d$-Wert von NMS in einem Radioligand-Bindungstest unter Gleichgewichtsbedingungen ermittelt (Schober et al., Mol. Pharmacol. 2014, 86, 116-123).

**B-6.3. Kinetischer Radioliganden-Bindungstest**

Mithilfe eines kinetischen Radioligand-Bindungstests kann die Kinetik der Dissoziation des radioaktiv markierten Agonisten $^3$H-Oxotremorin-M für den M2-Rezeptor in An- oder Abwesenheit von Testsubstanz untersucht werden. Hierdurch kann der Einfluss der allosterischen Aktivität der Testsubstanzen auf die Dissoziationskonstante ($k_{off}$-Rate) des M2-Agonisten bestimmt und so der Mechanismus der Allosterie der Testsubstanzen weitergehend charakterisiert werden (Lazareno, Determination of Allosteric Interactions Using Radioligand-Binding Techniques in Methods in Molecular Biology, vol. 259, Receptor Signal Transduction Protocols, 2nd ed.; Schrage et al.).

Der Radioliganden Dissoziationsbindungsassay für den M2 Rezeptor (Euroscreen, FAST-0261B) wird mit $^3$H-markiertem Oxotremorin-M (NET671) als Agonisten durchgeführt. Die Bindungsreaktion wird in Bindungspuffer (Natrium-/Kalium-Phosphat-Pufer, pH 7,4) auf 96 Mikrotiterplatten (Master Block, Greiner, 786201) vorgenommen. Hierzu werden pro Ansatz M2-Membranextrakte (20 μg Protein / 96 Well) mit einer definierten Konzentration von radioaktiv markiertem Agonisten (9,65 nM) allein oder in Anwesenheit von 1 μM oder 10 μM Testsubstanz oder Bindungspuffer allein für 60 min bei 37°C vorinkubiert. Anschließend wird NMS in 200-fachem Überschuss zu unterschiedlichen Zeitpunkten hinzugefügt (ein Zeitpunkt pro Ansatz) und die Ansätze in einem Gesamtvolumen von 0,1 mL bei 37°C inkubiert. Anschließend werden die Proben zum Stoppen der Bindungsreaktion über GF/C Filter (Perkin Elmer, 6005174), welche zuvor mit 0,5 %-iger PEI-Lösung benetzt wurden, für 2 h bei Raumtemperatur filtriert. Die Filter werden sechsmal mit je 0,5 mL eiskaltem Waschpuffer (10 mM Natrium-/Kalium-Phosphat-Puffer, pH 7.4) gewaschen und anschließend je 50 μL Microscint 20 Szintillationslösung (Packard) pro Ansatz zugegeben. Die Proben werden dann für 15 min auf einem Orbitalschüttler inkubiert, bevor die Messung der Radioaktivität mittels eines TopCountTM-Gerätes (1 min / Well) vorgenommen wird.

Die Testsubstanzen werden in DMSO mit einer Konzentration von 10 mM gelöst und entsprechend der finalen Testkonzentration weiter in DMSO verdünnt, um eine 100-fache Verdünnung der eingesetzten DMSO-Lösung in Bindungspuffer zu erhalten.

Der $k_{off}$-Wert wurde mit Hilfe eines "one phase" exponentiellen Zerfallsmodells der Dissoziation bestimmt (Hulme and Trevethick, Brit.J. Pharmacol. 2010, 161, 1219-1237; Kostenis and Mohr, Trends Pharmacol. Sci. 1996, 17(8), 280-283).

**B-7. Effekte der Testsubstanzen auf Acetylcholin vermittelte GIRK1/4-Kanalströme in primären atrialen Kardiomyozyten der Ratte**

Die Substanztestung erfolgt gemäß eines in der Literatur beschriebenen Patch-Clamp Protokolls zur elektrophysiologischen Messung Acetylcholin-induzierter GIRK1/4-Membranströme in nativen atrialen Myozyten der Ratte (siehe z.B. Beckmann and Rinne et al., G Protein-Activated (GIRK) Current in Rat Ventricular Myocytes is Masked by Constitutive Inward Rectifier Current (IK1), Cell Physiol Biochem 2008;21:259-268).

Zunächst wird eine Acetylcholin Dosis-Wirkungskurve in Abwesenheit von Testsubstanz (DMSO-Kontrolle) für die GIRK1/4-Aktivität bestimmt, indem Testlösungen mit ansteigender Acetylcholin-Konzentration perfundiert und die resultierenden Membranströme gemessen werden. Die Messung der Membranströme bzw. Änderung der Membranströme für eine gegebene ACh-Konzentration erfolgt dabei für ca. 10 bis 20 Sekunden. Nach Applikation der maximalen ACh-Konzentration innerhalb einer DWK-Reihe erfolgt die Perfusion einer Lösung mit Atropin (10 μM) gefolgt von einem

Auswaschen der Substanzlösungen, um die M2-Selektivität und Reversibilität der M2-Aktivierung sicherzustellen. Die Änderungen der Membranströme werden entsprechend aufgenommen. Dabei wird für jede Acetylcholin-Konzentration der jeweils gemessene Membranstrom auf den maximalen Acetylcholininduzierten Membranstrom normiert (I/IMax). Eine Acetylcholin Dosis-Wirkungskurve umfasst dabei fünf verschiedene Konzentrationen (1nM, 10 nM, 100 nM, 1 μM, 10 μM). Der EC50-Wert wird mit Hilfe einer 4-Parameter-Logistik-Funktion (Hill-Funktion) ermittelt.

Um den allosterischen Effekt der Testsubstanzen auf den M2 Rezeptor zu ermitteln, wird die Acetylcholin Dosis-Wirkungskurve für den GIRK1/4-Membranstrom in Anwesenheit einer konstanten Konzentration der jeweiligen Testsubstanz (z.B. 1 μM) bestimmt. Hierzu wird nach Präinkubation der Zelle mit der Testsubstanz für ca. 20 Sekunden und Messung der Membranströme eine Testlösung, welche dieselbe Substanzkonzentration und eine definierte ACh-Konzentrationen enthielt, für ca. 10 bis 20 Sekunden perfundiert und die Membranströme gemessen. Nach Applikation der maximalen Acetylcholin-Konzentration innerhalb einer Messreihe erfolgt wiederum die Perfusion einer Lösung mit Atropin (10 μM), um die M2-Selektivität des Substanzeffektes zu kontrollieren. Der EC50-Wert in Anwesenheit von Testsubstanz wird analog mit Hilfe einer 4-Parameter-Logistik-Funktion (Hill-Funktion) ermittelt (s.o.).

Die Verschiebung der Acetylcholin Dosis-Wirkungskurve wird anhand der Veränderung des EC50-Wertes für Acetylcholin in Ab- oder Anwesenheit der Testsubstanz ermittelt und quantifiziert.

## B-8. Effekte der Testsubstanzen auf das isolierte perfundierte Rattenherz

Männliche Wistar-Ratten (Stamm: HsdCpb:WU) mit einem Körpergewicht von 200-250g werden mit Narcoren (100 mg/kg) narkotisiert. Nach Eröffnen des Thorax wird das Herz frei präpariert, ausgeschnitten und durch Kanulieren der Aorta an eine Langendorff-Apparatur angeschlossen. Das Herz wird retrograd mit einer Krebs-Henseleit-Pufferlösung (begast mit 95% $O_2$ und 5% $CO_2$, pH 7,4, 35°C) mit folgender Zusammensetzung in mmol/l: NaCl 118; KCl 3; $NaHCO_3$ 22; $KH_2PO_4$ 1,2; Magnesiumsulfat 1,2; $CaCl_2$ 1,8; Glucose 10; Na-Pyruvat 2 mit 9 ml/min flußkonstant perfundiert. Für die Erfassung der Kontraktionskraft des Herzens wird durch eine Öffnung im linken Herzohr an einem PE-Schlauch befestigter und mit Wasser gefüllter Ballon aus dünner Kunststofffolie in den linken Ventrikel eingeführt. Der Ballon wird mit einem Druckaufnehmer verbunden. Der enddiastolische Druck wird auf 5-10 mmHg über das Ballonvolumen eingestellt. Die Daten werden von einem Brückenverstärker verstärkt und von einem Computer mit der LabChart Software (ADInstruments) registriert.

Um die allosterische Wirkung der Testsubstanzen zu untersuchen, werden die Herzen mit Zusatz von 300 nmol/l der Testsubstanz perfundiert. Nach 15 Min wird Carbachol kumulativ in ansteigenden Konzentrationen der Perfusionslösung hinzugefügt. Daraus resultierende Senkung der Herzfrequenz wird als Dosis-Wirkungs-Kurve mit Effekten auf Herzen verglichen, die mit Lösungsmittel statt Testsubstanz behandelt wurden. Die Verschiebung der Carbachol-Dosis-Wirkungskurve wird mittels GraphPad PRISM (sigmoidal dose-response) analysiert und quantifiziert.

## B-9. Effekte der Testsubstanzen auf die Herzfrequenz in narkotisierten Ratten

Männliche Ratten des Stamm (WI) WU Br des Züchters Charles River werden initial mit einer 4-5%-igen Isofluran-Inhalation ca. 3 min narkotisiert. Danach erfolgt eine Erhaltungsnarkose mit einer 1,5 %-igen Isofluran-Inhalation. Dafür werden die narkotisierten Tiere dorsal auf einer beheizten Operationsplatte fixiert. Über visuelle Kontrolle und Zwischenzehenreflex wird die Narkosetiefe überprüft.

Für die Applikation der Testsubstanz wird ein i.v. Zugang an der Vena jugularis angelegt. Im Folgenden wird ein Hautschnitt in Längsrichtung von kaudal nach kranial durchgeführt und sowohl die Halsmuskulatur als auch die Speicheldrüsen durchtrennt. Die rechte Arteria carotis communis wird dargestellt und sowohl proximal als auch distal werden Blutsperren angelegt. Mit Mikroinstrumentarium wird ein TIP- Katheter (1.2F) in das Gefäß eingebracht um den arteriellen Druck und die Herzfrequenz zu messen.

Zunächst werden beide Parameter für 10 min im Basalzustand ohne Substanzgabe erfasst. Die zu untersuchenden Substanzen werden in geeigneten Lösungsmittelgemischen gelöst und anschliessend in verschiedenen Dosierungen jeweils einer Gruppe von Tieren über die Vena jugalaris über eine Infusionspumpe über 5 Min verabreicht. Eine Lösungsmittel-behandelte Gruppe wird als Kontrolle unter den gleichen Versuchsbedingungen eingesetzt. Die Erfassung des arteriellen Blutdruckes sowie der Herzfrequenz unter Substanzgabe erfolgt für 20 min. Die Daten werden mit dem PowerLab-System (ADinstruments) registriert und mit dem Programm LabChart (ADinstruments) ausgewertet.

In der folgenden Tabelle 4 sind für individuelle Ausführungsbeispiele die so ermittelten Werte für die prozentuale Senkung der Herzfrequenz aufgeführt (zum Teil als Mittelwerte aus mehreren unabhängigen Einzelbestimmungen):

**Tabelle 4**

| Bsp.Nr. | Reduktion Herzfrequenz [%] |
|---|---|
| 28 | 9 |

(fortgesetzt)

| Bsp.Nr. | Reduktion Herzfrequenz [%] |
|---|---|
| 174 | 7 |
| 175 | 6 |
| 189 | 7 |
| 201 | 6 |
| 212 | 11 |
| 213 | 14 |
| 271 | 8 |
| 275 | 9 |
| 284 | 11 |
| 296 | 11 |
| 297 | 10 |
| 323 | 13 |
| 325 | 13 |
| 330 | 9 |
| 332 | 11 |
| 342 | 10 |
| 349 | 14 |
| 370 | 12 |
| 386 | 13 |
| 400 | 11 |
| 404 | 9 |
| 407 | 13 |
| 414 | 16 |
| 511 | 10 |
| 520 | 15 |
| 523 | 15 |
| 530 | 13 |
| 541 | 14 |
| 550 | 13 |
| 590 | 13 |

## B-10. Radiotelemetrische Messung von Blutdruck und Herzfrequenz an wachen Ratten

Für die im Folgenden beschriebenen Messungen an wachen Ratten wird ein im Handel erhältliches Telemetriesystem der Firma Data Sciences International DSI, USA, eingesetzt. Das System besteht aus 3 Hauptkomponenten: (1) Implantierbare Sender (PhysioTel® Telemetrietransmitter), (2) Empfänger (PhysioTel® Receiver), die über einen Multiplexer (DSI Data Exchange Matrix) mit einem (3) Datenakquisitionscomputer verbunden sind. Die Telemetrieanlage ermöglicht eine kontinuierliche Erfassung von Blutdruck, Herzfrequenz und Körperbewegung an wachen Tieren in ihrem gewohnten Lebensraum.

Die Untersuchungen werden an ausgewachsenen weiblichen Ratten (Wistar Unilever/WU oder Spontaneous Hypertensive Rat/SHR) mit einem Körpergewicht von > 200 g durchgeführt. Die Versuchstiere werden nach Senderimplantation einzeln in Makrolon®-Käfigen Typ III gehalten. Sie haben freien Zugang zu Standardfutter und Wasser. Der Tag/Nacht-Rhythmus im Versuchslabor wird durch Wechsel der Raumbeleuchtung eingestellt.

Senderimplantation:

Die eingesetzten Telemetriesender (z.B. PA-C40, HD-S10, DSI) werden den Versuchstieren mindestens 14 Tage vor dem ersten Versuchseinsatz unter aseptischen Bedingungen chirurgisch implantiert. Zur Implantation werden die nüchternen Tiere mit Isofluran narkotisiert (IsoFlo®, Abbott, Einleitung 5%, Erhaltung 2%) und an der Bauchseite weiträumig rasiert und desinfiziert. Nach Eröffnung des Bauchraumes entlang der Linea alba wird der flüssigkeitsgefüllte Messkatheter des Systems oberhalb der Bifurkation nach cranial in die Aorta descendens eingesetzt und mit Gewebekleber (Vetbond™, 3M) befestigt. Das Sendergehäuse wird intraperitoneal an der Bauchwandmuskulatur fixiert und die Wunde schichtweise verschlossen. Postoperativ wird zur Infektionsprophylaxe ein Antibiotikum (Ursocyclin® 10%, 60 mg/kg s.c., 0.06 ml/100 g Körpergewicht, Serumwerk Bernburg AG, Deutschland) sowie ein Analgetikum (Rimadyl®, 4 mg/kg s.c., Pfizer, Deutschland) verabreicht.

Substanzen und Lösungen:

Wenn nicht anders beschrieben, werden die zu untersuchenden Substanzen jeweils einer Gruppe von Tieren (M= 6) oral verabreicht. Entsprechend einem Applikationsvolumen von 2 ml/kg Körpergewicht werden die Testsubstanzen in geeigneten Lösungsmittelgemischen gelöst. Eine Lösungsmittel-behandelte Gruppe von Tieren wird als Kontrolle eingesetzt.

Versuchsablauf:

Die Telemetrie-Messeinrichtung ist für 24 Tiere konfiguriert. Den in der Anlage lebenden instrumentierten Ratten ist jeweils eine eigene Empfangsantenne zugeordnet (RPC-1 Receiver, DSI). Die implantierten Sender sind über einen eingebauten Magnetschalter von außen aktivierbar und werden bei Versuchsvorlauf auf Sendung geschaltet. Die ausgestrahlten Signale können durch ein Datenakquisitionssystem (Dataquest™ A.R.T. for Windows, DSI oder Ponemah, DSI) online erfasst und entsprechend aufgearbeitet werden. Im Standardablauf werden über je 10 Sekunden Dauer gemessen: (1) systolischer Blutdruck (SBP), (2) diastolischer Blutdruck (DBP), (3) arterieller Mitteldruck (MAP), (4) Herzfrequenz (HR) und (5) Aktivität (ACT). Diese Parameter werden im Anschluss an die Applikation über 24 Stunden gemessen. Die Messwerterfassung wird rechnergesteuert in 5 Minuten-Abständen wiederholt. Die als Absolutwert erhobenen Quelldaten werden im Diagramm mit dem aktuell gemessenen Barometerdruck (Ambient Pressure Reference Monitor, APR-1, DSI) korrigiert.

Auswertung:

Nach Versuchsende werden die erhobenen Einzeldaten mit der Analysis-Software (Dataquest™ A.R.T. 4.1 Analysis oder Ponemah, DSI) sortiert. Als Leerwert wird der Zeitpunkt 2 Stunden vor Substanz-Applikation angenommen. Die Daten werden über eine voreinstellbare Zeit durch Mittelwertbestimmung geglättet (30 Minuten-Mittelwert).

**B-11. Effekte der Testsubstanzen auf die Herzfrequenz in anästhesierten Hunden**

Männliche oder weibliche Mischlingshunde (Mongrels, Marshall BioResources, USA) mit einem Gewicht zwischen 20 und 30 Kg werden mit Pentobarbital (30 mg/kg iv, Narcoren®, Merial, Deutschland) annarkotisiert. Pancuroniumchlorid (Pancuronium-Actavis®, Actavis, Deutschland, 1 mg/Tier iv) dient dabei zusätzlich als Muskelrelaxans. Die Hunde werden intubiert und mit einem Sauerstoff-Raumluft-Gemisch (40/60%) beatmet (etwa 5-6L/min). Die Beatmung erfolgt mit einem Beatmungsgerät der Firma GE Healthcare (Avance), welches gleichzeitig als Narkoseüberwachung dient ($CO_2$-Analysator). Die Narkose wird aufrechterhalten durch eine ständige Infusion von Pentobarbital (50µg/kg/min); als Analgetikum dient Fentanyl (10µg/kg/h). Eine Alternative zu Pentobarbital besteht in der Verwendung von Isofluran (1-2Vol%).

Der Hund wird folgendermaßen instrumentiert:

- Blasenkatheter zur Blasenentlastung bzw. zur Messung des Urinflusses
- EKG-Ableitungen an den Extremitäten (zur EKG Messung)
- Einführen eines NaCl-gefüllten Fluidmedic-PE-300-Schlauches in die A. femoralis. Dieser ist verbunden mit einem

Drucksensor (Braun Melsungen, Melsungen, Deutschland) zur Messung des systemischen Blutdrucks

- Einführen eines NaCl-gefüllten Venenkatheter (Vygon, Deutschland) in die V.femoralis zur Infusion von Prüfsubstanzen oder zur Blutentnahme.
- Einführen eines Millar Tip Katheters (Typ 350 PC, Millar Instruments, Houston, USA) über den linken Vorhof oder über eine in der A. carotis eingebundene Schleuse zur Messung der Herz-Hämodynamik
- Einführen eines Swan-Ganz-Katheters (CCOmbo 7.5F, Edwards, Irvine, USA) über die V. jugularis in die A. pulmonalis zur Messung von Herzzeitvolumen, Sauerstoffsättigung, Pulmonalartiendrücken und zentralvenösem Druck.
- Anbringen einer Ultraschall-Flussmess-Sonde (Transsonic Systems, Ithaka, USA) an der Aorta descendens zur Messung des Aortenflusses
- Anbringen einer Ultraschall-Flussmess-Sonde (Transsonic Systems, Ithaka, USA) an der linken A. coronaria zur Messung des Koronarflusses.
- Platzieren einer Braunüle in den Venae cephalicae zur Infusion von Pentobarbital, der Flüssigkeitssubstitution und zur Blutentnahme (Bestimmung der Substanz-Plasmaspiegeln oder sonstiger klinischer Blutwerte)
- Platzieren einer Braunüle in der Venae saphenae zur Infusion von Fentanyl und zur Substanzapplikation

Die Primärsignale werden eventuell verstärkt (Gould Verstärker, Gould Instrument Systems, Valley View, USA) bzw. Edwards-Vigilance- Monitor (Edwards, Irvine, USA) und anschließend zur Auswertung in das Ponemah-System (DataSciences Inc, Minneapolis, USA) eingespeist. Die Signale werden kontinuierlich über den gesamten Versuchszeitraum aufgezeichnet, von dieser Software digital weiterarbeitet und über 30 s gemittelt.

**B-12. Effekte der Testsubstanzen auf die Herzfrequenz und Herzfrequenzvariabilität in gesunden, wachen Hunden**

Zur Charakterisierung von Prüfsubstanzen hinsichtlich ihrer Wirkung auf die Herzfrequenz, Herzfrequenzvariabilität (HRV) und Blutdruck werden telemetrische Messungen in gesunden, männlichen Beagle-Hunden durchgeführt. Unter Isofluran-Narkose wird den Tieren zunächst ein Telemetriesender (Modell L21, Firma Data Sciences International, USA) implantiert. Dabei werden nach linksseitiger Thorakotomie Drucksensoren in der Aorta und im linken Ventrikel platziert. Zur Registrierung eines Elektrokardiogramms (EKG) werden weiterhin Elektroden auf dem Herzen platziert. Die Tiere werden anschließend zur Wundheilung unter antibiotischer (Clindamycin, Zoetis, Deutschland) und analgetischer (Fentanyl, Janssen, Deutschland) Nachsorge zurück in den Stall verbracht. Mittels im Tierstall installierter Antennen werden die Blutdruck- und EKG-Signale an einen Datenakquisitionscomputer weitergeleitet und durch eine Analysesoftware (Ponemah, Data Sciences International, USA) ausgewertet. Die Telemetrie-Anlage ermöglicht eine kontinuierliche Erfassung von Blutdrücken und EKG-Signalen in wachen Tieren. Technische Details können der Dokumentation der Herstellerfirma (Data Sciences International, USA) entnommen werden.

Die zu untersuchenden Substanzen werden den gesunden Hunden mittels einer Gelatine-Kapsel in geeigneten Lösungsmittelgemischen oral verabreicht. Eine Vehikel-behandelte Gruppe von Tieren wird als Kontrolle eingesetzt. Die telemetrische Messung wird vor Substanzgabe gestartet und über einen Zeitraum von mehreren Stunden aufgezeichnet. Die graphische Darstellung der zeitlichen Verläufe der durch Mittelwertbestimmung geglätteten Daten erfolgt mit Hilfe der GraphPadPrism Software (GraphPad, USA). Zur Analyse der HRV werden die EKG-Daten einer frequenzbezogenen Herzfrequenzvariabilitäts-Analyse ("frequency-domain") unterzogen. Dazu werden die R-R-Intervalle der aufgezeichneten EKG's verwendet. Daten außerhalb des zuvor definierten Bereiches von 0,2s - 1,5s werden von der Analyse ausgeschlossen. Die ausgeschlossenen Daten werden durch Werte die durch lineare Interpolation gewonnen wurden ersetzt. Diese Daten werden durch Spline-Interpolation in gleichabständige Unterstützungspunkte konvertiert. Zur Analyse der Herzfrequenzvariabilität werden die Daten weiterhin in 30 s Schritten zu Paketen von 300s Länge unterteilt. Für jedes Datenpaket wird eine Fourier-Transformation kalkuliert. Daraus wird weiterhin die Leistung in drei Frequenzbändern (vlf=0.0033 - 0.04 1/s; lf=0.04 - 0.15 1/s; hf=0.15 - 0.5 1/s) kalkuliert. Zur Charakterisierung der Prüfsubstanz wird die Gesamtleistung (Summe aller drei Frequenzbänder) zur HRV- Analyse herangezogen.

## Patentansprüche

**1.** Verbindung der Formel (I)

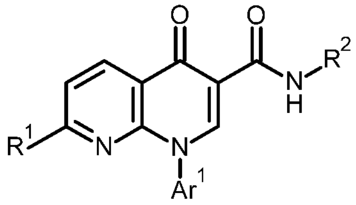

(I),

in welcher

$R^1$ für $NR^4R^5$ steht,
worin

$R^4$ Wasserstoff, Methyl, $(C_2\text{-}C_4)$-Alkyl oder $(C_3\text{-}C_6)$-Cycloalkyl bedeutet, wobei $(C_2\text{-}C_4)$-Alkyl mit Hydroxy oder bis zu dreifach mit Fluor substituiert sein kann
und
$R^5$ $(C_1\text{-}C_6)$-Alkyl, $(C_3\text{-}C_6)$-Cycloalkyl, 3- bis 6-gliedriges gesättigtes Heterocyclyl oder $(C_1\text{-}C_4)$-Alkylsulfonyl bedeutet,
wobei $(C_1\text{-}C_6)$-Alkyl, $(C_3\text{-}C_6)$-Cycloalkyl und 3- bis 6-gliedriges gesättigtes Heterocyclyl, bis zu dreifach, gleich oder verschieden, mit Methyl, Difluormethyl, Trifluormethyl, Hyd-roxy, Hydroxycarbonyl, Oxo, Methoxy, Difluormethoxy, Trifluormethoxy, Cyano und weiterhin bis zu vierfach mit Fluor, substituiert sein können,
oder
$R^4$ und $R^5$ bilden zusammen mit dem Stickstoffatom, an das sie gebunden sind, einen gesättigten oder partiell ungesättigten, 3- bis 6-gliedrigen monocyclischen oder 6- bis 10-gliedrigen bicyclischen Heterocyclus, der ein oder zwei weitere, gleiche oder verschiedene Heteroatome aus der Reihe N, O, S, SO und/oder $SO_2$ als Ringglieder enthalten kann,
wobei der 3- bis 6-gliedrige monocyclische und der 6- bis 10-gliedrige bicyclische Heterocyclus jeweils mit 1 bis 5 Substituenten unabhängig voneinander ausgewählt aus der Gruppe $(C_1\text{-}C_4)$-Alkyl, Difluormethyl, Trifluormethyl, Hydroxy, Hydroxycarbonyl, Oxo, $(C_1\text{-}C_3)$-Alkoxy, Difluormethoxy, Trifluormethoxy, Cyano, $(C_1\text{-}C_3)$-Alkoxycarbonyl, Aminocarbonyl, Mono-$(C_1\text{-}C_3)$-alkylaminocarbonyloxy, $-NHC(=O)R^{22A}$, $-CH_2NHC(=O)R^{22B}$, und weiterhin bis zu vierfach mit Fluor, substituiert sein können, worin
$R^{22A}$ und $R^{22B}$ unabhängig voneinander $(C_1\text{-}C_3)$-Alkyl oder Cyclopropyl darstellen,
und
worin $(C_1\text{-}C_4)$-Alkyl ein- oder zweifach, gleich oder verschieden, mit Hydroxy, $(C_1\text{-}C_3)$-Alkoxy, und bis zu vierfach mit Fluor, substituiert sein kann,

$R^2$ für eine Gruppe der Formel

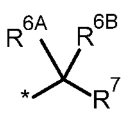

,

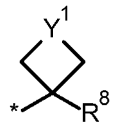

oder

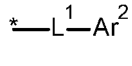

$*\text{-}L^1\text{-}Ar^2$ steht, worin

* die Verknüpfungsstelle mit dem N-Atom der Amid-Gruppierung markiert,
$R^{6A}$ Wasserstoff oder $(C_1\text{-}C_4)$-Alkyl bedeutet,

$R^{6B}$ Wasserstoff, $(C_1-C_4)$-Alkyl, Cyclopropyl, Trifluormethyl, Methoxymethyl oder Trifluormethoxymethyl bedeutet,
$R^7$ $(C_1-C_4)$-Alkyl, Cyclopropyl oder Cyclobutyl bedeutet,
wobei $(C_1-C_4)$-Alkyl bis zu fünffach und Cyclopropyl und Cyclobutyl bis zu vierfach mit Fluor substituiert sein können,
$Y^1$ $-(CH_2)_k-$, $-CF_2-$, $-O-CH_2-$, $-CH_2-O-$ oder $-CH_2-O-CH_2-$ bedeutet,
worin

k für 0, 1, 2 oder 3 steht,

$R^8$ bis zu fünffach mit Fluor substituiertes $(C_1-C_2)$-Alkyl oder Trifluormethoxymethyl bedeutet,
$L^1$ eine Bindung oder eine Gruppe der Formel $-C(R^{9A}R^{9B})-(C(R^{10A}R^{10B}))_m-$ bedeutet,
worin

m 0 oder 1 darstellt,
$R^{9A}$ Wasserstoff oder Methyl darstellt,
$R^{9B}$ Wasserstoff, Methyl, Trifluormethyl, Pentafluorethyl oder Trifluormethoxymethyl darstellt,
$R^{10A}$ und $R^{10B}$ unabhängig voneinander Wasserstoff oder Methyl darstellen,
$Ar^2$ Phenyl bedeutet, wobei Phenyl ein- bis dreifach, gleich oder verschieden, mit Fluor, Chlor, $(C_1-C_3)$-Alkyl, Difluormethoxymethyl, Trifluormethoxymethyl und/oder Trifluormethyl substituiert sein kann,
oder
für einen 5- bis 10-gliedrigen bicyclischen oder tricyclischen Carbocyclus steht,
wobei der 5- bis 10-gliedrige bicyclische oder tricyclische Carbocyclus bis zu dreifach, gleich oder verschieden, mit $(C_1-C_3)$-Alkyl, Trifluormethyl, und weiterhin bis zu vierfach mit Fluor substituiert sein kann,

$Ar^1$ für eine Gruppe der Formel

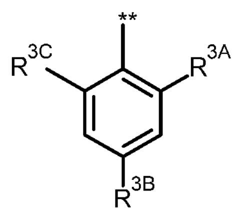

steht, worin

** die Verknüpfungsstelle mit dem N-Atom markiert,
$R^{3A}$ für Fluor, Chlor oder Trifluormethyl steht,
$R^{3B}$ für Wasserstoff oder Fluor steht
und
$R^{3C}$ für Wasserstoff, Fluor oder Chlor steht

sowie ihre *N*-Oxide, Salze, Solvate, Salze der *N*-Oxide und Solvate der *N*-Oxide und Salze.

**2.** Verbindung der Formel (I) nach Anspruch 1,
in welcher

$R^1$ für $NR^4R^5$ steht,
worin

$R^4$ Wasserstoff oder Methyl bedeutet,
und
$R^5$ $(C_1-C_4)$-Alkyl oder Methylsulfonyl bedeutet,
wobei $(C_1-C_4)$-Alkyl bis zu zweifach mit Hydroxy und weiterhin bis zu dreifach mit Fluor, substituiert sein kann,

oder

$R^4$ und $R^5$ bilden zusammen mit dem Stickstoffatom, an das sie gebunden sind, einen gesättigten oder partiell ungesättigten 4- bis 6-gliedrigen monocyclischen oder 6- bis 10-gliedrigen bicyclischen Heterocyclus, der ein oder zwei weitere Heteroatome aus der Reihe N, O, S, SO oder $SO_2$ als Ringglied enthalten kann,

wobei der 4- bis 6-gliedrige monocyclische und der 6- bis 10-gliedrige bicyclische Heterocyclus jeweils mit 1 bis 5 Substituenten unabhängig voneinander ausgewählt aus der Gruppe $(C_1$-$C_3)$-Alkyl, Difluormethyl, Trifluormethyl, Hydroxymethyl, Hydroxyethyl, Hydroxy, Oxo, Methoxy, Difluormethoxy, Trifluormethoxy, Methoxymethyl, Cyano, Methoxycarbonyl, Aminocarbonyl, Monomethylaminocarbonyloxy, und weiterhin bis zu vierfach mit Fluor, substituiert sein können,

$R^2$ für eine Gruppe der Formel

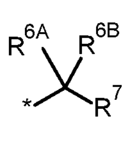 , 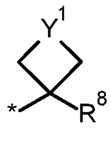 , $*-L^1-Ar^2$, 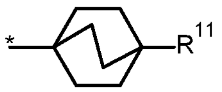 ,

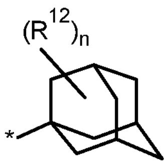 oder 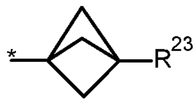

steht, worin

* die Verknüpfungsstelle mit dem N-Atom der Amid-Gruppierung markiert,
$R^{6A}$ Wasserstoff oder Methyl bedeutet,
$R^{6B}$ Wasserstoff, $(C_1$-$C_4)$-Alkyl, Cyclopropyl, Trifluormethyl oder Trifluormethoxymethyl bedeutet,
$R^7$ $(C_1$-$C_4)$-Alkyl, Cyclopropyl oder Cyclobutyl bedeutet,
wobei $(C_1$-$C_4)$-Alkyl bis zu fünffach mit Fluor substituiert sein kann,
$Y^1$ $-(CH_2)_k-$, $-CF_2-$, $-O-CH_2-$, $-CH_2-O-$ oder $-CH_2-O-CH_2-$ bedeutet, worin

k für 0, 1, 2 oder 3 steht,

$R^8$ Methyl, Trifluormethyl oder 2,2,2-Trifluorethyl bedeutet,
$L^1$ eine Bindung oder eine Gruppe der Formel $-CR^{9A}R^{9B}-$ bedeutet,
worin

$R^{9A}$ Wasserstoff oder Methyl darstellt,
$R^{9B}$ Wasserstoff, Methyl, Trifluormethyl oder Trifluormethoxymethyl darstellt,

$Ar^2$ Phenyl bedeutet,
welches ein- oder zweifach, gleich oder verschieden, mit Fluor, Chlor, Methyl und/oder Trifluormethyl substituiert sein kann,
$R^{11}$, $R^{12}$ und $R^{23}$ unabhängig voneinander Wasserstoff, Fluor, Methyl, Ethyl oder Trifluormethyl bedeuten,
n für die Zahl 1 oder 2 steht,
wobei im Fall, dass einer der Substituenten $R^{11}$, $R^{12}$ oder $R^{23}$ jeweils zweifach auftritt, seine Bedeutungen unabhängig voneinander gleich oder verschieden sein können,

$Ar^1$ für eine Gruppe der Formel

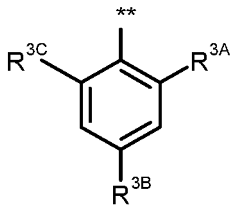

steht, worin

** die Verknüpfungsstelle mit dem N-Atom markiert,
$R^{3A}$ für Fluor, Chlor oder Trifluormethyl steht,
$R^{3B}$ für Wasserstoff oder Fluor steht
und
$R^{3C}$ für Wasserstoff, Fluor oder Chlor steht

sowie ihre Salze, Solvate und Solvate der Salze.

**3.** Verbindung der Formel (I) nach Anspruch 1 oder 2, in welcher

$R^1$ für $NR^4R^5$ steht,
worin

$R^4$ Wasserstoff oder Methyl bedeutet,
und
$R^5$ Methyl, Isopropyl, 2,2-Difluorethyl, 2,2,2-Trifluorethyl, 2-Hydroxyethyl oder 2-Hydroxypropyl bedeutet,
oder
für einen über ein Stickstoff-Atom gebundenen 4- bis 6-gliedrigen monocyclischen oder 6- bis 8-gliedrigen bicyclischen Heterocyclus der Formel

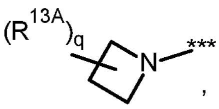 , 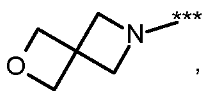 , 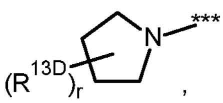 , 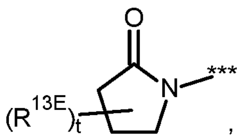 ,

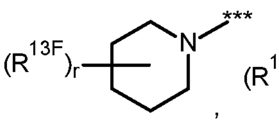 , 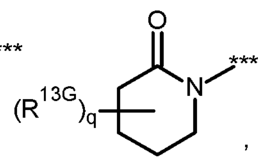 , 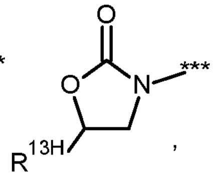 , 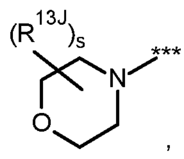 ,

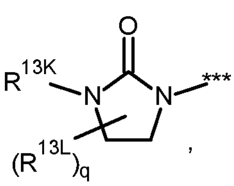 , 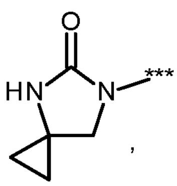 , 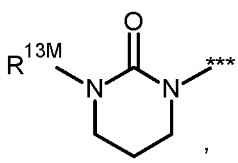 , 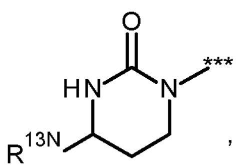 ,

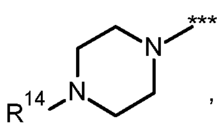 , 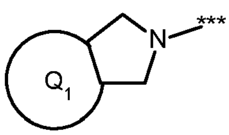 oder 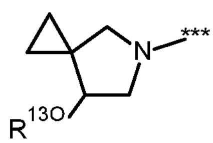

steht, worin
*** die Verknüpfungsstelle mit dem C-Atom des Pyridin-Rings markiert,

der Ring $Q_1$ für eine Gruppe der Formel

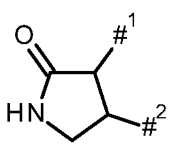 , 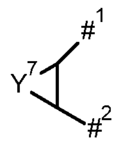 oder 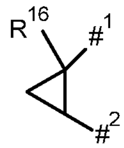

steht, worin
$\#^1$ und $\#^2$ die Verknüpfungsstelle mit dem C-Atom des Pyrrolidinrings markieren,
und
$Y^7$ für $-CF_2-$ oder $-CHR^{15}-$ steht,
worin
$R^{15}$ Methoxymethyl darstellt,
und
$R^{16}$ für Hydroxy steht,

$R^{13A}$ Fluor, Hydroxy, Hydroxymethyl, Methyl, Trifluormethyl oder Methoxy bedeutet,
$R^{13D}$ Wasserstoff, Fluor, Methyl, Hydroxy, Hydroxymethyl, Methoxy oder Difluormethoxy bedeutet,
$R^{13E}$ Wasserstoff, Fluor, Methyl, Hydroxy, Hydroxymethyl oder Methoxy bedeutet,
$R^{13F}$ Fluor, Methyl, Hydroxy, Hydroxymethyl oder Cyano bedeutet,
$R^{13G}$ Fluor oder Hydroxy bedeutet,
$R^{13H}$ Wasserstoff, Methyl, Hydroxymethyl, Aminocarbonyl oder Methoxycarbonyl bedeutet,
$R^{13J}$ Oxo, Hydroxymethyl oder Difluormethyl bedeutet,
$R^{13K}$ Wasserstoff, Methyl oder 2-Hydroxyethyl bedeutet,
$R^{13L}$ Wasserstoff oder Methyl bedeutet,
$R^{13M}$ Ethyl, 2-Hydroxyethyl oder Cyano bedeutet,
$R^{13N}$ Wasserstoff oder Ethyl bedeutet,
$R^{13O}$ Wasserstoff oder Hydroxy bedeutet,
$R^{14}$ Methyl, Methoxycarbonyl oder Aminocarbonyl bedeutet,
q die Zahl 0, 1 oder 2 bedeutet,
r die Zahl 0, 1, 2 oder 3 bedeutet,

s die Zahl 0 oder 1 bedeutet,
t die Zahl 0, 1, 2, 3 oder 4 bedeutet,

wobei im Fall, dass die Substituenten $R^{13A}$, $R^{13D}$, $R^{13E}$, $R^{13F}$, $R^{13G}$, $R^{13J}$, sowie $R^{13L}$ mehrfach auftreten, deren Bedeutungen jeweils gleich oder verschieden sein können,
$R^2$ für eine Gruppe der Formel

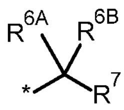 , 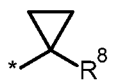 , $*-L^1-Ar^2$, 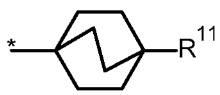 ,

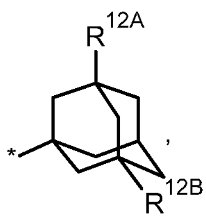 , 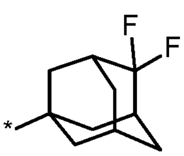 oder 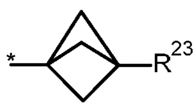

steht, worin

* die Verknüpfungsstelle mit dem N-Atom der Amid-Gruppierung markiert,
$R^{6A}$ Wasserstoff oder Methyl bedeutet,
$R^{6B}$ Methyl, Ethyl, Cyclopropyl, Trifluormethyl oder Trifluormethoxymethyl bedeutet,

$R^7$ Methyl, Ethyl, n-Propyl, Isopropyl, tert.-Butyl, 2-Methyl-prop-1-yl, Trifluormethyl, Difluormethyl, Pentafluorethyl, 2,2,2-Trifluorethyl oder Cyclopropyl bedeutet,
$R^8$ 2,2,2-Trifluorethyl bedeutet,
$L^1$ eine Bindung oder eine Gruppe der Formel $-CR^{9A}R^{9B}-$ bedeutet, worin
$R^{9A}$ Wasserstoff oder Methyl darstellt,
$R^{9B}$ Wasserstoff, Methyl, Trifluormethyl oder Trifluormethoxymethyl darstellt,
$Ar^2$ Phenyl bedeutet, welches ein- oder zweifach, gleich oder verschieden, mit Fluor, Chlor, Methyl und/oder Trifluormethyl substituiert sein kann,
$R^{11}$ Wasserstoff, Fluor oder Methyl bedeutet,
$R^{12A}$ Wasserstoff, Fluor, Methyl, Ethyl oder Trifluormethyl bedeutet,

$R^{12B}$ Wasserstoff oder Fluor bedeutet,
$R^{23}$ Wasserstoff, Fluor oder Trifluormethyl bedeutet,

und

$Ar^1$ für eine Gruppe der Formel

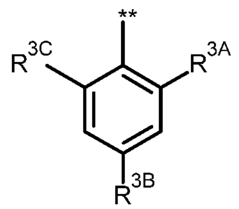

steht, worin

** die Verknüpfungsstelle mit dem N-Atom markiert,
$R^{3A}$ für Fluor oder Chlor steht,
$R^{3B}$ für Wasserstoff oder Fluor steht,
und
$R^{3C}$ für Wasserstoff, Fluor oder Chlor steht

sowie ihre Salze, Solvate und Solvate der Salze.

**4.** Verbindung der Formel (I) nach einem der Ansprüche 1 bis 3, in welcher

$R^1$ für eine Gruppe der Formel

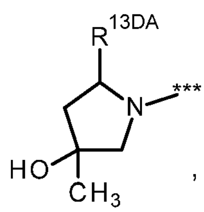 , 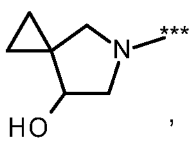 , 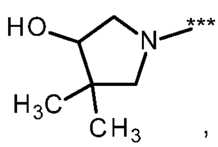 , 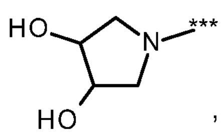 ,

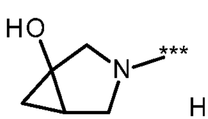 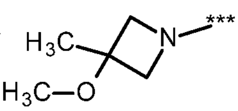 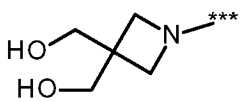 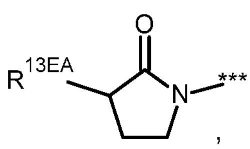 ,

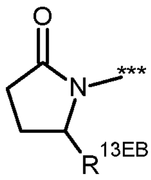 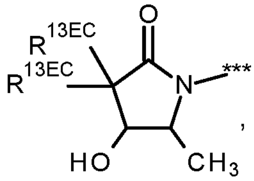, 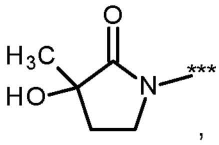, 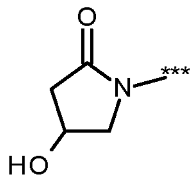

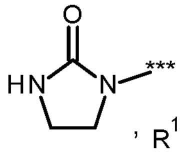, 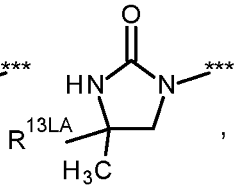, 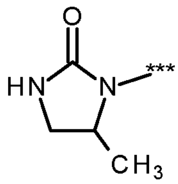 oder 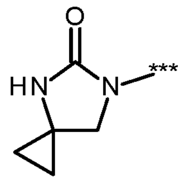

steht, worin

*** die Verknüpfungsstelle mit dem C-Atom des Pyridin-Rings markiert,
$R^{13DA}$ Wasserstoff oder Methyl bedeutet,
$R^{13EA}$ Hydroxy oder Hydroxymethyl bedeutet,
$R^{13EB}$ Methyl oder Hydroxymethyl bedeutet,
$R^{13EC}$ Wasserstoff oder Methyl bedeutet,
$R^{13LA}$ Wasserstoff oder Methyl bedeutet,

$R^2$ für eine Gruppe der Formel

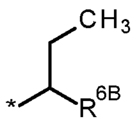, 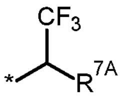, 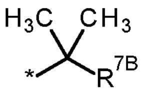, 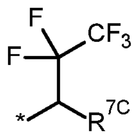 oder 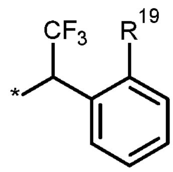

steht, worin

* die Verknüpfungsstelle mit dem N-Atom der Amid-Gruppierung markiert,
$R^{6B}$ Trifluormethoxymethyl bedeutet,
$R^{7A}$ Methyl, Ethyl, Trifluormethyl oder Cyclopropyl bedeutet,
$R^{7B}$ Trifluormethyl, Difluormethyl oder 2,2,2-Trifluorethyl bedeutet,
$R^{7C}$ Methyl oder Ethyl bedeutet,
$R^{19}$ Chlor bedeutet,
und
$Ar^1$ für eine Gruppe der Formel

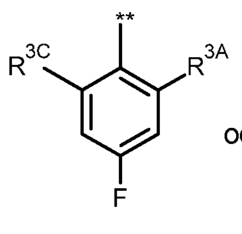 oder 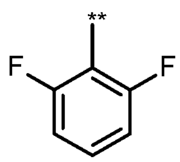

steht, worin

** die Verknüpfungsstelle mit dem N-Atom markiert,
$R^{3A}$ für Fluor oder Chlor steht, und
$R^{3C}$ für Wasserstoff oder Fluor steht,

sowie ihre Salze, Solvate und Solvate der Salze.

**5.** Verbindung der Formel (I) nach einem der Ansprüche 1 bis 4, in welcher

$R^1$ für eine Gruppe der Formel

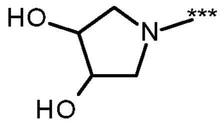
oder
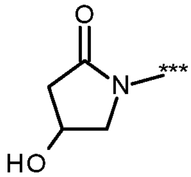

steht, worin

*** die Verknüpfungsstelle mit dem C-Atom des Pyridin-Rings markiert,

$R^2$ für eine Gruppe der Formel

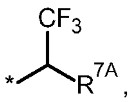
,
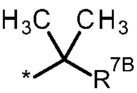
oder
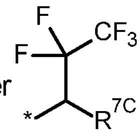

steht, worin

* die Verknüpfungsstelle mit dem N-Atom der Amid-Gruppierung markiert,
$R^{7A}$ Ethyl, Trifluormethyl oder Cyclopropyl bedeutet,
$R^{7B}$ Trifluormethyl bedeutet,

$R^{7C}$ Methyl oder Ethyl bedeutet,
und
$Ar^1$ für eine Gruppe der Formel

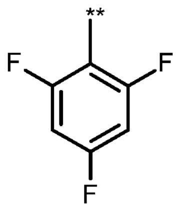
oder
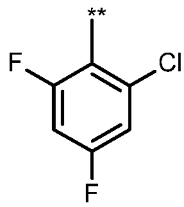

steht, worin

** die Verknüpfungsstelle mit dem N-Atom markiert,

sowie ihre Salze, Solvate und Solvate der Salze.

**6.** Verbindung der Formel (I) nach einem der Ansprüche 1 bis 5, in welcher

$R^1$ für eine Gruppe der Formel

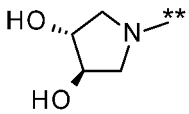
oder
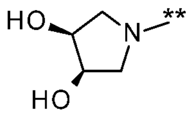

steht, worin

** die Verknüpfungsstelle mit dem C-Atom des Pyridin-Rings markiert.

7. Verbindung gemäß Anspruch 1, in welcher die Verbindung der Formel (I) die folgende Struktur hat

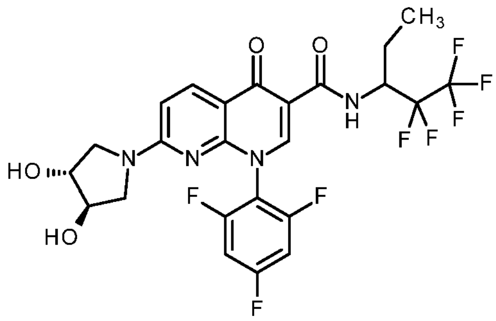

8. Verbindung gemäß Anspruch 1, in welcher die Verbindung der Formel (I) die folgende Struktur hat

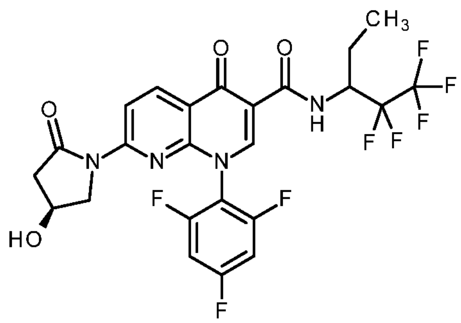

9. Verbindung gemäß Anspruch 1, in welcher die Verbindung der Formel (I) die folgende Struktur hat

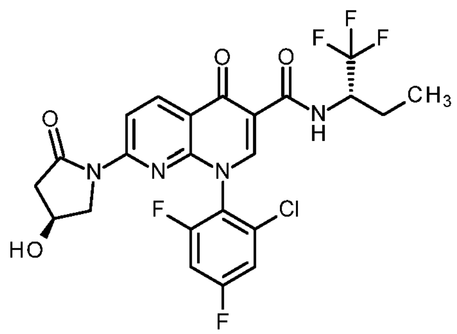

10. Verbindung gemäß Anspruch 1, in welcher die Verbindung der Formel (I) die folgende Struktur hat

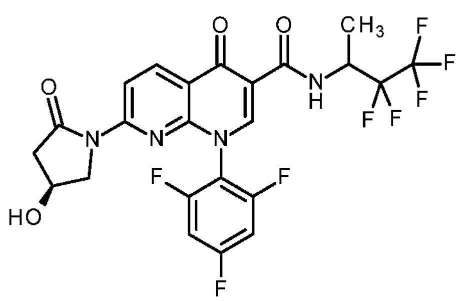

**11.** Verbindung gemäß Anspruch 1, in welcher die Verbindung der Formel (I) die folgende Struktur hat

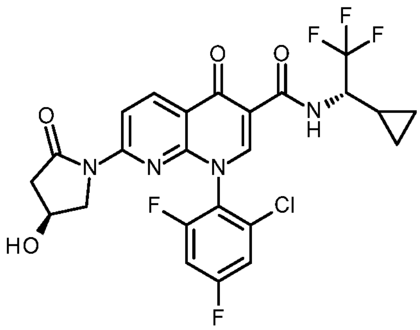

**12.** Verfahren zur Herstellung von Verbindungen der Formel (I) wie in einem der Ansprüche 1 bis 11 definiert, **dadurch gekennzeichnet, dass** man

[A] eine Verbindung der Formel (II)

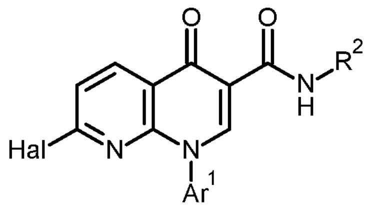

(II),

in welcher $R^2$ und $Ar^1$ die oben angegebenen Bedeutungen haben und
Hal für Fluor, Chlor, Brom oder Iod, bevorzugt für Chlor, steht, mit einer Verbindung der Formel (III)

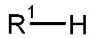

(III),

in welcher $R^1$ die oben angegebene Bedeutung hat,
zum Carbonsäureamid der Formel (I)

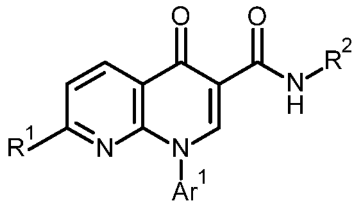

(I),

in welcher $R^1$, $R^2$ und $Ar^1$ die oben angegebenen Bedeutungen haben, umsetzt,
oder
[B] eine Verbindung der Formel (IV)

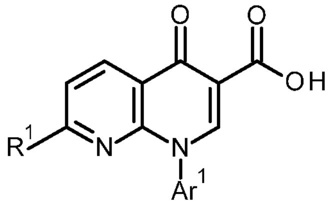

(IV),

in welcher $R^1$ und $Ar^1$ die oben angegebenen Bedeutungen haben,

mit einer Verbindung der Formel (V)

$R^2-NH_2$ (V),

in welcher $R^2$ die oben angegebene Bedeutung hat,
zum Carbonsäureamid der Formel (I)

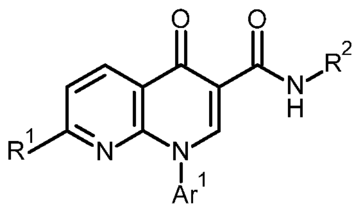

(I),

in welcher $R^1$, $R^2$ und $Ar^1$ die oben angegebenen Bedeutungen haben, umsetzt,
und gegebenenfalls die so erhaltenen Verbindungen der Formel (I) in ihre Enantiomere und/oder Diastereomere trennt und/oder mit den entsprechenden (*i*) Lösungsmitteln und/oder (*ii*) Basen oder Säuren in ihre Solvate, Salze und/oder Solvate der Salze überführt.

**13.** Verbindung der Formel (II)

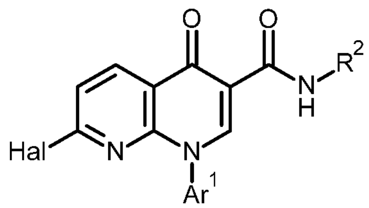

(II),

in welcher $R^2$ und $Ar^1$ die oben für Verbindungen der Formel (I) angegebenen Bedeutungen haben und Hal für Fluor, Chlor, Brom oder Iod, bevorzugt für Chlor, steht.

**14.** Verbindung der Formel (IV)

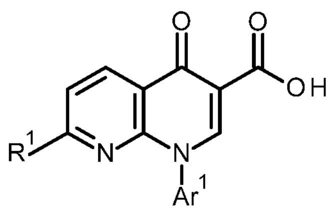

(IV),

in welcher $R^1$ und $Ar^1$ die oben für Verbindungen der Formel (I) angegebenen Bedeutungen haben.

**15.** Verwendung einer Verbindung der Formel (II)

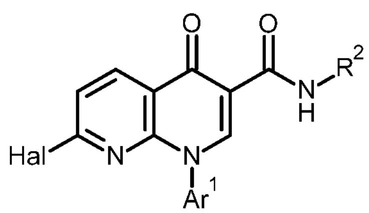

(II),

in welcher $R^2$ und $Ar^1$ die oben für Verbindungen der Formel (I) angegebenen Bedeutungen haben und

Hal für Fluor, Chlor, Brom oder Iod, bevorzugt für Chlor, steht.
oder
einer Verbindung der Formel (IV)

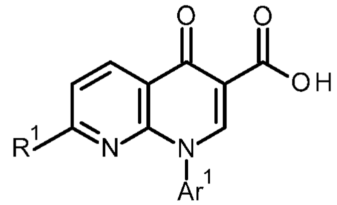
(IV),

in welcher $R^1$ und $Ar^1$ die oben für Verbindungen der Formel (I) angegebenen Bedeutungen haben, zur Herstellung einer Verbindung der Formel (I) gemäß einem der Ansprüche 1 bis 11.

**16.** Verbindung, wie in einem der Ansprüche 1 bis 11 definiert, zur Verwendung bei der Behandlung und/oder Prophylaxe von Krankheiten.

**17.** Verbindung der Formel (I), wie in einem der Ansprüche 1 bis 11 definiert, zur Verwendung in einen Verfahren zur Behandlung und/oder Prophylaxe von Herzinsuffizienz, koronarer Herzerkrankung, atrialen und ventrikulären Arrhythmien, Niereninsuffizienz und Nephropathien.

**18.** Arzneimittel enthaltend eine Verbindung, wie in einem der Ansprüche 1 bis 11 definiert, in Kombination mit einem oder mehreren weiteren Wirkstoffen ausgewählt aus der Gruppe bestehend aus den Blutdruck senkende Wirkstoffe, antiarrhythmische Wirkstoffe, Vasopressin-Rezeptor-Antagonisten, PDE 5-Inhibitoren, Thrombozytenaggregationshemmer, sGC-Aktivatoren und sGC-Stimulatoren.

**19.** Arzneimittel enthaltend eine Verbindung, wie in einem der Ansprüche 1 bis 11 definiert, in Kombination mit einem inerten, nichttoxischen, pharmazeutisch geeigneten Hilfsstoff.

**20.** Arzneimittel nach Anspruch 18 oder 19 zur Verwendung bei der Behandlung und/oder Prophylaxe von Herzinsuffizienz, koronarer Herzerkrankung, atrialen und ventrikulären Arrhythmien, Niereninsuffizienz und Nephropathien.

## Claims

**1.** Compound of the formula (I)

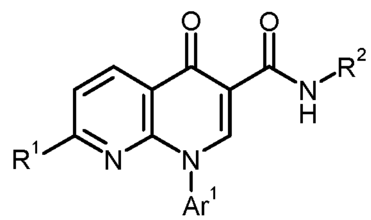
(I)

in which

$R^1$ is $NR^4R^5$,
in which

$R^4$ is hydrogen, methyl, $(C_2-C_4)$ -alkyl or $(C_3-C_6)$-cycloalkyl,
where $(C_2-C_4)$-alkyl may be substituted by hydroxyl or up to trisubstituted by fluorine
and
$R^5$ is $(C_1-C_6)$-alkyl, $(C_3-C_6)$-cycloalkyl, 3- to 6-membered saturated heterocyclyl or $(C_1-C_4)$-alkylsulfonyl, where $(C_1-C_6)$-alkyl, $(C_3-C_6)$-cycloalkyl and 3-to 6-membered saturated heterocyclyl may be up to trisubstituted, identically or differently, by methyl, difluoromethyl, trifluoromethyl, hydroxyl, hydroxycarbonyl, oxo,

methoxy, difluoromethoxy, trifluoromethoxy and cyano, and additionally up to tetrasubstituted by fluorine, or

$R^4$ and $R^5$ together with the nitrogen atom to which they are bonded form a saturated or partially unsaturated, 3- to 6-membered monocyclic or 6- to 10-membered bicyclic heterocycle which may contain one or two further, identical or different heteroatoms from the group of N, O, S, SO and/or $SO_2$ as ring members,
where the 3- to 6-membered monocyclic and the 6- to 10-membered bicyclic heterocycle may each be substituted by 1 to 5 substituents independently selected from the group of ($C_1$-$C_4$)-alkyl, difluoromethyl, trifluoromethyl, hydroxy, hydroxycarbonyl, oxo, ($C_1$-$C_3$)-alkoxy, difluoromethoxy, trifluoromethoxy, cyano, ($C_1$-$C_3$)-alkoxycarbonyl, aminocarbonyl, mono-($C_1$-$C_3$)-alkylaminocarbonyloxy,
$-NHC(=O)R^{22A}$ and $-CH_2NHC(=O)R^{22B}$, and additionally up to tetrasubstituted by fluorine, in which $R^{22A}$ and $R^{22B}$ independently represent ($C_1$-$C_3$)-alkyl or cyclopropyl, and
in which ($C_1$-$C_4$)-alkyl may be mono- or disubstituted, identically or differently, by hydroxyl and ($C_1$-$C_3$)-alkoxy, and up to tetrasubstituted by fluorine,

$R^2$ is a group of the formula

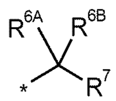 , 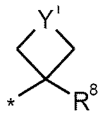 or 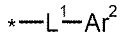

in which

* marks the bonding site to the nitrogen atom of the amide moiety,
$R^{6A}$ is hydrogen or ($C_1$-$C_4$)-alkyl,
$R^{6B}$ is hydrogen, ($C_1$-$C_4$)-alkyl, cyclopropyl, trifluoromethyl, methoxymethyl or trifluoromethoxymethyl,
$R^7$ is ($C_1$-$C_4$)-alkyl, cyclopropyl or cyclobutyl, where ($C_1$-$C_4$)-alkyl may be up to pentasubstituted and cyclopropyl and cyclobutyl up to tetrasubstituted by fluorine,
$Y^1$ is $-(CH_2)_k-$, $-CF_2-$, $-O-CH_2-$, $-CH_2-O-$ or $-CH_2-O-CH_2-$,
in which

k is 0, 1, 2 or 3,

$R^8$ is up to penta-fluorine-substituted ($C_1$-$C_2$)-alkyl or trifluoromethoxymethyl,
$L^1$ is a bond or a group of the formula $-C(R^{9A}R^{9B})-(C(R^{10A}R^{10B}))_m-$,
in which
m represents 0 or 1,
$R^{9A}$ represents hydrogen or methyl,
$R^{9B}$ represents hydrogen, methyl, trifluoromethyl, pentafluoroethyl or trifluoromethoxymethyl,
$R^{10A}$ and $R^{10B}$ independently represent hydrogen or methyl,
$Ar^2$ is phenyl,
where phenyl may be mono- to trisubstituted, identically or differently, by fluorine, chlorine, ($C_1$-$C_3$)-alkyl, difluoromethoxy-methyl, trifluoromethoxymethyl and/or trifluoromethyl,
or
is a 5- to 10-membered bicyclic or tricyclic carbocycle,
where the 5- to 10-membered bicyclic or tricyclic carbocycle may be up to trisubstituted, identically or differently, by ($C_1$-$C_3$)-alkyl and trifluoromethyl, and additionally up to tetrasubstituted by fluorine,

$Ar^1$ is a group of the formula

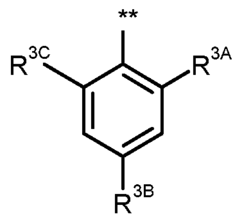

in which

** marks the bonding site to the nitrogen atom,
$R^{3A}$ is fluorine, chlorine or trifluoromethyl,
$R^{3B}$ is hydrogen or fluorine
and
$R^{3C}$ is hydrogen, fluorine or chlorine

and the N-oxides, salts, solvates, salts of the N-oxides and solvates of the N-oxides or salts thereof.

**2.** Compound of the formula (I) according to Claim 1 in which

$R^1$ is $NR^4R^5$,
in which

$R^4$ is hydrogen or methyl,
and
$R^5$ is $(C_1\text{-}C_4)$-alkyl or methylsulfonyl, where $(C_1\text{-}C_4)$-alkyl may be up to disubstituted by hydroxyl and additionally up to trisubstituted by fluorine,
or
$R^4$ and $R^5$ together with the nitrogen atom to which they are bonded form a saturated or partially unsaturated, 4- to 6-membered monocyclic or 6- to 10-membered bicyclic heterocycle which may contain one or two further heteroatoms from the group of N, O, S, SO and $SO_2$ as ring member,
where the 4- to 6-membered monocyclic and the 6- to 10-membered bicyclic heterocycle may each be substituted by 1 to 5 substituents independently selected from the group of $(C_1\text{-}C_3)$-alkyl, difluoromethyl, trifluoromethyl, hydroxymethyl, hydroxyethyl, hydroxyl, oxo, methoxy, difluoromethoxy, trifluoromethoxy, methoxymethyl, cyano, methoxycarbonyl, aminocarbonyl and monomethylaminocarbonyloxy, and additionally up to tetrasubstituted by fluorine,

$R^2$ is a group of the formula

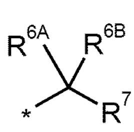 , 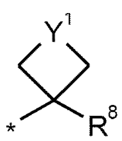 , $*-L^1-Ar^2$ , 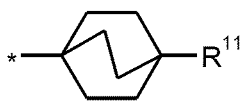 ,

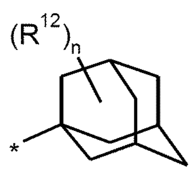 or 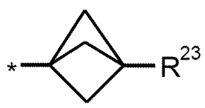

in which

* marks the bonding site to the nitrogen atom
of the amide moiety,
$R^{6A}$ is hydrogen or methyl,
$R^{6B}$ is hydrogen, $(C_1\text{-}C_4)$-alkyl, cyclopropyl, trifluoromethyl or trifluoromethoxymethyl,

$R^7$ is ($C_1$-$C_4$)-alkyl, cyclopropyl or cyclobutyl, where ($C_1$-$C_4$)-alkyl may be up to pentasubstituted by fluorine,
$Y^1$ is $-(CH_2)_k-$, $-CF_2-$, $-O-CH_2-$, $-CH_2-O-$ or $-CH_2-O-CH_2-$,
in which

k is 0, 1, 2 or 3,

$R^8$ is methyl, trifluoromethyl or 2,2,2-trifluoroethyl,
$L^1$ is a bond or a group of the formula $-CR^{9A}R^{9B}-$, in which

$R^{9A}$ represents hydrogen or methyl,
$R^{9B}$ represents hydrogen, methyl, trifluoromethyl or trifluoromethoxymethyl,

$Ar^2$ is phenyl,
which may be mono- or disubstituted, identically or differently, by fluorine, chlorine, methyl and/or trifluoromethyl,
$R^{11}$, $R^{12}$ and $R^{23}$ are each independently hydrogen, fluorine, methyl, ethyl or trifluoromethyl,
n is the number 1 or 2,

$Ar^1$ where, if one of the substituents $R^{11}$, $R^{12}$ or $R^{23}$ occurs twice in each case, its definitions may independently be the same or different, is a group of the formula

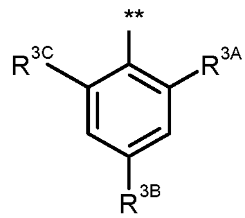

in which

** marks the bonding site to the nitrogen atom,
$R^{3A}$ is fluorine, chlorine or trifluoromethyl,
$R^{3B}$ is hydrogen or fluorine
and
$R^{3C}$ is hydrogen, fluorine or chlorine

and the salts, solvates and solvates of the salts thereof.

**3.** Compound of the formula (I) according to Claim 1 or 2
in which

$R^1$ is $NR^4R^5$,
in which

$R^4$ is hydrogen or methyl,
and
$R^5$ is methyl, isopropyl, 2,2-difluoroethyl, 2,2,2-trifluoroethyl, 2-hydroxyethyl or 2-hydroxypropyl,
or
is a 4- to 6-membered monocyclic or 6- to 8-membered bicyclic heterocycle which is bonded via a nitrogen atom and is of the formula

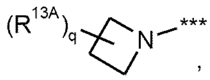 , 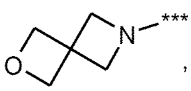 , 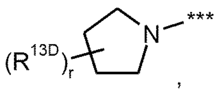 , 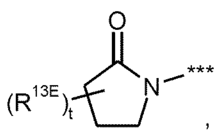 ,

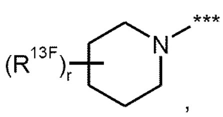, 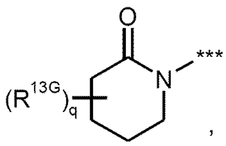, 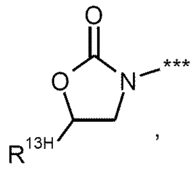, 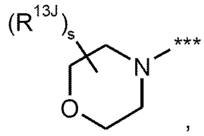,

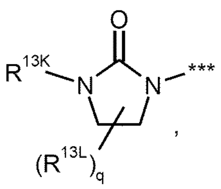, 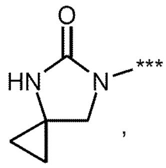, 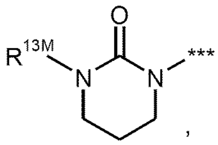, 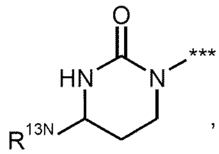,

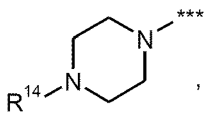, 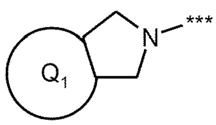 or 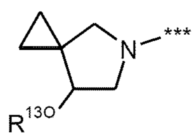

in which
*** marks the bonding site to the carbon atom of the pyridine ring,
the ring $Q_1$ is a group of the formula

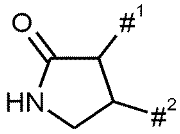, 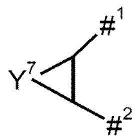 or 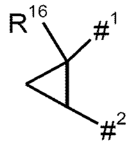

in which

$\#^1$ and $\#^2$ mark the bonding site to the carbon atom of the pyrrolidine ring, and
$Y^7$ is $-CF_2-$ or $-CHR^{15}-$,
in which

$R^{15}$ represents methoxymethyl,
and

$R^{16}$ is hydroxyl,

$R^{13A}$ is fluorine, hydroxyl, hydroxymethyl, methyl, trifluoromethyl or methoxy,
$R^{13D}$ is hydrogen, fluorine, methyl, hydroxyl, hydroxymethyl, methoxy or difluoromethoxy,
$R^{13E}$ is hydrogen, fluorine, methyl, hydroxyl, hydroxymethyl or methoxy,
$R^{13F}$ is fluorine, methyl, hydroxyl, hydroxymethyl or cyano,
$R^{13G}$ is fluorine or hydroxyl,
$R^{13H}$ is hydrogen, methyl, hydroxymethyl, aminocarbonyl or methoxycarbonyl,
$R^{13J}$ is oxo, hydroxymethyl or difluoromethyl,
$R^{13K}$ is hydrogen, methyl or 2-hydroxyethyl,
$R^{13L}$ is hydrogen or methyl,
$R^{13M}$ is ethyl, 2-hydroxyethyl or cyano,
$R^{13N}$ is hydrogen or ethyl,
$R^{13O}$ is hydrogen or hydroxyl,
$R^{14}$ is methyl, methoxycarbonyl or aminocarbonyl,
q is the number 0, 1 or 2,
r is the number 0, 1, 2 or 3,
s is the number 0 or 1,

t is the number 0, 1, 2, 3 or 4,
where, in the case that the substituents $R^{13A}$, $R^{13D}$, $R^{13E}$, $R^{13F}$, $R^{13G}$, $R^{13J}$ and $R^{13L}$ occur more than once, the definitions thereof may each be the same or different,
$R^2$ is a group of the formula

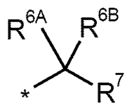 , 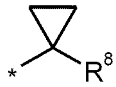 , 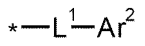 , 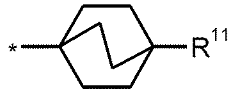 ,

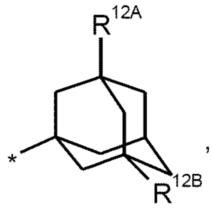 , 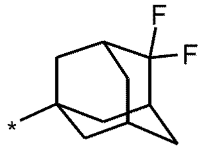 or 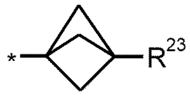

in which

* marks the bonding site to the nitrogen atom of the amide moiety,
$R^{6A}$ is hydrogen or methyl,
$R^{6B}$ is methyl, ethyl, cyclopropyl, trifluoromethyl or trifluoromethoxymethyl,
$R^7$ is methyl, ethyl, n-propyl, isopropyl, tert-butyl, 2-methylprop-1-yl, trifluoromethyl, difluoromethyl, pentafluoroethyl, 2,2,2-trifluoroethyl or cyclopropyl,
$R^8$ is 2,2,2-trifluoroethyl,
$L^1$ is a bond or a group of the formula $-CR^{9A}R^{9B}-$, in which

$R^{9A}$ represents hydrogen or methyl,
$R^{9B}$ represents hydrogen, methyl, trifluoromethyl or trifluoromethoxymethyl,

$Ar^2$ is phenyl,
which may be mono- or disubstituted, identically or differently, by fluorine, chlorine, methyl and/or trifluoromethyl,
$R^{11}$ is hydrogen, fluorine or methyl,
$R^{12A}$ is hydrogen, fluorine, methyl, ethyl or trifluoromethyl,
$R^{12B}$ is hydrogen or fluorine,
$R^{23}$ is hydrogen, fluorine or trifluoromethyl,

and
$Ar^1$ is a group of the formula

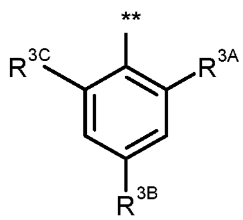

in which

** marks the bonding site to the nitrogen atom,
$R^{3A}$ is fluorine or chlorine,
$R^{3B}$ is hydrogen or fluorine,
and
$R^{3C}$ is hydrogen, fluorine or chlorine

and the salts, solvates and solvates of the salts thereof.

**4.** Compound of the formula (I) according to any of Claims 1 to 3 in which

$R^1$ is a group of the formula

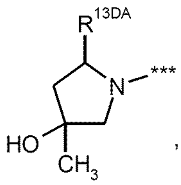 , 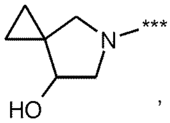 , 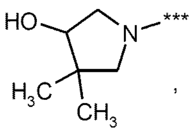 , 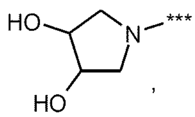 ,

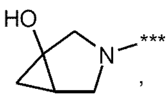 , 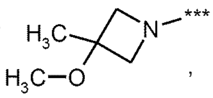 , 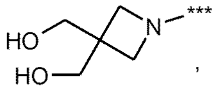 , 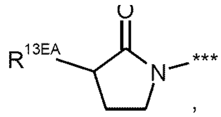 ,

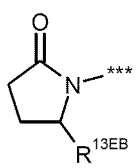 , 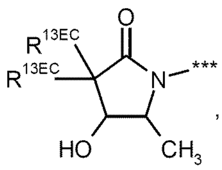 , 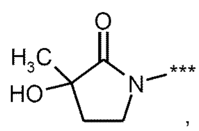 , 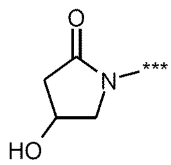

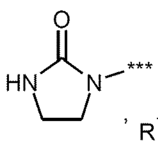 , 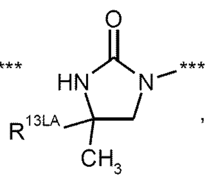 , 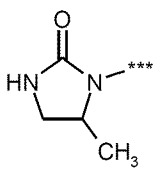 or 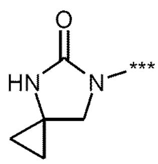

in which

*** marks the bonding site to the carbon atom of the pyridine ring,
$R^{13DA}$ is hydrogen or methyl,
$R^{13EA}$ is hydroxyl or hydroxymethyl,
$R^{13EB}$ is methyl or hydroxymethyl,
$R^{13EC}$ is hydrogen or methyl,
$R^{13LA}$ is hydrogen or methyl,

$R^2$ is a group of the formula

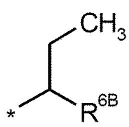 , 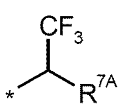 , 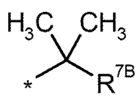 , 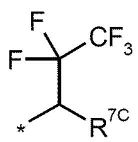 or 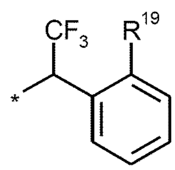

in which

* marks the bonding site to the nitrogen atom of the amide moiety,
$R^{6B}$ is trifluoromethoxymethyl,

$R^{7A}$ is methyl, ethyl, trifluoromethyl or cyclopropyl,
$R^{7B}$ is trifluoromethyl, difluoromethyl or 2,2,2-trifluoroethyl,
$R^{7C}$ is methyl or ethyl,
$R^{19}$ is chlorine, and
$Ar^1$ is a group of the formula

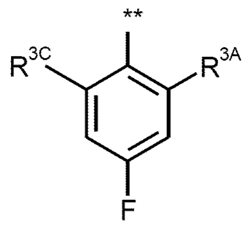
or
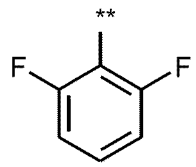

in which

** marks the bonding site to the nitrogen atom,
$R^{3A}$ is fluorine or chlorine, and
$R^{3C}$ is hydrogen or fluorine,

and the salts, solvates and solvates of the salts thereof.

**5.** Compound of the formula (I) according to any of Claims 1 to 4 in which

$R^1$ is a group of the formula

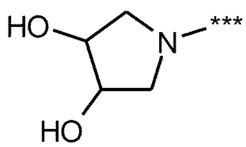
or
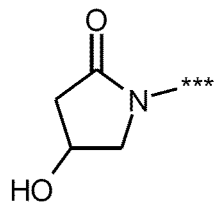

in which

*** marks the bonding site to the carbon atom of the pyridine ring,

$R^2$ is a group of the formula

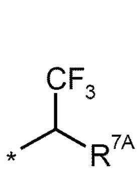
,
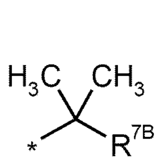
or
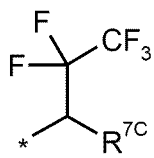

in which

* marks the bonding site to the nitrogen atom of the amide moiety,
$R^{7A}$ is ethyl, trifluoromethyl or cyclopropyl,
$R^{7B}$ is trifluoromethyl,
$R^{7C}$ is methyl or ethyl,
and
$Ar^1$ is a group of the formula

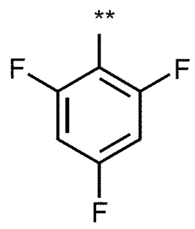

or

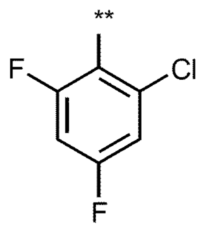

in which

** marks the bonding site to the nitrogen atom,

and the salts, solvates and solvates of the salts thereof.

**6.** Compound of the formula (I) according to any of Claims 1 to 5, in which

$R^1$ is a group of the formula

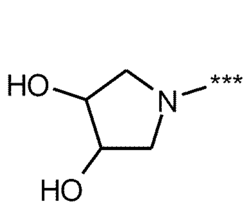

or

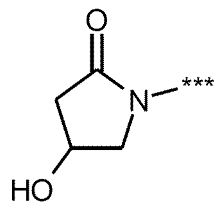

in which

** marks the bonding site to the carbon atom of the pyridine ring.

**7.** Compound according to Claim 1, in which the compound of the formula (I) has the following structure:

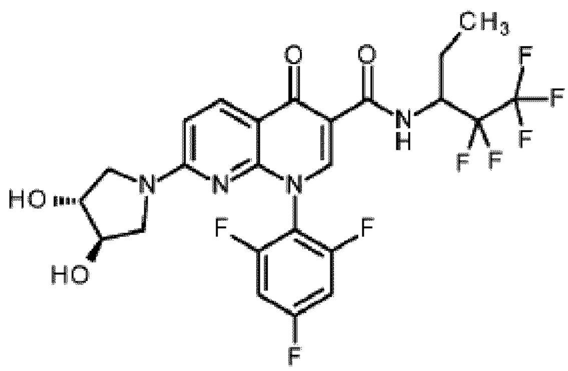

**8.** Compound according to Claim 1, in which the compound of the formula (I) has the following structure:

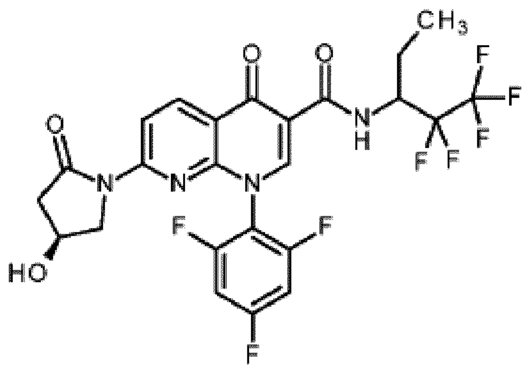

9. Compound according to Claim 1, in which the compound of the formula (I) has the following structure:

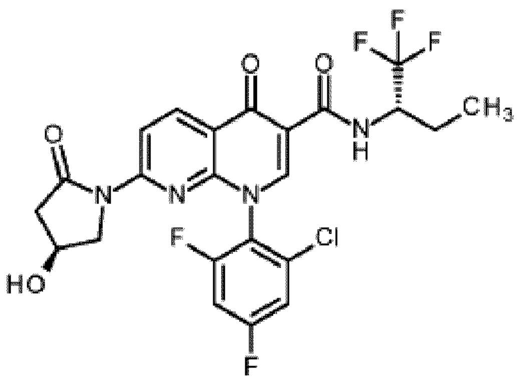

10. Compound according to Claim 1, in which the compound of the formula (I) has the following structure:

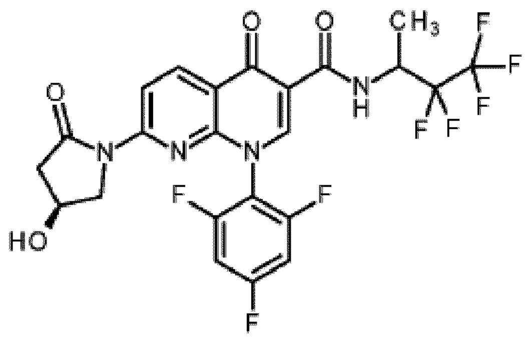

11. Compound according to Claim 1, in which the compound of the formula (I) has the following structure:

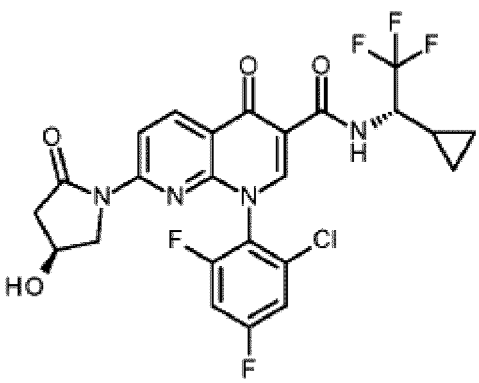

**12.** Process for preparing compounds of the formula (I) as defined in any of Claims 1 to 11, **characterized in that**

[A] a compound of the formula (II)

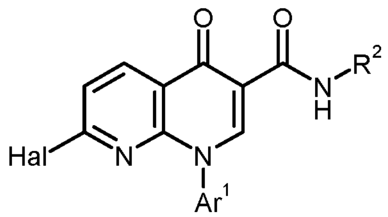

(II)

in which $R^2$ and $Ar^1$ have the definitions given above
and
Hal is fluorine, chlorine, bromine or iodine, preferably chlorine,
is reacted with a compound of the formula (III)

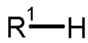

(III)

in which $R^1$ has the definition given above
to give the carboxamide of the formula (I)

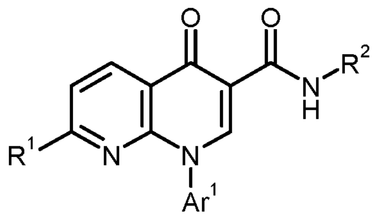

(I)

in which $R^1$, $R^2$ and $Ar^1$ have the definitions given above,
or
[B] a compound of the formula (IV)

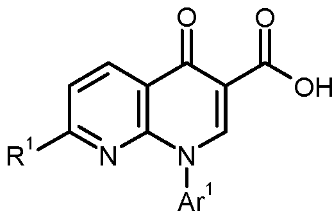

(IV)

in which $R^1$ and $Ar^1$ have the definitions given above is reacted with a compound of the formula (V)

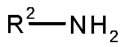

(V)

in which $R^2$ has the definition given above
to give the carboxamide of the formula (I)

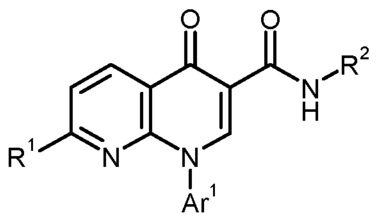

(I)

in which $R^1$, $R^2$ and $Ar^1$ have the definitions given above,
and, if appropriate, the compounds of the formula (I) thus obtained are separated into their enantiomers and/or diastereomers and/or converted with the appropriate (i) solvents and/or (ii) bases or acids to their solvates, salts and/or solvates of the salts.

**13.** Compound of the formula (II)

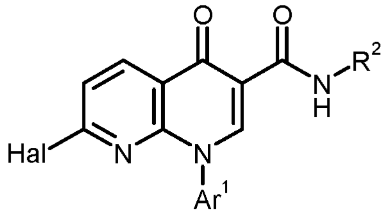

(II)

in which $R^2$ and $Ar^1$ have the definitions given above for compounds of the formula (I) and

Hal is fluorine, chlorine, bromine or iodine, preferably chlorine.

**14.** Compound of the formula (IV)

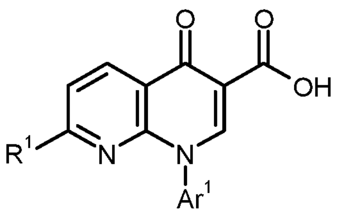

(IV)

in which $R^1$ and $Ar^1$ have the definitions given above for compounds of the formula (I).

**15.** Use of a compound of the formula (II)

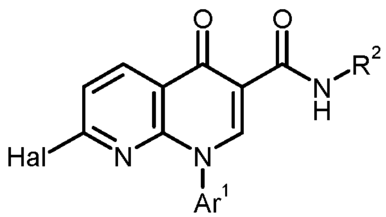

(II)

in which $R^2$ and $Ar^1$ have the definitions given above for compounds of the formula (I) and
Hal is fluorine, chlorine, bromine or iodine, preferably chlorine,
or

of a compound of the formula (IV)

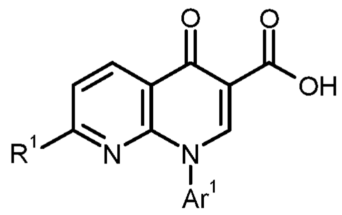

(IV)

in which $R^1$ and $Ar^1$ have the definitions given above for compounds of the formula (I) for preparation of a compound of the formula (I) according to any of Claims 1 to 11.

**16.** Compound as defined in any of Claims 1 to 11 for use in the treatment and/or prophylaxis of diseases.

**17.** Compound of the formula (I) as defined in any of Claims 1 to 11 for use in a method of treatment and/or prophylaxis of heart failure, coronary heart disease, atrial and ventricular arrhythmia, renal failure and nephropathy.

**18.** Medicament comprising a compound as defined in any of Claims 1 to 11 in combination with one or more further active ingredients selected from the group consisting of active hypotensive ingredients, active antiarrhythmic ingredients, vasopressin receptor antagonists, PDE 5 inhibitors, platelet aggregation inhibitors, sGC activators and sGC stimulators.

**19.** Medicament comprising a compound as defined in any of Claims 1 to 11 in combination with an inert, nontoxic, pharmaceutically suitable excipient.

**20.** Medicament according to Claim 18 or 19 for use in the treatment and/or prophylaxis of heart failure, coronary heart disease, atrial and ventricular arrhythmia, renal failure and nephropathy.

## Revendications

**1.** Composé de formule (I)

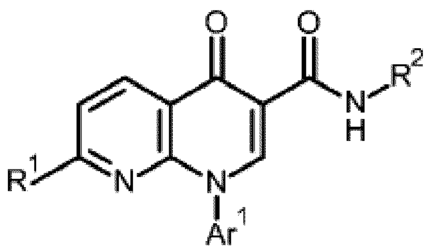

(I),

dans laquelle

$R^1$ représente $NR^4R^5$,
dans lequel

$R^4$ signifie hydrogène, méthyle, ($C_2$-$C_4$)-alkyle ou ($C_3$-$C_6$)-cycloalkyle, ($C_2$-$C_4$)-alkyle pouvant être substitué par hydroxy ou jusqu'à trois fois par fluor,
et
$R^5$ signifie ($C_1$-$C_6$)-alkyle, ($C_3$-$C_6$)-cycloalkyle, hétérocyclyle saturé à 3 à 6 chaînons ou ($C_1$-$C_4$)-alkylsulfonyle, ($C_1$-$C_6$)-alkyle, ($C_3$-$C_6$)-cycloalkyle et hétérocyclyle saturé à 3 à 6 chaînons pouvant être substitués jusqu'à trois fois, de manière identique ou différente, par méthyle, difluorométhyle, trifluorométhyle, hydroxy, hydroxycarbonyle, oxo, méthoxy, difluorométhoxy, trifluorométhoxy et cyano, et en outre jusqu'à quatre fois par fluor,

ou

$R^4$ et $R^5$ forment conjointement avec l'atome d'azote auquel ils sont liés, un hétérocycle saturé ou partiellement insaturé, monocyclique à 3 à 6 chaînons ou bicyclique à 6 à 10 chaînons, qui peut contenir, en tant qu'élément de cycle, un ou deux autres hétéroatomes, identiques ou différents, de la série N, O, S, SO et/ou $SO_2$,

l'hétérocycle monocyclique à 3 à 6 chaînons et l'hétérocycle bicyclique à 6 à 10 chaînons pouvant être substitués à chaque fois par 1 à 5 substituants choisis indépendamment les uns des autres dans le groupe de $(C_1\text{-}C_4)$-alkyle, difluorométhyle, trifluorométhyle, hydroxy, hydroxycarbonyle, oxo, $(C_1\text{-}C_3)$-alcoxy, difluorométhoxy, trifluorométhoxy, cyano, $(C_1\text{-}C_3)$-alcoxycarbonyle, aminocarbonyle, mono-$(C_1\text{-}C_3)$-alkylaminocarbonyloxy, -NHC(=O)$R^{22A}$ et-$CH_2$NHC(=O)$R^{22B}$, et en outre jusqu'à quatre fois par fluor, dans lesquels

$R^{22A}$ et $R^{22B}$ représentent indépendamment l'un de l'autre $(C_1\text{-}C_3)$-alkyle ou cyclopropyle,

et

dans lequel $(C_1\text{-}C_4)$-alkyle peut être substitué une ou deux fois, de manière identique ou différente, par hydroxy, $(C_1\text{-}C_3)$-alcoxy, et jusqu'à quatre fois par fluor,

$R^2$ représente un groupe de formule

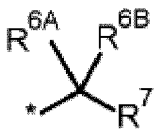 , 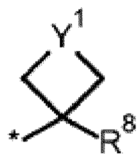 ou 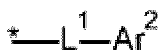 ,

dans lesquelles

* marque le point de liaison avec l'atome N du groupement amide,

$R^{6A}$ signifie hydrogène ou $(C_1\text{-}C_4)$-alkyle,

$R^{6B}$ signifie hydrogène, $(C_1\text{-}C_4)$-alkyle, cyclopropyle, trifluorométhyle, méthoxyméthyle ou trifluorométhoxyméthyle,

$R^7$ signifie $(C_1\text{-}C_4)$-alkyle, cyclopropyle ou cyclobutyle,

$(C_1\text{-}C_4)$-alkyle pouvant être substitué jusqu'à cinq fois par fluor et cyclopropyle et cyclobutyle pouvant être substitués jusqu'à quatre fois par fluor,

$Y^1$ signifie $-(CH_2)_k-$, $-CF_2-$, $-O-CH_2-$, $-CH_2-O-$ ou $-CH_2-O-CH_2-$,

dans lequel

k représente 0, 1, 2 ou 3,

$R^8$ signifie $(C_1\text{-}C_2)$-alkyle substitué jusqu'à cinq fois par fluor ou trifluorométhoxyméthyle,

$L^1$ signifie une liaison ou un groupe de formule $-C(R^{9A}R^{9B})-(C(R^{10A}R^{10B}))_m-$,

dans laquelle

m représente 0 ou 1,

$R^{9A}$ représente hydrogène ou méthyle,

$R^{9B}$ représente hydrogène, méthyle, trifluorométhyle, pentafluoroéthyle ou trifluorométhoxyméthyle,

$R^{10A}$ et $R^{10B}$ représentent indépendamment l'un de l'autre hydrogène ou méthyle,

$Ar^2$ signifie phényle, phényle pouvant être substitué une à trois fois, de manière identique ou différente, par fluor, chlore, $(C_1\text{-}C_3)$-alkyle, difluorométhoxyméthyle, trifluorométhoxyméthyle et/ou trifluorométhyle,

ou

représente un carbocycle bicyclique ou tricyclique à 5 à 10 chaînons,

le carbocycle bicyclique ou tricyclique à 5 à 10 chaînons pouvant être substitué jusqu'à trois fois, de manière identique ou différente, par $(C_1\text{-}C_3)$-alkyle, trifluorométhyle, et en outre jusqu'à quatre fois par fluor,

$Ar^1$ représente un groupe de formule

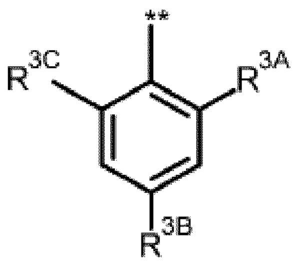

,

dans laquelle

** marque le point de liaison avec l'atome de N,
$R^{3A}$ représente fluor, chlore ou trifluorométhyle,
$R^{3B}$ représente hydrogène ou fluor
et
$R^{3C}$ représente hydrogène, fluor ou chlore

ainsi que N-oxydes, sels, solvates, sels des *N*-oxydes et solvates des N-oxydes et des sels.

**2.** Composé de formule (I) selon la revendication 1, dans laquelle

$R^1$ représente $NR^4R^5$,
dans lequel

$R^4$ signifie hydrogène ou méthyle,
et
$R^5$ signifie ($C_1$-$C_4$)-alkyle ou méthylsulfonyle, ($C_1$-$C_4$)-alkyle pouvant être substitué jusqu'à deux fois par hydroxy, et en outre jusqu'à trois fois par fluor,
ou
$R^4$ et $R^5$ forment conjointement avec l'atome d'azote auquel ils sont liés, un hétérocycle saturé ou partiellement insaturé, monocyclique à 4 à 6 chaînons ou bicyclique à 6 à 10 chaînons, qui peut contenir, en tant qu'élément de cycle, un ou deux autres hétéroatomes de la série N, O, S, SO ou $SO_2$,
$R^2$ l'hétérocycle monocyclique à 4 à 6 chaînons et l'hétérocycle bicyclique à 6 à 10 chaînons pouvant être substitués à chaque fois par 1 à 5 substituants choisis indépendamment les uns des autres dans le groupe de ($C_1$-$C_3$)-alkyle, difluorométhyle, trifluorométhyle, hydroxyméthyle, hydroxyéthyle, hydroxy, oxo, méthoxy, difluorométhoxy, trifluorométhoxy, méthoxyméthyle, cyano, méthoxycarbonyle, aminocarbonyle, monométhylaminocarbonyloxy, et en outre jusqu'à quatre fois par fluor, représente un groupe de formule

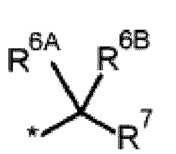 , 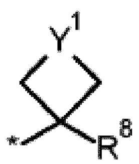 , 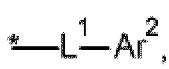 , 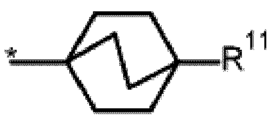 ,

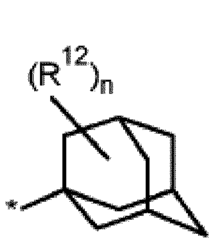 ou 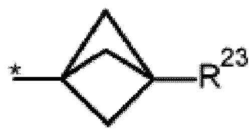 ,

dans lesquelles

* marque le point de liaison avec l'atome N du groupement amide,
$R^{6A}$ signifie hydrogène ou méthyle,
$R^{6B}$ signifie hydrogène, $(C_1-C_4)$-alkyle, cyclopropyle, trifluorométhyle ou trifluorométhoxyméthyle,
$R^7$ signifie $(C_1-C_4)$-alkyle, cyclopropyle ou cyclobutyle,
$(C_1-C_4)$-alkyle pouvant être substitué jusqu'à cinq fois par fluor,
$Y^1$ signifie $-(CH_2)_k-$, $-CF_2-$, $-O-CH_2-$, $-CH_2-O-$ ou $-CH_2-O-CH_2-$,
dans lequel

k représente 0, 1, 2 ou 3,

$R^8$ signifie méthyle, trifluorométhyle ou 2,2,2-trifluoroéthyle,
$L^1$ signifie une liaison ou un groupe de formule $-CR^{9A}R^{9B}-$, dans laquelle
$R^{9A}$ représente hydrogène ou méthyle,
$R^{9B}$ représente hydrogène, méthyle, trifluorométhyle, ou trifluorométhoxyméthyle,
$Ar^2$ signifie phényle, qui peut être substitué une ou deux fois, de manière identique ou différente, par fluor, chlore, méthyle et/ou trifluorométhyle,
$R^{11}$, $R^{12}$ et $R^{23}$ signifient indépendamment les uns des autres hydrogène, fluor, méthyle, éthyle ou trifluorométhyle,
n représente le nombre 1 ou 2, où, dans le cas où l'un des substituants $R^{11}$, $R^{12}$ ou $R^{23}$ est à chaque fois présent deux fois, ses significations peuvent être indépendamment l'une de l'autre identiques ou différentes,

$Ar^1$ représente un groupe de formule

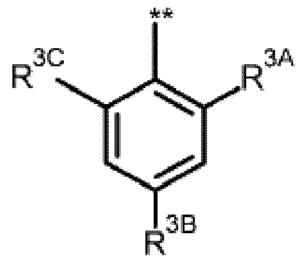

,

dans laquelle

** marque le point de liaison avec l'atome de N,
$R^{3A}$ représente fluor, chlore ou trifluorométhyle,
$R^{3B}$ représente hydrogène ou fluor
et
$R^{3C}$ représente hydrogène, fluor ou chlore ainsi que leurs sels, solvates et solvates des sels.

**3.** Composé de formule (I) selon la revendication 1 ou 2, dans laquelle

$R^1$ représente $NR^4R^5$,
dans lequel

$R^4$ signifie hydrogène ou méthyle,
et
$R^5$ signifie méthyle, isopropyle, 2,2-difluoroéthyle, 2,2,2-trifluoroéthyle, 2-hydroxyéthyle ou 2-hydroxypropyle,
ou
représente un hétérocycle lié par l'intermédiaire d'un atome d'azote, monocyclique à 4 à 6 chaînons ou bicyclique à 6 à 8 chaînons, de formule

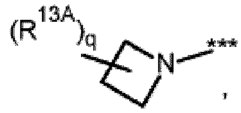 , 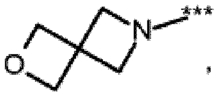 , 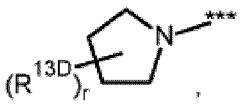 , 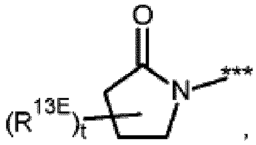 ,

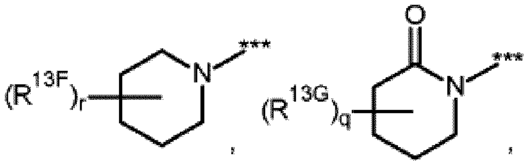 , 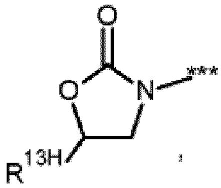 , 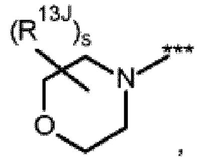 ,

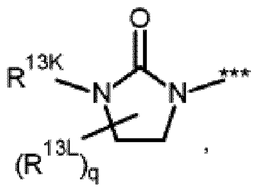 , 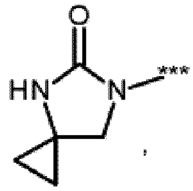 , 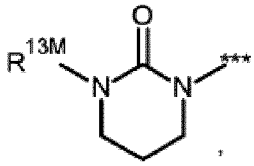 , 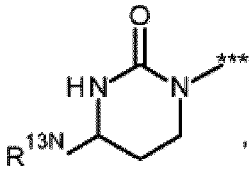 ,

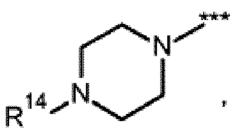 , 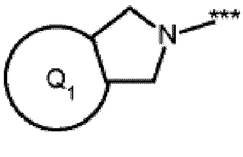 ou 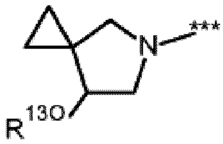 ,

dans lesquelles

*** marque le point de liaison avec l'atome de C du cycle pyridine, le cycle $Q_1$ représente un groupe de formule

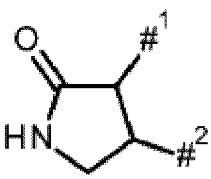 , 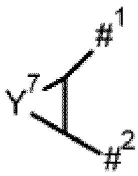 ou 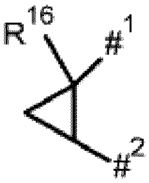 ,

dans lesquelles

$\#^1$ et $\#^2$ marquent le point de liaison avec l'atome de C du cycle pyrrolidine,
et
$Y^7$ représente $-CF_2-$ ou $-CHR^{15}-$, dans lequel
$R^{15}$ représente méthoxyméthyle,

et $R^{16}$ représente hydroxy, $R^{13A}$
signifie fluor, hydroxy, hydroxyméthyle, méthyle, trifluorométhyle ou méthoxy,
$R^{13D}$ signifie hydrogène, fluor, méthyle, hydroxy, hydroxyméthyle, méthoxy ou difluorométhoxy,
$R^{13E}$ signifie hydrogène, fluor, méthyle, hydroxy, hydroxyméthyle ou méthoxy,
$R^{13F}$ signifie fluor, méthyle, hydroxy, hydroxyméthyle ou cyano,
$R^{13G}$ signifie fluor ou hydroxy,
$R^{13H}$ signifie hydrogène, méthyle, hydroxyméthyle, aminocarbonyle ou méthoxycarbonyle,
$R^{13J}$ signifie oxo, hydroxyméthyle ou difluorométhyle,
$R^{13K}$ signifie hydrogène, méthyle ou 2-hydroxyéthyle,
$R^{13L}$ signifie hydrogène ou méthyle,
$R^{13M}$ signifie éthyle, 2-hydroxyéthyle ou cyano,
$R^{13N}$ signifie hydrogène ou éthyle,

$R^{13O}$ signifie hydrogène ou hydroxy,
$R^{14}$ signifie méthyle, méthoxycarbonyle ou aminocarbonyle,
q signifie le nombre 0, 1 ou 2,
r signifie le nombre 0, 1, 2 ou 3,
s signifie le nombre 0 ou 1,
t signifie le nombre 0, 1, 2, 3 ou 4, dans lequel au cas où les substituants $R^{13A}$, $R^{13D}$, $R^{13E}$, $R^{13F}$, $R^{13G}$, $R^{13J}$ et $R^{13L}$ sont présents plusieurs fois, leurs significations peuvent être à chaque fois identiques ou différentes,
$R^2$ représente un groupe de formule

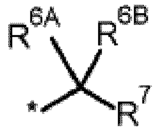 , 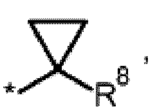 , 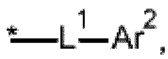 , 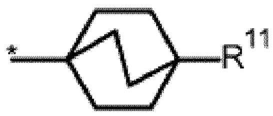 ,

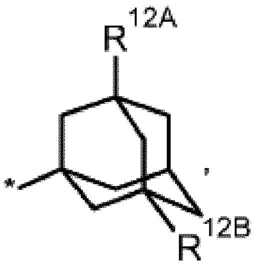 , 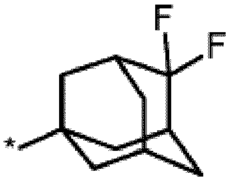 ou 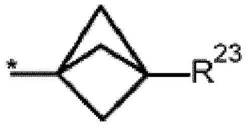 ,

dans lesquelles

* marque le point de liaison avec l'atome de N du groupement amide,
$R^{6A}$ signifie hydrogène ou méthyle,
$R^{6B}$ signifie méthyle, éthyle, cyclopropyle, trifluorométhyle ou trifluorométhoxyméthyle, $R^7$ signifie méthyle, éthyle, n-propyle, isopropyle, tert-butyle, 2-méthylprop-1-yle, trifluorométhyle, difluorométhyle, pentafluoroéthyle, 2,2,2-trifluoroéthyle ou cyclopropyle,
$R^8$ signifie 2,2,2-trifluoroéthyle,
$L^1$ signifie une liaison ou un groupe de formule $-CR^{9A}R^{9B}-$,
dans laquelle

$R^{9A}$ représente hydrogène ou méthyle,
$R^{9B}$ représente hydrogène méthyle, trifluorométhyle ou trifluorométhoxyméthyle,

$Ar^2$ signifie phényle, qui peut être substitué une ou deux fois, de manière identique ou différente, par fluor, chlore, méthyle et/ou trifluorométhyle,
$R^{11}$ signifie hydrogène, fluor ou méthyle,
$R^{12A}$ signifie hydrogène, fluor, méthyle, éthyle ou trifluorométhyle,
$R^{12B}$ signifie hydrogène ou fluor,
$R^{23}$ signifie hydrogène, fluor ou trifluorométhyle, et

$Ar^1$ représente un groupe de formule

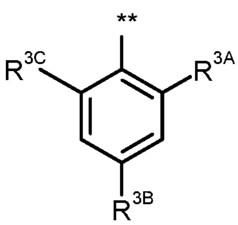 ,

dans laquelle

** marque le point de liaison avec l’atome de N,
$R^{3A}$ représente fluor ou chlore,
$R^{3B}$ représente hydrogène ou fluor,
et
$R^{3C}$ représente hydrogène, fluor ou chlore

ainsi que ses sels, solvates et solvates des sels.

**4.** Composé de formule (I) selon l’une quelconque des revendications 1 à 3, dans laquelle

$R^1$ représente un groupe de formule

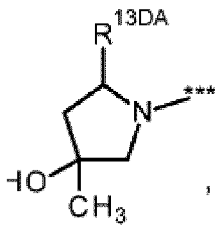 , 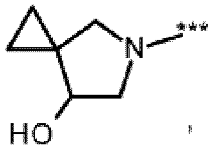 , 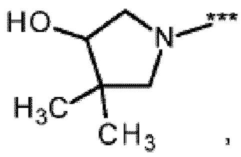 , 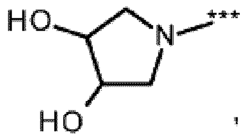 ,

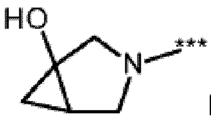 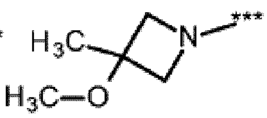 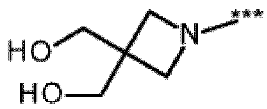 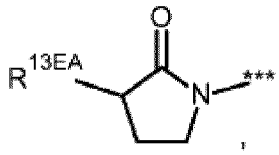 ,

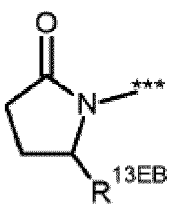 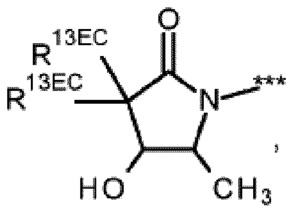 , 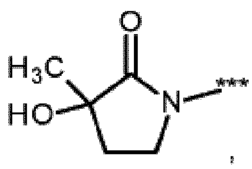 , 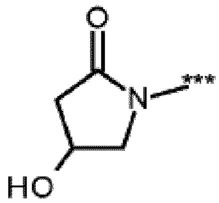

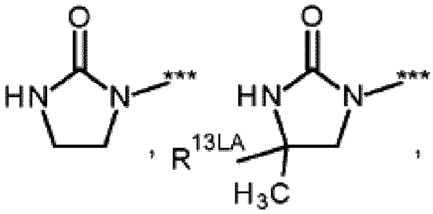 , 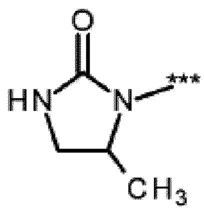 ou 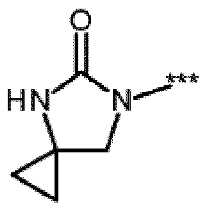

dans lesquelles

*** marque le point de liaison avec l’atome de C du cycle pyridine,
$R^{13DA}$ signifie hydrogène ou méthyle,
$R^{13EA}$ signifie hydroxy ou hydroxyméthyle,
$R^{13EB}$ signifie méthyle ou hydroxyméthyle,
$R^{13EC}$ signifie hydrogène ou méthyle,
$R^{13LA}$ signifie hydrogène ou méthyle,

$R^2$ représente un groupe de formule

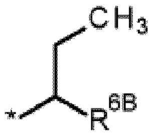 , 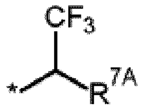 , 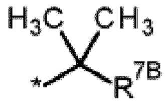 , 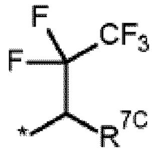 ou 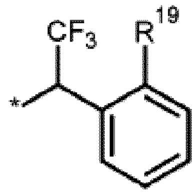 ,

dans lesquelles

* marque le point de liaison avec l'atome de N du groupement amide,
$R^{6B}$ signifie trifluorométhoxyméthyle,
$R^{7A}$ signifie méthyle, éthyle, trifluorométhyle ou cyclopropyle,
$R^{7B}$ signifie trifluorométhyle, difluorométhyle ou 2,2,2-trifluoroéthyle,
$R^{7C}$ signifie méthyle ou éthyle,
$R^{19}$ signifie chlore,

et
$Ar^1$ représente un groupe de formule

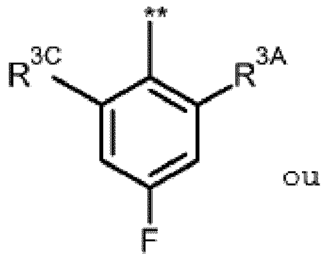 ou 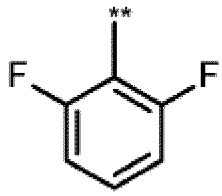 ,

dans lesquelles
** marque le point de liaison avec l'atome de N,
$R^{3A}$ représente fluor ou chlore, et
$R^{3C}$ représente hydrogène ou fluor,

ainsi que ses sels, solvates et solvates des sels.

**5.** Composé de formule (I) selon l'une quelconque des revendications 1 à 4, dans laquelle

$R^1$ représente un groupe de formule

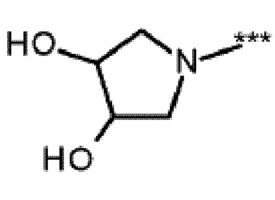 ou 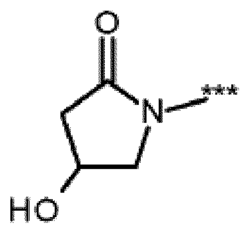 ,

dans lesquelles
*** marque le point de liaison avec l'atome de C du cycle pyridine,
$R^2$ représente un groupe de formule

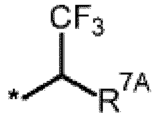, 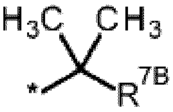 ou 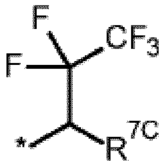,

dans lesquelles

* marque le point de liaison avec l'atome de N du groupement amide,
$R^{7A}$ signifie éthyle, trifluorométhyle ou cyclopropyle,
$R^{7B}$ signifie trifluorométhyle,
$R^{7C}$ signifie méthyle ou éthyle,

et
$Ar^1$ représente un groupe de formule

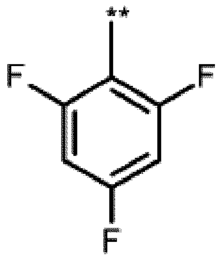 ou 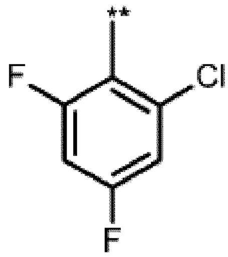,

dans lesquelles
** marque le point de liaison avec l'atome de N, ainsi que ses sels, solvates et solvates des sels.

**6.** Composé de formule (I) selon l'une quelconque des revendications 1 à 5, dans laquelle $R^1$ représente un groupe de formule

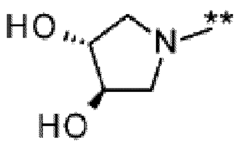 ou 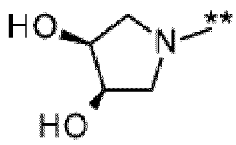,

dans lesquelles
** marque le point de liaison avec l'atome de C du cycle pyridine.

**7.** Composé selon la revendication 1, le composé de formule (I) possédant la structure suivante

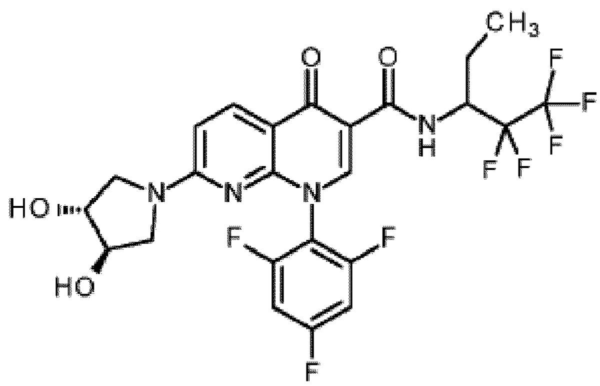

.

8. Composé selon la revendication 1, le composé de formule (I) possédant la structure suivante

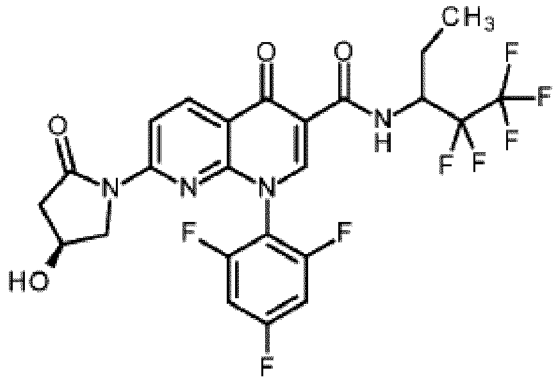

.

9. Composé selon la revendication 1, le composé de formule (I) possédant la structure suivante

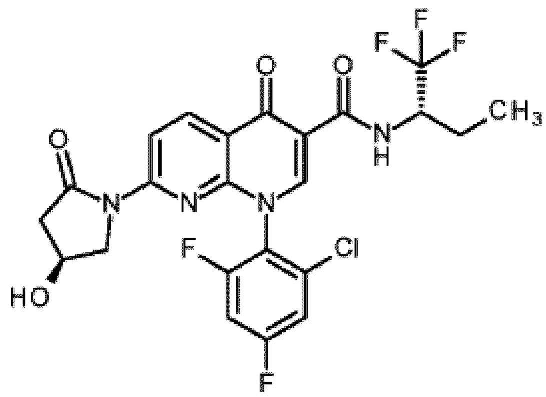

.

10. Composé selon la revendication 1, le composé de formule (I) possédant la structure suivante

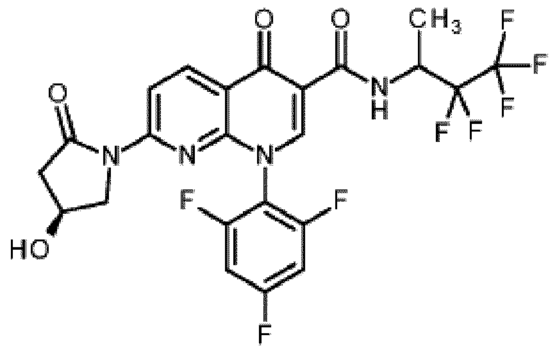

.

**11.** Composé selon la revendication 1, le composé de formule (I) possédant la structure suivante

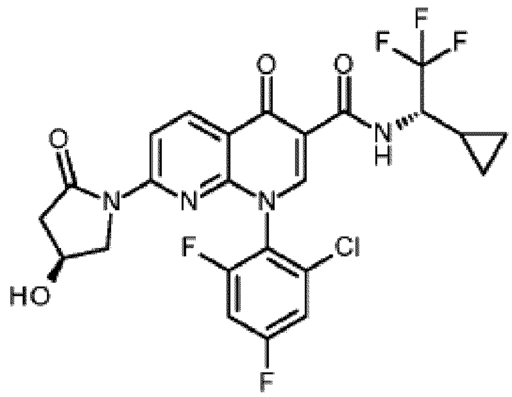

.

**12.** Procédé pour la préparation de composés de formule (I) comme définis dans l'une quelconque des revendications 1 à 11, **caractérisé en ce qu'**on transforme

[A] un composé de formule (II)

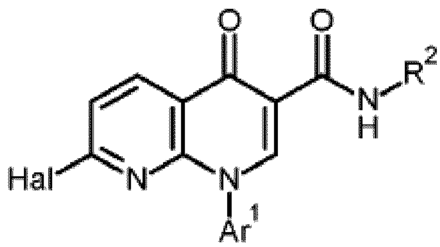

(II),

dans laquelle $R^2$ et $Ar^1$ possèdent les significations mentionnées ci-dessus
et
Hal représente fluor, chlore, brome ou iode, préférablement chlore,
avec un composé de formule (III)

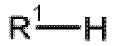

(III),

dans laquelle $R^1$ possède la signification mentionnée ci-dessus,
en amide d'acide carboxylique de formule (I)

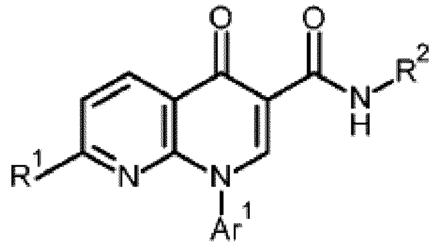

(I),

dans laquelle $R^1$, $R^2$ et $Ar^1$ possèdent les significations mentionnées ci-dessus,
ou
[B] un composé de formule (IV)

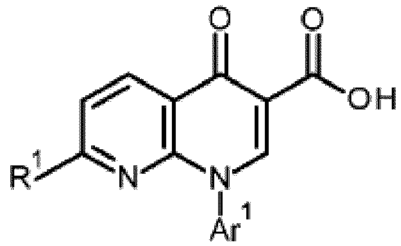

(IV),

dans laquelle $R^1$ et $Ar^1$ possèdent les significations mentionnées ci-dessus,
avec un composé de formule (V)

$$R^2—NH_2 \quad (V),$$

dans laquelle $R^2$ possède la signification mentionnée ci-dessus,
en amide de l'acide carboxylique de formule

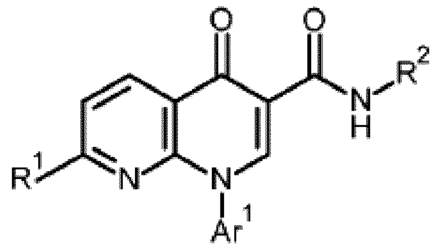

(I),

dans laquelle $R^1$, $R^2$ et $Ar^1$ possèdent les significations mentionnées ci-dessus, et éventuellement on sépare les composés de formule (I) ainsi obtenus en leurs énantiomères et/ou leurs diastéréoisomères et/ou on les convertit en leurs solvates, leurs sels et/ou leurs solvates des sels avec (*i*) les solvants et/ou (ii) les bases ou les acides correspondant(e)s.

**13.** Composé de formule (II)

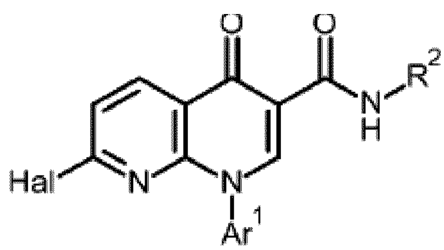

(II),

dans laquelle $R^2$ et $Ar^1$ possèdent les significations mentionnées ci-dessus pour les composés de formule (I) et
Hal représente fluor, chlore, brome ou iode, préférablement chlore.

**14.** Composé de formule (IV)

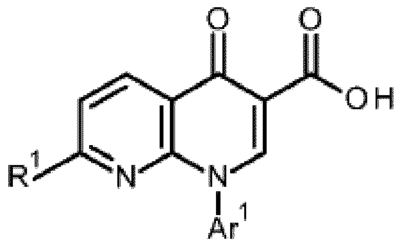

(IV),

dans laquelle $R^1$ et $Ar^1$ possèdent les significations mentionnées ci-dessus pour les composés de formule (I).

**15.** Utilisation d'un composé de formule (II)

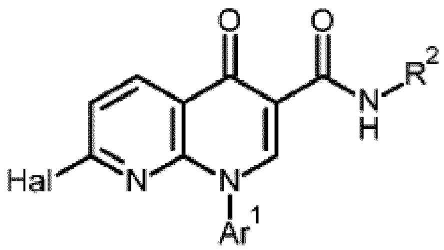

(II),

dans laquelle $R^2$ et $Ar^1$ possèdent les significations mentionnées ci-dessus pour les composés de formule (I) et
Hal représente fluor, chlore, brome ou iode, préférablement chlore,
ou
d'un composé de formule (IV)

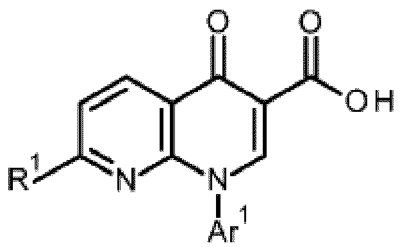

(IV),

dans laquelle $R^1$ et $Ar^1$ possèdent les significations mentionnées ci-dessus pour les composés de formule (I), pour la préparation d'un composé de formule (I) selon l'une quelconque des revendications 1 à 11.

**16.** Composé, comme défini dans l'une quelconque des revendications 1 à 11, pour une utilisation dans le traitement et/ou la prophylaxie de maladies.

**17.** Composé de formule (I), comme défini dans l'une quelconque des revendications 1 à 11, pour une utilisation dans un procédé pour le traitement et/ou la prophylaxie de l'insuffisance cardiaque, de maladies cardiaques coronariennes, d'arythmies atriales et ventriculaires, de l'insuffisance rénale et de néphropathies.

**18.** Médicament contenant un composé, comme défini dans l'une quelconque des revendications 1 à 11, en combinaison avec un ou plusieurs principes actifs supplémentaires choisis dans le groupe constitué par les principes actifs hypotenseurs, les principes actifs antiarythmiques, les antagonistes du récepteur de la vasopressine, les inhibiteurs de la PDE 5, les inhibiteurs d'agrégation plaquettaire, les activateurs de sGC et les stimulateurs de sGC.

**19.** Médicament contenant un composé, comme défini dans l'une quelconque des revendications 1 à 11, en combinaison avec un auxiliaire inerte, non toxique, pharmaceutiquement approprié.

**20.** Médicament selon la revendication 18 ou 19 pour une utilisation dans le traitement et/ou la prophylaxie de l'insuffisance cardiaque, de maladies cardiaques coronariennes, d'arythmies atriales et ventriculaires, de l'insuffisance rénale et de néphropathies.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0945435 B1
- WO 2002085886 A2
- WO 2003050107 A1
- WO 2005026145 A2
- WO 2005026165 A1
- WO 2005049602 A1
- EP 1650192 A1
- WO 2005009971 A1
- JP 2005012561 B
- US 3966781 A, J. G. Atkinson
- WO 2012112363 A, K. Kassahun
- EP 0607825 A1
- WO 0006568 A
- WO 0006569 A
- WO 0242301 A
- WO 03095451 A
- WO 0119355 A
- WO 0119776 A
- WO 0119778 A
- WO 0119780 A
- WO 02070462 A
- WO 02070510 A
- DE 4301246
- DE 1121617


### In der Beschreibung aufgeführte Nicht-Patentliteratur


- **SCHRAGE et al.** *Biochem. Pharmacol.,* 2014, vol. 90 (3), 307-319
- **NEUBIG et al.** *Pharmacol Rev.,* 2003, vol. 55, 597-606
- **KRUSE et al.** *Mol Pharmacol.,* 2013, vol. 84 (4), 528-540
- **GREGORY et al.** *Current Neuropharmacol,* 2007, vol. 5 (3), 157-167
- **CLARK ; MITCHELSON.** *Er. J. Pharmac.,* 1976, vol. 58, 323-331
- **CONN et al.** *Nat. Rev. Drug Disc.,* 2009, vol. 8, 41-54
- **CONN et al.** *Nat. Rev. Drug. Disc.,* 2014, vol. 13, 692-708
- **CHRISTOPOULOS.** *Mol. Pharmacol.,* 2014, vol. 86, 463-478
- **WANG et al.** *J. Pharmacol. Exp. Therap,* 2009, vol. 331, 340-348
- **KRUSE et al.** *Nature,* 2013, vol. 504, 101-106
- **CROY et al.** *Molecular Pharmacology,* Juli 2014, vol. 86 (1), 106-115
- **SCHOBER et al.** *Molecular Pharmacology,* Juli 2014, vol. 86 (1), 116-123
- **HE et al.** *Br. J. Pharmacol.,* 2014
- **RANPURIA et al.** *Nephrol Dial Transplant.,* 2008, vol. 23 (2), 444-4499
- **VINIK et al.** *Diabet Med.,* 2011, vol. 28 (6), 643-651
- **SYKORA et al.** *Stroke,* 2009, vol. 40 (12), 678-682
- **HALARIS et al.** *Mod Trends Pharmacopsychiatri,* 2013, vol. 28, 144-161
- **RASH ; AGUIRRE-CAMACHO.** *Atten Defic Hyperact Disord,* 2012, vol. 4 (4), 167-177
- **DE FERRARI.** *J. Cardiovasc. Transl. Res.,* 2014, vol. 7 (3), 310-320
- **DE FERRARI GM et al.** *Eur. Heart J.,* 2011, vol. 32, 847-855
- **PREMCHAND RK et al.** *J. Card. Fail.,* 2014, vol. 20 (11), 808-816
- **ZANNAD et al.** *Eur. Heart J.,* 2015, vol. 36 (7), 425-433
- **GOLD et al.** *J Am Coll Cardiol.,* 2016, vol. 16, 0735-1097
- **CHEN et al.** *Circ. Res.,* 2014, vol. 114 (9), 1500-1515
- **MAISEL ; STEVENSON.** *Am. J. Cardiol.,* 2003, vol. 91, 2D-8D
- **LEVALTER T.** *Fortbildungsprogramm Pharmazie,* 2011, vol. 5, 106-127
- **LEONG-SIT ; TANG.** *Curr. Opin. Cardiol.,* 2015
- **SCAMMELLS et al.** *ACS Chem. Neurosci.,* 2013, vol. 4 (7), 1026-1048
- **MISTRY et al.** *J. Med. Chem.,* 2013, vol. 56, 5151-5172
- Isotopic Compositions of the Elements 1997. *Pure Appl. Chem.,* 1998, vol. 70 (1), 217-235
- **H. J. LEIS et al.** *Curr. Org. Chem.,* 1998, vol. 2, 131
- **ESAKI et al.** *Tetrahedron,* 2006, vol. 62, 10954
- **ESAKI et al.** *Chem. Eur. J.,* 2007, vol. 13, 4052
- **H. J. LEIS et al.** *Curr. Org. Chem,* 1998, vol. 2, 131

- **J. R. MORANDI et al.** *J. Org. Chem.,* 1969, vol. 34 (6), 1889
- **N. H. KHAN.** *J. Am. Chem. Soc.,* 1952, vol. 74 (12), 3018
- **S. CHANDRASEKHAR et al.** *Tetrahedron,* 2011, vol. 52, 3865
- **HANZLIK et al.** *J. Org. Chem.,* 1990, vol. 55, 3992-3997
- **R. P. HANZLIK et al.** *Biochem. Biophys. Res. Commun,* 1989, vol. 160, 844
- **P. J. REIDER et al.** *J. Org. Chem.,* 1987, vol. 52, 3326-3334
- **M. JARMAN et al.** *Carcinogenesis,* 1993, vol. 16 (4), 683-688
- **J. ATZRODT et al.** *Angew. Chem., Int. Ed.,* 2007, vol. 46, 7744
- **K. MATOISHI et al.** *J. Chem. Soc, Chem. Commun,* 2000, 1519-1520
- **A. STREITWIESER et al.** *J. Am. Chem. Soc.,* 1963, vol. 85, 2759
- **C. L. PERRIN et al.** *J. Am. Chem. Soc.,* 2007, vol. 129, 4490
- **C. L. PERRIN et al.** *J. Am. Chem. Soc.,* 2003, vol. 125, 15008
- **C. L. PERRIN.** *Advances in Physical Organic Chemistry,* vol. 44, 144
- **C. L. PERRIN et al.** *J. Am. Chem. Soc.,* 2005, vol. 127, 9641
- **B. TESTA et al.** *Int. J, Pharm.,* 1984, vol. 19 (3), 271
- **D. J. KUSHNER et al.** *Can. J. Physiol. Pharmacol.,* 1999, vol. 77, 79
- **A. E. MUTLIB et al.** *Toxicol. Appl. Pharmacol,* 2000, vol. 169, 102
- **A. M. SHARMA et al.** *Chem. Res.Toxicol,* 2013, vol. 26, 410
- **UETRECHT et al.** *Chemical Research in Toxicology,* 2008, vol. 21 (9), 1862
- **A. J. MORALES et al.** *The 15th North American Meeting of the International Society of Xenobiotics,* 12. Oktober 2008
- **C. J. WENTHUR et al.** *J. Med. Chem.,* 2013, vol. 56, 5208
- **F. SCHNEIDER et al.** *Arzneim. Forsch. Drug. Res,* 2006, vol. 56, 295
- **F. MALTAIS et al.** *J. Med. Chem.,* 2009, vol. 52, 7993
- *Tetrahedron Lett.,* 1977, vol. 18, 4171-4174
- *CHEMICAL ABSTRACTS,* 635309-52-3
- *CHEMICAL ABSTRACTS,* 157373-27-8
- *CHEMICAL ABSTRACTS,* 15166-68-4
- *CHEMICAL ABSTRACTS,* 75702-99-7
- *CHEMICAL ABSTRACTS,* 3699-54-5
- *J. Med. Chem.,* 2011, vol. 54, 7334-7349
- *CHEMICAL ABSTRACTS,* 68108-18-9
- *CHEMICAL ABSTRACTS,* 22677-21-0
- *CHEMICAL ABSTRACTS,* 34368-52-0
- *CHEMICAL ABSTRACTS,* 77510-50-0
- **REUVENY et al.** *Nature,* Juli 1994, vol. 370, 143-146
- Determination of Allosteric Interactions Using Radioligand-Binding Techniques. **LAZARENO.** Methods in Molecular Biology. vol. 259
- **KOSTENIS ; MOHR.** *Trends Pharmacol. Sci.,* 1996, vol. 17 (8), 280-283
- **CROY et al.** *Mol. Pharmacol.,* 2014, vol. 86, 106-115
- **HULME ; TREVETHICK.** *Brit. J. Pharmacol.,* 2010, vol. 161, 1219-1237
- **SCHOBER et al.** *Mol. Pharmacol.,* 2014, vol. 86, 116-123
- **CHENG ; PRUSOFF.** *Biochem. Pharmacol.,* 1973, vol. 22 (23), 3099-3108
- **LAZARENO.** Determination of Allosteric Interactions Using Radioligand-Binding Techniques. *Methods in Molecular Biology,* vol. 259
- **HULME ; TREVETHICK.** *Brit.J. Pharmacol.,* 2010, vol. 161, 1219-1237
- **RINNE et al.** G Protein-Activated (GIRK) Current in Rat Ventricular Myocytes is Masked by Constitutive Inward Rectifier Current (IK1). *Cell Physiol Biochem,* 2008, vol. 21, 259-268